# EUROPEAN PATENT APPLICATION

(11) **EP 4 631 952 A1**
(43) Date of publication of application: **15.10.2025**
(21) Application number: 23915846.2
(22) Date of filing: 29.12.2023
(51) Int. Cl.: C07J 43/00, C07J 3/00, C07J 17/00, A61K 31/56, A61K 47/68, A61P 5/44

(54) **GLUCOCORTICOID RECEPTOR AGONIST AND CONJUGATE THEREOF**

(30) Priority: 10.01.2023 CN 202310031425; 30.06.2023 CN 202310796480
(71) Applicant: Sichuan Kelun-Biotech Biopharmaceutical Co., Ltd., Chengdu, Sichuan 611138 (CN)
(72) Inventor: TIAN, Qiang, Chengdu, Sichuan 611138 (CN); ZHANG, Yitao, Chengdu, Sichuan 611138 (CN); LONG, Hu, Chengdu, Sichuan 611138 (CN); SU, Donghai, Chengdu, Sichuan 611138 (CN); LIAO, Menchang, Chengdu, Sichuan 611138 (CN); LI, Yanlin, Chengdu, Sichuan 611138 (CN); ZHOU, Qin, Chengdu, Sichuan 611138 (CN); SONG, Hongmei, Chengdu, Sichuan 611138 (CN); GE, Junyou, Chengdu, Sichuan 611138 (CN)
(74) Representative: Turner, Craig Robert
(86) International application number: PCT/CN2023/143271
(87) International publication number: WO 2024/149093

(57) **Abstract**

The present application relates to the field of medicines, and in particular to an antibody-drug conjugate of a glucocorticoid receptor agonist. Specifically, the present application provides a glucocorticoid receptor agonist, which has good agonistic activity. The present application further provides a drug-linker compound for coupling, wherein the drug is a glucocorticoid receptor agonist. The present application further provides an antibody-drug conjugate prepared by coupling the drug-linker compound and the antibody, wherein the antibody-drug conjugate has good drug coupling uniformity, and has an excellent treatment effect on inflammatory diseases such as rheumatoid arthritis. The present application further provides a preparation method for the glucocorticoid receptor agonist and the antibody-drug conjugate thereof, and uses of the glucocorticoid receptor agonist and the antibody-drug conjugate thereof in the field of medicines.

## Description

### Technical field

The present application relates to the field of medicines, and in particular to an antibody-drug conjugate of a glucocorticoid receptor agonist. Specifically, the present application provides a glucocorticoid receptor agonist, which has good agonistic activity. The present application further provides a drug-linker compound for conjugation, wherein the drug is a glucocorticoid receptor agonist. The present application further provides an antibody-drug conjugate prepared by conjugating the drug-linker compound to an antibody, which has good drug conjugation uniformity, and has an excellent treatment effect on inflammatory diseases such as rheumatoid arthritis. The present application further provides a preparation method for the glucocorticoid receptor agonist and the antibody-drug conjugate thereof, as well as use of the glucocorticoid receptor agonist and the antibody-drug conjugate thereof in the field of medicines.

### Background

Steroid glucocorticoid receptor agonists are a type of therapeutic drugs that are widely used for inflammatory and immune diseases. Such drugs activate the glucocorticoid receptor in cells, intervene in the recruitment of leukocytes into inflammatory sites, and at the same time inhibit the formation and release of inflammatory mediators from leukocytes and tissue cells, thereby exerting the anti-inflammatory effect. If a large amount of steroid glucocorticoid receptor agonist drugs are used for a long period, it is possible to cause side effects such as infection, gastrointestinal reactions, endocrine disorders (such as full moon face, buffalo hump, weight gain, hypokalemia, abnormal blood sugar and blood pressure, etc.), and osteoporosis. To reduce the above side effects of the steroid glucocorticoid receptor agonist drugs, progress in inhalation preparations or topical preparations for application on skin has been gained; in addition, introduction of metabolically unstable functional groups in a steroid structure to accelerate inactivation and reduce systemic exposure is one of the measures to reduce adverse reactions.

Recently, in order to improve the efficacy and safety of steroid glucocorticoid receptor agonist drugs, conjugation of steroid glucocorticoid receptor agonists to antibodies is a new direction for developing such drugs.

### Summary

The present application relates to a steroid glucocorticoid receptor agonist as well as a linker conjugate and an antibody conjugate thereof. The steroid glucocorticoid receptor agonist and the conjugates thereof have excellent agonistic activity and good druggability, and therefore are expected to be used in the treatment of inflammatory and immune diseases.

In one aspect, the present application provides a steroid glucocorticoid receptor agonist, which is a compound of formula I, a pharmaceutically acceptable salt, a stereisomer, a tautomer, a polymorph, a solvate, an N-oxide, an isotope labeled compound, a metabolite, or a prodrug thereof: 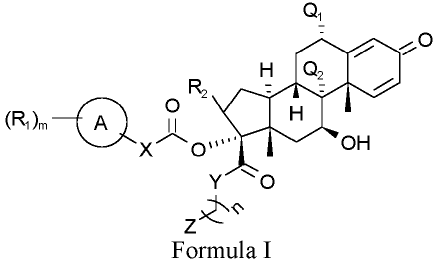 wherein,
R₁ is each independently selected from a group consisting of hydrogen, halogen, - NR^{a}R^{b}, hydroxyl, cyano, C₂₋₆ alkenyl, C₂₋₆ alkynyl, C₁₋₆ alkyl, C₁₋₆ alkyl substituted with one or more hydroxyl, C₁₋₆ alkoxy and C₁₋₆ haloalkyl;
R₂ is selected from a group consisting of hydrogen, C₁₋₆ alkyl, hydroxyl and C₁₋₆ alkoxy;
ring A is selected from a group consisting of a single bond, a C₆₋₁₀ aromatic ring and a 5-6 membered heteroaromatic ring;
X is selected from a group consisting of a single bond, C₁₋₆ alkylene, C₃₋₆ cycloalkylene, C₂₋₆ alkenylidene, C₂₋₆ alkynylidene and -NR^{a}-;
Y is selected from a group consisting of -O- and -S-;
Z is selected from a group consisting of hydroxyl, halogen and cyano;
Q₁ and Q₂ are each independently selected from a group consisting of hydrogen and halogen;
m is selected from a group consisting of 1, 2, 3, 4 and 5;
n is selected from a group consisting of 1 and 2;
R^{a} and R^{b} are each independently selected from a group consisting of hydrogen and C₁₋₆ alkyl;
provided that: when R₁ is each independently selected from a group consisting of halogen, amino and methyl, m is 2, R₂ is methyl, X is a single bond, Y is-S-, Z is fluorine, n is 1, Q₁ is hydrogen or fluorine, and Q₂ is fluorine, ring A is not phenyl;
when ring A is a single bond, R₁ is not hydrogen or C₁₋₆ alkyl; and
when ring A is a 5-6 membered heteroaromatic ring, R₁ is hydrogen and R₂ is C₁₋₆ alkyl, Q₁ is not halogen.

In another aspect, the present application provides an antibody-drug conjugate having a structure represented by formula Ab-[M-L-E-D]ₓ, wherein:
Ab is an antibody specifically binding to an antigen or an antigen binding fragment thereof;
M is a linkage site to the antibody or the antigen binding fragment thereof;
L is a linker between the linkage site M and E;
E is a structural fragment linking L with D;
D is a glucocorticoid drug fragment which is a monovalent structure obtained by losing one H from -OH, -NH₂ or secondary amino on the steroid glucocorticoid receptor agonist of the present invention; and
x is an integer selected from 1 to 10.

In another aspect, the present application provides a drug-linker compound having a structure represented by formula G-M-[L-E-D]ₓ, wherein:
G is a functional group or a leaving group which reacts with a specific amino acid or glycosyl and a derivative thereof in an antibody or an antigen binding fragment;
M is a linkage site to the antibody or the antigen binding fragment thereof;
L is a linker between the linkage site M and E;
E is a structural fragment linking L with D;
D is a glucocorticoid drug fragment which is a monovalent structure obtained by losing one H from -OH, -NH₂ or secondary amino on the steroid glucocorticoid receptor agonist of the present invention; and
x is an integer selected from 1 to 10.

In another aspect, the present application provides a pharmaceutical composition, comprising the steroid glucocorticoid receptor agonist of the present invention, the antibody-drug conjugate of the present invention or the drug-linker compound of the present invention, and one or more pharmaceutically acceptable carriers.

In another aspect, the present application provides use of the steroid glucocorticoid receptor agonist of the present invention, the antibody-drug conjugate of the present invention, the drug-linker compound of the present invention or the pharmaceutical composition of the present invention in the manufacture of a medicament for treating inflammatory or immune diseases.

In another aspect, the present application provides the steroid glucocorticoid receptor agonist of the present invention, the antibody-drug conjugate of the present invention, the drug-linker compound of the present invention or the pharmaceutical composition of the present invention, for use in the treatment of inflammatory or immune diseases.

In another aspect, the present application provides a method for treating an inflammatory or immune disease, comprising administering a therapeutically effective amount of the steroid glucocorticoid receptor agonist of the present invention, the antibody-drug conjugate of the present invention, the drug-linker compound of the present invention or the pharmaceutical composition of the present invention to a subject in need thereof.

In another aspect, the present application provides a method for preparing the compound of the present invention, comprising the following steps: 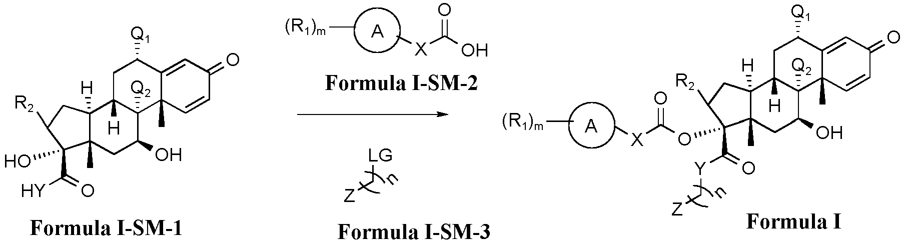 wherein, ring A, R₁, R₂, X, Y, Z, Q₁, Q₂, m and n are as defined above, and LG is a leaving group.

In another aspect, the present application provides a method for preparing the drug-linker compound of the present invention, comprising the following steps: 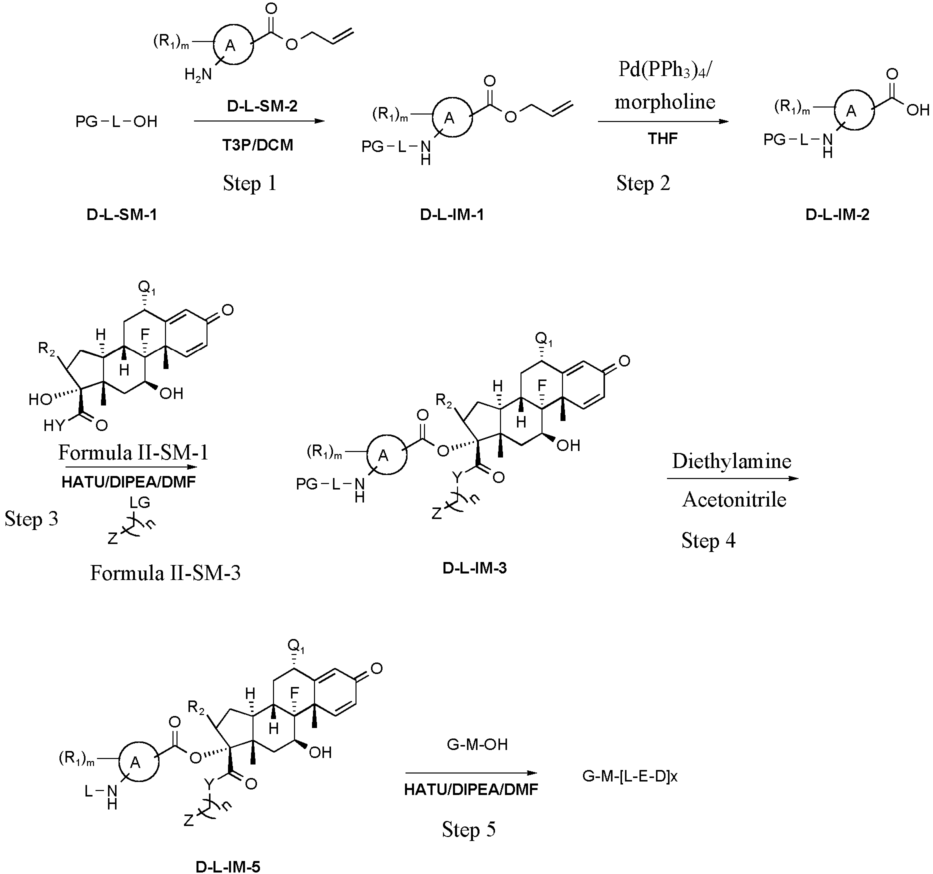 wherein,
ring A, R₁, R₂, X, Y, Z, Q₁, G, M, L, D, m and n are as defined above;
x=1;
E is a single bond;
LG is a leaving group, for example, but not limited to, halogen, methylsulfonyloxy (-OMs) and trifluoromethylsulfonyloxy (-OTf), preferably iodine; and
PG is an amino protecting group, for example, but not limited to, 9-fluorenylmethoxycarbonyl (Fmoc), tert-butoxycarbonyl (Boc), p-methoxytriphenylmethyl (MMt) and allyloxycarbonyl (Alloc), preferably Fmoc.

In another aspect, the present application provides a method for preparing the antibody-drug conjugate of the present invention, which is selected from a group consisting of:
conjugation method A:
   conjugating the drug-linker compound of the present invention with an antibody in a molar ratio of (8-10):1 to obtain the antibody-drug conjugate;
conjugation method B:
   conjugating the drug-linker compound of the present invention with an antibody in a molar ratio of (4-6):1 to obtain the antibody-drug conjugate;
conjugation method C:
   conjugating the drug-linker compound of the present invention with an antibody in a molar ratio of (4.0-4.5):1 to obtain the antibody-drug conjugate.

### Brief description of the drawings

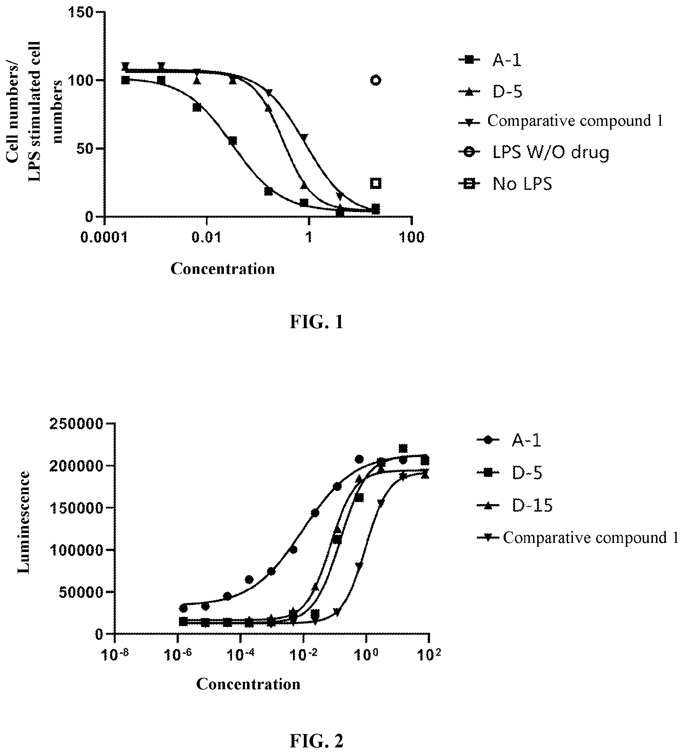

FIG. 1 shows the in vitro inhibitory results of the compound of the present invention on the B cell proliferation in mice.
FIG. 2 shows the in vitro activation results of the compound of the present invention on the activity of the glucocorticoid receptor in cells.
FIG. 3 shows the change in arthritis scores of a mouse CIA model by the compound of the present invention.
FIG. 4 shows the change in body weight of a mouse CIA model by the compound of the present invention.

### Detailed description of the invention

### Steroid glucocorticoid receptor agonist

In one aspect, the present application provides a steroid glucocorticoid receptor agonist, which is a compound of formula I, a pharmaceutically acceptable salt, a stereisomer, a tautomer, a polymorph, a solvate, an N-oxide, an isotope labeled compound, a metabolite, or a prodrug thereof: 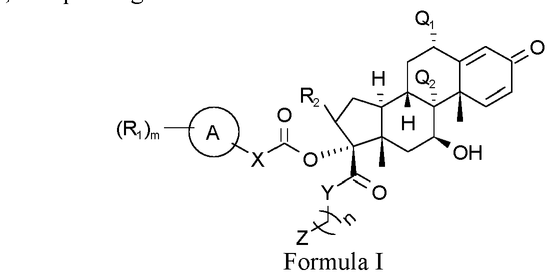 wherein,
R₁ is each independently selected from a group consisting of hydrogen, halogen, - NR^{a}R^{b}, hydroxyl, cyano, C₂₋₆ alkenyl, C₂₋₆ alkynyl, C₁₋₆ alkyl, C₁₋₆ alkyl substituted with one or more hydroxyl, C₁₋₆ alkoxy and C₁₋₆ haloalkyl;
R₂ is selected from a group consisting of hydrogen, C₁₋₆ alkyl, hydroxyl and C₁₋₆ alkoxy;
ring A is selected from a group consisting of a single bond, a C₆₋₁₀ aromatic ring and a 5-6 membered heteroaromatic ring;
X is selected from a group consisting of a single bond, C₁₋₆ alkylene, C₃₋₆ cycloalkylene, C₂₋₆ alkenylidene, C₂₋₆ alkynylidene and -NR^{a}-;
Y is selected from a group consisting of -O- and -S-;
Z is selected from a group consisting of hydroxyl, halogen and cyano;
Q₁ and Q₂ are each independently selected from a group consisting of hydrogen and halogen;
m is selected from a group consisting of 1, 2, 3, 4 and 5;
n is selected from a group consisting of 1 and 2;
R^{a} and R^{b} are each independently selected from a group consisting of hydrogen and C₁₋₆ alkyl;
provided that: when R₁ is each independently selected from a group consisting of halogen, amino and methyl, m is 2, R₂ is methyl, X is a single bond, Y is-S-, Z is fluorine, n is 1, Q₁ is hydrogen or fluorine, and Q₂ is fluorine, ring A is not phenyl;
when ring A is a single bond, R₁ is not hydrogen or C₁₋₆ alkyl; and
when ring A is a 5-6 membered heteroaromatic ring, R₁ is hydrogen and R₂ is C₁₋₆ alkyl, Q₁ is not halogen.

In some embodiments, R₁ is each independently selected from a group consisting of hydrogen, halogen, -NR^{a}R^{b}, hydroxyl, C₁₋₆ alkyl, C₁₋₆ alkyl-OH and C₁₋₆ alkoxy.

In some embodiments, R₁ is each independently selected from a group consisting of hydrogen, fluorine, chlorine, amino, hydroxyl, methyl, ethyl and hydroxymethyl.

In some embodiments, R₁ is each independently selected from a group consisting of hydrogen, fluorine, amino, hydroxyl, methyl, ethyl and hydroxymethyl.

In some embodiments, R₂ is selected from a group consisting of hydrogen, methyl, hydroxyl and methyoxy.

In some embodiments, R₂ is selected from a group consisting of hydrogen and methyl.

In some embodiments, ring A is selected from a group consisting of a single bond, a benzene ring and a 5-6 membered heteroaromatic ring.

In some embodiments, ring A is selected from a group consisting of a single bond, a benzene ring, a pyridine ring, a furan ring and a thiophene ring.

In some embodiments, X is selected from a group consisting of a single bond, methylene, ethylene, propylene, isopropylene, cyclopropylene, butylene, isobutylene, cyclobutylene, cyclohexylene, ethenylidene and imino.

In some embodiments, X is selected from a group consisting of a single bond, methylene, ethylene, propylene, butylene, cyclopropylene, cyclohexylene, ethenylidene and imino

In some embodiments, X is selected from a group consisting of a single bond, methylene, ethylene, propylene, butylene, 1,1-cyclopropylene, 1,2-cyclopropylene, 1,4-cyclohexylene and ethenylidene.

In some embodiments, Z is selected from a group consisting of hydroxyl, fluorine, chlorine and cyano.

In some embodiments, Q₁ and Q₂ are each independently selected from a group consisting of hydrogen, fluorine and chlorine.

In some embodiments, Q₁ is selected from a group consisting of hydrogen and fluorine, and Q₂ is fluorine.

In some embodiments, m is selected from a group consisting of 1 and 2.

In some embodiments, n is 1.

In some embodiments, the steroid glucocorticoid receptor agonist is a compound of formula II, a pharmaceutically acceptable salt, a stereisomer, a tautomer, a polymorph, a solvate, an N-oxide, an isotope labeled compound, a metabolite or a prodrug thereof: wherein, 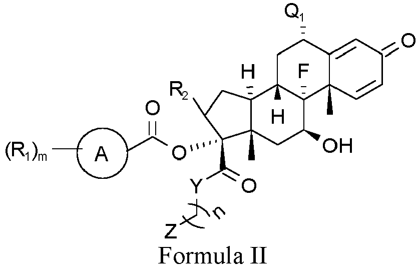 R₁, R₂, ring A, Y, Z, Q₁, m and n are as defined above for the compound of formula I.

In some embodiments, the steroid glucocorticoid receptor agonist is selected from a compound of formula III, a pharmaceutically acceptable salt, a stereisomer, a tautomer, a polymorph, a solvate, an N-oxide, an isotope labeled compound, a metabolite or a prodrug thereof: 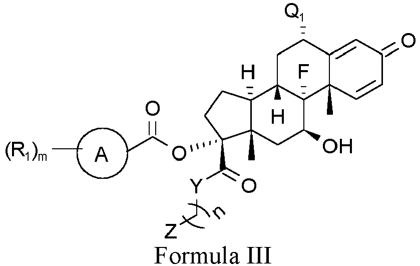 wherein,
R₁, ring A, Y, Z, Q₁, m and n are as defined above for the compound of formula I.

In some embodiments, the steroid glucocorticoid receptor agonist is selected from a compound of formula IV, a pharmaceutically acceptable salt, a stereisomer, a tautomer, a polymorph, a solvate, an N-oxide, an isotope labeled compound, a metabolite or a prodrug thereof: 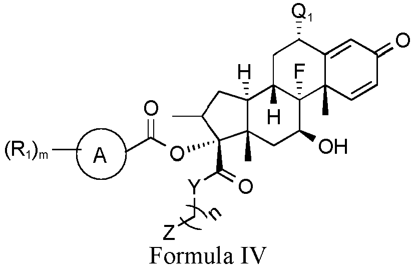 wherein,
R₁, R₂, ring A, Y, Z, Q₁, m and n are as defined above for the compound of formula I.

In some embodiments, the steroid glucocorticoid receptor agonist is selected from a compound of formula V, a pharmaceutically acceptable salt, a stereisomer, a tautomer, a polymorph, a solvate, an N-oxide, an isotope labeled compound, a metabolite or a prodrug thereof: 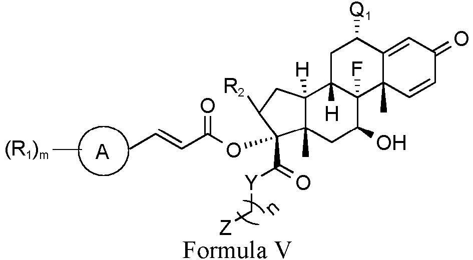 wherein,
R₁, R₂, ring A, Y, Z, Q₁, m and n are as defined above for the compound of formula I.

In some embodiments, the steroid glucocorticoid receptor agonist is selected from a compound of formula VI, a pharmaceutically acceptable salt, a stereisomer, a tautomer, a polymorph, a solvate, an N-oxide, an isotope labeled compound, a metabolite or a prodrug thereof: 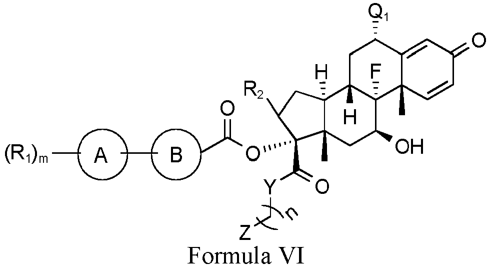 wherein,
ring B is C₃₋₆ cycloalkane, preferably cyclopropane; and
R₁, R₂, ring A, Y, Z, Q₁, m and n are as defined above for the compound of formula I.

In some embodiments, the steroid glucocorticoid receptor agonist is selected from a group consisting of the following compounds, pharmaceutically acceptable salts, stereisomers, tautomers, polymorphs, solvates, N-oxides, isotope labeled compounds, metabolites or prodrugs thereof:

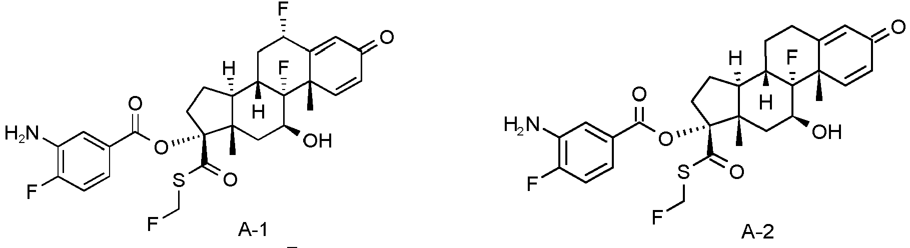

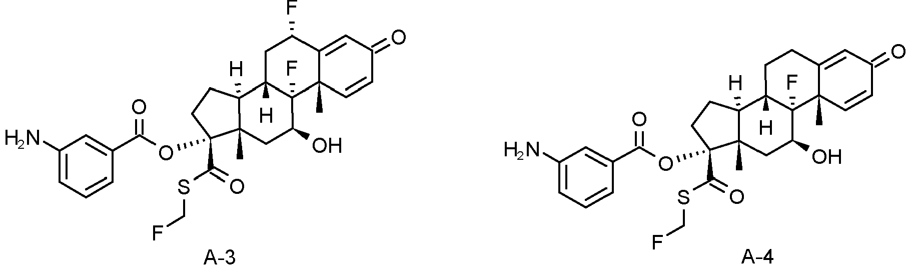

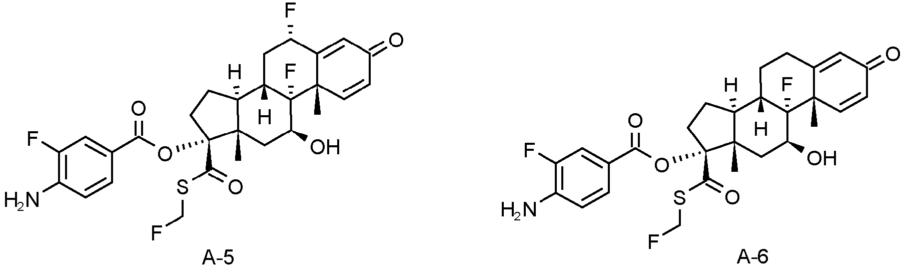

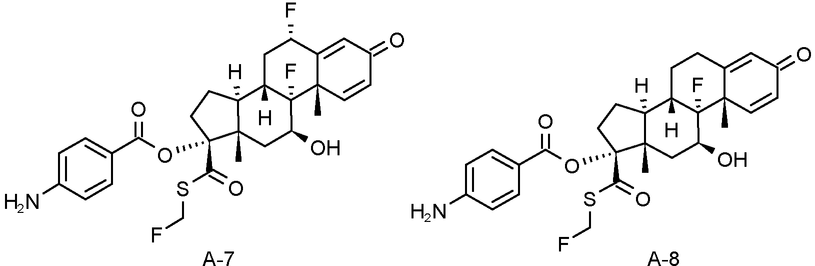

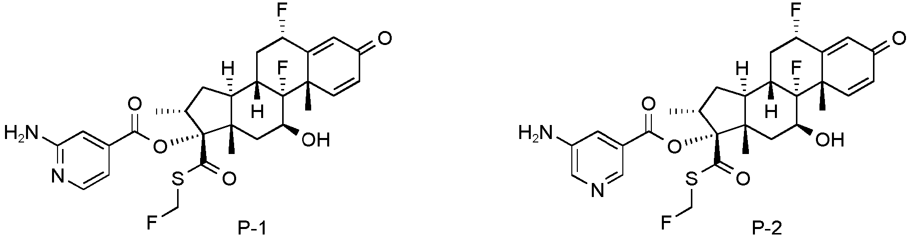

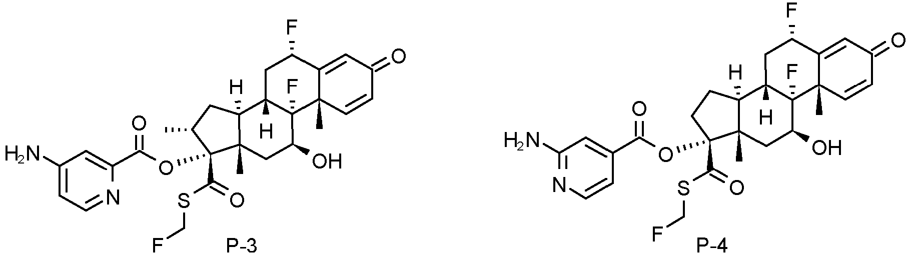

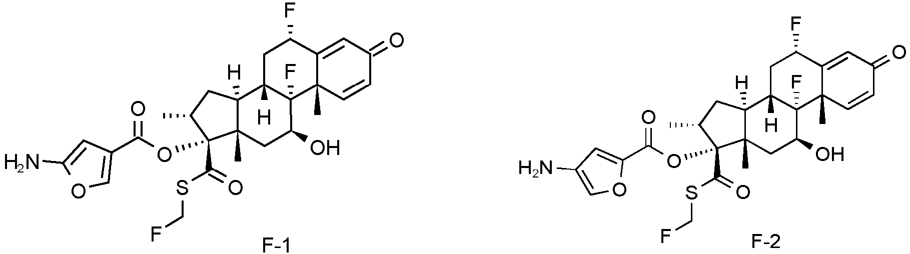

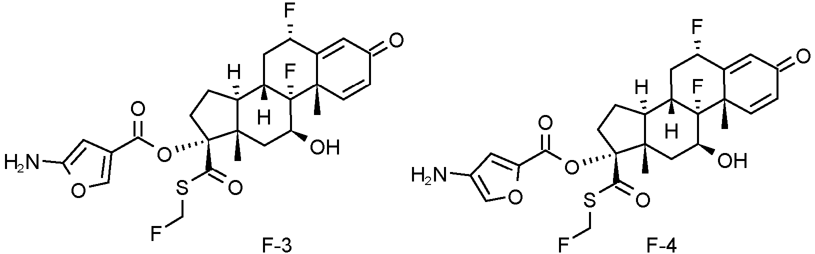

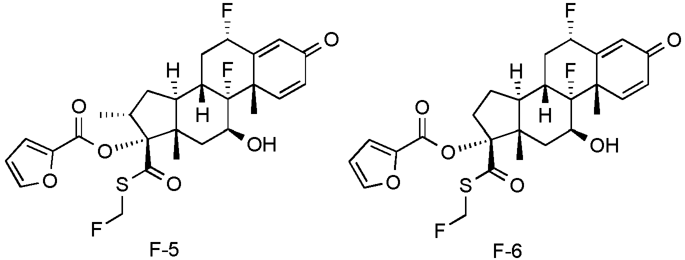

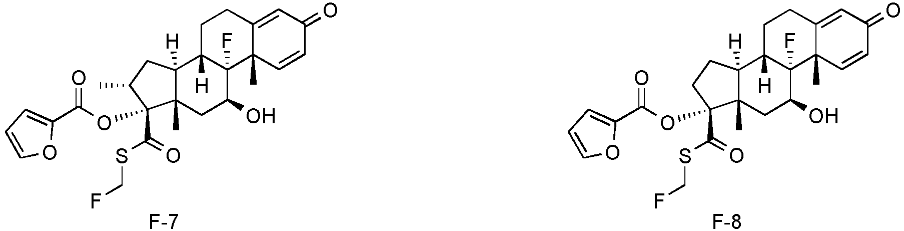

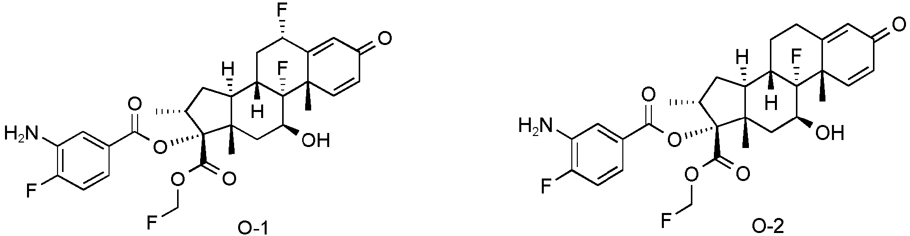

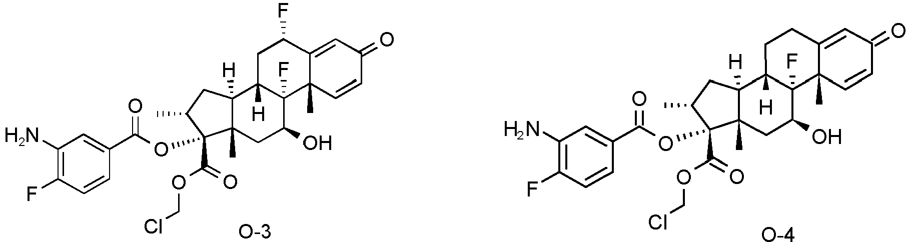

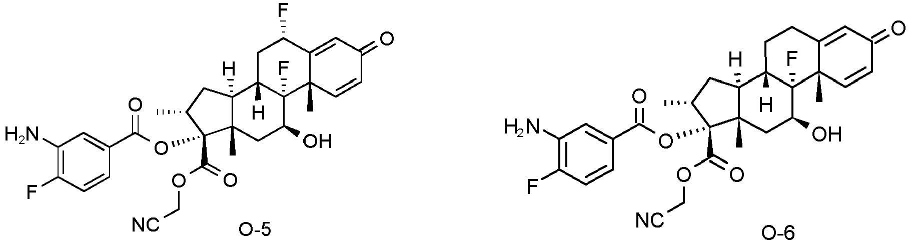

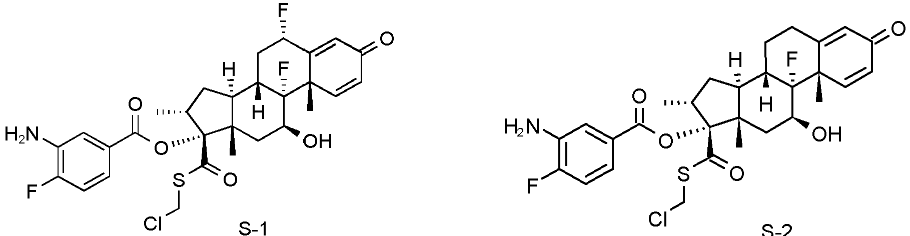

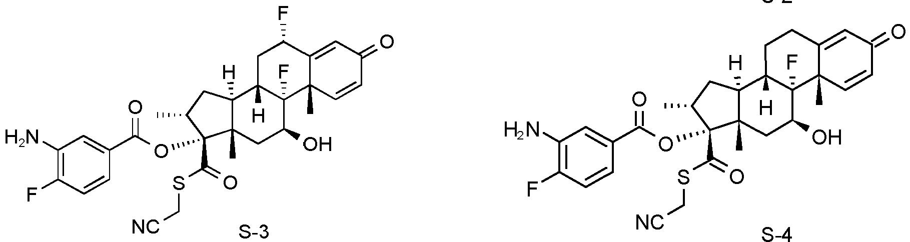

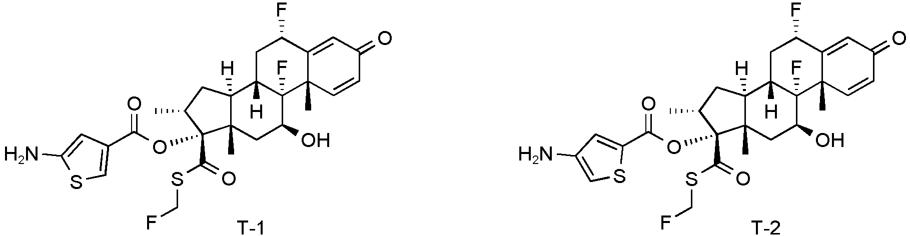

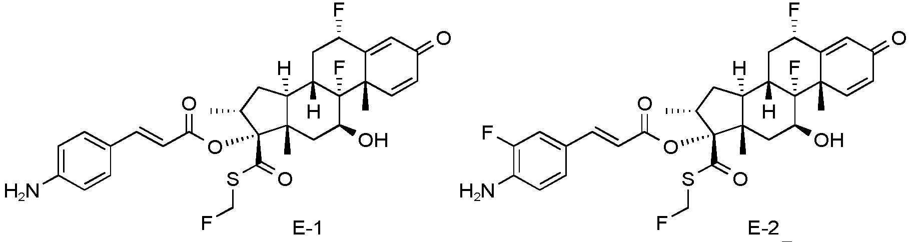

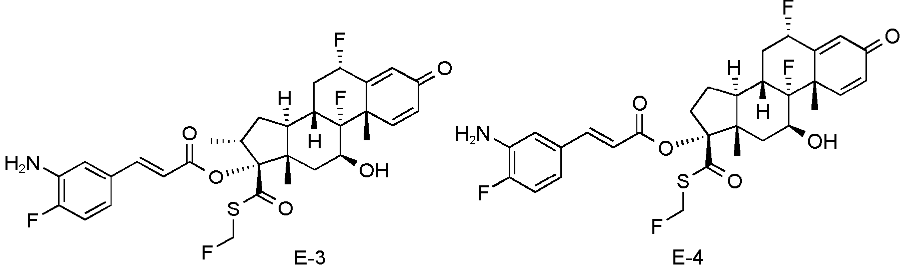

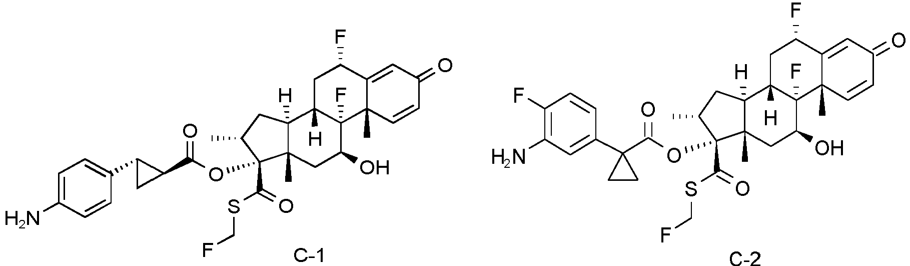

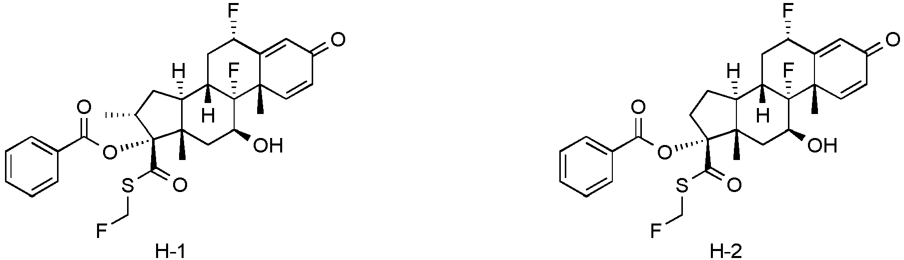

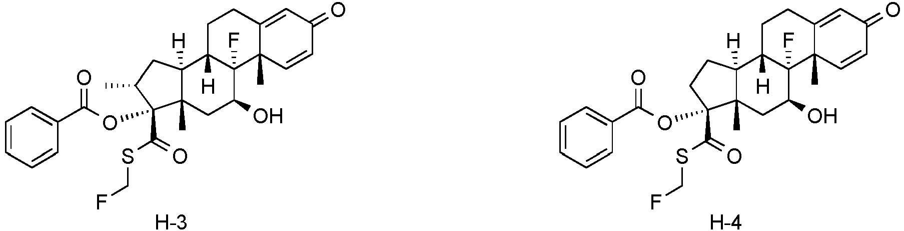

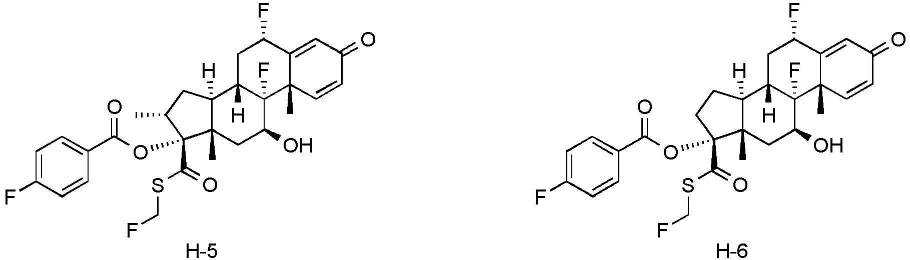

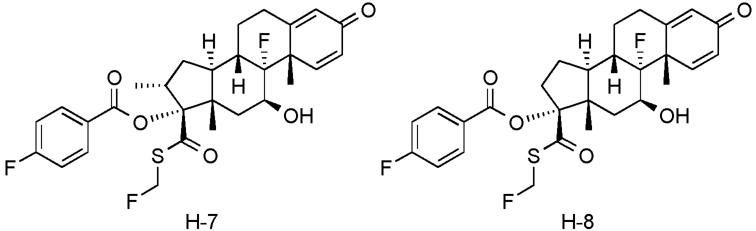

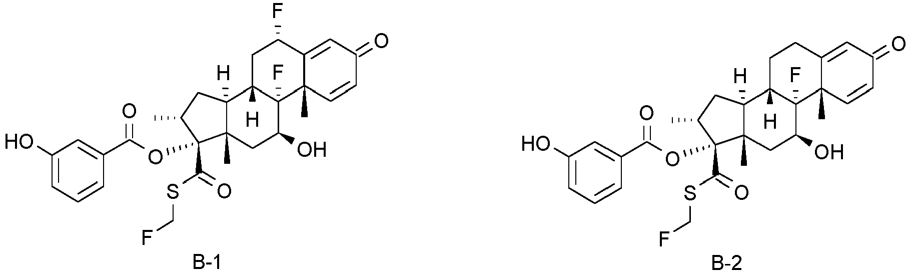

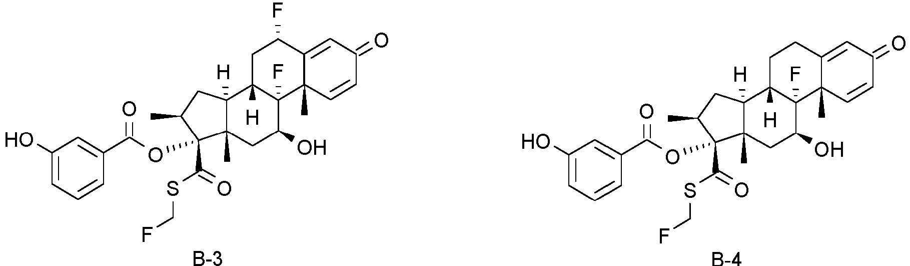

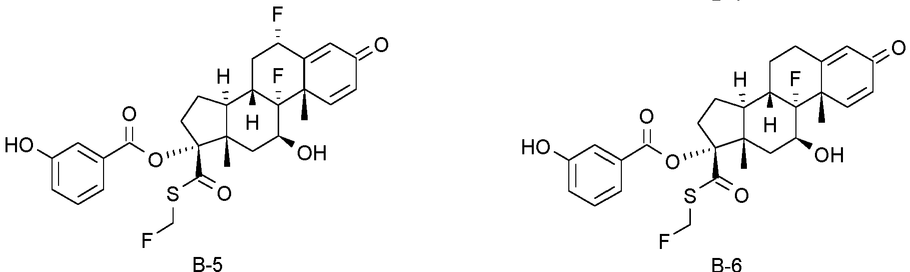

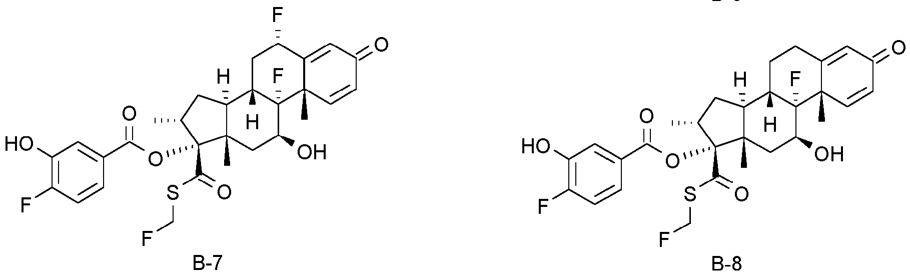

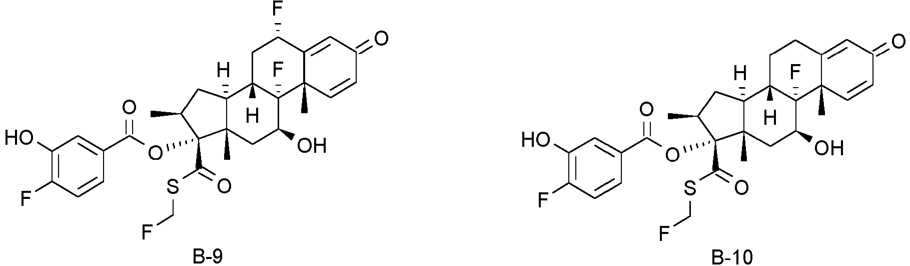

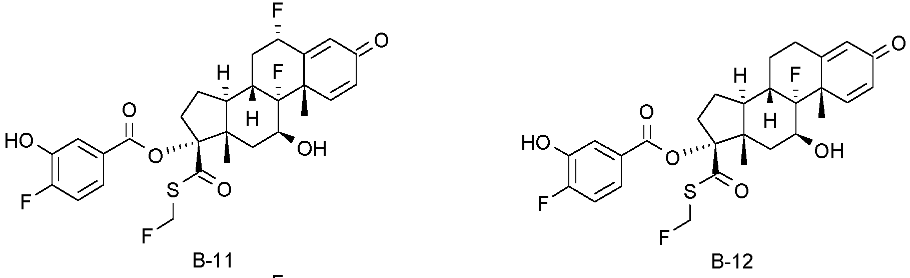

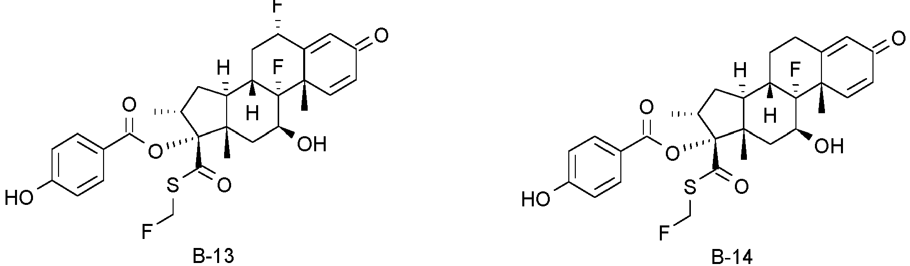

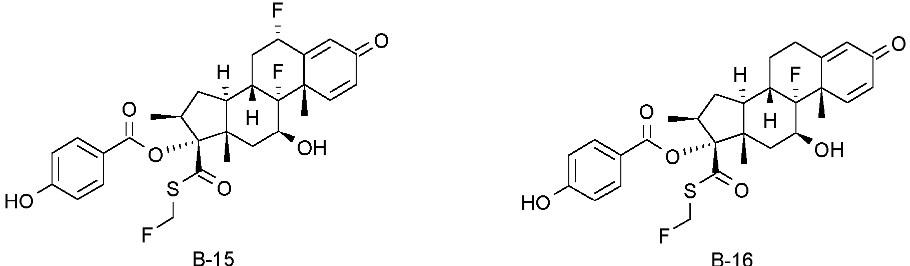

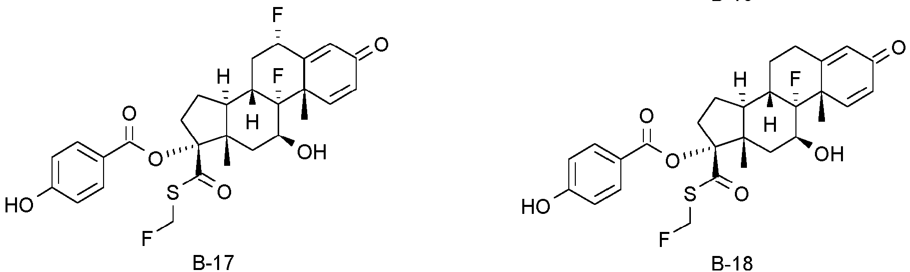

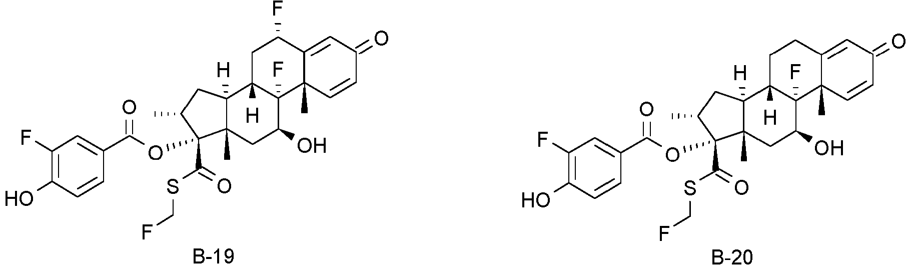

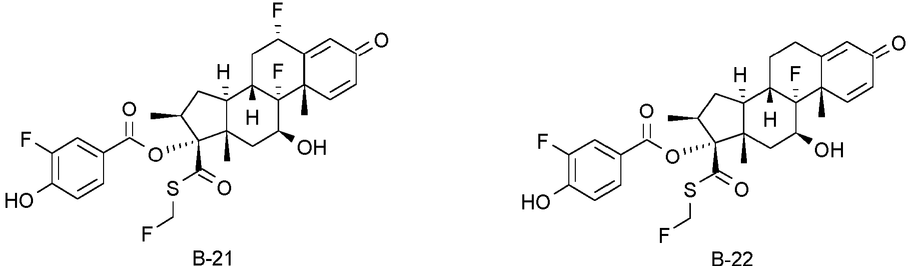

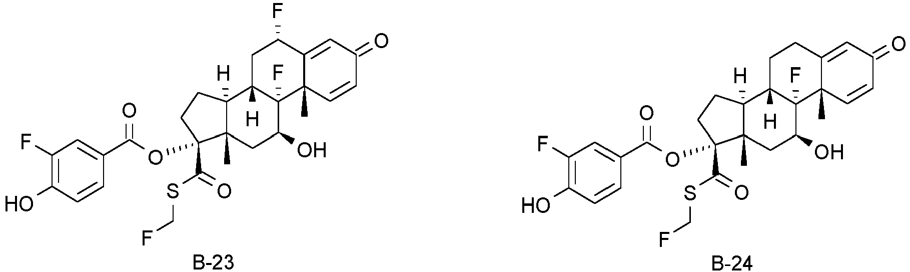

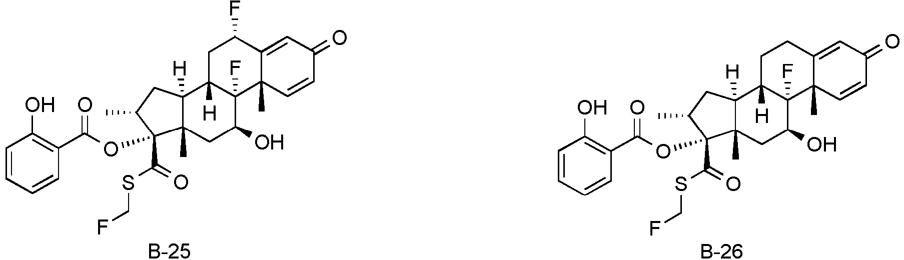

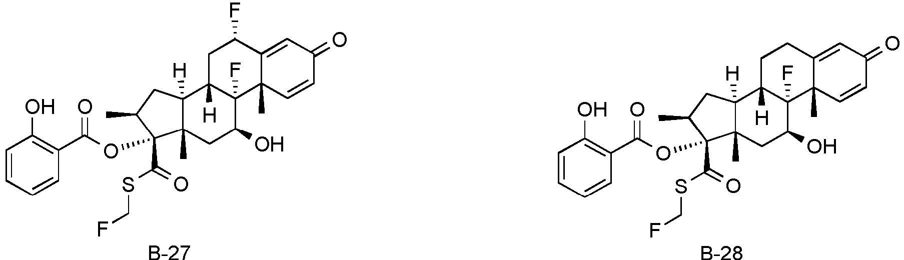

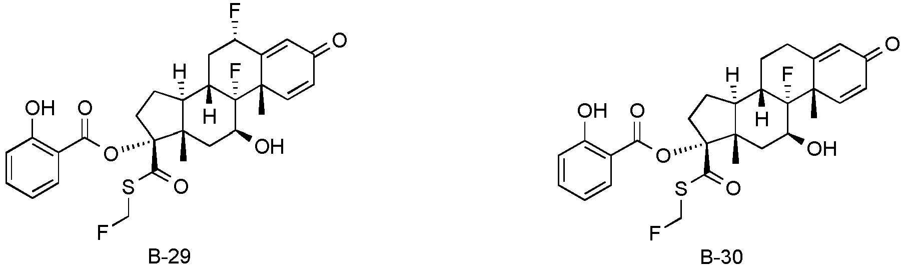

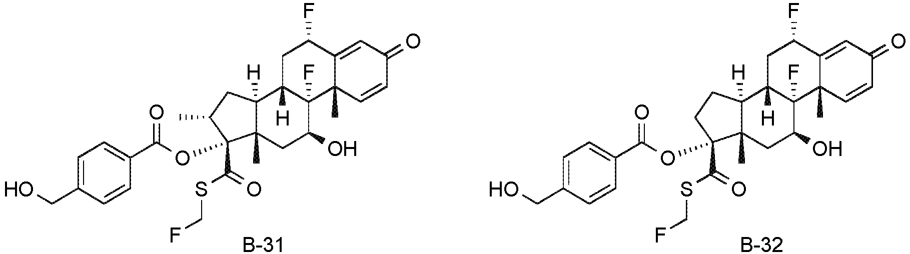

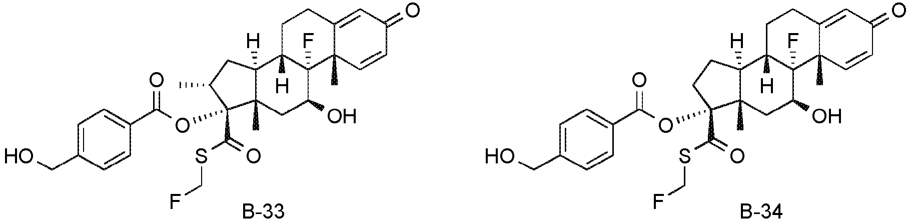

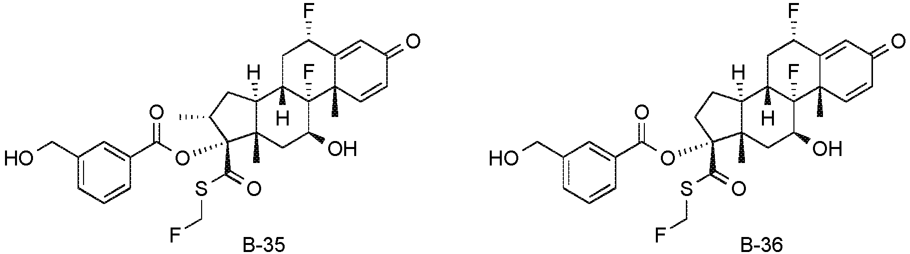

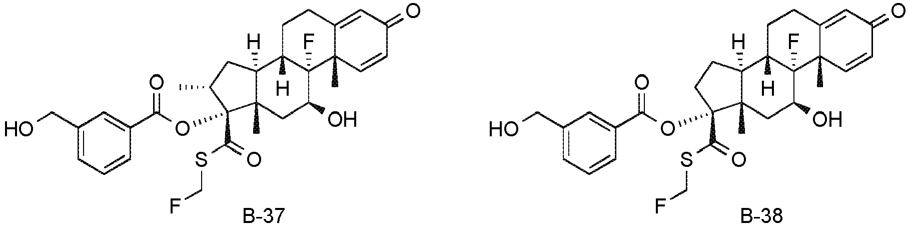

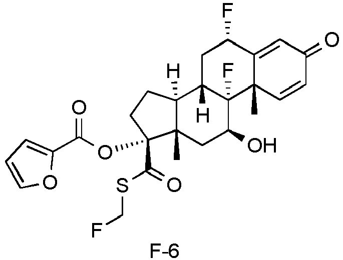

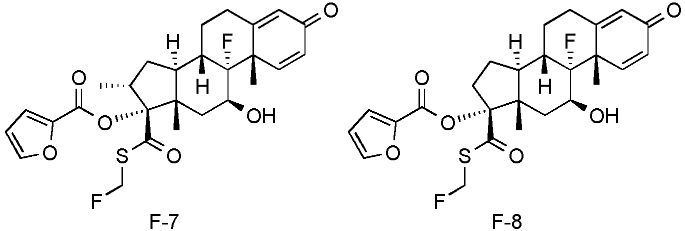

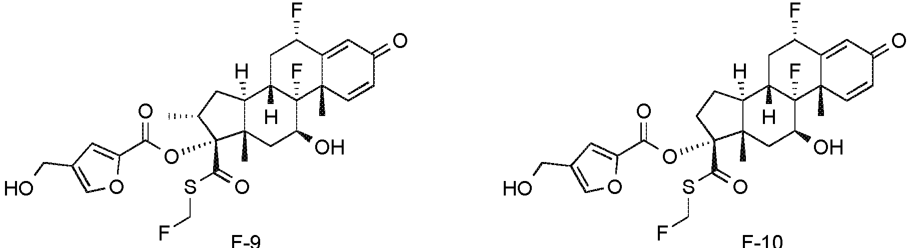

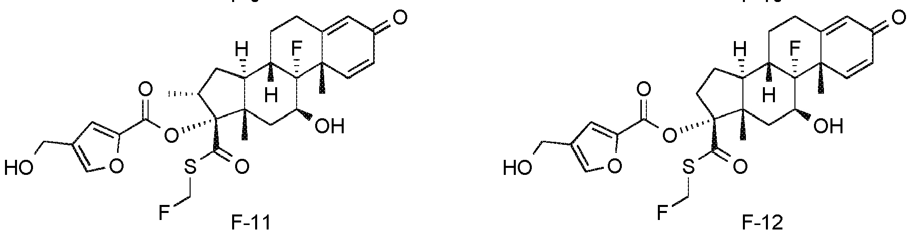

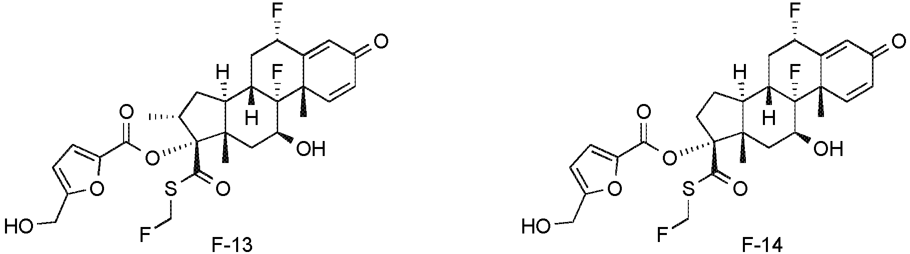

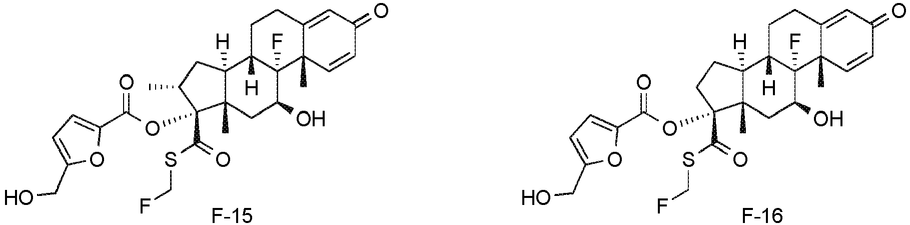

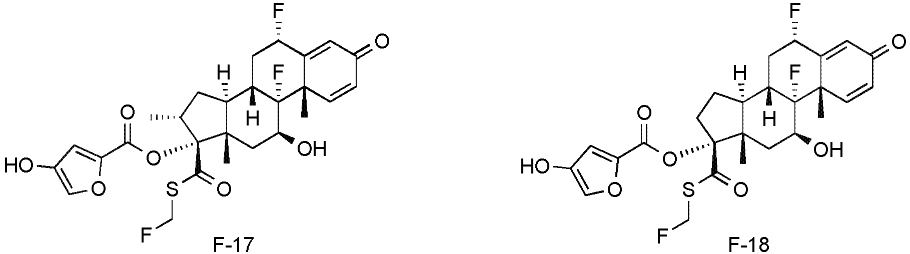

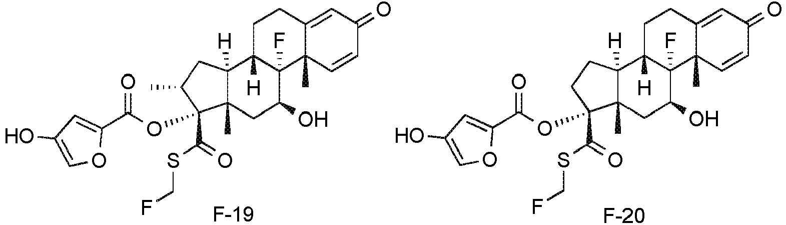

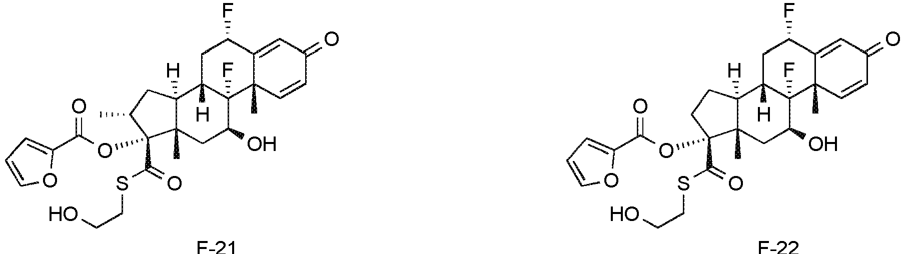

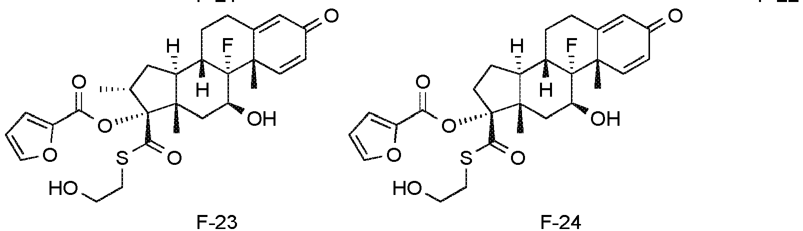

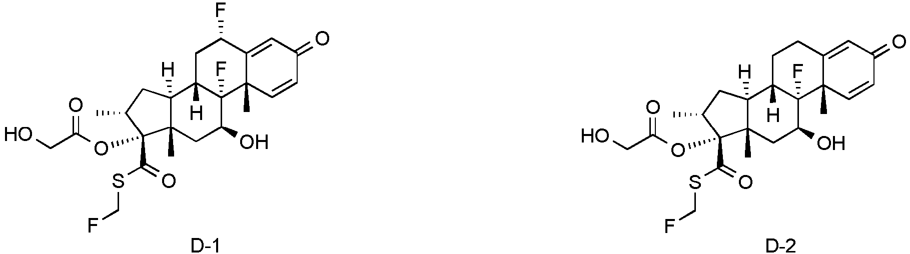

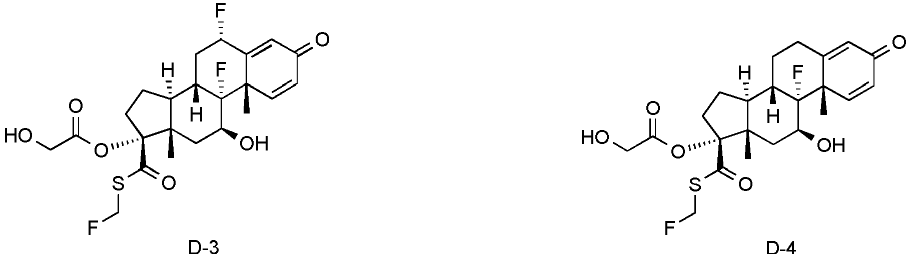

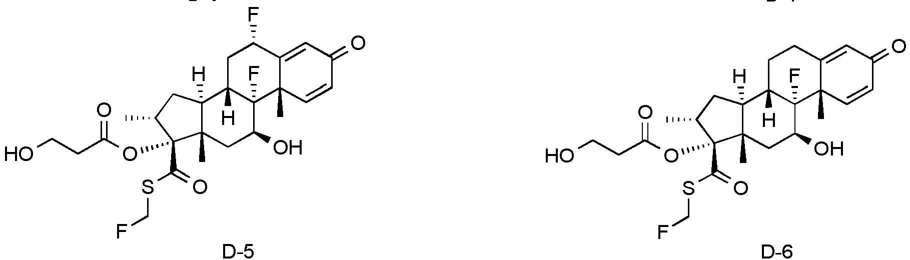

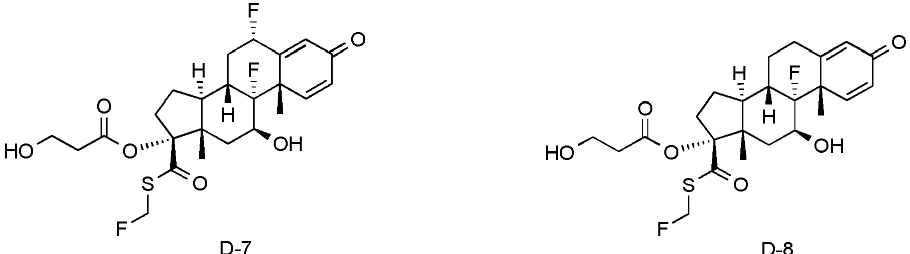

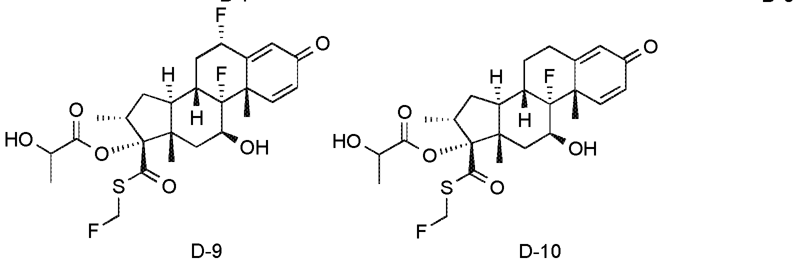

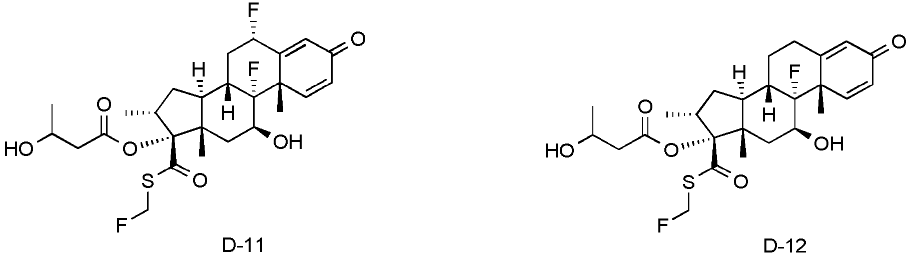

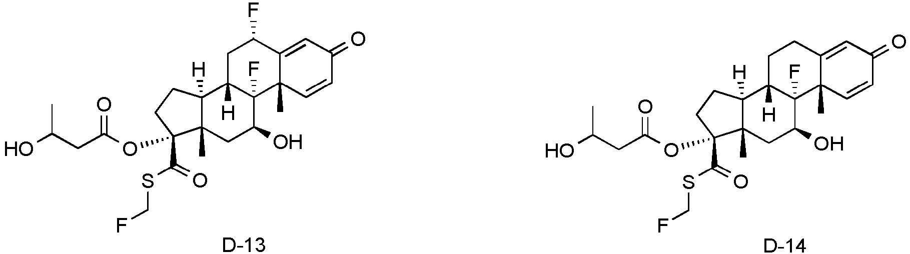

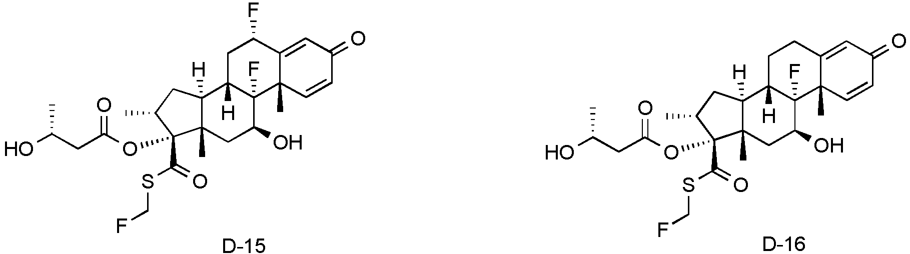

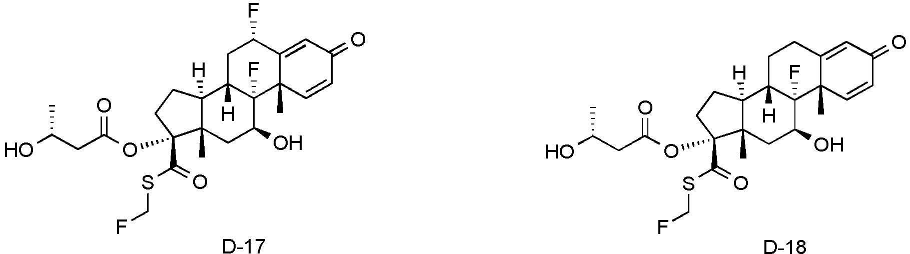

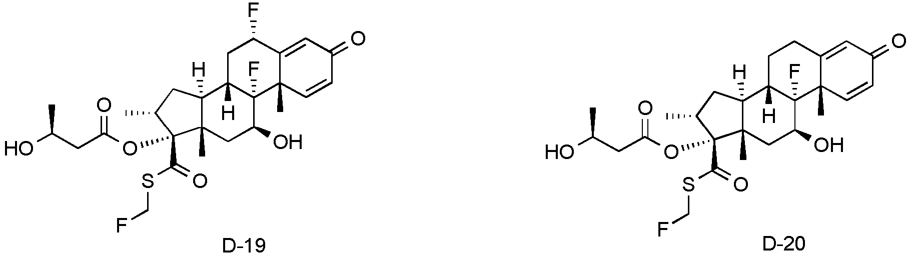

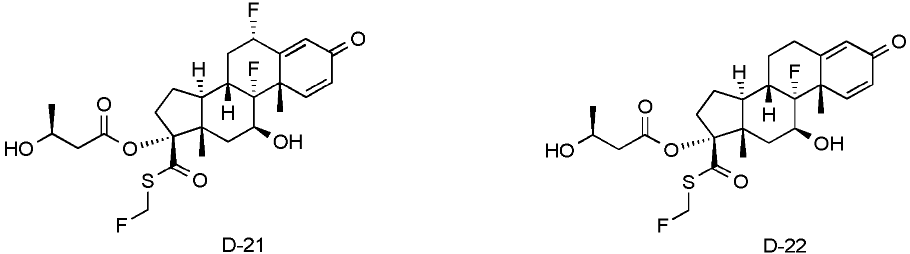

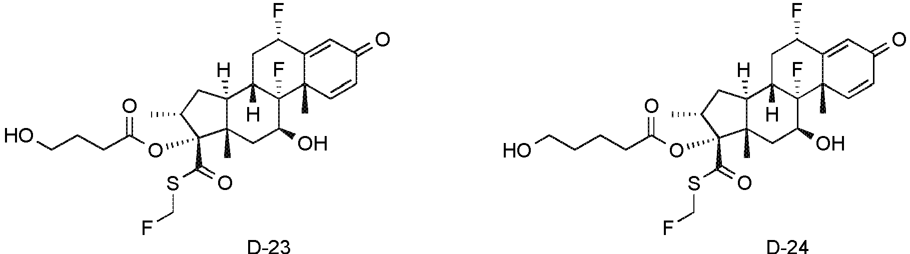

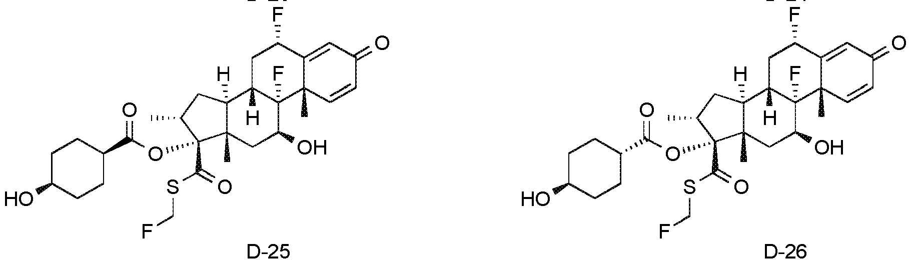

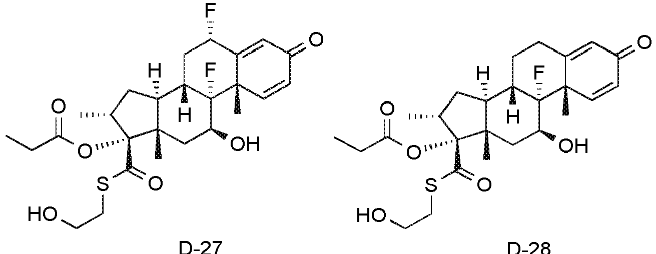

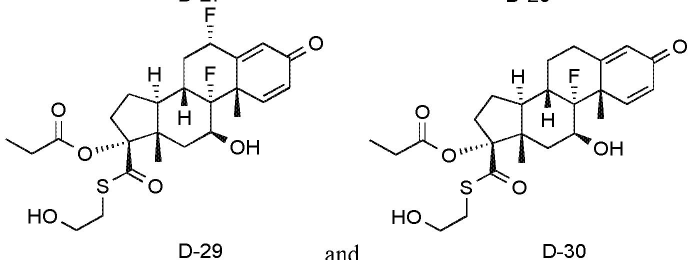

In some embodiments, the steroid glucocorticoid receptor agonist is selected from a group consisting of the following compounds, pharmaceutically acceptable salts, stereisomers, tautomers, polymorphs, solvates, N-oxides, isotope labeled compounds, metabolites or prodrugs thereof: 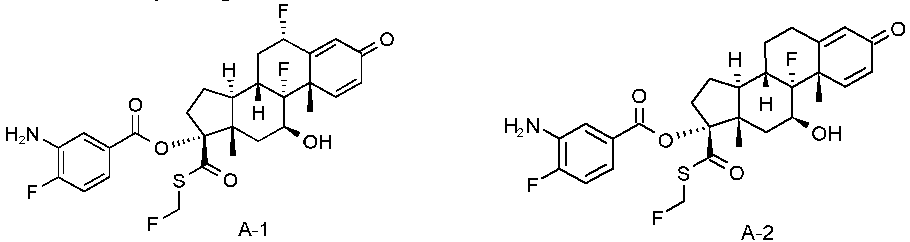 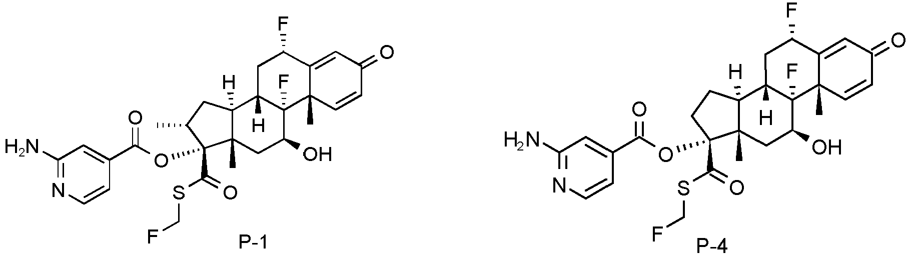 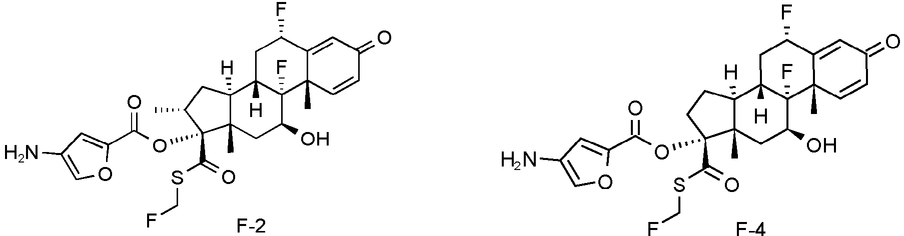 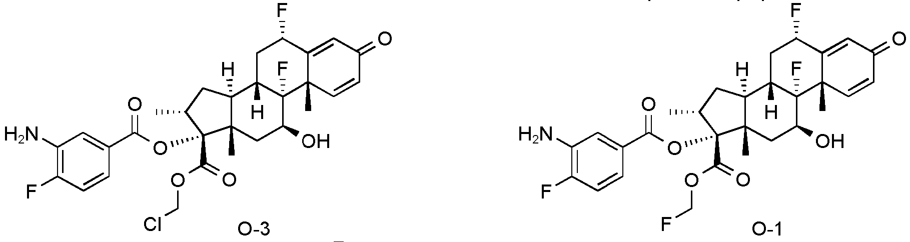 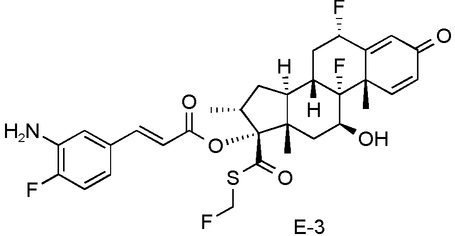 and

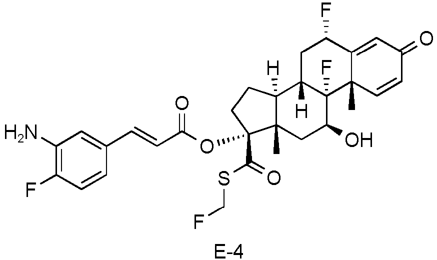

In some embodiments, the steroid glucocorticoid receptor agonist is selected from a group consisting of the following compounds, pharmaceutically acceptable salts, stereisomers, tautomers, polymorphs, solvates, N-oxides, isotope labeled compounds, metabolites or prodrugs thereof: 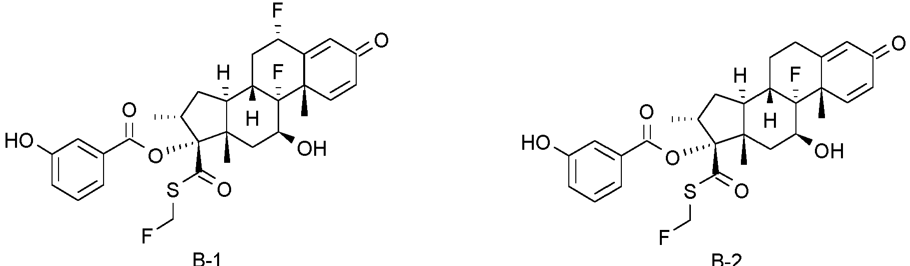 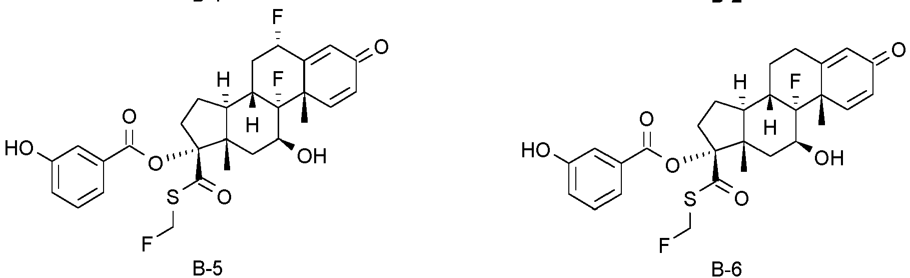 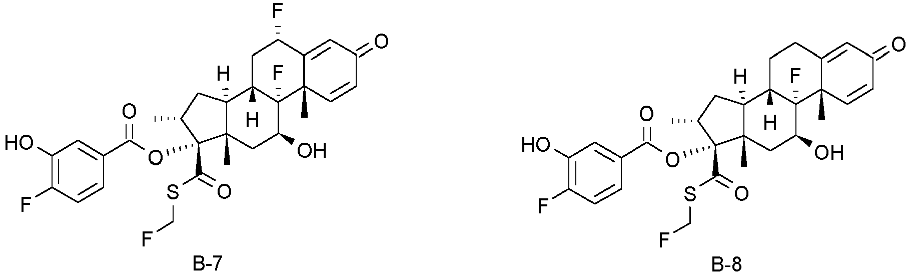 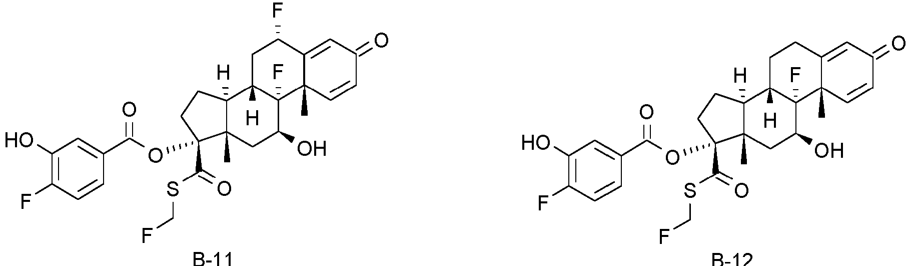 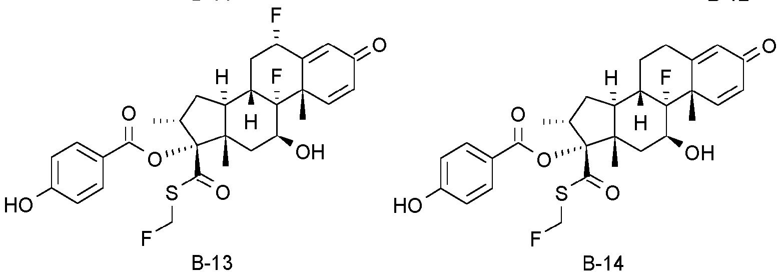 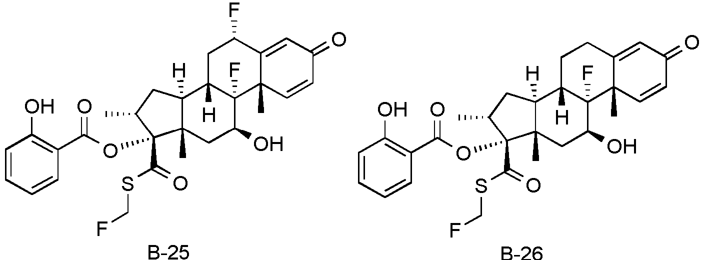 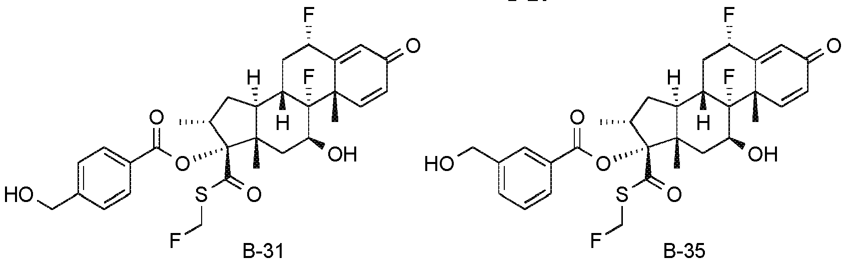 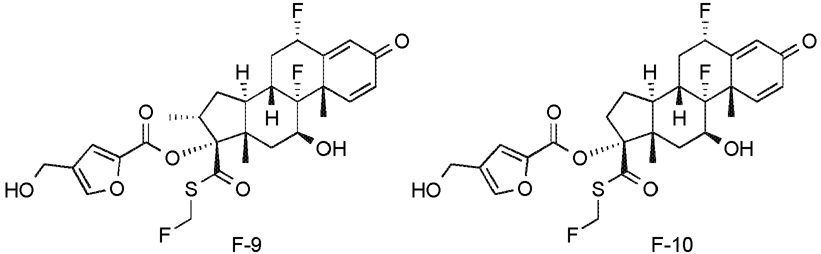 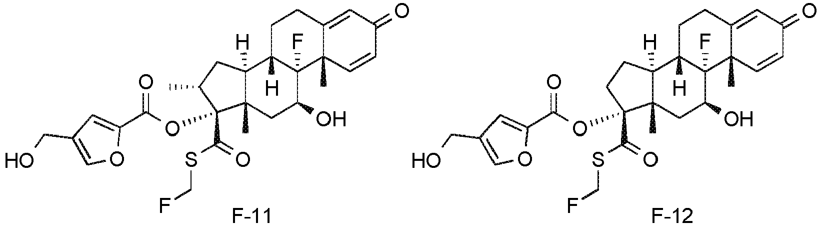 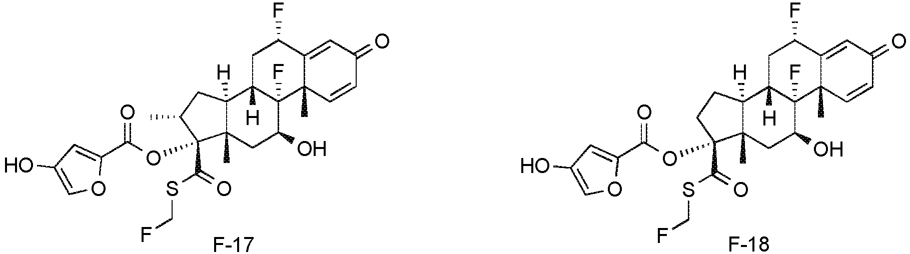 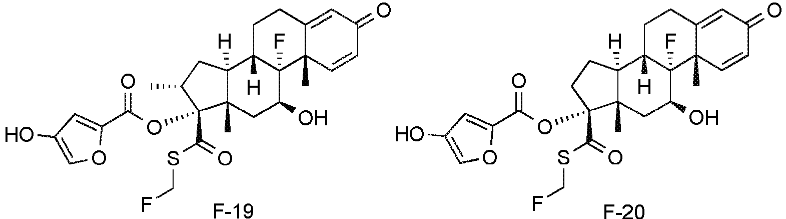 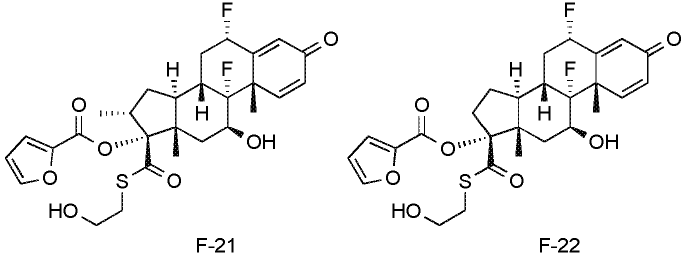 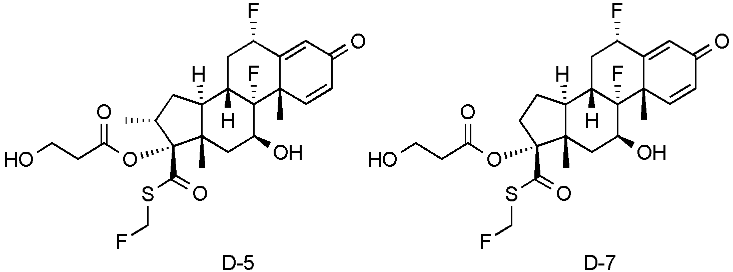 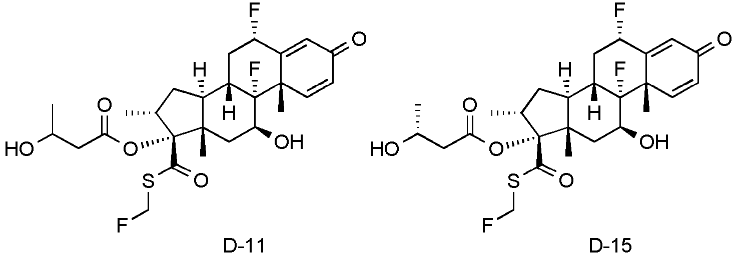 and

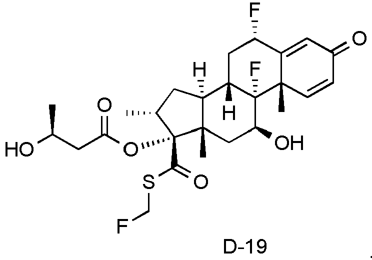

### Antibody-drug conjugate

In one aspect, the present application provides an antibody-drug conjugate having a structure represented by formula Ab-[M-L-E-D]ₓ, wherein:
Ab is an antibody specifically binding to an antigen or an antigen binding fragment thereof ;
M is a linkage site to the antibody or the antigen binding fragment thereof;
L is a linker between linkage site M and E;
E is a structural fragment linking L with D;
D is a glucocorticoid drug fragment, and the glucocorticoid drug is a glucocorticoid receptor agonist;
X is an integer selected from 1 to 10, preferably an integer selected from 3 to 8.

In the antibody-drug conjugate, the glucocorticoid receptor agonist can be linked to the antibody or the antigen binding fragment thereof through a linking moiety (such as the "M-L-E" fragment as shown in the present application).

In some embodiments, in the antibody-drug conjugate, the antigen binding to the antibody includes, but is not limited to, TNFα, IL6R, BDCA2, NR3C1, MSR1, PRLR, CD25, CD40, CD70, CD74, CD163 and the like.

In some embodiments, the antibody or the antigen binding fragment thereof includes, but is not limited to, Adalimumab, Tocilizumab and an IgG1 antibody.

In some embodiments, M is 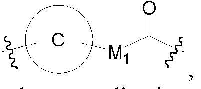 , wherein ring C is a single bond, halogen, a 5-6 membered aliphatic heterocyclic ring or a 5-20 membered aromatic ring system, the aliphatic heterocyclic ring or the aromatic ring system being optionally substituted with one or more groups each independently selected from a group consisting of oxo (=O), halogen, cyano, amino, carboxyl, mercapto and C₁₋₆ alkyl; and M₁ is selected from a group consisting of a single bond or a fragment consisting of one or more groups selected from: -O-, -NH-, -C(=O)-, -S(=O)₂-, -C=N-O-, -NH-S(=O)₂-NH-, phenylene, 5-10 membered heteroaryl, C₁₋₂₀ alkylene, C₂₋₂₀ alkenylidene and C₂₋₂₀ alkynylidene.

In some embodiments, M is 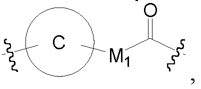 wherein ring C is a single bond, halogen, a 5 membered aliphatic heterocyclic ring, a 6 membered heteroaromatic ring or a polycyclic ring formed by 2-5 (preferably 3) units selected from a 6 membered heteroaromatic ring and a benzene ring linked through a single bond, the aliphatic heterocyclic ring, heteroaromatic ring or polycyclic ring being optionally substituted with one or more groups selected from a group consisting of oxo (=O), halogen and C₁₋₄ alkyl; and M₁ is selected from a group consisting of a single bond or a fragment consisting of one or more groups selected from -NH-, -C(=O)-, -NH-S(=O)₂-NH-, C₁₋₁₀ alkylene, C₂₋₁₀ alkenylidene and C₂₋₁₀ alkynylidene.

In some embodiments, M is 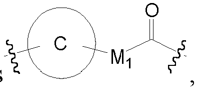 in which ring C is selected from a group consisting of 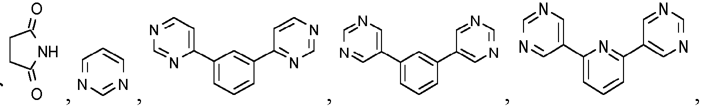 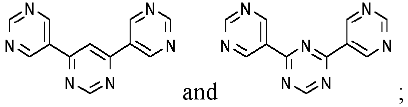 and M₁ is selected from a group consisting of a single bond, -NH-, -C(=O)-, -NH-S(=O)₂-NH-, C₁₋₆ alkylene, C₂₋₆ alkenylidene and C₂₋₆ alkynylidene.

In some embodiments, M is selected from a group consisting of 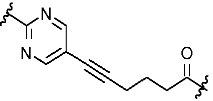 and

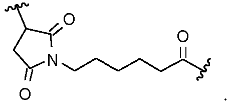

In some embodiments, M is selected from

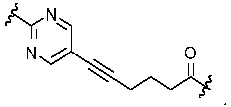

In some embodiments, M is selected from a group consisting of

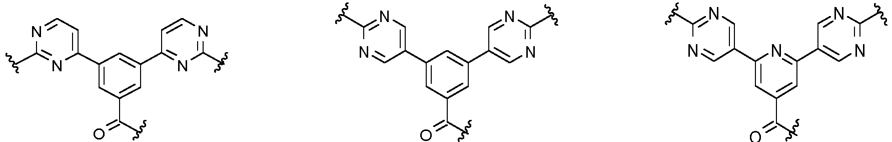

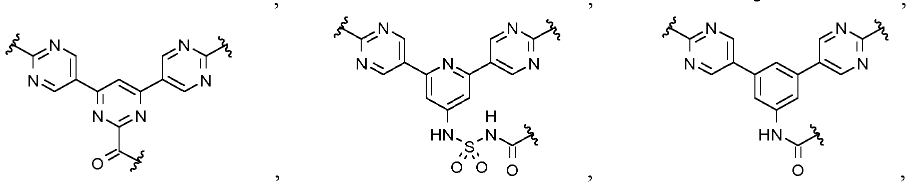

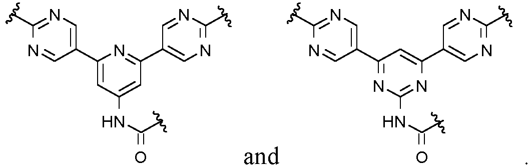

In some embodiments, M is selected from a group consisting of

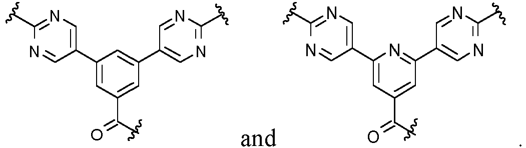

In some embodiments, L is a structure consisting of one or more moieties selected from a group consisting of: C₁₋₆ alkylene, -N(R')-, carbonyl, -O-, a natural amino acid or an unnatural amino acid and an analogue or a derivative thereof (such as Ala, Arg, Asn, Asp, Cit, Cys, Gln, Glu, Gly, His, Ile, Leu, Lys, Met, Phe, Pro, Ser, Thr, Trp, Tyr, Val, Lys(COCH₂CH₂(OCH₂CH₂)ᵣOCH₃)) and a short chain polypeptide consisting of amino acids (such as Ala-Ala, Ala-Lys, Ala-Lys(Ac), Ala-Pro, Gly-Glu, Gly-Gly, Phe-Lys, Phe-Lys(Ac), Val-Ala, Val-Lys, Val-Lys(Ac), Val-Cit, Ala-Ala-Ala, Ala-Ala-Asn, Leu-Ala-Glu, Gly-Gly-Arg, Gly-Glu-Gly, Gly-Gly-Gly, Gly-Ser-Lys, Glu-Val-Ala, Glu-Val-Cit, Ser-Ala-Pro, Val-Leu-Lys, Val-Lys-Ala, Val-Lys-Gly, Gly-Gly-Phe-Gly, Gly-Gly-Val-Ala, Gly-Phe-Leu-Gly, Glu-Ala-Ala-Ala, Gly-Gly-Gly-Gly-Gly), 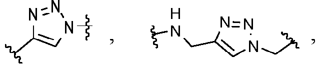 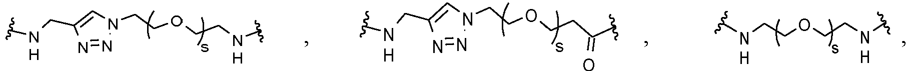 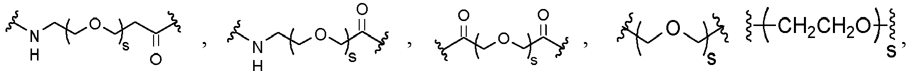 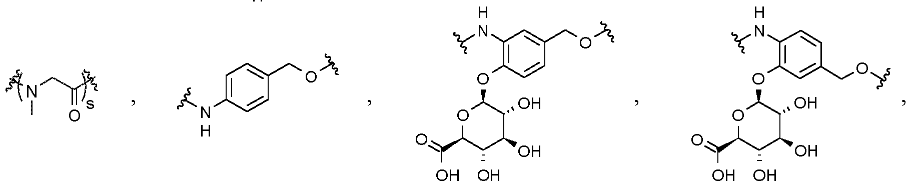 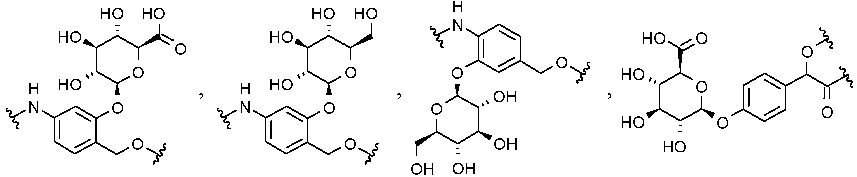 and 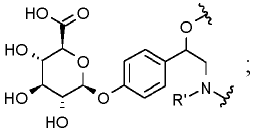 wherein R' represents hydrogen, C₁₋₆ alkyl or C₄₋₃₀ alkyl containing -(CH₂CH₂O)ᵣ-; r is an integer selected from 1 to 10; s is an integer selected from 1 to 20. Preferably, r is an integer selected from 1 to 6; preferably, s is an integer selected from 1 to 10.

In some embodiments, L is a structure consisting of one or more moieties selected from a group consisting of C₁₋₆ alkylene, carbonyl, -NH-, Ala-Ala, Ala-Lys, Ala-Pro, Gly-Glu, Gly-Gly, Phe-Lys, Val-Ala, Val-Lys, Val-Cit, Ala-Ala-Ala, Ala-Ala-Asn, Leu-Ala-Glu, Gly-Gly-Arg, Gly-Glu-Gly, Gly-Gly-Gly, Gly-Ser-Lys, Glu-Val-Ala, Glu-Val-Cit, Ser-Ala-Pro, Val-Leu-Lys, Val-Lys-Ala, Val-Lys-Gly, Gly-Gly-Phe-Gly, Gly-Gly-Val-Ala, Gly-Phe-Leu-Gly, Glu-Ala-Ala-Ala, Gly-Gly-Gly-Gly-Gly, 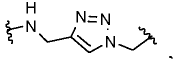 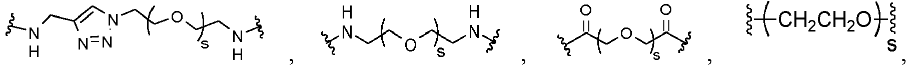 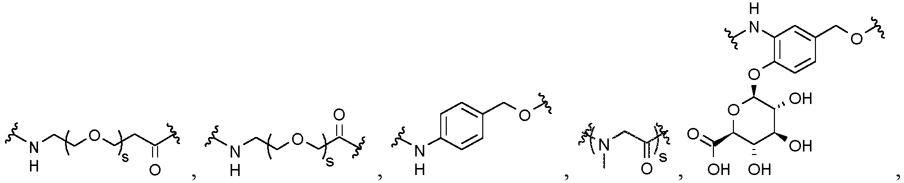 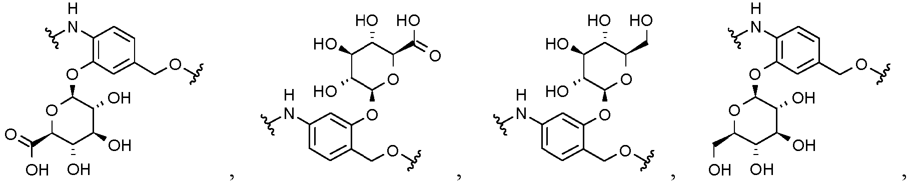 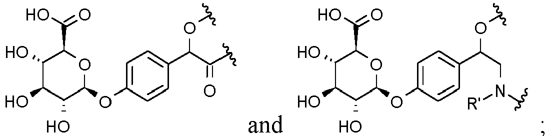 wherein s is an integer selected from 1 to 20.

In some embodiments, L is a structure consisting of one or more moieties selected from a group consisting of: C₁₋₆ alkylene, carbonyl, -NH-, Ala-Ala, Ala-Lys, Ala-Pro, Gly-Glu, Gly-Gly, Phe-Lys, Val-Ala, Val-Lys, Val-Cit, Ala-Ala-Ala, Ala-Ala-Asn, Leu-Ala-Glu, Gly-Gly-Arg, Gly-Glu-Gly, Gly-Gly-Gly, Gly-Ser-Lys, Glu-Val-Ala, Glu-Val-Cit, Ser-Ala-Pro, Val-Leu-Lys, Val-Lys-Ala, Val-Lys-Gly, Gly-Gly-Phe-Gly, Gly-Gly-Val-Ala, Gly-Phe-Leu-Gly, Glu-Ala-Ala-Ala, Gly-Gly-Gly-Gly-Gly, 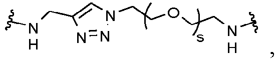 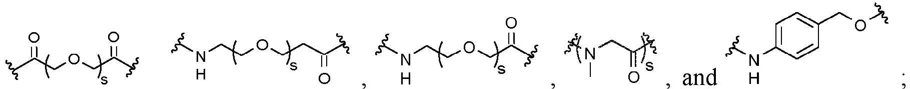 wherein s is an integer selected from 1 to 20, preferably an integer selected from 1 to 10.

In some embodiments, L is a structure consisting of one or more moieties selected from a group consisting of: 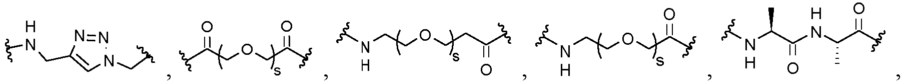 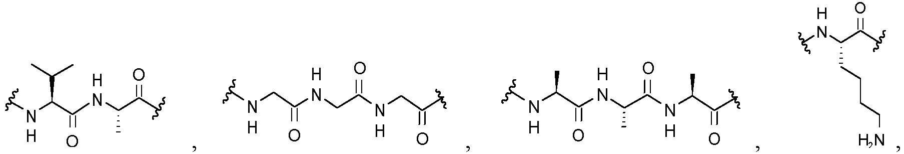 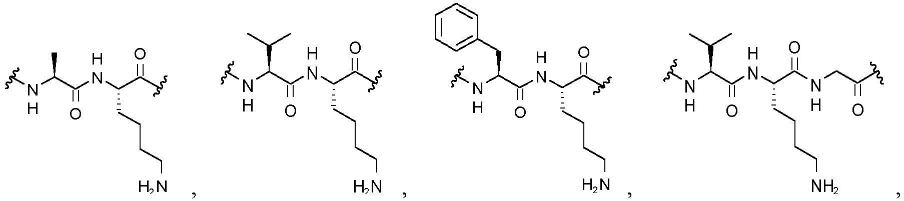 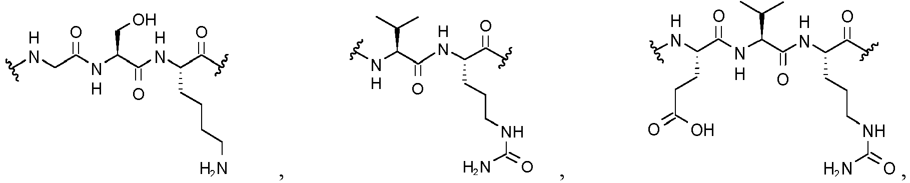 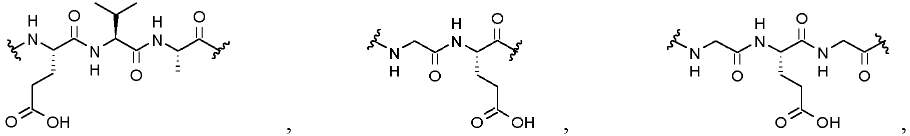 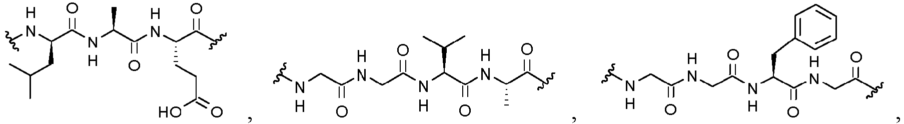 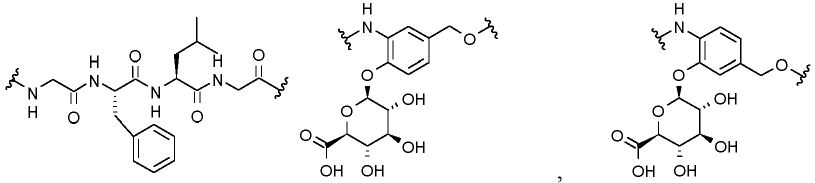 and 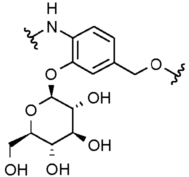 ; wherein s is an integer selected from 1 to 20, preferably an integer selected from 1 to 10.

In some embodiments, L is a structure consisting of one or more moieties selected from a group consisting of: 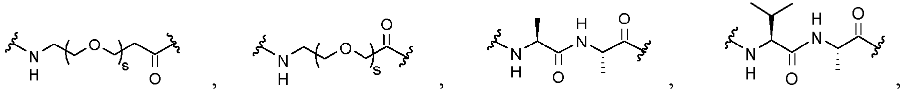 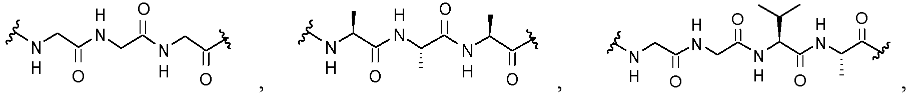 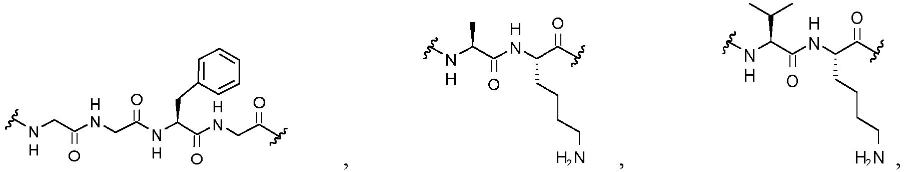 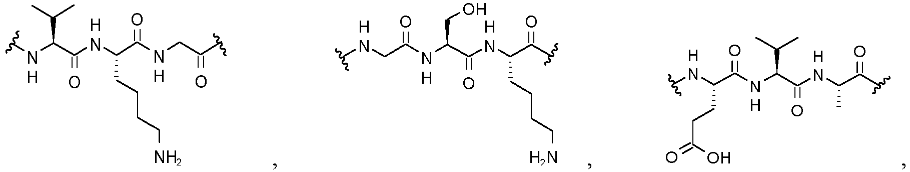 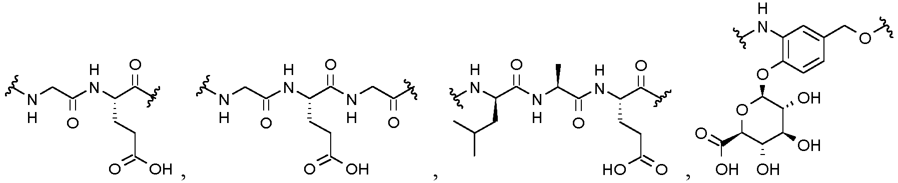 and 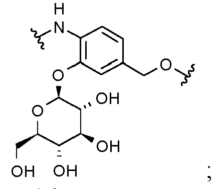 wherein s is an integer selected from 1 to 20, preferably an integer selected from 1 to 10.

In some embodiments, L is a structure consisting of one or more moieties selected from a group consisting of: 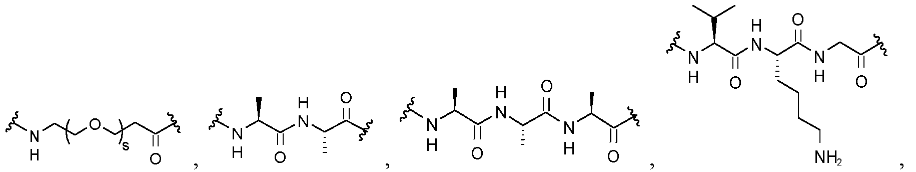 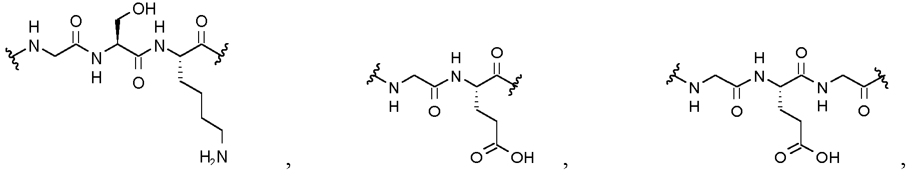 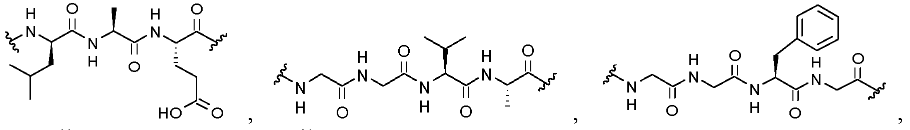 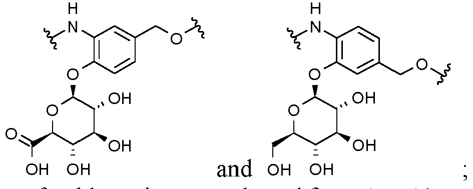 wherein s is an integer selected from 1 to 20, preferably an integer selected from 1 to 10.

In some embodiments, L is a structure consisting of one or more moieties selected from a group consisting of: 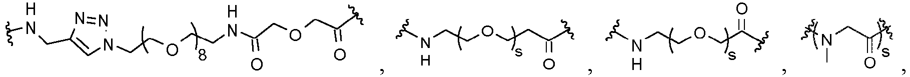 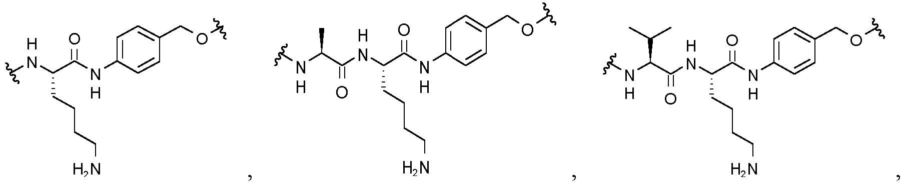 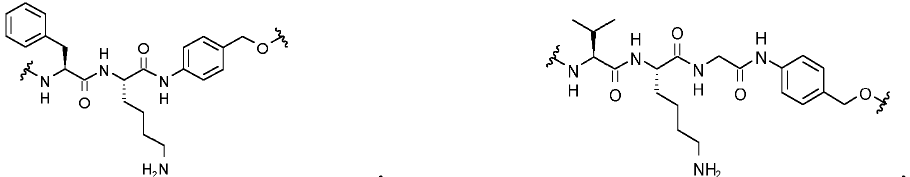 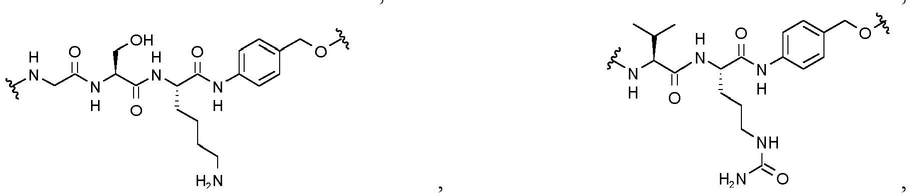 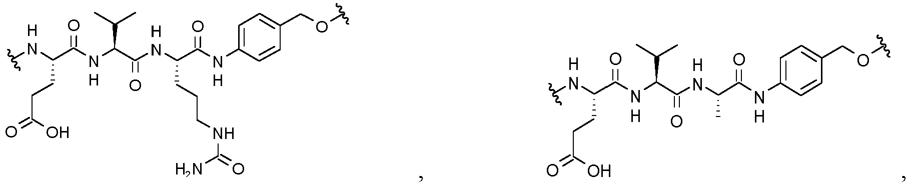 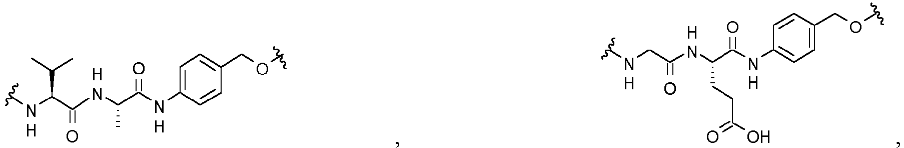 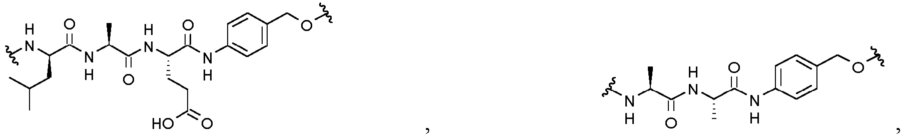 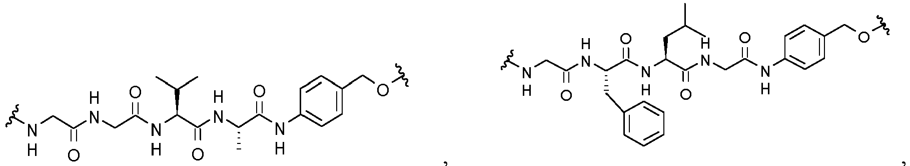 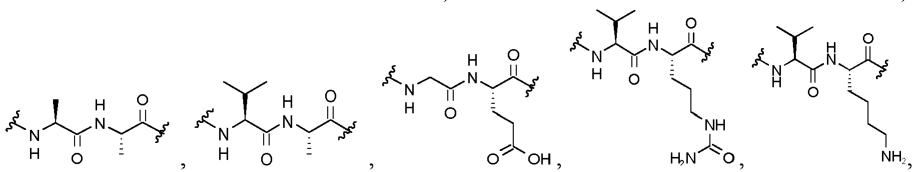 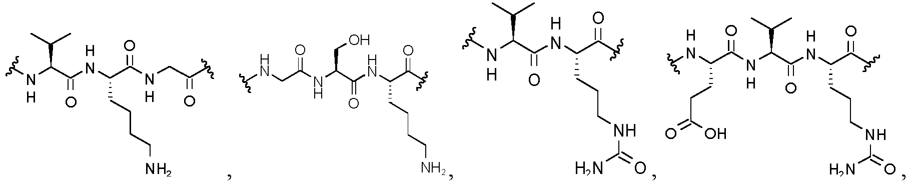 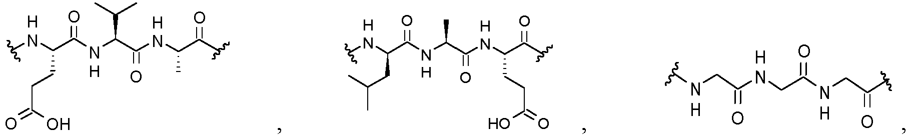 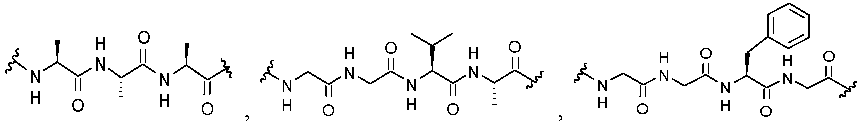 and 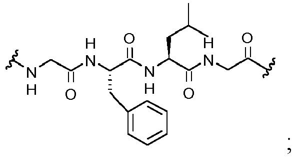 wherein s is an integer selected from 1 to 20, preferably an integer selected from 1 to 10.

In some embodiments, L is selected from the following structures: 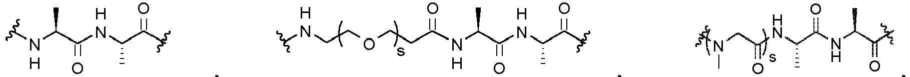 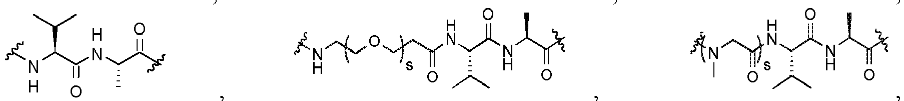 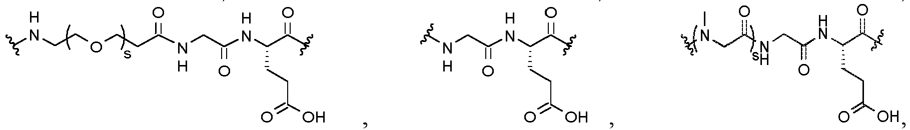 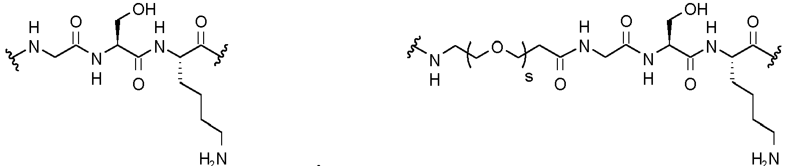 and 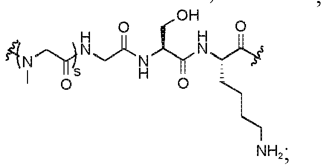 wherein s is an integer selected from 1 to 20, preferably an integer selected from 1 to 10.

In some embodiments, L is selected from the following structures: 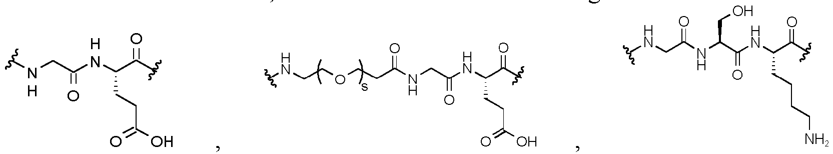 and 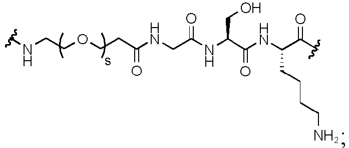 wherein s is an integer selected from 1 to 20, preferably an integer selected from 1 to 10.

In some embodiments, L is selected from the following structures: 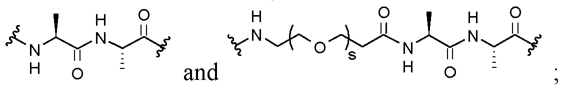 wherein s is an integer selected from 1 to 20, preferably an integer selected from 1 to 10.

In some embodiments, L is selected from the following structures: 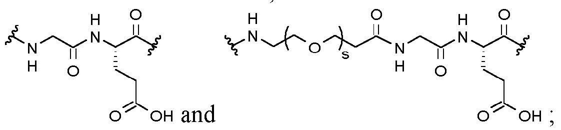 wherein s is an integer selected from 1 to 20, preferably an integer selected from 1 to 10.

In some embodiments, L is selected from the following structures: 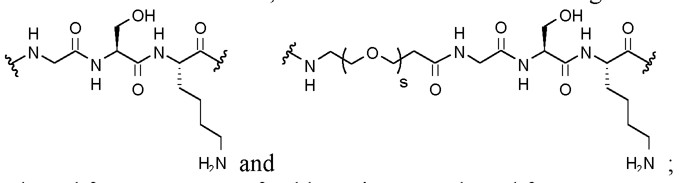 wherein s is an integer selected from 1 to 20, preferably an integer selected from 1 to 10.

In some embodiments, L is selected from the following structures:

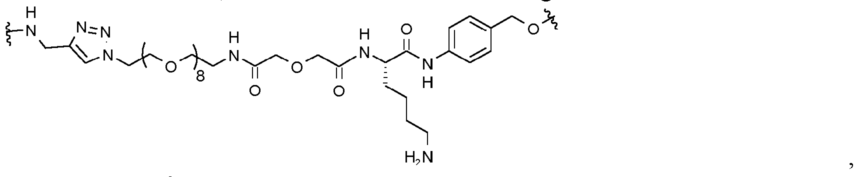

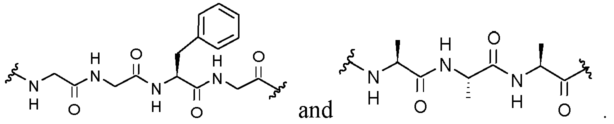

In some embodiments, L is selected from the following structures:

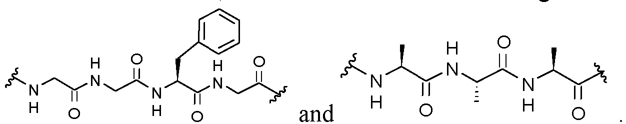

In some embodiments, E is a single bond, carbonyl, -NHCH₂- or a structure selected from a group consisting of: 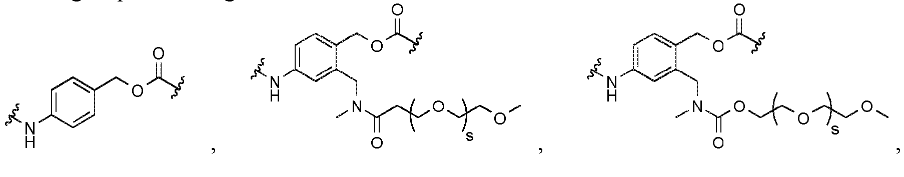 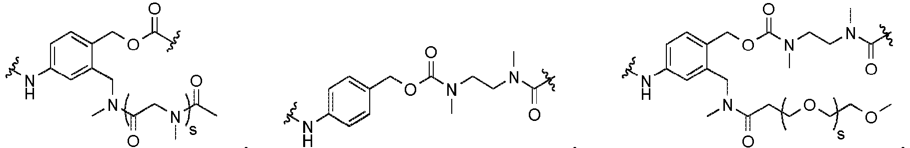 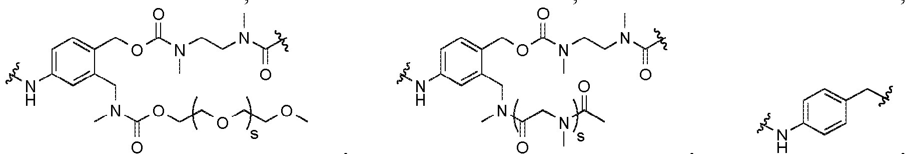 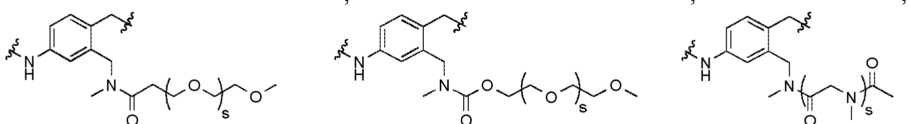 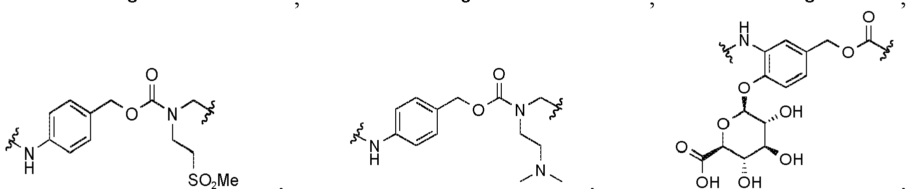 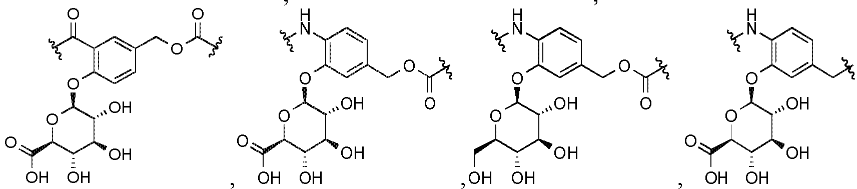 , and 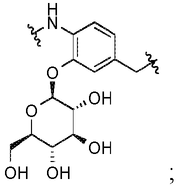 wherein s is an integer selected from 1 to 20, preferably an integer selected from 1 to 10.

In some embodiments, E is a single bond or -NHCH₂-.

In some embodiments, E is a single bond.

In some embodiments, 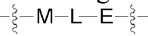 is selected from the following structures: 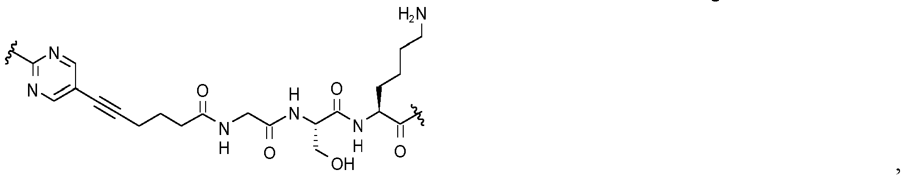 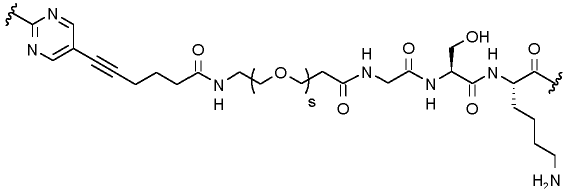 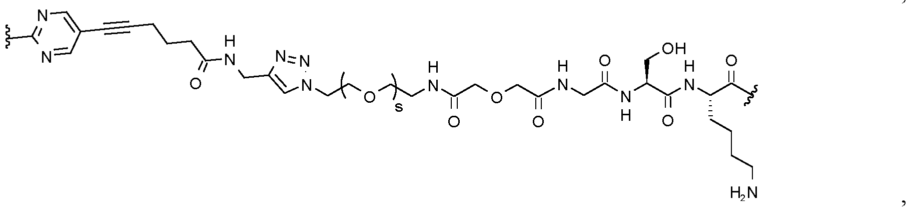 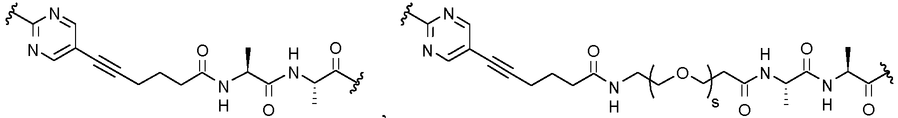 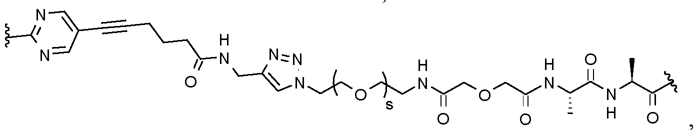 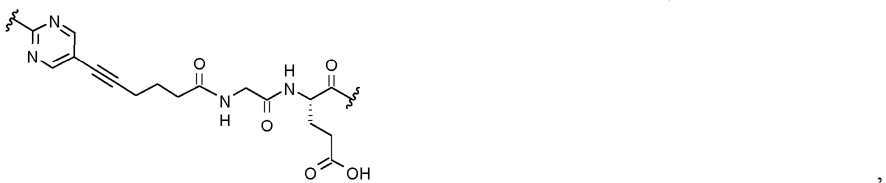 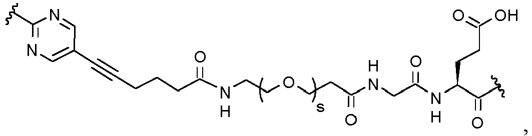 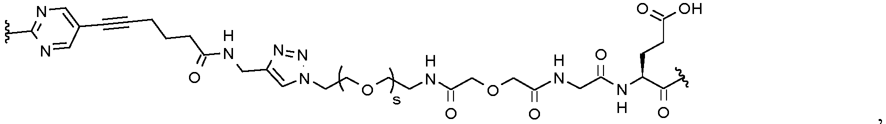 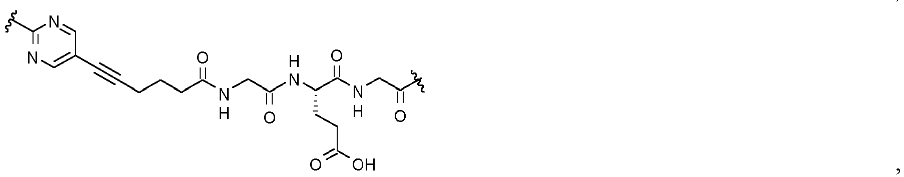 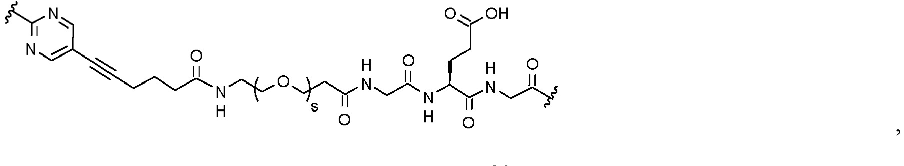 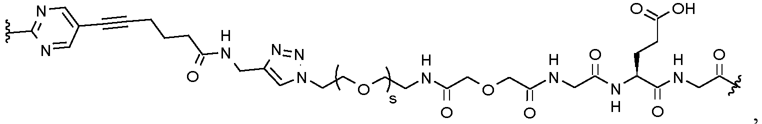 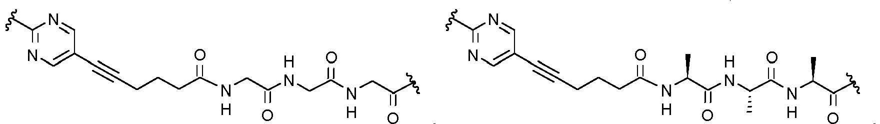 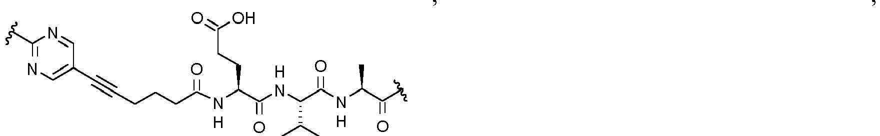 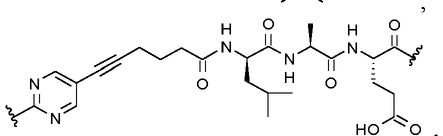 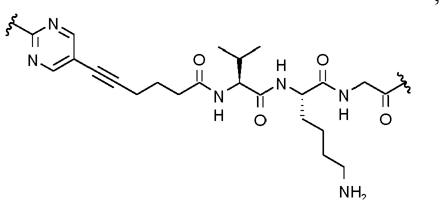 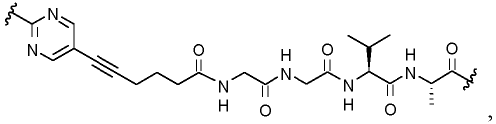 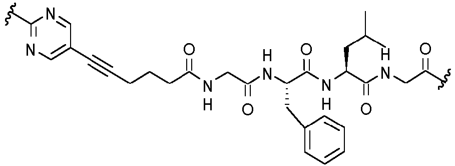 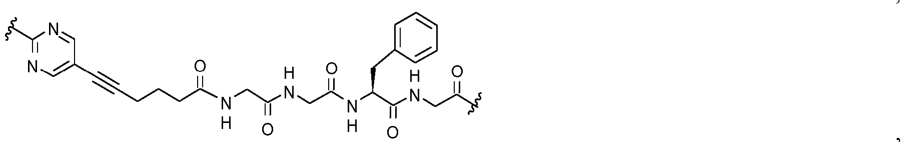 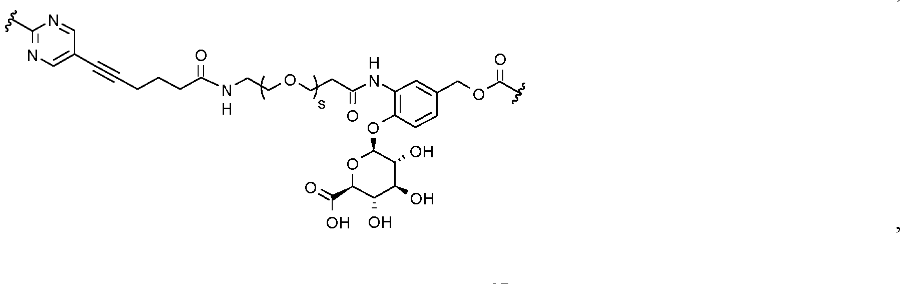 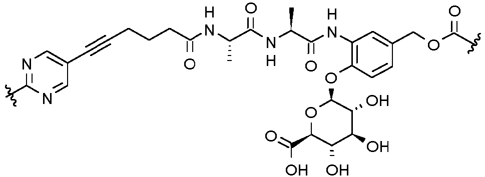 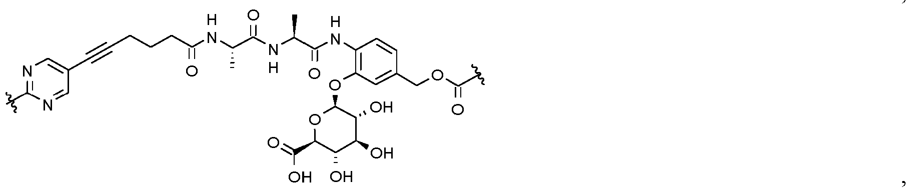 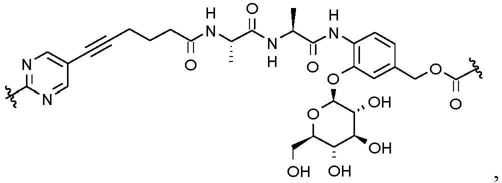 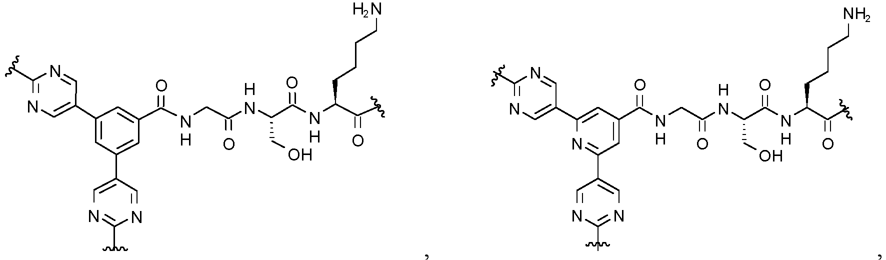 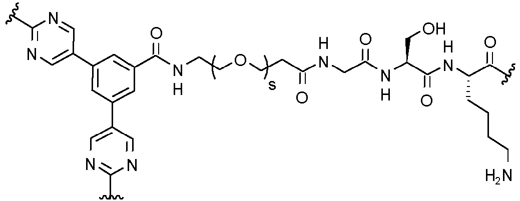 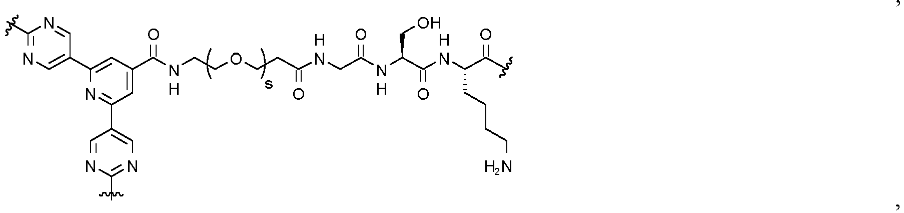 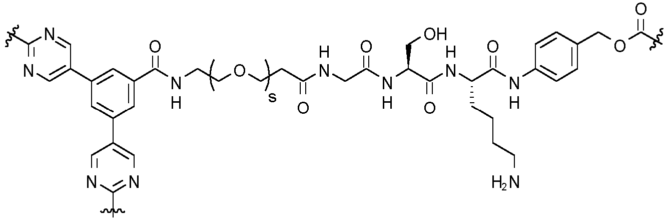 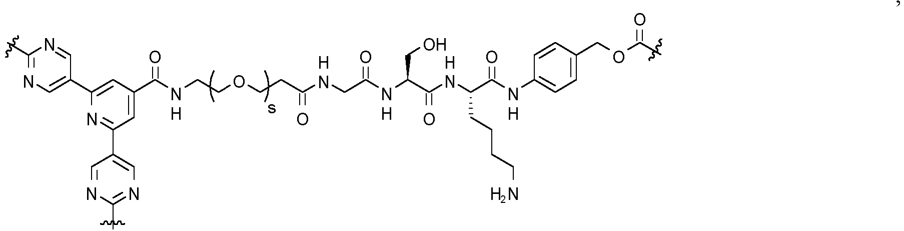 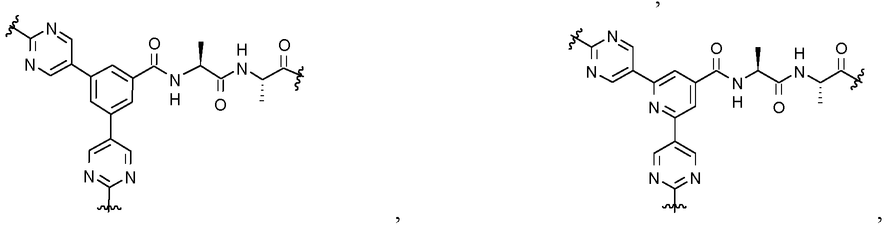 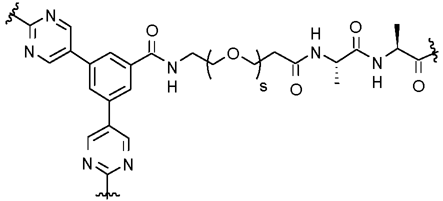 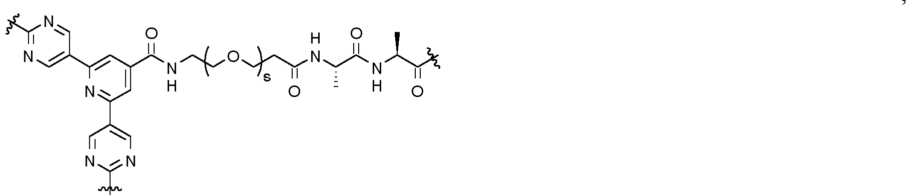 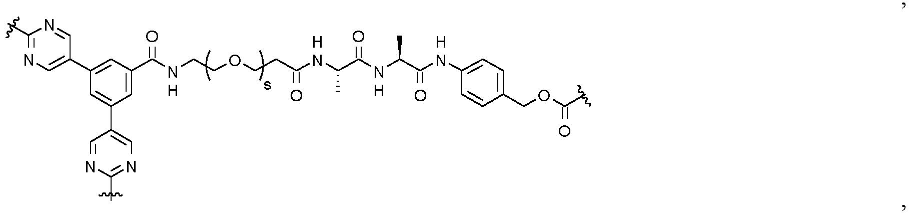 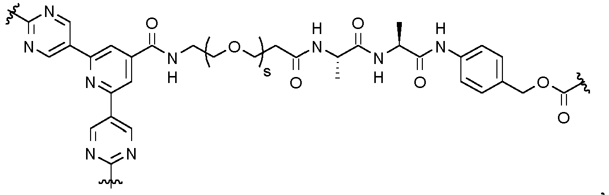 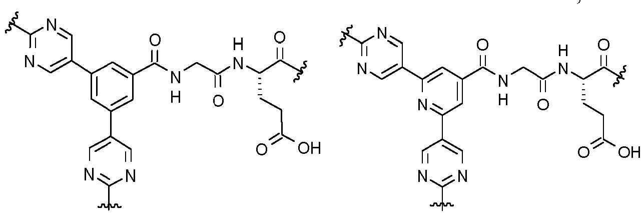 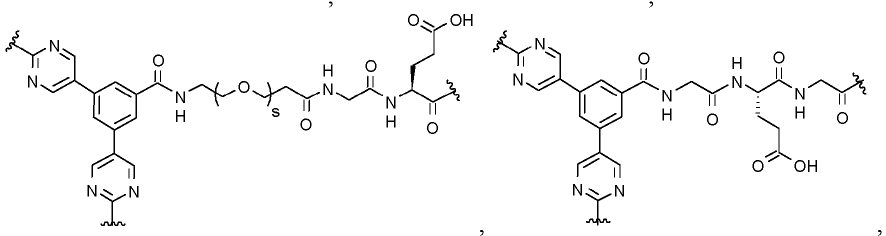 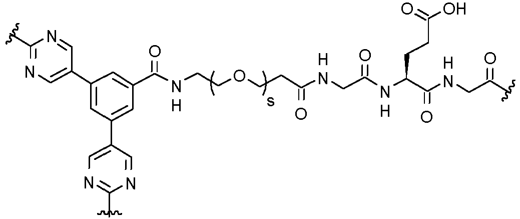 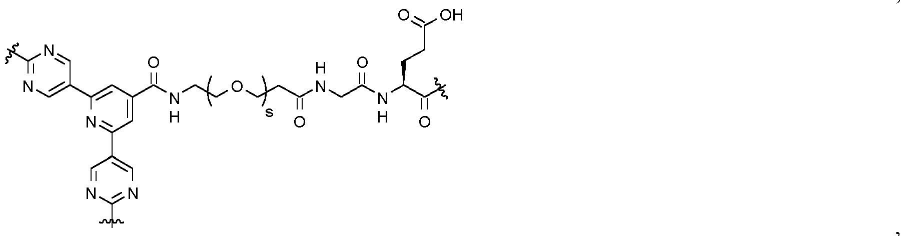 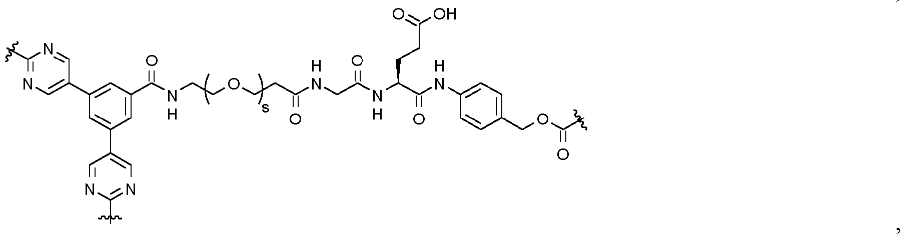 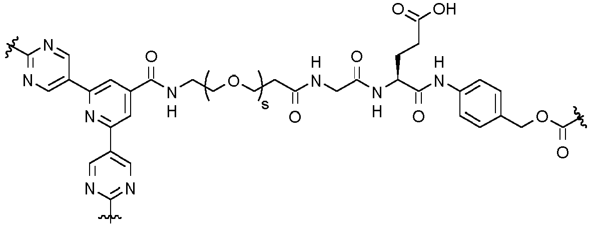 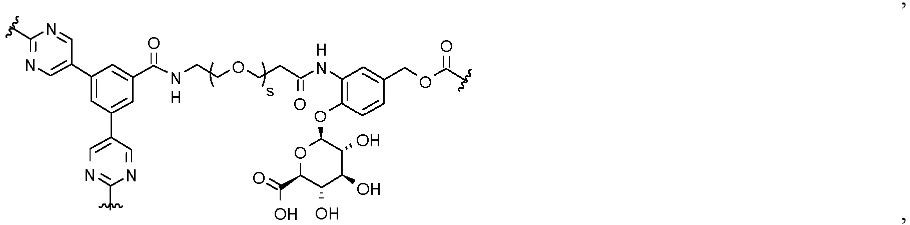 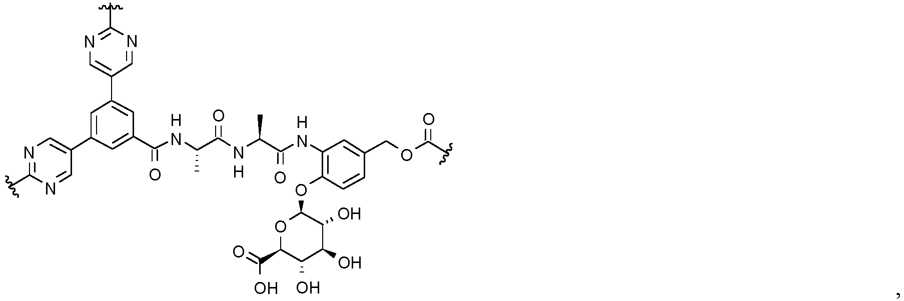 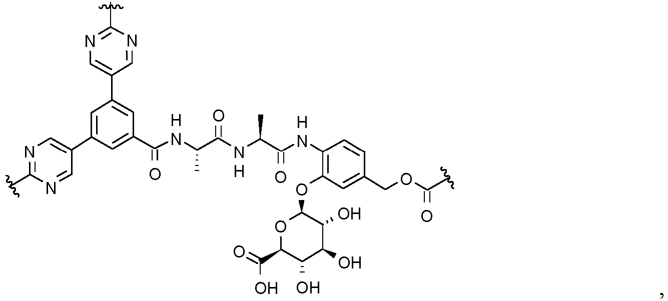 and 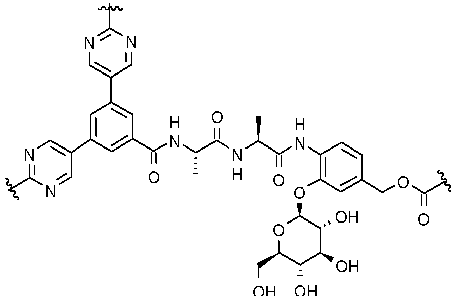 wherein, s is an integer selected from 1 to 20, preferably an integer selected from 1 to 10.

In some embodiments, 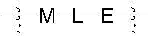 is selected from the following structures: 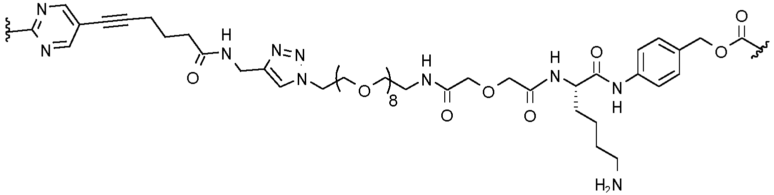 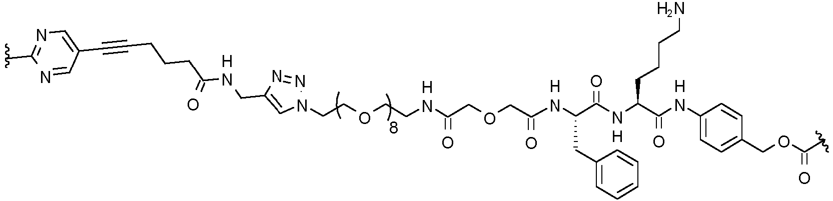 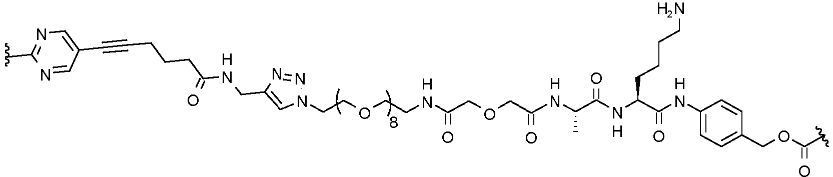 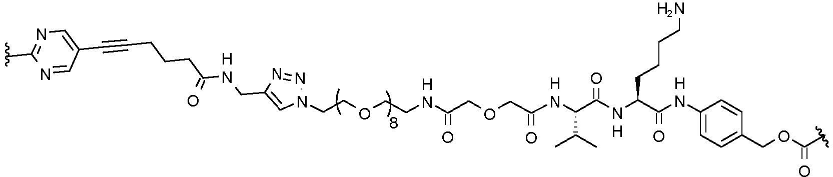 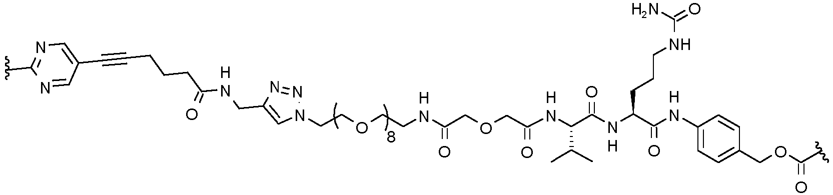 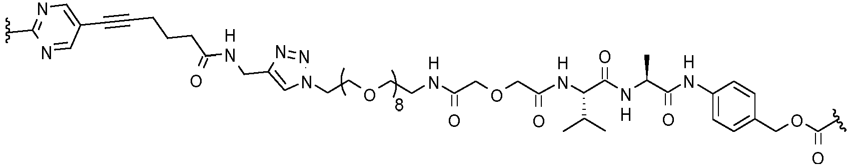 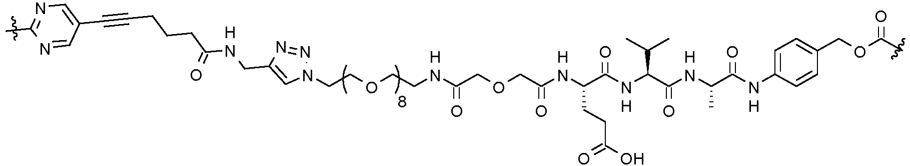 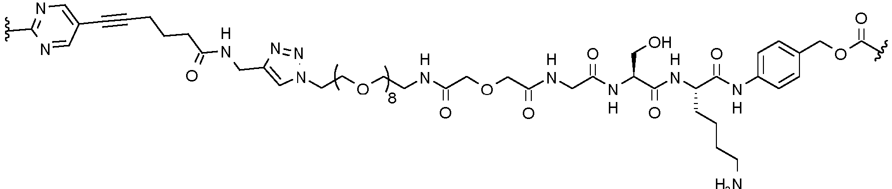 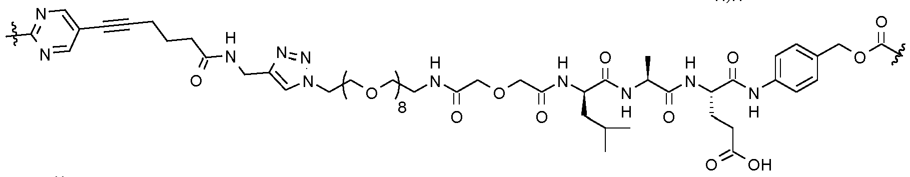 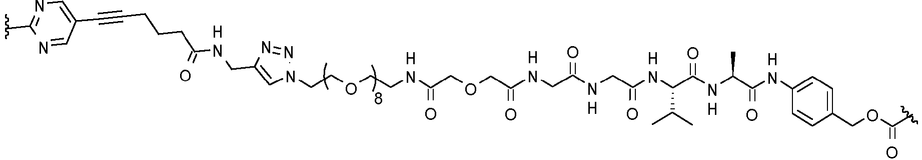 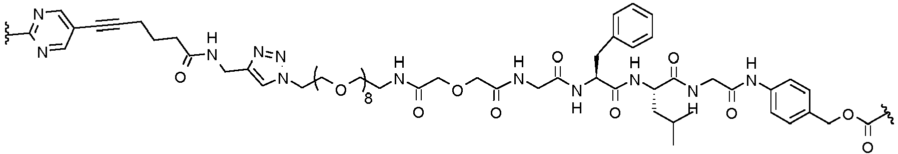 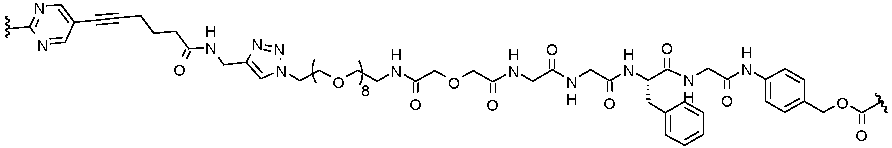 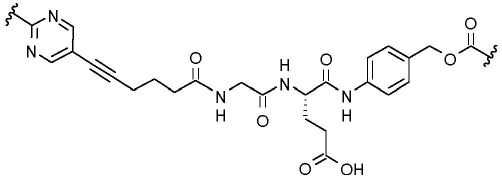 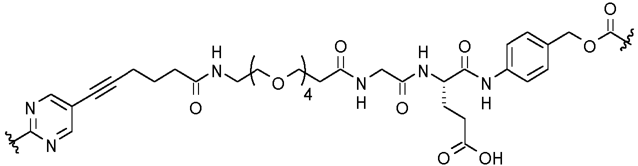 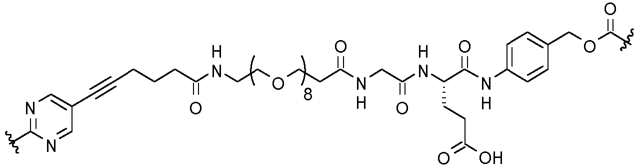 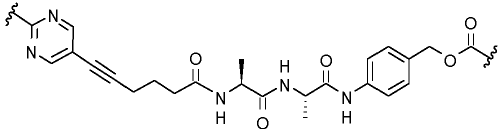 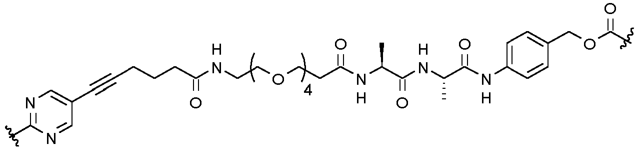 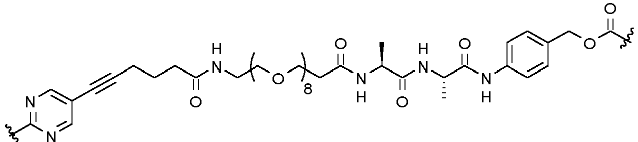 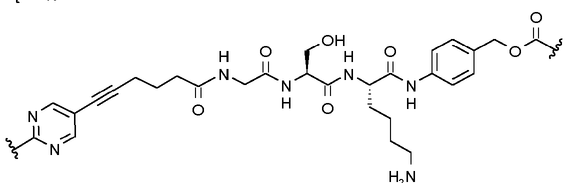 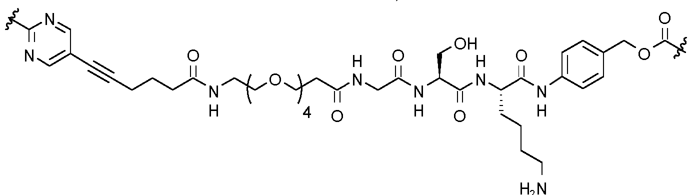 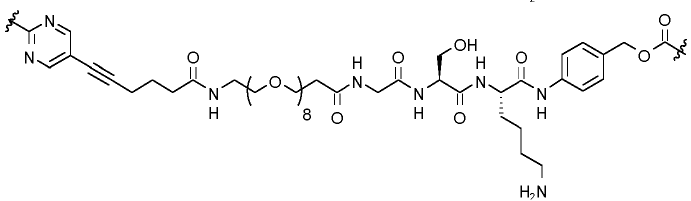 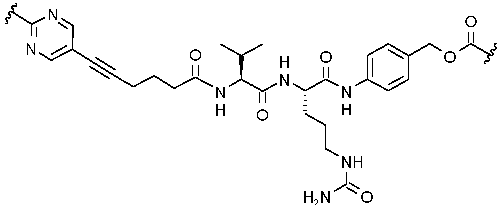 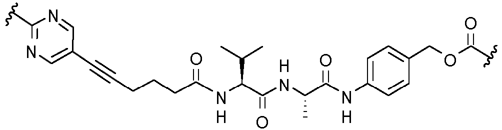 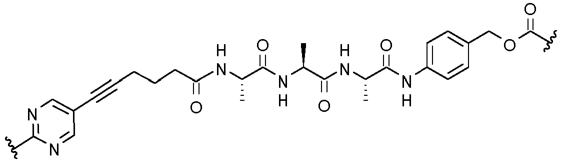 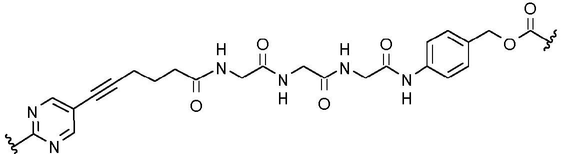 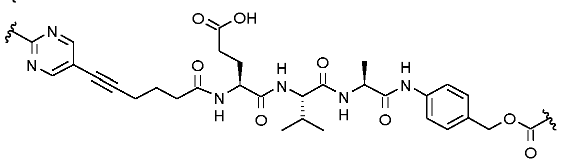 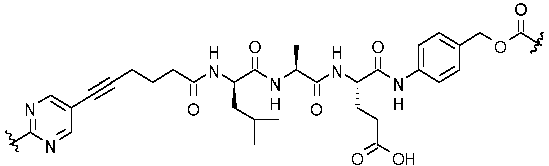 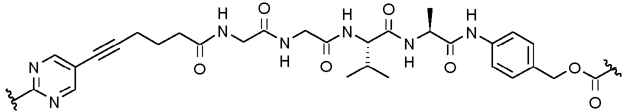 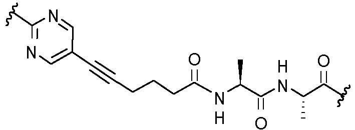 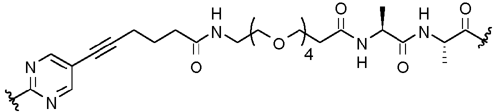 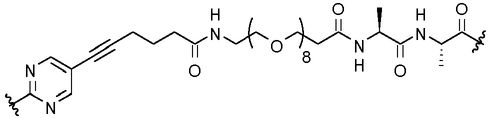 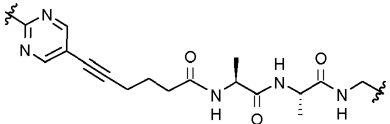 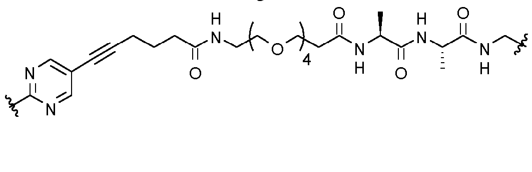 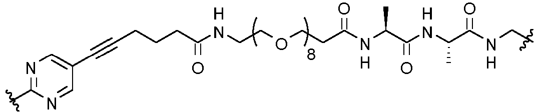 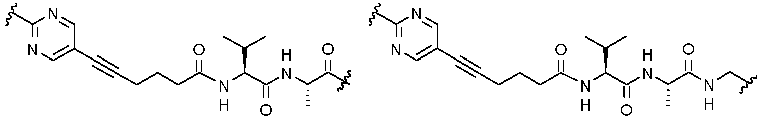 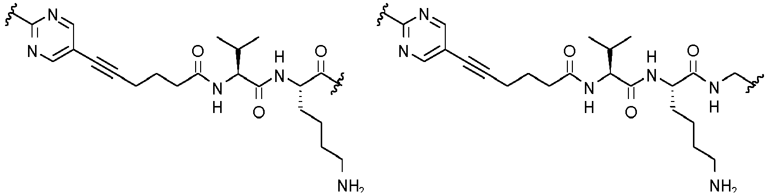 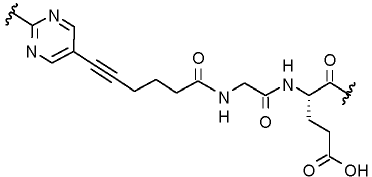 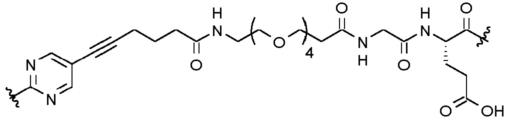 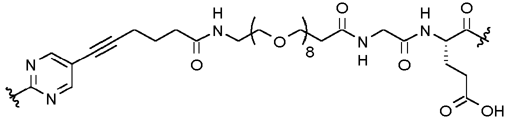 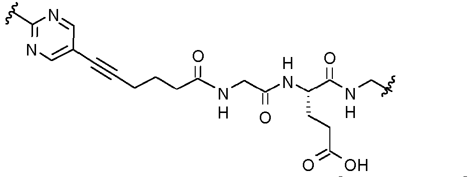 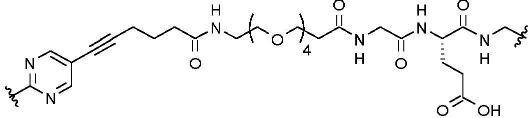 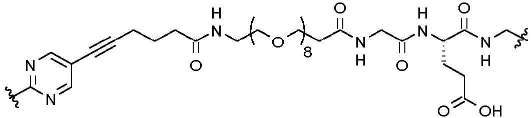 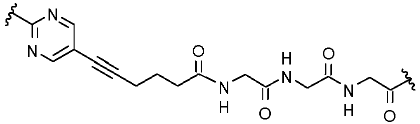 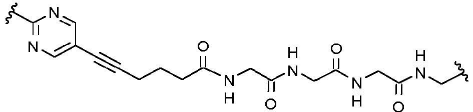 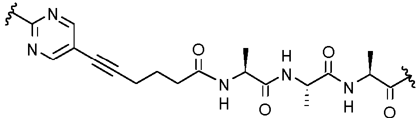 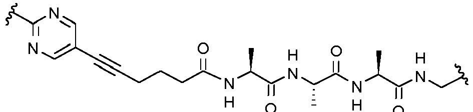 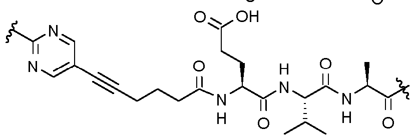 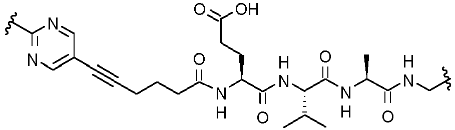 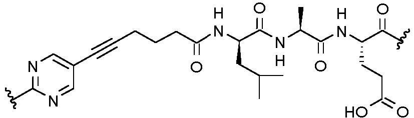 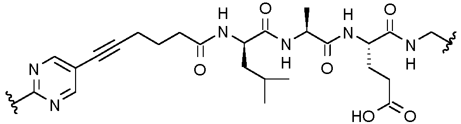 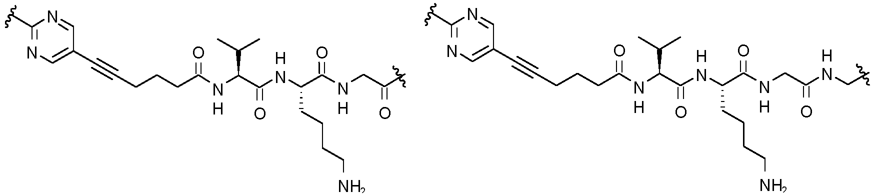 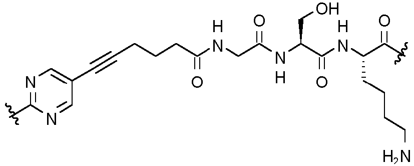 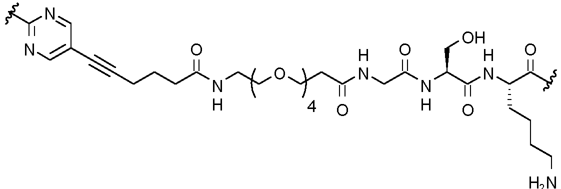 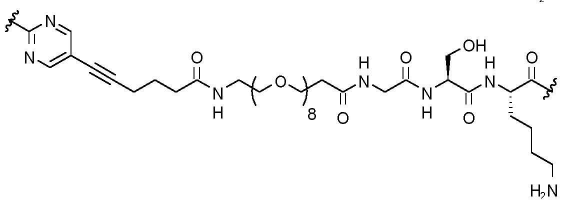 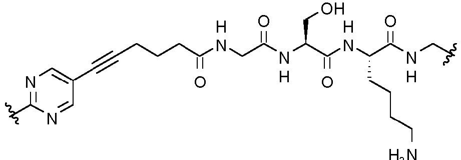 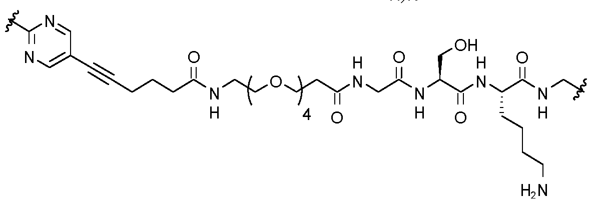 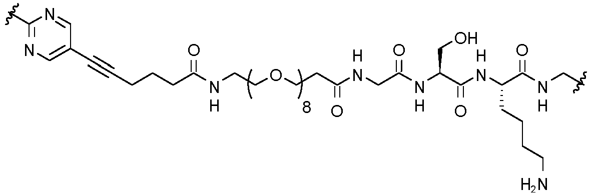 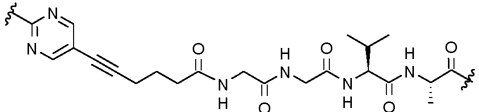 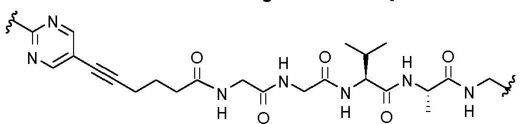 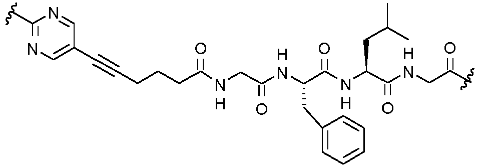 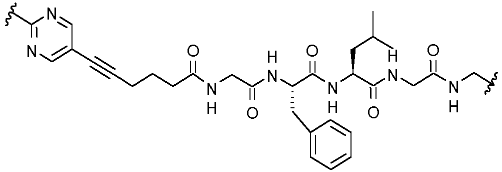 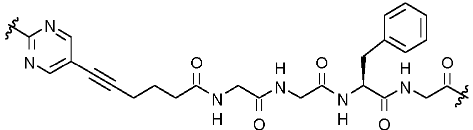 and

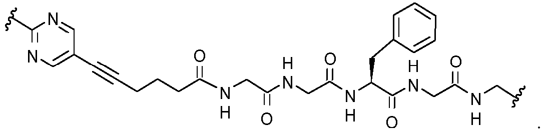

In some embodiments, 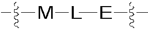 is selected from the following structures: 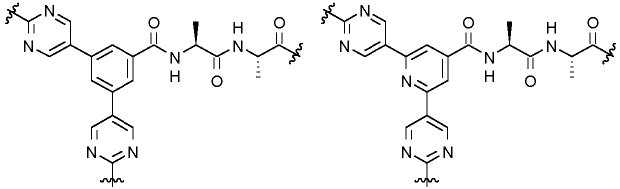 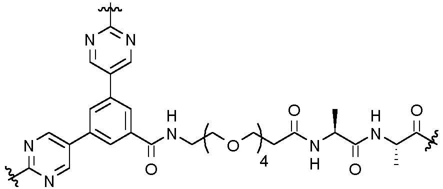 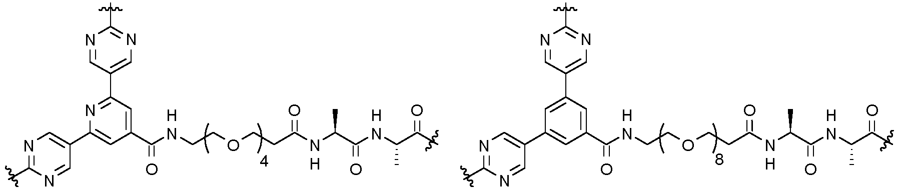 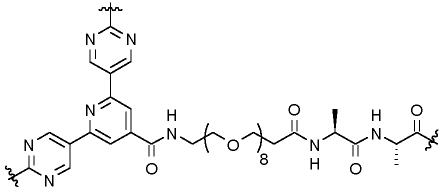 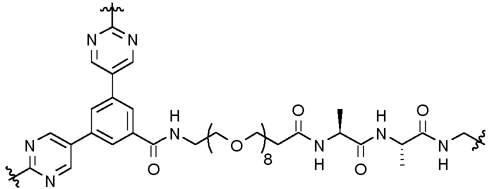 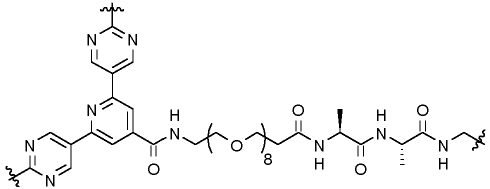 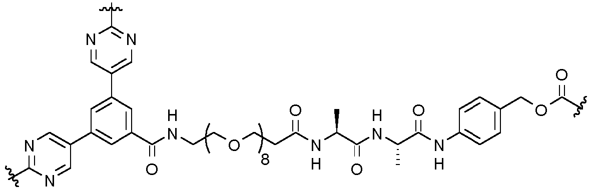 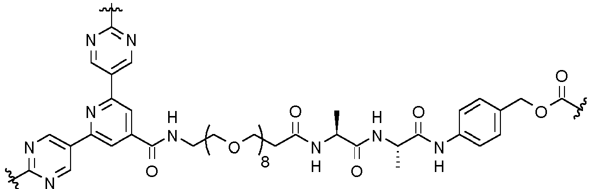 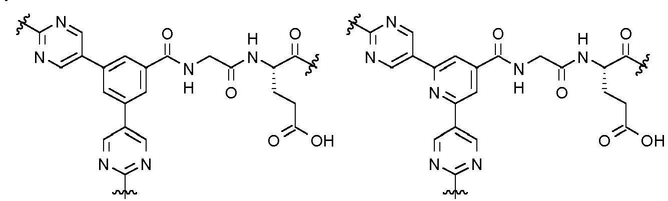 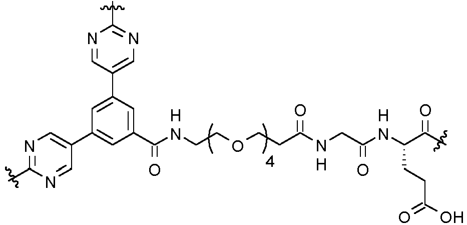 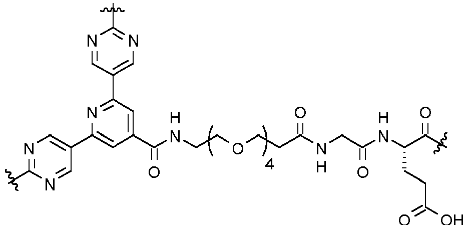 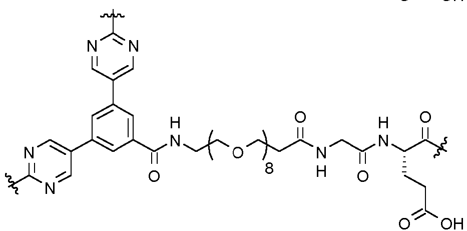 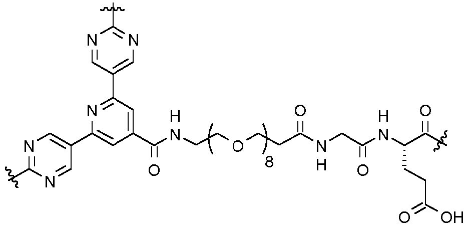 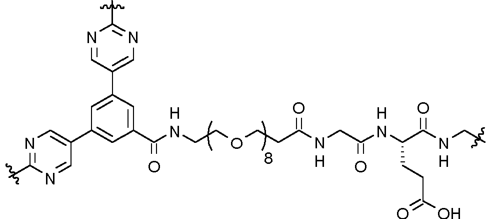 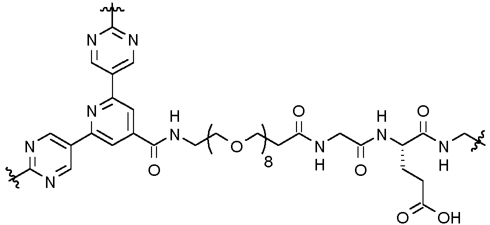 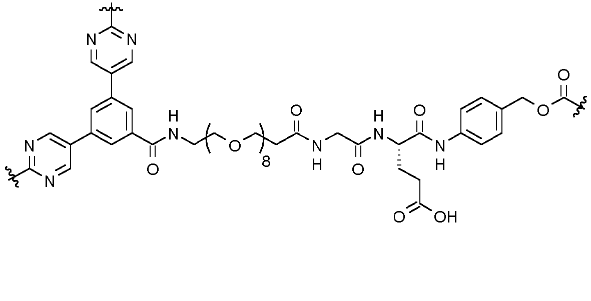 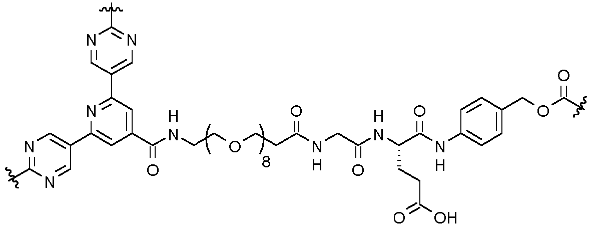 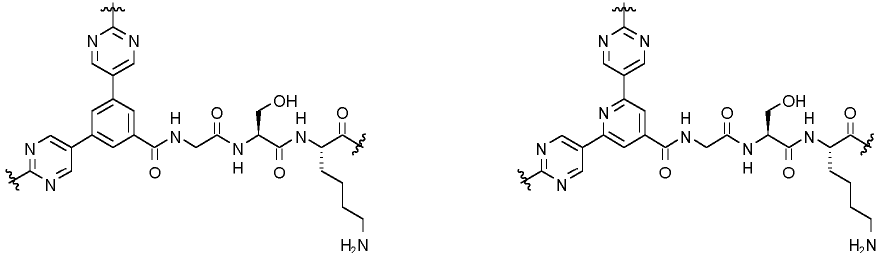 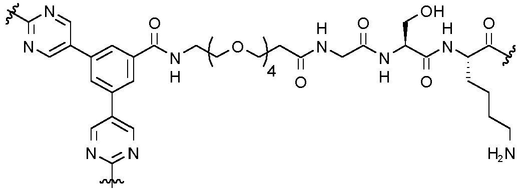 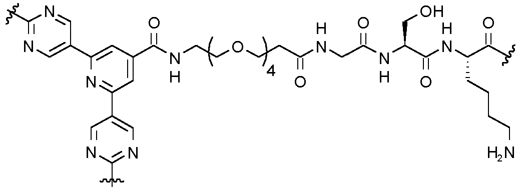 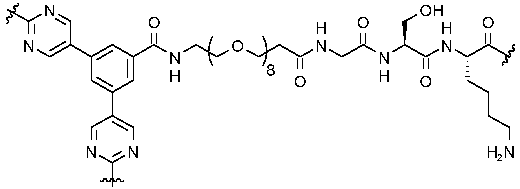 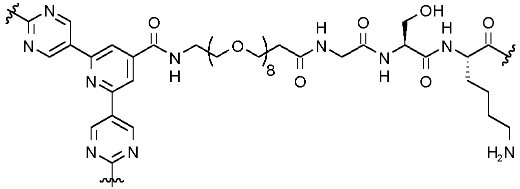 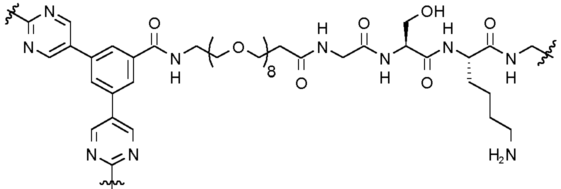 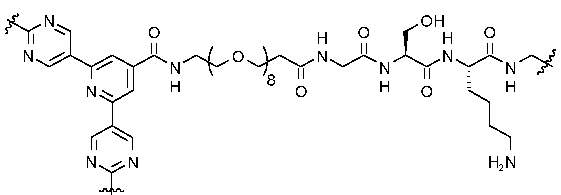 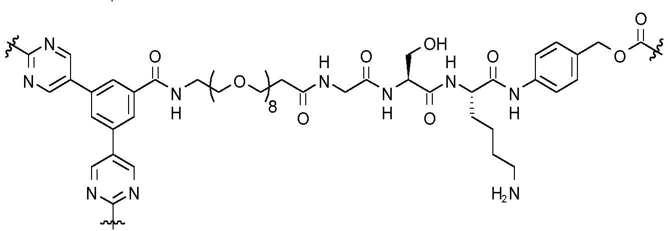 and

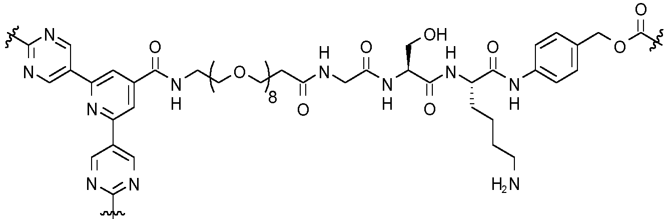

In some embodiments, D is a corresponding agonist fragment obtained by linking the steroid glucocorticoid receptor agonist of the present invention to a linking moiety.

In some embodiments, D is a monovalent structure obtained by losing one H from - OH, -NH₂ or secondary amino on the steroid glucocorticoid receptor agonist of the present invention.

In some embodiments, D is selected from the following structures:

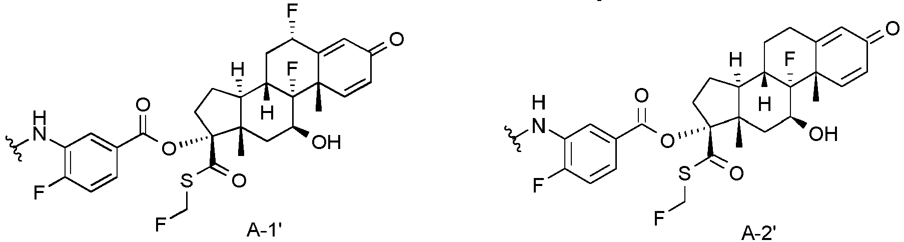

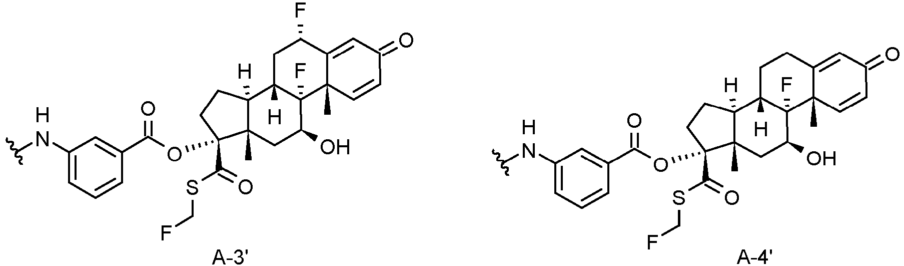

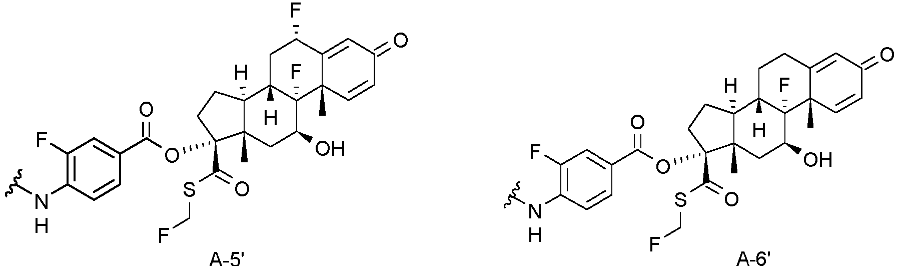

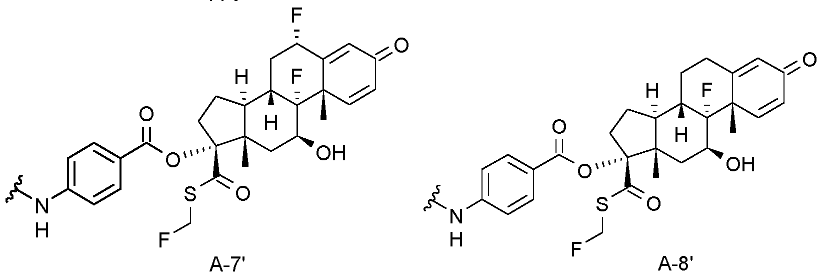

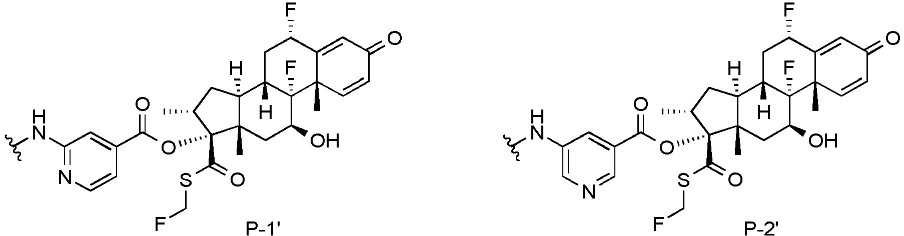

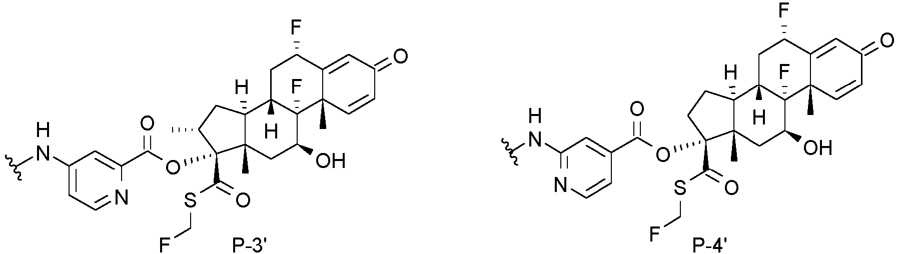

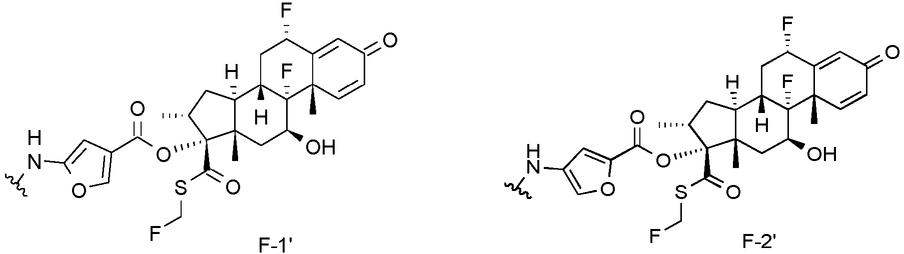

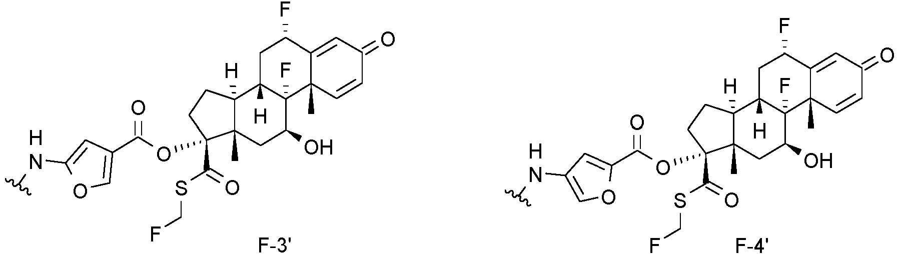

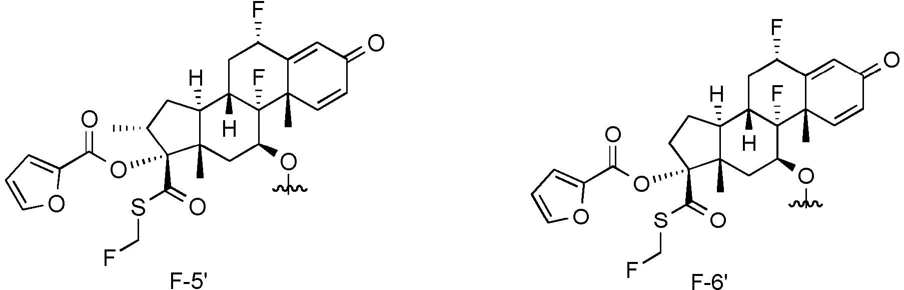

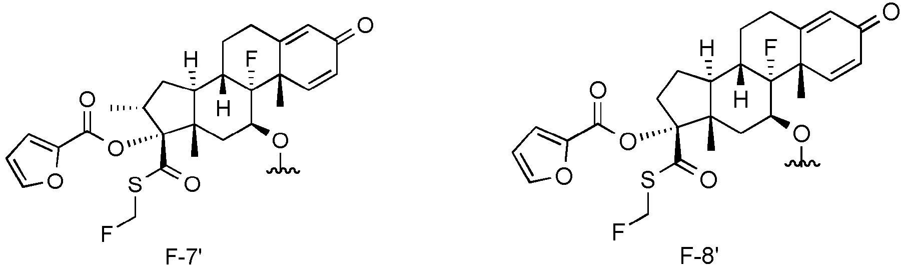

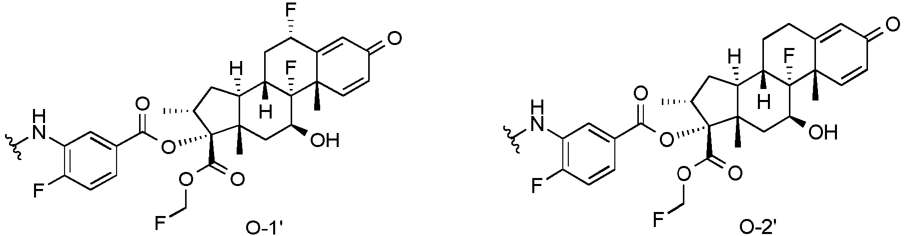

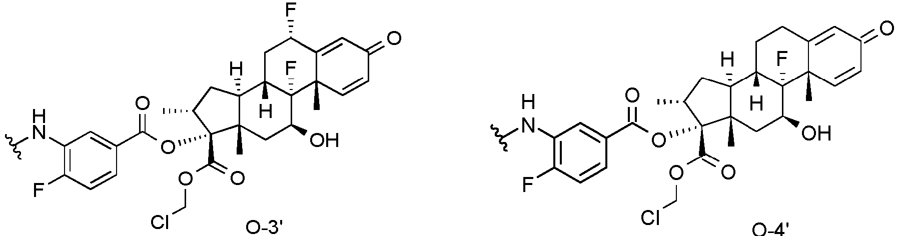

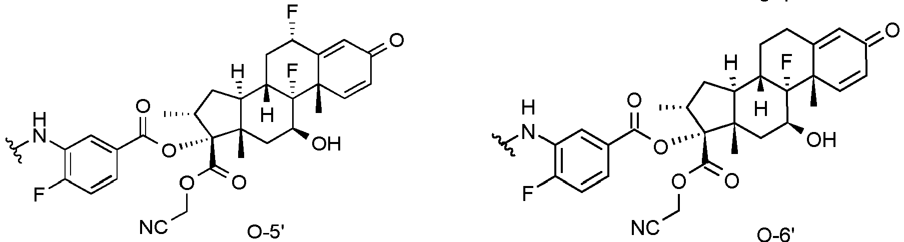

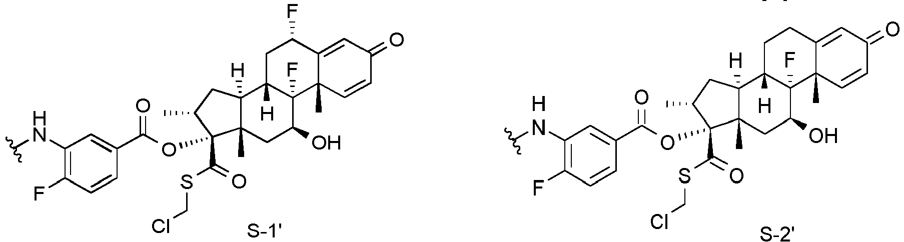

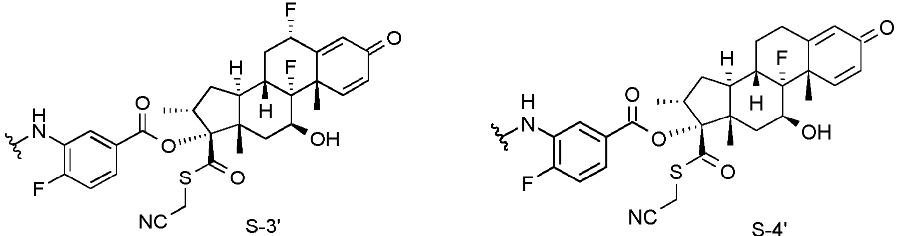

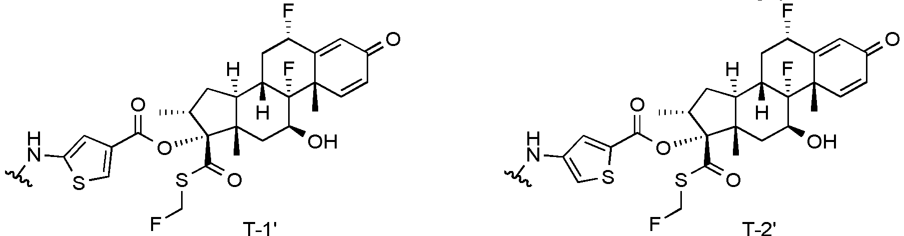

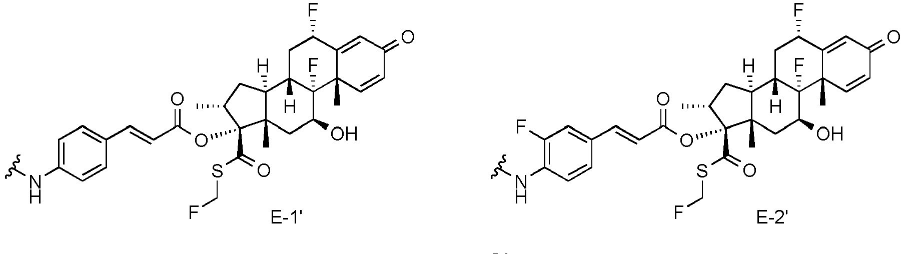

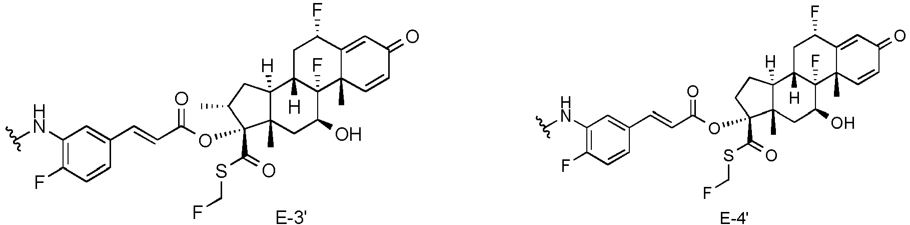

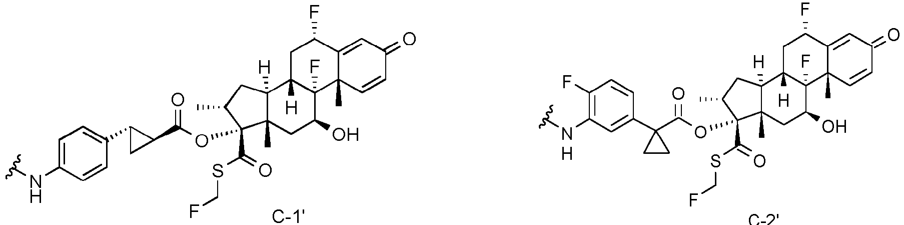

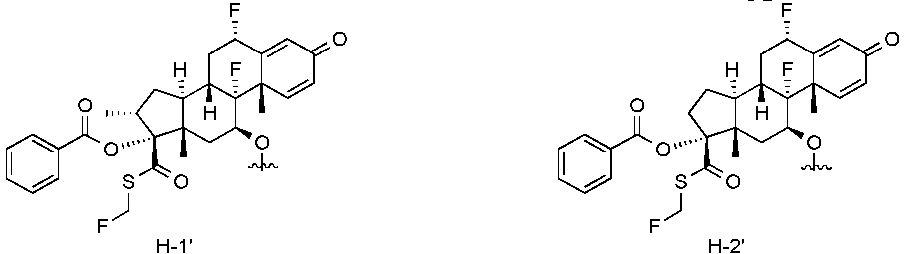

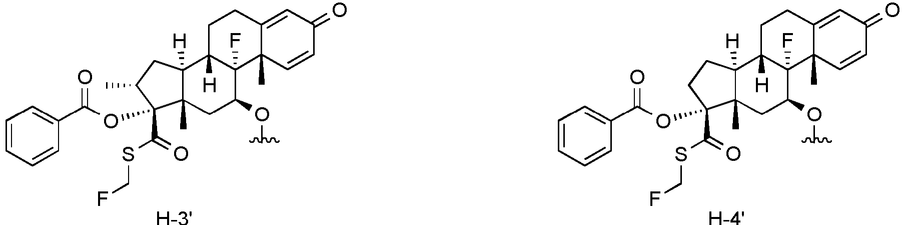

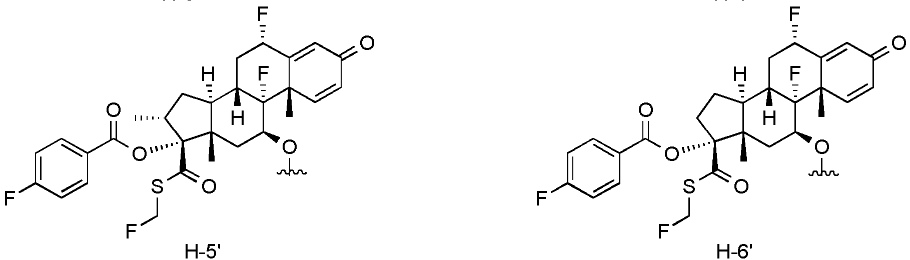

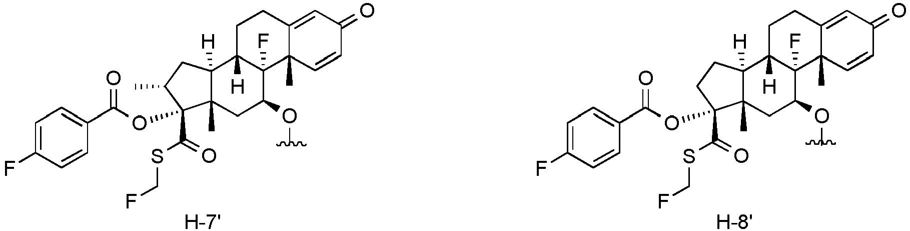

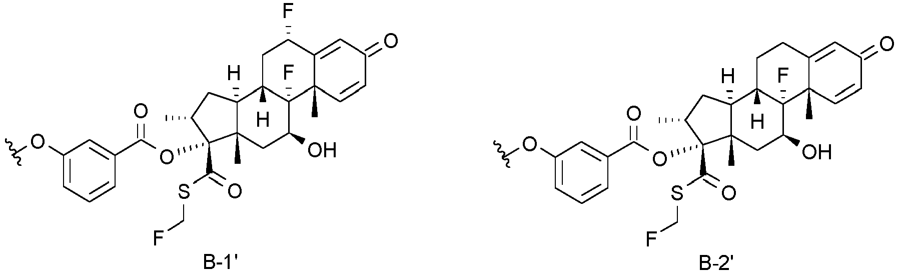

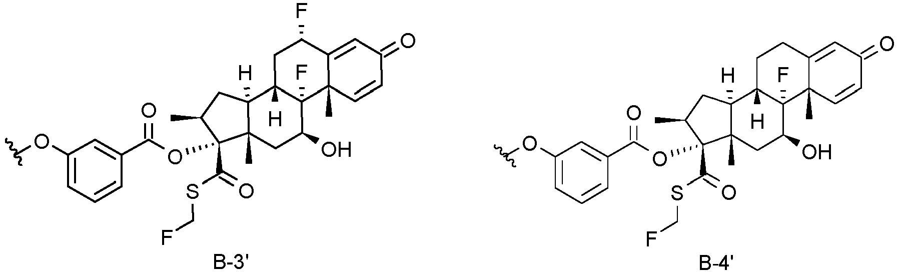

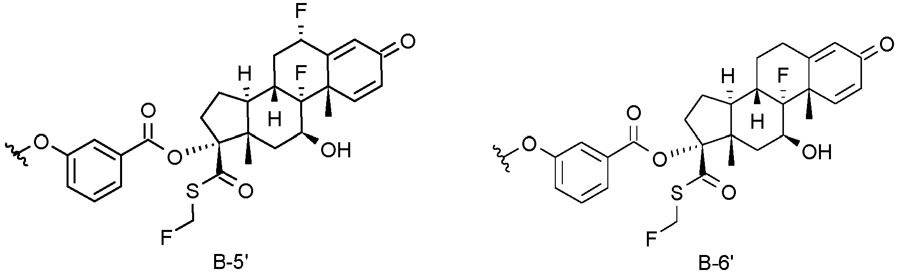

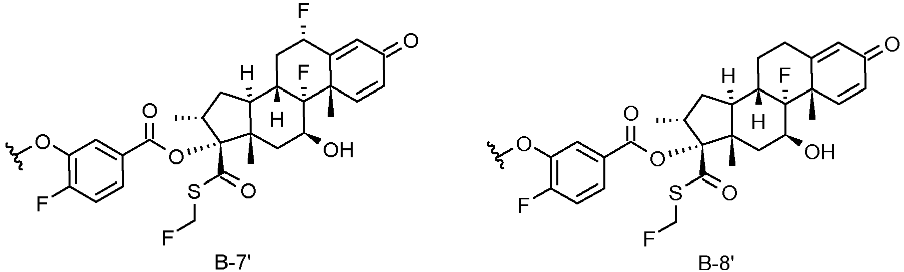

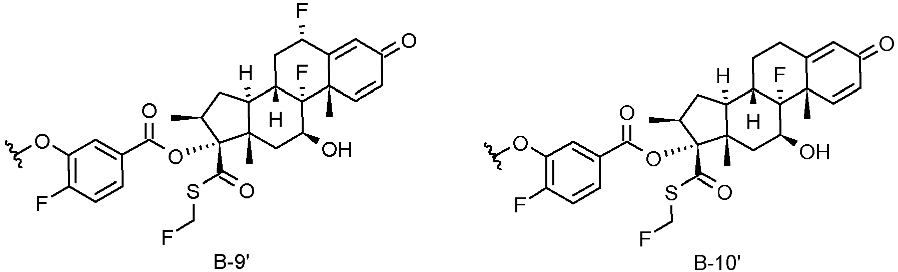

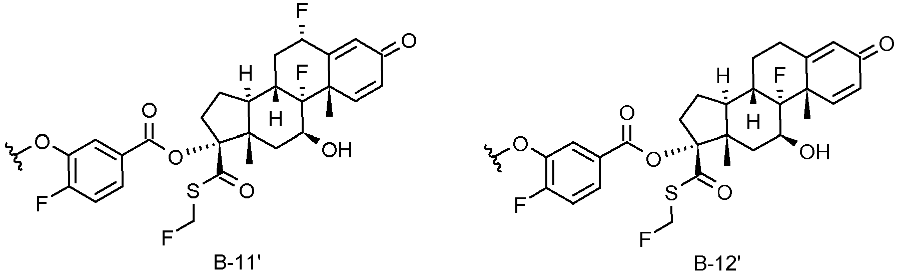

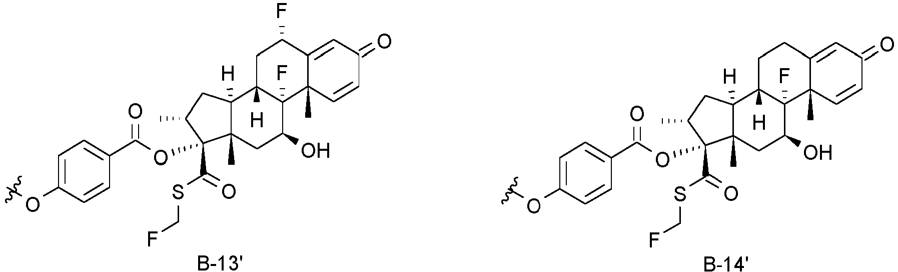

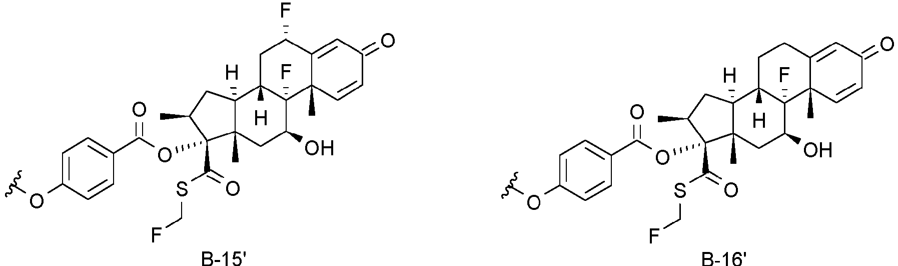

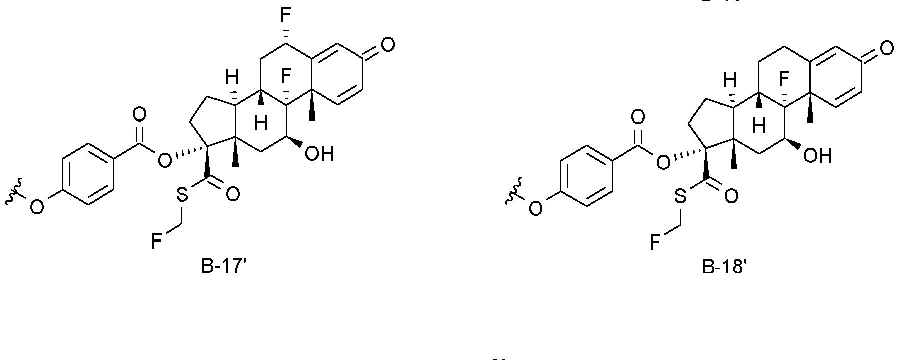

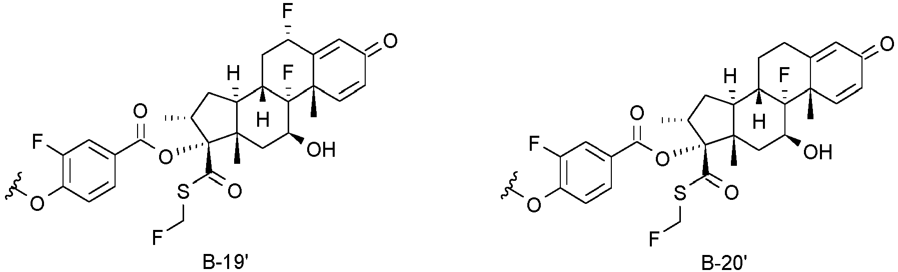

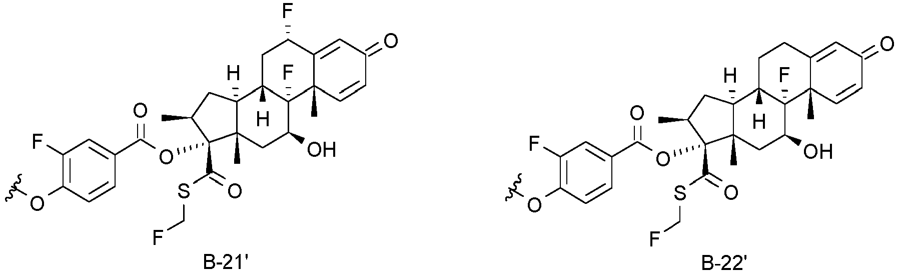

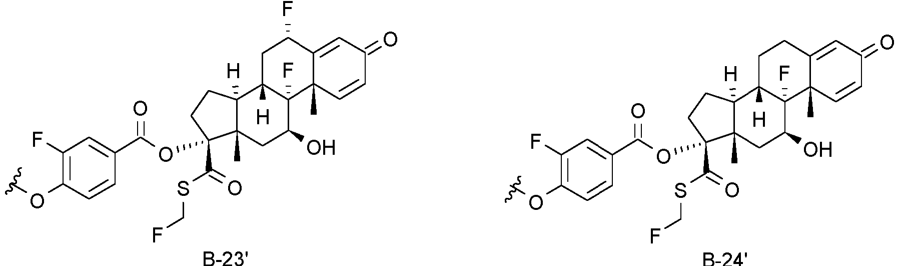

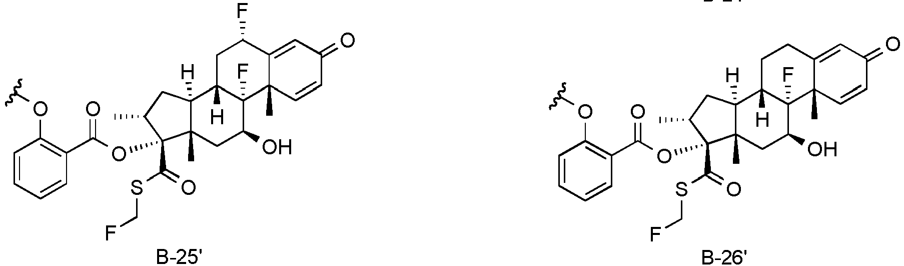

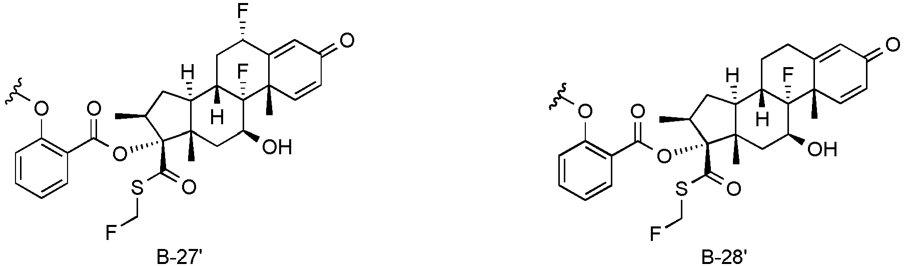

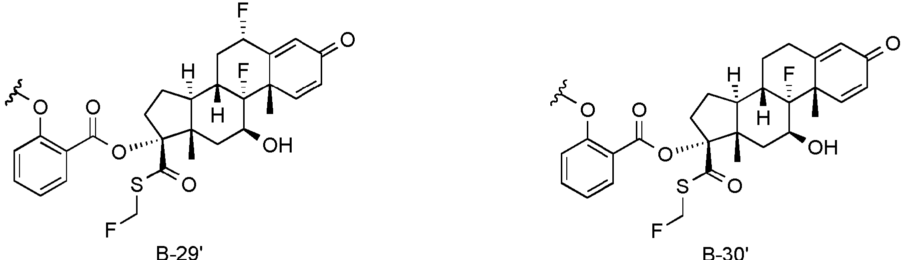

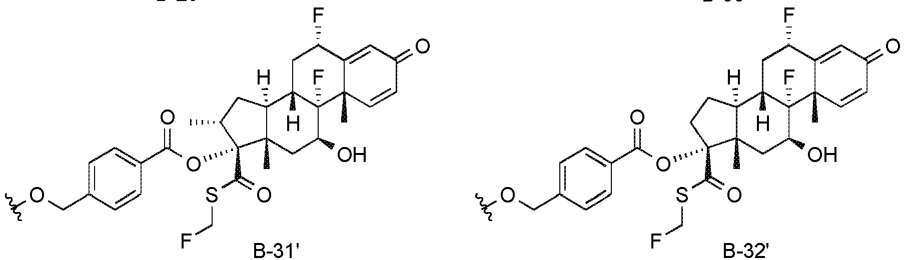

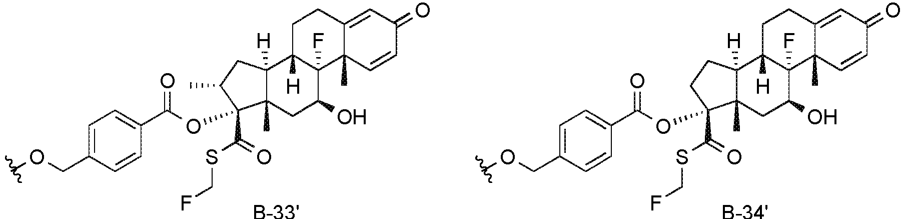

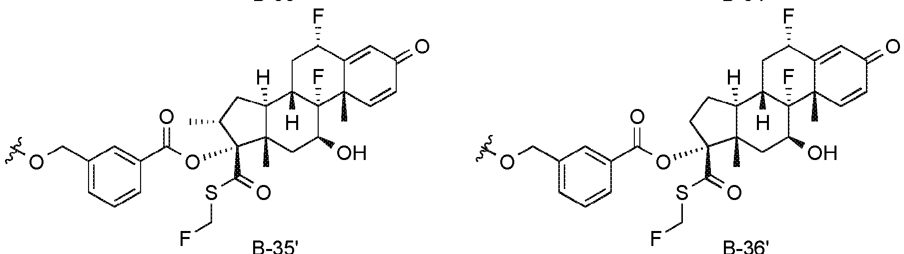

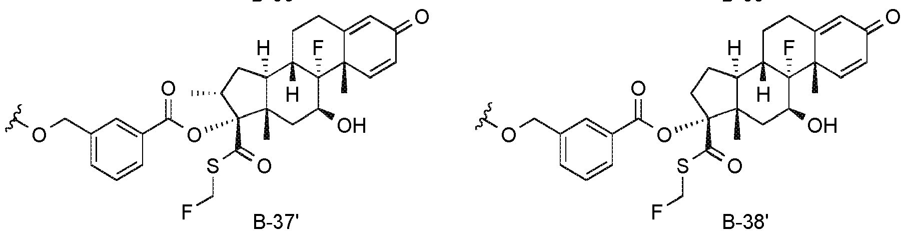

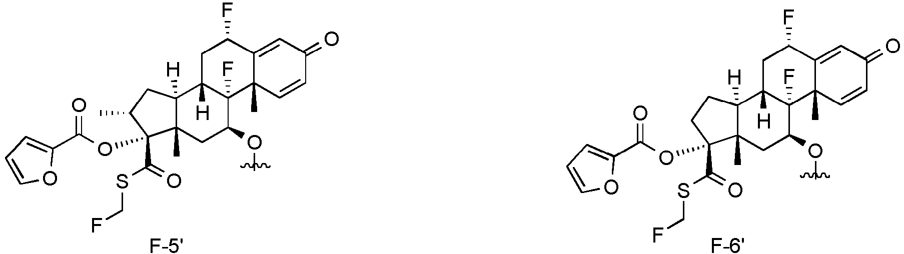

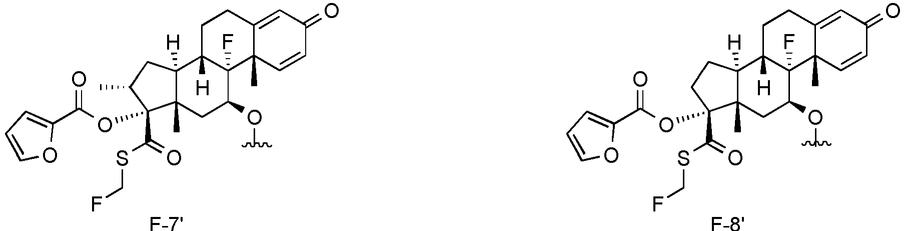

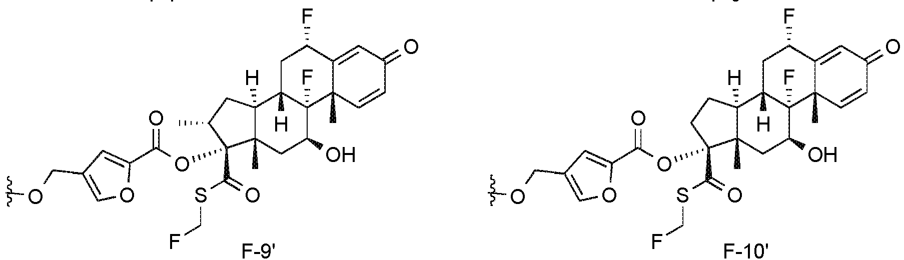

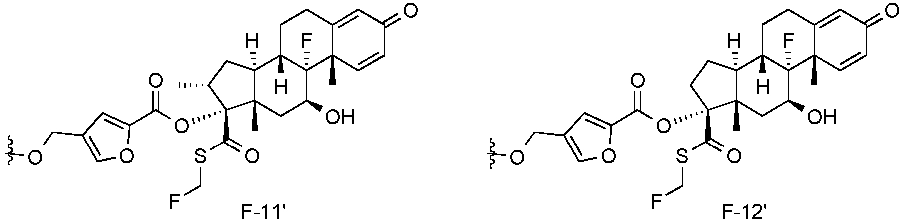

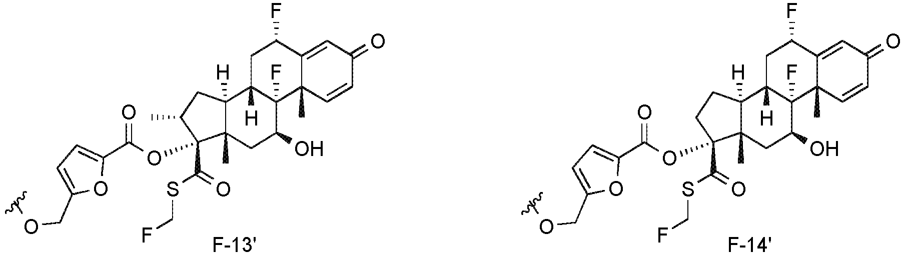

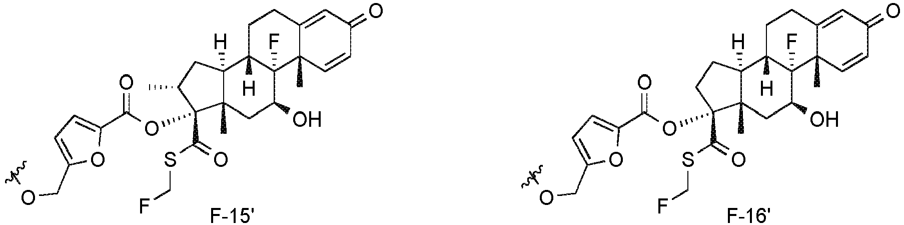

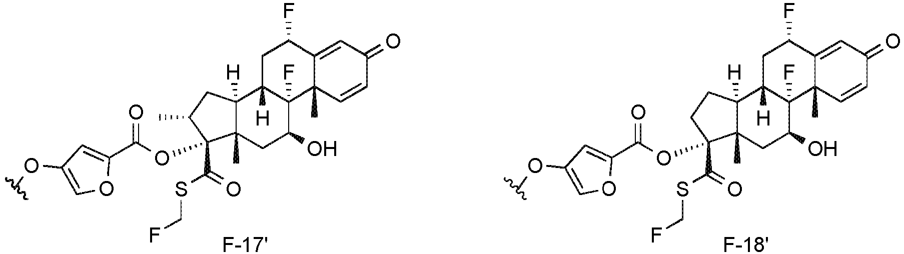

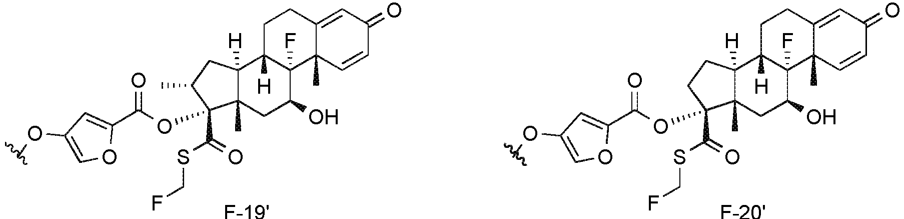

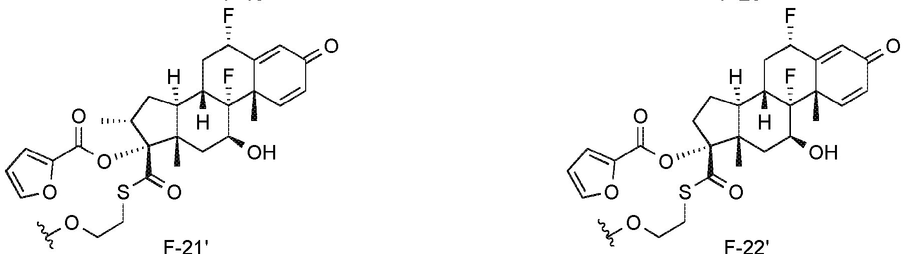

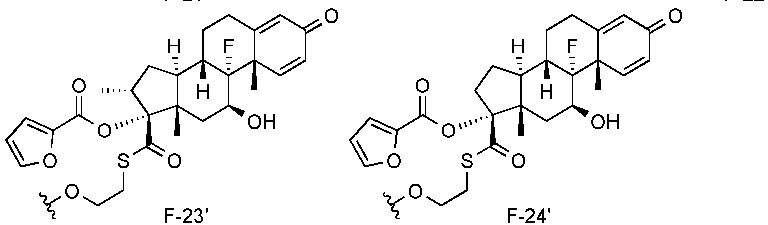

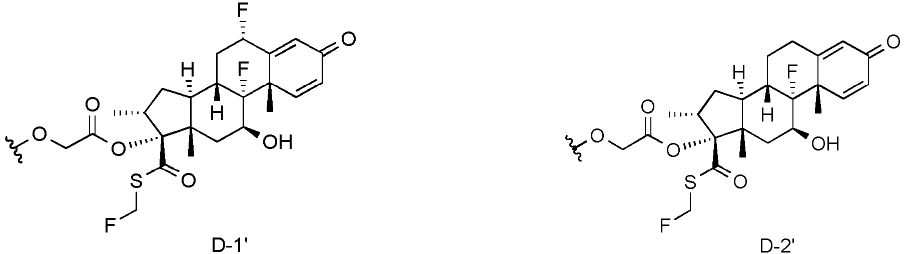

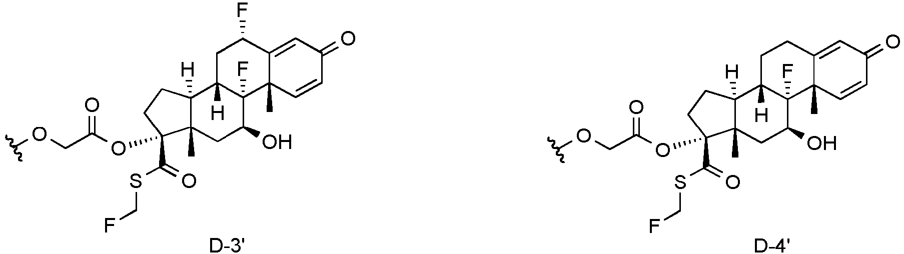

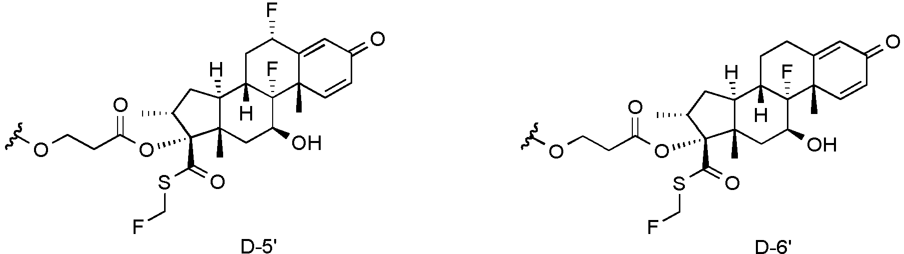

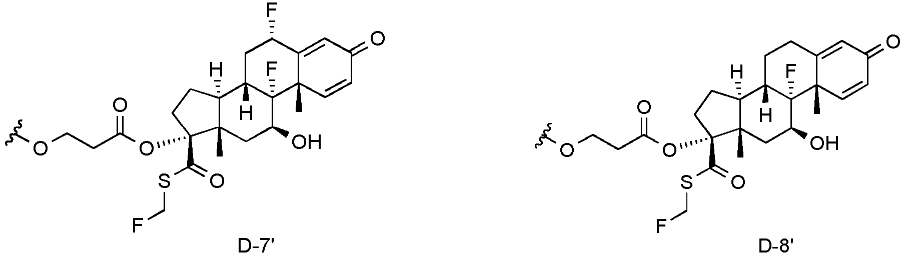

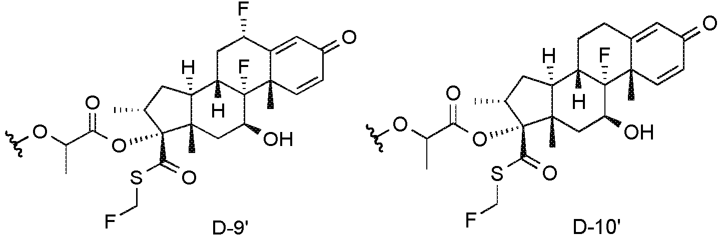

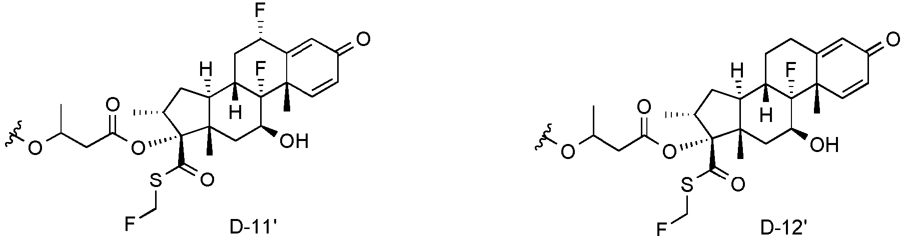

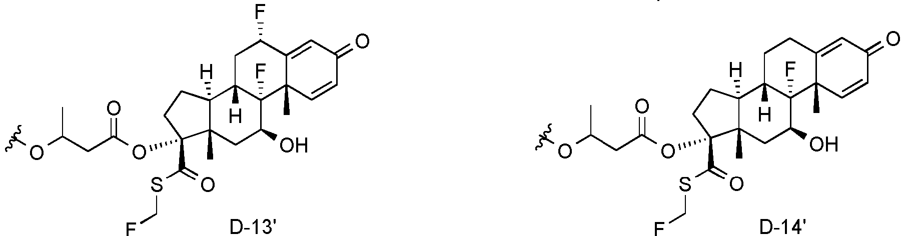

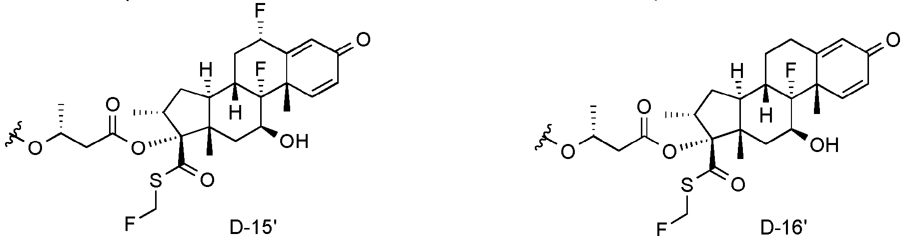

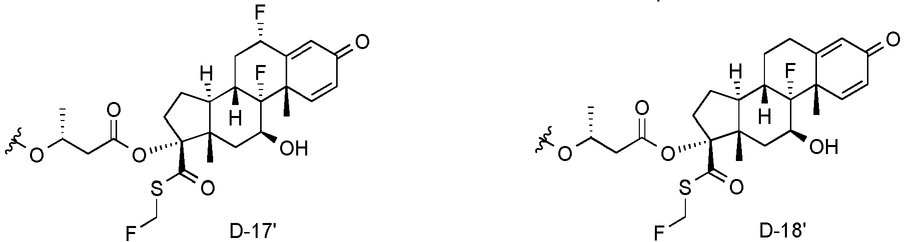

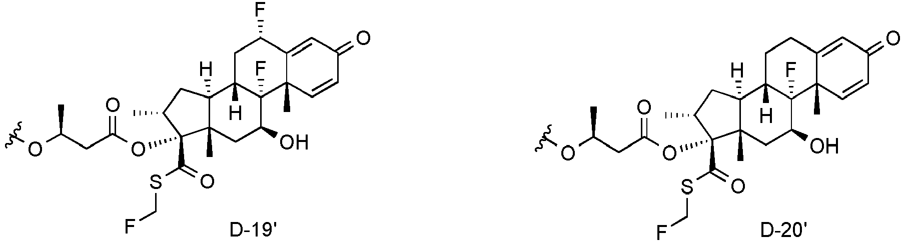

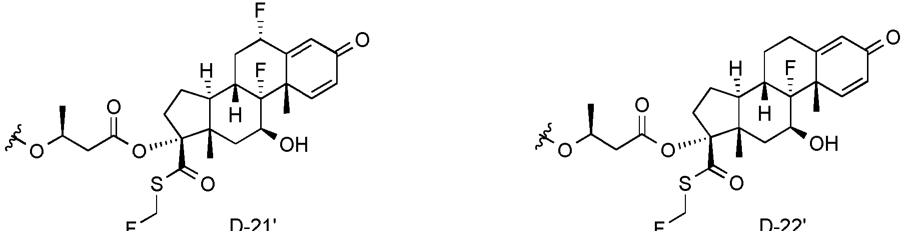

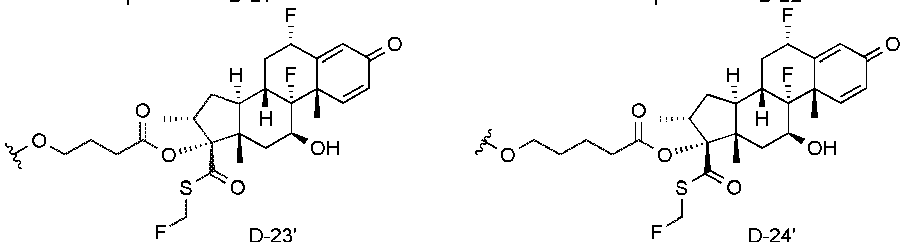

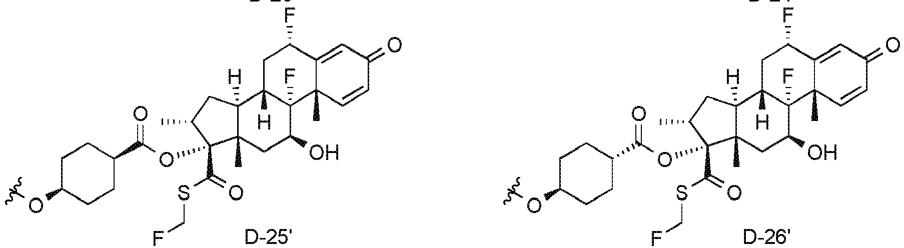

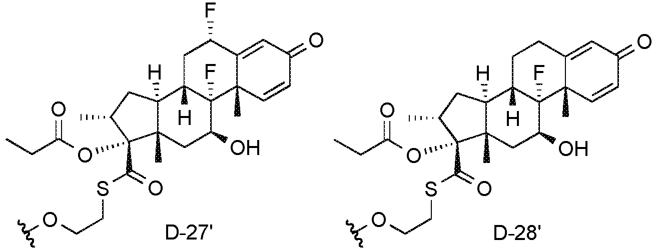

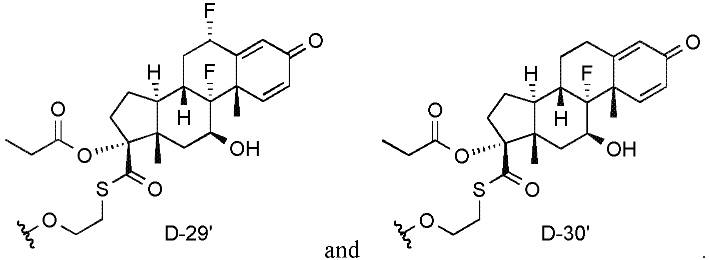

In some embodiments, D is selected from the following structures:

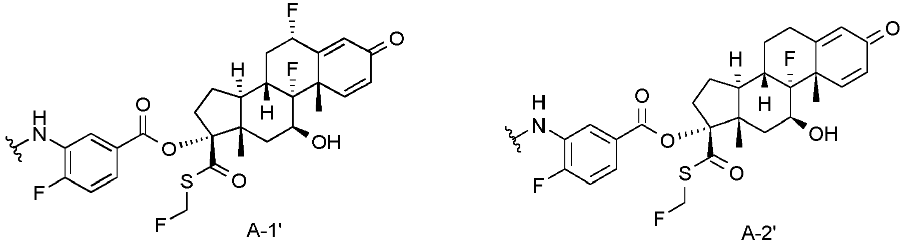

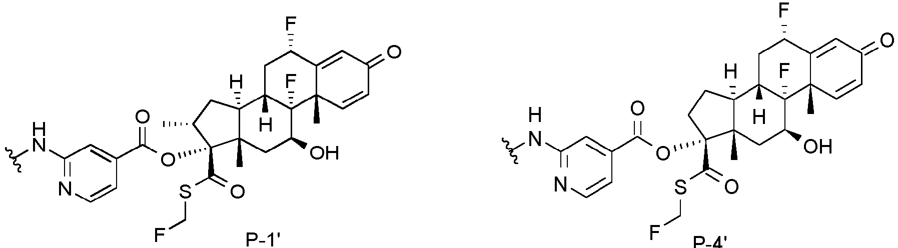

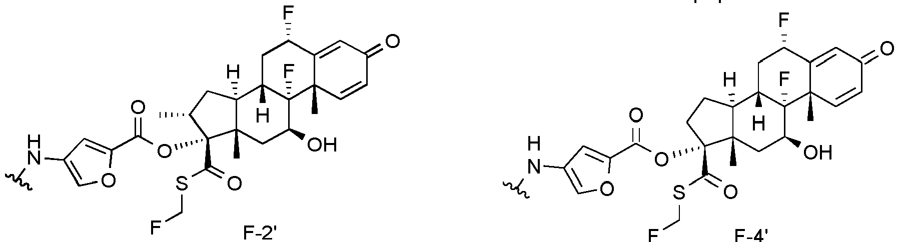

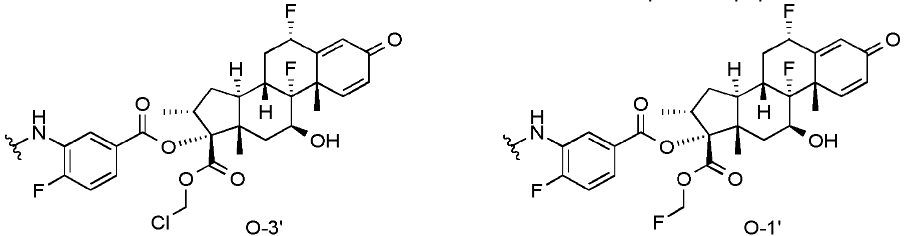

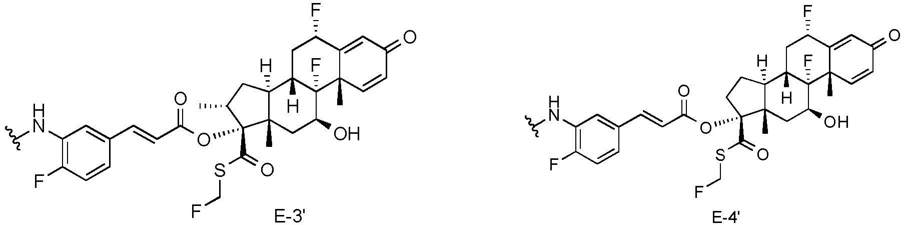

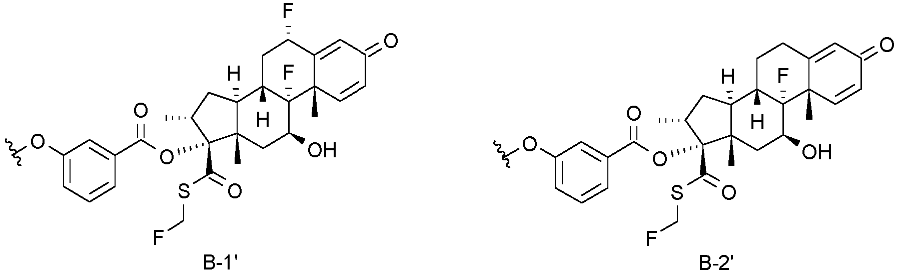

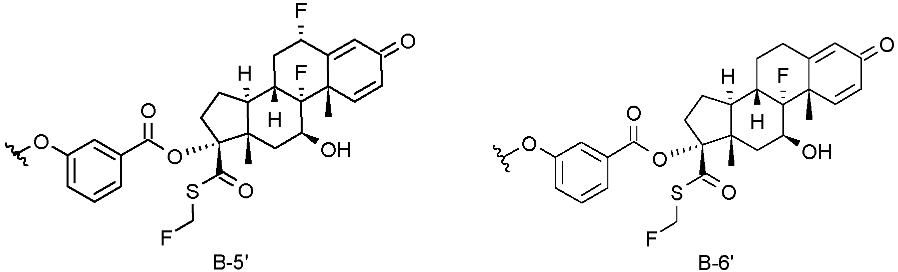

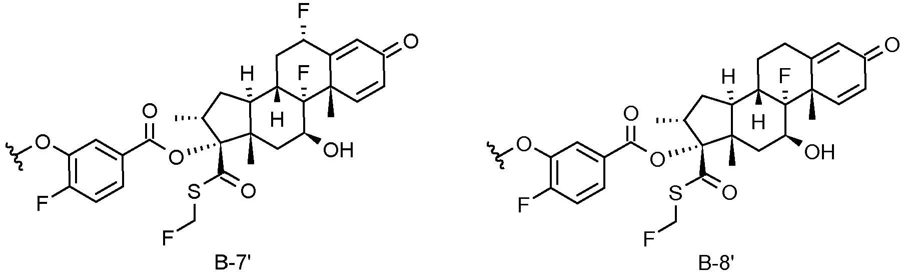

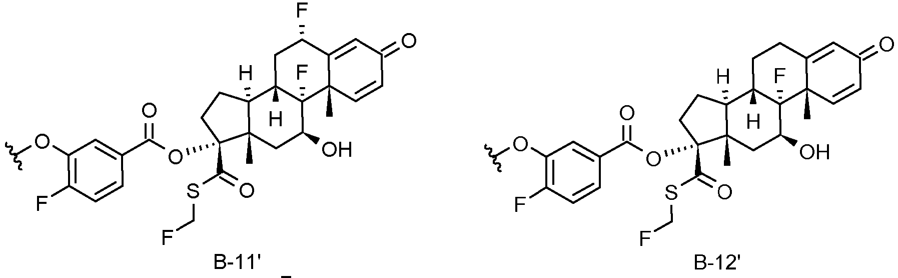

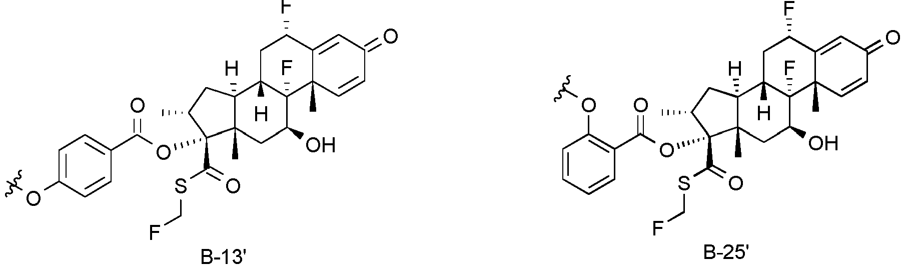

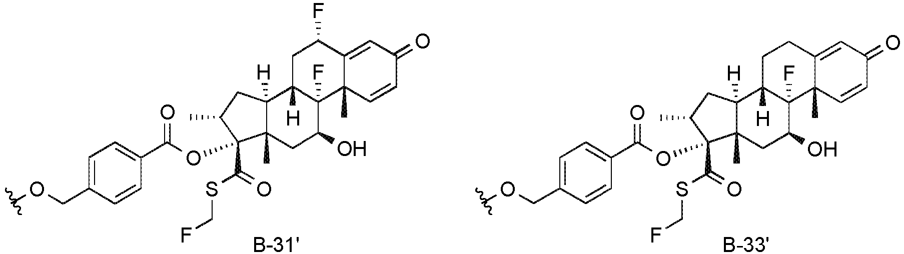

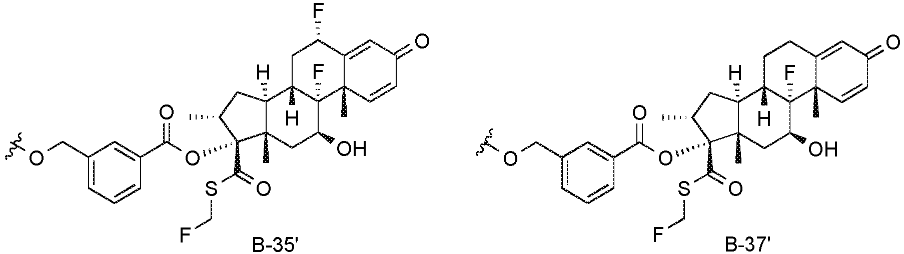

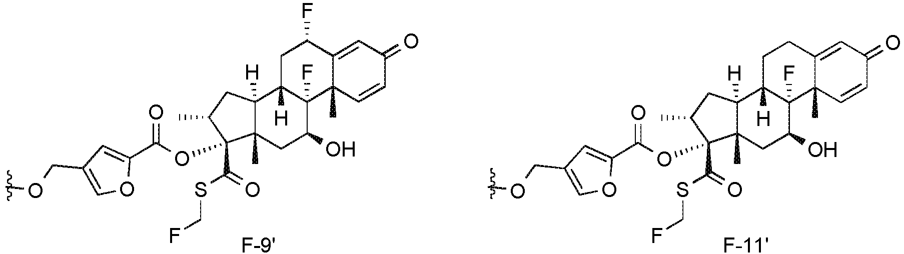

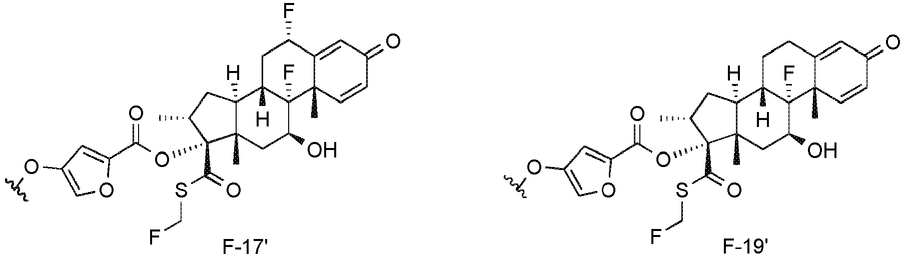

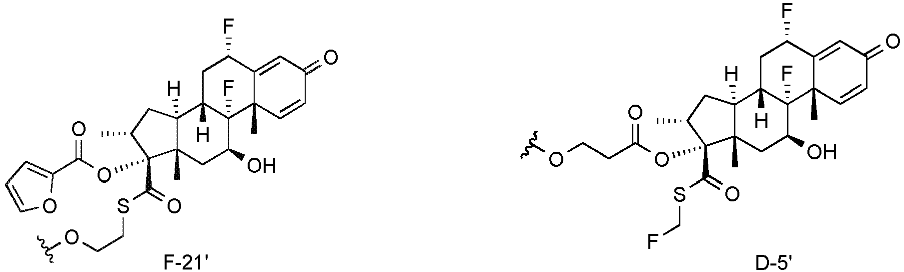

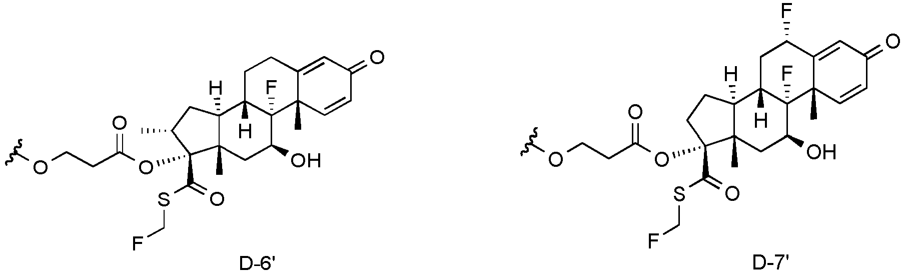

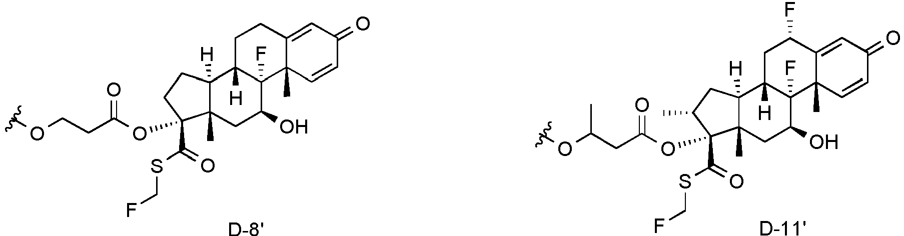

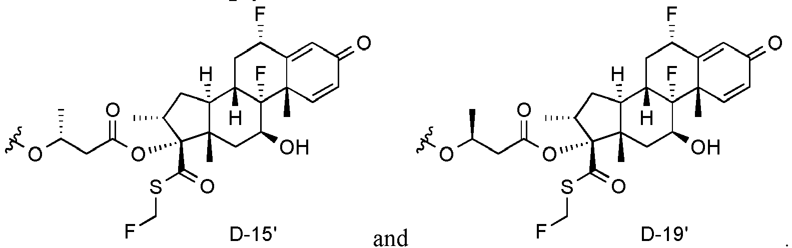

In some embodiments, D is selected from the following structures: 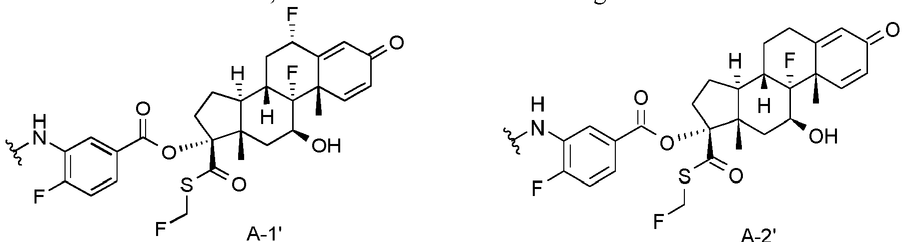 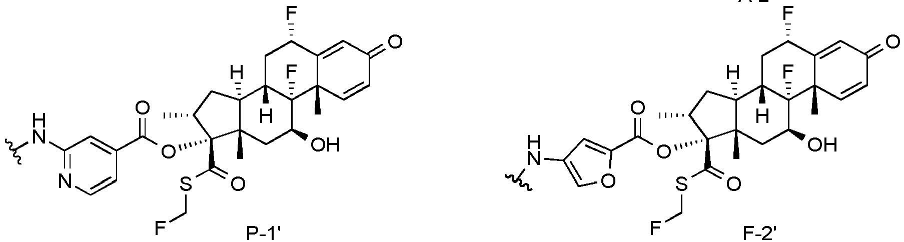 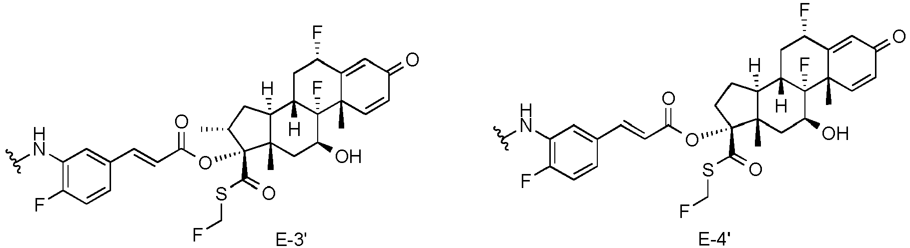 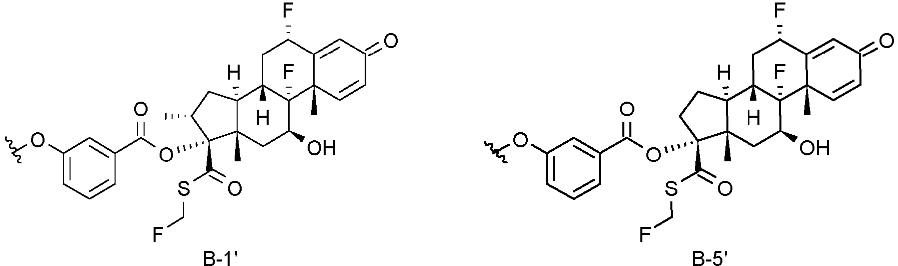 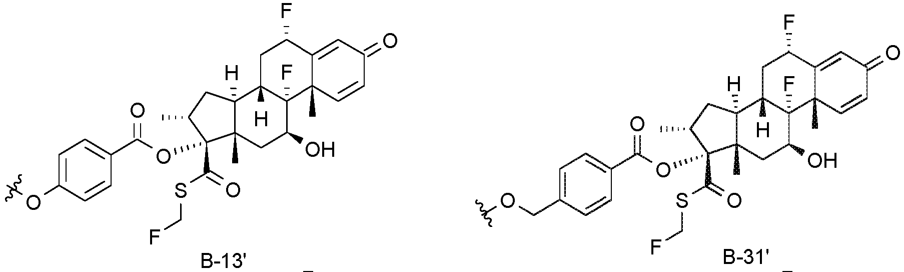 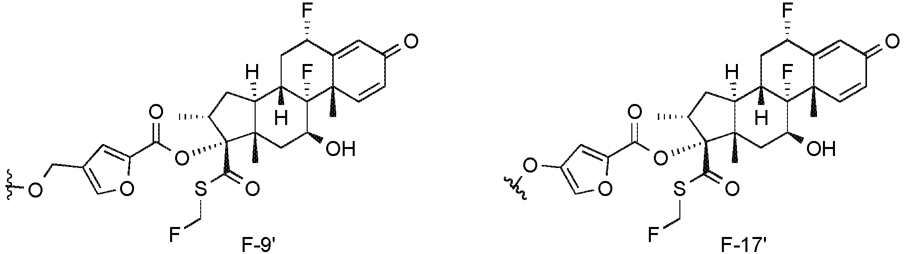 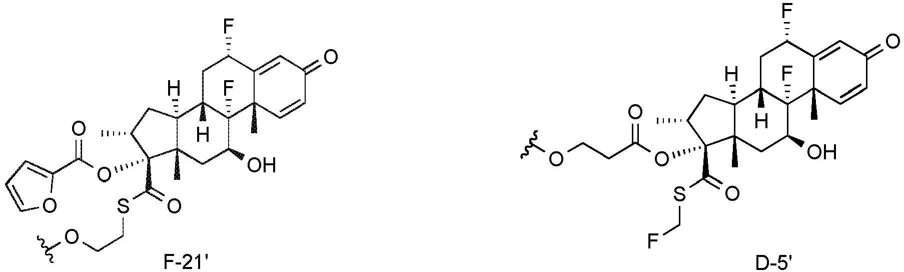 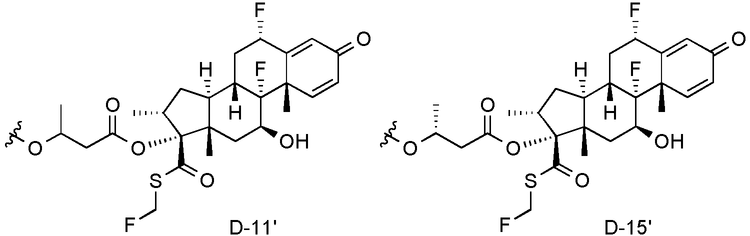 and

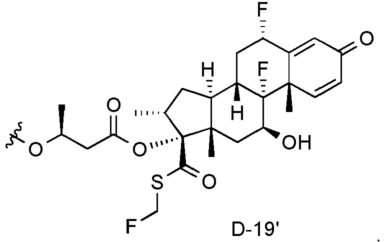

In some embodiments, the antibody-drug conjugate is selected from a group consisting of the following formulae: ADC-A-01 to ADC-A-145, ADC-B-01 to ADC-B-146, wherein the definition of Ab in the following formulae is as described above, in which the mercapto on the antibody and the drug-linker compound form a thioether bond through addition reaction or substitution reaction so as to obtain an intact antibody-drug conjugate, x represents the drug load capacity, and s represents an integer of 1 to 20, preferably an integer of 1 to 10. 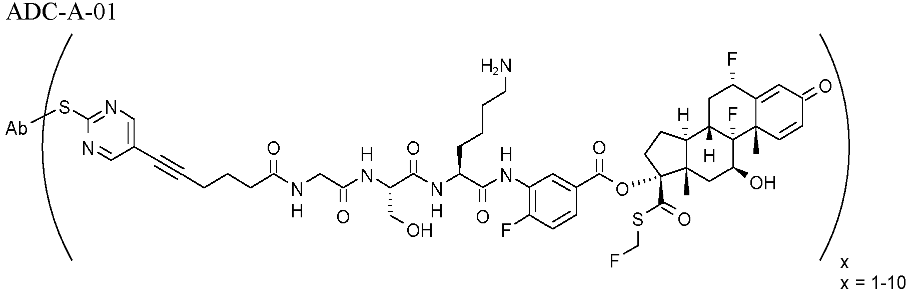 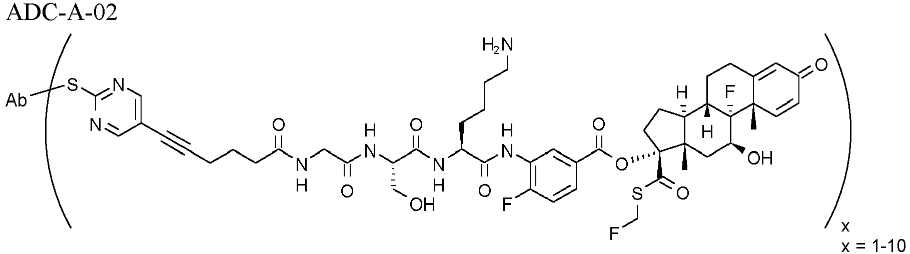 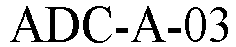 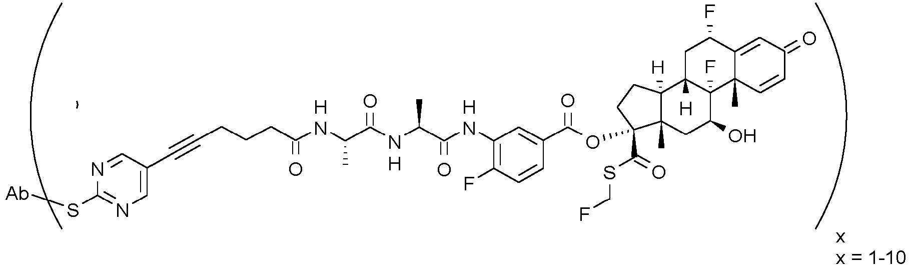 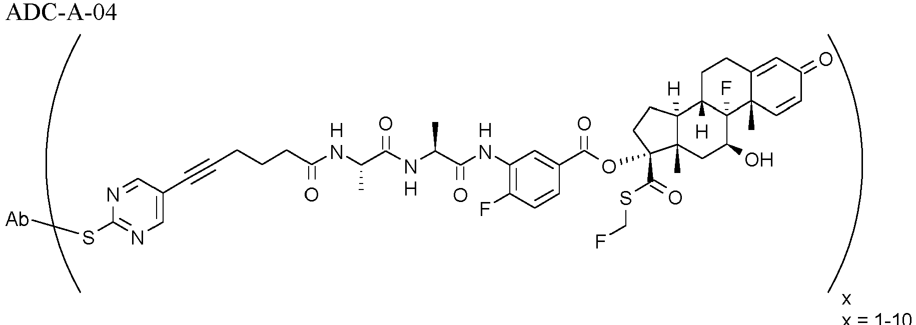 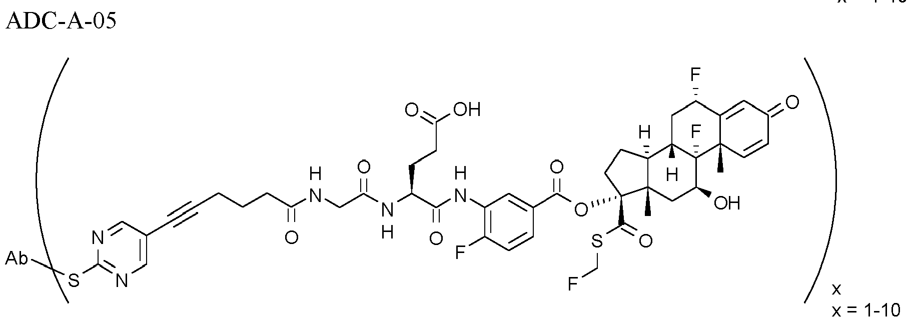 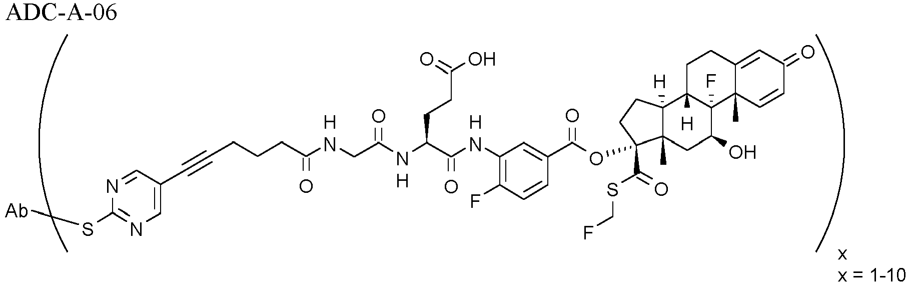 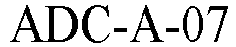 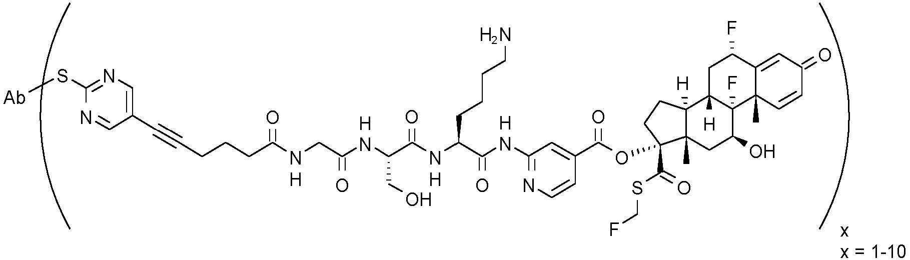 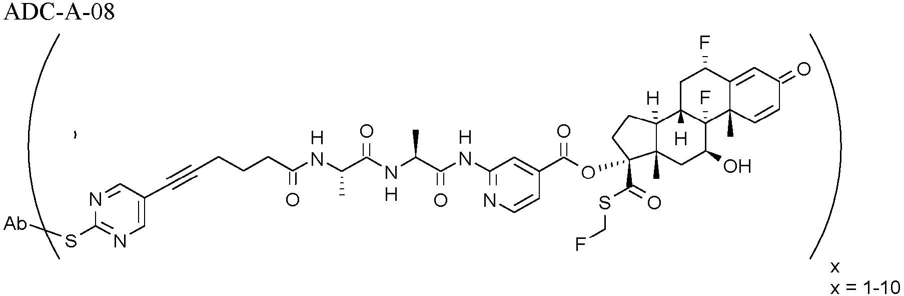 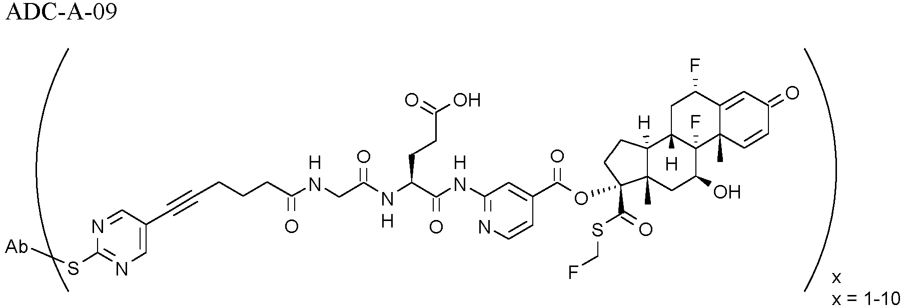 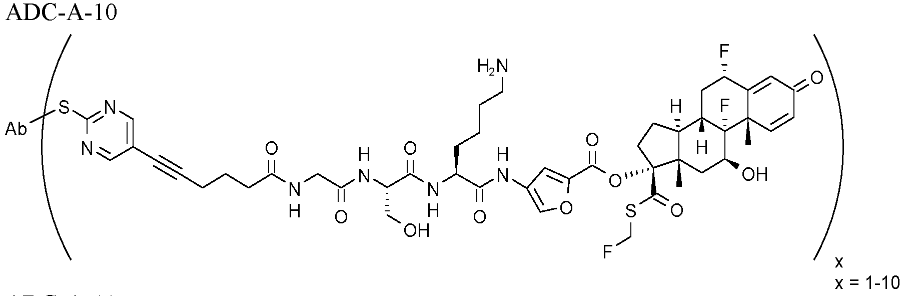 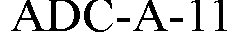 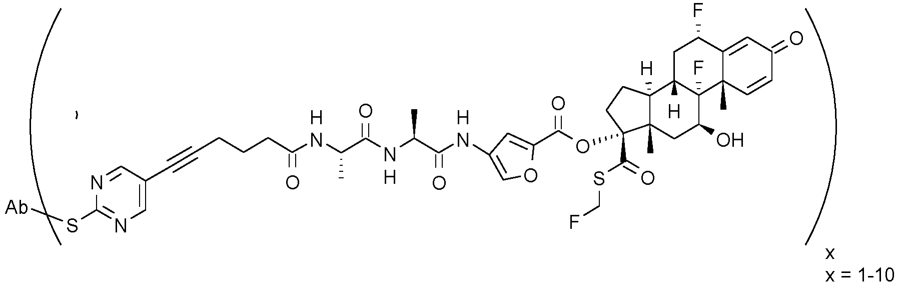 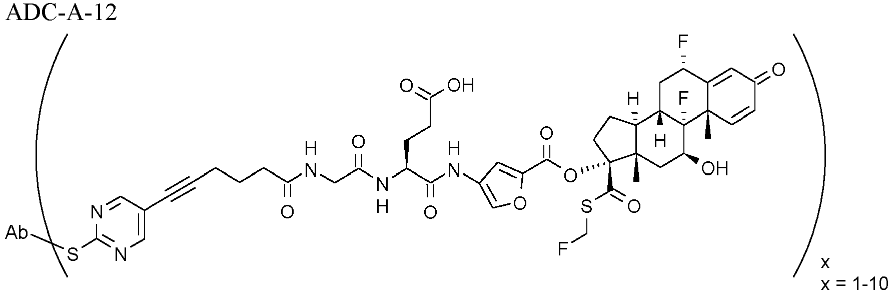 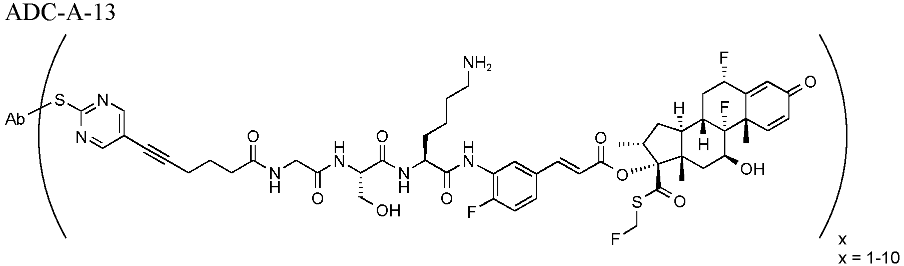 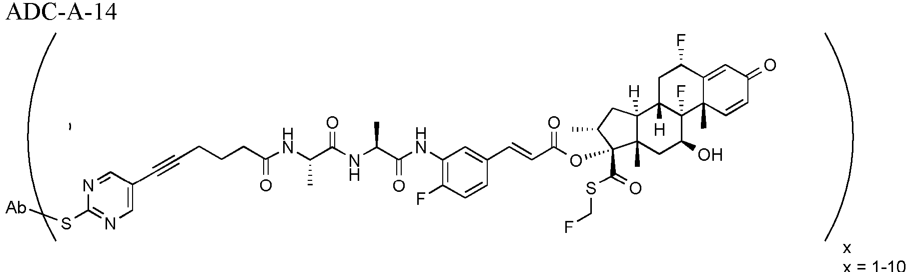 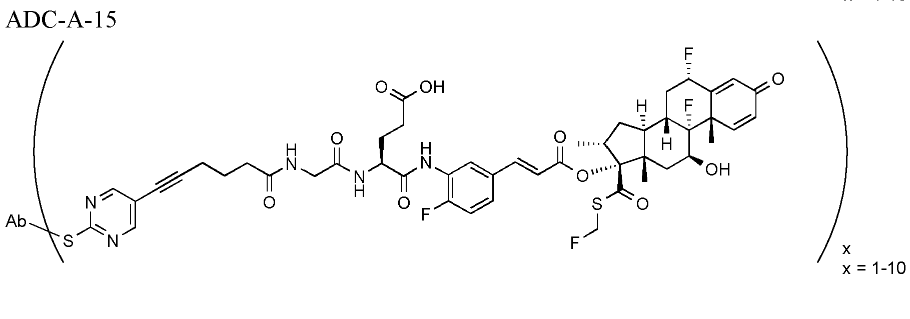 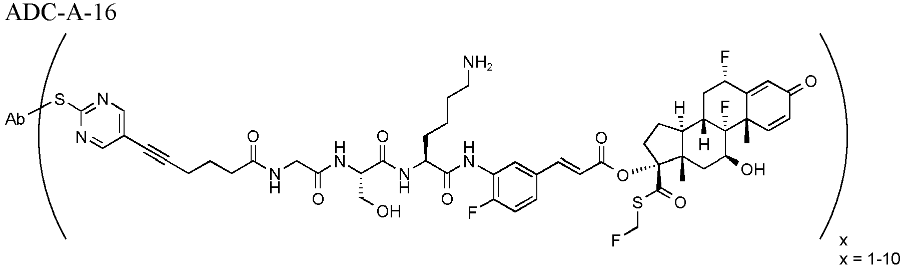 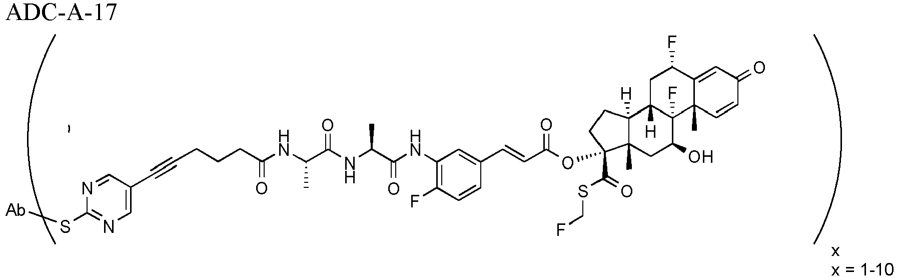 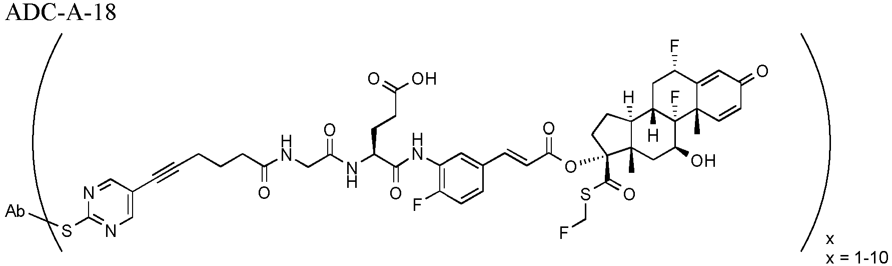 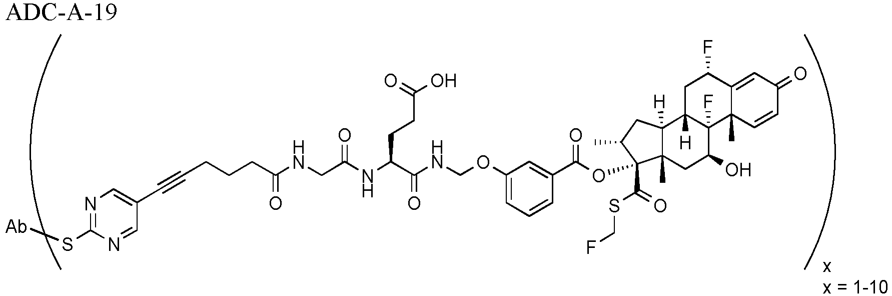 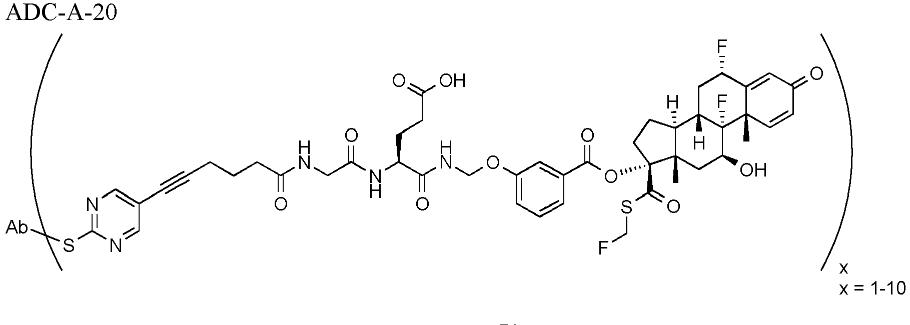 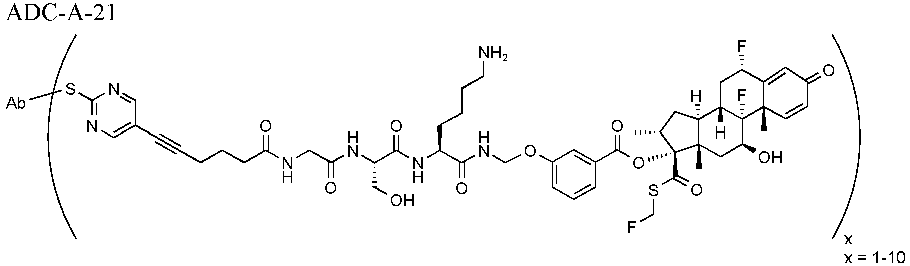 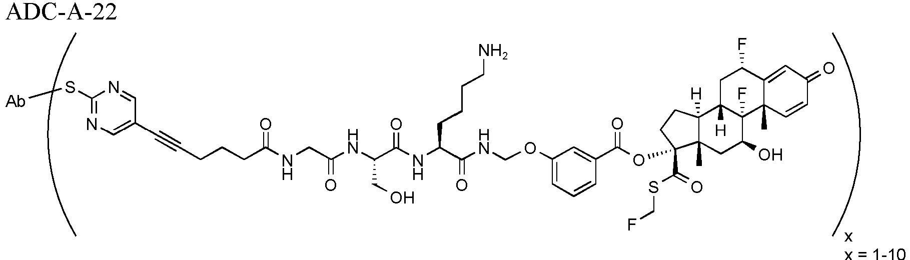 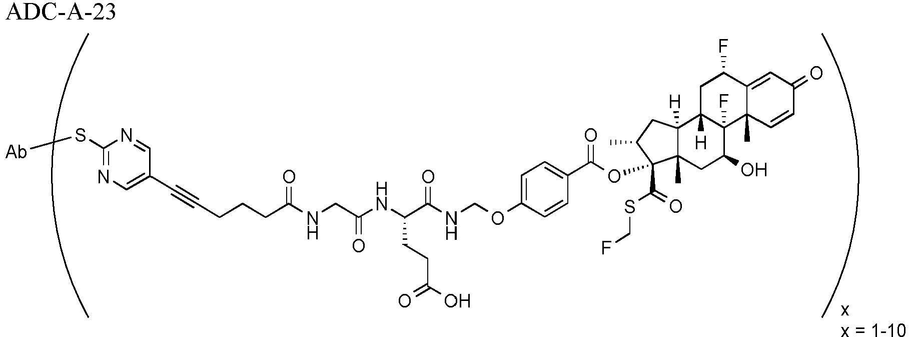 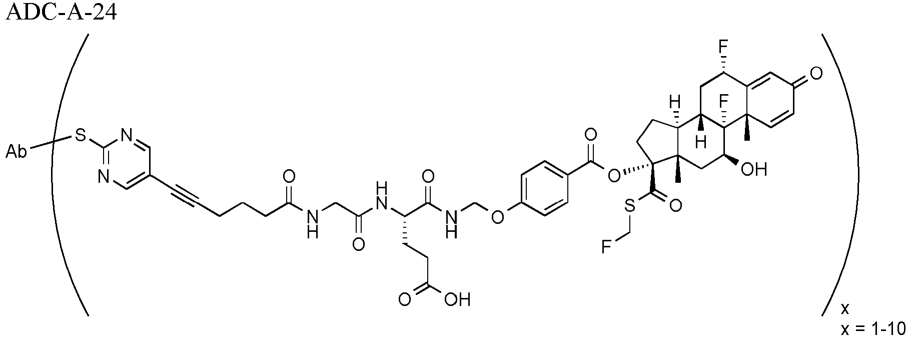 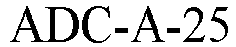 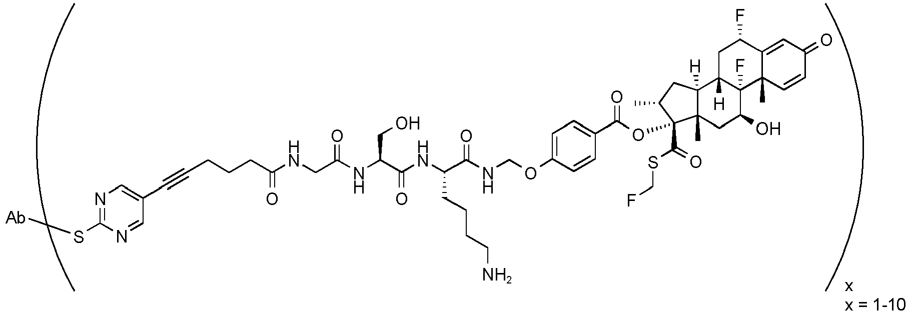 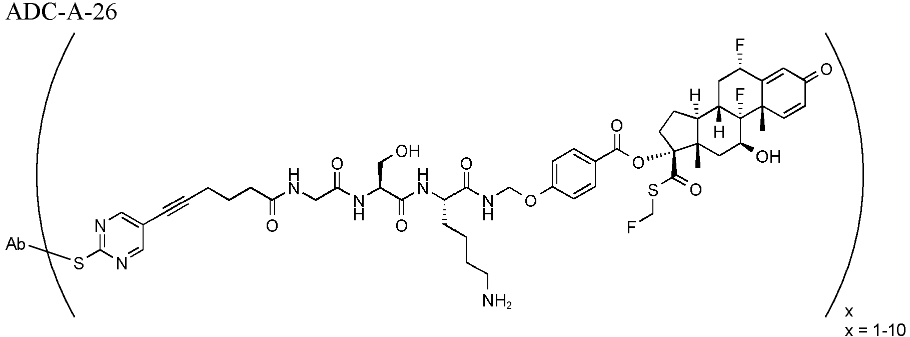 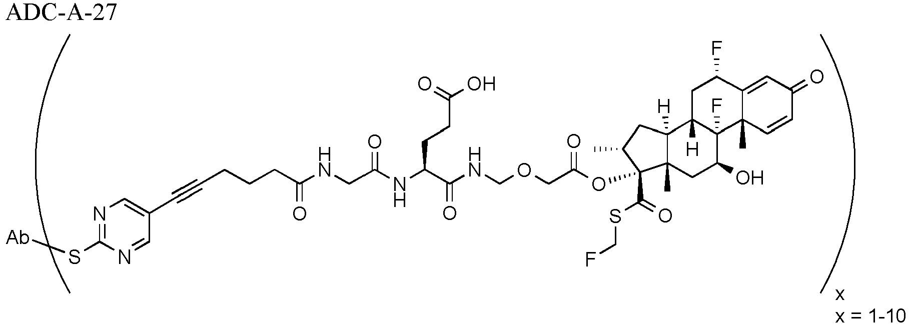 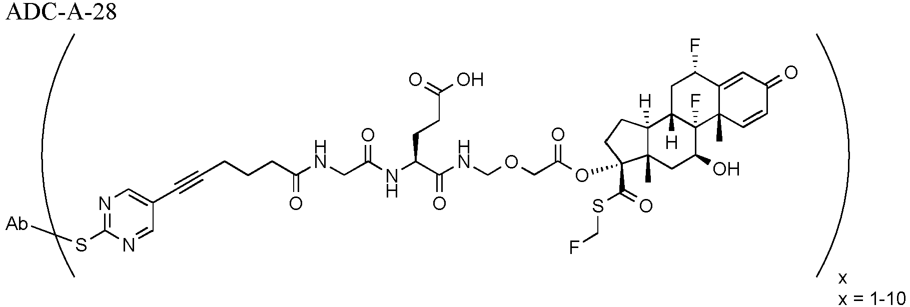 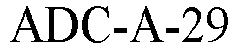 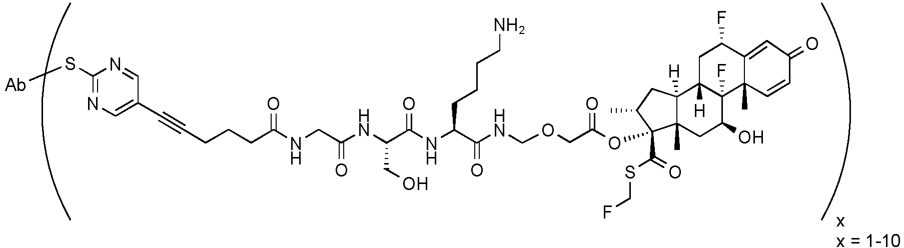 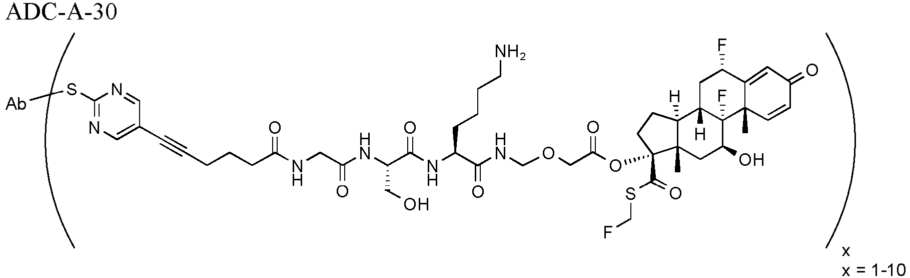 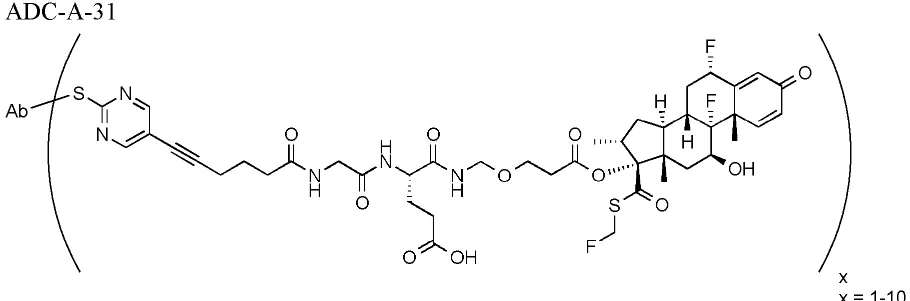 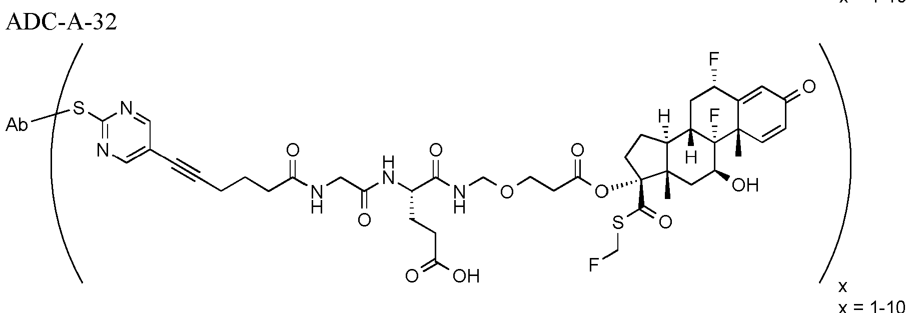 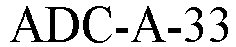 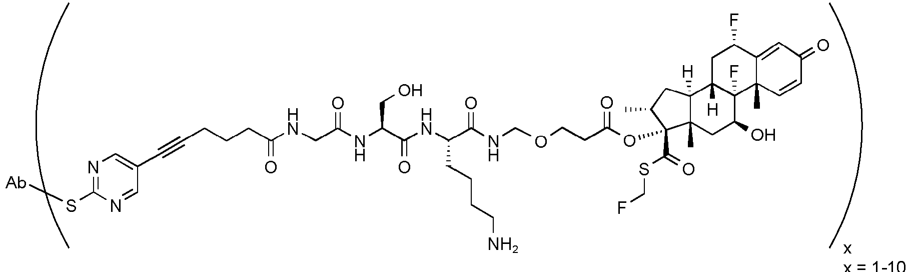 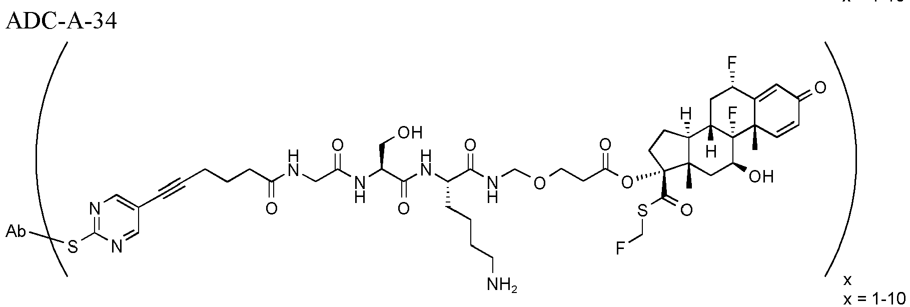 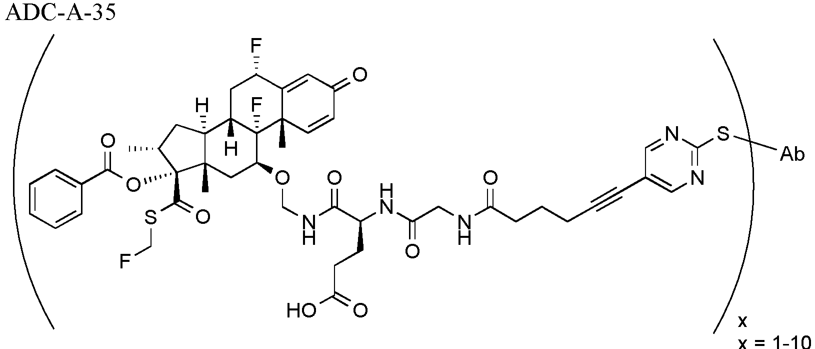 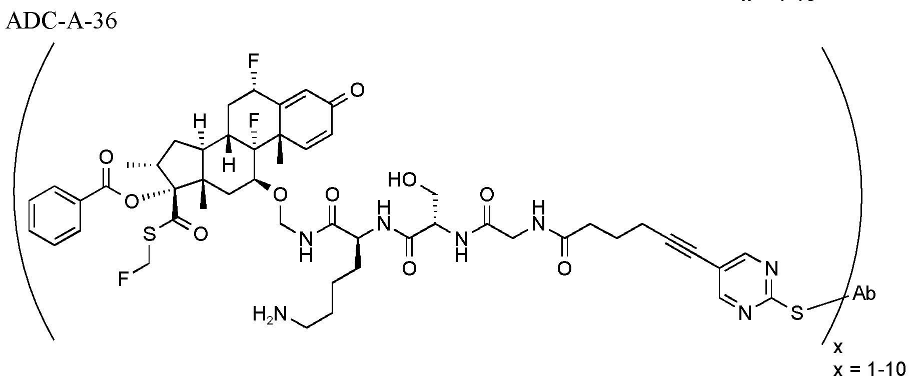 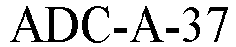 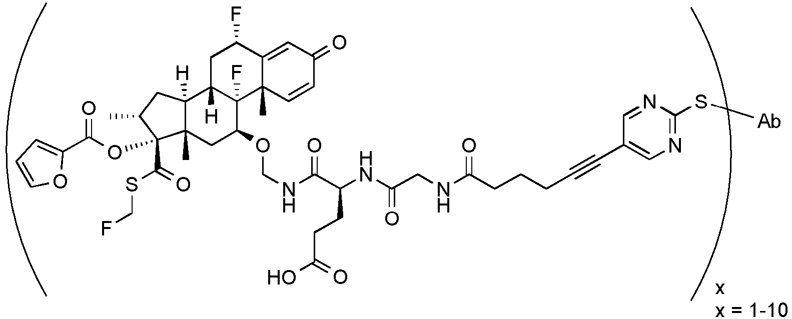 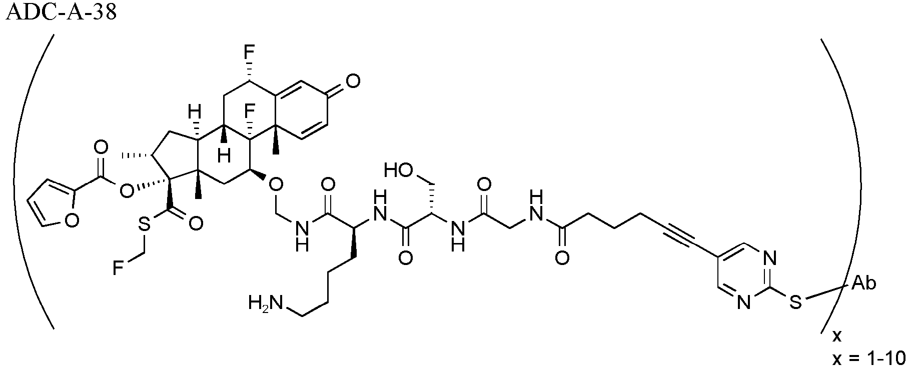 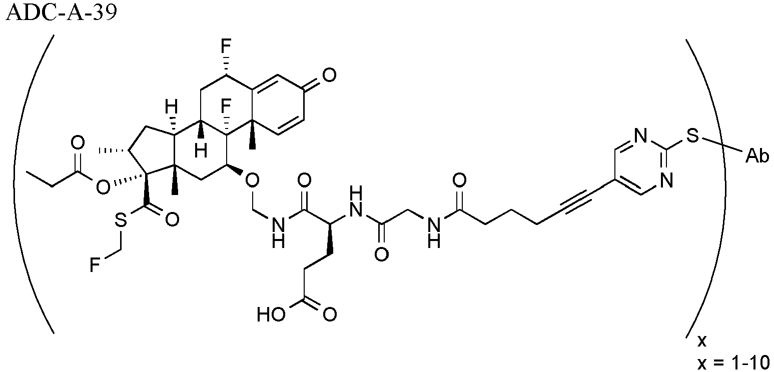 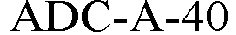 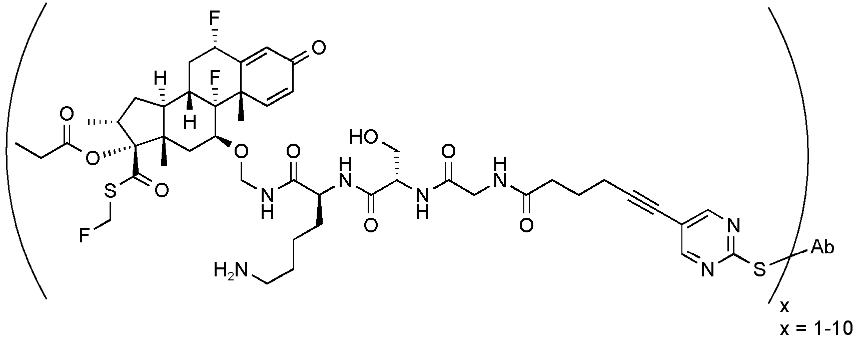 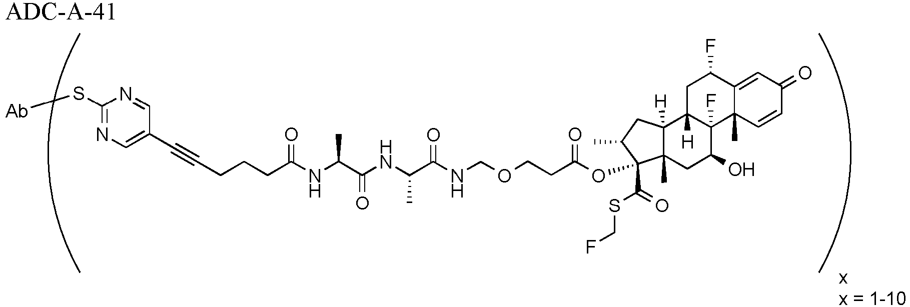 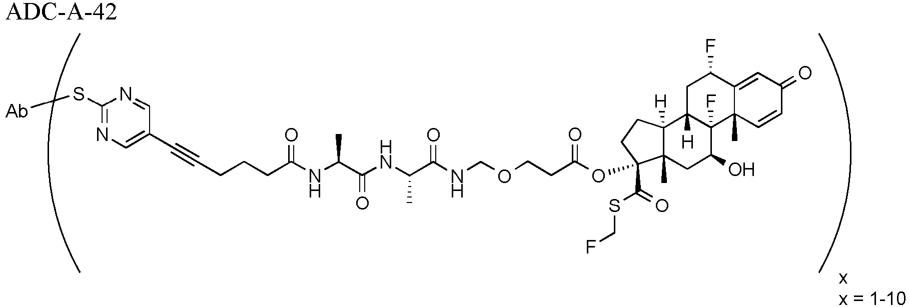 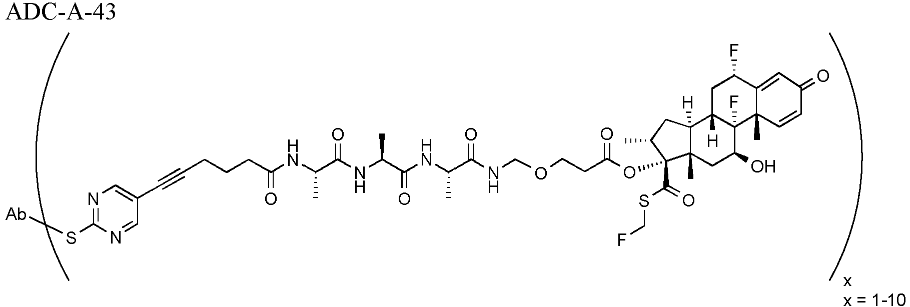 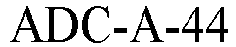 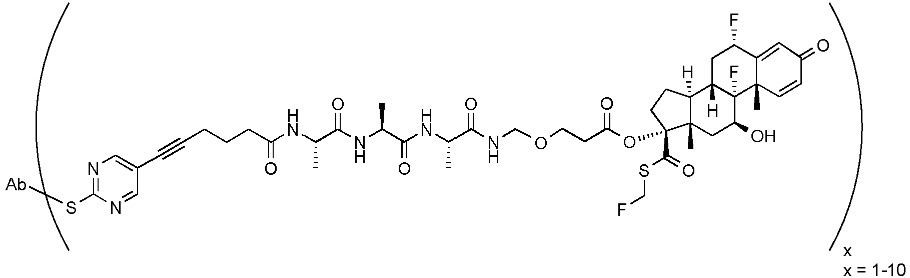 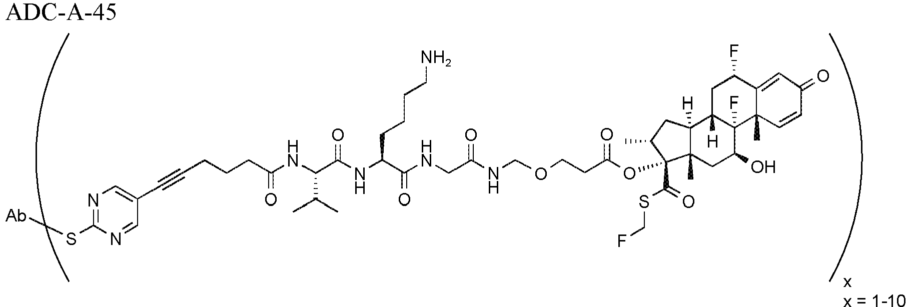 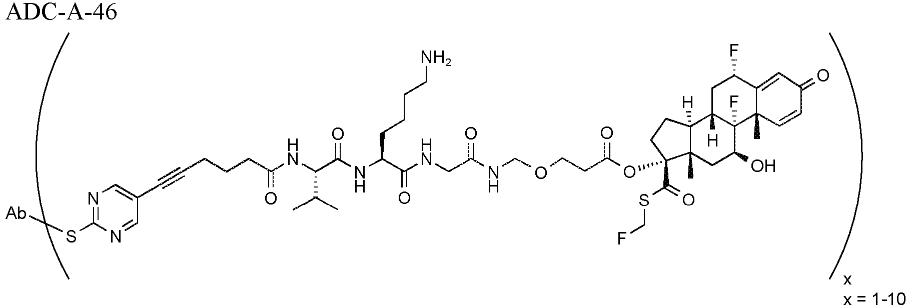 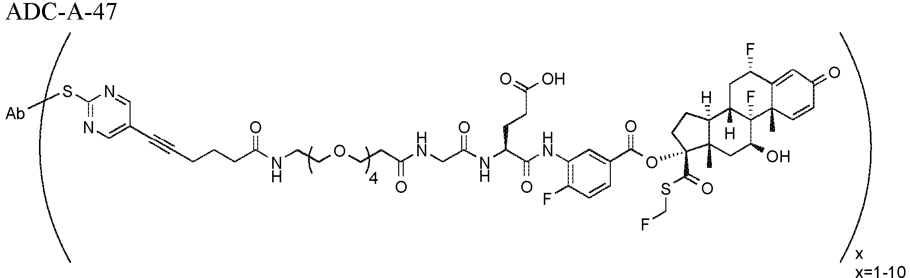 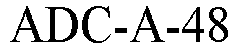 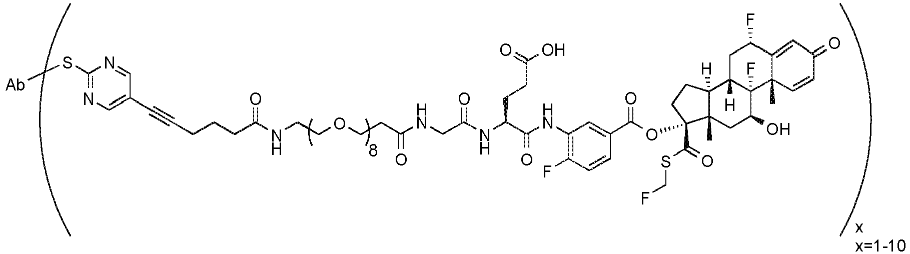 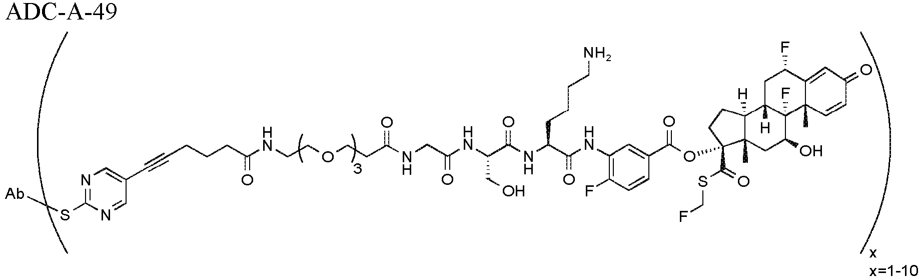 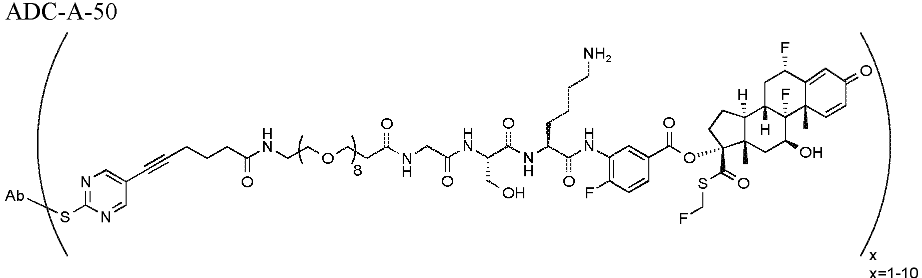 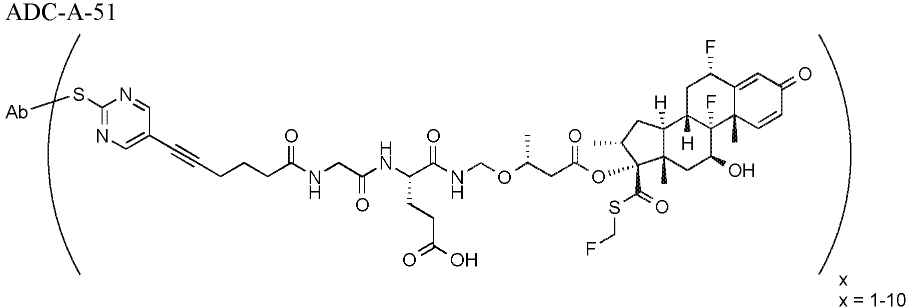 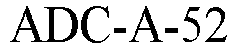 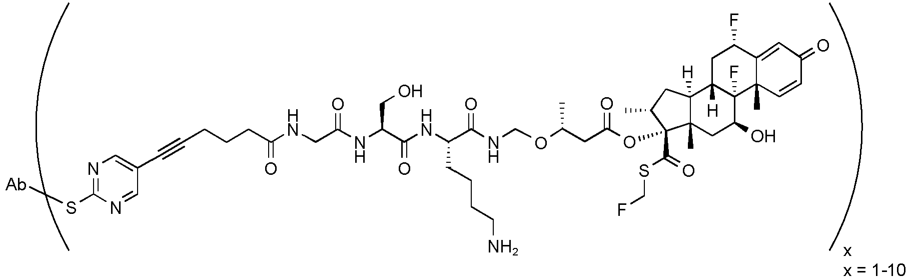 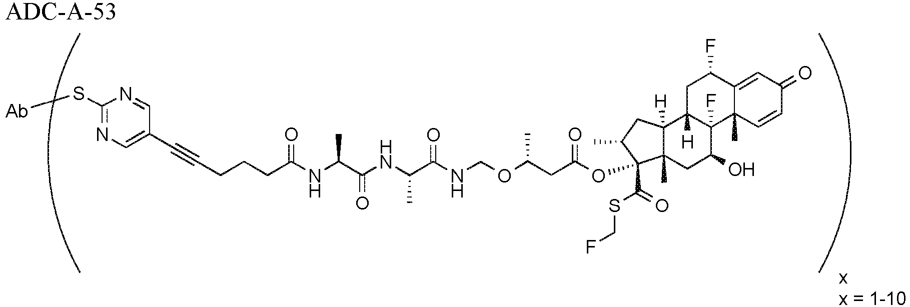 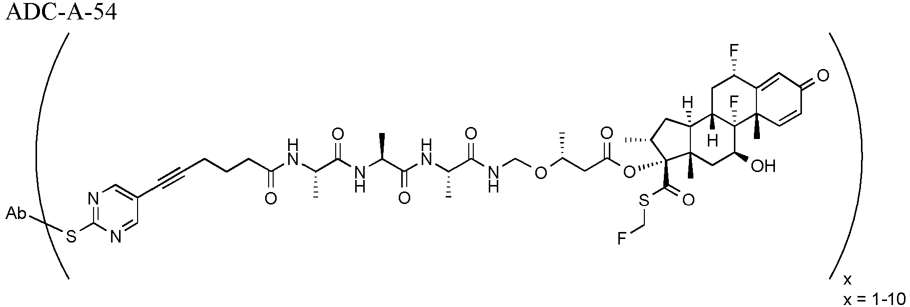 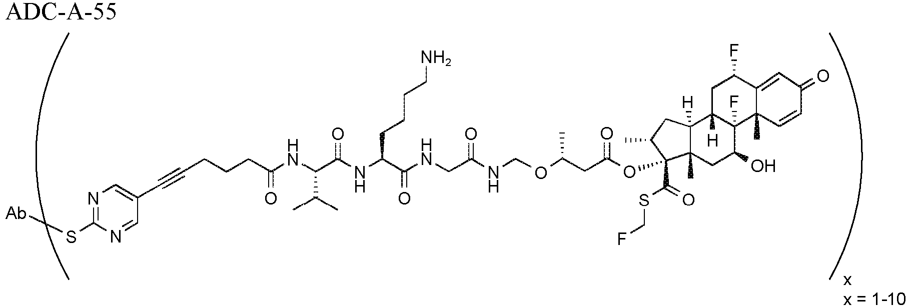 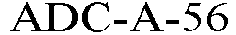 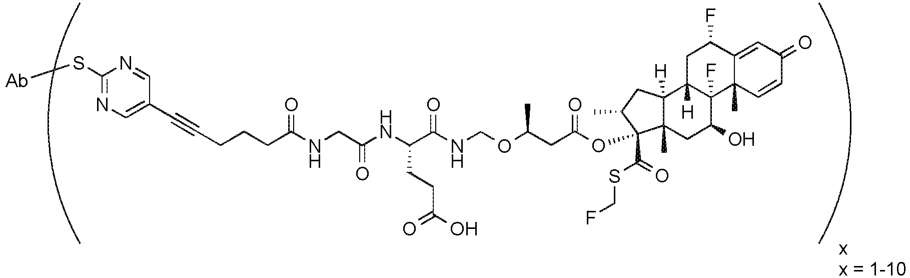 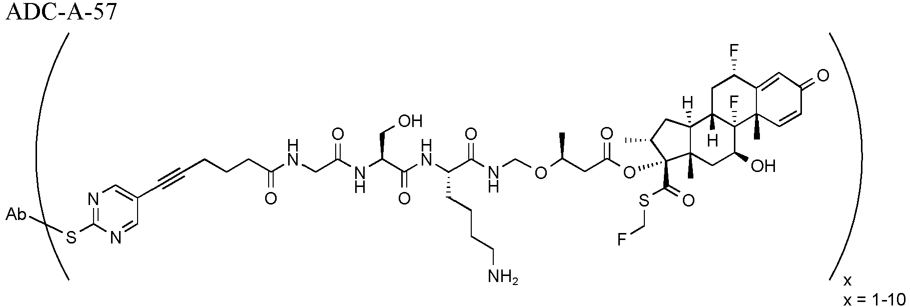 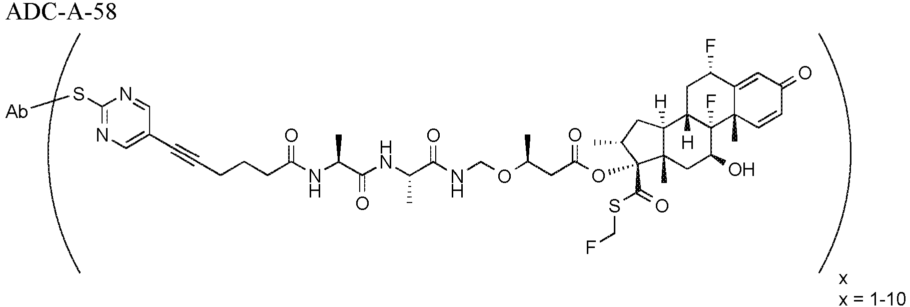 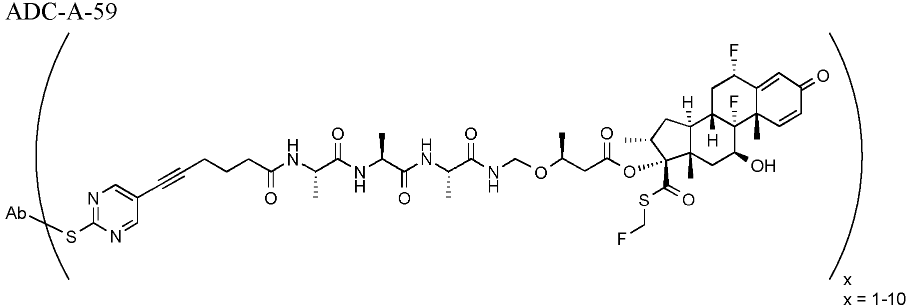 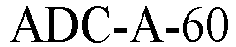 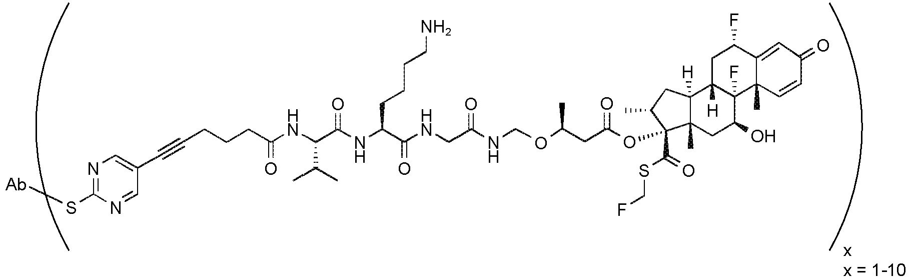 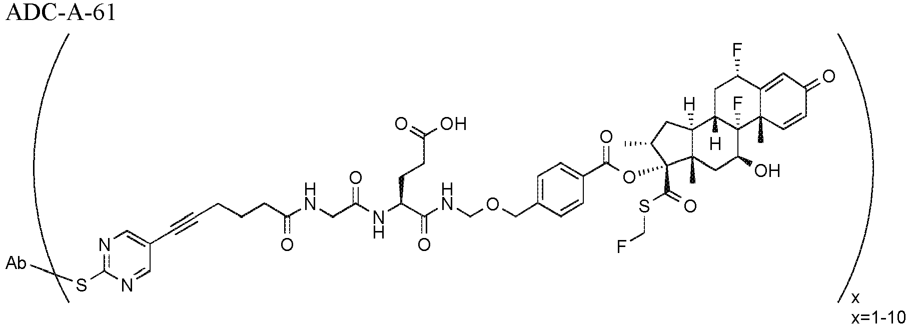 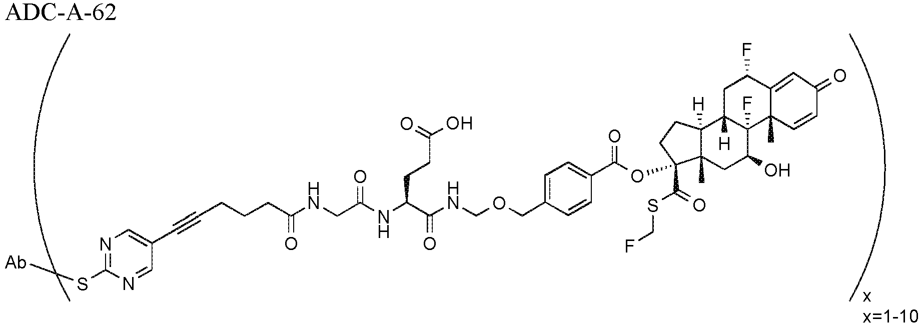 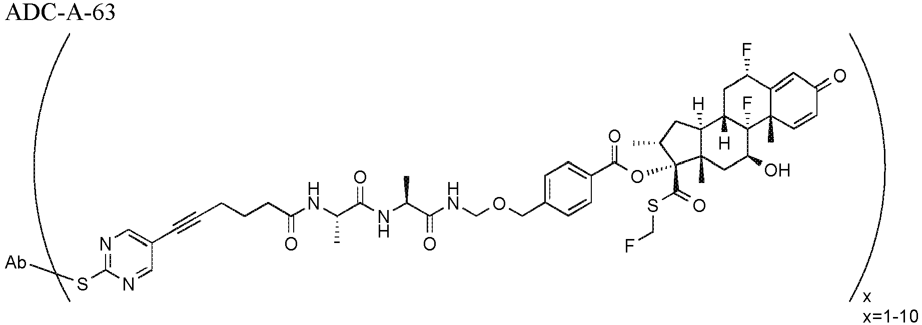 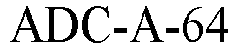 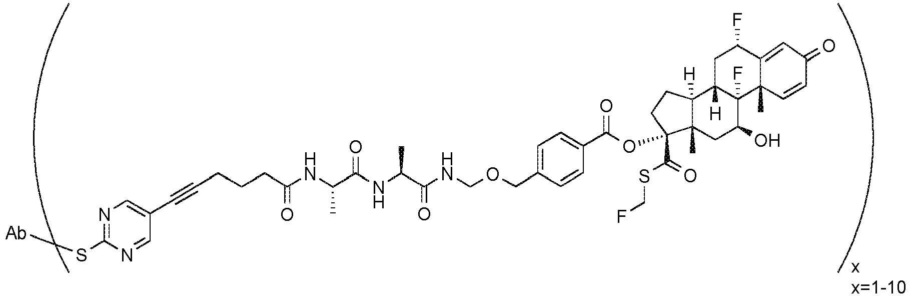 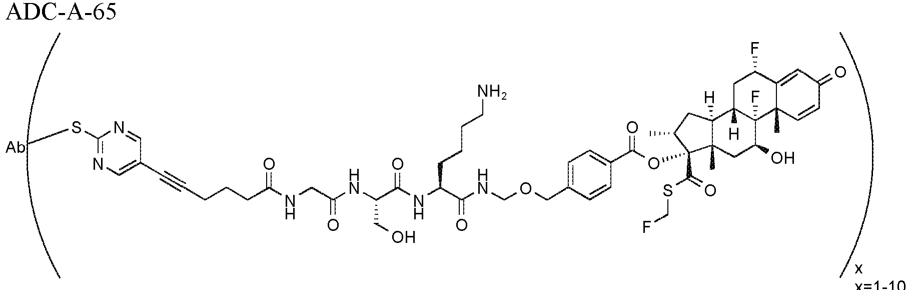 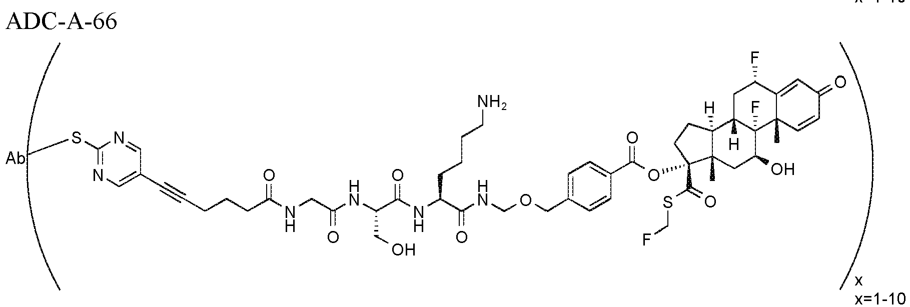 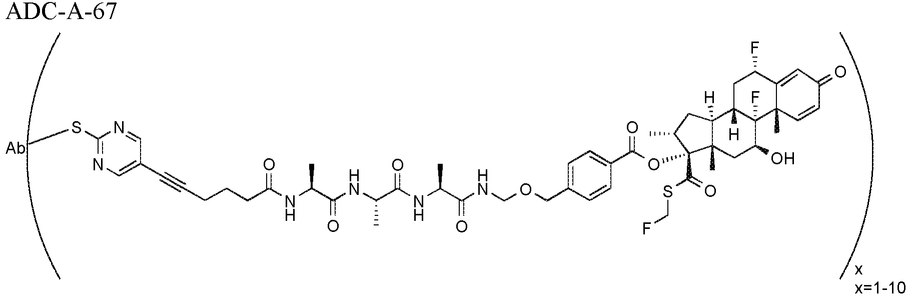 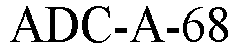 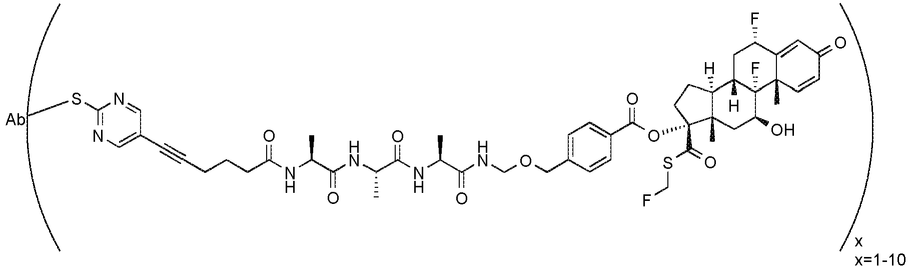 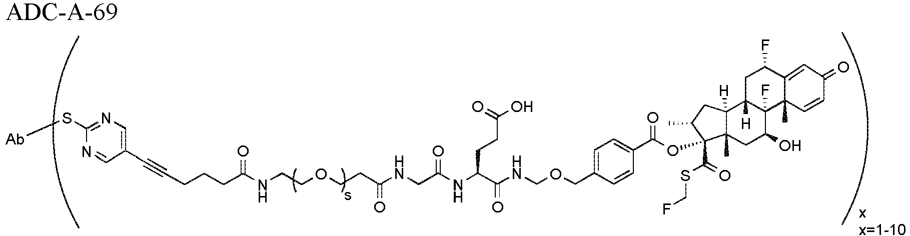 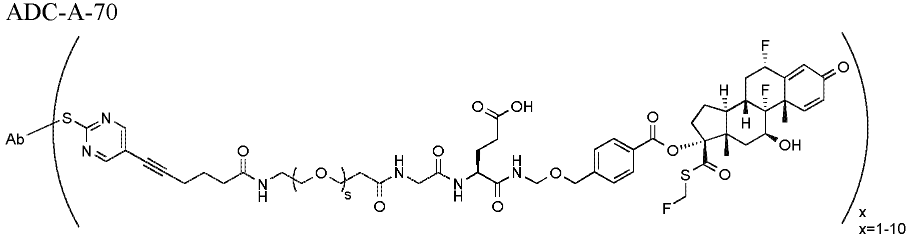 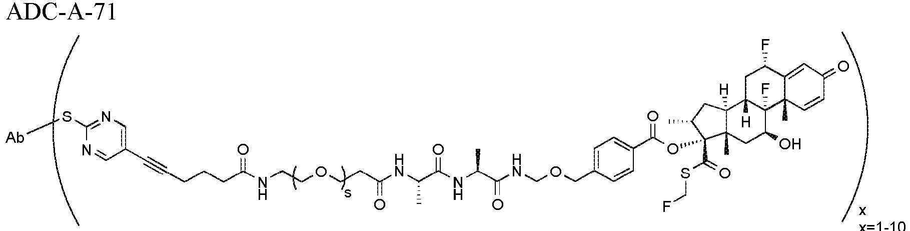 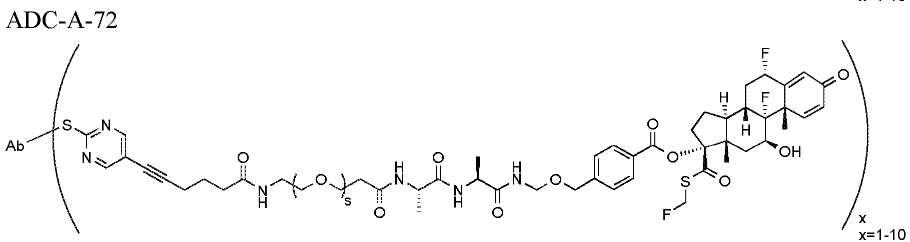 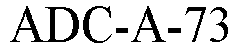 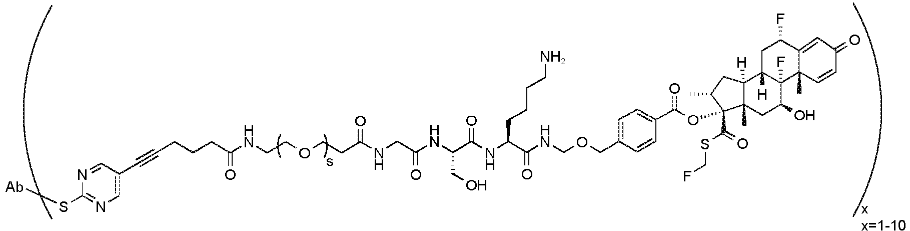 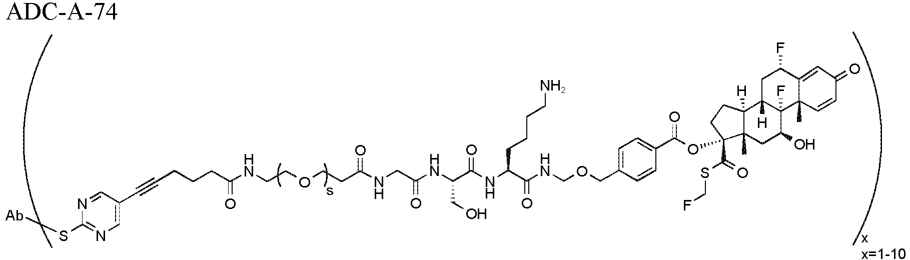 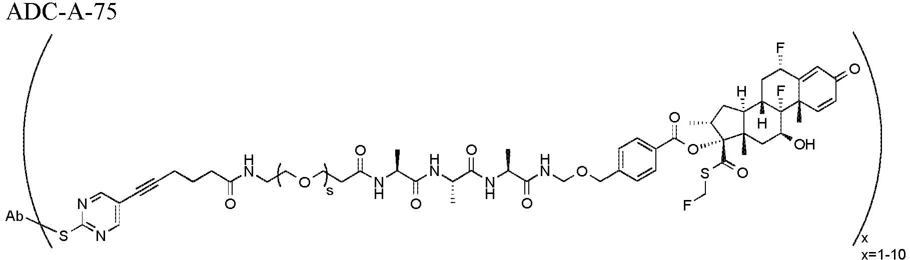 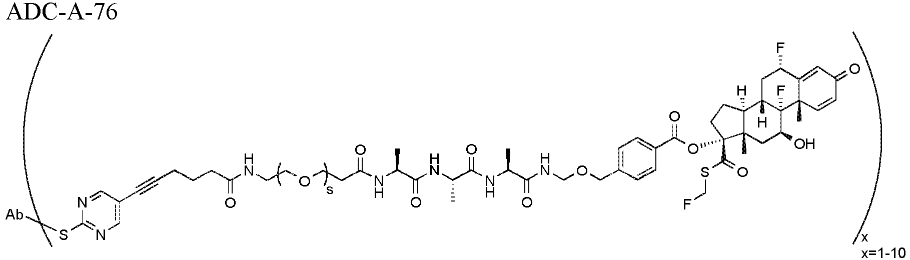 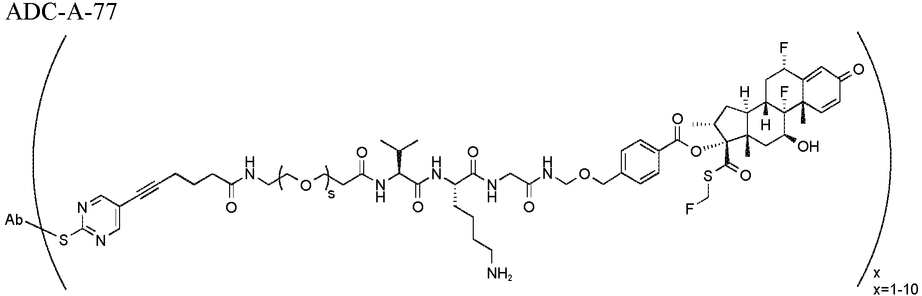 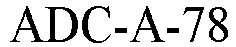 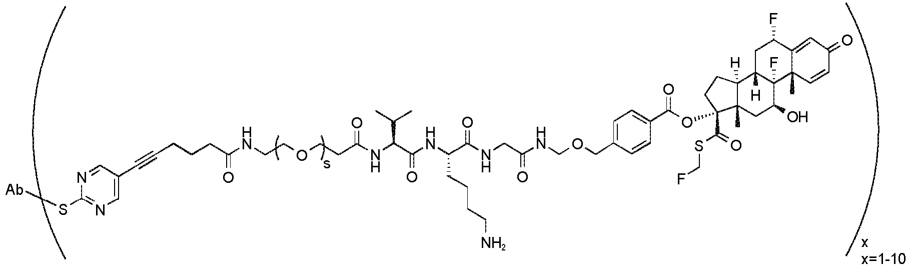 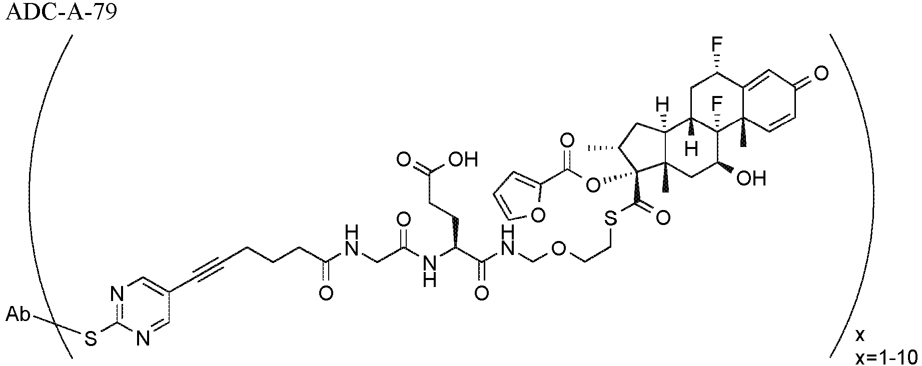 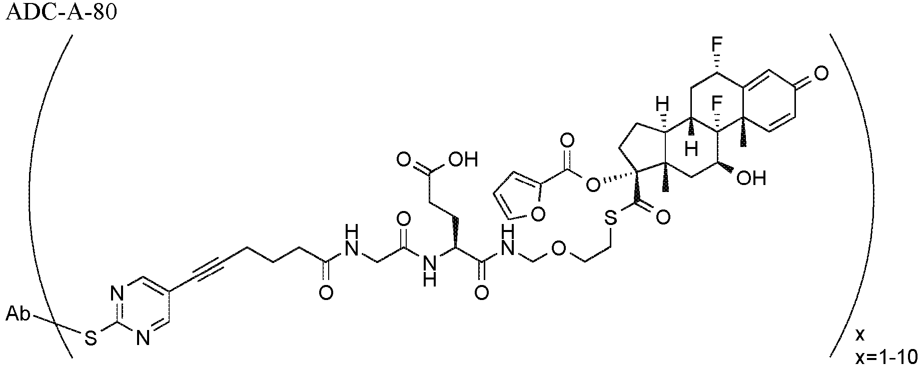 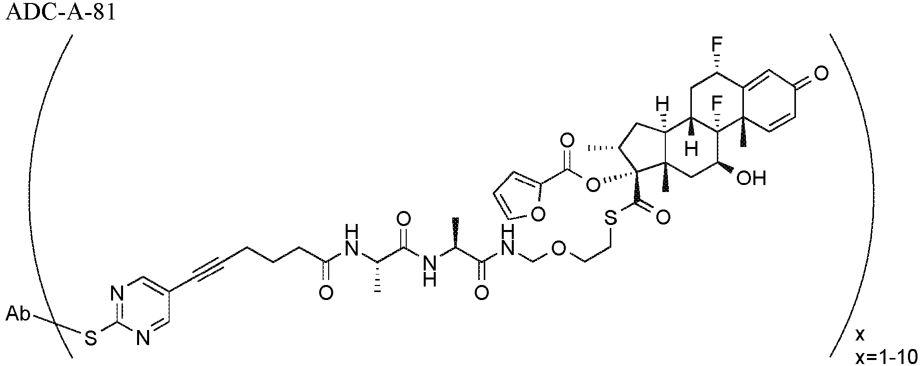 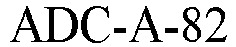 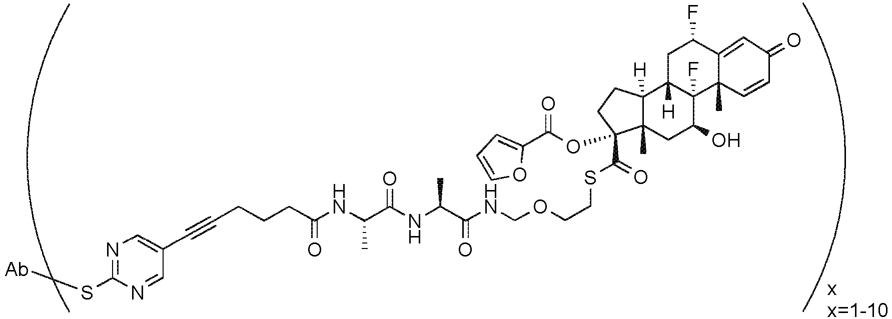 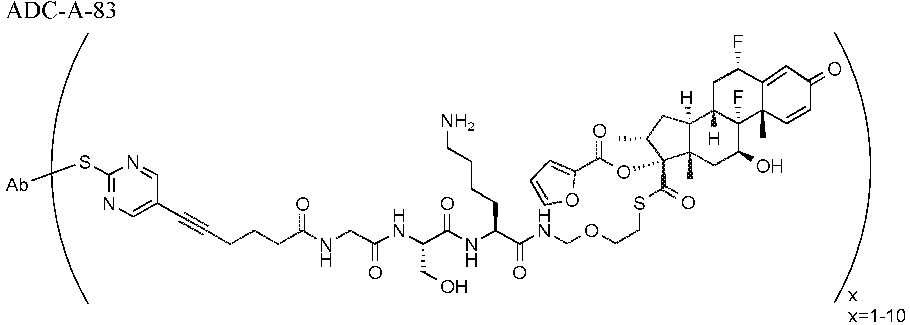 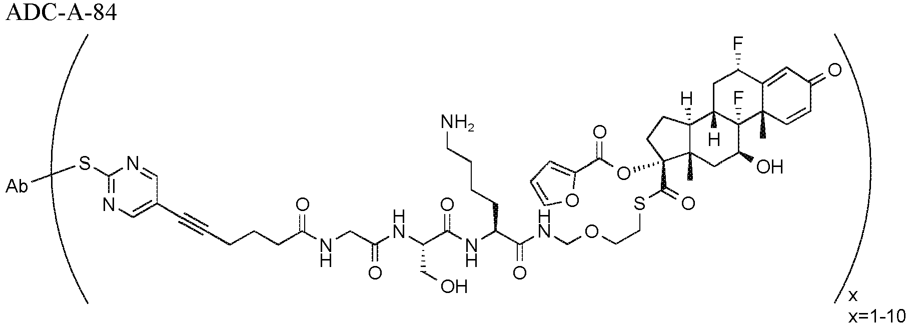 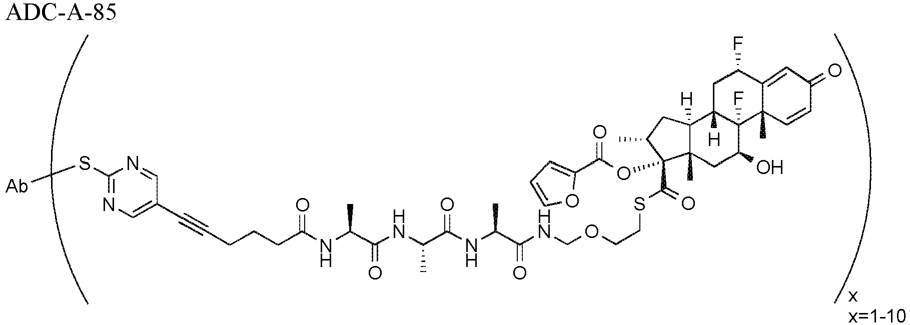 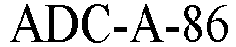 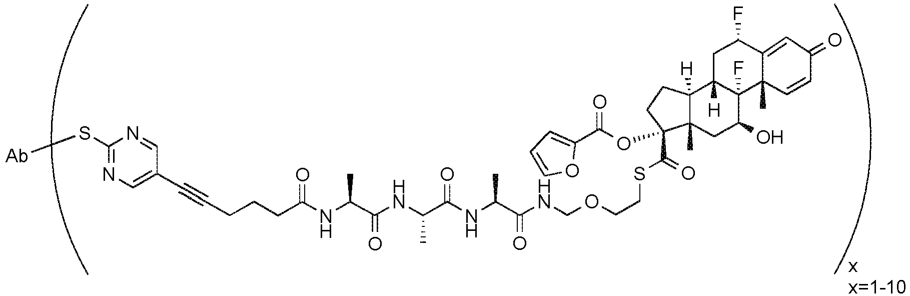 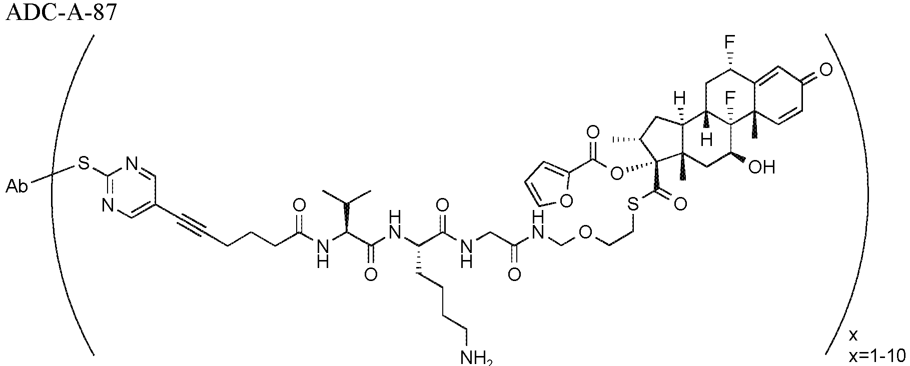 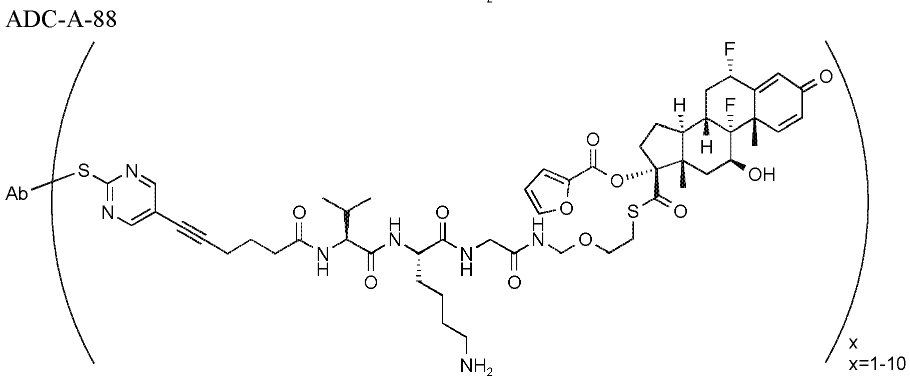 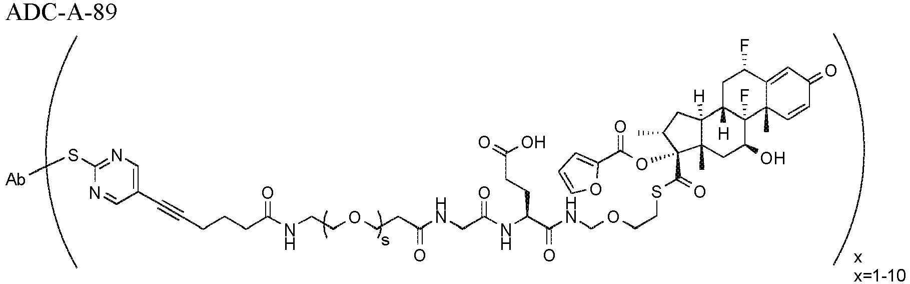 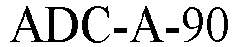 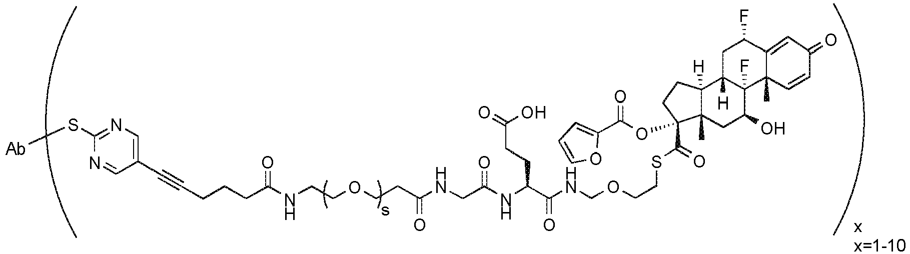 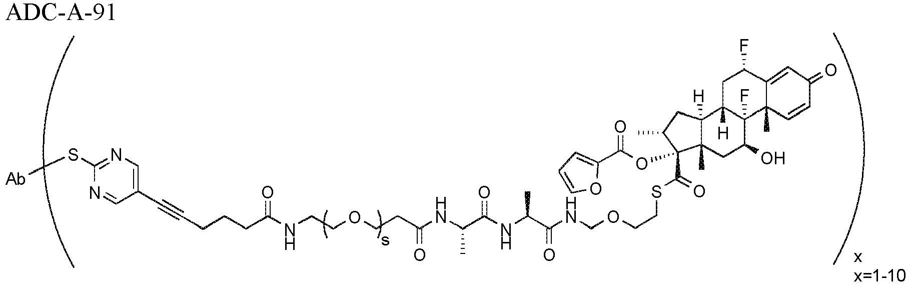 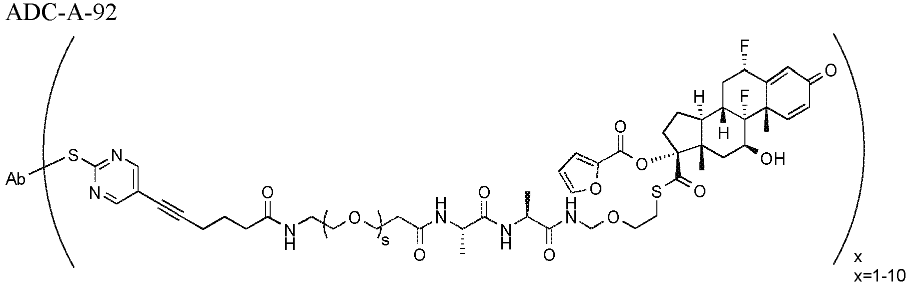 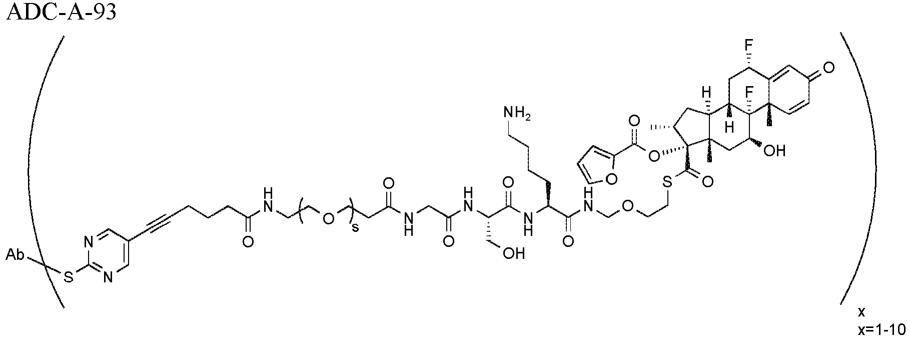 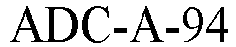 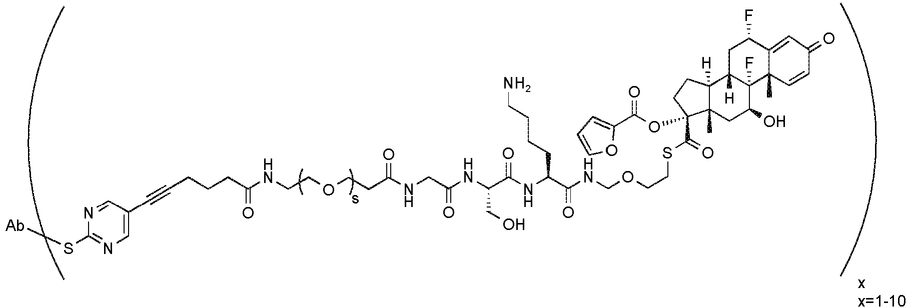 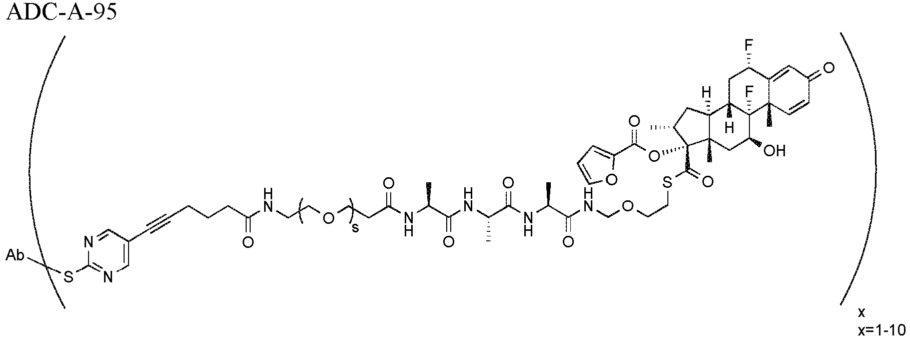 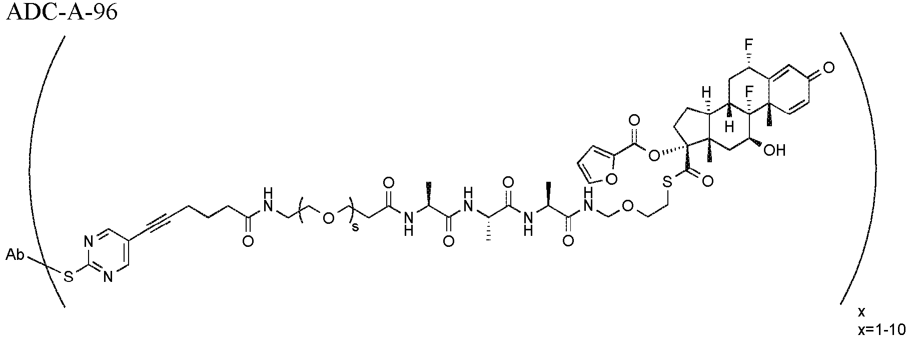 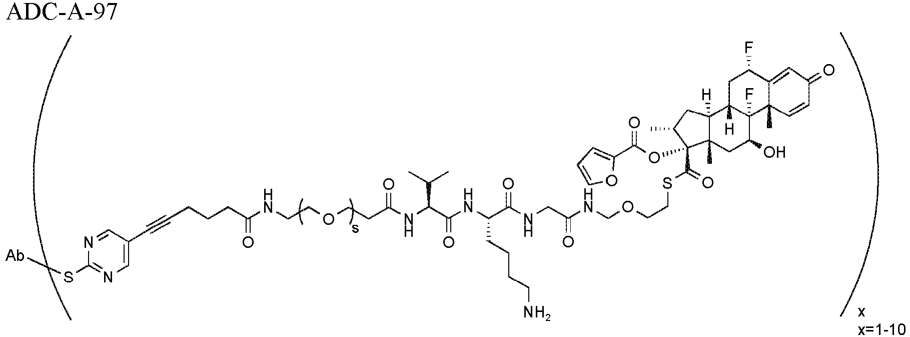 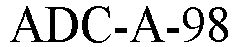 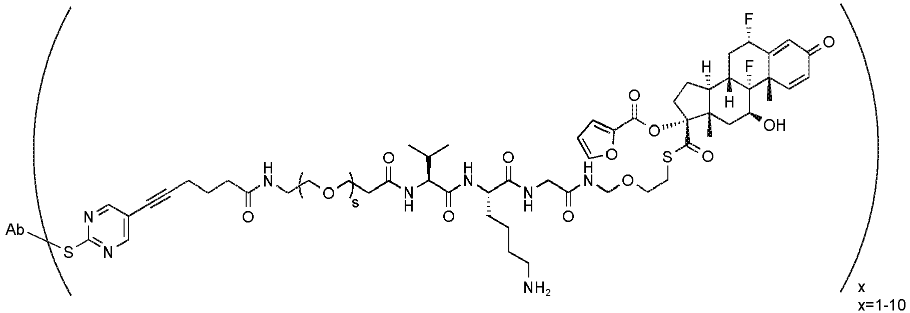 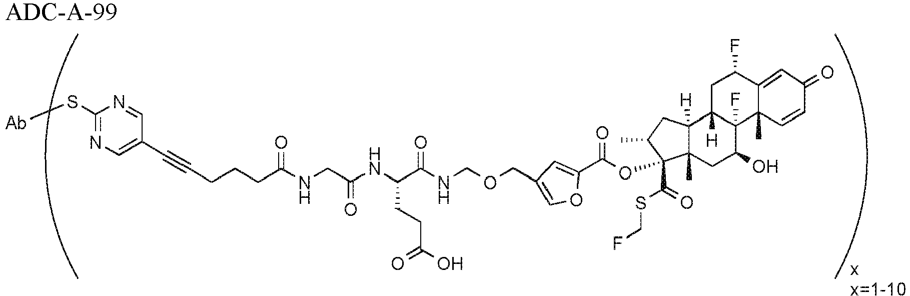 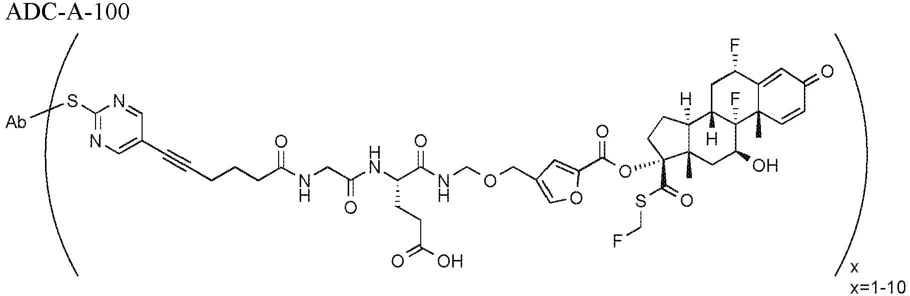 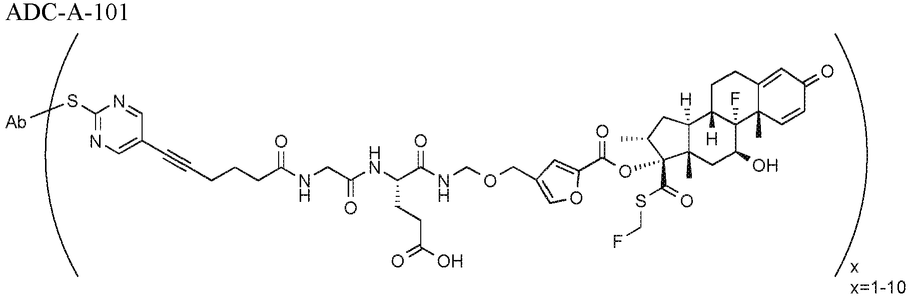 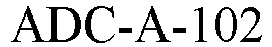 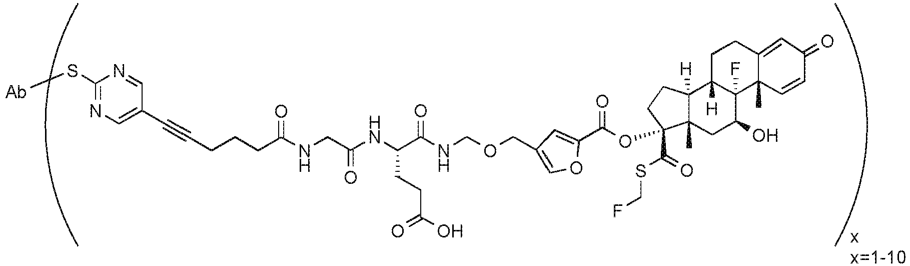 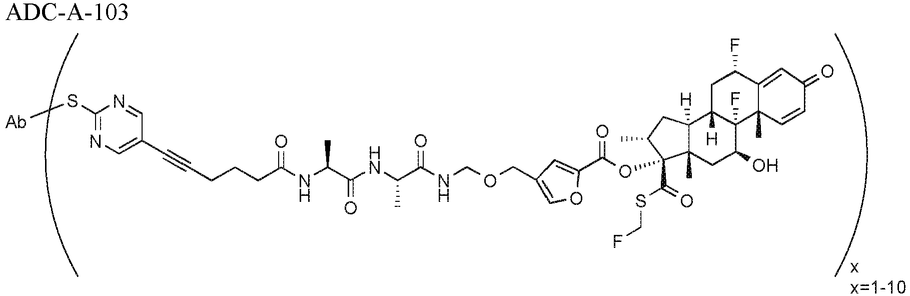 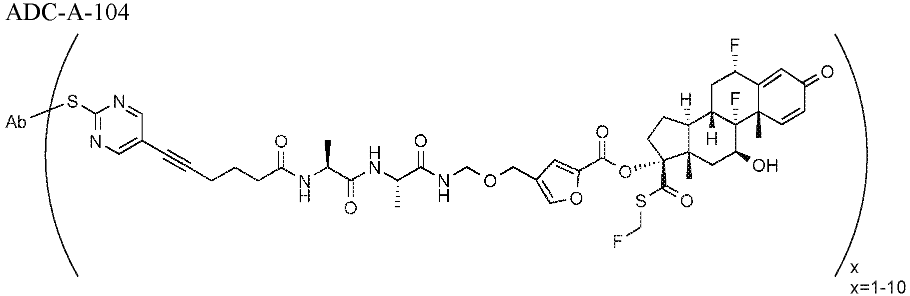 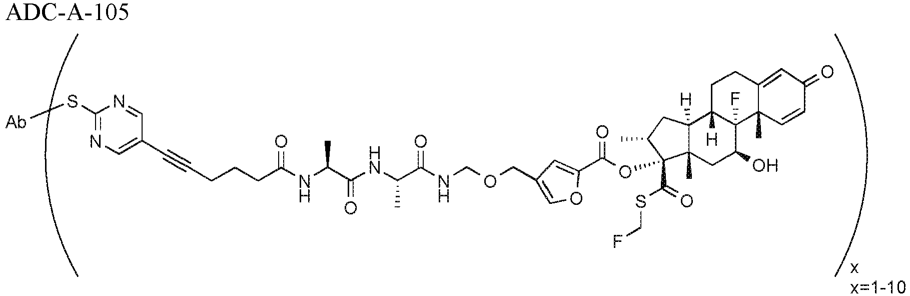 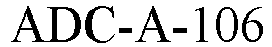 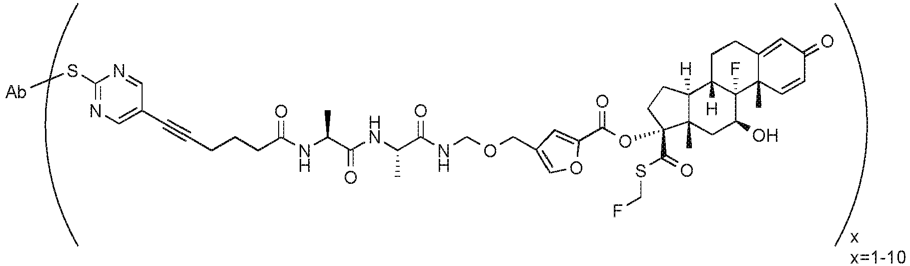 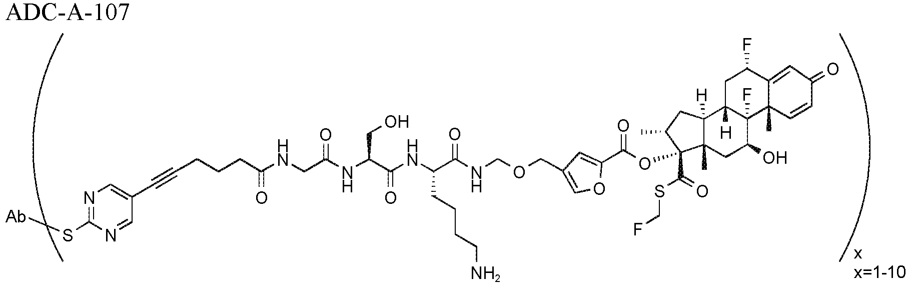 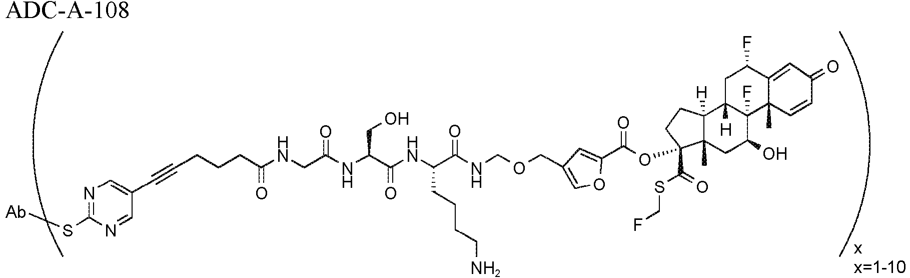 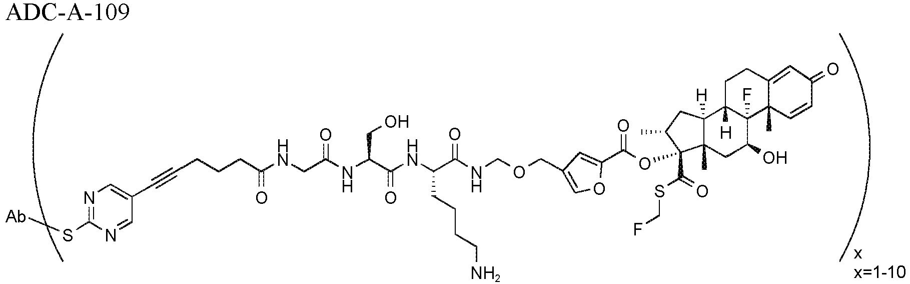 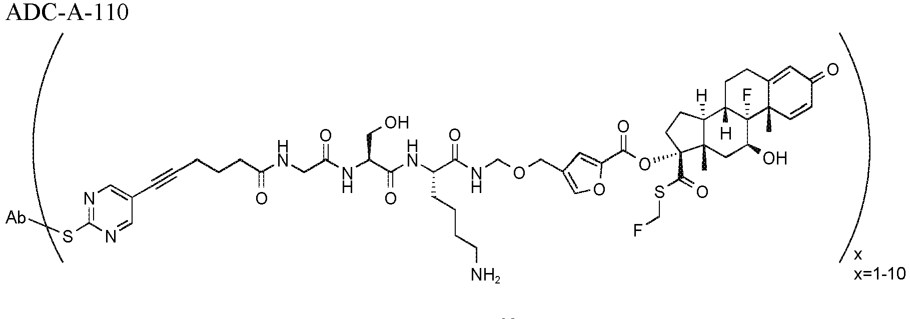 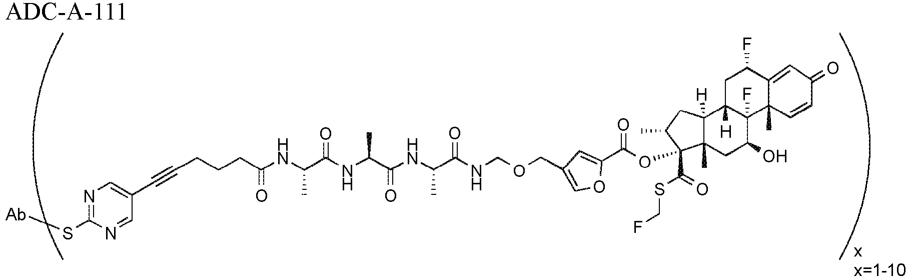 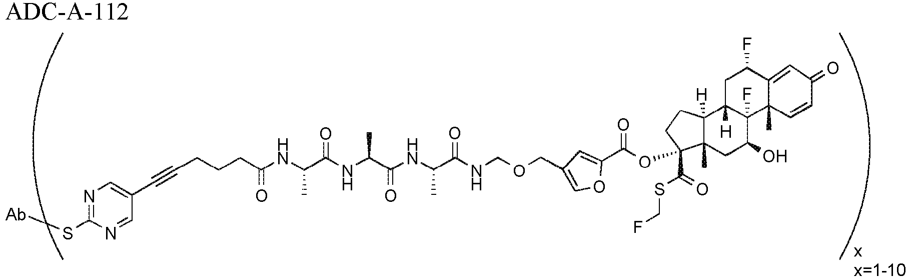 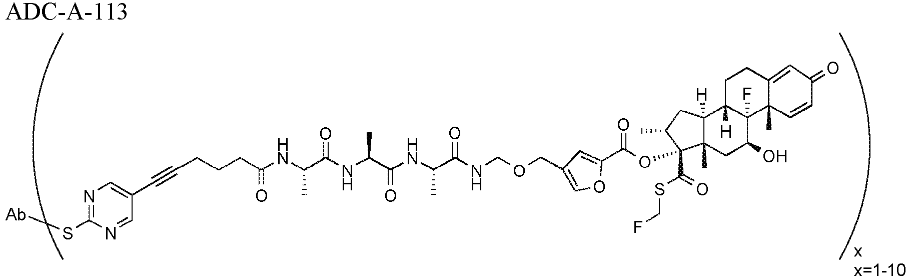 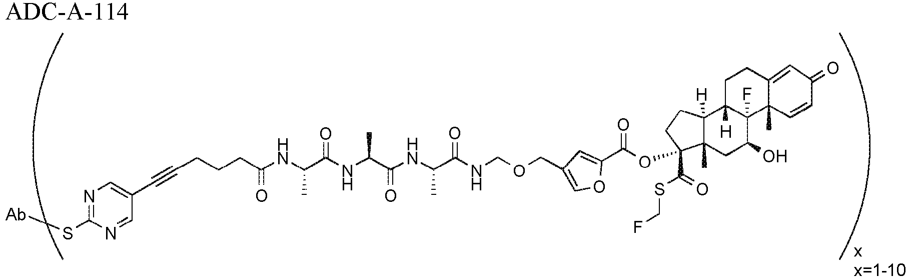 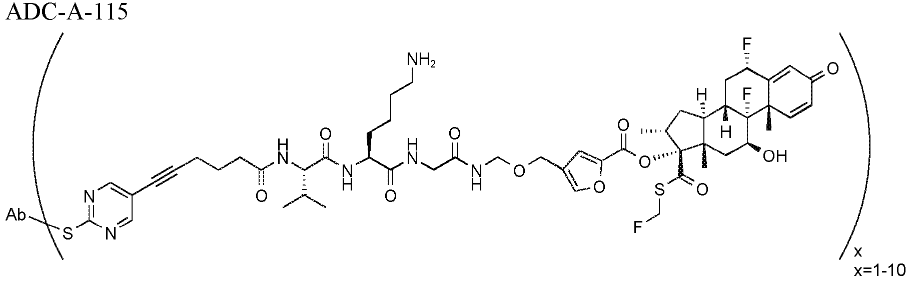 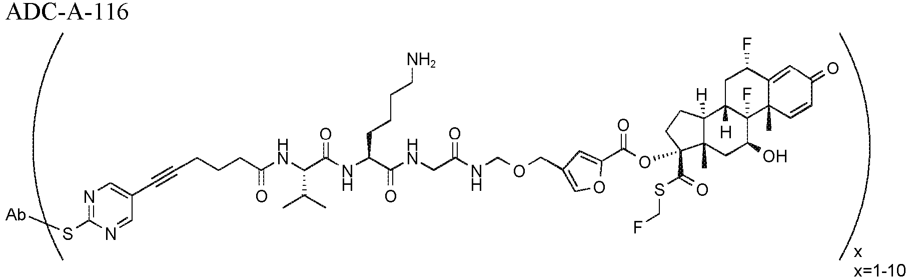 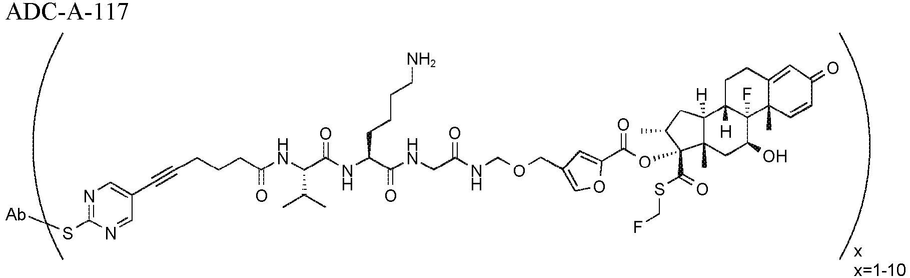 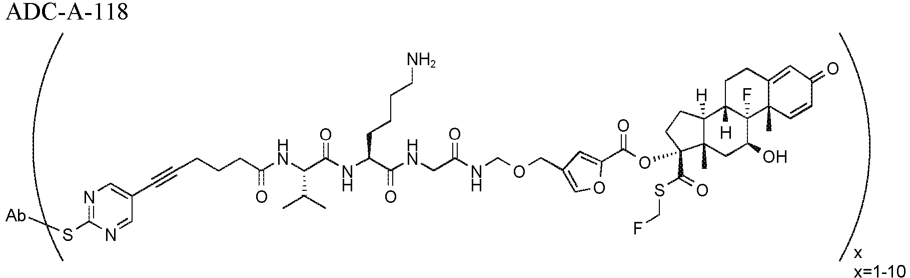 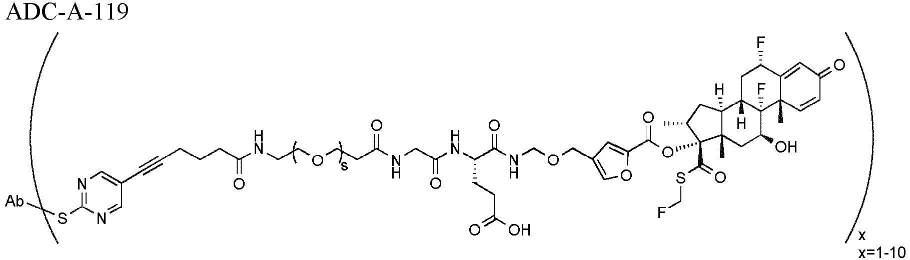 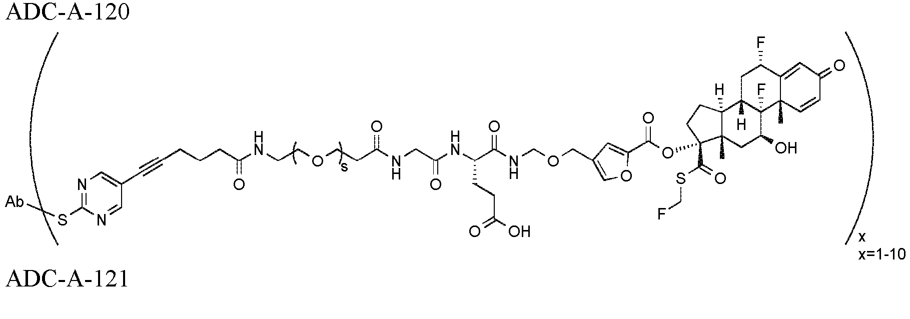 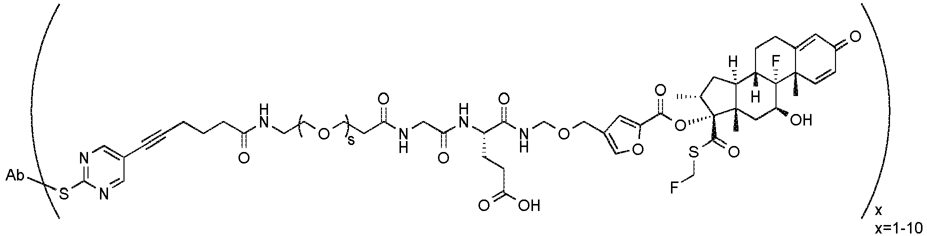 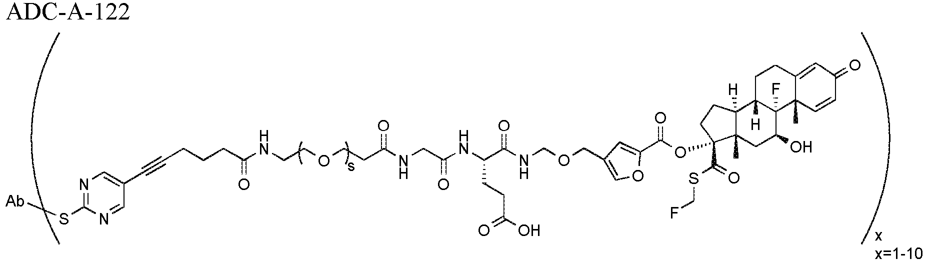 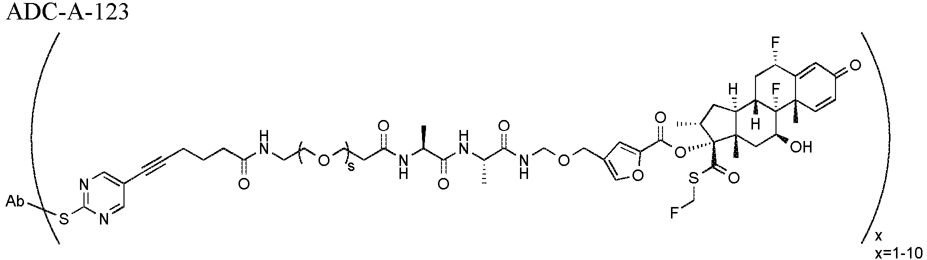 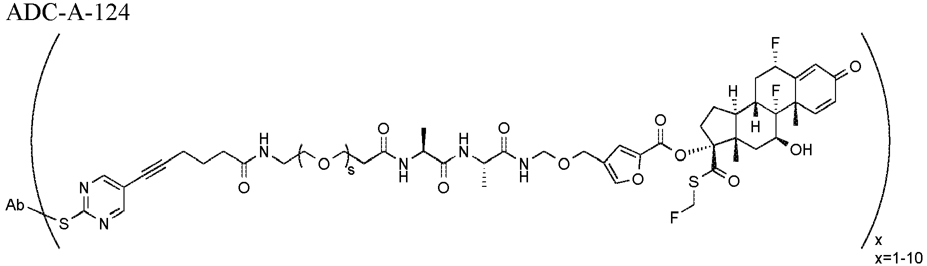 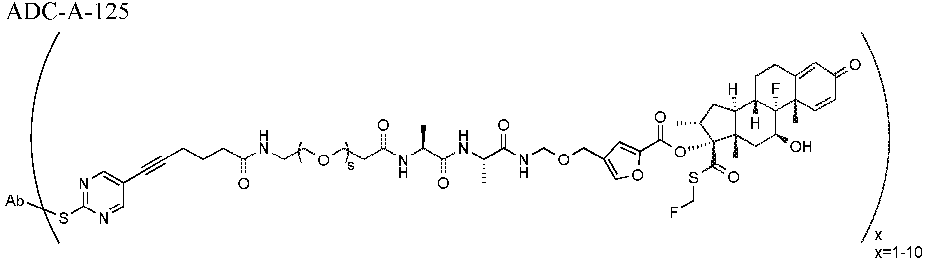 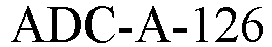 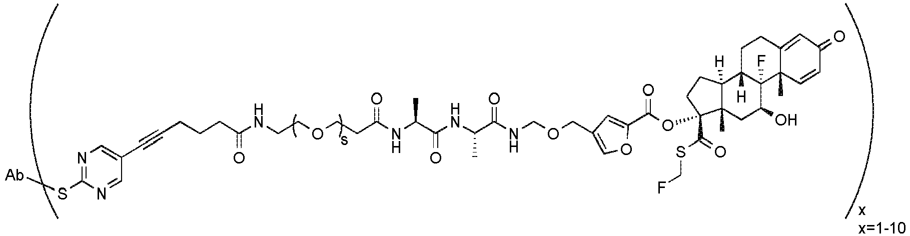 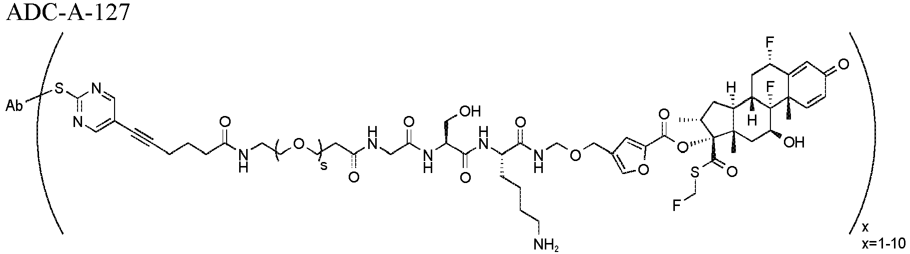 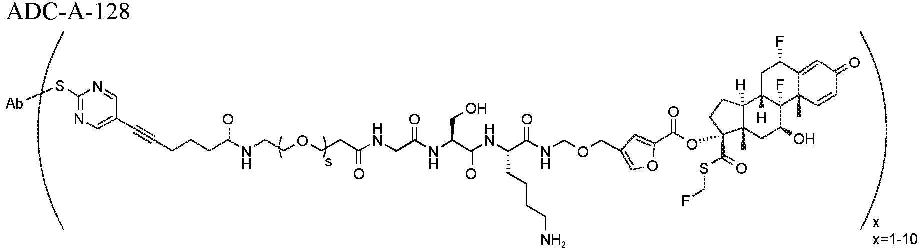 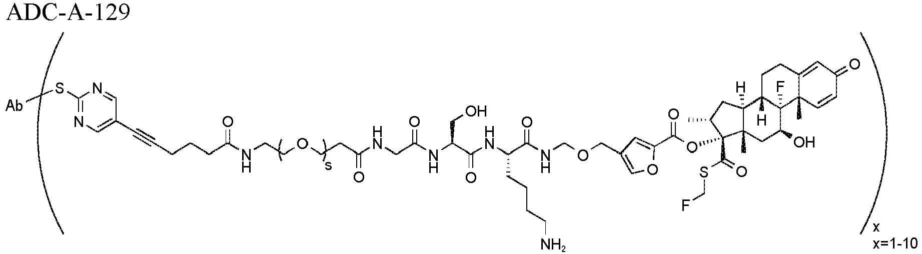 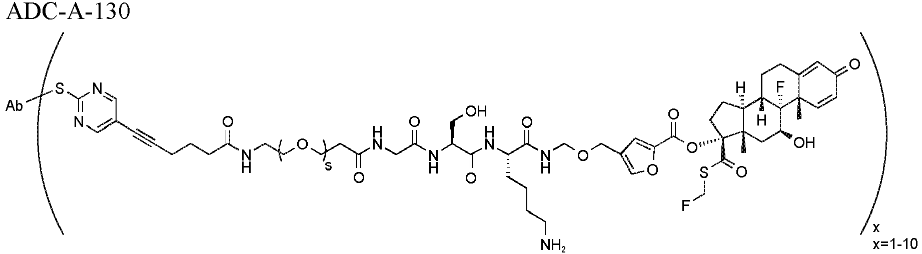 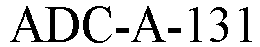 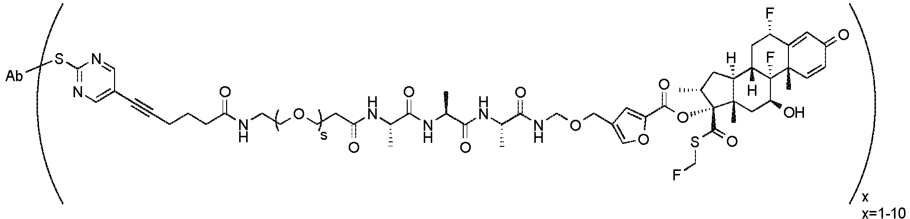 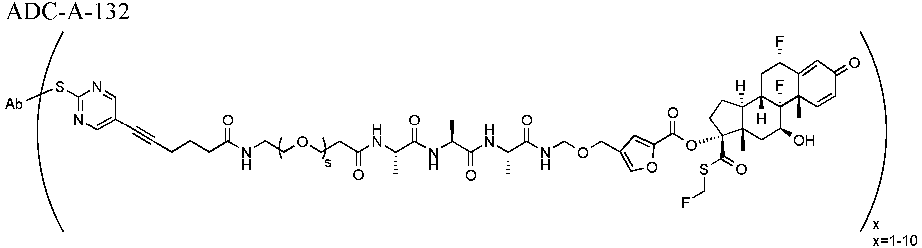 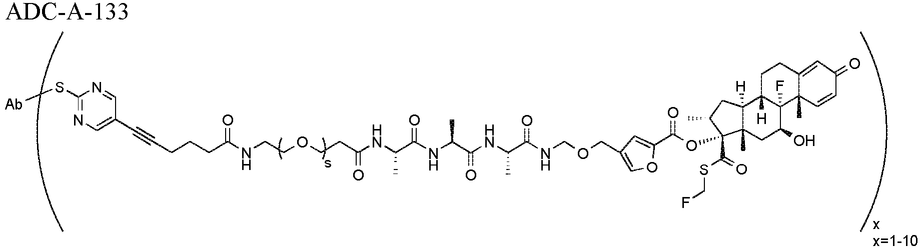 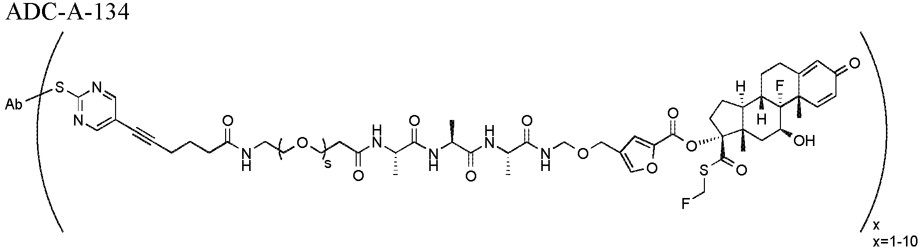 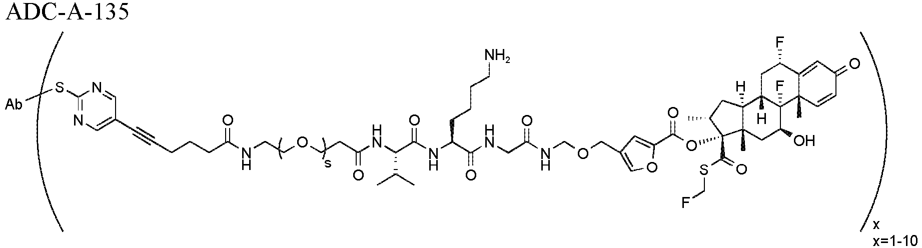 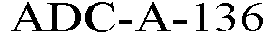 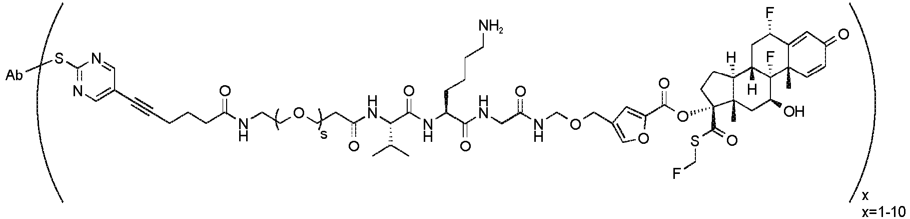 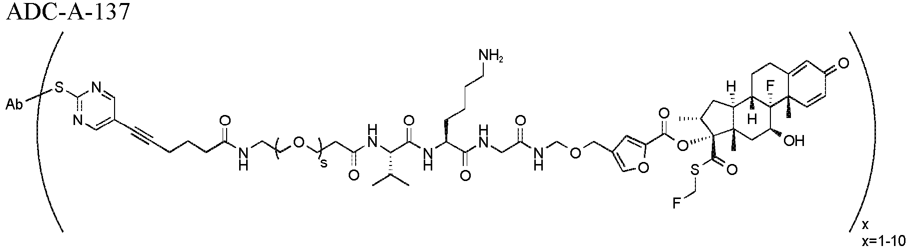 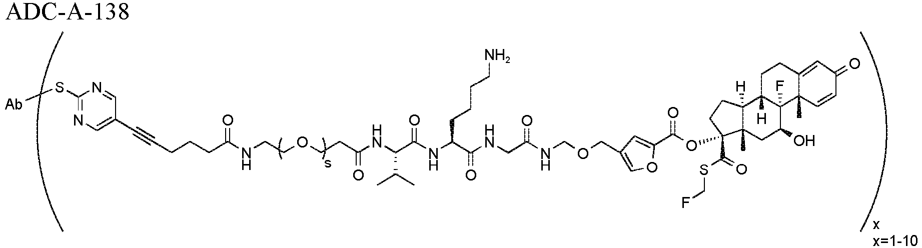 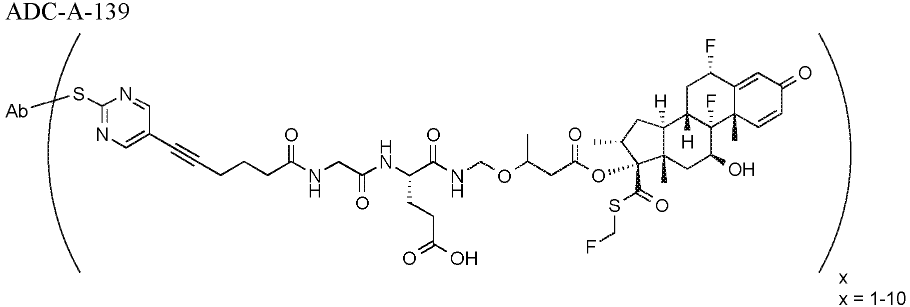 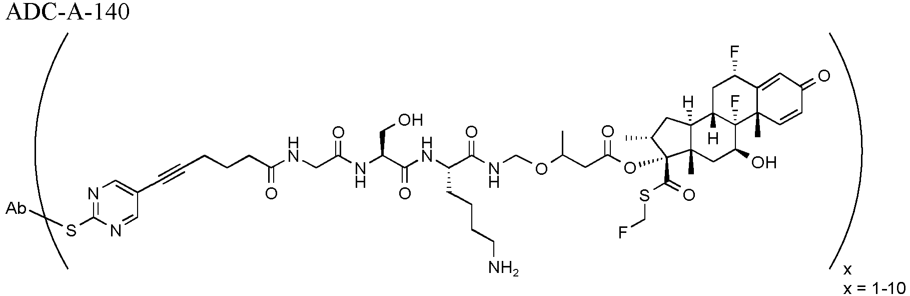 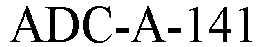 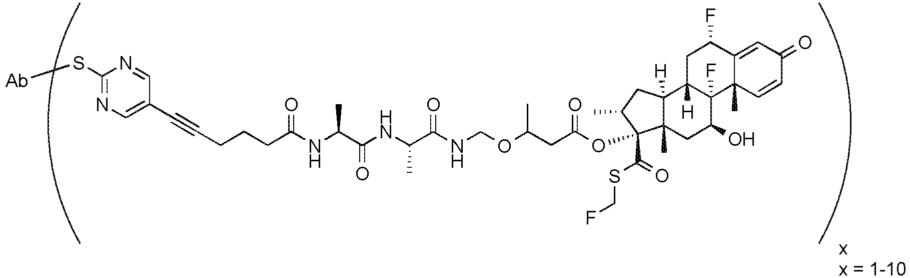 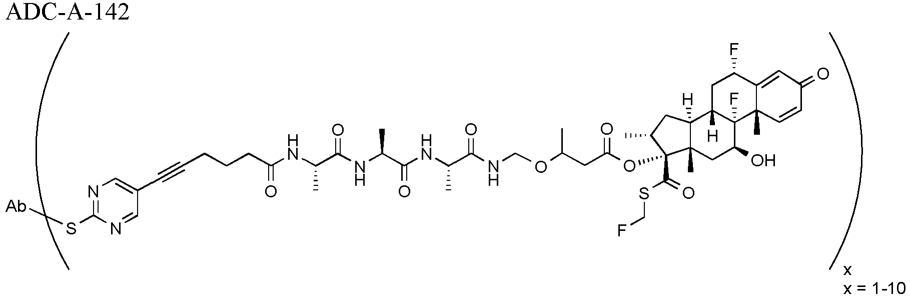 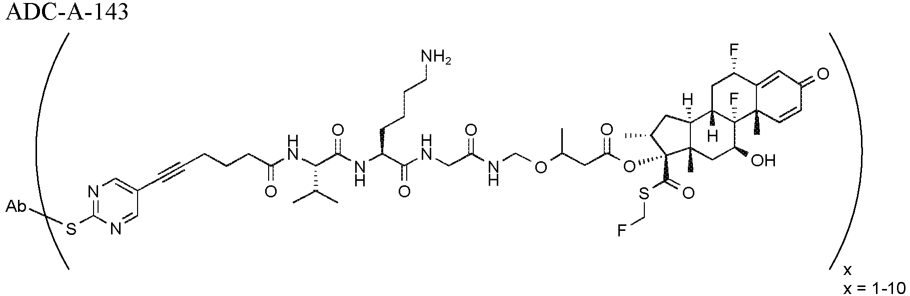 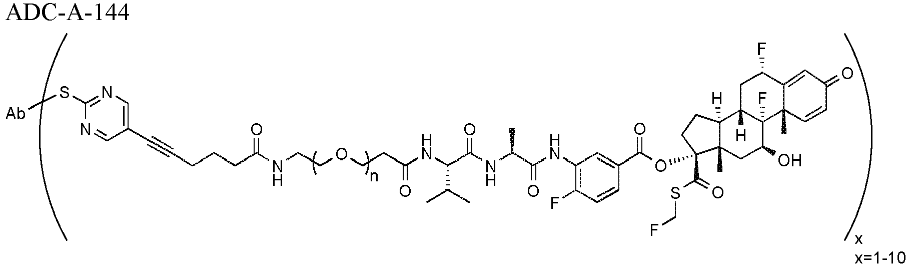 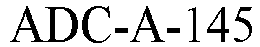 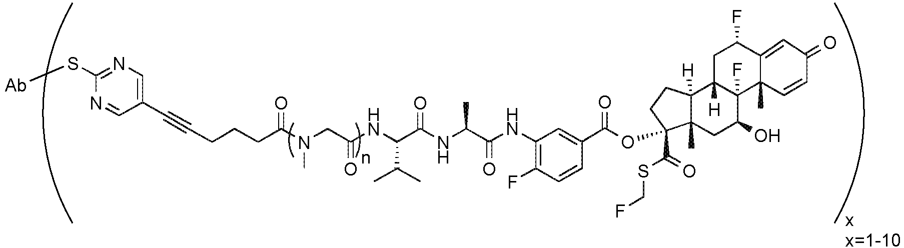 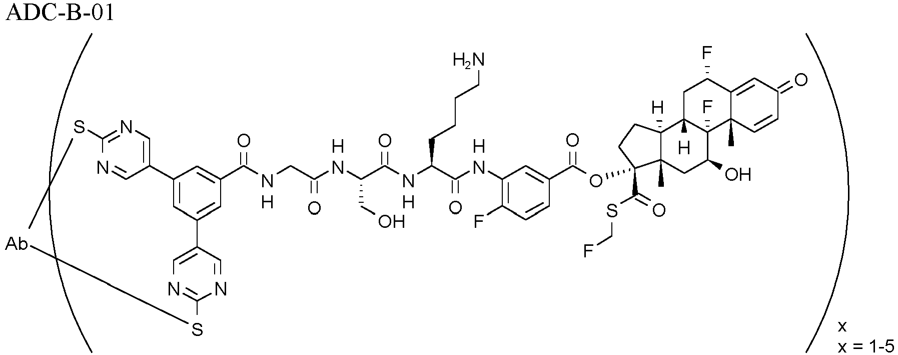 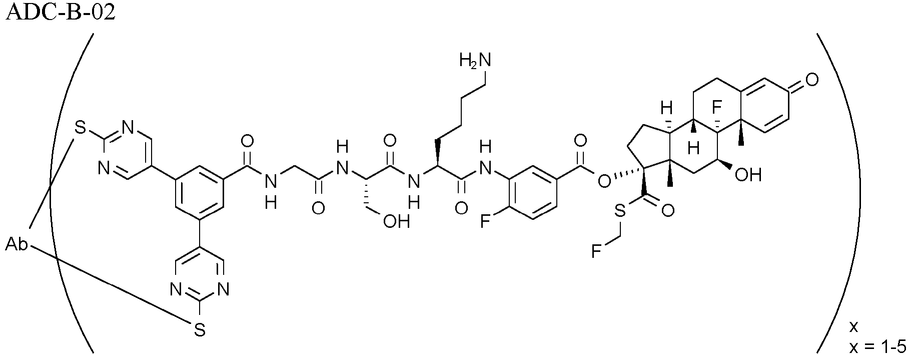 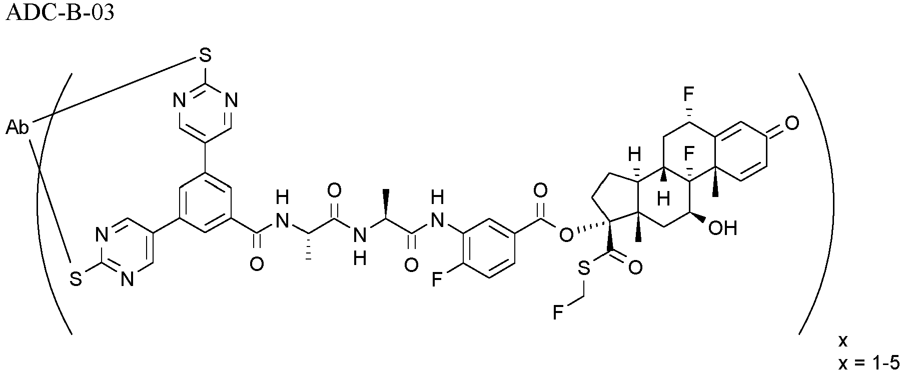 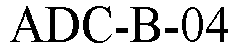 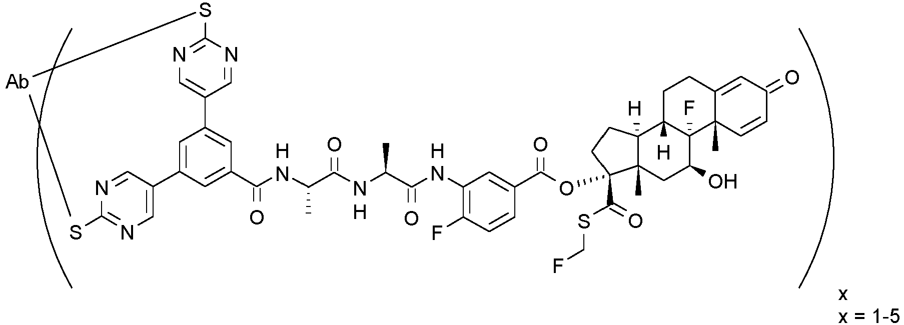 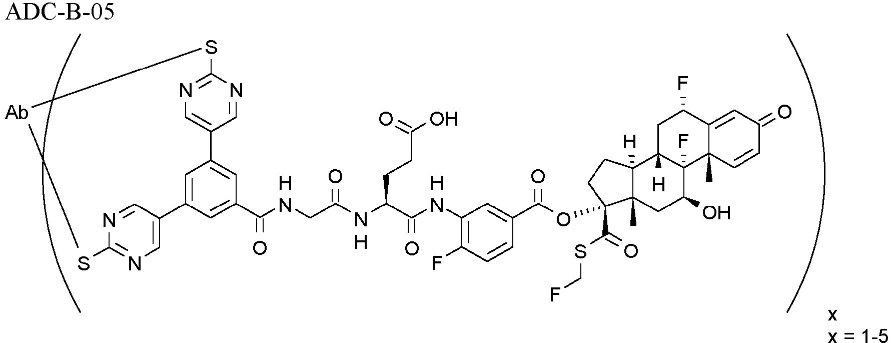 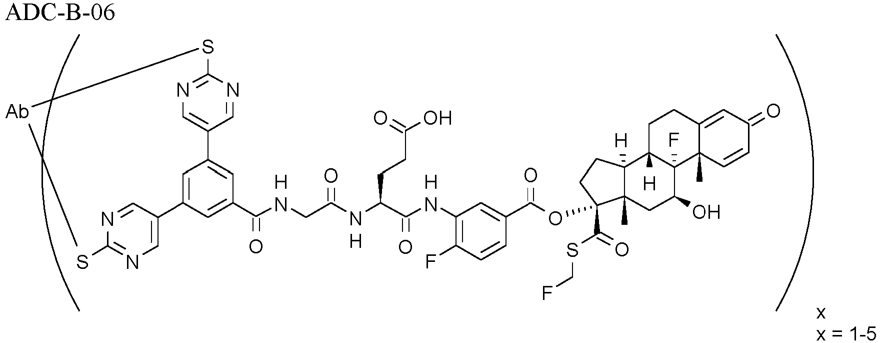 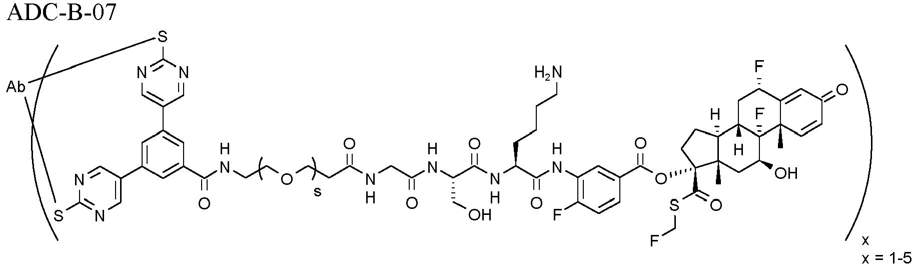 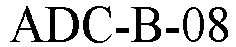 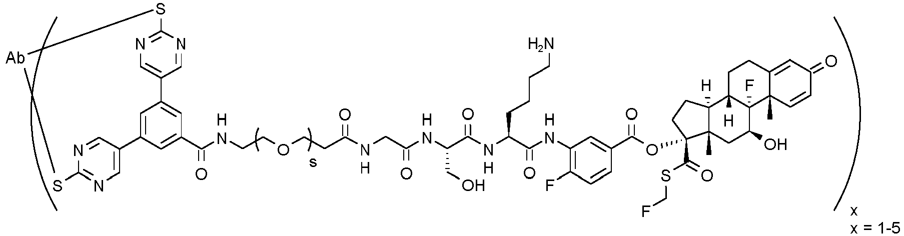 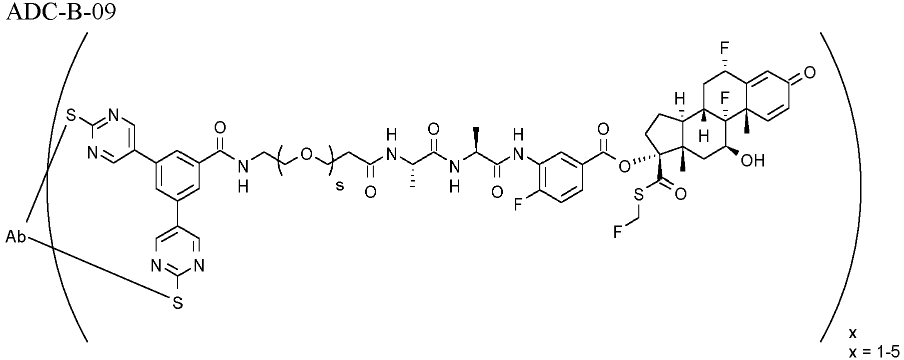 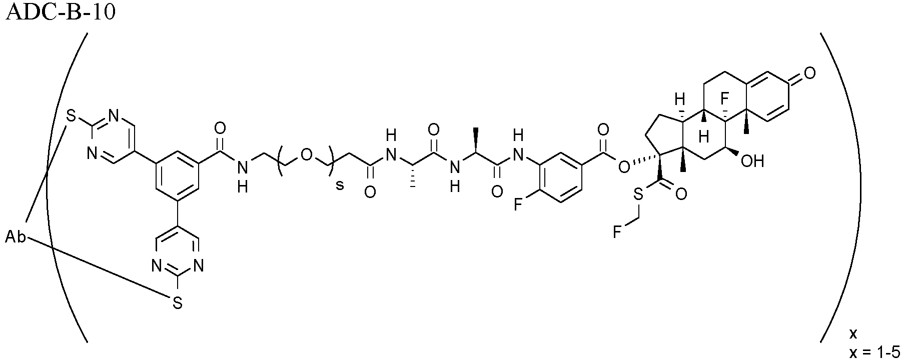 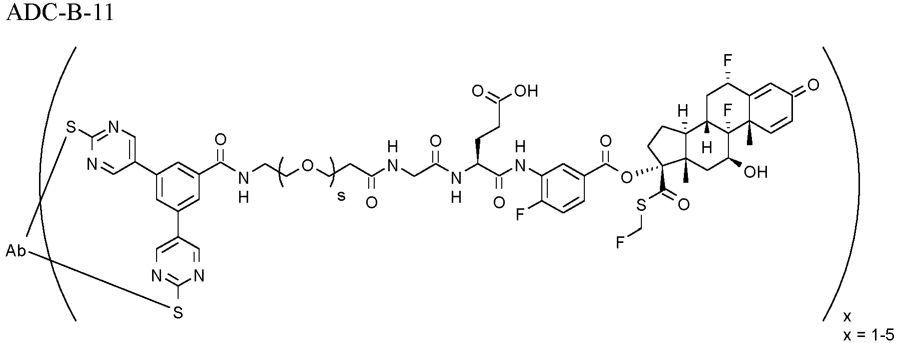 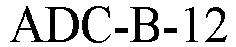 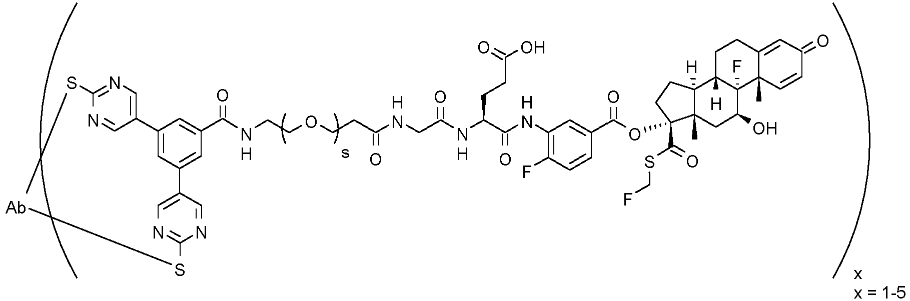 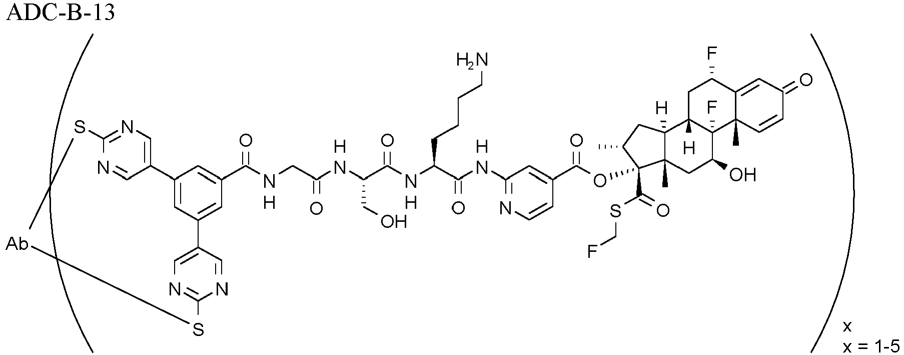 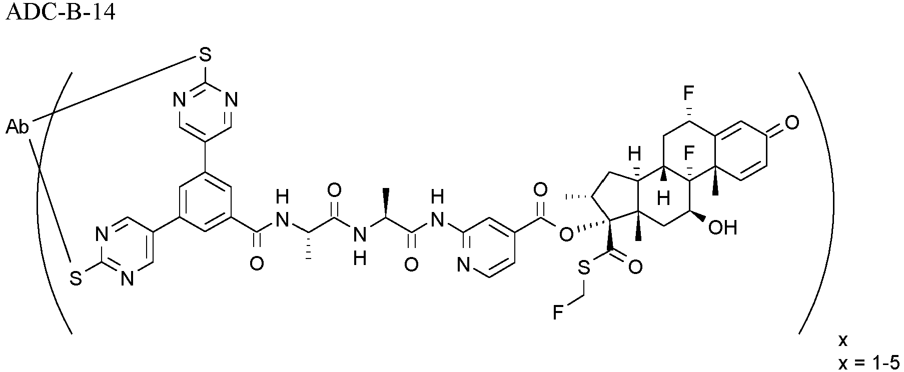 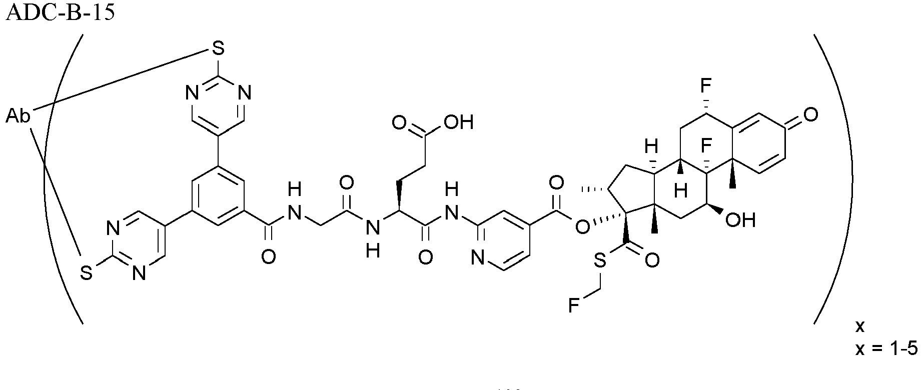 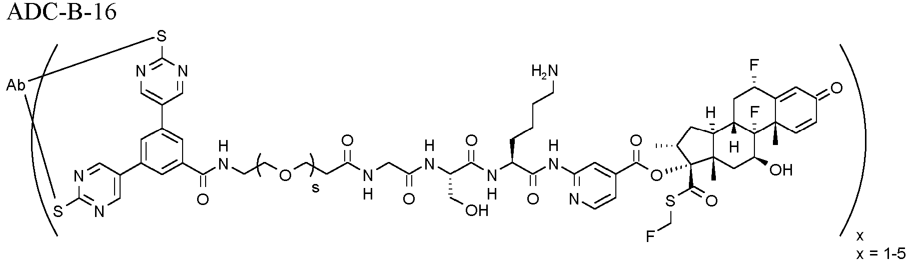 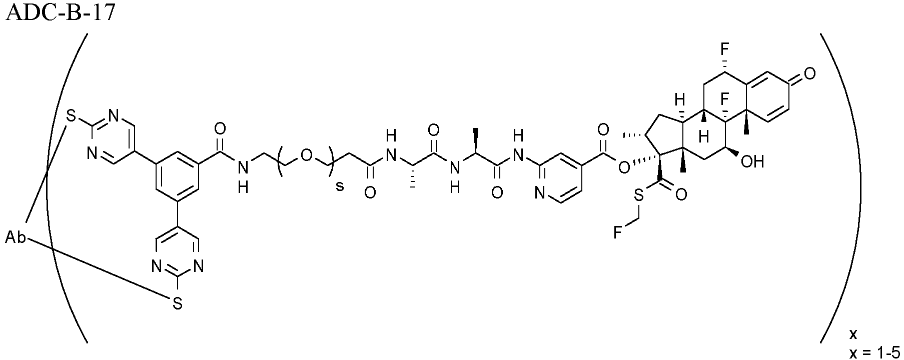 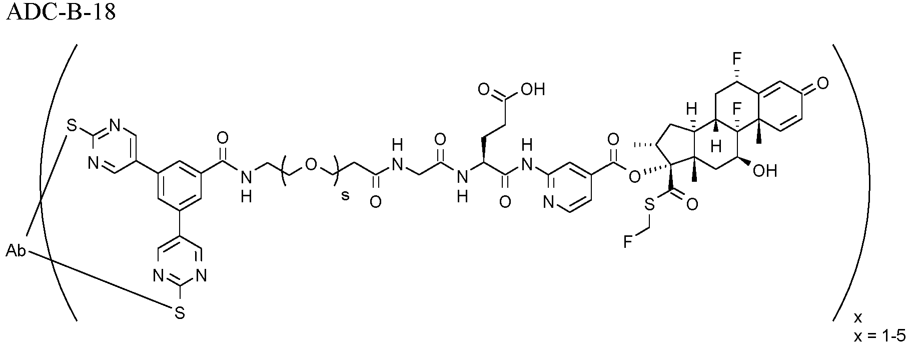 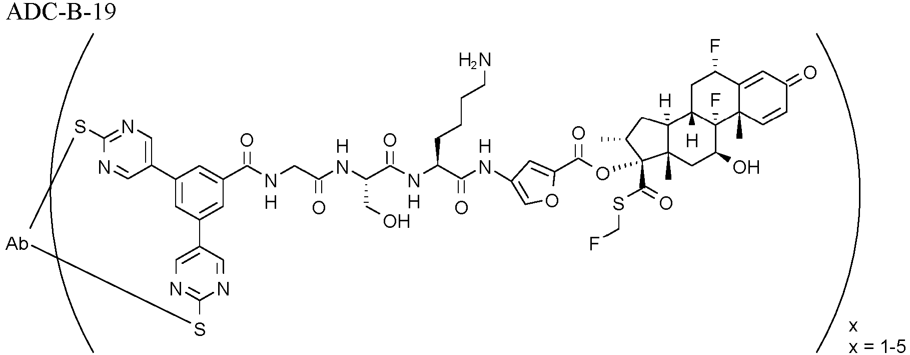 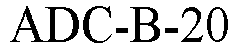 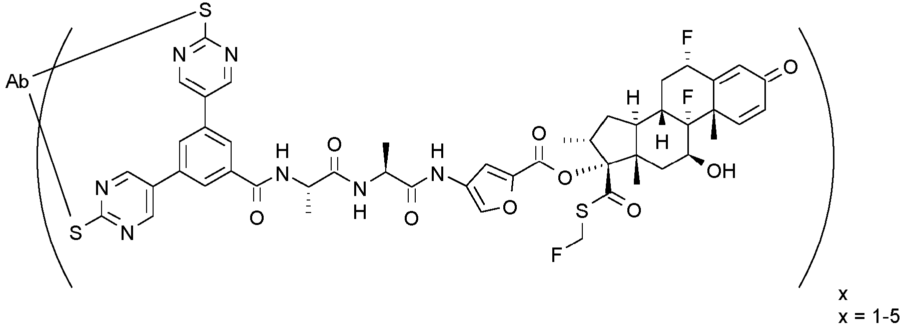 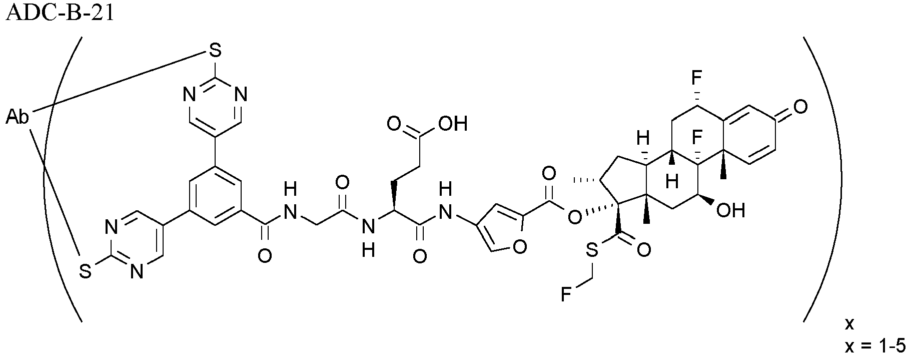 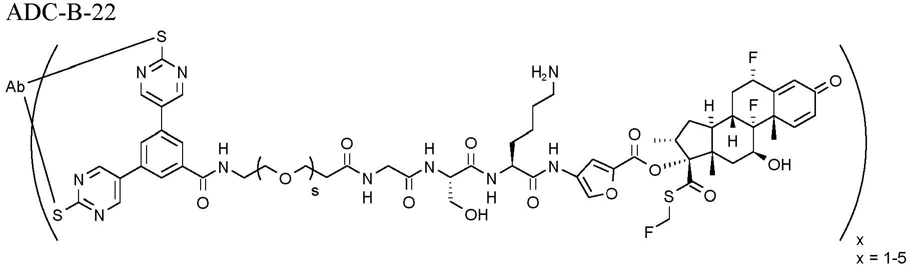 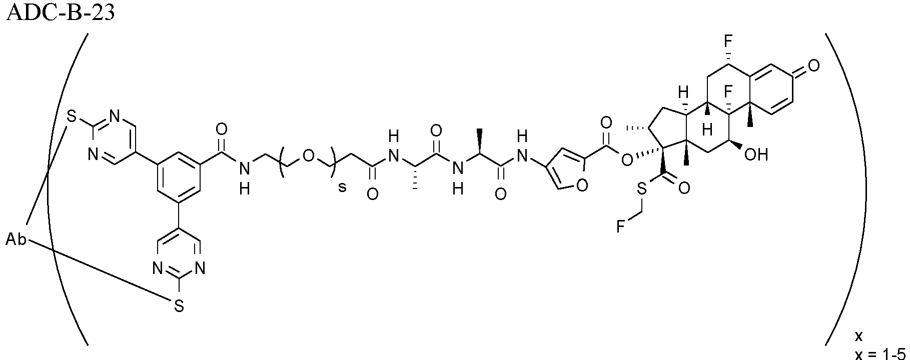 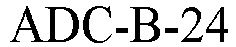 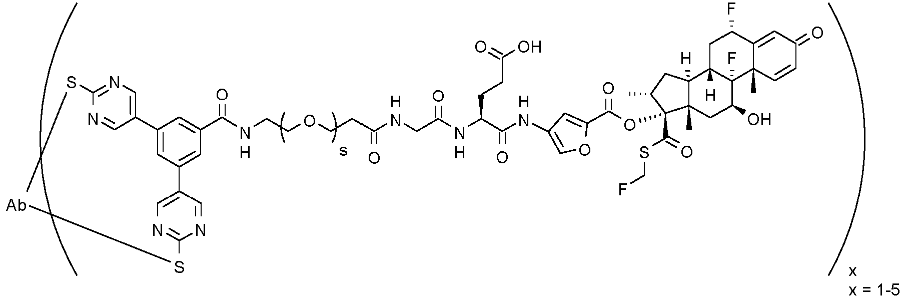 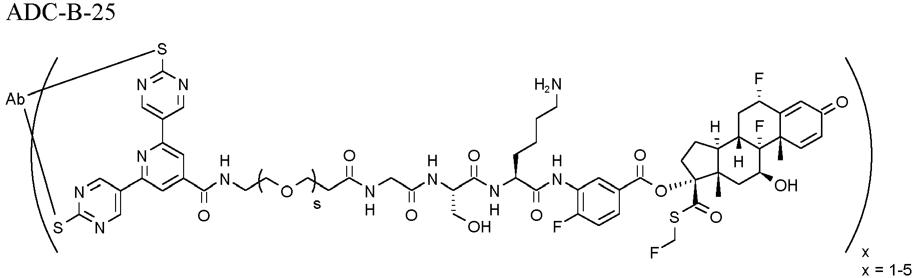 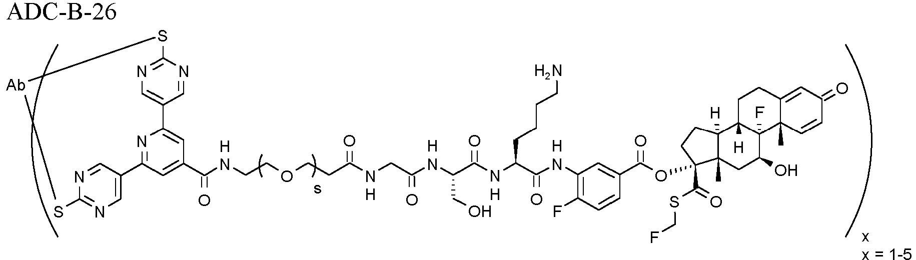 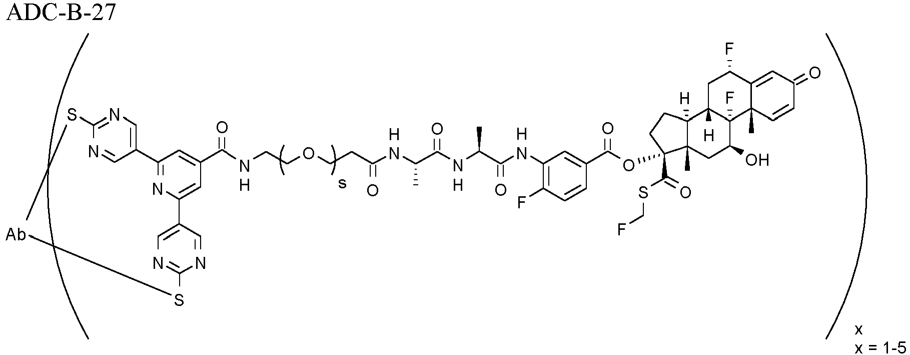 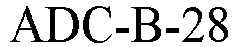 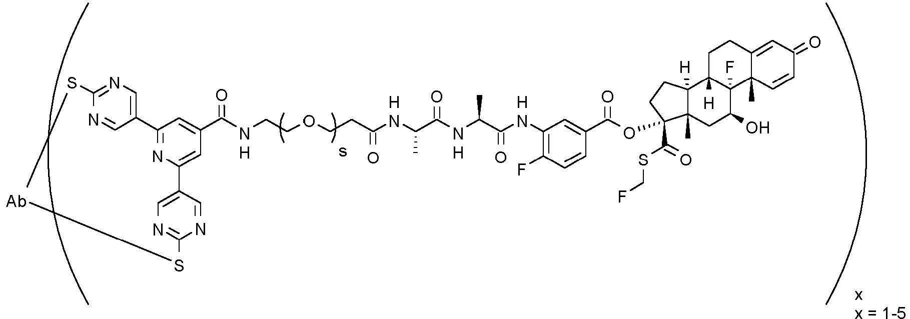 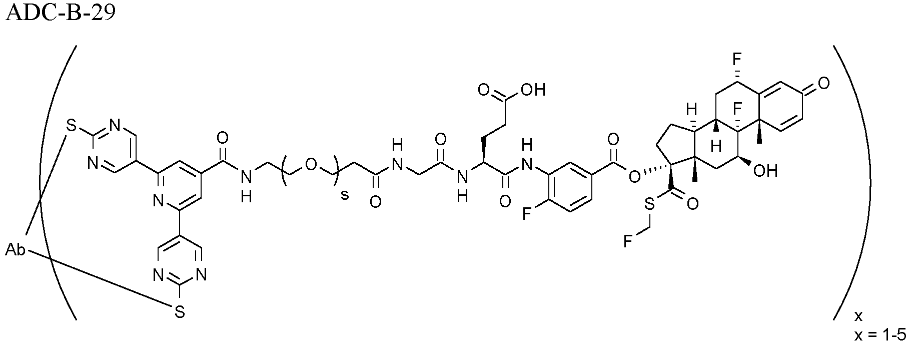 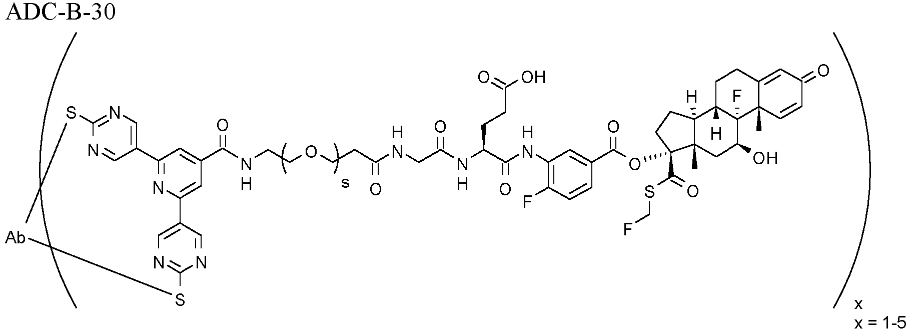 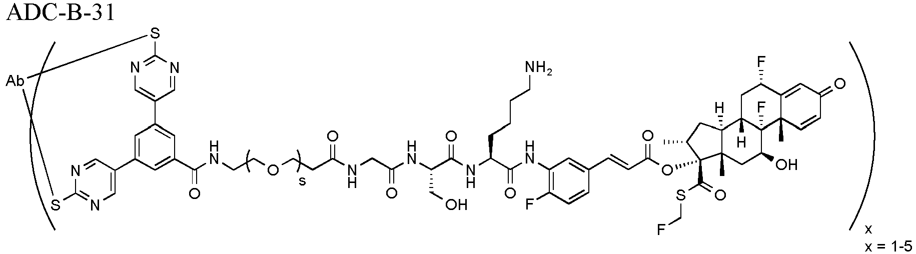 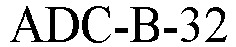 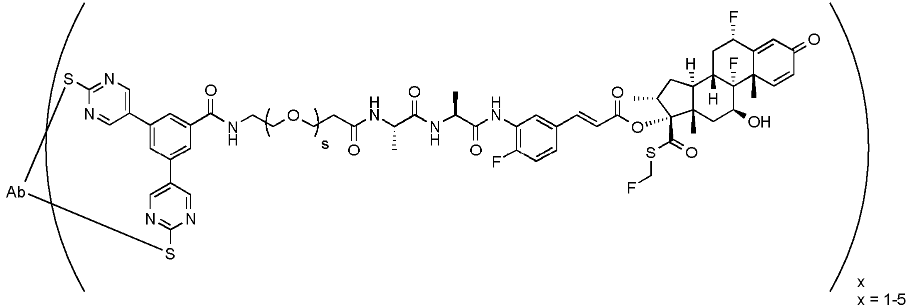 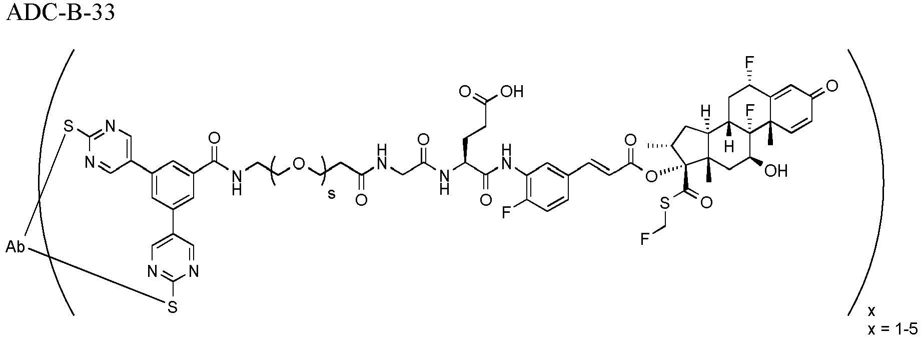 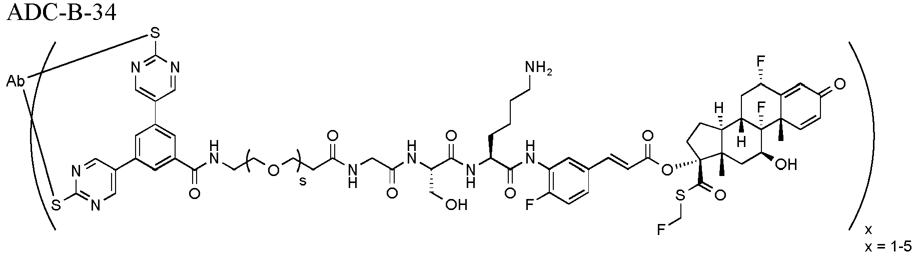 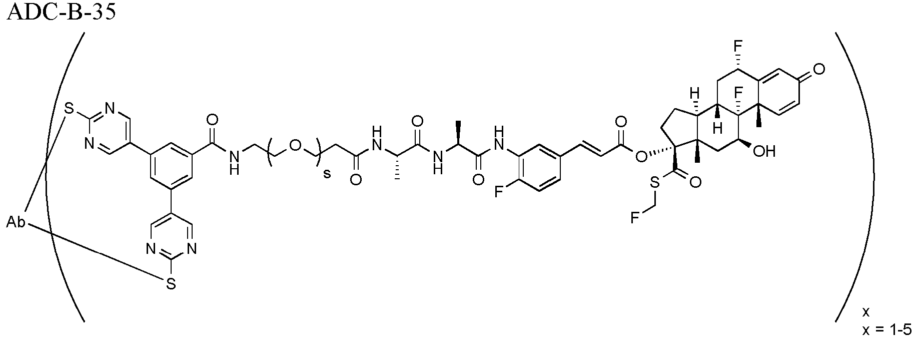 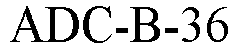 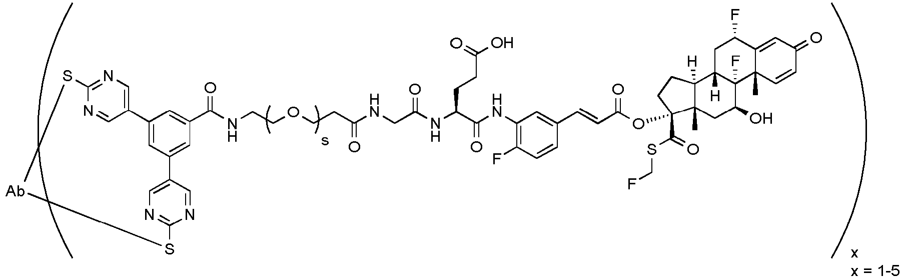 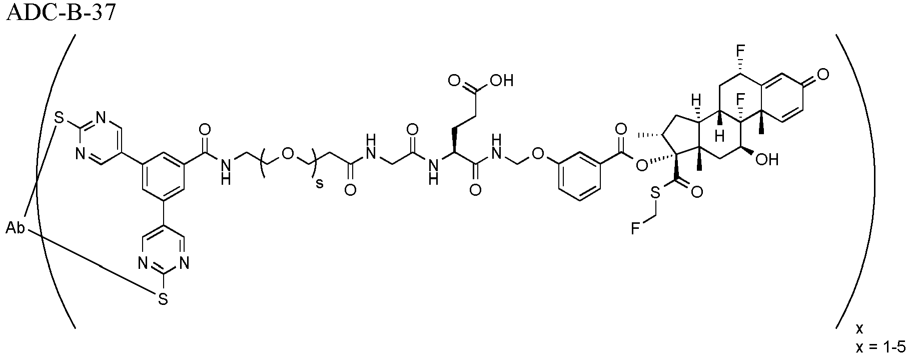 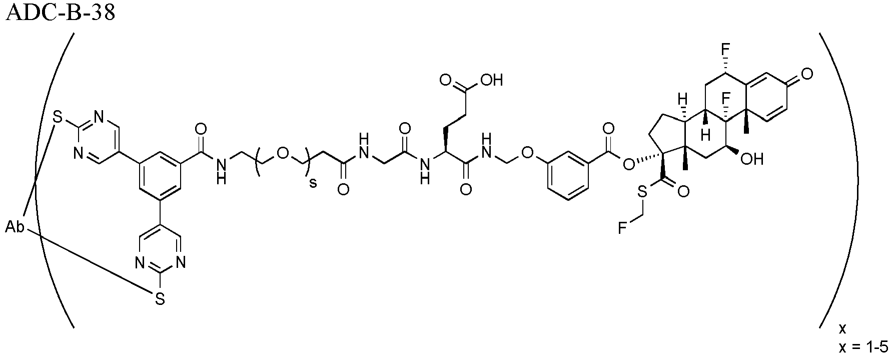 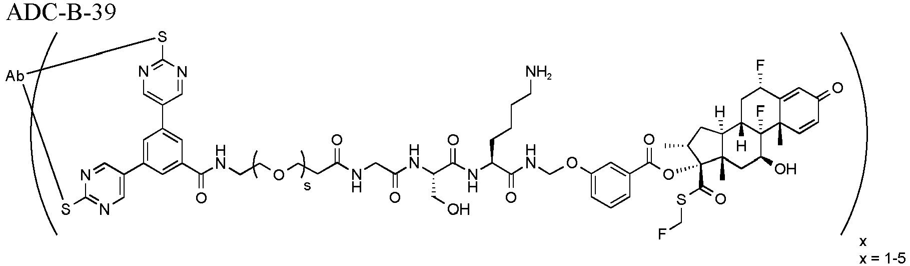 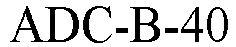 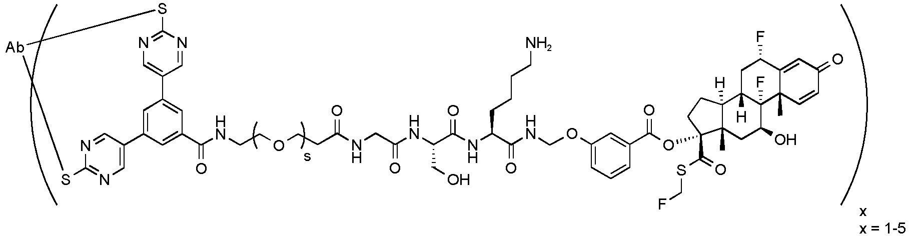 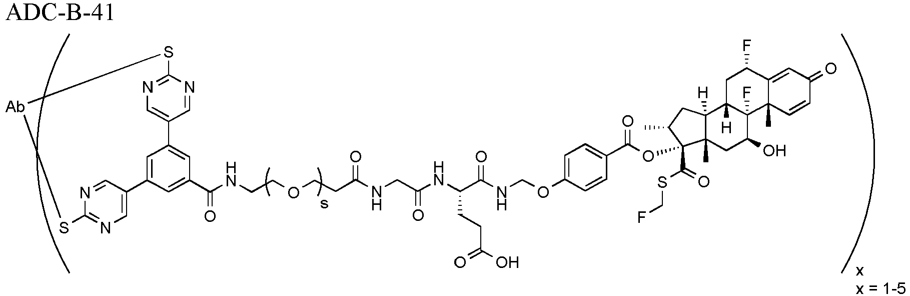 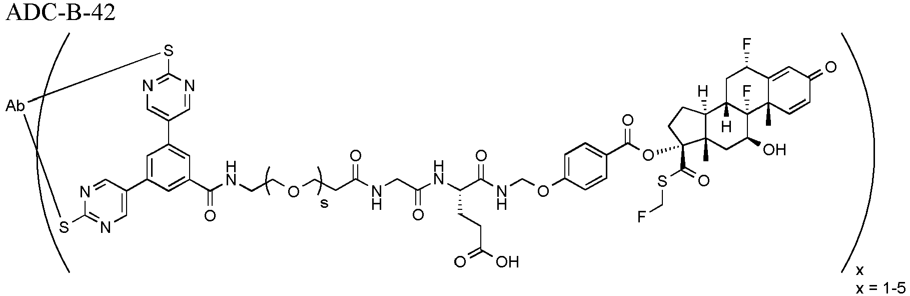 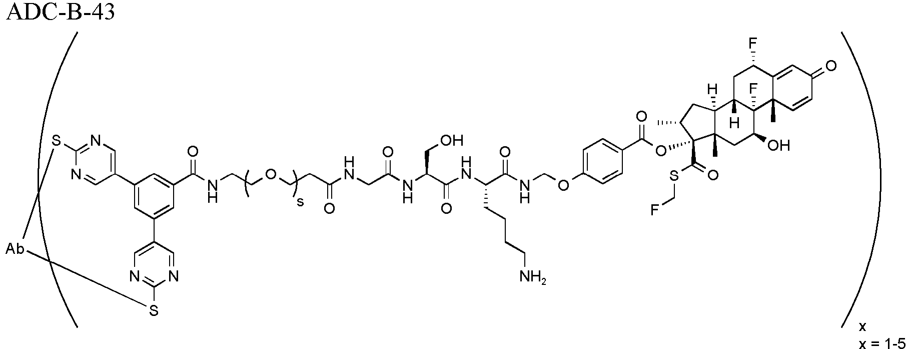 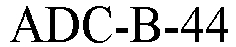 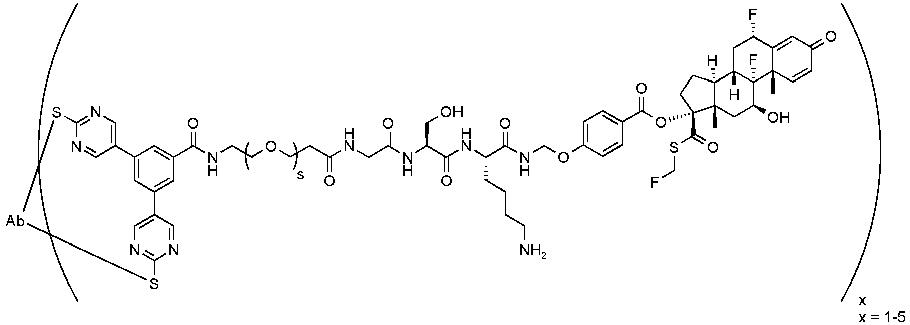 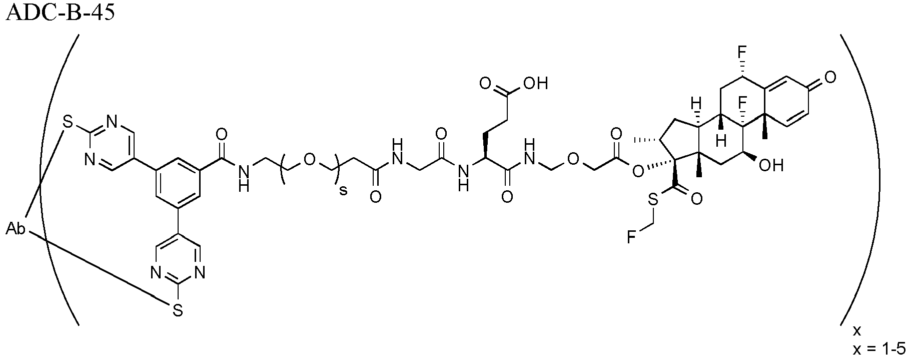 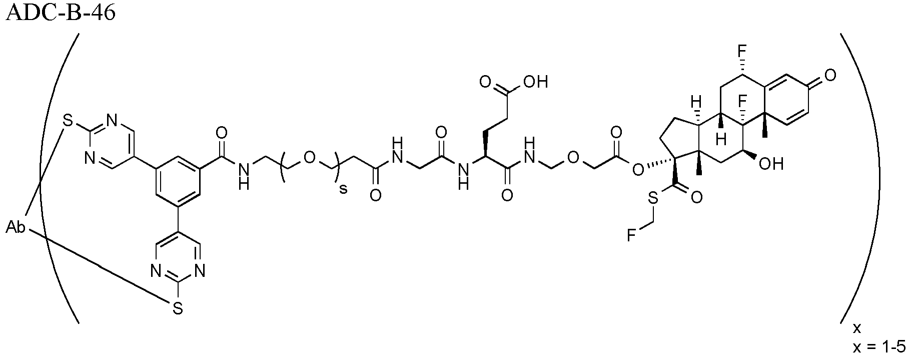 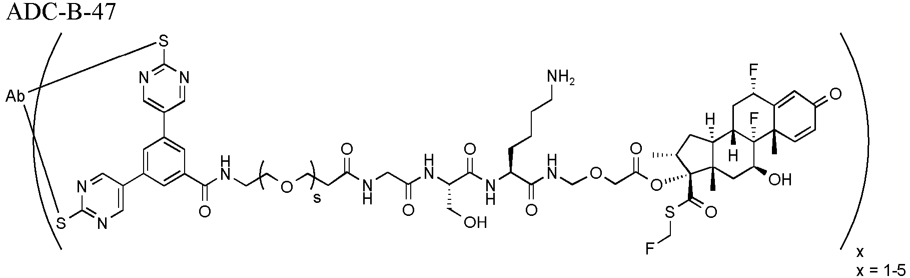 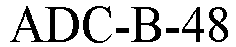 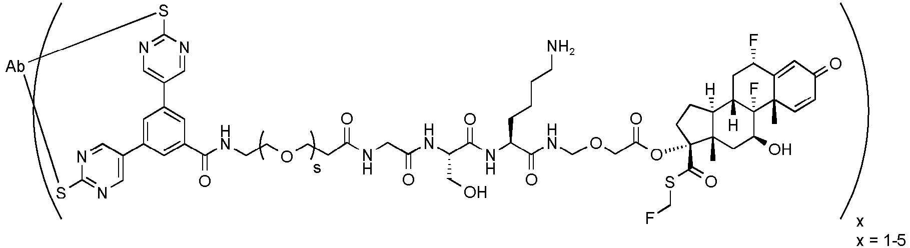 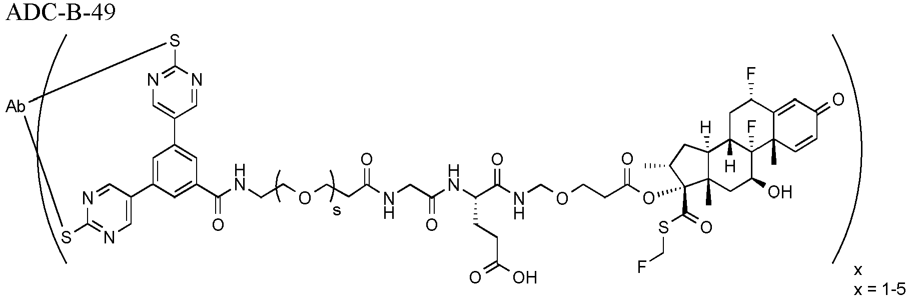 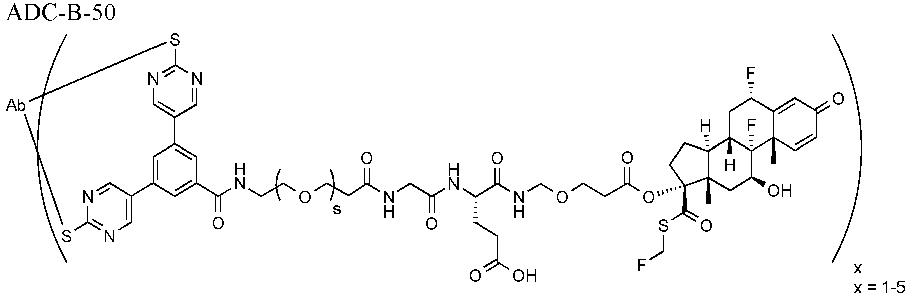 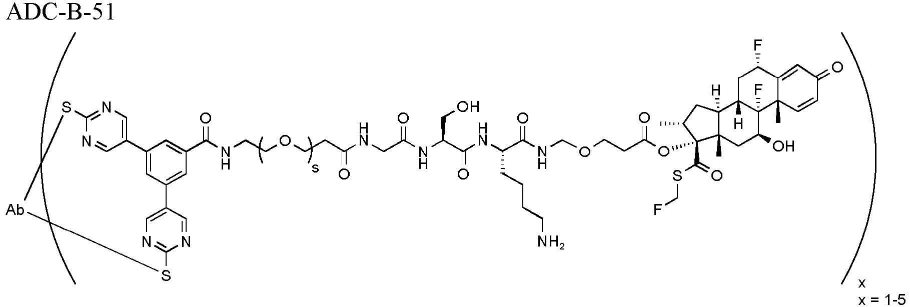 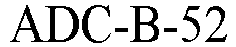 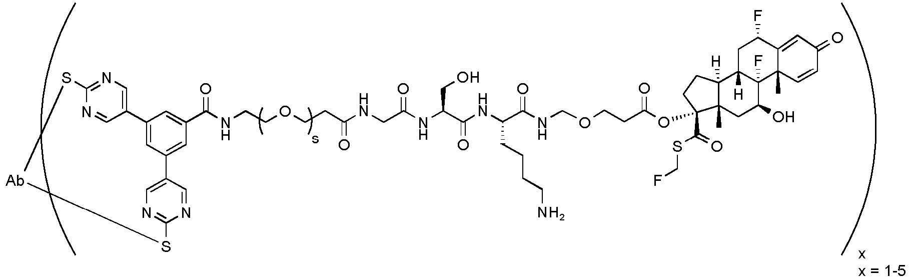 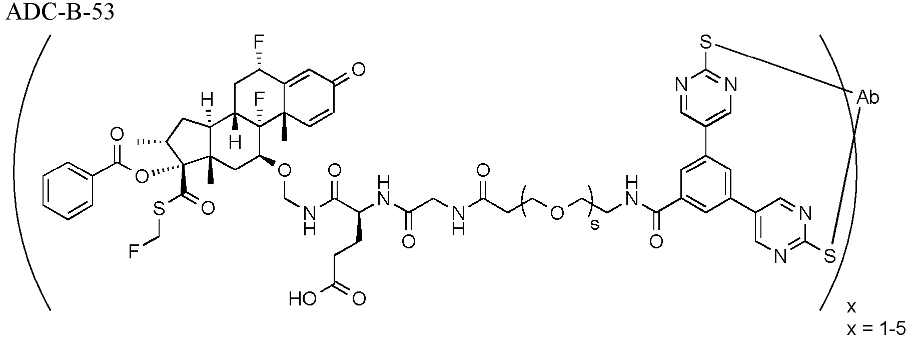 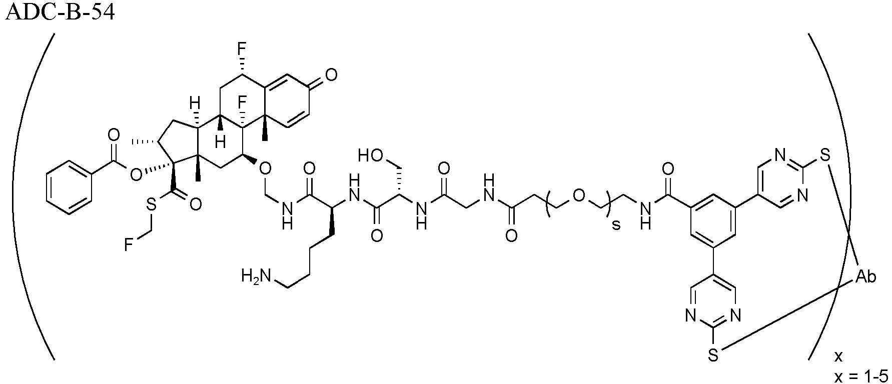 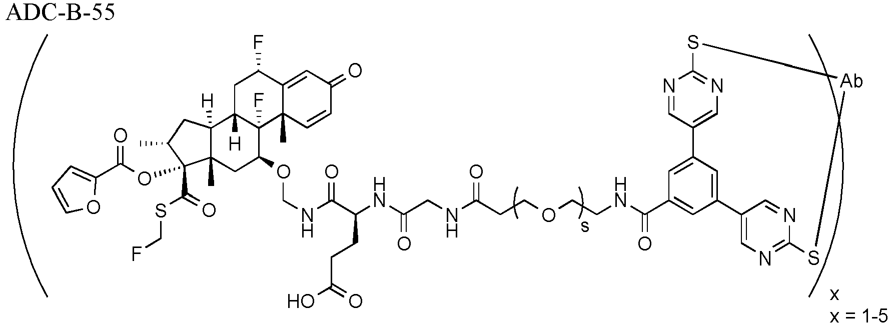 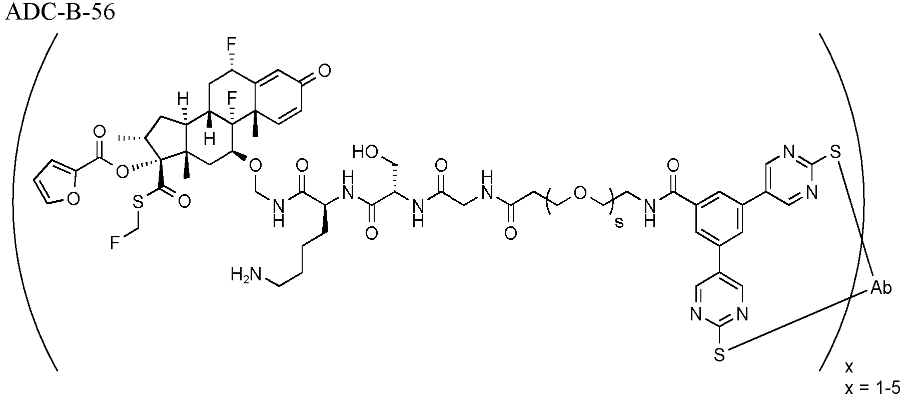 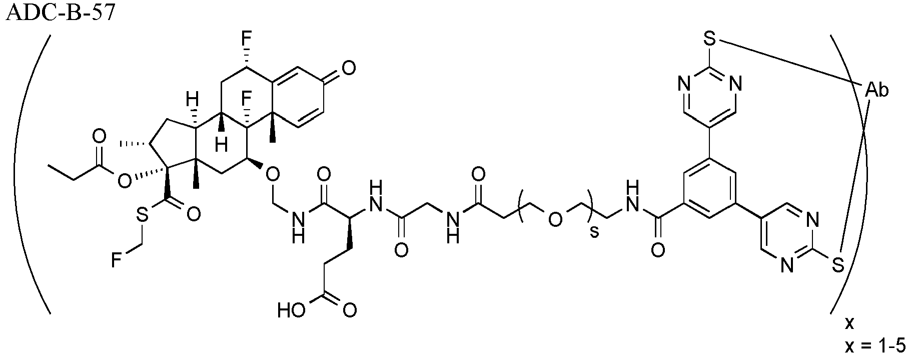 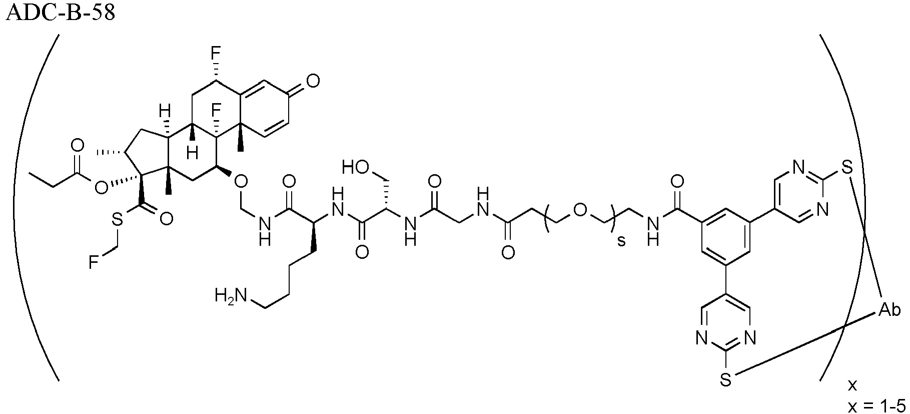 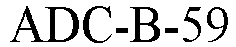 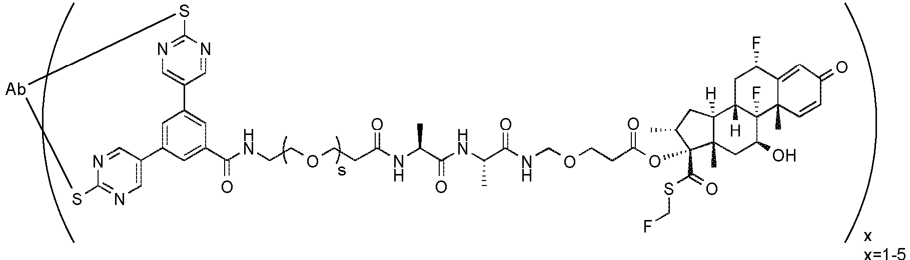 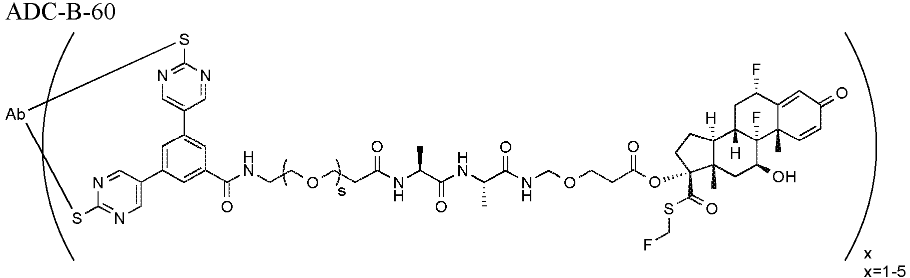 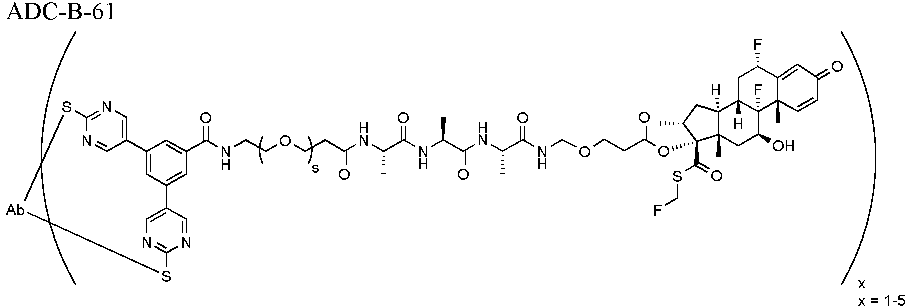 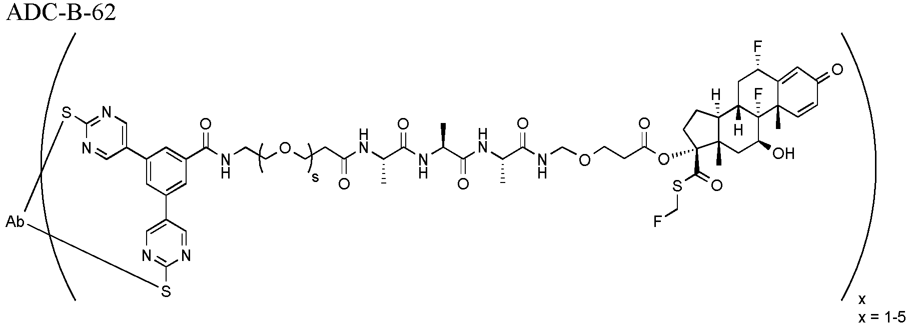 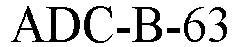 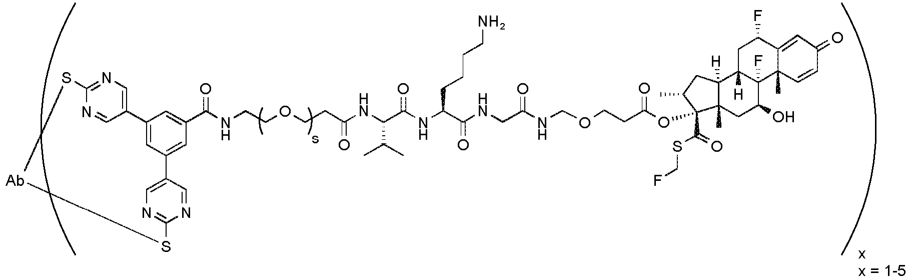 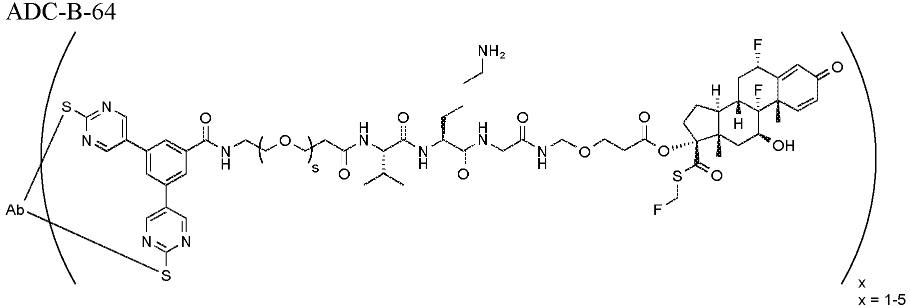 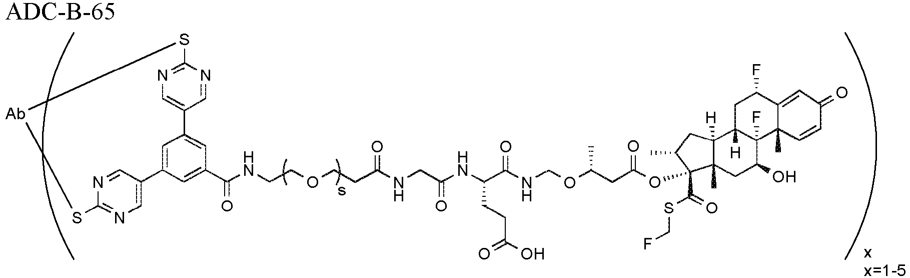 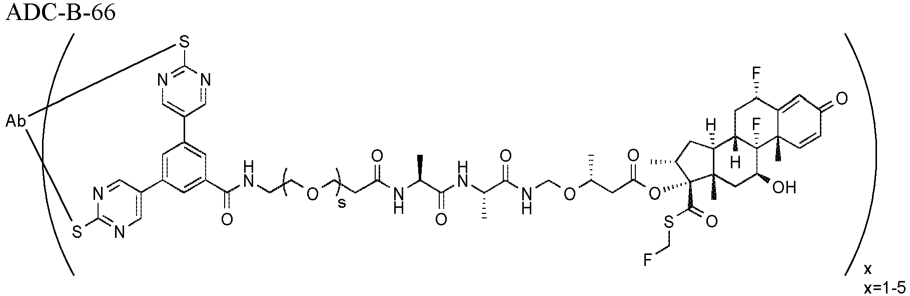 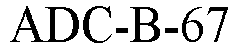 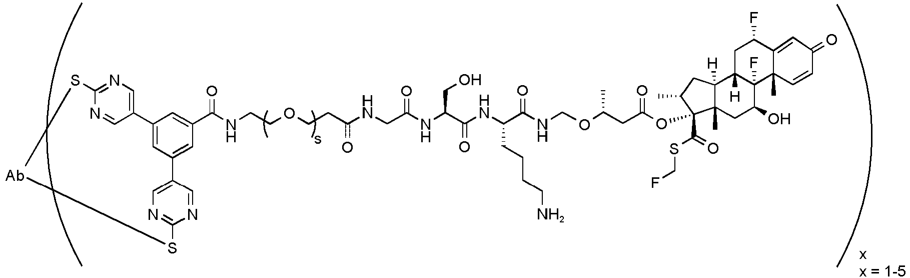 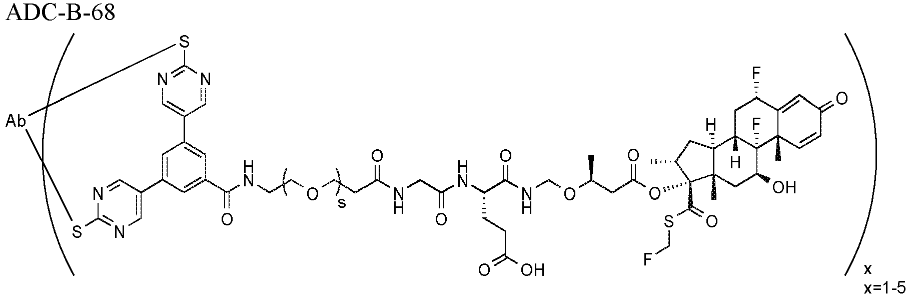 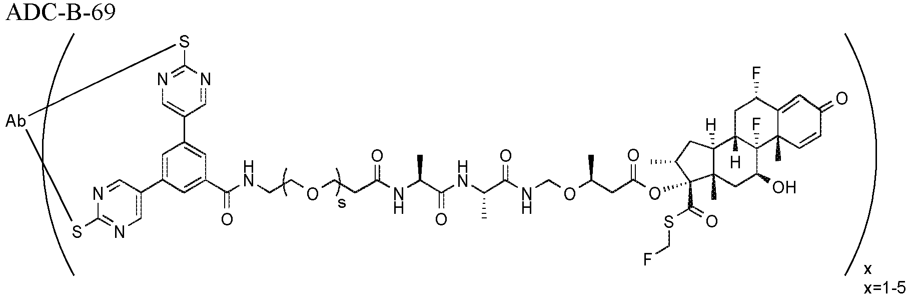 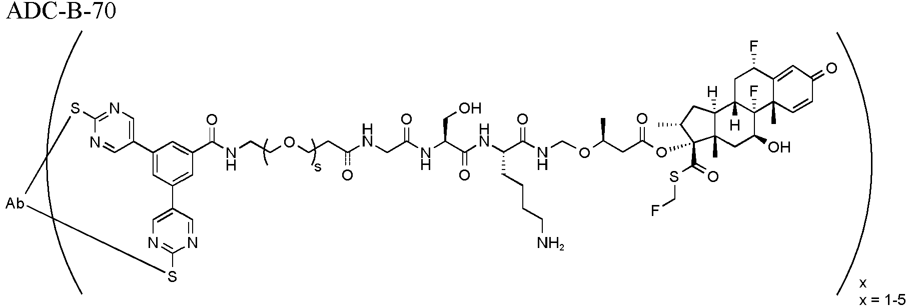 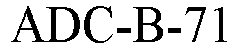 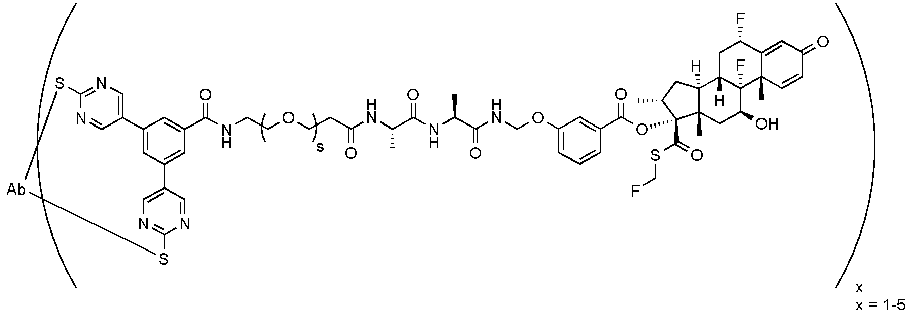 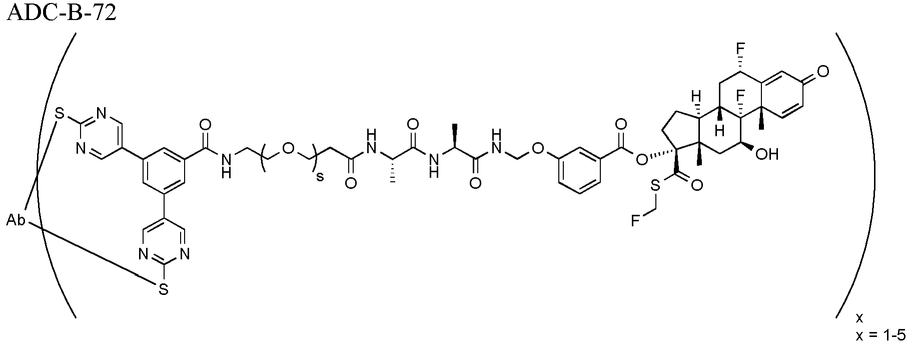 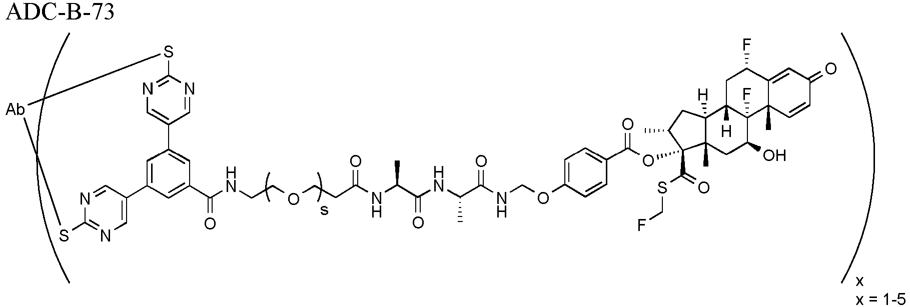 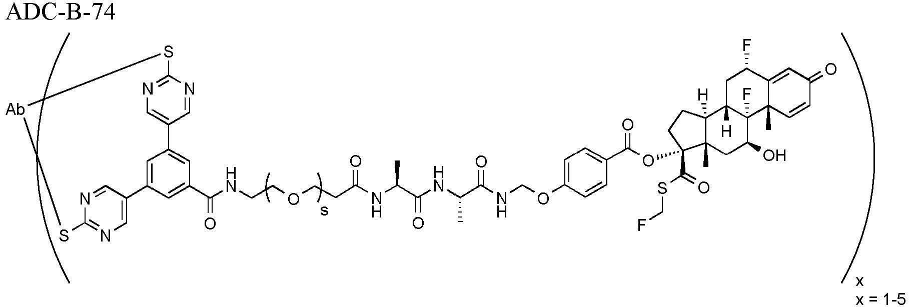 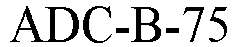 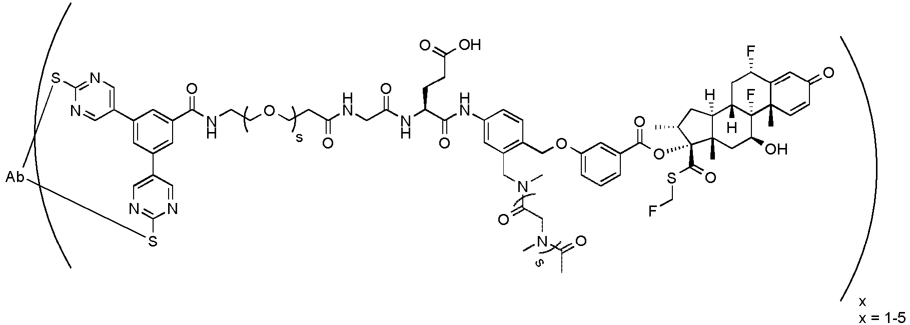 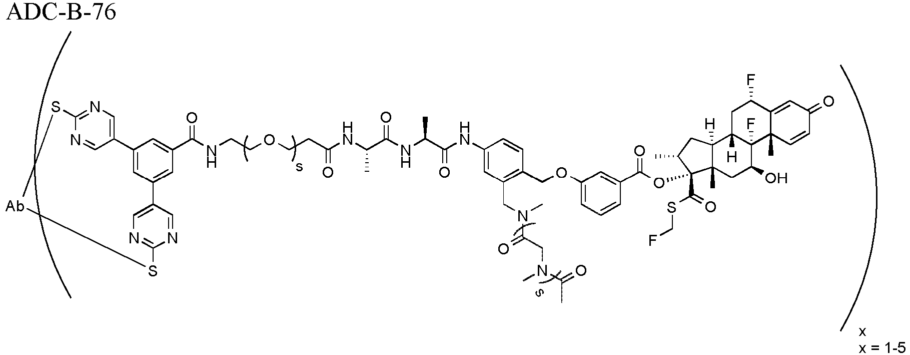 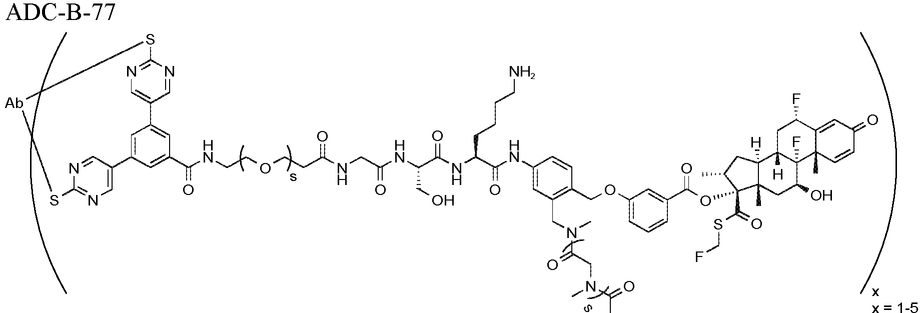 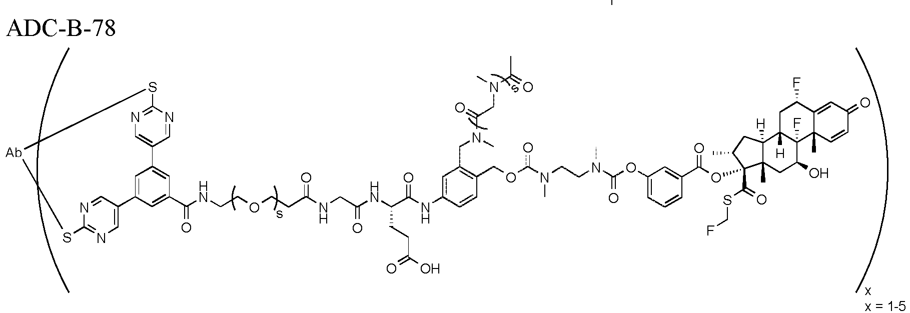 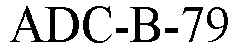 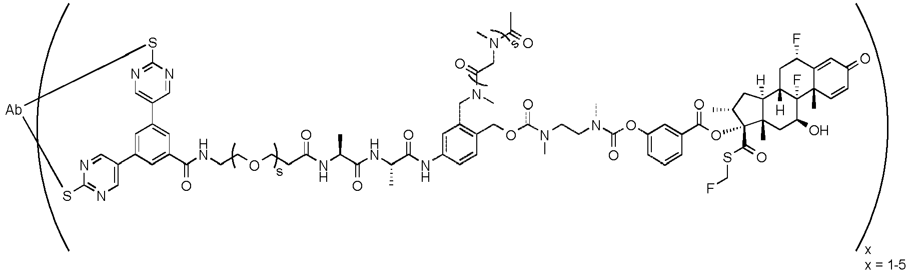 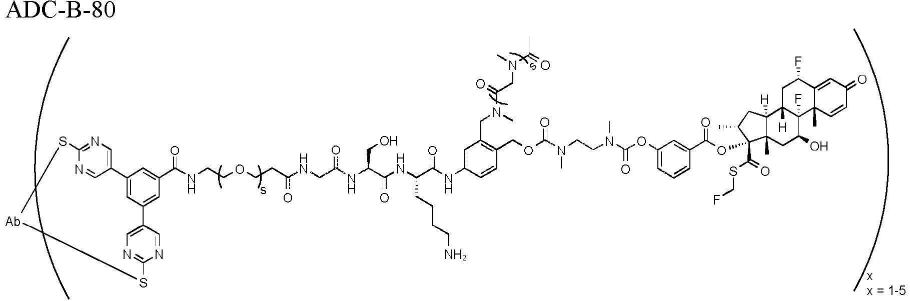 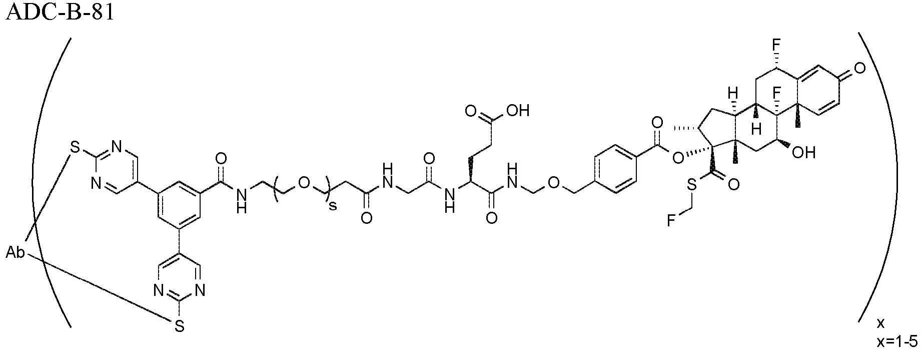 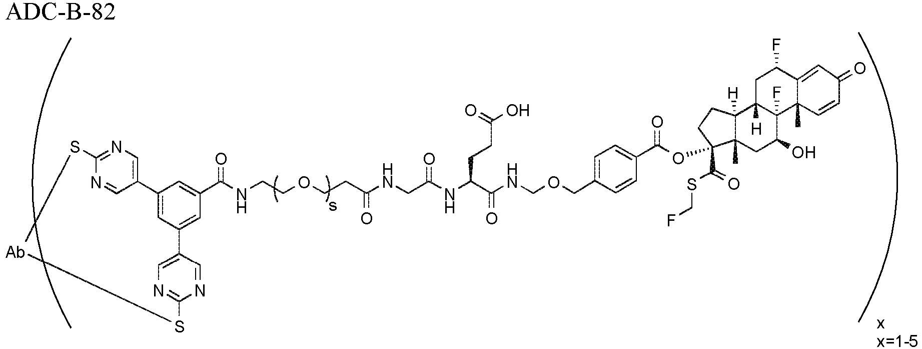 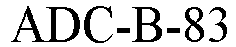 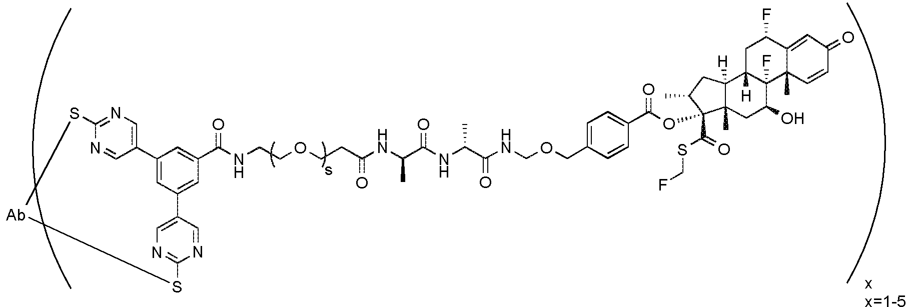 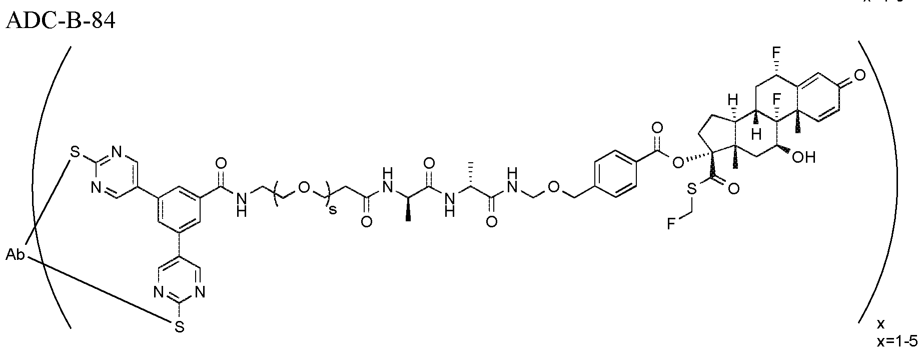 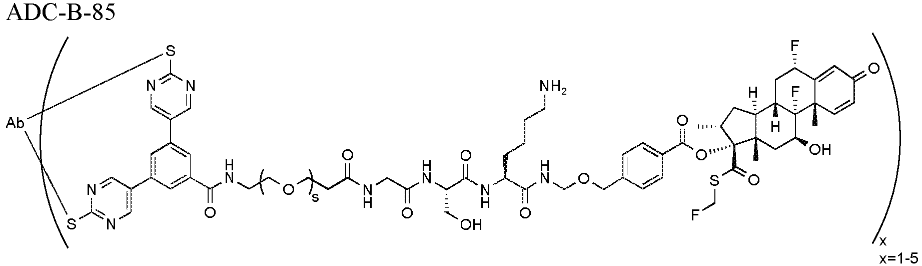 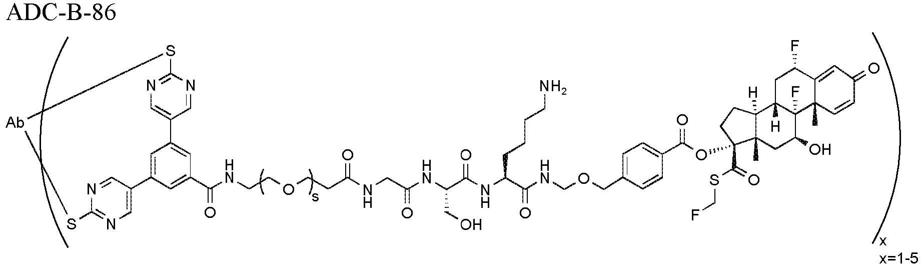 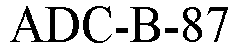 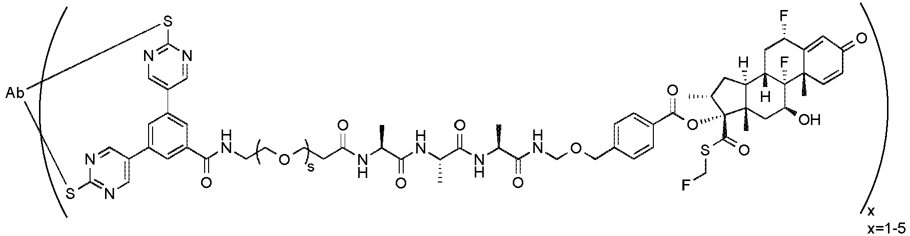 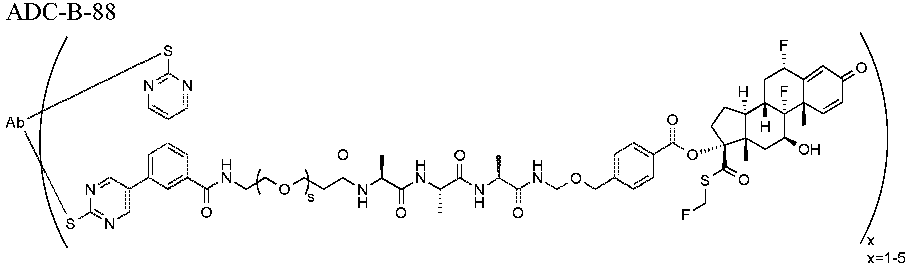 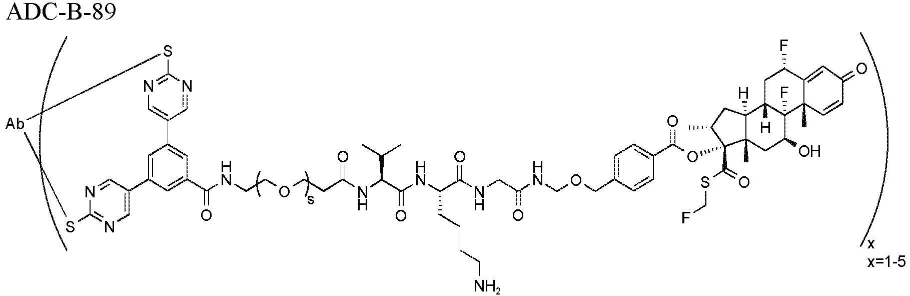 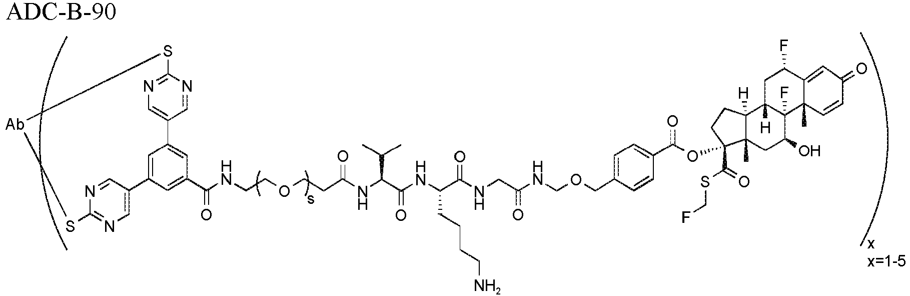 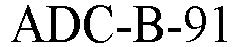 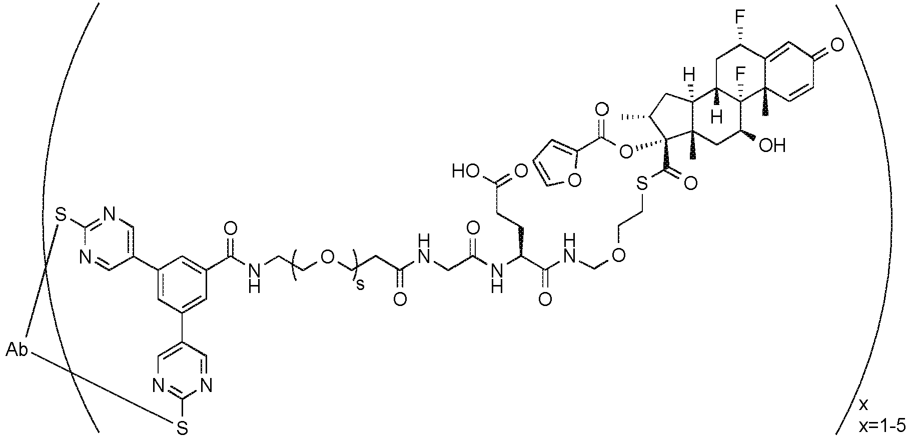 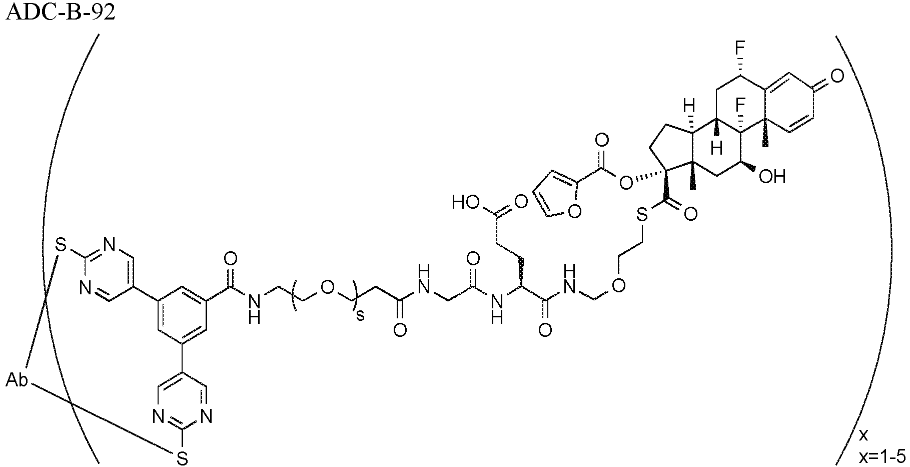 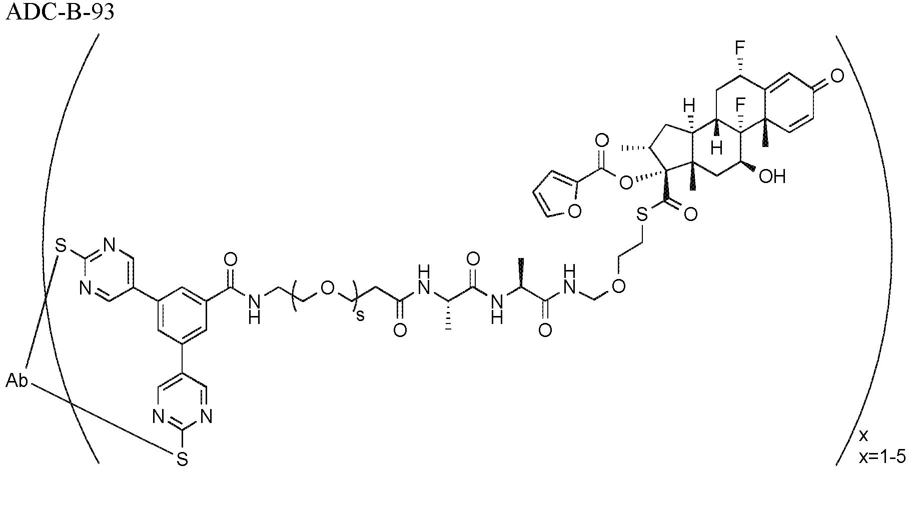 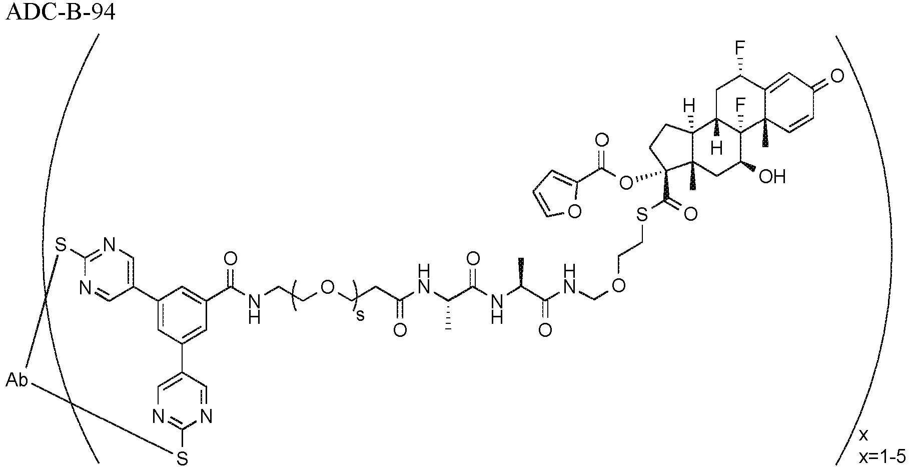 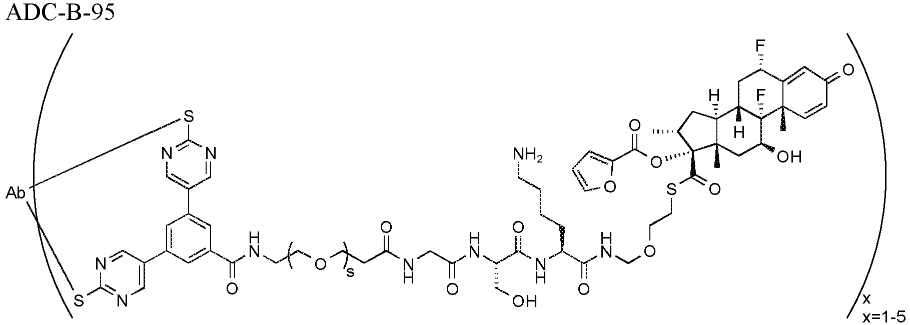 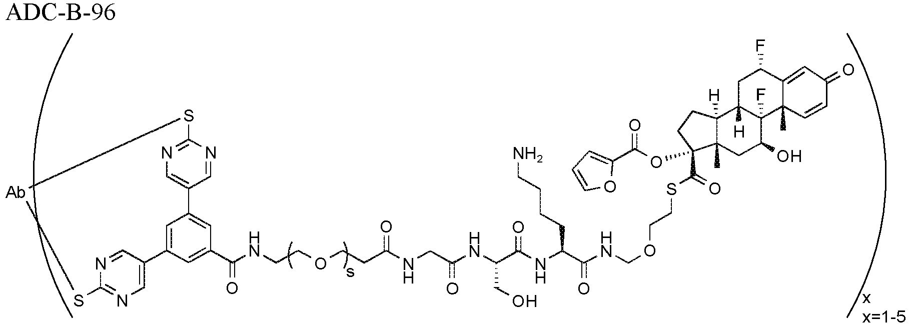 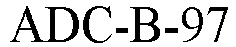 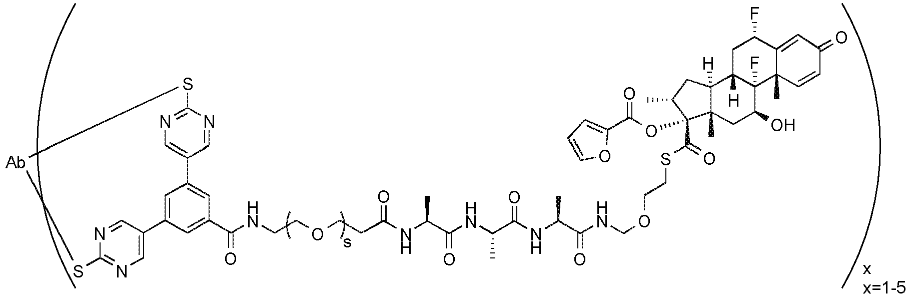 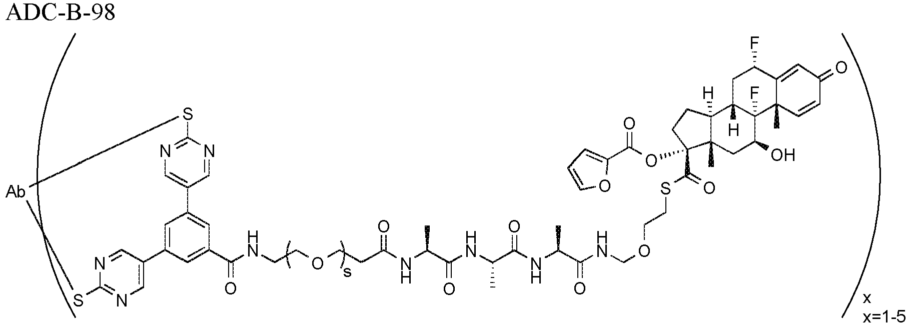 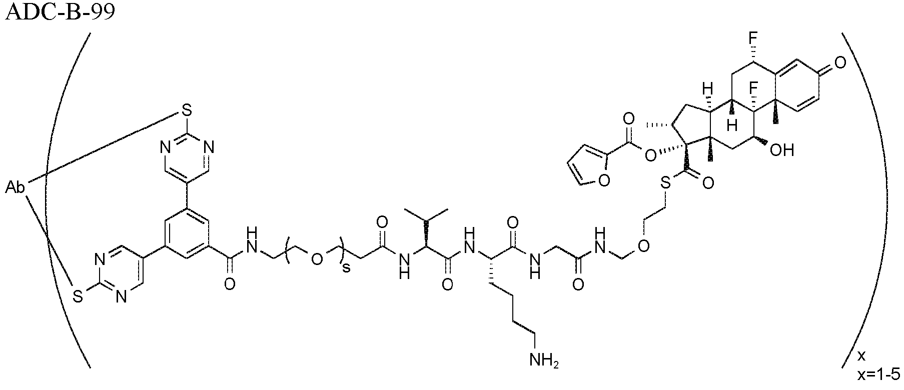 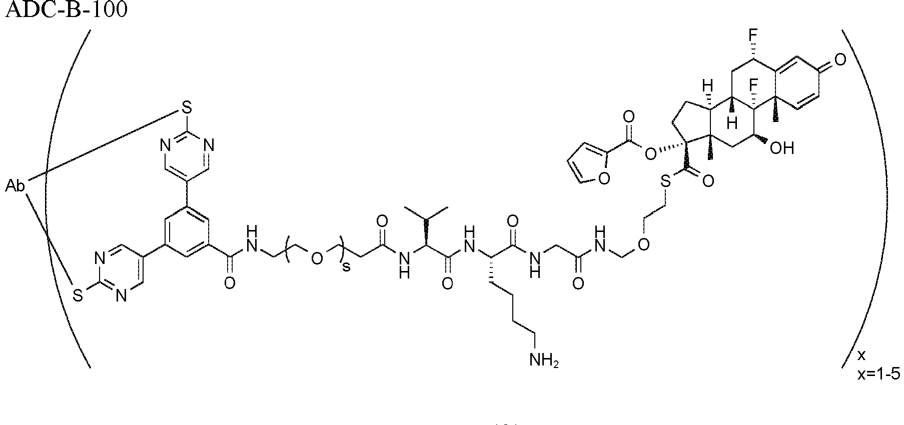 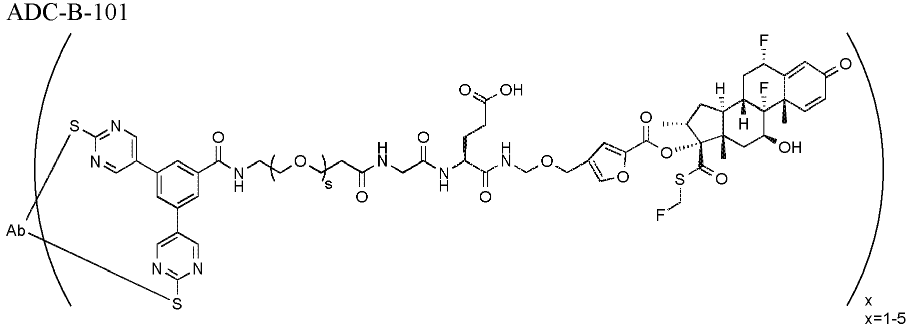 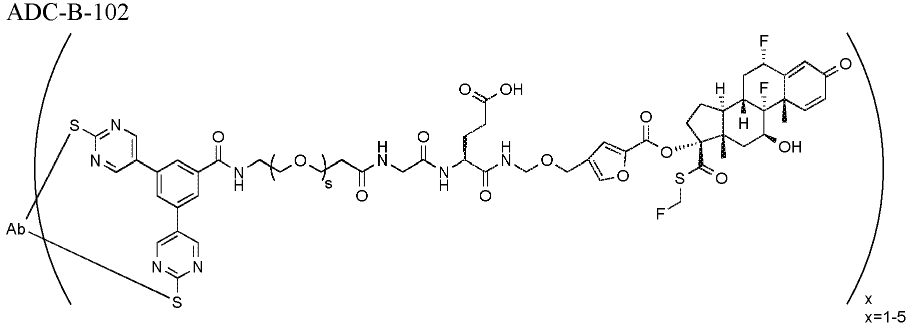 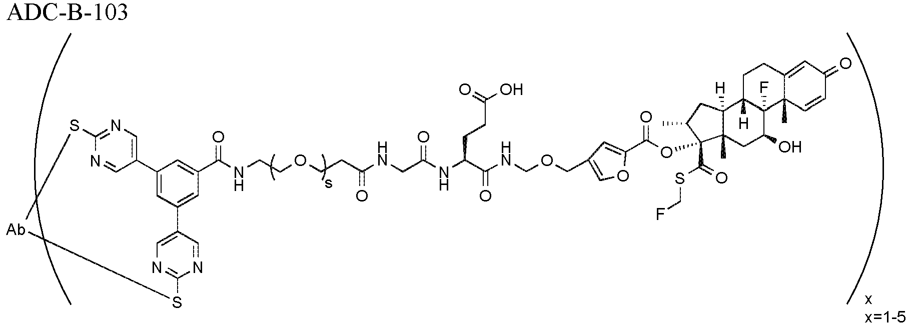 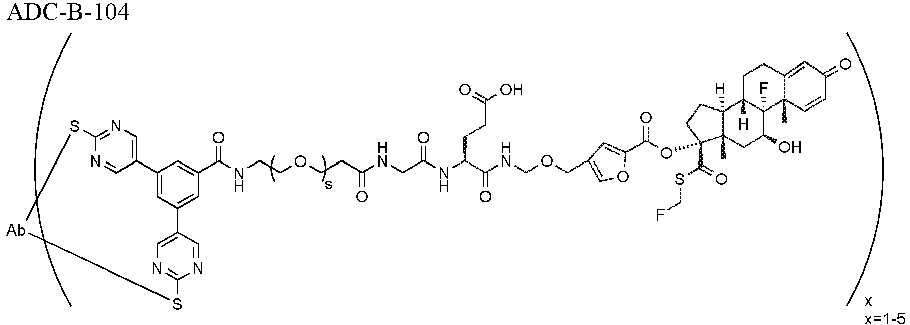 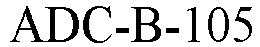 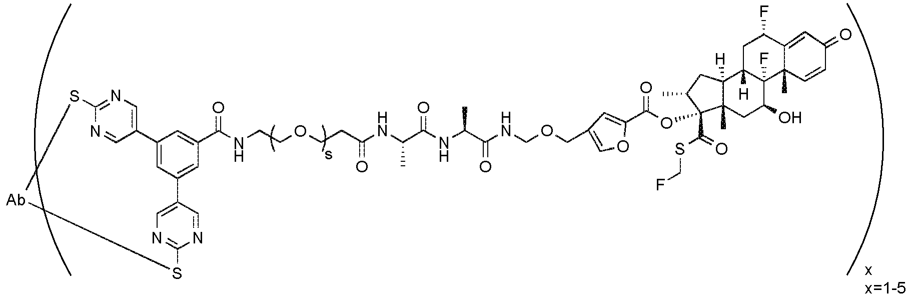 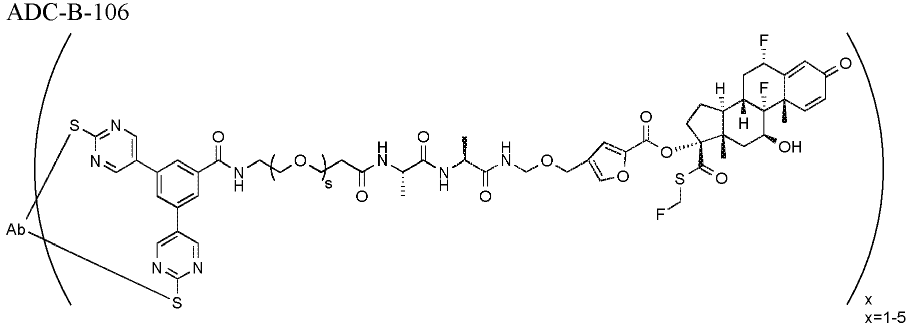 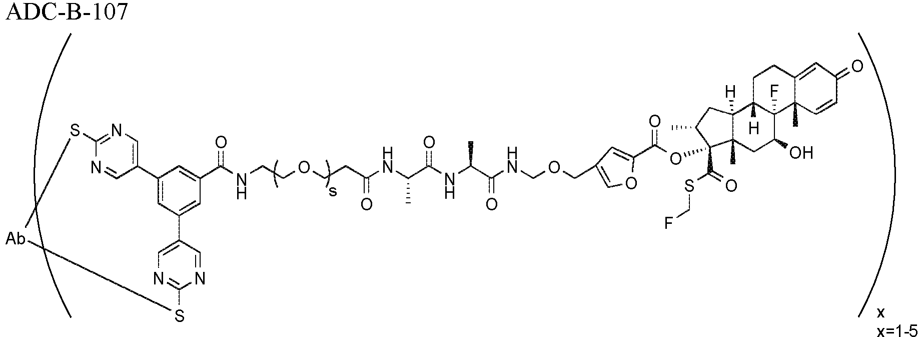 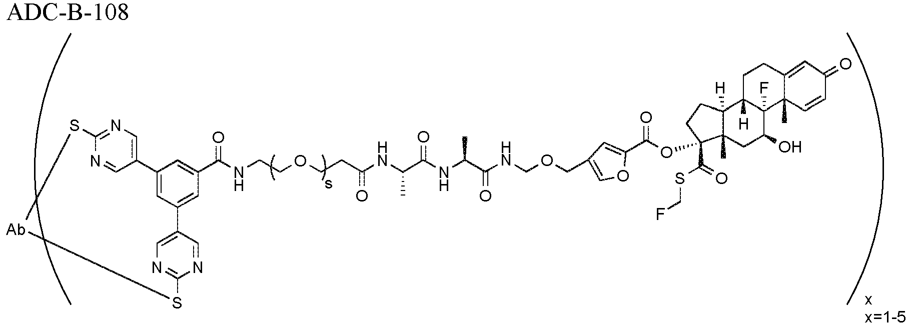 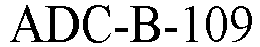 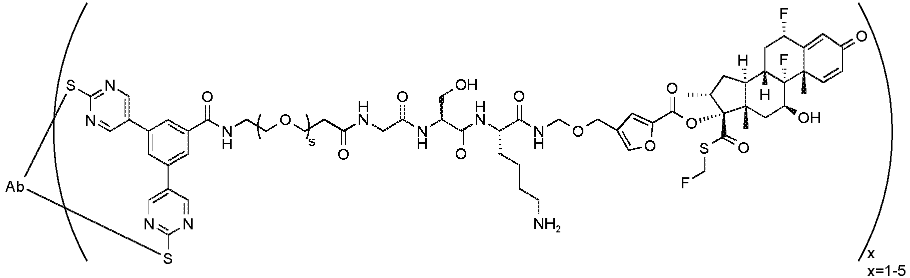 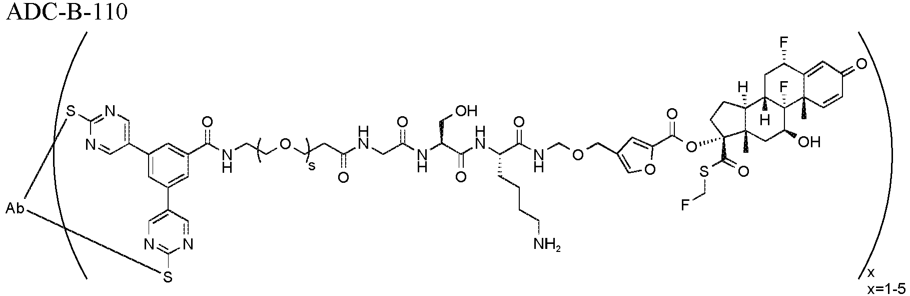 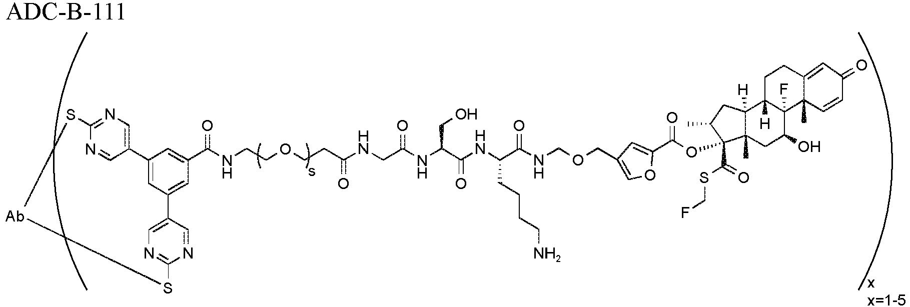 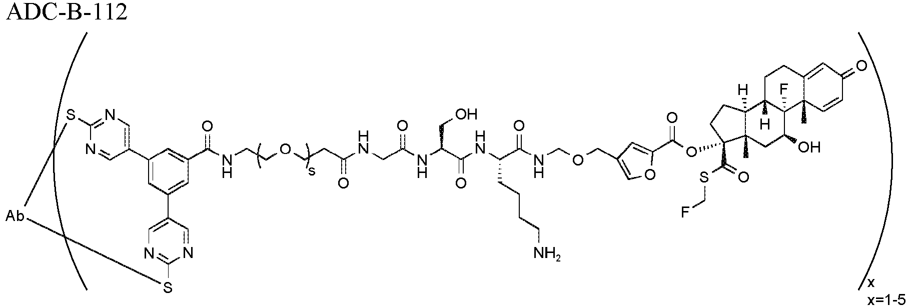 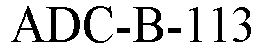 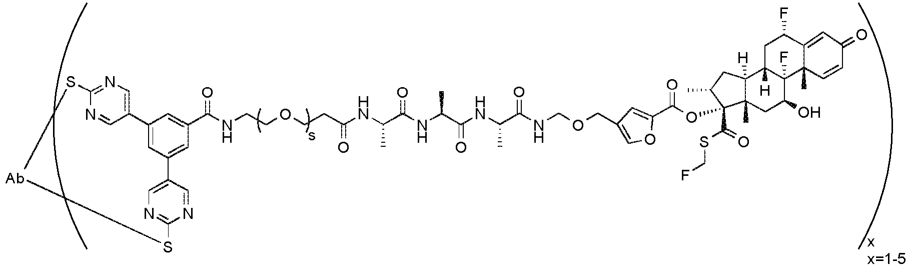 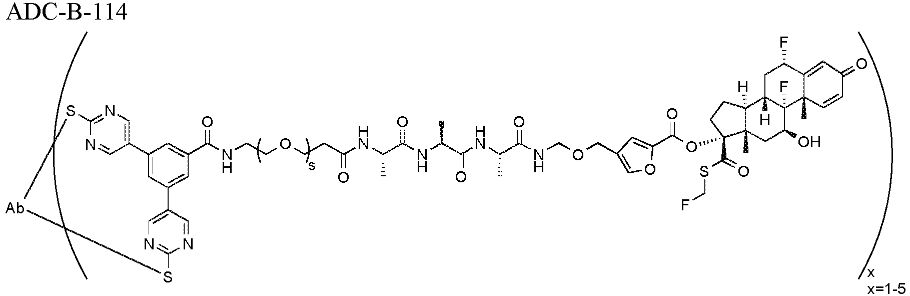 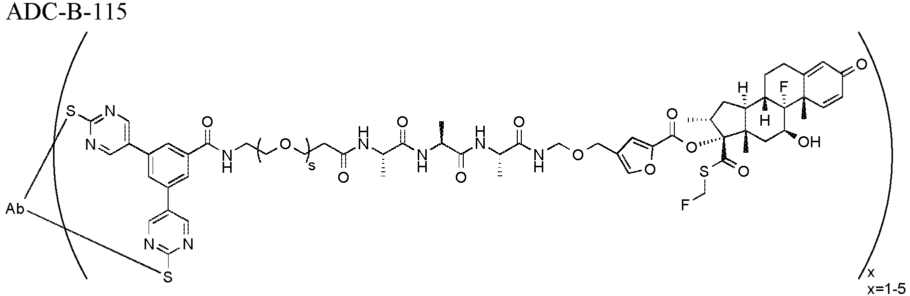 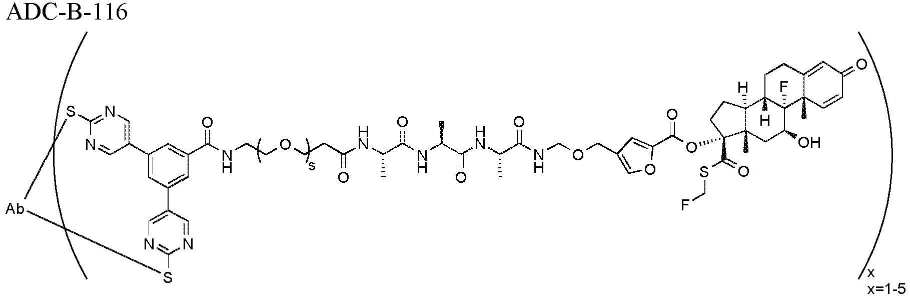 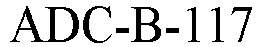 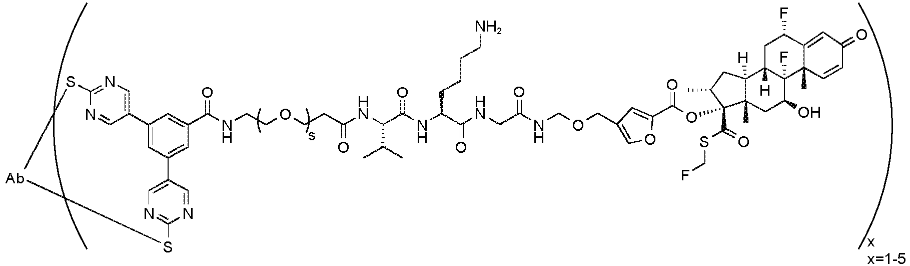 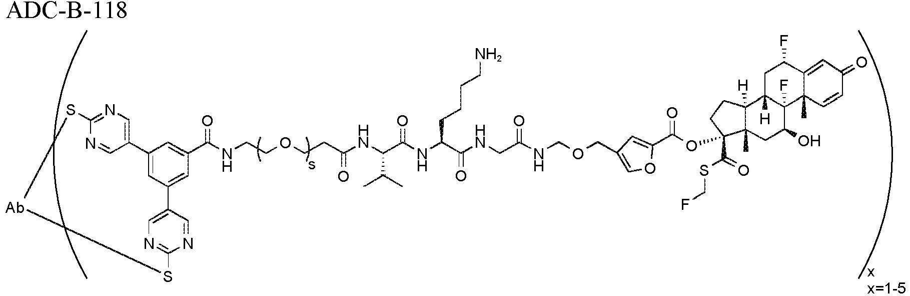 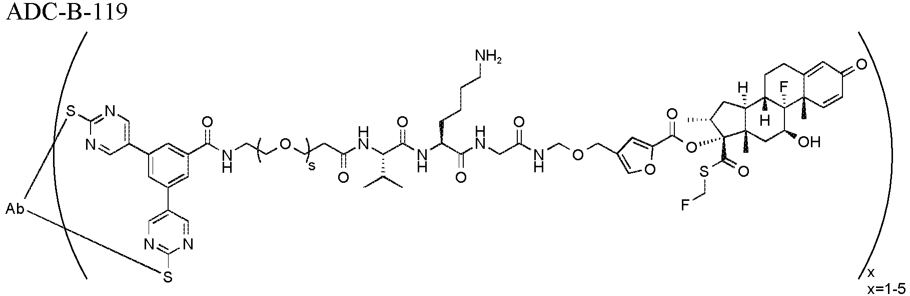 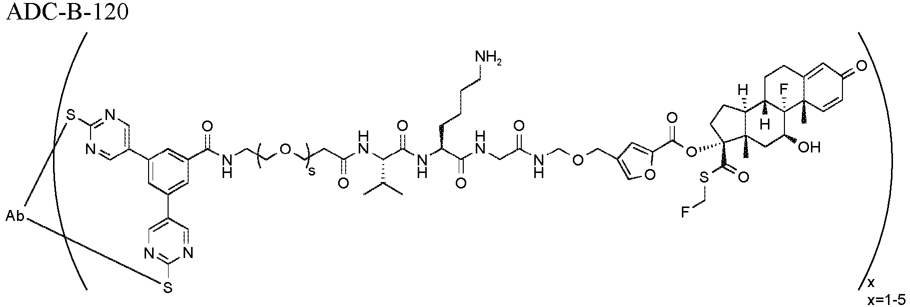 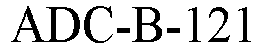 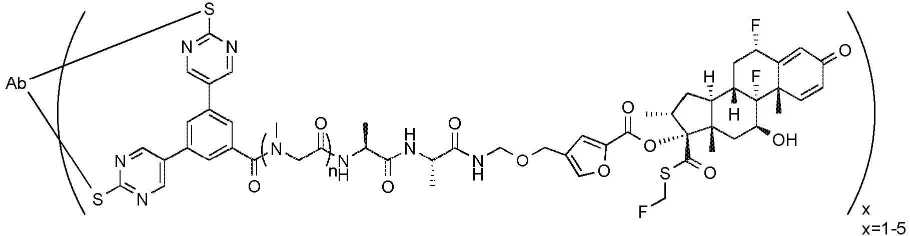 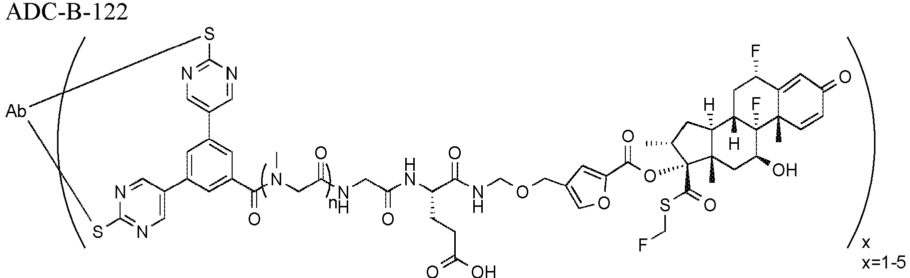 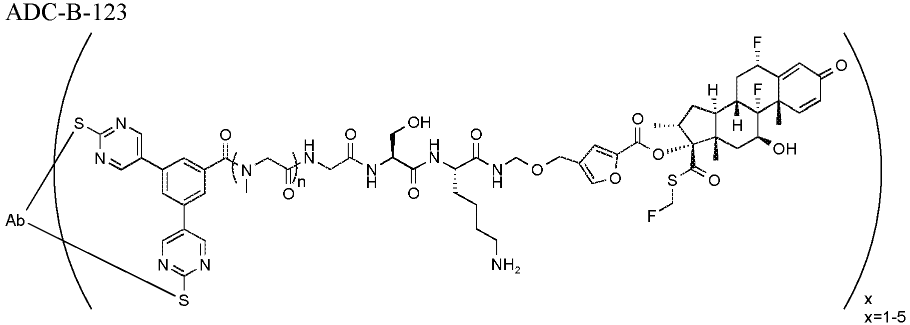 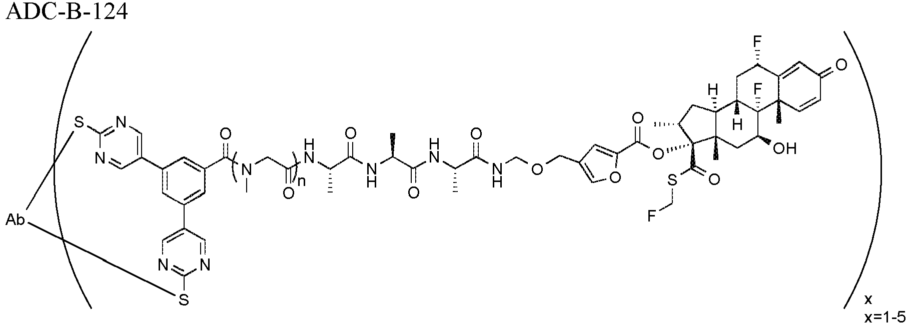 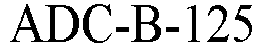 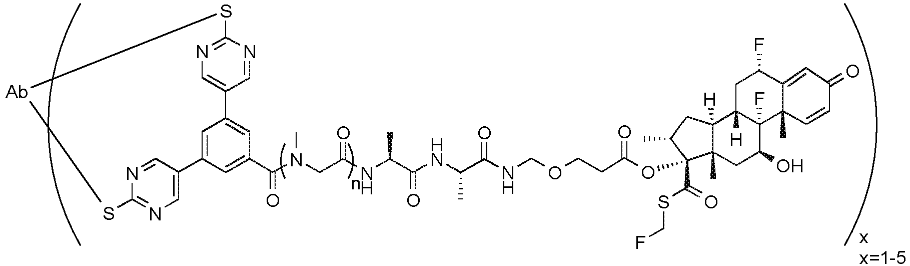 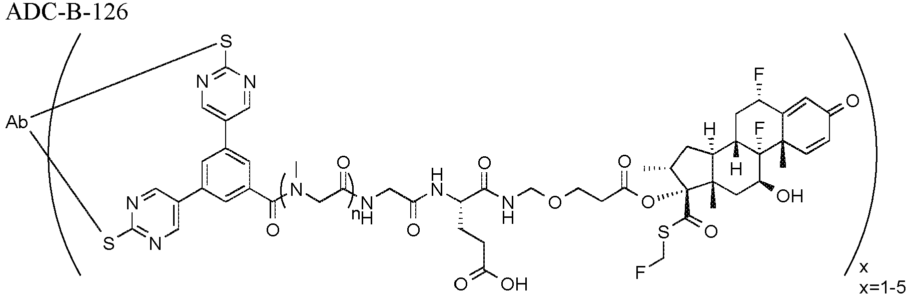 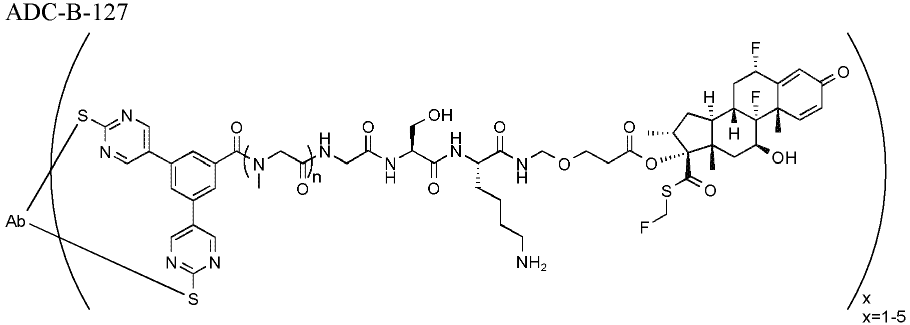 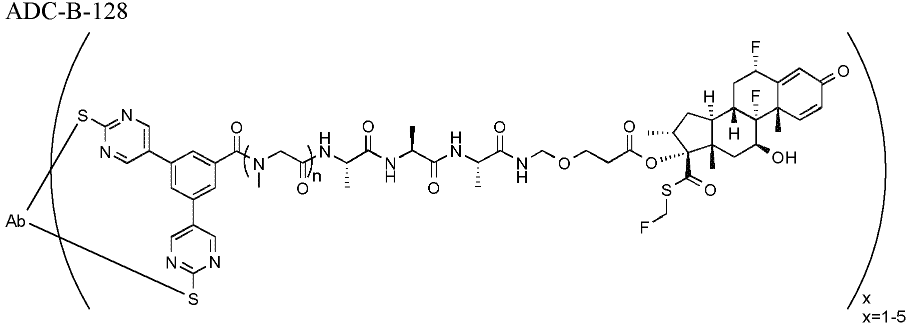 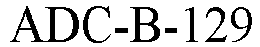 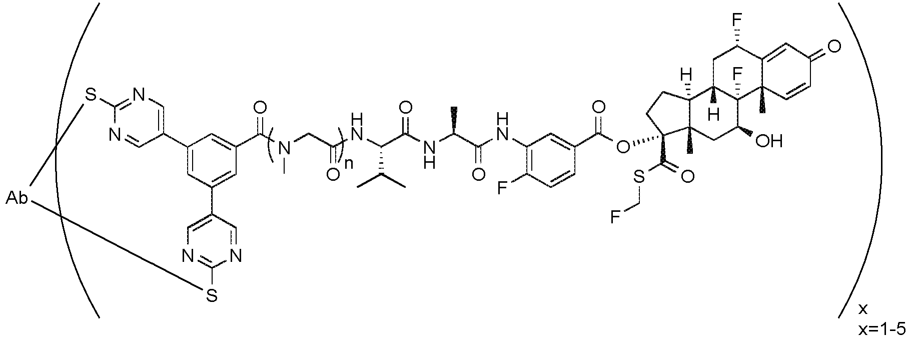 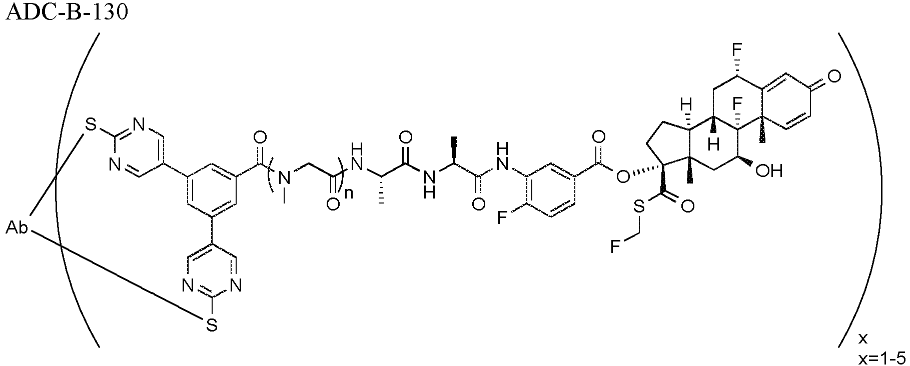 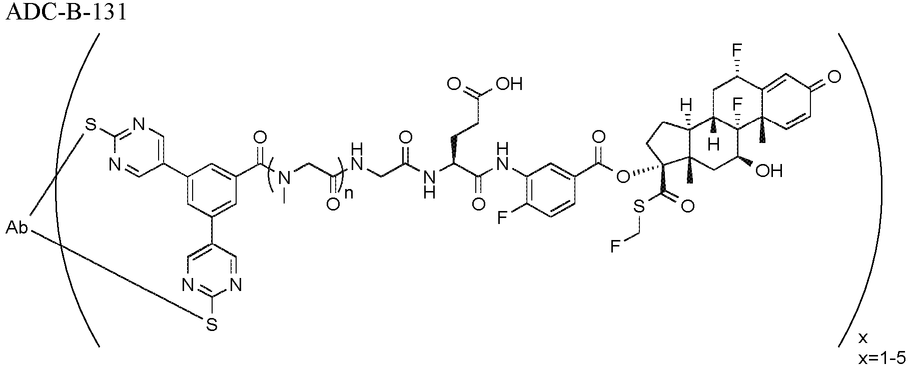 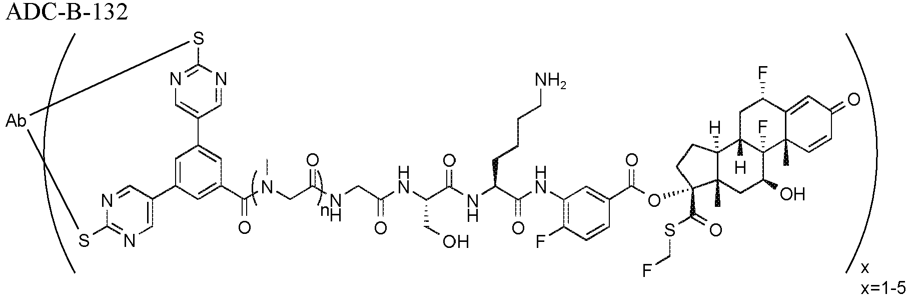 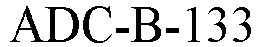 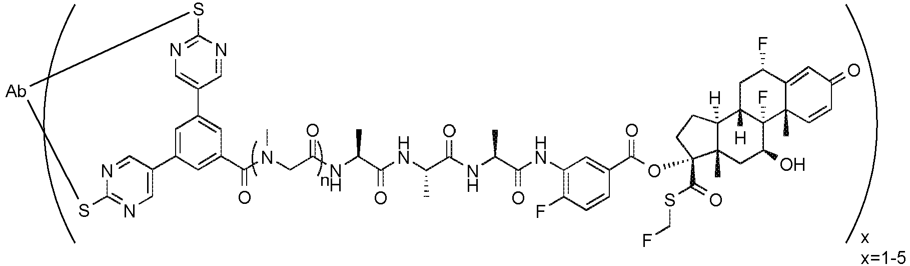 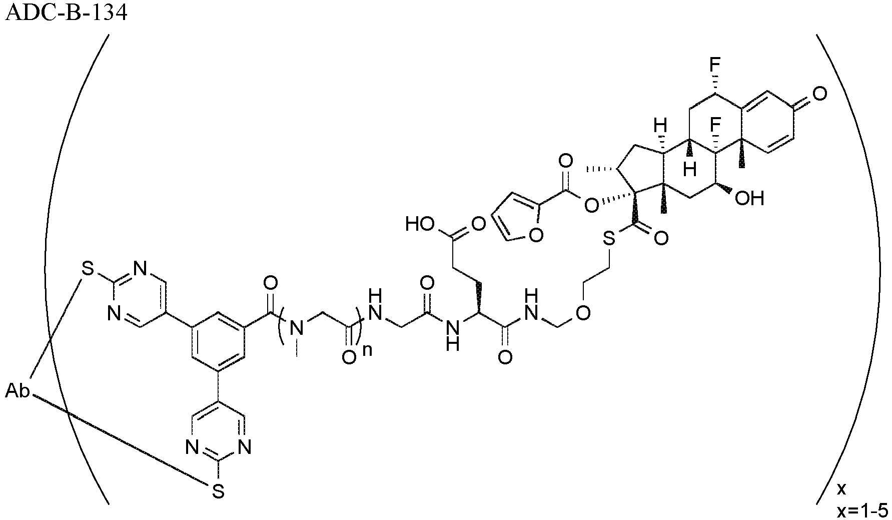 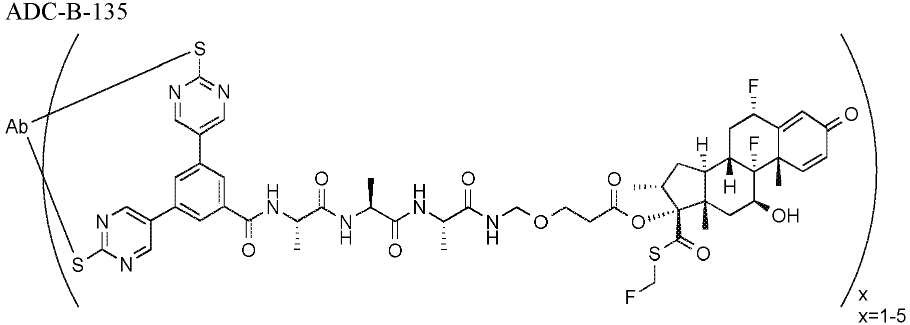 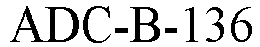 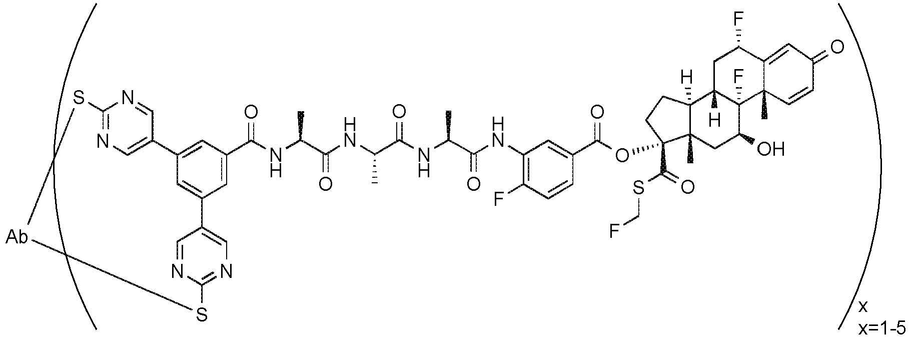 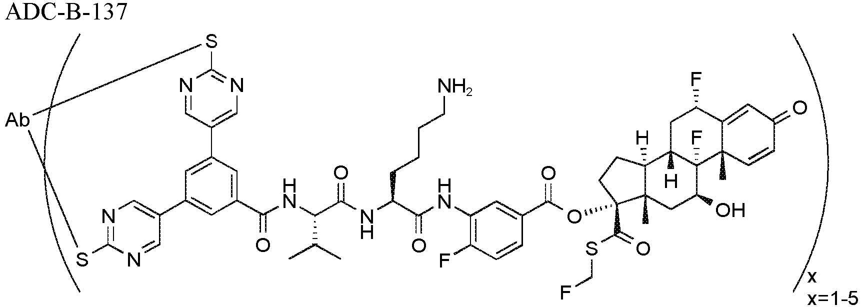 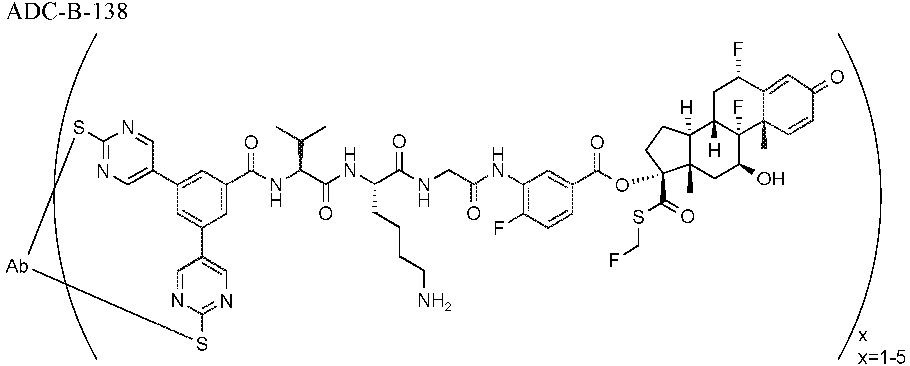 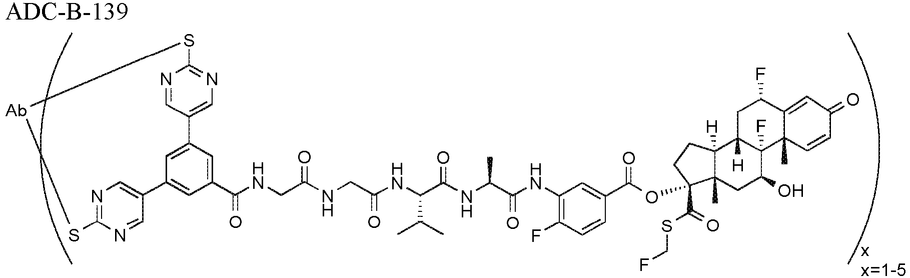 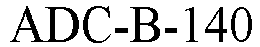 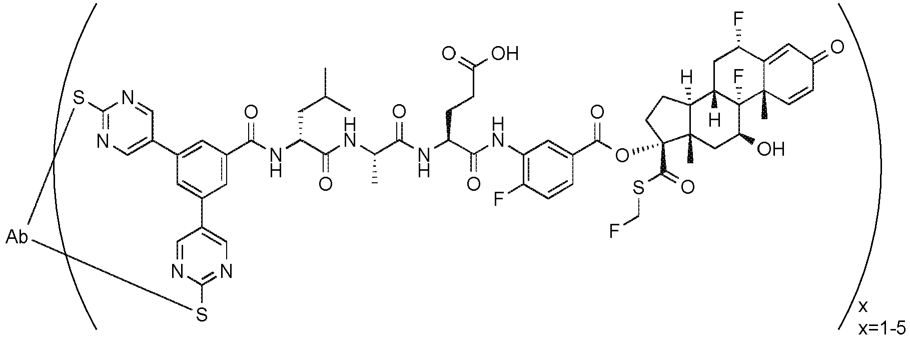 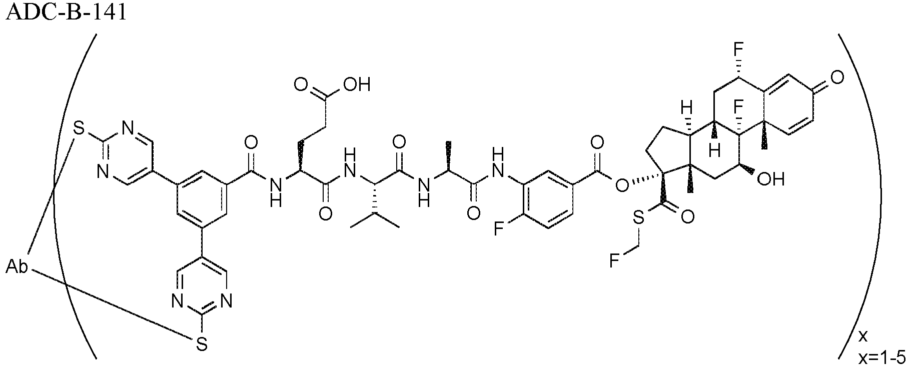 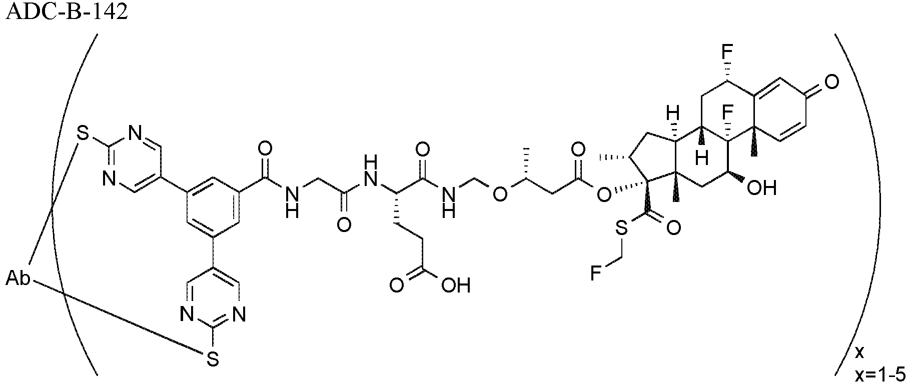 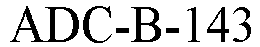 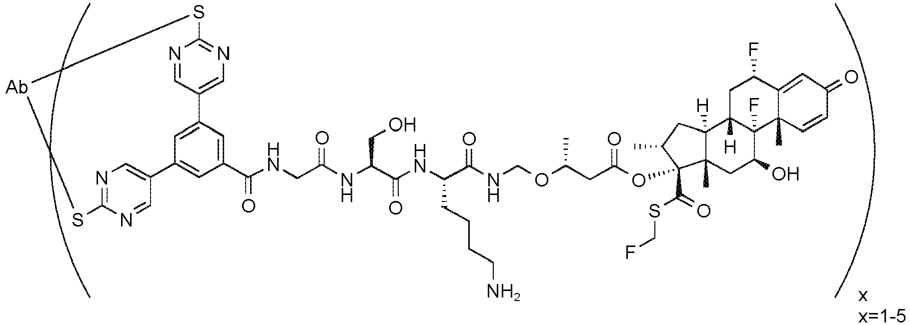 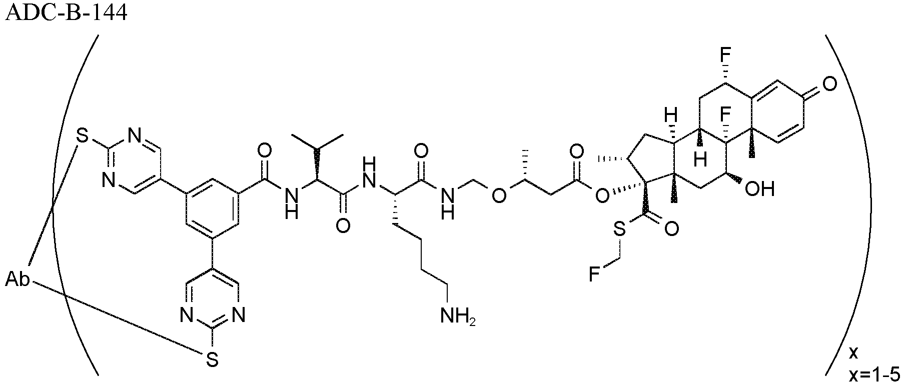 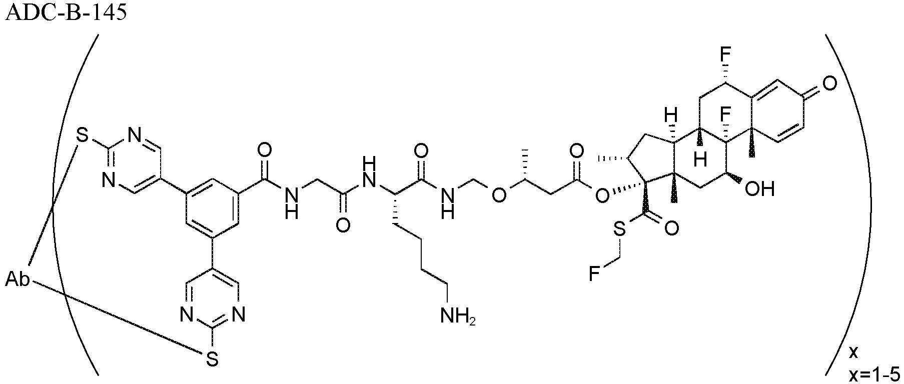 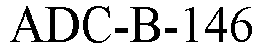 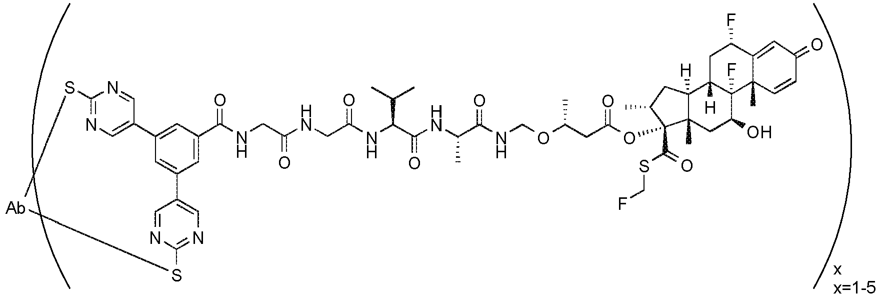
wherein, 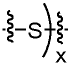 represents the specific linking manner of the mercapto in the antibody or the antigen binding fragment thereof with a linking moiety.
s is an integer selected from 1 to 20, for example 1, 2, 3, 4, 5, 6, 7, 8, 9, 10, 11, 12, 13, 14, 15, 16, 17, 18, 19 or 20; and
n is an integer selected from 0 to 20, for example 0, 1, 2, 3, 4, 5, 6, 7, 8, 9, 10, 11, 12, 13, 14, 15, 16, 17, 18, 19 or 20.

In some embodiments, Ab in the antibody-drug conjugate represents an antibody or an antigen binding fragment thereof comprising VH as shown in SEQ ID NO.1 and VL as shown in SEQ ID NO.2, for example an antibody or an antigen binding fragment thereof comprising VH as shown in SEQ ID NO.1 and CH (constant region of heavy chain) as shown in SEQ ID NO.3, and VL as shown in SEQ ID NO.2 and CL (constant region of light chain) as shown in SEQ ID NO.4.
SEQ ID NO.1
SEQ ID NO.2
SEQ ID NO.3
SEQ ID NO.4


。

In some embodiments, Ab in the antibody-drug conjugate represents an antibody or an antigen binding fragment thereof comprising VH as shown in SEQ ID NO.7 and VL as shown in SEQ ID NO.8, for example an antibody or an antigen binding fragment thereof comprising VH as shown in SEQ ID NO.7 and CH (constant region of heavy chain) as shown in SEW ID NO.9 or CH (constant region of mutant heavy chain) as shown in SEQ ID NO. 11, and VL as shown in SEQ ID NO.8 and CL (constant region of light chain) as shown in SEQ ID NO.10.
SEQ ID NO.7
SEQ ID NO.8
SEQ ID NO.9 
ASTKGPSVFPLAPSSKSTSGGTAALGCLVKDYFPEPVTVSWNSGALTSGVHTF
PAVLQSSGLYSLSSVVTVPSSSLGTQTYICNVNHKPSNTKVDKKVEPKSCDKTHTC
PPCPAPELLGGPSVFLFPPKPKDTLMISRTPEVTCVVVDVSHEDPEVKFNWYVDG
VEVHNAKTKPREEQYNSTYRVVSVLTVLHQDWLNGKEYKCKVSNKALPAPIEKT
ISKAKGQPREPQVYTLPPSREEMTKNQVSLTCLVKGFYPSDIAVEWESNGQPENN
YKTTPPVLDSDGSFFLYSKLTVDKSRWQQGNVFSCSVMHEALHNHYTQKSLSLSP
GK
SEQ ID NO.10 

。
SEQ ID NO.11 (constant region of mutant heavy chain)

ASTKGPSVFPLAPSSKSTSGGTAALGCLVKDYFPEPVTVSWNSGALTSGVHTF
PAVLQSSGLYSLSSVVTVPSSSLGTQTYICNVNHKPSNTKVDKKVEPKSCDKTHTC
PPCPAPEAAGAPSVFLFPPKPKDTLMISRTPEVTCVVVDVSHEDPEVKFNWYVDG
VEVHNAKTKPREEQYNSTYRVVSVLTVLHQDWLNGKEYKCKVSNKALPAPIEKT
ISKAKGQPREPQVYTLPPSREEMTKNQVSLTCLVKGFYPSDIAVEWESNGQPENN
YKTTPPVLDSDGSFFLYSKLTVDKSRWQQGNVFSCSVMHEALHNHYTQKSLSLSP
GK.

In some embodiments, the DAR value (the drug-to-antibody conjugating ratio) of the antibody-drug conjugate is 1-10, for example 1-2, 1-3, 1-4, 1-5, 1-6, 1-7, 1-8, 1-9, 1-10, 2-3, 2-4, 2-5, 2-6, 2-7, 2-8, 2-9, 2-10, 3-4, 3-5, 3-6, 3-7, 3-8, 3-9, 3-10, 4-5, 4-6, 4-7, 4-8, 4-9, 4-10, 5-6, 5-7, 5-8, 5-9, 5-10, 6-7, 6-8, 6-9, 6-10, 7-8, 7-9, 7-10, 8-9, 8-10, or 9-10, preferably 3-9, for example 3.0-3.5, 3.0-4.0, 3.0-4.5, 3.0-5.0, 3.0-5.5, 3.0-6.0, 3.5-4.0, 3.5-4.5, 3.5-5.0, 3.5-5.5, 3.5-6.0, 3.5-6.5, 3.5-7.0, 3.5-7.5, 3.5-8.0, 4.0-4.5, 4.0-5.0, 4.0-5.5, 4.0-6.0, 4.0-6.5, 4.0-7.0, 4.0-7.5, 4.0-8.0, 4.5-5.0, 4.5-5.5, 4.5-6.0, 4.5-6.5, 4.5-7.0, 4.5-7.5, 4.5-8.0, 5.0-5.5, 5.0-6.0, 5.0-6.5, 5.0-7.0, 5.0-7.5, 5.0-8.0, 5.5-6.0, 5.5-6.5, 5.5-7.0, 5.5-7.5, 5.5-8.0, 6.0-6.5, 6.0-7.0, 6.0-7.5, 6.0-8.5, 6.5-7.0, 6.5-7.5, 6.5-8.5, 7.0-7.5, 7.0-9.0 or 7.5-9.0.

In all the above embodiments, the wavy line 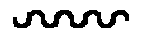 indicates the point of attachment between the moiety and the remainder of the molecule.

### Drug-linker compound

Those skilled in the art should understand that the antibody-drug conjugate of the present application can be modularly prepared. For example, a "drug-linker" in a free form (which can be understood as G-M-[L-E-D]ₓ, wherein G-M is a structural form before covalent linking to the antibody or the antigen binding fragment thereof, and x is an integer selected from 1 to 10) is obtained, and then subjected to covalent linking with the antibody or the antigen binding fragment thereof to obtain the antibody-drug conjugate of the present application. Correspondingly, G-M in the free form of the "drug-linker" is linked with one or more mercapto (-SH), amino (-NH₂) or carboxyl (-COOH) on the antibody or the antigen binding fragment thereof through substitution reaction (for example, a structure such as - SO₂Me or -Br is removed thereon) or through addition reaction, or the like.

In another aspect, the present invention provides a drug-linker compound having a structure represented by formula G-M-[L-E-D]ₓ, wherein:
G is a functional group or a leaving group which reacts with a specific amino acid or glycosyl and a derivative thereof in an antibody or an antigen binding fragment;
M is a linkage site to the antibody or the antigen binding fragment thereof;
L is a linker between the linkage site M and E;
E is a structural fragment linking L with D;
D is a glucocorticoid drug fragment which is a monovalent structure obtained by losing one H from -OH, -NH₂ or secondary amino on the steroid glucocorticoid receptor agonist of the present invention; and
x is an integer selected from 1 to 10, preferably 1 or 2.

In some embodiments, M, L, E, D and x are as defined above in the section "antibody-drug conjugate".

In some embodiments, G is selected from a group consisting of halogen, halophenoxy, C₁₋₆ haloalkyl, sulfonate (HOS(=O)₂-), C₁₋₆ alkylsulfonyl, C₁₋₆ haloalkylsulfonyl, halosulfonyl, C₁₋₆ alkyl sulfonate, C₁₋₆ haloalkyl sulfonate, C₁₋₆ alkyl sulfite, halosulfonate, C₁₋₆ alkyl sulfoxide, methylsulfonyl methacryloyl, dimethylsulfonyl methacryloyl 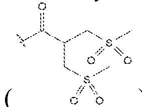 ), haloforml, haloacetyl, formyl, acetyl, nitro, azido, cyano, cyanovinyl, N-methyl-vinylsulfonamido 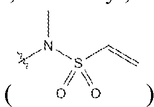 , tetrazinyl, methyl tetrazinyl, trans-cyclooctenyl carbonate group , C₂₋₆ alkenyl, C₂₋₆ alkynyl, benzazacyclooctynyl, (1R,8S,9S)-bicyclo[6.1.0]non-4-yn-9-ylmethoxy.

In some embodiments, G-M is 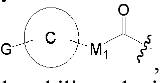 wherein,
G is a leaving group for nucleophilic substitution reaction, for example, halogen, methylsulfonyl, haloacetyl, fluorophenoxy or 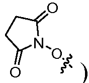 ; or G is a group for addition reaction or cyclization reaction, for example methylsulfonyl methacryloyl, cyanovinyl, N-methyl-vinylsulfonamido, azido, tetrazinyl, methyl tetrazinyl, trans-cyclooctenyl carbonate group, C₂₋₆ alkynyl, benzazacyclooctynyl, and (1R,8S,9S)-bicyclo[6.1.0]non-4-yn-9-ylmethoxy.

Ring C is a 5-6 membered aliphatic heterocyclic ring or a 5-20 membered aromatic ring system, the aliphatic heterocyclic ring or the aromatic ring system being optionally substituted with one or more groups each independently selected from a group consisting of oxo (=O), halogen, cyano, amino, carboxyl, mercapto and C₁₋₆ alkyl; and
M₁ is selected from a group consisting of a single bond or a fragment consisting of one or more groups selected from: -O-, -NH-, -C(=O)-, -S(=O)₂-, -C=N-O-, -NH-S(=O)₂-NH-, phenylene, 5-10 membered heteroarylidene, C₁₋₂₀ alkylene, C₂₋₂₀ alkenylidene and C₂₋₂₀ alkynylidene.

In some embodiments, G-M is 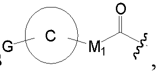 wherein,
G is methylsulfonyl or sulfonate;
ring C is a 5 membered aliphatic heterocyclic ring, a 6 membered heteroaromatic ring or a polycyclic ring formed by 2-5 (preferably 3) units selected from 6 membered heteroaromatic ring and a benzene ring linked through a single bond, the aliphatic heterocyclic ring, heteroaromatic ring or polycyclic ring being optionally substituted with one or more groups selected from a group consisting of oxo (=O), halogen and C₁₋₄ alkyl; and
M₁ is selected from a group consisting of a single bond, -O-, -NH-, -NH-S(=O)₂-NH-, C₁₋₁₀ alkylene, C₂₋₁₀ alkenylidene and C₂₋₁₀ alkynylidene.

In some embodiments, G-M is 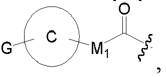 wherein, 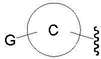
is selected from a group consisting of 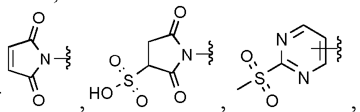 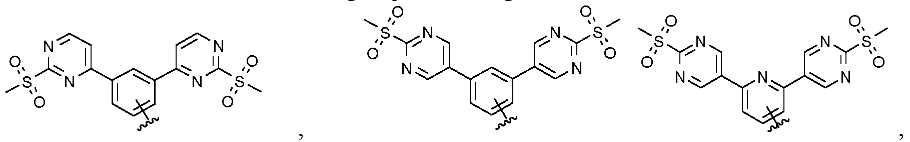 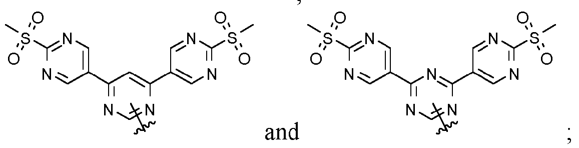 and
M₁ is selected from a group consisting of a single bond, -O-, -NH-, -NH-S(=O)₂-NH-, C₁₋₁₀ alkylene, C₂₋₆ alkenylidene and C₂₋₆ alkynylidene.

In some embodiments, G-M is

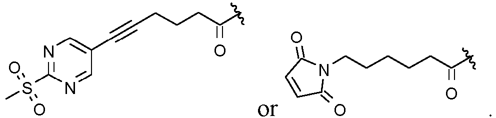

In some embodiments, G-M is

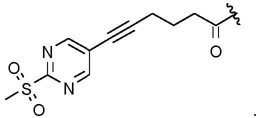

In some embodiments, G-M is

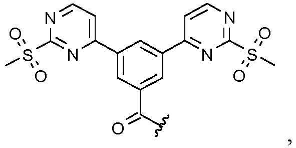

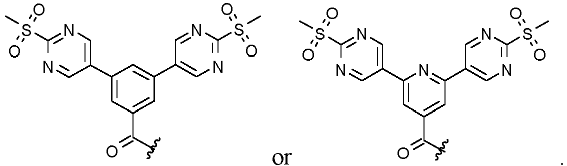

In some embodiments, G-M is 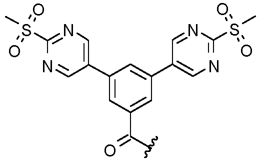 or

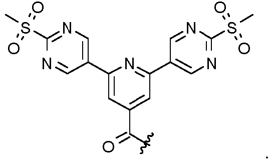

In some embodiments, the "drug-linker" compound in a free form is selected from a group consisting of: 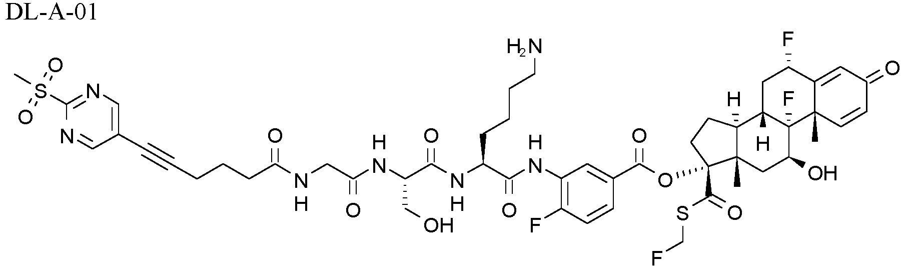 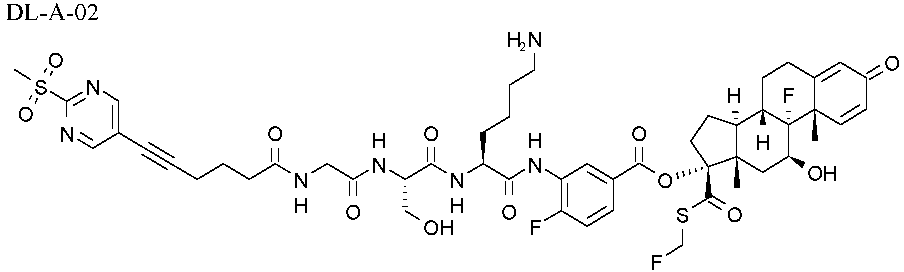 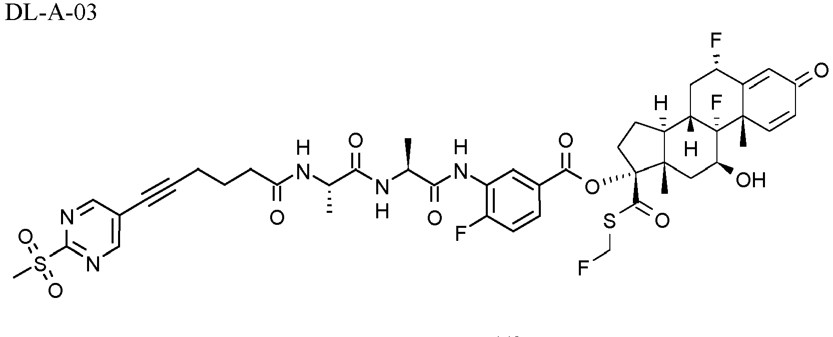 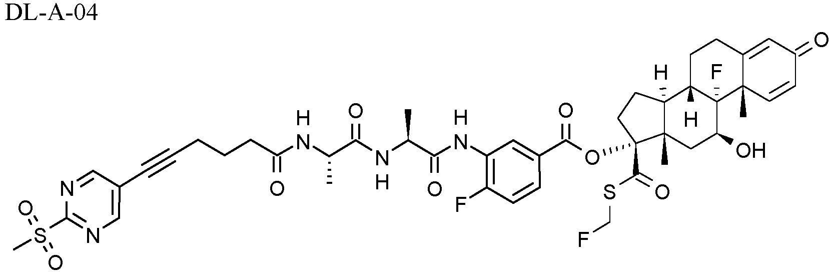 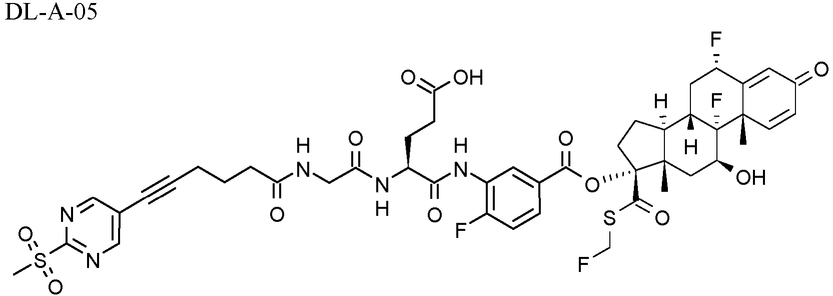 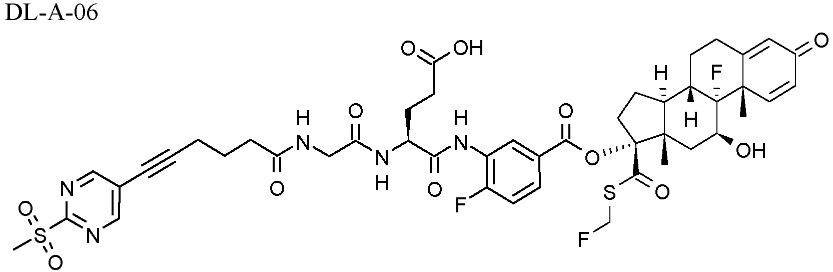 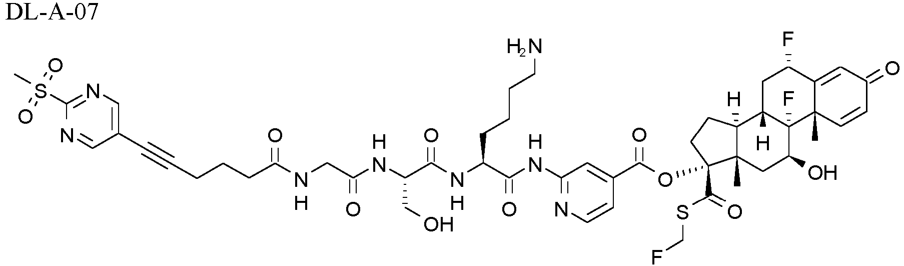 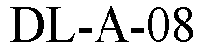 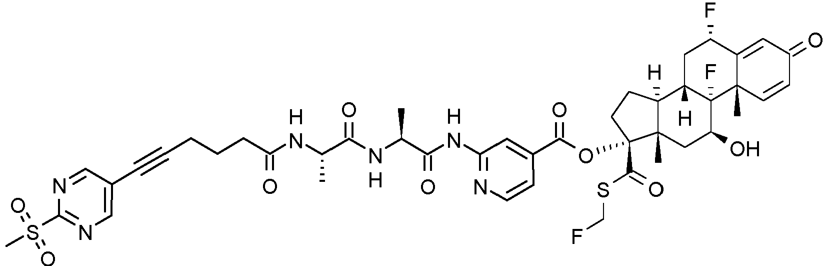 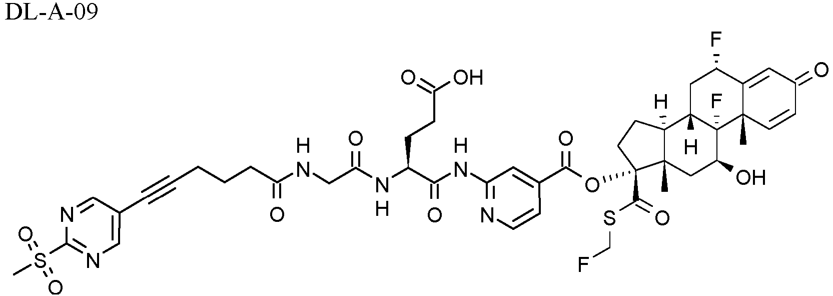 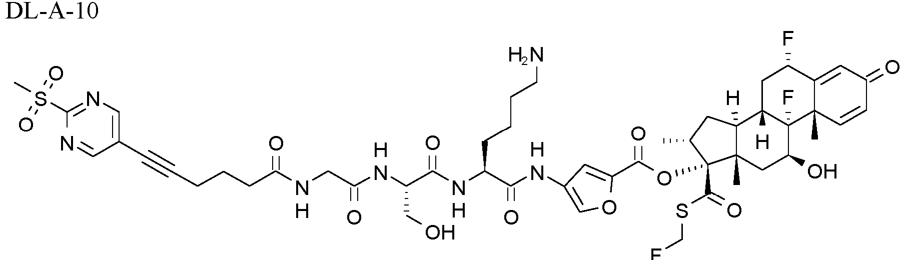 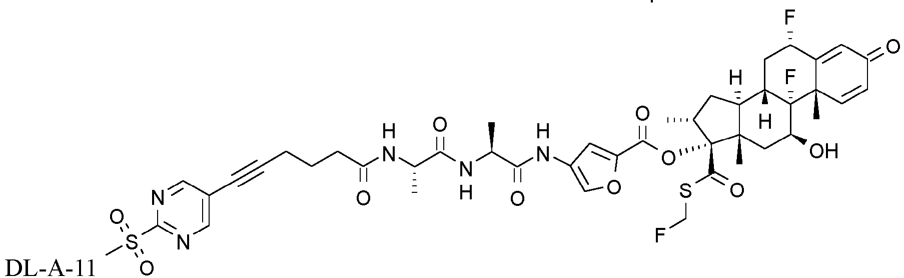 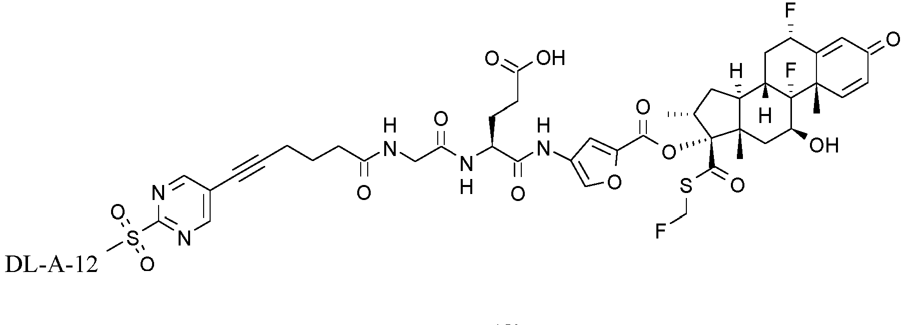 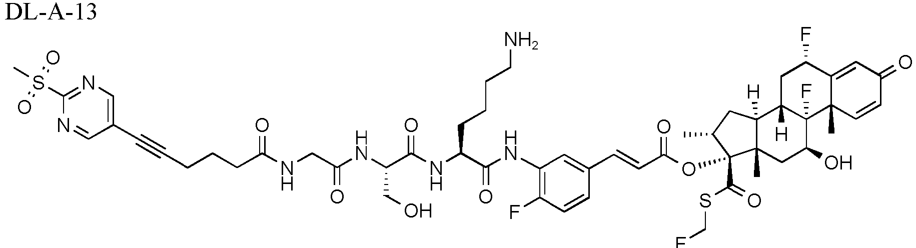 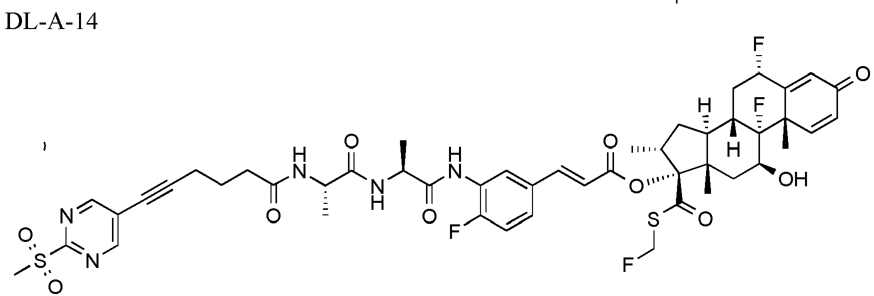 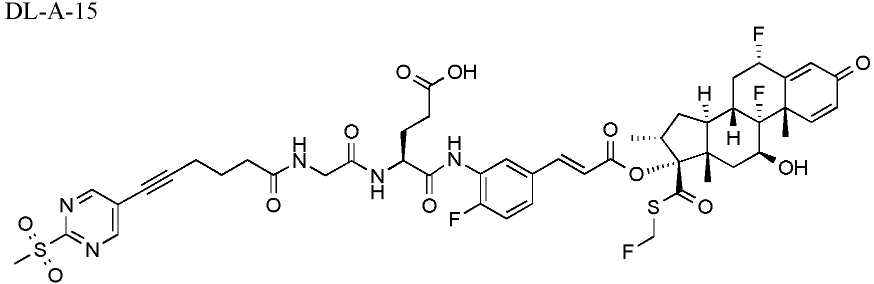 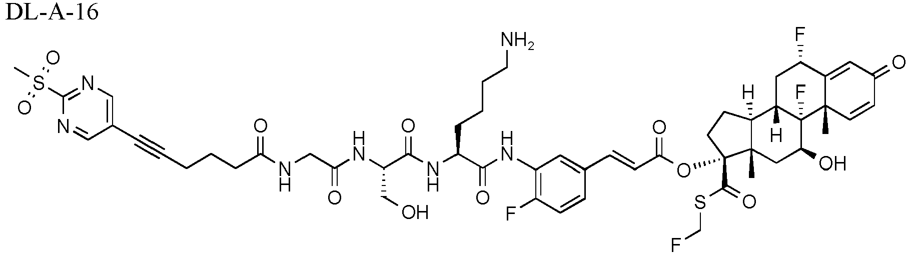 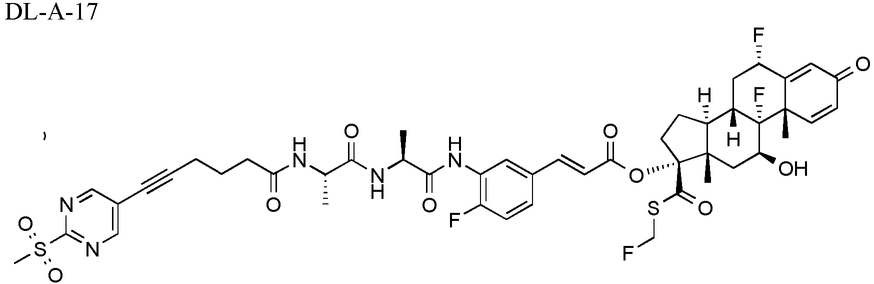 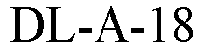 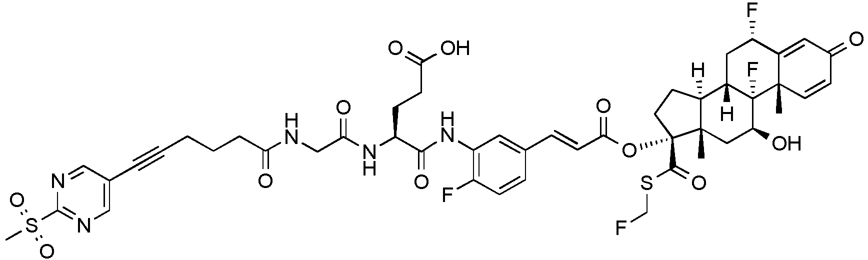 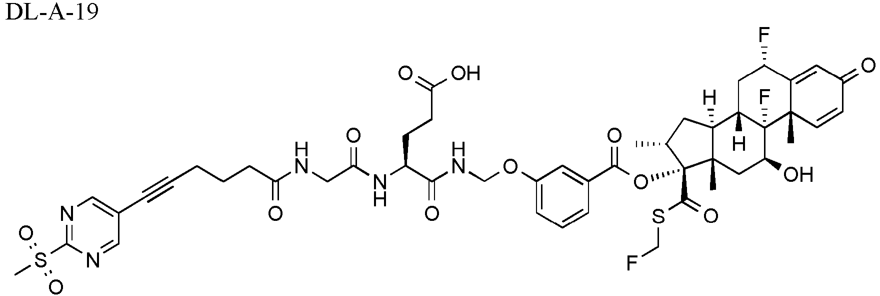 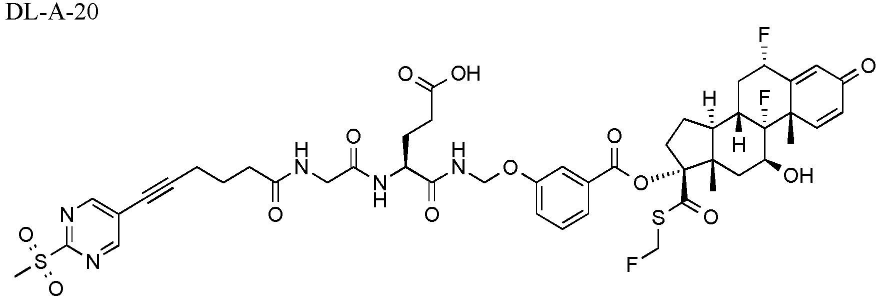 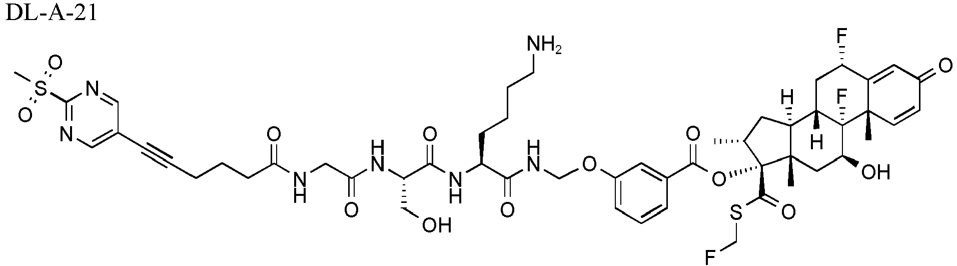 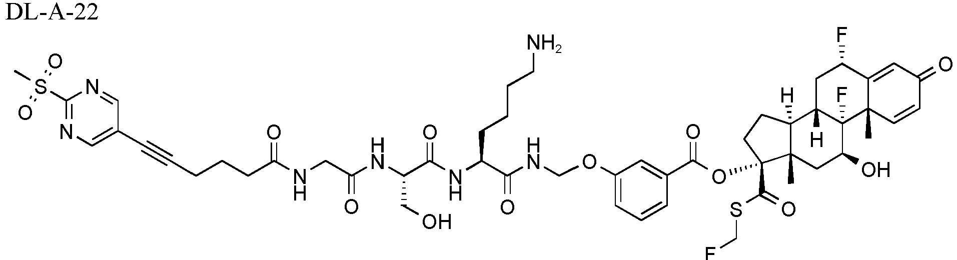 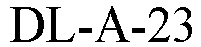 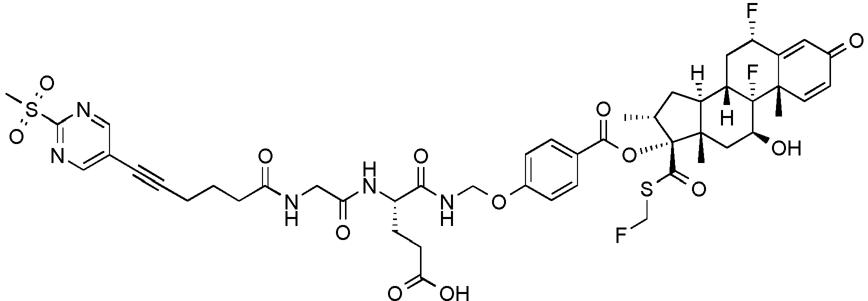 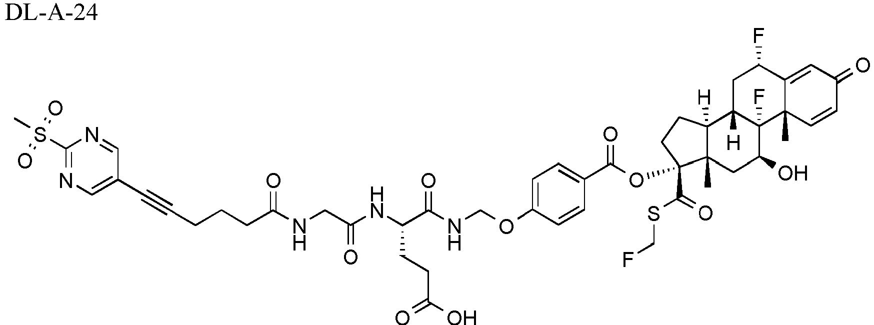 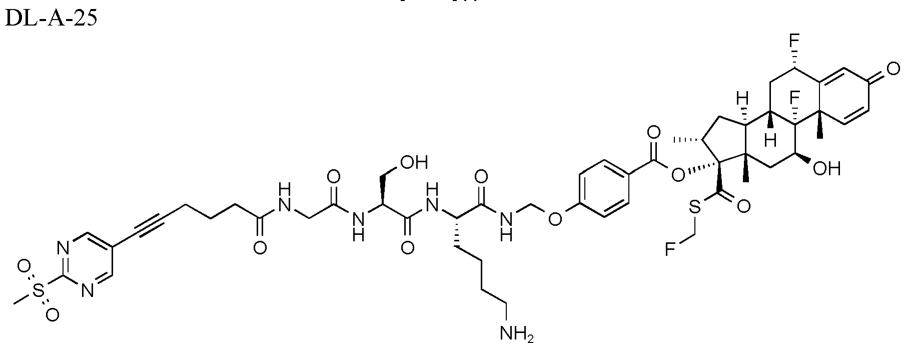 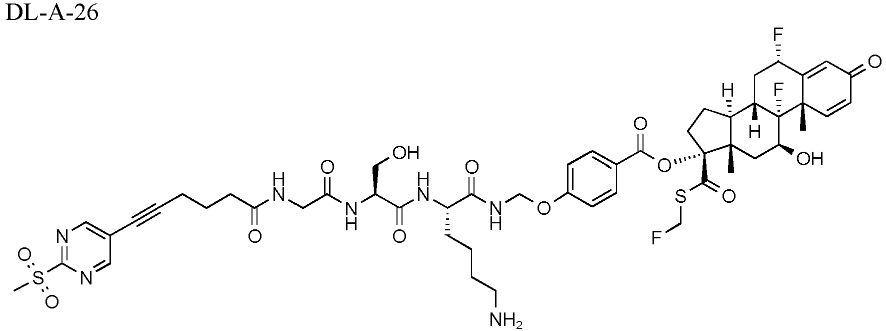 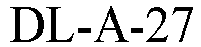 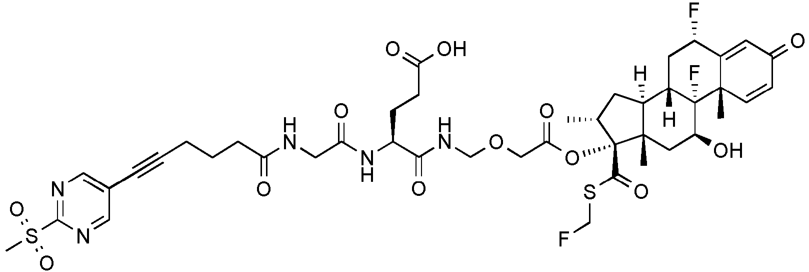 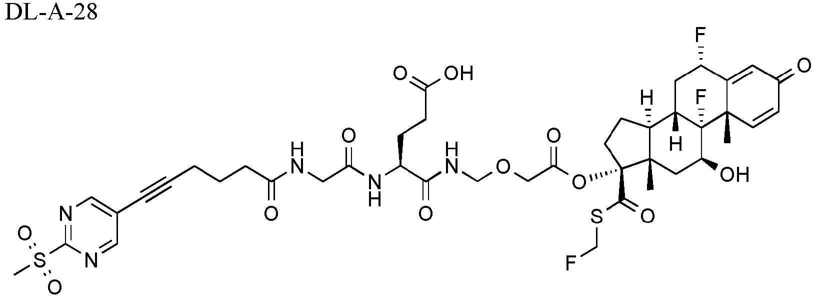 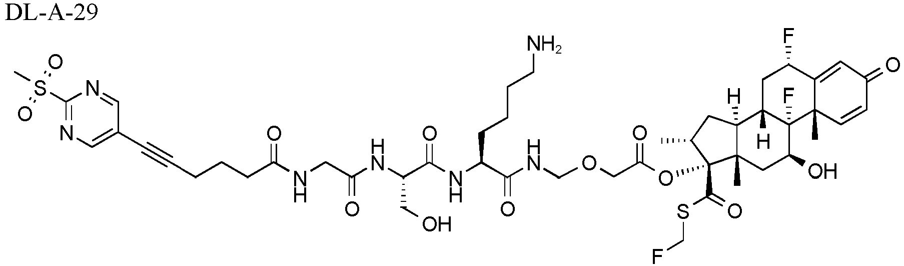 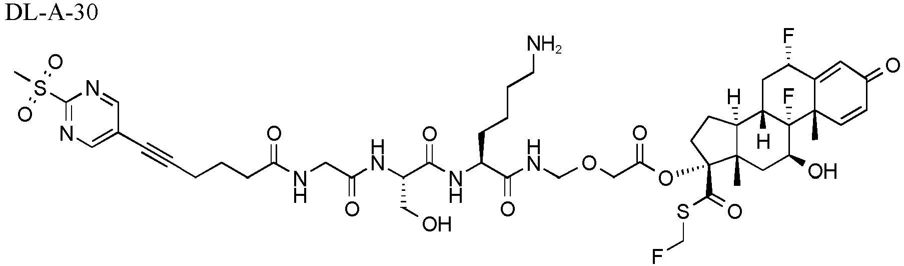 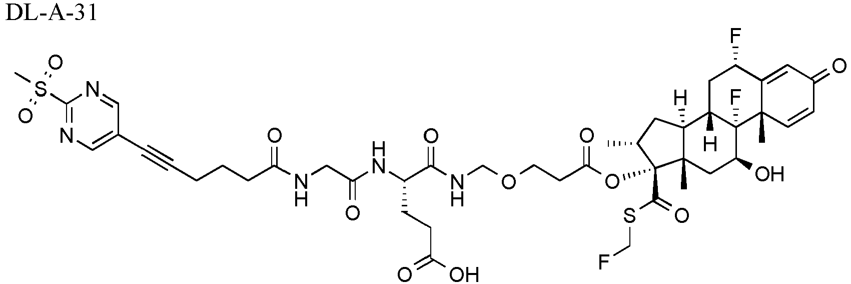 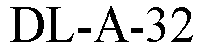 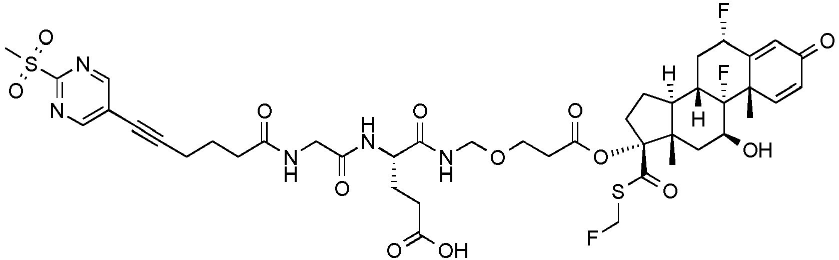 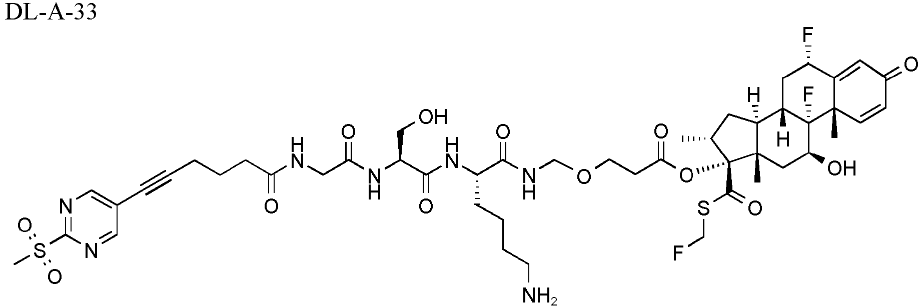 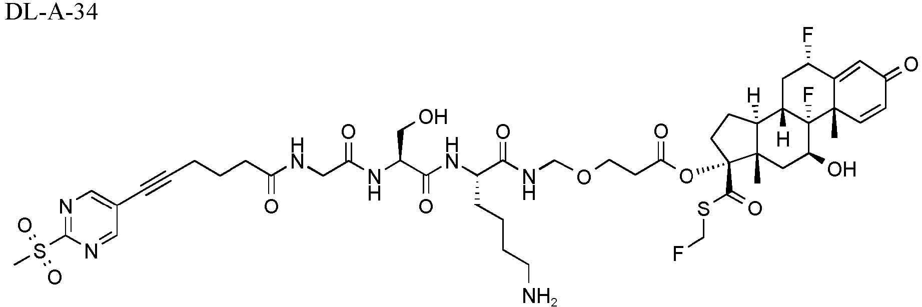 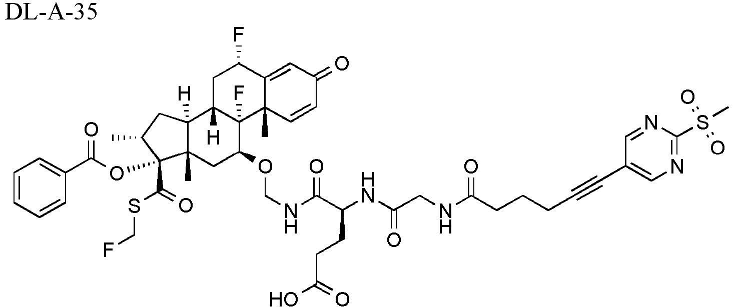 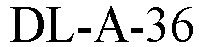 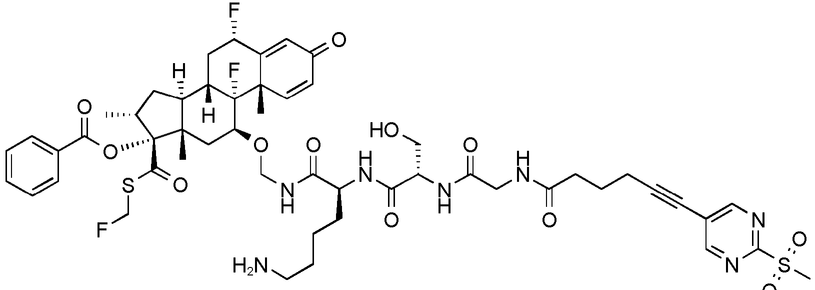 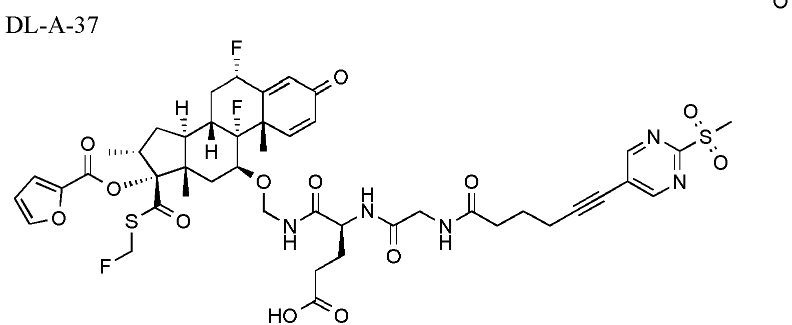 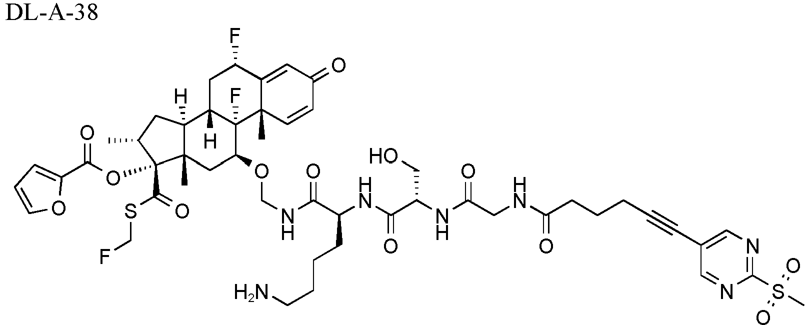 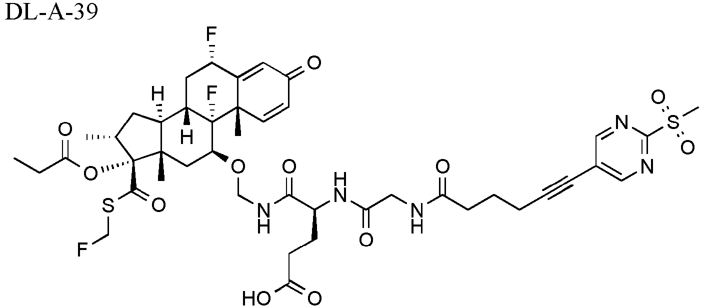 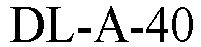 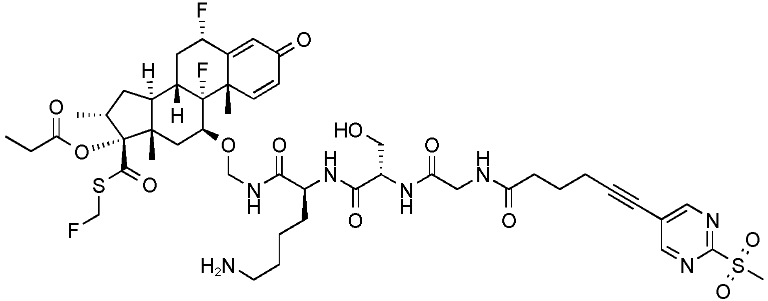 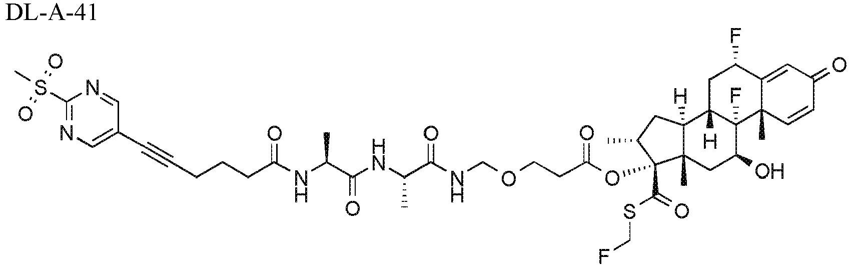 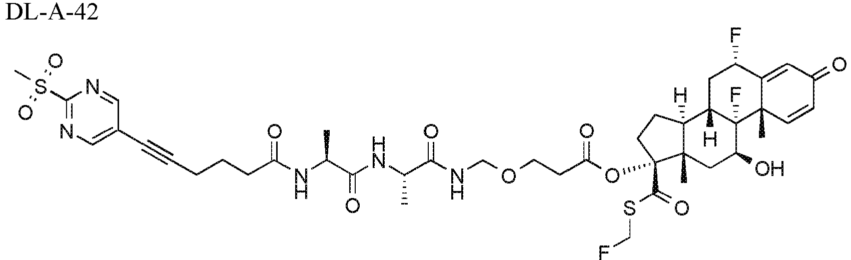 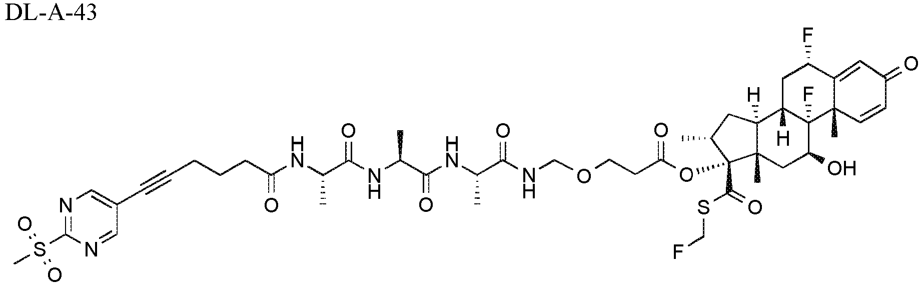 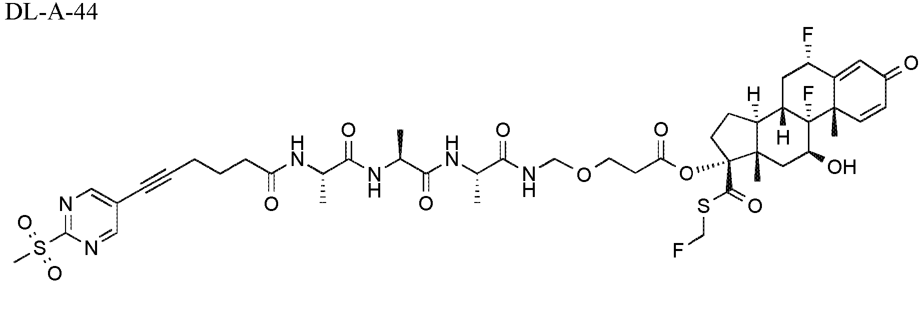 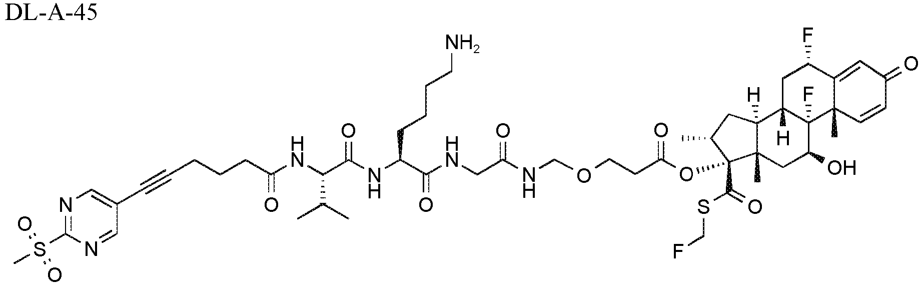 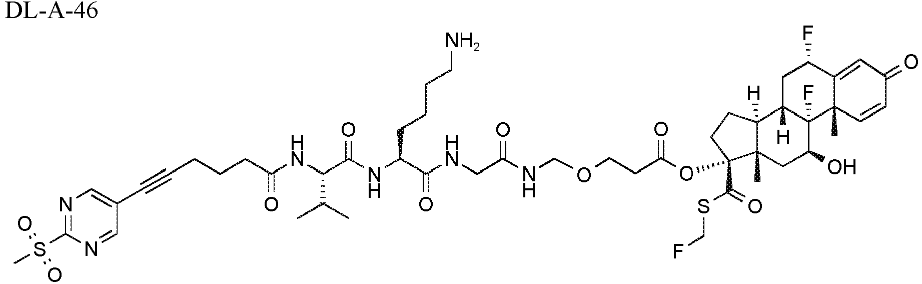 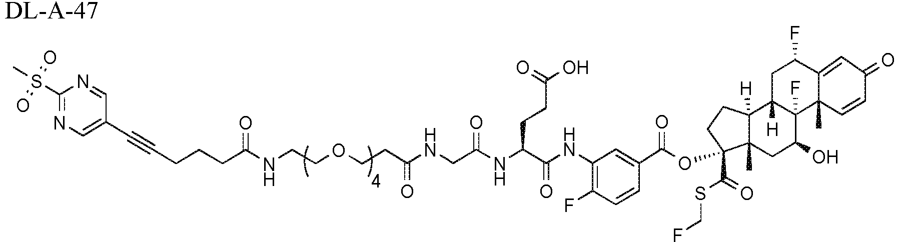 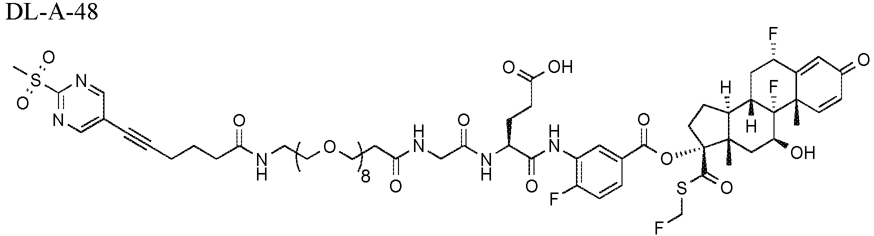 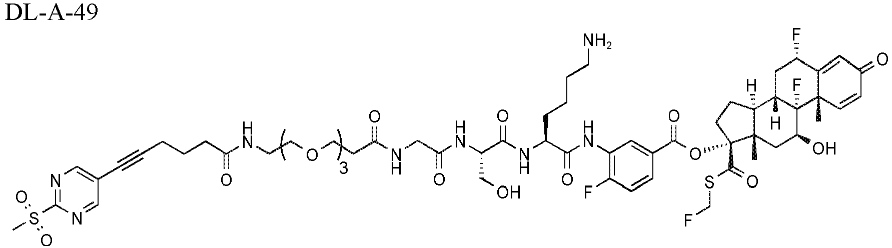 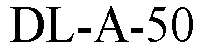 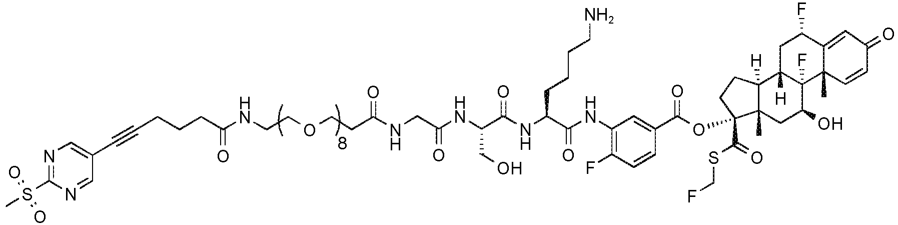 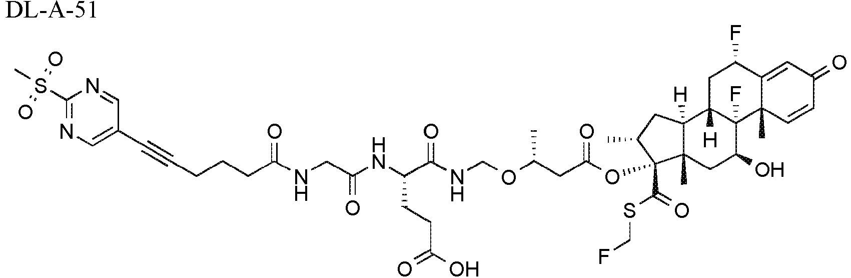 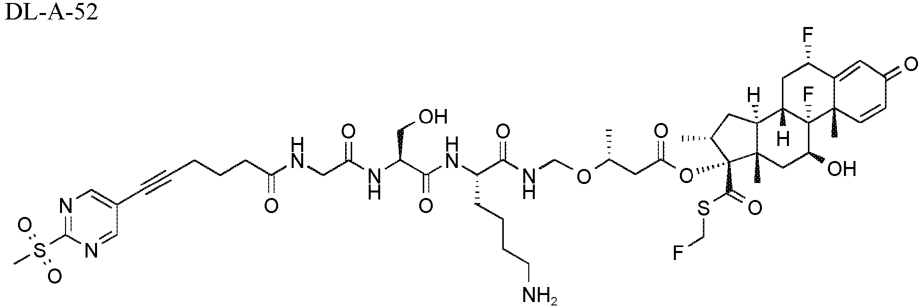 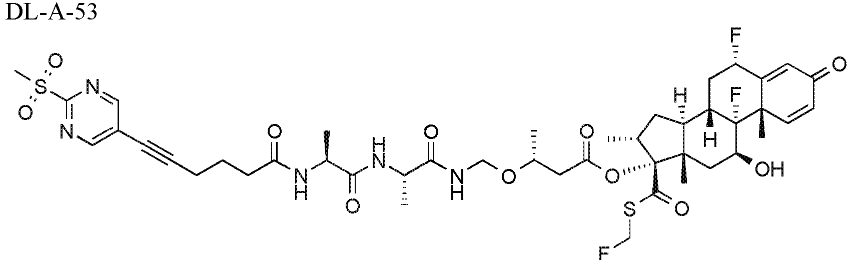 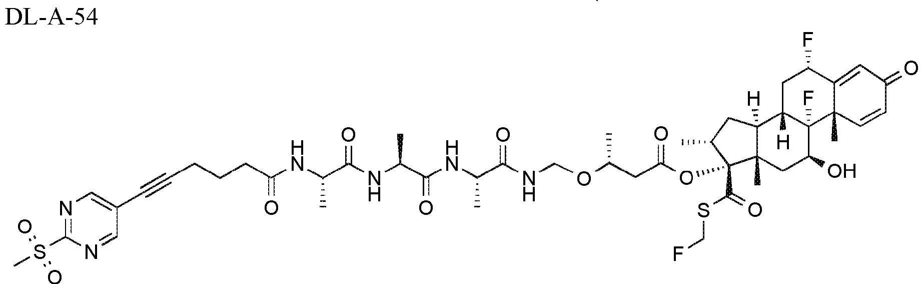 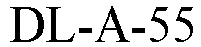 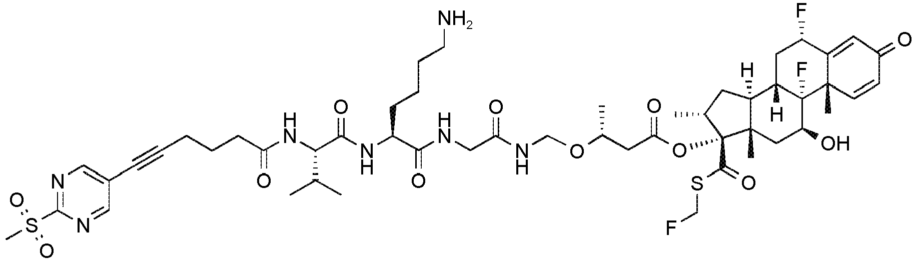 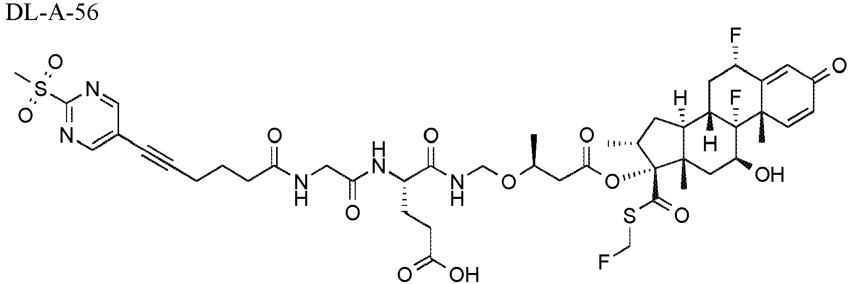 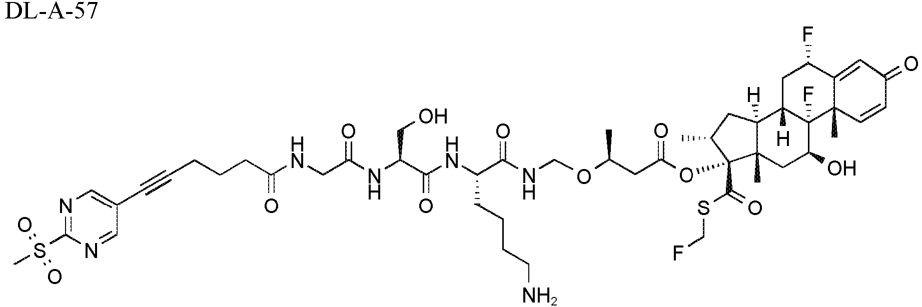 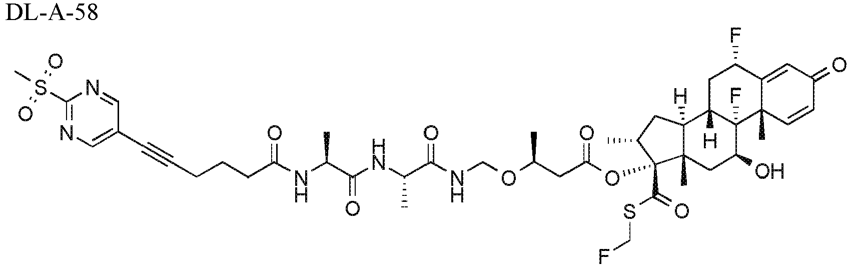 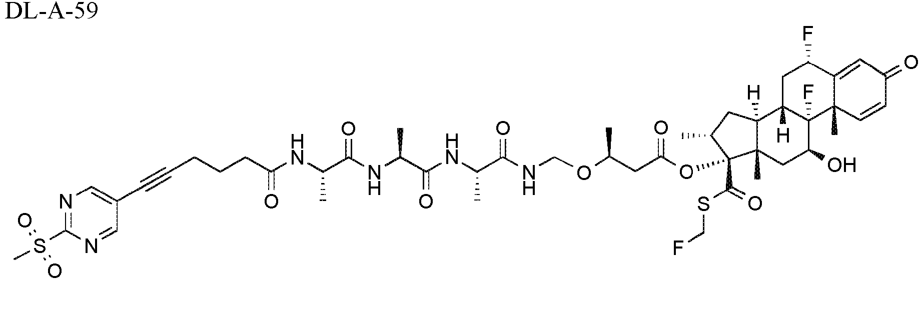 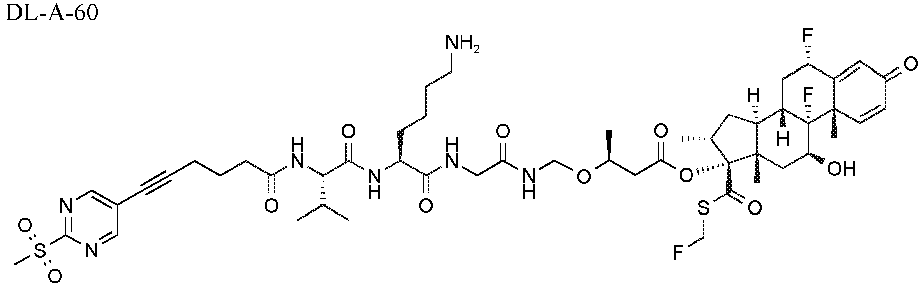 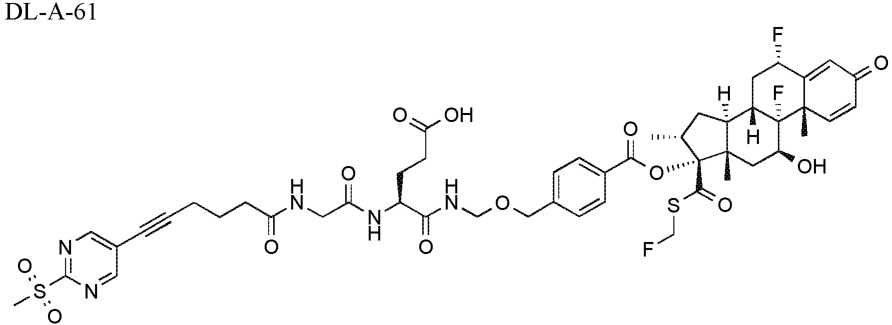 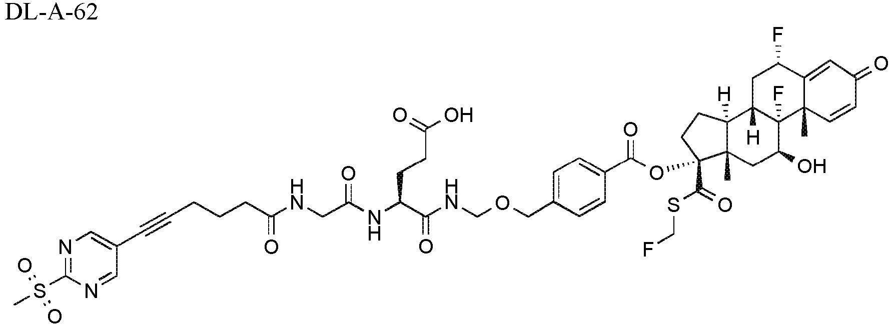 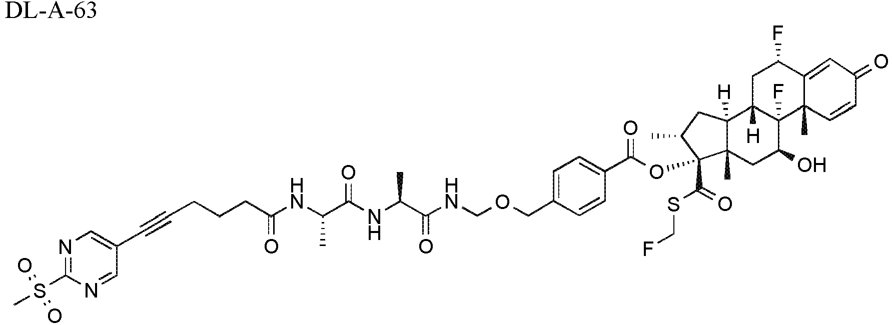 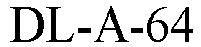 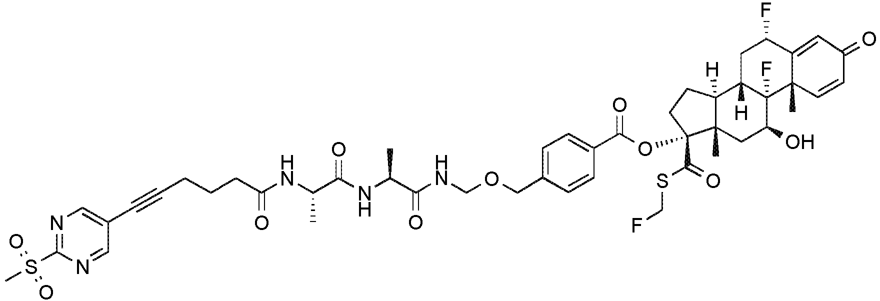 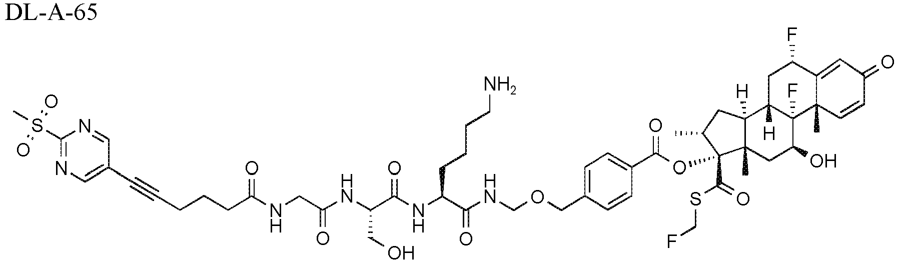 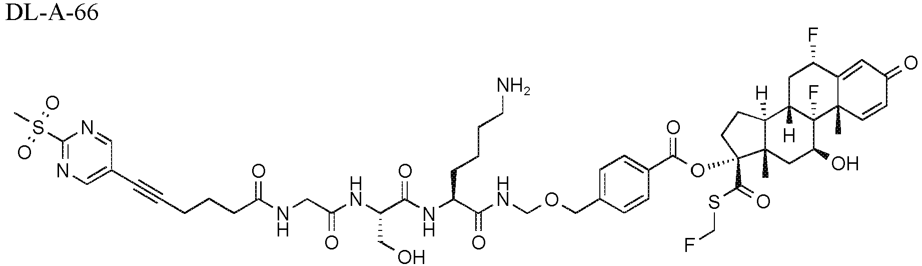 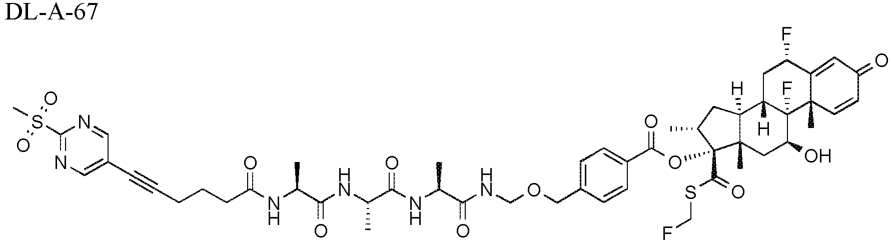 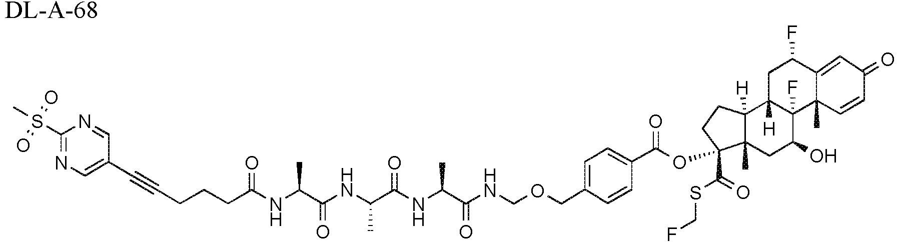 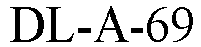 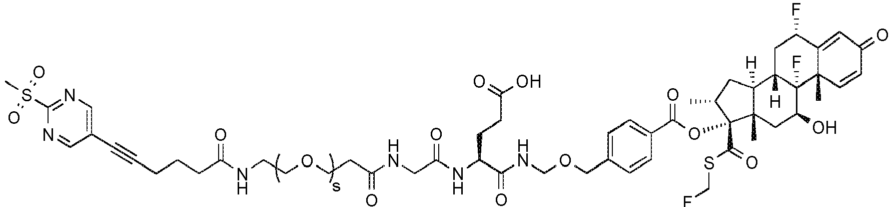 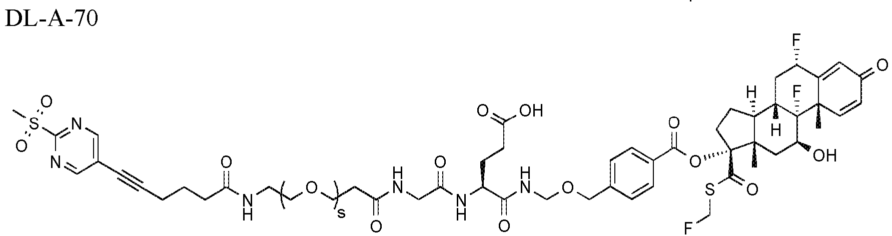 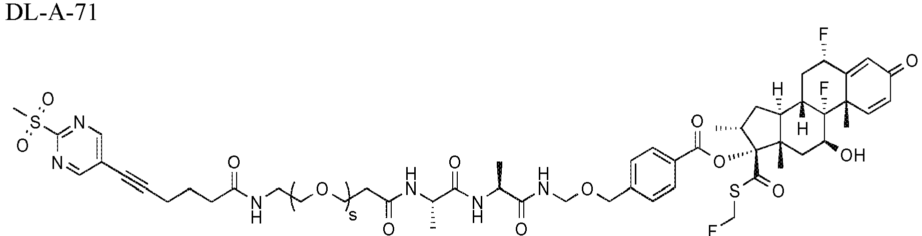 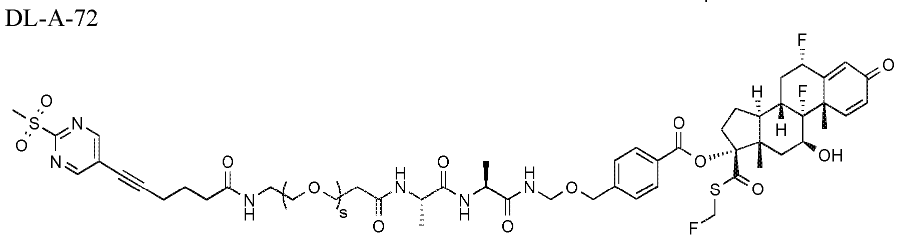 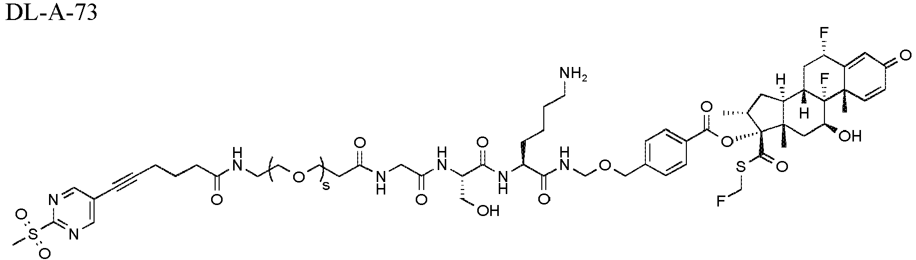 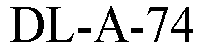 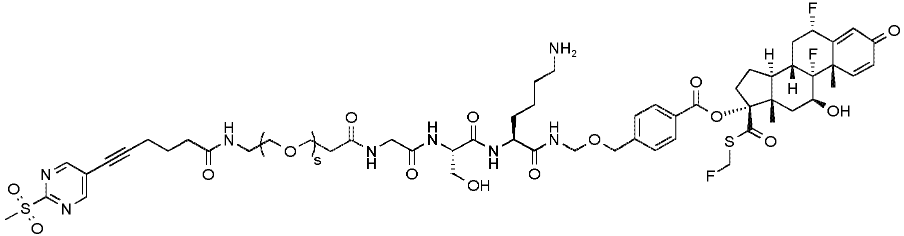 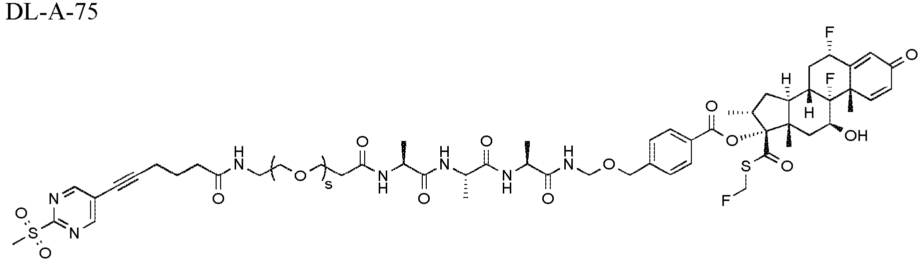 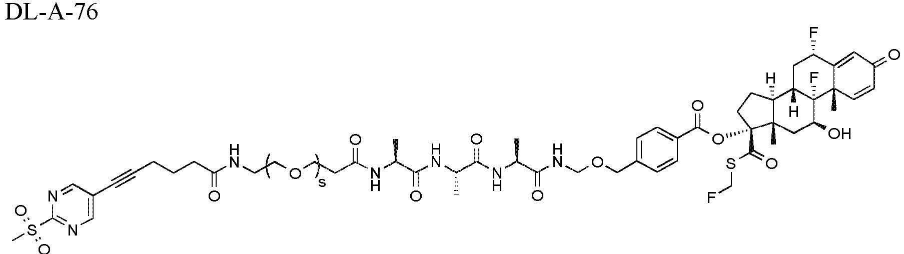 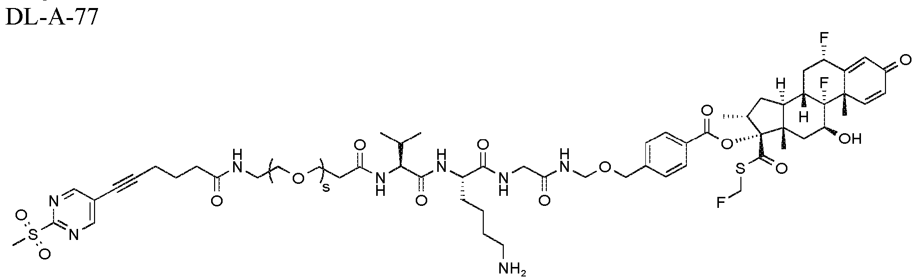 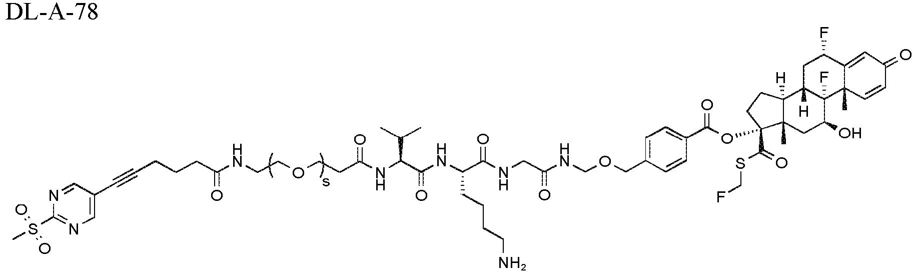 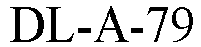 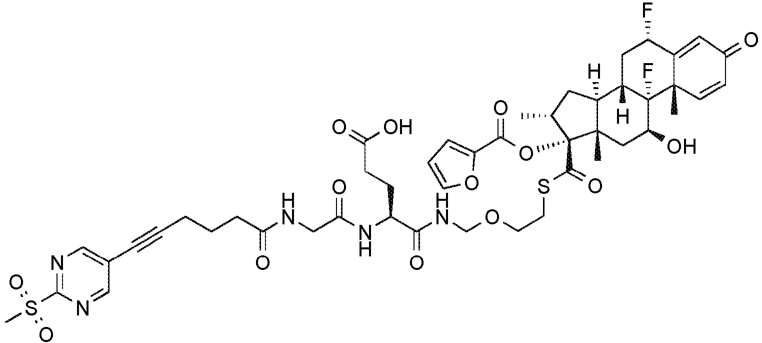 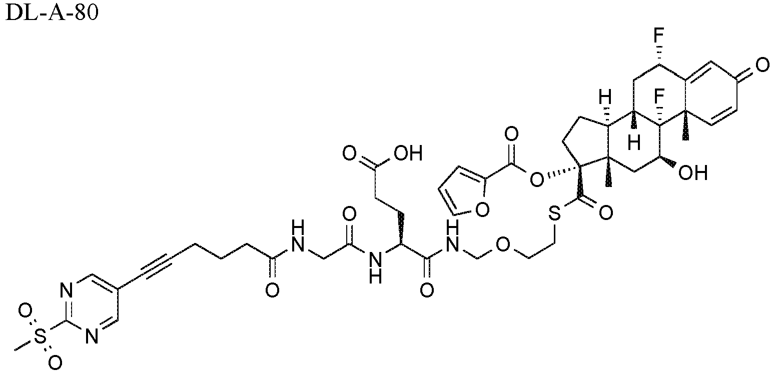 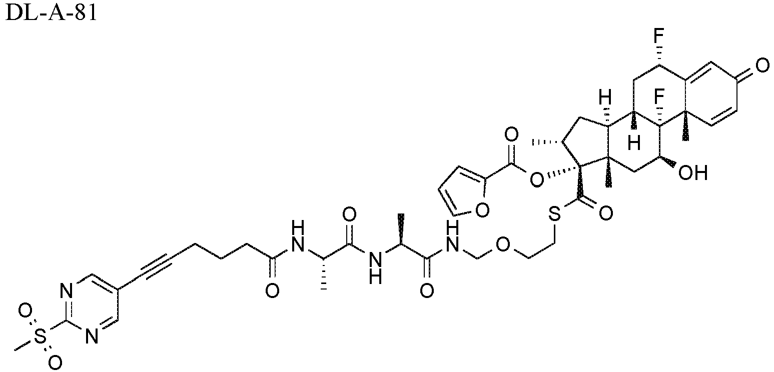 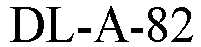 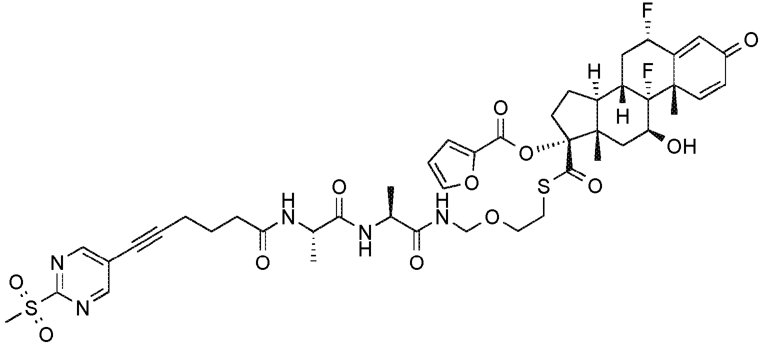 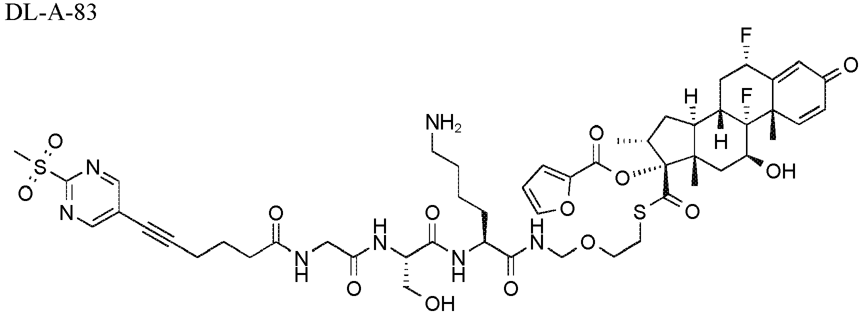 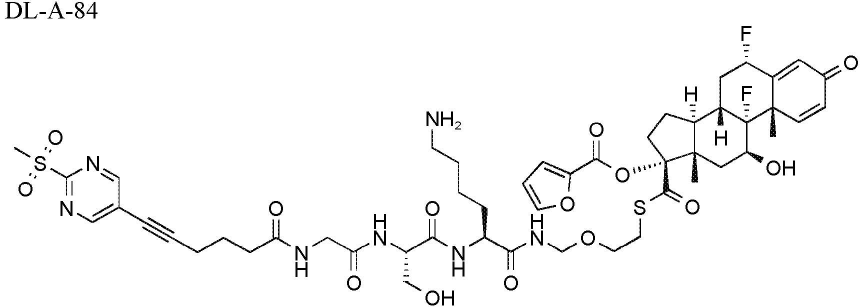 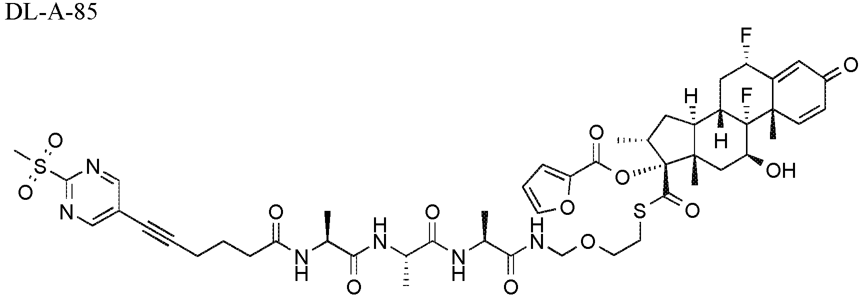 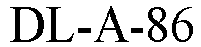 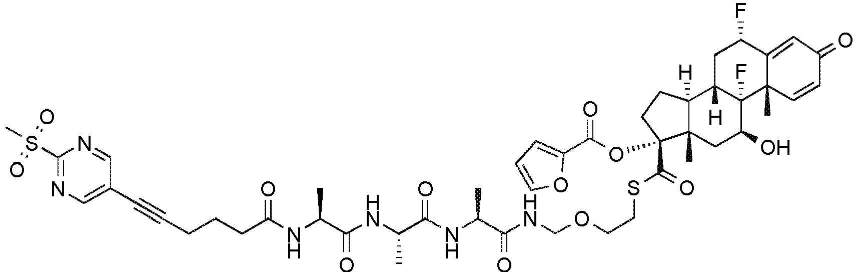 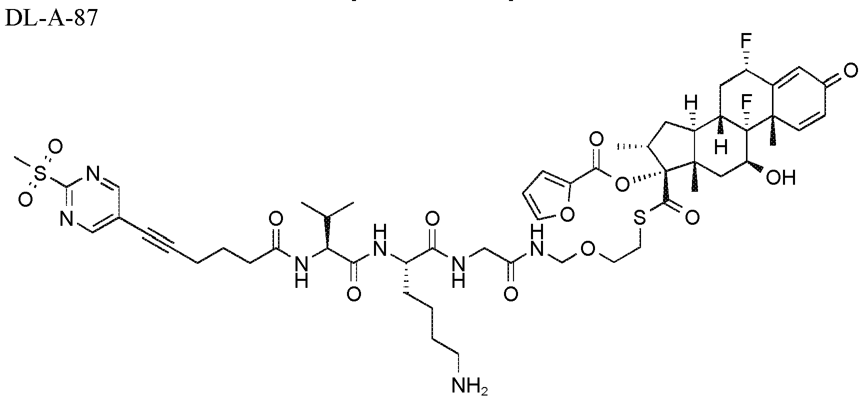 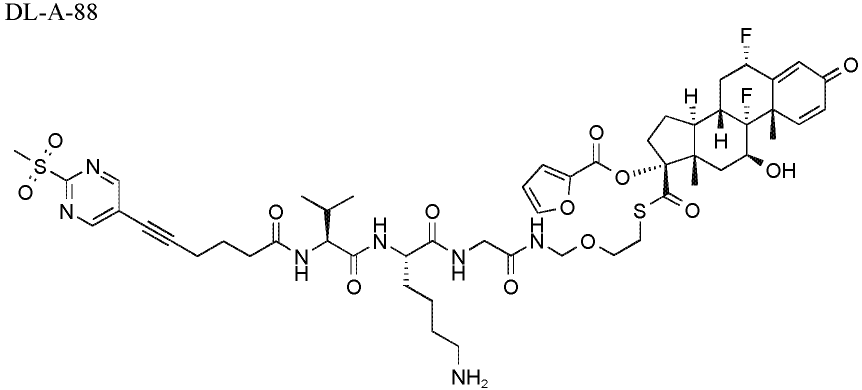 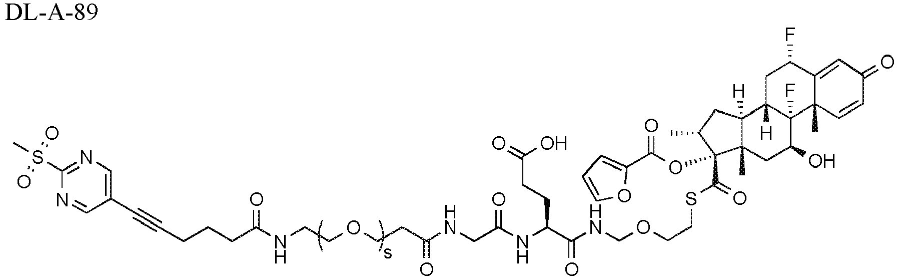 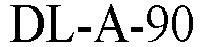 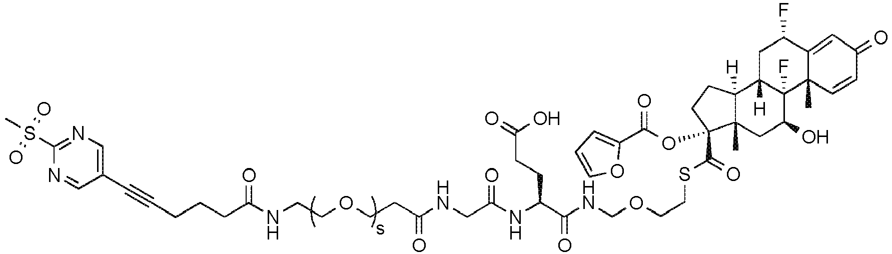 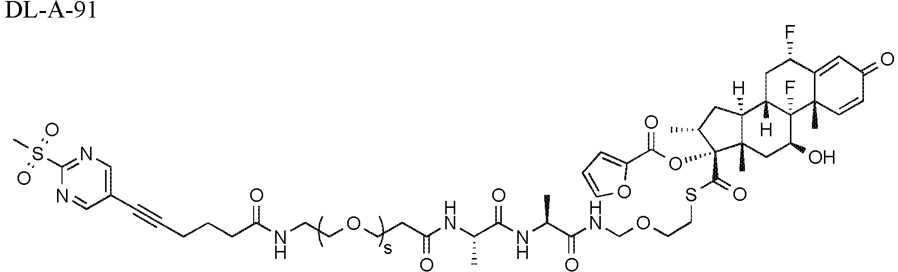 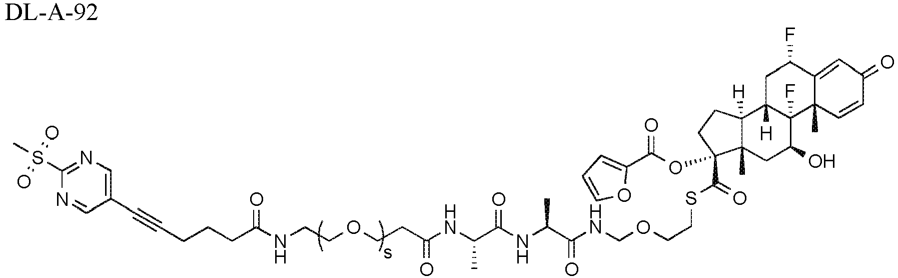 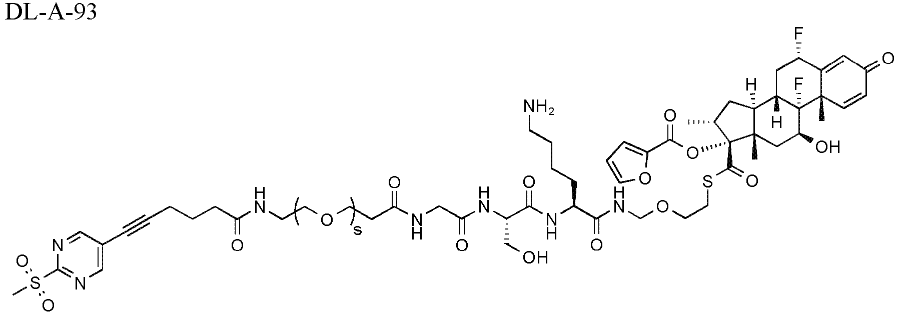 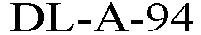 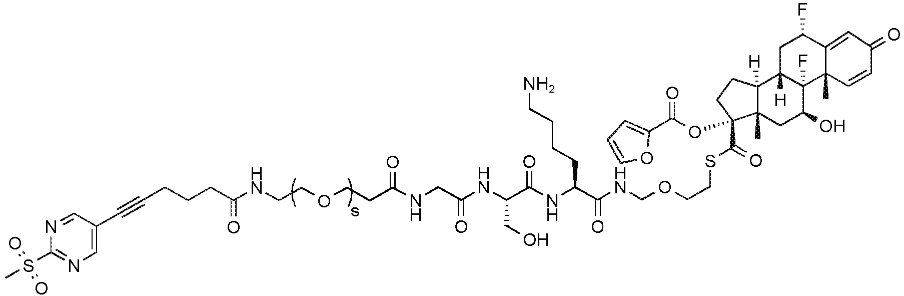 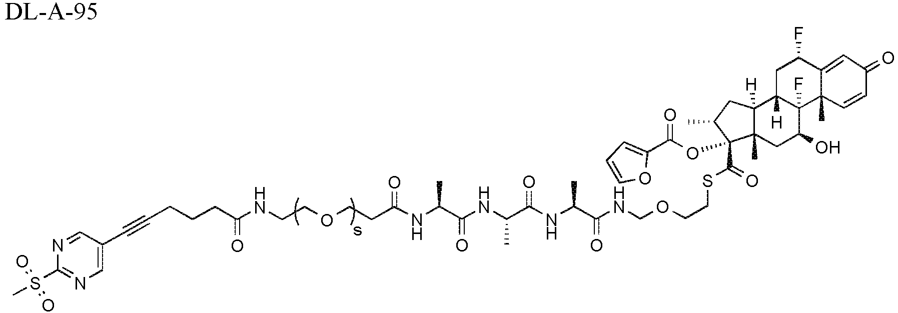 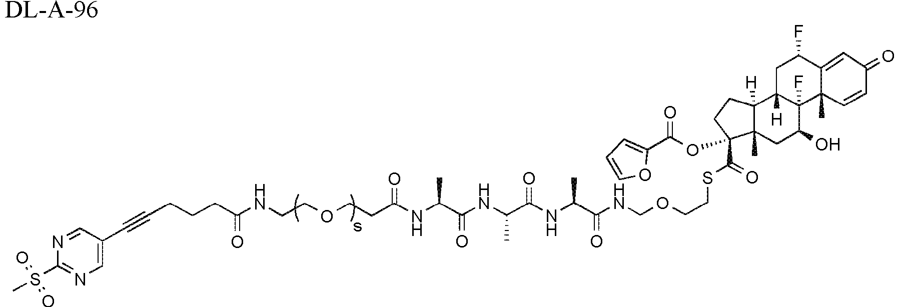 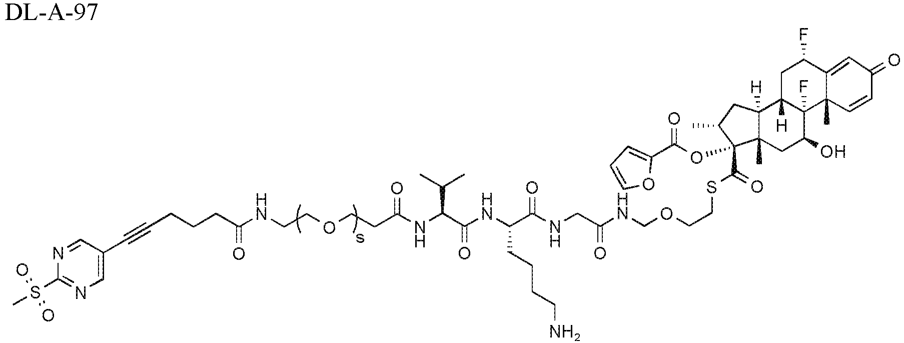 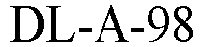 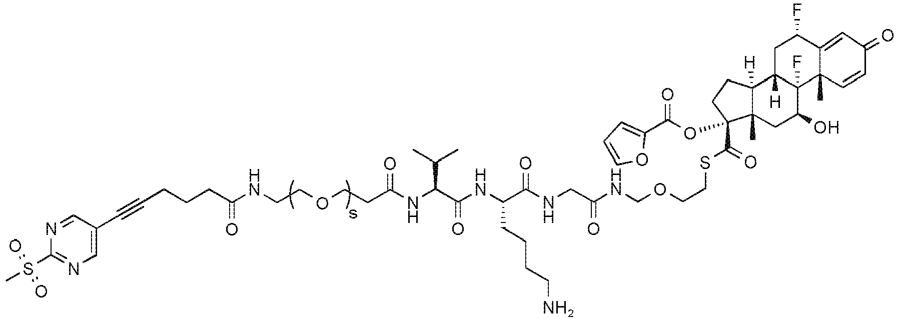 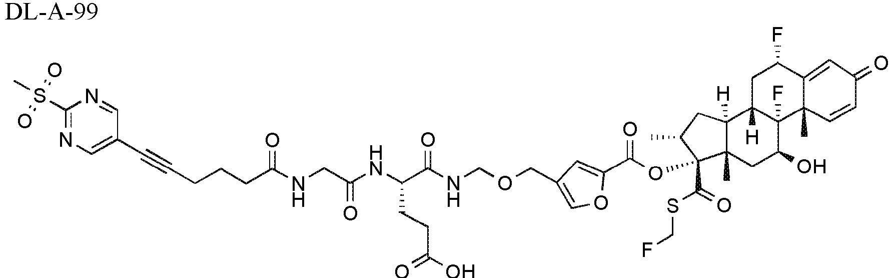 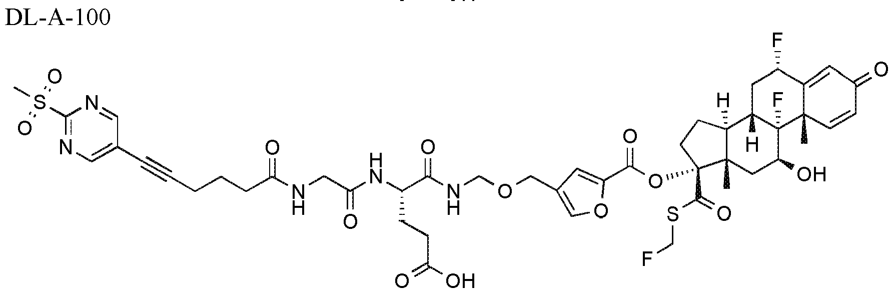 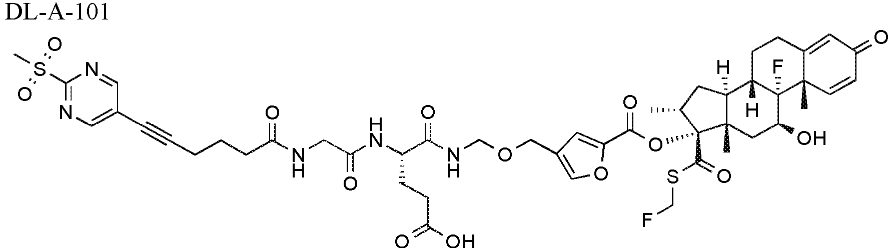 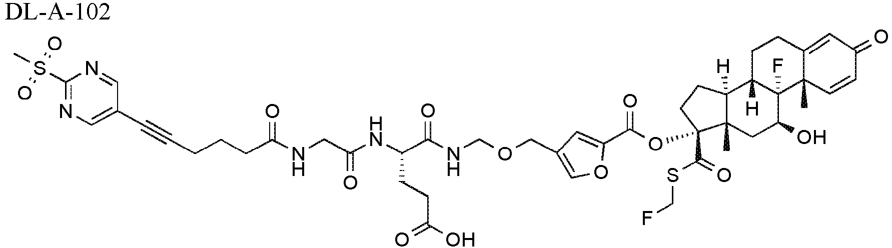 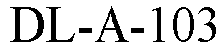 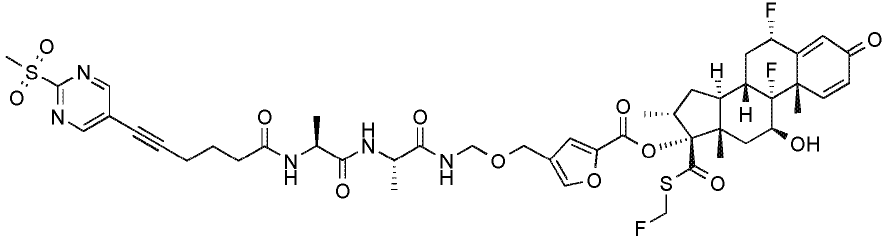 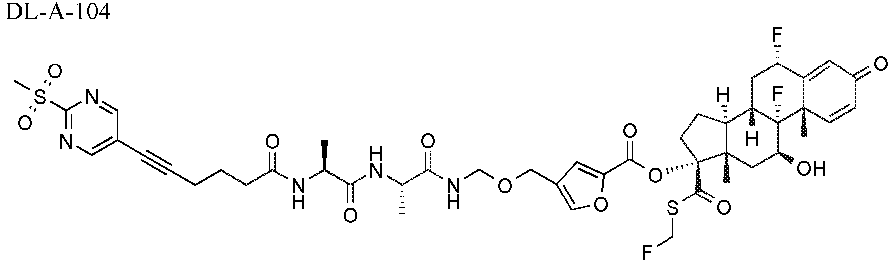 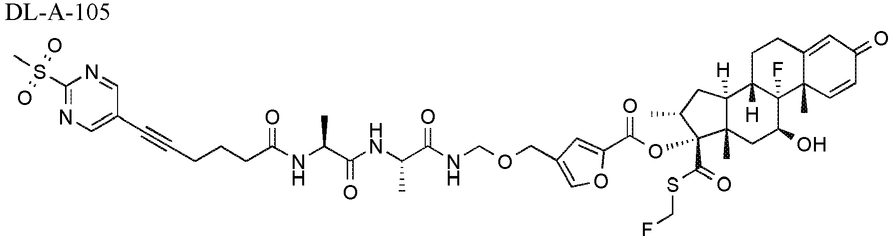 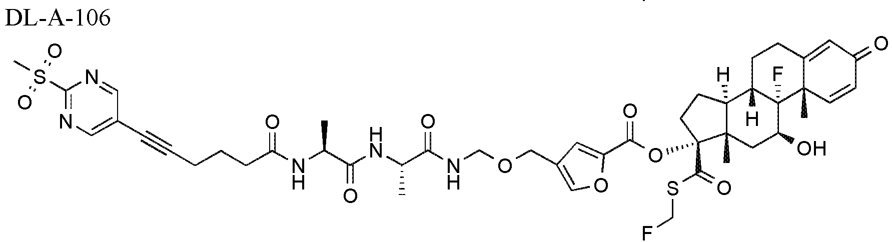 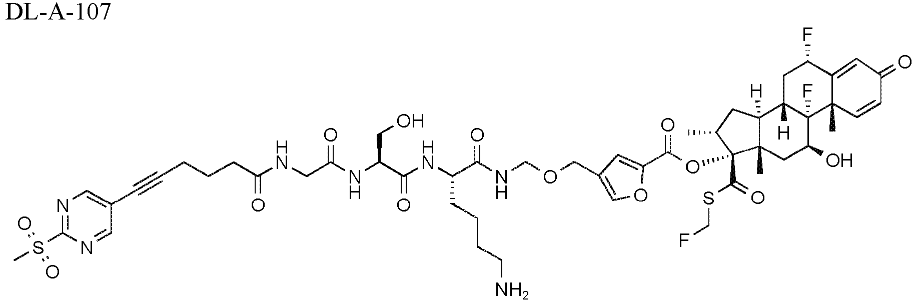 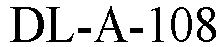 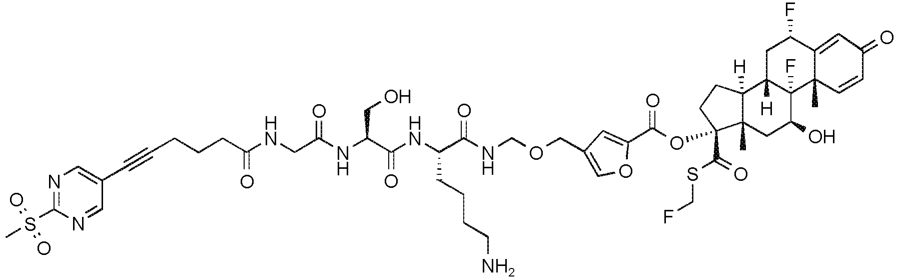 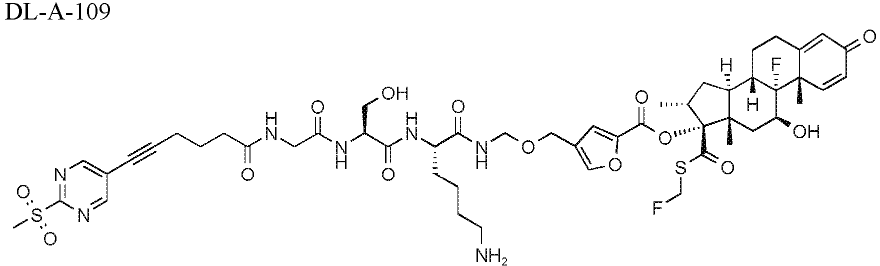 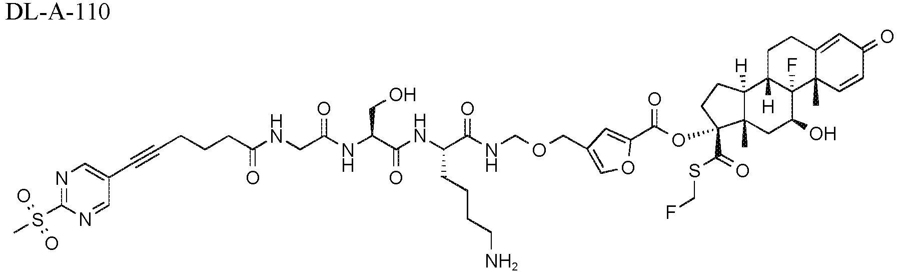 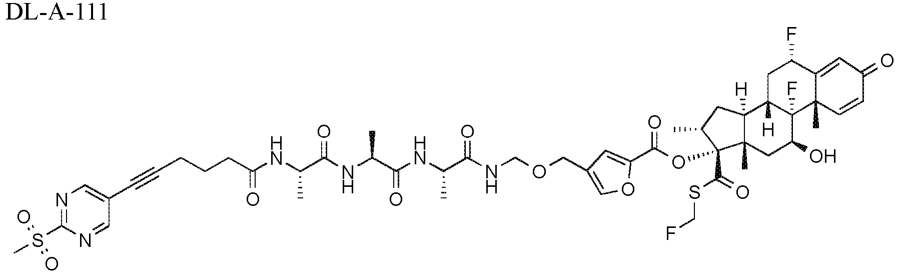 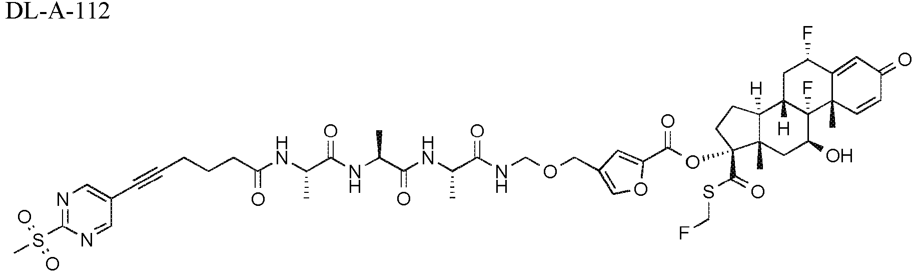 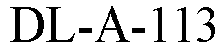 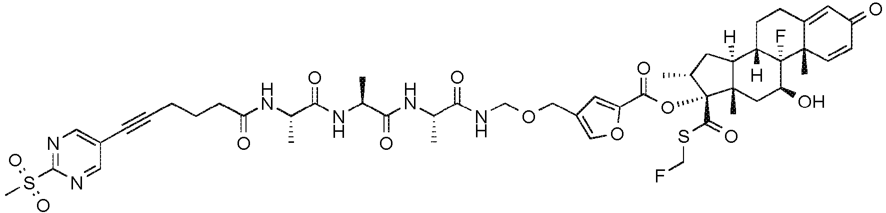 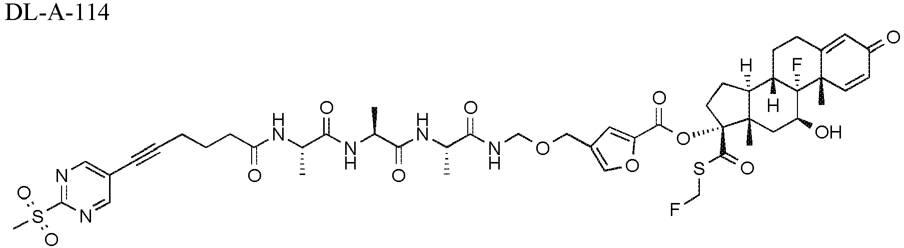 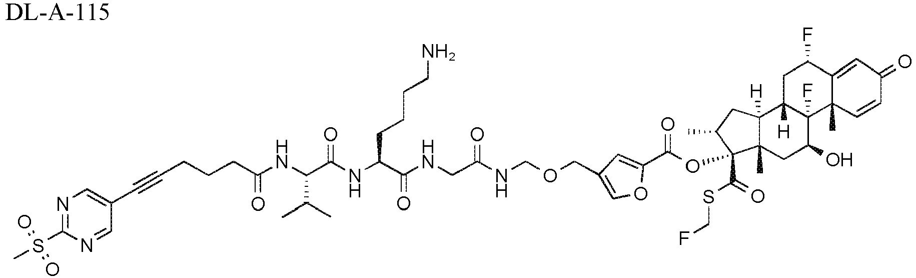 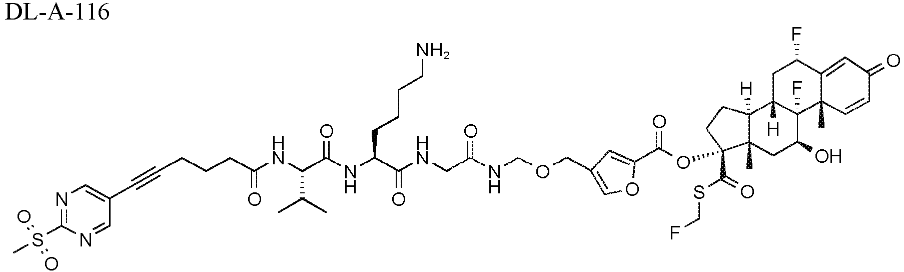 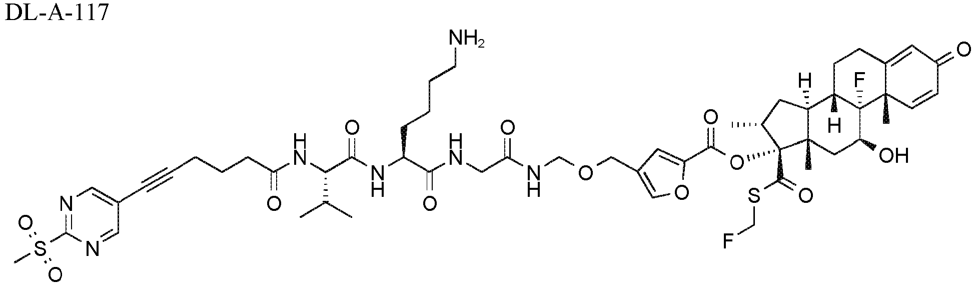 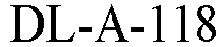 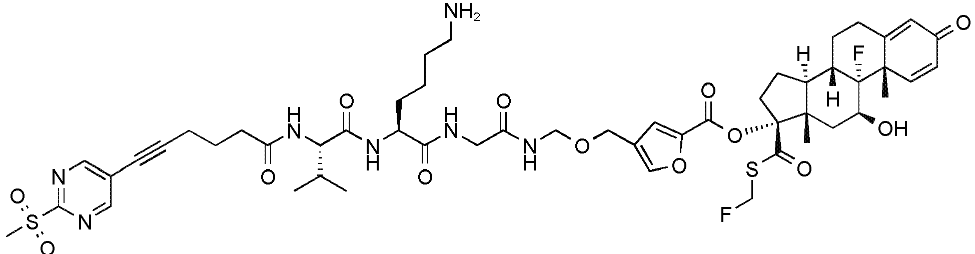 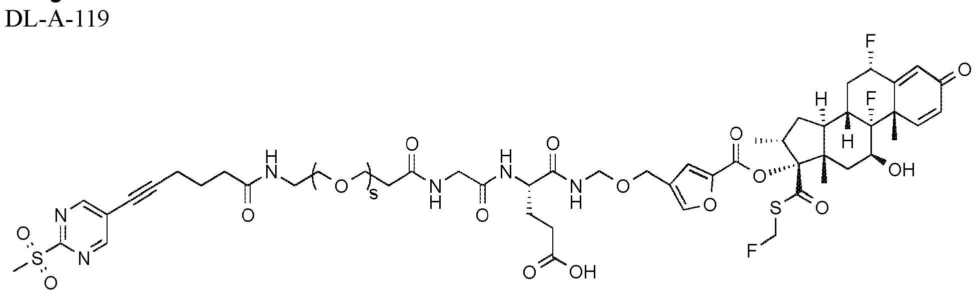 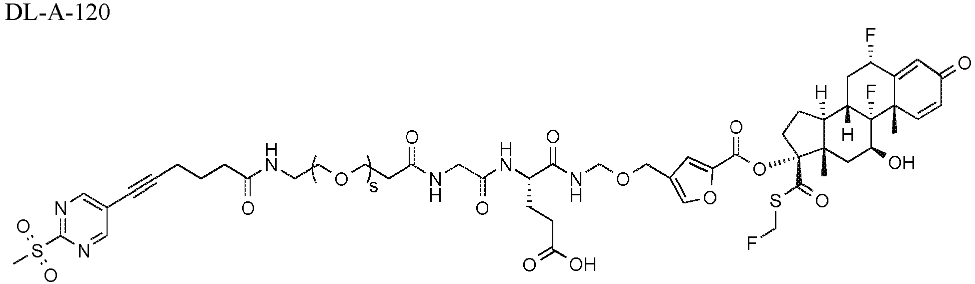 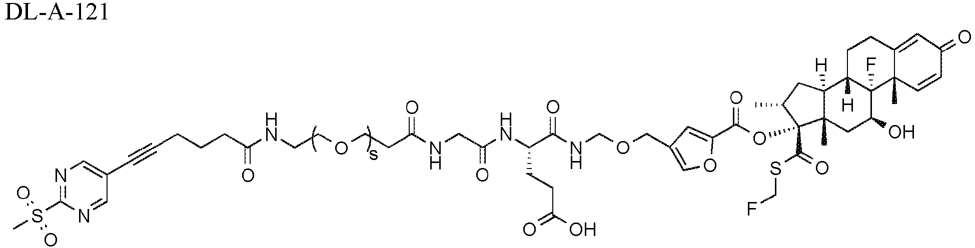 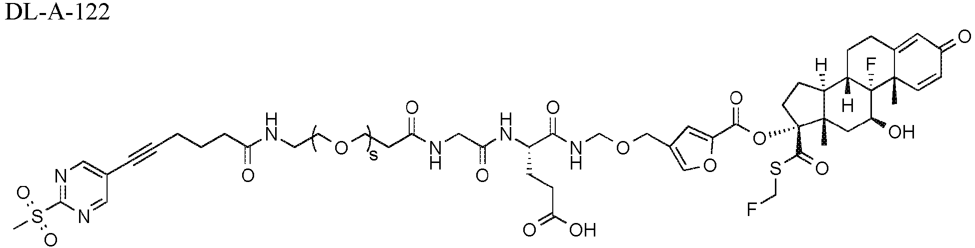 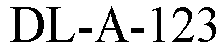 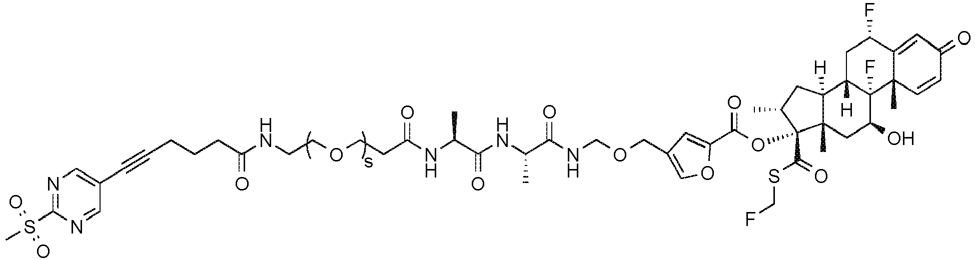 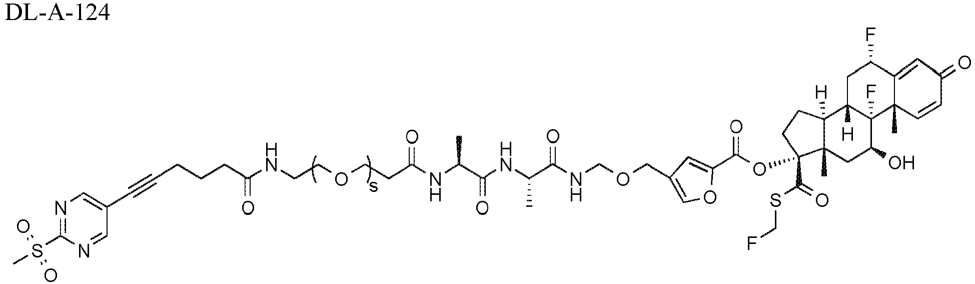 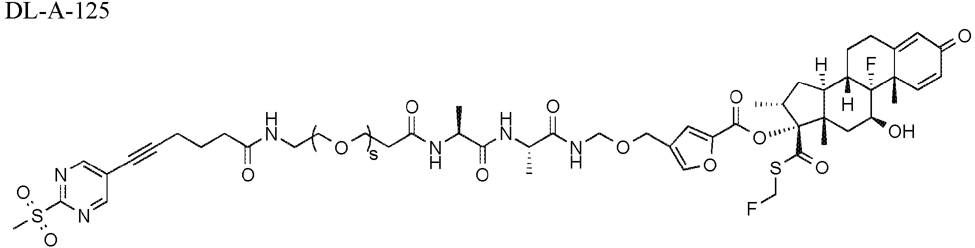 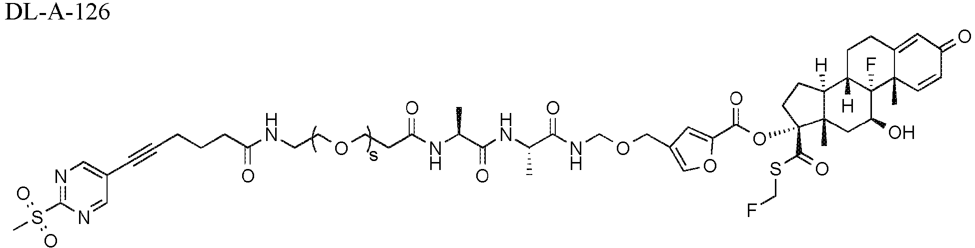 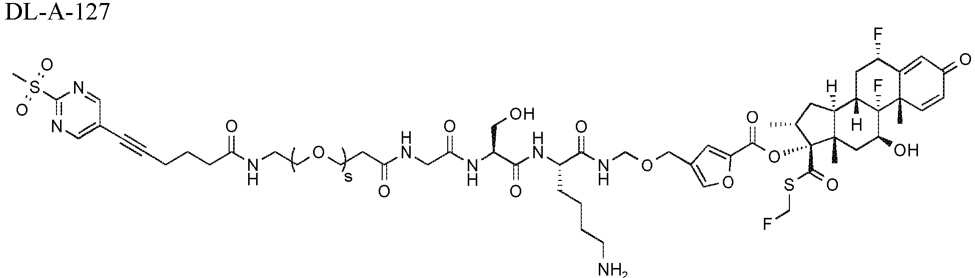 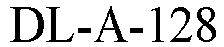 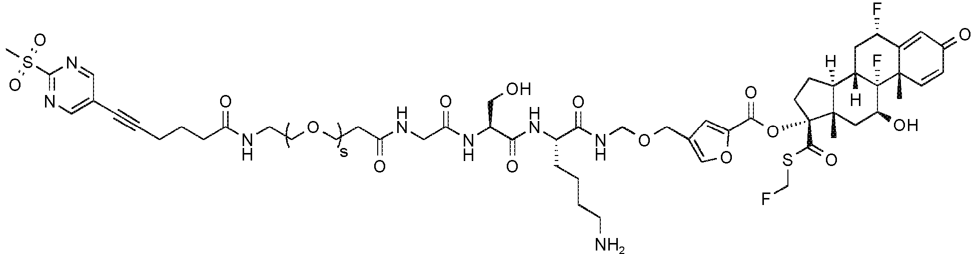 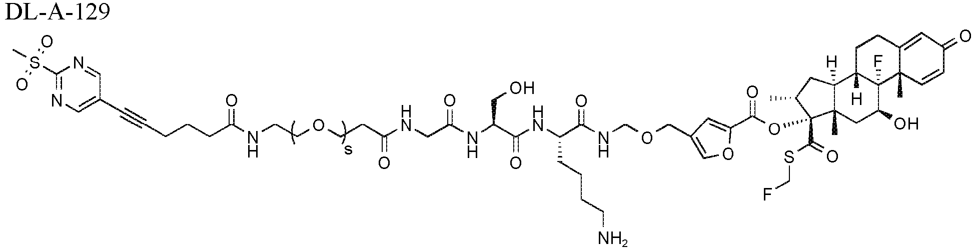 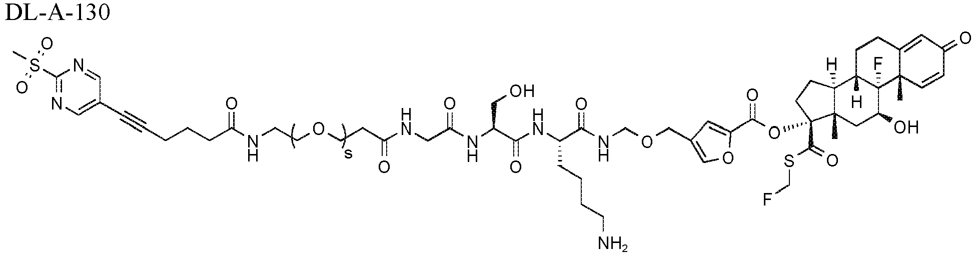 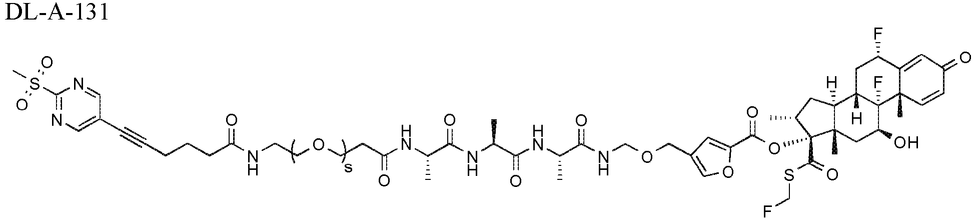 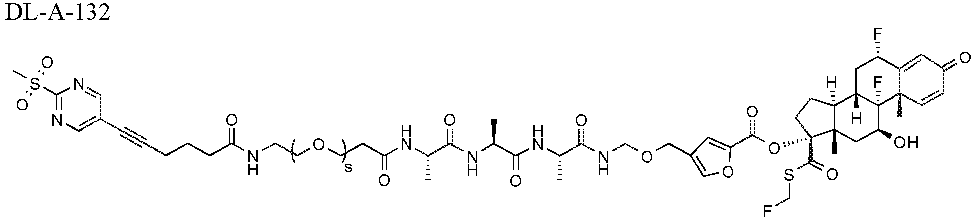 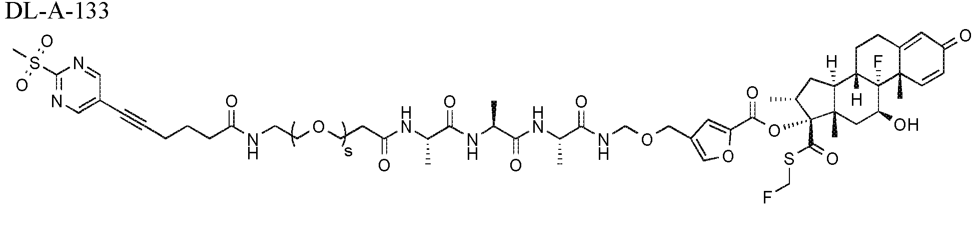 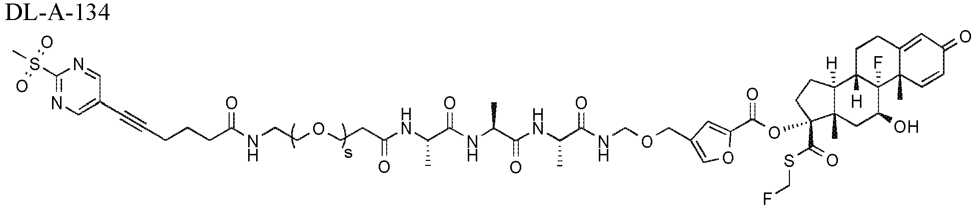 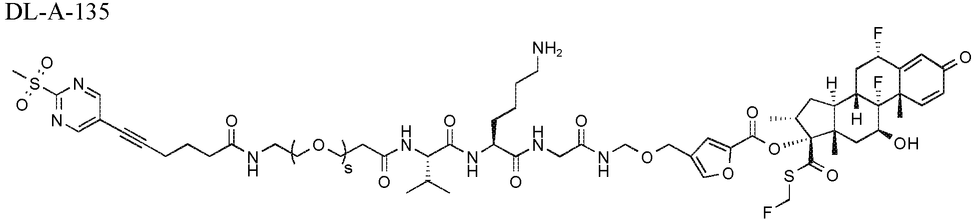 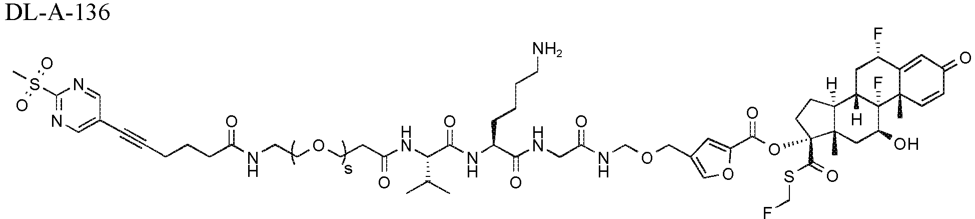 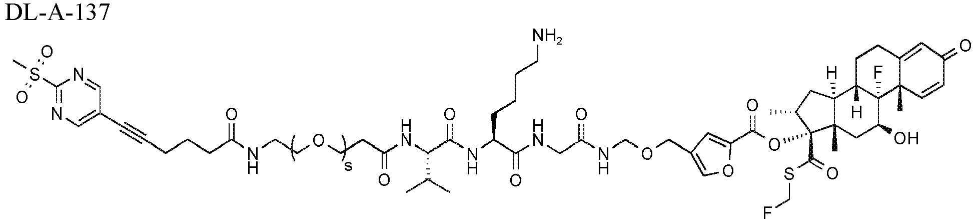 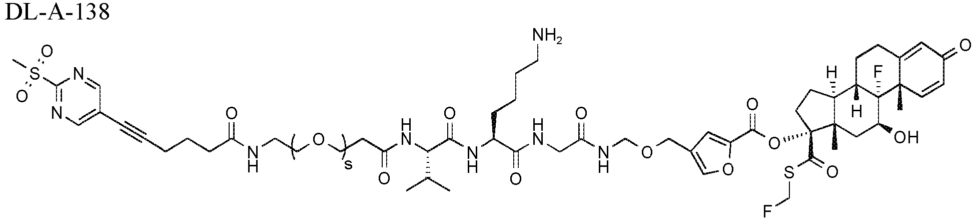 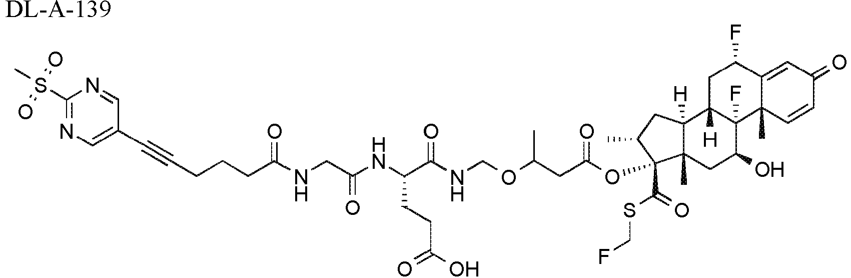 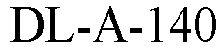 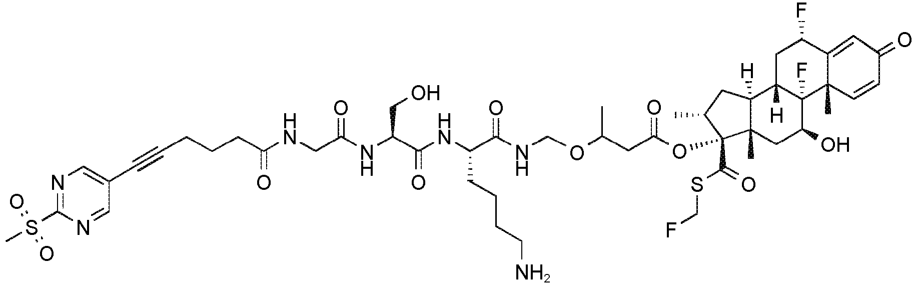 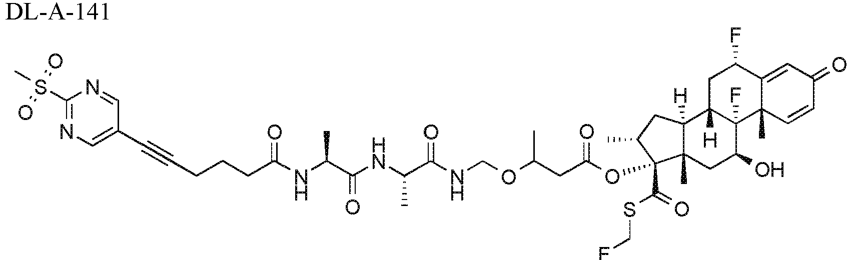 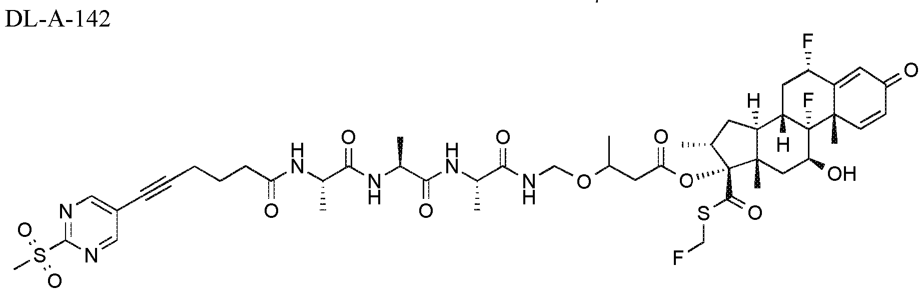 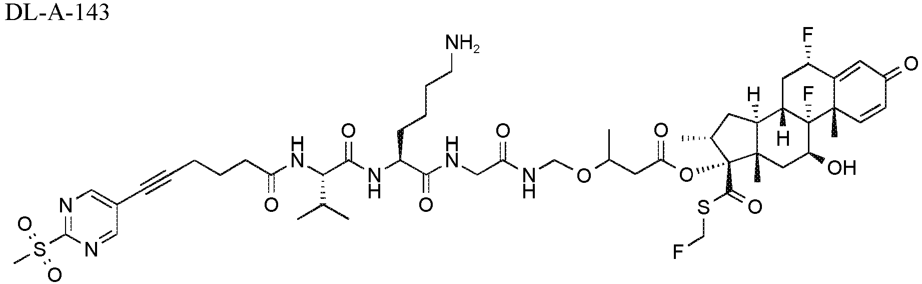 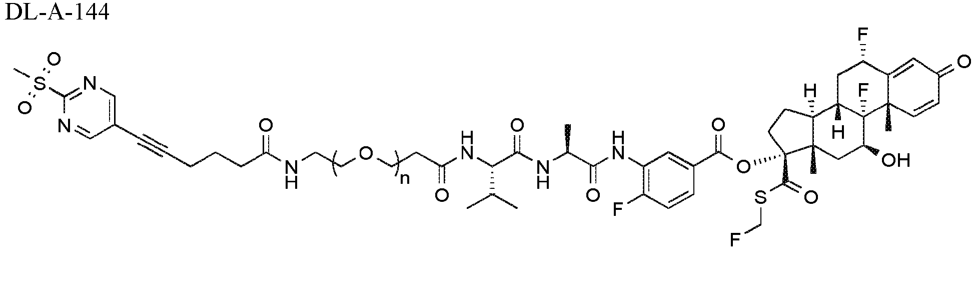 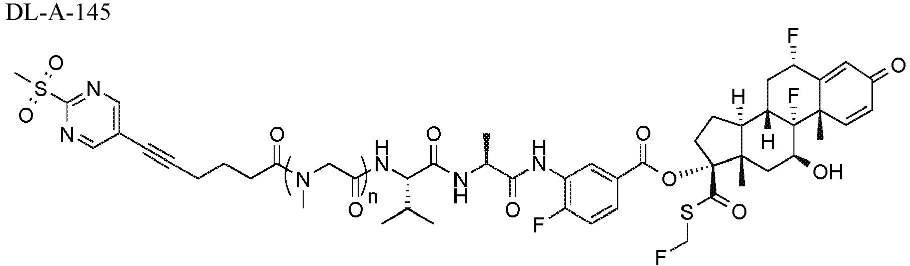 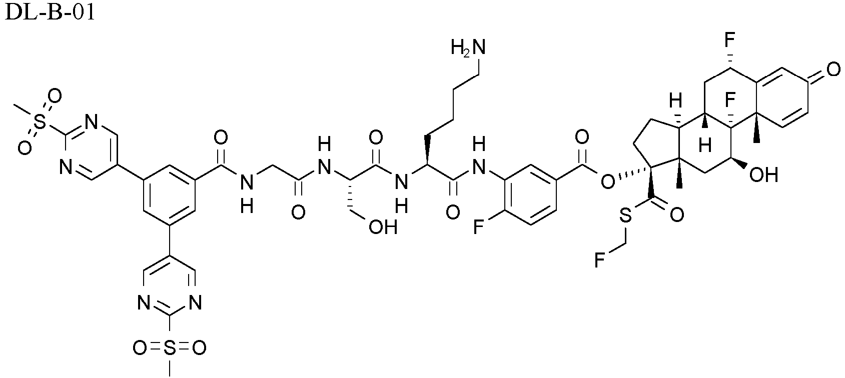 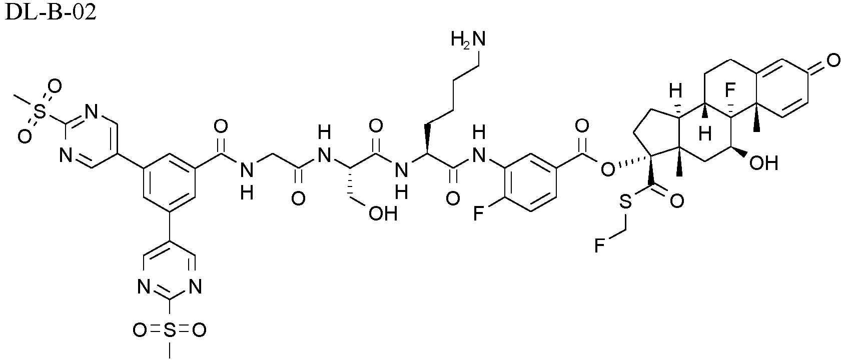 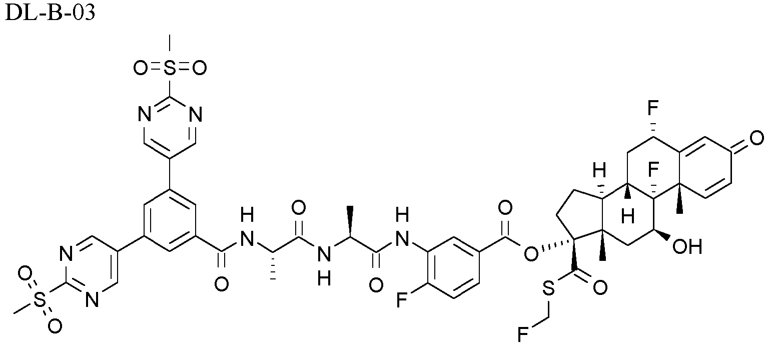 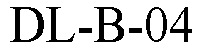 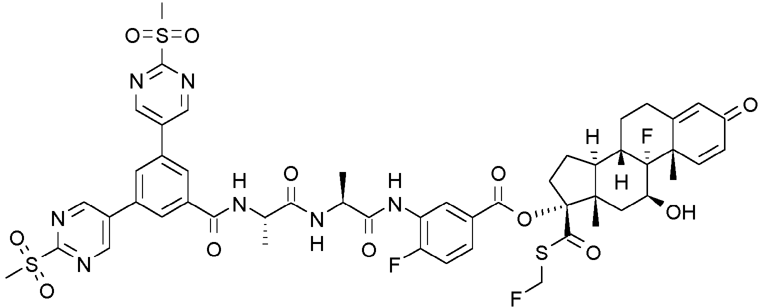 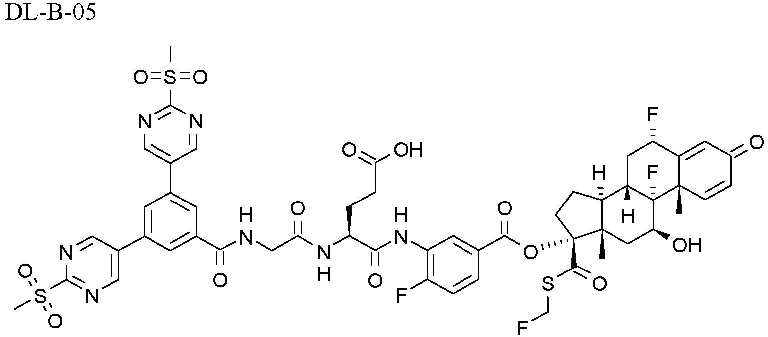 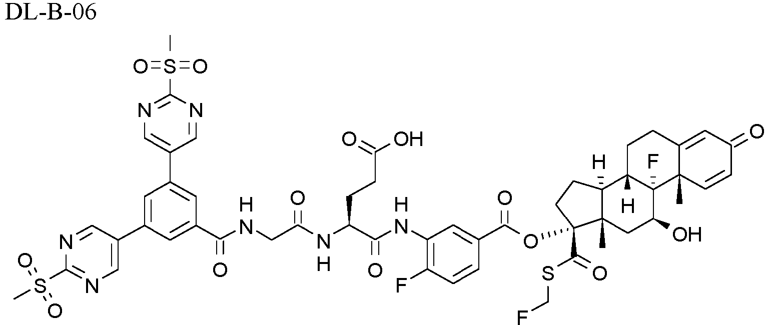 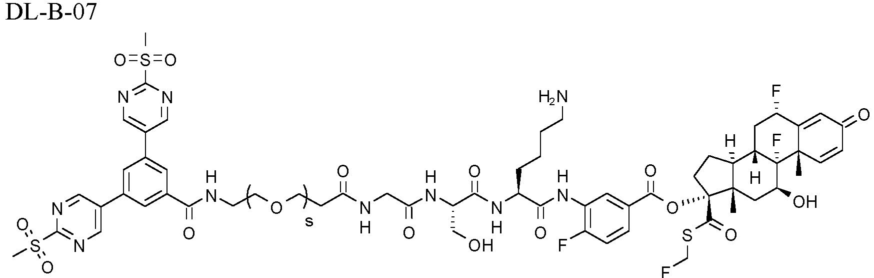 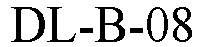 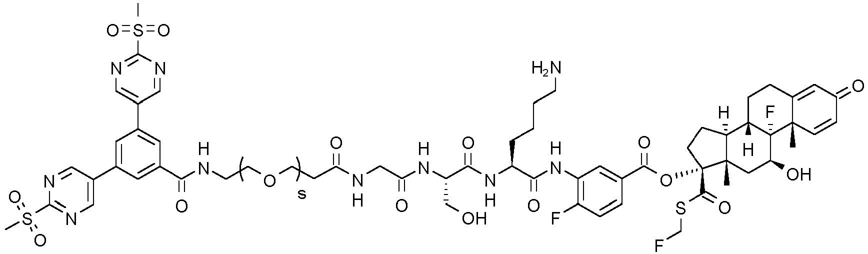 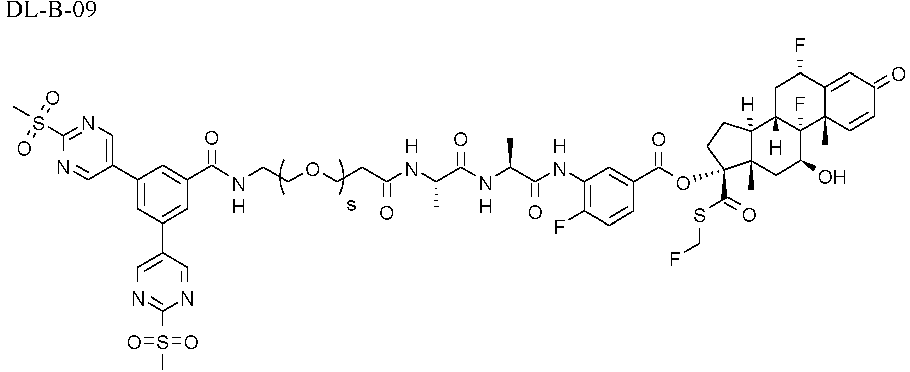 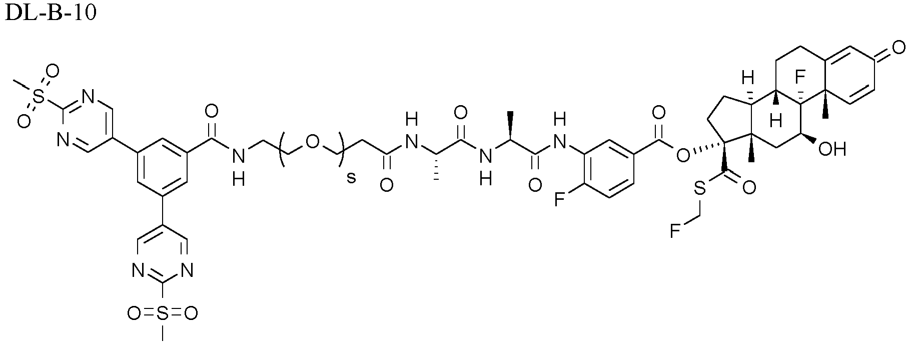 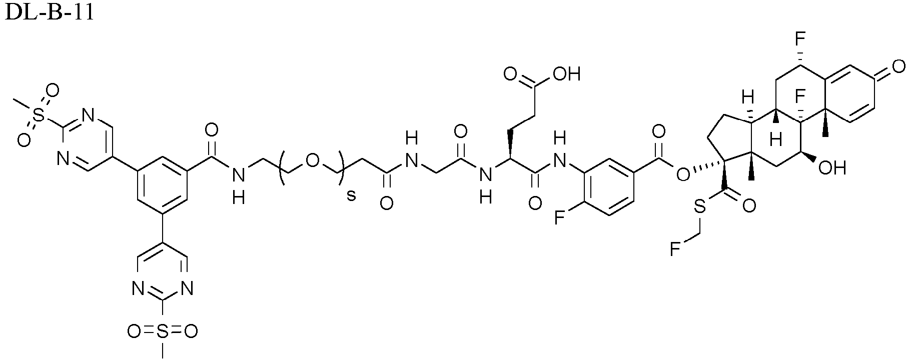 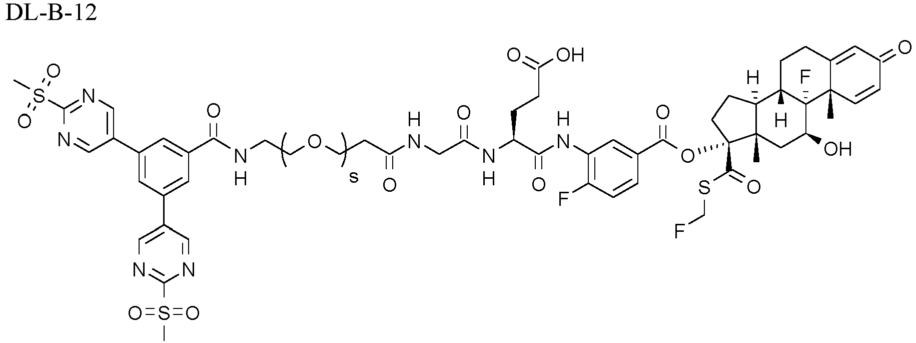 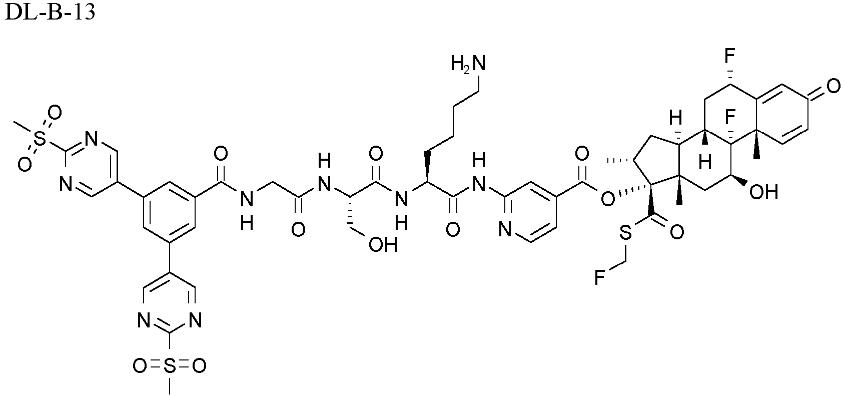 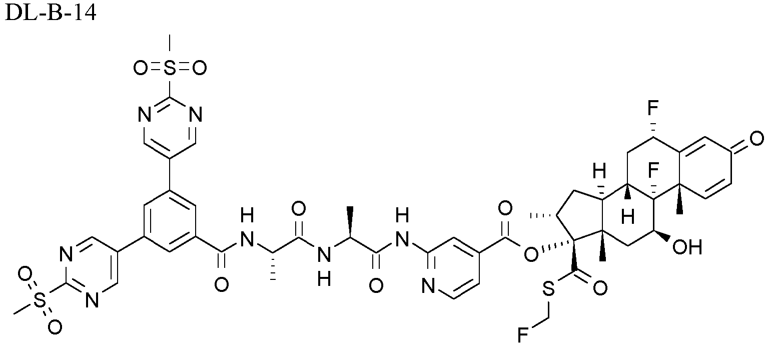 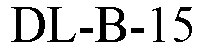 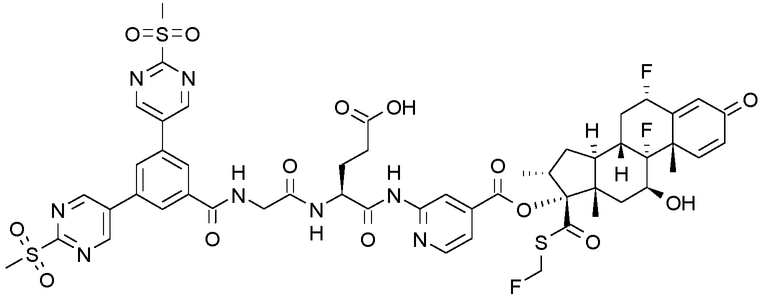 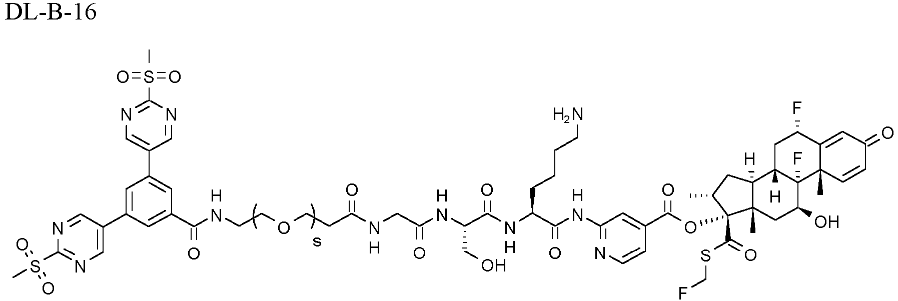 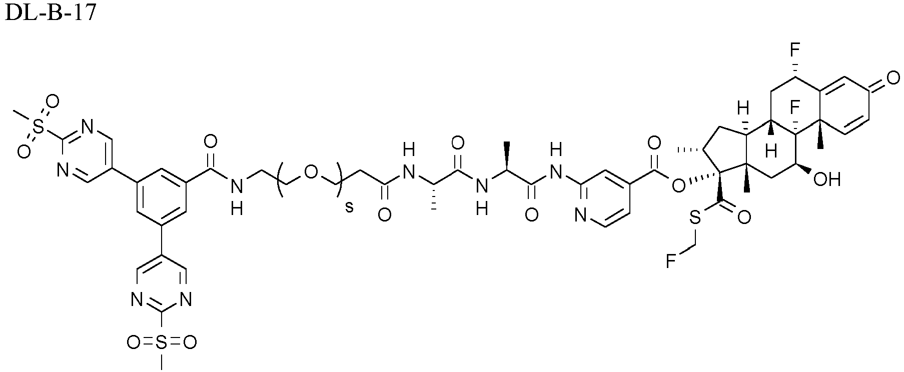 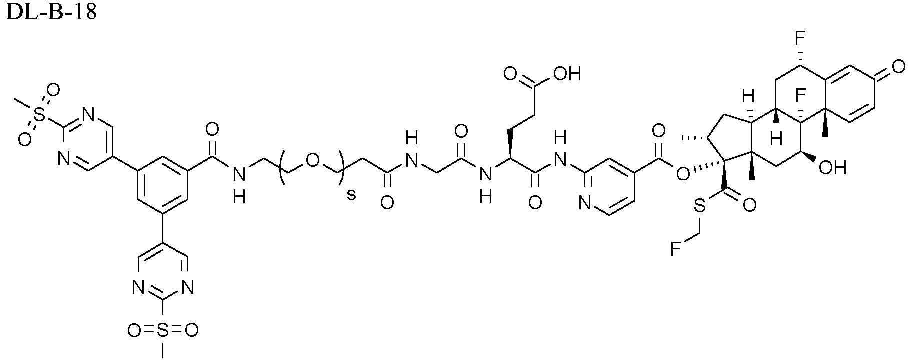 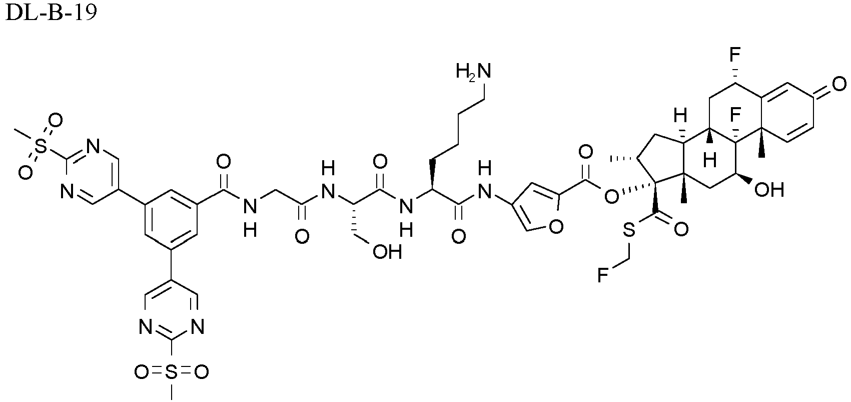 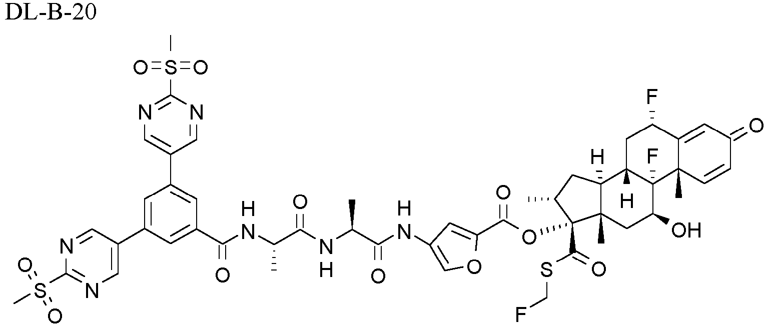 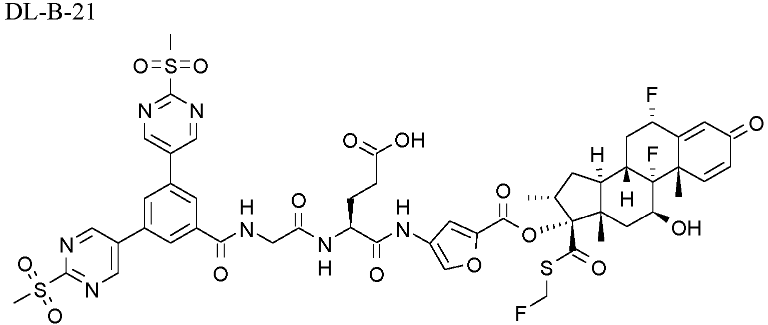 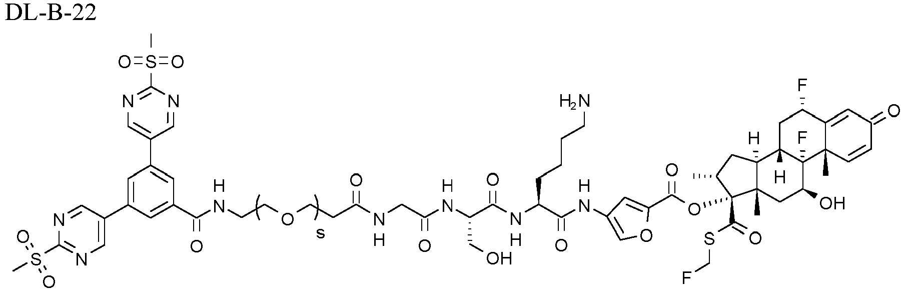 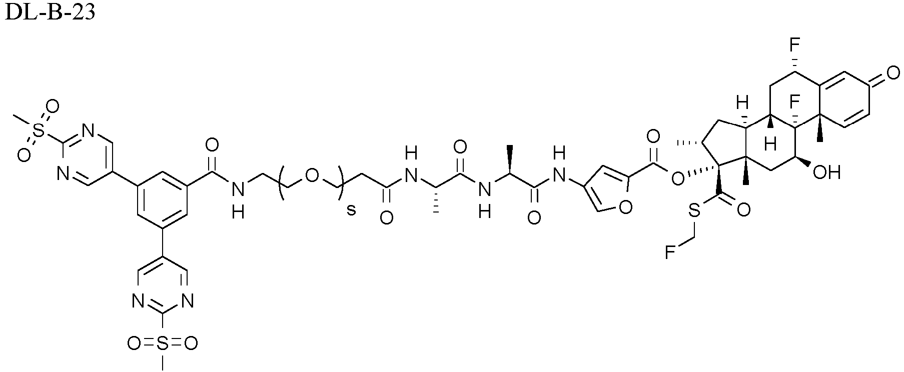 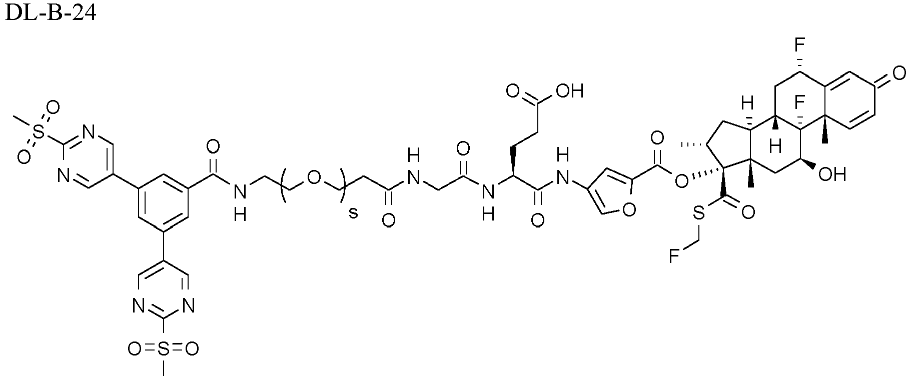 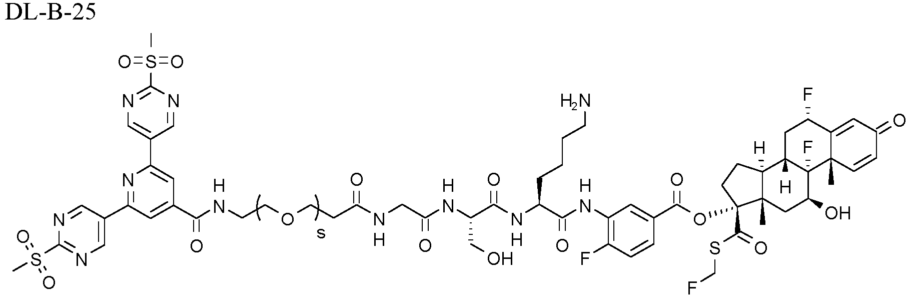 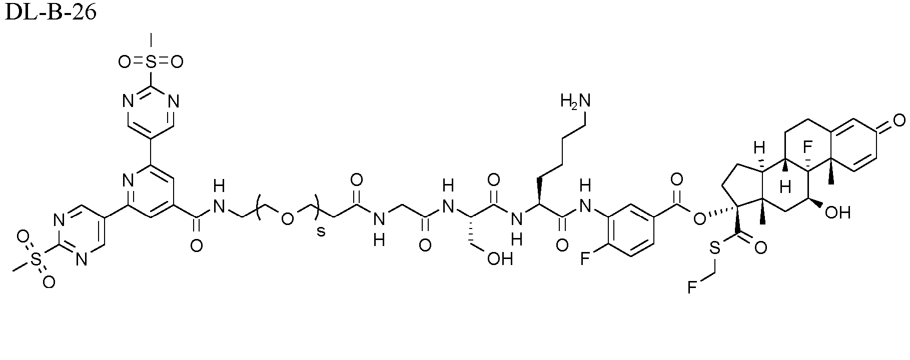 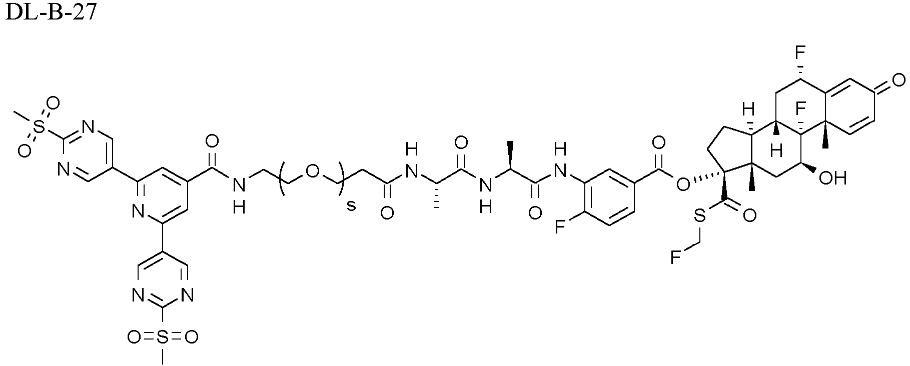 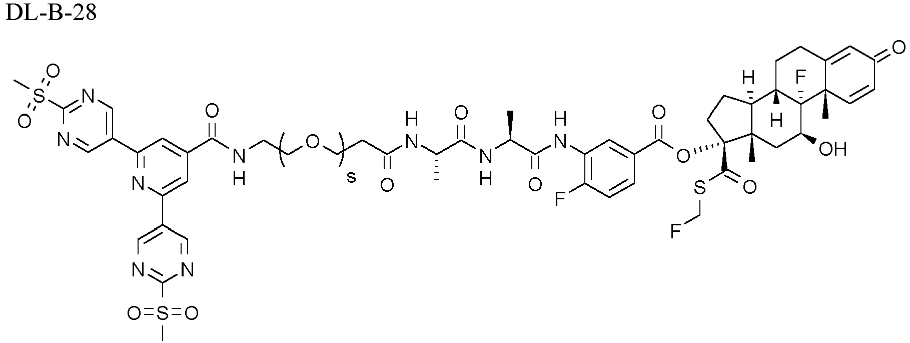 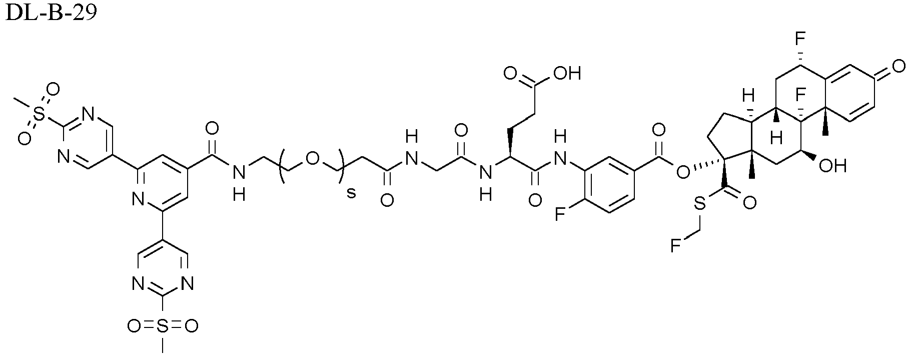 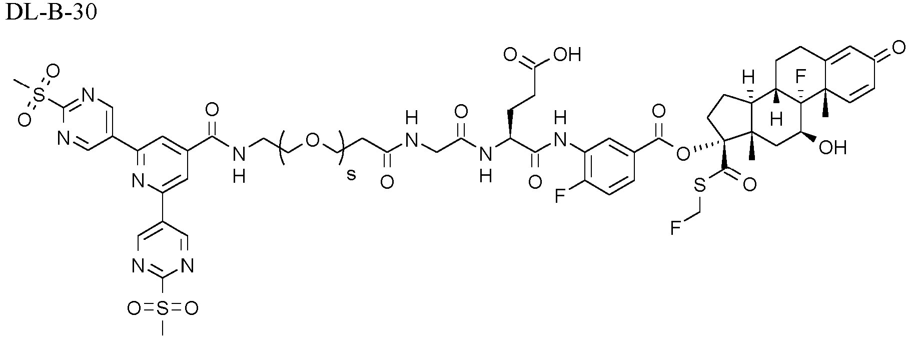 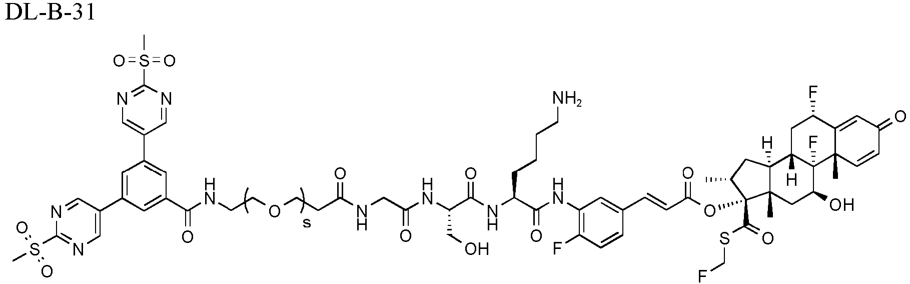 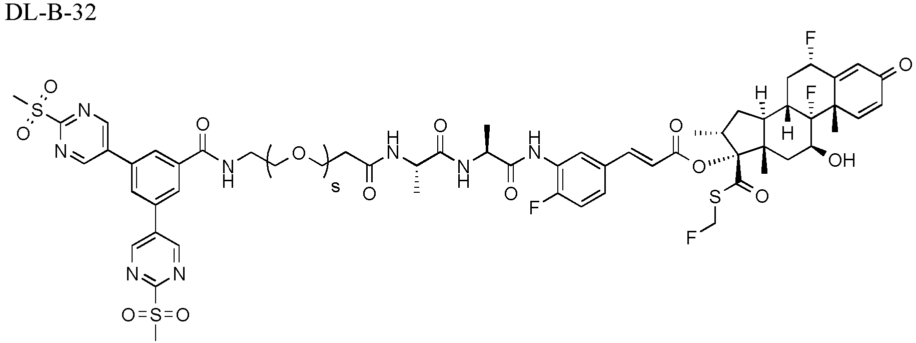 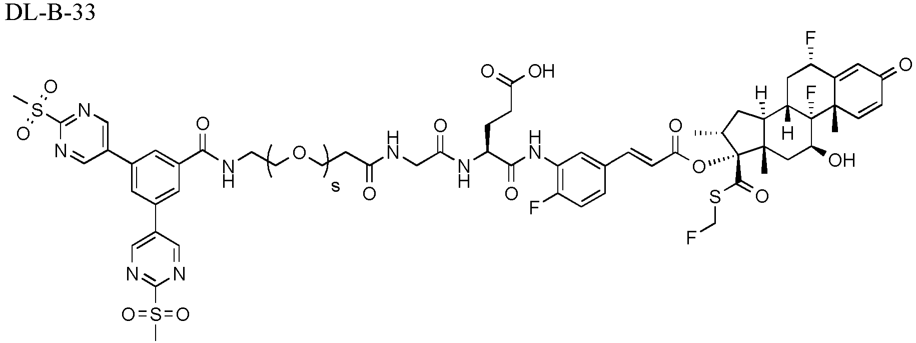 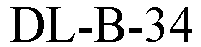 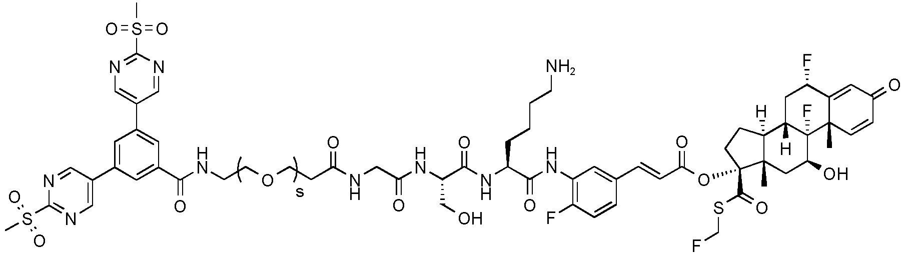 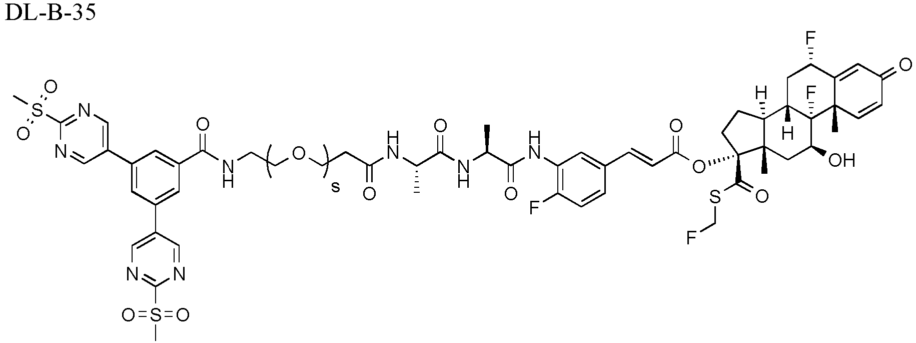 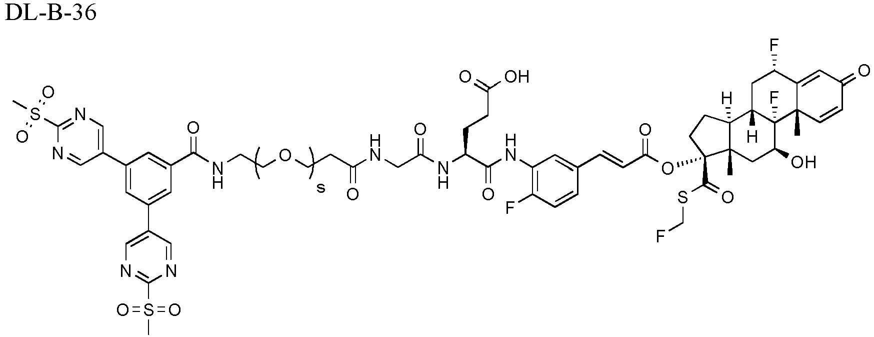 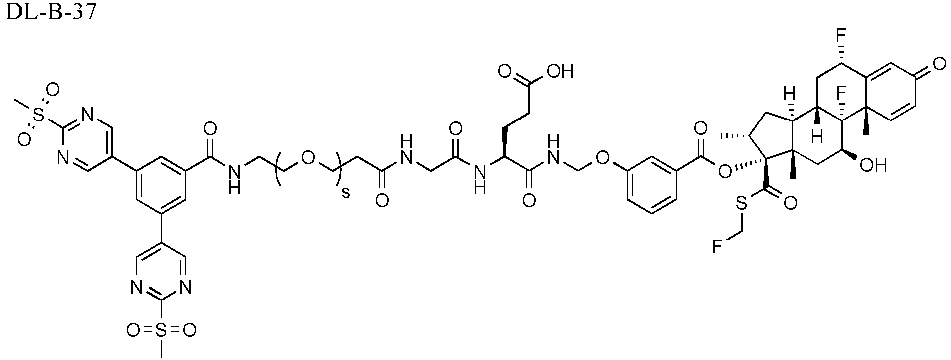 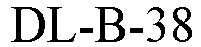 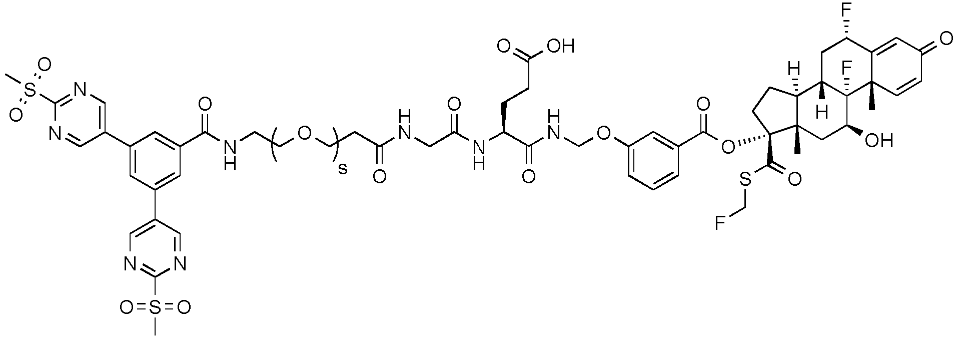 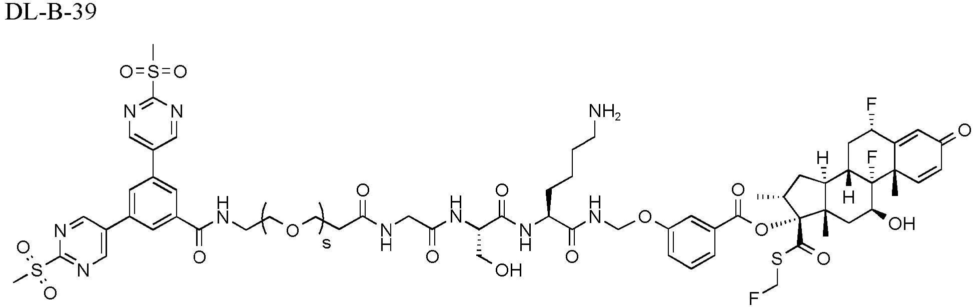 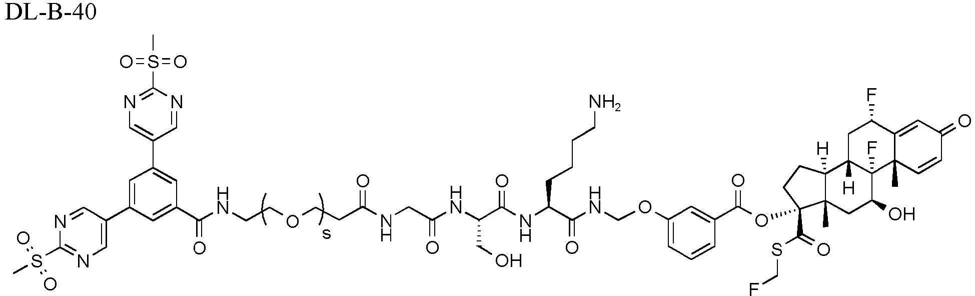 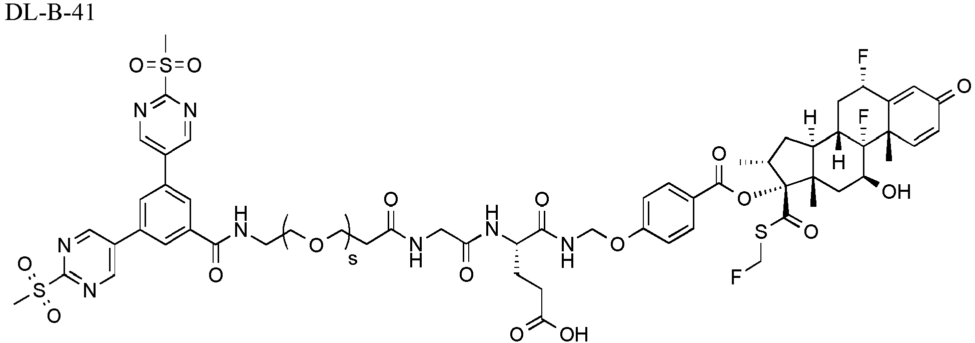 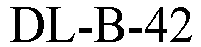 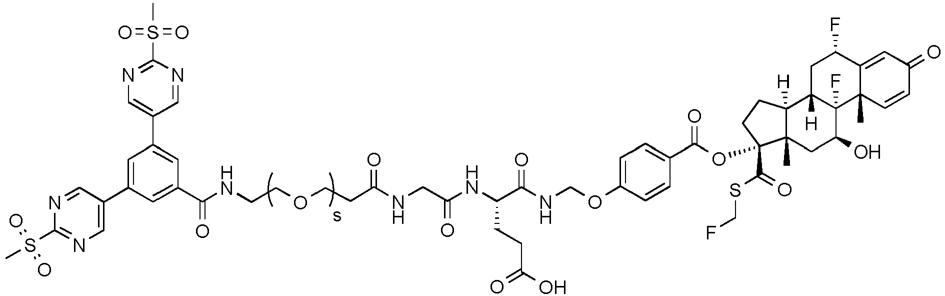 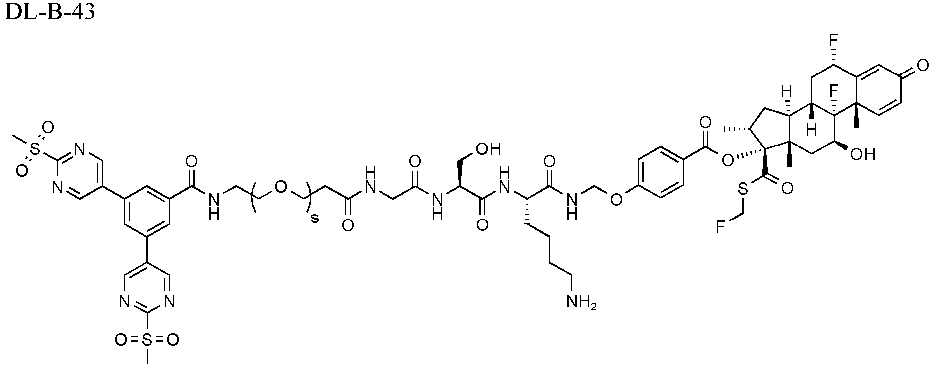 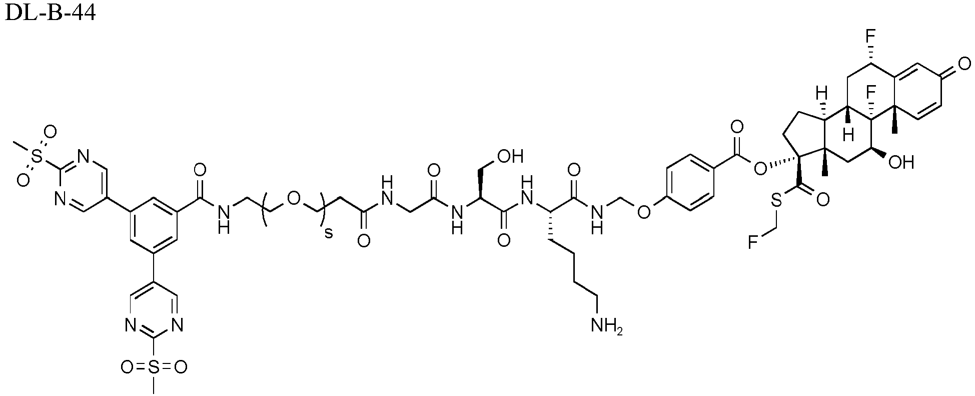 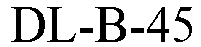 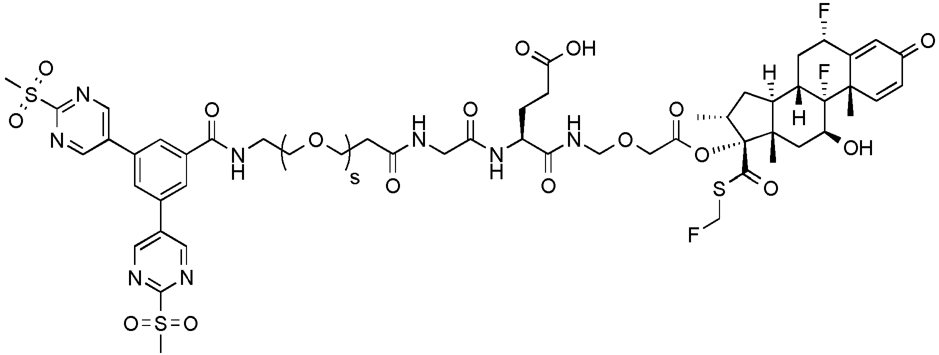 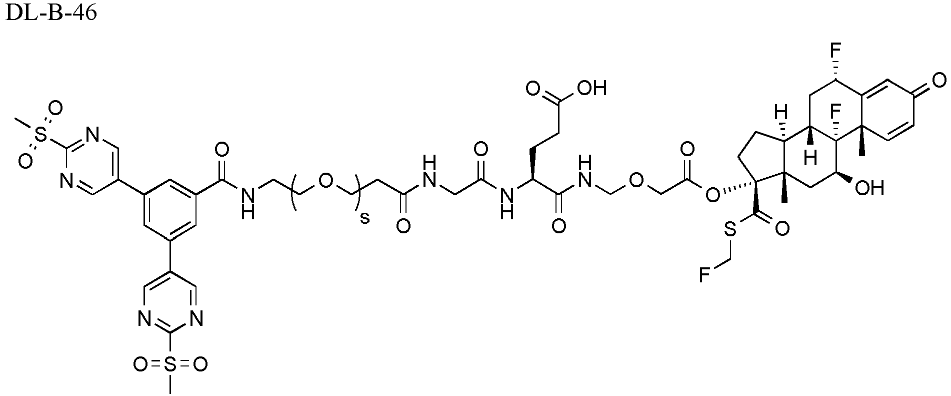 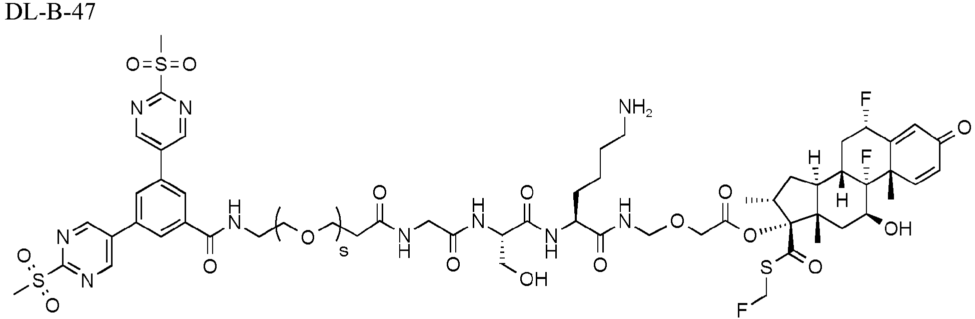 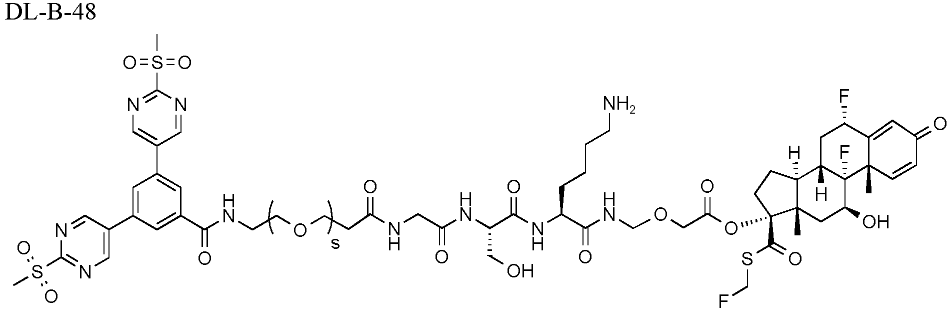 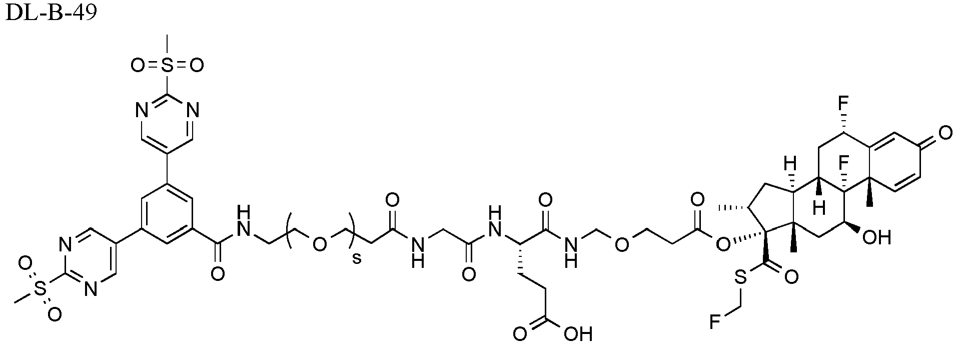 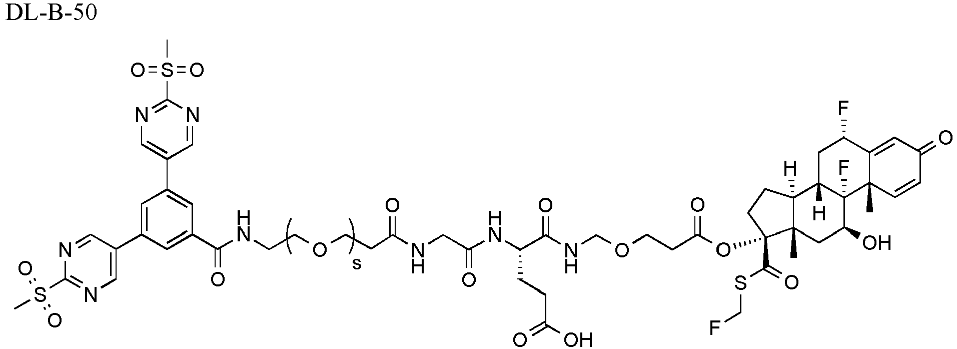 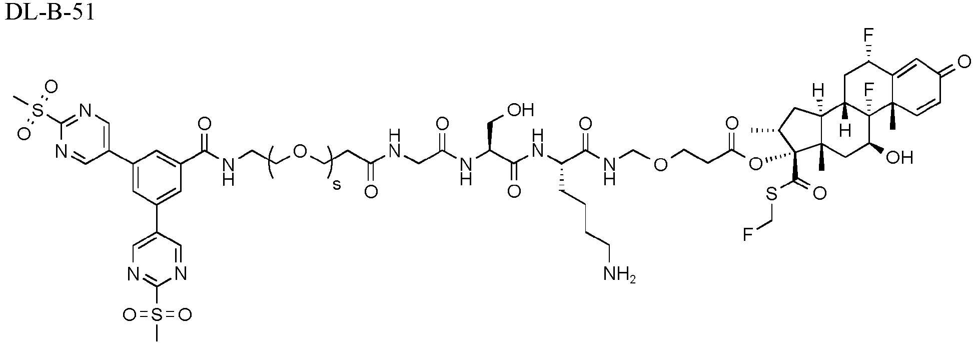 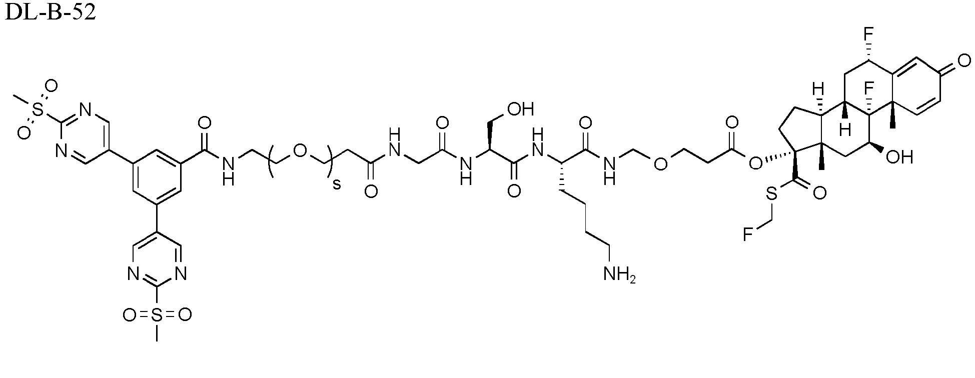 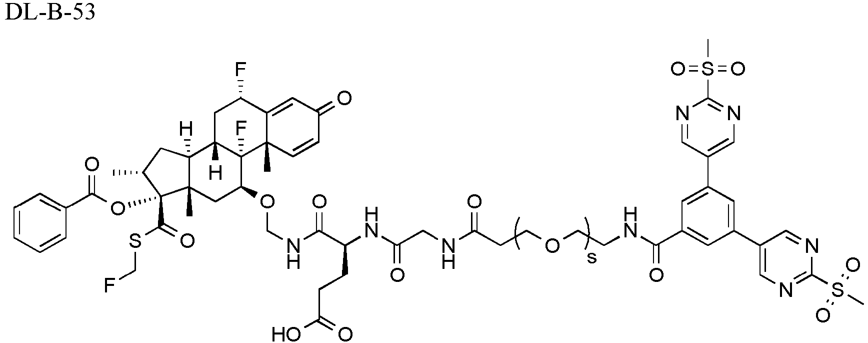 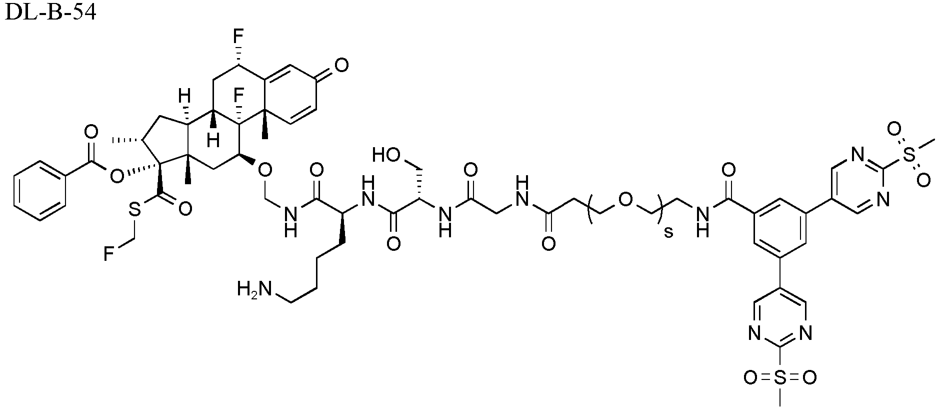 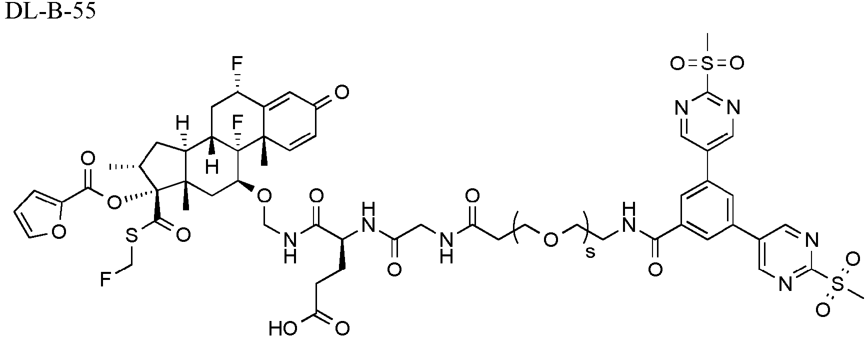 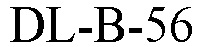 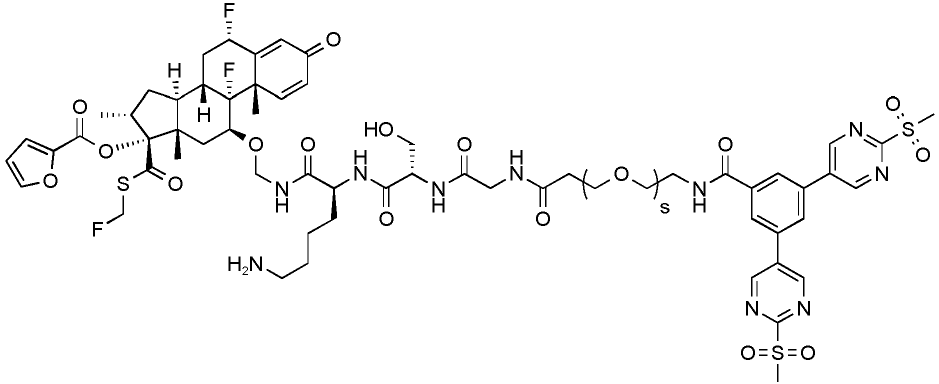 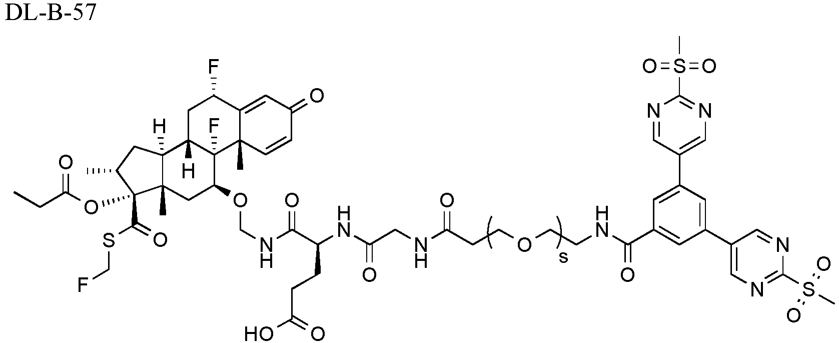 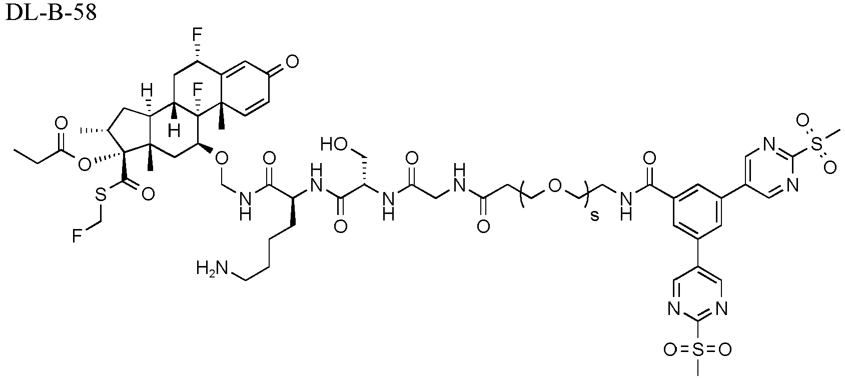 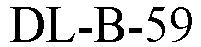 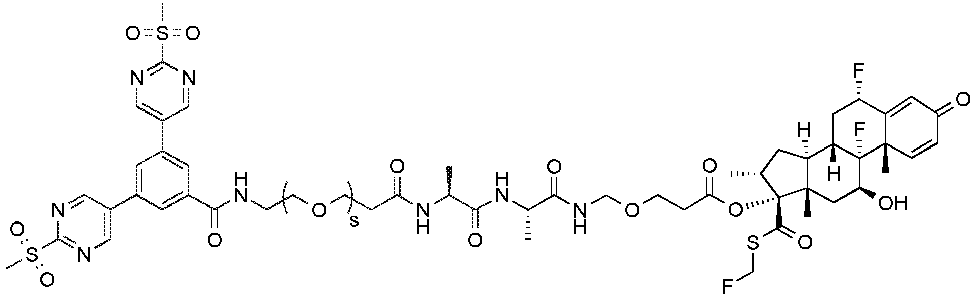 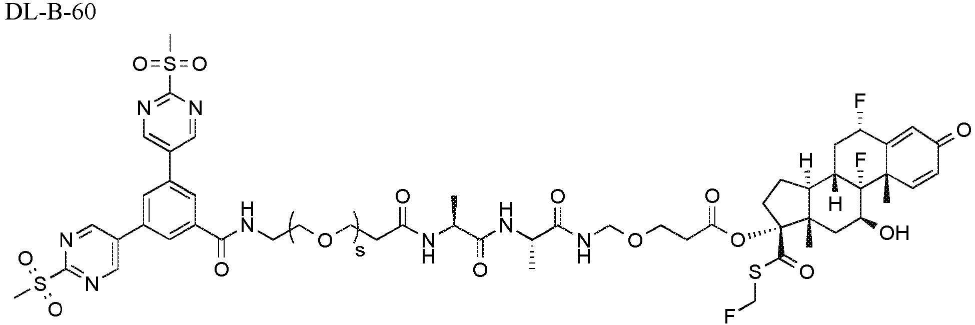 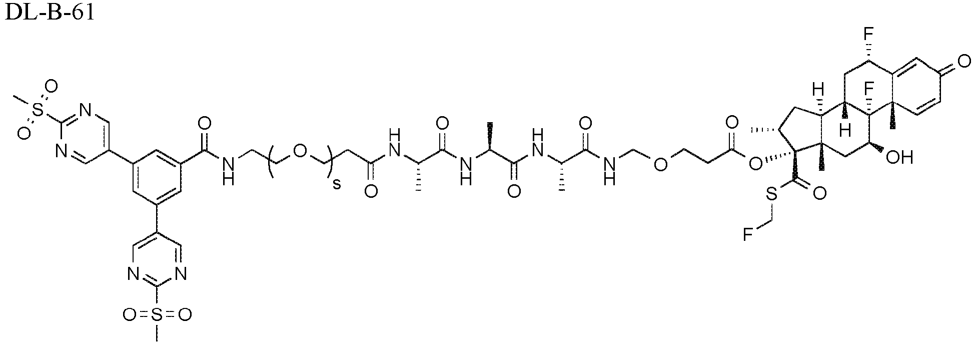 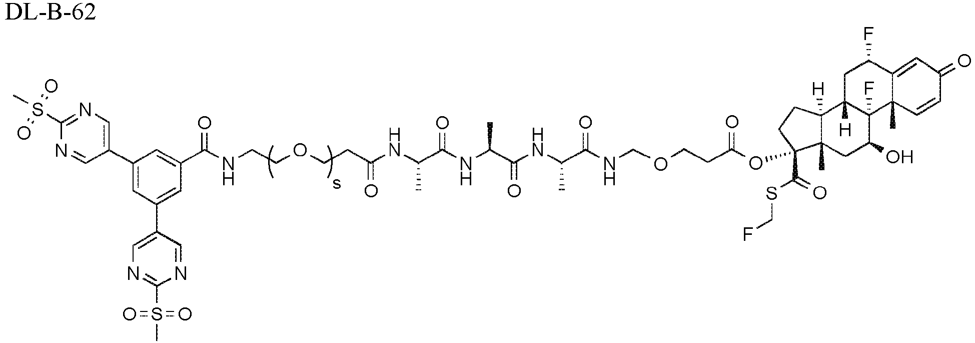 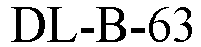 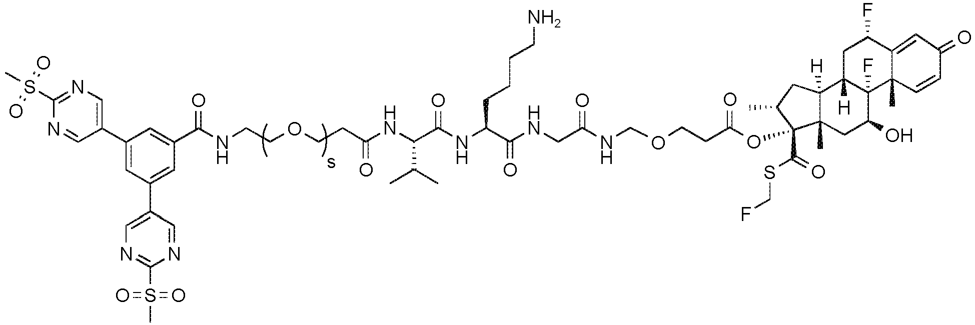 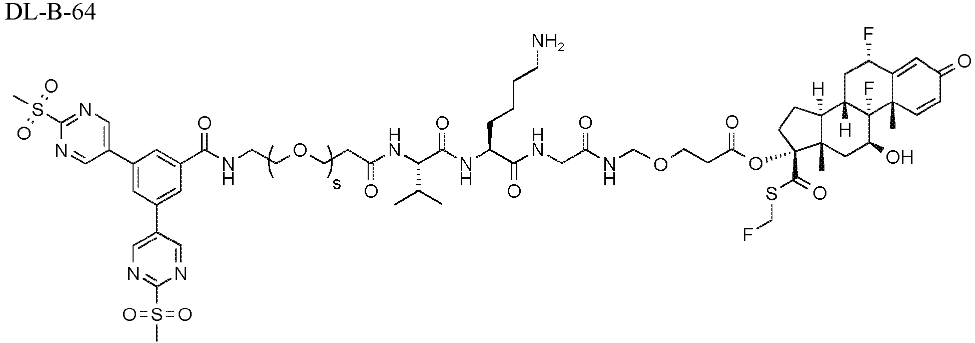 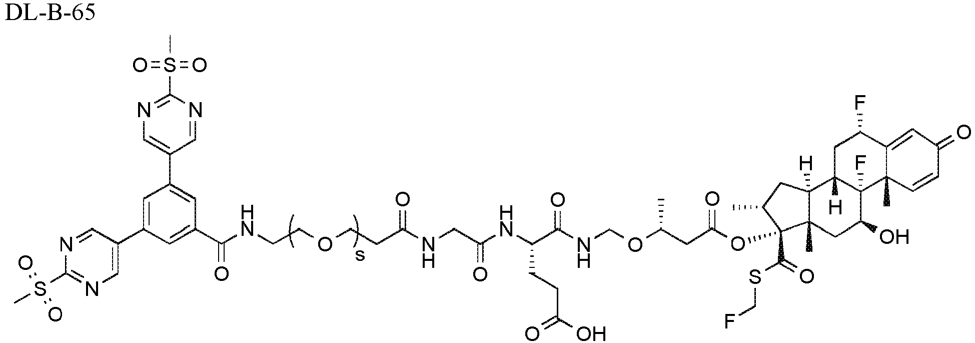 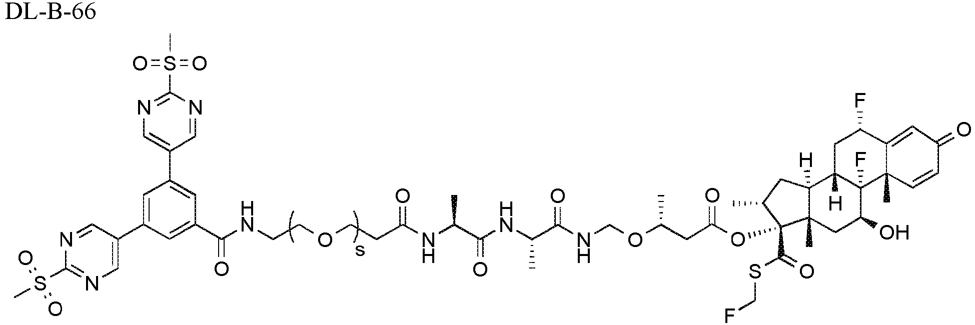 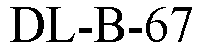 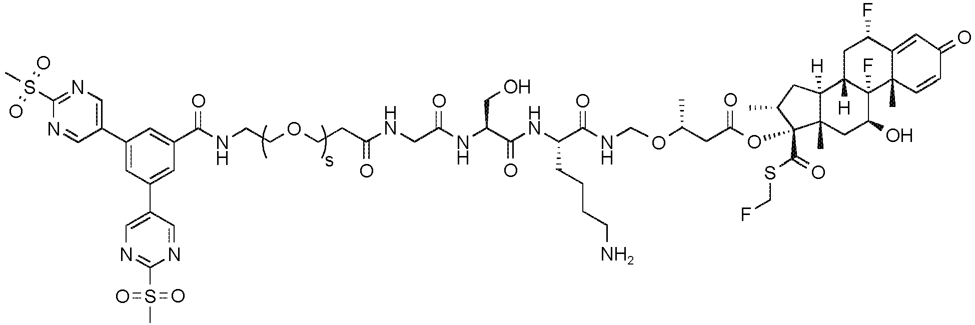 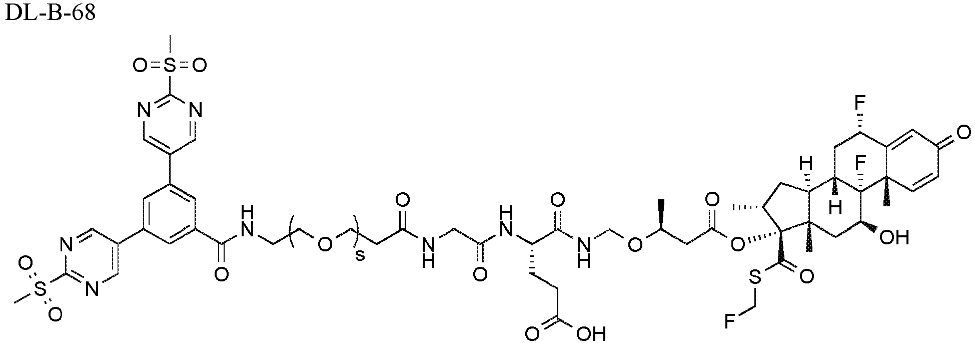 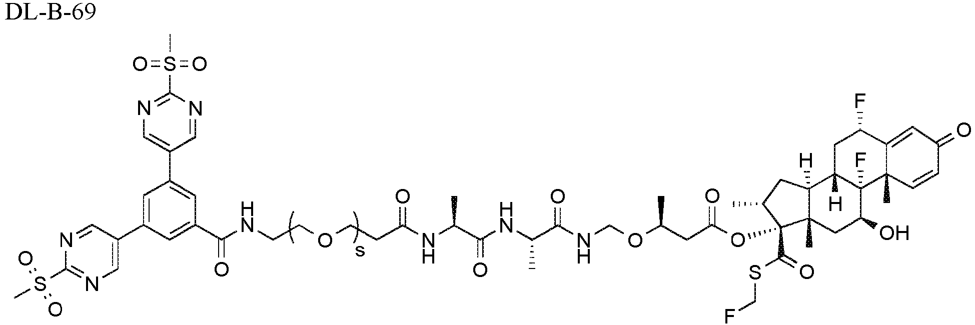 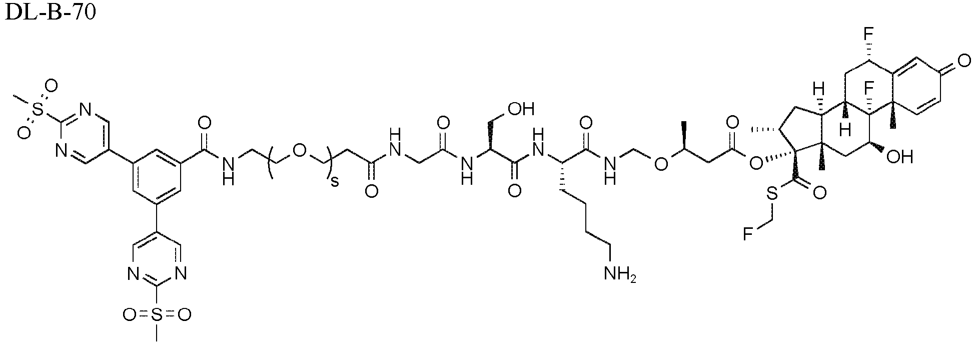 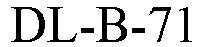 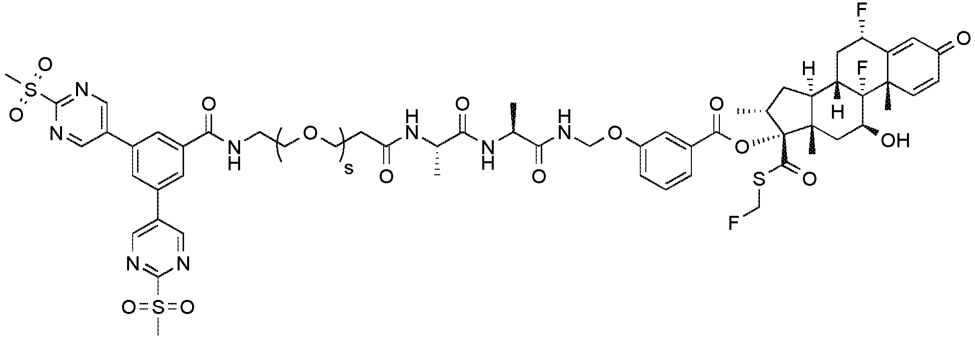 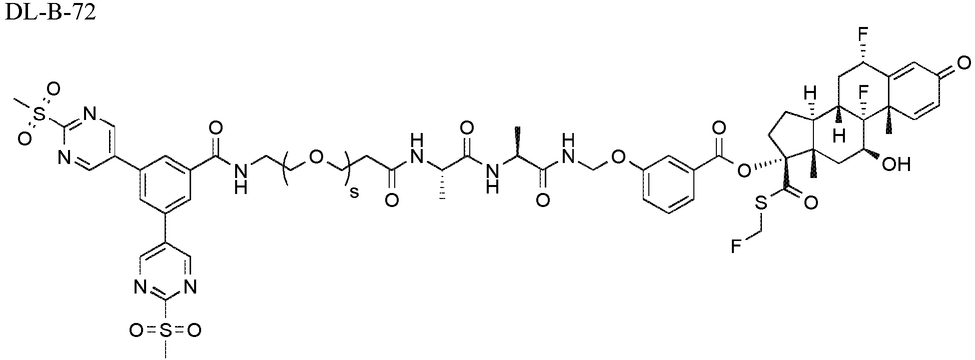 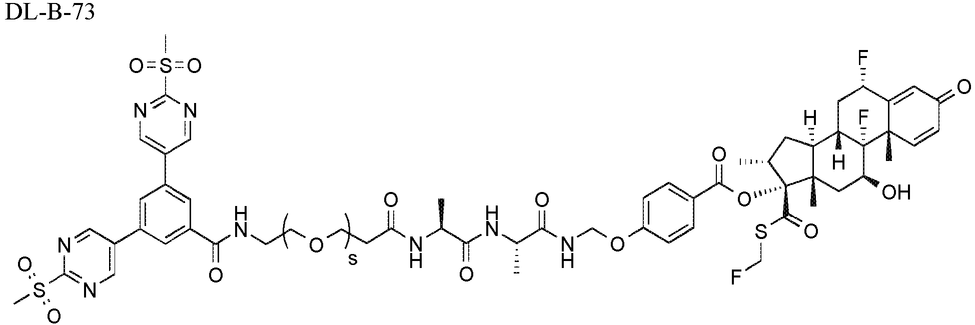 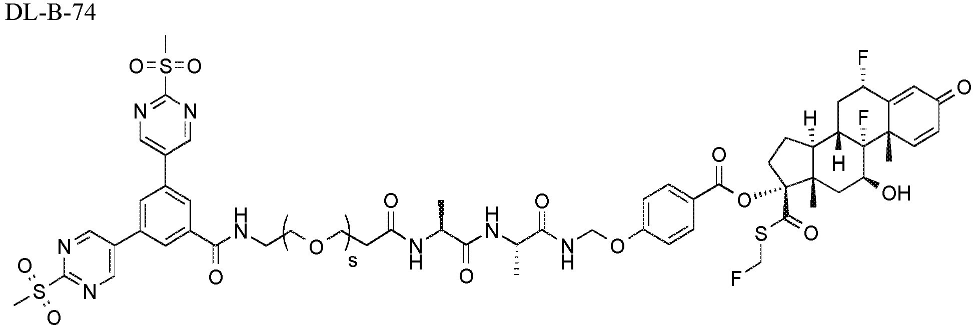 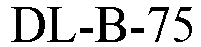 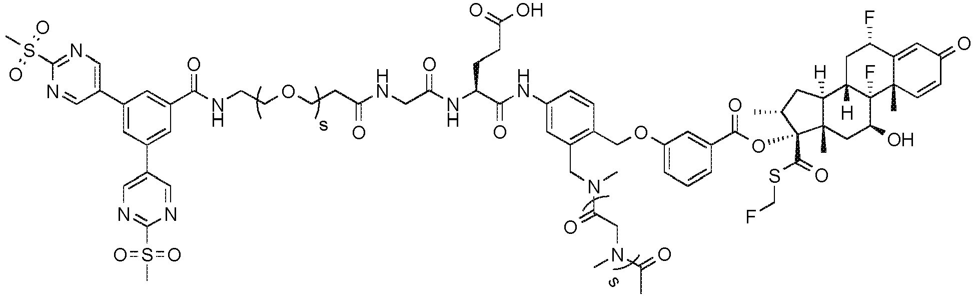 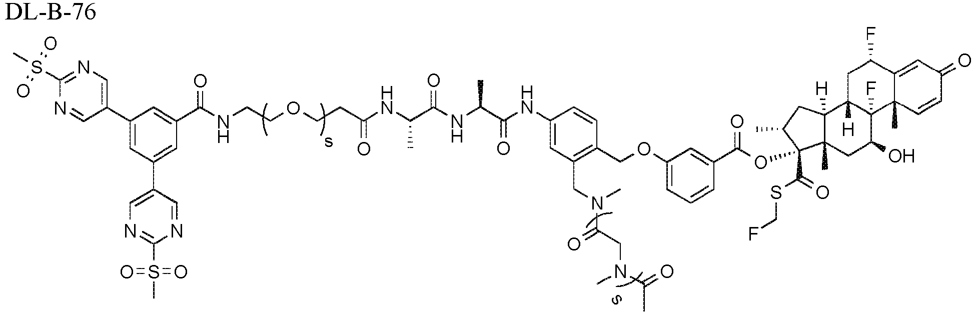 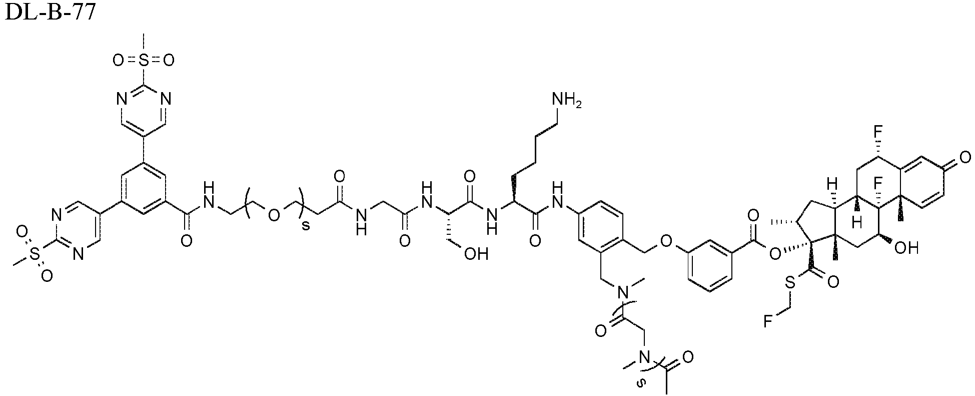 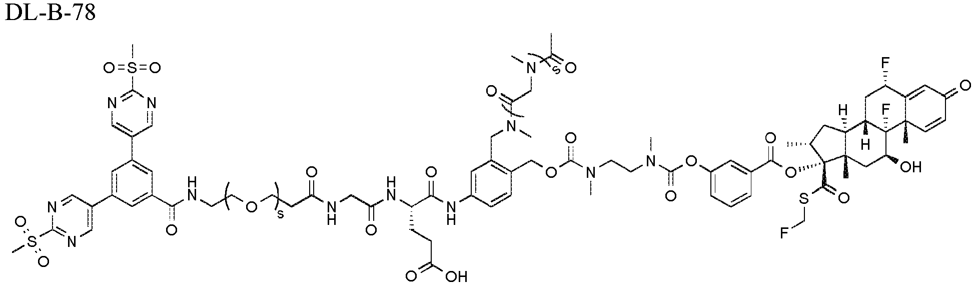 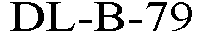 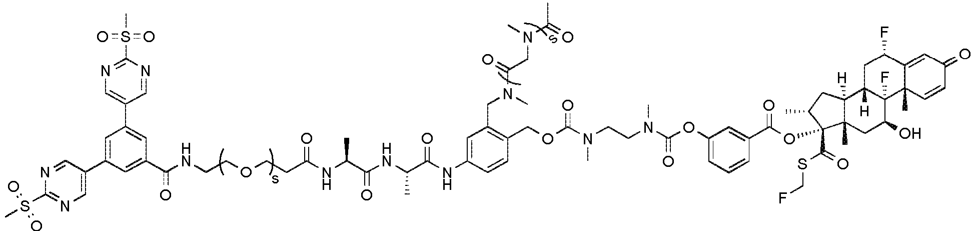 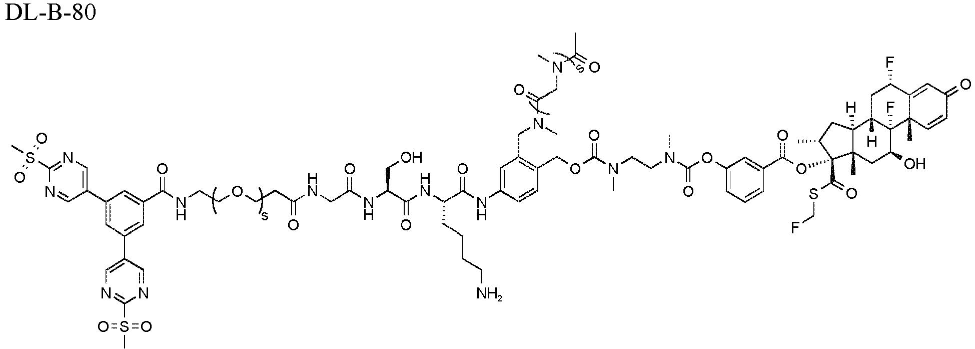 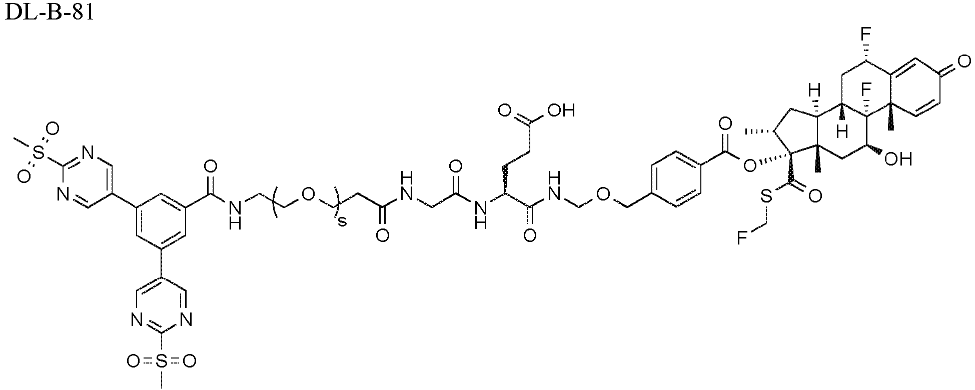 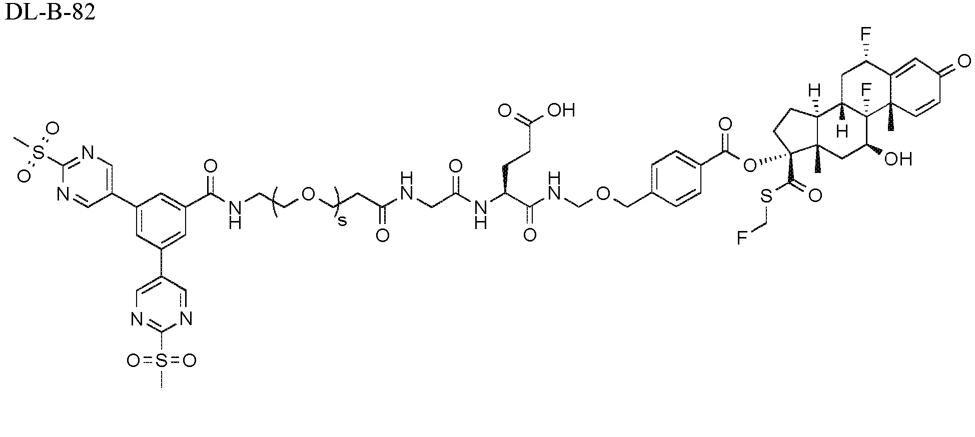 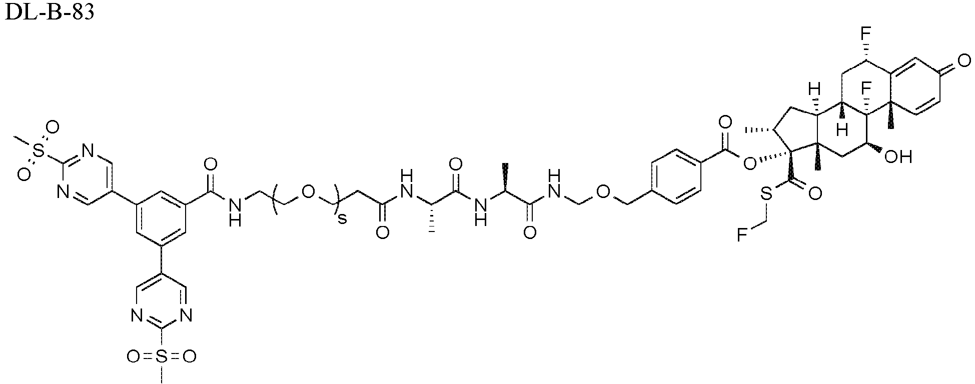 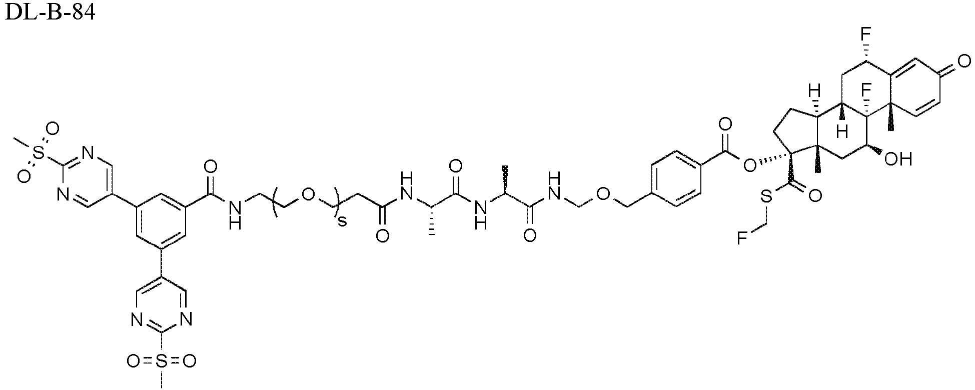 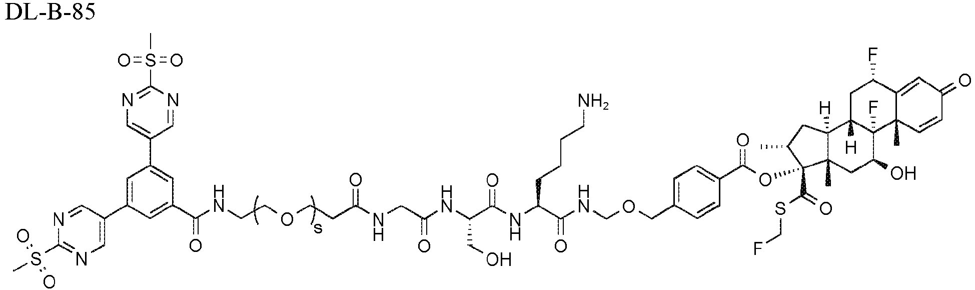 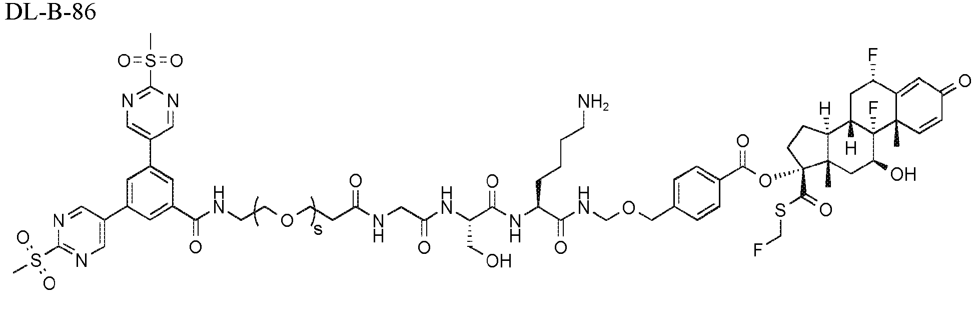 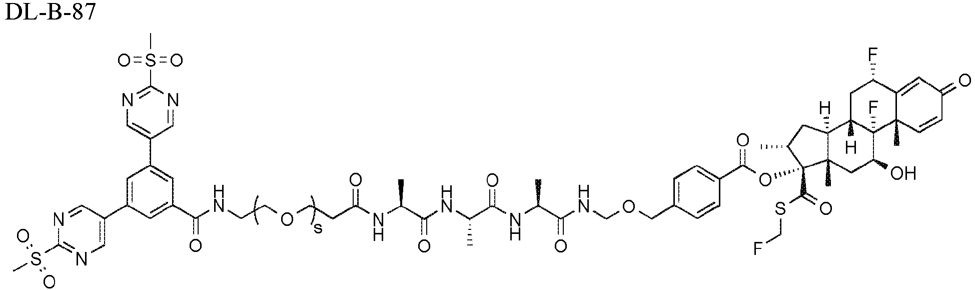 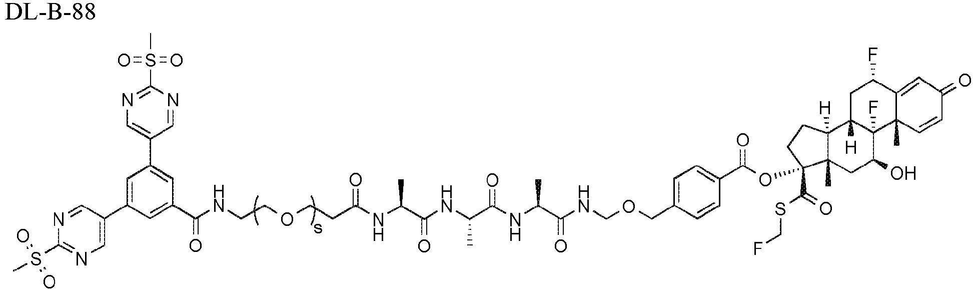 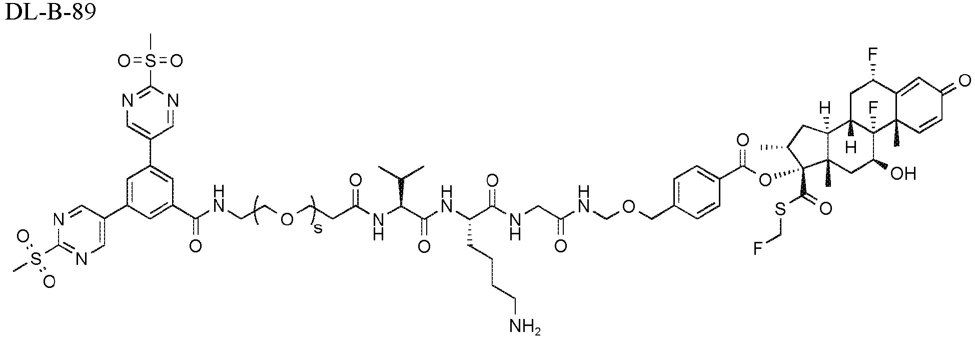 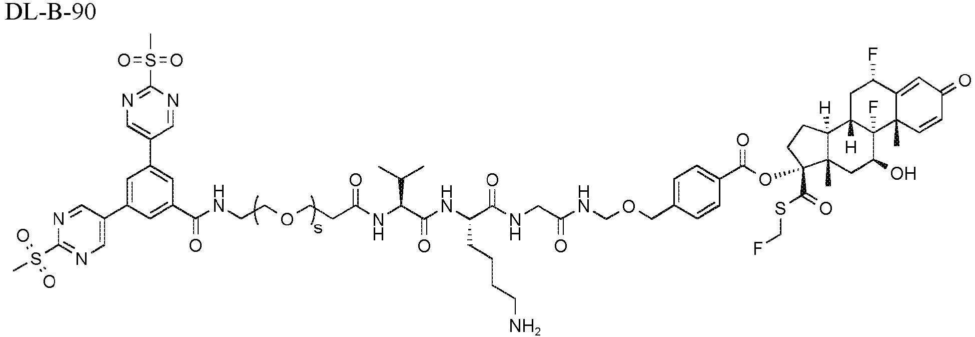 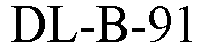 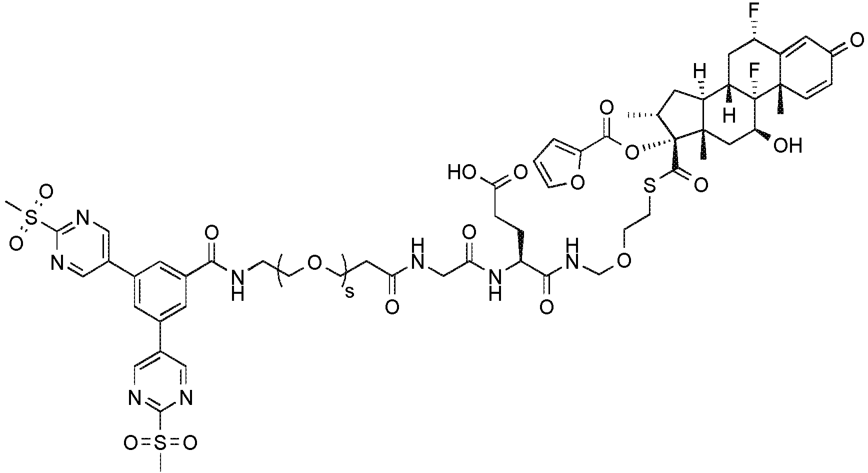 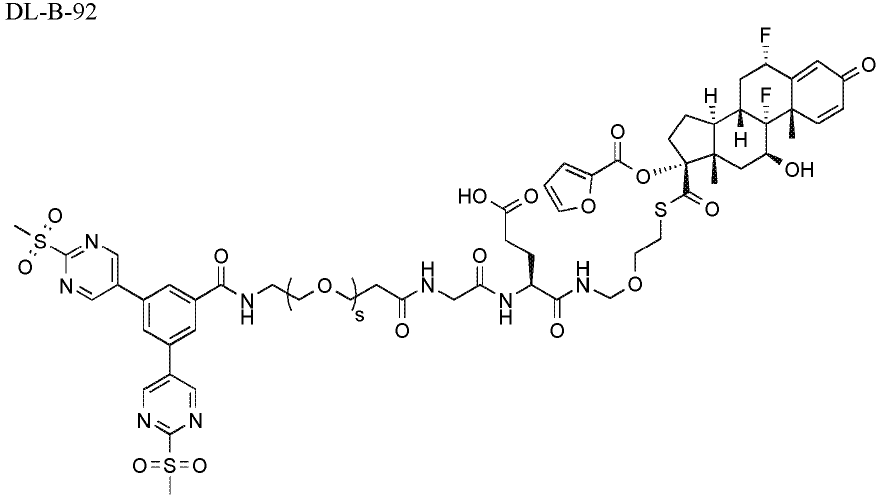 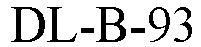 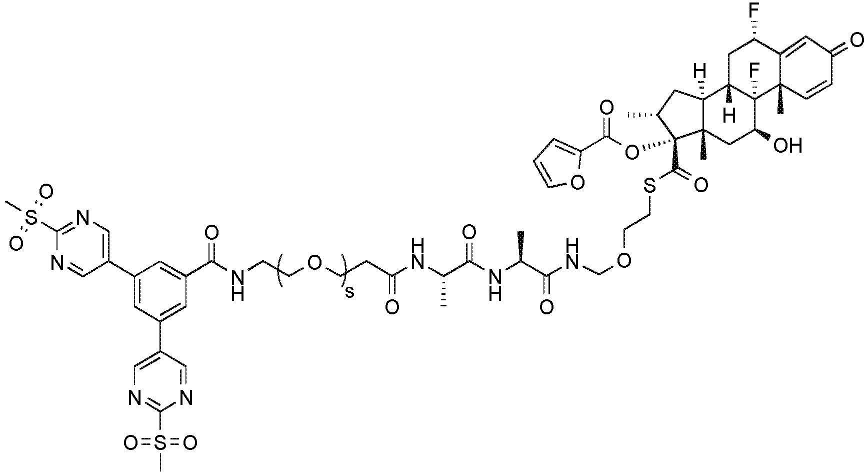 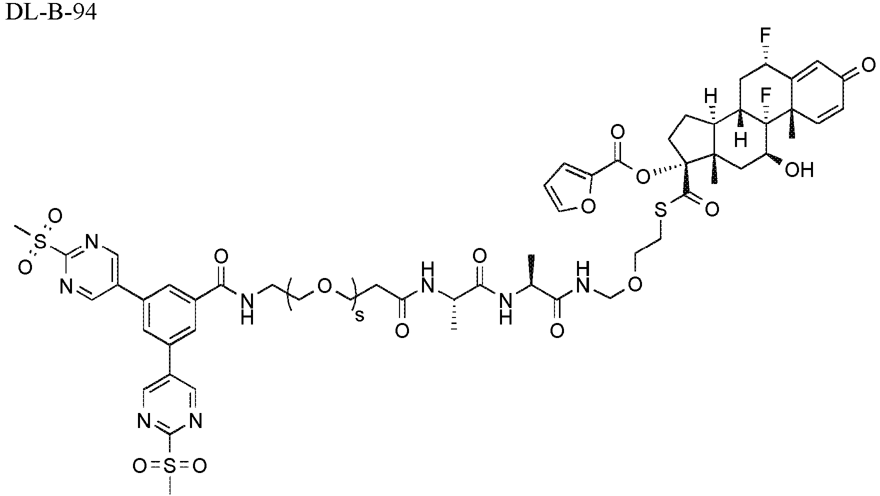 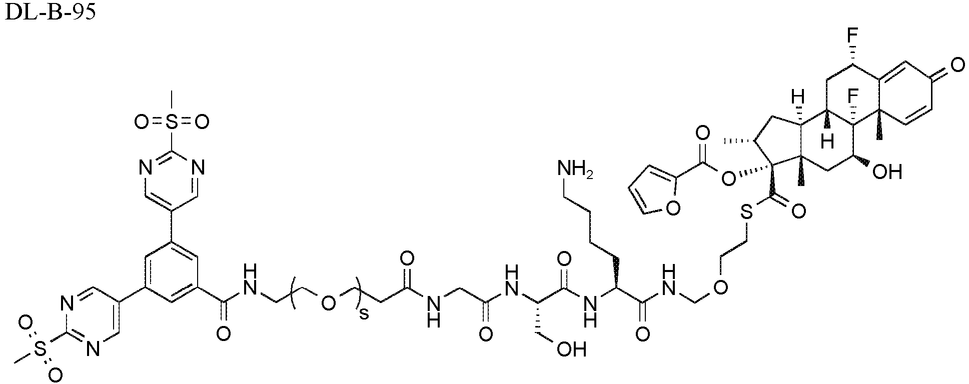 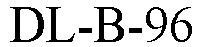 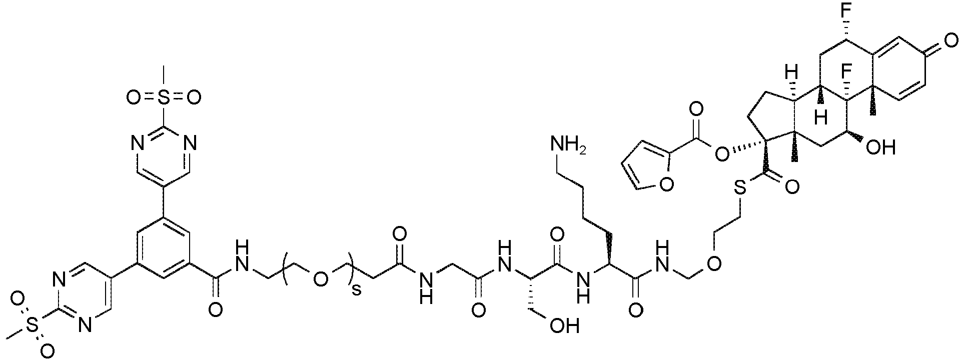 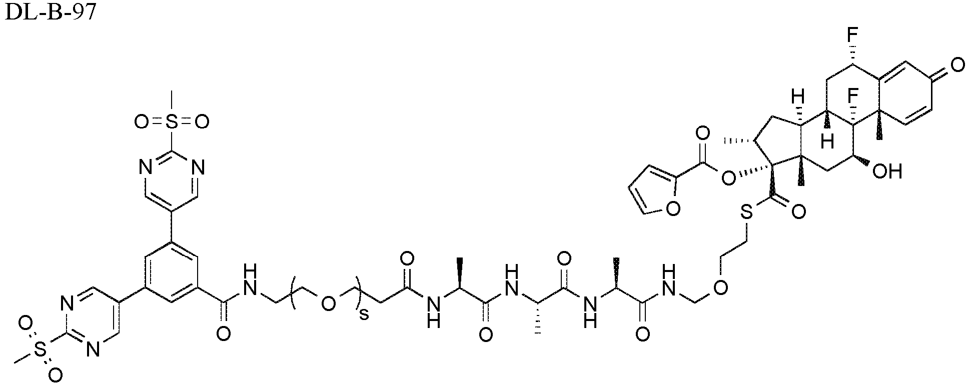 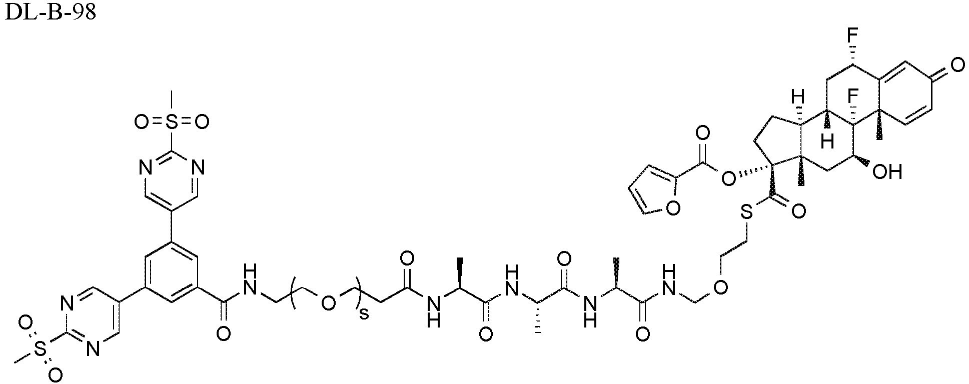 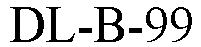 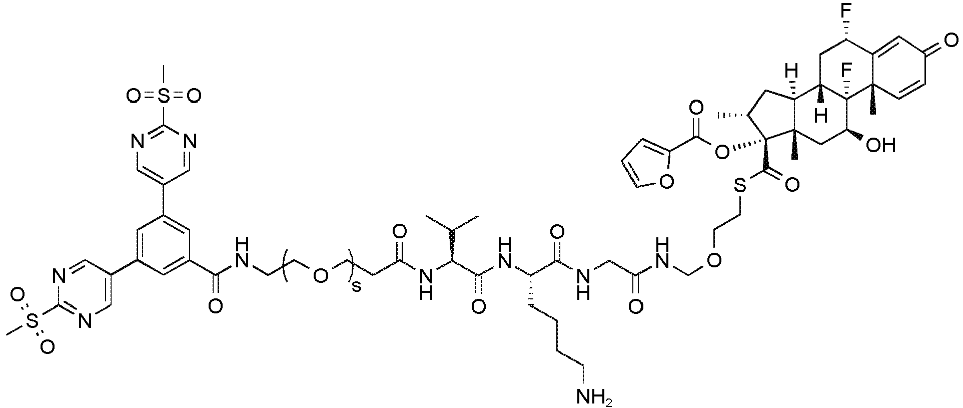 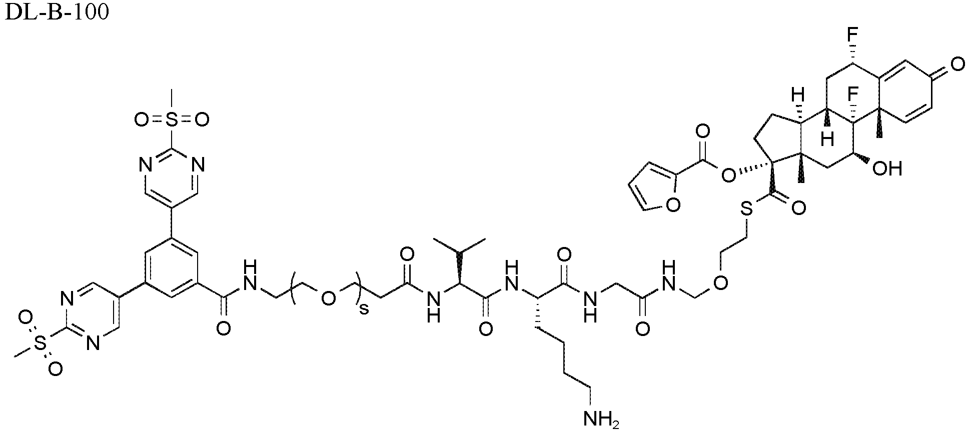 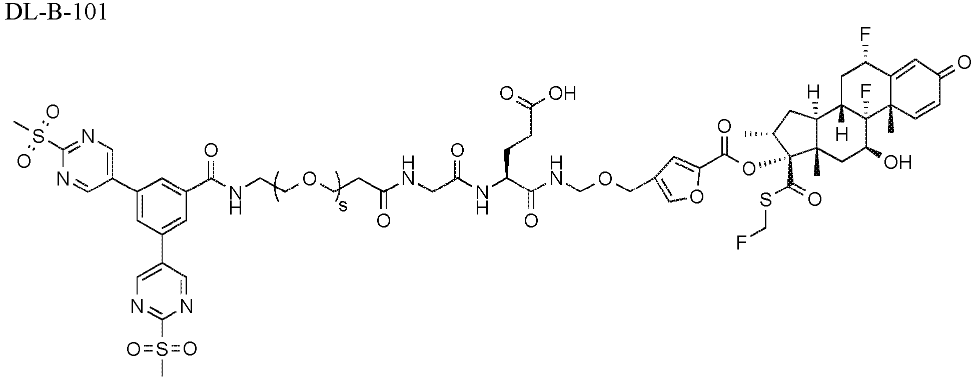 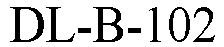 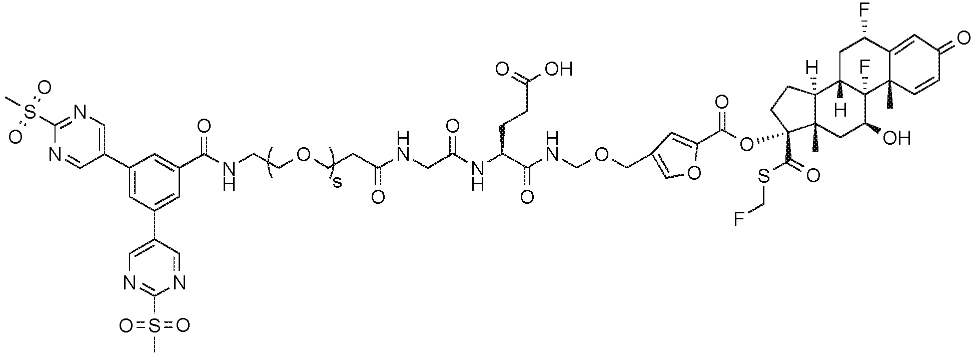 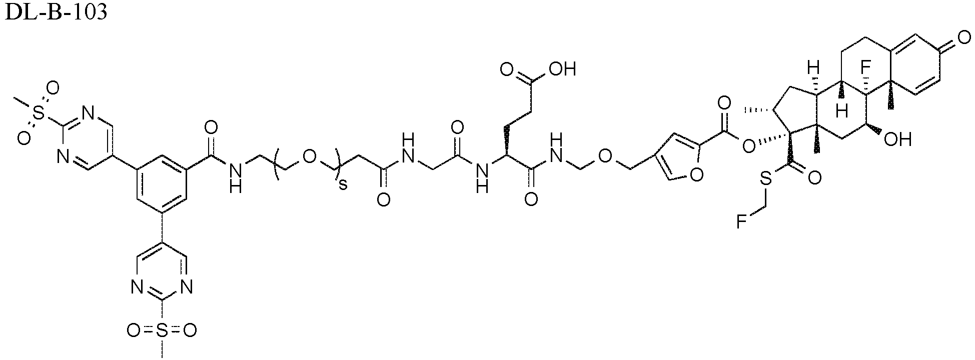 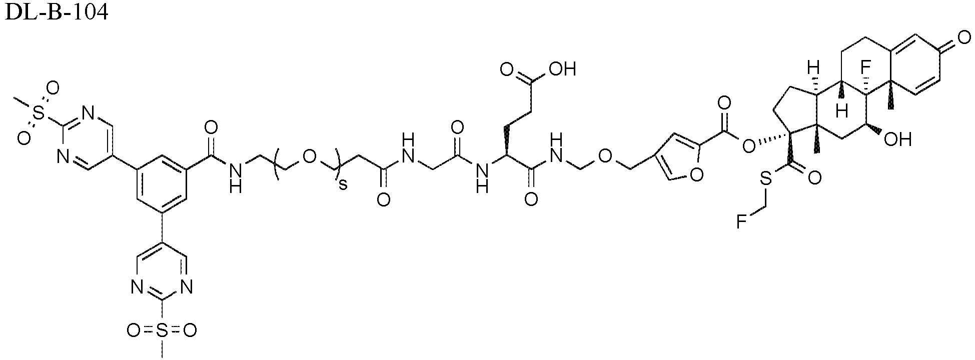 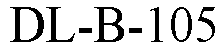 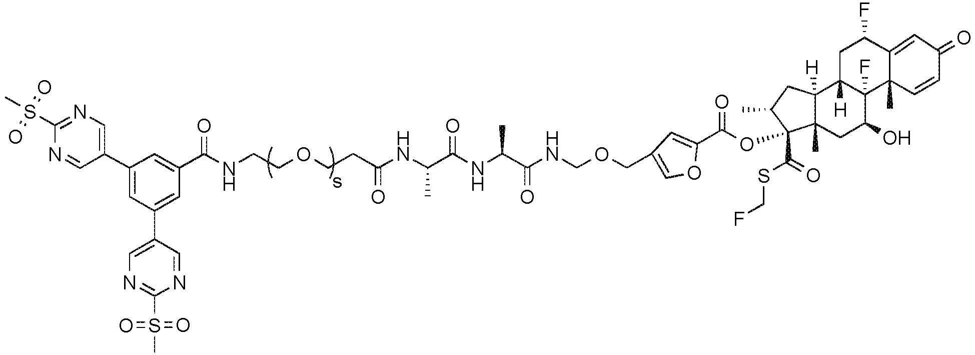 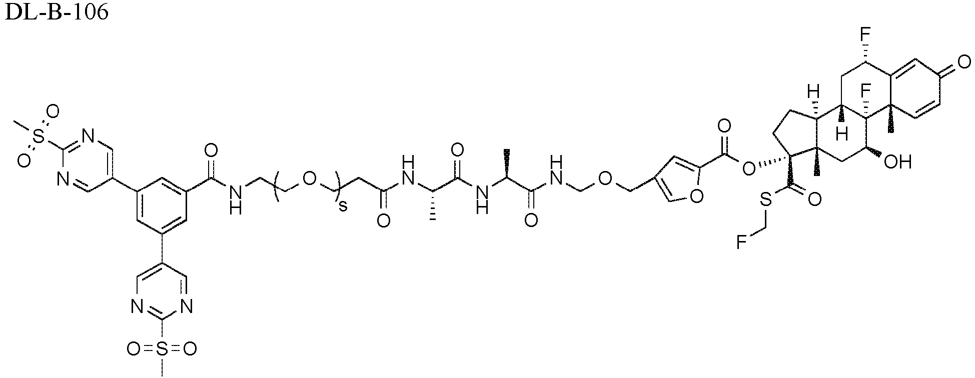 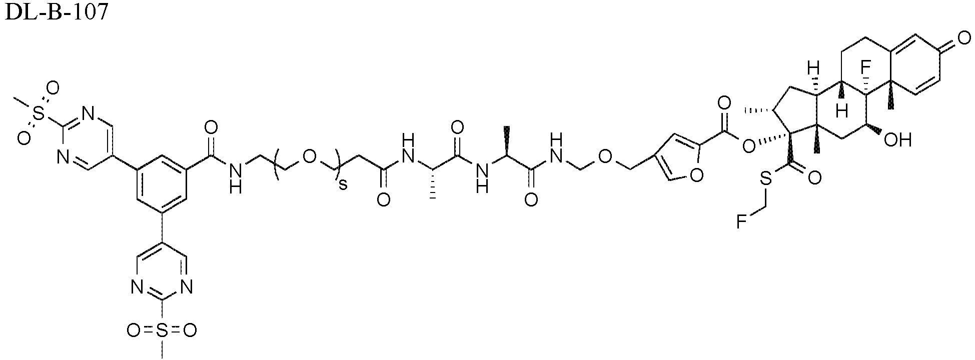 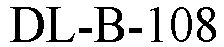 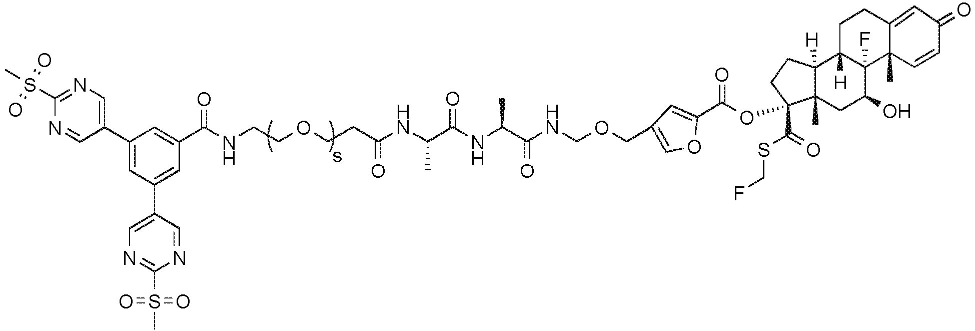 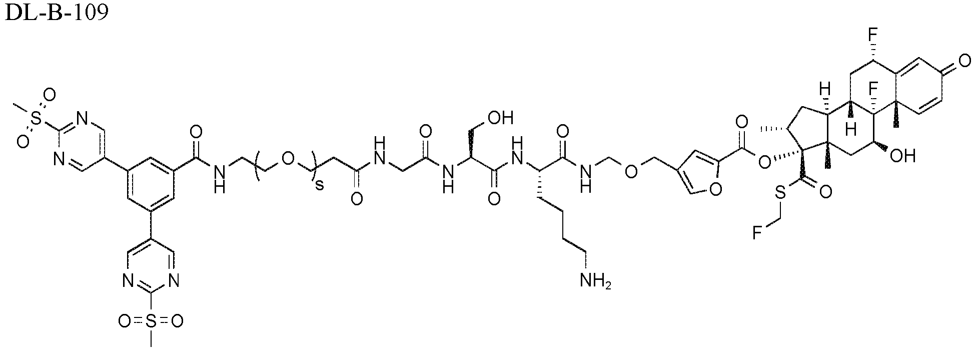 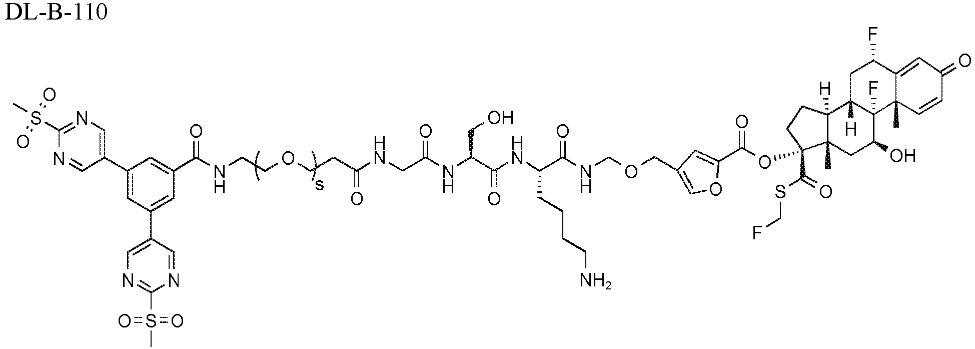 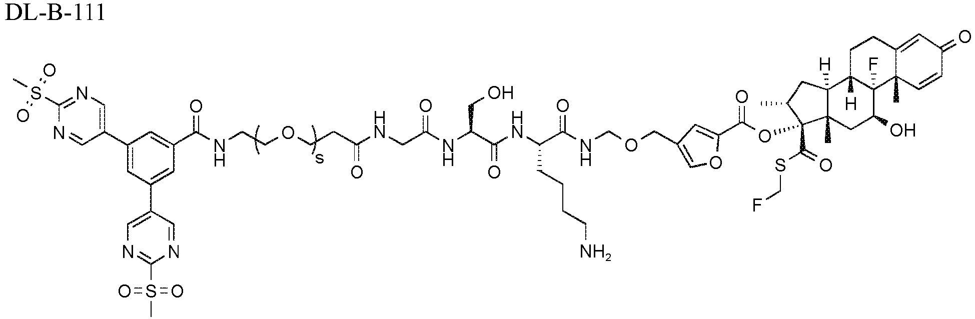 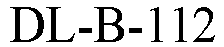 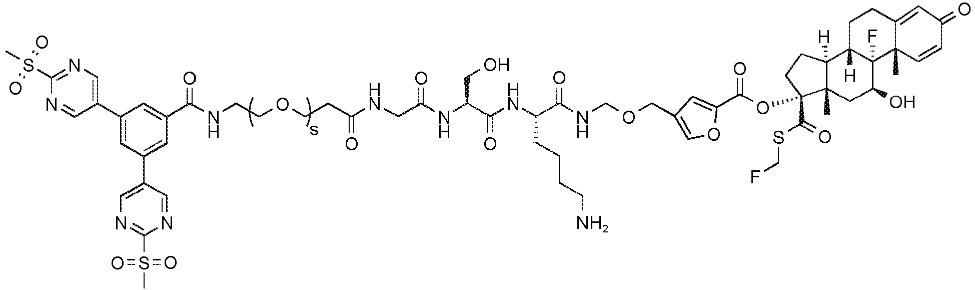 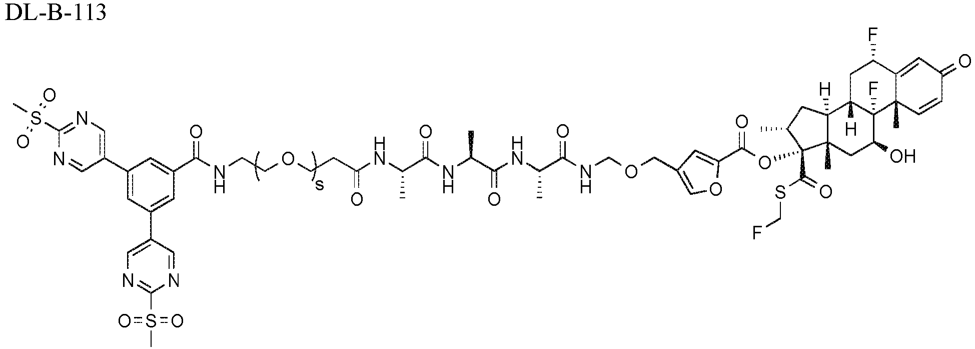 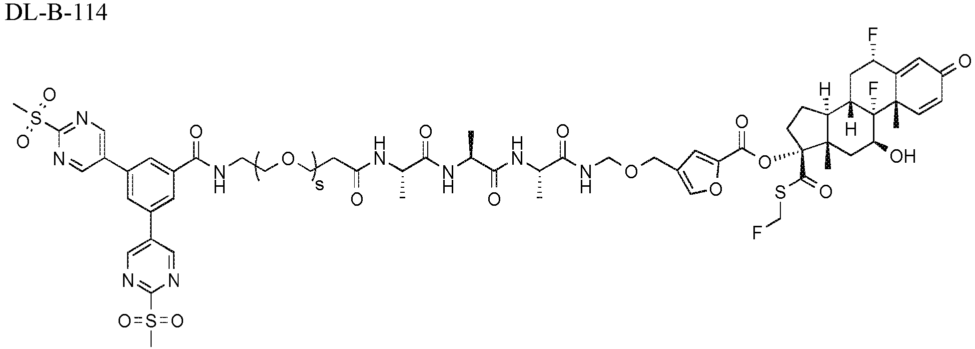 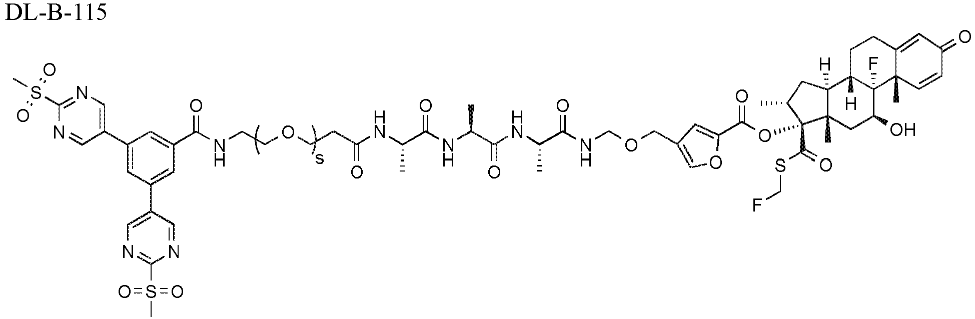 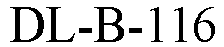 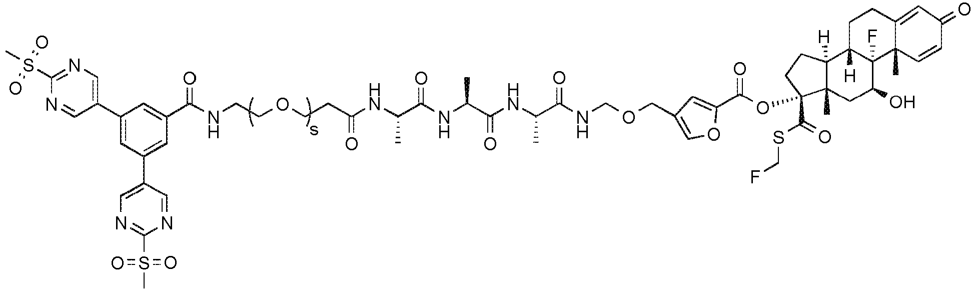 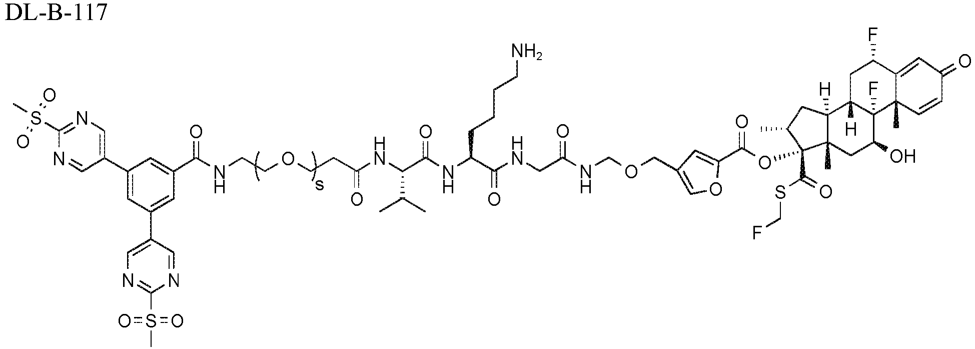 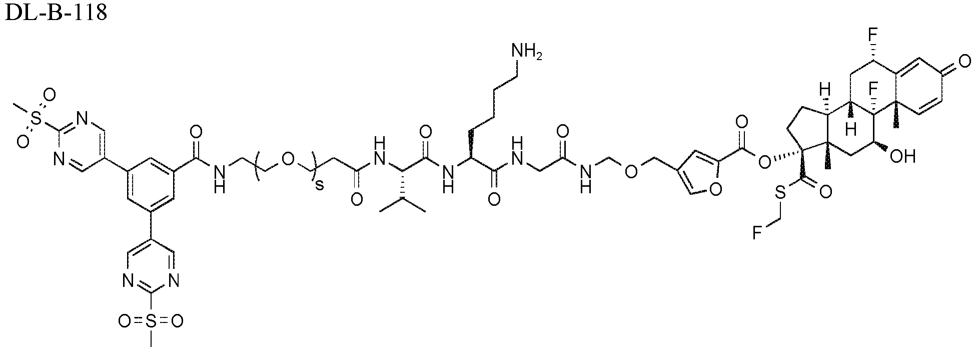 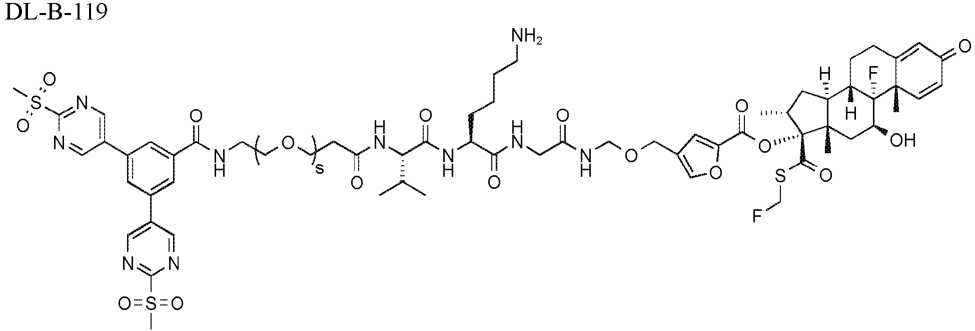 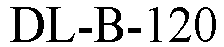 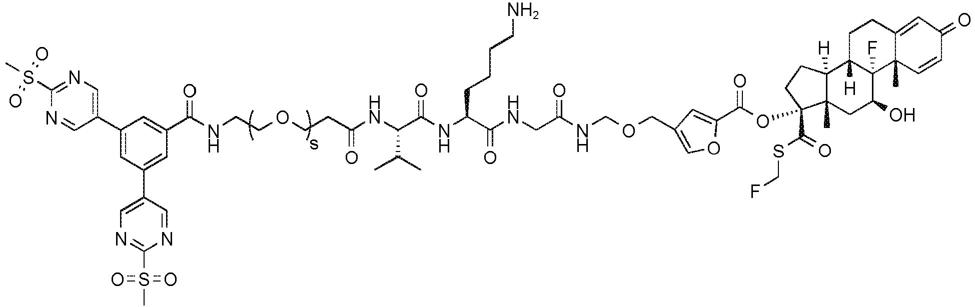 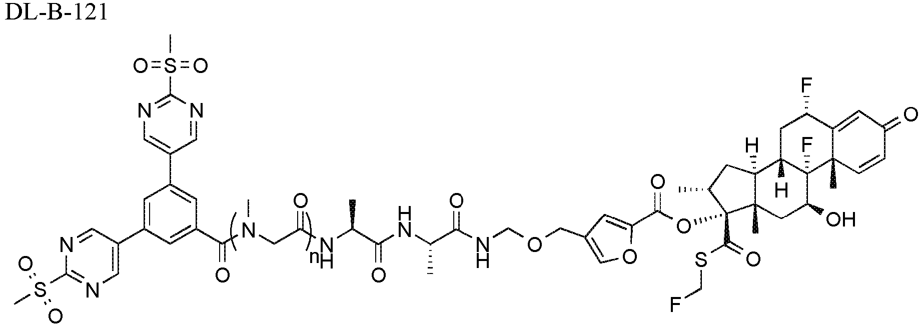 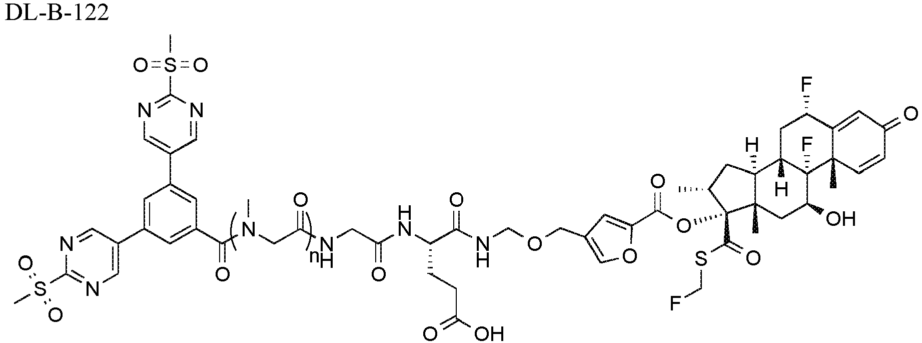 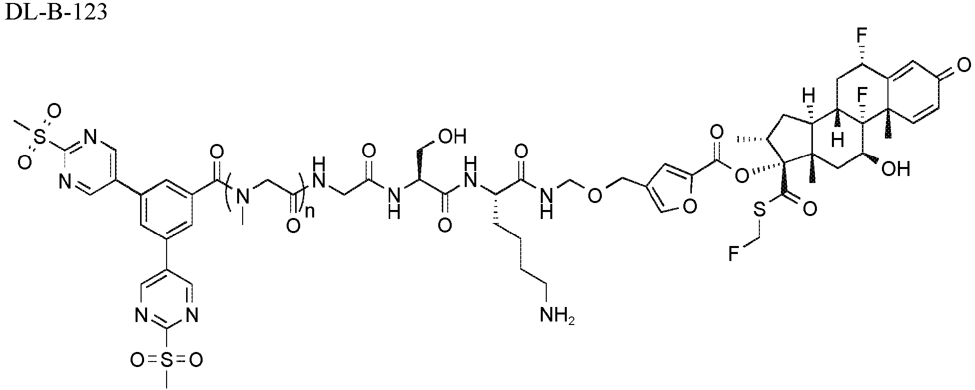 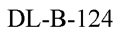 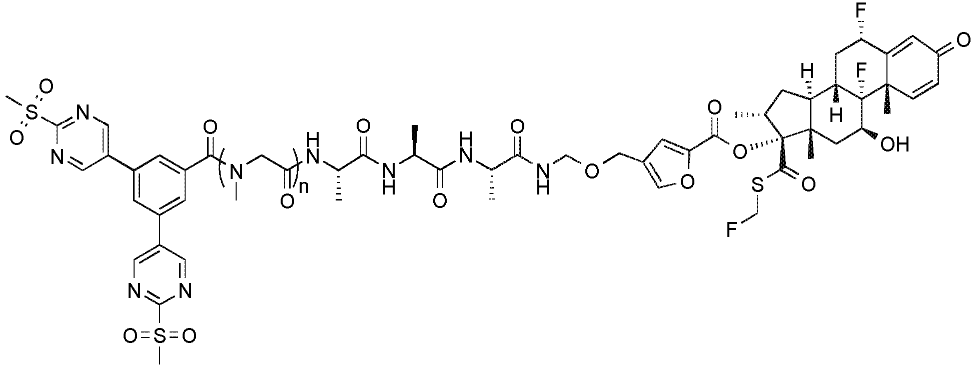 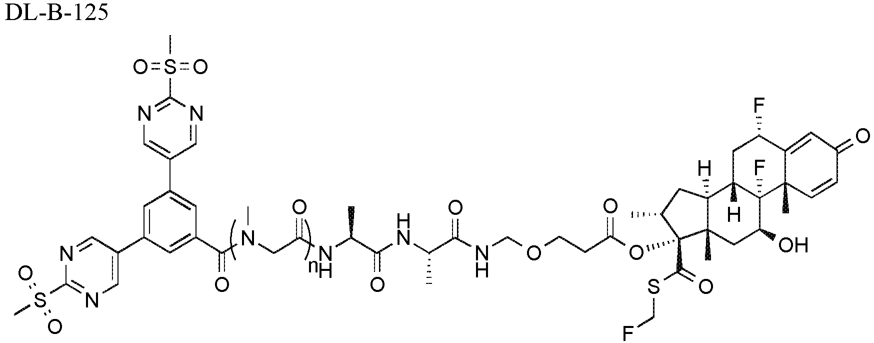 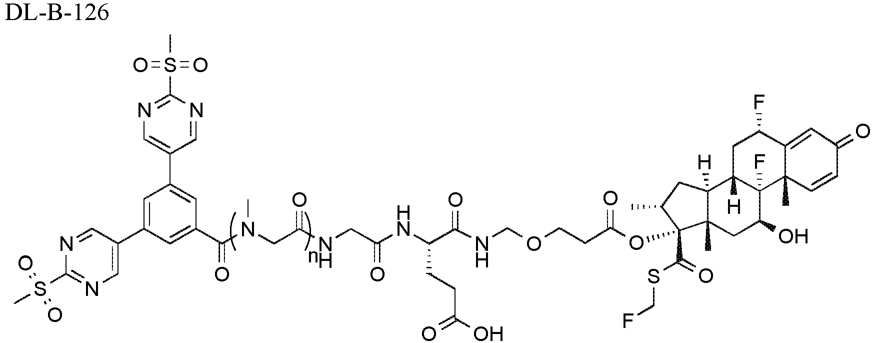 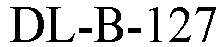 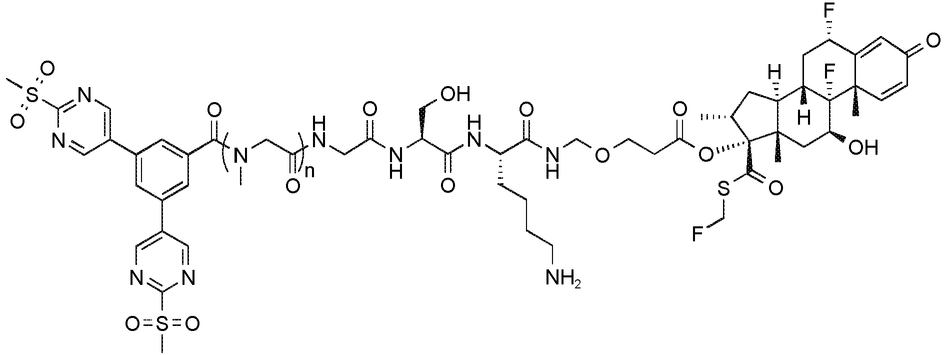 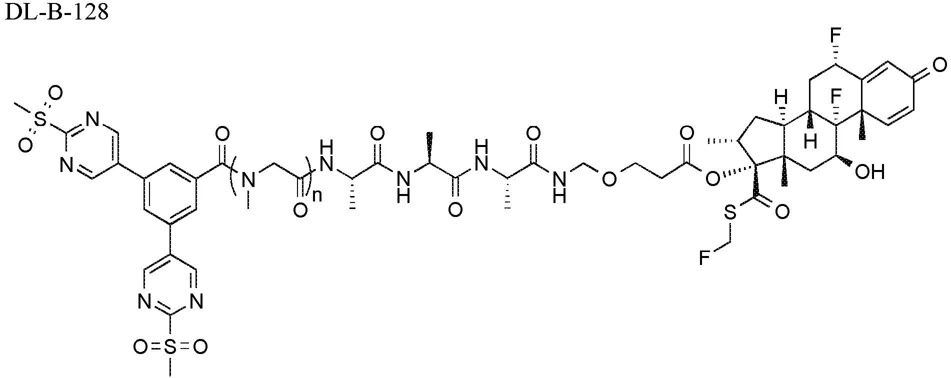 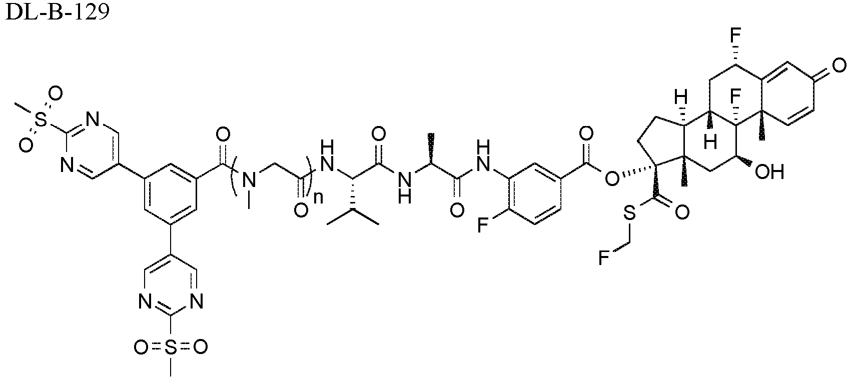 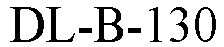 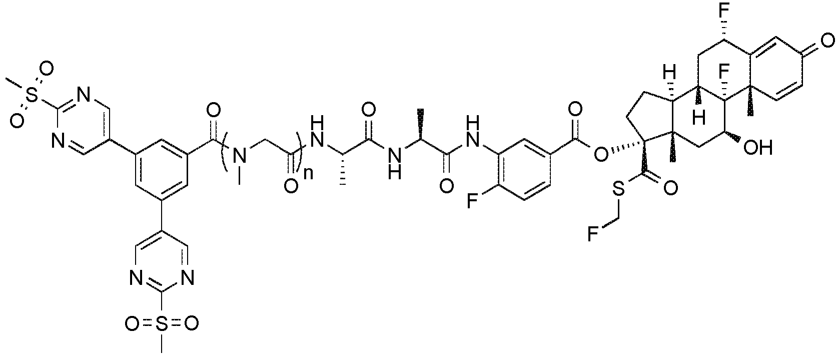 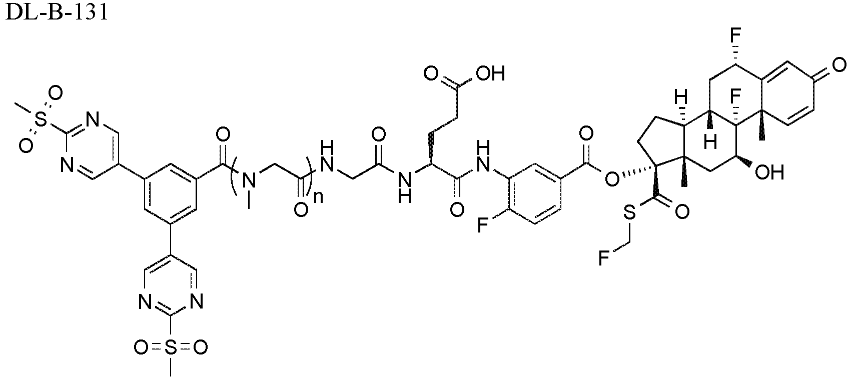 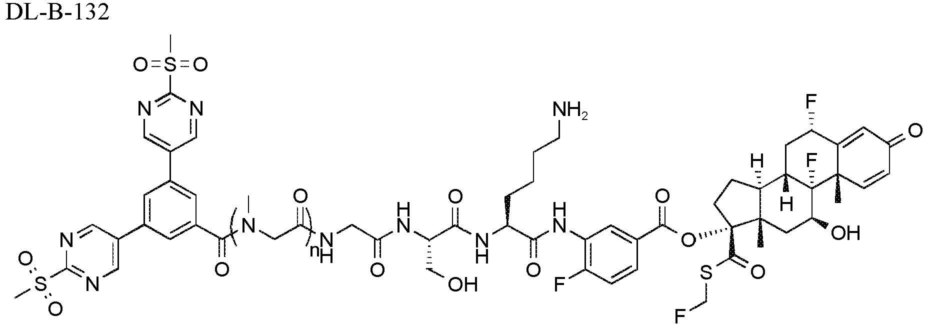 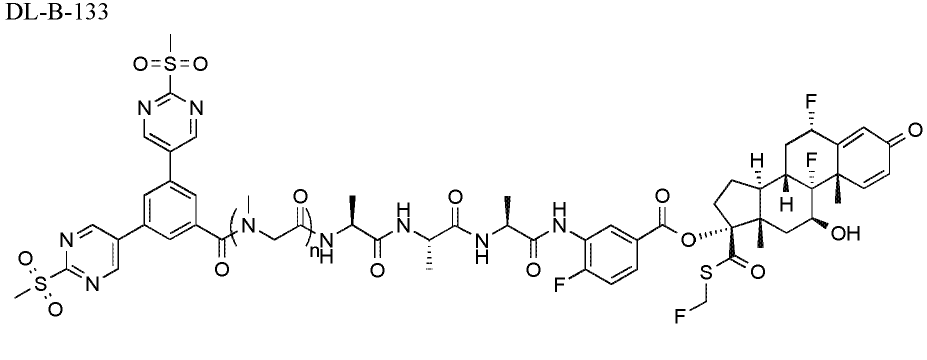 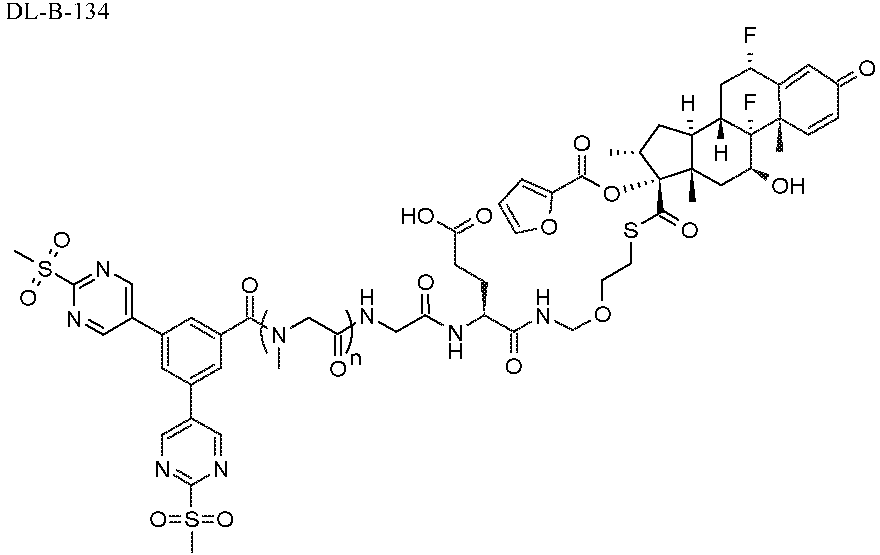 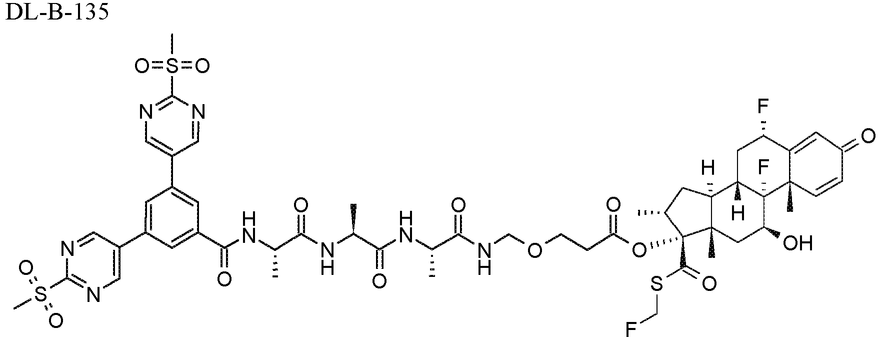 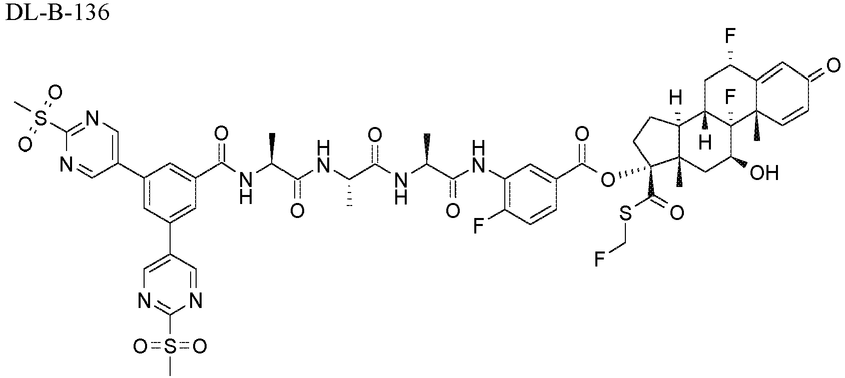 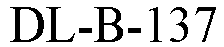 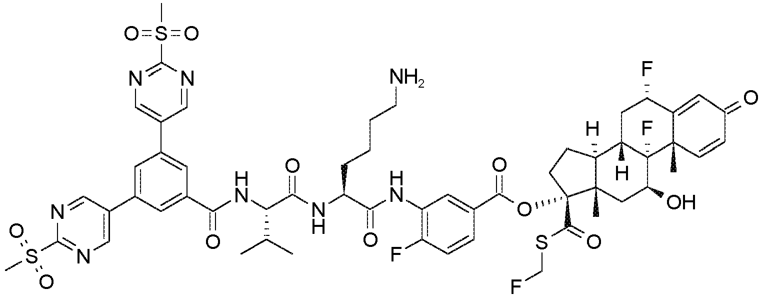 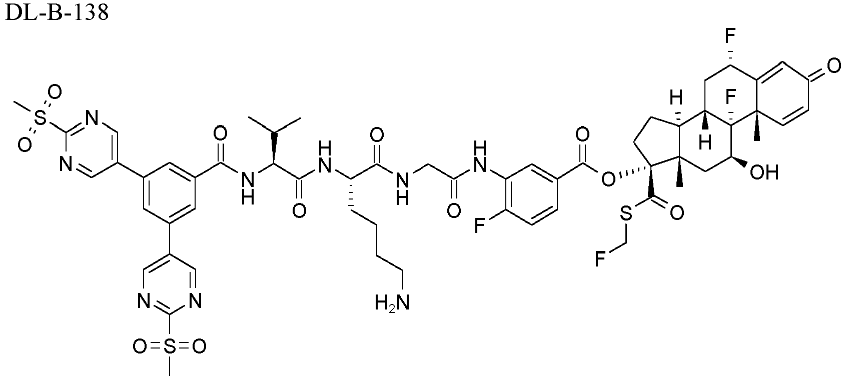 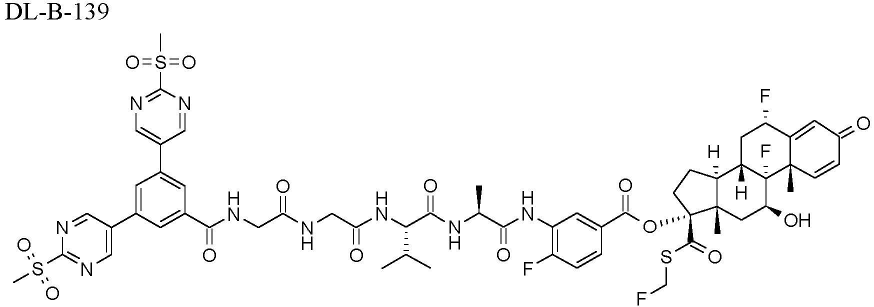 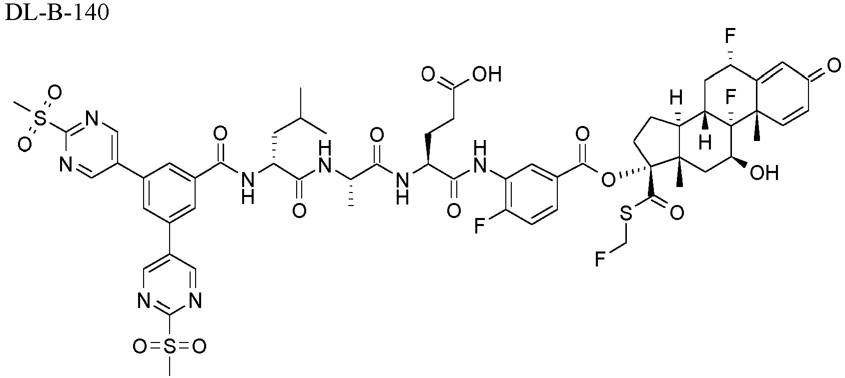 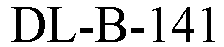 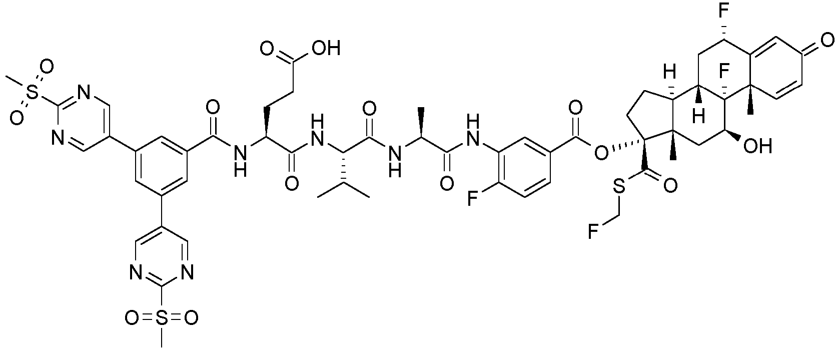 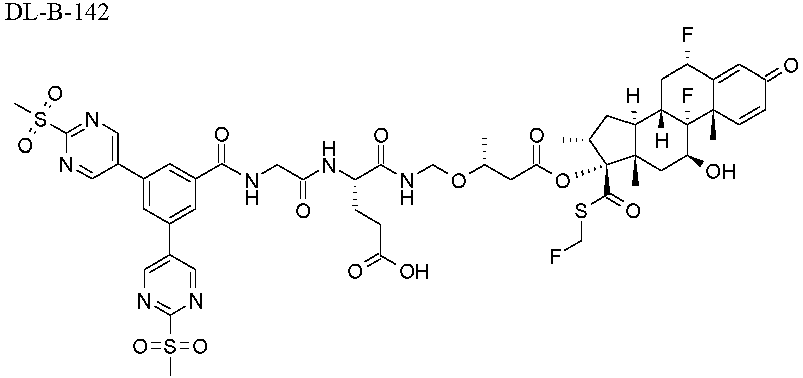 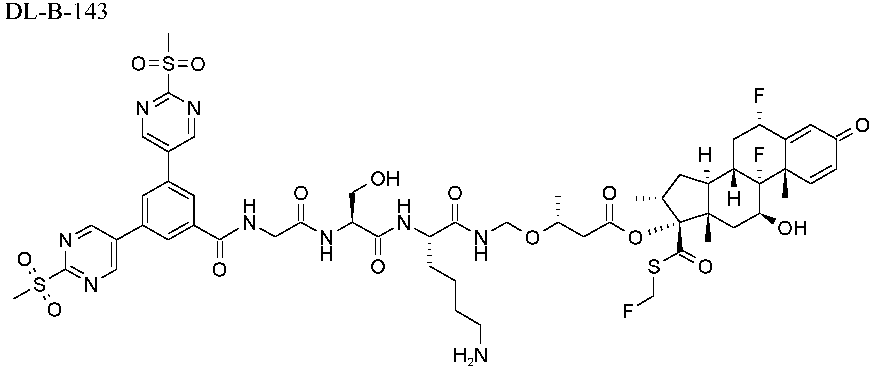 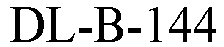 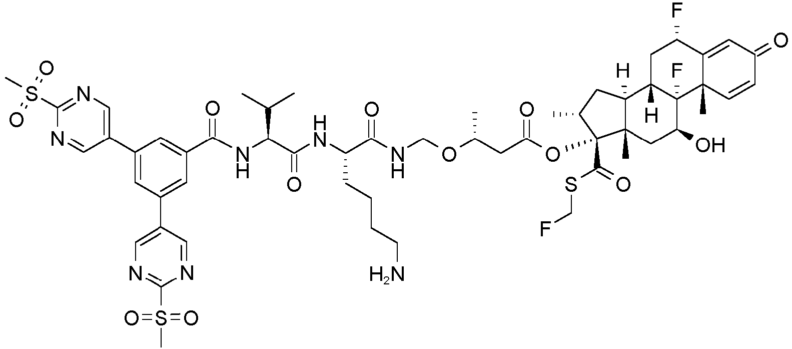 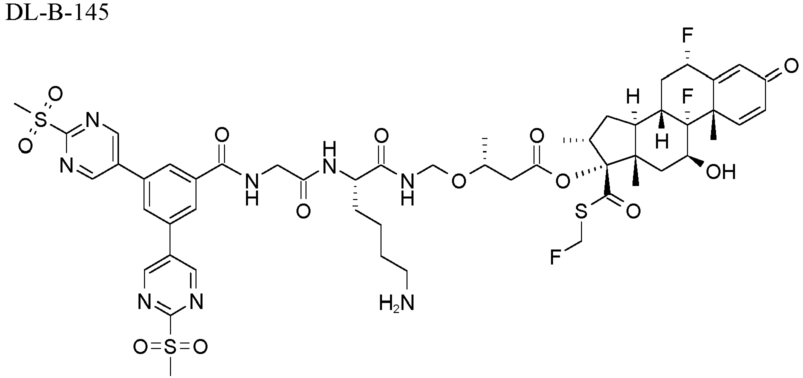 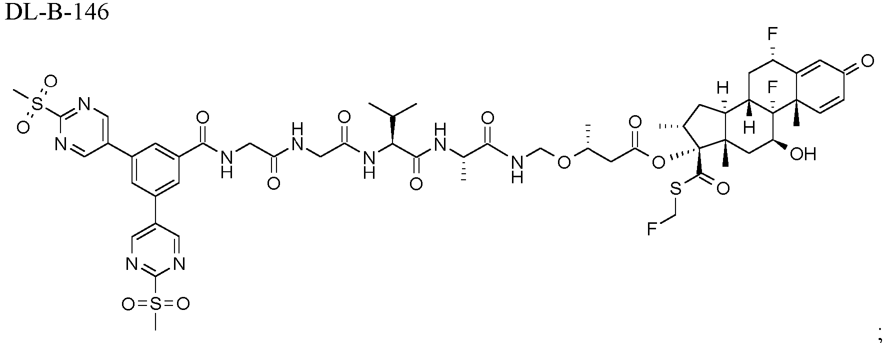 wherein, s is an integer selected from 1 to 20, preferably an integer selected from 1 to 10.

In some embodiments, the "drug-linker" compound in a free form is selected from a group consisting of: 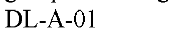 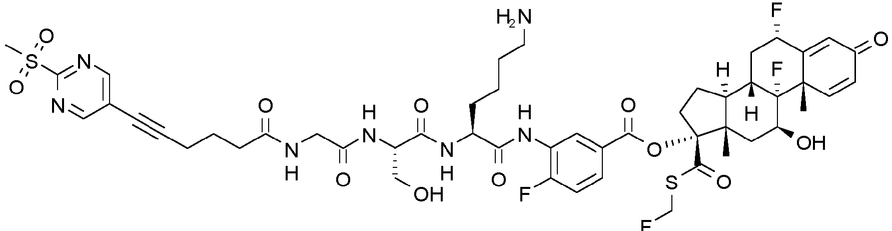 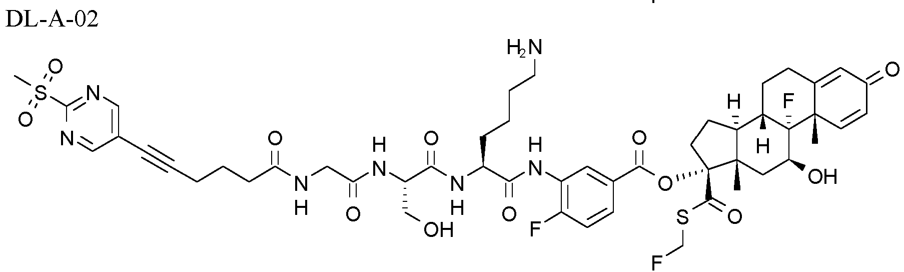 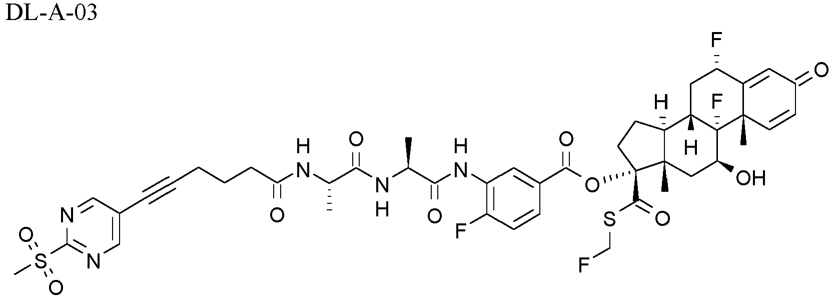 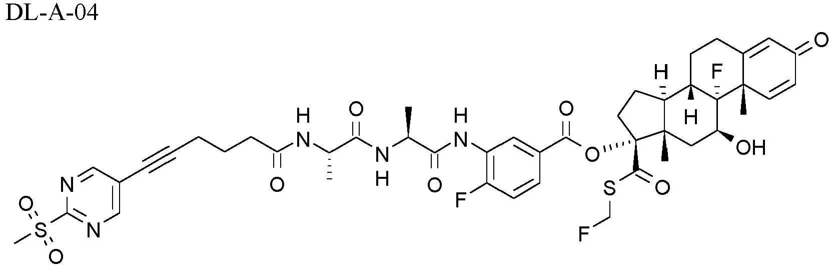 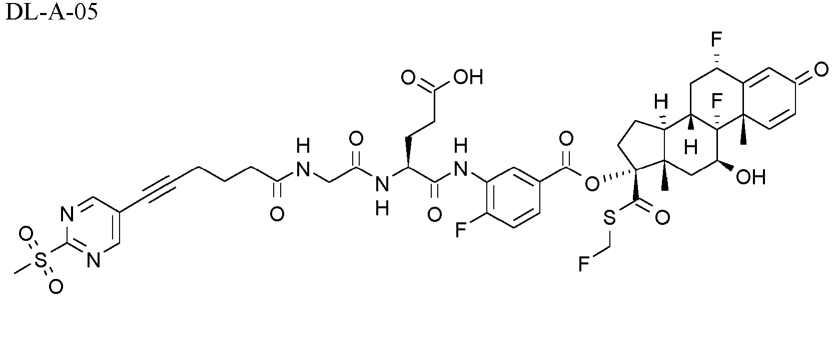 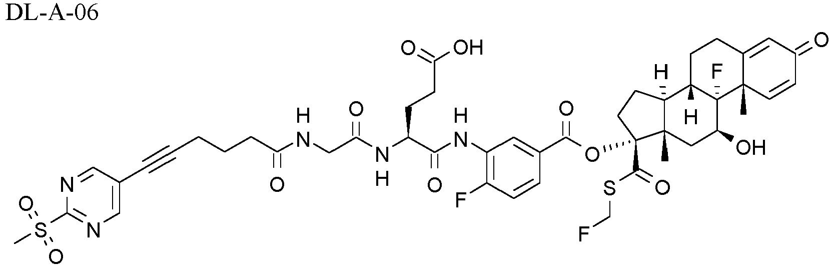 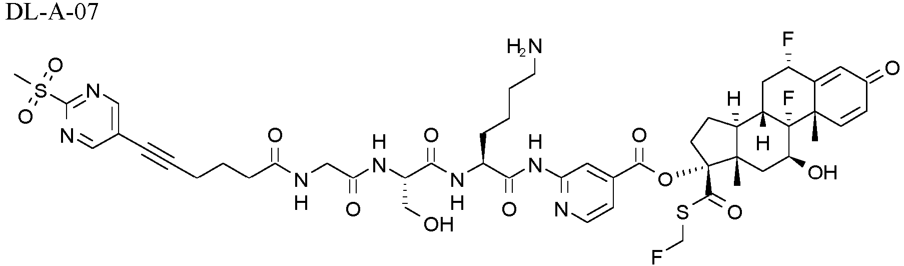 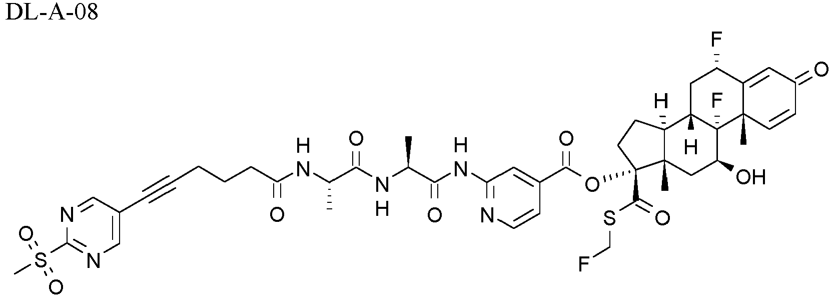 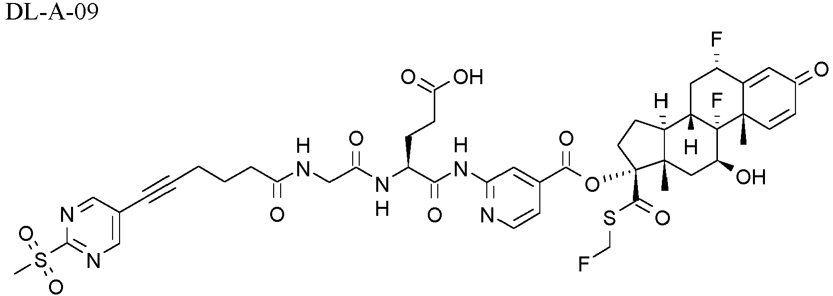 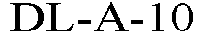 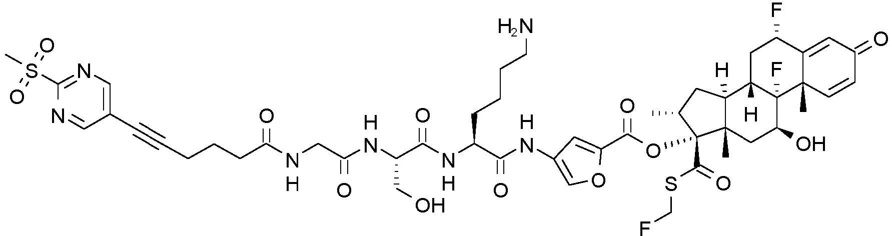 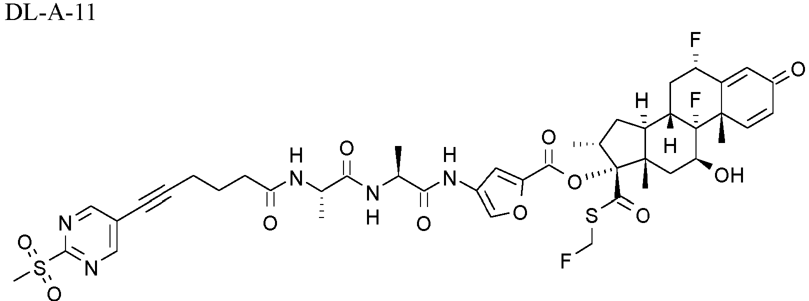 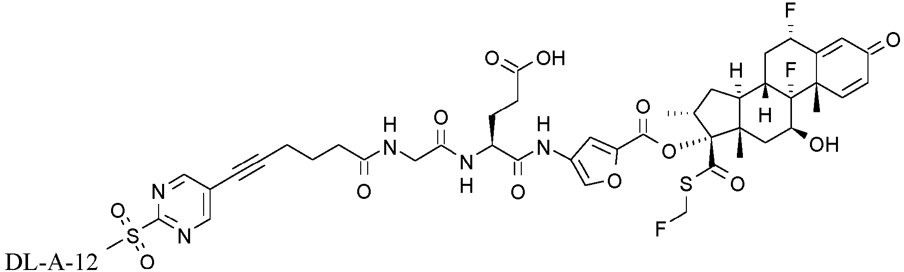 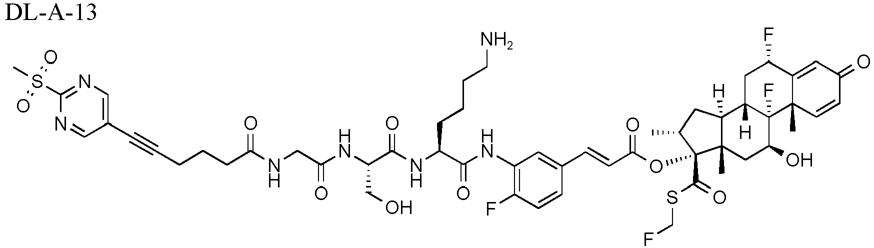 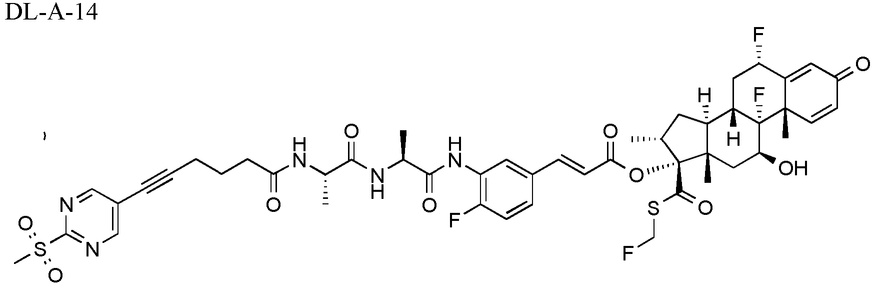 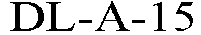 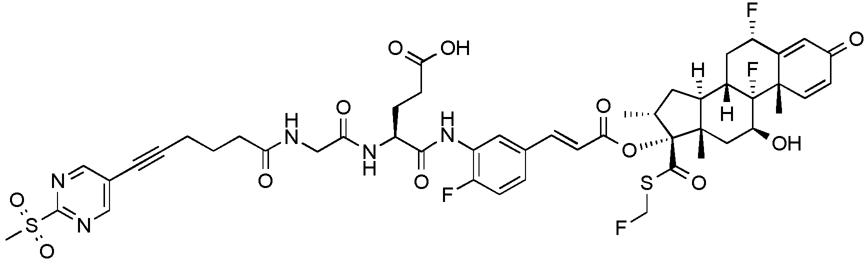 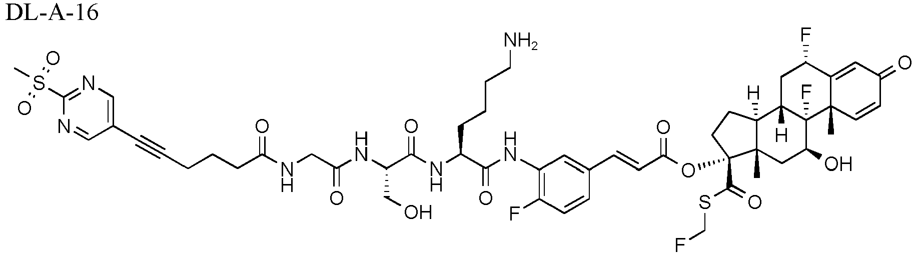 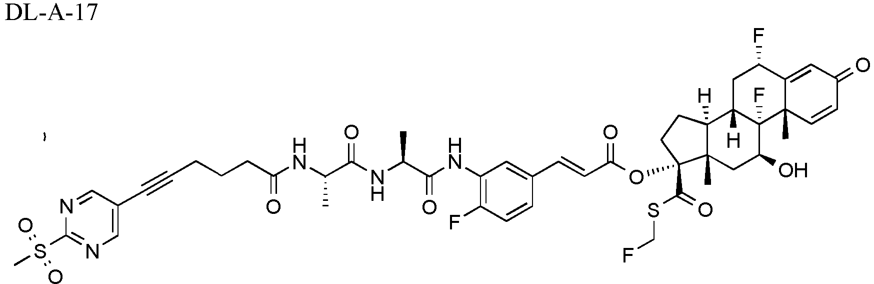 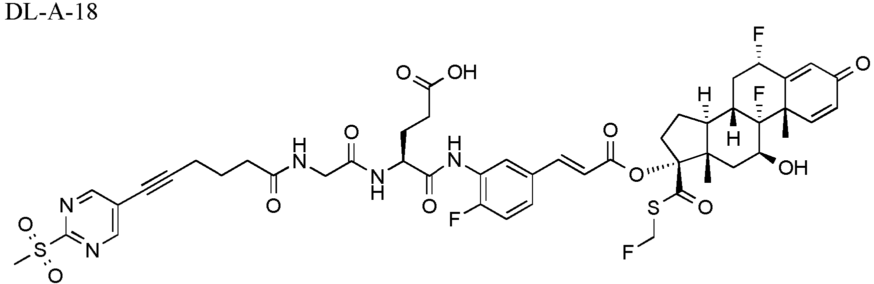 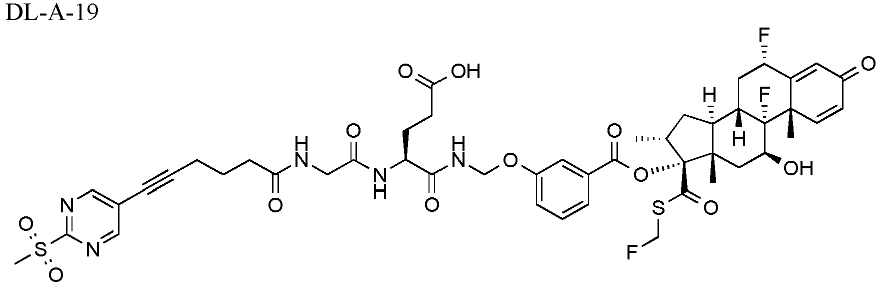 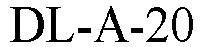 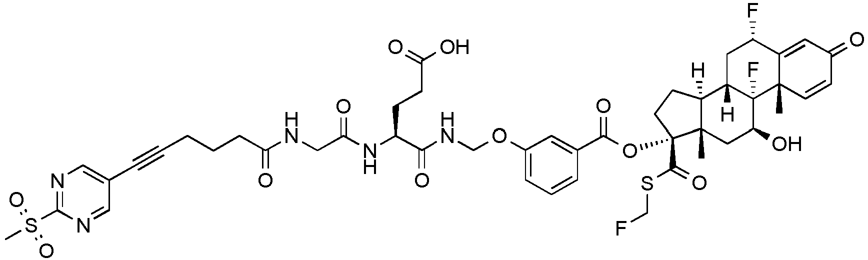 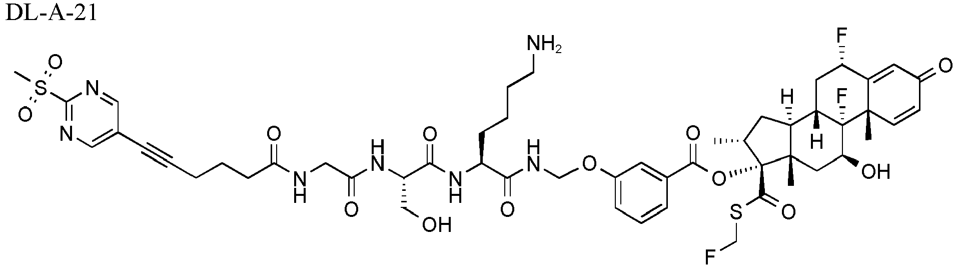 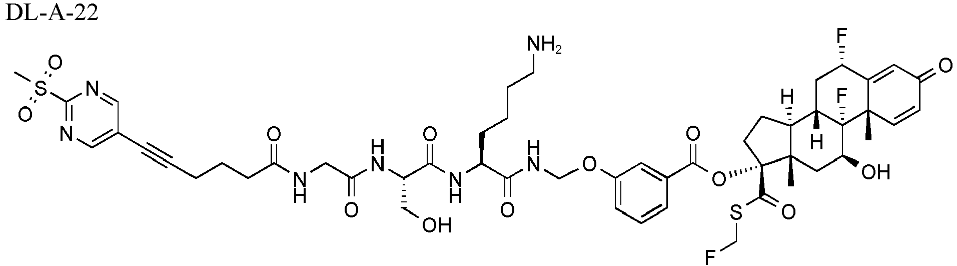 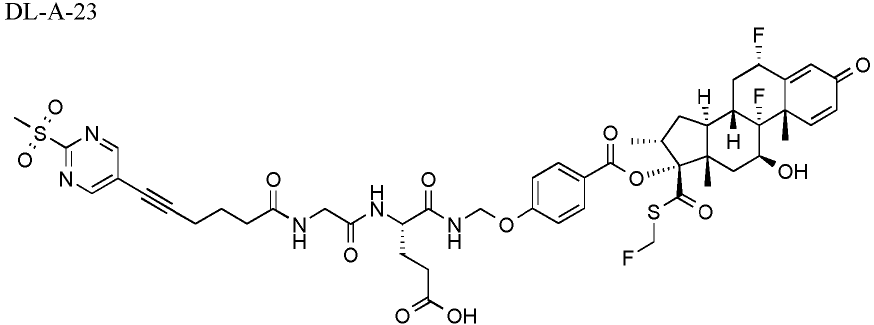 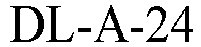 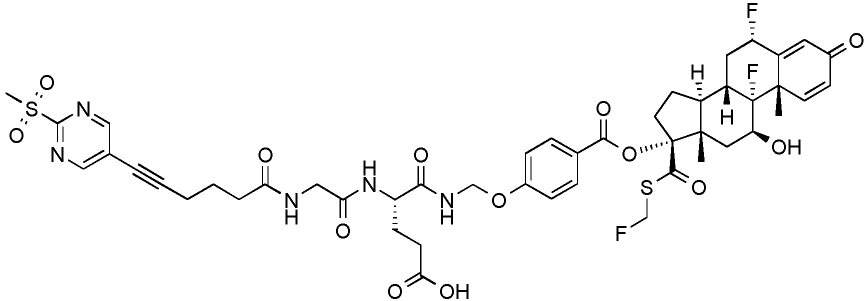 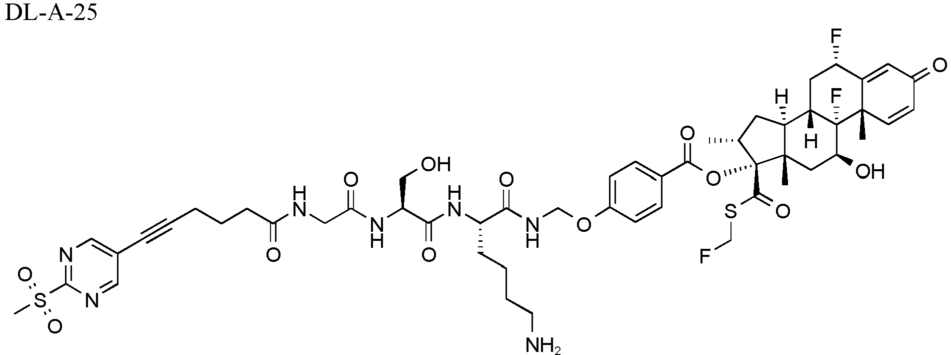 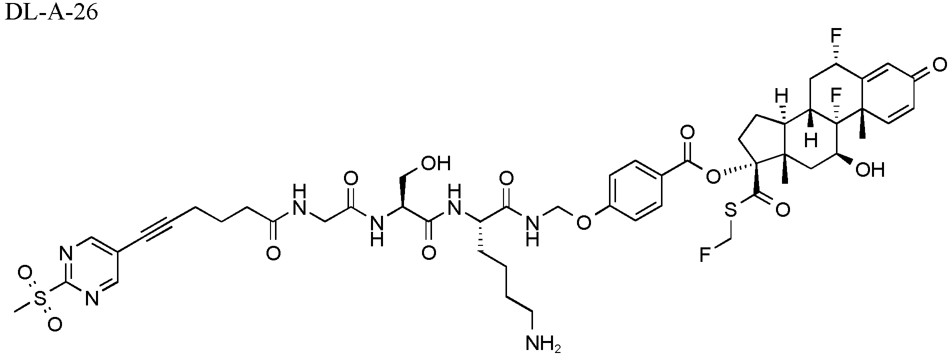 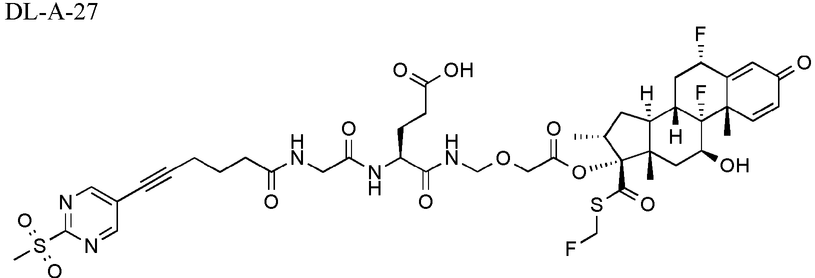 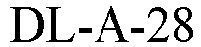 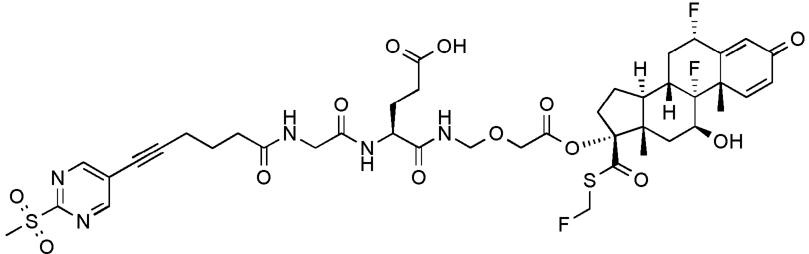 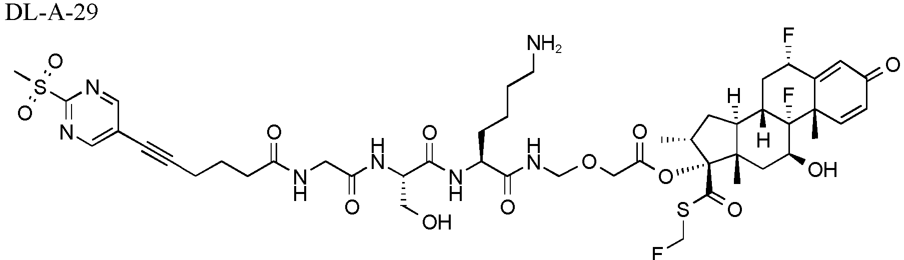 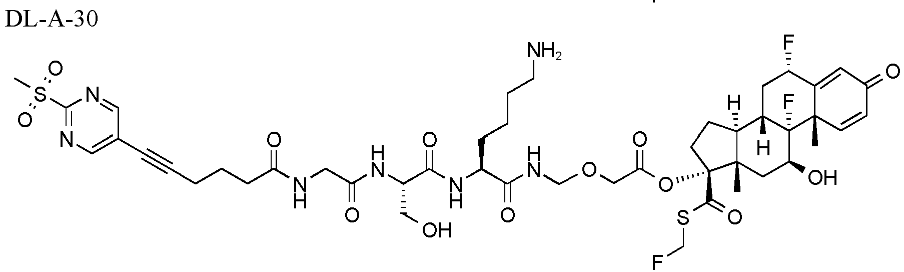 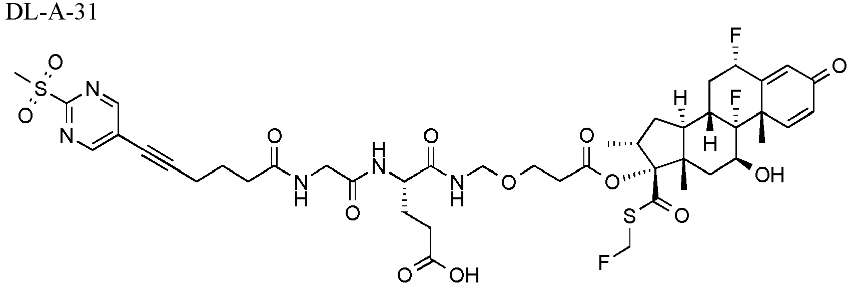 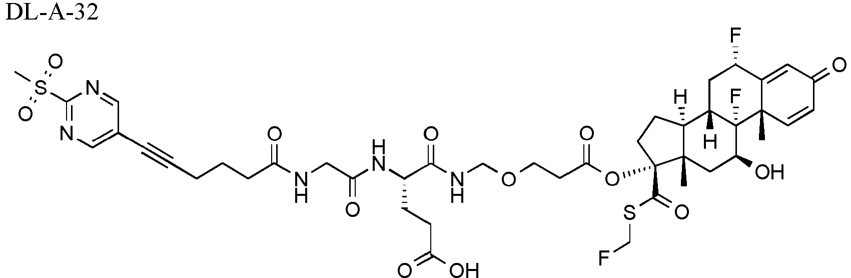 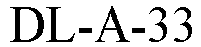 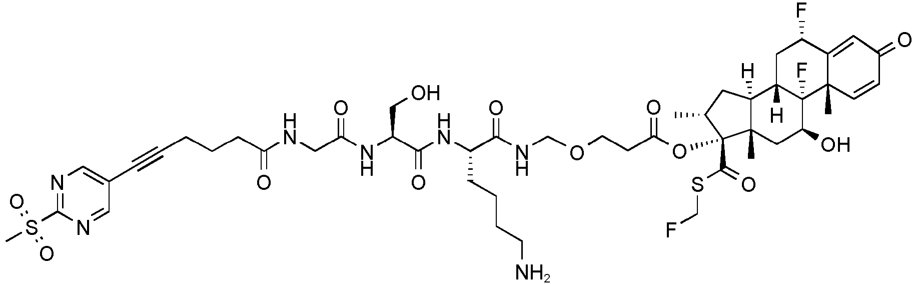 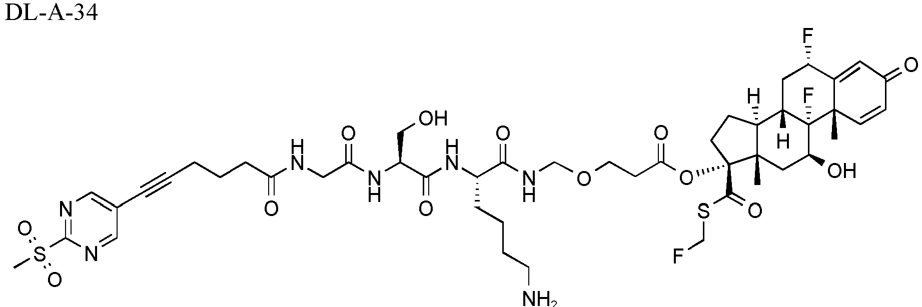 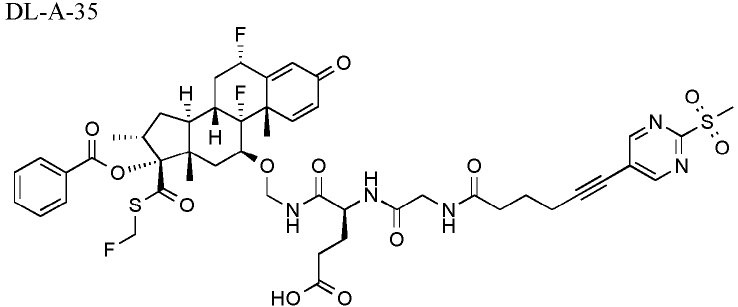 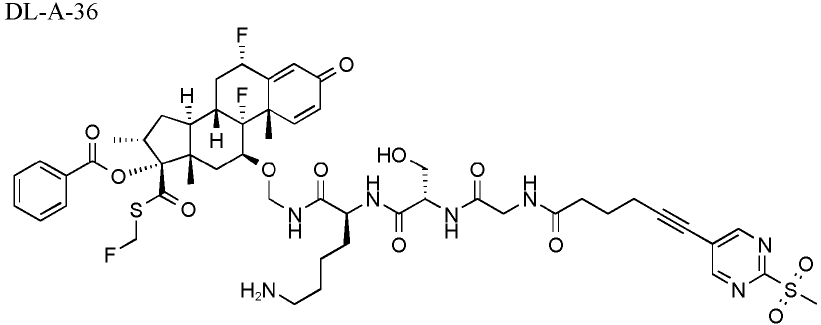 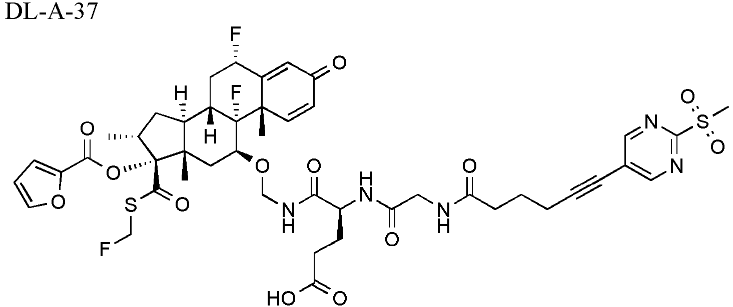 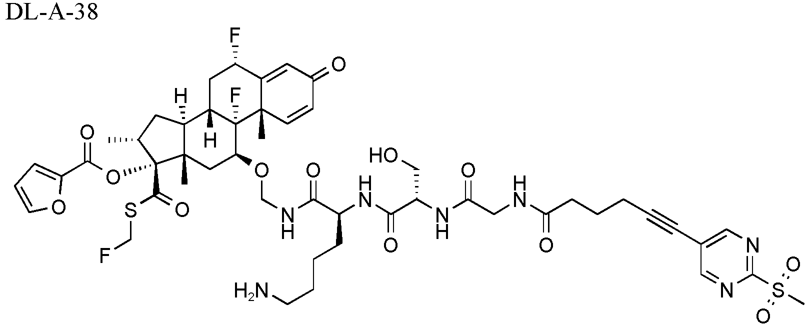 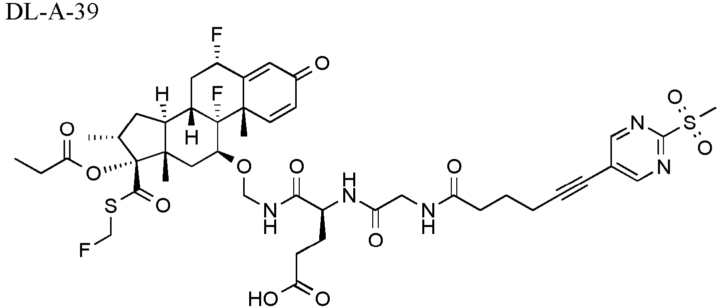 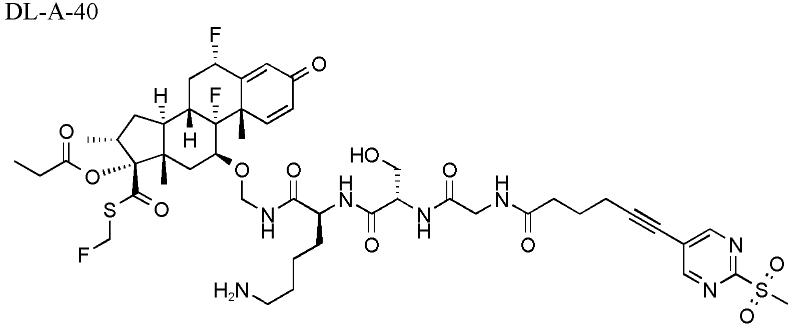 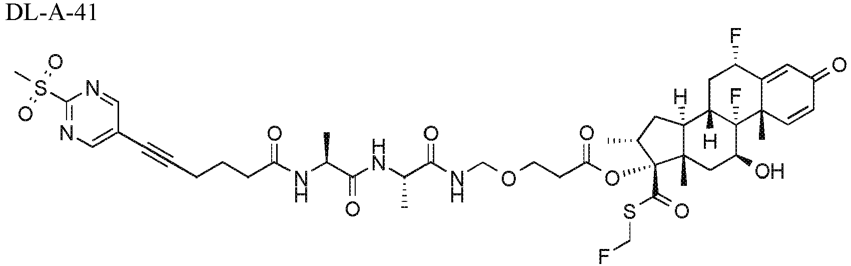 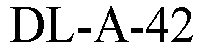 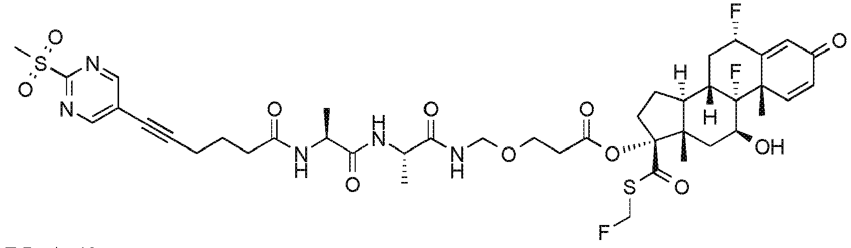 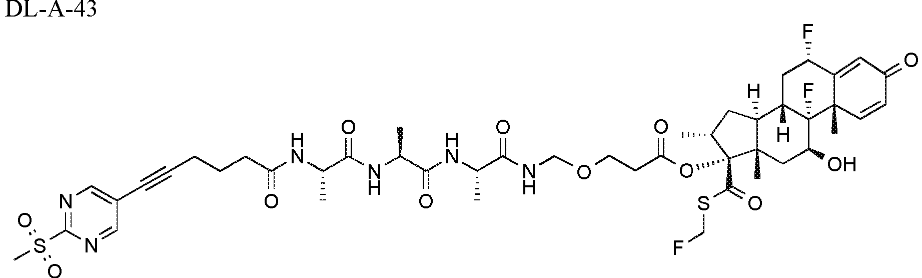 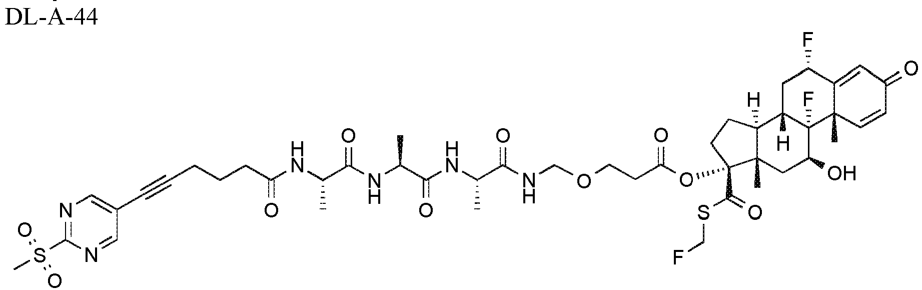 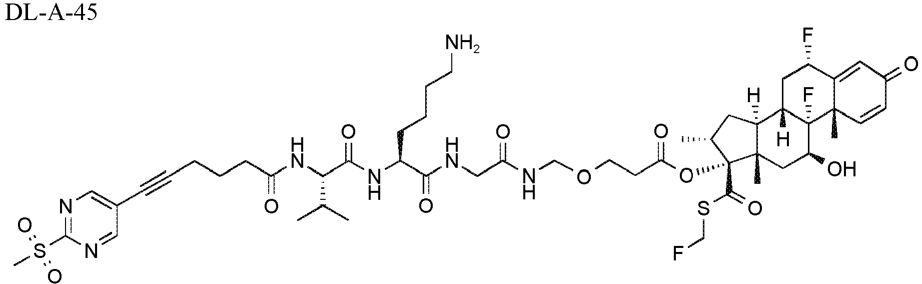 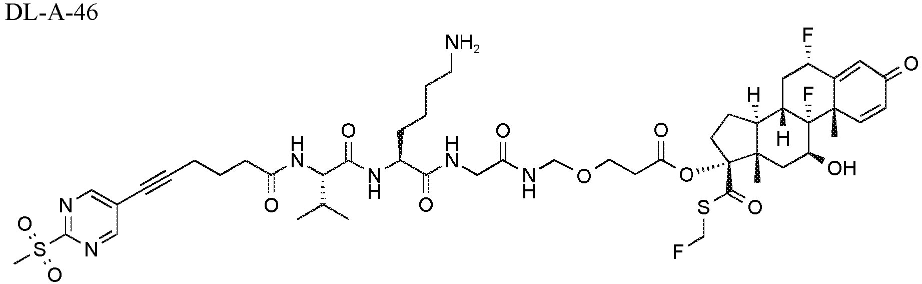 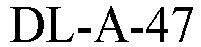 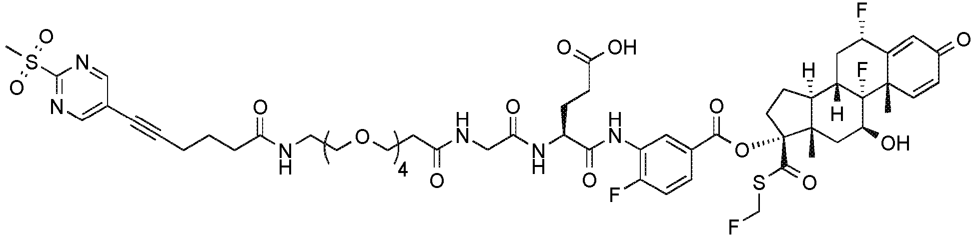 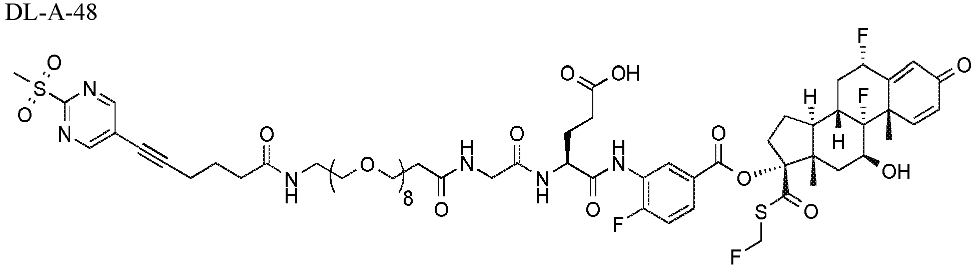 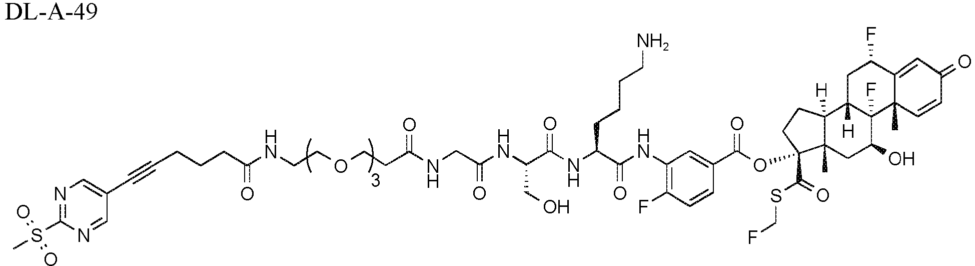 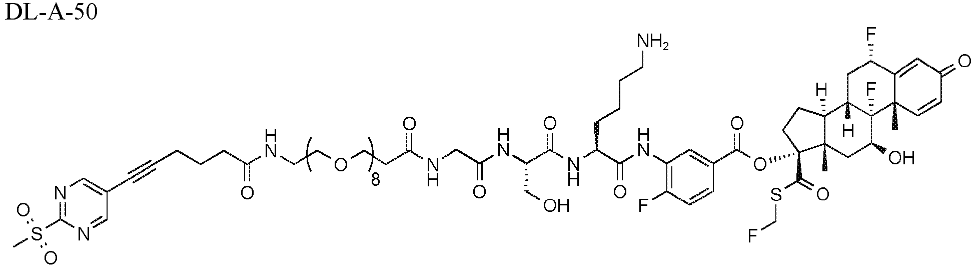 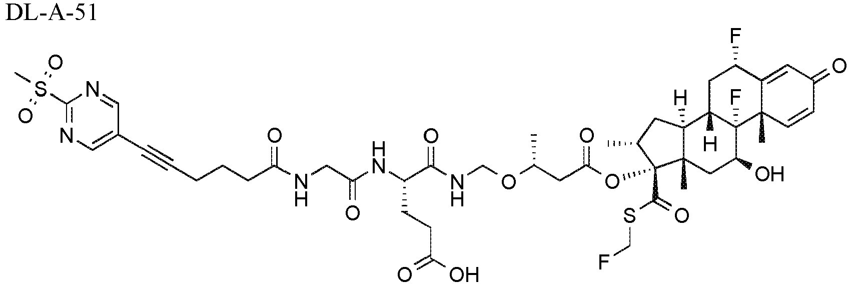 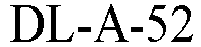 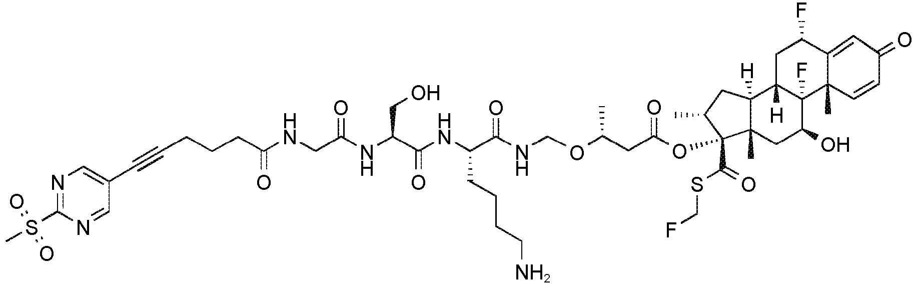 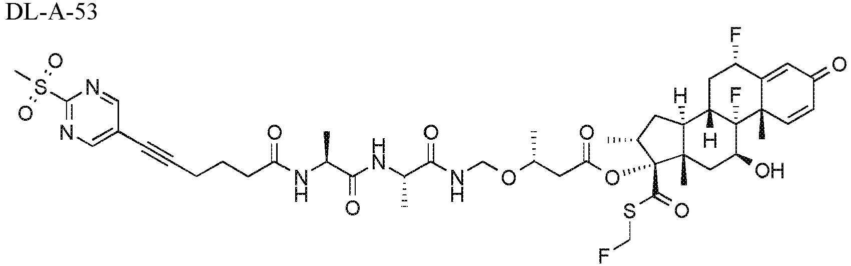 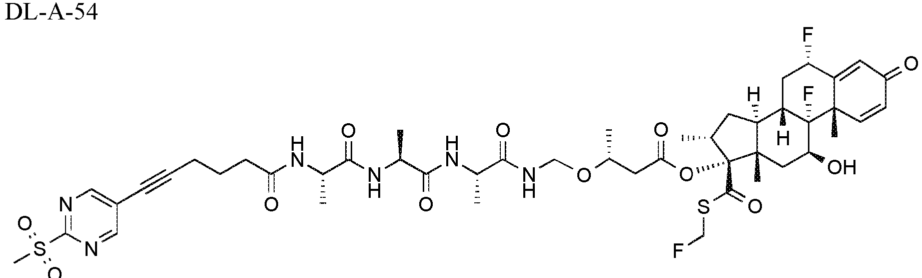 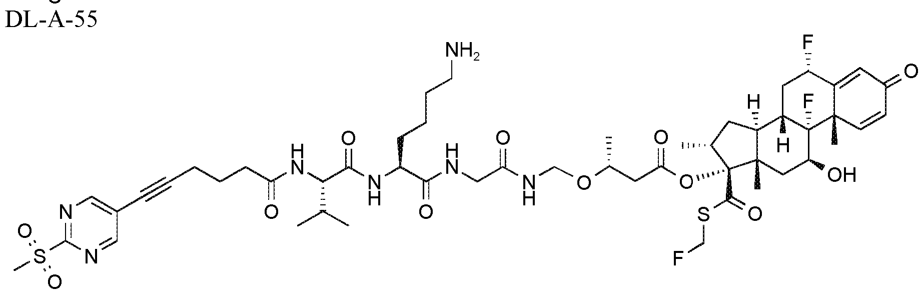 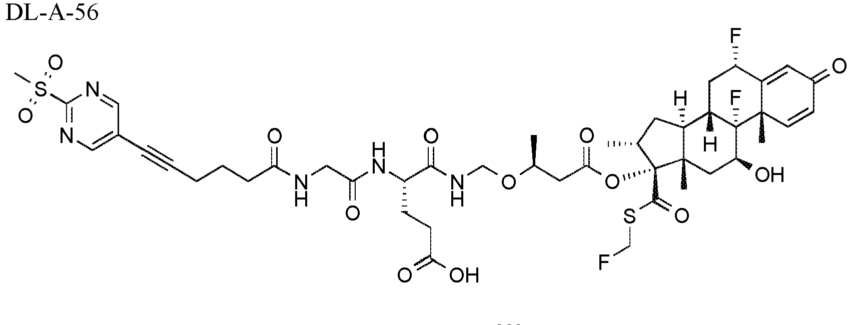 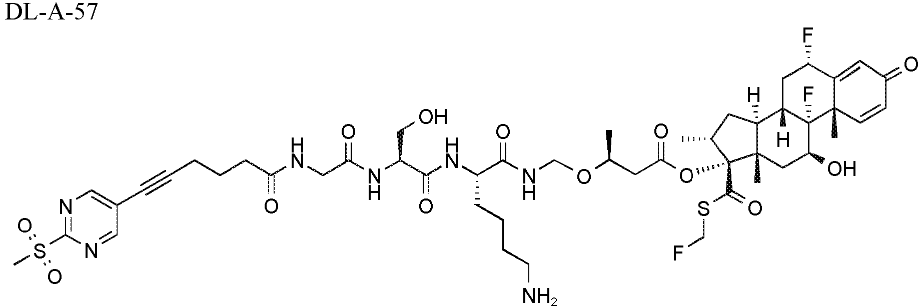 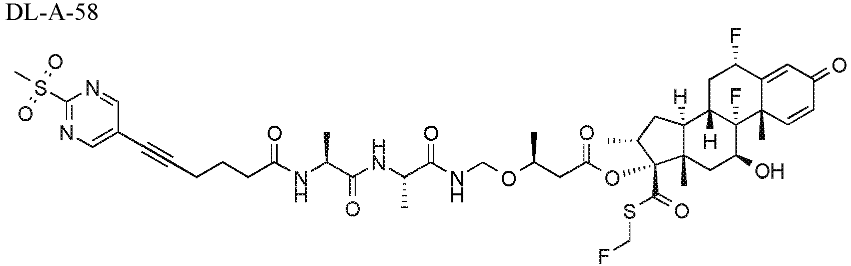 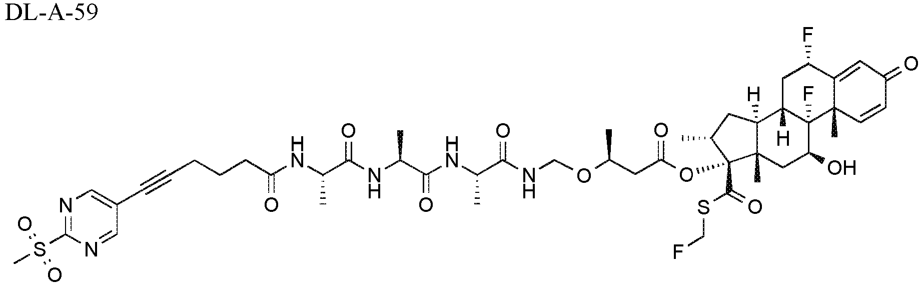 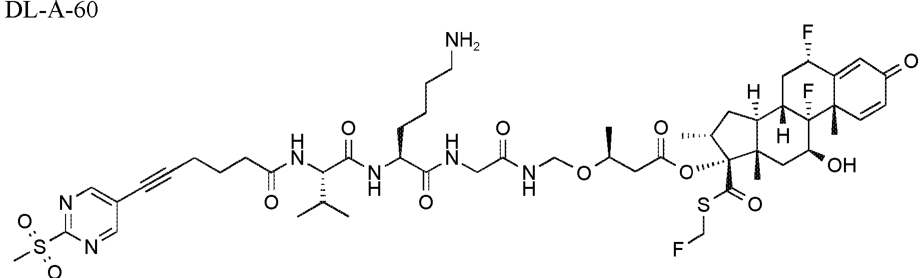 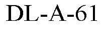 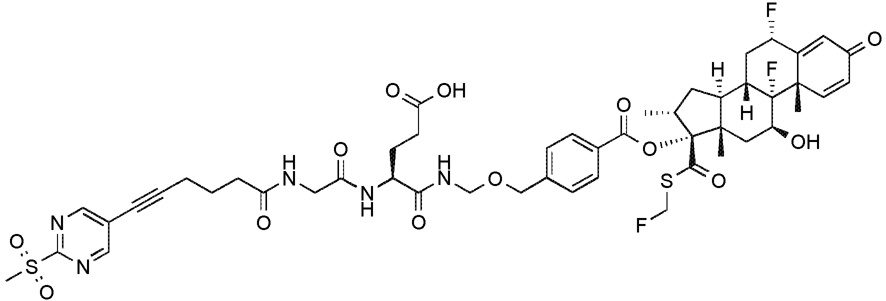 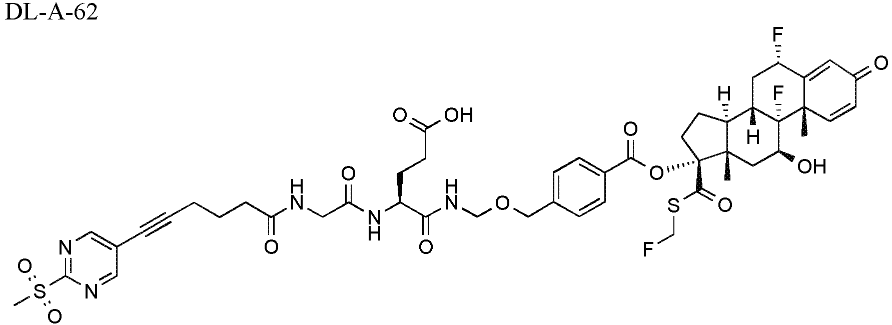 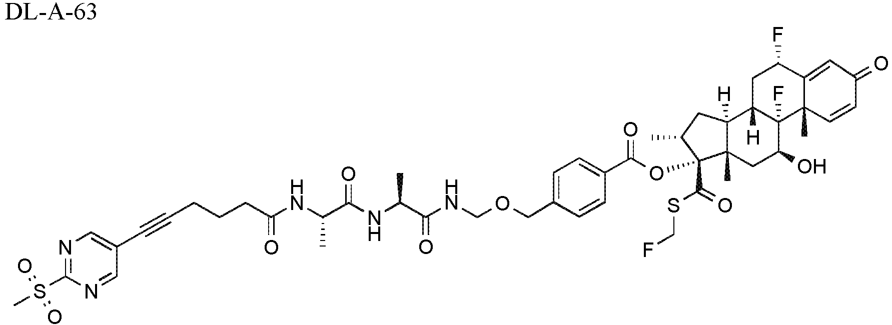 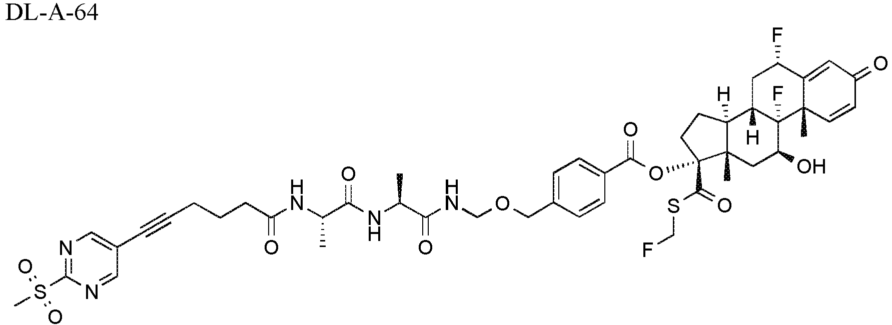 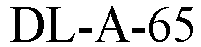 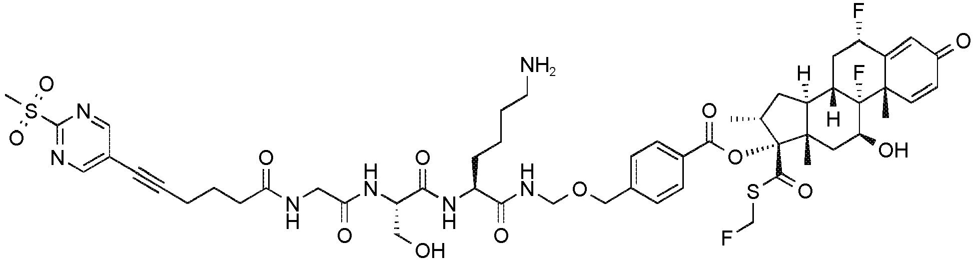 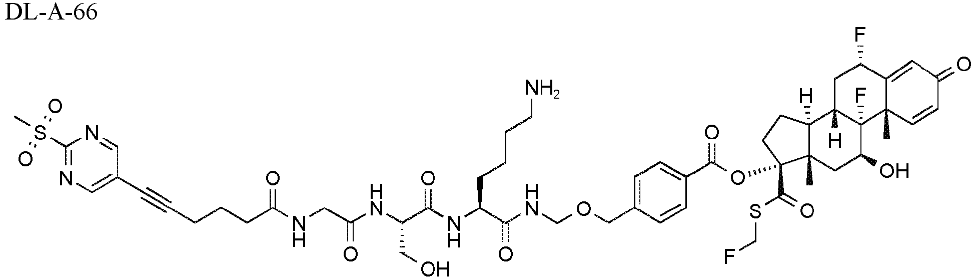 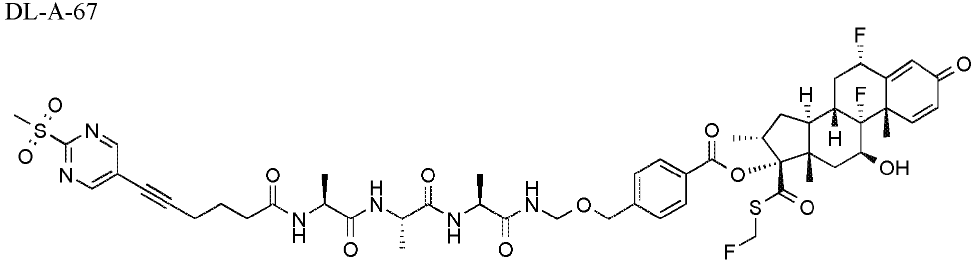 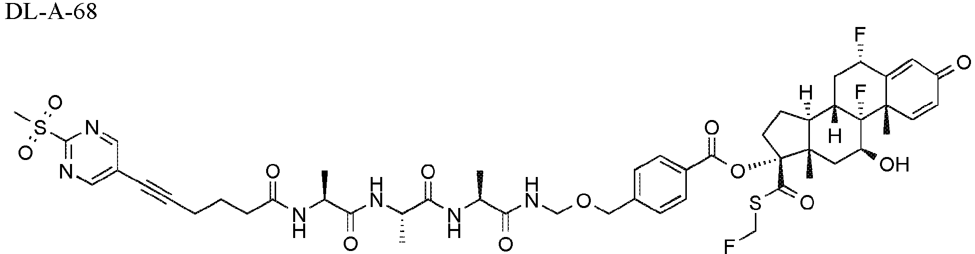 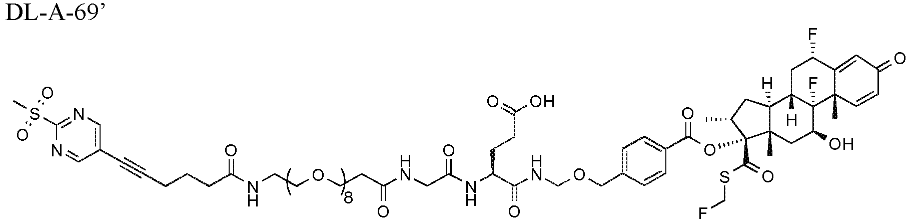 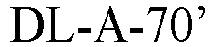 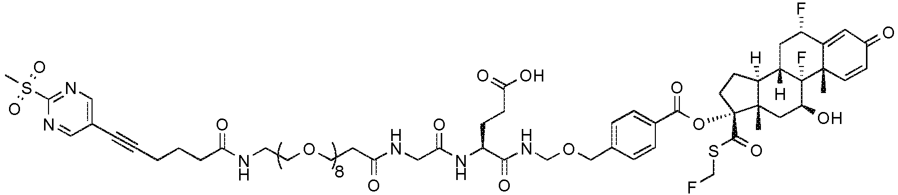 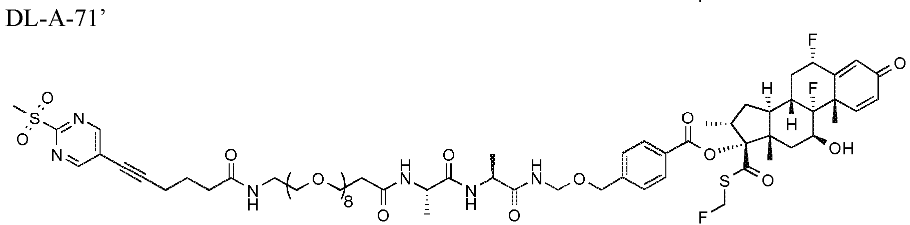 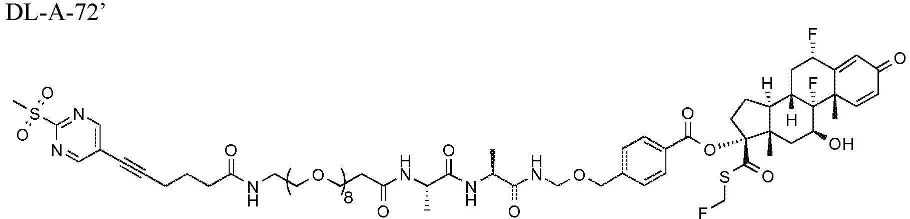 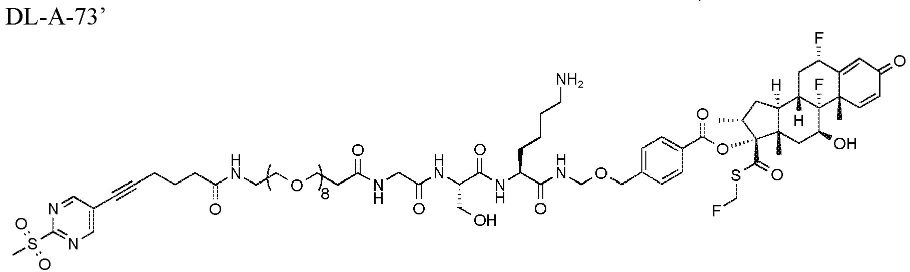 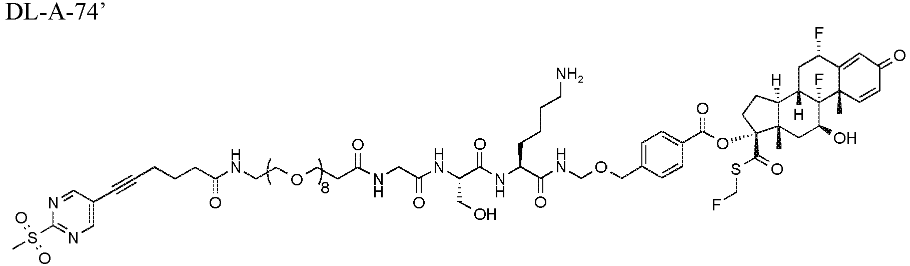 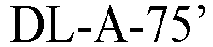 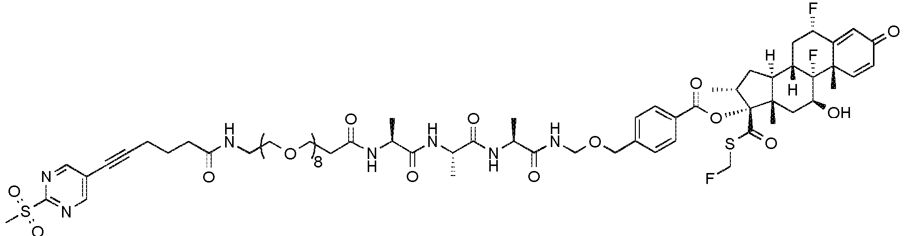 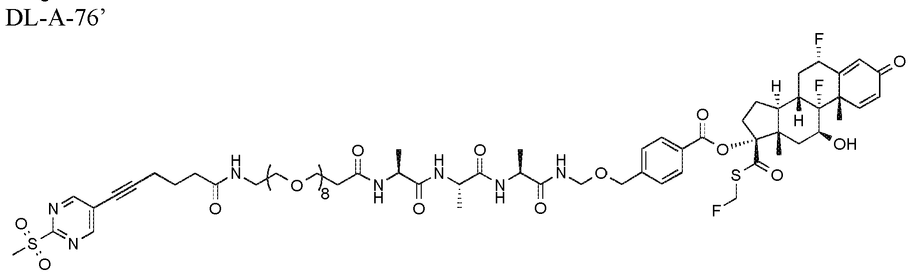 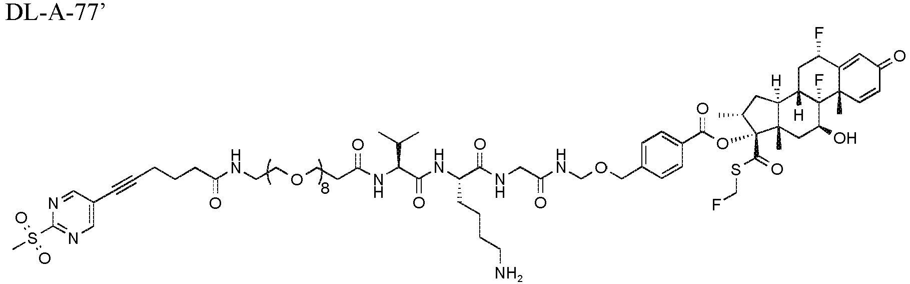 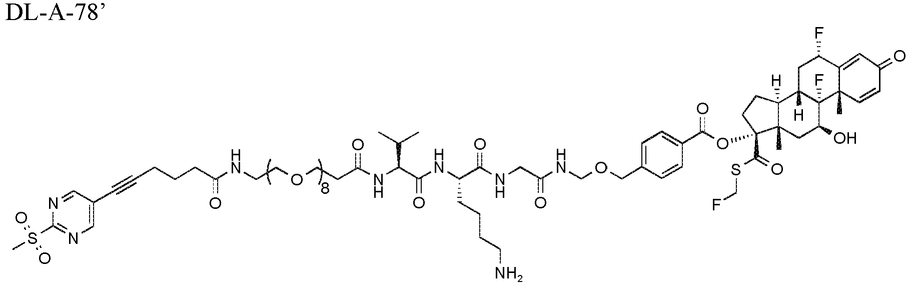 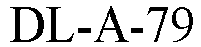 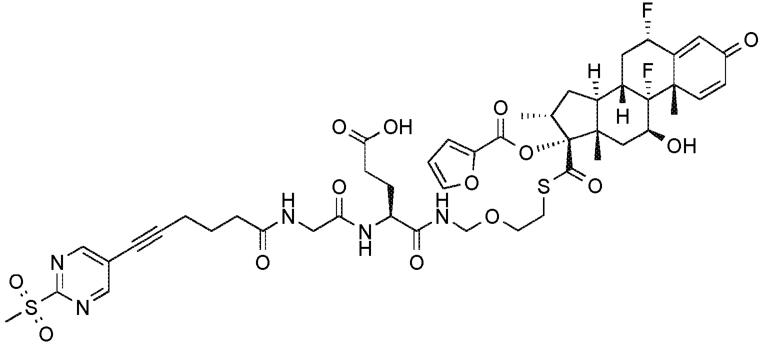 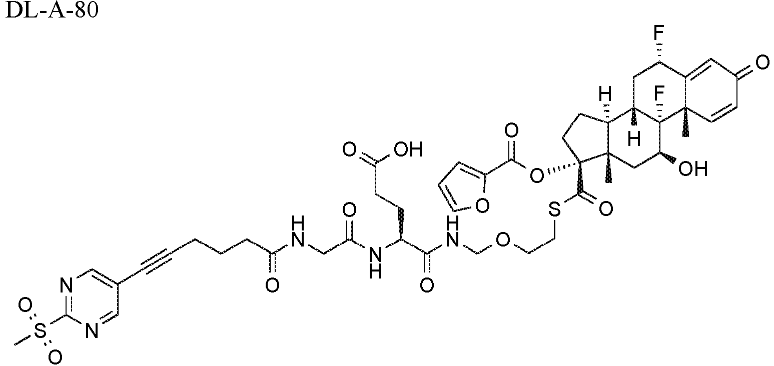 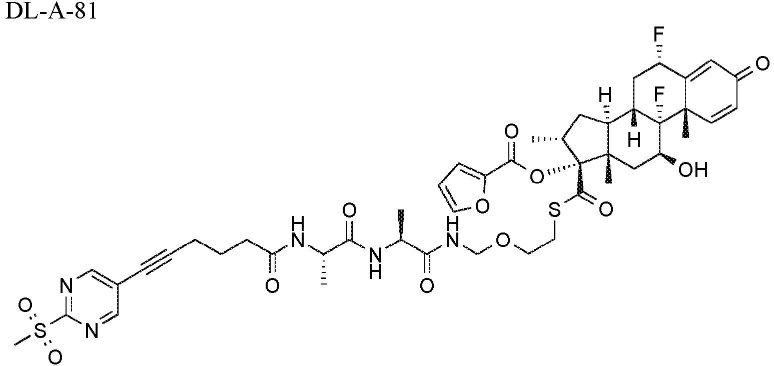 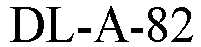 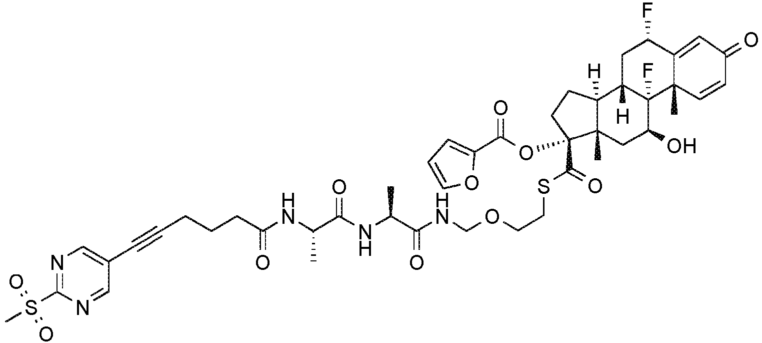 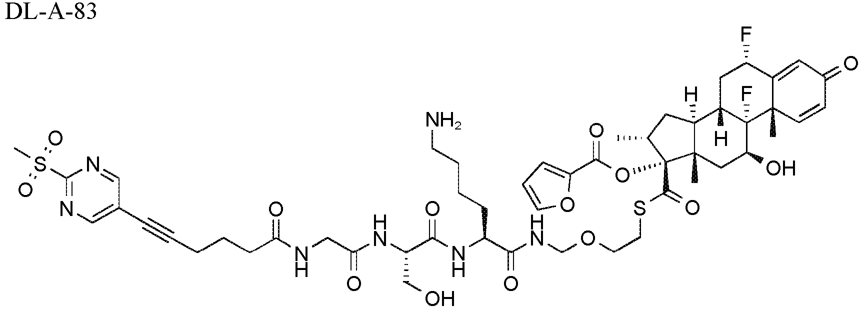 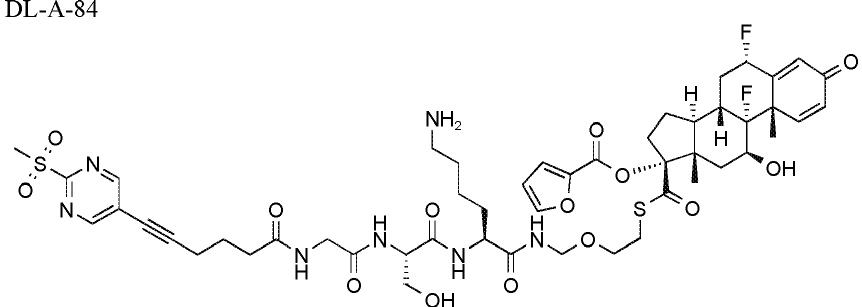 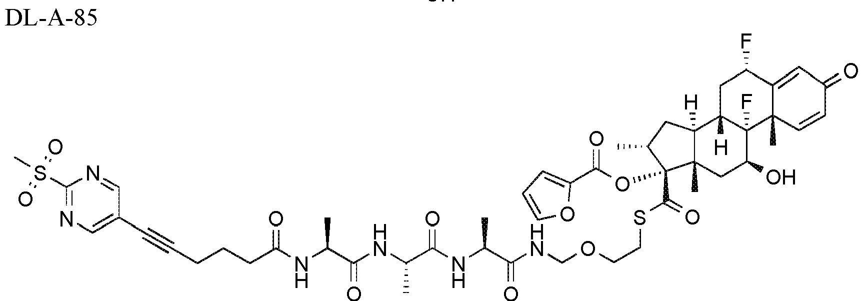 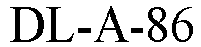 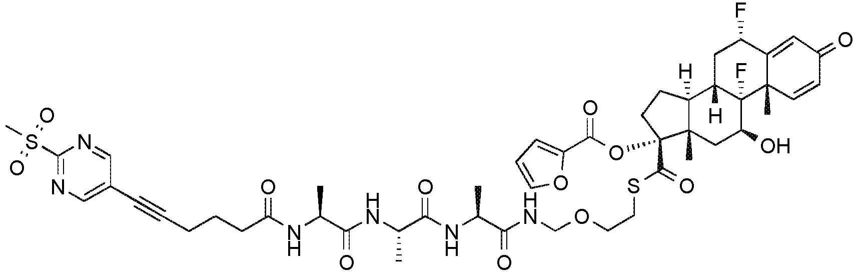 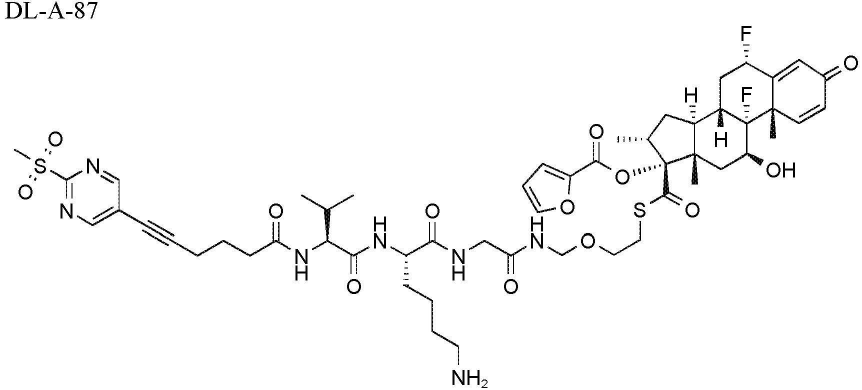 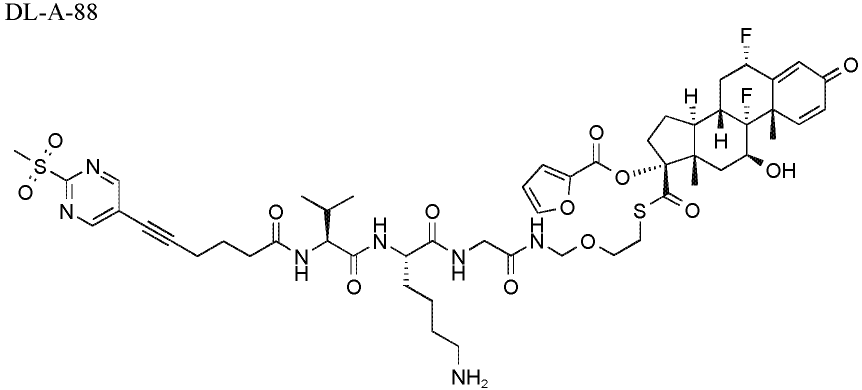 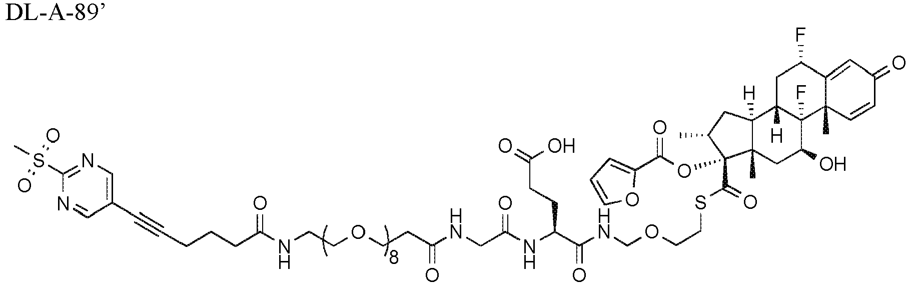 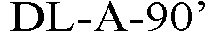 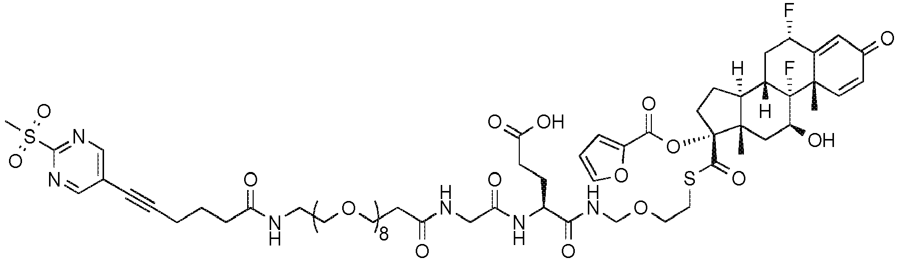 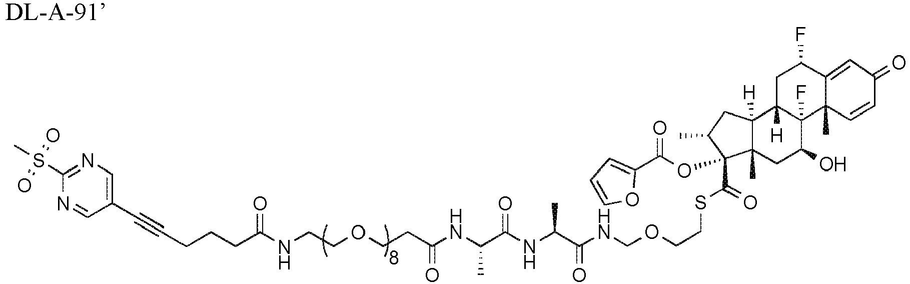 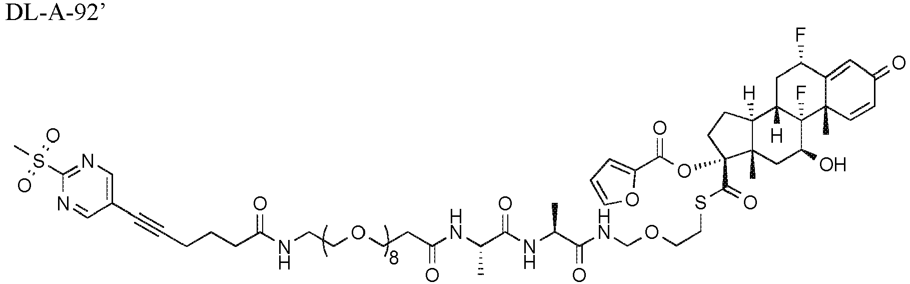 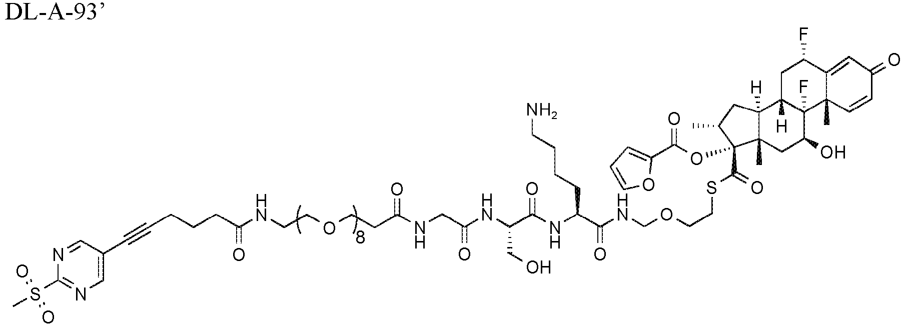 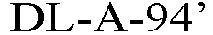 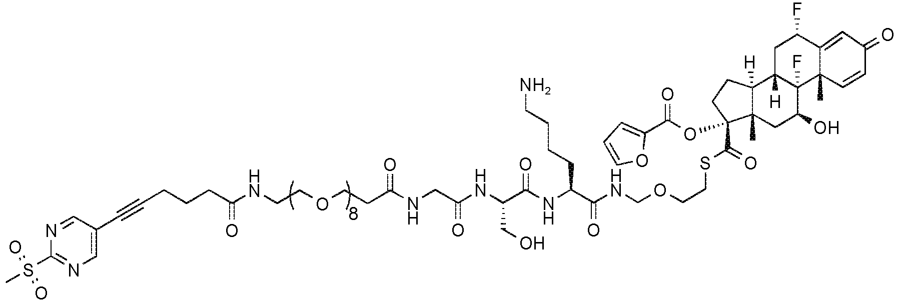 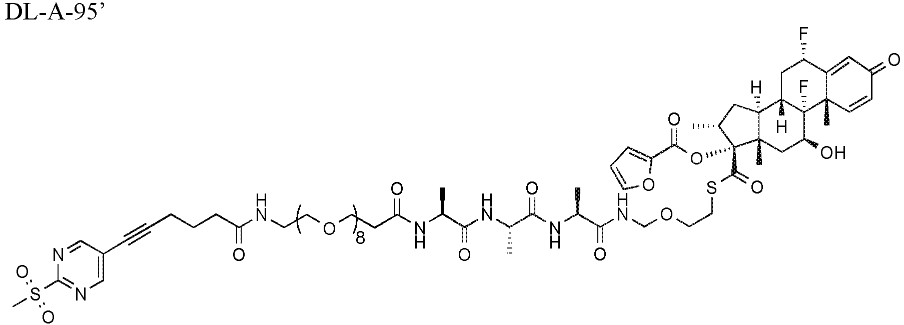 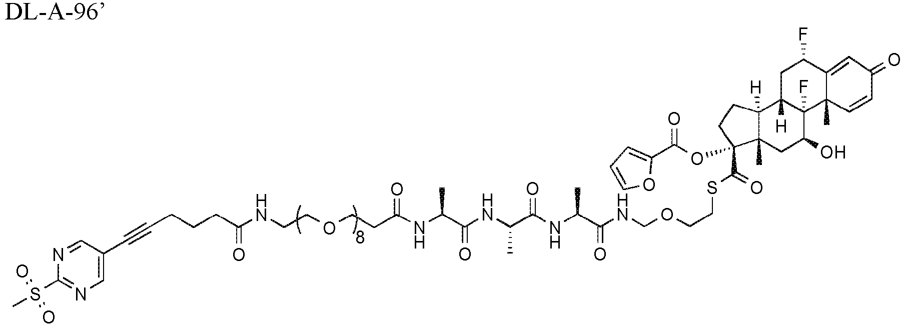 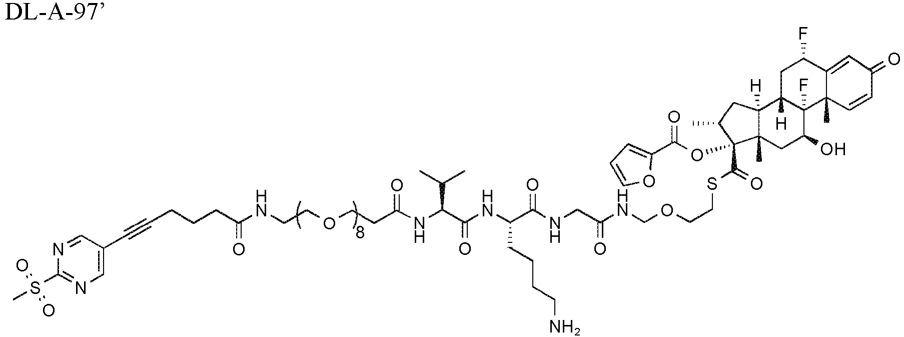 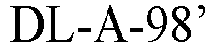 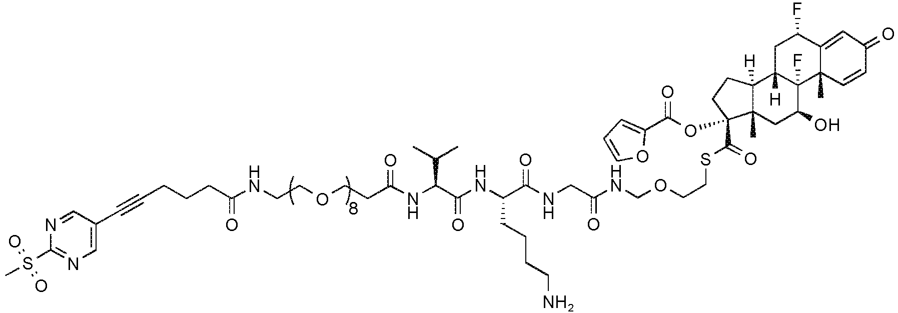 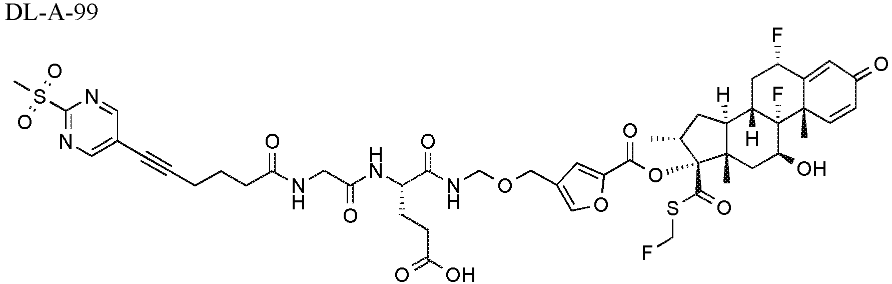 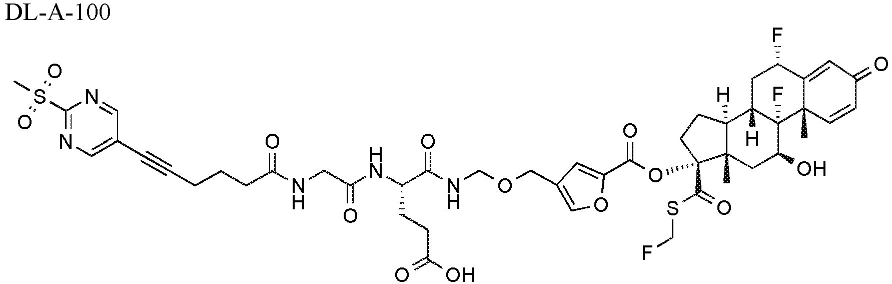 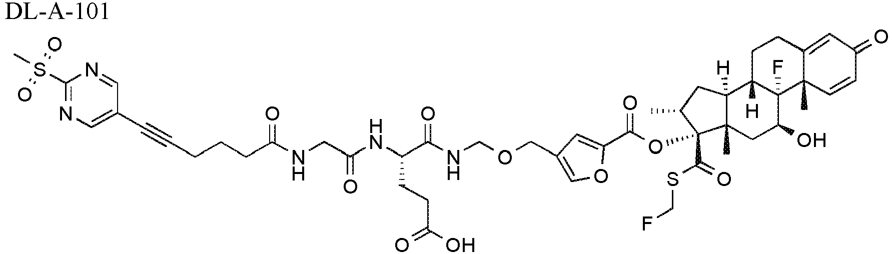 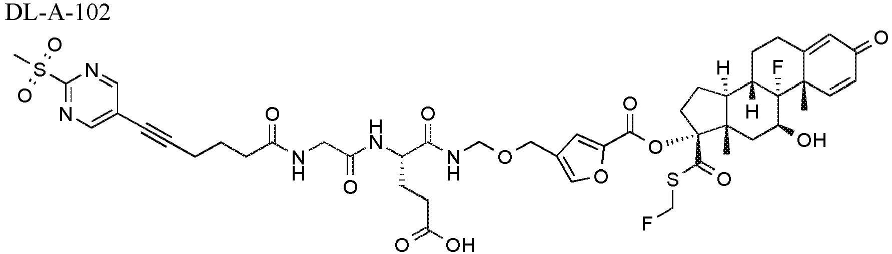 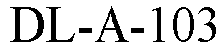 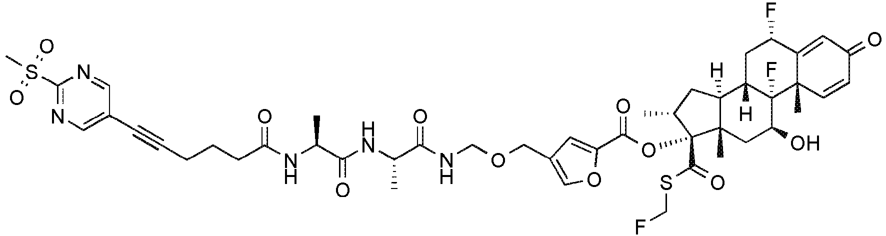 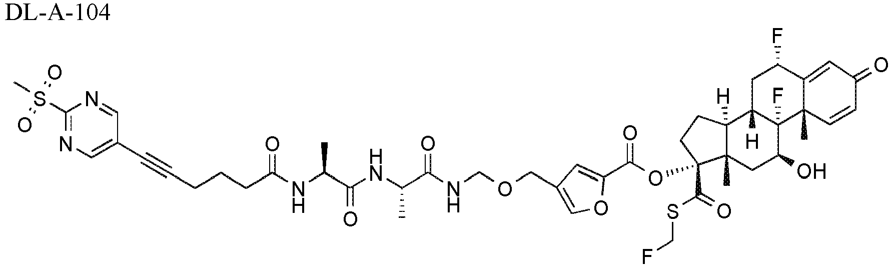 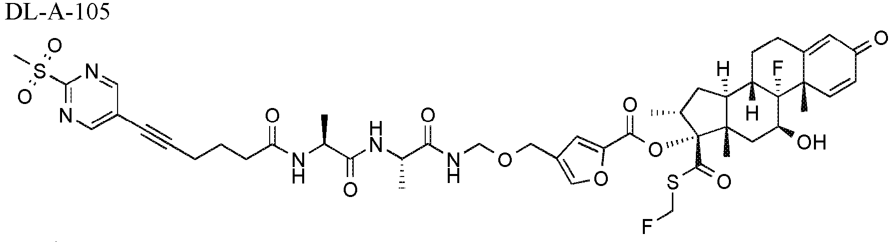 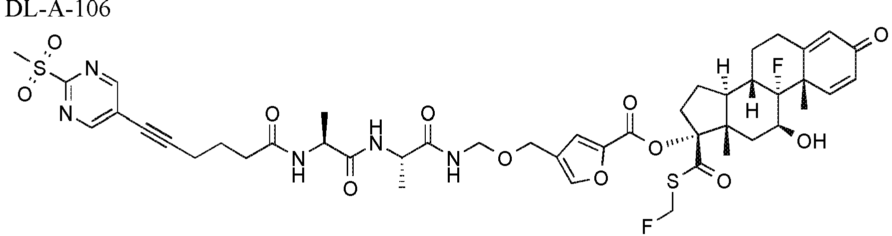 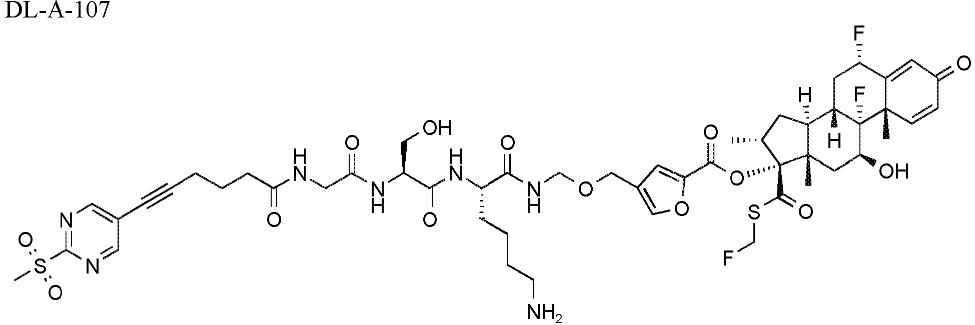 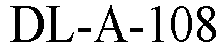 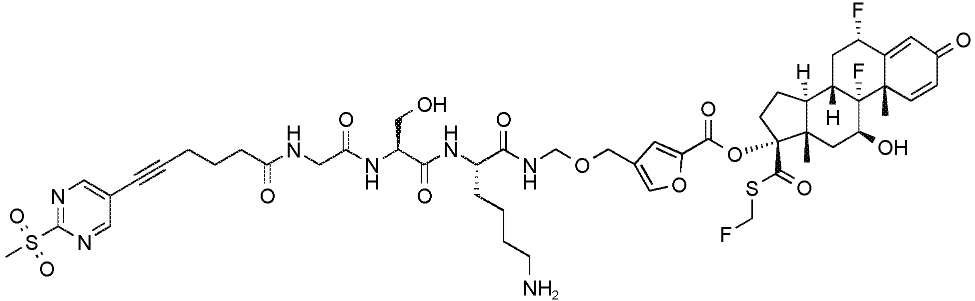 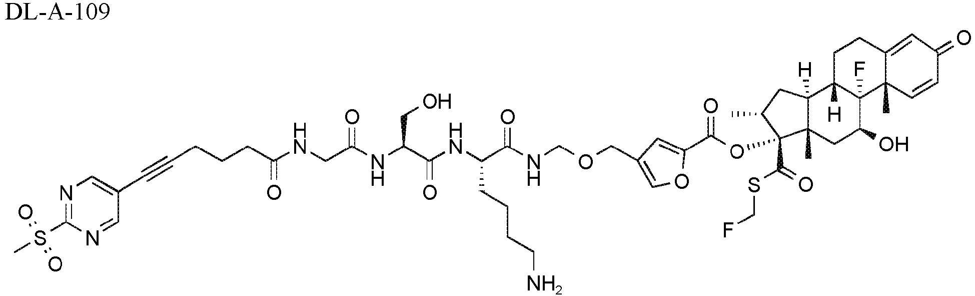 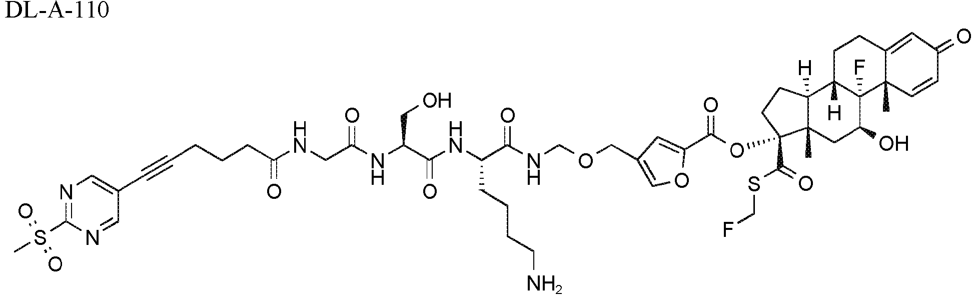 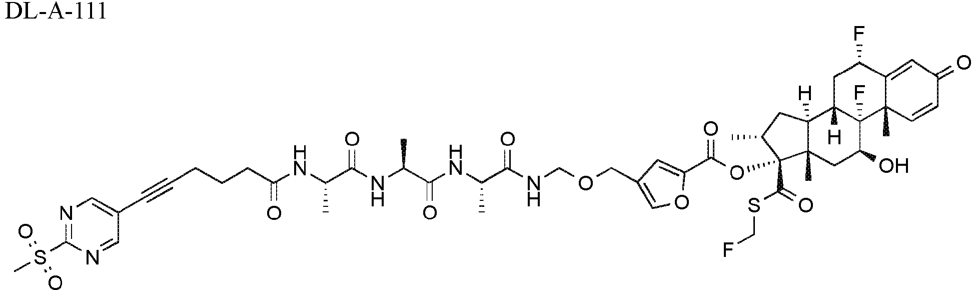 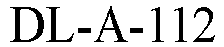 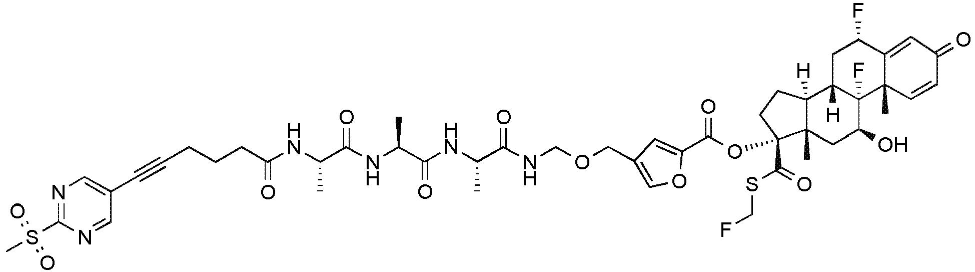 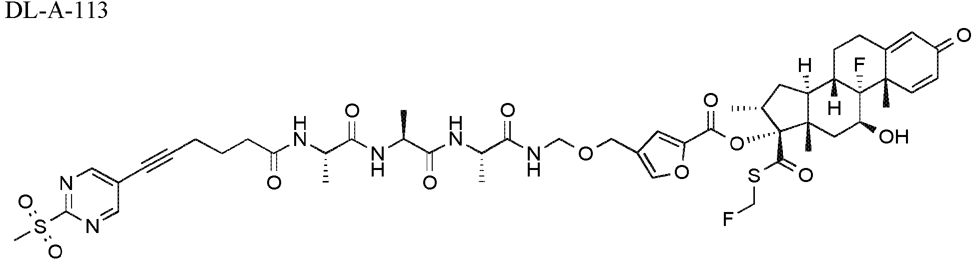 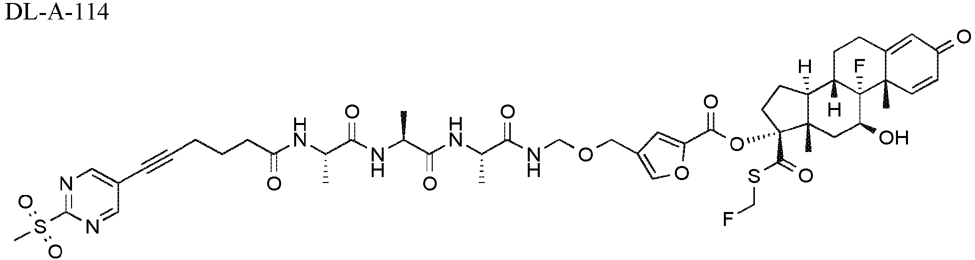 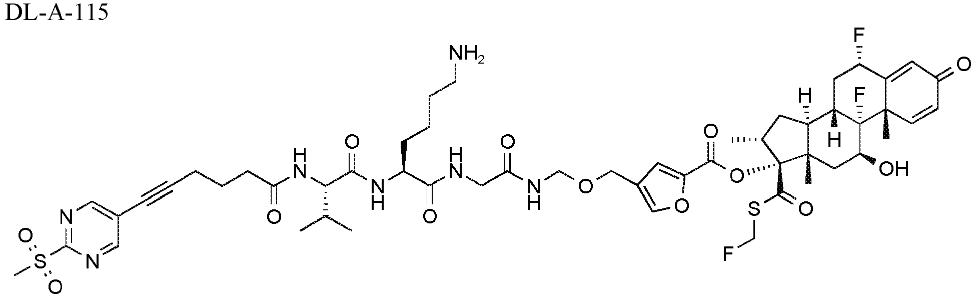 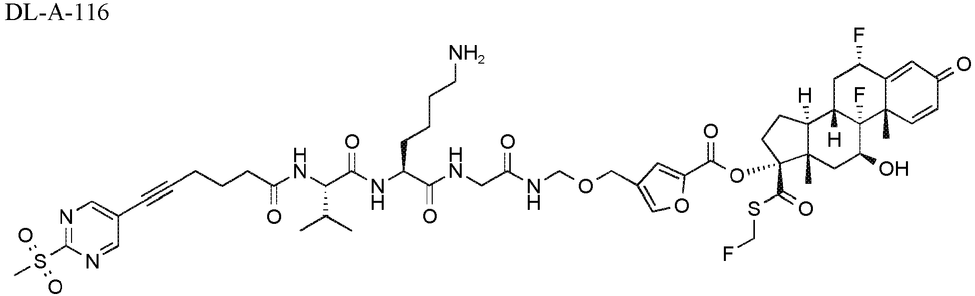 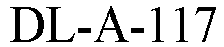 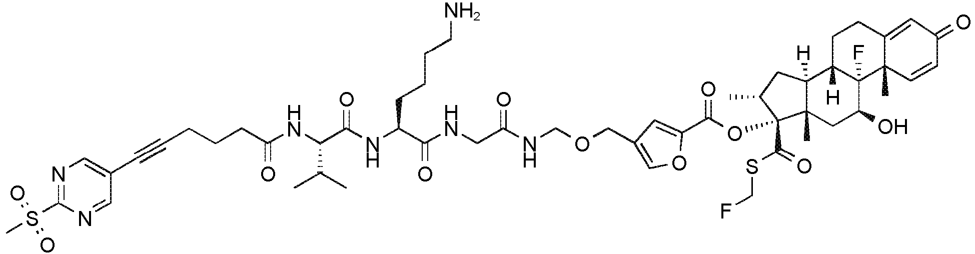 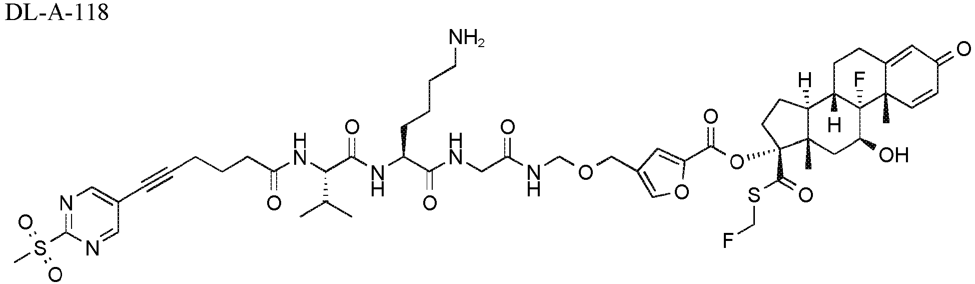 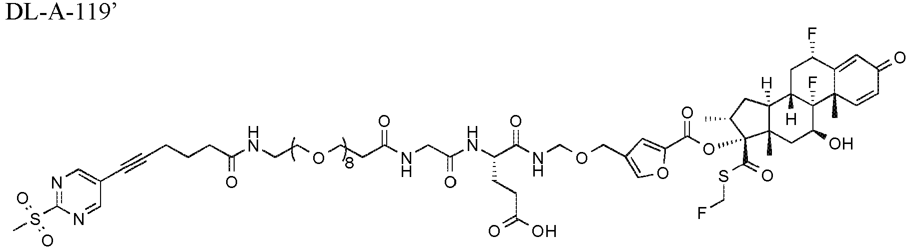 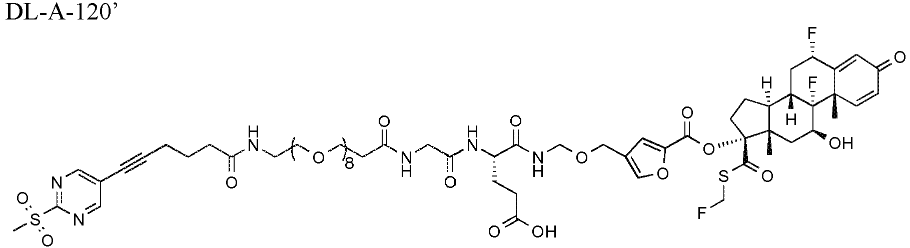 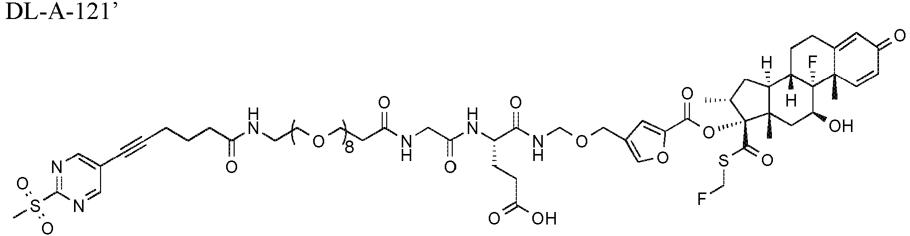 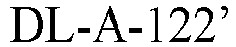 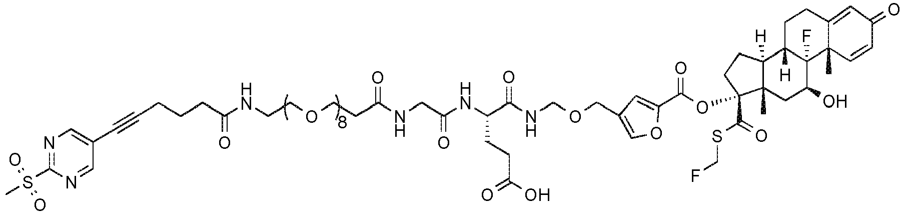 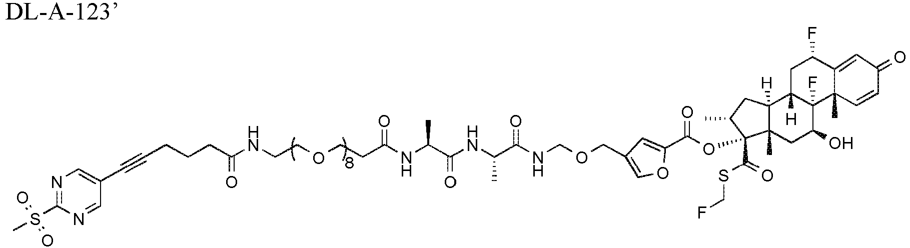 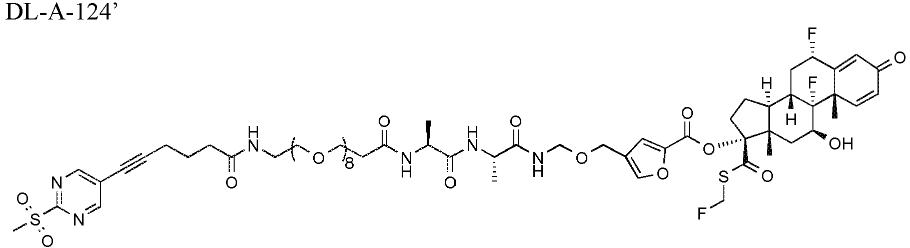 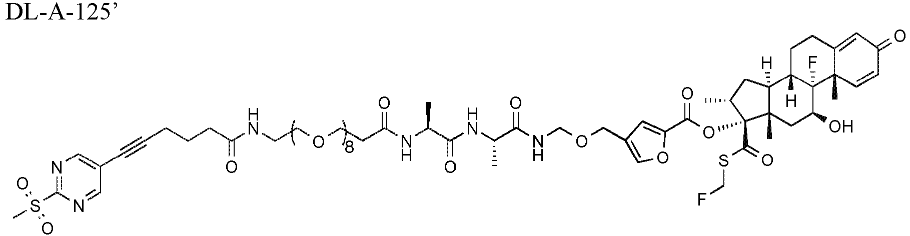 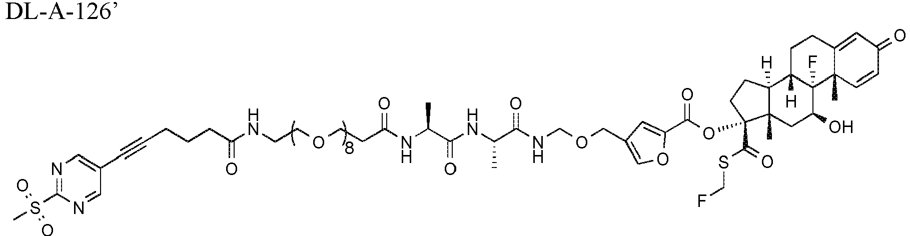 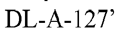 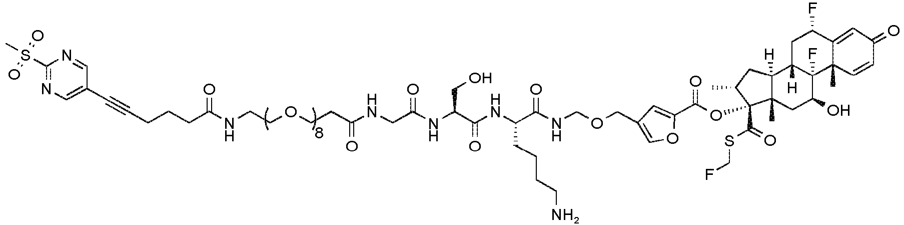 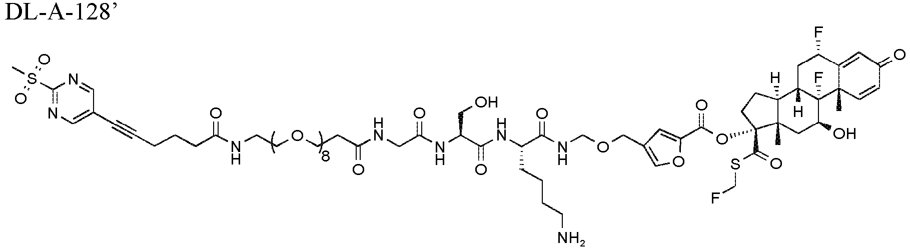 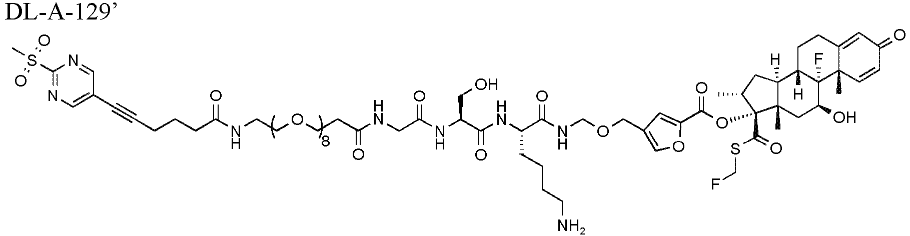 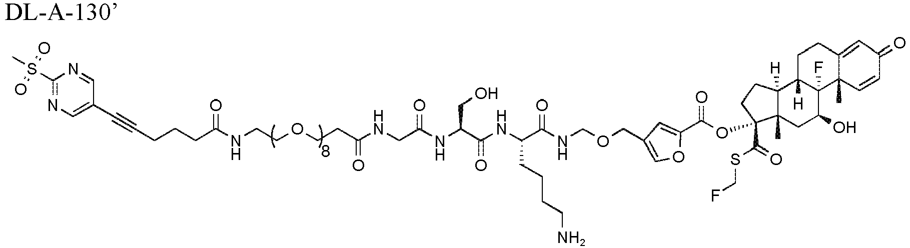 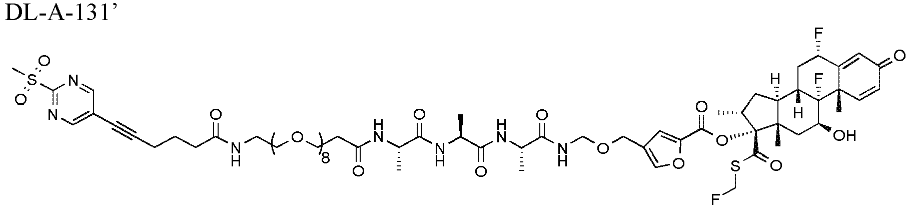 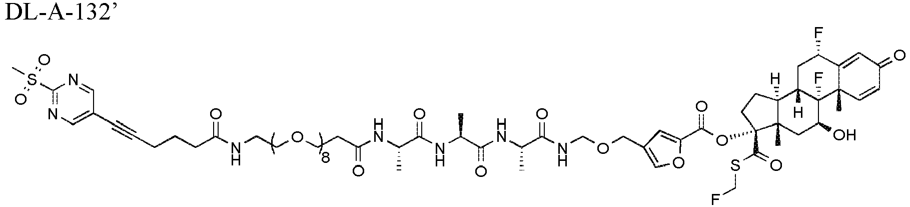 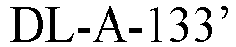 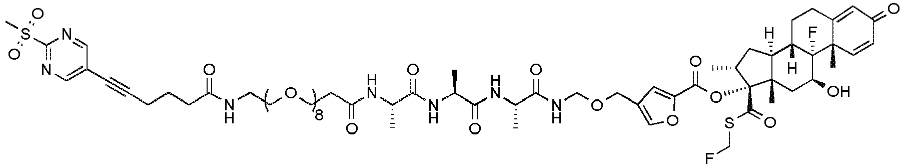 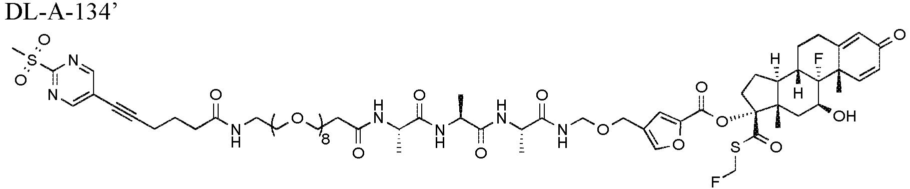 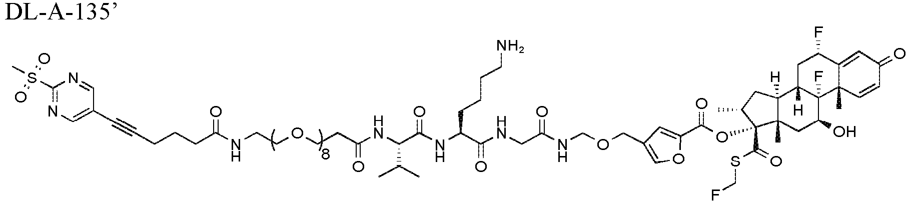 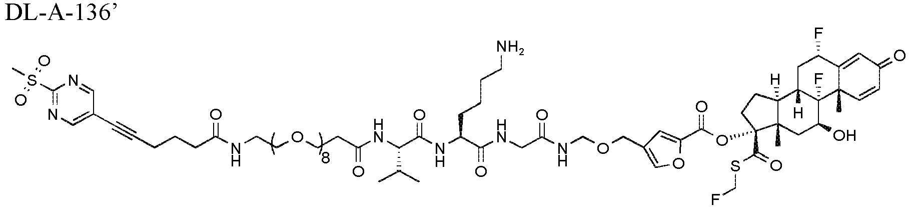 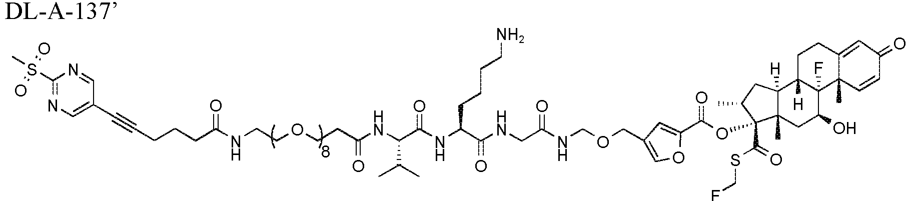 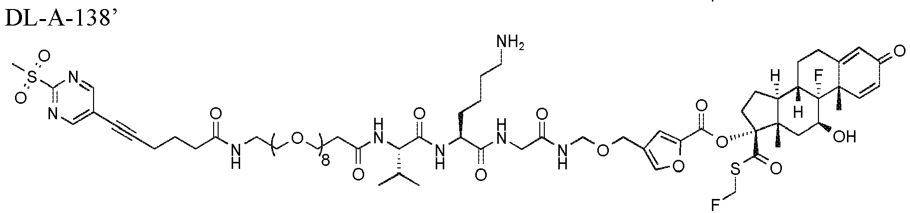 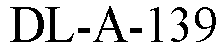 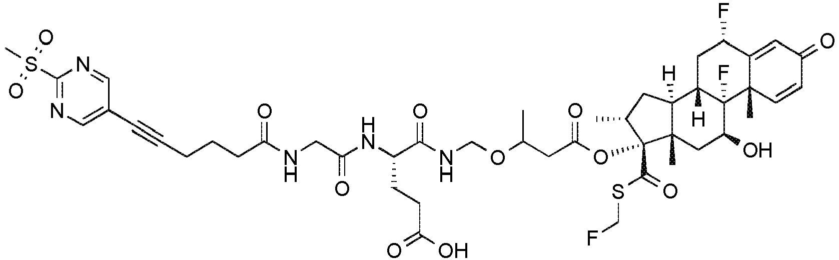 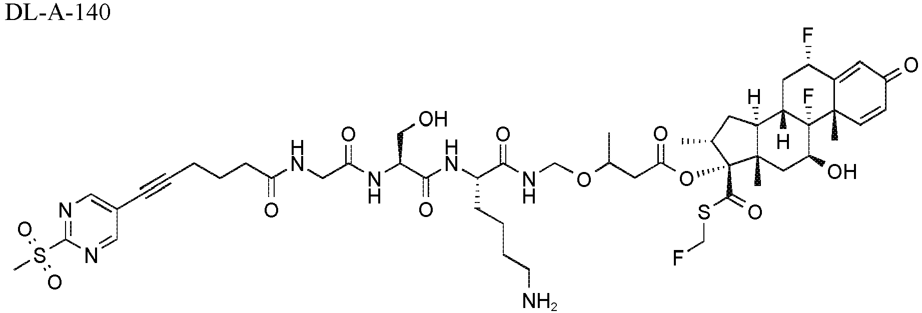 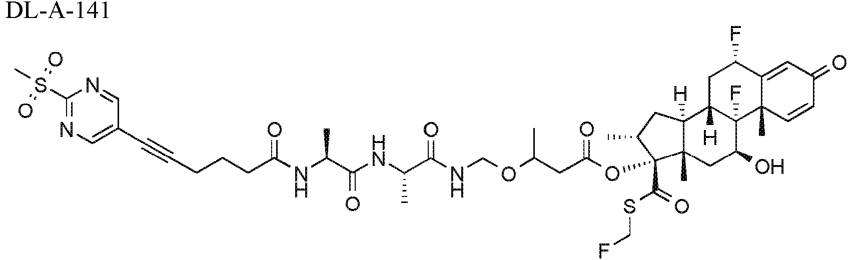 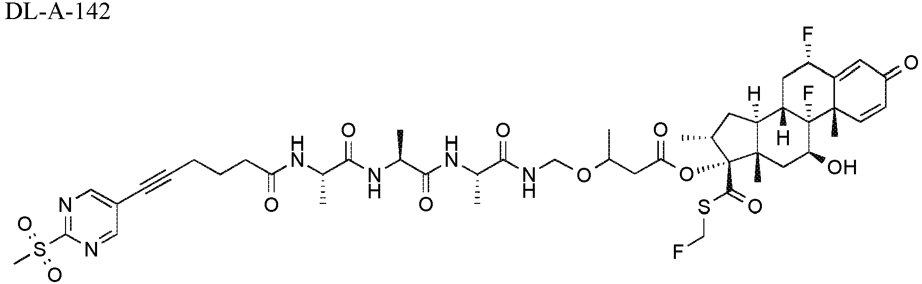 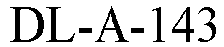 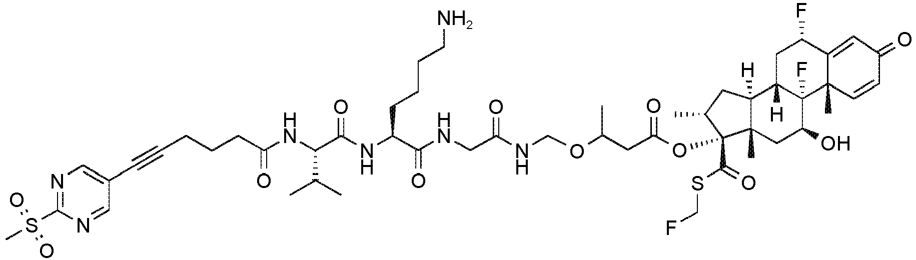 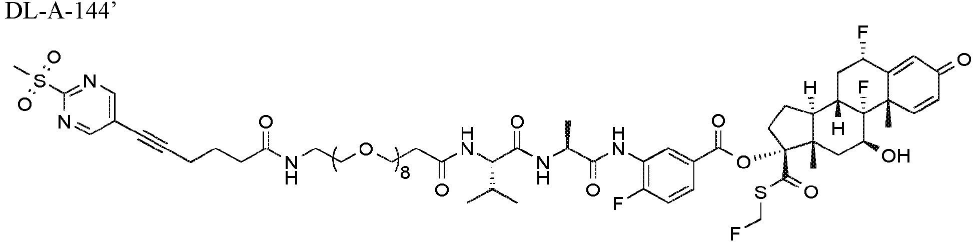 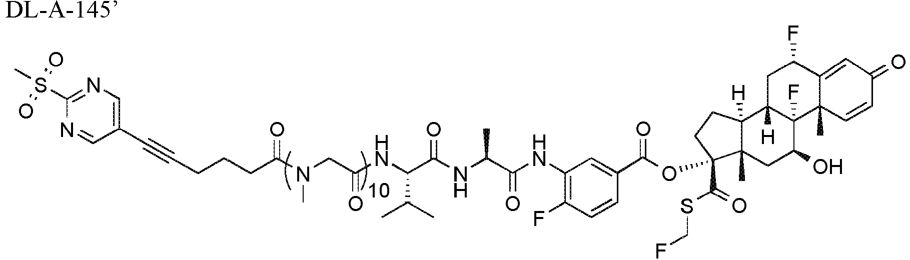 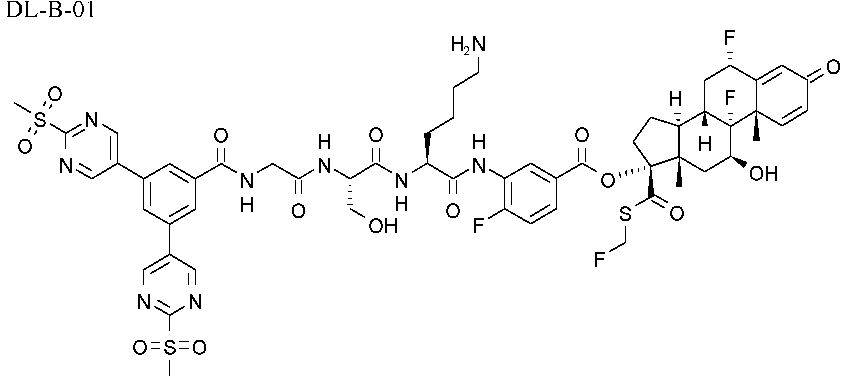 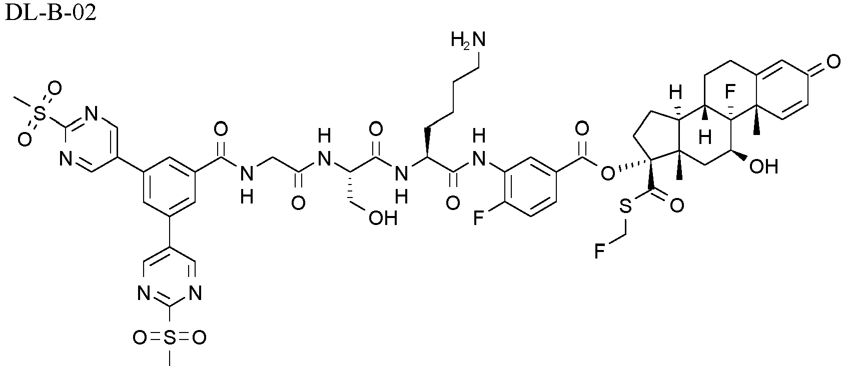 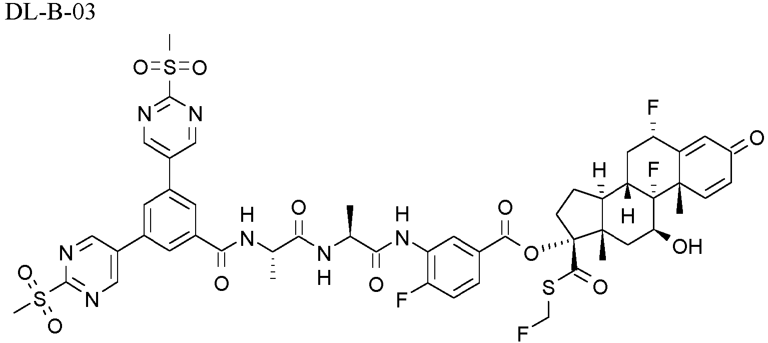 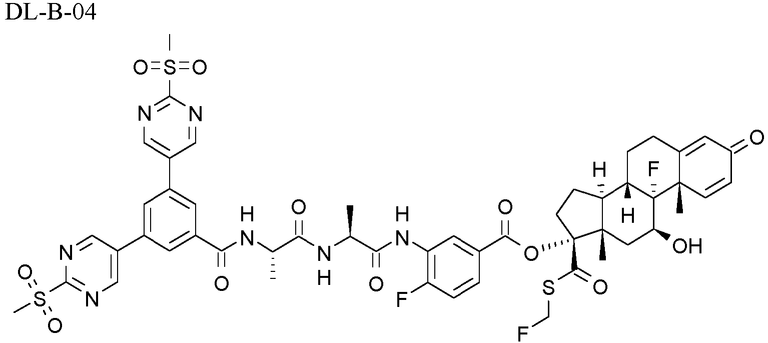 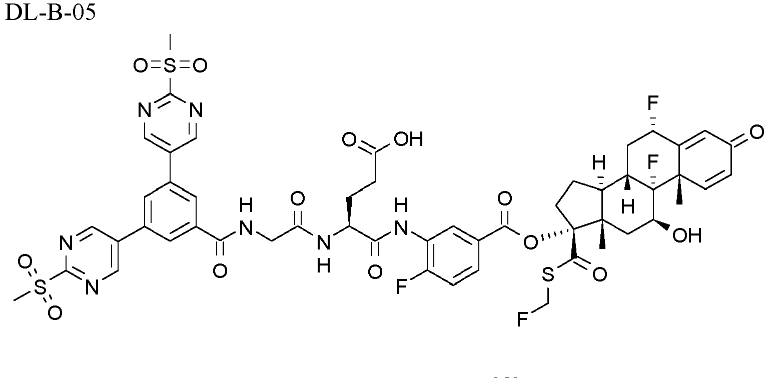 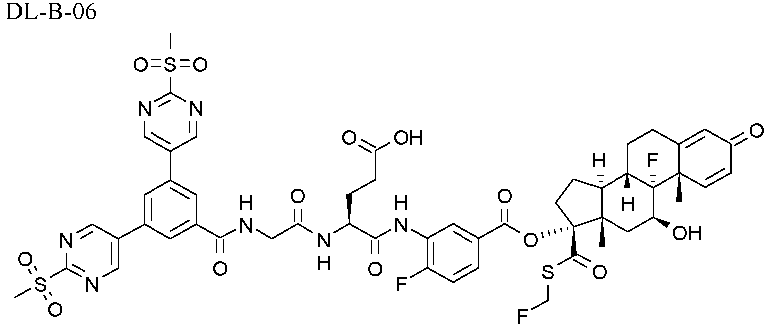 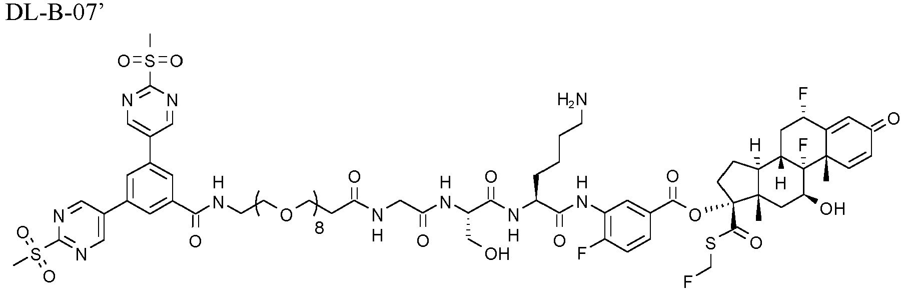 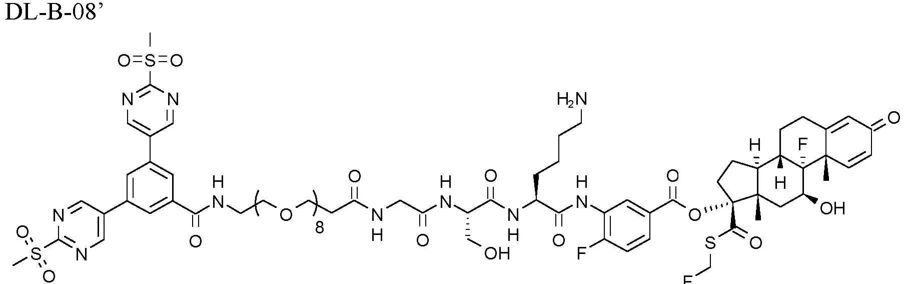 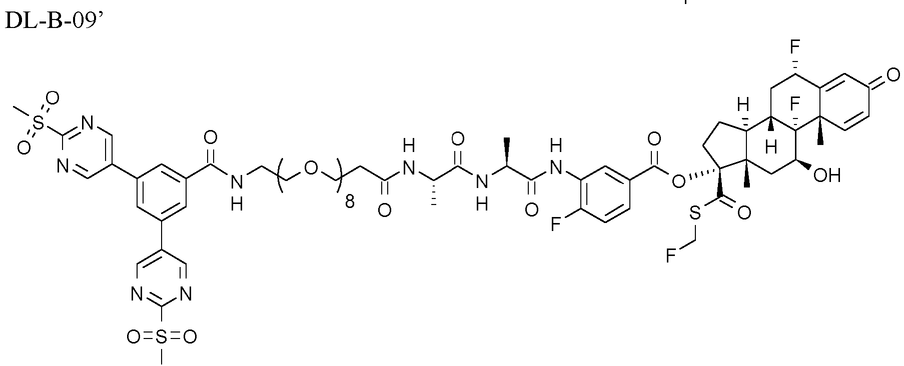 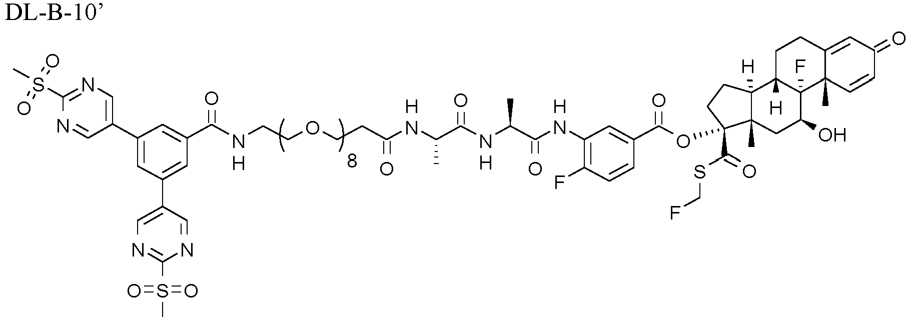 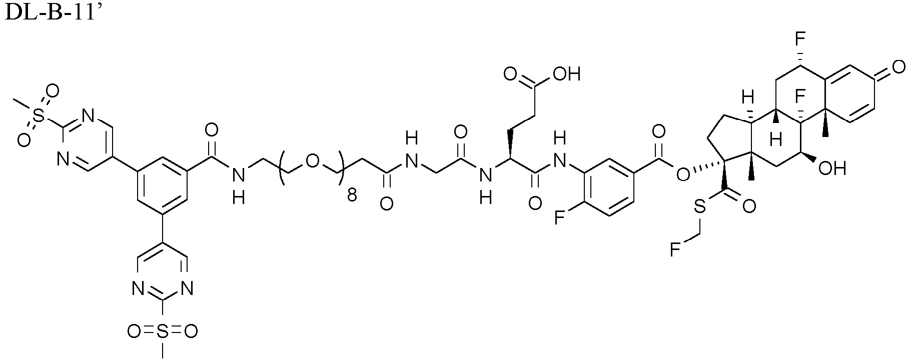 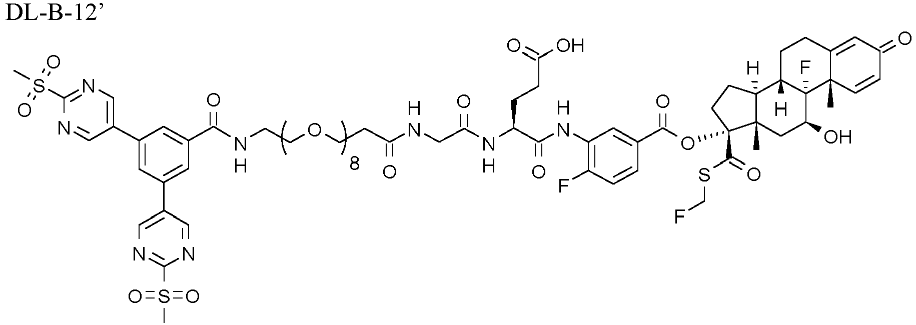 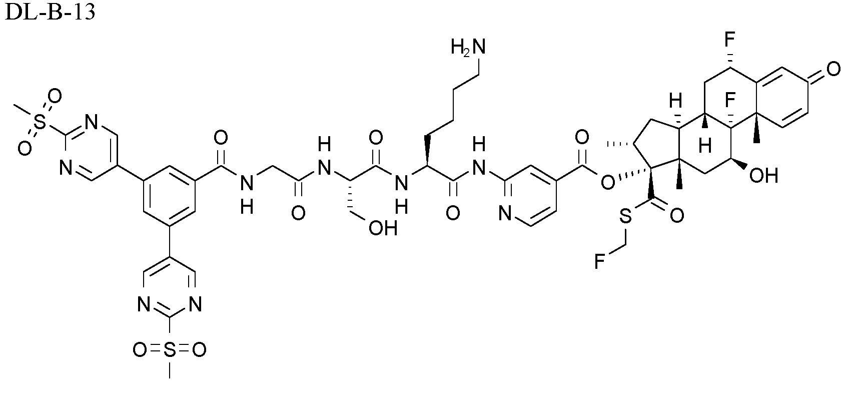 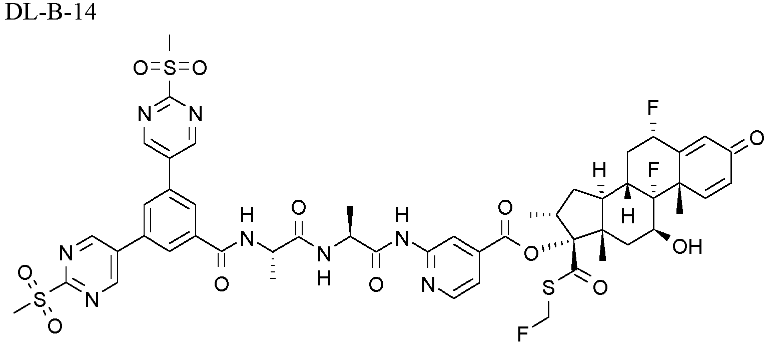 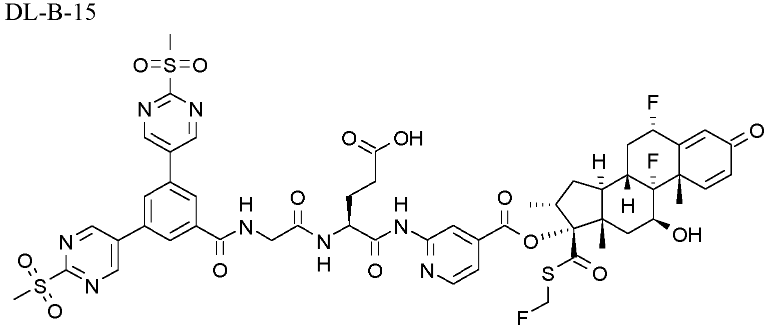 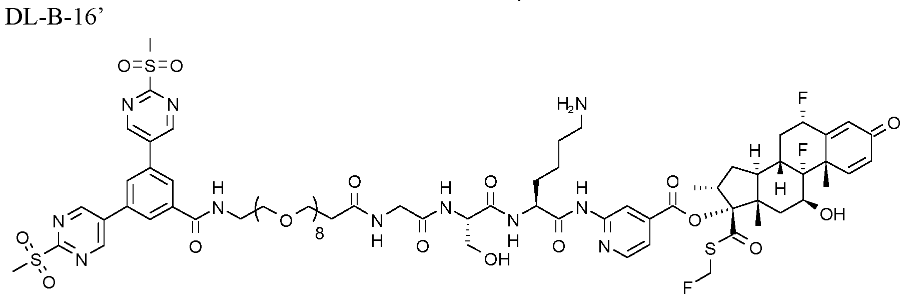 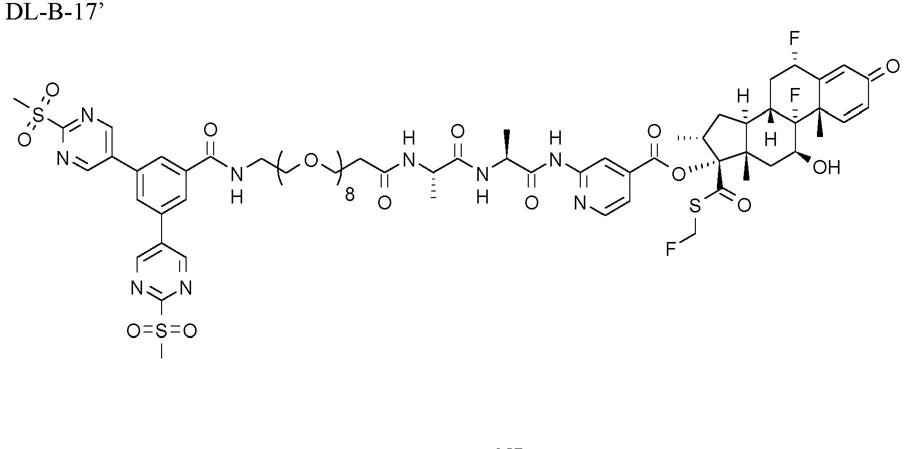 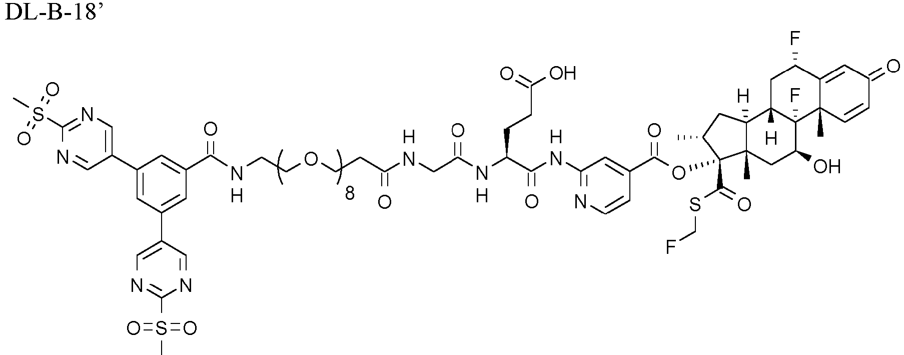 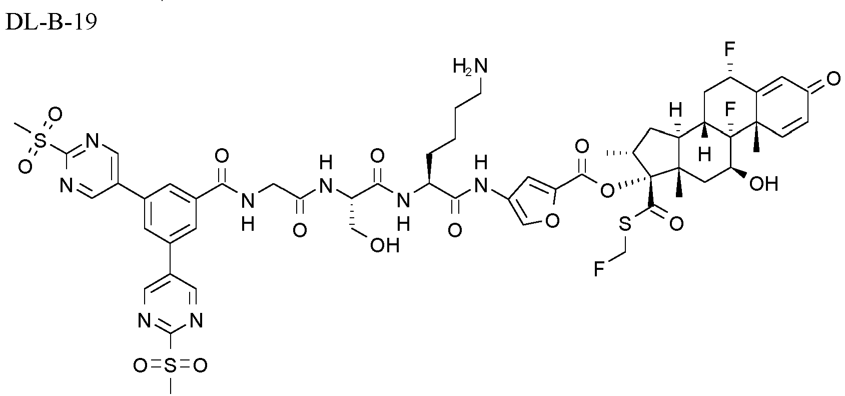 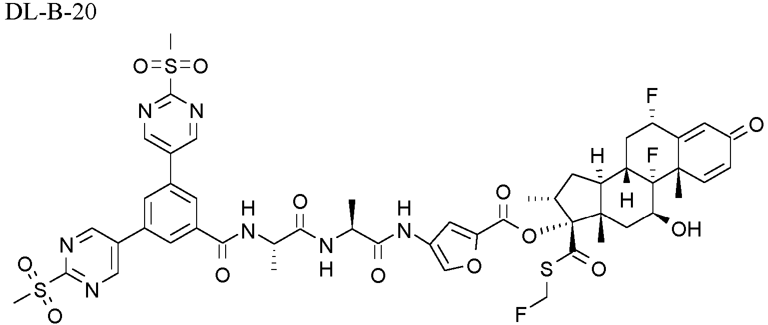 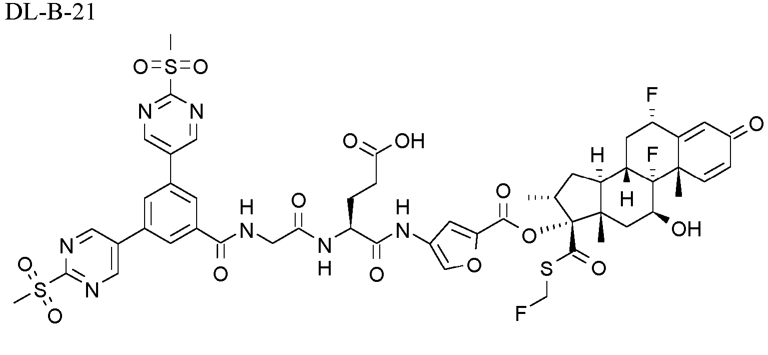 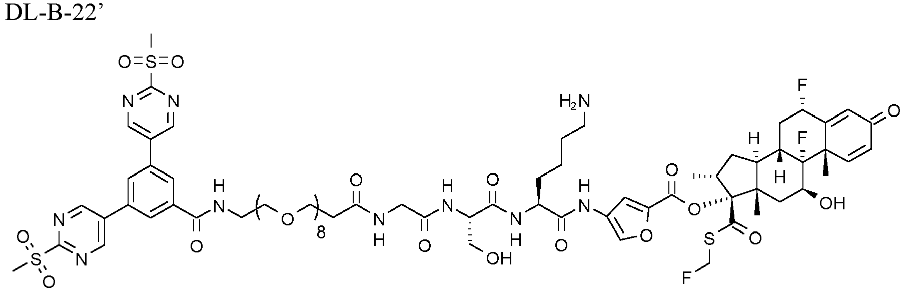 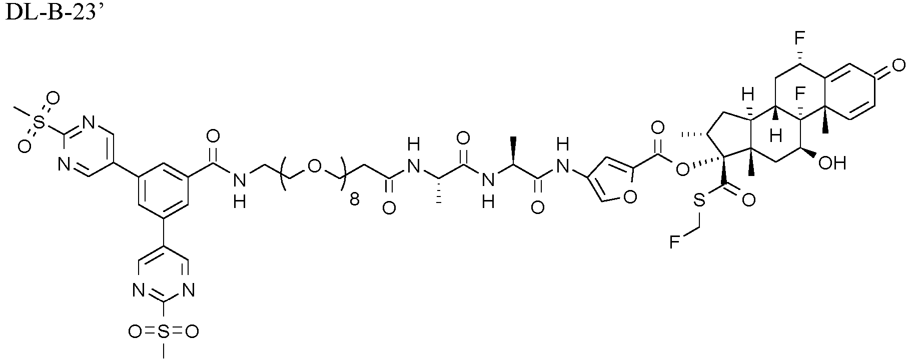 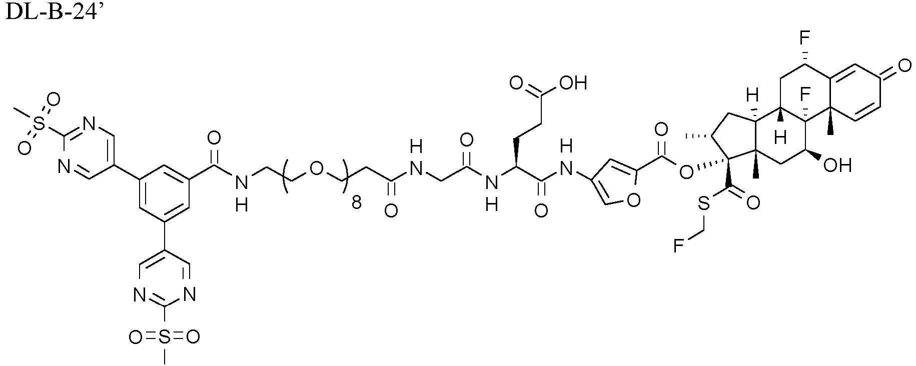 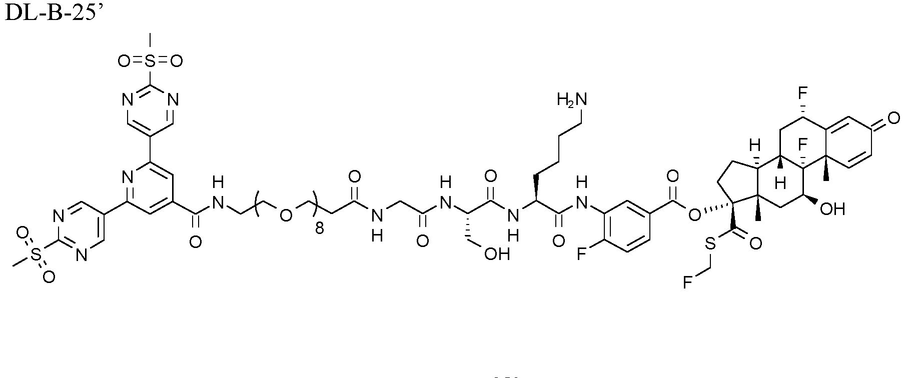 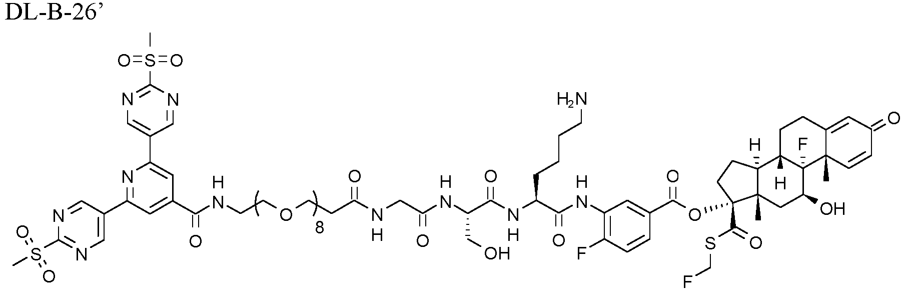 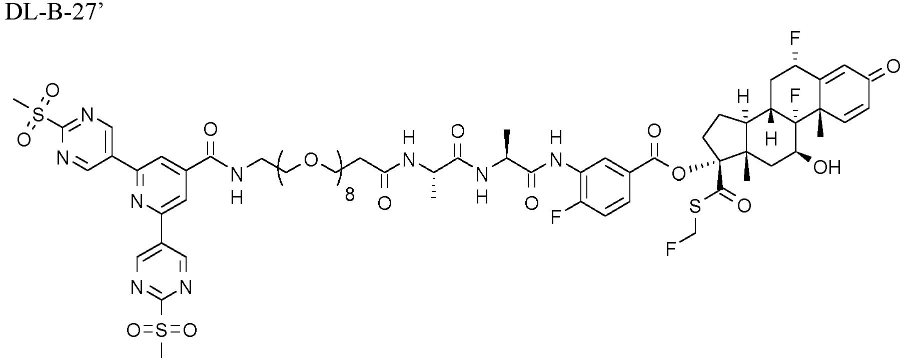 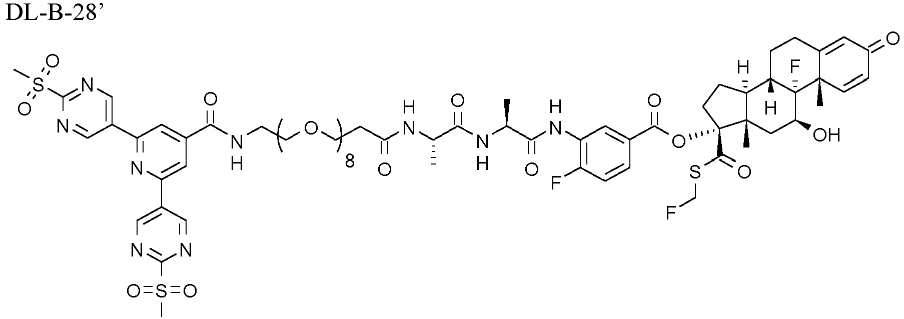 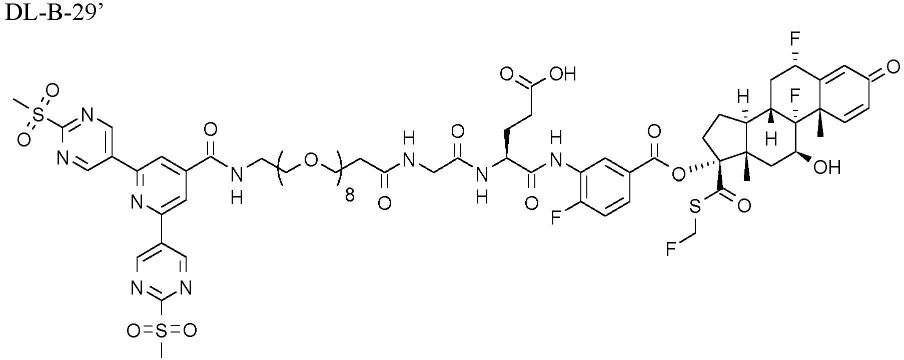 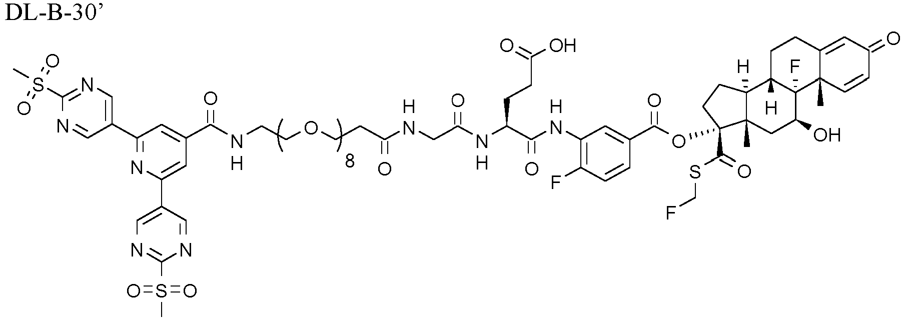 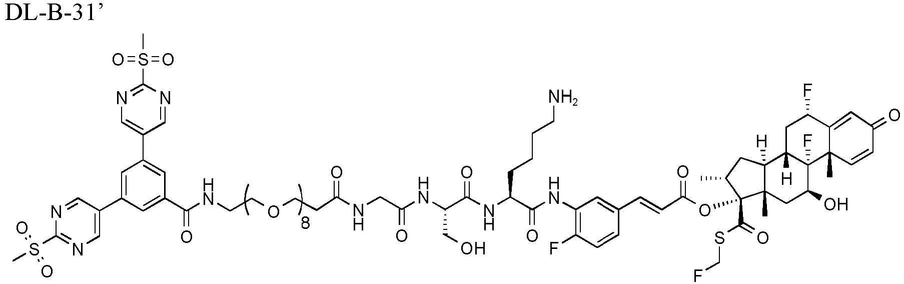 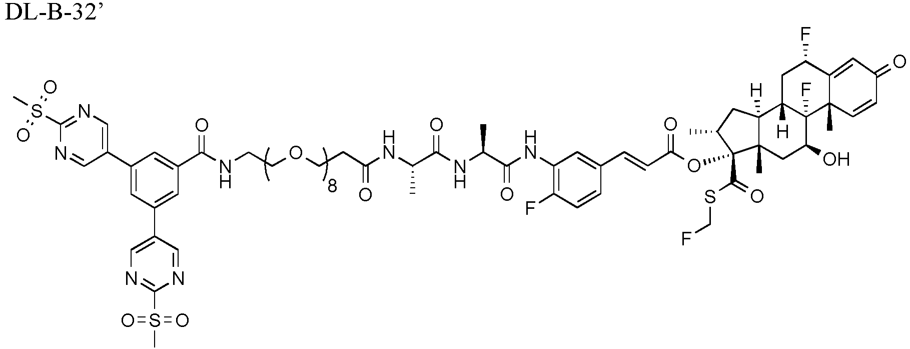 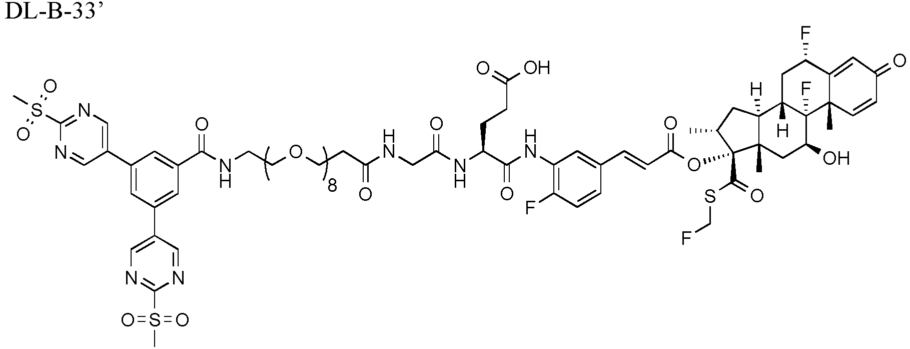 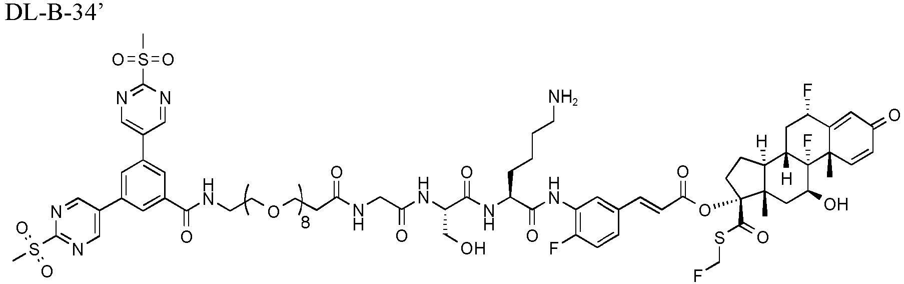 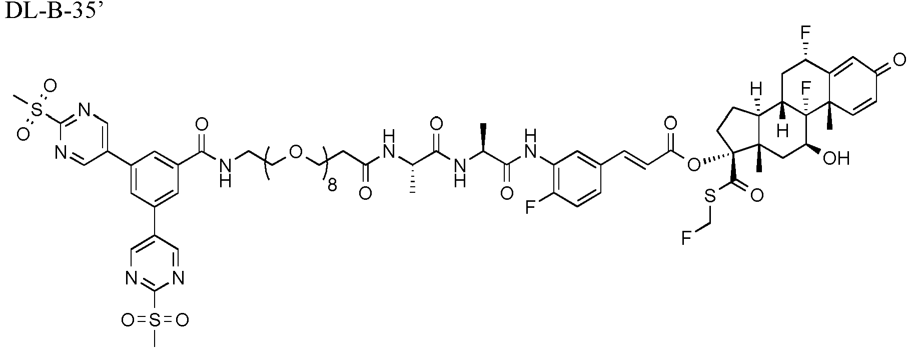 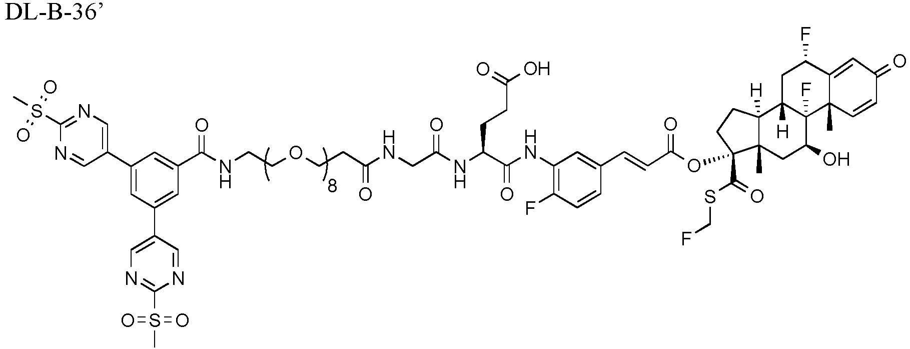 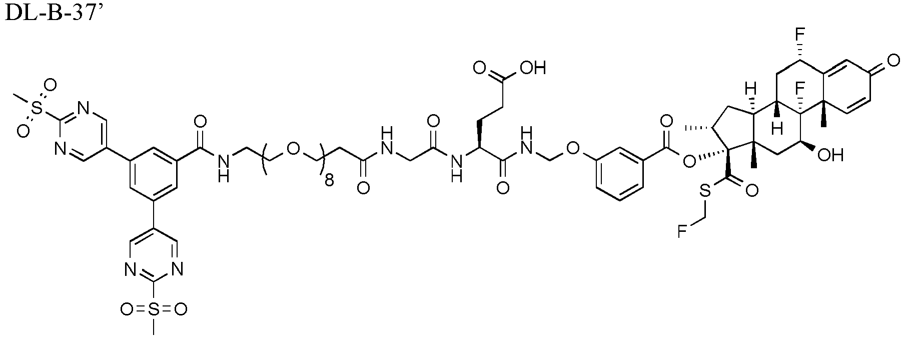 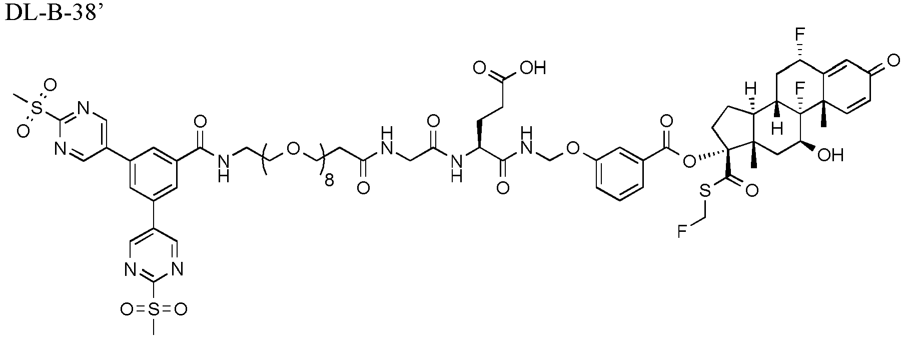 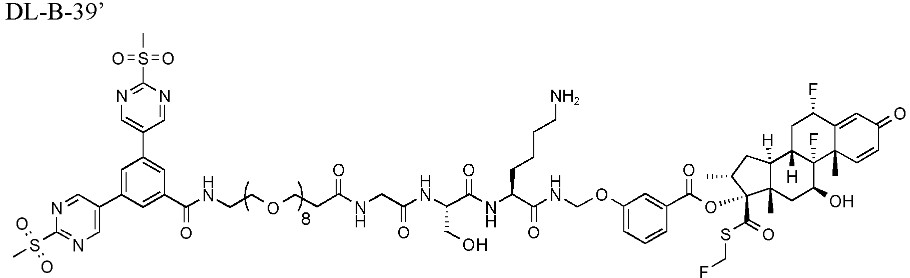 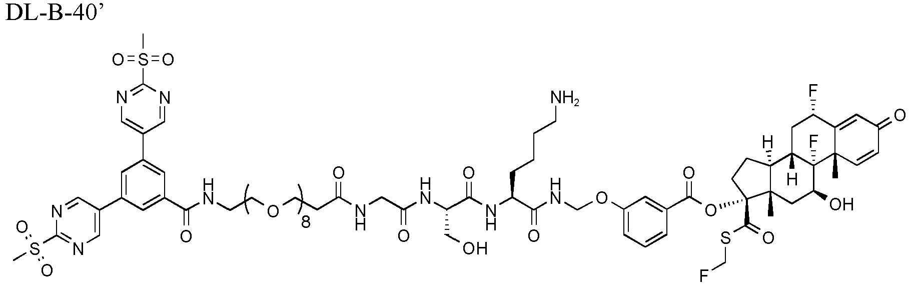 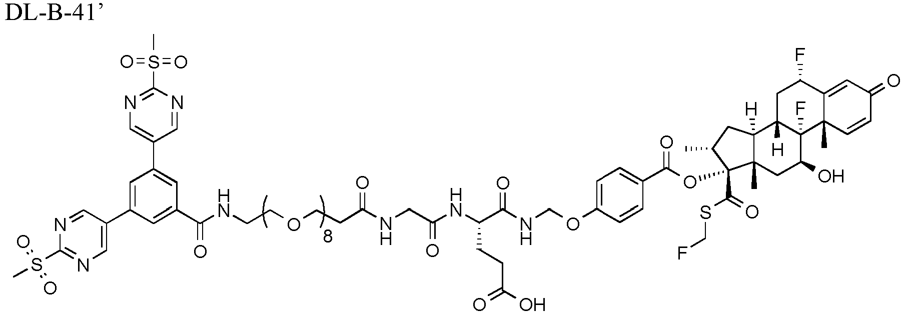 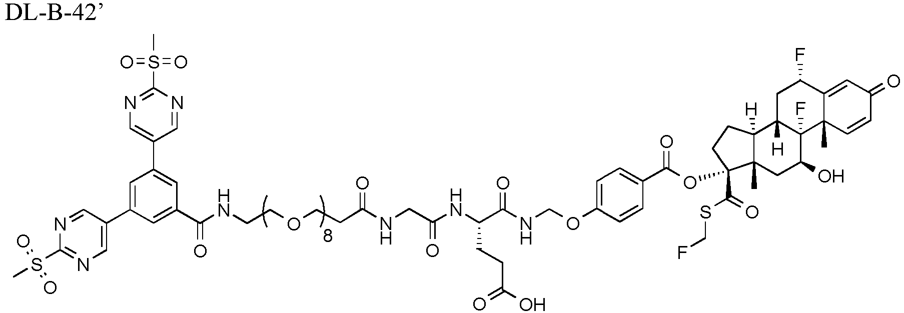 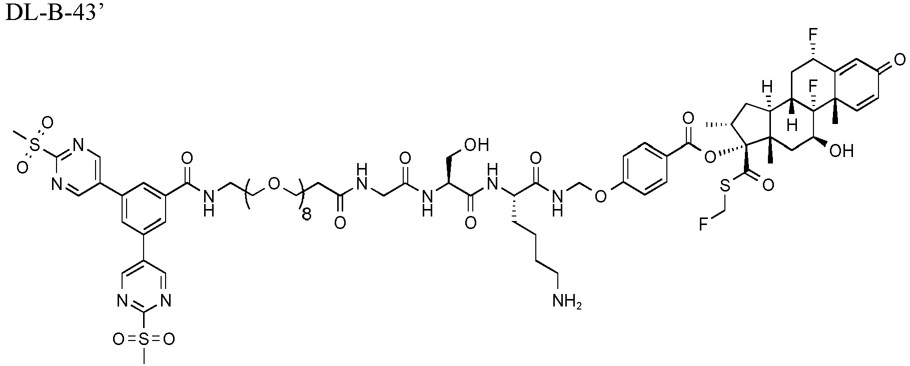 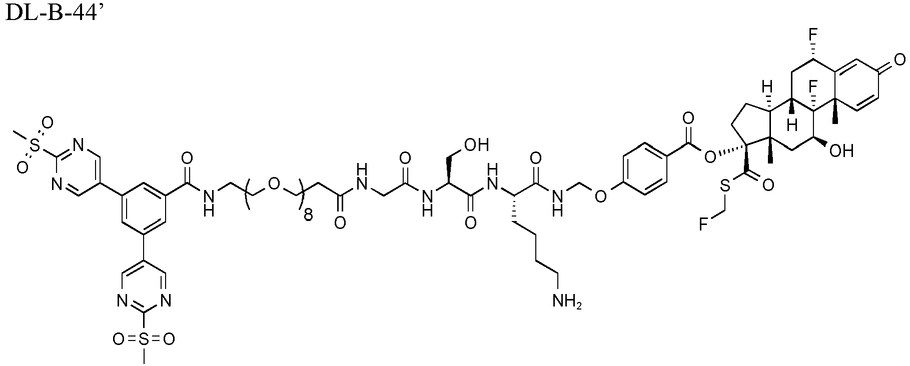 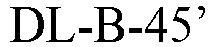 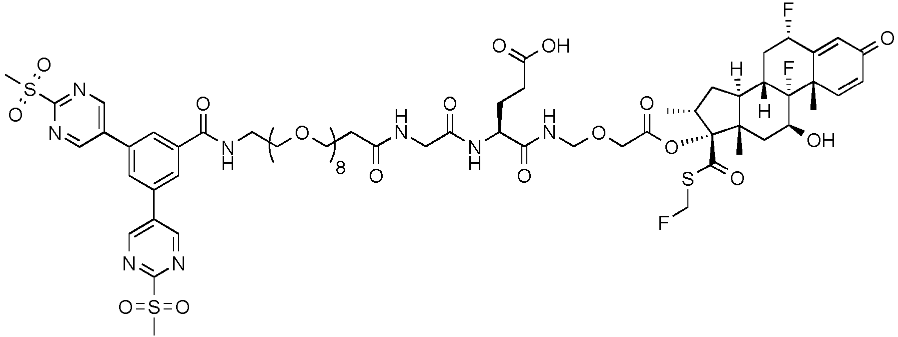 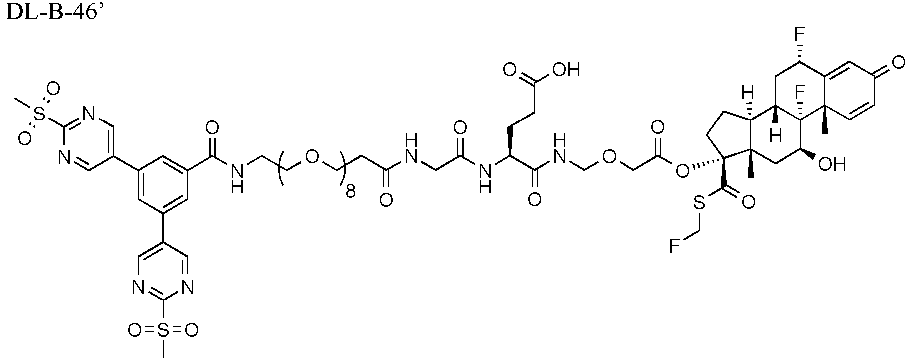 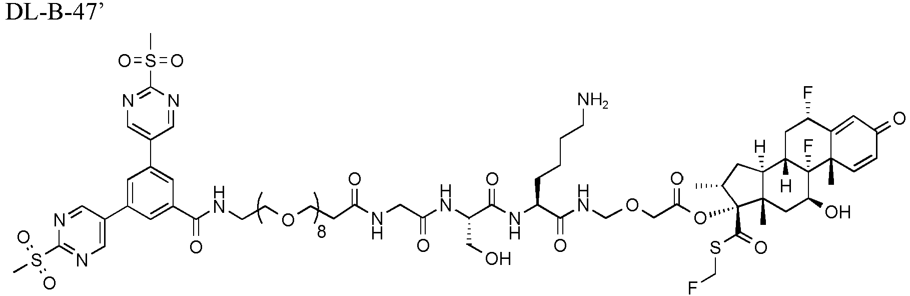 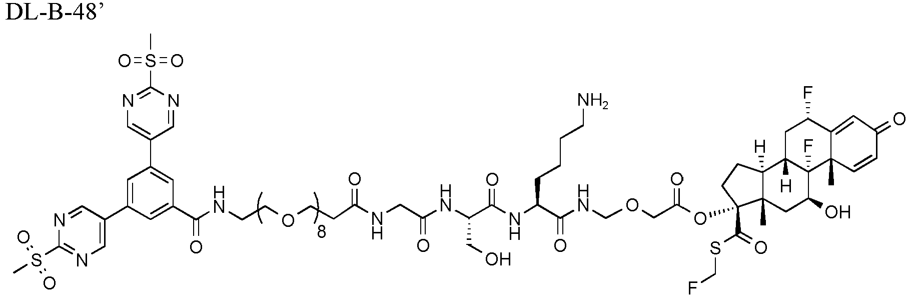 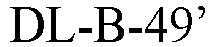 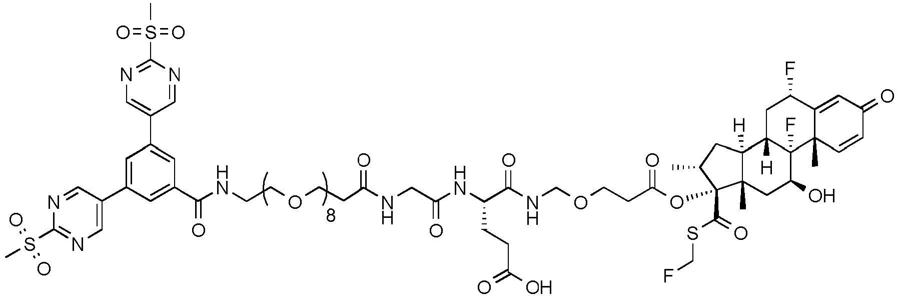 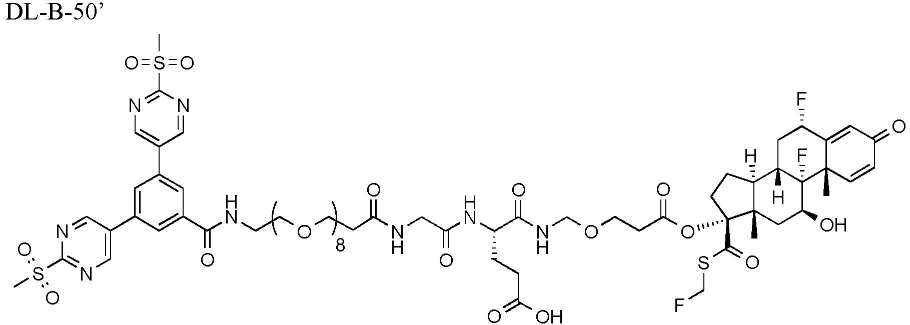 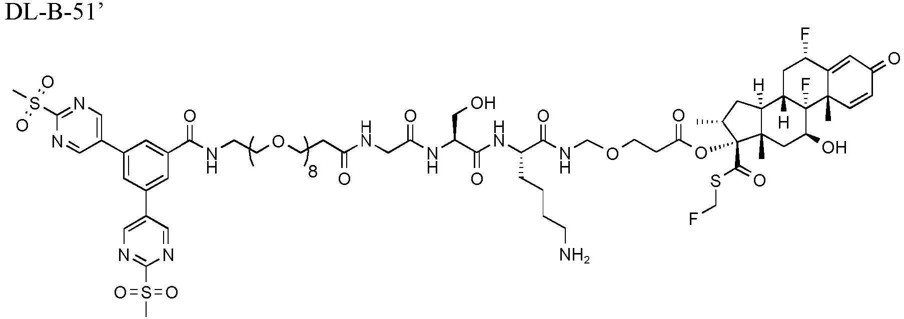 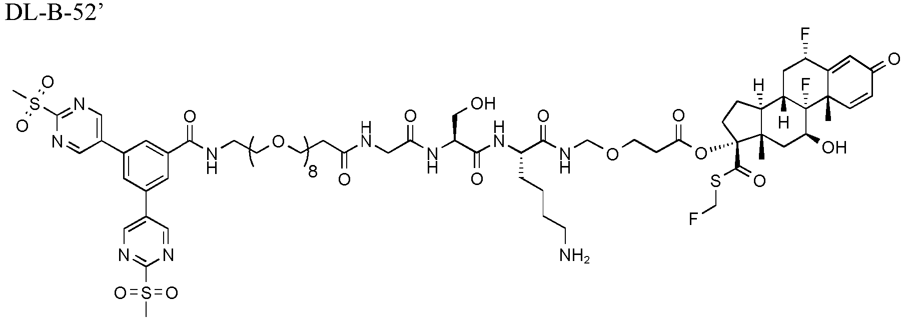 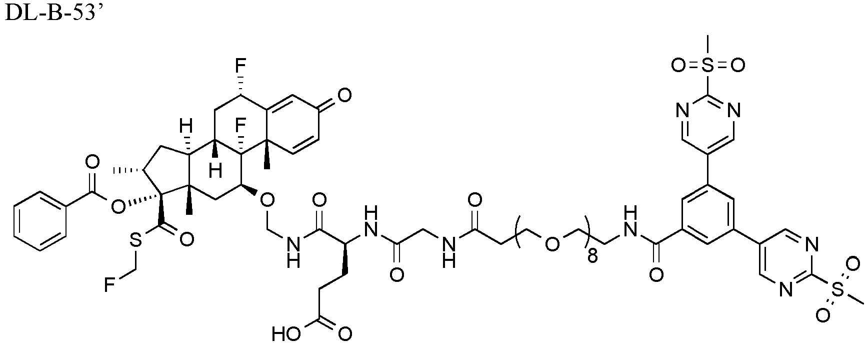 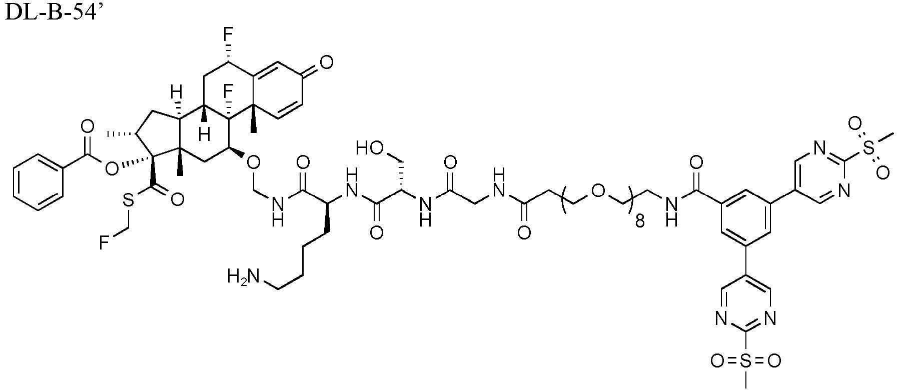 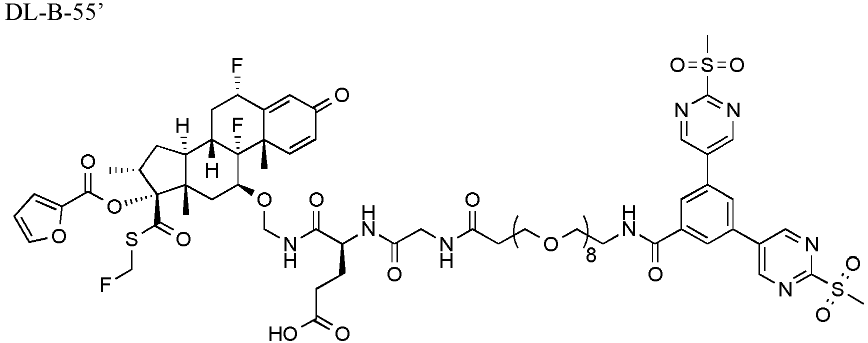 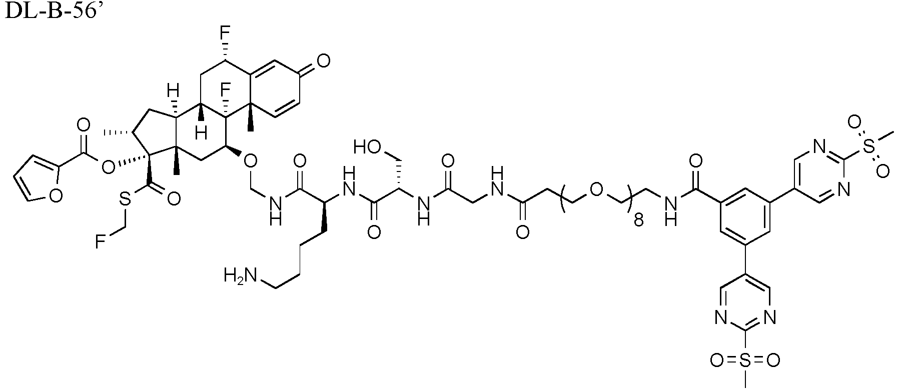 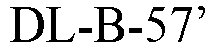 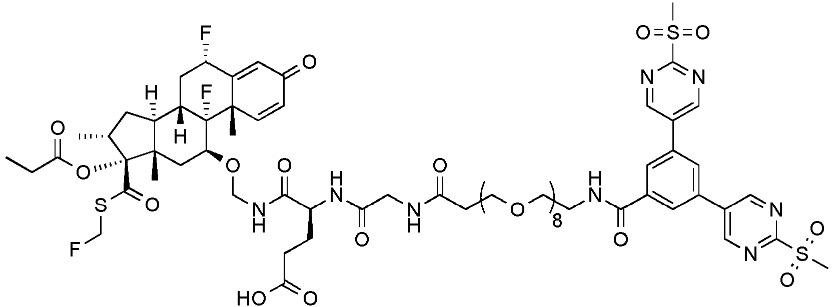 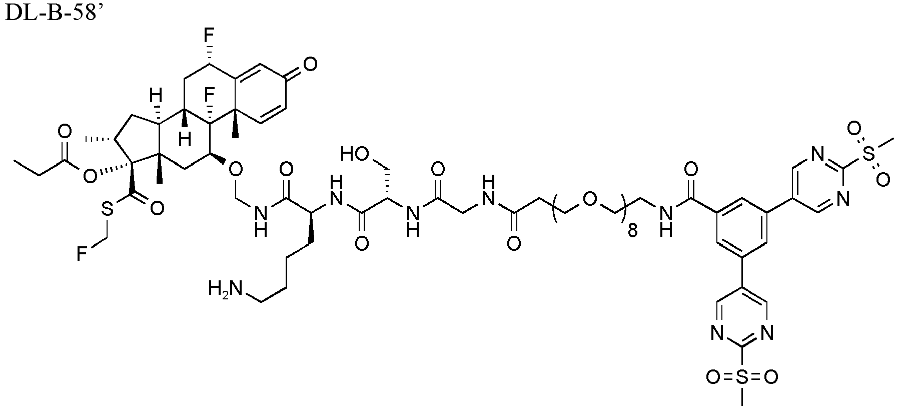 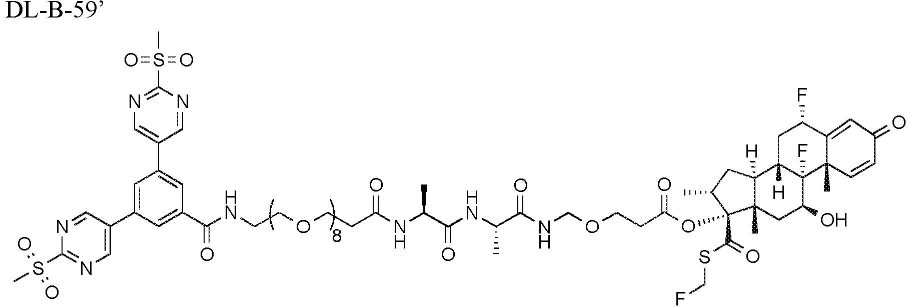 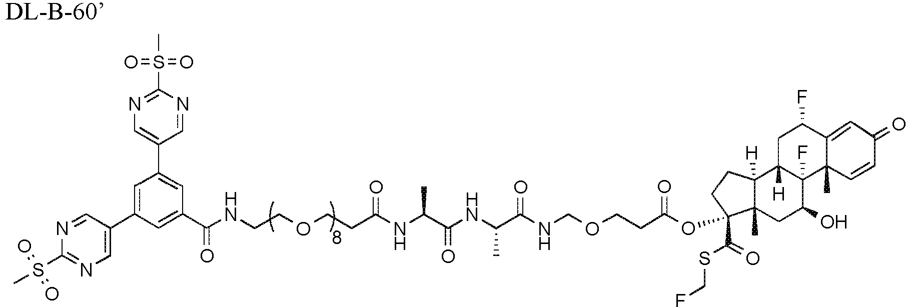 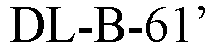 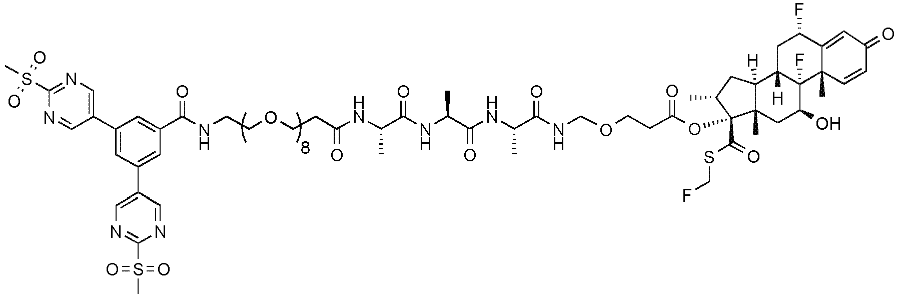 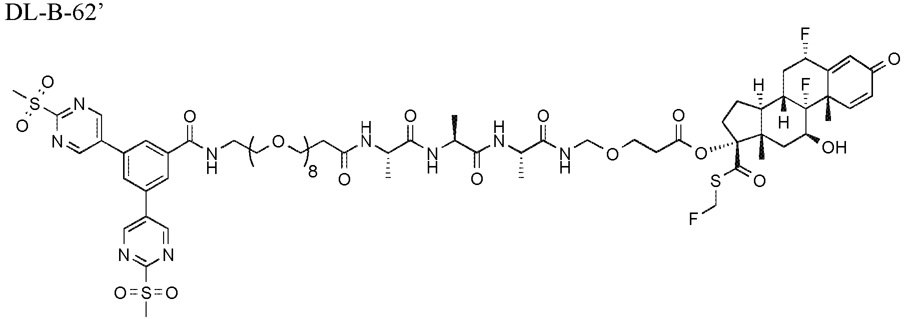 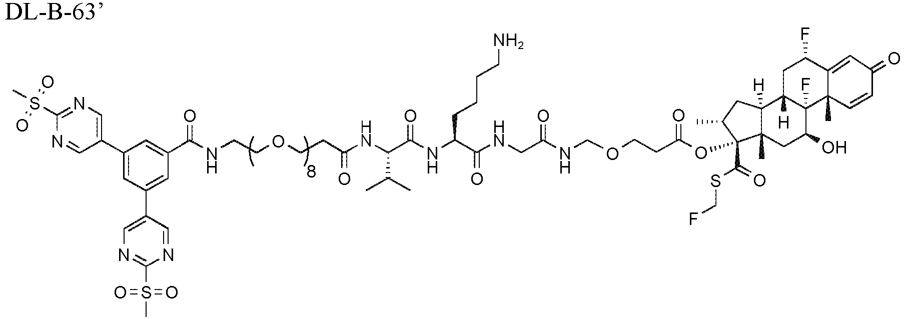 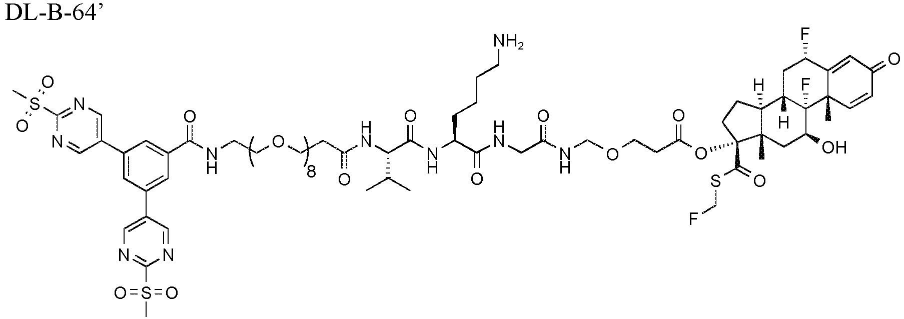 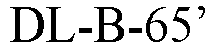 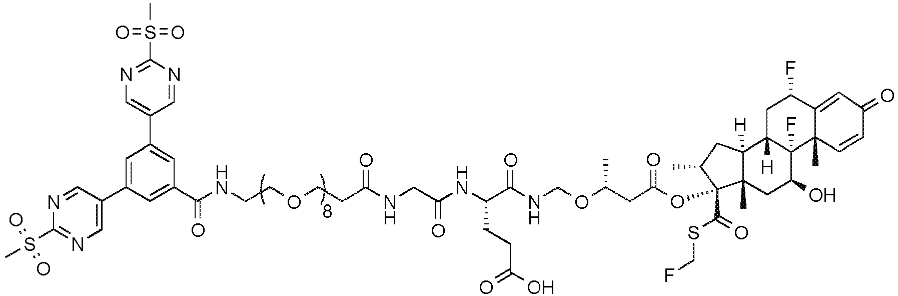 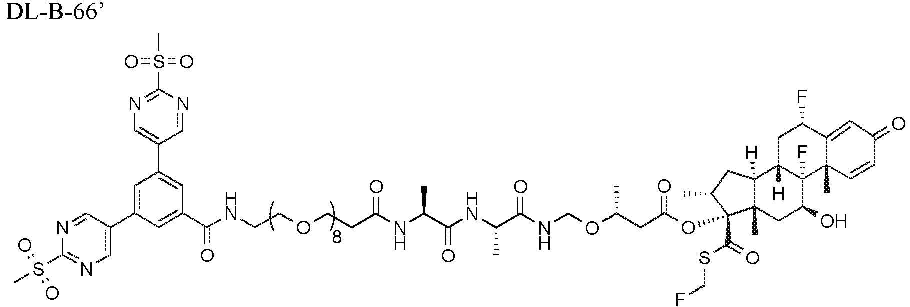 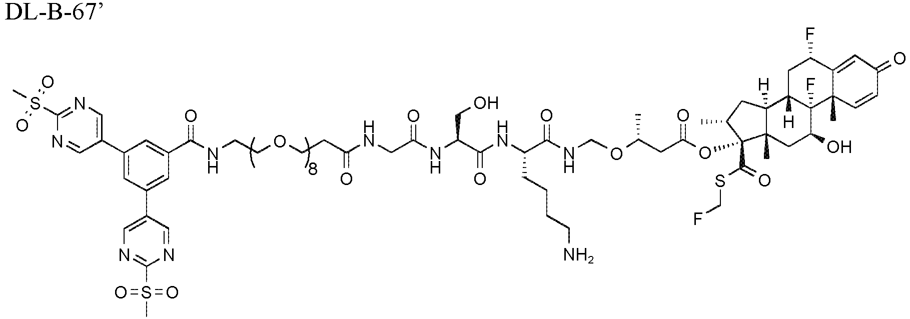 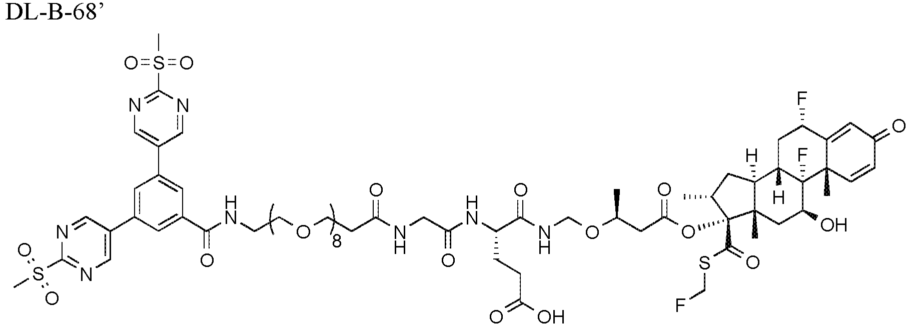 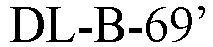 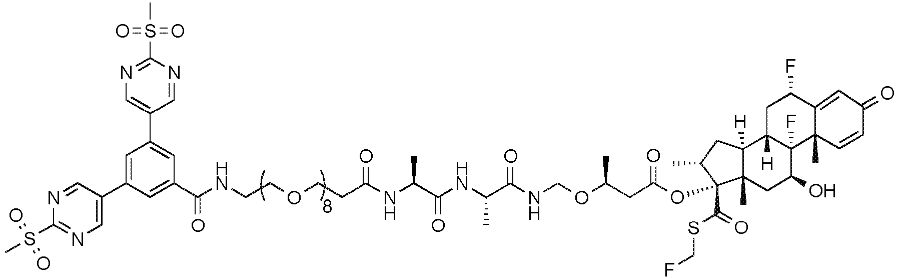 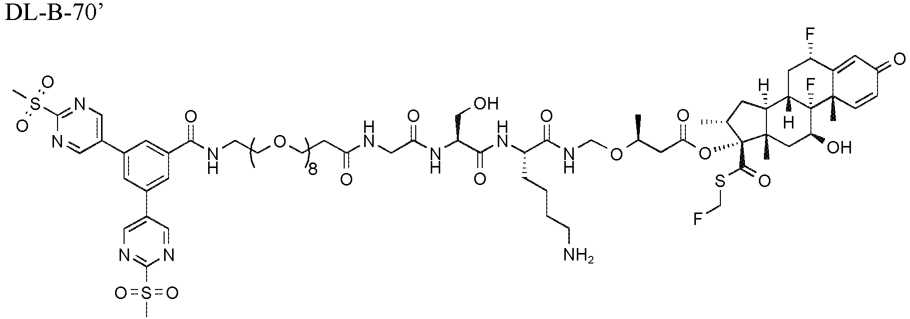 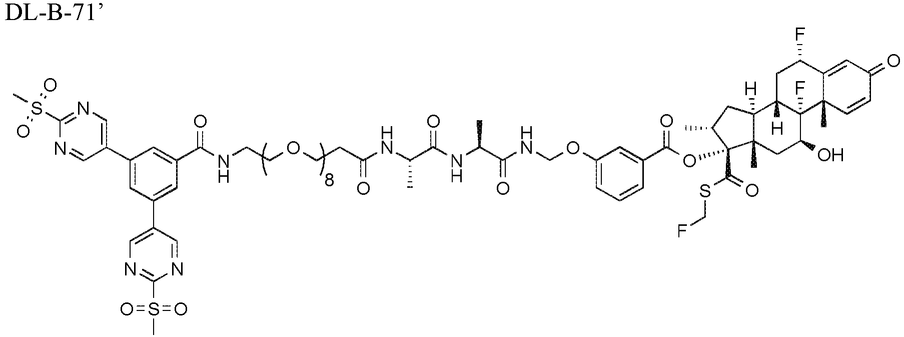 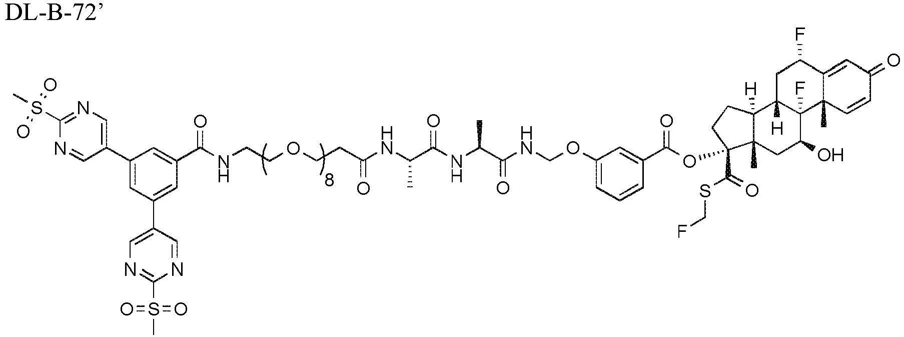 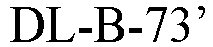 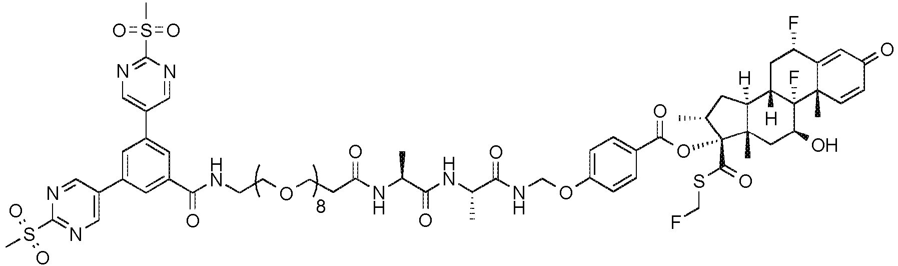 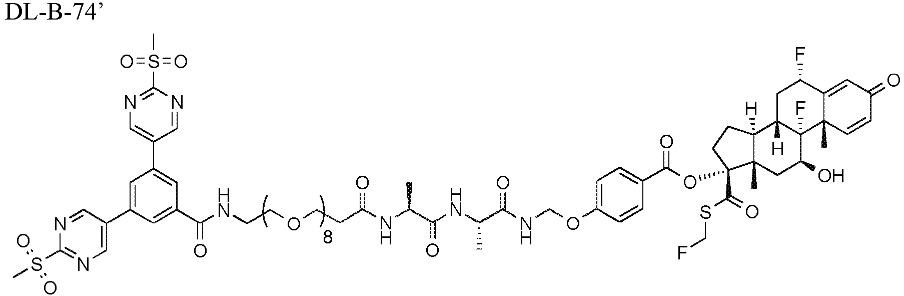 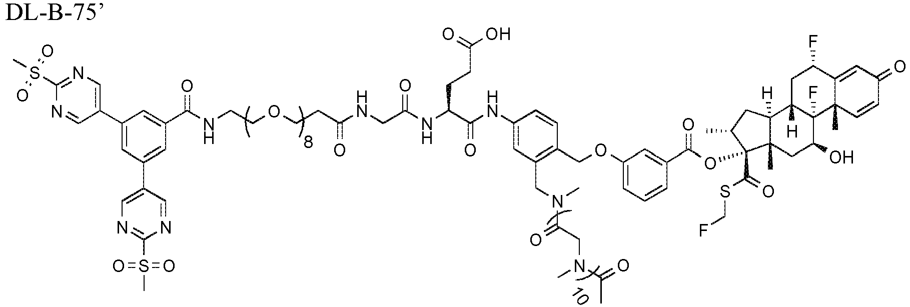 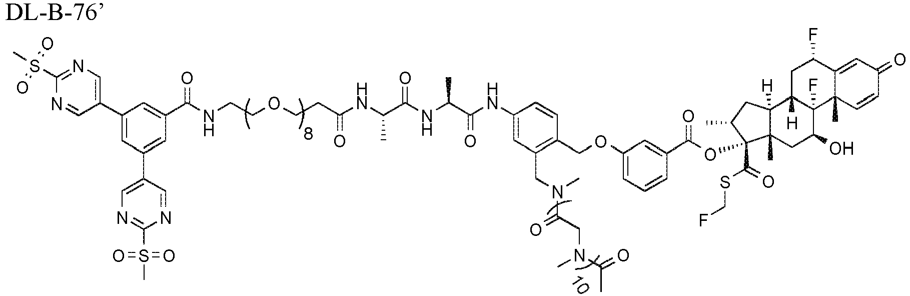 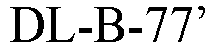 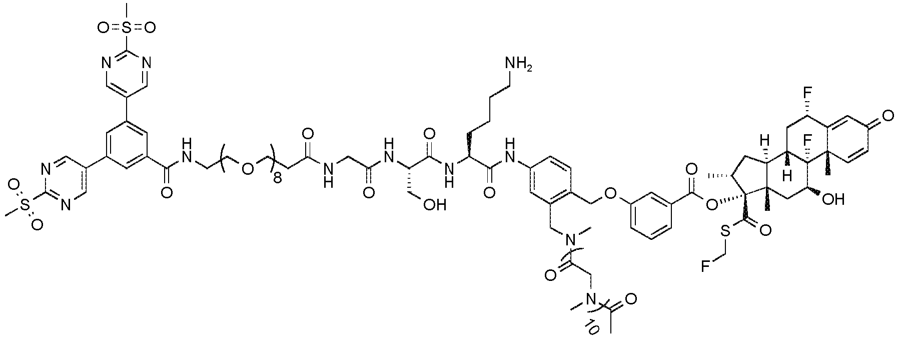 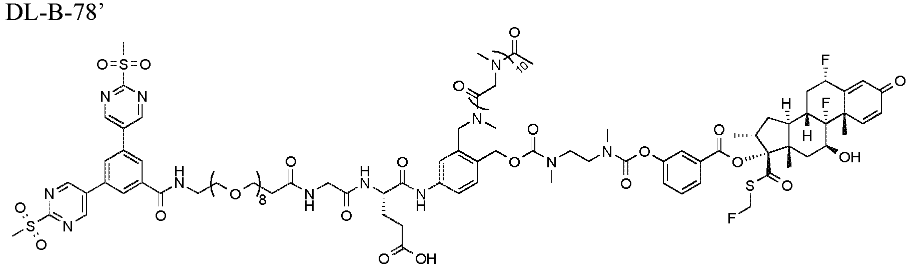 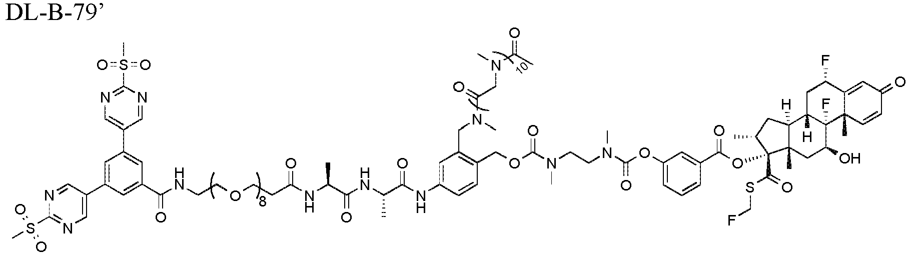 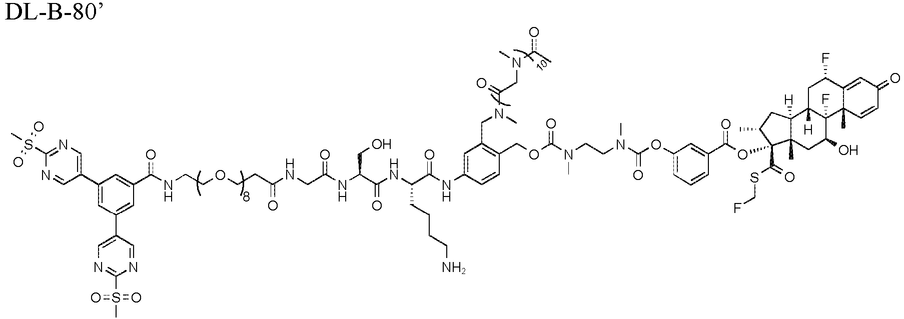 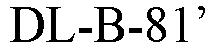 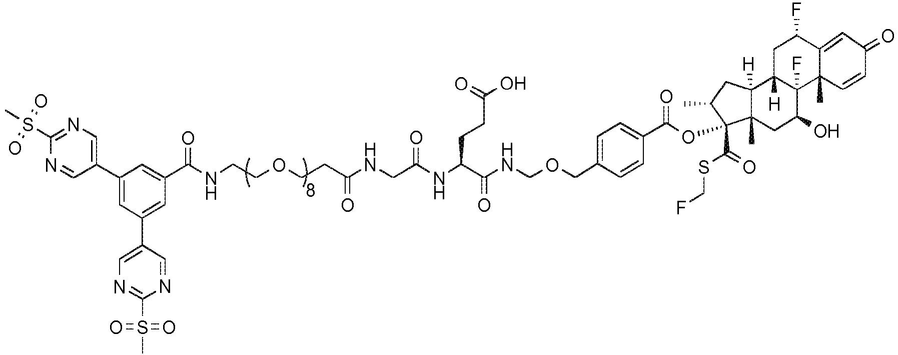 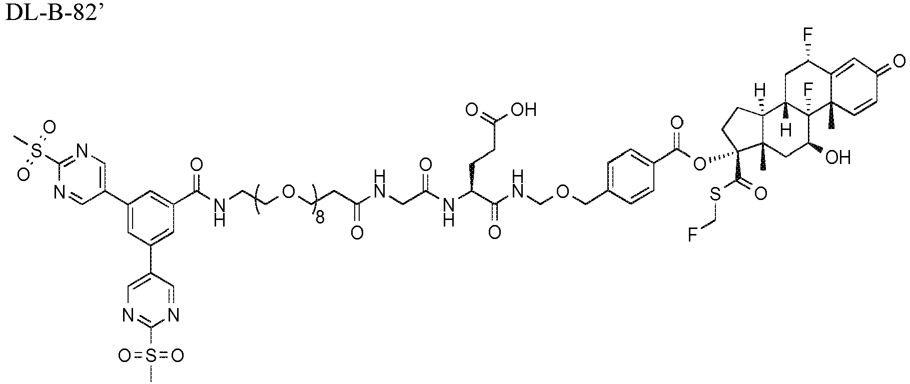 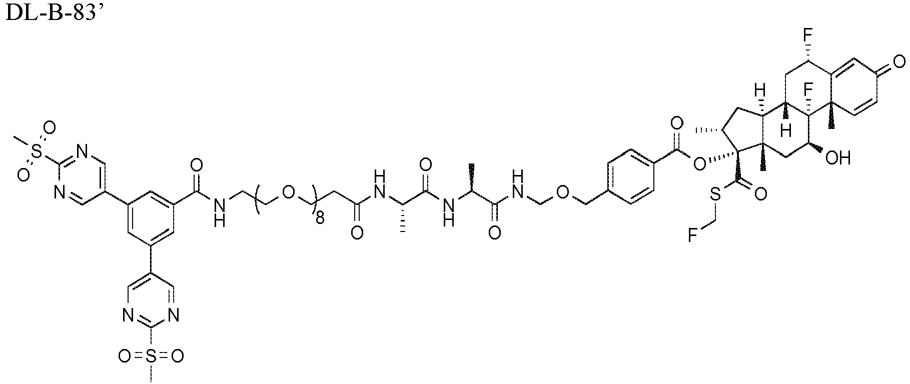 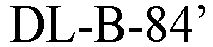 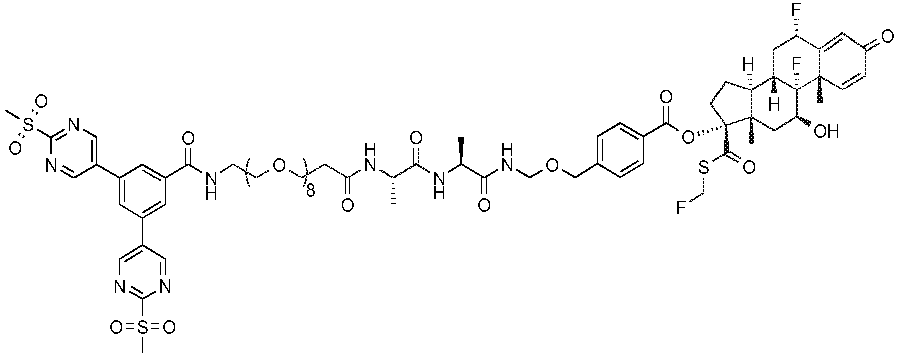 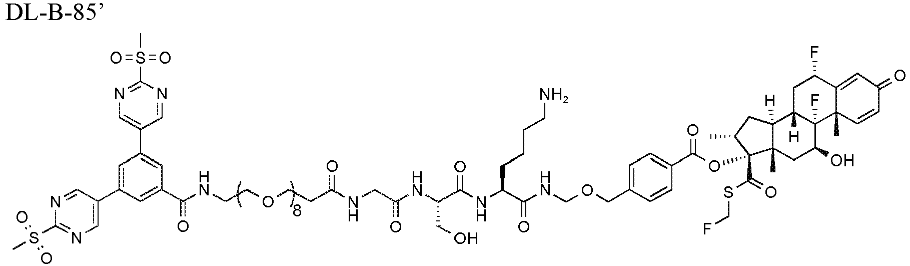 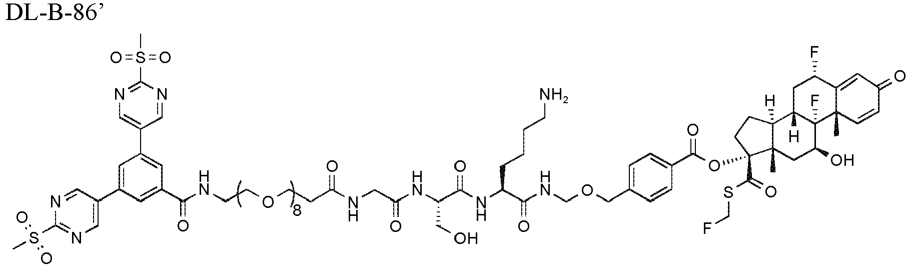 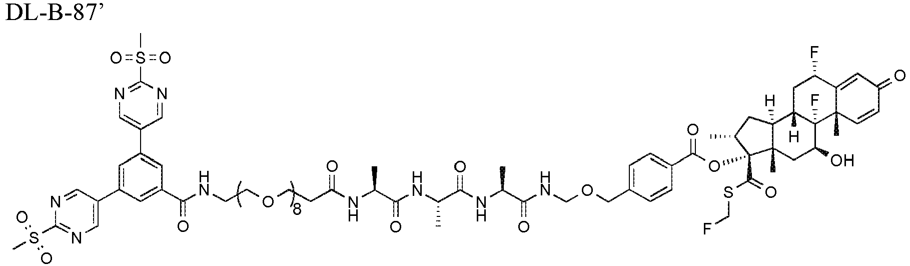 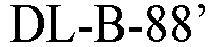 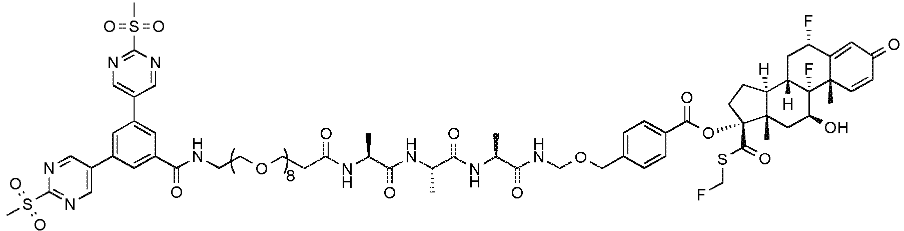 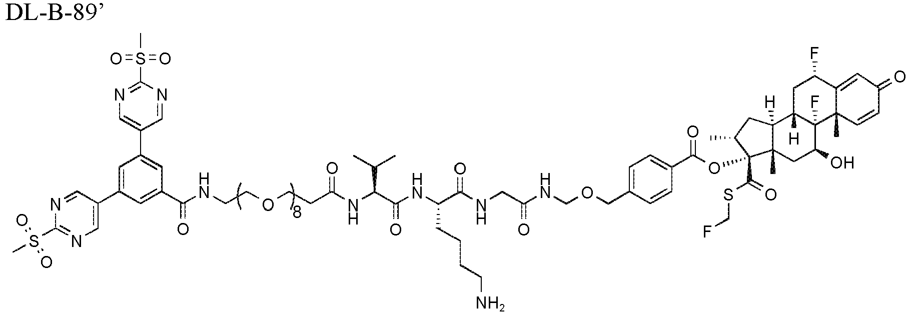 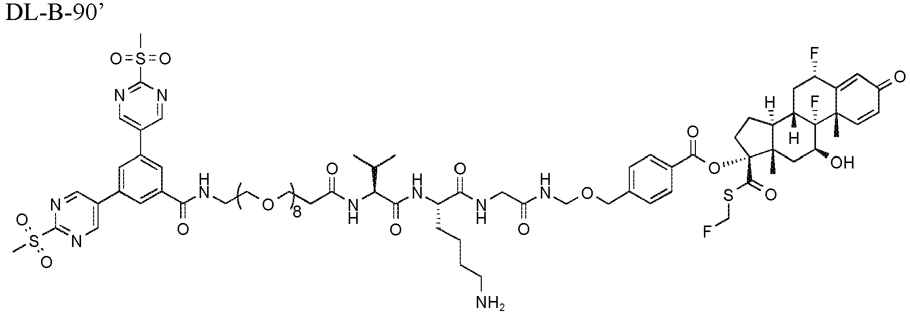 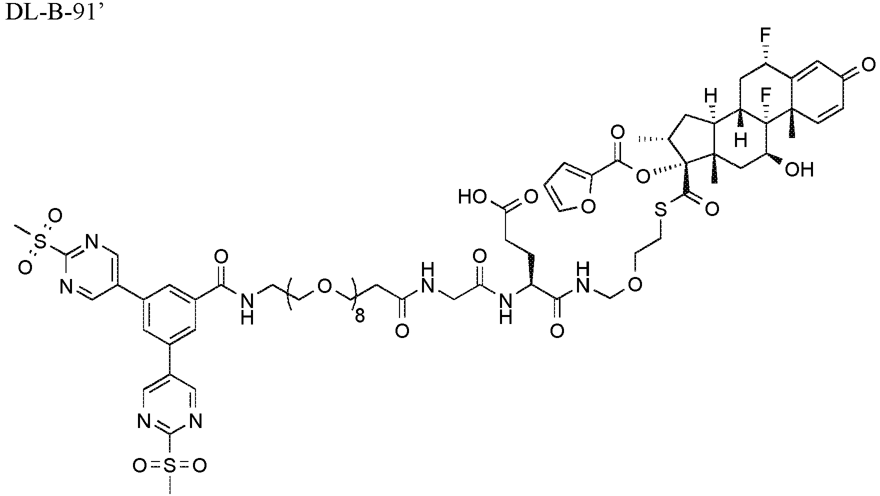 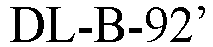 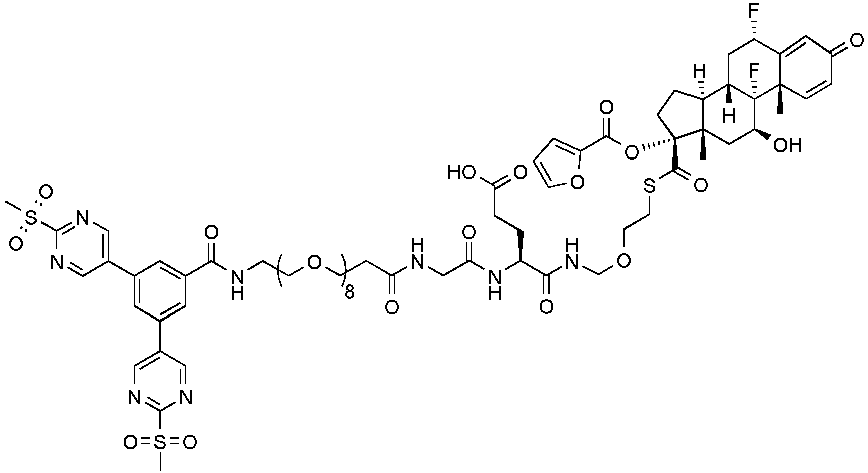 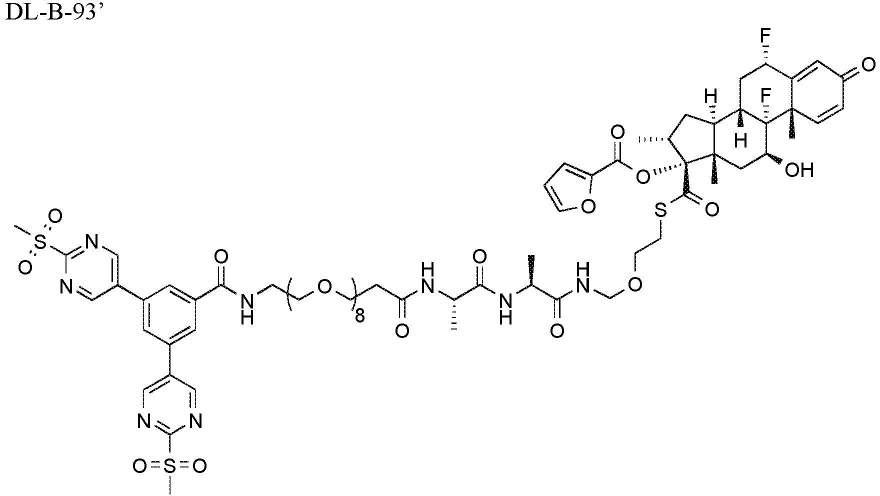 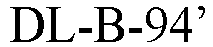 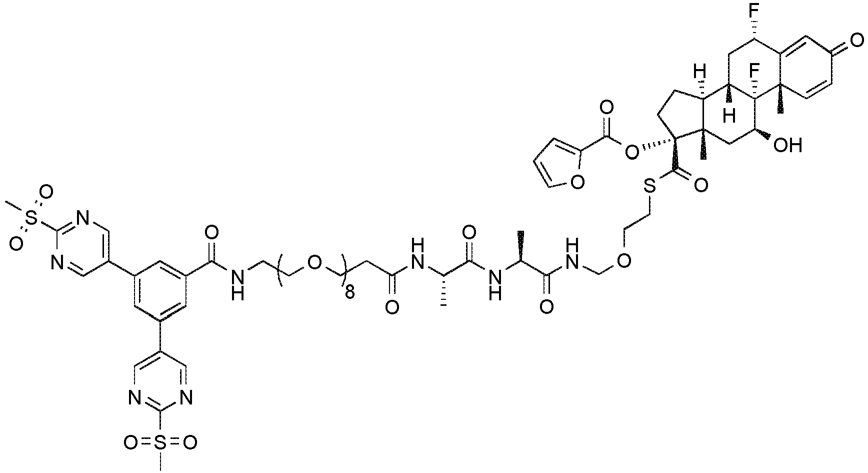 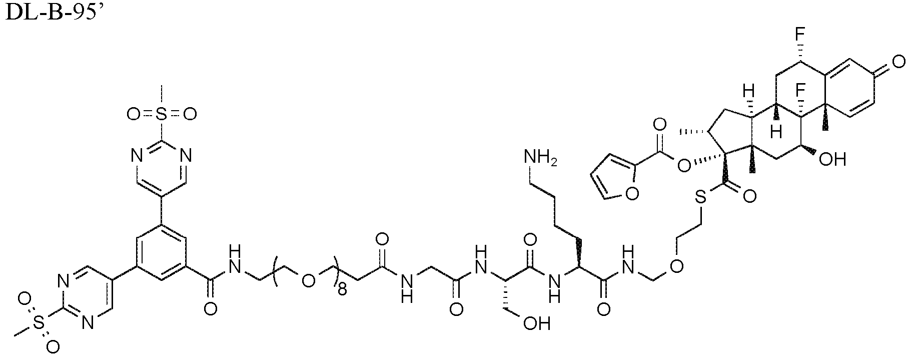 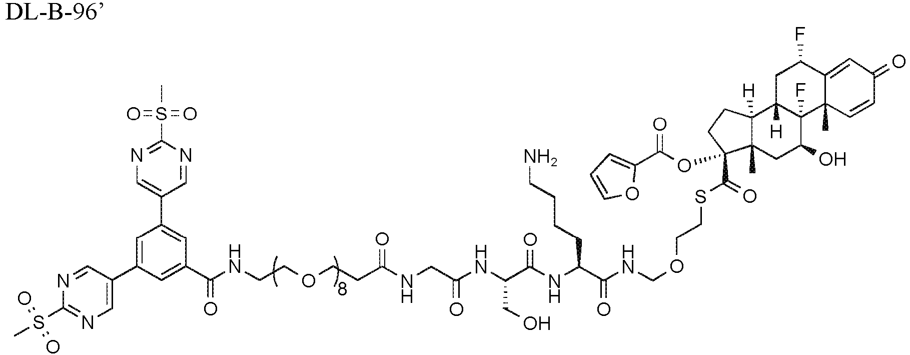 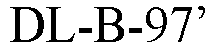 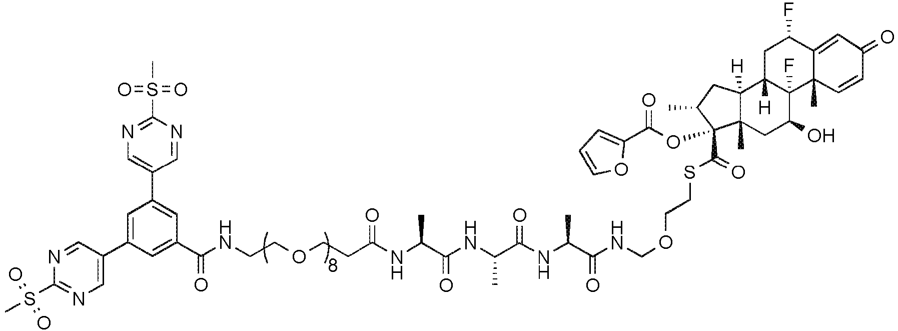 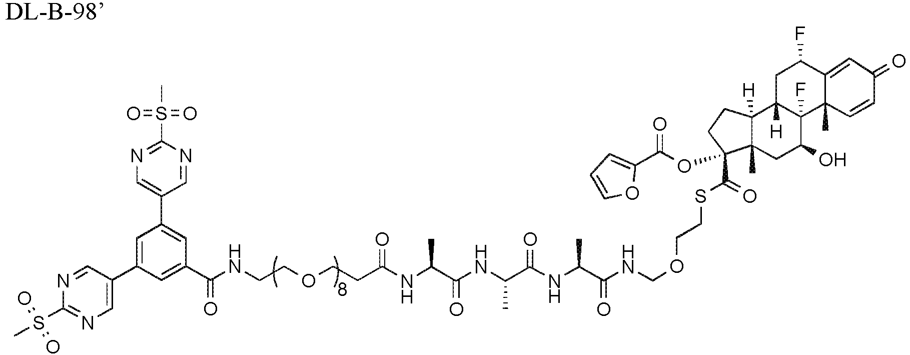 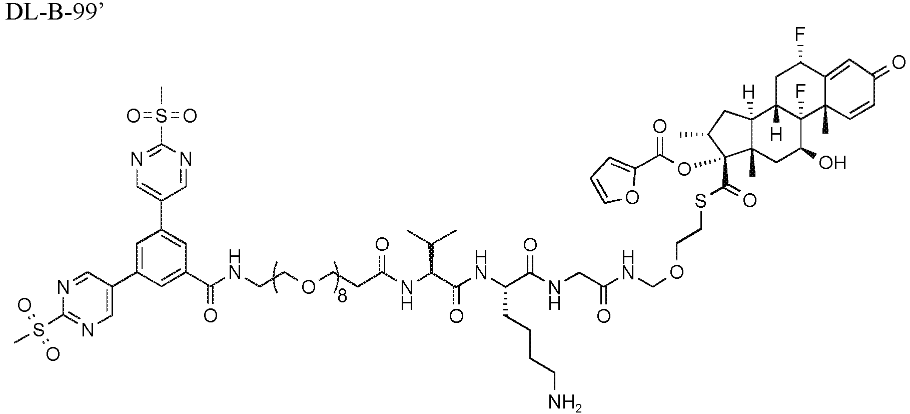 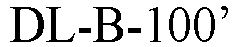 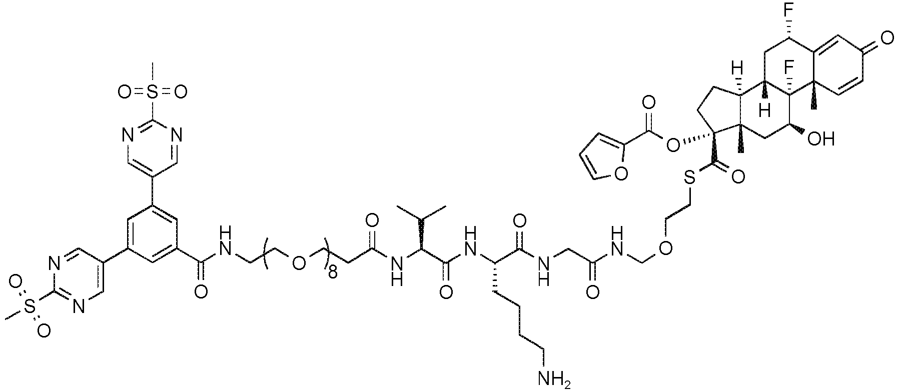 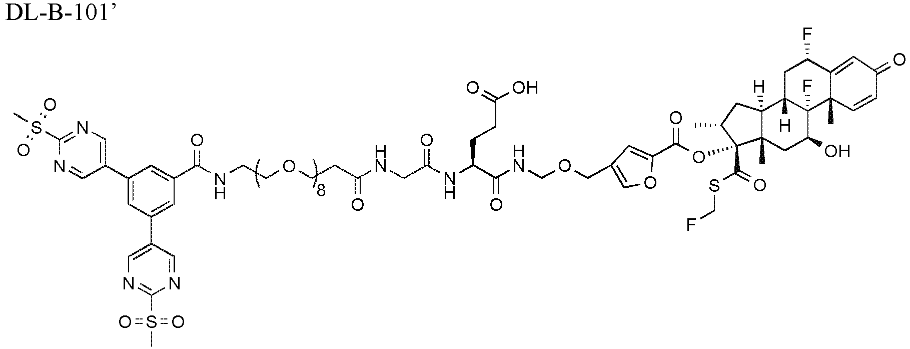 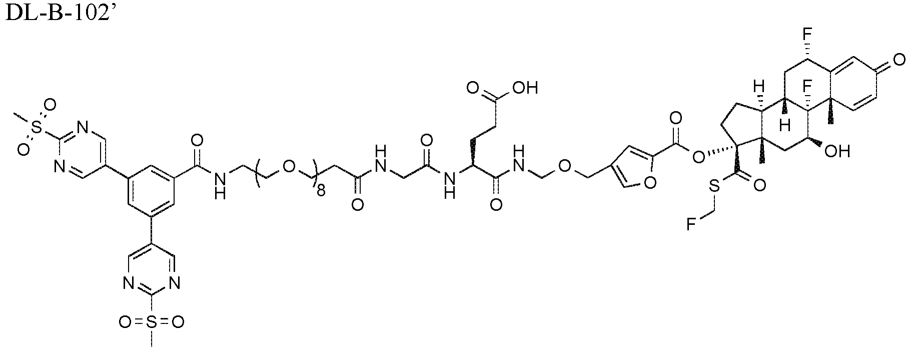 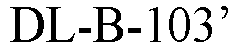 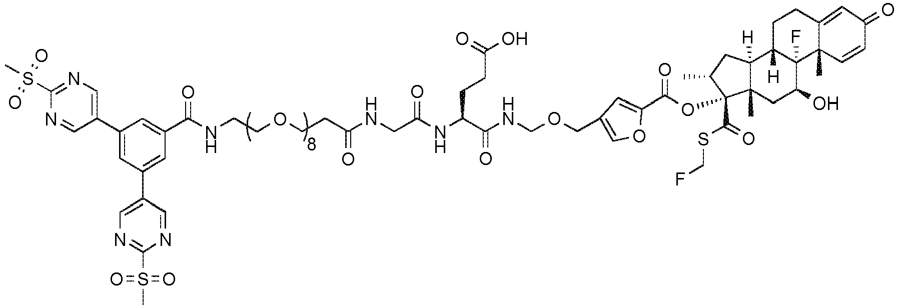 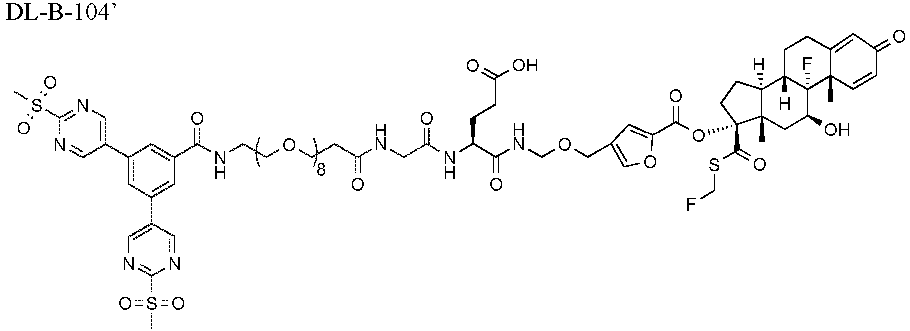 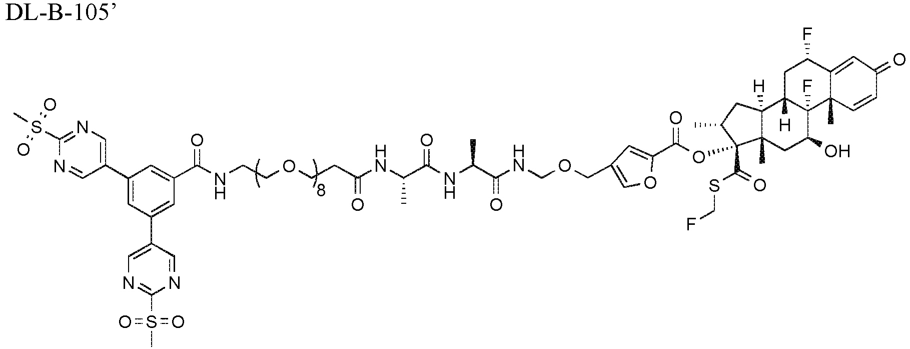 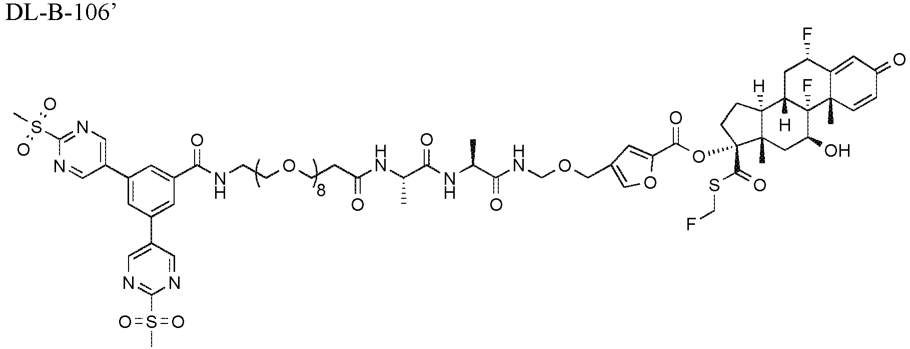 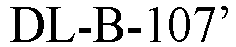 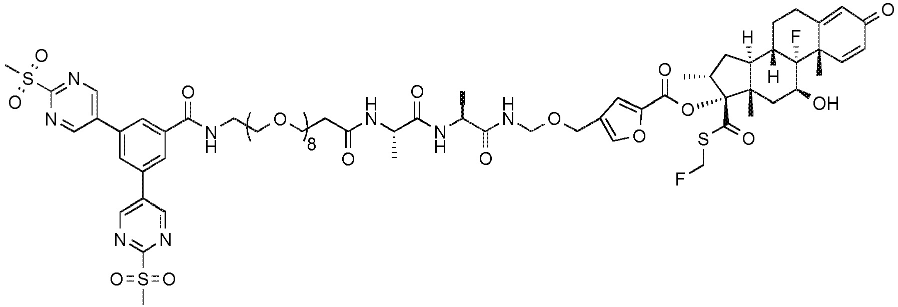 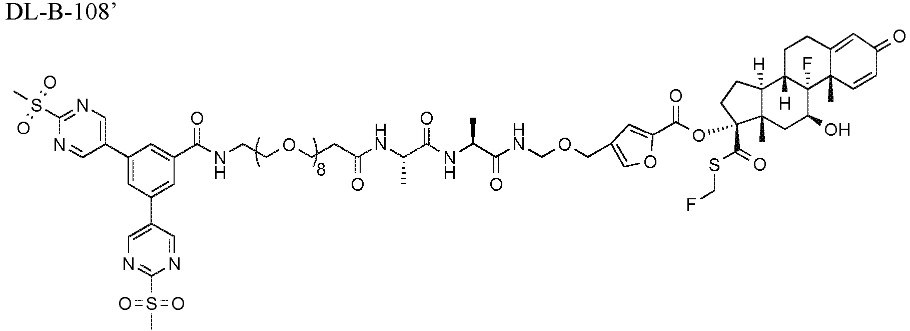 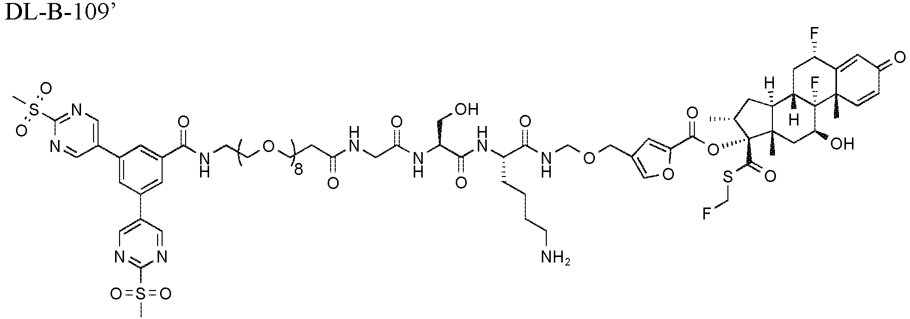 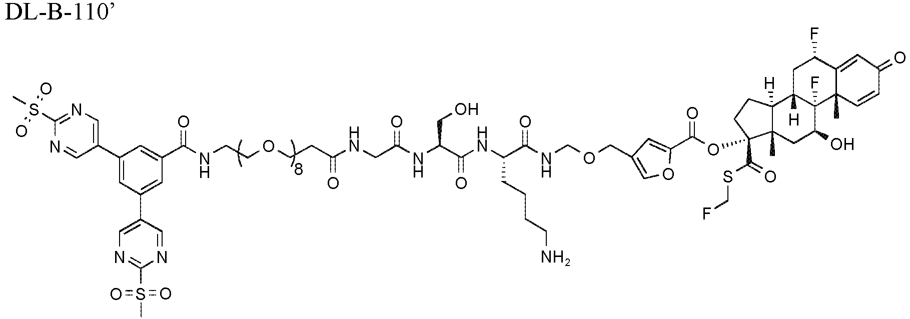 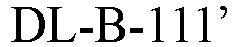 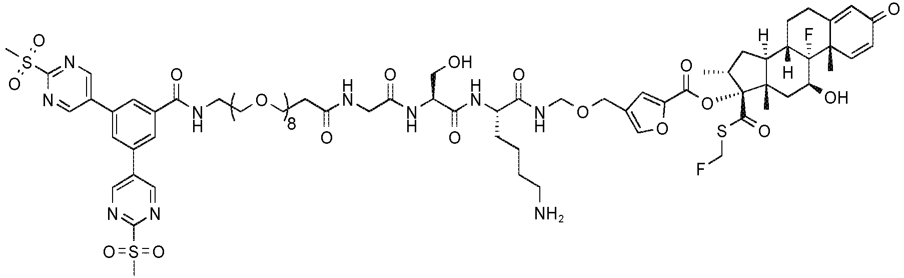 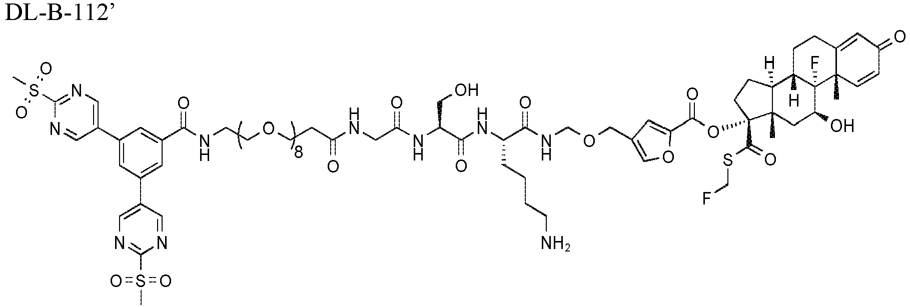 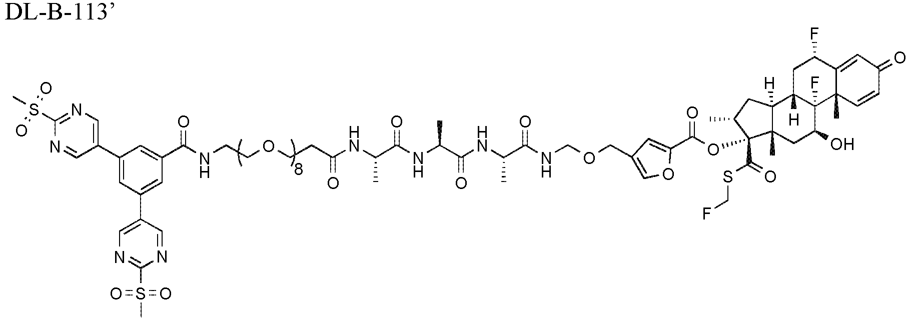 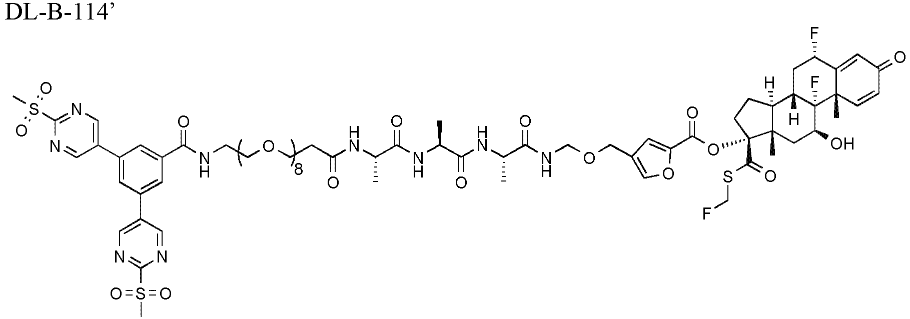 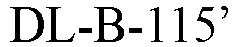 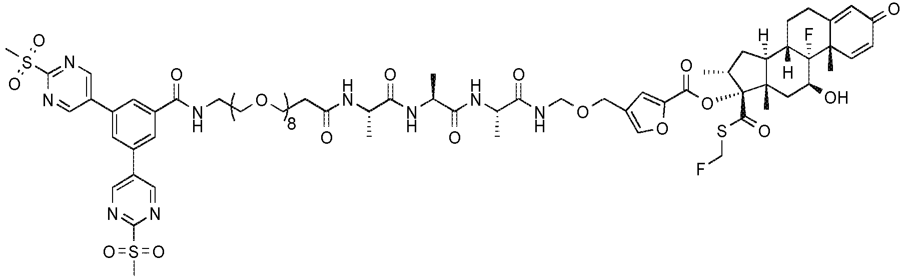 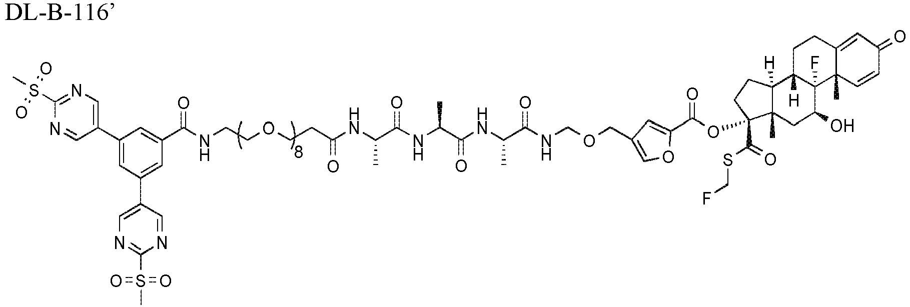 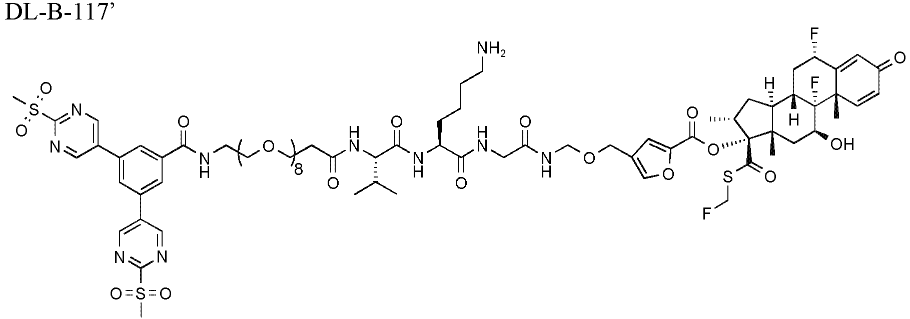 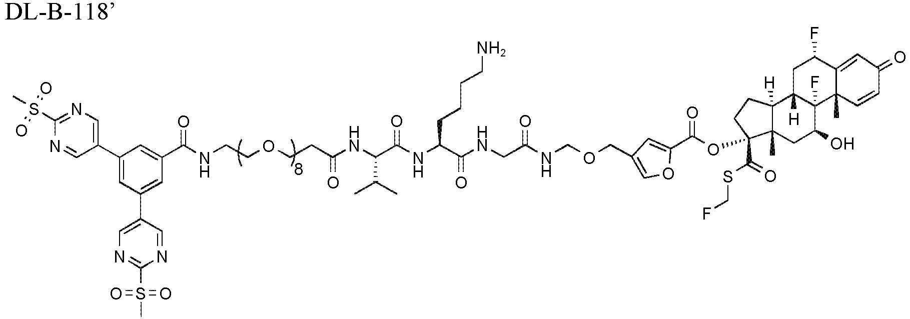 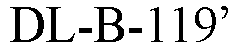 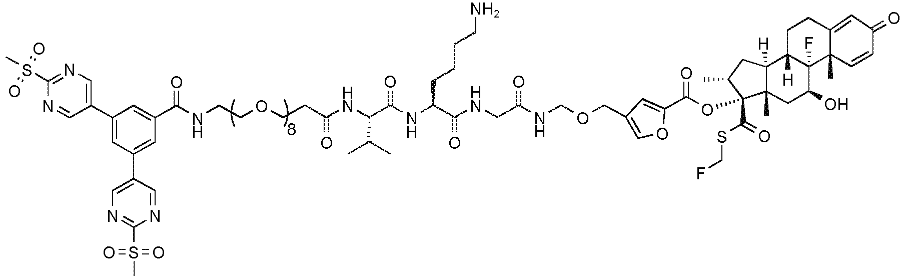 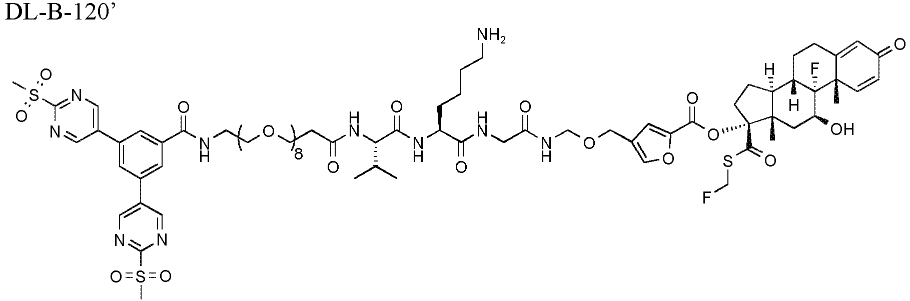 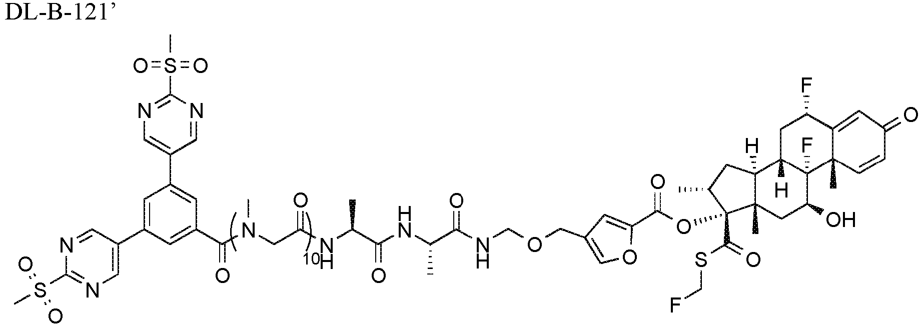 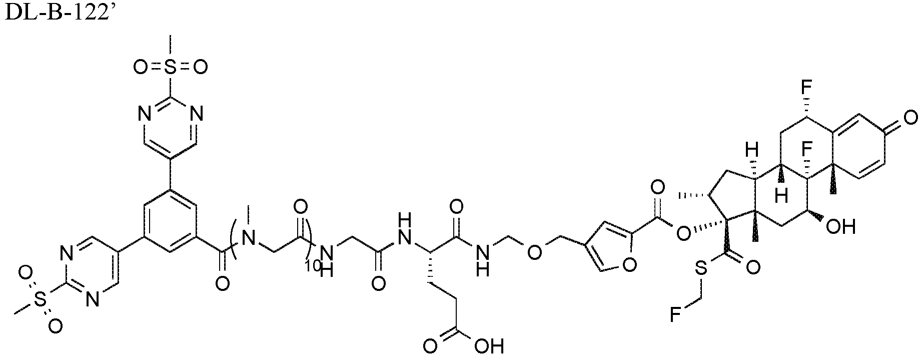 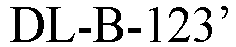 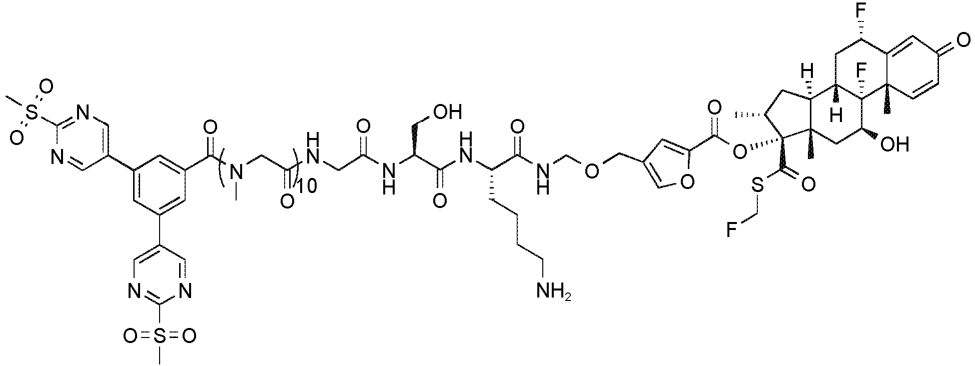 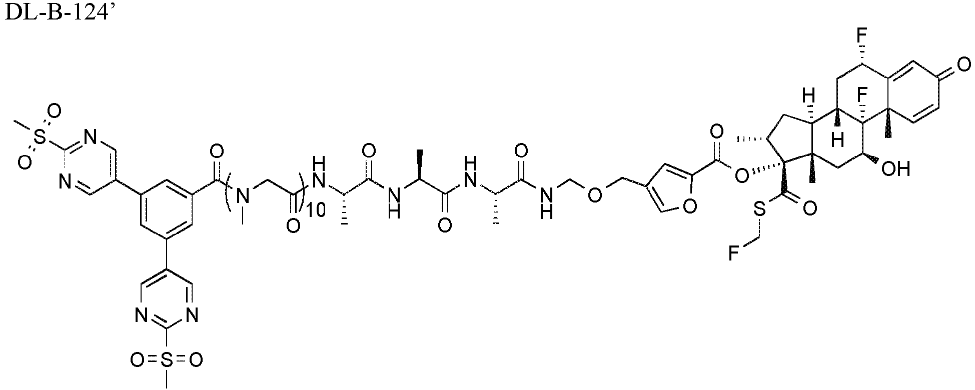 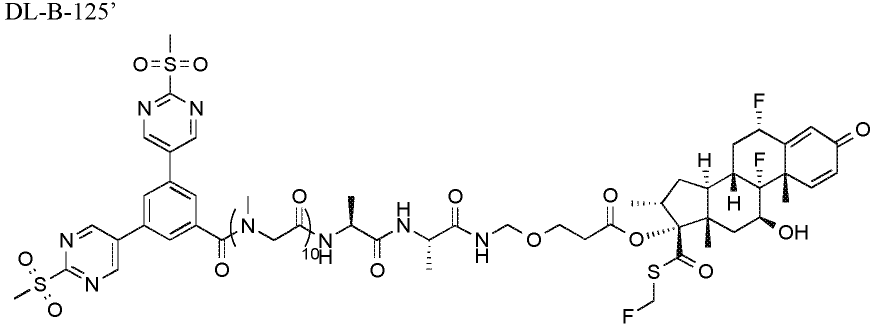 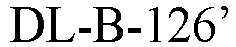 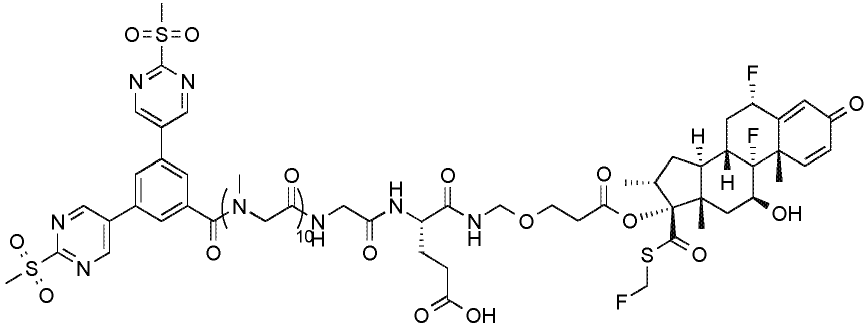 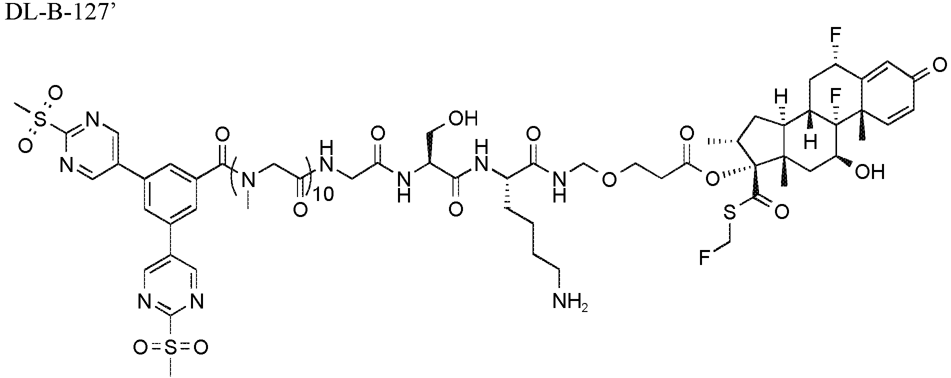 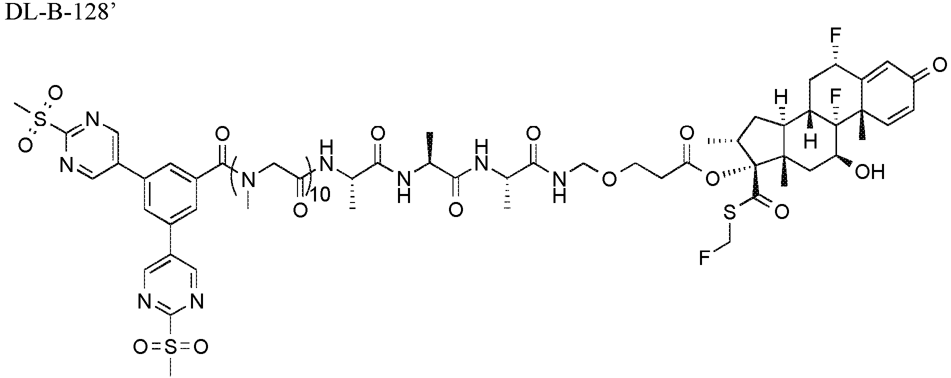 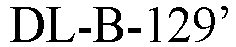 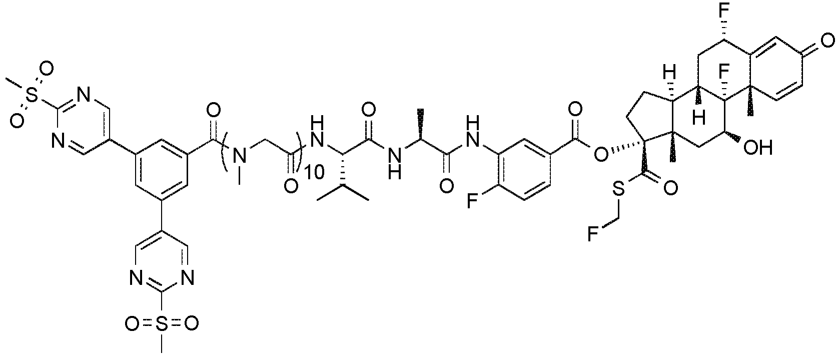 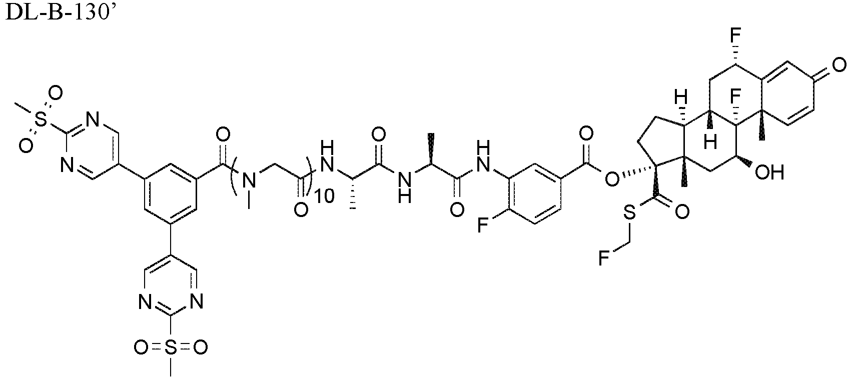 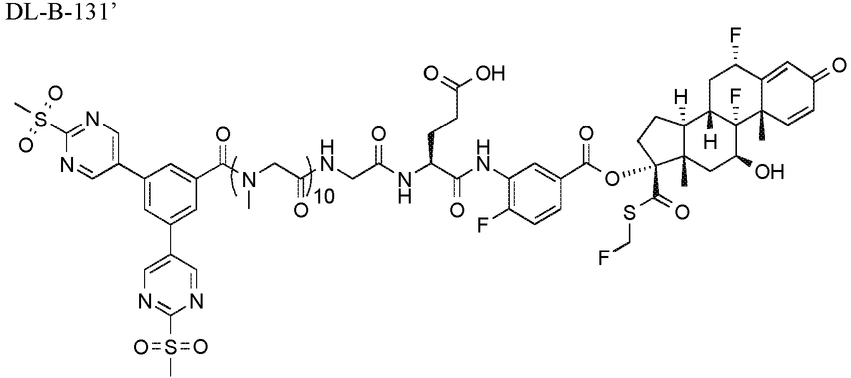 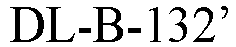 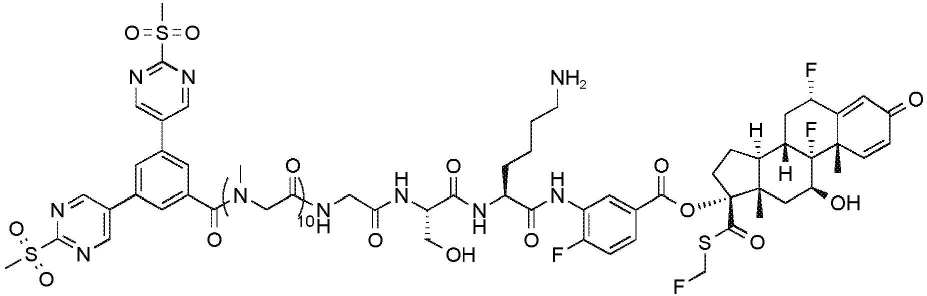 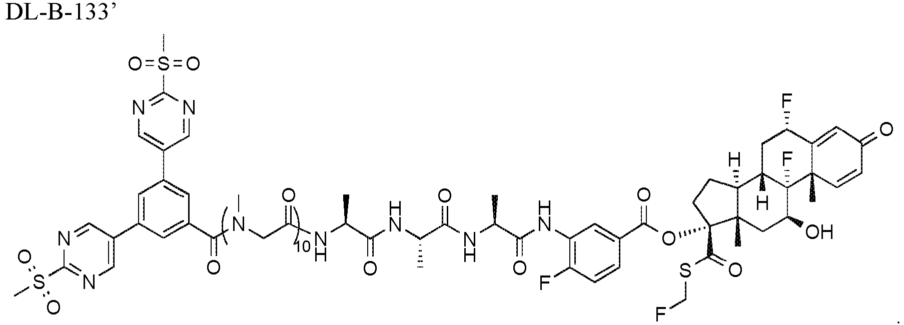 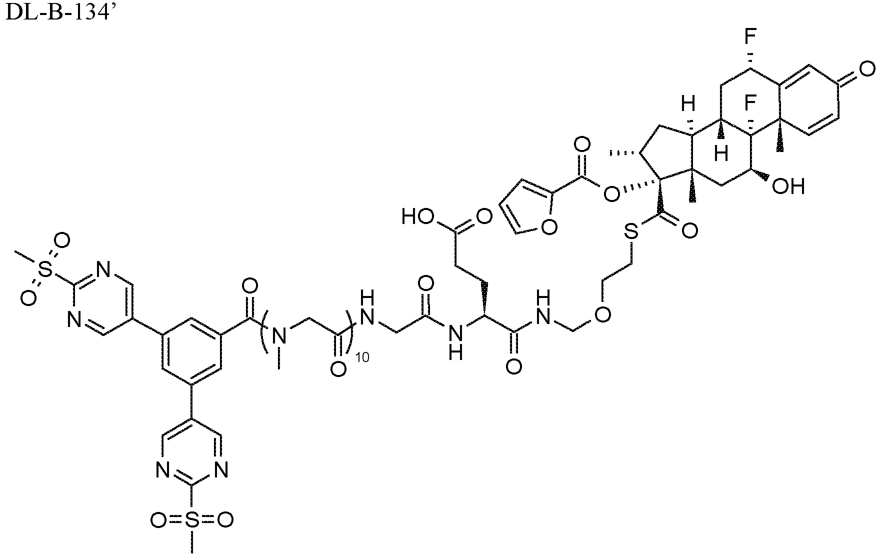 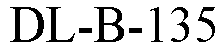 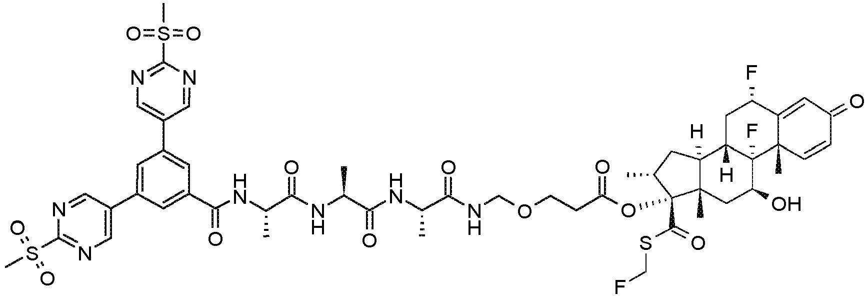 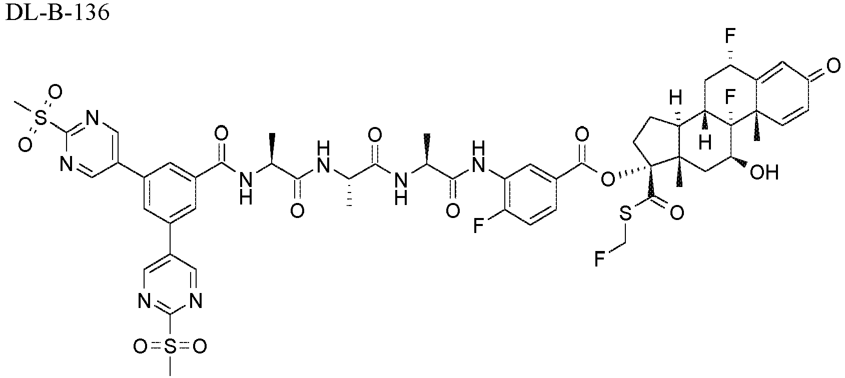 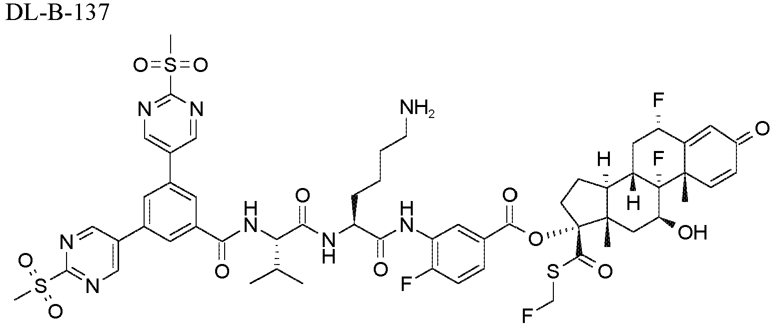 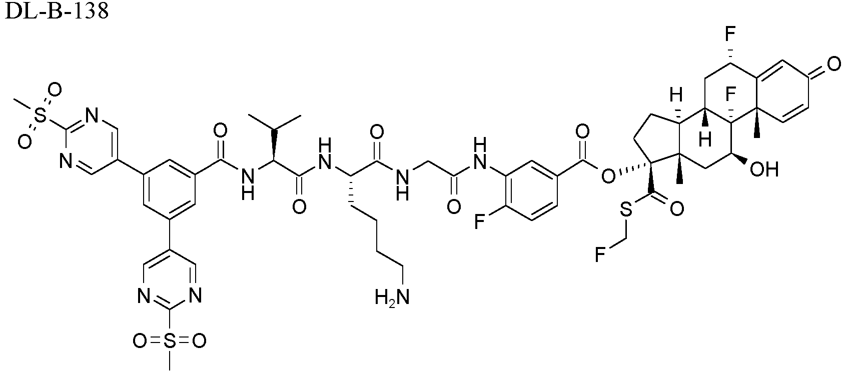 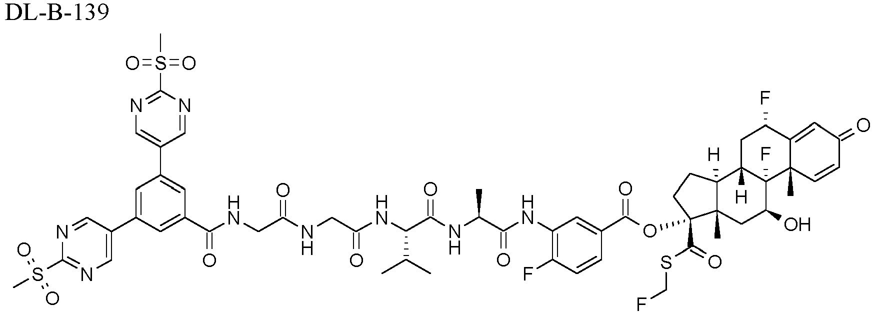 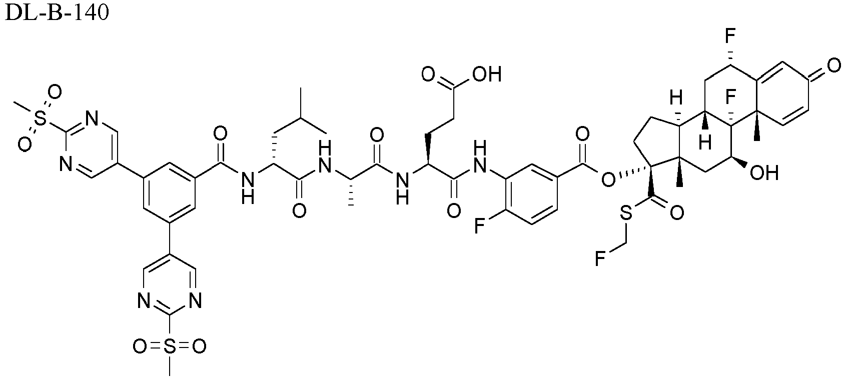 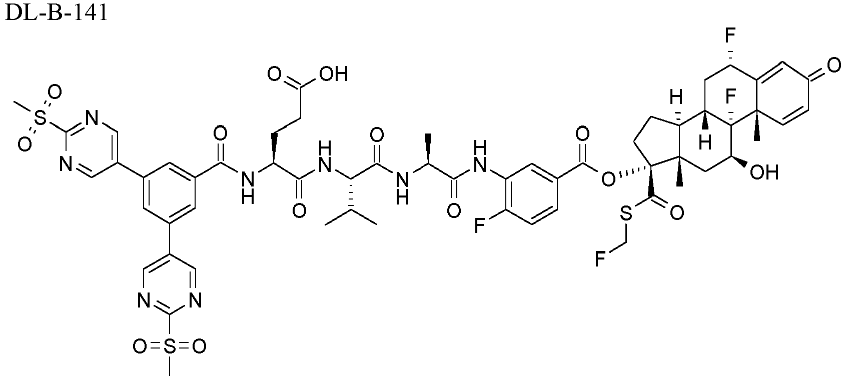 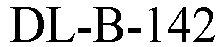 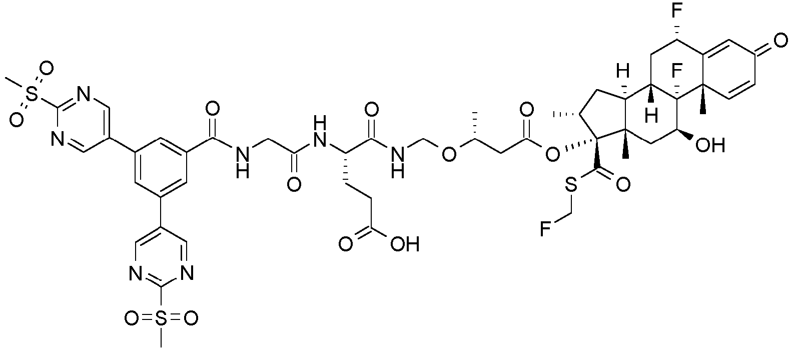 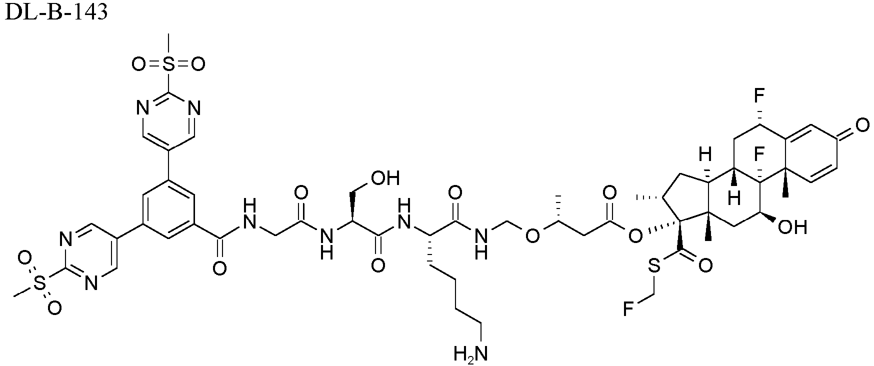 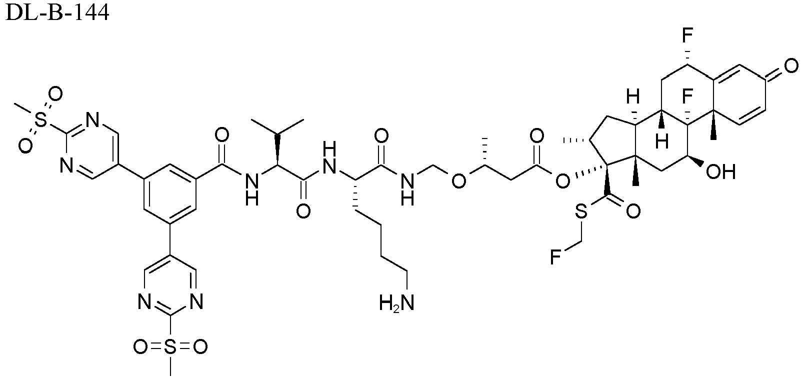 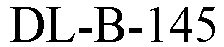 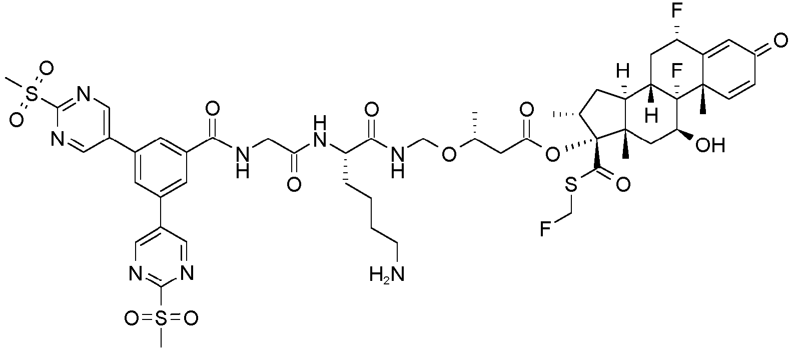 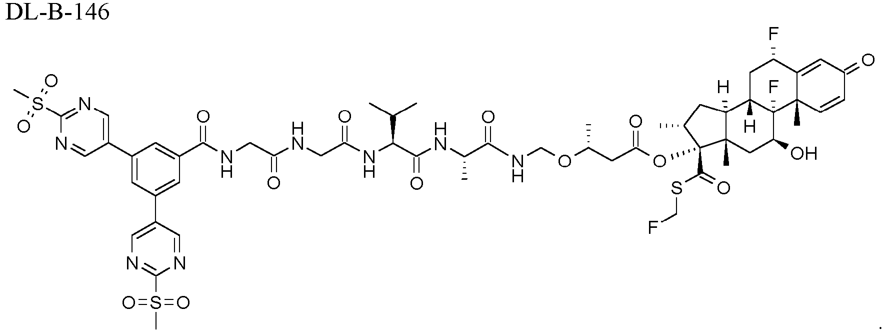 wherein,
s is selected from a group consisting of integers ranging from 1 to 20, for example 1, 2, 3, 4, 5, 6, 7, 8, 9, 10, 11, 12, 13, 14, 15, 16, 17, 18, 19, or 20; and
n is selected from a group consisting of integers ranging from 0 to 20, for example 0, 1, 2, 3, 4, 5, 6, 7, 8, 9, 10, 11, 12, 13, 14, 15, 16, 17, 18, 19, or 20.

### Pharmaceutical composition

In another aspect, the present application provides a pharmaceutical composition, comprising the glucocorticoid receptor agonist of the present invention, the antibody-drug conjugate of the present invention or the drug-linker compound of the present invention, and one or more pharmaceutically acceptable carriers.

The selection of the pharmaceutically acceptable carriers depends on the dosage form of the pharmaceutical composition, which first depends on the administration route of the dosage form, and second depends on the formulation of the dosage form. For example, the pharmaceutically acceptable carrier can comprise water (such as water for injection), a buffer solution, an isotonic salt solution such as PBS (phosphate buffered saline), glucose, mannitol, dextrose, lactose, starch, magnesium stearate, cellulose, magnesium carbonate, 0.3% glycerol, hyaluronic acid, ascorbic acid, lactic acid, ethanol, polyalkylene glycol such as polyethylene glycol (e.g., polyethylene glycol 4000), polypropylene glycol, triglycerides, etc.

The glucocorticoid receptor agonist, antibody-drug conjugate or drug-linker compound described herein is usually formulated as a unit injectable form for parenteral use, for example subcutaneous injection, intramuscular injection, intravenous injection, intravenous drip, local injection to diseased tissues, intratumoral injection, etc., together with pharmaceutically acceptable parenteral vehicles. Optionally, the glucocorticoid receptor agonist, antibody-drug conjugate or drug-linker compound having desired purity is mixed with a pharmaceutically acceptable dilute, carrier, excipient or stabilizer in the form of a lyophilized preparation or a solution (Remington's Pharmaceutical Sciences (1980) 16th edition, Osol, A. Ed.). The glucocorticoid receptor agonist, antibody-drug conjugate or drug-linker compound described herein or the pharmaceutical composition described herein can be administrated via any route suitable for a subject to be treated.

### Treatment method and use

In another aspect, the present application provides use of the steroid glucocorticoid receptor agonist of the present invention, the antibody-drug conjugate of the present invention, the drug-linker compound of the present invention or the pharmaceutical composition of the present invention in the manufacture of a medicament for treating an inflammatory or immune disease.

In another aspect, the present application provides the steroid glucocorticoid receptor agonist of the present invention, the antibody-drug conjugate of the present invention, the drug-linker compound of the present invention or the pharmaceutical composition of the present invention, for use in the treatment of an inflammatory or immune disease.

In another aspect, the present application provides a method for treating an inflammatory or immune disease, comprising administering a therapeutically effective amount of the steroid glucocorticoid receptor agonist of the present invention, the antibody-drug conjugate of the present invention, the drug-linker compound of the present invention or the pharmaceutical composition of the present invention to a subject in need thereof.

In one embodiment, the inflammatory or immune disease is a TNFα or IL-6R overexpression disease, including but not limited to rheumatoid arthritis, idiopathic arthritis, asthma, ulcerative colitis, optic neuromyelitis and autoimmune liver diseases.

### Definitions

Unless otherwise specified hereinafter, the meanings of all the technical and scientific terms used herein are intended to be the same as those commonly understood by a person skilled in the art. The techniques used herein are intended to refer to the techniques commonly understood in the art, including changes or equivalent replacements that are obvious to those skilled in the art. Furthermore, the laboratory procedures used herein, such as genomics, nucleic acid chemistry, and molecular biology, are all routine procedures widely used in their respective fields. Although it is believed that the following terms are easy for those skilled in the art to understand, the following definitions are illustrated to better explain the present invention.

The term "antibody" refers to an immunoglobulin molecule consisting of two pairs of polypeptide chains (each pair has a light chain (LC) and a heavy chain (HC)). The light chain of an antibody can be classified into κ (kappa) and λ (lambda) light chains. The heavy chain can be classified into µ, δ, γ, α or ε, and the isoptypes of an antibody are defined as IgM, IgD, IgG, IgA and IgE, respectively. In the light chain and heavy chain, the variable regions and constant regions are linked through the "J" regions of about 12 or more amino acids, and the heavy chain also comprises the "D" regions of about 3 or more amino acids. Each heavy chain is composed of a heavy chain variable region (VH) and a heavy chain constant region (CH). The heavy chain constant region is composed of 3 domains (CH1, CH2 and CH3). Each light chain is composed of a light chain variable region (VL) and a light chain constant region (CL). The light chain constant region is composed of one domain CL. A constant domain does not directly participates in the binding of the antibody to the antigen, but exhibits multiple effector functions, such as mediation of immunoglobulins and host tissues or factors, including the binding of various cells (for example effector cells) of an immune system and the first component (C1q) of a classical complement system. The VH and VL regions can also be subdivided into regions with high variability (referred to as complementary determining regions (CDR)), in which conservative regions, referred to as framework regions (FR), are dispersed. Each of VH and VL is composed of 3 CDRs and 4 FRs which are arranged from an amino terminus to a carboxyl terminus in an order of FR1, CDR1, FR2, CDR2, FR3, CDR3 and FR4. The variable region of each heavy chain/light chain pair respectively forms an antigen binding site. The distribution of amino acid in each region or domain follows each numbering system known in the art.

The term "complementary determining region" or "CDR" refers to an amino acid residue which is in charge of antigen binding in a variable region of an antibody. The variable regions of the heavy chain and the light chain each contain 3 CDRs which are named CDR1, CDR2 and CDR3. The precise boundaries of these CDRs can be defined according to the numbering system known in the art, for example, they can be defined according to the Kabat numbering system (Kabat et al., Sequences of Proteins of Immunological Interest, 5th Ed. Public Health Service, National Institutes of Health, Bethesda, Md., 1991), the Chothia numbering system (Chothia & Lesk (1987) J. Mol. Biol. 196:901-917; Chothia et al., (1989) Nature 342:878-883), the IMGT numbering system (Lefranc et al., Dev. Comparat. Immunol. 27:55-77, 2003) or the AbM numbering system (Martin ACR, Cheetham JC, Rees AR (1989) Modelling antibody hypervariable loops: A combined algorithm. Proc Natl Acad Sci USA 86:9268-9272). For a given antibody, those skilled in the art will easily identify the CDR defined according to each numbering system. Furthermore, the corresponding relationship between different numbering systems is known by those skilled in the art (for example, refer to Lefranc et al., Dev. Comparat. Immunol. 27:55-77, 2003).

In the present invention, the CDR contained in the antibody or the antigen binding fragment thereof can be determined according to various numbering system known in the art, for example, it can be determined according to the Kabat, Chothia, IMGT or AbM numbering system. In some embodiments, the CDR contained in the antibody or the antigen binding fragment thereof is defined according to the Chothia numbering system.

The term "framework region" or "FR" residue refers to those amino acid residues in the variable region of an antibody except the above defined CDR residues.

The term "antigen binding fragment" of an antibody refers to a polypeptide of a fragment of the antibody, for example a polypeitide of a fragment of a full-length antibody, which retains the capability of specifically binding to the same antigen to which the full-length antibody binds, and/or competes with the full-length antibody for specific binding to the antigen, and is referred to as an "antigen binding portion". Reference is generally made to Fundamental Immunology, Ch. 7 (Paul, W., ed., 2nd edition), Raven Press, N.Y. (1989), the disclosure of which is incorporated by reference herein in its entirety for all the purposes. The antigen binding fragment of an antibody can be produced by the recombinant DNA technology or by enzymatic or chemical cleavage of an intact antibody. The nonlimiting examples of antigen binding fragments include Fab fragments, Fab' fragments, F(ab)'₂ fragments, F(ab)'₃ fragments, Fd, Fv, scFv, d-scFv, (scFv)₂, disulfide bond stabilized Fv proteins ("dsFv"), single domain antibodies (sdAb, nanobody) and polypeptides that comprise at least a portion of an antibody sufficient to confer specific antigen binding capability to the polypeptides. A review of engineered antibody variants is summarized in Holliger et al, 2005; Nat Biotechnol, 23: 1126-1136.

The term "Fd" means an antibody fragment consisting of VH and CH1 domains; the term "dAb fragment" means an antibody fragment consisting of VH domains (Ward et al, Nature 341:544 546 (1989)); the term "Fab fragment" means an antibody fragment consisting of VL, VH, CL and CH1 domains; the term "F(ab')₂ fragment" means an antibody fragment comprising two Fab fragments linked by a disulfide bridge on a hinge region; the term "Fab' fragment" means a fragment obtained after reducing the disulfide bond that links the two heavy chain fragments in an F(ab')₂ fragment, which consists of an intact light chain and the Fd fragment of a heavy chain (composed of VH and CH1 domains).

The term "Fv" means an antibody fragment consisting of VL and VH domains of a single arm of an antibody. The Fv fragment is generally considered as a minimum antibody fragment capable of forming an intact antigen binding site. It is generally recognized that six CDRs endow the antigen binding specificity of an antibody. However, even one variable region (such as the Fd fragment, which only contains three CDRs specific to the antigen) can recognize and bind to the antigen, although its affinity may be lower than that of the intact binding site.

The term "Fc" means an antibody fragment formed by disulfide bond linkage of the second and third constant regions of a first heavy chain of an antibody and the second and third constant regions of a second heavy chain of the antibody. The Fc fragment of an antibody has various different functions, but does not participate in antigen binding.

The term "scFv" refers to a single polypeptide chain comprising VL and VH domains, wherein the VL and VH are linked via a linker (see, for example, Bird et al, Science 242:423-426 (1988); Huston et al, Proc. Natl. Acad. Sci. USA 85:5879-5883 (1988); and Pluckthun, The Pharmacology of Monoclonal Antibodies, Volume 113, edited by Roseburg and Moore, Springer-Verlag, New York, page 269-315 (1994)). Such an scFv molecule can have a general structure of NH₂-VL-linker-VH-COOH or NH₂-VH-linker-VL-COOH. An appropriate existing linker consists of repeated GGGGS amino acid sequences or variants thereof. For example, a linker having an amino acid sequence (GGGGS)₄ can be used, but its variant can also be used (Holliger et al (1993), Proc. Natl. Acad. Sci. USA 90: 6444-6448). Other linkers that can be used in the present invention are described by Alfthan et al (1995), Protein Eng. 8:725-731, Choi et al (2001), Eur. J. Immunol. 31: 94-106, Hu et al (1996), Cancer Res. 56:3055-3061, Kipriyanov et al (1999), J. Mol. Biol. 293:41-56 and Roovers et al (2001), Cancer Immunol. In some instances, there may also be a disulfide bond between VH and VL in scFv. In some embodiments, VH and VL domains can be positioned relative to each other in any suitable arrangement, for example, scFv comprising NH₂-VH-VH-COOH and NH₂₋VL-VL-COOH.

The term "single-domain antibody (sdAb) has a meaning generally understood by those skilled in the art, which refers to an antibody fragment consisting of a single monomer variable antibody domain (such as a single heavy chain variable region), and retains the capability of specifically binding to the same antigen to which the full-length antibody binds (Holt, L. et al, Trends in Biotechnology, 21(11):484-490, 2003). The single-domain antibody is also referred to as a nanobody.

The above antibody fragments all retain the capability of specifically binding to the same antigen to which the full-length antibody binds, and/or compete with the full-length antibody for specific binding to the antigen.

Herein, unless explicitly specified in the context, when referring to the term "antibody", it includes not only the intact antibody, but also the antigen binding fragment of the antibody.

The antigen binding fragment of an antibody (for example, the above antibody fragments) can be obtained from the given antibody (for example, the antibodies provided in the present invention) through a conventional technology known by those skilled in the art (for example, a recombinant DNA technology or an enzymatic or chemical cleavage method), and the antigen binding fragment of the antibody is screened in a manner the same as that for an intact antibody in terms of specificity.

The term "murine-derived antibody" refers to an antibody obtained by the following methods: fusing B cells of immunized mice with myeloma cells, screening mouse hybrid fusion cells that can both achieve infinite proliferation and secrete antibodies, followed by screening, antibody preparation and antibody purification; or refers to an antibody secreted by plasma cells formed by the differentiation and proliferation of B cells after the invasion of antigens into a mouse.

The term "humanized antibody" refers to an genetically engineered non-human antibody, the amino acid sequence of which is modified to improve the homology to the sequence of a human antibody. Generally speaking, all or a part of the CDR regions of a humanized antibody are derived from a non-human antibody (donor antibody), all or a part of non-CDR regions (for example, the variable region FR and/or constant region) are derived from human immunoglobulin (receptor antibody). A humanized antibody generally retains the expected properties of the donor antibody, including, but not limited to, antigen specificity, affinity, reactivity, the capability of enhancing immune cell activity, the capability of strengthening immune response, etc. The donor antibody can be an antibody of mice, rats, rabbits, or non-human primates (for example, cynomolgus monkeys) having expected properties (for example, antigen specificity, affinity, reactivity, the capability of enhancing immune cell activity and/or the capability of strengthening immune response).

The term "identity" refers to the matching of sequences between two polypeptides or two nucleic acids. When a certain position in two compared sequences is both occupied by a same base or amino acid monomer subunit (for example, a certain position of each of two DNA molecules is both occupied by adenine, or a certain position of each of two polypeptides is both occupied by lysine), each molecule is identical at this position. The "percentage identity" between two sequences refers to a function calculated by dividing the number of matched positions shared by the two sequences by the total number of positions compared, and then multiplying by 100. For example, if 6 out of 10 positions in two sequences are matched, the two sequences have 60% identity. For example, DNA sequences CTGACT and CAGGTT have 50% identity (3 out of 6 positions are matched). Generally, comparison is made when two sequences is aligned to produce maximum identity. Such an alignment can be achieved by, for example, using a method that can be conveniently conducted using a computer program such as Align program (DNAstar, Inc.) (Needleman et al, (1970) J. Mol. Biol. 48: 443-453). The percentage identity between two amino acid sequences can also be determined using the algorithm of E. Meyers and W. Miller (Comput. Appl. Biosci., 4:11-17 (1988)) which has been integrated into the ALIGN program (version 2.0), using the PAM120 weight residue table, with a penalty score for gap length of 12 and a penalty score for gap length of 4. In addition, the percentage identity between two amino acid sequences can be determined using the algorithm of Needleman and Wunsch (J MoI Biol. 48:444-453 (1970)) which has been integrated into the GAP program of the GCC software package (available at www.gcg.com), using the Blossum 62 matrix or the PAM250 matrix, with a gap weight of 16, 14, 12, 10, 8, 6 or 4 and a length weight of 1, 2, 3, 4, 5 or 6.

The term "conservative replacement" means an amino acid replacement that does not adversely affect or change the expected properties of a protein/polypeptide comprising an amino acid sequence. For example, conservative replacement can be introduced through standard techniques known in the art such as site-directed mutagenesis and PCR-mediated mutagenesis. The conservative amino acid replacement includes replacements of an amino acid residue with an amino acid residue having a similar side chain, for example, replacements using a residue that is physically or functionally similar (for example, having similar size, shape, charge, chemical properties, including the capability of forming a covalent bond or a hydrogen bond, and the like) to a corresponding amino acid residue. Families of amino acid residues having similar side chains have been defined in the art. These families include, but are not limited to, amino acids having basic side chains (e.g., lysine, arginine, and histidine), acidic side chains (e.g., aspartic acid and glutamic acid), uncharged polar side chains (e.g., glycine, asparagine, glutamine, serine, threonine, tyrosine, cysteine, and tryptophan), non-polar side chains (e.g., alanine, valine, leucine, isoleucine, proline, phenylalanine, and methionine), β branched side chains (e.g., threonine, valine, and isoleucine), and aromatic side chains (e.g., tyrosine, phenylalanine, tryptophan, and histidine). Therefore, it is preferable to replace a corresponding amino acid residue with another amino acid residue from the same side chain family. Methods for identifying conservative amino acid replacement are well-known in the field (see, for example, Brummell et al, Biochem. 32:1180-1187 (1993); Kobayashi et al, Protein Eng. 12(10):879-884 (1999); and Burks et al, Proc. Natl Acad. Set USA 94:412-417 (1997), the disclosure of which is incorporated herein by reference).

The twenty conventional amino acids involved herein are designated according to conventional usage. See, for example, Immunology-A Synthesis (2nd Edition, E. S. Golub and D. R. Gren, Eds., Sinauer Associates, Sunderland, Mass. (1991)), the disclosure of which is incorporated herein by reference. In the present invention, amino acids are generally represented by their well-known one-letter and three-letter abbreviations in the art. For example, alanine can be represented by A or Ala.

The terms "include", "comprise", "have", "contain" or "involve" and other variant forms thereof herein are inclusive or in an open manner, and other elements or method steps that are not listed are not excluded.

The term "alkyl" represents a group obtained by removing 1 hydrogen atom from a straight or branched alkane, for example, "C₁₋₂₀ alkyl", "C₁₋₁₀ alkyl", "C₁₋₆ alkyl", "C₁₋₄ alkaly", "C₁₋₃ alkaly", etc. Specific examples include, but are not limited to, methyl, ethyl, n-propyl, isopropyl, n-butyl, isobutyl, sec-butyl, tert-butyl, n-pentyl, isopentyl, 2-methylbutyl, neopentyl, 1-ethylpropyl, n-hexyl, isohexyl, 3-methylpentyl, 2-methylpentyl, 1-methylpentyl, 3,3-dimethylbutyl, 2,2-dimethylbutyl, 2,2-dimethylbutyl, 1,2-dimethylbutyl, 1,3-dimethylbutyl, 2,3-dimethylbutyl, 2-ethylbutyl and 1,2-dimethylpropyl.

The term "alkenyl" represents a straight or branched aliphatic hydrocarbon group having more than one unsaturated double bond, for example, C₂₋₂₀ alkenyl, C₂₋₁₀ alkenyl, C₂₋₆ alkenyl, C₂₋₄ alkenyl, etc. Specific examples include, but are not limited to, vinyl, 1-propenyl, 2-propenyl, 2-methyl-1-propenyl, 2-methyl-2-propenyl, 2-buten-1-yl, 3-buten-1-yl, 2-penten-1-yl, 3-penten-1-yl, 4-penten-1-yl, 5-hexen-1-yl, 4-hexen-1-yl, 3-hexen-1-yl, 2-hexen-1-yl, 3-methyl-2-buten-1-yl, 3-methyl-3-penten-1-yl, 3-methyl-2-penten-1-yl, 4-methyl-3-penten-1-yl, 4-methyl-2-penten-1-yl, 2-methyl-2-penten-1-yl, etc.

The term "alkynyl" refers to a straight or branched aliphatic hydrocarbon group having more than one unsaturated triple bond, for example, "C₂₋₂₀ alkynyl", "C₂₋₁₀ alkynyl", "C₂₋₆ alkynyl", "C₂₋₄ alkynyl", etc. Specific examples include, but are not limited to, ethynyl, 1-propyn-1-yl, 2-propyn-1-yl, 2-butyn-1-yl, 3-butyn-1-yl, 2-pentyn-1-yl, 3-pentyn-1-yl, 4-pentyn-1-yl, 5-hexyn-1-yl, 4-hexyn-1-yl, 3-hexyn-1-yl, 2-hexyn-1-yl, etc.

The term "alkylene" represens a group obtained by removing 2 hydrogen atoms from a straight or branched alkane, for example, "C₁₋₂₀ alkylene", "C₁₋₁₀ alkylene", "C₃₋₁₀ alkylene", "C₅₋₈ alkylene", "C₁₋₆ alkylene", "C₁₋₄ alkylene", "C₁₋₃ alkylene", etc. Specific examples include, but are not limited to, methylene, ethylene, 1,3-propylene, 1,4-butylene, 1,5-pentylene or 1,6-hexylene, etc.

The term "alkenylidene" refers to a divalent group obtained by losing two hydrogen atoms from a straight or branched alkene, including, for example, "C₂₋₂₀ alkenylidene", "C₃₋₁₀ alkenylidene", "C₅₋₈ alkenylidene", etc. Examples include, but are not limited to, ethenylidene, 1-propenylidene, 2-propenylidene, 1-butenylidene, 2-butenylidene, 1,3-butadienylidene, 1-pentenylidene, 2-pentenylidene, 3-pentenylidene, 1,3-pentadienylidene, 1,4-pentadienylidene, 1-hexenylidene, 2-hexenylidene, 3-hexenylidene, 1,4-hexadienylidene, etc.

The term "alkynylidene" refers to a bivalent group obtained by losing two hydrogen atoms from a straight or branched alkyne, including, for example, "C₂₋₂₀ alkynylidene", "C₃₋₁₀ alkynylidene", "C₅₋₈ alkynylidene", etc. Examples include, but are not limited to, ethynylidene, 1-propynylidene, 2-propynylidene, 1-butynylidene, 2-butynylidene, 1,3-butadiynylidene, 1-pentynylidene, 2-pentynylidene, 3-pentynylidene, 1,3-pentadiynylidene, 1,4-pentadiynylidene, 1-hexynylidene, 2-hexynylidene, 3-hexynylidene, 1,4-hexadiynylidene, etc.

The term "cycloalkylene" represents a group obtained by removing 2 hydrogen atoms from a cycloalkane, for example, "C₃₋₂₀ cycloalkylene", "C₃₋₁₀ cycloalkylene", "C₃₋₆ cycloalkylene", etc. Specific examples include, but are not limited to, cyclopropylene, cyclobutylene, cyclopentylene, cyclohexylene, etc.

The term "alkoxy" means an "alkyl" as defined above, which is attached to a parent molecule moiety through an oxygen atom, for example, C₁-C₆ alkoxy, and C₁-C₃ alkoxy. Specific examples include, but are not limited to, methoxy, ethoxy, propoxy, isopropoxy, butoxy, isobutyloxy, pentoxy, isopentyloxy, hexyloxy, etc.

The term "halo" or "halogen" refers to fluorine (F), chlorine (Cl), bromine (Br) and iodine (I).

The term "haloalkyl" refers to an alkyl substituted with one or more (such as 1-3) identical or different halogen atoms. For example, the term "C₁-C₆ haloalkyl" refers to a haloalkyl having 1-6 carbon atoms, including but not limited to -CH₂F, -CHF₂, -CF₃, - CH₂CF₃, -CF₂CF₃, -CH₂CH₂CF₃, -CH₂Cl, etc.

The term "aromatic ring" refers to an all-carbon monocyclic or polycyclic aromatic hydrocarbon. Common aromatic rings include, but are not limited to, benzene, naphthalene, anthracene, phenanthrene, acenaphthene, azulene, fluorene, indene, pyrene, etc. For example, the term "C₆-C₁₀ aromatic ring" refers to an aromatic ring containing 6-10 carbon atoms, such as benzene or naphthalene.

The term "aliphatic heterocyclic ring" refers to a saturated or partially saturated cyclic structure containing at least one ring member selected from a group consisting of N, O and S, including but not limited to a 5-10 membered aliphatic heterocyclic ring, a 5-6 membered aliphatic heterocyclic ring, etc, for example, a 5-6 membered nitrogen-containing aliphatic heterocyclic ring, a 5-6 membered oxygen-containing aliphatic heterocyclic ring, etc. Specific examples include, but are not limited to, tetrahydrofuran, pyrrolidine, piperidine, tetrahydropyran, etc.

The term "heteroaromatic ring" refers to an aromatic cyclic structure containing at least one ring member selected from a group consisting of N, O and S, including but not limited to a 5-10 membered heteroaromatic ring, a 5-6 membered heteroaromatic ring, etc, for example, a 5-6 membered nitrogen-containing heteroaromatic ring, a 5-6 membered oxygen-containing heteroaromatic ring, etc. Specific examples include, but are not limited to, furan, thiophene, pyrrole, thiazole, isothiazole, thiadiazole, oxazole, isoxazole, oxadiazole, imidazole, pyrazole, 1,2,3-triazole, 1,2,4-triazole, 1,2,3-oxadiazole, 1,2,4-oxadiazole, 1,2,5-oxadiazole, 1,3,4-oxadiazole, pyridine, pyrimidine, pyridazine, pyrazine, 1,2,3-triazine, 1,3,5-triazine, 1,2,4,5-tetrazine, etc.

The term "aromatic ring system" refers to a monocyclic or polycyclic system containing at least one aromatic ring (e.g., a benzene ring) or heteroaromatic ring (e.g., a pyrimidine ring). Two or more aromatic rings and/or heteroaromatic rings can form a fused ring or be linked through a single bond (e.g., dipyrimidyl phenyl). The aromatic ring system can be bivalent or higher (e.g., trivalent or tetravalent), for example, a 5-20 membered aromatic ring system.

If a substituent is described as "optionally substituted with ...", the substituent can be (1) unsubstituted or (2) substituted. If a carbon in the substituent is described as being optionally substituted with one or more substituents in a substituent list, one or more hydrogens (to the extent that any hydrogen that exists) on the carbon alone and/or together can be substituted with an independently selected substituent or unsubstituted. If a nitrogen in the substituent is described as being optionally substituted with one or more substituents in a substituent list, one or more hydrogen (to the extent that any hydrogen that exists) on the nitrogen alone and/or together can each be substituted with an independently selected substituent or unsubstituted.

If a substituent is described as being "independently selected from a group consisting of" a group of groups, each substituent is selected independent from another substituent. Therefore, each substituent can be the same as or different from another (other) substituent (s).

Unless specified otherwise, as used herein, the attachment point of a substituent can be from any suitable site of the substituent.

When the bond of a substituent is depicted as passing through a bond connecting two atoms in a ring, such a substituent can be bonded to any ring-forming atom in the substitutable ring.

The pharmaceutically acceptable salt of the compound of the present invention includes acid addition salts and base addition salts thereof. A suitable acid addition salt is formed by an acid which forms the pharmaceutically acceptable salt. A suitable base addition salt is formed by a base which forms the pharmaceutically acceptable salt. The review of suitable salts are summarized in "Handbook of Pharmaceutical Salts: Properties, Selection, and Use" (Wiley-VCH, 2002) by Stahl and Wermuth. Methods for preparing the pharmaceutically acceptable salt of the compound of the present invention is known by those skilled in the art.

The term "stereisomer" (or referred to as "enantiomer") refers to a stable isomer that has a perpendicular asymmetric plane due to the presence of at least one chiral factor (including the chiral center, chiral axis, chiral plane, etc.), allowing for the rotation of plane polarized light. Since the compound of the present invention has an asymmetric center and other chemical structures that may cause stereoisomerism, the present invention also comprises these stereisomers and mixtures thereof. Since the compound of the present invention (or a pharmaceutically acceptable salt thereof) comprises an asymmetric carbon atom(s), it can be present in a form of a single stereoisomer, a racemate, an enantiomer and a mixture of diastereomers. Generally, these compounds can be prepared in a form of racemates. However, if necessary, such compounds can be prepared or separated to obtain pure stereoisomers, that is, a single enantiomer or a diastereomer, or a mixture enriched with a single stereoisomer (with a purity of ≥98%, ≥95%, ≥93%, ≥90%, ≥88%, ≥85% or ≥80%). As described below, the single stereoisomer of a compound is obtained by synthesis from an optically active starting material containing a desired chiral center, or obtained by preparing a mixture of enantiomers followed by separation or resolution, for example, transforming into a mixture of diastereomers, followed by separation or recrystalization, chromatographic treatment, uing a chiral separation reagent, or directly separating the enantiomer on a chiral HPLC column. Starting compounds having specific stereochemistry can be not only commercially available, but also prepared using the methods described below followed by resolution using methods known in the art. The term "enantiomer" refers to a pair of stereoisomers that are mirror images of each other and cannot be superimposed. The term "diastereoisomer" refers to optical isomers that are not mirror images of each other. The term "racemic mixture" or "racemate" refers to a mixture containing equal amounts of single enantiomers (i.e., an equal molar mixture of two R and S enantiomers). The term "non-racemic mixture" refers to a mixture containing unequal amounts of single enantiomers. Unless specified otherwise, all the stereoisomer forms of the compound of the present invention are included within the scope of the present invention.

The term "tautomer" (or referred to as "tautomeric form" refers to structural isomers having different energies and mutually transformable through low-energy barriers. If tautomerism is possible (such as in a solution), a chemical equilibrium of tautomers can be achieved. For example, proton tautomers (also known as proton transfer tautomers) include (but are not limited to) interconversion through proton transfer, such as ketone-enol isomerization, imine-enamine isomerization, amide-imine alcohol isomerization, etc. Unless specified otherwise, all the tautomer forms of the compound of the present invention are included within the scope of the present invention.

The term "polymorph" (or referred to as "polymorphic form") refers to a solid crystaline form of a compound or a composite. Polymorphs can be detected, classified and identified by known techniques, including (but not limited to) differential scanning calorimetry (DSC), thermogravimetric analysis (TGA), X-ray powder diffraction (XRPD), single crystal X-ray diffraction (SCXRD), solid nuclear magnetic resonance (NMR), infrared spectroscopy (IR), Raman spectroscopy, scanning electron microscopy (SEM), etc.

The term "solvate" refers to a substance formed by binding the compound of the present invention (or a pharmaceutically acceptable salt) to at least one solvent molecule through non-covalent intermolecular force.

The term "N-oxide" refers to a compound formed by oxidation of a nitrogen atom in the structure of a tertiary amine or nitrogen-containing (aromatic) heterocyclic compound.

The present invention also includes all pharmaceutically acceptable isotope-labeled compounds that are identical to the compound of the present invention except that one or more atoms are replaced with atoms with the same atomic number as but different atomic mass or mass numbers from those dominant in nature. Examples of isotopes suitable for inclusion in the compound of the present invention include, but are not limited to, isotopes of hydrogen (such as 2H, 3H, deuterium D, tritium T); isotopes of carbon (such as 11C, 13C, and 14C); isotope of chlorine (such as 37Cl); isotopes of fluorine (such as 18F); isotopes of iodine (such as 123I and 125I); isotopes of nitrogen (such as 13N and 15N); isotopes of oxygen (such as 150, 170, and 180); and isotopes of sulfur (such as 35S).

Within the scope of the present invention, also included is metabolites of the compound of the present invention, that is, substances formed in vivo when the compound of the present invention is administrated. Such products can be produced by, for example, oxidation, reduction, hydrolysis, amidation, deamidation, esterification, enzymatic hydrolysis and the like of the administered compound. Therefore, the present invention includes metabolites of the compound of the present invention, and includes compounds produced by a method comprising contacting the compound of the present invention with a mammal for a period sufficient to generate the metabolites thereof.

Within the scope of the present invention, also included is prodrugs of the compound of the present invention, which are some derivatives of the compound of the present invention that have relatively small pharmacological activity or no pharmacological activity on their own, and can be transformed into the compound of the present invention with the desired activity by, for example, hydrolytical cleavage when being administered into or onto the body. Generally, such prodrugs are functional derivatives of the compound, which can be easily transformed into the compound with the desired therapeutic activity in vivo. Other information about the use of prodrugs can be seen in "Pro-drugs as Novel Delivery Systems", volume 14, ACS Symposium Series (T. Higuchi and V. Stella) and "Bioreversible Carriers in Drug Design," Pergamon Press, 1987 (E. B. Roche edition, American Pharmaceutical Association). The prodrugs of the present invention can be prepared by replacing suitable functional groups in the compound of the present invention with some moieties known as "pro-moieties" by those skilled in the art (for example, those described in "Design of Prodrugs", H. Bundgaard (Elsevier, 1985)).

### Preparation of the compound

In another aspect, the present invention provides a method for preparing the compound of formula I.

In some embodiments, the compound of formula I of the present invention can be prepared according to the following scheme: 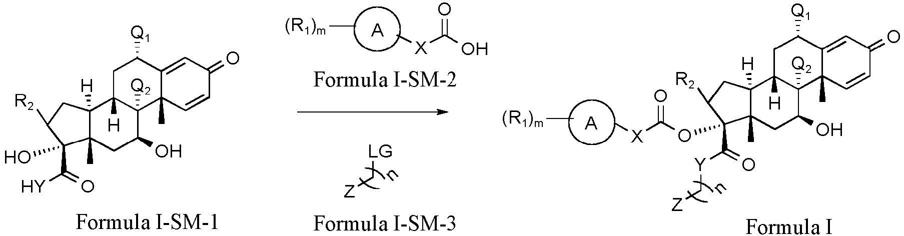
wherein, ring A, R₁, R₂, X, Y, Z, Q₁, Q₂, m and n are as defined above; and
LG is a leaving group, for example, but not limited to, halogen, methylsulfonyloxy (-OMs) or trifluoromethylsulfonyloxy (-OTf), preferably iodine.

The method comprises the following steps: subjecting the compounds of formulae ISM-1 and I-SM-2 to an esterification reaction, followed by a substitution reaction with the compound of formula I-SM-3 to obtain the compound of formula I.

In some embodiments, the above steps are carried out in the presence of a suitable condensation reagent, which can be selected from a group consisting of HATU, HBTU, EDCI, DCC, T3P, POCl₃ and HOBT, preferably HATU.

In some embodiments, the above steps are carried out at a suitable temperature, which is 0-140°C, for example 0°C, 20°C, 25°C, 40°C, 50°C, 60°C, 100°C, or 140°C, preferably 0-35°C.

In some embodiments, the above steps are carried out in a suitable organic solvent, which can be selected from a group consisting of halogenated hydrocarbons (such as dichloromethane (DCM), chloroform (TCM), 1,2-dichloroethane (such as 1,2-DCE) and the like), nitriles (such as acetonitrile (AN)), N-methylpyrrolidone (NMP), N,N-dimethylformamide (DMF), N,N-dimethylacetamide (DMA), tetrahydrofuran (THF), 1,4-dioxane (Dioxane), dimethyl sulfoxide (DMSO), and any combinations thereof, preferably N,N-dimethylformamide (DMF).

In some embodiments, the above steps are carried out in the presence of a suitable base, which comprises organic bases or inorganic bases. The organic base can be selected from a group consisting of N,N-diisopropylethylamine (DIPEA), triethylamine (TEA), potassium tert-butoxide (t-BuOK) and pyridine (Py). The inorganic base can be selected from a group consisting of potassium phosphate (K₃PO₄), sodium hydride (NaH), potassium carbonate (K₂CO₃), sodium carbonate (Na₂CO₃), sodium bicarbonate (NaHCO₃), cesium carbonate (Cs₂CO₃) and NaOH, preferably N,N-diisopropylethylamine (DIPEA).

### Preparation of the drug-linker compound

In another aspect, the present invention provides a method for synthesizing a drug-linker compound of formula G-M-[L-E-D]ₓ. 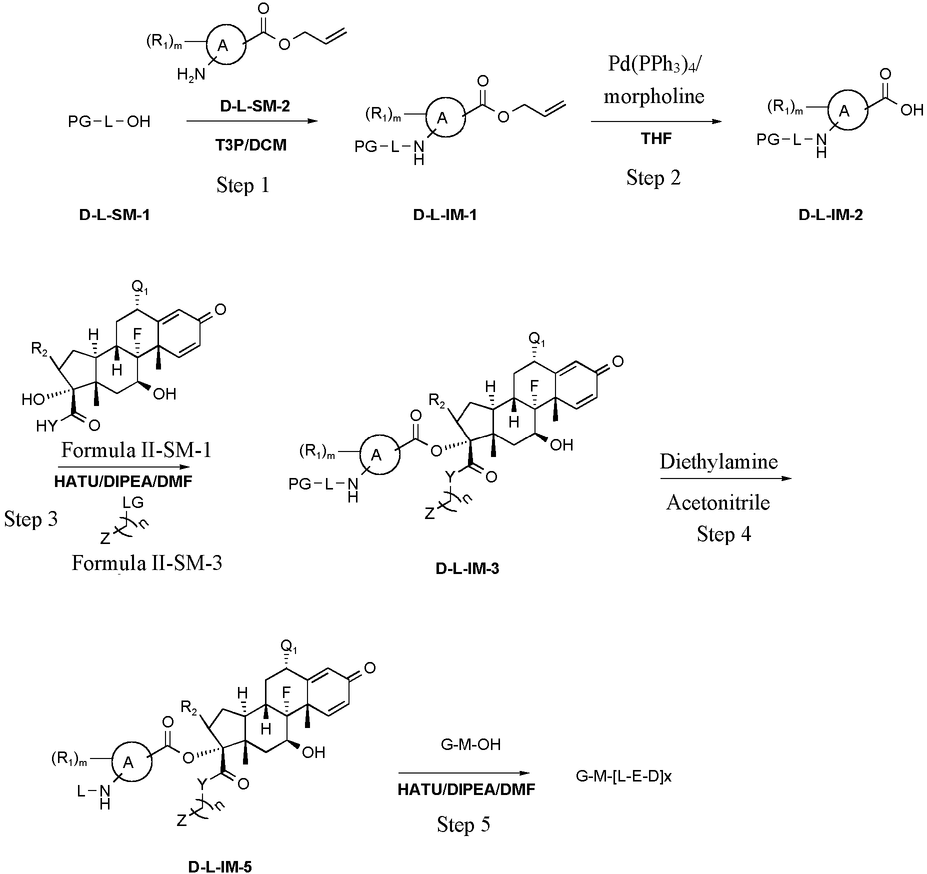 wherein,
ring A, R₁, R₂, X, Y, Z, Q₁, G, M, L, D, m and n are as defined above.
x=1;
E is a single bond;
LG is a leaving group, for example, but not limited to, halogen, -OMs and -OTf, preferably iodine; and
PG is an amino protecting group, for example, but not limited to, Fmoc, Boc, MMt and Alloc, preferably Fmoc.

### Step 1: performing a condensation reaction on the compounds of formulae D-L-SM-1 and D-L-SM-2 to obtain the compound of formula D-L-IM-1.

In some embodiments, the step is carried out in the presence of a suitable condensation reagent, which can be selected from a group consisting of HATU, HBTU, EDCI, DCC, T3P, POCl₃ and HOBT, preferably HATU and T3P.

In some embodiments, the step is carried out at a suitable temperature. The temperature is 0°C, 20°C, 25°C, 40°C, 50°C, 60°C, 100°C, or 140°C, preferably 0-35°C.

In some embodiments, the step is carried out in a suitable organic solvent, which can be selected from a group consisting of halogenated hydrocarbons (such as dichloromethane (DCM), chloroform (TCM), 1,2-dichloroethane (such as 1,2-DCE) and the like), nitriles (such as acetonitrile (AN)), N-methylpyrrolidone (NMP), N,N-dimethylformamide (DMF), N,N-dimethylacetamide (DMA), tetrahydrofuran (THF), 1,4-dioxane (Dioxane), dimethyl sulfoxide (DMSO), and any combinations thereof, preferably dichloromethane (DCM) and N,N-dimethylformamide (DMF).

In some embodiments, the step is carried out in the presence of a suitable base, which comprises organic bases or inorganic bases. The organic base can be selected from a group consisting of N,N-diisopropylethylamine (DIPEA), triethylamine (TEA), potassium tert-butoxide (t-BuOK) and pyridine (Py). The inorganic base can be selected from a group consisting of potassium phosphate (K₃PO₄), sodium hydride (NaH), potassium carbonate (K₂CO₃), sodium carbonate (Na₂CO₃), sodium bicarbonate (NaHCO₃), cesium carbonate (Cs₂CO₃) and NaOH, preferably pyridine (Py) and N,N-diisopropylethylamine (DIPEA).

### Step 2: performing a hydrolysis reaction on the compound of formula D-L-IM-1 to obtain the compound of formula D-L-IM-2

In some embodiments, the step is carried out at a suitable temperature. The temperature is 0°C, 20°C, 25°C, 40°C, 50°C, 60°C, 100°C, or 140°C, preferably 0-35°C.

In some embodiments, the step is carried out in a suitable organic solvent, which can be selected from a group consisting of halogenated hydrocarbons (such as dichloromethane (DCM), chloroform (TCM), 1,2-dichloroethane (such as 1,2-DCE) and the like), nitriles (such as acetonitrile (AN)), N-methylpyrrolidone (NMP), N,N-dimethylformamide (DMF), N,N-dimethylacetamide (DMA), tetrahydrofuran (THF), 1,4-dioxane (Dioxane), dimethyl sulfoxide (DMSO), and any combinations thereof, preferably tetrahydrofuran (THF).

In some embodiments, the step is carried out in the presence of a suitable Pd reagent, which can be selected from a group consisting of Pd(PPh₃)₄ and PdCl₂(PPh₃)₂, preferably Pd(PPh₃)₄.

In some embodiments, the step is carried out in the presence of a suitable base, which can be selected from a group consisting of diethylamine, pyrrolidine, morpholine, piperidine, and piperazine, preferably morpholine.

### Step 3: performing an esterification reaction on the compounds of formula D-L-IM-2 and II-SM-1, followed by a substitution reaction with the compound of formula D-L-IM-3

In some embodiments, the step is carried out in the presence of a suitable condensation reagent, which can be selected from a group consisting of HATU, HBTU, EDCI, DCC, T3P, POCl₃and HOBT, preferably HATU.

In some embodiments, the step is carried out at a suitable temperature. The temperature is 0°C, 20°C, 25°C, 40°C, 50°C, 60°C, 100°C, or 140°C, preferably 0-35°C.

In some embodiments, the step is carried out in a suitable organic solvent, which can be selected from a group consisting of halogenated hydrocarbons (such as dichloromethane (DCM), chloroform (TCM), 1,2-dichloroethane (such as 1,2-DCE) and the like), nitriles (such as acetonitrile (AN)), N-methylpyrrolidone (NMP), N,N-dimethylformamide (DMF), N,N-dimethylacetamide (DMA), tetrahydrofuran (THF), 1,4-dioxane (Dioxane), dimethyl sulfoxide (DMSO), and any combinations thereof, preferably N,N-dimethylformamide (DMF).

In some embodiments, the step is carried out in the presence of a suitable base, which comprises organic bases or inorganic bases. The organic base can be selected from a group consisting of N,N-diisopropylethylamine (DIPEA), triethylamine (TEA), potassium tert-butoxide (t-BuOK) and pyridine (Py). The inorganic base can be selected from a group consisting of potassium phosphate (K₃PO₄), sodium hydride (NaH), potassium carbonate (K₂CO₃), sodium carbonate (Na₂CO₃), sodium bicarbonate (NaHCO₃), cesium carbonate (Cs₂CO₃) and NaOH, preferably N,N-diisopropylethylamine (DIPEA).

### Step 4: removing the amino protecting group from the compound of formula D-L-IM-3 to obtain the compound of formula D-L-IM-4

In some embodiments, the step is carried out at a suitable temperature. The temperature is 0°C, 20°C, 25°C, 40°C, 50°C, 60°C, 100°C, or 140°C, preferably 0-35°C.

In some embodiments, the step is carried out in a suitable organic solvent, which can be selected from a group consisting of halogenated hydrocarbons (such as dichloromethane (DCM), chloroform (TCM), 1,2-dichloroethane (such as 1,2-DCE) and the like), nitriles (such as acetonitrile (AN)), N-methylpyrrolidone (NMP), N,N-dimethylformamide (DMF), N,N-dimethylacetamide (DMA), tetrahydrofuran (THF), 1,4-dioxane (Dioxane), dimethyl sulfoxide (DMSO), and any combinations thereof, preferably acetonitrile (AN).

In some embodiments, the step is carried out in the presence of a suitable base, which can be selected from a group consisting of diethylamine, pyrrolidine, morpholine, piperidine and piperazine, preferably morpholine and diethylamine.

### Step 5: performing a condensation reaction on the compounds of formulae D-L-SM-4 and G-M-OH to obtain the compound of formula G-M-[L-E-D]x

In some embodiments, the step is carried out in the presence of a suitable condensation reagent, which can be selected from a group consisting of HATU, HBTU, EDCI, DCC, T3P, POCl₃ and HOBT, preferably HATU.

In some embodiments, the step is carried out at a suitable temperature. The temperature is 0°C, 20°C, 25°C, 40°C, 50°C, 60°C, 100°C, or 140°C, preferably 0-35°C.

In some embodiments, the step is carried out in a suitable organic solvent, which can be selected from a group consisting of halogenated hydrocarbons (such as dichloromethane (DCM), chloroform (TCM), 1,2-dichloroethane (such as 1,2-DCE) and the like), nitriles (such as acetonitrile (AN)), N-methylpyrrolidone (NMP), N,N-dimethylformamide (DMF), N,N-dimethylacetamide (DMA), tetrahydrofuran (THF), 1,4-dioxane (Dioxane), dimethyl sulfoxide (DMSO), and any combinations thereof, preferably N,N-dimethylformamide (DMF).

In some embodiments, the step is carried out in the presence of a suitable base, which comprises organic bases or inorganic bases. The organic base can be selected from a group consisting of N,N-diisopropylethylamine (DIPEA), triethylamine (TEA), potassium tert-butoxide (t-BuOK) and pyridine (Py). The inorganic base can be selected from a group consisting of potassium phosphate (K₃PO₄), sodium hydride (NaH), potassium carbonate (K₂CO₃), sodium carbonate (Na₂CO₃), sodium bicarbonate (NaHCO₃), cesium carbonate (Cs₂CO₃) and NaOH, preferably pyridine (Py) and N,N-diisopropylethylamine (DIPEA).

### Preparation of the antibody-drug conjugate

In another aspect, the present application provides a method for preparing the antibody-drug conjugate of the present invention, which is selected from a group consisting of:
conjugation method A:
   reducing an antibody interchain disulfide bond with a reducing agent such as tris(2-carboxyethyl) phosphine in a buffer solution of pH 7.0-9.0, and conjugating the drug-linker compound of the present invention with an antibody in a molar ratio of (8-10):1 to obtain the antibody-drug conjugate;
conjugation method B:
   reducing an antibody interchain disulfide bond with a reducing agent such as tris(2-carboxyethyl) phosphine in a buffer solution of pH 7.0-9.0, and conjugating the drug-linker compound of the present invention with an antibody in a molar ratio of (4-6):1 to obtain the antibody-drug conjugate;
conjugation method C:
   reducing an antibody interchain disulfide bond with a reducing agent such as tris(2-carboxyethyl) phosphine in a buffer solution of pH 7.0-9.0, and conjugating the drug-linker compound of the present invention with an antibody in a molar ratio of (4.0-4.5):1 to obtain the antibody-drug conjugate.

In some embodiments, the method for preparing the antibody-drug conjugate of the present invention is selected from a group consisting of:
conjugation method A: (suitable for the single pyrimidine linker series, target DAR8)
   diluting about 0.5 mL of an antibody (3-10 mg/mL) with a 0.1 M disodium edetate solution (pH 7.60), then adjusting the pH to 7.60 with a 1 M Na₂HPO₄ solution, adding 5.5 equivalents of 10 mM TCEP (tris (2-carboxyethyl) phosphine) solution (pH 7.60), homogeneously mixing, and then standing for 1.5 h at room temperature; and adding a drug-linker compound which is in a molar amount 8-10 times that of the antibody and dissolved in DMSO into the above solution system, standing for 2 h at room temperature after addition is finished, and then replacing the buffer solution with a 20 mM histidine buffer solution of pH 6.0 using a NAP-5 gel column (Cytiva) to obtain an ADC product.
conjugation method B: (suitable for the dipyrimidine linker series, target DAR4)
   diluting about 0.5 mL of an antibody (3-10 mg/mL) with a 0.1 M disodium edetate solution (pH 7.60), then adjusting the pH to 8.0 with a 1 M Na₂HPO₄ solution, adding 5.5 equivalents of 10 mM TCEP (tris(2-carboxyethyl) phosphine) solution (pH 7.60), homogeneously mixing, and then standing for 2 h at room temperature; and adding a drug-linker compound which is in a molar amount 4-6 times that of the antibody and dissolved in DMSO (10 mM, 1 equivalent is added every 30 min) into the above solution system, standing for 18 h at room temperature after addition is finished, and then replacing the buffer solution with a 20 mM histidine buffer solution of pH 6.0 using a NAP-5 gel column (Cytiva) to obtain an ADC product.
conjugation method C: (suitable for the bromoacetyl linker series (comparative compounds), target DAR4)
   diluting about 0.5 mL of an antibody (3-10 mg/mL) with a 0.1 M disodium edetate solution (pH 7.60), then adjusting the pH to 8.0 with a 1 M Na₂HPO₄ solution, adding 2.4 equivalents of 10 mM TCEP (tris(2-carboxyethyl) phosphine) solution (pH 7.60), homogeneously mixing, and then standing for 2 h at room temperature; and adding a drug-linker compound which is in a molar amount 4.0-4.5 times that of the antibody and dissolved in DMSO into the above solution system, standing for 18 h at room temperature after addition is finished, and then replacing the buffer solution with a 20 mM histidine buffer solution of pH 6.0 using a NAP-5 gel column (Cytiva) to obtain an ADC product.

### Detailed embodiments

Below the present invention will be further illustrated with specific examples, but these examples are not intended to limit the scope of the present invention. Those skilled in the art can make various modifications or improvements according to the teaching of the present invention without departing from the the basic spirits and scope of the present invention.

The information about the Adalimumab sequence involved in the present invention is described below:
The full length of a heavy chain (SEQ ID NO.5): 
EVQLVESGGGLVQPGRSLRLSCAASGFTFDDYAMHWVRQAPGKGLEWVSAI
TWNSGHIDYADSVEGRFTISRDNAKNSLYLQMNSLRAEDTAVYYCAKVSYLSTAS
SLDYWGQGTLVTVSSASTKGPSVFPLAPSSKSTSGGTAALGCLVKDYFPEPVTVS
WNSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSHE
DPEVKFNWYVDGVEVHNAKTKPREEQYNSTYRVVSVLTVLHQDWLNGKEYKC
KVSNKALPAPIEKTISKAKGQPREPQVYTLPPSRDELTKNQVSLTCLVKGFYPSDIA
VEWESNGQPENNYKTTPPVLDSDGSFFLYSKLTVDKSRWQQGNVFSCSVMHEAL
HNHYTQKSLSLSPGK.
The full length of a light chain (SEQ ID NO.6):

DIQMTQSPSSLSASVGDRVTITCRASQGIRNYLAWYQQKPGKAPKLLIYAAST
LQSGVPSRFSGSGSGTDFTLTISSLQPEDVATYYCQRYNRAPYTFGQGTKVEIKRTV
AAPSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC.

The information about the Tocilizumab sequence involved in the present invention is described below:
The full length of a heavy chain (SEQ ID NO.12): 
QVQLQESGPGLVRPSQTLSLTCTVSGYSITSDHAWSWVRQPPGRGLEWIGYIS
YSGITTYNPSLKSRVTMLRDTSKNQFSLRLSSVTAADTAVYYCARSLARTTAMDY
WGQGSLVTVSSASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKKVEP
KSCDKTHTCPPCPAPELLGGPSVFLFPPKPKDTLMISRTPEVTCVVVDVSHEDPEV
KFNWYVDGVEVHNAKTKPREEQYNSTYRVVSVLTVLHQDWLNGKEYKCKVSN
KALPAPIEKTISKAKGQPREPQVYTLPPSREEMTKNQVSLTCLVKGFYPSDIAVEW
ESNGQPENNYKTTPPVLDSDGSFFLYSKLTVDKSRWQQGNVFSCSVMHEALHNH
YTQKSLSLSPGK.
The full length of a light chain (SEQ ID NO.13): 



.
The full length of a mutant heavy chain (SEQ ID NO. 14):

QVQLQESGPGLVRPSQTLSLTCTVSGYSITSDHAWSWVRQPPGRGLEWIGYIS
YSGITTYNPSLKSRVTMLRDTSKNQFSLRLSSVTAADTAVYYCARSLARTTAMDY
WGQGSLVTVSSASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKKVEP
KSCDKTHTCPPCPAPEAAGAPSVFLFPPKPKDTLMISRTPEVTCVVVDVSHEDPEV
KFNWYVDGVEVHNAKTKPREEQYNSTYRVVSVLTVLHQDWLNGKEYKCKVSN
KALPAPIEKTISKAKGQPREPQVYTLPPSREEMTKNQVSLTCLVKGFYPSDIAVEW
ESNGQPENNYKTTPPVLDSDGSFFLYSKLTVDKSRWQQGNVFSCSVMHEALHNH
YTQKSLSLSPGK.

The abbreviations used herein have the following meaning:

| Abbreviation | Meaning | Abbreviation | Meaning |
|---|---|---|---|
| HATU | N,N,N',N'-tetramethyl-O-(7-azabenzotriazol-1-yl)uronium hexafluorophosphate | HOBt | 1-hydroxybenzotriazole |
| DIPEA | Diisopropylethylamine | EDCI | 1-(3- |
| | | | dimethylaminopropyl)-3-ethylcarbodiimide hydrochloride |
| TFA | Trifluoroacetic acid | DMF | N,N-dimethylformamide |
| Pd/C | Palladium on carbon | DMTMM | 4-(4,6-dimethoxytriazin-2-yl)-4-methylmorpholine hydrochloride |
| NBS | N-bromosuccinimide | IPA | Isopropanol |
| NaIO₄ | Sodium periodate | RuCl₃.H₂O | Ruthenium trichloride hydrate |
| XPhosPd G3 | (2-dicyclohexylphosphon-2',4',6'-triisopropyl-1,1'-biphenyl)(2'-amino-1,1'-biphen-2-yl)palladium (II) methanesulfonate | LiOH.H₂O | Lithium hydroxide hydrate |
| K₃PO₄ | Potassium phosphate | m-CPBA | m-chloroperoxybenzoic acid |
| THF | Tetrahydrofuran | DMSO | Dimethyl sulfoxide |
| DCM | Dichloromethane | MeOH | Methanol |
| HPLC | High-performance liquid chromatography | LC-MS | Liquid chromatography-mass spectrometry |
| TCEP | Tris(2-carboxyethyl) phosphine | Na₂HPO₄ | Disodium hydrogen phosphate |
| EDTA | Ethylenediamine tetraacetic acid | PB | Phosphate buffered saline |
| DAR | Drug-to-load ratio | CDI | Carbonyldiimidazole |
| T3P | 1-propylphosphoric anhydride | EtOH | Ethanol |

The structures of the compounds described in the following examples are determined by nuclear magnetic resonance (¹H NMR) or mass spectrometry (MS).

The Nuclear Magnetic Resonance (¹H NMR) measurement is carried out using a Bruker 400 MHz nuclear magnetic resonance spectrometer; the deuterated reagent is hexadeuterated dimethyl sulfoxide (DMSO-d6); and the internal standard substance is tetramethylsilane (TMS).

The abbreviations in the nuclear magnetic resonance (NMR) spectra used in the examples are shown below.

s: singlet, d: doublet, t: triplet, q: quartet, m: multiplet, br: broad, J: coupling constant, Hz: hertz, DMSO-d6: deuterated dimethyl sulfoxide. The δ value is represented in ppm.

The determination of MS is carried out using an Agilent (ESI) mass spectrometer with a model Agilent 6120B.

### Preparation example 1: (6S,8S,9R,10S,11S,13 S, 14S, 17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-amino-4-fluorobenzoate (A-1)

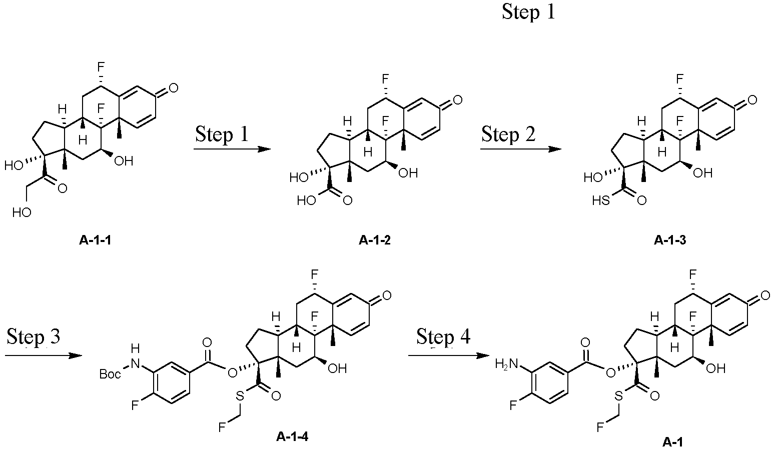

### Step 1:

Periodic acid (1.72 g, 7.57 mmol) was added into a solution of A-1-1 (1 g, 2.52 mmol) in THF (13.5 mL) and water (7.5 mL). The reaction system was stirred for 2 h at 25°C and then filtered. The filter cake was washed with water (30 ml x 3) and then dried in vacuum to obtain a crude product A-1-2 (900 mg, 2.35 mmol). The crude product was directly used in the next step without purification.

ESI-MS (m/z): 383.1 (M+H)⁺.

### Step 2:

A-1-2 (0.9 g, 2.35 mmol) was dissolved in DMF (3 mL), followed by addition of CDI (763 mg, 4.71 mmol), and then the reaction solution was stirred for 2 h at 25°C. DMF (7 mL) was added into the above reaction solution, followed by cooling to -20°C. Hydrogen sulfide gas (15 PSI) was introduced continuously within 30 min, and then the temperature was raised to 25°C and stirring was continued for 2 h. Water (100 mL) was added into the reaction solution to precipitate a solid, filtered, and the filter cake was dried in vacuum to obtain a crude product A-1-3 (1.3g, 2.11 mmol), which was directly used in the next step without purification.

ESI-MS (m/z): 399.2 (M+H)⁺.

### Step 3:

3-(tert-butoxycarbonylamino)-4-fluorobenzoic acid (289 mg, 1.13 mmol) was dissolved in DMF (7 mL), HATU (517 mg, 1.36 mmol) and DIPEA (440 mg, 3.40 mmol) were added, and then the reaction mixture was stirred for 1.5 h at 25°C. Then, A-1-3 (700 mg, 1.13 mmol) was added, and the reaction was continued for 1.5 h. Then, fluoroiodomethane (272 mg, 1.70 mmol) was added into the above reaction solution, and the reaction was continued for 1 h. Water (50 mL) was added into the reaction solution to precipitate a solid, filtered, and the filter cake was dried in vacuum to obtain a crude product A-1-4 (950 mg, 754 µmol), which was directly used in the next step without purification.

ESI-MS (m/z): 612.5 (M+H)⁺.

### Step 4:

A-1-4 (900 mg, 715 µmol) was dissolved in dichloromethane (5 mL), then trifluoroacetic acid (1.47 g, 12.9 mmol) was added, and the reaction solution was stirred for 1 h at 25°C. The reaction solution was directly concentrated to obtain a crude product, which was purified by high-performance liquid chromatography to obtain A-1 (310.39 mg, 532 µmol).
Chromatographic column: Phenomenex C18 150mm×25mm×10µm
Mobile phase A: acetonitrile; mobile phase B: water (0.05% ammonium bicarbonate)

| Time [min] | Mobile phase A [%] | Mobile phase B [%] | Flow rate [mL/min] |
|---|---|---|---|
| 0.00 | 42 | 58 | 24 |
| 8.00 | 72 | 28 | 24 |

The structure of A-1 was characterized as follows:
ESI-MS (m/z): 568.5 [M+H]⁺.

¹H NMR (400 MHz, DMSO) δ 7.24-7.37 (m, 2H), 7.15 (dd, J = 11.2, 8.4 Hz, 1H), 7.05 (ddd, J = 8.4, 4.4, 2.1 Hz, 1H), 6.33 (dd, J = 10.0, 1.8 Hz, 1H), 6.12 (s, 1H), 5.90-6.02 (m, 1H), 5.78-5.88 (m, 1H), 5.57-5.74 (m, 2H), 5.53 (s, 1H), 4.32 (s, 1H), 2.86-2.94 (m, 1H), 2.53-2.75 (m, 2H), 2.25-2.38 (m, 2H), 2.01-2.17 (m, 2H), 1.94 (d, J = 13.6 Hz, 1.2 H), 1.69-1.82 (m, 1H), 1.43-1.59 (m, 5H), 0.88-1.00 (m, 3H).

### Preparation example 2: (6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 2-aminoisonicotinate (P-1)

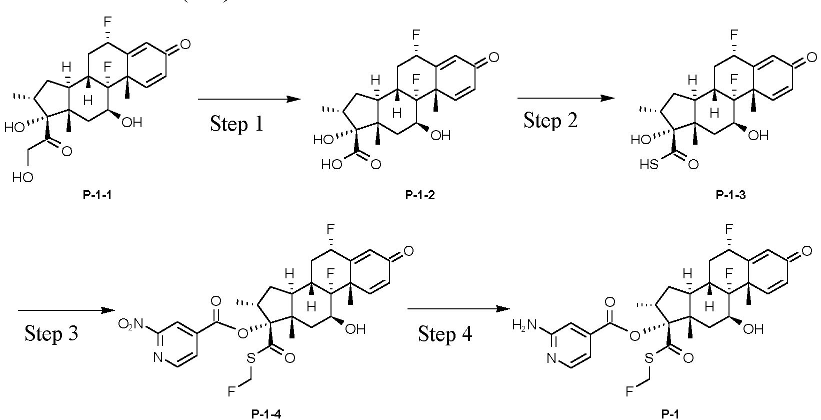

### Step 1:

P-1-1 (10 g, 24.3 mmol) was dissolved in THF (135 mL) and water (75 mL), and then periodic acid (16.6 g, 73.0 mmol, 16.6 mL) was added. The reaction solution was stirred for 2 h at 25°C, and then concentrated under reduced pressure to remove THF. The filter cake was washed with water (100ml x 3) and then dried in vacuum to obtain a crude product P-1-2 (9.00 g, 22.7 mmol, yield 93.18%), which was directly used in the next step without purification.

ESI-MS (m/z): 397.0 (M+H)⁺.

### Step 2:

P-1-2 (9.00 g, 22.7 mmol) was dissolved in DMF (100 mL), followed by adding CDI (7.36 mg, 45.41 mmol), and then the reaction solution was stirred for 4 h at 25°C. Hydrogen sulfide gas (15 PSI) was continuously introduced into the above reaction solution for 30 min, and then stirring was continued for 3 h. The reaction solution was adjusted to pH 2-3 using a 1 N hydrochloric acid aqueous solution to precipitate a solid, filtered, and then the filter cake was dried in vacuum to obtain P-1-3 (8.5 g, 20.61 mmol).

ESI-MS (m/z): 413.2 (M+H)⁺.

### Step 3

P-1-3 (300 mg, 727 µmol) and 2-nitropyridine-4-carboxylic acid (146 mg, 872 µmol) were dissolved in DMF (10.0 mL). T3P (694 mg, 1.09 mmol) and DIPEA (141 mg, 1.09 mmol) were added, and then the reaction mixture was stirred for 0.5 h at 25°C. Then, fluoroiodomethane (174 mg, 1.09 mmol) and DIPEA were added into the above reaction solution, and stirring was continued for 2 h. The reaction solution was filtered and then concentrated to obtain a crude product P-1-4 (350 mg), which was directly used in the next step without purification,

ESI-MS (m/z): 595.5 (M+H)⁺.

### Step 4:

At 25°C, P-1-4 (350 mg, 588 µmol) was added into EtOH (5.00 mL) and water (5.00 mL), and subsequently NH₄Cl (251 mg, 4.71 mmol) and iron powder (263 mg, 4.71 mmol) were added. After the addition was finished, the reaction solution was heated to 80°C and then react for 6 h. The reaction solution was filtered and then concentrated to obtain a crude product, which was purified by high performance liquid chromatography to obtain P-1 (0.169 g, 284 µmol).
Chromatographic column: Phenomenex C18 250 mm×50 mm×10 µm
Mobile phase A: acetonitrile; mobile phase B: water (0.05% ammonium bicarbonate)

| Time [min] | Mobile phase A [%] | Mobile phase B [%] | Flow rate [mL/min] |
|---|---|---|---|
| 0.00 | 33 | 67 | 24 |
| 8.00 | 63 | 37 | 24 |

The structure of P-1 was characterized as follows:
ESI-MS (m/z):565.5[M+H]⁺.

¹H NMR (400 MHz, DMSO) δ 8.1-8.3 (d, J = 5.2 Hz, 1H), 7.1-7.2 (d, J = 10 Hz, 1H), 7.0-7.1 (dd, J = 5.2 Hz, 1H), 6.94 (s, 1H), 6.3-6.5 (m, 2H), 5.7-6.1 (m, 2H), 5.3-5.5 (m, 1H), 4.6-4.8 (s, 2H), 4.4-4.6 (m, 1H), 3.4-3.6 (m, 1H), 2.3-2.6 (m, 4H), 1.9-2.0 (m, 3H), 1.8-1.9 (m, 1H), 1.56 (s, 3H), 1.4-1.5 (m, 1H), 1.18 (s, 3H), 1.0-1.1 (d, J = 7.2 Hz, 3H).

### Preparation example 3: (6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-aminofuran-2-carboxylate (F-2)

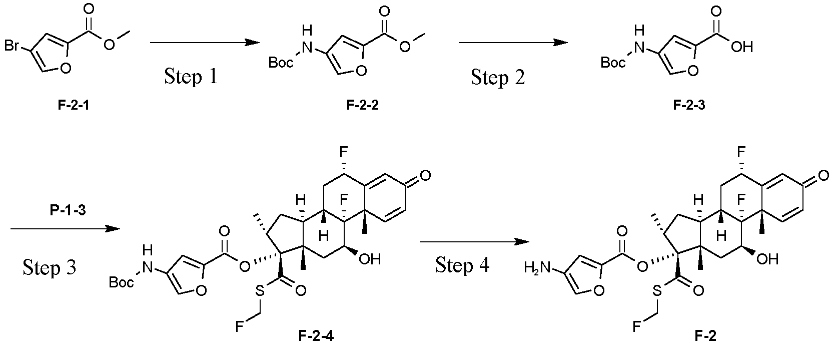

### Step 1:

methyl 4-bromofuran-2-formate (F-2-1, 1.5 g, 7.32 mmol) and tert-butyl carbamate (4.29 g, 36.5 mmol) were added into toluene (20.0 mL), and then N,N'-dimethylethylenediamine (322 mg, 3.66 mmol, 393 µL), cuprous iodide (836 mg, 4.39 mmol) and potassium carbonate (3.03 g, 21.9 mmol) were sequentially added. The reaction solution was heated to 130°C and stirred for 18 h. Water (200 mL) and dichloromethane (100 mL x 3) were added into the reaction solution for extraction. The organic phases were combined, dried, filtered and concentrated to obtain a crude product, which was purified via flash column chromatography (petroleum ether/ethyl acetate=100/1 to 5/1) to obtain F-2-2 (700 mg, 2.90 mmol).

ESI-MS (m/z): 242.0 (M+H)⁺.

### Step 2:

F-2-2 (700 mg, 2.90 mmol) was added into methanol (4.00 mL), tetrahydrofuran (4.00 mL) and water (2.00 mL), and then lithium hydroxide (138 mg, 5.80 mmol) was added. The system was allowed to react for 1 h at 25°C. Water and a 1 N hydrochloric acid aqueous solution (10.0 mL) were added into the reaction solution, followed by extraction with dichloromethane (20.0 mL x 3). The organic phases were combined, dried, filtered and concentrated to obtain F-2-3 (350 mg, 1.54 mmol).

ESI-MS (m/z): 228.1 (M+H)⁺.

### Step 3:

F-2-3 (100 mg, 440 µmol) and HATU (200 mg, 528 µmol) were added into DMF (10.0 mL), and then DIPEA (284 mg, 2.20 mmol, 383 µL) was added. The reaction solution was allowed to react for 2 h at 25°C, and P-1-3 (181 mg, 440 µmol) was added. Stirring was continued for 2 h, followed by adding fluoroiodomethane (105 mg, 660 µmol), and then the system was allowed to react for 1 h at 25°C. Water (100 mL) and ethyl acetate (100 mL x 3) were added into the reaction solution for extraction. The organic phases were combined, dried, filtered and concentrated to obtain a crude product F-2-4 (220 mg, 336 µmol), which was used in the next step without purification.

ESI-MS (m/z): 654.2 (M+H)⁺.

### Step 4:

F-2-4 (150 mg, 229 µmol) was added into dichloromethane (3 mL), and trifluoroacetic acid (1.54 g, 13.5 mmol) was added. The reaction solution was stirred for 1 h at 25°C and then concentrated to directly obtain a crude product, which was purified via high performance liquid chromatography to obtain F-2 (27.9 mg, 45.54 µmol).
Chromatographic column: Phenomenex C18 150mm×25mm×10 µm
Mobile phase A: acetonitrile; mobile phase B: water (0.05% ammonium bicarbonate)

| Time [min] | Mobile phase A [%] | Mobile phase B [%] | Flow rate [mL/min] |
|---|---|---|---|
| 0.00 | 35 | 65 | 24 |
| 8.00 | 65 | 35 | 24 |

The structure of F-2 was characterized as follows:
ESI-MS (m/z):554.2[M+H]⁺.

¹H NMR (400 MHz, DMSO) δ 7.34 (dd, J=1.2, 10.0 Hz, 1H), 6.87-7.31 (m, 1H), 6.27-6.38 (m, 2H), 5.88-6.03 (m, 1H), 5.74-5.88 (m, 1H), 5.47-5.65 (m, 1H), 4.33 (d, J=9.6 Hz, 1H), 3.46 (dt, J=3.2, 6.97 Hz, 1H), 2.54-2.72 (m, 1H), 2.36 (dd, J=3.2, 11.2 Hz, 3H), 1.86-2.08 (m, 2H), 1.62-1.71 (m, 1H), 1.56-1.60 (m, 3H), 1.32-1.42 (m, 1H), 1.16 (s, 3H), 0.98-1.06 (m, 3H).

### Preparation example 4: 6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (1S)-2-(4-aminophenyl)cyclopropane-1-carboxylate (C-1)

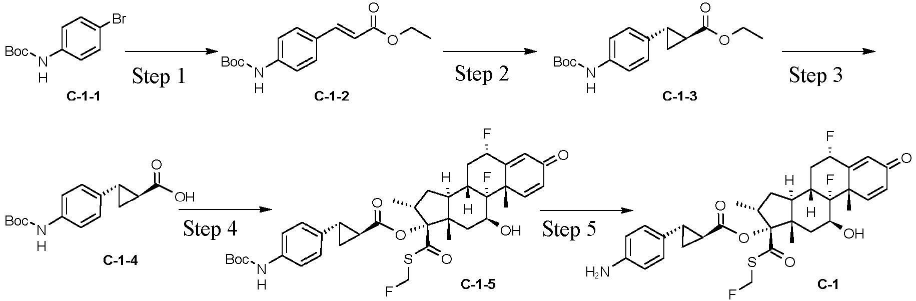

### Step 1:

Tert-butyl N-(4-bromophenyl)carbamate (C-1-1, 5.00 g, 18.3 mmol), ethyl (E)-3-(4,4,5,5-tetramethyl-1,3,2-dioxaboran-2-yl)prop-2-enoate (4.57 g, 20.2 mmol), XPhos Pd G3 (1.24 g, 1.47 mmol) and potassium phosphate (11.70 g, 55.1 mmol) were added into 1,4-dioxane (50.0 mL) and water (17.0 mL). The reaction mixture was heated to 100°C after nitrogen replacement for 3 times, and allowed to react for 3 h. The reaction system was filtered via a silica gel pad, and extracted with water (80.0 mL) and ethyl acetate (50.0 mL x 3). The organic phases were combined, dried, filtered and concentrated to obtain a crude product, which was purified via flash column chromatography (petroleum ether/ethyl acetate=100/1 to 3/1) and concentrated to obtain C-1-2 (4.20 g, 12.9 mmol).

ESI-MS (m/z): 292.1 (M+H)⁺.

### Step 2:

C-1-2 (3.60 g, 12.3 mmol) was added into DMSO (40.0 mL), and trimethylsulfoxonium iodide (3.26 g, 14.8 mmol) and potassium t-butoxide (1.53 g, 13.5 mmol) were added. The reaction system was stirred for 2 h at 25 °C, and then extracted with water (50.0 mL) and ethyl acetate (50.0 mL x 3). The organic phases were combined, dried, filtered and concentrated to obtain a crude product, which was purified via flash column chromatography (petroleum ether/ethyl acetate=100/1 to 3/1) and concentrated to obtain C-1-3 (400 mg, 1.31 mmol).

ESI-MS (m/z): 306.0 (M+H)⁺.

### Step 3:

C-1-3 (400 mg, 1.31 mmol) was added into methanol (2.00 mL), water (2.00 mL) and THF (2.00 mL), followed by adding lithium hydroxide (62.7 mg, 2.62 mmol), and the reaction system was allowed to react for 1 h at 25°C. The reaction solution was adjusted to pH 2-3 with a 1 N hydrochloric acid aqueous solution to precipitate a solid, filtered, and the filter cake was dried in vacuum to obtain C-1-4 (300 mg, 1.08 mmol), which was directly used in the next step without purification.

ESI-MS (m/z): 278.1 (M+H)⁺.

### Step 4: Synthesis of (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (1S,2S)-2-(4-(tert-butoxycarbonyl)amino)phenyl)cyclopropane-1-carboxylate (C-1-5)

C-1-4 (200 mg, 721 µmol), DIPEA (466 mg, 3.61 mmol, 628 µL) and HATU (548 mg, 1.44 mmol) were added into DMF (15.0 mL). The reaction system was stirred for 1 h at 25°C, followed by adding P-1-3 (356 mg, 865 µmol), and then stirred for additional 2 h. Fluoroiodomethane (173 mg, 1.08 mmol) was then added and the reaction system continued to react for 1 h. The reaction system was extracted with water (10.0 mL) and dichloromethane (10.0 mL x 3). The organic phases were combined, dried, filtered and concentrated to obtain C-1-5 (150 mg, 213 µmol), which was directly used in the next step without purification.

ESI-MS (m/z): 704.3 (M+H)⁺.

### Step 5:

C-1-5 (50.0 mg, 71.0 µmol) was added into dichloromethane (1.00 mL), and then TFA (462 mg, 4.05 mmol, 0.30 mL) was added. The reaction system was allowed to react for 1 h at 25°C, and then directly concentrated to obtain a crude product, which was purified via high performance liquid chromatography to obtain C-1 (11.89 mg, 18.4 µmol).
Chromatographic column: Phenomenex C18 150mm×25mm×10µm
Mobile phase A: acetonitrile; mobile phase B: water (0.05% ammonium bicarbonate)

| Time [min] | Mobile phase A [%] | Mobile phase B [%] | Flow rate [mL/min] |
|---|---|---|---|
| 0.00 | 39 | 61 | 24 |
| 8.00 | 69 | 31 | 24 |

The structure of C-1 was characterized as follows:
ESI-MS (m/z): 604.2 [M+H]⁺.

¹H NMR (400 MHz, DMSO) δ 7.32 (dd, J = 6.0, 9.57 Hz, 1H), 6.78-6.96 (m, 2H), 6.65 (dd, J = 1.6, 8.4 Hz, 2H), 6.25-6.41 (m, 2H), 5.91-6.00 (m, 1H), 5.78-5.87 (m, 1H), 5.33-5.66 (m, 2H), 4.30 (t, J = 10.0 Hz, 1H), 3.41 (d, J = 4.00 Hz, 2H), 2.52-2.66 (m, 1H), 2.19-2.41 (m, 4H), 1.93-2.06 (m, 2H), 1.78 (ddd, J = 4.8, 9.0, 13.6 Hz, 1H), 1.55-1.69 (m, 4H), 1.41-1.48 (m, 1H), 1.35 (tt, J = 3.74, 7.52 Hz, 2H), 1.12 (d, J = 1.8 Hz, 3H), 0.92-1.10 (m, 3H).

### Preparation example 5: fluoromethyl (6S,8S,9R,10S,11S,13S,14S,16R,17R)-17-((3-amino-4-fluorobenzoyl)oxy)-6,9-difluoro-11-hydroxy-10,13,16-trimethyl-3-oxo-6,7,8,9,10,11,12,13,14,15, 16, 17-dodecahydro-3H-cyclopenta[a]phenanthrene-17-carboxylate (O-1)

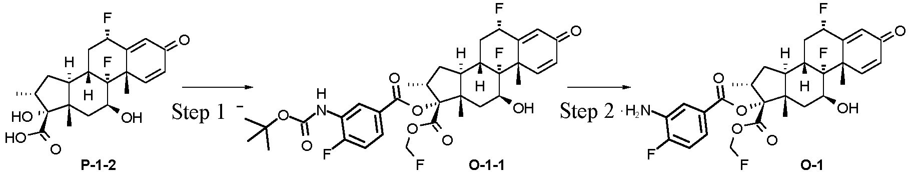

### Step 1:

Starting materials P-1-2 (193.16 mg, 756.77 µmol), DIPEA (146.71 mg, 1.14 mmol) and HATU (345.30 mg, 908.12 µmol) were added into DMF (15 mL), and the reaction system was allowed to react for 15 min at 25°C. DIPEA (146.71 mg, 1.14 mmol) was added into the reaction system, and then 3- (tert-butoxycarbonylamino)-4-fluorobenzoic acid (300 mg, 756.77 µmol) was added, and the reaction system continued to react for 16 h at 25°C after the addition was finished. The reaction was monitored via LC-MS, fluoroiodomethane (604.49 mg, 3.78 mmol) was added into the reaction system, and the reaction system was allowed to react for 4 h at 25°C. The reaction was monitored via LC-MS, and water was added into the reaction system to precipitate a pale yellow solid, which was filtered and collected to obtain a crude product, which was purified via column chromatography (ethyl acetate/petroleum ether = 0-35%) to obtain O-1-1 (390 mg).

ESI-MS (m/z):610.1 [M+1-56]⁺.

### Step 2:

Compound O-1-2 (70 mg, 105.16 µmol) was added into DCM (3 mL) and TFA (1 mL), and the reaction system was allowed to react for 1.5 h at 25°C. The reaction was monitored via LC-ML until it was completed. The reaction system was concentrated to dryness to obtain a crude product, which was purified via high performance liquid chromatography to obtain O-1 (23.8 mg).
Chromatographic column: Waters XBridge Prep C18OBD (5 µm*19mm*150 mm)
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 30 | 70 | 28 |
| 2.00 | 30 | 70 | 28 |
| 12.00 | 90 | 10 | 28 |

The structure of O-1 was characterized as follows:
ESI-MS (m/z):566.3 [M+1]⁺.

¹H NMR (400 MHz, DMSO-d6): δ 7.34-7.24 (m, 2H), 7.16 (dd, J = 11.2 Hz, 8.4 Hz, 1H), 7.07-7.01 (m, 1H), 6.33 (dd, J = 10.0 Hz, 2.0 Hz, 1H), 6.14 (s, 1H), 5.97-5.55 (m, 4H), 4.30-4.20 (m, 1H), 3.36-3.25 (m, 1H), 2.68-2.54 (m, 1H), 2.33-2.21 (m, 2H), 2.20-2.12 (m, 1H), 1.95-1.83 (m, 1H), 1.81-1.73 (m, 1H), 1.67-1.54 (m, 1H), 1.51 (s, 3H), 1.34-1.25 (m, 1H), 1.06 (s, 3H), 0.87 (d, J = 7.2 Hz, 3H).

### Preparation example 6: chloromethyl (6S,8S,9R,10S,11S,13S,14S,16R,17R)-17-((3-amino-4-fluorobenzoyl)oxy)-6,9-difluoro-11-hydroxy-10,13,16-trimethyl-3-oxo-6,7,8,9,10,11,12,13,14,15, 16, 17-dodecahydro-3H-cyclopenta[a]phenanthrene-17-carboxylate (O-3)

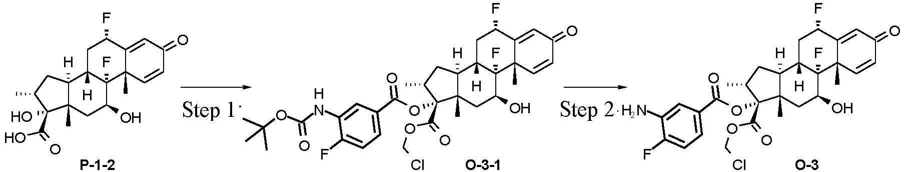

### Step 1:

P-1-2 (96.58mg, 378.33 µmol), DIPEA (73.35 mg, 567.57 µmol) and HATU (172.65 mg, 454.06 µmol) were added into DMF (172.65 mg, 454.06 µmol), and the reaction mixture was allowed to react for 15 min at 25°C. Then DIPEA (73.35 mg, 567.57 µmol) and 3-(tert-butoxycarbonylamino)-4-fluorobenzoic acid (150 mg, 378.38 µmol) were added, and the reaction mixture was allowed to react for 3 h at 25°C after the addition was finished. The reaction was monitored via LC-MS, then chloroiodomethane (668.05 mg, 3.78 mmol) was added into the reaction system, and the reaction system was allowed to react for 20 h at 45°C. The reaction was monitored via LC-MS. The reaction system was extracted with water and dichloromethane. The organic phase was dried and concentrated to obtain 230 mg of a crude product O-3-1, which was directly used in the next step reaction.

### Step 2:

Compound O-3-1 (150 mg, 105.16 µmol) was added into DCM (4 mL) and TFA (2 mL), and the reaction system was allowed to react for 1.5 h at 25°C. The reaction was monitored via LC-MS. The reaction system was concentrated be dryness to obtain a crude product, which was purified by high performance liquid chromatography to obtain O-3 (20 mg).
Chromatographic column: Waters XBridge Prep C18OBD (5 µm*19mm*150 mm)
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 40 | 60 | 28 |
| 2.00 | 40 | 60 | 28 |
| 20.00 | 90 | 10 | 28 |

The structure of O-3 was characterized as follows:
ESI-MS (m/z):582.2 [M+1]⁺.

¹H NMR (400 MHz, DMSO-d6): δ 7.33-7.26 (m, 2H), 7.16 (dd, J = 11.2 Hz, 8.8 Hz, 1H), 7.05-6.99 (m, 1H), 6.33 (dd, J = 10.0 Hz, 2.0 Hz, 1H), 6.14 (s, 1H), 5.96 (d, J = 6.0 Hz, 1H), 5.86 (d, J = 6.0 Hz, 1H), 5.76-5.56 (m, 2H), 4.28-4.20 (m, 1H), 3.48-3.25 (m, 1H), 2.65-2.55 (m, 1H), 2.31-2.11 (m, 3H), 1.95-1.82 (m, 1H), 1.78-1.70 (m, 1H), 1.64-1.54 (m, 1H), 1.51 (s, 3H), 1.34-1.24 (m, 1H), 1.07 (s, 3H), 0.87 (d, J = 7.2 Hz, 3H).

### Preparation example 7: (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (E)-3-(3-amino-4-fluorophenyl)acrylate (E-3)

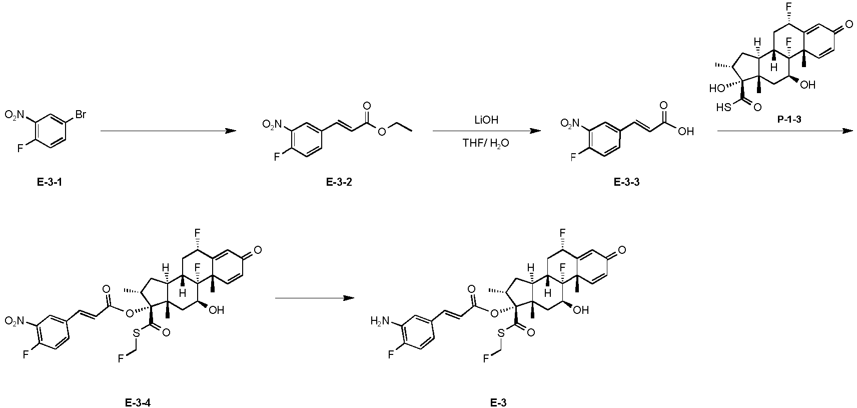

### Step 1:

4-bromo-1-fluoro-2-nitrobenzene (1.00 g, 4.55 mmol), ethyl (E)-3-(4,4,5,5-tetramethyl-1,3,2-dioxaboran-2-yl)acrylate (1.25 g, 5.45 mmol), Xphos-Pd-G3 (384.76 mg, 455 µmol) and potassium phosphate (2.89 g, 13.6 mmol) were added into 1,4-dioxane (12 mL) and water (4 mL). The reaction mixture was heated to 100°C after nitrogen replacement for 3 times, and was allowed to react for 2 h. Water (100 mL) was added into the reaction solution, followed by extraction with ethyl acetate for 3 times (60 mL x 3). The organic phases were combined and then dried over anhydrous sodium sulfate, filtered and concentrated to obtain a crude product, which was purified via flash column chromatography (petroleum ether/ethyl acetate = 100/1 to 5/1) and then concentrated again to obtain ethyl (E)-3-(4-fluoro-3-nitrophenyl)acrylate (450 mg, 1.76 mmol).

ESI-MS (m/z): 240.1 (M+H)⁺.

### Step 2:

Ethyl (E)-3-(4-fluoro-3-nitrophenyl)acrylate (300 mg, 1.25 mmol) was dissolved in tetrahydrofuran (3 mL) and water (3 mL). Then lithium hydroxide monohydrate (210 mg, 5.02 mmol) was added, and the reaction solution was stirred for 2 h at room temperature. The reaction solution was adjusted to pH 2-3 using 1 N diluted hydrochloric acid to precipitate a solid, and filtered. The filter cake was dried in vacuum to obtain (E)-3-(4-fluoro-3-nitrophenyl)acrylic acid (305 mg, 895 µmol).

ESI-MS (m/z): 212.0 (M+H)⁺.

### Step 3:

(E)-3-(4-fluoro-3-nitrophenyl) acrylic acid (270 mg, 1.28 mmol) was dissolved in dichloromethane (10 mL), DIPEA (826 mg, 6.39 mmol) and T3P (2.44 g, 3.84 mmol, 2.28 mL, 50% purity) were added, and then the reaction solution was stirred for 1 h at 25°C. (6S,8S,9R,10S,11S,13S,14S,16R,17R)-6,9-difluoro-11,17-dihydroxy-10,13,16-trimethyl-3-oxo-6,7,8,9,10,11,12,13,14,15,16,17-dodecahydro-3H-cyclopenta[a]phenanthrene-17-carboxylic acid (527.46 mg, 1.28 mmol) was added into the above reaction solution, and the reaction solution was stirred for additional 2 h. Fluoroiodomethane (224 mg, 1.41 mmol) was added, and then the reaction solution was stirred for additional 0.5 h. Water (100 mL) was added, and the reaction solution was extracted with dichloromethane for 3 times (60 mL x 3). The organic phases were combined and dried over anhydrous sodium sulfate, filtered and concentrated to obtain a crude product, which was purified via flash column chromatography (petroleum ether/ethyl acetate = 100/1 to 1/1), and then concentrated to obtain (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (E)-3-(4-fluoro-3-nitrophenyl)acrylate (52.0 mg, 28.6 µmol).

ESI-MS (m/z): 638.2 (M+H)⁺.

### Step 4:

(6S,8S,9R,10S,11S,13S,14S,16R,17R)-6,9-difluoro-17-((fluoromethyl)thio)carbonyl) -11-hydroxy-10,13,16-trimethyl-3-oxo-6,7,8,9,10,11,12,13,14,15,16,17-dodecahydro-3H-cyclopenta[a]phenanthren-17-yl (E)-3-(4-fluoro-3-nitrophenyl)acrylate (50.0 mg, 78.4 µmol) was dissolved in ethanol (1.5 mL) and water (0.5 mL), followed by adding iron powder (43.79 mg, 784 µmol) and ammonium chloride (20.9 mg, 392 µmol). The reaction solution was heated to 80°C and stirred for 2 h. Water (50 mL) was added into the reaction solution, then the reaction solution was extracted with ethyl acetate for 3 times (40 mL x 3). The organic phases were combined and then dried over anhydrous sodium sulfate, filtered and concentrated to obtain a crude product, which was purified via high performance liquid chromatography to obtain (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (E)-3-(3-amino-4-fluorophenyl)acrylate (4.49 mg, 7.15 µmol).
Chromatographic column: henomenex luna C18 150 mm×25 mm×10 µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 51 | 49 | 20 |
| 15.00 | 81 | 19 | 20 |

The structure thereof was characterized as follows:
ESI-MS (m/z): 608.2 [M+H]⁺.

¹H NMR (400 MHz, DMSO) δ 7.50 (d, J = 16.0 Hz, 1H), 7.35 (dd, J = 10.4, 1.2 Hz, 1H), 7.07 (dd, J = 8.4, 2.4 Hz, 1H), 6.97 (dd, J = 10.8, 8.4 Hz, 1H), 6.82-6.90 (m, 1H), 6.34-6.42 (m, 2H), 6.32 (s, 1H), 5.91-6.03 (m, 1H), 5.79-5.90 (m, 1H), 5.46-5.67 (m, 1H), 4.34 (d, J = 9.6 Hz, 1H), 3.43-3.50 (m, 1H), 2.52-2.72 (m, 1H), 2.28-2.40 (m, 3H), 1.95-2.08 (m, 2H), 1.63-1.73 (m, 1H), 1.59 (s, 3H), 1.33-1.40 (m, 1H), 1.16 (s, 3H), 1.01 (d, J = 7.2 Hz, 3H).

### Preparation example 8: (S)-1-(3,5-bis(2-(methylsulfonyl)pyrimidin-5-yl)phenyl)-34-(5-((((6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl)oxy)carbonyl)-2-fluorophenyl)carbamoyl)-1,29,32-trioxo-5,8,11,14,17,20,23,26-octaoxa-2,30,33-triazaheptatriacontan-37-ynoic acid (DL-B-11')

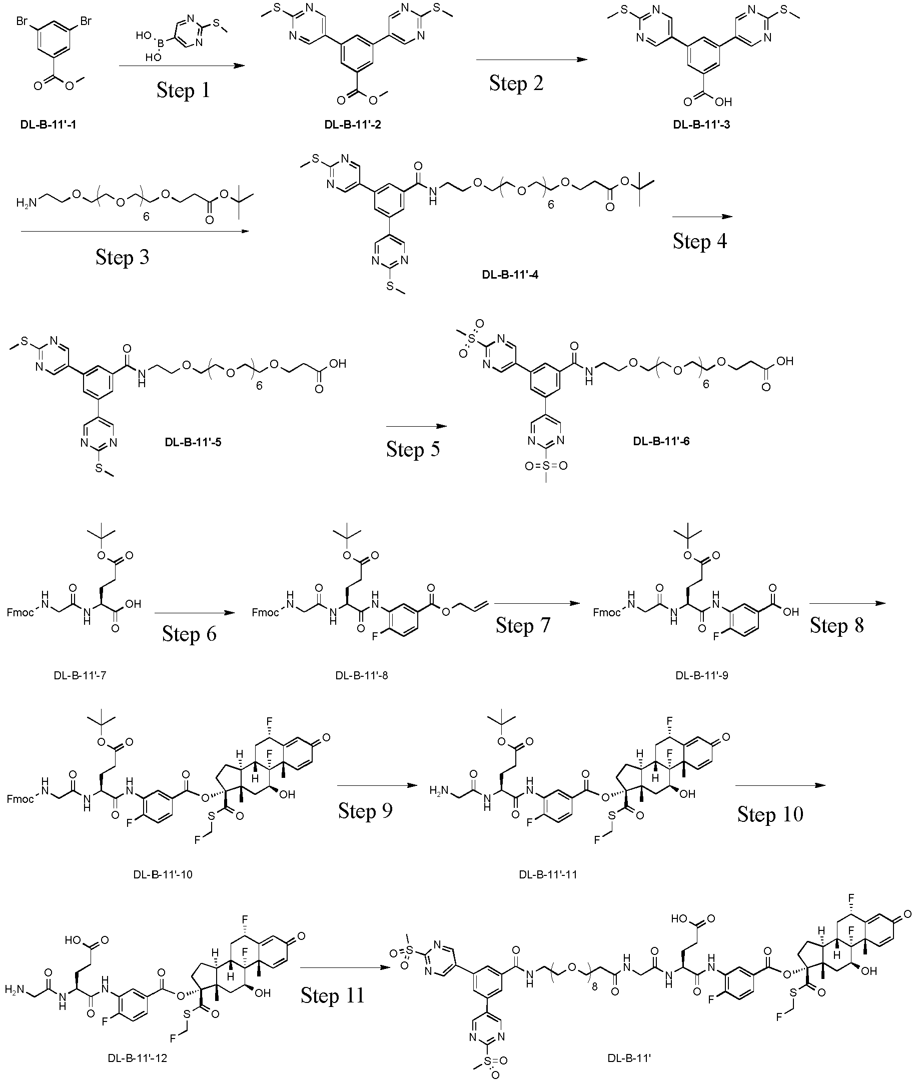

### Step 1:

Starting materials methyl 3,5-dibromobenzoate (720 mg, 2.45 mmol), 2-methylthiopyrimidine-5-boronic acid (874 mg, 5.14 mmol), XPhosPd G3 (207 mg, 245 µmol) and K₃PO₄ (1.56 g, 7.35 mmol) were added to dioxane (12 mL) and water (4 mL), and the reaction system was allowed to react at 90°C for 3 h under stirring under a nitrogen atmosphere. The reaction was monitored via LC-MS. The reaction system was filtered through a diatomite pad, and the filtrate was extracted with water and ethyl acetate and concentrated to obtain a crude product, which was purified by column chromatography (EA/PE = 0-25%) to obtain 710 mg of methyl 3,5-bis(2-(methylthio)pyrimidin-5-yl)benzoate.

The structure characterization data is as follows:
ESI-MS (m/z): 385.1 [M+H]⁺.

### Step 2:

Compound methyl 3,5-bis(2-(methylthio)pyrimidin-5-yl)benzoate (650 mg, 1.69 mol) and lithium hydroxide (121 mg, 5.07 mmol) were dissolved in THF (2 mL), MeOH (2 mL) and H₂O (2 mL). The reaction mixture was allowed to react for 2 h at 25°C under stirring. The reaction was monitored via LC-MS. The system was adjusted with 1 N HCl to about pH 2 to precipitate a large amount of a solid, which was filtered, and the filter cake was collected and dried to obtain 560 mg of 3,5-bis(2-(methylthio)pyrimidin-5-yl)benzoic acid.

The structure characterization data is as follows:
ESI-MS (m/z):371.1 [M+H]⁺.

### Step 3:

3,5-bis(2-(methylthio)pyrimidin-5-yl)benzoic acid (3.00 g, 8.10 mmol) and tert-butyl 1-amino-3,6,9,12,15,18,21,24-octaoxaheptan-27-carboxylate (4.03 g, 8.10 mmol) were added into DMF (40 mL), followed by sequentially adding HOBt (3.28 g, 24.3 mmol), EDCI (4.66 g, 24.3 mmol) and DIPEA (4.19 g, 32.4 mmol, 5.64 mL), and the reaction system was stirred for 2 h at 60°C. The reaction solution was extracted with water (100 mL) and ethyl acetate (60 mL x 3). The organic phases were combined, dried over anhydrous sodium sulfate, filtered and concentrated to obtain tert-butyl 1-(3,5-bis(2-(methylthio)pyrimidin-5-yl)phenyl)-1-oxo-5,8,11,14,17,20,23,26-octaoxa-2-azanonacosane-29-carboxylate (4.20 g, 4.14 mmol), which was directly used in the next step without purification.

### Step 4:

Tert-butyl 1-(3,5-bis(2-(methylthio)pyrimidin-5-yl)phenyl)-1-oxo-5,8,11,14,17,20,23,26-octaoxa-2-azanonacosane-29-carboxylate (3.60 g, 4.24 mmol) was dissolved in dichloromethane (30 mL), followed by adding TFA (15.3 g, 134 mmol, 10 mL), and the reaction system was stirred for 6 h at 25°C. The reaction solution was extracted with water (60 mL) and ethyl acetate (40 mL x 3). The organic phases were combined and dried over anhydrous sodium sulfate, filtered and concentrated to obtain a crude product, which was purified via high performance liquid chromatography and then freeze-dried to obtain 1-(3,5-bis(2-(methylthio)pyrimidin-5-yl)phenyl)-1-oxo-5,8,11,14,17,20,23,26-octaoxa-2-azanonacosane-29-carboxylic acid (2.93 g, 3.63 mmol).

The structure characterization data is as follows:
ESI-MS (m/z):794.3 [M+H]⁺.

The purification method is as follows:
Chromatographic column: Phenomenex luna C18 (250 mm*70mm*10µm)
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 30 | 70 | 30 |
| 15.00 | 60 | 40 | 30 |

### Step 5:

1-(3,5-bis(2-(methylthio)pyrimidin-5-yl)phenyl)-1-oxo-5,8,11,14,17,20,23,26-octaoxa-2-azanonacosane-29-carboxylic acid (148 mg, 0.186 mmol) was added into acetonitrile (15 mL) and water (7.5 mL), sodium periodate (398.71 mg, 1.86 mmol) and ruthenium trichloride hydrate (15.47 mg, 74.56 µmol) were added, and the reaction system was allowed to react for 30 min at 25°C under stirring. The reaction system was extracted with water and ethyl acetate and concentrated to obtain 1-(3,5-bis(2-(methylsulfonyl)pyrimidin-5-yl)phenyl)-1-oxo-5,8,11,14,17,20,23,26-octaoxa-2-azanonacosane-29-carboxylic acid (155 mg).

The structure characterization data is as follows:
ESI-MS (m/z):858.3 [M+H]⁺.

### Step 6:

(S)-2-(2-(((9H-fluoren-9-yl)methoxy)carbonyl)amino)acetylamino)-5-(tert-butoxy)-5-oxovaleric acid (764 mg, 1.58 mmol), allyl 3-amino-4-fluorobenzoate (324.50 mg, 1.66 mmol), pyridine (375.73 mg, 4.75 mmol), 50% 1-propyl phosphoric anhydride-DMF (5.57 g) solution were successively added into DCM (1 mL), and the reaction mixture was allowed to react for 3 h at 25°C under stirring after the addition was finished. The reaction was monitored via TLC (PE:EA = 0:1). After the reaction was completed, 12 mL of water was added into the reaction solution, followed by adding DCM (15mL*2) for extraction. The organic phase was washed with water (8 mL), dried over anhydrous sodium sulfate (3 g) and filtered, and the filtrate was concentrated under reduced pressure and then purified via column chromatography (SiO₂, CH₂Cl₂/MeOH = 100/1 to 10/1) to obtain allyl (S)-3-(2-(2-(((9H-fluoren-9-yl)methoxy)carbonyl)amino)acetylamino)-5-(tert-butoxy)-5-oxopentanamide)-4-fluorobenzoate (814 mg,1.14 mmol).

The structure characterization data is as follows:
ESI-MS (m/z): 659.70(M+H)⁺.

### Step 7:

Allyl (S)-3-(2-(2-(((9H-fluoren-9-yl)methoxy)carbonyl)amino)acetylamino)-5-(tert-butoxy)-5-oxopentanamide)-4-fluorobenzoate (734 mg,1.11 mmol), morpholine (193.86 mg, 2.23 mmol), Pd(PPh₃)₄ (64.29 mg, 55.63 µmol) were sequentially added to THF (7 mL), and the reaction solution was allowed to react for 2 h at 25°C after the addition was finished. Water (26 mL) was added, and the reaction solution was extracted with DCM (26 mL *2). The organic phase was washed with water (8 mL) and saturated saline (8 mL) respectively, dried over anhydrous sodium sulfate (4 g), and concentrated under reduced pressure to almost dryness. PE (8 mL) was added, followed by slurrying for 1 h at room temperature, and filtered. The filter cake was dried under reduced pressure to obtain (S)-3-(2-(2-(((9H-fluoren-9-yl)methoxy)carbonyl)amino)acetylamino)-5-(tert-butoxy)-5-oxopentanamide)-4-fluorobenzoic acid (450 mg, 697.18 µmol).

The structure characterization data is as follows:
ESI-MS (m/z): 620 (M+H)⁺.

### Step 8:

(S)-3-(2-(2-(((9H-fluoren-9-yl)methoxy)carbonyl)amino)acetylamino)-5-(tert-butoxy)-5-oxopentanamide)-4-fluorobenzoic acid (200 mg, 322.77 µmol), DIPEA (83.43 mg, 645.54 µmol) and HATU (147.73 mg, 387.32 µmol) were successively added into DMF (4 mL), and the reaction solution was allowed to react for 35 min at 16°C under stirring after the addition was finished. A-1-3 (128.86 mg, 320.28 µmol) was added, and the reaction solution was allowed to react for 1 h at 20°C under stirring after the addition was finished. DIPEA (31.02 mg, 239.98 µmol) was added into the above reaction solution, followed by adding fluoroiodomethane (211.09 mg, 1.32 mmol), and then the reaction solution was allowed to react for 1 h at 20°C under stirring after the addition was finished. Water (12 mL) was added into the reaction solution, and EA (15 mL*2) was added for extraction. The organic phase was washed with water (8 mL) and saturated brine (8 mL) respectively, and separated. The organic phase was concentrated under reduced pressure to dryness, and purified via column chromatography (SiO₂, PE/EA = 12/1 to 1/1) to obtain (6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-2-((((9H-fluoren-9-yl)methoxy)carbonylamino) acetamino)-5-(tert-butoxy)-5-oxopentanamide)-4-fluorobenzoate (88 mg, 80.15 µmol).

The structure characterization data is as follows:
ESI-MS (m/z): 1032 (M+H)⁺.

### Step 9:

(6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-2-((((9H-fluoren-9-yl)methoxy)carbonylamino) acetamino)-5-(tert-butoxy)-5-oxopentanamide)-4-fluorobenzoate (88 mg, 80.15 µmol) and diethylamine (31.18 mg, 426.32 µmol) were successively added into MeCN (4 mL), and the reaction mixture was allowed to react for 2 h at 25°C under stirring after the addition was finished. The reaction solution was concentrated under reduced pressure to obtain a (6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-2-(2-aminoacetamide)-5-(tert-butoxy)-5-oxopentanamide)-4-fluorobenzoate crude product (69 mg, 80.94 µmol), which was directly used in the next step without purification.

The structure characterization data is as follows:
ESI-MS (m/z): 810 (M+H)⁺.

### Step 10:

(6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-2-(2-aminoacetamide)-5-(tert-butoxy)-5-oxopentanamide)-4-fluorobenzoate crude product (69 mg, 80.94 µmol) was added into DCM (3 mL), followed by adding TFA (0.5 mL), and the reaction solution was allowed to react for 3 h at 25°C under stirring after the addition was finished. The reaction solution was concentrated under reduced pressure to remove DCM and TFA so as to obtain a (S)-4-(2-aminoacetamide)-5-(5-(((6S,8S,9R,10S,11S,13S,14S,17R)-6,9-difluoro-17-(fluoromethyl)thio)carbonyl)-11-hydroxy-10,13-dimethyl-3-oxo-6,7,8,9,10,11,12,13,14,15, 16, 17-dodecahydro-3H-cyclopenta[a]phenanthren-17-yl)oxy)carbonyl)-2-fluorophenyl)amino)-5-oxovaleric acid crude product (88 mg, 83.15 µmol), which was directly used in the next step without purification.

The structure characterization data is as follows:
ESI-MS (m/z): 754 (M+H)⁺.

### Step 11:

The (S)-4-(2-aminoacetamide)-5-(5-(((6S,8S,9R,10S,11S,13S,14S,17R)-6,9-difluoro-17-(fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl)oxy)carbonyl)-2-fluorophenyl)amino)-5-oxovaleric acid crude product (24 mg, 27.66 µmol), 1-(3,5-bis(2-(methylsulfonyl)pyrimidin-5-yl)phenyl)-1-oxo-5,8,11,14,17,20,23,26-octaoxa-2- azanonacosane-29-carboxylic acid (23.73 mg, 27.66 µmol), DIPEA (17.87 mg, 138.28 µmol) and HATU (20 mg, 52.60 µmol) were successively added into DMF (2 mL), and the reaction solution was allowed to react for 1 h at 15°C under stirring after the addition was finished. The reaction solution was directly purified via high performance liquid chromatography to obtain the title compound (4.55 mg, 2.77 µmol).

The purification method is as follows:
Chromatographic column: SunFire Prep C18 OBD 19*150 mm*5 µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 35 | 65 | 30 |
| 3.00 | 35 | 65 | 30 |
| 8.00 | 95 | 5 | 30 |

The structure characterization data is as follows:
ESI-MS (m/z): 1594 (M+H)⁺.

### Preparation example 9: (S)-5-((5-((((6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl)oxy)carbonyl)-2-fluorophenyl)amino)-4-(2-(6-(2-(methylsulfonyl)pyrimidin-5-yl)hex-5-ynylamido)acetamino)-5-oxovaleric acid (DL-A-05)

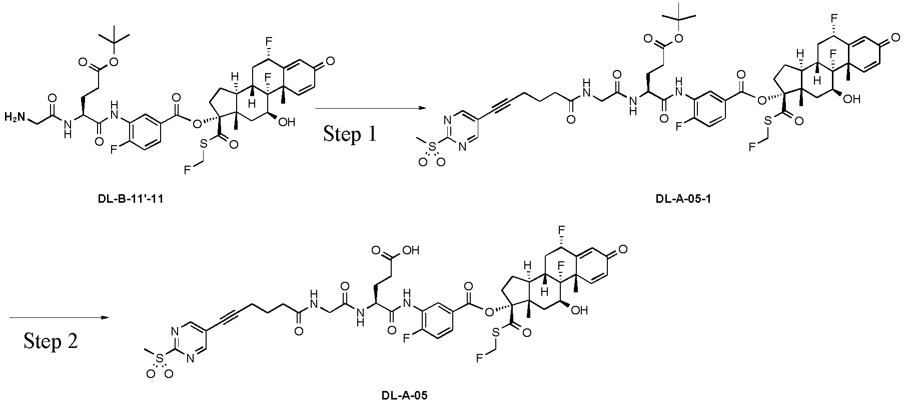

### Step 1:

(6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-2-(2-aminoacetamide)-5-(tert-butoxy)-5-oxopentanamide)-4-fluorobenzoate (67 mg, 82.73 µmol), (2,5-dioxopyrrolidin-1-yl) 6-(2-methylsulfonylpyrimidin-5-yl)hex-5-ynoate (33.25 mg, 91.00 µmol) and DIPEA(21.38 mg, 165.46 µmol) were successively added into DMF (2 mL), and the reaction solution was allowed to react for 1 h at 15°C under stirring after the addition was finished. The reaction solution was extracted with purified water (10 mL) and ethyl acetate (6 mL*2), separated, dried over anhydrous sodium sulfate (3 g) and filtered, and the filtrate was concentrated under reduced pressure and then purified via column chromatography (SiO₂, PE/EA = 100/1 to 1/4) so as to obtain (6S,8S,9R,108,118,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-5-(tert-butoxy)-2-(2-(6-(2-(methylsulfonyl)pyrimidin-5-yl)hex-5-ynylamido)acetamino)-5-oxopentanamide)-4-fluorobenzoate (64 mg, 54.33 µmol).

### Step 2:

(6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-5-(tert-butoxy)-2-(2-(6-(2-(methylsulfonyl)pyrimidin-5-yl)hex-5-ynylamido)acetamino)-5-oxopentanamide)-4-fluorobenzoate (64 mg, 54.33 µmol) and TFA (1 mL) were successively added into DCM (2 mL), and the reaction solution was allowed to react for 1.5 h at 16°C after the addition was finished. The reaction solution was purified via high performance liquid chromatography to obtain the title compound (42.00 mg, 41.41 µmol).

The purification method is as follows:
Chromatographic column: SunFire Prep C18 OBD 19*150 mm*5 µm
Mobile phase A: acetonitrile; mobile B: water (0.05% TFA)

| Time [min] | Mobile phase A [%] | Mobile phase B [%] | Flow rate [mL/min] |
|---|---|---|---|
| 0.00 | 30 | 70 | 28 |
| 2.00 | 30 | 70 | 28 |
| 20.00 | 90 | 10 | 28 |

The structure characterization data is as follows:
ESI-MS (m/z): 1004.3 (M+H)⁺.

### Preparation example 10: (6S,8S,9R,10S,11S,13 S, 14S, 17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-(34S, 37S)-37-(4-aminobutyl)-1-(3,5-bis(2-(methylsulfonyl)pyrimidin-5-yl)phenyl)-34-(hydroxymethyl)-1,29,32,35-tetraoxo-5,8,11,14,17,20,23,26-octaoxa-2,30,33,36-tetraazaoctatriacontane-38-amino-4-fluorobenzoate (DL-B-07')

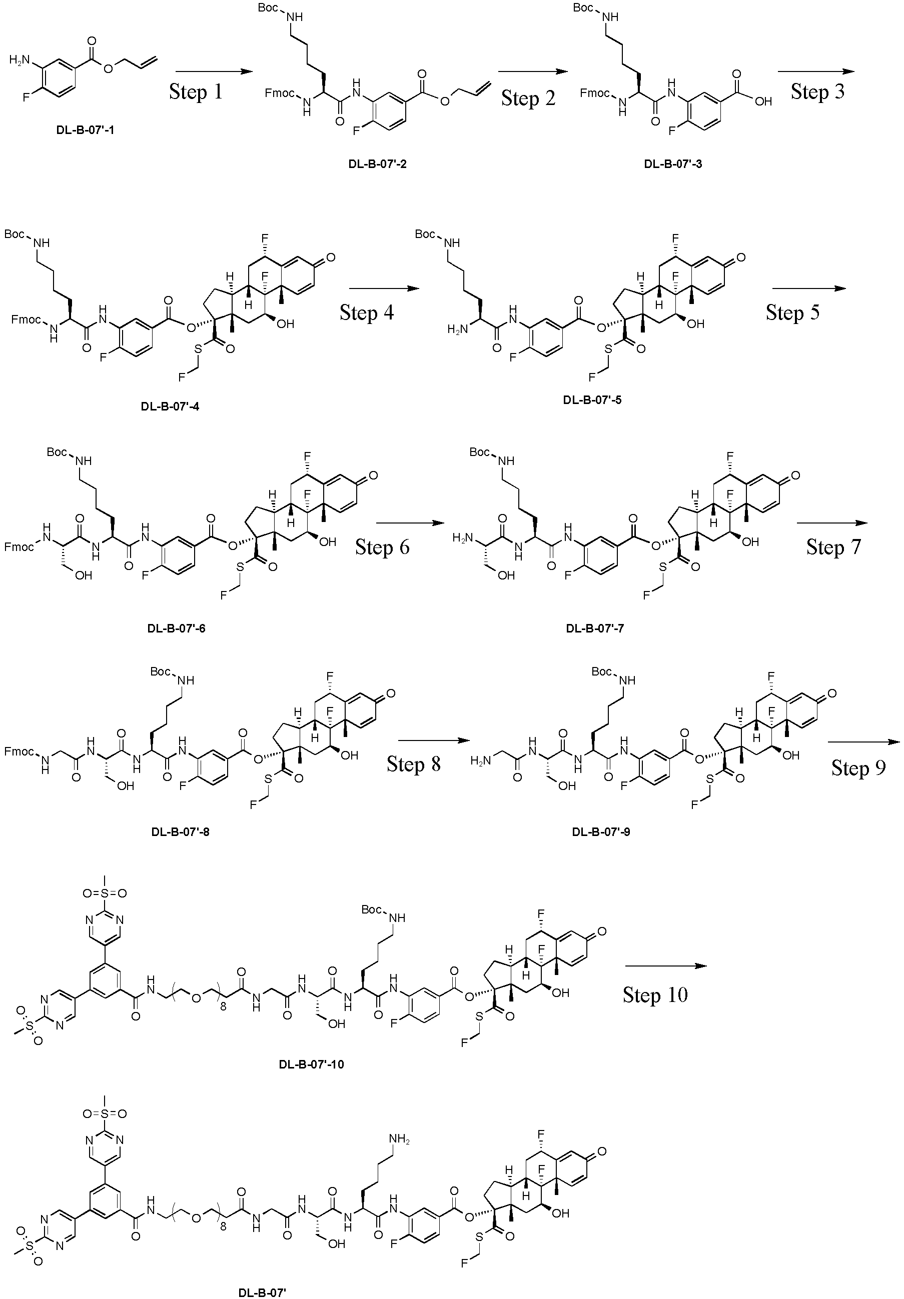

### Step 1:

Allyl 3-amino-4-fluorobenzoate (5.00 g, 25.6 mmol) and N2-(((9H-fluoren-9-yl))methoxy)carbonyl)-N6-(tert-butoxycarbonyl)-L-lysine (10.8 g, 23.0 mmol) were dissolved in dichoromethane (100 mL), followed by adding T3P (24.6 g, 38.7 mmol, 23.0 mL, 50.0% purity) and DIPEA (9.93 g, 76.8 mmol, 13.3 mL), and then the reaction solution was stirred for 2 h at 25°C. Water was added into the reaction solution, followed by extraction with dichoromethane for 3 times (100 mL x 3). The organic phases were combined, dried over anhydrous sodium sulfate, filtered and concentrated to obtain a crude product, which was purified via a silica gel column (SiO₂, CH₂Cl₂/MeOH = 100/1 to 10/1) and then concentrated again to obtain DL-B-07'-2 (10.5 g, 16.2 mmol).

The structure characterization data is as follows:
ESI-MS (m/z):668.4 [M+Na]⁺.

### Step 2:

DL-B-07'-2 (10.0 g, 15.4 mmol) was dissolved in THF (100 mL), followed by adding morpholine (2.70 g, 30.9 mmol, 2.73 mL) and Pd(PPh₃)₄ (894 mg, 774 µmol), and the reaction solution was stirred for 1 h at 25°C. The reaction solution was adjusted to pH 2-3 using 1 N diluted hydrochloric acid, then diluted with water (500 mL), followed by extraction with dichloromethane for 3 times (500 mL x 3). The organic phases were combined, dried over anhydrous sodium sulfate, filtered and concentrated to obtain a crude product, which was stirred for 2 h at 25°C in petroleum ether/ethyl acetate = 5/1, then filtered, and dried to obtain DL-B-07'-3 (8.00 g, 13.2 mmol).

The structure characterization data is as follows:
ESI-MS (m/z):623.2 [M+H]⁺.

### Step 3:

DL-B-07'-3 (5.00 g, 8.26 mmol) was dissolved in DMF (100 mL), followed by adding HATU (3.14 g, 8.26 mmol) and DIPEA (3.20 g, 24.7 mmol, 4.31 mL), and the reaction solution was stirred for 2 h at 25°C. Then (6S,8S,9R,10S,11S,13S,14S,17R)-6,9-difluoro-11,17-dihydroxy-10,13-dimethyl-3-oxo-6,7,8,11,12,14,15,16-octahydrocyclopenta[a]phenanthrene-17-carboxylic acid (3.29 g, 8.26 mmol) was added into the above reaction solution, and the reaction solution was stirred for additional 2 h. Fluoroiodomethane (1.32 g, 8.26 mmol) was then added into the reaction solution, and the reaction solution was stirred for additional 2 h. Water (200 mL) was added into the reaction solution, followed by extraction with ethyl acetate for 3 times (100 mL x 3). The organic phases were combined, dried over anhydrous sodium sulfate, filtered and concentrated to obtain a crude product, which was purified via silica gel column (SiO₂, CH₂Cl₂/MeOH = 100/1 to 10/1) and then concentrated again to obtain DL-B-07'-4 (2.60 g, 2.55 mmol).

The structure characterization data is as follows:
ESI-MS (m/z): 1018.1 [M+H]⁺.

### Step 4:

DL-B-07'-4 (2.50 g, 2.46 mmol) was dissolved in DMF (25.0 mL), followed by adding DBU (373 mg, 2.46 mmol, 370 µL), and then the reaction solution was stirred for 1 h at 25°C. The reaction solution was directly used in the next step without treatment.

### Step 5:

(((9H-fluoren-9-yl))methoxy)carbonyl)-L-serine (781 mg, 2.39 mmol), EDCI (686 mg, 3.58 mmol) and HOBt (483 mg, 3.58 mmol) were added into the reaction solution obtained in the previous step, and the reaction solution was stirred for 1 h at 25°C. Water (200 mL) was then added into the reaction solution, followed by extraction with ethyl acetate for 3 times (100 mL x 3). The organic phases were combined, dried over anhydrous sodium sulfate, filtered and concentrated to obtain a crude product, which was purified via silica gel column (SiO₂, CH₂Cl₂/MeOH = 100/1 to 10/1) and then concentrated again to obtain DL-B-07'-6 (2.00 g, 1.81 mmol).

The structure characterization data is as follows:
ESI-MS (m/z): 1105.5 [M+H]⁺.

### Step 6:

DL-B-07'-6 (2.00 g, 1.81 mmol) was dissolved in DMF (25.0 mL), followed by adding DBU (275 mg, 1.81 mmol, 272 µL), and then the reaction solution was stirred for 1 h at 25°C. The reaction solution was directly used in the next step without treatment.

### Step 7:

((9H-fluoren-9-yl)methoxy)carbonyl)glycine (538 mg, 1.81 mmol), EDCI (521 mg, 2.72 mmol) and HOBt (367 mg, 2.72 mmol) were added into the reaction solution obtained in the previous step, and then the reaction solution was stirred for 1 h at 25°C. Water (200 mL) was added into the reaction solution, followed by extraction with ethyl acetate for 3 times (50.0 mL x 3). The organic phases were combined, dried over anhydrous sodium sulfate, filtered and concentrated to obtain a crude product, which was purified via silica gel column (SiO₂, CH₂Cl₂/MeOH = 100/1 to 10/1) and then concentrated again to obtain DL-B-07'-8 (903 mg, 700 µmol).

The structure characterization data is as follows:
ESI-MS (m/z): 1162.5 [M+H]⁺.

### Step 8:

DL-B-07'-8 (500 mg, 0.43 mmol) was dissolved in DMF (5 mL), followed by adding diethylamine (125.8 mg, 1.72 mmol), and then the reaction solution was allowed to react for 1 h at 25°C. The reaction solution was concentrated to remove most of DMF so as to obtain a crude product, which was purified via a C18 reverse column (H₂O/CAN = 10-60%, 0.1% formic acid) and freeze-dried to obtain a formate of DL-B-07'-9 (325 mg).

The structure characterization data is as follows:
ESI-MS (m/z):940.4 [M+H]⁺.

### Step 9:

DL-B-11'-6 (27.4 mg, 0.032 mmol) was dissolved in DMF (0.5 mL), followed by successively adding HATU (23.14 mg, 0.061 mmol), DIPEA (11.8 mg, 0.091 mmol), and the formate of DL-B-07'-9 (30 mg, 0.030 mmol), and the reaction mixture was allowed to react for 1 h at 25°C. The reaction solution was directly purified via Pre-HPLC to obtain DL-B-07'-10 (21 mg).

The purification method is as follows:
Chromatographic column: SunFire Prep C18 OBD 19*150 mm*5 µm
Mobile phase A: acetonitrile; mobile phase B: water (0.05% formic acid

| Time [min] | Mobile phase A [%] | Mobile phase B [%] | Flow rate [mL/min] |
|---|---|---|---|
| 0.00 | 35 | 65 | 30 |
| 2.00 | 35 | 65 | 30 |
| 18.00 | 90 | 10 | 30 |

Retention time: 7.00-9.00 min

The structure characterization data is as follows:
ESI-MS (m/z): 1779.5 (M+H)⁺.

### Step 10:

Compound DL-B-07'-10 (21 mg, 0.012 mmol) was dissolved in dichloromethane ( 1 mL), followed by adding TFA (67.3 mg, 0.59 mmol), and the reaction mixture was allowed to react for 2 h at 25°C and concentrated to obtain a crude product, which was purified via Pre-HPLC to obtain a trifluoroacetate of DL-B-07' (14.0 mg).

The purification method is as follows:
Chromatographic column: SunFire Prep C18 OBD 19*150 mm*5 µm
Mobile phase A: acetonitrile; mobile phase B: water (0.05% formic acid)

| | Mobile phase A [%] | Mobile phase B [%] | Flow rate [mL/min] |
|---|---|---|---|
| 0.00 | 10 | 90 | 28 |
| 2.00 | 10 | 90 | 28 |
| 20.00 | 70 | 30 | 28 |

Retention time: 10.5-12.5 min.

The structure characterization data is as follows:
ESI-MS (m/z): 1679.6 (M+H)⁺.

### Preparation example 11: (6S,8S,9R,10S,11S,13 S, 14S, 17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-6-amino-2-(S)-3-hydroxy-2-(2-(methylsulfonyl)pyrimidin-5-yl)hex-5-ynylamido)acetamidopropionamido)-4-fluorobenzoate (DL-A-01)

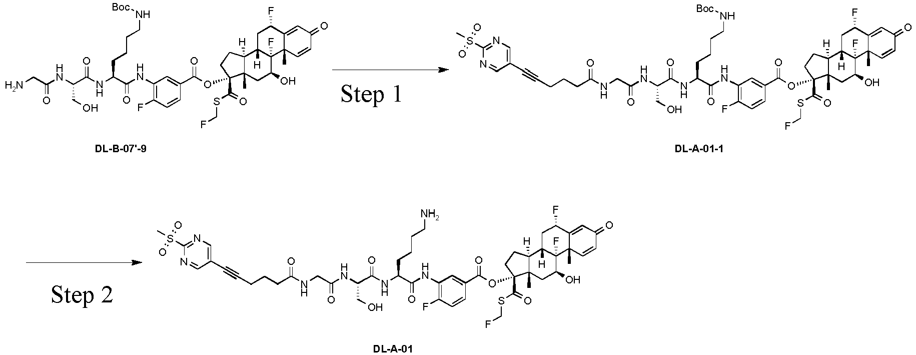

### Step 1:

The formate of DL-B-07'-9 (30.0 mg, 0.030 mmol), (2,5-dioxopyrrolidin-1-yl) 6-(2-methylsulfonylpyrimidin-5-yl)hex-5-ynoate (11.7 mg, 0.032 mmol) and DIPEA (11.8 mg, 0.091 mmol) were added into DMF (0.5 mL), and the reaction mixture was allowed to react for 2 h at 25°C. The reaction solution was directly purified via Pre-HPLC and then freeze-dried to obtain DL-A-01-1 (22.0 mg).

The purification method is as follows:
Chromatographic column: SunFire Prep C18 OBD 19*150 mm*5 µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 35 | 65 | 28 |
| 2.00 | 35 | 65 | 28 |
| 18.00 | 90 | 10 | 28 |

Retention time: 7.00-9.00 min

The structure characterization data is as follows:
ESI-MS (m/z): 1190.5 (M+H)⁺.

### Step 2:

DL-A-01-1 (22.0 mg, 0.018 mmol) was dissolved in DCM (1 mL), then TFA (105.4 mg, 0.924 mmol) was added, and the reaction solution was allowed to react for 1 h at 25°C. The reaction solution was directly concentrated to obtain a crude product, which was purified via Pre-HPLC to obtain a trifluoroacetate of DL-A-01 (14.6 mg).

The purification method is as follows:
Chromatographic column: SunFire Prep C18 OBD 19*150 mm*5 µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 10 | 90 | 28 |
| 2.00 | 10 | 90 | 28 |
| 20.00 | 70 | 30 | 28 |

Retention time: 10.50-12.50 min

The structure characterization data is as follows:
ESI-MS (m/z): 1090.4 (M+H)⁺.

### Preparation example 12: (6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((31S,34S)-1-(3,5-bis(2-(methylsulfonyl)pyrimidin-5-yl)phenyl)-31,34-dimethyl-1,2,32-trioxo-5,8,11,14,17,20,23,26-octaoxa-2,30,33-triazapentatriacontan-35-amino)-4-fluorobenzoate (DL-B-09')

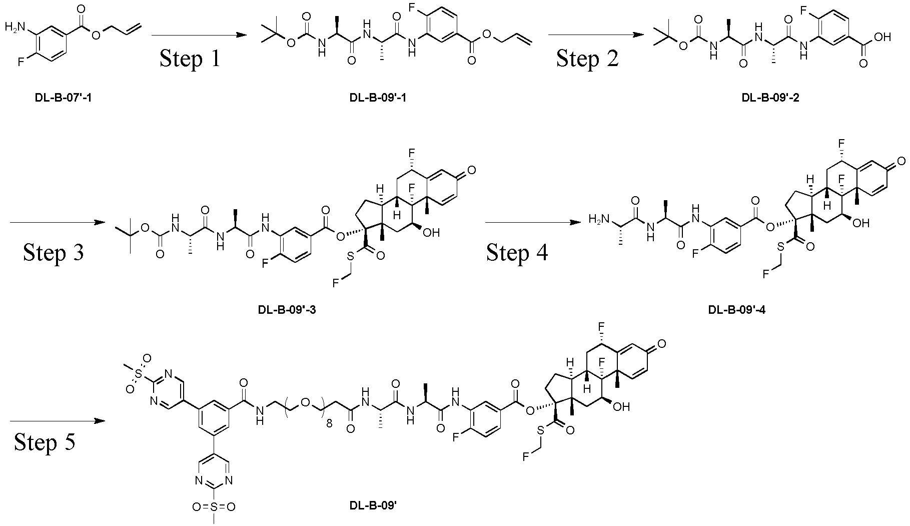

### Step 1:

Allyl 3-amino-4-fluorobenzoate (3.30 g, 16.91 mmol) and (tert-butoxycarbonyl)-L-alanyl-L-alanine (4.40 g, 16.91 mmol) were dissolved in DMF (40.00 mL), and then T3P (23.67g, 37.19 mmol, 50.0% purity) and pyridine (5.35 g, 67.63 mmol) were added. The reaction solution was stirred for 16 h at 25°C, and the reaction was monitored via LC-MS until it was completed. Then the reaction solution was extracted with water and ethyl acetate, and concentrated to obtain a crude product, which was purified via column chromatography (MeOH/DCM = 0-10%) to obtain DL-B-09'-1 (3.40 g, 7.77 mmol).

The structure thereof was characterized as follows:
ESI-MS (m/z):382.2 (M+H-56)⁺.

### Step 2:

Under the protection of nitrogen, DL-B-09'-1 (3.39 g, 7.75 mmol) was dissolved in THF (100.00 mL), and then tetra(triphenylphosphine)palladium (895.47 mg, 774.93 µmol) and morpholine (1.35 g, 15.50 mmol) were added. The reaction solution was stirred for 16 h at 25°C, and the reaction was monitored via LC-MS until it was completed. The system was adjusted to about pH 8 using sodium bicarbonate, and extracted with ethyl acetate (to remove impurities). The water phase was adjusted to about pH 3 using diluted hydrochloric acid to obtain a product, which was extracted with ethyl acetate, dried and concentrated to obtain DL-B-09'-2 (2.40 g, 6.04 mmol) as a white solid.

The structure thereof was characterized as follows:
ESI-MS (m/z): 342.2 (M+H-56)⁺.

### Step 3:

DL-B-09'-2 (300 mg, 754.91 µmol), HTUA (287.05 mg, 754.91 µmol) and DIPEA (146.35 mg, 1.13 mmol) were added into DMF (9.00 mL), and the reaction solution was stirred for 30 min at 25°C. DIPEA (146.35 mg, 1.13 mmol) and (6S,8S,9R,10S,11S,13S,14S,17R)-6,9-difluoro-11,17-dihydroxy-10,13-dimethyl-3-oxo-6,7,8,11,12,14,15,16-octahydrocyclopenta[a]phenanthrene-17-carboxylic acid (300.80 mg, 754.91 µmol) were added, and the reaction solution was allowed to react under stirring for 1 h. Fluoroiodomethane (482.93 mg, 3.02 mmol) was added, and the reaction solution was stirred for 2 h at 25°C. The reaction was monitored via LC-MS until it was completed. The reaction solution was extracted with water and ethyl acetate and then concentrated to obtain a crude product, which was purified via column chromatography (ACN/H₂O = 0-60%, 0.05% formic acid) and freeze-dried to obtain a crude product DL-B-09'-3 (199 mg).

The structure thereof was characterized as follows:
ESI-MS (m/z): 810.3 (M+H)⁺.

### Step 4:

DL-B-09'-3 (255 mg, 316.42 mmol) was added into DCM (8.00 mL), TFA (4.00 mL) was then added, and the reaction solution was stirred for 1 h at 25°C. The reaction was monitored via LC-MS until it was completed. The reaction system was concentrated to obtain a crude product, which was purified by high performance liquid chromatography to obtain DL-B-09'-4 (100 mg, 121.39 µmol) as a white solid.

The purification method is as follows:
Chromatographic column: Phenomenex C18 250mm×50mm×10µm
Mobile phase A: acetonitrile; mobile B: water (0.05% TFA)

| Time [min] | Mobile phase A [%] | Mobile phase B [%] | Flow rate [mL/min] |
|---|---|---|---|
| 0.00 | 15 | 85 | 30 |
| 2.00 | 15 | 85 | 30 |
| 18.00 | 90 | 10 | 30 |

The structure thereof was characterized as follows:
ESI-MS (m/z): 710.3 (M+H)⁺.

### Step 5:

DL-B-09'-4 (30 mg, 36.42 µmol), DL-B-11'-6 (31.24 mg, 36.42 µmol), HATU (27.70 mg, 72.84 µmol) and DIPEA (23.53 mg, 182.09 µmol) were added into DMF (2.00 mL), and the reaction solution was allowed to react for 1 h at 25°C. The reaction solution was filtered to obtain a crude product, which was purified via high performance liquid chromatography to prepare DL-B-09' (4 mg, 2.56 µmol).

The purification method is as follows:
Chromatographic column: Phenomenex C18 250mm×50mm×10µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 35 | 65 | 30 |
| 2.00 | 35 | 65 | 30 |
| 18.00 | 95 | 5 | 30 |

The structure thereof was characterized as follows:
ESI-MS (m/z):1549.5 (M+H)⁺.

### Preparation example 13: Preparation of (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 2-((31S,34R)-1-(3,5-bis(2-(methylsulfonyl)pyrimidin-5-yl)phenyl)-31,34-dimethyl-1,2,32-trioxo-5,8,11,14,17,20,23,26-octaoxa-2,30,33-triazapentatriacontan-35-amino)isonicotinate DL-B-17')

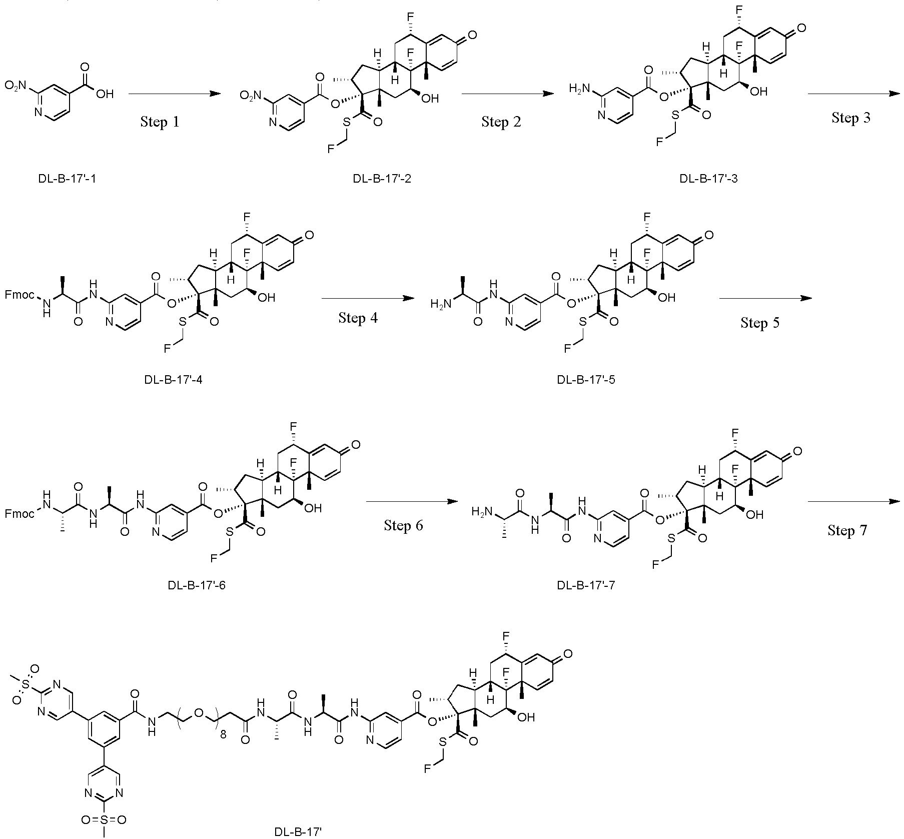

### Step 1:

2-nitroisonicotinic acid (500 mg, 2.97 mmol) and (6S,8S,9R,10S,11S,13S,14S,16R,17R)-6,9-difluoro-11,17-dihydroxy-10,13,16-trimethyl-3-oxo-6,7,8,9,10,11,12,13,14,15,16,17-dodecahydro-3H-cyclopenta[a]phenanthrene-17-thiocarboxylic acid (1.23g, 2.97 mmol) was dissolved in DMF (10.00 mL), and then T3P (10.00 g, 15.7 mmol, 50.0% purity) and DIPEA (1.15 g, 8.92 mmol) were added. After the reaction solution was stirred for 1 h at 25°C, fluoroiodomethane (475 mg, 2.97 mmol) was added into the reaction system, and the reaction solution was stirred for 1 h at 25°C. The reaction was monitored via LC-MS until it was completed, then the reaction solution was extracted with water and ethyl acetate and dried over sodium sulfate to obtain a crude product (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 2-nitroisonicotinate (2.1 g) as a yellow oil, which was directly used in the next step without purification.

The structure thereof was characterized as follows:
ESI-MS (m/z):595.2 (M+H)⁺.

### Step 2:

(6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 2-nitroisonicotinate (1.50 g, 2.40 mmol) was dissolved in ethanol (10.00 mL) and water (3.00 mL), and then iron powder (1.34 g, 23.9 mmol) and ammonium chloride (641 mg, 11.9 mmol) were added. After the system was heated to 80°C and stirred for 2 h, the reaction solution was filtered via a diatomite pad, and the filtrate was concentrated to obtain a crude product, which was purified via column chromatography (MeOH/MeOH = 0-5%) to obtain (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 2-aminoisonicotinate (1.02 g, 1.69 mmol).

The structure thereof was characterized as follows:
ESI-MS (m/z): 565.1 (M+H)⁺.

¹H NMR: (400 MHz, CD₃OD)*δ* 8.04 (d, *J* = 5.6 Hz, 1H), 7.29-7.39 (m, 1H), 7.00 (s, 1H), 6.92-6.96 (m, 1H), 6.36 (dd, *J* = 10.0, 1.6 Hz, 1H), 6.32 (s, 1H), 5.94-6.02 (m, 1H), 5.80-5.89 (m, 1H), 5.49-5.66 (m, 1H), 4.33-4.41 (m, 1H), 3.46-3.57 (m, 1H), 2.57-2.72 (m, 1H), 2.33-2.42 (m, 3H), 1.99-2.08 (m, 2H), 1.65-1.77 (m, 1H), 1.59 (s, 3H), 1.36-1.44 (m, 1H), 1.19 (s, 3H), 1.00 (d, *J* = 7.2 Hz, 3H).

### Step 3:

(6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 2-aminoisonicotinate (700 mg, 1.24 mmol), N-fluorenylmethoxycarbonyl-L-alanine (385 mg, 1.24 mmol) and pyridine (980 mg, 12.4 mmol) were added into dichloromethane (15.00 mL), and then phosphorus oxychloride (2.17 g, 14.1 mmol) was added. The system was stirred for 1 h at 0°C, then extracted with water and DCM. The organic phase was dried over sodium sulfate, filtered and concentrated to obtain a crude product (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 2-(((9H-fluoren-9-yl)methoxy)carbonyl)amino)propionamido)isonicotinate (1.5 g), which was directly used in the next step without purification.

The structure thereof was characterized as follows:
ESI-MS (m/z): 858.3 (M+H)⁺.

### Step 4:

(6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 2-(((9H-fluoren-9-yl)methoxy)carbonyl)amino)propionamido)isonicotinate (1.50 g, 1.75 mmol) was added into acetonitrile (10.00 mL), and then diethylamine (2.13 g, 29.1 mmol) was added. After being stirred for 1 h at 25°C, the reaction solution was directly concentrated to obtain a crude product (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 2-((S)-2-aminopropionamido)isonicotinate (1.5 g), which was directly used in the next step without purification.

The structure thereof was characterized as follows:
ESI-MS (m/z):636.1 (M+H)⁺.

### Step 5:

(6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 2-((S)-2-aminopropionamido)isonicotinate (1.30 g, 2.05 mmol) and N-fluorenylmethoxycarbonyl-L-alanine (636 mg, 2.05 mmol) were added into DMF (10.00 mL), and then DIPEA (792 mg, 6.14 mmol, 1.07 mL) and T3P (6.84 g, 10.7 mmol, 6.40 mL, 50% purity) were added. After being stirred for 1 h at 25°C, the reaction solution was extracted with water and ethyl acetate. The organic phase was dried over anhydrous sodium sulfate, filtered and concentrated to obtain a crude product, which was purified via column chromatography (petroleum ether/ethyl acetate = 0-40%) to obtain (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 2-(((S)-2-(((9H-fluoro-yl)methoxy)carbonylamino)propionamido)propanamide)isonicotinate (264 mg, 252 µmol).

The structure thereof was characterized as follows:
ESI-MS (m/z):929.5 (M+H)⁺.

### Step 6:

(6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 2-(((S)-2-(((9H-fluoro-yl)methoxy)carbonylamino)propionamido)propanamide)isonicotinate (50 mg, 53.82 µmol) was dissolved in DMF (2.00 mL), and then diethylamine (19.68 mg, 269.10 µmol) was added. After being stirred for 2 h at 25°C, the reaction solution was concentrated under reduced pressure to remove DMF and diethylamine so as to obtain a crude product (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 2-(((S)-2-aminopropionamido)propanamido)isonicotinate (38 mg, 53.77 µmol), which was directly used in the next step without purification.

The structure thereof was characterized as follows:
ESI-MS (m/z):707.3 (M+H)⁺.

### Step 7:

At 25°C, (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 2-(((S)-2-aminopropionamido)propanamido)isonicotinate (38 mg, 53.77 µmol), 1-(3,5-bis(2-(methylsulfonyl)pyrimidin-5-yl)phenyl)-1-oxo-5,8,11,14,17,20,23,26-octaoxa-2-azanonacosane-29-carboxylic acid (46.13 mg, 53.77 µmol), HATU (40.89 mg, 107.53 µmol) and DIPEA (34.74 mg, 268.83 µmol) were added into DMF (2.00 mL), and the reaction solution was allowed to react for 1 h at 25°C. The reaction solution was filtered to obtain a crude product, which was purified via high performance liquid chromatography to obtain the title compound (28 mg, 17.74 µmol).

The preparation and purification conditions are as follows:
Chromatographic column: Phenomenex C18 250mm×50mm×10µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 40 | 60 | 28 |
| 2.00 | 40 | 60 | 28 |
| 20.00 | 80 | 20 | 28 |

The structure thereof was characterized as follows:
ESI-MS (m/z):1547.6[M+H]⁺.

### Preparation example 14: (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 2-(((R)-2-(6-2-(methylsulfonyl)pyrimidin-5-yl)hex-5-ynylamido)propionamido)isonicotinate (DL-A-08)

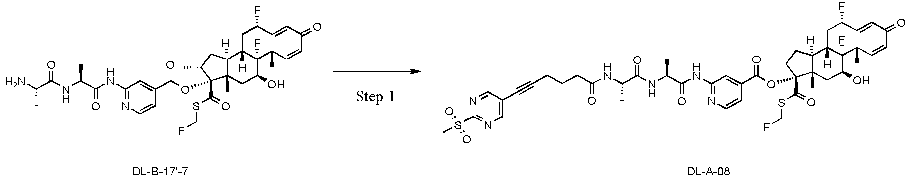

At 25°C, (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 2-(((S)-2-aminopropionamido)propionamidoisonicotinate (38 mg, 53.77 µmol), (2,5-dioxopyrrolidin-1-yl) 6-(2-methylsulfonylpyrimidin-5-yl)hex-5-ynoic acid (19.64 mg, 53.77 µmol) and DIPEA (13.90 mg, 107.53 µmol) were added into DMF (3.00 mL), and then the reaction solution was allowed to react for 1 h at 25°C. The reaction solution was filtered to obtain a crude product, which was purified via high performance liquid chromatography to obtain the title compound (21 mg, 21.72 µmol).

The preparation and purification conditions are as follows:
Chromatographic column: Phenomenex C18 250mm×50mm×10µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 30 | 70 | 30 |
| 2.00 | 30 | 70 | 30 |
| 18.00 | 90 | 10 | 30 |

The structure thereof was characterized as follows:
ESI-MS (m/z): 957.3[M+H]⁺.

### Preparation example 15: (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 3-hydroxybenzoate (B-1)

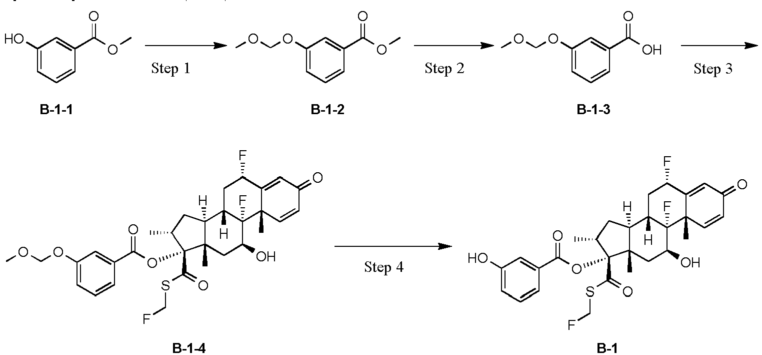

### Step 1:

Methyl 3-hydroxybenzoate (1.00 g, 6.57 mmol) was dissolved in tetrahydrofuran (10.0 mL) and then cooled to 0 °C, and the reaction solution was stirred for 30 min after NaH (526 mg, 13.1 mmol) was added. Then, chloromethyl methyl ether (1.26 g, 15.7 mmol) was added, and subsequently the reaction solution was heated to 25°C and stirred for 2 h. A saturated ammonium chloride aqueous solution (10.0 mL) was added into the reaction system, follwed by extraction with ethyl acetate for 3 times (10.0 mL x 3). The organic phases were combined and dried over anhydrous sodium sulfate, filtered and concentrated to obtain a crude product methyl 3-(methoxymethoxy)benzoate (1.40 g), which was directly used in the next step without purification.

The structure thereof was characterized as follows:
ESI-MS (m/z): 197.1 (M+H)⁺.

### Step 2:

Methyl 3-(methoxymethoxy)benzoate (700 mg, 3.57 mmol) was dissolved in tetrahydrofuran (3.00 mL), methanol (2.00 mL) and water (1.00 mL), then lithium hydroxide monohydrate (449 mg, 10.7 mmol) was added, and the reaction solution was stirred for 2 h at 25°C. Ethyl acetate (10.0 mL) and water (10.0 mL) were added into the reaction solution, and liquid separation was performed. The water phase was adjusted to pH 3 using 10% citric acid aqueous solution and then extracted with ethyl acetate for 3 times (10.0 mL x 3). The organic phases were combined and dried over anhydrous sodium sulfate, filtered and concentrated to obtain a crude product 3-(methoxymethoxy)benzoic acid (351 mg), which was directly used in the next step without purification.

The structure thereof was characterized as follows:
ESI-MS (m/z): 183.1 (M+H)⁺.

### Step 3:

3-(methoxymethoxy)benzoic acid (200 mg, 1.10 mmol) and HATU (417 mg, 1.10 mmol) were added into DMF (4.00 mL), and the reaction solution was stirred for 1 h at 25°C after DIPEA (568 mg, 4.39 mmol) was added. (6S,8S,9R,10S,11S,13S,14S,16R,17R)-6,9-difluoro-11,17-dihydroxy-10,13,16-trimethyl-3-oxo-6,7,8,9,10,11,12,13,14,15,16,17-dodecahydro-3H-cyclopenta[a]phenanthrene-17-S-carboxylic acid (435 mg, 1.10 mmol) was added, and the reaction solution was stirred for additional 2 h. Then, fluoroiodomethane (351 mg, 2.20 mmol) was added, and the reaction solution was stirred for additional 1 h. DCM (30.0 mL) and water (30.0 mL) were added into the reaction solution, and liquid separation was performed. The organic phase was dried over anhydrous sodium sulfate and then filtered and concentrated to obtain a crude product (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 3-(methoxymethoxy)benzoate (1.00 g), which was directly used in the next step without purification.

The structure thereof was characterized as follows:
ESI-MS (m/z): 609.2 (M+H)⁺.

### Step 4:

(6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 3-(methoxymethoxy)benzoate (1.00 g, 1.64 mmol) was dissolved in dichloromethane (15.0 mL), and the reaction solution was stirred for 1 h at 25°C after trifluoroacetic acid (7.68 g, 67.3 mmol) was added. The reaction solution was directly concentrated and then purified via high performance liquid chromatography to obtain the title compound (134 mg, 230 µmol).

The purification conditions are as follows:
Chromatographic column: Phenomenex luna C18 150 mm×25 mm×10 µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 42 | 58 | 24 |
| 10.00 | 52 | 48 | 24 |

The structure thereof was characterized as follows:
ESI-MS (m/z):565.3 [M+H]⁺.

¹H NMR (400 MHz, DMSO) δ 9.97 (s, 1H), 7.23-7.26 (m, 1H), 7.21-7.38 (m, 3H), 7.02-7.09 (m, 1H), 6.32 (dd, J = 10, 1.8 Hz, 1H), 6.14 (s, 1H), 6.02 (s, 1H), 5.90 (s, 1H), 5.74 (m, 1H), 4.22-4.35 (m, 1H), 3.36-3.46 (m, 1H), 2.56-2.64 (m, 1H), 2.20-2.31 (m, 3H), 1.87-2.00 (m, 2H), 1.49-1.62 (m, 4H), 1.29-1.38 (m, 1H), 1.07 (s, 3H), 0.92 (d, J = 7.2 Hz, 3H).

### Preparation example 16: (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-hydroxybenzoate (B-13)

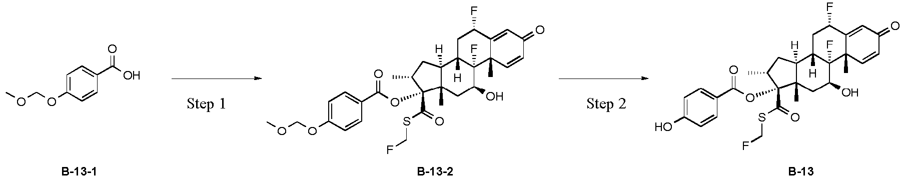

### Step 1:

At 25°C, 4-(methoxymethoxy)benzoic acid (146 mg, 800 µmol) and HATU (277 mg, 727 µmol) were dissolved in DMF (3.00 mL), DIPEA (376 mg, 2.91 mmol) was added, and the reaction solition was stirred for 1 h. (6S,8S,9R,10S,11S,13S,14S,16R,17R)-6,9-difluoro-11,17-dihydroxy-10,13,16-trimethyl-3-oxo-6,7,8,9,10,11,12,13,14,15,16,17-dodecahydro-3H-cyclopenta[a]phenanthrene-17-S-carboxylic acid (300 mg, 727 µmol) was added, and then the reaction solution was stirred for additional 6 h. Then, fluoroiodomethane (232 mg, 1.45 mmol) was added, and the reaction system was stirred for additional 1 h. Dichloromethane (30.0 mL) and water (30.0 mL) were added into the reaction solution, and then liquid separation was performed. The organic phase was dried over anhydrous sodium sulfate, filtered and concentrated to obtain a crude product (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-(methoxymethoxy)benzoate (1.02 g), which was directly used in the next step without purification.

The structure thereof was characterized as follows:
ESI-MS (m/z): 609.3 (M+H)⁺.

### Step 2:

(6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-(methoxymethoxy)benzoate (1.02 g, 1.68 mmol) was dissolved in dichloromethane (30.0 mL), and the reaction solution was stirred for 30 min at 25°C after trifluoroacetic acid (15.7 g, 137 mmol) was added. The reaction solution was directly concentrated to obtain a crude product, which was purified via high performance liquid chromatography to obtain the title compound (116 mg, 205 µmol).

The purification conditions are as follows:
Chromatographic column: Phenomenex luna C18 250mm×70mm×15 µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 40 | 60 | 28 |
| 24.00 | 70 | 30 | 28 |

The structure thereof was characterized as follows:
ESI-MS (m/z): 565.3 [M+H]⁺.

¹H NMR (400 MHz, DMSO) δ 10.50 (s, 1H), 7.72 (d, J = 8.8 Hz, 2H), 7.28 (d, J = 10 Hz, 1H), 6.82-6.94 (m, 2H), 6.33 (dd, J = 10.2, 1.8 Hz, 1H), 6.14 (s, 1H), 6.01 (s, 1H), 5.88 (s, 1H), 5.57-5.74 (m, 2H), 4.27-4.28 (m, 1H), 3.40-3.37 (m, 1H), 2.59-2.70 (m, 1H), 2.19-2.31 (m, 3H), 1.84-2.01 (m, 2H), 1.49-1.62 (m, 4H), 1.26-1.37 (m, 1H), 1.05 (s, 3H), 0.91 (d, J = 7.0 Hz, 3H).

### Preparation example 17:

### (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 2-hydroxybenzoate (B-25)

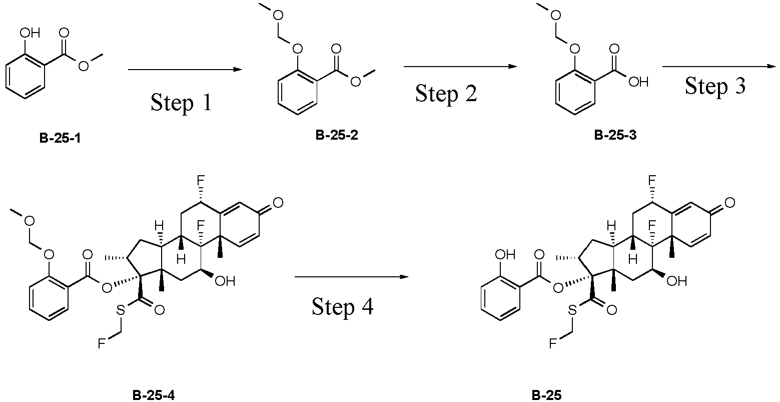

### Step 1:

Methyl 3-hydroxybenzoate (3.00 g, 19.7 mmol) was dissolved in tetrahydrofuran (30.0 mL) and then cooled to 0°C, NaH (1.58 g, 39.4 mmol, 60.0% purity) was added, and then the reaction solution was stirred for half an hour. Then, chloromethyl methyl ether (3.18 g, 39.4 mmol) was added, the reaction solution was heated to 25°C and then stirred for 2.5 h. Water (20.0 mL) was added into the reaction solution for quenching, and the reaction solution was extracted with ethyl acetate for 3 times (20.0 mL x 3). The organic phases were combined, dried over anhydrous sodium sulfate, filtered and concentrated to obtain a crude product methyl 2-(methoxymethoxy)benzoate (4.00 g), which was directly used in the next step without purification.

The structure thereof was characterized as follows:
ESI-MS (m/z): 197.1 (M+H)⁺.

### Step 2:

Methyl 2-(methoxymethoxy)benzoate (2.00 g, 10.2 mmol) was dissolved in ethanol (5.00 mL), and the reaction solution was stirred for 2 h at 25°C after lithium hydroxide monohydrate (1.28 g, 30.6 mmol) was added. Water (20.0 mL) and ethyl acetate (20.0 mL) were added, and the reaction system was separated. Then the water phase was adjusted to pH 3 using 1 N diluted hydrochloric acid, followed by extraction with ethyl acetate for 3 times (25.0 mL x 3). The organic phases were combined, dried over anhydrous sodium sulfate, adjusted to pH 9 using triethylamine, filtered and concentrated to obtain a crude product 2-(methoxymethoxy)benzoic acid (2.00 g), which was directly used in the next step without purification.

The structure thereof was characterized as follows:
ESI-MS (m/z): 183.1 (M+H)⁺.

### Step 3:

2-(methoxymethoxy)benzoic acid (350 mg, 1.92 mmol) was dissolved in DMF (1.00 mL), and the reaction solution was stirred for 2 h at 25°C after DIPEA (993 mg, 7.69 mmol) and HATU (657 mg, 1.73 mmol) were added. Then, (6S,8S,9R,10S,11S,13S,14S,16R,17R)-6,9-difluoro-11,17-dihydroxy-10,13,16-trimethyl-3-oxo-6,7,8,9,10,11,12,13,14,15,16,17-dodecahydro-3H-cyclopenta[a]phenanthrene-17-S-carboxylic acid (713 mg, 1.73 mmol) was added, and the reaction solution was heated to 30°C and stirred for 12 h. Finally, fluoroiodomethane (614 mg, 3.84 mmol) was added, and then the reaction solution was stirred for additional 1 h. After cooling to 20°C, water (20.0 mL) was added into the reaction solution for quenching, followed by extraction with dichloromethane for 3 times (20.0 mL x 3). The organic phases were combined and dried over anhydrous sodium sulfate, filtered and concentrated to obtain a crude product (6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 2-(methoxymethoxy)benzoate (1.30 g), which was directly used in the next step without purification.

The structure thereof was characterized as follows:
ESI-MS (m/z): 609.2 (M+H)⁺.

### Step 4:

(6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 2-(methoxymethoxy)benzoate (1.20 g, 1.97 mmol) was dissolved in dichloromethane (10.0 mmol), and the reaction solution was stirred for 2 h at 25°C after trifluoroacetic acid (3.07 g, 26.92 mmol) was added. The reaction solution was directly concentrated to obtain a crude product, which was purified via high performance liquid chromatography to obtain the title compound (168 mg, 297 µmol).

The purification conditions are as follows:
Chromatographic column: Phenomenex luna C18 150mm×25mm×10 µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 54 | 46 | 24 |
| 10.00 | 84 | 16 | 24 |

The structure thereof was characterized as follows:
ESI-MS (m/z): 565.3 [M+H]⁺.

¹H NMR (400 MHz, DMSO) δ 10.23 (s, 1H), 7.70-7.72 (m, 1H), 7.48-7.49 (m, 1H), 7.12-7.19 (m, 1H), 6.97 (d, J = 8.5 Hz, 1 H), 6.88-6.94 (m, 1H), 6.39-6.49 (m, 2H), 5.75-6.05 (m, 2H), 5.25-5.58 (m, 1H), 4.39-4.56 (m, 1H), 3.50-3.54 (m, 1H), 2.40-2.59 (m, 3H), 2.28-2.37 (m, 1H), 1.73-2.05 (m, 6H), 1.38-1.40 (m, 1H), 1.18 (s, 3H), 1.06 (d, J = 7.0 Hz, 3H).

### Preparation example 18: (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl benzoate (H-1)

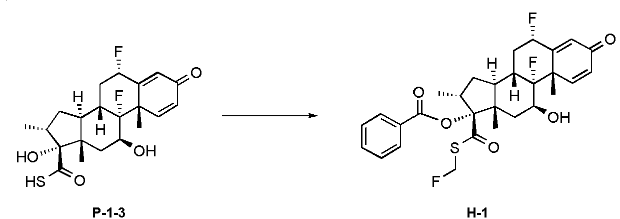

Benzoic acid (25 mg, 204.71 µmol) was dissolved in DMF (1 mL), then DIPEA (79.37 mg, 614.14 µmol) and HATU (65.13 mg, 171.29 µmol) were successively added, and the reaction solution was stirred for 1 h at 15°C. Then (6S,8S,9R,10S,11S,13S,14S,16R,17R)-6,9-difluoro-11,17-dihydroxy-10,13,16-trimethyl-3-oxo-6,7,8,9,10,11,12,13,14,15,16,17-dodecahydro-3H-cyclopenta[a]phenanthren-17-thiocarboxylic acid (84.44 mg, 204.71 µmol) was added, and the reaction solution was stirred for 4 h at 15°C after the addition was finished. Then fluoroiodomethane (130.96 mg, 818.86 µmol) was added, and the reaction solution was stirred for additional 1 h at 15°C. The reaction solution was directly treated via high performance liquid chromatography, freeze-dried, and then purified via preparative thin layer chromatography (developing agent: petroleum ether/ethyl acetate = 1/1, R_{f}= 0.4) to obtain the title compound (10 mg,16.41 µmol).

The purification conditions are as follows:
Chromatographic column: Waters Sunfire Prep C18 OBD 150 mm×19 mm×5 µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 40 | 60 | 28 |
| 20.00 | 80 | 20 | 28 |

The structure thereof was characterized as follows:
ESI-MS (m/z): 549.3[M+H]⁺, 1197.4[2M+H]⁺.

¹H NMR (400 MHz, CDCl3) δ 7.95 (dd, J = 8.4, 1.2 Hz, 2H), 7.60 (tt, J = 7.6, 1.2 Hz, 1H), 7.46 (t, J = 8.0 Hz, 2H), 7.14 (dd, J = 10.4, 1.2 Hz, 1H), 6.48 (s, 1H), 6.42 (dd, J = 10.0, 1.6 Hz, 1H), 5.91 (ddd, J = 50.0, 44.8, 9.2 Hz, 2H), 5.34-5.51 (m, 1H), 4.50 (dt, J = 8.4, 2.4 Hz, 1H), 3.48-3.55 (m, 1H), 2.59 (dt, J = 14.8, 3.2 Hz, 1H), 2.51-2.42 (m, 2H), 2.31-2.35 (m, 1H), 1.94-2.01 (m, 2H), 1.83 (t, J = 9.6 Hz, 1H), 1.55 (s, 3H), 1.38-1.44 (m, 1H), 1.18 (s, 3H), 1.04 (d, J = 7.2 Hz, 3H).

### Preparation example 19: (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-fluorobenzoate (H-5)

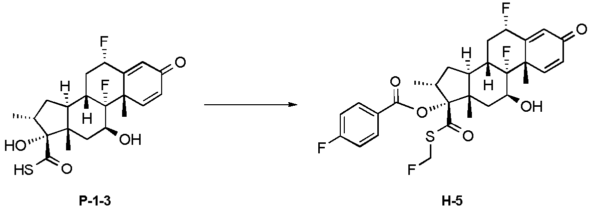

4-fluorobenzoic acid (20 mg, 142.74 µmol) was dissolved in DMF (1 mL), then DIPEA (55.34 mg, 428.23 µmol) and HATU (65.13 mg, 171.29 µmol) were successively added, and the reaction solution was stirred for 1 at 15°C. Then, (6S,8S,9R,10S,11S,13S,14S,16R,17R)-6,9-difluoro-11,17-dihydroxy-10,13,16-trimethyl-3-oxo-6,7,8,9,10,11,12,13,14,15,16,17-dodecahydro-3H-cyclopenta[a]phenanthren-17-thiocarboxylic acid (58.88 mg, 142.74 µmol) was added, and the reaction solution was stirred for 16 h at 15°C after the addtion was finished. Then, fluoroiodomethane (91.32 mg, 570.97 µmol) was added, and the reaction system was stirred for additional 1 h at 15°C. The reaction solution was directly treated via high performance liquid chromatography, freeze-dried, and then purified via preparative thin layer chromatography (developing agent: petroleum ether/ethyl acetate = 1/1, R_{f}=0.3) to obtain the title compound (13 mg, 21.80 µmol).

The purification conditions are as follows:
Chromatographic column: Waters Sunfire Prep C18 OBD 150 mm×19 mm×5 µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 40 | 60 | 28 |
| 20.00 | 80 | 20 | 28 |

The structure thereof was characterized as follows:
ESI-MS (m/z): 567.3[M+H]⁺, 1133.4[2M+H]⁺.

¹H NMR (400 MHz, CDCl₃) δ 7.90-8.00 (m, 2H), 7.09 - 7.17 (m, 3H), 6.48 (s, 1H), 6.43 (dd, J = 10.0, 1.6 Hz, 1H), 5.91 (ddd, J = 50.0, 36.4, 9.2 Hz, 2H), 5.32-5.52 (m, 1H), 4.50 (ddd, J = 6.4, 4.0, 2.8 Hz, 1H), 3.51 (ddd, J = 10.4, 6.8, 3.2 Hz, 1H), 2.27-2.61(m, 4H), 1.92-2.05 (m, 2H), 1.82 (t, J = 12.0 Hz, 1H), 1.55 (s, 3H), 1.36-1.45 (m, 1H), 1.18 (s, 3H), 1.04 (d, J = 7.2 Hz, 3H).

### Preparation example 20: (6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 2-hydroxyacetate (D-1)

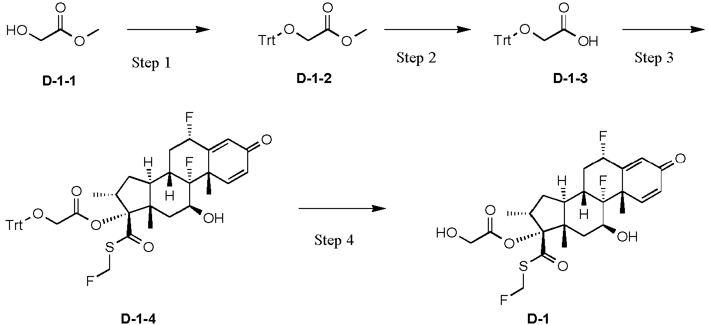

### Step 1: Synthesis of methyl 2-(triphenylmethoxy)acetate (D-1-2)

Methyl 2-hydroxyacetate (323 mg, 3.59 mmol, 276.88 µL) and triphenylmethyl chloride (1.00 g, 3.59 mmol) were dissolved in pyridine (4.00 mL), and then the reaction solution was heated to 80°C and allowed to react for 15 h. The reaction solution was extracted with dichloromethane for 3 times (50.0 mL x 3) after 1 N diluted hydrochloric acid (50.0 mL x 3) was added. The organic phases were combined, dried over anhydrous sodium sulfate, filtered and concentrated to obtain the title compound as a crude product (1.10 g, 3.31 mmol), which was directly used in the next step without purification.

The structure thereof was characterized as follows:
¹H NMR (400 MHz, DMSO) δ 7.33-7.41 (m, 12H), 7.27-7.31 (m, 3H), 3.69-3.75 (m, 2H), 3.53-3.58 (m, 3H).

### Step 2: Synthesis of 2-(triphenylmethoxy)acetic acid (D-1-3)

Methyl 2-(triphenylmethoxy)acetate (1.10 g, 3.31 mmol) was dissolved in a mixed solution of tetrahydrofuran (3.00 mL), methanol (3.00 mL) and water (3.00 mL), lithium hydroxide monohydrate (416 mg, 9.93 mmol) was added, and the reaction solution was heated to 40°C and allowed to react for 3 h. Water (50.0 mL) was added into the reaction solution, followed by extraction with dichloromethane (50.0 mL). The water phase was adjusted to pH 1 using 1 N diluted hydrochloric acid and then filtered. The filtrate was extracted with dichloromethane for 3 times (50.0 mL x 3). The organic phases were combined, dried over anhydrous sodium sulfate, filtered and concentrated to obtain the title compound as a crude product (700 mg, 2.20 mmol), which was directly used in the next step without purification.

The structure thereof was characterized as follows:
¹H NMR (400 MHz, DMSO) δ 7.42 (s, 6H), 7.33-7.38 (m, 6H), 7.27-7.30 (m, 3H), 3.54-3.59 (m, 2H).

### Step 3: synthesis of (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 2-(triphenylmethoxy)acetate (D-1-4)

2-(triphenylmethoxy)acetic acid (350 mg, 1.10 mmol) was dissolved in DMF (2.00 mL), HATU (418 mg, 1.10 mmol) and DIEA (426 mg, 3.30 mmol, 574 µL) were successively added, and the reaction solution was stirred for 1 h at 25°C. (6S,8S,9R,10S,11S,13S,14S,16R,17R)-6,9-difluoro-11,17-dihydroxy-10,13,16-trimethyl-3-oxo-6,7,8,9,10,11,12,13,14,15,16,17-dodecahydro-3H-cyclopenta[a]phenanthrene-17-thiocarboxylic acid (453 mg, 1.10 mmol) was added, and the reaction solution was stirred for additional 2 h. Then, fluoroiodomethane (175 mg, 1.10 mmol) was added, and the reaction solution was stirred for additional 0.4 h. The reaction solution was extracted with ethyl acetate for 3 times (50.0 mL x 3) after water (50.0 mL) was added. The organic phases were combined, dried over anhydrous sodium sulfate, filtered and concentrated to obtain the crude title compound (600 mg) as yellow oil.

3-(triphenylmethoxy)propionic acid (300 mg, 902 µmol) was dissolved in DMF (2.00 mL), then HATU (343 mg, 903 µmol) and DIPEA (349 mg, 2.71 mmol, 472 µL) were added, and the reaction solution was stirred for 1 h at 25°C. (6S,8S,9R,10S,11S,13S,14S,16R,17R)-6,9-difluoro-11,17-dihydroxy-10,13,16-trimethyl-3-oxo-6,7,8,9,10,11,12,13,14,15,16,17-dodecahydro-3H-cyclopenta[a]phenanthrene-17-thiocarboxylic acid (372 mg, 902 µmol) was added, and the reaction solution was stirred for additional 12 h. Then, fluoroiodomethane (144 mg, 903 µmol) was added, and the reaction solution was stirred for additional 1 h. Water (100 mL) was added into the reaction solution, followed by extraction with ethyl acetate for 3 times (80.0 mL x 3). The organic phases were combined, dried over anhydrous sodium sulfate, filtered and concentrated to obtain the title compound (450 mg), which was directly used in the next step without purification.

The structure thereof was characterized as follows:
ESI-MS (m/z): 745.3 (M+H)⁺.

### Step 4: Synthesis of (6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 2-hydroxyacetate (D-1)

(6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 2-(triphenylmethoxy)acetate (500 mg, 671 µmol) was dissolved in a mixed solvent of dichloromethane (5.00 mL) and methanol (1.00 mL), trifluoroacetic acid (1.54 g, 13.4 mmol, 1.00 mL) was added, and the reaction solution was stirred for 1 h at 25°C. The reaction solution was directly concentrated to obtain a crude product, which was purified via high performance liquid chromatography to obtain the title compound (141 mg, 259 µmol).
Chromatographic column: Phenomenex luna C18 200mm×40mm×10 µm
Mobile phase A: acetonitrile; mobile phase B: water (0.05% trifluoroacetic acid)

| Time [min] | Mobile phase A [%] | Mobile phase B [%] | Flow rate [mL/min] |
|---|---|---|---|
| 0.00 | 32 | 68 | 28 |
| 10.00 | 62 | 38 | 28 |

The structure thereof was characterized as follows:
ESI-MS (m/z):503.3 [M+H]⁺.

¹H NMR (400 MHz, CD₃OD) δ 7.27-7.39 (m, 1H), 6.31-6.40 (m, 1H), 6.22-6.31 (m, 1H), 5.90-5.99 (m, 1H), 5.78-5.89 (m, 1H), 5.44-5.63 (m, 1H), 4.25-4.32 (m, 1H), 4.10-4.23 (m, 2H), 3.38-3.46 (m, 1H), 2.49-2.69 (m, 1H), 2.29-2.37 (m, 1H), 2.17-2.27 (m, 2H), 1.90-2.03 (m, 2H), 1.59-1.69 (m, 1H), 1.57 (s, 3H), 1.31-1.40 (m, 1H), 1.10-1.18 (m, 3H), 0.97-1.05 (m, 3H).

### Preparation example 21: (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 2-hydroxypropionate (D-9)

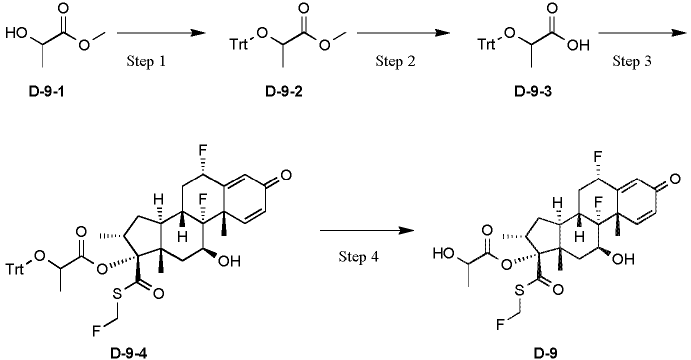

### Step 1: synthesis of methyl 2-(triphenylmethoxy)propionate (D-9-2)

Methyl 2-hydroxypropionate (373 mg, 3.59 mmol, 341 µL) and triphenylmethyl chloride (1.00 g, 3.59 mmol) were dissolved in pyridine (4.00 mL), and the reaction solution was heated to 80°C and allowed to react for 15 h. 1 N diluted hydrochloric acid (50.0 mL) was added into the reaction solution, followed by extraction with dichloromethane for 3 times (50.0 mL x 3). The organic phases were combined, washed with 1 N diluted hydrochloric acid twice (50.0 mL x 2), dried over anhydrous sodium sulfate, then filtered and concentrated to obtain the title compound as a crude product (1.00 g, 2.89 mmol), which was directly used in the next step without purification.

The structure thereof was characterized as follows:
¹H NMR (400 MHz, DMSO) δ 7.37-7.40 (m, 6H), 7.30-7.34 (m, 6H), 7.18-7.23 (m, 3H), 4.00-4.06 (m, 1H), 3.14-3.21 (m, 3H), 1.24-1.28 (m, 3H).

### Step 2: Synthesis of 2-(triphenylmethoxy)propionic acid (D-9-3)

Methyl 2-(triphenylmethoxy)propionate (1.00 g, 2.89 mmol) was dissolved in a mixed solvent of tetrahydrofuran (3.00 mL), methanol (3.00 mL) and water (3.00 mL), lithium hydroxide monohydrate (363 mg, 8.66 mmol) was added, and then the reaction solution was heated to 40°C and allowed to react for 3 h. The reaction solution was extracted with dichloromethane (50.0 mL) after water (50.0 mL) was added. The water phase was adjusted to pH 1 using 1 N diluted hydrochloric acid, and filtered. Then the filtrate was extracted with dichloromethane for 3 times (50.0 mL x 3). The organic phases were combined, dried over anhydrous sodium sulfate, filtered and concentrated to obtain the title compound (250 mg, 752 µmol), which was directly used in the next step without purification.

The structure thereof was characterized as follows:
¹H NMR (400 MHz, DMSO) δ 7.38-7.48 (m, 6H), 7.30-7.35 (m, 6H), 7.25-7.29 (m, 3H), 3.89-4.00 (m, 1H), 0.92-1.04 (m, 3H).

### Step 3: synthesis of (6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 2-(triphenylmethoxy)propionate (D-9-4)

2-(triphenylmethoxy)propionic acid (200 mg, 601 µmol) was dissolved in DMF (5.00 mL), HATU (228 mg, 601 µmol) and DIPEA (233 mg, 1.81 mmol, 314 µL) were added, and then the reaction solution was stirred for 1 h at 25°C. (6S,8S,9R,10S,11S,13S,14S,16R,17R)-6,9-difluoro-11,17-dihydroxy-10,13,16-trimethyl-3-oxo-6,7,8,9,10,11,12,13,14,15,16,17-dodecahydro-3H-cyclopenta[a]phenanthrene-17-thiocarboxylic acid (248 mg, 601 µmol) was added, and the reaction solution was stirred for additional 2 h. Then, fluoroiodomethane (96.2 mg, 601 µmol) was added, and the reaction solution was stirred for additional 0.5 h. Water (100 mL) was added into the reaction solution, followed by extraction with ethyl acetate for 3 times (50.0 mL x 3). The organic phases were combined, dried over anhydrous sodium sulfate, filtered and concentrated to obtain the title compound (500 g, crude), which was directly used in the next step without purification.

The structure thereof was characterized as follows:
ESI-MS (m/z): 759.3 (M+H)⁺.

### Step 4: Synthesis of (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 2-hydroxypropionate (D-9)

(6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 2-(triphenylmethoxy)propionate (300 mg, 395 µmol) was dissolved in a mixed solvent of dichloromethane (4.00 mL) and methanol (1.00 mL), trifluoroacetic acid (1.54 g, 13.4 mmol, 1.00 mL) was added, and then the reaction solution was stirred for 1 h at 25°C. The reaction solution was directly concentrated to obtain a crude product, which was purified via high performance liquid chromatography to obtain the title compound (102 mg, 189 µmol).
Chromatographic column: Phenomenex luna C18 200mm×40mm×10 µm
Mobile phase A: acetonitrile; mobile phase B: water (0.05% trifluoroacetic acid)

| Time [min] | Mobile phase A [%] | Mobile phase B [%] | Flow rate [mL/min] |
|---|---|---|---|
| 0.00 | 32 | 68 | 28 |
| 10.00 | 62 | 38 | 28 |

The structure thereof was characterized as follows:
ESI-MS (m/z): 517.1 [M+H]⁺.

¹H NMR (400 MHz, CD₃OD) δ 7.28-7.37 (m, 1H), 6.31-6.37 (m, 1H), 6.26-6.31 (m, 1H), 5.90-5.99 (m, 1H), 5.77-5.86 (m, 1H), 5.44-5.65 (m, 1H), 4.22-4.37 (m, 2H), 3.37-3.50 (m, 1H), 2.50-2.70 (m, 1H), 2.30-2.38 (m, 1H), 2.19-2.29 (m, 2H), 1.91-2.05 (m, 2H), 1.59-1.70 (m, 1H), 1.57 (s, 3H), 1.38-1.44 (m, 3H), 1.30-1.38 (m, 1H), 1.08-1.18 (m, 3H), 0.95-1.04 (m, 3H).

### Preparation example 22: (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 3-hydroxypropionate (D-5)

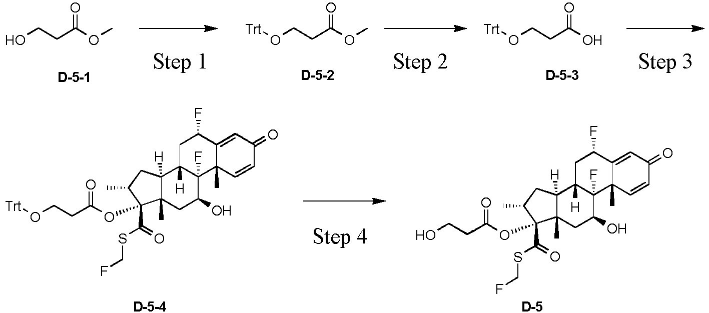

### Step 1: synthesis of methyl 3-(triphenylmethoxy)propionate (D-5-2)

Methyl 3-hydroxypropionate (375 mg, 3.60 mmol) was dissolved in pyridine (4.00 mL), triphenylmethyl chloride (1.00 g, 3.60 mmol) was added, and then the reaction solution was heated to 80°C and stirred for 12 h. 1 N diluted hydrochoric acid (60.0 mL) was added into the reaction solution, followed by extraction with dichloromethane for 3 times (50.0 mL x 3). The organic phases were combined, dried over anhydrous sodium sulfate, filtered and concentrated to obtain the title compound (1.10 g, 3.18 mmol).

The structure thereof was characterized as follows:
¹H NMR (400 MHz, DMSO) δ 7.21-7.47 (m, 19 H), 3.60 (s, 3H), 3.21 (t, J = 6.4 Hz, 2H), 2.58 (t, J = 6.4 Hz, 2H).

### Step 2: Synthesis of 3-(triphenylmethoxy)propionic acid (D-5-3)

Methyl 3-(triphenylmethoxy)propionate (1.10 g, 3.18 mmol) was added into a mixed solvent of methanol (4.00 mL), water (4.00 mL), and tetrahydrofuran (4.00 mL), lithium hydroxide monohydrate (399 mg, 9.53 mmol) was added, and then the reaction solution was stirred at 25 °C for 2 h. Water (60.0 mL) was added, and then the reaction solution was adjusted to pH 3-4 using 1 N diluted hydrochloric acid, followed by extraction with dichloromethane for 3 times (80.0 mL x 3). The organic phases were combined, dried over anhydrous sodium sulfate, filtered and concentrated to obtain the title compound (980 mg, 2.95 mmol).

The structure thereof was characterized as follows:
¹H NMR (400 MHz, DMSO) δ 7.19-7.42 (m, 17H), 3.19 (t, J = 6.4 Hz, 2H), 2.44-2.49 (m, 2H).

### Step 3: synthesis of (6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 3-(triphenylmethoxy)propionate (D-5-4)

3-(triphenylmethoxy)propionic acid (300 mg, 902 µmol) was dissolved in DMF (2.00 mL), then HATU (343 mg, 903 µmol) and DIPEA (349 mg, 2.71 mmol, 472 µL) were added, and the reaction solution was stirred for 1 h at 25°C. (6S,8S,9R,10S,11S,13S,14S,16R,17R)-6,9-difluoro-11,17-dihydroxy-10,13,16-trimethyl-3-oxo-6,7,8,9,10,11,12,13,14,15,16,17-dodecahydro-3H-cyclopenta[a]phenanthrene-17-thiocarboxylic acid (372 mg, 902 µmol) was added, and the reaction solution was stirred for additional 12 h. Then, fluoroiodomethane (144 mg, 903 µmol) was added, and the reaction solution was stirred for additional 1 h. Water (100 mL) was added into the reaction solution, followed by extraction with ethyl acetate for 3 times (80.0 mL x 3). The organic phases were combined, dried over anhydrous sodium sulfate, filtered and concentrated to obtain the title compound (450 mg, crude).

The structure thereof was characterized as follows:
ESI-MS (m/z): 781.3 (M+Na)⁺.

### Step 4: Synthesis of (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 3-hydroxypropionate (D-5)

(6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 3-(triphenylmethoxy)propionate (450 mg, 592 µmol) was dissolved in dichloromethane (5.00 mL), TFA (1.68 g, 14.8 mmol, 1.10 mL) was added, and then the reaction solution was stirred for 2 h at 25°C. The reaction solution was directly concentrated to obtain a crude product, which was purified via high performance liquid chromatography to obtain the title compound (152.45 mg, 295.12 µm).
Chromatographic column: Phenomenex luna C18 150 mm×25 mm×5 µm
Mobile phase A: acetonitrile; mobile phase B: water (0.05% trifluoroacetic acid)

| Time [min] | Mobile phase A [%] | Mobile phase B [%] | Flow rate [mL/min] |
|---|---|---|---|
| 0.00 | 37 | 63 | 24 |
| 3.00 | 67 | 33 | 24 |

The structure thereof was characterized as follows:
ESI-MS (m/z):517.2 [M+H]⁺.

¹H NMR (400 MHz, DMSO) δ 7.25 (d, J = 10.0 Hz, 1H), 6.30 (dd, J = 10.0, 1.6 Hz, 1H), 6.11 (s, 1H), 5.99 (s, 1H), 5.86 (s, 1H), 5.52-5.75 (m, 2H), 4.16-4.26 (m, 1H), 3.61 (t, J = 6.4 Hz, 2H), 3.22-3.32 (m, 1H), 2.51-2.67 (m, 1H), 2.45-2.48 (m, 2H), 2.23-2.26 (m, 1H), 2.05-2.19 (m, 2H), 1.79-1.93 (m, 2H), 1.50-1.54 (m, 1H), 1.48 (s, 3H), 1.20-1.30 (m, 1H), 0.99 (s, 3H), 0.91 (d, J = 7.2 Hz, 3H).

### Preparation example 23: (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 3-hydroxybutyrate (D-11)

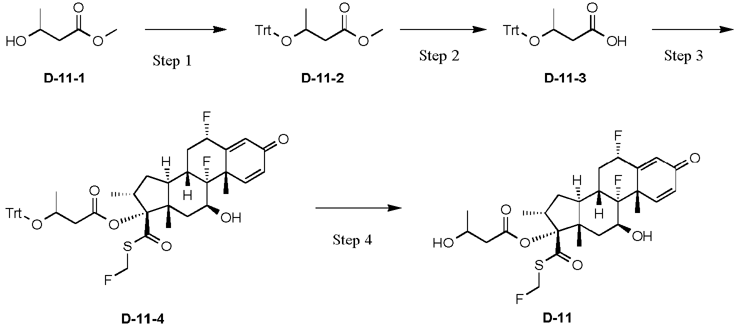

### Step 1: synthesis of methyl 3-(triphenylmethoxy)butyrate (D-11-2)

Methyl 3-hydroxybutyrate (211 mg, 1.79 mmol) and triphenylmethyl chloride (500 mg, 1.79 mmol) were dissolved in pyridine (3.00 mL), and then the reaction solution was heated to 80°C and allowed to react for 10 h. 1 N diluted hydrochloric acid (50.0 mL x 3 ) was added into the reaction solution, followed by extraction with dichloromethane for 3 times (50.0 mL x 3). The organic phases were combined, dried over anhydrous sodium sulfate, filtered and concentrated to obtain the title compound (520 mg, 1.44 mmol).

The structure thereof was characterized as follows:
¹H NMR (400 MHz, DMSO) δ 7.41 (d, J = 7.2 Hz, 6H), 7.33 (t, J = 7.2 Hz, 6H), 7.26-7.28 (m, 3H), 3.76-3.84 (m, 1H), 3.48 (s, 3H), 2.21 (dd, J = 15.2, 7.6 Hz, 1H), 2.02-2.11 (m, 1H), 0.88 (d, J = 6.0 Hz, 3H).

### Step 2: Synthesis of 3-(triphenylmethoxy)butyric acid (D-11-3)

Methyl 3-(triphenylmethoxy)butyrate (500 mg, 1.39 mmol) was dissolved in a mixed solvent of tetrahydrofuran (1.50 mL), methanol (1.50 mL) and water (1.50 mL), lithium hydroxide monohydrate (174 mg, 4.16 mmol) was added, and then the reaction solution was heated to 40°C and allowed to react for 2 h. The reaction solution was extracted with dichloromethane (50.0 mL) after water (50.0 mL) was added. The water phase was adjusted to pH 1 using 1 N diluted hydrochloric acid, and filtered. The filtrate was extracted with dichloromethane for 3 times (50.0 mL x 3). The organic phases were combined, dried over anhydrous sodium sulfate, filtered and concentrated to obtain the title compound (250 mg, 721 µmol), which was directly used in the next step without purification.

The structure thereof was characterized as follows:
¹H NMR (400 MHz, DMSO) δ 7.39-7.45 (m, 6H), 7.29-7.36 (m, 6H), 7.22-7.28 (m, 3H), 3.79-3.85 (m, 1H), 1.98-2.10 (m, 1H), 1.86-1.95 (m, 1H), 0.77-0.87 (m, 3H).

### Step 3: synthesis of (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 3-(triphenylmethoxy)butanoate (D-11-4)

3-(triphenylmethoxy)butyric acid (190 mg, 548 µmol) was dissolved in DMF (6.00 mL), HATU (208 mg, 548 µmol) and DIPEA (212 mg, 1.65 mmol, 286 µL) were added, and then the reaction solution was stirred for 1 h at 25°C. (6S,8S,9R,10S,11S,13S,14S,16R,17R)-6, 9-difluoro-11,17-dihydroxy-10,13,16-trimethyl-3-oxo-6,7,8,9,10,11,12,13,14,15,16,17-dodecahydro-3H-cyclopenta[a]phenanthren-17-thiocarboxylic acid (226 mg, 548 µmol) was added, and the reaction solution was stirred for additional 2 h. Then fluoroiodomethane (87.7 mg, 548 µmol) was added, and then the reaction solution was stirred for additional 0.5 h. Water (100 mL) was added into the reaction solution, followed by extraction with ethyl acetate for 3 times (50.0 mL x 3). The organic phases were combined, dried over anhydrous sodium sulfate, filtered and concentrated to obtain the title compound as a crude product (600 mg, crude), which was directly used in the next step without purification.

The structure thereof was characterized as follows:
ESI-MS (m/z): 773.3 (M+H)⁺.

### Step 4: Synthesis of (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 3-hydroxybutyrate (D-11)

(6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 3-(triphenylmethoxy)butanoate (550 mg, 711 µmol) was dissolved in dichloromethane (5.00 mL) and methanol (1.00 mL), trifluoroacetic acid (1.54 g, 13.4 mmol, 1.00 mL) was added, and then the reaction solution was stirred for 0.5 h at 25°C. The reaction solution was directly concentrated to obtain a crude product, which was purified via high performance liquid chromatography to obtain the title compound (76.0 mg, 142 µmol).
Chromatographic column: Welch Xtimate C18 150 mm×25 mm×5 µm
Mobile phase A: acetonitrile; mobile phase B: water (0.05% trifluoroacetic acid)

| Time [min] | Mobile phase A [%] | Mobile phase B [%] | Flow rate [mL/min] |
|---|---|---|---|
| 0.00 | 43 | 57 | 24 |
| 3.00 | 63 | 33 | 24 |

The structure thereof was characterized as follows:
ESI-MS (m/z): 531.2 [M+H]⁺.

¹H NMR (400 MHz, CD₃OD) δ 7.26-7.37 (m, 1H), 6.32-6.36 (m, 1H), 6.28-6.31 (m, 1H), 5.89-5.98 (m, 1H), 5.77-5.86 (m, 1H), 5.45-5.64 (m, 1H), 4.25-4.32 (m, 1H), 4.10-4.19 (m, 1H), 3.35-3.43 (m, 1H), 2.52-2.66 (m, 1H), 2.45 (s, 2H), 2.29-2.36 (m, 1H), 2.20-2.29 (m, 2H), 1.89-2.01 (m, 2H), 1.59-1.71 (m, 1H), 1.54-1.59 (m, 3H), 1.30-1.41 (m, 1H), 1.17-1.25 (m, 3H), 1.10-1.15 (m, 3H), 1.02 (d, J = 7.2 Hz, 3H).

### Preparation example 24: (6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (S)-3-hydroxybutyrate (D-15)

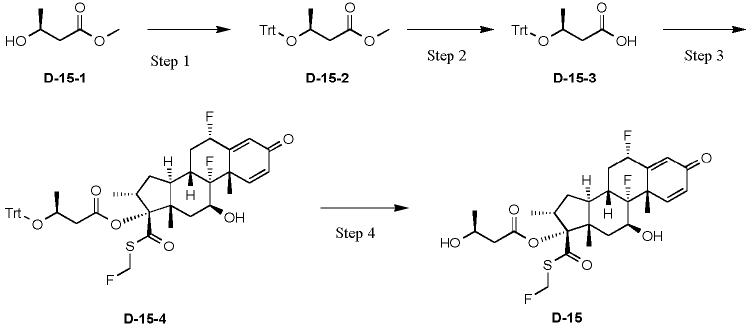

### Step 1: synthesis of methyl (S)-3-(triphenylmethyloxy)butyrate (D-15-2)

Methyl (S)-3-hydroxybutyrate (300 mg, 2.54 mmol) was added into a single necked flask, pyridine (3 mL) and triphenylmethyl chloride (849 mg, 3.05 mmol) were then added, and the reaction solution was heated to 80°C, allowed to react for 15 h under stirring, and cooled to room temperature. Then water (15 mL) was added into the reaction solution, followed by extraction with ethyl acetate twice (8 mL x 2). The organic phases were combined, dried over anhydrous sodium sulfate, filtered and concentrated to obtain a crude product, which was purified via flash column chromatography (petroleum ether/ethyl acetate = 100/1 to 3/1) to obtain the title compound (460 mg, 1.15 mmol).

The structure thereof was characterized as follows:
¹H NMR (400 MHz, DMSO) δ 7.48 - 7.41 (m, 6H), 7.36 - 7.26 (m, 6H), 7.26 - 7.21 (m, 3H), 3.86 - 3.80 (m, 1H), 3.46 (s, 3H), 2.20 (dd, J = 14.8, 7.6 Hz, 1H), 2.06 (dd, J = 14.8, 4.8 Hz, 1H), 0.89 (d, J = 6.0 Hz, 3H).

### Step 2: Synthesis of (S)-3-(triphenylmethyloxy)butyric acid (D-15-3)

Methyl (S)-3-(triphenylmethyloxy)butyrate (460 mg, 1.15 mmol) was added into a mixed solvent of methanol (1 mL), tetrahydrofuran (1 mL) and water (0.5 mL), lithium hydroxide (36.9 mg, 1.54 mmol) was added, and then the reaction solution was stirred for 2 h at 25°C. Water and 1 N diluted hydrochloric acid (2 mL) were added into the reaction solution, followed by extraction with dichloromethane for 3 times (10 mL x 3). The organic phases were combined, dried over anhydrous sodium sulfate, filtered and concentrated to obtain the title compound (142 mg, 0.41 mmol).

### Step 3: synthesis of (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (S)-3-(triphenylmethoxy)butanoate (D-15-4)

(S)-3-(triphenylmethyloxy)butyric acid (50 mg, 0.14 mmol) and HATU (60.4 mg, 0.16 mmol) were added into DMF (2.0 mL), then DIPEA (56.0 mg, 0.43 mmol) was added, and the reaction solution was allowed to react for 2 h at 25°C. (6S,8S,9R,10S,11S,13S,14S,16R,17R)-6,9-difluoro-11,17-dihydroxy-10,13,16-trimethyl-3-oxo-6,7,8,9,10,11,12,13,14,15,16,17-dodecahydro-3H-cyclopenta[a]phenanthrene-17-thiocarboxylic acid (63.2 mg, 0.16 mmol) was added, and then the reaction solution was stirred for additional 2 h. Then, fluoroiodomethane (34.7 mg, 0.22 mmol) was added, and the reaction solution continued to react for 1 h at 25°C. Water (10 mL) was added into the reaction solution, followed by extraction with ethyl acetate for 3 times (10 mL x 3). The organic phases were combined, dried over anhydrous sodium sulfate, filtered and concentrated to obtain the title compound as a crude product (60 mg), which was directly used in the next step without purification.

The structure thereof was characterized as follows:
ESI-MS (m/z): 773.3 (M+H)⁺.

### Step 4: Synthesis of (6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (S)-3-hydroxybutyrate (D-15)

(6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (S)-3-(triphenylmethoxy)butanoate (32 mg, 0.04 mmol) was added into dichloromethane (1 mL), and then acetic acid (0.5 mL) was added. After being stirred for 3 h at 40°C, the reaction solution was concentrated to obtain a crude product, which was purified via high performance liquid chromatography to obtain the title compound (4.2 mg, 0.008 mmol).
Chromatographic column: SunFire prep C18 OBD 150 mm×19 mm×5 µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 20 | 80 | 32 |
| 4.00 | 20 | 80 | 32 |
| 24.00 | 90 | 10 | 32 |

The structure thereof was characterized as follows:
ESI-MS (m/z): 574.1[M+Na]⁺.

¹H NMR (400 MHz, DMSO) δ 7.25 (d, J = 10.2 Hz, 1H), 6.30 (d, J = 10.2, 1H), 6.11 (s, 1H), 5.99 (s, 1H), 5.87 (s, 1H), 5.74 - 5.54 (m, 1H), 5.58 (d, J = 4.0 Hz, 1H), 4.74 (d, J = 5.2 Hz, 1H), 4.20 (s, 1H), 3.98 - 3.90 (m, 1H), 3.30 - 3.25 (m, 1H), 2.47 - 2.36 (m, 3H), 2.28 - 2.21 (m, 1H), 2.17 - 2.07 (m, 2H), 1.92 - 1.80 (m, 2H), 1.58 - 1.42 (m, 1H), 1.48 (s, 3H), 1.31 - 1.15 (m, 2H), 1.09 (d, J = 6.2 Hz, 3H), 0.99 (s, 2H), 0.91 (d, J = 7.1 Hz, 3H).

### Preparation example 25: (6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (R)-3-hydroxybutyrate (D-19)

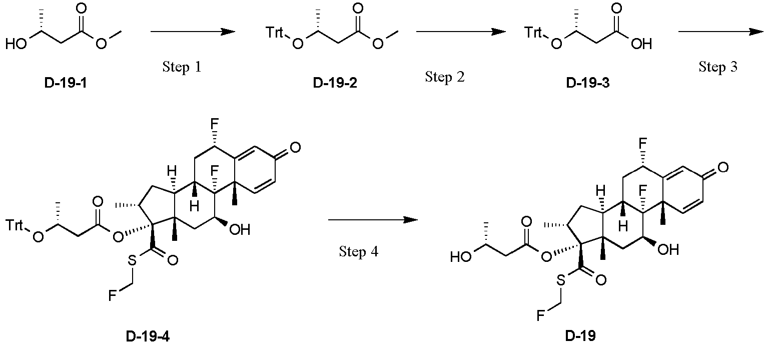

### Step 1: Synthesis of methyl (R)-3-(triphenylmethoxy)butyrate (D-19-2)

Methyl (R)-3-hydroxybutyrate (300 mg, 2.54 mmol) was added into a single necked flask, pyridine (3 mL) and triphenylmethyl chloride (849 mg, 3.05 mmol) were added, and the reaction solution was heated to 80°C, allowed to react for 15 h under stirring, and then cooled to room temperature. Water (15 mL) was added into the reaction solution, followed by extraction with ethyl acetate twice (8 mL x 2). The organic phases were combined, dried over anhydrous sodium sulfate, filtered and concentrated to obtain a crude product, which was purified via flash column chromatography (SiO₂, petroleum ether/ethyl acetate = 100/1 to 5/1) to obtain the title compound (278 mg, 0.77 mmol).

The structure thereof was characterized as follows:
¹H NMR (400 MHz, DMSO) δ 7.47 - 7.41 (m, 6H), 7.37 - 7.28 (m, 6H), 7.28 - 7.23 (m, 3H), 3.88 - 3.80 (m, 1H), 3.48 (s, 3H), 2.22 (dd, J = 14.8, 7.6 Hz, 1H), 2.07 (dd, J = 14.8, 4.8 Hz, 1H), 0.89 (d, J = 6.0 Hz, 3H).

### Step 2: Synthesis of (R)-3-(triphenylmethoxy)butyric acid (D-19-3)

Methyl (R)-3-(triphenylmethoxy)butyrate (278 mg, 0.77 mmol) was added into a mixed solvent of methanol (1 mL), tetrahydrofuran (1 mL) and water (0.5 mL), then lithium hydroxide (36.9 mg, 1.54 mmol) was added, and the reaction solution was stirred for 2 h at 25°C. Water and 1 N diluted hydrochloric acid (2 mL) were added into the reaction solution, followed by extraction with dichloromethane for 3 times (10 mL x 3). The organic phases were combined, dried over anhydrous sodium sulfate, filtered and concentrated to obtain the title compound (142 mg, 0.41 mmol).

### Step 3: synthesis of (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (R)-3-(triphenylmethoxy)butanoate (D-19-4)

(R)-3-(triphenylmethoxy)butyric acid (50 mg, 0.14 mmol) and HATU (60.4 mg, 0.16 mmol) were added into DMF (2.0 mL), and then DIPEA (56.0 mg, 0.43 mmol) was added. After the reaction solution was allowed to react for 2 h at 25°C, (6S,8S,9R,10S,11S,13S,14S, 16R,17R)-6,9-difluoro-11,17-dihydroxy-10,13,16-trimethyl-3-oxo-6,7,8,9,10,11,12,13,14,15,16,17-dodecahydro-3H-cyclopenta[a]phenanthrene-17-thiocarboxylic acid (63.2 mg, 0.16 mmol) was added, and then the reaction solution was stirred for additional 2 h. Then, fluoroiodomethane (34.7 mg, 0.22 mmol) was added, and the reaction solution continued to react for 1 h at 25°C. Water (10 mL) was added into the reaction solution, followed by extraction with ethyl acetate for 3 times (10 mL x 3). The organic phases were combined, dried over anhydrous sodium sulfate, filtered and concentrated to obtain the title compound as a crude product, which was purified via flash column chromatography (C18, water/acetonitrile = 0.8) to obtain the title compound (32 mg, 0.04 mmol).

The structure thereof was characterized as follows:
ESI-MS (m/z): 773.3 (M+H)⁺.

### Step 4: Synthesis of (6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (R)-3-hydroxybutyrate (D-19)

(6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (R)-3-(triphenylmethoxy)butanoate (32 mg, 0.04 mmol) was added into dichloromethane (1 mL), and then acetic acid (0.5 mL) was added. After being stirred for 3 h at 40°C, the reaction solution was concentrated to obtain a crude product, which was purified via high performance liquid chromatography to obtain the title compound (5.2 mg, 0.01 mmol).
Chromatographic column: SunFire prep C18 OBD 150 mm×19 mm×5 µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 20 | 80 | 32 |
| 4.00 | 20 | 80 | 32 |
| 24.00 | 90 | 10 | 32 |

The structure thereof was characterized as follows:
ESI-MS (m/z): 531.2[M+H]⁺.

¹H NMR (400 MHz, CD₃OD) δ 7.33 (d, J = 10.0 Hz, 1H), 6.37 - 6.28 (m, 2H), 5.94 - 5.90 (m, 1H), 5.85 - 5.76 (m, 1H), 5.65 - 5.45 (m, 1H), 4.29 (d, J = 9.6 Hz, 1H), 4.20 - 4.10 (m, 1H), 3.45 - 3.35 (m, 1H), 2.68 - 2.52 (m, 1H), 2.49 - 2.45 (m, 2H), 2.37 - 2.20 (m, 3H), 2.02 - 1.90 (m, 2H), 1.70 - 1.60 (m, 1H), 1.57 (s, 3H), 1.39 - 1.32 (m, 1H), 1.21 (d, J = 6.4 Hz, 3H), 1.13 (s, 3H), 1.02 (d, J = 7.2 Hz, 3H).

### Preparation example 26: (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-hydroxybutanoate (D-23)

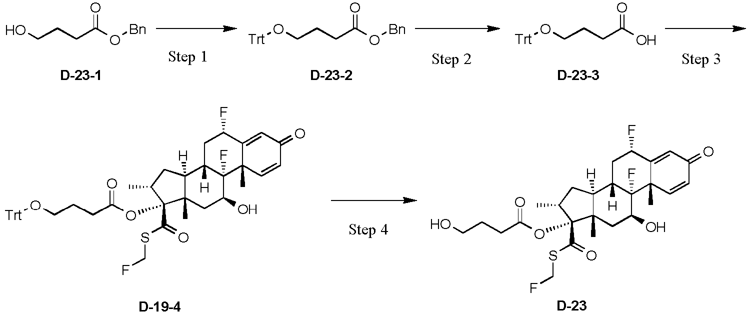

### Step 1: Synthesis of benzyl 4-(triphenylmethoxy)butyrate (D-23-2)

Benzyl 4-hydroxybutyrate (500 mg, 2.57 mmol) was dissolved in pyridine (3.00 mL), triphenylmethyl chloride (718 mg, 2.57 mmol) was added, and then the reaction solution was heated to 90 °C and allowed to react for 10 h. 1N diluted hydrochloric acid (20.0 mL) was added into the reaction solution, followed by extraction with dichloromethane for 3 times (30.0 mL x 3). The organic phases were combined, washed with 1 N hydrochloric acid (20.0 mL x 3), dried over anhydrous sodium sulfate, filtered and concentrated to obtain the title compound as a crude product (900 mg, crude), which was directly used in the next step without purification.

The structure thereof was characterized as follows:
ESI-MS (m/z): 459.3 (M+Na)⁺.

### Step 2: Synthesis of 4-(triphenylmethoxy)butyric acid (D-23-3)

Benzyl 4-(triphenylmethoxy)butyrate (850 mg, 1.95 mmol) was dissolved in a mixed solvent of methanol (0.50 mL), tetrahydrofuran (0.50 mL) and water (0.50 mL), lithium hydroxide (140 mg, 5.84 mmol) was added, and then the reaction solution was heated to 40°C and allowed to react for 1 h. The reaction solution was adjusted to pH 2-3 using 1 N diluted hydrochloric acid, and extracted with ethyl acetate for 3 times (20.0 mL x 3) after water (10.00 mL) was added. The organic phases were combined, dried over anhydrous sodium sulfate, filtered and concentrated to obtain the title compound as a crude product (230 mg, crude), which was directly used in the next step without purification.

The structure thereof was characterized as follows:
ESI-MS (m/z): 369.2 (M+H)⁺.

### Step 3: Synthesis of (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-(triphenylmethoxy)butanoate (D-23-4)

4-(triphenylmethoxy)butyric acid (180 mg, 519 µmol) was dissolved in DMF (3.00 mL), HATU (198 mg, 520 µmol) and DIPEA (201 mg, 1.56mmol, 272 µL) were added, and then the reaction solution was allowed to react for 1 h at 25°C. (6S,8S,9R,10S,11S,13S,14S,16R,17R)-6,9-difluoro-11,17-dihydroxy-10,13,16-trimethyl-3-oxo-6,7,8,9,10,11,12,13,14,15,16,17-dodecahydro-3H-cyclopenta[a]phenanthrene-17-thiocarboxylic acid (193 mg, 468 µmol) was added, then the reaction solution was stirred for additional 0.5 h. Then fluoroiodomethane (83.1 mg, 520 µmol) was added, and the reaction solution was stirred for additional 0.5 h. Water (10.0 mL) was added into the reaction solution, followed by extraction with ethyl acetate for 3 times (20.0 mL x 3). The organic phases were combined, dried over anhydrous sodium sulfate, filtered and concentrated to obtain the title compound as a crude product (480 mg, crude), which was directly used in the next step without purification.

The structure thereof was characterized as follows:
ESI-MS (m/z): 796.3 (M+H)⁺.

### Step 4: Synthesis of (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-hydroxybutanoate (D-23)

(6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-(triphenylmethoxy)butanoate (430 mg, 556 µmol) was dissolved in a mixed solvent of dichloromethane (4.00 mL) and methanol (1.00 mL), and trifluoroacetic acid (1.54 g, 13.5 mmol, 1.00 mL) was added, and then the reaction solution was stirred for 1 h at 25°C. The reaction solution was directly concentrated to obtain a crude product, which was purified via high performance liquid chromatography to obtain the title compound (49.0 mg, 92.2 µmol).
Chromatographic column: Phenomenex Luna C18 200 mm×40 mm×10 µm
Mobile phase A: acetonitrile; mobile phase B: water (0.05% trifluoroacetic acid)

| Time [min] | Mobile phase A [%] | Mobile phase B [%] | Flow rate [mL/min] |
|---|---|---|---|
| 0.00 | 30 | 70 | 28 |
| 13.00 | 60 | 30 | 28 |

The structure thereof was characterized as follows:
ESI-MS (m/z): 531.1 [M+H]⁺.

¹H NMR (400 MHz, CD₃OD) δ 7.32 (m, 1H), 6.33 (m, 1H), 6.30 (s, 1H), 5.91-5.97 (m, 1H), 5.79-5.85 (m, 1H), 5.44-5.64 (m, 1H), 4.27-4.32 (m, 1H), 3.56 (t, J = 6.4 Hz, 2H), 3.41 (m, 1H), 2.51-2.65 (m, 1H), 2.46 (t, J = 7.6 Hz, 2H), 2.30-2.35 (m, 1H), 2.18-2.28 (m, 2H), 1.93-2.01 (m, 2H), 1.81 (m, 2H), 1.58-1.66 (m, 1H), 1.57 (s, 3H), 1.34 (m, 1H), 1.12 (s, 3H), 0.99 (d, J = 7.2 Hz, 3H).

### Preparation example 27: (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 5-hydroxyvalerate (D-24)

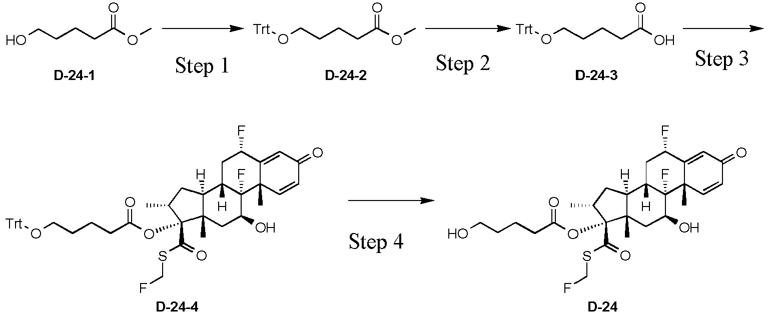

### Step 1: Synthesis of methyl 5-(triphenylmethoxy)valerate

Methyl 5-hydroxyvalerate (450 mg, 3.41 mmol) was dissolved in pyridine (15.0 mL), triphenylmethyl chloride (949 mg, 3.41 mmol) was added, and then the reaction solution was heated to 80°C and allowed to react for 10 h. 1 N diluted hydrochloric acid (20.0 mL) was added into the reaction solution, followed by extraction with dichloromethane for 3 times (30.0 mL x 3). The organic phases were combined, washed with 1 N diluted hydrochloric acid twice (20.0 mL x 2), dried over anhydrous sodium sulfate, filtered and concentrated to obtain the title compound as a crude product (937 mg, crude).

The structure thereof was characterized as follows:
¹H NMR (400 MHz, DMSO) δ 7.35-7.37 (m, 6H), 7.27-7.30 (m, 6H), 7.21 (d, J = 1.6 Hz, 3H), 3.55-3.59 (m, 3H), 2.93-2.98 (m, 2H), 2.22-2.29 (m, 2H), 1.53-1.63 (m, 4H).

### Step 2: Synthesis of 5-(triphenylmethoxy)valeric acid (D-24-3)

Methyl 5-(triphenylmethoxy)valerate (937 mg, 2.50 mmol) was dissolved in a mixed solvent of tetrahydrofuran (2.00 mL), water (2.00 mL) and methanol (2.00 mL), lithium hydroxide monohydrate (315 mg, 7.51 mmol) was added, and then the reaction solution was heated to 40°C and stirred for 1 h. The reaction solution was adjusted to pH 2-3 using 1 N diluted hydrochloric acid, and extracted with ethyl acetate for 3 times (20.0 mL x 3) after water (10.0 mL) was added. The organic phases were combined, dried over anhydrous sodium sulfate, filtered and concentrated to obtain the title compound as a crude product (285 mg, crude), which was directly used in the next step without purification.

The structure thereof was characterized as follows:
¹H NMR (400 MHz, DMSO) δ 7.34-7.39 (m, 8H), 7.31-7.34 (m, 4H), 7.26 (m, 3H), 2.92-2.99 (m, 2H), 2.17 (m, 2H), 1.53-1.60 (m, 4H).

### Step 3: Synthesis of (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 5-(triphenylmethoxy)valerate (D-24-4)

5-(triphenylmethoxy)valeric acid (230 mg, 638 µmol) was dissolved in DMF (3.00 mL), HATU (243 mg, 638 µmol) and DIPEA (247 mg, 1.91 mmol, 333 µL) were added, and then the reaction solution was stirred for 1 h at 25°C. (6S,8S,9R,10S,11S,13S,14S,16R,17R)-6,9-difluoro-11,17-dihydroxy-10,13,16-trimethyl-3-oxo-6,7,8,9,10,11,12,13,14,15,16,17-dodecahydro-3H-cyclopenta[a]phenanthrene-17-thiocarboxylic acid (237 mg, 574 µmol) was added, and the reaction solution continued to react for 0.5 h. Then, fluoroiodomethane (102 mg, 638 µmol) was added, and then the reaction solution was stirred for additional 0.5 h. Water (10.0 mL) was added into the reaction solution, followed by extraction with ethyl acetate for 3 times (20.0 mL x 3). The organic phases were combined, dried over anhydrous sodium sulfate, filtered and concentrated to obtain the title compound as a crude product (600 mg, crude), which was directly used in the next step without purification.

The structure thereof was characterized as follows:
ESI-MS (m/z): 809.3 (M+Na)⁺.

### Step 4: Synthesis of (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 5-hydroxyvalerate (D-24)

(6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 5-(triphenylmethoxy)valerate (600 mg, 762 µmol) was dissolved in a mixed solvent of dichloromethane (4.00 mL) and methanol (1.00 mL), trifluoroacetic acid (1.54 g, 13.5 mmol, 1.00 mL) was added, and then the reaction solution was stirred for 1 h at 25°C. The reaction solution was directly concentrated to obtain a crude product, which was purified via high performance liquid chromatography to obtain the title compound (72.0 mg, 132 µmol).
Chromatographic column: Phenomenex Luna C18 200 mm×40 mm×10 µm
Mobile phase A: acetonitrile; mobile phase B: water (0.05% trifluoroacetic acid)

| **Time [min]** | Mobile phase A [%] | Mobile phase B [%] | Flow rate [mL/min] |
|---|---|---|---|
| 0.00 | 30 | 70 | 28 |
| 13.00 | 60 | 40 | 28 |

The structure thereof was characterized as follows:
ESI-MS (m/z): 545.2 [M+H]⁺.

¹H NMR (400 MHz, CD₃OD) δ 7.32 (m, 1H), 6.34 (m, 1H), 6.30 (s, 1H), 5.91-5.97 (m, 1H), 5.79-5.85 (m, 1H), 5.44-5.64 (m, 1H), 4.27-4.32 (m, 1H), 3.54 (t, J = 6.4 Hz, 2H), 3.40 (m, 1H), 2.51-2.63 (m, 1H), 2.39-2.44 (m, 2H), 2.30-2.37 (m, 1H), 2.19-2.28 (m, 2H), 1.93-2.01 (m, 2H), 1.64-1.70 (m, 2H), 1.59 (m, 2H), 1.57 (s, 3H), 1.53-1.56 (m, 1H), 1.34 (m, 1H), 1.12 (s, 3H), 0.99 (d, J = 7.2 Hz, 3H).

### Preparation example 28: (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (1S,4S) -4-hydroxycyclohexane-1-formate (D-25)

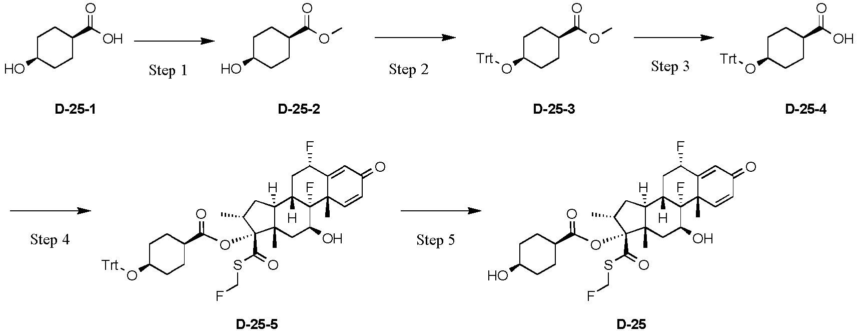

### Step 1: Synthesis of methyl (15,4S)-4-hydroxycyclohexane-1-formate (D-25-2)

(1S,4S)-4-hydroxycyclohexane-1-formic acid (345 mg, 2.5 mmol) was added into DMF (2 mL), anhydrous potassium carbonate (345 mg, 2.5 mmol) and methyl iodide (591.6 mg, 4.17 mmol) were added, and then the reaction solution was allowed to react for 2 h at 25°C under stirring. Water (20 mL) was added into the reaction solution, and the reaction solution was extracted with ethyl acetate for 3 times (10 mL x 3) and washed with a saturated sodium chloride aqueous solution twice (10 mL x 2). The organic phases were combined, dried over anhydrous sodium sulfate, filtered and concentrated to obtain the title compound as a crude product (310 mg, 1.96 mmol), which was directly used in the next step without purification.

### Step 2: Synthesis of methyl (1S,4S)-4-(triphenylmethoxy)cyclohexane-1-formate (D-25-3)

Methyl (1S,4S)-4-hydroxycyclohexane-1-formate (310 mg, 1.96 mmol) was added into a single necked flask, pyridine (3 mL) and triphenylmethyl chloride (820.5 mg, 2.94 mmol) were added, and then the reaction solution was heated to 80°C, allowed to react for 15 h under stirring, and cooled to room temperature. Water (15 mL) was added into the reaction solution, followed by extraction with ethyl acetate for 3 times (8 mL x 3). The organic phases were combined, dried over anhydrous sodium sulfate, filtered and concentrated to obtain a crude product, which was purified via flash column chromatography (SiO₂, petroleum ether/ethyl acetate = 100/1 to 5/1) to obtain the title compound (320 mg, 0.8 mmol).

The structure thereof was characterized as follows:
¹H NMR (400 MHz, DMSO) δ 7.46 - 7.41 (m, 6H), 7.36 -7.29 (m, 6H), 7.28 - 7.22 (m, 3H), 3.50 (s, 3H), 1.68 (d, J = 10.4 Hz, 2H), 1.26 - 1.01 (m, 8H).

### Step 3: Preparation of (1S,4S)-4-(triphenylmethoxy)cyclohexane-1-formic acid (D-25-4)

Methyl (1S,4S)-4-(triphenylmethoxy)cyclohexane-1-formate (320 mg, 0.8 mmol) was added into tetrahydrofuran (2 mL), methanol (2 mL) and water (1 mL), lithium hydroxide (67.2 mg, 1.6 mmol) was added, and then the reaction solution was stirred for 2 h at 25°C. Water and 1 N diluted hydrochloric acid (3 mL) were added into the reaction solution, followed by extraction with dichloromethane for 3 times (67.2 mg, 1.6 mmol). The organic phases were combined, dried over anhydrous sodium sulfate, filtered and concentrated to obtain a crude product, which was purified via flash column chromatography (SiO₂, petroleum ether/ethyl acetate = 100/1 to 3/1) to obtain the title compound (280 mg, 0.72 mmol).

### Step 4: Synthesis of (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (1S,4S)-4-(triphenylmethoxy)cyclohexane-1-formate (D-25-5)

(18,4S)-4-(triphenylmethoxy)cyclohexane-1-formic acid (50 mg, 0.13 mmol) and HATU (59.0 mg, 0.16 mmol) were added into DMF (2.0 mL), DIPEA(50.1 mg, 0.39 mmol) was added, and then the reaction solution was allowed to react for 2 h at 25°C. (6S,8S,9R,10S,11S,13S,14S,16R,17R)-6,9-difluoro-11,17-dihydroxy-10,13,16-trimethyl-3-oxo-6,7,8,9,10,11,12,13,14,15,16,17-dodecahydro-3H-cyclopenta[a]phenanthrene-17-thiocarboxylic acid (63.2 mg, 0.16 mmol) was added into the above reaction solution, and then the reaction solution was stirred for additional 2 h. Then, fluoroiodomethane (31.0 mg, 0.19 mmol) was added, and then the reaction solution continued to react for 1 h at 25°C. Water (10 mL) was added into the reaction solution, followed by extraction with ethyl acetate for 3 times (8 mL x 3). The organic phases were combined, dried over anhydrous sodium sulfate, filtered and concentrated to obtain a crude product, which was purified via flash column chromatography (C18, water/acetonitrile = 0.8) to obtain the title compound (16 mg, 0.02 mmol).

### Step 5: Synthesis of (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (1S,4S) -4-hydroxycyclohexane-1-formate (D-25)

(6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (1S,4S)-4-(triphenylmethoxy)cyclohexane-1-formate (60 mg, 0.073 mmol) was dissolved in dichloromethane (1 mL), acetic acid (0.5 mL) was then added, and then the reaction solution was stirred for 3 h at 40°C. The reaction solution was directly concentrated to obtain a crude product, which was purified via high performance liquid chromatography to obtain the title compound (3.1 mg, 0.005 mmol).
Chromatographic column: SunFire prep C18 OBD 150 mm×19 mm×5 µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 30 | 70 | 32 |
| 4.00 | 30 | 70 | 32 |
| 24.00 | 90 | 10 | 32 |

The structure thereof was characterized as follows:
ESI-MS (m/z): 571.1[M+H]⁺.

¹H NMR (400 MHz, CD₃OD) δ 7.33 (dd, J = 10.0, 1.6 Hz, 1H), 6.38 - 6.28 (m, 2H), 5.98 - 5.88 (m, 1H), 5.86 - 5.76 (m, 1H), 5.65 - 5.45 (m, 1H), 4.35 - 4.26 (m, 1H), 3.51 - 3.45 (m, 1H), 2.69 - 2.50 (m, 1H), 2.39 - 2.18 (m, 4H), 2.08 - 1.90 (m, 6H), 1.57 (s, 3H), 1.54 -1.41 (m, 2H), 1.36 - 1.21 (m, 4H), 1.11 (s, 3H), 0.96 (d, J = 7.2 Hz, 3H).

### Preparation example 29: (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (1R,4R)-4-hydroxycyclohexane-1-formate (D-26)

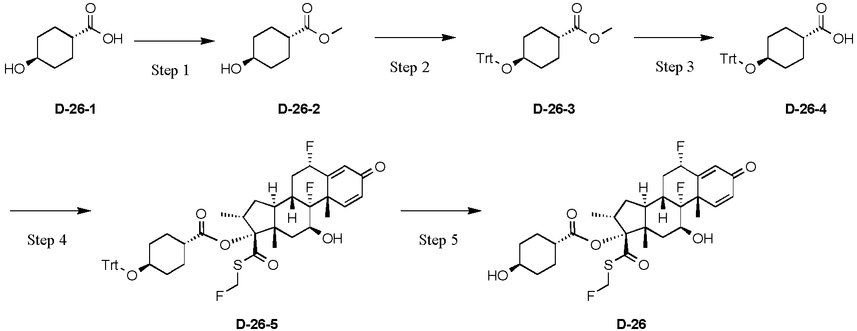

### Step 1: Synthesis of methyl (1R,4R)-4-hydroxycyclohexane-1-carboxylate (D-26-2)

(1R,4R)-4-hydroxycyclohexane-1-formic acid (300 mg, 2.1 mmol) was dissolved in DMF (2 mL), anhydrous potassium carbonate (345 mg, 2.5 mmol) and methyl iodide (591.6 mg, 4.17 mmol) were added, and the reaction solution was allowed to react for 2 h at 25°C under stirring. Water (20 mL) was added into the reaction solution, and the the reaction solution was extracted with ethyl acetate for 3 times (10 mL x 3) and washed with a saturated sodium chloride aqueous solution twice (10 mL x 2). The organic phases were combined, dried over anhydrous sodium sulfate, filtered and concentrated to obtain the title compound as a crude product (310 mg, 1.96 mmol), which was directly used in the next step without purification.

### Step 2: Synthesis of methyl (1R,4R)-4-(triphenylmethoxy)cyclohexane-1-carboxylate (D-26-3)

Methyl (1R,4R)-4-hydroxycyclohexane-1-carboxylate (310 mg, 1.96 mmol) was added into a single necked flask, pyridine (3 mL) and triphenylmethyl chloride (820.5 mg, 2.94 mmol) were added, and then the reaction solution was heated to 80°C and allowed to react for 15 h under stirring. The reaction solution was cooled to room temperature. Water (15 mL) was added into the reaction solution, followed by extraction with ethyl acetate for 3 times (8 mL x 3). The organic phases were combined, dried over anhydrous sodium sulfate, filtered and concentrated to obtain a crude product, which was purified via flash column chromatography (SiO₂, petroleum ether/ethyl acetate = 100/1 to 5/1) to obtain the title compound (691 mg, 1.73 mmol).

The structure thereof was characterized as follows:
¹H NMR (400 MHz, DMSO) δ 7.44 - 7.40 (m, 6H), 7.38 -7.30 (m, 6H), 7.28 - 7.20 (m, 3H), 3.48 (s, 3H), 1.67 (d, J = 10.4 Hz, 2H), 1.24 - 1.01 (m, 8H).

### Step 3: Preparation of (1R,4R)-4-(triphenylmethoxy)cyclohexane-1-carboxylic acid (D-26-4)

Methyl (1R,4R)-4-(triphenylmethoxy)cyclohexane-1-carboxylate (691 mg, 1.73 mmol) was added into tetrahydrofuran (6 mL), methanol (6 mL) and water (3 mL), lithium hydroxide (123.96 mg, 5.18 mmol) was added, and then the reaction solution was stirred for 2 h at 25°C. Water and 1 N diluted hydrochloric acid (3 mL) were added into the reaction solution, followed by extraction with dichloromethane for 3 times (10 mL * 3). The organic phases were combined, dried with anhydrous sodium sulfate, filtered and concentrated to obtain a crude product, which was purified via flash column chromatography (SiO₂, petroleum ether/ethyl acetate = 100/1 to 3/1) to obtain the title compound (410 mg, 1.06 mmol).

### Step 4: Synthesis of (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (1R,4R)-4-(triphenylmethoxy)cyclohexane-1-carboxylate (D-26-5)

(1R,4R)-4-(triphenylmethoxy)cyclohexane-1-carboxylic acid (50 mg, 0.13 mmol) and HATU (59.0 mg, 0.16 mmol) were added into DMF (2.0 mL), DIPEA(50.1 mg, 0.39 mmol) was added, and then the reaction solution was allowed to react for 2 h at 25°C. (6S,8S,9R,10S,11S,13S,14S,16R,17R)-6,9-difluoro-11,17-dihydroxy-10,13,16-trimethyl-3-oxo-6,7,8,9,10,11,12,13,14,15,16,17-dodecahydro-3H-cyclopenta[a]phenanthren-17-thiocarboxylic acid (63.2 mg, 0.16 mmol) was added, and the reaction solution was stirred for additional 2 h. Then, fluoroiodomethane (31.0 mg, 0.19 mmol) was added, and the reaction solution continued to react for 1 h at 25°C. Water (10 mL) was added into the reaction solution, followed by extraction with ethyl acetate for 3 times (10 mL x 3). The organic phases were combined, dried over anhydrous sodium sulfate, filtered and concentrated to obtain the title compound as a crude product (60 mg, 0.073 mmol), which was directly used in the next step without purification.

### Step 5: Synthesis of (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (1R,4R) -4-hydroxycyclohexane-1-carboxylate (D-26)

(6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (1R,4R)-4-(triphenylmethoxy)cyclohexane-1-carboxylate (60 mg, 0.073 mmol) was dissolved in dichloromethane (1 mL), acetic acid (0.5 mL) was added, and then the reaction solution was stirred for 3 h at 40°C. The reaction solution was directly concentrated to obtain a crude product, which was purified via high performance liquid chromatography to obtain the title compound (3.5 mg, 0.006 mmol).
Chromatographic column: SunFire prep C18 OBD 150 mm×19 mm×5 µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 20 | 80 | 32 |
| 4.00 | 20 | 80 | 32 |
| 24.00 | 90 | 10 | 32 |

The structure thereof was characterized as follows:
ESI-MS (m/z): 594.2[M+Na]⁺.

¹H NMR (400 MHz, DMSO) δ 7.26 (d, J = 10.1 Hz, 1H), 6.30 (dd, J = 10.2, 1.8 Hz, 1H), 6.11 (s, 1H), 5.98 (s, 1H), 5.86 (s, 1H), 5.73 - 5.54 (m, 1H), 5.58 (d, J = 4.0 Hz, 1H), 4.59 (d, J = 4.3 Hz, 1H), 4.22 (s, 1H), 3.32 - 3.25 (m, 1H), 2.61 - 2.32 (m, 2H), 2.30 - 2.20 (m, 2H), 2.14 - 2.03 (m, 2H), 1.89 - 1.77 (m, 5H), 1.59 - 1.44 (m, 1H) 1.48 (s, 3H), 1.39 - 1.21 (m, 4H), 1.20 - 1.09 (m, 2H), 0.98 (s, 3H), 0.86 (d, J = 7.2 Hz, 3H).

### Preparation example 30: (8S,9R,10S,11S,13S,14S,17R)-9-fluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-amino-4-fluorobenzoate (A-2)

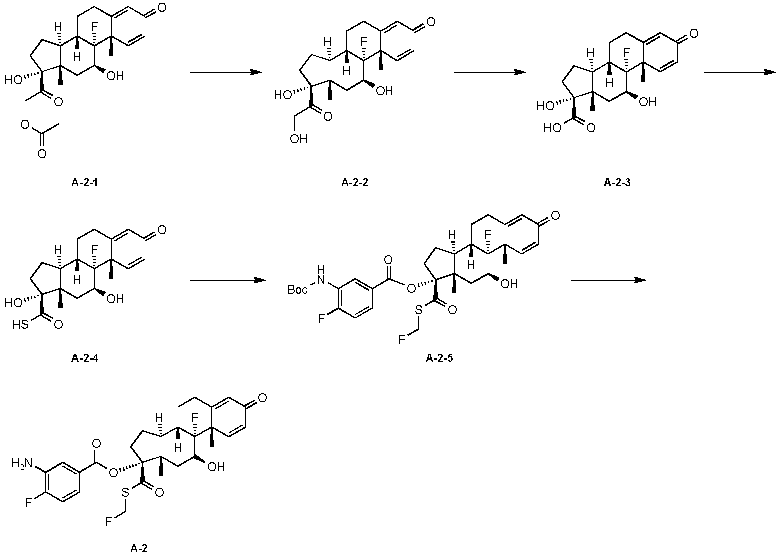

### Step 1: synthesis of (8S,9R,10S,11S,13S,14S,17R)-9-fluoro-11,17-dihydroxy-17-(2-hydroxyacetyl)-10,13-dimethyl-6,7,8,9,10,11,12,13,14,15,16,17-dodecahydro-3H-cyclopenta[a]phenanthrene-3-one (A-2-2)

2-((8S,9R,10S,11S,13S,14S,17R)-9-fluoro-11,17-dihydroxy-10,13-dimethyl-3-oxo-6,7,8,9,10,11,12,13,14,15,16,17-dodecahydro-3H-cyclopenta[a]phenanthren-17-yl)-2-oxoethyl acetate (150 mg, 356 µmol) was dissolved in a mixed solvent of water (1.00 mL), dichloromethane (1.00 mL) and methanol (1.00 mL), sodium hydroxide (21.4 mg, 535 µmol) was added, and then the reaction solution was stirred for 1 h at 25°C. The reaction solution was adjusted to pH 2-3 using 1 N diluted hydrochloric acid. Water (30.0 mL) was added into the reaction solution, followed by extraction with dichloromethane for 3 times (20.0 mL x 3). The organic phases were combined, dried over anhydrous sodium sulfate, filtered and concentrated to obtain the title compound (110 mg, 290 µmol).

The structure thereof was characterized as follows:
ESI-MS (m/z): 378.9 [M+H]⁺.

### Step 2: synthesis of (8S,9R,10S,11S,13S,14S,17R)-9-fluoro-11,17-dihydroxy-10,13-dimethyl-3-oxo-6,7,8,9,10,11,12,13,14,15,16,17-dodecahydro-3H-cyclopenta[a]phenanthrene-17-carboxylic acid (A-2-3)

(8S,9R,10S,11S,13S,14S,17R)-9-fluoro-11,17-dihydroxy-17-(2-hydroxyacetyl)-10,13-dimethyl-6,7,8,9,10,11,12,13,14,15,16,17-dodecahydro-3H-cyclopenta[a]phenanthrene-3-one (200 mg, 528 µmol) was dissolved in a mixed solvent of tetrahydrofuran (1.30 mL) and water (1.00 mL), periodic acid (361 mg, 1.59 mmol, 361 µL) was added, and then the reaction solution was stirred for 1 h at 25°C . The reaction solution was adjusted to pH 2-3 using 1 N diluted hydrochloric acid, filtered, and the filter cake was dried to obtain the title compound (100 mg, 274 µmol).

The structure thereof was characterized as follows:
ESI-MS (m/z): 365.0 [M+H]⁺.

### Step 3: synthesis of (8S,9R,10S,11S,13S,14S,17R)-9-fluoro-11,17-dihydroxy-10,13-dimethyl-3-oxo-6,7,8,9,10,11,12,13,14,15,16,17-dodecahydro-3H-cyclopenta[a]phenanthrene-17-thiocarboxylic acid (A-2-4)

(8S,9R,10S,11S,13S,14S,17R)-9-fluoro-11,17-dihydroxy-10,13-dimethyl-3-oxo-6,7,8,9,10,11,12,13,14,15,16,17-dodecahydro-3H-cyclopenta[a]phenanthrene-17-carboxylic acid (100 mg, 274 µmol) was dissolved in DMF (2.00 mL), CDI (88.9 mg, 548 µmol) was added, and then the reaction solution was stirred for 2 h at 25°C. The reaction system was stirred for additional 2.5 h after a H₂S gas (9.35 mg, 274 µmol, 4.52 µL) was introduced. The reaction solution was adjusted to pH 2-3 using 1 N diluted hydrochloric acid, filtered, and the filter cake was dried to obtain the title compound (70.0 mg, 183 µmol).

The structure thereof was characterized as follows:
ESI-MS (m/z): 380.9 [M+Na]⁺.

### Step 4: Synthesis of (8S,9R,108,11S,13S,148S,17R)-9-fluoro-17-((fluoromethyl) thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((tert-butoxycarbonyl)amino)-4-fluorobenzoate (A-2-5)

3-(tert-butoxycarbonyl)amino)-4-fluorobenzoic acid (40.2 mg, 157 µmol) was dissolved in DMF (4.00 mL), HATU (59.9 mg, 157 µmol) and DIPEA (20.3 mg, 157 µmol, 27.4 µL) were added, and then the reaction solution was allowed to react for 2 h at 25°C. The reaction solution was stirred for additional 1 h after (8S,9R,10S,11S,13S,14S,17R)-9-fluoro-11,17-dihydroxy-10,13-dimethyl-3-oxo-6,7,8,9,10,11,12,13,14,15,16,17-dodecahydro-3H-cyclopenta[a]phenanthrene-17-thiocarboxylic acid (60.0 mg, 157 µmol) was added. Then, fluoroiodomethane (25.2 mg, 157 µmol) was added, and the reaction solution was allowed to react for 1 h at 25°C. Water (10 mL) was added into the reaction solution, followed by extraction with dichloromethane for 3 times (10.0 mL x 3). The organic phases were combined, dried over anhydrous sodium sulfate, filtered and concentrated to obtain the title compound as a crude product, which was purified via p-TLC (SiO₂, CH₂Cl₂/ MeOH = 100/1 to 10/1) and then concentrated again to obtain the title compound (12.0 mg, 18.4 µmol).

The structure thereof was characterized as follows:
ESI-MS (m/z): 650.2 [M+H]⁺.

### Step 5: Synthesis of (8S,9R,10S,11S,13S,14S,17R)-9-fluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-amino-4-fluorobenzoate (A-2)

(8S,9R,10S,11S,13S,14S,17R)-9-fluoro-17-((fluoromethyl) thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((tert-butoxycarbonyl)amino)-4-fluorobenzoate (10.0 mg, 15.3 µmol) was dissolved in dichloromethane (1.00 mL), and then the reaction solution was stirred for 1 h at 25°C after trifluoroacetic acid (460 mg, 4.04 mmol, 0.30 mL) was added. The reaction solution was directly concentrated to obtain a crude product, which was purified via high performance liquid chromatography to obtain the title compound (2.36 mg, 4.07 µmol).
Chromatographic column: Phenomenex luna C18 150mm×25mm×10µm
Mobile phase A: acetonitrile; mobile phase B: water (0.05% ammonium bicarbonate)

| Time [min] | Mobile phase A [%] | Mobile phase B [%] | Flow rate [mL/min] |
|---|---|---|---|
| 0.00 | 40 | 60 | 24 |
| 10.00 | 70 | 30 | 24 |

The structure of A-2 was characterized as follows:
ESI-MS (m/z): 550.2 [M+H]⁺.

¹H NMR (400 MHz, CD30D) δ 7.36-7.49 (m, 2H), 7.22-7.32 (m, 1H), 7.06 (dd, J = 10.8, 8.4 Hz, 1H), 6.33 (dd, J = 10.0, 1.8 Hz, 1H), 6.11 (s, 1H), 5.68-6.00 (m, 2H), 4.40 (d, J = 8.4 Hz, 1H), 3.03 (dd, J = 14.4, 11.2 Hz, 1H), 2.76 (td, J = 13.6, 5.6 Hz, 1H), 2.53-2.67 (m, 1H), 2.38-2.51 (m, 2H), 2.18-2.27 (m, 1H), 2.02-2.14 (m, 2H), 1.94-2.01 (m, 1H), 1.73-1.83 (m, 1H), 1.62 (s, 3H), 1.50-1.60 (m, 2H), 1.07 (s, 3H).

### Preparation example 31: (6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-(hydroxymethyl)furan-2-carboxylate (F-9)

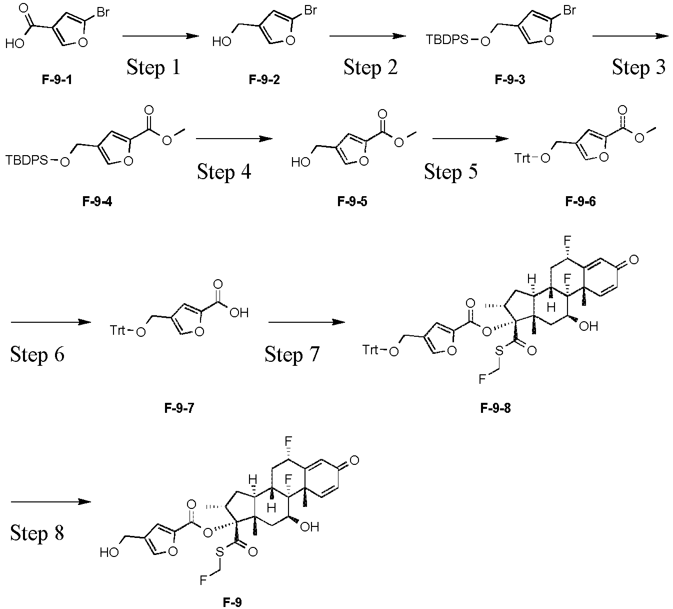

### Step 1: synthesis of (5-bromofuran-3-yl)methanol (F-9-2)

5-bromofuran-3-formic acid (1.00 g, 5.24 mmol) was dissolved in tetrahydrofuran (20.0 mL), and then the reaction solution was cooled to 0°C. After borane-tetrahydrofuran complex (2.5 M, 4.19 mL) was added under nitrogen atmosphere, the reaction solution was heated to 50°C, stirred for 3 h, and cooled to 25°C. Then methanol (10.0 mL) was added into the reaction system for quenching, and the reaction solution was concentrated. A 2 M sodium hydroxide aqueous solution (30.0 mL) was added into the reaction solution, followed by extraction with ethyl acetate twice (30.0 mL x 2). The organic phase was dried over anhydrous sodium sulfate, filtered and concentrated to obtain the title compound as a crude product (1.06 g, crude), whic was directly used in the next step without purification.

The structure thereof was characterized as follows:
ESI-MS (m/z): 177.1 [M+H]⁺.

### Step 2: synthesis of (5-bromofuran-3-yl)methoxy)(tert-butyl)diphenylsilane (F-9-3)

(5-bromofuran-3-yl) methanol (1.00 g, 5.65 mmol) was dissolved in dichloromethane (10.0 mL), and then the reaction solution was stirred for 3 h at 25°C after imidazole (1.15 g, 16.9 mmol) and tert-butyldiphenylsilane chloride (2.33 g, 8.47 mmol) were added. Water (20.0 mL) was added into the reaction solution, followed by extraction with dichloromethane (20.0 mL). The organic phase was dried over anhydrous sodium sulfate, filtered and concentrated to obtain a crude product, which was purified via silica gel column chromatography (petroleum ether: ethyl acetate = 100/0 to 100/5) and then concentrated again to obtain the title compound as a crude product (1.20 g, crude).

The structure thereof was characterized as follows:
ESI-MS (m/z): 415.1 [M+H]⁺.

### Step 3: synthesis of methyl 4-((tert-butyldiphenylsilyl)oxy)methyl)furan-2-carboxylate (F-9-4)

(5-bromofuran-3-yl)methoxy)(tert-butyl)diphenylsilane (900 mg, 2.17 mmol) was dissolved in methanol (45.0 mL), triethylamine (438 mg, 4.33 mmol) and Pd(dppf)Cl₂ (158 mg, 216 µmol) were added under the atmosphere of nitrogen, and then a CO gas (40 psi) was introduced into the reaction system, followed by reaction for 6 h at 60°C. The reaction solution was concentrated to obtain a crude product, which was purified via silica gel column chromatography (petroleum ether: ethyl acetate = 10:0 to 10:2) and then concentrated again to obtain the title compound (746 mg, 1.89 mmol).

The structure thereof was characterized as follows:
ESI-MS (m/z): 395.3 [M+H]⁺.

### Step 4: synthesis of methyl 4-(hydroxymethyl)furan-2-carboxylate (F-9-5)

Methyl 4-((tert-butyldiphenylsilyl)oxy)methyl)furan-2-carboxylate (643 mg, 1.63 mmol) was dissolved in tetrahydrofuran (6.00 mL), and then the reaction solution was stirred for 1 h at 20 °C after a tetrabutylammonium fluoride solution (1 M, 1.63 mL) was added. The reaction solution was concentrated to obtain a crude product, which was purified via silica gel column chromatography (petroleum ether: ethyl acetate = 10/1 to 1/1) and then concentrated again to obtain the title compound as a crude product (190 mg, crude).

The structure thereof was characterized as follows:
ESI-MS (m/z): 157.0 [M+H]⁺.

### Step 5: synthesis of methyl 4-((triphenylmethoxy)methyl)furan-2-carboxylate (F-9-6)

Methyl 4-(hydroxymethyl)furan-2-carboxylate (191 mg, 1.22 mmol) was dissolved in pyridine (2.00 mL), triphenylmethyl chloride (341 mg, 1.22 mmol) was added, and the reaction solution was heated to 80°C and allowed to react for 12 h. 0.5 M diluted hydrochloric acid (20.0 mL) was added into the reaction solution, followed by extraction with dichloromethane (20.0 mL). The organic phase was dried over anhydrous sodium sulfate, filtered and concentrated to obtain the title compound as a crude product (517 mg, crude), which was directly used in the next step without purification.

### Step 6: Synthesis of 4-((triphenylmethoxy)methyl)furan-2-carboxylic acid (F-9-7)

Methyl 4-((triphenylmethoxy)methyl)furan-2-carboxylate (517 mg, 1.30 mmol) was dissolved in a mixed solvent of (4.00 mL), methanol (4.00 mL) and water (4.00 mL), lithium hydroxide monohydrate (163 mg, 3.89 mmol) was added, and then the reaction solution was heated to 40°C and stirred for 1 h. Water (30.0 mL) was added into the reaction solution, followed by extraction with dichloromethane (30.0 mL). The water phase was adjusted to pH 2-3 using 0.5 M diluted hydrochloric acid, and then extracted with dichloromethane for 3 times (10.0 mL x 3). The organic phases were combined, dried over anhydrous sodium sulfate, filtered and concentrated to obtain the title compound as a crude product (98.0 mg, crude), which was directly used in the next step without purification.

### Step 7: synthesis of (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-((triphenylmethoxy)methyl)furan-2-carboxylate (F-9-8)

4-((triphenylmethoxy)methyl)furan-2-carboxylic acid (65.0 mg, 169 µmol) and (6S,8S,9R,10S,11D,13D,14S,16R,17R)-6,9-difluoro-11,17-dihydroxy-10,13,16-trimethyl-3-oxo-6,7,8,9,10,11,12,13,14,15,16,17-dodecahydro-3H-cyclopenta[a]phenanthrene-17-thiocarboxylic acid (69.8 mg, 169 µmol) were dissolved in DMF (2.00 mL), and then the reaction solution was stirred for 2 h at 25°C after HATU (64.3 mg, 169 µmol) and DIPEA (65.6 mg, 507 µmol) were added. Then, fluoroiodomethane (27.1 mg, 169 µmol) was added, and then the reaction solution was stirred for additional 3 h. The reaction solution was concentrated, followed by adding a saturated sodium chloride aqueous solution (30.0 mL), and then the reaction solution was extracted with ethyl acetate twice (30.0 mL x 2). The organic phases were combined, dried over anhydrous sodium sulfate, filtered and concentrated to obtain the title compound as a crude product (234 mg, crude), which was directly used in the next step without purification.

### Step 8: Synthesis of (6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-(hydroxymethyl)furan-2-carboxylate (F-9)

(6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-((triphenylmethoxy)methyl)furan-2-carboxylate (315 mg, 388 µmol) was dissolved in a mixed solvent of dichloromethane (3.00 mL) and methanol (3.00 mL), and then the reaction solution was stirred for 1 h at 20°C after trifluoroacetic acid (4.61 g, 40.4 mmol) was added. The reaction solution was directly concentrated to obtain a crude product, which was purified via high performance liquid chromatography to obtain the title compound (18.0 mg, 0.031 mmol).
Chromatographic column: Phenomenex luna C18 150mm×25mm×10 µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 40 | 60 | 24 |
| 10.00 | 70 | 30 | 24 |

The structure thereof was characterized as follows:
ESI-MS (m/z): 569.3 [M+H]⁺.

¹H NMR (400 MHz, CD₃OD) δ 7.72 (d, J = 0.8 Hz, 1H), 7.34 (dd, J = 1.4, 10.2 Hz, 1H), 7.20 (s, 1H), 6.41 - 6.28 (m, 2H), 6.03 - 5.78 (m, 2H), 5.68 - 5.47 (m, 1H), 4.47 (s, 2H), 4.38 - 4.29 (m, 1H), 3.49 (dd, J = 3.6, 10.4 Hz, 1H), 2.74 - 2.53 (m, 1H), 2.37 (dd, J = 3.4, 10.8 Hz, 3H), 2.05 - 1.96 (m, 2H), 1.74 - 1.63 (m, 1H), 1.59 (s, 3H), 1.37 (dd, J = 7.8, 11.8 Hz, 1H), 1.17 (s, 3H), 1.02 (d, J = 7.4 Hz, 3H).

### Preparation example 32: (6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 4-(hydroxymethyl)furan-2-carboxylate (F-10)

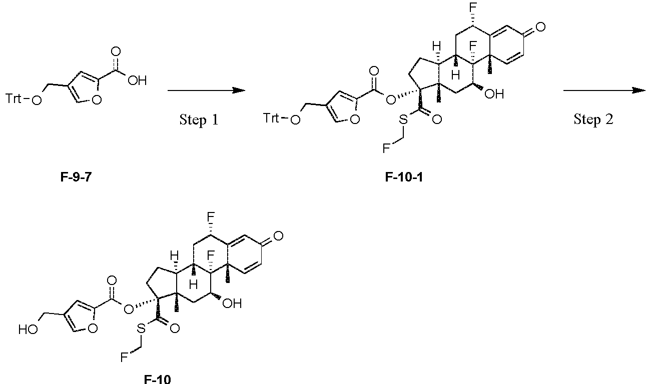

### Step 1: synthesis of (6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 4-((tributoxy)methyl)furan-2-carboxylate (F-10-1)

4-((triphenylmethoxy)methyl)furan-2-carboxylic acid (850 mg, 2.21 mmol) was dissolved in DMF (30.0 mL) under the atmosphere of nitrogen, and then the reaction solution was stirred for 1 h at 25°C after DIPEA (857 mg, 6.63 mmol) and HATU (841 mg, 2.21 mmol) were added. (6S,8S,9R,10S,11S,13S,14S,17R)-6,9-difluoro-11,17-dihydroxy-10,13-dimethyl-3-oxo-6,7, 8,9,10,11,12,13,14,15,16,17-dodecahydro-3H-cyclopenta[a]phenanthrene-17-thiocarboxylic acid (881 mg, 2.21 mmo) was added, and the reaction system was stirred for additional 2 h. Then, fluoroiodomethane (354 mg, 2.21 mmol) was added, and the reaction solution was stirred for additional 1 h. The reaction solution was poured into water (100 mL), stirred for 30 min, and filtered. The filter cake was washed with water for 3 times (10.0 mL x 3), and then dried in vacuum to obtain the title compound (1.40 g, 1.76 mmol), which was directly used in the next step without purification.

The structure thereof was characterized as follows:
ESI-MS (m/z): 796.3 [M+H]⁺.

### Step 2: Synthesis of (6S,85,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 4-(hydroxymethyl)furan-2-carboxylate (F-10)

(6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 4-((tributoxy)methyl)furan-2-carboxylate (900 mg, 1.13 mmol) was dissolved in dichloromethane (6.00 mL) and methanol (2.00 mL), and then the reaction solution was stirred for 2 h at 25°C after trifluoroacetic acid (1.54 g, 13.5 mmol) was added. The reaction solution was directly concentrated to obtain a crude product, which was purified via silica gel column chromatography (dichloromethane/methanol = 100/1 to 10/1) and concentrated again to obtain the title compound (461 mg, 803.34 µmol).

The structure thereof was characterized as follows:
ESI-MS (m/z): 555.0 [M+H]⁺.

¹H NMR (400 MHz, CD3OD) δ 7.68 (s, 1H), 7.31 (d, J = 10.2 Hz, 1H), 7.20 (s, 1H), 6.25-6.34 (m, 2H), 5.66-5.95 (m, 2H), 5.44-5.61 (m, 1H), 4.44 (s, 2H), 4.32 (br d, J = 8.4 Hz, 1H), 3.27 (s, 1H), 2.94-3.03 (m, 1H), 2.56-2.69 (m, 1H), 2.32-2.43 (m, 2H), 2.19-2.27 (m, 1H), 2.06-2.13 (m, 1H), 1.97 (d, J = 14.2 Hz, 1H), 1.72-1.80 (m, 1H), 1.64 (d, J = 12.8 Hz, 1H), 1.55 (s, 3H), 1.51 (s, 1H), 1.01 (s, 3H).

### Preparation example 33: (6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 5-(hydroxymethyl)furan-2-carboxylate (F-13)

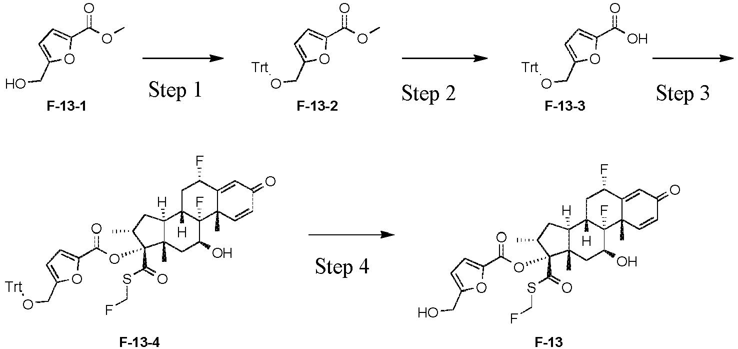

### Step 1: synthesis of methyl 5-((triphenylmethoxy)methyl)furan-2-carboxylate (F-13-2)

Methyl 5-(hydroxymethyl)furan-2-carboxylate (200 mg, 1.28 mmol) was dissolved in pyridine (2.00 mL), triphenylmethyl chloride (357 mg, 1.28 mmol) was added, and then the reaction solution was heated to 90°C and stirred for 12 h. 0.5 M diluted hydrchloric acid (20.0 mL) was added into the reaction solution, followed by extraction with dichloromethane (20.0 mL). The organic phase was dried over anhydrous sodium sulfate, filtered and concentrated to obtain the title compound as a crude product (621 mg, crude), which was directly used in the next step without purification.

### Step 2: Synthesis of 5-((triphenylmethoxy)methyl)furan-2-carboxylic acid (F-13-3)

Methyl 5-((triphenylmethoxy)methyl)furan-2-carboxylate (612 mg, 1.54 mmol) was dissolved in a mixed solvent of methanol (5.00 mL), tetrahydrofuran (5.00 mL) and water (5.00 mL), and then the reaction solution was stirred for 3 h at 25°C after lithium hydroxide monohydrate (193 mg, 4.61 mmol) was added. Water (30.0 mL) was added into the reaction solution, followed by extraction with dichloromethane (30.0 mL). The water phase was adjusted to pH 2-3 using 0.5 M dilute hydrochloric acid, and then extracted with dichloromethane for 3 times (10.0 mL x 3). The organic phases were combined, dried over anhydrous sodium sulfate, filtered and concentrated to obtain the title compound as a crude product (246 mg, crude), which was directly used in the next step without purification.

### Step 3: synthesis of (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 5-((triphenylmethoxy)methyl)furan-2-carboxylate (F-13-4)

5-((triphenylmethoxy)methyl)furan-2-carboxylic acid (183 mg, 476 µmol) and (6S,8S,9R,10S,11S,13S,14S,16R,17R)-6,9-difluoro-11,17-dihydroxy-10,13,16-trimethyl-3-oxo-6,7,8,9,10,11,12,13,14,15,16,17-dodecahydro-3H-cyclopenta[a]phenanthrene-17-thiocarboxylic acid (196 mg, 476 µmol) were dissolved in DMF (10.0 mL), and then the reaction solution was stirred for 2 h at 25°C after DIPEA (184 mg, 1.43 mmol) and HATU (181 mg, 476 µmol) were added. Then, fluoroiodomethane (76.1 mg, 476 µmol) was added, and the reaction solution was stirred for additional 3 h. The reaction solution was concentrated, followed by adding a saturated sodium chloride aqeuous solution (30.0 mL), and then the reaction solution was extracted with ethyl acetate twice (30.0 mL x 2). The organic phases were combined, dried over anhydrous sodium sulfate, filtered and concentrated to obtain the title compound as a crude product (488 mg, crude), which was directly used in the next step without purification.

### Step 4: Synthesis of (6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 5-(hydroxymethyl)furan-2-carboxylate (F-13)

(6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 5-((triphenylmethoxy)methyl)furan-2-carboxylate (488 mg, 601 µmol) was dissolved in a mixed solvent of dichloromethane (4.00 mL) and methanol (4.00 mL), and then the reaction solution was stirred for 1 h at 25°C after trifluoroacetic acid (6.14 g, 53.9 mmol) was added. The reaction solution was directly concentrated to obtain a crude product, which was purified via high performance liquid chromatography to obtain the title compound (53.8 mg, 93.9 µmol).
Chromatographic column: Welch Xtimate C18 150 mm×25 mm×5 µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 35 | 65 | 24 |
| 10.00 | 65 | 35 | 24 |

The structure thereof was characterized as follows:
ESI-MS (m/z): 569.3 [M+H]⁺.

¹H NMR (400 MHz, CD₃OD) δ 7.34 (d, J = 10.0 Hz, 1H), 7.13 (d, J = 3.4 Hz, 1H), 6.50 (d, J = 3.4 Hz, 1H), 6.40 - 6.26 (m, 2H), 6.02 - 5.77 (m, 2H), 5.68 - 5.45 (m, 1H), 4.55 (s, 2H), 4.34 (d, J = 9.2 Hz, 1H), 3.55 - 3.42 (m, 1H), 2.74 - 2.51 (m, 1H), 2.44 - 2.26 (m, 3H), 2.01 (d, J = 13.0 Hz, 2H), 1.74 - 1.61 (m, 1H), 1.58 (s, 3H), 1.41 - 1.31 (m, 1H), 1.17 (s, 3H), 1.02 (d, J = 7.2 Hz, 3H).

### Preparation example 34: (6S,8S,9R,10S,11S,13S,14S,16R,17R)-6,9-difluoro-11-hydroxy-17-((2-hydroxyethyl)thio)carbonyl-10,13,16-trimethyl-3-oxo-6,7,8,9,11,12,13,14,15,16,17-dodecahydro-3H-cyclopenta[a]phenanthren-17-yl furan-2-carboxylate (F-21)

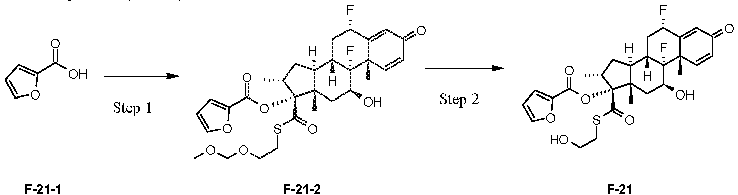

### Step 1: synthesis of (6S,8S,9R,10S,11S,13S,14S,16R,17R)-6,9-difluoro-11-hydroxy-17-((2-(methoxymethoxy)ethyl)thio)carbonyl)-10,13,16-trimethyl-3-oxo-6,7,8,9,11,12,13,14,14,15,16,17-dodecahydro-3H-cyclopenta[a]phenanthren-17-yl furan-2-carboxylate (F-21-2)

Furan-2-carboxylic acid (108 mg, 969 µmol) was dissolved in DMF (15.0 mL), and then the reaction solution was stirred for 1 h at 25°C after HATU (368 mg, 969 µmol) and DIPEA (375 mg, 2.91 mmol, 506 µL) were added. (6S,8S,9R,10S,11S,13S,14S,16R,17R)-6,9-difluoro-11,17-dihydroxy-10,13,16-trimethyl-3-oxo-6,7,8,9,10,11,12,13,14,15,16,17-dodecahydro-3H-cyclopenta[a]phenanthrene-17-thiocarboxylic acid (400 mg, 969 µmol) was added, and the reaction solution was stirred for additional 1 h. Then 1-iodo-2-(methoxymethoxy)ethane (209 mg, 969 µmol) was added, and the reaction solution was stirred for additional 1 h. Water was added into the reaction solution to precipitate a solid, filtered, and the filter cake was dried in vacuum to obtain the title compound as a crude product (458 mg, crude), which was directly used in the next step without purification.

The structure thereof was characterized as follows:
ESI-MS (m/z): 595.2 [M+H]⁺.

### Step 2: synthesis of (6S,8S,9R,10S,11S,13S,14S,16R,17R)-6,9-difluoro-11-hydroxy-17-((2-hydroxyethyl)thio)carbonyl-10,13,16-trimethyl-3-oxo-6,7,8,9,11,12,13,14,15,16,17-dodecahydro-3H-cyclopenta[a]phenanthren-17-yl furan-2-carboxylate (F-21)

(6S,8S,9R,10S,11S,13S,14S,16R,17R)-6,9-difluoro-11-hydroxy-17-((2-(methoxymethoxy)ethyl)thio)carbonyl)-10,13,16-trimethyl-3-oxo-6,7,8,9,11,12,13,14,14,15,16,17-dodecahydro-3H-cyclopenta[a]phenanthren-17-yl furan-2-carboxylate (450 mg, 756 µmol) was dissolved in dichloromethane (13.5 mL), and then the reaction solution was stirred for 1 h at 25°C after trifluoroacetic acid (4.14 g, 36.3 mmol, 2.70 mL) was added. The reaction solution was directly concentrated to obtain a crude product, which was purified via high performance liquid chromatography to obtain the title compound (86.9 mg, 154 µmol).
Chromatographic column: Welch Xtimate C18 200 mm×40 mm×10 µm
Mobile phase A: acetonitrile; mobile phase B: water (0.05% trifluoroacetic acid)

| Time [min] | Mobile phase A [%] | Mobile phase B [%] | Flow rate [mL/min] |
|---|---|---|---|
| 0.00 | 30 | 70 | 24 |
| 13.00 | 60 | 40 | 24 |

The structure thereof was characterized as follows:
ESI-MS (m/z): 551.1 [M+H]⁺.

¹H NMR (400 MHz, DMSO-*d*₆) δ 8.00 (d, J = 1.2 Hz, 1H), 7.27 (d, J = 10.8 Hz, 1H), 7.18 (d, J = 3.2 Hz, 1H), 6.68-6.71 (m, 1H), 6.29-6.34 (m, 1H), 6.12 (s, 1H), 5.54-5.63 (m, 2H), 4.22-4.27 (m, 1H), 3.47-3.51 (m, 3H), 3.00 (t, J = 6.8 Hz, 2H), 2.53-2.68 (m, 2H), 2.17-2.28 (m, 3H), 1.84-1.93 (m, 2H), 1.53-1.61 (m, 1H), 1.50 (s, 3H), 1.26-1.32 (m, 1H), 1.04 (s, 3H), 0.94 (m, 3H).

### Preparation example 35: (6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl (E)-3-(3-amino-4-fluorophenyl)acrylate (E-4)

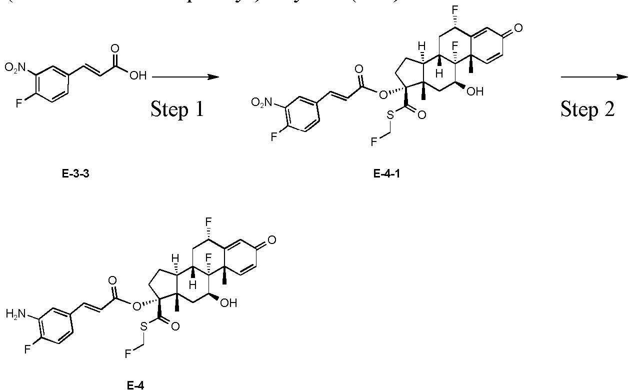

### Step 1: synthesis of (6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl (E)-3-(3-nitro-4-fluorophenyl)acrylate (E-4-1)

(E)-3-(4-fluoro-3-nitro-phenyl)prop-2-enoic acid (263 mg, 1.25 mmol), pyridine (344.84 mg, 4.36 mmol) and a 50% solution of 1-propylphosphonic anhydride (2.30 g, 7.22 mmol) in DMF were successively added into dichloromethane (10 mL), and then the reaction solution was allowed to react for 1 h at 25°C after the addition was finished. (6S,8S,9R,10S,11S,13S,14S,17R)-6,9-difluoro-11,17-dihydroxy-10,13-dimethyl-3-oxo-6,7,8,9,10,11,12,13,14,15,16,17-dodecahydro-3H-cyclopenta[a]phenanthrene-17-thiocarboxylic acid (71 mg, 181.76 mmol) was added, and then the reaction solution was allowed to react for 3 h at room temperature after the addition was finished. Fluoroiodomethane (400 mg, 2.50 mmol) was added, and then the reaction solution was allowed to react at room temperature after the addition was finished. After the reaction was completed, the reaction solution was extracted with water (30 mL) and dichloromethane (20 mL x 2), dried and then concentrated, and the concentrate was purified via a silica gel column (petroleum ether/ethyl acetate = 100/1 to 50/1) and then concentrated again to obtain the title compound as a crude product (94 mg).

The structure characterization data is as follows:
MS m/z (ESI): 624.1[M+H]⁺.

### Step 2: synthesis of (6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl (E)-3-(3-amino-4-fluorophenyl)acrylate (E-4)

(6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl (E)-3-(3-nitro-4-fluorophenyl)acrylate (94 mg, crude), iron powder (24.18 mg, 432.96 µmol) and ammonium chloride (11.58 mg, 216.48 µmol) were added into ethanol (1 mL) and water (0.3 mL), and then the reaction solution was heated to 80°C and allowed to react after the addition was finished. After the reaction was completed, water (8 mL) was added, and then the reaction solution was extracted with ethyl acetate (6 mL x 2) and filtered through diatomite. The organic phase was dried, concentrated to remove the solvent, and then purified via preparative high performance liquid chromatography and freeze-dried to obtain the title compound (2.46 mg, 0.004 mmol).

The structure thereof was characterized as follows:
MS m/z (ESI): 594.2[M+H]⁺.

The purification method is as follows:
Chromatographic column: Waters SunFire Prep C18 OBD (5 µm x 19 mm x 150 mm)
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 40 | 60 | 28 |
| 2.00 | 40 | 60 | 28 |
| 24.00 | 90 | 10 | 28 |

### Preparation example 36: (6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-(hydroxymethyl)benzoate (B-31)

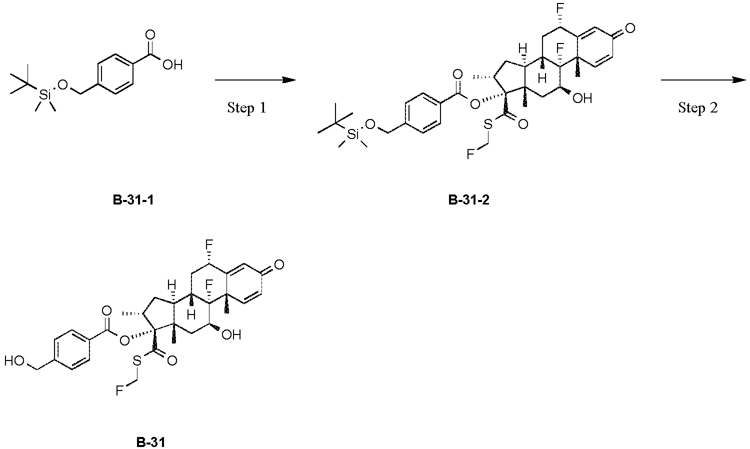

### Step 1: synthesis of (6S,8S,9R,10S,1IS,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-((tert-butyldimethylsilyl)oxy)methyl)benzoate (B-31-2)

4-((tert-butyldimethylsilyl)oxy)methyl)benzoic acid (70 mg, 262.76 µmol) was added into DMF (8.00 mL), followed by adding HATU (99.91 mg, 262.76 µmol) and DIPEA (50.94 mg, 394.15 µmol), and then the reaction solution was allowed to react for 15 min at 25°C under stirring. (6S,8S,9R,10S,1IS,13S,14S,16R,17R)-6,9-difluoro-11,17-dihydroxy-10,13,16-trimethyl-3-oxo-6,7,8,9,10,11,12,13,14,15,16,17-dodecahydro-3H-cyclopenta[a]phenanthrene-17-S-carboxylic acid (127.51 mg, 262.76 µmol) was added into the reaction system, DIPEA (50.94 mg, 394.15 µmol) was then added, and the reaction solution was stirred for 1.5 h at 45°C. Then fluoroiodomethane (210.11 mg, 1.31 mmol) was added dropwise into the reaction system, and then DIPEA (102 mg, 788.28 µmol) was added. The reaction solution was stirred for 45 min at 45°C, extracted with water and EA, and concentrated to obtain a crude product, which was purified via high performance liquid chromatography to obtain the title compound (14 mg, 20.20 µmol).
Chromatographic column: Phenomenex C18 250mm×50mm×10µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 50 | 50 | 28 |
| 2.00 | 50 | 50 | 28 |
| 15.00 | 95 | 5 | 28 |

The structure thereof was characterized as follows:
ESI-MS (m/z): 693.4 (M+H)⁺.

### Step 2: synthesis of (6S,85,9R,10S,118,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-(hydroxymethyl)benzoate (B-31)

(6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-((tert-butyldimethylsilyl)oxy)methyl)benzoate (16 mg, 23.09 µmol) was dissolved in DCM (3.00 mL) and TFA (1.5 mL), and the reaction solution was stirred for 1 h at 25°C and then concentrated to obtain a crude product, which was purified via high performance liquid chromatography to obtain the title compound (7 mg, 11.98 µmol).
Chromatographic column: Phenomenex C18 250mm×50mm×10µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 40 | 60 | 30 |
| 2.00 | 40 | 60 | 30 |
| 18.00 | 100 | 0 | 30 |

The structure thereof was characterized as follows:
ESI-MS (m/z): 579.3 (M+H)⁺.

¹H NMR (400 MHz, DMSO) δ 7.83 (d, J = 8.4 Hz, 2H), 7.49 (d, J = 8.4 Hz, 2H), 7.29 (d, J = 10.0 Hz, 1H), 6.33 (dd, J = 1.6 Hz, 10.0 Hz, 1H), 6.14 (s, 1H), 6.03 (s, 1H), 5.90 (s, 1H), 5.80-5.56 (m, 2H), 5.38 (t, J = 6.0 Hz, 1H), 4.56 (d, J = 5.6 Hz, 2H), 4.33-4.24 (m, 1H), 3.48-3.37 (m, 1H), 2.67-2.53 (m, 1H), 2.32-2.21 (m, 3H), 2.02-1.88 (m, 2H), 1.65-1.54 (m, 1H), 1.51 (s, 3H), 1.38-1.29 (m, 1H), 1.07 (s, 3H), 0.92 (d, J = 6.8 Hz, 3H).

### Preparation example 37: (6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 3-(hydroxymethyl)benzoate (B-35)

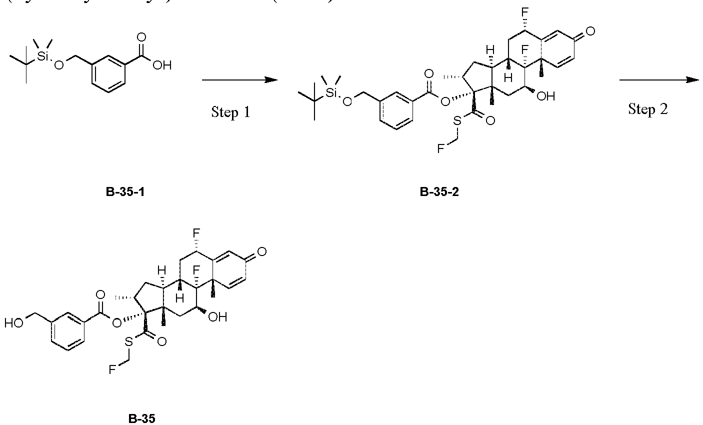

### Step 1: synthesis of (6S,85,9R,108,118,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 3-((tert-butyldimethylsilyl)oxy)methyl)benzoate (B-35-2)

3-((tert-butyldimethylsilyl)oxy)methyl)benzoic acid (280 mg, 1.05 mmol) was added into DMF (15.00 mL), followed by adding HATU (399.64 mg, 1.05 mmol) and DIPEA (203.75 mg, 1.58 mmol), and then the reaction solution was allowed to react for 15 min at 25°C under stirring. (6S,8S,9R,10S,11S,13S,14S,16R,17R)-6,9-difluoro-11,17-dihydroxy-10,13,16-trimethyl-3-oxo-6,7,8,9,10,11,12,13,14,15,16,17-dodecahydro-3H-cyclopenta[a]phenanthrene-17-S-carboxylic acid (510.04 mg, 1.05 mmol) was added into the reaction system, DIPEA (203.75 mg, 1.58 mmol) was added, and the reaction solution was stirred for 1.5 h at 45°C. Then fluoroiodomethane (672.36 mg, 4.20 mmol) was added dropwise into the reaction system, and then DIPEA (407.50 mg, 3.16 mmol) was added. After the reaction solution was stirred for 2 h at 45°C, the reaction was monitored via LC-MS until it was completed. The reaction solution was extracted with water and EA, and concentrated to obtain a crude product, which was purified via high performance liquid chromatography to obtain the title compound (35 mg, 50.51 µmol).
Chromatographic column: Phenomenex C18 250 mm×50 mm×10 µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 70 | 30 | 30 |
| 2.00 | 70 | 30 | 30 |
| 12.00 | 90 | 10 | 30 |
| 18.00 | 90 | 10 | 30 |

The structure thereof was characterized as follows:
ESI-MS (m/z): 693.4 (M+H)⁺.

### Step 2: synthesis of (6S,8S,9R,10S,118,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 3-(hydroxymethyl)benzoate (B-35)

(6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 3-((tert-butyldimethylsilyl)oxy)methyl)benzoate (35 mg, 50.51 µmol) was dissolved in DCM (4.00 mL) and TFA (2.00 mL), and the reaction solution was stirred for 1.5 h at 25°C. The reaction was monitored via LC-MS until it was completed. The reaction solution was concentrated to obtain a crude product, which was purified via high performance liquid chromatography to obtain the title compound (11 mg, 18.82 µmol).
Chromatographic column: Phenomenex C18 250 mm×50 mm×10 µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 35 | 65 | 30 |
| 2.00 | 35 | 65 | 30 |
| 18.00 | 100 | 0 | 30 |

The structure thereof was characterized as follows:
ESI-MS (m/z): 579.3 (M+H)⁺.

¹H NMR (400 MHz, DMSO) δ 7.87 (s, 1H), 7.74 (d, J = 7.6 Hz, 1H), 7.61 (d, J = 8.0 Hz, 1H), 7.52 (d, J = 7.6 Hz, 1H), 7.29 (dd, J = 1.2 Hz, 10.4 Hz, 1H), 6.33 (dd, J = 2.0 Hz, 10.0 Hz, 1H), 6.14 (s, 1H), 6.03 (s, 1H), 5.90 (s, 1H), 5.80-5.55 (m, 2H), 5.34 (t, J = 6.0 Hz, 1H), 4.52 (d, J = 5.6 Hz,2H), 4.33-4.25 (m, 1H), 3.48-3.36 (m, 1H), 2.69-2.54 (m, 1H), 2.33-2.21 (m, 3H), 2.02-1.88 (m, 2H), 1.67-1.52 (m, 1H), 1.51 (s, 3H), 1.38-1.30 (m, 1H), 1.08 (s, 3H), 0.93 (d, J = 6.8 Hz, 3H).

### Preparation example 38: (6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-hydroxyfuran-2-carboxylate (F-17)

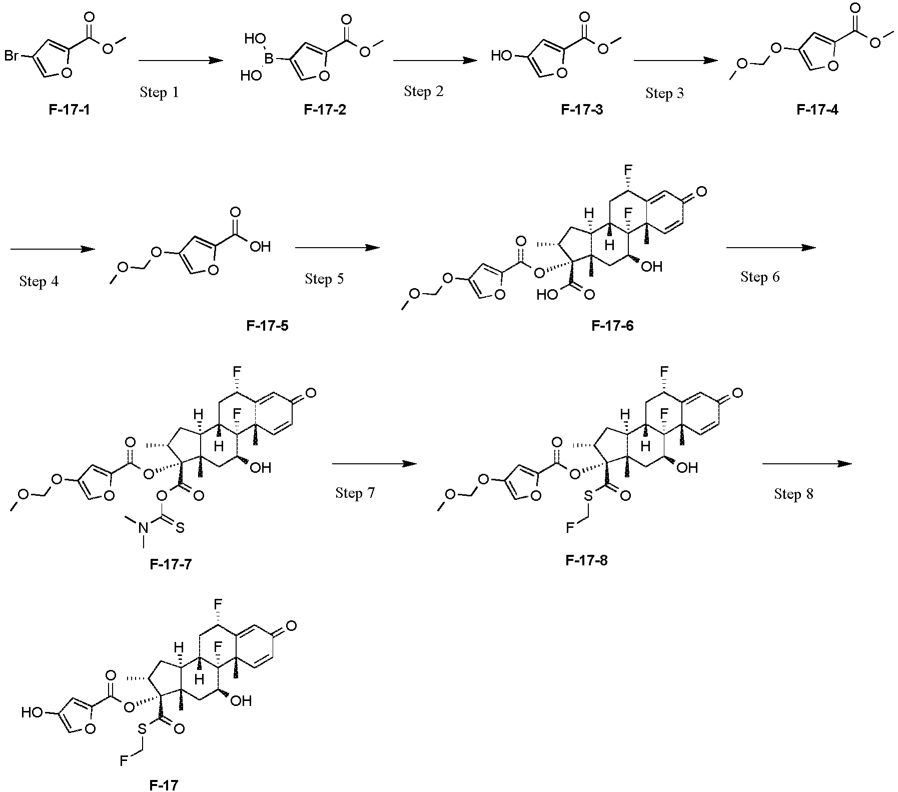

### Step 1: synthesis of (5-(methoxycarbonyl)furan-3-yl)boric acid (F-17-2)

Methyl 4-bromo-furan-2-carboxylate (2 g, 9.76 mmol), bis(pinacolato)diboron (4.95 g, 19.51 mmol) and KOAc (2.87 g, 29.27 mmol, 167.68 µL) were added into 1,4-dioxane (200 mL), nitrogen was introduced in the reaction system for replacement, and then Pd(dppf)Cl₂ (722.38 mg, 975.58 µmol) was added. Nitrogen was introduced again into the reaction system for replacement for 3 times, and then the reaction system was allowed to react in an 80 °C oil bath for 24 h. After the reaction was completed, the reaction solution was cooled to room temperature and filtered through diatomite. The filter cake was washed with ethyl acetate, and then the filtrate was directly spinned to dryness to obtain a crude product, which was purified via silica gel column chromatography (ethyl acetate/petroleum ether = 0% to 40%) and then concentrated again to obtain the title compound (4.6 g, 9.12 mmol) (overweight, with a borate residue).

ESI-MS (m/z): 253.2 (M+H)⁺.

### Step 2: synthesis of methyl 4-hydroxyfuran-2-carboxylate (F-17-3)

(5-(methoxycarbonyl)furan-3-yl)boric acid (4.7 g, 27.66 mmol) was dissolved in THF (40 mL), H₂O₂ (40.00 g, 352.79 mmol, 40 mL, 30% purity) was slowly added dropwise, during which a large amount of heat was released from the system, and then the reaction system was allowed to react for 5 h while maintaining the temperature. After the reaction was completed, 20 mL of water was added into the reaction solution, followed by extraction with ethyl acetate twice (10 mL x 2). The organic phase was washed alternately with a saturated sodium sulfite aqueous solution and a saturated sodium chloride aqueous solution, until the separated water layer does not show blue color when being detected using starchpotassium iodide test paper. The organic phases were combined, dried over anhydrous sodium sulfate, and concentrated under reduced pressure to obtain the title compound as a crude product (1.54 g, 10.84 mmol), which was directly used in the next step without purification.

ESI-MS (m/z): 143.0 (M+H)⁺.

### Step 3: synthesis of methyl 4-(methoxymethoxy)furan-2-carboxylate (F-17-4)

Methyl 4-hydroxyfuran-2-carboxylate (1.64 g, 11.54 mmol) was dissolved in DMF (50 mL), and the reaction solution was cooled to 0 °C under the protection of nitrogen. Then NaH (553.89 mg, 13.85 mmol, 60% purity) was added in batches, during which the system turned from orange yellow to brick red. The system was allowed to react for 30 min at 0°C under stirring while maintaining the temperature. At the controlled temperature of 0°C, a solution of bromomethyl methyl ether (2.88 g, 23.08 mmol) in DMF (1 mL) was slowly added dropwise, during which the system gradually turned yellow. After the addition was finished, the system was allowed to react for 30 min while maintaining the temperature. After the reaction was completed, water (100 mL) was added into the reaction solution for quenching. The reaction solution was extracted with ethyl acetate for 3 times (50 mL x 3). The organic phases were combined, washed with a saturated saline solution and then concentrated under reduced pressure to obtain a crude product, which was purified via silica gel column chromatography (ethyl acetate/petroleum ether = 0% to 20%) and then concentrated again to obtain the title compound (400 mg, 2.15 mmol).

ESI-MS (m/z): 187.1 (M+H)⁺.

### Step 4: synthesis of 4-(methoxymethoxy) furan-2-carboxylic acid (F-17-5)

Methyl 4-(methoxymethoxy)furan-2-carboxylate (400 mg, 2.15 mmol) was dissolved in methanol (10 mL), followed by adding KOH (301.38 mg, 5.37 mmol), and then the reaction solution heated to 35°C and was allowed to react for 6 h under stirring. After the reaction was completed, the reaction solution was directly spinned to dryness. Then water (5 mL) was added, and the system was adjusted to pH 4-5 using 2 N diluted hydrochloric acid, and then extracted with ethyl acetate, with an undesired effect. The organic phase was concentrated under reduced pressure, combined with a water phase, and then purified via high performance liquid chromatography to obtain the title compound (180 mg, 1.05 mmol).

The purification conditions are as follows:
Chromatographic column: Waters Sunfire Prep C18 OBD (5 µm x19 mm x 150 mm)
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 10 | 90 | 30 |
| 2.00 | 10 | 90 | 30 |
| 18.00 | 90 | 10 | 30 |

The structure thereof was characterized as follows:
ESI-MS (m/z):173.1 [M+H]⁺.

### Step 5: synthesis of (6S,8S,9R,10S,11S,13S,14S,16R,17R)-6,9-difluoro-11-hydroxy-17-(4-(methoxymethoxy)furan-2-carbonyl)oxy)-10,13,16-trimethyl-3-oxo-6,7,8,9,10,11,12,13,14,15,16,17-dodecahydro-3H-cyclopenta[a]phenanthrene-17-carboxylic acid (F-17-6)

4-(methoxymethoxy) furan-2-carboxylic acid (85 mg, 493.80 µmol) was dissolved in DMF (10 mL), followed by adding DIPEA (191.46 mg, 1.48 mmol) and HATU (187.76 mg, 493.80 µmol), and then the reaction solution was heated to 45°C, allowed to react for 1 h under stirring, and then cooled to room temperature. A DMF solution (2 mL) of (6S,8S,9R,10S,11S,13S,14S,16R,17R)-6,9-difluoro-11,17-dihydroxy-10,13,16-trimethyl-3-oxo-8,9,10,11,12,13,14,15,16,17-dodecahydro-3H-cyclopenta[a]phenanthrene-17-carboxylic acid (217.50 mg, 493.80 µmol) was added, and the reaction solution was stirred for 16 h at room temperature. After the reaction was completed, water (60 mL) was added, and then the reaction solution was adjusted to pH 4-5 using a 0.5 M citric acid aqueous solution so as to precipitate a large amount of white flocculent solid. The solid was filtered, and the filter cake was collected and dried in vacuum to obtain the title compound as a crude product (220 mg, 399.60 µmol), which was directly used in the next step without purification.

ESI-MS (m/z):c551.2 [M+H]⁺.

### Step 6: synthesis of (6S,8S,9R,10S,11S,13S,14S,16R,17R)-6,9-difluoro-11-hydroxy-17-(4-(methoxymethoxy)furan-2-carbonyl)oxy)-10,13,16-trimethyl-3-oxo-6,7,8,9,10,11,12,13,14,15,16,17-dodecahydro-3H-cyclopenta[a]phenanthrene-17-carboxylic dimethylaminothioformic anhydride (F-17-7)

(6S,8S,9R,10S,11S,13S,14S,16R,17R)-6,9-difluoro-11-hydroxy-17-(4-(methoxymethoxy)furan-2-carbonyl)oxy)-10,13,16-trimethyl-3-oxo-6,7,8,9,10,11,12,13,14,15,16,17-dodecahydro-3H-cyclopenta[a]phenanthrene-17-carboxylic acid (100 mg, 181.64 µmol) was dissolved in acetone (2 mL), subsequently DIPEA (35.21 mg, 272.46 µmol) and KI (18.09 mg, 108.98 µmol) were added, and then dimethylaminothioformyl chloride (56.13 mg, 454.10 µmol) was added in batches under the protection of nitrogen. The reaction solution was allowed to react for 4 h at room temperature under stirring after the addition was finished. After the reaction was completed, water (10 mL) was added into the reaction solution, followed by extraction with ethyl acetate (5 mL x 3). The organic phase was washed with a saturated saline solution, dried over anhydrous sodium sulfate and then concentrated under reduced pressure to obtain the title compound as a crude product (110 mg, 172.50 µmol), which was directly used in the next step without purification.

ESI-MS (m/z): 639.3 (M+H)⁺.

### Step 7: synthesis of (6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-(methoxymethoxy)furan-2-carboxylate (F-17-8)

(6S,8S,9R,10S,11S,13S,14S,16R,17R)-6,9-difluoro-11-hydroxy-17-(4-(methoxymethoxy)furan-2-carbonyl)oxy)-10,13,16-trimethyl-3-oxo-6,7,8,9,10,11,12,13,14,15,16,17-dodecahydro-3H-cyclopenta[a]phenanthrene-17-carboxylic dimethylaminothioformic anhydride (110 mg, 172.50 µmol) was dissolved in DMAc (4.0 mL), and then NaSH (29.01 mg, 517.50 µmol) was added in batches, so that the system instantly turned dark green. After the addition was finished, the system was allowed to react for 3 h at room temperature under stirring. The reaction was monitored via LCMS for the remaining starting material, NaSH (29.01 mg, 517.50 µmol) was supplemented, and the system was stirred for additional 0.5 h at room temperature. The reaction was monitored via LCMS until no starting material remained, and there was a significant amount of the thiocarboxylic acid intermediate. Fluoroiodomethane (275.87 mg, 1.72 mmol) was added dropwise, and then the system instantly turned earthy yellow, which was stirred for 0.5 h at room temperature. After the reaction was completed, water (10 mL) was added into the reaction solution, followed by extraction with ethyl acetate (10 mL x 2). The organic phases were combined, washed with a saturated saline solution, dried over anhydrous sodium sulfate and concentrated under reduced pressure to obtain a crude product, which was purified via thin-layer chromatography (ethyl acetate/petroleum ether = 1:1) to obtain the title compound (86 mg, 143.66 µmol).

ESI-MS (m/z): 599.2 (M+H)⁺.

### Step 8: synthesis of (6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-hydroxyfuran-2-carboxylate (F-17)

A hydrogen chloride-1,4-dioxane solution (4 mL) was added into (6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-(methoxymethoxy)furan-2-carboxylate (35 mg, 58.47 µmol), and then the reaction solution was allowed to react for 1 h at room temperature under stirring after the addition was finished. After the reaction was completed, the reaction solution was directly concentrated under reduced pressure to obtain a crude product, which was purified via high performance liquid chromatography to obtain the title compound (180 mg, 1.05 mmol).

The preparation conditions are as follows:
Chromatographic column: Waters Sunfire Prep C18 OBD (5 µm x19 mm x 150 mm)
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 40 | 60 | 28 |
| 2.00 | 40 | 60 | 28 |
| 22.00 | 90 | 10 | 28 |

The structure thereof was characterized as follows:
ESI-MS (m/z): 555.3 [M+H]⁺.

¹H NMR (400 MHz, DMSO) δ 9.13 (s, 1H), 7.50 (d, J = 1.2 Hz, 1H), 7.28 (dd, J = 10.2, 1.6 Hz, 1H), 6.86 (d, J = 1.1 Hz, 1H), 6.30 (dd, J = 10.4, 1.6 Hz, 1H), 6.16 (s, 1H), 6.00 (s, 1H), 5.88 (s, 1H), 5.72 - 5.53 (m, 1H), 5.46 - 5.42 (m, 1H), 4.29 (d, J = 7.2 Hz, 1H), 3.37 (ddd, J = 10.6, 7.2, 3.6 Hz, 1H), 2.61 (ddd, J = 21.6, 16.4, 3.2 Hz, 1H), 2.35 - 2.19 (m, 3H), 1.95 (dd, J = 22.4, 11.6 Hz, 2H), 1.65 - 1.54 (m, 1H), 1.53 (s, 3H), 1.37 - 1.29 (m, 1H), 1.09 (s, 3H), 0.96 (d, J = 7.2 Hz, 3H).

### Preparation example 39: (6S,8S,9R,10S,11S,13 S, 14S, 17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 4-fluoro-3-((S)-2-((S)-2-(6-(2-(methylsulfonyl)pyrimidin-5-yl)hex-5-ynylamino)propionamino)benzoate (DL-A-03)

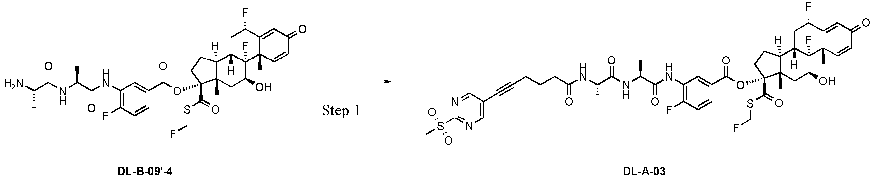

(6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-2-((S)-2-aminopropionamido)propionamido)-4-fluorobenzoate (25 mg, 30.35 µmol), 2,5-dioxopyrrolidin-1-yl 6-(2-(methylsulfonyl)pyrimidin-5-yl)hex-5-ynoate (11.09 mg, 30.35 µmol) and DIPEA (11.77 mg, 91.04 µmol) were added into DMF (2.00 mL), and the reaction solution was allowed to react for 1 h at 25°C. The reaction solution was filtered to obtain a crude product, which was purified via high performance liquid chromatography to obtain the title compound (18 mg, 18.56 µmol).

The purification method is as follows:
Chromatographic column: Phenomenex C18 250mm×50mm×10µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 30 | 70 | 30 |
| 2.00 | 30 | 70 | 30 |
| 18.00 | 90 | 10 | 30 |

The structure thereof was characterized as follows:
ESI-MS (m/z): 960.3 (M+H)⁺.

### Preparation example 40: (S)-4-((5-((((6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl)oxy)carbonyl-2-fluorophenyl)carbamoyl)-30-(2-(methylsulfonyl)pyrimidin-5-yl)-6,9,25-trioxo-12,15,18,21-tetraoxa-5,8,24-triazatriacontan-29-ynoic acid (DL-A-47)

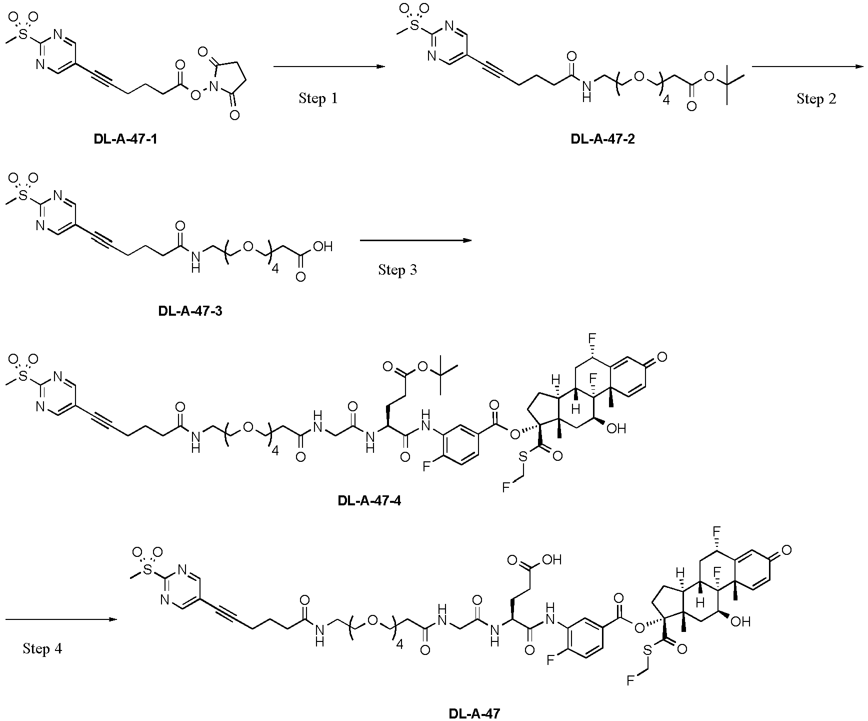

### Step 1: preparation of tert-butyl 22-(2-(methylsulfonyl)pyrimidin-5-yl)-17-oxo-4,7,10,13-tetraoxa-16-azadocosan-21-ynoate (DL-A-47-2)

Tert-butyl 1-amino-3,6,9,12-tetraoxypentadecane-15-carboxylate (117 mg, 364.02 µmol), 2,5-dioxopyrrolidin-1-yl 6-(2-(methylsulfonyl)pyrimidin-5-yl)hex-5-ynoate (146.30 mg, 400.43 µmol) and DIPEA (94.09 mg, 728.05 µmol) were successively added into DMF (2 mL), and the reaction solution was allowed to react for 3 h at 25°C. Water (8 mL) was added into the reaction solution, followed by extraction with ethyl acetate for 3 times (8 mL x 3). The organic phases were combined, washed with a saturated sodium chloride aqueous solution, dried over anhydrous sodium sulfate, filtered and concentrated to obtain the title compound (259 mg), which was directly used in the next step without purification.

### Step 2: preparation of 22-(2-(methylsulfonyl)pyrimidin-5-yl)-17-oxo-4,7,10,13-tetraoxa-16-azadocosan-21-ynoic acid (DL-A-47-3)

Tert-butyl 22-(2-(methylsulfonyl)pyrimidin-5-yl)-17-oxo-4,7,10,13-tetraoxa-16-azadocosan-21-ynoate (100 mg, 167.93 µmol, FR) was dissolved in dichloromethane (2 mL), trifluoroacetic acid (0.5 mL) was then added, and the reaction solution was allowed to react for 2 h at 25°C under stirring. The reaction solution was directly concentrated under reduced pressure to remove dichloromethane, and then placed on a freeze dryer to remove trifluoroacetic acid so as to obtain the title compound as a crude product (86 mg), which was directly used in the next step without purification.

### Step 3: preparation of tert-butyl (S)-4-((5-((((6S,8S,9R,10S,11S,13S,14S,17R)-6, 9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl)oxy)carbonyl-2-fluorophenyl)carbamoyl)-30-(2-(methylsulfonyl)pyrimidin-5-yl)-6,9,25-trioxo-12,15,18,21-tetraoxa-5,8,24-triazatriacontan-29-ynoate (DL-A-47-4)

(6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-2-(2-aminoacetamido)-5-(tert-butoxy)-5-oxopentanamido)-4-fluorobenzoate (40mg, 49.39 µmol), 22-(2-(methylsulfonyl)pyrimidin-5-yl)-17-oxo-4,7,10,13-tetraoxa-16-azadocosan-21-ynoic acid (25.46 mg, 49.39 µmol) and DIPEA (19.15 mg, 148.17 µmol) were successively added into DMF (2 mL), and then the reaction solution was allowed to react for 1.5 h at 16°C after the addition was finished. Purified water (8 mL) was added into the reaction solution, followed by extraction with ethyl acetate twice (6 mL*2). The organic phase was washed with a saturated saline solution (4 mL) once. After the solvent was removed under reduced pressure, the title compound as a crude product (130 mg) was obtained, which was directly used in the next step without purification.

### Step 4: preparation method of (S)-4-((5-((((6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl)oxy)carbonyl-2-fluorophenyl)carbamoyl)-30-(2-(methylsulfonyl)pyrimidin-5-yl)-6,9,25-trioxo-12,15,18,21-tetraoxa-5,8,24-triazatriacontan-29-ynoic acid (DL-A-47)

Tert-butyl (S)-4-((5-((((6S,8S,9R,10S,11S,13S,14S,17R)-6, 9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl)oxy)carbonyl-2-fluorophenyl)carbamoyl)-30-(2-(methylsulfonyl)pyrimidin-5-yl)-6,9,25-trioxo-12,15,18,21-tetraoxa-5,8,24-triazatriacontan-29-ynoate crude product (51 mg) was dissolved in dichloromethane (3 mL), trifluoroacetic acid (0.5 mL) was added, and then the reaction solution was allowed to react for 3 h at 16°C after the addition was finished. The reaction solution was directly purified via high performance liquid chromatography to obtain the title compound (17.51 mg, 13.85 µmol).

The purification method is as follows:
Chromatographic column: SunFire Prep C18 OBD 19*150 mm*5 µm
Mobile phase A: acetonitrile; mobile phase B: water (0.05%TFA)

| Time [min] | Mobile phase A [%] | Mobile phase B [%] | Flow rate [mL/min] |
|---|---|---|---|
| 0.00 | 30 | 70 | 28 |
| 2.00 | 30 | 70 | 28 |
| 20.00 | 90 | 10 | 28 |

The structure characterization data is as follows:
ESI-MS (m/z): 1251.4 (M+H)⁺.

### Preparation example 41: (S)-4-((5-((((6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl)oxy)carbonyl)-2-fluorophenyl)carbamoyl)-42-(2-(methylsulfonyl)pyrimidin-5-yl)-6,9,37-trioxo-12, 15, 18,21,24,27,30,33-octaoxa-5,8,36-triazatetratriacontan-41-ynoic acid (DL-A-48)

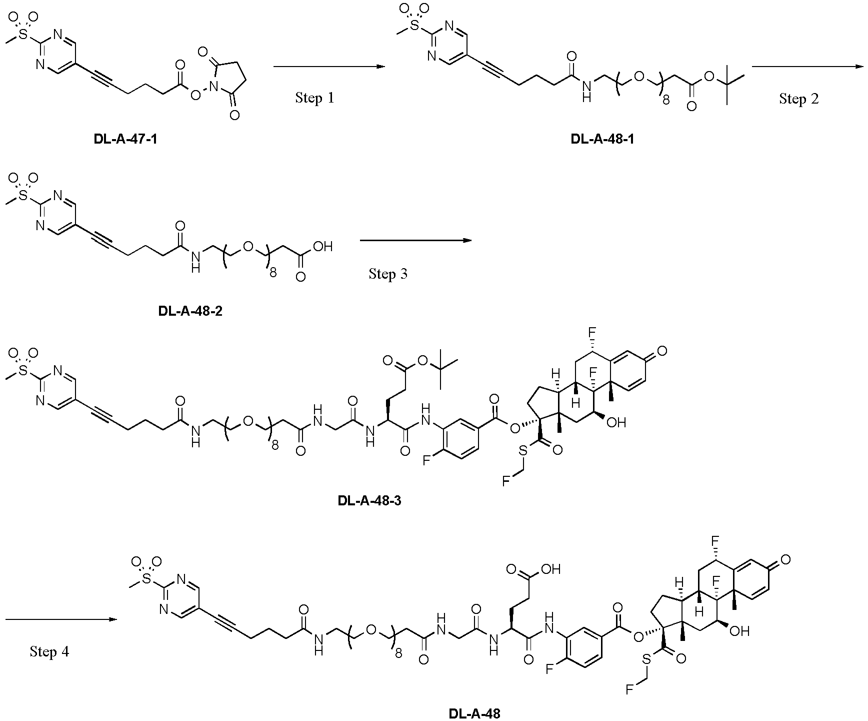

### Step 1: preparation of tert-butyl 34-(2-(methylsulfonyl)pyrimidin-5-yl)-29-oxo-4,7,10,13,16,19,22,25-octaoxa-28-azatetratriacontan-ynoate (DL-A-48-1)

2,5-dioxopyrrolidin-1-yl 6-(2-(methylsulfonyl)pyrimidin-5-yl)hex-5-ynoate (100 mg, 0.274 mmol), Tert-butyl 1-amino-3,6,9,12,15,18,21,24-octaoxaheptacosane-27-carboxylate (136.2 mg, 0.274 mmol) and DIPEA (70.8 mg, 0.547 mmol) were added into THF (2 mL), and then the reaction solution was allowed to react for 2 h at 25°C. Ethyl acetate (20 ml) and water (6 ml) were added, and the reaction solution was stirred for 5 min. The organic phase was separated out, washed with a saturated sodium chloride aqueous solution (5 ml), dried over anhydrous sodium sulfate, and concentrated to obtain the title compound (174 mg), which was directly used in the next step without purification.

The structure characterization data is as follows:
ESI-MS (m/z): 748.4 (M+H)⁺.

### Step 2: preparation of 34-(2-(methylsulfonyl)pyrimidin-5-yl)-29-oxo-4,7,10,13,16,19,22,25-octaoxa-28-azatetratriacontan-ynoic acid (DL-A-48-2)

Tert-butyl 34-(2-(methylsulfonyl)pyrimidin-5-yl)-29-oxo-4,7,10,13,16,19,22,25-octaoxa-28-azatetratriacontan-ynoate (174 mg, 232.65 µmol, FR) and trifluoroacetic acid (530.54 mg, 4.65 mmol) were added into dichloromethane (3 mL), and the reaction solution was heated to 35°C and allowed to react for 2 h. The reaction solution was directly concentrated to obtain the title compound (185 mg), which was directly used in the next step without purification.

The structure characterization data is as follows:
ESI-MS (m/z): 692.4 (M+H)⁺.

### Step 3: preparation of tert-butyl (S)-4-((5-((((6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl)oxy)carbonyl)-2-fluorophenyl)carbamoyl)-42-(2-(methylsulfonyl)pyrimidin-5-yl)-6,9,37-trioxo-12,15,18,21,24,27,30,33-octaoxa-5,8,36-triazatetratriacontan-41-yonate (DL-A-48-1)

(6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-2-(2-aminoacetamido)-5-(tert-butoxy)-5-oxopentanamido)-4-fluorobenzoate (43 mg, 53.10 µmol), 34-(2-(methylsulfonyl)pyrimidin-5-yl)-29-oxo-4,7,10,13,16,19,22,25-octaoxa-28-azatetratriacontan-ynoic acid (36.73 mg, 53.10 µmol), DIPEA (20 mg, 154.7 µmol) and HATU (24.23 mg, 63.71 µmol) were added into DMF (2 mL), and then the reaction solution was allowed to react for 1.5 h at 16°C under stirring after the addition was finished. Water (8 mL) was added into the reaction solution, followed by extraction with ethyl acetate twice (6 mL x 2). The organic phase was washed with a saturated saline solution (6 mL) once, dried over anhydrous sodium sulfate, filtered and concentrated to obtain the title compound as a crude product (40 mg), which was directly used in the next step without purification.

The structure characterization data is as follows:
ESI-MS (m/z): 1483.6 (M+H)⁺.

### Step 4: preparation of (S)-4-((5-((((6S,8S,9R, 10S,11S,13 S, 14S, 17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl)oxy)carbonyl)-2-fluorophenyl)carbamoyl)-42-(2-(methylsulfonyl)pyrimidin-5-yl)-6,9,37-trioxo-12,15,18,21,24,27,30,33-octaoxa-5,8,36-triazatetratriacontan-41-ynoic acid (DL-A-48)

Tert-butyl (S)-4-((5-((((6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl)oxy)carbonyl)-2-fluorophenyl)carbamoyl)-42-(2-(methylsulfonyl)pyrimidin-5-yl)-6,9,37-tiioxo-12,15,18,21,24,27,30,33-octaoxa-5,8,36-tiiazatetratriacontan-41-yonate (40 mg) was dissolved in dichloromethane (3 mL), trifluoroacetic acid (1 mL) was added, and then the reaction solution was allowed to react for 2 h at 15°C under stirring after the addition was finished. The reaction solution was directly purified via high performance liquid chromatography to obtain the title compound (16.54 mg, 11.01 µmol).

The purification method is as follows:
Chromatographic column: SunFire Prep C18 OBD 19*150 mm*5 µm
Mobile phase A: acetonitrile; mobile phase B: water (0.05%TFA)

| Time [min] | Mobile phase A [%] | Mobile phase B [%] | Flow rate [mL/min] |
|---|---|---|---|
| 0.00 | 30 | 70 | 28 |
| 2.00 | 30 | 70 | 28 |
| 20.00 | 90 | 10 | 28 |

The structure characterization data is as follows:
ESI-MS (m/z): 1427.6 (M+H)⁺.

### Preparation example 42: (6S,8S,9R,10S,11S,13 S, 14S, 17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((2S,5R)-2-(4-aminobutyl)-5-(hydroxymethyl)-28-(2-(methylsulfonyl)pyrimidin-5-yl)-4,7,10,10,16,19-trioxo-3,6,9,22-tetraazaoctacosan-27-ynamide)-4-fluorobenzoate (DL-A-49)

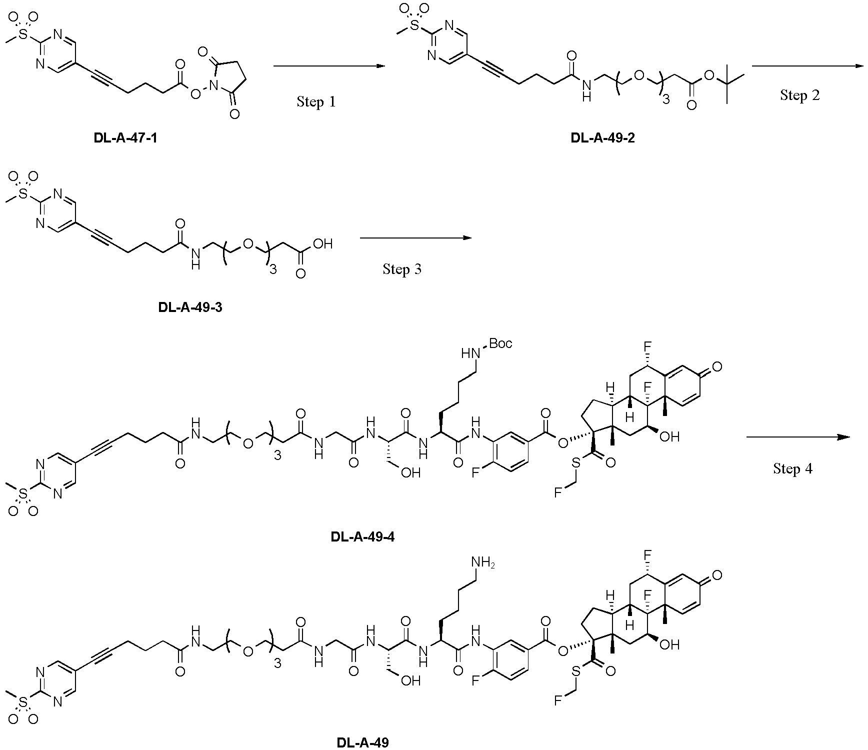

### Step 1: preparation of tert-butyl 19-(2-(methylsulfonyl)pyrimidin-5-yl)-14-oxo-4,7,10-trioxa-13-azacyclononadecan-18-ynoate (DL-A-49-2)

2,5-dioxopyrrolidin-1-yl 6-(2-(methylsulfonyl)pyrimidin-5-yl)hex-5-ynoate (200 mg, 0.547 mmol), tert-butyl 3-(2-(2-aminoethoxy)ethoxy)propionate (151.8 mg, 0.547 mmol), and DIPEA (141.5 mg, 1.09 mmol) were added into DMF (3 mL), and the reaction solution was allowed to react for 2 h at 25°C. Then EA (30 ml) and water (10 ml) were added, and the reaction solution was stirred for 5 min. The organic phase was separated out and concentrated to obtain a crude product, which was purified via column chromatography (MeOH/DCM = 5-20%) to obtain the title compound (260 mg, 443.49 µmol), which was directly used in the next step without purification.

The structure characterization data is as follows:
ESI-MS (m/z): 528.2 [M+H]⁺.

### Step 2: preparation of 9-(2-(methylsulfonyl)pyrimidin-5-yl)-14-oxo-4,7,10-trioxa-13-azacyclononadecan-18-ynoic acid (DL-A-49-3)

Tert-butyl 19-(2-(methylsulfonyl)pyrimidin-5-yl)-14-oxo-4,7,10-trioxa-13-azacyclononadecan-18-ynoate (260 mg, 0.493 mmol) was dissolved in dichloromethane (3 mL), trifluoroacetic acid (1.12 g, 9.86 mmol) was then added, and the reaction solution was heated to 35-40°C, allowed to react for 3 h, and then concentrated. ACN (10 ml) and deionized water (5ml) were added into the residue for dispersion, followed by freeze-drying to obtain the title compound (290 mg, 492.02 µmol), which was directly used in the next step without purification.

The structure characterization data is as follows:
ESI-MS (m/z): 472.2 [M+H]⁺.

### Step 3: preparation of (6S,85,9R,108,118S,138S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((2S, 5S)-2-(4)-(tert-butoxycarbonyl)amino)butyl)-5-(hydroxymethyl)-28-(2-(methylsulfonyl)pyrimidin-5-yl)-4,7,10,23-tetraoxo-13,16,19-trioxa-3,6,9,22-tetraazaoctacosan-27-ynamide)-4-fluorobenzoate (DL-A-49-4)

9-(2-(methylsulfonyl)pyrimidin-5-yl)-14-oxo-4,7,10-trioxa-13-azacyclononadecan-18-ynoic acid (32.9 mg, 0.056 mmol) was dissolved in DMF (1 mL), HATU (38.6 mg, 0.10 mmol), DIPEA (19.7 mg, 0.152 mmol) and (6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S) - 2-((S)-2-(2-aminoacetamido)-3-hydroxypropionamino)-6-(tertbutoxycarbonyl)amino)hexanamido)-4-fluorobenzoate (50.0 mg, 0.051 mmol, formate) were then added, and the reaction solution was reacted for 1 h at 25°C. EA (10 ml)/H₂O (3 ml) was added, and the reaction solution was stirred for 5 min. The organic phase was separated out and concentrated to obtain a crude product, which was purified via thin layer chromatography (MeOH/DCM = 10%) to obtain the title compound (41 mg, 27.95 µmol).

The structure characterization data is as follows:
ESI-MS (m/z): 1393.4 [M+H]⁺.

### Step 4: preparation of (6S,85,9R,108,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((2S,5R)-2-(4-aminobutyl)-5-(hydroxymethyl)-28-(2-(methylsulfonyl)pyrimidin-5-yl)-4,7,10,10,16,19-trioxo-3,6,9,22-tetraazaoctacosan-27-ynamide)-4-fluorobenzoate (DL-A-49)

(6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((2S,5S)-2-(4)-(tert-butoxycarbonyl)amino)butyl)-5-(hydroxymethyl)-28-(2-(methylsulfonyl)pyrimidin-5-yl)-4,7,10,23-tetraoxo-13,16,19-trioxa-3,6,9,22-tetraazaoctacosan-27-ynamide)-4-fluorobenzoate (41.0 mg, 0.029 mmol) was dissolved in dichloromethane (1 mL), trifluoroacetic acid (167.7 mg, 1.47 mmol) was then added, and the reaction solution was allowed to react for hours at 25°C and concentrated to obtain a crude product, which was purified via Pre-HPLC to obtain trifluoroacetate of the title compound (22.9 mg, 15.78 µmol).

The purification method is as follows:
Chromatographic column: SunFire Prep C18 OBD 19*150 mm*5 µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 5 | 95 | 28 |
| 2.00 | 5 | 95 | 28 |
| 20.00 | 80 | 20 | 28 |

The structure characterization data is as follows:
ESI-MS (m/z): 1293.5 (M+H)⁺.

### Preparation example 43: (6S,8S,9R,10S,11S,13 S, 14S, 17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((2 S, 5 S)-2-(4-aminobutyl)-5-(hydroxymethyl)-43-(2-(methylsulfonyl)pyrimidin-5-yl)-4,7,10,38-tetraoxo-13,16,19,22,25,28,31,34,34-octaoxa-3,6,9,37-tetraazatritetracontan-42-ynamide)-4-fluorobenzoate (DL-A-50)

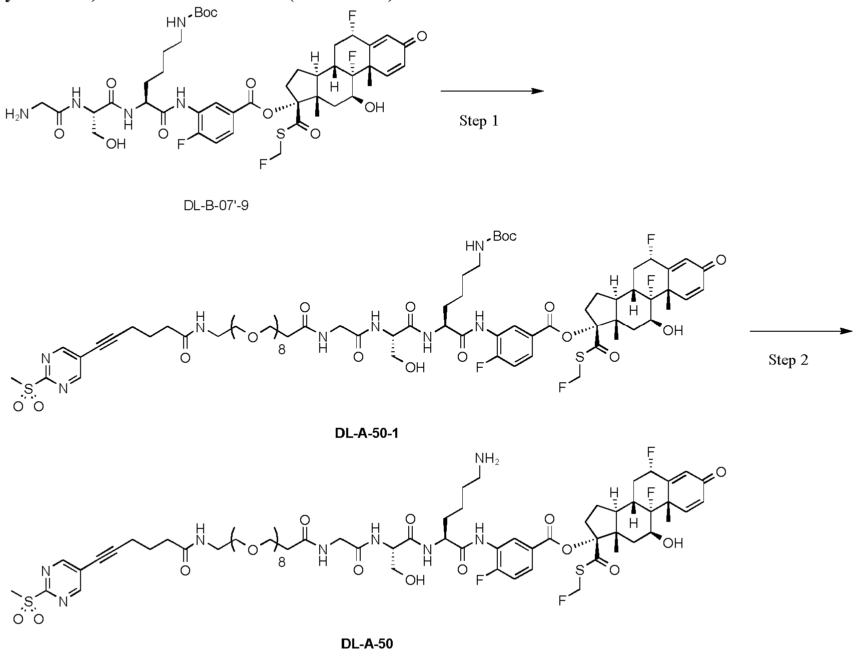

### Step 1: preparation of (6S,8S,9R,10S,11S,13 S, 14S, 17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((2S,5S)-2-(4-(tert-butoxycarbonyl)amino)butyl)-5-(hydroxymethyl)-43-(2-(methylsulfonyl)pyrimidin-5-yl)-4,7,10,38-tetraoxo-13,16,19,22,25,28,31,34-octaoxa-3,6,9,37-tetraazatritetracontan-42-ynamide)-4-fluorobenzoate (DL-A-50-1)

34-(2-(methylsulfonyl)pyrimidin-5-yl)-29-oxo-4,7,10,13,16,19,22,25-octaoxa-28-azatetratriacontan-33-ynoic acid (28.9 mg, 0.033 mmol) was dissolved in DMF (1 mL), followed by adding HATU (23.1 mg, 0.061 mmol), DIPEA (11.8 mg, 0.091 mmol), (6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl-3-((S)-2-((S)-2-(2-aminoacetamido)-3-hydroxypropanamido)-6-((tert-butoxycarbonyl)amino)hexamido)-4-fluorobenzoate (30.0 mg, 0.030 mmol, formate), and the reaction solution was allowed to react for 1 h at 25°C. Ethyl acetate (10 ml) and water (3 ml) were added, followed by stirring for 5 min. The organic phase was separated out and concentrated to obtain a crude product, which was purified via thin layer chromatography (methanol/dichloromethane = 10%) and concentrated again to obtain the title compound (17 mg, 10.01 µmol).

The structure characterization data is as follows:
ESI-MS (m/z): 1613.7 (M+H)⁺.

### Step 2: preparation of (6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((2 S, 5 S)-2-(4-aminobutyl)-5-(hydroxymethyl)-43-(2-(methylsulfonyl)pyrimidin-5-yl)-4,7,10,38-tetraoxo-13,16,19,22,25,28,31,34,34-octaoxa-3,6,9,37-tetraazatritetracontan-42-ynamide)-4-fluorobenzoate (DL-A-50)

(6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((2S,5S)-2-(4-(tert-butoxycarbonyl)amino)butyl)-5-(hydroxymethyl)-43-(2-(methylsulfonyl)pyrimidin-5-yl)-4,7,10,38-tetraoxo-13,16,19,22,25,28,31,34-octaoxa-3,6,9,37-tetraazatritetracontan-42-ynamide)-4-fluorobenzoate (17.0 mg, 0.010 mmol) was dissolved in dicloromethane (0.5 mL), followed by adding trifluoroacetic acid (60.0 mg, 0.527 mmol), and the reaction solution was allowed to react for 2 h at 25°C. The reaction solution was concentrated to obtain a crude product, which was purified via Pre-HPLC to obtain a trifluoroacetate of the title compound (8.4 mg, 5.01 µmol).

The purification method is as follows:
Chromatographic column: Waters SunFire Prep C18 OBD (5 µm*19 mm*150 mm)
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 20 | 80 | 28 |
| 4.00 | 20 | 80 | 28 |
| 24.00 | 90 | 10 | 28 |

The structure characterization data is as follows:
ESI-MS (m/z): 1513.6 (M+H)⁺.

### Preparation example 44: (4S)-5-((((4-(((6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl)oxy)-4-oxobut-2-yl)oxy)methyl)amino)-4-(2-(2-(methylsulfonyl)pyrimidin-5-yl)hex-5-ynamido)acetamido)-5-oxovaleric acid (DL-A-139)

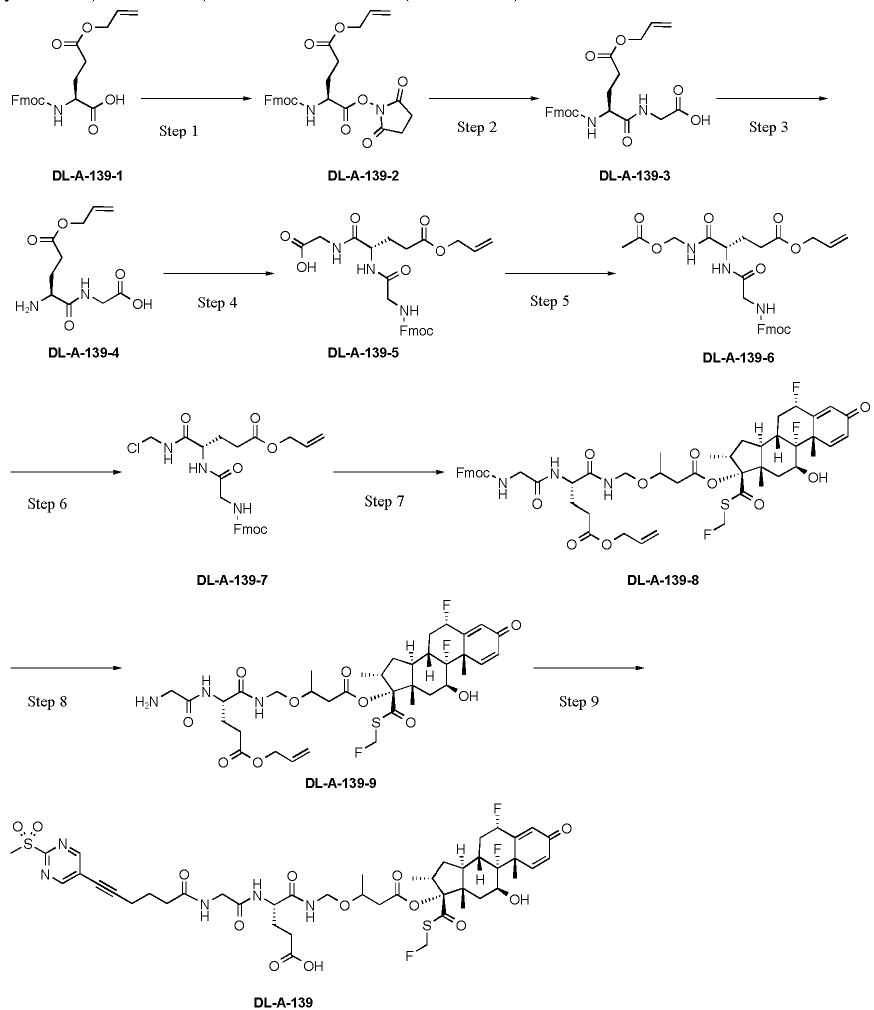

### Step 1: preparation of 5-allyl-1-(2,5-dioxopyrrolidin-1-yl)(((9H-fluoren-9-yl)methoxy)carbonyl)-L-glutamic acid (DL-A-139-2)

N,N'-dicyclohexylcarbodiimide (2.22 g, 10.75 mmol), (S)-2-(((9H-fluoren-9-yl)methoxy)carbonyl)amino)-5-(allyloxy)-5-oxovaleric acid (4.0 g, 9.77 mmol) and N-hydroxysuccinimide (1.19 g, 10.34 mmol) were added into tetrahydrofuran (40 mL), and the reaction solution was allowed to react for 2 h at 16°C under stirring after the addition was finished. The reaction solution was filtered, and the filtrate was concentrated under reduced pressure to dryness to obtain the title compound as a crude product (4.9 g, 9.67 mmol), which was directly used in the next step without purification.

The structure characterization data is as follows:
ESI-MS (m/z): 507.2(M+H)⁺.

### Step 2: preparation of (S)-(2-(((9H-fluoren-9-yl)methoxy)carbonyl)amino)-5-(allyloxy)-5-oxopentanoyl)glycine (DL-A-139-3)

5-allyl-1-(2,5-dioxopyrrolidin-1-yl)(((9H-fluoren-9-yl)methoxy)carbonyl)-L-glutamic acid (4.9 g, 9.67 mmol), glycine (1.45 g, 19.35 mmol) and sodium bicarbonate (1.63 g, 19.35 mmol) were added into acetone (50 mL) and water (50 mL), and the reaction solution was allowed to react for hours at 15°C after the addition was finished. Purified water (120 mL) was added, and then the reaction solution was adjusted to pH 3-4 using 1 N diluted hydrochloric acid and extracted with ethyl acetate for 3 times (50 mL x 3). The organic phases were combined and dried over anhydrous sodium sulfate, filtered and concentrated to obtain the title compound as a crude product (5.6 g), which was directly used in the next step without purification.

The structure characterization data is as follows:
ESI-MS (m/z): 467.2(M+H)⁺.

### Step 3: preparation of (S)-(5-(allyloxy)-2-amino-5-oxopentanoyl)glycine (DL-A-139-4)

The (S)-(2-(((9H-fluoren-9-yl)methoxy)carbonyl)amino)-5-(allyloxy)-5-oxopentanoyl)glycine crude product (5.6 g) was added into DMF (12 mL), diethylamine (2.5 mL) was then added, and then the reaction solution was allowed to react for hours at 16°C under stirring after the addition was finished. The reaction solution was concentrated under reduced pressure to remove residual diethylamine to obtain the title compound as a crude product (2.3 g), which was directly used in the next step without purification.

The structure characterization data is as follows:
ESI-MS (m/z): 245.1 (M+H)⁺.

### Step 4: preparation of (S)-(2-(2-(((9H-fluoren-9-yl)methoxy)carbonyl)amino)acetamido)-5-(allyloxy)-5-oxopentanoyl)glycine

The (S)-(5-(allyloxy)-2-amino-5-oxopentanoyl)glycine crude product (2.2 g), 2,5-dioxopyrrolidin-1-yl ((9H-fluoren-9-yl)methoxy)carbonyl)glycine (4.26 g,10.81 mmol) and DIPEA (2.33 g,18.01 mmol) were added into DMF (24 mL), and then the reaction solution was allowed to react for 3 h at 17°C under stirring after the addition was finished. Water (110 mL) was added into the reaction solution, followed by extraction with ethyl acetate (15mL x 2) to remove impurities. The water phase was adjusted to pH 3-4 using 1 N diluted hydrochloric acid and then extracted with ethyl acetate for 3 times (50 mL x 3). The organic phase was dried and then concentrated under reduced pressure to dryness to obtain the title compound (3.8 g, 7.25 mmol).

The structure characterization data is as follows:
ESI-MS (m/z): 524.2 (M+H)⁺.

### Step 5: preparation of allyl (S)-4-(2-(((9H-fluoren-9-yl)methoxy)carbonyl)amino)acetamido)-5-((acetoxymethyl)amino)-5-oxovalerate (DL-A-139-6)

(S)-(2-(2-(((9H-fluoren-9-yl)methoxy)carbonyl)amino)acetamido)-5-(allyloxy)-5-oxopentanoyl)glycine (602 mg, 1.15 mmol), pyridine (272.89 mg, 3.45 mmol), and lead tetraacetate (1.02 g, 2.30 mmol) were added into tetrahydrofuran (9 mL) and toluene (3 mL), and nitrogen was introduced into the system for replacement for 3 times after the addition was finished. Then the system was heated to 84°C and allowed to react for 3 h. The reaction solution was filtered, purified via column chromatography (petroleum ether/ethyl acetate = 4/1 to 1/4) and then concentrated again to obtain the title compound (177 mg, 296.34 µmol).

The structure characterization data is as follows:
ESI-MS (m/z): 560.3(M+H)⁺.

### Step 6: preparation of allyl (S)-4-(2-(((9H-fluoren-9-yl)methoxy)carbonyl)amino)acetamido)-5-((chloromethyl)amino)-5-oxovalerate (DL-A-139-7)

Allyl (S)-4-(2-(((9H-fluoren-9-yl)methoxy)carbonyl)amino)acetamido)-5-((acetoxymethyl)amino)-5-oxovalerate (80 mg, 148.82 µmol) and trimethylsilyl chloride (162 mg, 1.49 mmol) were added into DCM (2 mL), and the reaction solution was allowed to react for 0.5 h at 17°C after the addition was finished. The reaction solution was spinned to dryness under reduced pressure to obtain the title compound as a crude product (49 mg), which was directly used in the next step without purification.

### Step 7: preparation of (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (8S)-8-(3-(allyloxy)-3-oxopropyl)-1-(9H-fluoren-9-yl)-13-methyl-3,6,9-trioxo-2,12-dioxa-4,7,10-triazepentadecane-15-propionate (DL-A-139-8)

The allyl (S)-4-(2-(((9H-fluoren-9-yl)methoxy)carbonyl)amino)acetamido)-5-((chloromethyl)amino)-5-oxovalerate crude product (49 mg) and (6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 3-hydroxybutyrate (30 mg, 56.54 µmol) were added into DCM (4 mL), and after the addition was finished, the reaction solution was heated to reflux and then allowed to react for 5 h. The reaction solution was quenched using methanol, then purified via column chromatography (SiO₂, petroleum ether/ethyl acetate = 4/1 to 1/4) to obtain the title compound as a crude product (39 mg), which was directly used in the next step without purification.

The structure characterization data is as follows:
ESI-MS (m/z): 1008.4 (M+H)⁺.

### Step 8: preparation of (4S)-4-(2-aminoacetamide)-5-((4(((6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl)oxy)-4-oxobut-2-yl)oxy)methyl)amino)-5-oxovaleric acid (DL-A-139-9)

The (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (8S)-8-(3-(allyloxy)-3-oxopropyl)-1-(9H-fluoren-9-yl)-13-methyl-3,6,9-trioxo-2,12-dioxa-4,7,10-triazepentadecane-15-propionate crude product (39 mg), diethylamine (16 mg, 218.7 µmol), and tetra(triphenylphosphine) palladium (13 mg, 11.25 µm) were added into DMF (3 mL), and then the reaction solution was allowed to react for 1 h at 17°C after the addition was finished. The residual diethylamine was removed from reaction solution under reduced pressure to obtain the title compound as a crude product (28 mg), which was directly used in the next step without purification.

The structure characterization data is as follows:
ESI-MS (m/z): 746.2(M+H)⁺.

### Step 9: preparation of (4S)-5-((((4-(((6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl)oxy)-4-oxobut-2-yl)oxy)methyl)amino)-4-(2-(2-(methylsulfonyl)pyrimidin-5-yl)hex-5-ynamido)acetamido)-5-oxovaleric acid (DL-A-139)

The (4S)-4-(2-aminoacetamide)-5-((4(((6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl)oxy)-4-oxobut-2-yl)oxy)methyl)amino)-5-oxovaleric acid crude product (28 mg), 2,5-dioxopyrrolidin-1-yl 6-(2-(methylsulfonyl)pyrimidin-5-yl)hex-5-ynoate (13.72 mg, 37.54 µmol) and DIPEA (4.85 mg, 37.54 µmol) were added into DMF (3 mL), and the reaction solution was allowed to react for 1 h at 17°C after the addition was finished. The reaction solution was purified via high performance liquid chromatography to obtain the title compound (9.49 mg, 9.24 µmol).

The purification method is as follows:
Chromatographic column: SunFire Prep C18 OBD 19*150 mm*5 µm
Mobile phase A: acetonitrile; mobile phase B: water (0.05%TFA)

| Time [min] | Mobile phase A [%] | Mobile phase B [%] | Flow rate [mL/min] |
|---|---|---|---|
| 0.00 | 30 | 70 | 28 |
| 4.00 | 30 | 70 | 28 |
| 24.00 | 90 | 10 | 28 |

The structure characterization data is as follows:
ESI-MS (m/z): 996.3 (M+H)⁺.

### Preparation example 45: (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-((34S,37S)-37-(4-aminobutyl)-1-(3,5-bis(2-(methylsulfonyl)pyrimidin-5-yl)phenyl)-34-(hydroxymethyl)-1,29,32,35-tetraoxo-5,8,11,14,17,20,23,26-octaoxa-2,30,33,36-tetraazaoctatriacontan-38-amino)furan-2-carboxylate DL-B-22'

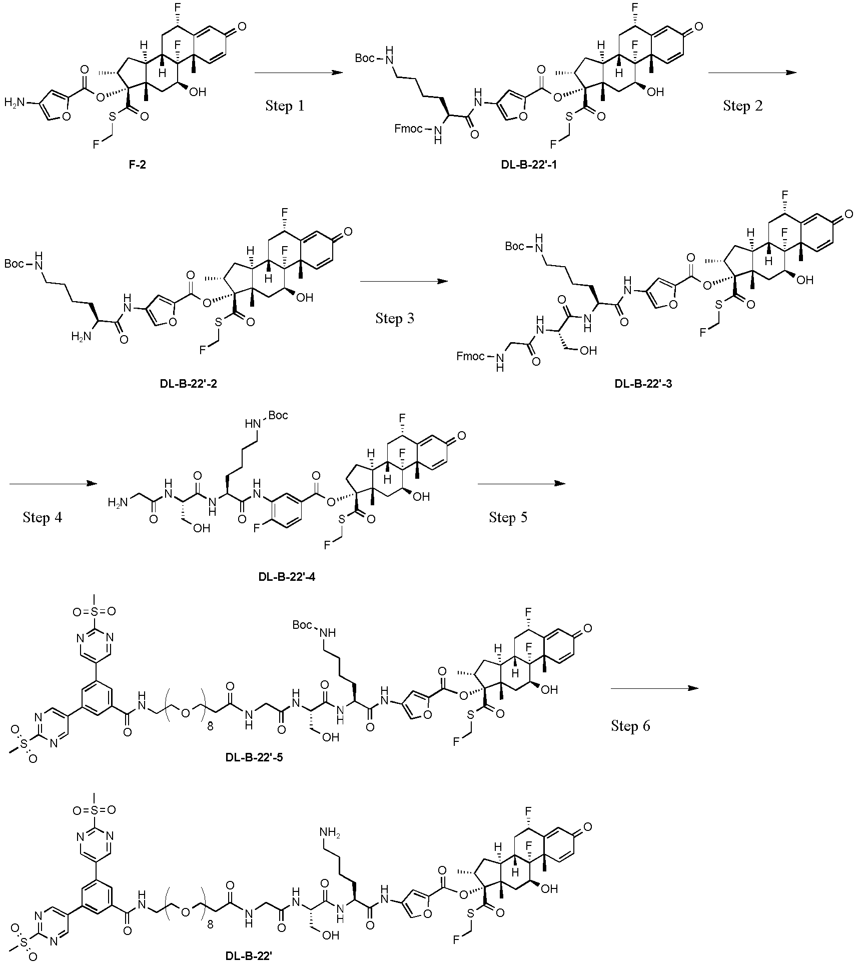

### Step 1: preparation of (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-((S)-2-(((9H-fluoren-9-yl)methoxy)carbonyl)amino)-6-(tertbutoxycarbonyl)amino)hexanamido)furan-2-carboxylate (DL-B-22'-1)

N²-((9H-fluoren-9-yl)methoxy)carbonyl)-N⁶-(tert-butoxycarbonyl)-L-lysine (224 mg, 479 µmol) was dissolved in DMF (10 mL), HATU (191 mg, 503 µmol) and DIPEA (185 mg, 1.44 mmol, 250 µL) were added, and then the reaction solution was stirred for 1.5 h at 25°C. Then, a trifluoroacetate of (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-aminofuran-2-carboxylate (320 mg, 479 µmol) was added, and then the reaction solution was stirred for additional 5 h at 25°C. Water (150 mL) was added into the reaction solution followed by extraction with ethyl acetate (150 mL). The organic phase was dried over anhydrous sodium sulfate, filtered and concentrated to obtain the title compound as a crude product (450 mg), which was directly used in the next step without purification.

The structure characterization data is as follows:
ESI-MS (m/z): 904.0 (M+H-Boc)⁺.

### Step 2: preparation of (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-((S)-2-amino-6-(tert-butoxycarbonyl)amino)hexanamido)furan-2-carboxylate (DL-B-22'-2)

(6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-((S)-2-(((9H-fluoren-9-yl)methoxy)carbonyl)amino)-6-(tertbutoxycarbonyl)amino)hexanamido)furan-2-carboxylate (387 mg, 385 µmol) was dissolved in DMF (6.00 mL), DBU (58.6 mg, 385 µmol, 58.0 µL) was added, and the reaction solution was stirred for 1 h at 25°C, and then directly used in the next step without purification.

The structure characterization data is as follows:
ESI-MS (m/z): 782.0 (M+H)⁺.

### Step 3: preparation of (6S,8S,9R,10S,11S,13S,14S,16R,17S)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-((8S,11S)-11-(4-(tert-butoxycarbonyl)amino)butyl)-1-(9H-fluoren-9-yl)-8-(hydroxymethyl)-3,6,9-trioxo-2-oxa-4,7,10-triazadodecan-12-amino)furan-2-carboxylate (DL-B-22'-3)

((9H-fluoren-9-yl)methoxy)carbonyl)glycyl-L-serine (147 mg, 384 µmol) and HOBt (78.0 mg, 577 µmol) were added into the DMF (6.00 mL) solution of (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-((S)-2-amino-6-(tertbutoxycarbonyl)amino)hexanamido)furan-2-carboxylate (301 mg, 384 µmol) obtained in the previous step, EDCI (110 mg, 577 µmol) was then added, and the reaction solution was stirred for 1 h at 25°C. Water (100 mL) was added into the reaction solution, followed by extraction with ethyl acetate (100 mL). The organic phase was dried over anhydrous sodium sulfate, filtered and concentrated to obtain the title compound as a crude product, which was purified via high performance liquid chromatography and freeze-dried to obtain the title compound (33.4 mg, 28.5 µmol).

The purification method is as follows:
Chromatographic column: Phenomenex C18 (10 µm*25 mm*150 mm)
Mobile phase A: acetonitrile; mobile phase B: water (0.05% ammonium bicarbonate)

| Time [min] | Mobile phase A [%] | Mobile phase B [%] | Flow rate [mL/min] |
|---|---|---|---|
| 0.00 | 52 | 48 | 28 |
| 10.00 | 82 | 18 | 28 |

The structure characterization data is as follows:
ESI-MS (m/z): 1148.5 (M+H)⁺.

### Step 4: preparation of 6S,8S,9R,10S,11S,13S,14S,16R,17S)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-((S)-2-((S)-2-(2-aminoacetamido)-3-hydroxypropionamido)-6-(tertbutoxycarbonyl)amino)hexanamido)furan-2-carboxylate (DL-B-22'-4)

(6S,8S,9R,10S,11S,13S,14S,16R,17S)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-((8S,11S)-11-(4-(tert-butoxycarbonyl)amino)butyl)-1-(9H-fluoren-9-yl)-8-(hydroxymethyl)-3,6,9-trioxo-2-oxa-4,7,10-triazadodecan-12-amino)furan-2-carboxylate (30 mg, 26.13 µmol, FR) was dissolved in DMF (1 mL), diethylamine (9.55 mg, 130.63 µmol) was then added, and the reaction solution was allowed to react for 1 h at 25°C. The reaction solution was directly purified via high performance liquid chromatography and freeze-dried to obtain a formate of the title compound (23.0 mg, 22.48 µmol).

The purification method is as follows:
Chromatographic column: Waters SunFire Prep C18 OBD (5 µm*19 mm*150 mm)
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 20 | 80 | 28 |
| 4.00 | 20 | 80 | 28 |
| 24.00 | 90 | 10 | 28 |

The structure characterization data is as follows:
ESI-MS (m/z): 926.4 (M+H)⁺.

### Step 5: preparation of (6S,8S,9R,10S,11S,13S,14S,16R,17S)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-((34S,37S)-1-(3,5-bis(2-(methylsulfonyl)pyrimidin-5-yl)phenyl)-37-(4-(tertbutoxycarbonyl)amino)butyl)-34-(hydroxymethyl)-1,29,32,35-tetraoxo-5,8,11,14,17,20,23,26-octaoxa-2,30,33,36-tetraazaoctatriacontan-38-amino)furan-2-carboxylate (DL-B-22'-5)

1-(3,5-bis(2-(methylsulfonyl)pyrimidin-5-yl)phenyl)-1-oxo-5,8,11,14,17,20,23,26-octaoxa-2-azanonacosane-29-carboxylic acid (22.4 mg, 0.026 mmol) was dissolved in DMF (1 mL), HATU (18.9 mg, 0.050 mmol), DIPEA (9.6 mg, 0.074 mmol) and 6S,8S,9R,10S,11S,13S,14S,16R,17S)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-((S)-2-((S)-2-(2-aminoacetamido)-3-hydroxypropionamido)-6-(tert-butoxycarbonyl)amino)hexanamido)furan-2-carboxylate (23.0 mg, 0.025 mmol, formate) were added, and then the reaction solution was allowed to react for 1 h at 25°C. The reaction solution was directly purified via high performance liquid chromatography and then freeze-dried to obtain the title compound (20 mg, 10.76 µmol).

The purification method is as follows:
Chromatographic column: Waters SunFire Prep C18 OBD (5 µm*19 mm*150 mm)
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 40 | 60 | 28 |
| 4.00 | 40 | 60 | 28 |
| 24.00 | 90 | 10 | 28 |

The structure characterization data is as follows:
ESI-MS (m/z): 1765.8 (M+H)⁺.

### Step 6: preparation of (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-((34S,37S)-37-(4-aminobutyl)-1-(3,5-bis(2-(methylsulfonyl)pyrimidin-5-yl)phenyl)-34-(hydroxymethyl)-1,29,32,35-tetraoxo-5,8,11,14,17,20,23,26-octaoxa-2,30,33,36-tetraazaoctatriacontan-38-amino)furan-2-carboxylate (DL-B-22')

(6S,8S,9R,10S,11S,13S,14S,16R,17S)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-((34S,37S)-1-(3,5-bis(2-(methylsulfonyl)pyrimidin-5-yl)phenyl)-37-(4-(tertbutoxycarbonyl)amino)butyl)-34-(hydroxymethyl)-1,29,32,35-tetraoxo-5,8,11,14,17,20,23,26-octaoxa-2,30,33,36-tetraazaoctatriacontan-38-amino)furan-2-carboxylate (20.0 mg, 0.011 mmol) was dissolved in dichloromethane (1 mL), trifluoroacetic acid (64.6 mg, 0.566 mmol) was then added, and the reaction solution was allowed to react for 2 h at 25°C. The reaction solution was purified via high performance liquid chromatography and then freeze-dried to obtain a trifluoroacetate of the title compound (12.2 mg, 6.65 µmol).

The purification method is as follows:
Chromatographic column: Waters SunFire Prep C18 OBD (5 µm*19 mm*150 mm)
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 20 | 80 | 28 |
| 4.00 | 20 | 80 | 28 |
| 24.00 | 80 | 20 | 28 |

The structure characterization data is as follows:
ESI-MS (m/z): 1665.7 (M+H)⁺.

### Preparation example 46: Preparation of (S)-1-(3,5-bis(2-(methylsulfonyl)pyrimidin-5-yl)phenyl)-34-(((R)-4-((6S,8S,9R,10S,11S,13S,14S,16R,17S)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl)oxy)-4-oxobut-2-yl)oxy)methyl)carbamoyl)-1,29,32-trioxo-5,8,11,14,17,20,23,26-octoxa-2,30,33-triazaheptatriacontan-37-ynoic acid (DL-B-65')

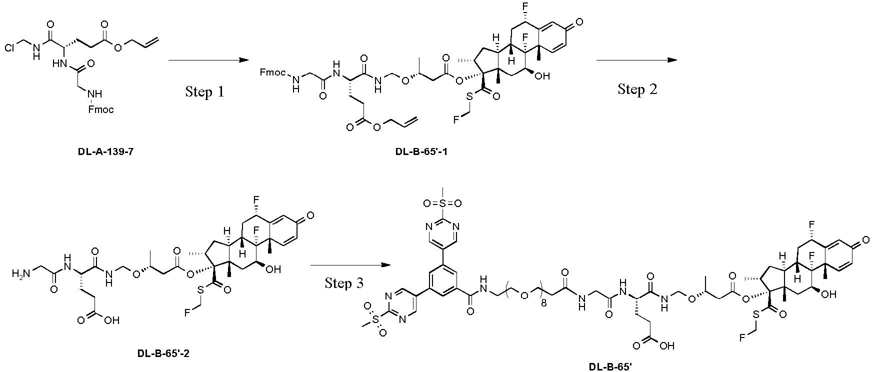

### Step 1: preparation of (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (8S,13R)-8-(3-(allyloxy)-3-oxopropyl)-1-(9H-fluoren-9-yl)-13-methyl-3,6,9-trioxo-2,12-dioxa-4,7,10-triazapentadecane-15-propionate (DL-B-65'-1)

Ally (S)-4-(2-(((9H-fluoren-9-yl)methoxy)carbonyl)amino)acetamido)-5-((chloromethyl)amino)-5-oxovalerate crude product (65 mg, 126.47 µmol) and (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (R)-3-hydroxybutyrate (67.10 mg, 126.47 µmol) were added into DCM (8 mL), and after the addition was finished, the reaction solution was heated to reflux and allowed to react for 6 h. Water (40 mL) was added into the reaction solution for quenching, and the reaction solution was extracted with DCM twice (4 mL x 2). The organic phase was dried and concentrated to obtain a crude product, which was purified via column chromatography (SiO₂, petroleum ether/ethyl acetate = 4/1 to 1/4) and then concentrated again to obtain the title compound (27 mg, 26.78 µmol).

The structure characterization data is as follows:
ESI-MS (m/z): 1008.4 (M+H)⁺.

### Step 2: preparation of (S)-4-(2-aminoacetamido)-5-(R)-4-((6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl)oxy)-4-oxobut-2-yl)oxy)methyl)amino)-5-oxovaleric acid (DL-B-65'-2)

(6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (8S,13R)-8-(3-(allyloxy)-3-oxopropyl)-1-(9H-fluoren-9-yl)-13-methyl-3,6,9-trioxo-2,12-dioxa-4,7,10-triazapentadecane-15-propionate (27 mg, 26.78 µmol), diethylamine (16 mg, 219 µmol), and tetra(triphenylphosphine)palladium (7 mg, 6.06 µmol) were added into DMF (3 mL), and the reaction solution was allowed to react for 3 h at 18°C after the addition was finished. Diethylamine was removed from the reaction solution under reduced pressure to obtain the title compound as a crude product (19 mg), which was directly used in the next step without purification.

### Step 3: preparation of (S)-1-(3,5-bis(2-(methylsulfonyl)pyrimidin-5-yl)phenyl)-34-(((R)-4-((6S,8S,9R,10S,11S,13S,14S,16R,17S)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl)oxy)-4-oxobut-2-yl)oxy)methyl)carbamoyl)-1,29,32-trioxo-5,8,11,14,17,20,23,26-octoxa-2,30,33-triazaheptatriacontan-37-ynoic acid (DL-B-65')

(S)-4-(2-aminoacetamido)-S-(R)-4-((6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl)oxy)-4-oxobut-2-yl)oxy)methyl)amino)-5-oxovaleric acid (19 mg), 1-(3,5-bis(2-(methylsulfonyl)pyrimidin-5-yl)phenyl)-1-oxo-5,8,11,14,17,20,26-octoxa-2-azanonacosane-29-propionyl succinimide (24.33 mg, 25.48 µmol) and DIPEA (6.59 mg, 50.95 µmol) were added into DMF (3 mL), and then the reaction solution was allowed to react for 2 h at 18°C after the addition was finished. The reaction solution was directly purified via high performance liquid chromatography to obtain the title compound (1.44 mg, 0.899 µmol).

The purification method is as follows:
Chromatographic column: SunFire Prep C18 OBD 19*150 mm*5 µm
Mobile phase A: acetonitrile; mobile B: water (0.05% TFA

| Time [min] | Mobile phase A [%] | Mobile phase B [%] | Flow rate [mL/min] |
|---|---|---|---|
| 0.00 | 40 | 60 | 32 |
| 4.00 | 40 | 60 | 32 |
| 24.00 | 90 | 10 | 32 |

The structure characterization data is as follows:
ESI-MS (m/z): 793.5(MI2+H)⁺.

### Preparation example 47: (6S,8S,9R,10S,11S,13 S, 14S, 17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-hydroxybenzoate (B-5)

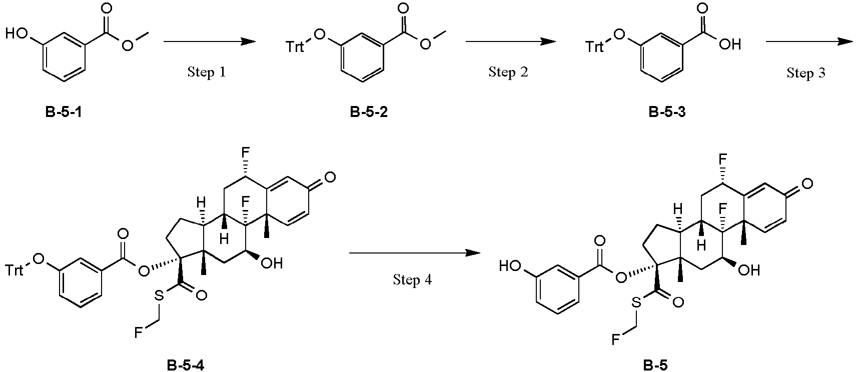

### Step 1: preparation of methyl 3-(triphenylmethoxy)benzoate (B-5-2)

Methyl 3-hydroxybenzoate (2.00 g, 13.2 mmol) was dissolved in pyridine (20.0 mL), triphenylmethyl chloride (3.66 g, 13.2 mmol) was added, and then the reaction solution was heated to 80°C and allowed to react for 12 h. The reaction solution was cooled to 25°C, then a 1 M diluted hydrochloric acid aqueous solution (40.0 mL) was added, and the reaction solution was extracted with dichloromethane for 3 times (50.0 mL x 3). The organic phases were combined, dried over anhydrous sodium sulfate, filtered and concentrated to obtain the title compound as a crude product (6.80 g), which was directly used in the next step without purification.

The structure thereof was characterized as follows:
ESI-MS (m/z): 417.0 (M+Na)⁺.

### Step 2: Preparation of 3-(triphenylmethoxy)benzoic acid (B-5-3)

Methyl 3-(triphenylmethoxy)benzoate (6.80 g, 17.2 mmol) was dissolved in a mixed solvent of tetrahydrofuran (22.0 mL), methanol (22.0 mL) and water (22.0 mL), lithium hydroxide monohydrate (2.17 g, 51.7 mmol) was added, and then the reaction solution was heated to 40°C and allowed to react for 1 h. Water (30.0 mL) and ethyl acetate (20.0 mL) were added, and then the reaction solution was adjusted to pH 2-3 using 1 N diluted hydrochloric acid. Liquid separation was performed, and the water phase was extracted with ethyl acetate for 3 times (30.0 mL). The organic phases were combined and dried over anhydrous sodium sulfate, filtered and concentrated to obtain the title compound as a crude product (554 mg, 646 µmol), which was directly used in the next step without purification.

The structure thereof was characterized as follows:
ESI-MS (m/z): 381.0 (M+H)⁺.

### Step 3: Preparation of (6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-(triphenylmethoxy)benzoate (B-5-4)

3-(triphenylmethoxy)benzoic acid (334 mg, 878 µmol) was dissolved in DMF (3.00 mL), HATU (334 mg, 878 µmol) and DIPEA (340 mg, 2.63 mmol, 459 µL) were then added, and then the reaction solution was stirred for 1 h. Then, (6S,8S,9R,10S,11S,13S,14S,17R)-6,9-difluoro-11,17-dihydroxy-10,13-dimethyl-3-oxo-6,7,8,9,10,11,12,13,14,15,16,17-dodecahydro-3H-cyclopenta[a]phenanthrene-17-thiocarboxylic acid (140 mg, 351 µmol) was added, and then the reaction solution was stirred for additional 1 h. Then, fluoroiodomethane (140 mg, 878 µmol) was added, and the reaction solution was stirred for additional 0.5 h. The reaction solution was poured into water (30.0 mL), stirred for 30 min, and filtered. The filter cake was washed with water for 3 times (10.0 mL x 4) and then dried in vacuum to obtain a crude product, which was purified via a silica gel column (dichloromethane/methanol = 100/1 to 10/1) and then concentrated again to obtain the title compound (200 mg, 22.5 µmol).

The structure thereof was characterized as follows:
ESI-MS (m/z): 815.3 (M+Na)⁺.

### Step 4: Preparation of (6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-hydroxybenzoate (B-5)

(6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-(triphenylmethoxy)benzoate (170 mg, 214 µmol) was dissolved in dichloromethane (3.00 mL), a hydrogen chloride/1,4-dioxane solution (2 M, 1.00 mL) was added, and then the reaction solution was stirred for 1 h at 25°C. The reaction solution was directly concentrated to obtain a crude product, which was purified via high performance liquid chromatography to obtain the title compound (2.83 mg, 3.03 µmol).

The purification method is as follows:
Chromatographic column: Phenomenex Luna C18 100mm×30mm×5µm
Mobile phase A: acetonitrile; mobile phase B: water (0.05% hydrochloric acid)

| Time [min] | Mobile phase A [%] | Mobile phase B [%] | Flow rate [mL/min] |
|---|---|---|---|
| 0.00 | 40 | 60 | 24 |
| 8.00 | 70 | 30 | 24 |

The structure thereof was characterized as follows:
ESI-MS (m/z): 551.0 [M+H]⁺.

¹H NMR (400 MHz, CD₃OD) δ7.39 (s, 1H), 7.34-7.37 (m, 2H), 7.33 (dd, *J =* 2.4 Hz, 1H), 7.02-7.06 (m, 1H), 6.34-6.40 (m, 2H), 5.75-5.95 (m, 2H), 5.49-5.56 (m, 1H), 4.34 (d, *J=* 1.2 Hz, 1H), 3.04 (d, *J* = 7.2 Hz, 1H), 2.61-2.67 (m, 1H), 2.52 (d, *J* = 3.6 Hz, 1H), 2.29-2.35 (m, 2H), 1.99-2.07 (m, 2H), 1.65 (s, 1H), 1.61 (d, *J =* 3.6 Hz, 5H), 1.07 (s, 3H).

### Preparation example 48: (6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 4-hydroxybenzoate (B-17)

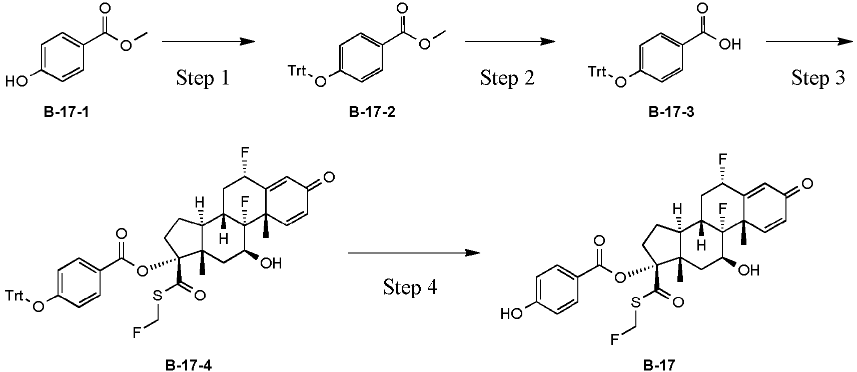

### Step 1: preparation of methyl 4-(triphenylmethoxy)benzoate (B-17-2)

Methyl 4-hydroxybenzoate (1.50 g, 9.86 mmol) was dissolved in pyridine (15.0 mL), triphenylmethyl chloride (2.75 g, 9.86 mmol) was added, and then the reaction solution was heated to 80°C and allowed to react for 12 h. The reaction solution was cooled to 25°C, and ethyl acetate (100 mL) and 1 M diluted hydrochloric acid (100 mL) were than added, followed by liquid separation. The organic phase was washed with a saturated sodium bicarbonate aqueous solution (100 mL), dried over anhydrous sodium sulfate, filtered and concentrated to obtain the title compound as a crude product (9.21 g), which was directly used in the next step without purification.

The structure thereof was characterized as follows:
ESI-MS (m/z): 417.0 (M+Na)⁺.

### Step 2: Preparation of 4-(triphenylmethoxy)benzoic acid (B-17-3)

Methyl 4-(triphenylmethoxy)benzoate (9.00 g, 22.8 mmol) was dissolved in a mixed solvent of tetrahydrofuran (30.0 mL), methanol (30.0 mL) and water (30.0 mL), lithium hydroxide monohydrate (2.87 g, 68.4 mmol) was added, and then the reaction solution was heated to 45 °C and allowed to react for 2 h. Dichloromethane (100 mL) and water (100 mL) were added into the reaction solution, followed by liquid separation. The organic phase was dried over anhydrous sodium sulfate, filtered and concentrated to obtain the title compound as a crude product (1.30 g), which was directly used in the next step without purification.

The structure thereof was characterized as follows:
ESI-MS (m/z): 403.0 (M+Na)⁺.

### Step 3: Preparation of (6S,8S,9R,10S,11S,138S,148S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 4-(triphenylmethoxy)benzoate (B-17-4)

4-(triphenylmethoxy)benzoic acid (50.0 mg, 52.5 µmol) was dissolved in DMF (2.00 mL), HATU (19.9 mg, 52.5 µmol) and DIPEA (20.3 mg, 157 µmol, 27.4 µL) were added, and then the reaction solution was stirred for 1 h. Then, (6S,8S,9R,10S,11S,13S,14S,17R)-6,9-difluoro-11,17-dihydroxy-10,13-dimethyl-3-oxo-6,7,8,9,10,11,12,13,14,15,16,17-dodecahydro-3H-cyclopenta[a]phenanthrene-17-thiocarboxylic acid (16.7 mg, 42.0 µmol) was added, and then the reaction solution was stirred for additional 1 h. Then, fluoroiodomethane (8.41 mg, 52.5 µmol) was added, and the reaction solution was stirred for additional 0.5 h. After water (20.0 mL) was added, the reaction solution was directly freeze-dried to obtain the title compound as a crude product (60 mg), which was directly used in the next step without purification.

The structure thereof was characterized as follows:
ESI-MS (m/z): 793.3 (M+H)⁺.

### Step 4: Preparation of (6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 4-hydroxybenzoate (B-17)

(6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 4-(triphenylmethoxy)benzoate (130 mg, 163 µmol) was dissolved in a mixed solvent of dichloromethane (1.00 mL) and methanol (1.00 mL), a hydrochloric acid/1,4-dioxane solution (2 M, 1.00 mL) was added, and then the reaction solution was stirred for 1 h at 25°C. The reaction solution was directly concentrated to obtain a crude product, which was purified via high performance liquid chromatography to obtain the title compound (6.30 mg, 11.2 µmol).

The purification method is as follows:
Chromatographic column: Welch Ultimate C18 150 mm×25 mm×5µm
Mobile phase A: acetonitrile; mobile phase B: water (0.05% trifluoroacetic acid)

| Time [min] | Mobile phase A [%] | Mobile phase B [%] | Flow rate [mL/min] |
|---|---|---|---|
| 0.00 | 38 | 62 | 20 |
| 10.00 | 68 | 32 | 20 |

The structure thereof was characterized as follows:
ESI-MS (m/z): 551.0 [M+H]⁺.

¹H NMR (400 MHz, CD₃OD) δ77.70-7.86 (m, 2H), 7.37 (dd, *J =* 10.0, 1.2 Hz, 1H), 6.72-6.92 (m, 2H), 6.38 (dd, *J =* 10.0, 2.0 Hz, 1H), 6.32 (s, 1H), 5.67-6.02 (m, 2H), 5.47-5.66 (m, 1H), 4.34-4.45 (m, 1H), 2.96-3.09 (m, 1H), 2.60-2.78 (m, 1H), 2.48 (dt, *J =* 14.0, 3.2 Hz, 1H), 2.25-2.43 (m, 2H), 2.02-2.18 (m, 2H), 1.74-1.86 (m, 1H), 1.51-1.71 (m, 5H), 1.07 (s, 3H).

### Preparation example 49: (6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 4-hydroxymethylbenzoate (B-32)

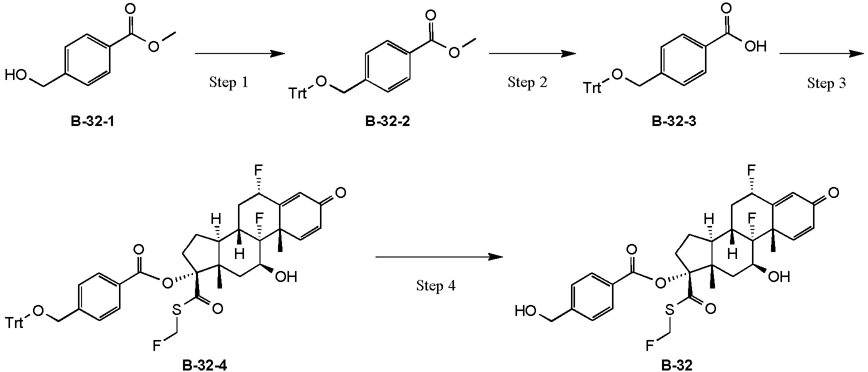

### Step 1: preparation of methyl 4-(triphenylmethoxy)methyl)benzoate (B-32-2)

Methyl 4-hydroxymethylbenzoate (3.00 g, 18.1 mmol) was dissolved in pyridine (30.0 mL), triphenylmethyl chloride (5.03 g, 18.1 mmol) was added, and then the reaction solution was heated to 80°C and allowed to react for 12 h. The reaction solution was cooled to 25°C, 1 M diluted hydrochloric acid (40.0 mL) was added, and then the reaction solution was extracted with dichloromethane for 3 times (50.0 mL x 3). The organic phases were combined and dried over anhydrous sodium sulfate, filtered and concentrated to obtain the title compound as a crude product (7.26 g), which was directly used in the next step without purification.

### Step 2: Preparation of 4-((triphenylmethoxy)methyl)benzoic acid (B-32-3)

Methyl 4-(triphenylmethoxy)methyl)benzoate (7.26 g, 17.8 mmol) was dissolved in a mixed solvent of tetrahydrofuran (24.0 mL), methanol (24.0 mL) and water (24.0 mL), lithium hydroxide monohydrate (2.24 g, 53.3 mmol) was added, and then the reaction solution was heated to 40°C and allowed to react for 1 h. Water (30.0 mL) and ethyl acetate (20.0 mL) were added, and the reaction solution was adjusted to pH 2-3 using 1 N diluted hydrochloric acid, followed by liquid separation. The water phase was extracted with ethyl acetate for 3 times (30.0 mL x 3). The organic phases were combined and dried over anhydrous sodium sulfate, filtered and concentrated to obtain the title compound as a crude product (6.41 g, crude).

The structure thereof was characterized as follows:
ESI-MS (m/z): 417.0 (M+Na)⁺.

### Step 3: Preparation of (6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 4-((triphenylmethoxy)methyl)benzoate (B-32-4)

4-((triphenylmethoxy)methyl)benzoic acid (150 mg, 380 µmol) was dissolved in DMF (3.00 mL), HATU (145 mg, 380 µmol) and DIPEA (148 mg, 1.14 mmol, 199 µL) were added, and then the reaction solution was stirred for 1 h. Then,(6S,8S,9R,10S,11S,13S,14S,17R)-6,9-difluoro-11,17-dihydroxy-10,13-dimethyl-3-oxo-6,7,8,9,10,11,12,13,14,15,16,17-dodecahydro-3H-cyclopenta[a]phenanthrene-17-thiocarboxylic acid (121 mg, 304 µmol) was added, and then the reaction solution was stirred for additional 1 h. Then, fluoroiodomethane (60.8 mg, 380 µmol) was added, and the reaction solution was stirred for additional 0.5 h. Water (30.0 mL) was added, and the reaction solution was filtered after being stirred for 30 min. The filter cake was washed with water for 3 times (10.0 mL x 3) to obtain the title compound as a crude product (300 mg), which was directly used in the next step without purification.

The structure thereof was characterized as follows:
ESI-MS (m/z): 829.3 (M+Na)⁺.

### Step 4: Preparation of (6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 4-hydroxymethylbenzoate (B-32)

(6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 4-((triphenylmethoxy)methyl)benzoate (300 mg, 372 µmol) was dissolved in a mixed solvent of dichloromethane (6.00 mL) and methanol (1.20 mL), trifluoroacetic acid (1.84 g, 16.1 mmol, 1.20 mL) was added, and then the reaction solution was stirred for 0.5 h at 25°C. The reaction solution was directly concentrated to obtain a crude product, which was purified via high performance liquid chromatography to obtain the title compound (62.0 mg, 108 µmol).

The purification method is as follows:
Chromatographic column: Phenomenex Luna C18200 mm×40 mm×10µm
Mobile phase A: acetonitrile; mobile phase B: water (0.05% trifluoroacetic acid)

| Time [min] | Mobile phase A [%] | Mobile phase B [%] | Flow rate [mL/min] |
|---|---|---|---|
| 0.00 | 30 | 70 | 28 |
| 12.00 | 60 | 40 | 28 |

The structure thereof was characterized as follows:
ESI-MS (m/z): 565.2 [M+H]⁺.

¹H NMR (400 MHz, CD₃OD) δ7.93 (d, J = 8.4 Hz, 2H), 7.48 (d, *J =* 8.4 Hz, 2H), 7.37 (dd, *J* = 10.4, 1.2 Hz, 1H), 6.38 (dd, *J =* 10.4, 2.0 Hz, 1H), 6.32 (s, 1H), 5.68-6.01 (m, 2H), 5.47-5.67 (m, 1H), 4.67 (s, 2H), 4.37-4.48 (m, 1H), 3.06 (dd, *J =* 16.0, 2.4 Hz, 1H), 2.62-2.78 (m, 1H), 2.46-2.56 (m, 1H), 2.29-2.44 (m, 2H), 2.05-2.20 (m, 2H), 1.77-1.86 (m, 1H), 1.56-1.71 (m, 5H), 1.08 (s, 3H).

### Preparation example 50: (8S,9R,10S,11S,13 S, 14S, 16R, 17R)-9-fluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 3-hydroxypropionate (D-6)

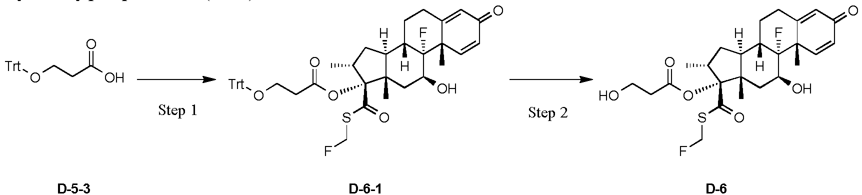

### Step 1: synthesis of (8S,9R,10S,11S,13S,14S,16R,17R)-9-fluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 3-(triphenylmethoxy)propionate (D-6-1)

3-(triphenylmethoxy)propionic acid (500 mg, 1.50 mmol) was dissolved in DMF (10.0 mL), HATU (572 mg, 1.50 mmol) and DIPEA (583 mg, 4.51 mmol, 786 µL) were added, and then the reaction solution was stirred for 1 h at 25°C. (8S,9R,10S,11S,13S,14S,16R,17R)-9-fluoro-11,17-dihydroxy-10,13,16-trimethyl-3-oxo-6,7,8,9,10,11,12,13,14,15,16,17-dodecahydro-3H-cyclopenta[a]phenanthrene-17-thiocarboxylic acid (297 mg, 752 µmol) was added, and then the reaction solution was stirred for additional 12 h. Then, fluoroiodomethane (241 mg, 1.50 mmol) was added, and the reaction solution was stirred for additional 0.5 h. The reaction solution was added into water to precipitate a solid, filtered, and the filter cake was dissolved with dichloromethane and concentrated to obtain a crude product, which was purified via a silica gel column (dichloromethane/methanol = 100/1 to 15/1) and then concentrated again to obtain the title compound (628 mg, 653 µmol).

The structure thereof was characterized as follows:
ESI-MS (m/z): 762.9 (M+Na)⁺.

### Step 2: Synthesis of (8S,9R,10S,11S,13S,14S,16R,17R)-9-fluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 3-hydroxypropionate (D-6)

(8S,9R,10S,11S,13S,14S,16R,17R)-9-fluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 3-(triphenylmethoxy)propionate (500 mg, 675 µmol) was dissolved in dichloromethane (9.00 mL), a hydrogen chloride/1,4-dioxane solution (2 M, 3.00 mL) was added, and then the reaction solution was stirred for 1 h at 25°C. The reaction solution was directly concentrated to obtain a crude product, which was purified via high performance liquid chromatography to obtain the title compound (81.1 mg, 162 µmol).
Chromatographic column: Phenomenex luna C18 200mm×40mm×10 µm
Mobile phase A: acetonitrile; mobile phase B: water (0.05% hydrochloric acid)

| Time [min] | Mobile phase A [%] | Mobile phase B [%] | Flow rate [mL/min] |
|---|---|---|---|
| 0.00 | 25 | 75 | 24 |
| 12.00 | 55 | 45 | 24 |

The structure thereof was characterized as follows:
ESI-MS (m/z): 499.0 [M+H]⁺.

¹H NMR (400 MHz, CD₃OD) δ 7.39 (d, *J* = 10.0 Hz, 1H), 6.30 (dd, *J* = 10.0, 1.9 Hz, 1H), 6.10 (s, 1H), 5.89-5.97 (m, 1H), 5.77-5.85 (m, 1H), 4.26-4.31 (m, 1H), 3.78 (t, *J* = 6.4 Hz, 2H), 2.68-2.78 (m, 1H), 2.57 (m, 2H), 2.47-2.54 (m, 1H), 2.37-2.47 (m, 2H), 2.21-2.28 (m, 1H), 2.12-2.19 (m, 1H), 1.89-1.97 (m, 3H), 1.59 (s, 3H), 1.47-1.57 (m, 1H), 1.32 (m, 1H), 1.13 (s, 3H), 0.99 (d, *J* = 7.2 Hz, 3H).

### Preparation example 51: (6S,8S,9R,10S,11S,13S,14S,16R,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-hydroxypropionate (D-7)

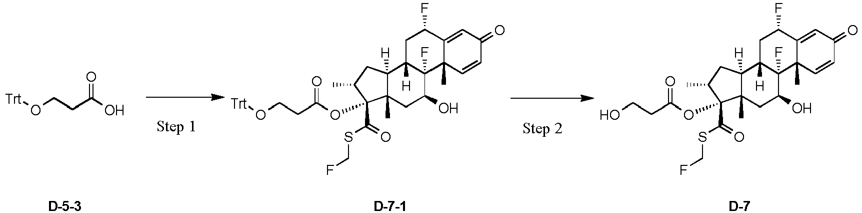

### Step 1: synthesis of (6S,8S,9R,10S,11S,13S,14S,16R,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-(triphenylmethoxy)propionate (D-7-1)

3-(triphenylmethoxy)propionic acid (100 mg, 300 µmol) was dissolved in DMF (2.00 mL), HATU (114 mg, 300 µmol) and DIPEA (116 mg, 905 µmol, 157 µL) were added, and then the reaction solution was stirred for 1 h at 25°C. (6S,8S,9R,10S,11S,13S,14S,16R,17R)-6,9-difluoro-11,17-dihydroxy-10,13-dimethyl-3-oxo-6,7,8,9,10,11,12,13,14,15,16,17-dodecahydro-3H-cyclopenta[a]phenanthrene-17-thiocarboxylic acid (95.9 mg, 240 µmol) was added, and then the reaction solution was stirred for additional 12 h. Then, fluoroiodomethane (48.1 mg, 300 µmol) was added, and the reaction solution was stirred for additional 0.5 h. After water (50.0 mL) was added, the reaction solution was directly freeze-dried to obtain the title compound as a crude product (320 mg), which was directly used in the next step without purification.

The structure thereof was characterized as follows:
ESI-MS (m/z): 744.6 (M+H)⁺.

### Step 2: Synthesis of (6S,8S,9R,10S,11S,13S,14S,16R,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-hydroxypropionate (D-7)

(6S,8S,9R,10S,11S,13S,14S,16R,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-(triphenylmethoxy)propionate (200 mg, 268 µmol) was dissolved in a mixed solution of dichloromethane (4.00 mL) and methanol (0.80 mL), trifluoroacetic acid (1.23 g, 10.7 mmol, 0.80 mL) was added, and then the reaction solution was stirred for 1 h at 25°C. The reaction solution was directly concentrated to obtain a crude product, which was purified via high performance liquid chromatography to obtain the title compound (33.0 mg, 64.3 µmol).
Chromatographic column: Welch Ultimate C18 150 mm×25 mm×5 µm
Mobile phase A: acetonitrile; mobile phase B: water (0.05% trifluoroacetic acid)

| Time [min] | Mobile phase A [%] | Mobile phase B [%] | Flow rate [mL/min] |
|---|---|---|---|
| 0.00 | 35 | 65 | 24 |
| 10.00 | 55 | 45 | 24 |

The structure thereof was characterized as follows:
ESI-MS (m/z): 502.9 [M+H]⁺.

¹H NMR (400 MHz, CD₃OD) δ 7.33 (dd, *J =* 10.0, 1.2 Hz, 1H), 6.34 (dd, *J =* 10.0, 1.6 Hz, 1H), 6.30 (s, 1H), 5.68-6.01 (m, 2H), 5.45-5.67 (m, 1H), 4.25-4.38 (m, 1H), 3.70-3.88 (m, 2H), 2.89-2.99 (m, 1H), 2.51 (d, *J* = 6.4 Hz, 3H), 2.26-2.41 (m, 2H), 2.20 (td, *J* = 12.0, 7.2 Hz, 1H), 2.07 (ddd, *J =* 16.0, 9.2, 7.2 Hz, 1H), 1.95 (d, *J* = 13.2 Hz, 1H), 1.73-1.83 (m, 1H), 1.49-1.70 (m, 5H), 1.00 (s, 3H).

### Preparation example 52: (6S,8S,9R,10S,11S,13S,14S,16R,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl (R)-3-hydroxybutyrate (D-17)

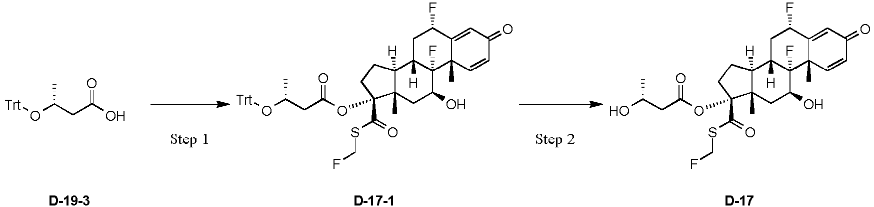

### Step 1: synthesis of (6S,8S,9R,10S,11S,13S,14S,16R,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl (R)-3-(triphenylmethoxy)butanoate (D-17-1)

(R)-3-(triphenylmethoxy)butyric acid (150 mg, 433 µmol) was dissolved in DMF (0.50 mL), HATU (165 mg, 433 µmol) and DIPEA (168 mg, 1.30 mmol, 226 µL) were added, and then the reaction solution was stirred for 1 h at 25°C. Then, 6S,8S, 9R,10S,11S,13S,14S,16R,17R)-6,9-difluoro-11,17-dihydroxy-10,13-dimethyl-3-oxo-6,7,8,9,10,11,12,13,14,15,16,17-dodecahydro-3H-cyclopenta[a]phenanthrene-17-thiocarboxylic acid (138 mg, 346 µmol) was added, and then the reaction solution was stirred for additional 0.5 h. Then, fluoroiodomethane (69.3 mg, 433 µmol) was added, and the reaction solution continued to react for 0.5 h at 25°C. The reaction solution was added into water to precipitate a solid, and the filter cake was dissolved with dichloromethane, dried over anhydrous sodium sulfate, filtered and concentrated to obtain the title compound as a crude product (317 mg, 418 µmol), which was directly used in the next step without purification.

The structure thereof was characterized as follows:
ESI-MS (m/z): 759.3 (M+H)⁺.

### Step 2: Synthesis of (6S,8S,9R,10S,11S,13S,14S,16R,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl (R)-3-hydroxybutyrate (D-17)

(6S,8S,9R,10S,11S,13S,14S,16R,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl (R)-3-(triphenylmethoxy)butanoate (300 mg, 395 µmol) was dissolved in a mixed solvent of dichloromethane (0.40 mL) and methanol (0.10 mL), TFA (45.1 mg, 395 µmol, 29.4 µL) was added, and then the reaction solution was allowed to react for 0.5 h at 25°C. The reaction solution was directly concentrated to obtain a crude product, which was purified via high performance liquid chromatography and then freeze-dried to obtain the title compound (31.0 mg, 57.6 µmol).
Chromatographic column: Phenomenex Luna C18200 mm×40 mm×10 µm
Mobile phase A: acetonitrile; mobile phase B: water (0.05% trifluoroacetic acid)

| Time [min] | Mobile phase A [%] | Mobile phase B [%] | Flow rate [mL/min] |
|---|---|---|---|
| 0.00 | 25 | 75 | 32 |
| 12.00 | 55 | 45 | 32 |

The structure thereof was characterized as follows:
ESI-MS (m/z):517.2[M+H]⁺.

¹H NMR (400 MHz, CD₃OD) δ 7.33 (dd, *J* = 10.0, 1.4 Hz, 1H), 6.28-6.38 (m, 2H), 5.69-6.00 (m, 2H), 5.47-5.65 (m, 1H), 4.32 (m, 1H), 4.12-4.19 (m, 1H), 2.93 (m, 1H), 2.59-2.72 (m, 1H), 2.48-2.54 (m, 1H), 2.11-2.47 (m, 5H), 1.92-2.08 (m, 2H), 1.62-1.82 (m, 3H), 1.58 (s, 3H), 1.52 (m, 1H), 1.21 (d, *J =* 6.3 Hz, 3H), 1.00 (s, 3H).

### Preparation example 53: (6S,8S,9R,10S,11S,13S,14S,16R,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl (R)-3-hydroxybutyrate (D-21)

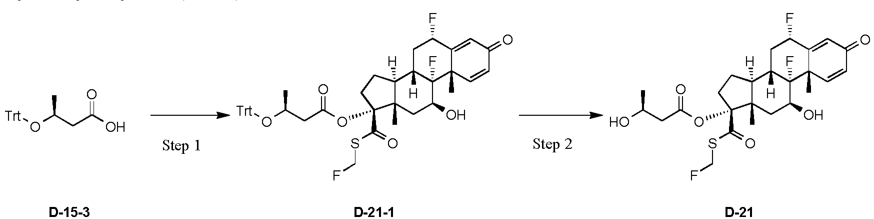

### Step 1: synthesis of (6S,8S,9R,10S,11S,13S,14S,16R,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl (S)-3-(triphenylmethoxy)butanoate (D-21-1)

(R)-3-(triphenylmethoxy)butyric acid (200 mg, 577 µmol) was dissolved in DMF (2 mL), HATU (220 mg, 577 µmol) and DIPEA (224 mg, 1.73 mmol, 302 µL) were added, and then the reaction solution was stirred for 1 h at 25°C. Then, 6S,8S,9R,10S,11S,13 S,14S, 16R,17R)-6,9-difluoro-11,17-dihydroxy-10,13-dimethyl-3-oxo-6,7,8,9,10,11,12,13,14,15,16,17-dodecahydro-3H-cyclopenta[a]phenanthrene-17-thiocarboxylic acid (161 mg, 404 µmol) was added, and then the reaction solution was stirred for additional 1 h. Then, fluoroiodomethane (92.3 mg, 577 µmol) was added, and the reaction solution continued to react for 0.5 h at 25°C. Water (30 mL) was added into the reaction solution to precipitate a solid, and was stirred for additional 30 min and then filtered. The filter cake was washed with water for 3 times (10 mL x 3) and then dried in vacuum to obtain the title compound as a crude product (410 mg), which was directly used in the next step without purification.

The structure thereof was characterized as follows:
ESI-MS (m/z): 781.3 (M+Na)⁺.

### Step 2: Synthesis of (6S,8S,9R,10S,11S,13S,14S,16R,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl (R)-3-hydroxybutyrate (D-21)

(6S,8S,9R,10S,11S,13S,14S,16R,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl (S)-3-(triphenylmethoxy)butanoate (360 mg, 474 µmol) was dissolved in dichloromethane (6 mL), a hydrogen chloride/1,4-dioxane solution (2 M, 2.00 mL) was added, and then the reaction solution was allowed to react for 1 h at 25°C. The reaction solution was directly concentrated to obtain a crude product, which was purified via high performance liquid chromatography and then freeze-dried to obtain the title compound (58.0 mg, 110 µmol).
Chromatographic column: Phenomenex Luna C18100 mm×30 mm×5 µm
Mobile phase A: acetonitrile; mobile phase B: water (0.05% hydrochloric acid)

| Time [min] | Mobile phase A [%] | Mobile phase B [%] | Flow rate [mL/min] |
|---|---|---|---|
| 0.00 | 30 | 70 | 24 |
| 8.00 | 60 | 40 | 24 |

The structure thereof was characterized as follows:
ESI-MS (m/z):517.2[M+H]⁺.

¹H NMR (400 MHz, CD₃OD) δ 7.33 (dd, *J* = 10.4 Hz, 1H), 6.16-6.50 (m, 2H), 5.69-6.00 (m, 2H), 5.44-5.67 (m, 1H), 4.89-4.96 (m, 2H), 4.32 (dt, *J* = 6.4 Hz, 1H), 4.07-4.21 (m, 1H), 2.95 (dd, *J* = 16.0 Hz, 1H), 2.57-2.71 (m, 1H), 2.42-2.57 (m, 2H), 2.35-2.41 (m, 1H), 2.30 (dt, *J* = 14.4 Hz, 1H), 2.20 (td, *J* = 12.4 Hz, 1H), 2.06 (dd, *J* = 16.4 Hz, 1H), 1.94 (d, *J* = 13.6 Hz, 1H), 1.73-1.82 (m, 1H), 1.50-1.68 (m, 5H), 1.21 (d, *J* = 6.4 Hz, 3H), 1.00 (s, 3H).

### Preparation example 54: (8S,9R,10S,11S,13S,14S,16R,17R)-9-fluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-(hydroxymethyl)furan-2-carboxylate (F-11)

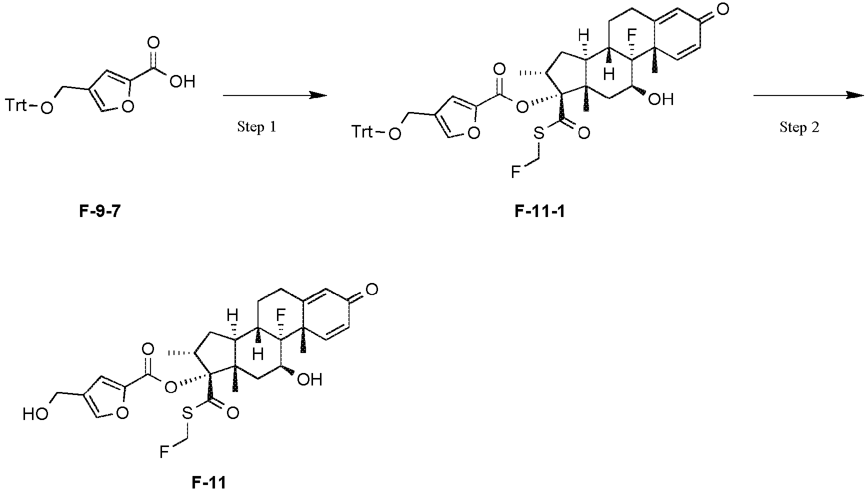

### Step 1: synthesis of (6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 4-((triphenylmethoxy)methyl)furan-2-carboxylate (F-11-1)

4-((triphenylmethoxy)methyl)furan-2-carboxylic acid (200 mg, 520 µmol) was dissolved in DMF (5.0 mL) under the atmosphere of nitrogen, DIPEA (201 mg, 1.56 mmol, 271 µL) and HATU (197 mg, 520 µmol) were added, and then the reaction solution was stirred for 1 h at 25°C. (8S,9R,10S,11S,13S,14S,16R,17R)-9-fluoro-11,17-dihydroxy-10,13,16-trimethyl-3-oxo-6,7,8,9,10,11,12,13,14,15,16,17-dodecahydro-3H-cyclopenta[a]phenanthrene-17-thiocarboxylic acid (123 mg, 312 µmol) was added, and then the reaction system was stirred for additional 1 h. Then, fluoroiodomethane (83.2 mg, 520 µmol) was added, and then the reaction solution was stirred for additional 0.5 h. The reaction solution was poured into water (100 mL) and then extracted with ethyl acetate for 3 times (50.0 mL x 3). The organic phases were combined and dried over anhydrous sodium sulfate, filtered and concentrated to obtain the title compound as a crude product (450 mg), which was directly used in the next step without purification.

The structure thereof was characterized as follows:
ESI-MS (m/z): 815.2 [M+Na]⁺.

### Step 2: Synthesis of (8S,9R,10S,11S,13S,14S,16R,17R)-9-fluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-(hydroxymethyl)furan-2-carboxylate (F-11)

(6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 4-((triphenylmethoxy)methyl)furan-2-carboxylate (450 mg, 567 µmol) was dissolved in dichloromethane (5.00 mL) and methanol (1.00 mL), trifluoroacetic acid (1.54 g, 13.4 mmol, 1.00 mL) was added, and then the reaction solution was stirred for 1 h at 25°C. The reaction solution was then concentrated to obtain a crude product, which was purified via high performance liquid chromatography (preparative column: DAICEL CHIRALPAK IC (250 mm × 30 mm × 5 um); mobile phase: [CO₂-MeOH]; B%: 45%) and then dried to obtain the title compound (29.0 mg, 49.6 µmol).

The structure thereof was characterized as follows:
ESI-MS (m/z): 551.3 [M+H]⁺.

¹H NMR (400 MHz, CD₃OD) δ7.60-7.82 (m, 1H), 7.35-7.48 (m, 1H), 7.20 (s, 1H), 6.32 (d, J = 10.0 Hz, 1H), 6.12 (s, 1H), 5.77-6.03 (m, 2H), 4.47 (s, 2H), 4.29-4.39 (m, 1H), 3.47 (s, 1H), 2.68-2.82 (m, 1H), 2.49-2.62 (m, 1H), 2.26-2.46 (m, 3H), 1.91-2.08 (m, 3H), 1.60 (s, 3H), 1.26-1.46 (m, 2H), 1.18 (s, 3H), 1.01 (d, J = 6.8 Hz, 3H).

### Preparation example 55: Preparation of (S)-5-(((3-(((6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl)oxy)-3-oxopropoxy)methyl)amino)-4-(2-(6-(2-(methylsulfonyl)pyrimidin-5-yl)hex-5-ynylamino)acetamido)-5-oxovaleric acid (DL-A-31)

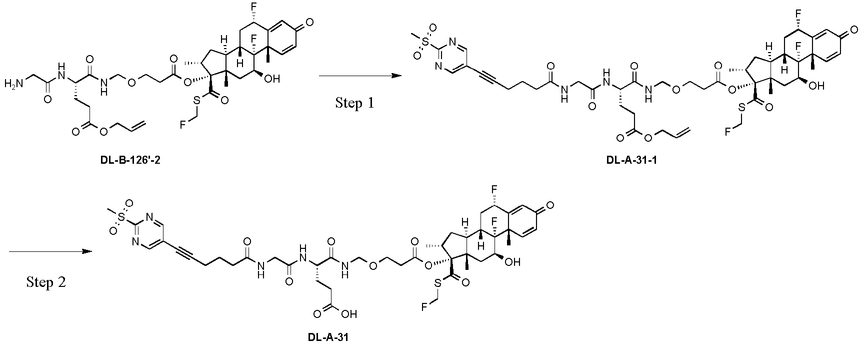

### Step 1: preparation of (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (S)-8-(3-(allyloxy)-3-oxopropyl)-18-(2-(methylsulfonyl)pyrimidin-5-yl)-7,10,13-trioxo-4-oxa-6,9,12-triazaoctadecan-17-ynoate (DL-A-31-1)

Allyl (S)-4-(2-aminoacetamide)-5-((3-(((6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,15,16,17-dodecahydro-3H-cyclopenta[a]phenanthren-17-yl)oxy)-3-oxopropoxy)methyl)amino)-5-oxovalerate (30 mg, 36.68 µmol), 2,5-dioxopyrrolidin-1-yl-6-(2-(methylsulfonyl)pyrimidin-5-yl)hex-5-ynoate (16 mg, 44.02 µmol) and DIPEA (16 mg, 123.80 µmol) were added into DMF (1 mL), and then the reaction solution was allowed to react for 2 h at 22°C after the addition was finished. After the reaction was completed, a solvent was removed from the reaction solution under reduced pressure to obtain the title compound as a crude product (37 mg), which was directly used in the next step without purification.

### Step 2: preparation of (S)-5-(((3-(((6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl)oxy)-3-oxopropoxy)methyl)amino)-4-(2-(6-(2-(methylsulfonyl)pyrimidin-5-yl)hex-5-ynylamino)acetamido)-5-oxovaleric acid (DL-A-31)

(6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (S)-8-(3-(allyloxy)-3-oxopropyl)-18-(2-(methylsulfonyl)pyrimidin-5-yl)-7,10,13-trioxo-4-oxa-6,9,12-triazaoctadecan-17-ynoate (37 mg, crude) was added into DCM (2 mL), tetra(triphenylphosphine)palladium (20.92 mg, 18.10 µmol), formic acid (10 µL) and N-methylmorpholine (20 µL) were added under the protection of nitrogen, and then the reaction solution was allowed to react for 2 h at 22°C under stirring after the addition was finished. After the reaction was completed, the reaction solution was purified via high performance liquid chromatography and then freeze-dried to obtain the title compound (7.99 mg, 7.97 µmol).

The purification method is as follows:
Chromatographic column: SunFire Prep C18 OBD 19×150 mm×5 µm
Mobile phase A: acetonitrile; mobile B: water (0.05% TFA)

| Time [min] | Mobile phase A [%] | Mobile phase B [%] | Flow rate [mL/min] |
|---|---|---|---|
| 0.00 | 30 | 70 | 32 |
| 4.00 | 30 | 70 | 32 |
| 24.00 | 90 | 10 | 32 |

The structure characterization data is as follows:
ESI-MS (m/z): 982.4(M+H)⁺.

### Preparation example 56: preparation of (6S,8S,9R,10S,11S,13S,14S,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (8S,11S)-8-(4-aminobutyl)-11-hydroxymethyl-21-(2-(methylsulfonyl)pyrimidin-5-yl)-7,10,13,16-tetraoxo-4-oxa-6,9,12,15-tetraazahexan-20-ynoate (DL-A-33)

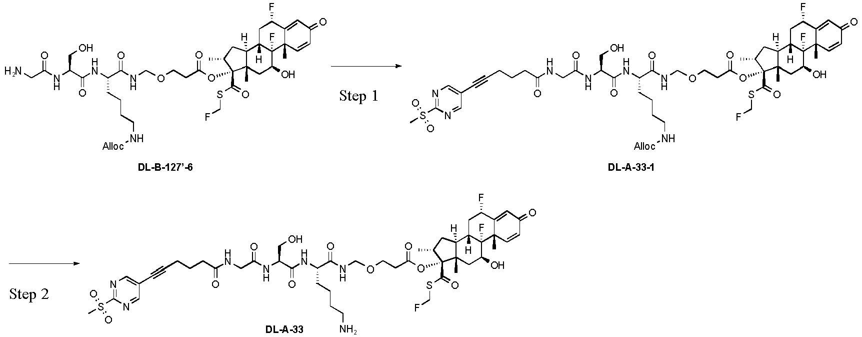

### Step 1: preparation of (6S,8S,9R,10S,11S,13S,14S,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (8S,11S)-8-(4-(((allyloxy)carbonyl)amino)butyl)-11-hydroxymethyl-21-(2-(methylsulfonyl)pyrimidin-5-yl)-7,10,13,16-tetraoxo-4-oxa-6,9,12,15-tetraazaeicosan-20-ynoate (DL-A-33-1)

(6S,8S,9R,10S,11S,13S,14S,17R)-6,9-difluoro-17-((fluoromethyl)thio)carbonyl)-11-hydroxy-10,13,16-trimethyl-3-oxo-6,7,8,9,10,11,12,13,15,16,17-dodecahydro-3H-cyclopenta[a]phenanthren-17-yl (S)-8-((S)-2-(2-aminoacetamido)-3-hydroxypropionamido)-7,14-dioxo-4,15-dioxa-6,13-diazaoctadecan-17-enoate (15.0 mg, 16.63 µmol) and 2,5-dihydroxypyrrolidin-1-yl 6-(2-(methylsulfonyl)pyrimidin-5-yl)hexane-5-ynoate (7.3 mg, 19.96 µmol) were added into DMF (0.5 mL), DIPEA (2.2 mg, 16.63 µmol) was added, and the reaction solution was allowed to react for 1 h at 25°C and then directly purified via high performance liquid chromatography to obtain the title compound (14.0 mg, 11.54 µmol).

The purification method is as follows:
Chromatographic column: SunFire Prep C18 OBD 19*150 mm*5 µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 40 | 60 | 28 |
| 4.00 | 40 | 60 | 28 |
| 24.00 | 90 | 10 | 28 |

The structure characterization data is as follows:
ESI-MS (m/z): 1152.4 (M+H)⁺.

### Step 2: preparation of (6S,8S,9R,10S,11S,13S,14S,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (8S,11S)-8-(4-aminobutyl)-11-hydroxymethyl-21-(2-(methylsulfonyl)pyrimidin-5-yl)-7,10,13,16-tetraoxo-4-oxa-6,9,12,15-tetraazahexan-20-ynoate (DL-A-33)

(6S,8S,9R,10S,11S,13S,14S,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (8S,11S)-8-(4-(((allyloxy)carbonyl)amino)butyl)-11-hydroxymethyl-21-(2-(methylsulfonyl)pyrimidin-5-yl)-7,10,13,16-tetraoxo-4-oxa-6,9,12,15-tetraazaeicosan-20-ynoate (14.0 mg, 12.15 µmol) was dissolved in DMF (1 mL), formic acid (8.8 mg, 182.25 µmol), N-methylmorpholine (12.3 mg, 121.50 µmol) and tetra(triphenylphosphine)palladium (7.0 mg, 6.08 µmol) were successively added, and the reaction solution was allowed to react for 2 h at 25°C. The reaction solution was directly purified via high performance liquid chromatography to obtain the title compound (7.40 mg, 6.31 µmol).

The purification method is as follows:
Chromatographic column: SunFire Prep C18 OBD 19*150 mm*5 µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 10 | 90 | 30 |
| 2.00 | 10 | 90 | 30 |
| 24.00 | 80 | 20 | 30 |

The structure characterization data is as follows:
ESI-MS (m/z): 1068.4 (M+H)⁺.

### Preparation example 57: Preparation of (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (8S,11S)-8,11-dimethyl-18-(2-(methylsulfonyl)pyrimidin-5-yl)-7,10,13-trioxo-4-oxa-6,9,12-triazaoctadecan-17-ynoate (DL-A-41)

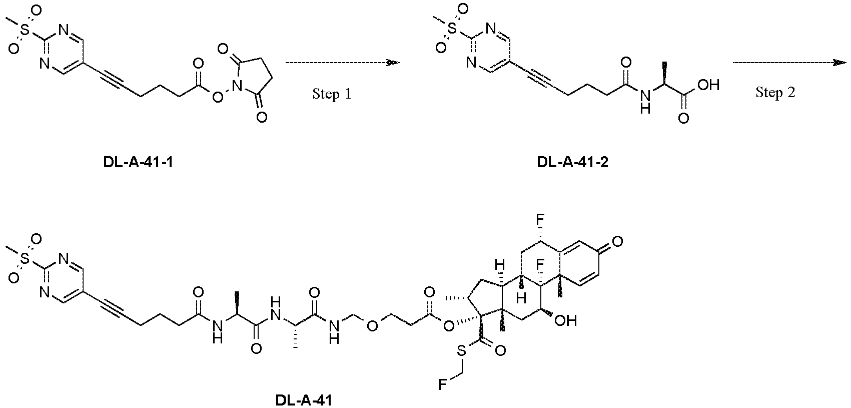

### Step 1: preparation of (6-(2-(methylsulfonyl)pyrimidin-5-yl)hex-5-ynyl)-L-alanine (DL-A-41-2)

2-(2,5-dioxopyrrolidin-1-yl) 6-(2-methylsulfonylpyrimidin-5-yl)hex-5-ynoate (200 mg, 0.547 mmol) and L-alanine (97.54 mg, 1.09 mmol) were added into DMSO (5 mL), and then the reaction solution was allowed to react for 16 h at 50°C. The reaction solution was purified via reverse phase column chromatography (acetonitrile/0.05% formic acid aqueous solution = 25-30%) and then freeze-dried to obtain the title compound (20 mg, 58.93 µmol).

The structure characterization data is as follows:
ESI-MS (m/z): 340.2 [M+H]⁺.

### Step 2: preparation of (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (8S,11S)-8,11-dimethyl-18-(2-(methylsulfonyl)pyrimidin-5-yl)-7,10,13-trioxo-4-oxa-6,9,12-triazaoctadecan-17-ynoate (DL-A-41)

(6-(2-(methylsulfonyl)pyrimidin-5-yl)hex-5-ynyl)-L-alanine (5 mg, 14.73 µmol), DIPEA (9.52 mg, 73.63 µmol), and HATU (8.40 mg, 22.10 µmol) were dissolved in DMF (2 mL), (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 3-(((S)-2-aminopropanamido)methoxy)propionate (9.09 mg, 14.73 µmol). The reaction solution was allowed to react for 2 h at 25°C. The reaction solution was purified via high performance liquid chromatography and then freeze-dried to obtain the title compound (6.29 mg, 6.79 µmol).

The purification method is as follows:
Chromatographic column: SunFire Prep C18 OBD 19*150 mm*5 µm
Mobile phase A: acetonitrile; mobile phase B: water (0.05% FA)

| Time [min] | Mobile phase A [%] | Mobile phase B [%] | Flow rate [mL/min] |
|---|---|---|---|
| 0.00 | 30 | 70 | 30 |
| 2.00 | 30 | 70 | 30 |
| 18.00 | 90 | 10 | 30 |

The structure characterization data is as follows:
ESI-MS (m/z): 960.4 [M+Na]⁺.

### Preparation example 58: Preparation of (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (8S,11S,14S)-8,11,14-trimethyl-21-(2-(methylsulfonyl)pyrimidin-5-yl)-7,10,13,16-tetraoxo-4-oxa-6,9,12,15-tetraazaheneicosan-20-ynoate (DL-A-43)

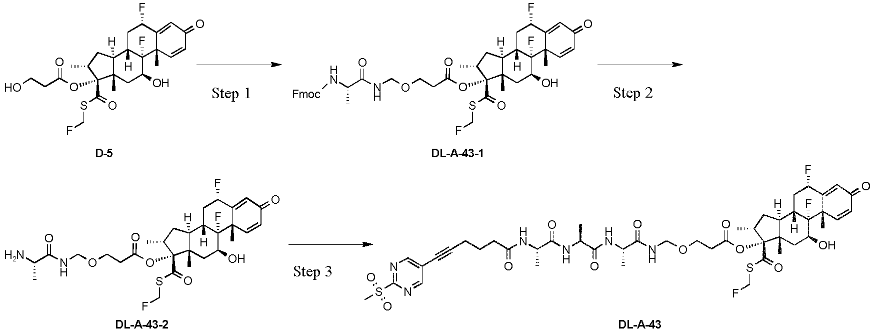

### Step 1: preparation of (6S,8S,9R,108,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (S)-1-(9H-fluoren-9-yl)-5-methyl-3,6-dioxo-2,9-dioxa-4,7-diazadodecane-12-carboxylate (DL-A-43-1):

Methyl (S)-(2-(((9H-fluoren-9-yl)methoxy)carbonyl)amino)propionamido)acetate (55.52 mg, 145.19 µmol), (6S,8S,9R, 10S, 11 S, 13S, 14S, 16R, 17R)-6,9-difluoro-17-((fluoromethyl)thio)carbonyl)-11-hydroxy-10,13,16-trimethyl-3-oxo-6,7,8,9,10,12,13,14,15,16,17-dodecahydro-3H-cyclopenta[a]phenanthren-17-yl 3-hydroxypropionate (50 mg, 96.79 µmol) and 4-methylbenzenesulfonyl pyridine (24.32 mg, 96.79 µmol) were added into DCM (5 mL), and then the reaction solution was heated to reflux and allowed to react for 4 h after the addition was finished. The reaction solution was purified via a silica gel column (petroleum ether/ethyl acetate = 20/1 to 1/1) and then concentrated to obtain the title compound (20 mg, 22.65 µmol).

### Step 2: preparation of (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 3-(((S)-2-aminopropanamido)methoxy)propionate (DL-A-43-2)

(6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (S)-1-(9H-fluoren-9-yl)-5-methyl-3,6-dioxo-2,9-dioxa-4,7-diazadodecane-12-carboxylate (20 mg, 22.65 µmol) and diethylamine (3.49 mg, 47.68 µmol) were added into DMF (1 mL), and then the reaction solution was stirred for 2 h at 22°C after the addition was finished. After the reaction was completed, the reaction solution was spinned to dryness under reduced pressure to remove the solvent to obtain the title compound as a crude product (14 mg), which was directly used in the next step without purification.

The structure characterization data is as follows:
ESI-MS (m/z): 617.4(M+H)⁺.

### Step 3: preparation of (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (8S,11S,14S)-8,11,14-trimethyl-21-(2-(methylsulfonyl)pyrimidin-5-yl)-7,10,13,16-tetraoxo-4-oxa-6,9,12,15-tetraazaheneicosan-20-ynoate (DL-A-43)

The (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 3-(((S)-2-aminopropanamido)methoxy)propionate crude product (14 mg), (6-(2-(methylsulfonyl)pyrimidin-5-yl)hex-5-ynyl)-L-alanine-L-alanine (10.25 mg, 24.97 µmol), DIPEA (16 mg, 123.83 µmol) and HATU (12.94 mg, 34.05 µmol) were added into DMF (1 mL), and the reaction solution was allowed to react for 2 h at 22°C under stirring after the addition was finished. After the reaction was completed, the reaction solution was purified via high performance liquid chromatography and then freeze-dried to obtain the title compound (7.77 mg, 7.62 µmol).

The purification method is as follows:
Chromatographic column: SunFire Prep C18 OBD 19×150 mm×5 µm
Mobile phase A: acetonitrile; mobile B: water (0.05% TFA)

| Time [min] | Mobile phase A [%] | Mobile phase B [%] | Flow rate [mL/min] |
|---|---|---|---|
| 0.00 | 30 | 70 | 30 |
| 2.00 | 30 | 70 | 30 |
| 18.00 | 95 | 5 | 30 |

The structure characterization data is as follows:
ESI-MS (m/z): 1026.4 (M+H₂O)⁺.

### Preparation example 59: Preparation of (S)-1-(3,5-bis(2-(methylsulfonyl)pyrimidin-5-yl)phenyl)-34-((3-(((6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,11,12,13,14,15,16,17-dodecahydro-3H-cyclopenta[a]phenanthren-17-yl)oxy)-3-oxopropoxy)methyl)carbamoyl)-1,29,32-trioxo-5,8,11,14,17,20,20,23,26-octoxa-2,30,33-triazaheptatriacontane-37-carboxylic acid (DL-B-50')

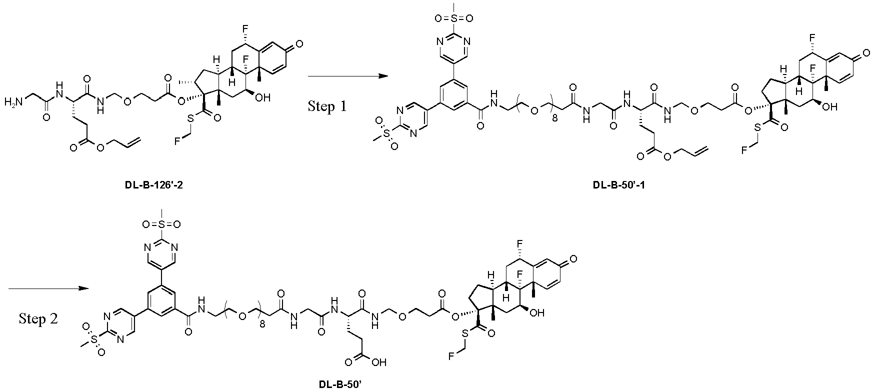

### Step 1: preparation of (6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (S)-34-(3-(allyloxy)-3-oxopropyl)-1-(3,5-bis(2-(methylsulfonyl)pyrimidin-5-yl)phenyl)-1,29,32,35-tetraoxo-5,8,11,14,17,20,23,26,38-nonaoxa-2,30,33,36-tetraazahentetracontane-41-carboxylate (DL-B-50'-1)

Allyl (S)-4-(2-aminoacetamide)-5-((3-(((6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl)oxy)-3-oxopropoxy)methyl)amino)-5-oxovalerate (30 mg, 36.68 µmol), 1-(3,5-bis(2-(methylsulfonyl))pyrimidin-5-yl)phenyl)-1-oxo-5,8,11,14,17,20,23,26-octaoxa-2-azanonacosane-29-carboxylic acid (31.47mg, 36.68 µmol), DIPEA (16 mg, 123.80 µmol) and HATU (18.12 mg, 47.66 µmol) were added into DMF (1 mL), and then the reaction solution was allowed to react for 2 h at 22°C under stirring after the addition was finished. After the reaction was completed, the reaction solution was spinned to dryness under reduced pressure to remove the solvent to obtain the title compound as a crude product (59 mg), which was directly used in the next step without purification.

### Step 2: preparation of (S)-1-(3,5-bis(2-(methylsulfonyl)pyrimidin-5-yl)phenyl)-34-((3-(((6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,11,12,13,14,15,16,17-dodecahydro-3H-cyclopenta[a]phenanthren-17-yl)oxy)-3-oxopropoxy)methyl)carbamoyl)-1,29,32-trioxo-5,8,11,14,17,20,20,23,26-octoxa-2,30,33-triazaheptatriacontane-37-carboxylic acid (DL-B-50')

(6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (S)-34-(3-(allyloxy)-3-oxopropyl)-1-(3,5-bis(2-(methylsulfonyl)pyrimidin-5-yl)phenyl)-1,29,32,35-tetraoxo-5,8,11,14,17,20,23,26,38-nonaoxa-2,30,33,36-tetraazahentetracontane-41-carboxylate (59 mg) was added into DMF (1 mL), tetra(triphenylphosphine)palladium (21 mg, 18.30 µmol), formic acid (10 µL), and N-methylmorpholine (20 µL) were added under the protection of nitrogen, and then the reaction solution was allowed to react for 2 h at 22°C under stirring after the addition was finished. After the reaction was completed, the reaction solution was purified via high performance liquid chromatography and then freeze-dried to obtain the title compound (3.92 mg, 2.47 µmol).

The purification method is as follows:
Chromatographic column: SunFire Prep C18 OBD 19×150 mm×5 µm
Mobile phase A: acetonitrile; mobile B: water (0.05% TFA)

| Time [min] | Mobile phase A [%] | Mobile phase B [%] | Flow rate [mL/min] |
|---|---|---|---|
| 0.00 | 20 | 80 | 32 |
| 4.00 | 20 | 80 | 32 |
| 24.00 | 90 | 10 | 32 |

The structure characterization data is as follows:
ESI-MS (m/z): 1571.4(M+H)⁺.

### Preparation example 60: Preparation of (6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-((6S,9S)-39-(3,5-bis(2-(methylsulfonyl)pyrimidin-5-yl)phenyl)-6,9-dimethyl-5,8,11,39-tetraoxo-2,14,17,20,23,26,29,32,35-nonaoxa-4,7,10,38-tetraazanonatriacontyl)furan-2-carboxylate (DL-B-105')

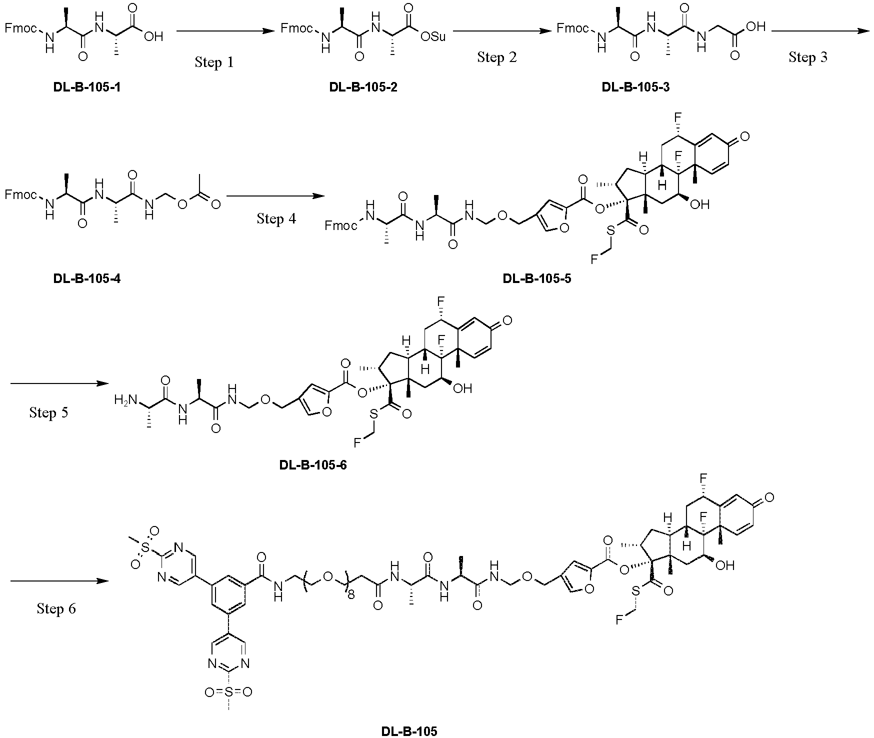

### Step 1: preparation of 2,5-dioxopyrrolidin-1-yl (9H-fluoren-9-yl)methoxy)carbonyl)-L-alanine-L-alanine (DL-B-105-2)

(((9H-fluoren-9-yl)methoxy)carbonyl)- L-alanine-L-alanine (10.0 g, 26.2 mmol) was dissolved in dichloromethane (100 mL), DCC (8.09 g, 39.2 mmol, 7.93 mL) and 1-hydroxypyrrolidine-2,5-dione (4.51 g, 39.2 mmol) were added, and then the reaction solution was stirred for 12 h at 25°C. The reaction solution was directly filtered (to remove DCU), and the filter cake was washed with dichloromethane for 3 times (10.0 mL x 3). The filtrate was concentrated and then added into tetrahydrofuran (100 mL), and then the solution was cooled to 0°C and stirred for additional 1 h. The reaction solution was filtered again (to remove DCU), and the filtrate was concentrated to obtain the title compound as a crude product (11.7 g), which was directly used in the next step without purification.

The structure characterization data is as follows:
ESI-MS (m/z): 502.1 (M+Na)⁺.

### Step 2: preparation of ((9H-fluoren-9-yl)methoxy)carbonyl)-L-alanine-L-alanine glycine (DL-B-105-3)

2,5-dioxopyrrolidin-1-yl (9H-fluoren-9-yl)methoxy)carbonyl)-L-alanine-L-alanine (5.00 g, 10.4 mmol) and glycine (783 mg, 10.4 mmol) were dissolved in a mixed solvent of tetrahydrofuran (50.0 mL) and water (25.0 mL), a sodium bicarbonate aqueous solution (1.75 g, 20.9 mmol, 812 µL) was added, and then the reaction solution was stirred for 2 h at 25°C. The reaction solution was adjusted to pH 2-3 using 1 N diluted hydrochloric acid, and the reaction solution was extracted with dichloromethane for 3 times (60.0 mL x 3). The organic phases were combined, dried over anhydrous sodium sulfate, filtered and concentrated to obtain the title compound as a crude product (5.70 g), which was directly used in the next step without purification.

The structure characterization data is as follows:
ESI-MS (m/z): 440.0 (M+H)⁺.

### Step 3: preparation of (5S,8S)-1-(9H-fluoren-9-yl)-5,8-dimethyl-3,6,9-trioxo-2-oxa-4,7,10-triazaundecan-11-yl acetate (DL-B-105-4)

((9H-fluoren-9-yl)methoxy)carbonyl)-L-alanine-L-alanine glycine (4.70 g, 10.7 mmol) was dissolved in DMF (47.0 mL), copper acetate (777 mg, 4.28 mmol), lead tetraacetate (5.69 g, 12.8 mmol) and acetic acid (1.41 g, 23.5 mmol, 1.35 mL) were added, and then the reaction solution was heated to 60°C and stirred for 2 h. The reaction solution was poured into water (100 mL), stirred for 30 min and then filtered. The filter cake was washed with water for 3 times (20.0 mL x 3) and then dried in vacuum to obtain a crude product, which was purified via a silica gel column (dichloromethane/methanol = 100/1 to 100/3) and then concentrated again to obtain the title compound (2.10 g, 4.30 mmol).

The structure characterization data is as follows:
ESI-MS (m/z): 476.0 (M+Na)⁺.

### Step 4: preparation of (6S,8S,9R,108,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-((6S,9S)-13-(9H-fluoren-9-yl)-6,9-dimethyl-5,8,11-trioxo-2,12-dioxa-4,7,10-triazatridecyl)furan-2-carboxylate (DL-B-105-5)

(5S,8S)-1-(9H-fluoren-9-yl)-5,8-dimethyl-3,6,9-trioxo-2-oxa-4,7,10-triazaundecan-11-yl acetate (934 mg, 2.06 mmol) was dissolved in dichloromethane (20.0 mL), PPTS (414 mg, 1.65 mmol) and (6S,8S,9R,10S,11S,13S,14S,16R,17R)-6,9-difluoro-17-((fluoromethyl)thio)carbonyl-11-hydroxy-10,13,16-trimethyl-3-oxo-6,7,8,9,10,11,12,13,14,15,15,16,17-dodecahydro-3H-cyclopenta[a]phenanthren-17-yl 4-(hydroxymethyl)furan-2-carboxylate (1.17 g, 2.06 mmol) were added, and then the reaction solution was heated to 50°C and stirred for 2 h. The reaction solution was cooled to 25°C and added with water (50.0 mL), and then extracted with dichloromethane for 3 times (30.0 mL x 3). The organic phases were combined, dried over anhydrous sodium sulfate, filtered and concentrated to obtain a crude product, which was purified via a silica gel column (dichloromethane/methanol = 100/1 to 100/3) and then concentrated again to obtain the title compound (1.07 g, 940 µmol).

The structure characterization data is as follows:
ESI-MS (m/z): 962.3 (M+H)⁺.

### Step 5: preparation of (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-(((S)-2-((S)-2-aminopropanamido)propanamido)methoxy)methyl)furan-2-carboxylate (DL-B-105'-6)

(6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-((6S,9S)-13-(9H-fluoren-9-yl)-6,9-dimethyl-5,8,11-trioxo-2,12-dioxa-4,7,10-triazatridecyl)furan-2-carboxylate (200 mg, 207.89 µmol, FR) was dissolved in DMF (5 mL), DBU (94.95 mg, 623.68 µmol) was added, and then the reaction solution was allowed to react for 1 h at 25°C. The reaction solution was directly purified via high performance liquid chromatography and freeze-dried to obtain a formate of the title compound (100.0 mg, 120.89 µmol).

The purification method is as follows:
Chromatographic column: WatersSunFire Prep C18 OBD (5 µm*19 mm*150 mm)
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 15 | 85 | 30 |
| 2.00 | 15 | 85 | 30 |
| 18.00 | 80 | 20 | 30 |

The structure characterization data is as follows:
ESI-MS (m/z): 740.3 (M+H)⁺.

### Step 6: preparation of (6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-((6S,9S)-39-(3,5-bis(2-(methylsulfonyl)pyrimidin-5-yl)phenyl)-6,9-dimethyl-5,8,11,39-tetraoxo-2,14,17,20,23,26,29,32,35-nonaoxa-4,7,10,38-tetraazanonatriacontyl)furan-2-carboxylate (DL-B-105')

1-(3,5-bis(2-(methylsulfonyl)pyrimidin-5-yl)phenyl)-1-oxo-5,8,11,14,17,20,23,26-octaoxa-2-azanonacosane-29-carboxylic acid (22 mg, 25.64 µmol), DIPEA (16.57 mg, 128.21 µmol), and HATU (14.62 mg, 38.46 µmol) were added into DMF (2 mL), and the reaction solution was allowed to react for 0.5 h at 25°C. Then (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-(((S)-2-((S)-2-aminopropanamido)propanamido)methoxy)methyl)furan-2-carboxylate (18.97 mg, 25.64 µmol) was added, and the reaction solution was allowed to react for 2 h at 25°C. The reaction solution was directly purified via high performance liquid chromatography and then freeze-dried to obtain the title compound (6.15 mg, 4.03 µmol).

The purification method is as follows:
Chromatographic column: SunFire Prep C18 OBD 19*150 mm*5 µm
Mobile phase A: acetonitrile; mobile phase B: water (0.05% FA)

| Time [min] | Mobile phase A [%] | Mobile phase B [%] | Flow rate [mL/min] |
|---|---|---|---|
| 0.00 | 30 | 70 | 32 |
| 4.00 | 30 | 70 | 32 |
| 24.00 | 90 | 10 | 32 |

The structure characterization data is as follows:
ESI-MS (m/z): 1601.5 (M+Na)⁺.

### Preparation example 61: preparation of (6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-((6R,9S)-41-(3,5-bis(2-(methylsulfonyl)pyrimidin-5-yl)phenyl)-6,9,13,16,19,22,25,28,28,31,31,34,37,40-dodecamethyl-5,8,11,14,17,20,23,26,29,32,35,38,41-tridecaoxo-2-oxa-4,7,10,13,16,19,22,25,28,31,34,37,40-tridecaazahentetracontane)furan-2-carboxylate (DL-B-121')

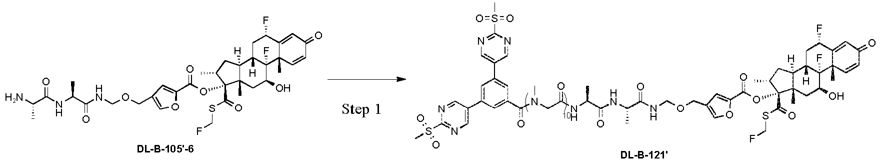

1-(3,5-bis(2-(methylsulfonyl)pyrimidin-5-yl)phenyl)-2,5,8,11,14,17,20,23,26,29-decamethyl-1,4,7,10,13,16,19,22,25,28-decaoxo-2,5,8,11,11,14,17,20,23,26,29-decaazahentriacontane-31-carboxylic acid (72.87 mg, 63.63 µmol) was dissolved in DMF (3 mL), HATU (24.19 mg, 63.63 µmol), DIPEA (20.56 mg, 159.07 µmol) and (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-(((S)-2-((S)-2-aminopropanamido)propanamido)methoxy)methyl)furan-2-carboxylate (23.77 mg, 31.81 µmol, formate) were added, and then the reaction solution was allowed to react for 1 h at 25°C. The reaction solution was directly purified via high performance liquid chromatography and then freeze-dried to obtain the title compound (40.0 mg, 20.35 µmol).

The purification method is as follows:
Chromatographic column: WatersSunFire Prep C18 OBD (5 µm*19 mm*150 mm)
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 30 | 70 | 30 |
| 2.00 | 30 | 70 | 30 |
| 24.00 | 90 | 10 | 30 |

The structure characterization data is as follows:
ESI-MS (m/z): 1868.0 (M+H)⁺.

### Preparation example 62: preparation of (S)-1-(3,5-bis(2-(methylsulfonyl)pyrimidin-5-yl)phenyl)-36-((5-(((6S,8S,9R,10S,11S,13S,14S,16R,17R)-6,9-difluoro-17-((fluoromethyl) thio)carbonyl)-11-hydroxy-10,13,16-trimethyl-3-oxo-6,7,8,10,11,12,13,14,15,16,17-dodecahydro-3H-cyclopenta[a]phenanthren-17-yl)oxy)carbonyl)furan-3-yl)methoxy)methyl)carbamoyl)-2,5,8,11,14,17,23,26,29-decamethyl-1,4,7,10,13,16,19,22,25,28,31,34-dodecoxo-2,5,8,11,14,17,20,23,26,29,32,35-dodecaazanonatriacontane-39-carboxylic acid (DL-B-122')

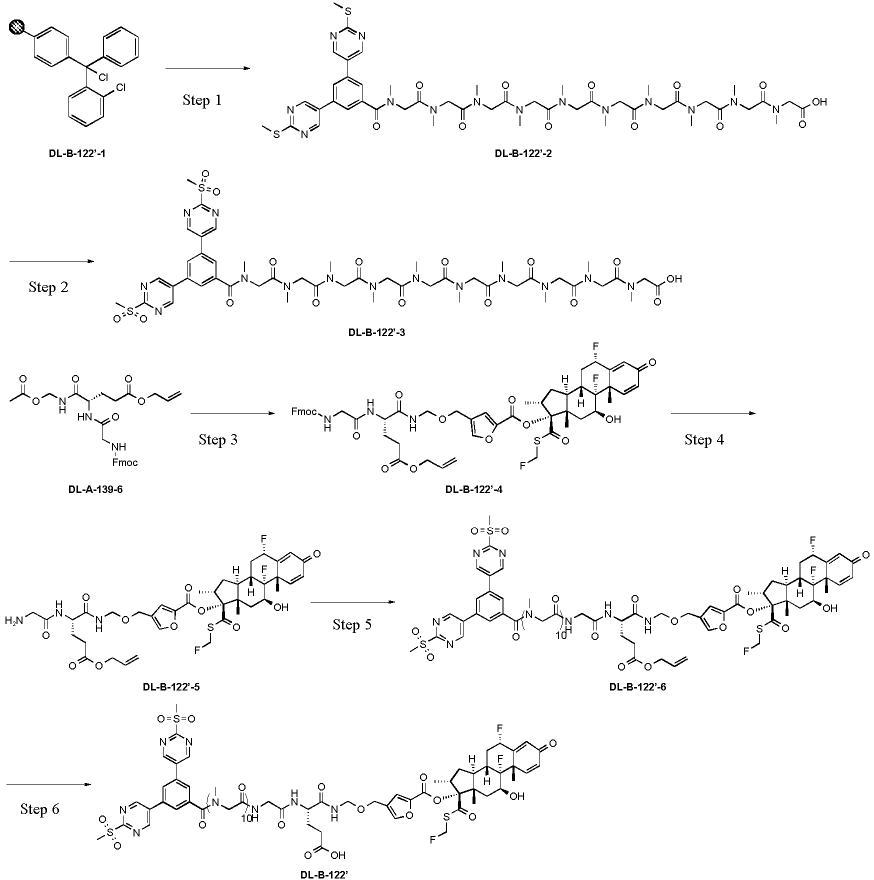

### Step 1: preparation of 1-(3,5-bis(2-(methylthio)pyrimidin-5-yl)phenyl)-2,5,8,11,14,17,20,23,26,29-decamethyl-1,4,7,10,13,16,19,22,25,28-decaoxo-2,5,8,11,14,17,20,23,26,29-decaazahentriacontane-31-carboxylic acid (DL-B-122'-2)

A standard solid phase synthesis method was used:
1) Resin preparation: 2-CTC resin (DL-B-122'-1, 3.00 mmol, 3.70 g, 0.81 mmol/g), N-((9H-fluoren-9-yl)methoxy)carbonyl)-N-methylglycine (3.00 mol, 933 mg, 3.00 equivalents) and DIPEA (4.00 equivalents) were added into dichloromethane (10.0 mL), and the reaction solution was allowed to react for 2 h under the atmosphere of nitrogen. Then, MeOH (1.0 mL) was added into the resin within 30 min under the atmosphere of nitrogen bubbling, followed by filtration to obtain a resin.
2) Conjugation: a solution of N-((9H-fluoren-9-yl)methoxy)carbonyl)-N-methylglycine (5.60 g, 6.00 equivalents) and HATU (6.58 g, 5.70 equivalents) in DMF (10.0 mL) was added into the resin under the nitrogen bubbling. After DIPEA (6.00 equivalents) was added dropwise, the resin was subjected to nitrogen bubbling for 30 min at 20°C. Before the next step was performed, the resin was washed with DMF (30.0 mL X 5).
3) Deprotection: a 20% piperidine solution in DMF (30.0 mL) was added into the resin, and the reaction mixture was subjected to nitrogen bubbling for 30 min at 20°C. Then, the resin was washed with DMF (30.0 mL×5).
4) Steps 2 and 3 were repeated using the amino acids listed in table 1: numbers 2-10 in table 1.
5) The resin was washed with DMF (30.0 mL × 5) and MeOH (30.0 mL × 5) and then dried in vacuum.

**Table 1:**

| ## | Materials | Conjugating reagent |
|---|---|---|
| 2 | N-((9H-fluoren-9-yl)methoxy)carbonyl)-N-methyl glycine (6.00 equivalent) | HATU (5.70 equivalent) and DIPEA (6.00 equivalent) |
| 3 | N-((9H-fluoren-9-yl)methoxy)carbonyl)-N-methyl glycine (6.00 equivalent) | HATU (5.70 equivalent) and DIPEA (6.00 equivalent) |
| 4 | N-((9H-fluoren-9-yl)methoxy)carbonyl)-N-methyl glycine (6.00 equivalent) | HATU (5.70 equivalent) and DIPEA (6.00 equivalent) |
| 5 | N-((9H-fluoren-9-yl)methoxy)carbonyl)-N-methyl glycine (6.00 equivalent) | HATU (5.70 equivalent) and DIPEA (6.00 equivalent) |
| 6 | N-((9H-fluoren-9-yl)methoxy)carbonyl)-N-methyl glycine (6.00 equivalent) | HATU (5.70 equivalent) and DIPEA (6.00 equivalent) |
| 7 | N-((9H-fluoren-9-yl)methoxy)carbonyl)-N-methyl glycine (6.00 equivalent) | HATU (5.70 equivalent) and DIPEA (6.00 equivalent) |
| 8 | N-((9H-fluoren-9-yl)methoxy)carbonyl)-N-methyl glycine (6.00 equivalent) | HATU (5.70 equivalent) and DIPEA (6.00 equivalent) |
| 9 | N-((9H-fluoren-9-yl)methoxy)carbonyl)-N-methyl glycine (6.00 equivalent) | HATU (5.70 equivalent) and DIPEA (6.00 equivalent) |
| 10 | 3,5-bis(2-(methylthio)pyrimidin-5-yl)benzoic acid (1.30 equivalent) | HATU (1.23 equivalent) and DIPEA (2.60 equivalent) |

### Cleavage and purification of polypeptide:

1) At room temperature, a lysis solution (TFA/DCM, 1/100, v/v, 200.0mL) was added into a flask containing a side chain-protected peptide and stirred for 3 min twice.
2) After filtration, the filtrates were combined and concentrated.
3) The crude product was purified via high performance liquid chromatography and then freeze-dried to obtain the title compound (1117.1 mg, TFA salt).

The structure characterization data is as follows:
ESI-MS (m/z): 1010.4 (M+H)⁺.

The purification method is as follows:

| Separation conditions | |
|---|---|
| Dissolution condition | DMF |
| Instrument | Auno DAC-100 |
| Mobile phase | A: Water (0.1% TFA in H₂O) |
| | B: Acetonitrile |
| Gradient | 20-50%--42 min; retention time: 21 min |
| Preparative column | Phenomenex Luna C18 250*100 mm, 10 um, 100Å |
| Flow rate | 250 mL/min |
| Detection wavelength | 220/254 nm |
| Temperature | 25°C |

### Step 2: preparation of 1-(3,5-bis(2-(methylsulfonyl)pyrimidin-5-yl)phenyl)-2,5,8,11,14,17,20,23,26,29-decamethyl-1,4,7,10,13,16,19,22,25,28-decaoxo-2,5,8,11,14,17,20,23,26,29-decaazahentriacontane-31-carboxylic acid (DL-B-122'-3)

1- (3,5-bis (2-(methylthio)pyrimidin-5-yl)phenyl)-2,5,8,11,14,17,20,23,26-nonamethyl-1,4,7,10,13,16,19,22,25-nonaoxo-2,5,8,11,14,17,20,23,26-nonazaoctacosane-28-ynoic acid (665 mg, 615.04 µmol) was added into water (7.5 mL) and acetonitrile (15 mL), sodium periodate (1.32 g, 6.15 mmol) and ruthenium trichloride hydrate (51.03 mg, 246.02 µmol) were added, and then the reaction solution was allowed to react for 1 h at 25°C. The reaction solution was directly purified via reverse phase column chromatography (acetonitrile/water (0.05% formic acid) = 0-25%) and then freeze-dried to obtain the title compound (515 mg, 449.69 µmol).

The structure characterization data is as follows:
ESI-MS (m/z): 1074.4 (M+H)⁺.

### Step 3: preparation of (6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-((S)-6-(3-(allyloxy)-3-oxopropyl)-13-(9H-fluoren-9-yl)-5,8,11-trioxo-2,12-dioxa-4,7,10-triazatridecyl)furan-2-carboxylate (DL-B-122'-4)

Allyl (S)-4-(2-(((9H-fluoren-9-yl)methoxy)carbonyl)amino)acetylamino)-5-(acetoxymethyl)amino)-5-oxovalerate (1.42 g, 2.64 mmol), (6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-(hydroxymethyl)furan-2-carboxylate (1.00 g, 1.76 mmol) was dissolved in dichloromethane (15.0 mL), PPTS (441 mg, 1.76 mmol) was added, and then the reaction solution was heated to 50°C and allowed to react for 3 h. Water (300 mL) was added into the reaction solution, followed by extraction with dichloromethane for 3 times (100 mL x 3). The organic phases were combined, dried over anhydrous sodium sulfate, filtered and concentrated to obtain a crude product, which was purified via a silica gel column (dichloromethane/methanol = 100/1 to 20/1) and then concentrated again to obtain the title compound (910 mg, 870 µmol).

The structure characterization data is as follows:
ESI-MS (m/z): 1046.3 (M+H)⁺.

### Step 4: preparation of (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-(((S)-5-(allyloxy)-2-(2-aminoacetamido)-5-oxopentanamido)methoxy)methyl)furan-2-carboxylate (DL-B-122'-5)

(6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-((S)-6-(3-(allyloxy)-3-oxopropyl)-13-(9H-fluoren-9-yl)-5,8,11-trioxo-2,12-dioxa-4,7,10-triazatridecyl)furan-2-carboxylate (37 mg, 35.37 µmol) and diethylamine (5.17 mg, 70.74 µmol) were added into DMF (3 mL), and then the reaction solution was allowed to react for 3 h at 22°C under stirring after the addition was finished. After the reaction was completed, the residual diethylamine was removed under reduced pressure to obtain the title compound as a crude product (29 mg), which was directly used in the next step without purification.

The structure characterization data is as follows:
ESI-MS (m/z): 824.2(M+H)⁺.

### Step 5: preparation of allyl (S)-1-(3,5-bis(2-(methylsulfonyl)pyrimidin-5-yl)phenyl)-36-((((5-(((6S,8S,9R, 10S, 11 S, 13S, 14S, 16R, 17R)-6,9-difluoro-17-((fluoromethyl)thio)carbonyl)-11-hydroxy-10,13,16-trimethyl-3-oxo-6,7,8,10,11,12,13,14,15,16,17-dodecahydro-3H-cyclopenta[a]phenanthren-17-yl)oxy)carbonyl)furan-3-yl)methoxy)methyl)carbamoyl)-2,5,8,11,14,17,20,23,26,29-decamethyl-1,4,7,10,13,16,19,22,25,28,31,34-dodecaoxo-2,5,8,11,14,17,20,23,26,29,32,35-dodecaazanonatriacontane-39-carboxylate (DL-B-122'-6)

(6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-(((S)-5-(allyloxy)-2-(2-aminoacetamido)-5-oxopentanamido)methoxy)methyl)furan-2-carboxylate (41 mg, 35.80 µmol), DIPEA (13.65 mg, 105.60 µmol) and HATU (17.40 mg, 45.76 µmol) were added into DMF (3 mL), and then the reaction solution was stirred for 5 min at 22°C after the addition was finished. (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-(((S)-5-(allyloxy)-2-(2-aminoacetamide)-5-oxopentanamide)methoxy)methyl)furan-2-carboxylate (29 mg, crude) was finally added, and then the reaction solution was allowed to react for 2 h at 22°C under stirring after the addition was finished. After the reaction was completed, the solvent was removed under reduced pressure to obtain the title compound as a crude product (40 mg, crude), which was directly used in the next step without purification.

The structure characterization data is as follows:
ESI-MS (m/z): 1968.7(M+H)⁺.

### Step 6: preparation of preparation of (S)-1-(3,5-bis(2-(methylsulfonyl)pyrimidin-5-yl)phenyl)-36-((5-(((6S,8S,9R,10S,11S,13S,14S,16R,17R)-6,9-difluoro-17-((fluoromethyl) thio)carbonyl)-11-hydroxy-10,13,16-trimethyl-3-oxo-6,7,8,10,11,12,13,14,15,16,17-dodecahydro-3H-cyclopenta[a]phenanthren-17-yl)oxy)carbonyl)furan-3-yl)methoxy)methyl)carbamoyl)-2,5,8,11,14,17,23,26,29-decamethyl-1,4,7,10,13,16,19,22,25,28,31,34-dodecoxo-2,5,8,11,14,17,20,23,26,29,32,35-dodecaazanonatriacontane-39-carboxylic acid (DL-B-122')

Allyl (S)-1-(3,5-bis(2-(methylsulfonyl)pyrimidin-5-yl)phenyl)-36-((((5-(((6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,10,11,12,13,14,15,16,17-dodecahydro-3H-cyclopenta[a]phenanthren-17-yl)oxy)carbonyl)furan-3-yl)methoxy)methyl)carbamoyl)-2,5,8,11,14,17,20,23,26,29-decamethyl-1,4,7,10,13,16,19,22,25,28,31,34-dodecaoxo-2,5,8,11,14,17,20,23,26,29,32,35-dodecaazanonatriacontane-39-carboxylate (40 mg, crude) was added into DCM (3 mL), tetra(triphenylphosphine)palladium (20 mg, 17.31 µmol), formic acid (20 µL) and N-methylmorpholine (40 µL) were added under the protection of nitrogen, and then the reaction solution was allowed to react for 2 h at 22°C under stirring under the protection of nitrogen after the addition was finished. The reaction solution was purified via high performance liquid chromatography to obtain the title compound (27.66 mg, 14.33 µmol).

The purification method is as follows:
Chromatographic column: SunFire Prep C18 OBD 19×150 mm×5 µm
Mobile phase A: acetonitrile; mobile B: water (0.05% TFA)

| Time [min] | Mobile phase A [%] | Mobile phase B [%] | Flow rate [mL/min] |
|---|---|---|---|
| 0.00 | 30 | 70 | 32 |
| 4.00 | 30 | 70 | 32 |
| 24.00 | 90 | 10 | 32 |

The structure characterization data is as follows:
ESI-MS (m/z): 1910.7(M+H)⁺.

### Preparation example 63: Preparation of (6S,8S,9R,10S,11S,13S,14S,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-((6S,9S)-6-(4-aminobutyl)-44-(3,5-bis(2-(methylsulfonyl)pyrimidin-5-yl)phenyl)-9-hydroxymethyl-16,19,22,25,28,31,34,37,40,43-decamethyl-5,8,11,14,17,20,23,26,29,32,35,38,41,44-tetradecaoxo-2-oxa-4,7,10,13,16,19,22,25,28,31,34,37,40,43-tetradecaazatetratetracontyl)furan-2-carboxylate (DL-B-123')

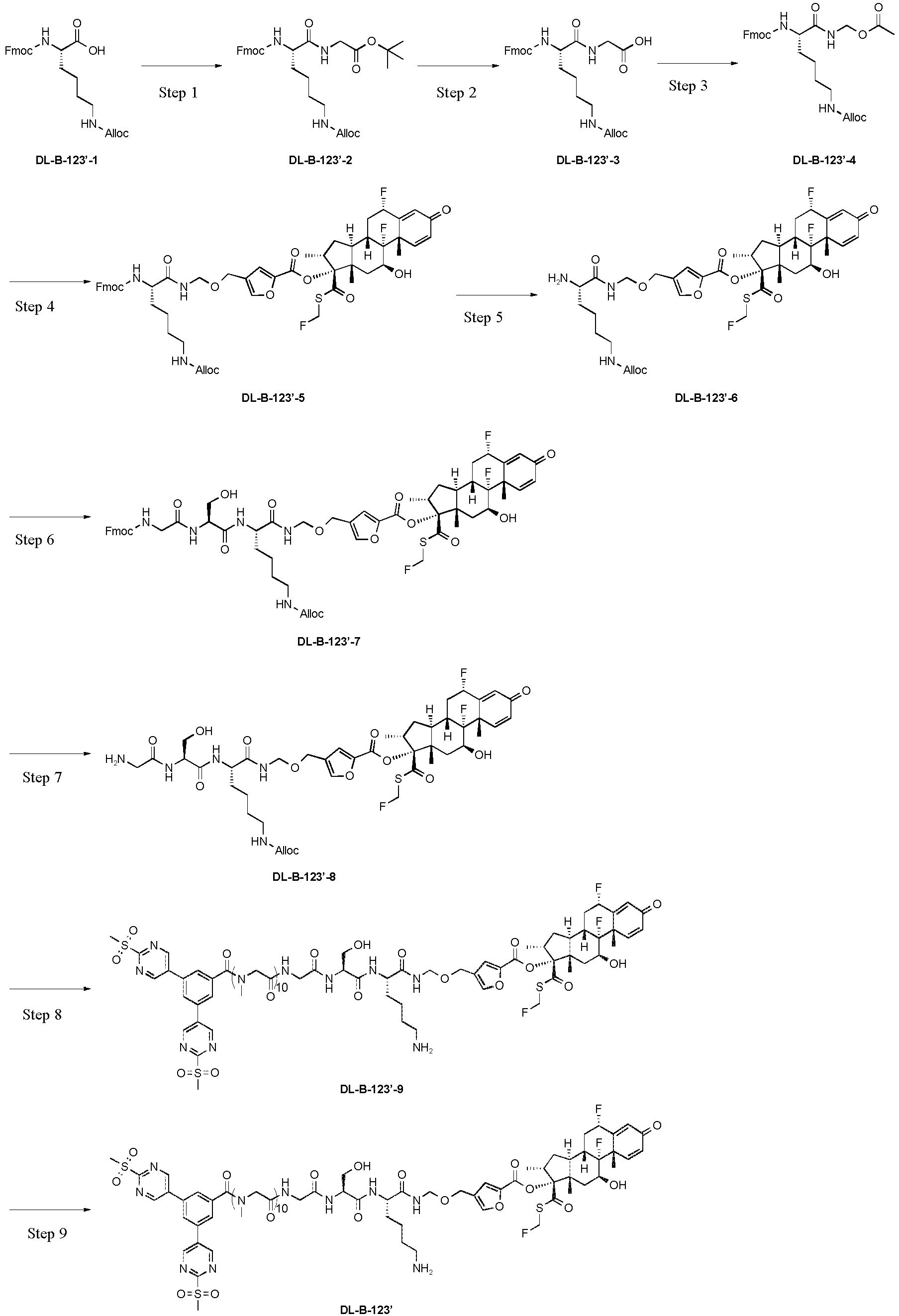

### Step 1: preparation of tert-butyl N²-((9H-fluoren-9-yl)methoxy)carbonyl)-N⁶-(allyloxy)carbonyl)-L-lysine glycinate (DL-B-123'-2)

N²- ((9H-fluoren-9-yl)methoxy)carbonyl)-N⁶-(allyloxy)carbonyl)-L-lysine (10.0 g, 22.1 mmol) was dissolved in DMF (100 mL), HATU (12.6 g, 33.2 mmol), DIPEA (8.57 g, 66.3 mmol, 11.6 mL) and tert-butyl glycinate (2.90 g, 22.1 mmol) were added, and then the reaction solution was allowed to react for 2 h at 25°C. Water (500 mL) was added into the reaction solution, followed by extraction with ethyl acetate for 3 times (300 mL x 3). The organic phases were combined, dried over anhydrous sodium sulfate, filtered and concentrated to obtain a crude product, which was purified via a silica gel column (dichloromethane/methanol = 100/1 to 20/1), and then concentrated again to obtain the title compound (9.76 g, 15.7 mmol).

The structure characterization data is as follows:
ESI-MS (m/z): 566.2 (M+Na)⁺.

### Step 2: preparation of N²-((9H-fluoren-9-yl)methoxy)carbonyl)-N⁶-((allyloxy)carbonyl)- L-lysine glycine (DL-B-123'-3)

Tert-butyl N²-((9H-fluoren-9-yl)methoxy)carbonyl)-N⁶-(allyloxy)carbonyl)-L-lysine glycinate (9.76 g, 17.3 mmol) was dissolved in DCM (50.0 mL), trifluoroacetic acid (25.6 g, 225 mmol, 16.7 mL) was added, and then the reaction solution was allowed to react for 5 h at 25°C. The reaction solution was directly concentrated to obtain a trifluoroacetate of the title compound as a crude product (12.5 g, 20.1 mmol), which was directly used in the next step without purification.

The structure characterization data is as follows:
ESI-MS (m/z): 510.2 (M+Na)⁺.

### Step 3: preparation of methyl (S)-(2-(((9H-fluoren-9-yl)methoxy)carbonyl)amino)-6-(((allyloxy)carbonyl)amino)hexanamido)acetate (DL-B-123'-4)

N²-((9H-fluoren-9-yl)methoxy)carbonyl)-N⁶-((allyloxy)carbonyl)- L-lysine glycine (12.5 g, 20.1 mmol, trifluoroacetate) was dissolved in DMF (130 mL), copper acetate (1.46 g, 8.02 mmol), lead tetraacetate (10.7 g, 24.1 mmol) and acetic acid (2.65 g, 44.1 mmol, 2.52 mL) were added, and then the reaction solution was heated to 60°C and allowed to react for 2 h. Water (50.0 mL) was added into the reaction solution, followed by extraction with dichloromethane for 3 times (30.0 mL x 3). The organic phases were combined, dried over anhydrous sodium sulfate, filtered and concentrated to obtain a crude product, which was purified via a silica gel column (dichloromethane/methanol = 100/1 to 10/3) to obtain the title compound (3 g, 5.73mmol).

The structure characterization data is as follows:
ESI-MS (m/z): 546.0 (M+Na)⁺.

### Step 4: preparation of (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-((S)-6-(((9H-fluoren-9-yl)methoxy)carbonyl)amino)-5,12-dioxo-2,13-dioxa-4,11-diazahexadec-15-en-1-yl)furan-2-carboxylate (DL-B-123'-5)

Methyl (S)-(2-(((9H-fluoren-9-yl)methoxy)carbonyl)amino)-6-(((allyloxy)carbonyl)amino)hexanamido)acetate (1.00 g, 1.91 mmol) was dissolved in dichloromethane (20.0 mL), PPTS (384 mg, 1.53 mmol) and (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-(hydroxymethyl)furan-2-carboxylate (1.09 g, 1.91 mmol) were added, and then the reaction solution was heated to 45°C and allowed to react for 2 h. Water (500 mL) was added into the reaction solution, followed by extraction with ethyl acetate for 3 times (300 mL x 3). The organic phases were combined, washed with a saturated sodium chloride aqueous solution for 3 times (200 mL x 3), dried over anhydrous sodium sulfate, filtered and concentrated to obtain a crude product, which was purified via a silica gel column (dichloromethane/methanol = 100/1 to 20/1) and then concentrated again to obtain the title compound (1.06 g, 1.03 mmol).

The structure characterization data is as follows:
ESI-MS (m/z): 1032.3 (M+H)⁺.

### Step 5: preparation of (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-((S)-6-amino-5,12-dioxo-2,13-dioxa-4,11-diazahexadec-15-en-1-yl)furan-2-carboxylate (DL-B-123'-6)

(6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-((S)-6-(((9H-fluoren-9-yl)methoxy)carbonyl)amino)-5,12-dioxo-2,13-dioxa-4,11-diazahexadec-15-en-1-yl)furan-2-carboxylate (527 mg, 511 µmol) was dissolved in DMF (5.00 mL), DBU (62.2 mg, 408 µmol, 61.6 µL) was added, and then the reaction solution was allowed to react for 0.5 h at 25°C. The reaction solution was directly used in the next step without post-treatment.

The structure characterization data is as follows:
ESI-MS (m/z): 810.4 (M+H)⁺.

### Step 6: preparation of (6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-((6S,9S)-6-(4-(((allyloxy)carbonyl)amino)butyl)-16-(9H-fluoren-9-yl)-9-(hydroxymethyl)-5,8,11,14-tetraoxo-2,15-dioxa-4,7,10,13-tetraazahexadecyl)furan-2-carboxylate (DL-B-123'-7)

EDCI (147 mg, 766 µmol), HOBt (104 mg, 766 µmol) and ((9H-fluoren-9-yl)methoxy)carbonyl)glycyl-L-serine (196 mg, 511 µmol) were added into the reaction solution obtained in the previous step, and then the reaction solution was allowed to react for 1 h at 25°C. The reaction solution was poured into water (50.0 mL), stirred for additional 30 min, and then filtered, and then the filter cake was washed with water for 3 times (10.0 mL x 3). The filter cake was dried and then purified via a silica gel column (dichloromethane/methanol = 100/1 to 100/6) and concentrated again to obtain the title compound (308 mg, 231 µmol).

The structure characterization data is as follows:
ESI-MS (m/z): 1176.7 (M+H)⁺.

### Step 7: preparation of (6S,8S,9R,10S,11S,13S,14S,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-((S)-6-((S)-2-(2-aminoacetamido)-3-hydroxypropanamido)-5,12-dioxo-2,13-dioxa-4,11-diazahexadec-15-en-1-yl)furan-2-carboxylate (DL-B-123'-8)

Compound (6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-((6S,9S)-6-(4-(((allyloxy)carbonyl)amino)butyl)-16-(9H-fluoren-9-yl)-9-(hydroxymethyl)-5,8,11,14-tetraoxo-2,15-dioxa-4,7,10,13-tetraazahexadecyl)furan-2-carboxylate (24.0 mg, 20.40 µmol) was dissolved in DMF (0.5 mL), diethylamine (7.5 mg, 102.02 µmol) was added, the reaction solution was allowed to react for 1 h at 25°C. Then the reaction solution was directly purified via high performance liquid chromatography to obtain the title compound (12.0 mg, 12.58 µmol).

The purification method is as follows:
Chromatographic column: SunFire Prep C18 OBD 19*150 mm*5 µm
Mobile phase A: acetonitrile; mobile phase B: water (0.05% ammonium bicarbonate)

| Time [min] | Mobile phase A [%] | Mobile phase B [%] | Flow rate [mL/min] |
|---|---|---|---|
| 0.00 | 30 | 70 | 28 |
| 2.00 | 30 | 70 | 28 |
| 22.00 | 90 | 10 | 28 |

The structure characterization data is as follows:
ESI-MS (m/z): 954.3 (M+H)⁺.

### Step 8: preparation of (6S,8S,9R,10S,11S,13S,14S,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-(6S,9S)-6-(4-(((allyloxy)carbonyl)amino)butyl)-44-(3,5-bis(2-(methylsulfonyl)pyrimidin-5-yl)phenyl)-9-hydroxymethyl-16,19,22,25,28,31,34,37,40,43-decamethyl-5,8,11,14,17,20,23,26,29,32,35,38,41,44-tetradecaoxo-2-oxa-4,7,10,13,16,19,22,25,28,31,34,37,40,43-tetradecaazatetratetracontyl)furan-2-carboxylate (DL-B-123'-9)

(6S,8S,9R,10S,11S,13S,14S,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-((S)-6-((S)-2-(2-aminoacetamido)-3-hydroxypropanamido)-5,12-dioxo-2,13-dioxa-4,11-diazahexadec-15-en-1-yl)furan-2-carboxylate (11.0 mg, 11.53 µmol) was dissolved in DMF (1 mL), HATU (8.8 mg, 23.06 µmol), DIPEA (4.5 mg, 34.59 µmol) and 1-(3,5-bis(2-(methylsulfonyl)pyrimidin-5-yl)phenyl)-2,5,8,11,14,17,20,23,26,29-decamethyl-1,4,7,10,13,16,19,22,25,28-decaoxo-2,5,8,11,14,17,20,23,26,29-decaazahentriacontane-31-carboxylic acid (13.9 mg, 12.11 µmol) were added, and then the reaction solution was allowed to react for 1 h at 25°C. Subsequently the reaction solution was directly purified via high performance liquid chromatography to obtain the title compound (9.0 mg, 4.19 µmol).

The purification method is as follows:
Chromatographic column: SunFire Prep C18 OBD 19*150 mm*5 µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 30 | 70 | 28 |
| 2.00 | 30 | 70 | 28 |
| 22.00 | 90 | 10 | 28 |

The structure characterization data is as follows:
ESI-MS (m/z): 1041.0 (M+H)⁺.

### Step 9: preparation of (6S,8S,9R,10S,11S,13S,14S,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-((6S,9S)-6-(4-aminobutyl)-44-(3,5-bis(2-(methylsulfonyl)pyrimidin-5-yl)phenyl)-9-hydroxymethyl-16,19,22,25,28,31,34,37,40,43-decamethyl-5,8,11,14,17,20,23,26,29,32,35,38,41,44-tetradecaoxo-2-oxa-4,7,10,13,16,19,22,25,28,31,34,37,40,43-tetradecaazatetratetracontyl)furan-2-carboxylate (DL-B-123')

Under the protection of nitrogen, compound (6S,8S,9R,10S,11S,13S,14S,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-(6S,9S)-6-(4-(((allyloxy)carbonyl)amino)butyl)-44-(3,5-bis(2-(methylsulfonyl)pyrimidin-5-yl)phenyl)-9-hydroxymethyl-16,19,22,25,28,31,34,37,40,43-decamethyl-5,8,11,14,17,20,23,26,29,32,35,38,41,44-tetradecaoxo-2-oxa-4,7,10,13,16,19,22,25,28,31,34,37,40,43-tetradecaazatetratetracontyl)furan-2-carboxylate (8.0 mg, 3.84 µmol) was dissolved in DMF (0.5 mL), formic acid (2.77 mg, 57.66 µmol), N-methylmorpholine (3.89 mg, 38.44 µmol) and tetra(triphenylphosphine)palladium (2.2 mg, 1.92 µmol) were successively added, and then the reaction solution was allowed to react for 2 h at 25°C. The reaction solution was directly purified via high performance liquid chromatography to obtain the title compound (3.88 mg, 1.84 µmol).

The purification method is as follows:
Chromatographic column: SunFire Prep C18 OBD 19*150 mm*5 µm
Mobile phase A: acetonitrile; mobile B: water (0.05% TFA)

| Time [min] | Mobile phase A [%] | Mobile phase B [%] | Flow rate [mL/min] |
|---|---|---|---|
| 0.00 | 10 | 90 | 30 |
| 2.00 | 10 | 90 | 30 |
| 20.00 | 80 | 20 | 30 |

The structure characterization data is as follows:
ESI-MS (m/z): 999.3 (M/2+H)⁺, 1997.7 (M+H)⁺.

### Preparation example 64: Preparation of (6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,15,16,17-dodecahydro-3H-cyclopenta[a]phenanthren-17-yl 4-((6S,9S,12S)-44-(3,5-bis(2-(methylsulfonyl)pyrimidin-5-yl)phenyl)-6,9,12,16,19,22,25,28,31,34,37,40,43-tridecamethyl-5,8,11,14,17,20,23,26,29,32,35,38,41,44-tetradeoxo-2-oxa-4,7,10,13,16,19,22,25,28,31,34,37,40,43-tetradecaazatetratetracontyl)furan-2-carboxylate (DL-B-124')

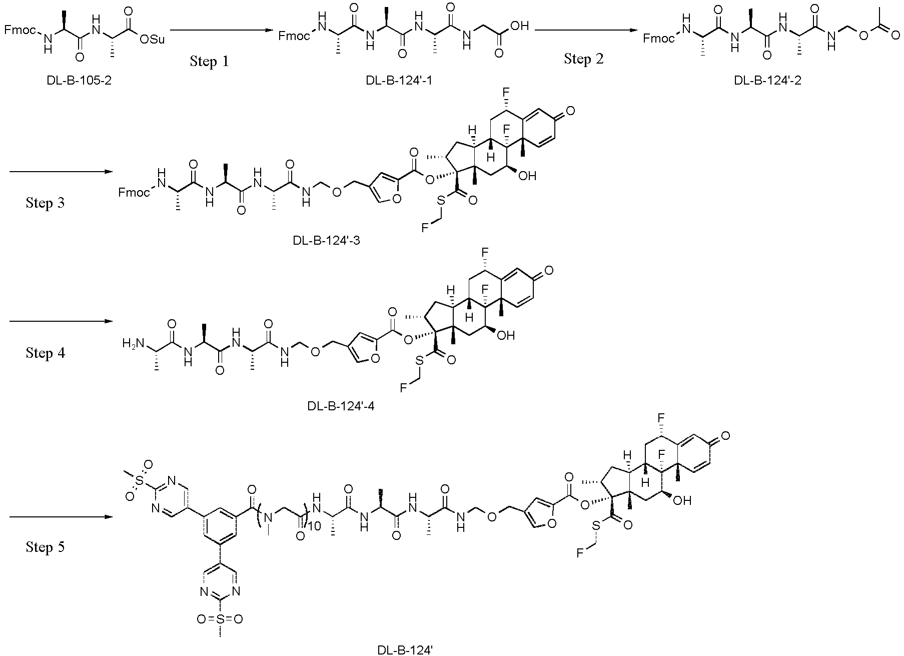

### Step 1: preparation of ((9H-fluoren-9-yl)methox)carbonyl)-L-alanine-L-alanine-L-alanine glycine (DL-B-124'-1)

2,5-dioxopyrrolidin-1-yl (9H-fluoren-9-yl)methoxy)carbonyl)-L-alanine-L-alanine (5.00 g, 10.4 mmol) and L-alanine glycine (1.52 g, 10.4 mmol) were added into a mixed solvent of water (25.0 mL) and tetrahydrofuran (50.0 mL), a sodium bicarbonate aqueous solution (1.75 g, 20.8 mmol, 811 µL) was added, and then the reaction solution was stirred for 2 h at 25°C. The reaction solution was adjusted to pH 2-3 using 1 N diluted hydrochloric acid, and the reaction solution was extracted with dichloromethane for 3 times (60.0 mL x 3). The organic phases were combined, dried over anhydrous sodium sulfate, filtered and concentrated to obtain the title compound as a crude product (3.18 g, 6.23 mmol), which was directly used in the next step without purification.

The structure characterization data is as follows:
ESI-MS (m/z): 511.1 (M+H)⁺.

### Step 2: preparation of (5S,8S,11S)-1-(9H-fluoren-9-yl)-5,8,11-trimethyl-3,6,9,12-tetraoxo-2-oxa-4,7,10,13-tetraazatetradecan-14-yl acetate (DL-B-124'-2)

((9H-fluoren-9-yl)methox)carbonyl)-L-alanine-L-alanine-L-alanine glycine (3.10 g, 6.07 mmol), copper acetate (882 mg, 4.86 mmol) and lead tetraacetate (6.46 g, 14.5 mmol) were dissolved in DMF (40.0 mL), glacial acetic acid (1.60 g, 26.7 mmol, 1.53 mL) was added, and then the reaction solution was heated to 60°C and stirred for 1 h. The reaction solution was directly concentrated to obtain a crude product, which was purified via a silica gel column (dichloromethane/methanol = 100/1 to 10/1) and then concentrated again to obtain the title compound as a crude product (709 mg, 1.28 mmol), which was directly used in the next step without purification.

The structure characterization data is as follows:
¹H NMR (400 MHz, DMSO) δ 8.85 (m, 1H), 7.97 (m, 2H), 7.89 (d, J = 7.6 Hz, 2H), 7.72 (m, 2H), 7.53 (m, 1H), 7.39-7.44 (m, 2H), 7.30-7.35 (m, 2H), 5.04-5.10 (m, 2H), 4.24-4.28 (m, 3H), 3.17 (d, J = 5.2 Hz, 3H), 1.98 (s, 3H), 1.18-1.22 (m, 9H).

### Step 3: preparation of (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-((6S,9S,12S)-16-(9H-fluoren-9-yl)-6,9,12-trimethyl-5,8,11,14-tetraoxo-2,15-dioxa-4,7,10,13-tetraazahexadecyl)furan-2-carboxylate (DL-B-124'-3)

(5S,8S,11S)-1-(9H-fluoren-9-yl)-5,8,11-trimethyl-3,6,9,12-tetraoxo-2-oxa-4,7,10,13-tetraazatetradecan-14-yl acetate (583 mg, 1.03 mmol) were dissolved in dichloromethane (15.0 mL), PPTS (206 mg, 821 µmol) was added, and then the reaction solution was heated to 45°C and stirred for 10 h. The reaction solution was cooled to 25°C, added with water (20.0 mL), and then extracted with dichloromethane for 3 times (30.0 mL x 3). The organic phases were combined, dried over anhydrous sodium sulfate, filtered and concentrated to obtain a crude product, which was purified via a silica gel column (petroleum ether/ethyl acetate = 100/1 to 20/1) and then concentrated again to obtain the title compound (390 mg, 283 µmol).

The structure characterization data is as follows:
ESI-MS (m/z): 1033.5 (M+H)⁺.

### Step 4: preparation of (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-((6S,9S,12S)-12-amino-6,9-dimethyl-5,8,11-trioxo-2-oxa-4,7,10-triazatridecyl)furan-2-carboxylate (DL-B-124'-4)

(6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-((6S,9S,12S)-16-(9H-fluoren-9-yl)-6,9,12-trimethyl-5,8,11,14-tetraoxo-2,15-dioxa-4,7,10,13-tetraazahexadecyl)furan-2-carboxylate (70 mg, 67.76 µmol) was added into DMF (2 mL), and then diethylamine (9.91 mg, 135.51 µmol) was added. The reaction solution was allowed to react for 1 h at 25°C. The reaction solution was directly purified via high performance liquid chromatography and then freeze-dried to obtain the title compound (35 mg, 41.01 µmol).

The purification method is as follows:
Chromatographic column: SunFire Prep C18 OBD 19*150 mm*5 µm
Mobile phase A: acetonitrile; mobile phase B: water (0.05% FA)

| Time [min] | Mobile phase A [%] | Mobile phase B [%] | Flow rate [mL/min] |
|---|---|---|---|
| 0.00 | 20 | 80 | 32 |
| 4.00 | 20 | 80 | 32 |
| 24.00 | 90 | 10 | 32 |

The structure characterization data is as follows:
ESI-MS (m/z): 811.5 (M+H)⁺.

### Step 5: preparation of (6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,15,16,17-dodecahydro-3H-cyclopenta[a]phenanthren-17-yl 4-((6S,9S,12S)-44-(3,5-bis(2-(methylsulfonyl)pyrimidin-5-yl)phenyl)-6,9,12,16,19,22,25,28,31,34,37,40,43-tridecamethyl-5,8,11,14,17,20,23,26,29,32,35,38,41,44-tetradeoxo-2-oxa-4,7,10,13,16,19,22,25,28,31,34,37,40,43-tetradecaazatetratetracontyl)furan-2-carboxylate (DL-B-124')

1-(3,5-bis(2-(methylsulfonyl)pyrimidin-5-yl)phenyl)-2,5,8,11,14,17,20,23,26,29-decamethyl-1,4,7,10,13,16,19,22,25,28-decaoxo-2,5,8,11,14,17,20,23,26,29-decaazahentriacontane-31-carboxylic acid (49.43 mg, 43.16 µmol), DIPEA (16.74 mg, 129.49 µmol) and HATU (24.60 mg, 64.74 µmol) were added into DMF (2 mL), and the reaction solution was stirred for 30 min. then (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 4-((6S,9S,12S)-12-amino-6,9-dimethyl-5,8,11-trioxo-2-oxa-4,7,10-triazatridecyl)furan-2-carboxylate (35 mg, 43.16 µmol) was added. The reaction solution was allowed to react for 1 h at 25°C. The reaction solution was directly purified via high performance liquid chromatography and then freeze-dried to obtain the title compound (21 mg, 10.29 µmol).

The purification method is as follows:
Chromatographic column: SunFire Prep C18 OBD 19*150 mm*5 µm
Mobile phase A: acetonitrile; mobile phase B: water (0.05% FA)

| Time [min] | Mobile phase A [%] | Mobile phase B [%] | Flow rate [mL/min] |
|---|---|---|---|
| 0.00 | 30 | 70 | 32 |
| 4.00 | 30 | 70 | 32 |
| 24.00 | 90 | 10 | 32 |

The structure characterization data is as follows:
ESI-MS (m/z): 1836.7 (M+H)⁺.

### Preparation example 65 Preparation of (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (33 S,36S)-1-(3,5-bis(2(methylsulfonyl)pyrimidin-5-yl)phenyl)-2,5,8,11,14,17,20,23,26,29,33,36-dodecamethyl-1,4,7,10,13,16,19,22,25,28,31,34,37-tridecaoxo-40-oxa-2,5,8,11,14,17,20,23,26,29,32,35,38-tridecaazatritetracontane-43-carboxylate (DL-B-125')

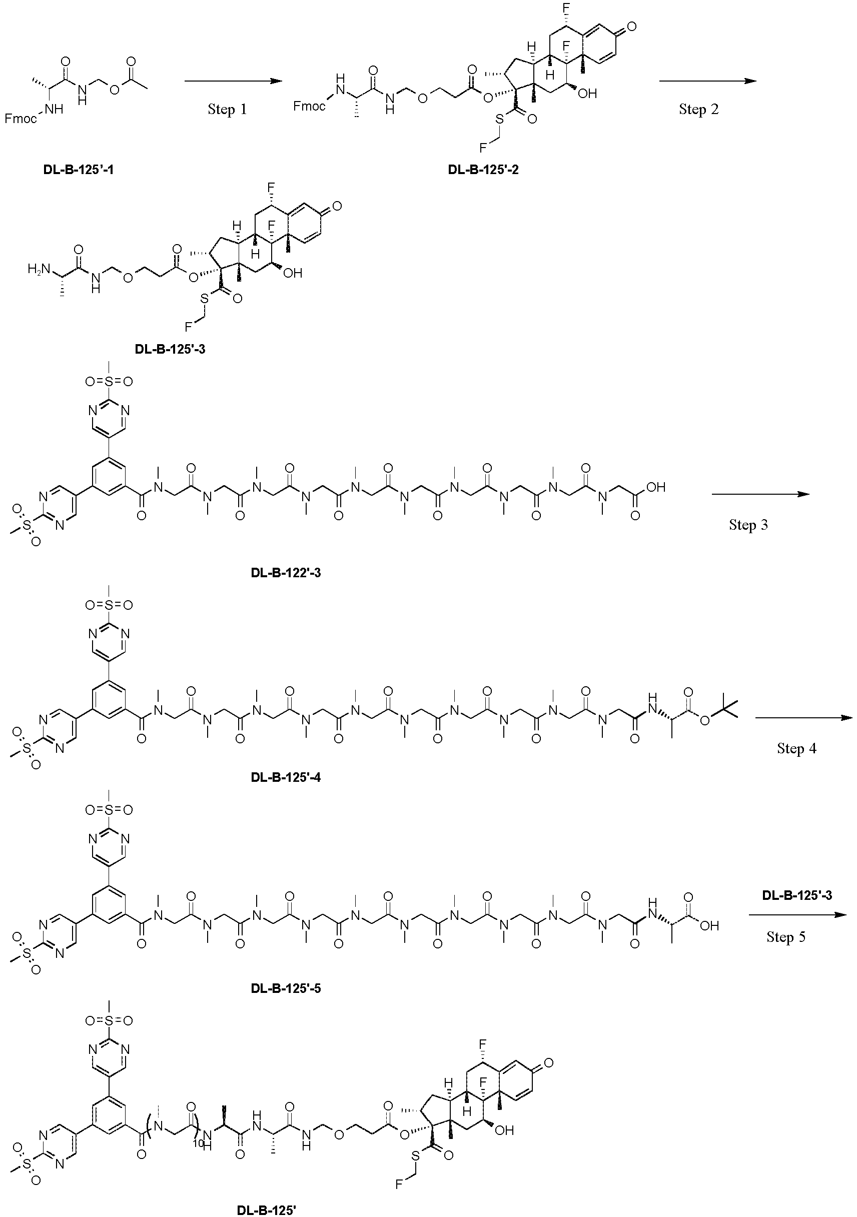

### Step 1: preparation of (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (S)-1-(9H-fluoren-9-yl)-5-methyl-3,6-dioxo-2,9-dioxa-4,7-diazadodecane-12-carboxylate (DL-B-125'-2)

Under the protection of nitrogen, (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 3-hydroxypropionate (50 mg, 96.79 µmol) was dissolved in DCM (5 mL), methyl (R)-(2-(((((9H-fluoren-9-yl)methoxy)carbonyl)amino)propionamido)acetate (55.52 mg, 145.19 µmol) and PPTS (24.32 mg, 96.79 µmol) were added, and then the reaction solution was subjected to reflux and allowed to react for 6 h at 40°C. The reaction solution was quenched with water (5 mL) and extracted with DCM twice (4 mL x 2). The organic phase was dried and then concentrated to obtain a crude product, which was purified via a silica gel column (SiO₂, methanol/dichloromethane = 5% to 40%) to obtain the title compound (44 mg, 49.83 µmol)

The structure characterization data is as follows:
ESI-MS (m/z): 839.4 (M+H)⁺.

### Step 2: preparation of (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 3-((S)-2-aminopropanamidomethoxy)propionate (DL-B-125'-3)

(6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (S)-1-(9H-fluoren-9-yl)-5-methyl-3,6-dioxo-2,9-dioxa-4,7-diazadodecane-12-carboxylate (44 mg, 49.83 µmol) was added into DMF (1 mL), diethylamine (0.2 mL) was added, and then the reaction solution was allowed to react for 1 h at 25°C. The reaction solution was purified via high performance liquid chromatography and then freeze-dried to obtain the title compound (17.0 mg, 27.29 µmol).

The purification method is as follows:
Chromatographic column: SunFire Prep C18 OBD 19*150 mm*5 µm
Mobile phase A: acetonitrile; mobile B: water (0.05% TFA)

| Time [min] | Mobile phase A [%] | Mobile phase B [%] | Flow rate [mL/min] |
|---|---|---|---|
| 0.00 | 15 | 85 | 30 |
| 2.00 | 15 | 85 | 30 |
| 18.00 | 80 | 20 | 30 |

The structure characterization data is as follows:
ESI-MS (m/z): 617.3 (M+H)⁺.

### Step 3: preparation of tert-butyl (1-(3,5-bis(2-(methylsulfonyl)pyrimidin-5-yl)phenyl)-2,5,8,11,14,17,20,23,26,29-decamethyl-1,4,7,10,13,16,19,22,25,28-decaoxo-2,5,8,11,14,17,20,23,26,29-decaazahentriacontan-31-oyl)-L-alanine (DL-B-125'-4)

1-(3,5-bis(2-(methylsulfonyl)pyrimidin-5-yl)phenyl)-2,5,8,11,14,17,20,23,26,29-decamethyl-1,4,7,10,13,16,19,22,25,28-decaoxo-2,5,8,11,14,17,20,23,26,29-decaazahentriacontane-31-carboxylic acid (50.0 mg, 43.66 µmol) was dissolved in DMF (2 mL), HATU (24.9 mg, 65.49 µmol), DIPEA (22.6 mg, 174.64 µmol) and and tert-butyl L-alaninate (7.93 mg, 43.66 µmol, hydrochloride) were added, and then the reaction solution was allowed to react for 1 h at 25°C. The reaction solution was directly purified via high performance liquid chromatography and then freeze-dried to obtain the title compound (44 mg, 33.89 µmol).

The purification method is as follows:
Chromatographic column: WatersSunFire Prep C18 OBD (5 µm*19 mm*150 mm)
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 20 | 80 | 32 |
| 4.00 | 20 | 80 | 32 |
| 24.00 | 90 | 10 | 32 |

The structure characterization data is as follows:
ESI-MS (m/z): 1273.6 (M+H)⁺.

### Step 4: preparation of (1-(3,5-bis(2-(methylsulfonyl)pyrimidin-5-yl)phenyl)-2,5,8,11,14,17,20,23,26,29-decamethyl-1,4,7,10,13,16,19,22,25,28-decaoxo-2,5,8,11,14,17,20,23,26,29-decaazahentriacontane-31-oyl)-L-alanine (DL-B-125'-5)

Tert-butyl (1-(3,5-bis(2-(methylsulfonyl)pyrimidin-5-yl)phenyl)-2,5,8,11,14,17,20,23,26,29-decamethyl-1,4,7,10,13,16,19,22,25,28-decaoxo-2,5,8,11,14,17,20,23,26,29-decaazahentriacontane-31-oyl)-L-alanine (42.1 mg, 31.44 µmol) was dissolved in DCM (2 mL), TFA (3.6 mg, 31.44 µmol) was added, and then the reaction solution was allowed to react for 1 h at 25°C. The reaction solution was directly purified via high performance liquid chromatography and then freeze-dried to obtain the title compound (30 mg, 23.43 µmol).

The purification method is as follows:
Chromatographic column: WatersSunFire Prep C18 OBD (5 µm*19 mm*150 mm)
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 10 | 90 | 30 |
| 3.00 | 10 | 90 | 30 |
| 18.00 | 70 | 30 | 30 |

The structure characterization data is as follows:
ESI-MS (m/z): 1217.5 (M+H)⁺.

### Step 5: preparation of (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (33S,36S)-1-(3,5-bis(2(methylsulfonyl)pyrimidin-5-yl)phenyl)-2,5,8,11,14,17,20,23,26,29,33,36-dodecamethyl-1,4,7,10,13,16,19,22,25,28,31,34,37-tridecaoxo-40-oxa-2,5,8,11,14,17,20,23,26,29,32,35,38-tridecaazatritetracontane-43-carboxylate (DL-B-125')

(1-(3,5-bis(2-(methylsulfonyl)pyrimidin-5-yl)phenyl)-2,5,8,11,14,17,20,23,26,29-decamethyl-1,4,7,10,13,16,19,22,25,28-decaoxo-2,5,8,11,14,17,20,23,26,29-decaazahentriacontane-31-oyl)-L-alanine (19.27 mg, 15.84 µmol) was dissolved in DMF (2 mL), HATU (23.19 mg, 60.36 µmol), DIPEA (9.75 mg, 75.45 µmol) and(6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 3-((S)-2-aminopropanamidomethoxy)propionate (10.0 mg, 15.09 µmol, formate) were added, and then the reaction solution was allowed to react for 1 h at 25°C. The reaction solution was directly purified via high performance liquid chromatography and then freeze-dried to obtain the title compound (7.7 mg, 4.16 µmol).

The purification method is as follows:
Chromatographic column: WatersSunFire Prep C18 OBD (5 µm*19 mm*150 mm)
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 30 | 70 | 30 |
| 2.00 | 30 | 70 | 30 |
| 18.00 | 90 | 10 | 30 |

The structure characterization data is as follows:
ESI-MS (m/z): 1836.7 (M+H)⁺.

### Preparation example 66: Preparation of (S)-1- (3,5-bis (2-(methylsulfonyl)pyrimidin-5-yl)phenyl)-36-((3-(((6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl)oxy)-3-oxopropoxy)methyl)carbamoyl)-2,5,8,11,14,17,20,23,26,29-decamethyl-1,4,7,10,13,16,19,22,25,28,31,34-dodecaoxo-2,5,8,11,14,17,20,26,29,32,35-decaazanonatriacontane-39-carboxylic acid (DL-B-126')

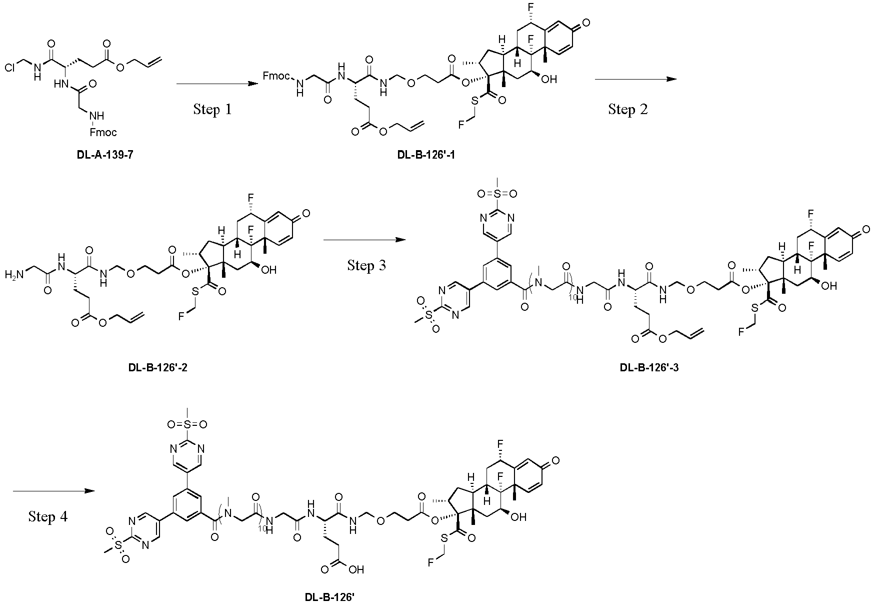

### Step 1: preparation of (6S,8S,9R,108,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (S)-8-(3-(allyloxy)-3-oxopropyl)-1-(9H-fluoren-9-yl)-3,6,9-trioxo-2,12-dioxa-4,7,10-triazapentadecane-15-carboxylate (DL-B-126'-1)

Allyl (S)-4-(2-(((9H-fluoren-9-yl)methoxy)carbonyl)amino)acetamido)-5-((chloromethyl)amino)-5-oxovalerate (168 mg) and (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 3-hydroxypropionate (169mg, 327.16 µmol) were added into anhydrous DCM (6 mL), and then the reaction solution was allowed to react for 2 h at 20°C under stirring after the addition was finished. After the reaction was completed, the reaction solution was spinned to dryness under reduced pressure to obtain the title compound as a crude product (325 mg), which was directly used in the next step without purification.

### Step 2: preparation of allyl (S)-4-(2-aminoacetamido)-5-((3-(((6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl)oxy)-3-oxopropoxy)methyl)amino)-5-oxovalerate (DL-B-126'-2)

The (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (S)-8-(3-(allyloxy)-3-oxopropyl)-1-(9H-fluoren-9-yl)-3,6,9-trioxo-2,12-dioxa-4,7,10-triazapentadecane-15-carboxylate crude product (325 mg) and diethylamine (47.82 mg, 653.87 µmol) were added into DMF (2 mL), and then the reaction solution was allowed to react for 2 h at 22°C under stirring after the addition was finished. After the reaction was completed, the reaction solution was directly purified via reverse phase column chromatography (acetonitrile/water (0.05% formic acid) = 0-50%) and then freeze-dried to obtain the title compound (98 mg, 126 µmol).

The structure characterization data is as follows:
ESI-MS (m/z): 772.4(M+H)⁺.

### Step 3: preparation of (6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (S)-36-(3-(allyloxy)-3-oxopropyl)-1-(3,5-bis(2-(methylsulfonyl)pyrimidin-5-yl)phenyl)-2,5,8,11,14,17,20,23,26,29-decamethyl-1,4,7,10,13,16,19,22,25,28,31,34,34,37-tridecaoxo-40-oxa-2,5,8,11,14,17,20,23,26,29,32,35,38-tridecaazatritetracontane-43-carboxylate (DL-B-126'-3)

Allyl (S)-4-(2-aminoacetamido)-5-((3-(((6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl)oxy)-3-oxopropoxy)methyl)amino)-5-oxovalerate (30 mg, 30.68 µmol), 1-(3,5-bis(2-(methylsulfonyl))pyrimidin-5-yl)phenyl)-2,5,8,11,14,17,20,23,26,29-decamethyl-1,4,7,10,13,16,19,22,25,28-decaoxo-2,5,8,11,14,17,20,23,26,29-decaazahentriacontane-31-carboxylic acid (42 mg, 30.68 µmol), DIPEA (16 mg, 9.76 mmol) and HATU (18.13mg, 47.69 µmol) were added into DMF (1 mL), and then the reaction solution was allowed to react for 2 h at 22°C under stirring after the addition was finished. After the reaction was completed, the solvent was removed from the reaction solution under reduced pressure to obtain the title compound as a crude product (69 mg), which was directly used in the next step without purification.

### Step 4: preparation of (S)-1- (3,5-bis (2-(methylsulfonyl)pyrimidin-5-yl)phenyl)-36-((3-(((6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl)oxy)-3-oxopropoxy)methyl)carbamoyl)-2,5,8,11,14,17,20,23,26,29-decamethyl-1,4,7,10,13,16,19,22,25,28,31,34-dodecaoxo-2,5,8,11,14,17,20,26,29,32,35-decaazanonatriacontane-39-carboxylic acid (DL-B-126')

(6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (S)-36-(3-(allyloxy)-3-oxopropyl)-1-(3,5-bis(2-(methylsulfonyl)pyrimidin-5-yl)phenyl)-2,5,8,11,14,17,20,23,26,29-decamethyl-1,4,7,10,13,16,19,22,25,28,31,34,34,37-tridecaoxo-40-oxa-2,5,8,11,14,17,20,23,26,29,32,35,38-tridecaazatritetracontane-43-carboxylate (69 mg, crude) was added into DCM (1 mL), tetra(triphenylphosphine)palladium (20.99 mg, 18.17 µmol), formic acid (20 µL) and N-methylmorpholine (40 µL) were added under the protection of nitrogen, and then the reaction solution was allowed to react for 2 h at 22°C under stirring after the addition was finished. After the reaction was completed, the reaction solution was purified via high performance liquid chromatography and then freeze-dried to obtain the title compound (16.75 mg, 8.92 µmol).

The purification method is as follows:
Chromatographic column: SunFire Prep C18 OBD 19×150 mm×5 µm
Mobile phase A: acetonitrile; mobile B: water (0.05% TFA)

| Time [min] | Mobile phase A [%] | Mobile phase B [%] | Flow rate [mL/min] |
|---|---|---|---|
| 0.00 | 30 | 70 | 30 |
| 2.00 | 30 | 70 | 30 |
| 18.00 | 90 | 10 | 30 |

The structure characterization data is as follows:
ESI-MS (m/z): 1880.8(M+Na)⁺.

### Preparation example 67: Preparation of (6S,8S,9R,10S,11S,13S,14S,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (36S,39S)-39-(4-aminobutyl)-1-(3,5-bis(2-(methylsulfonyl)pyrimidin-5-yl)phenyl)-36-hydroxymethyl-2,5,8,11,14,20,23,26,29-decamethyl-1,7,10,13,16,19,22,25,28,31,34,37,40-tetradecaoxo-43-oxa-2,5,8,11,14,17,20,23,26,29,32,35,38,41-tetradecaazahexatetracontane-46-carboxylate (DL-B-127')

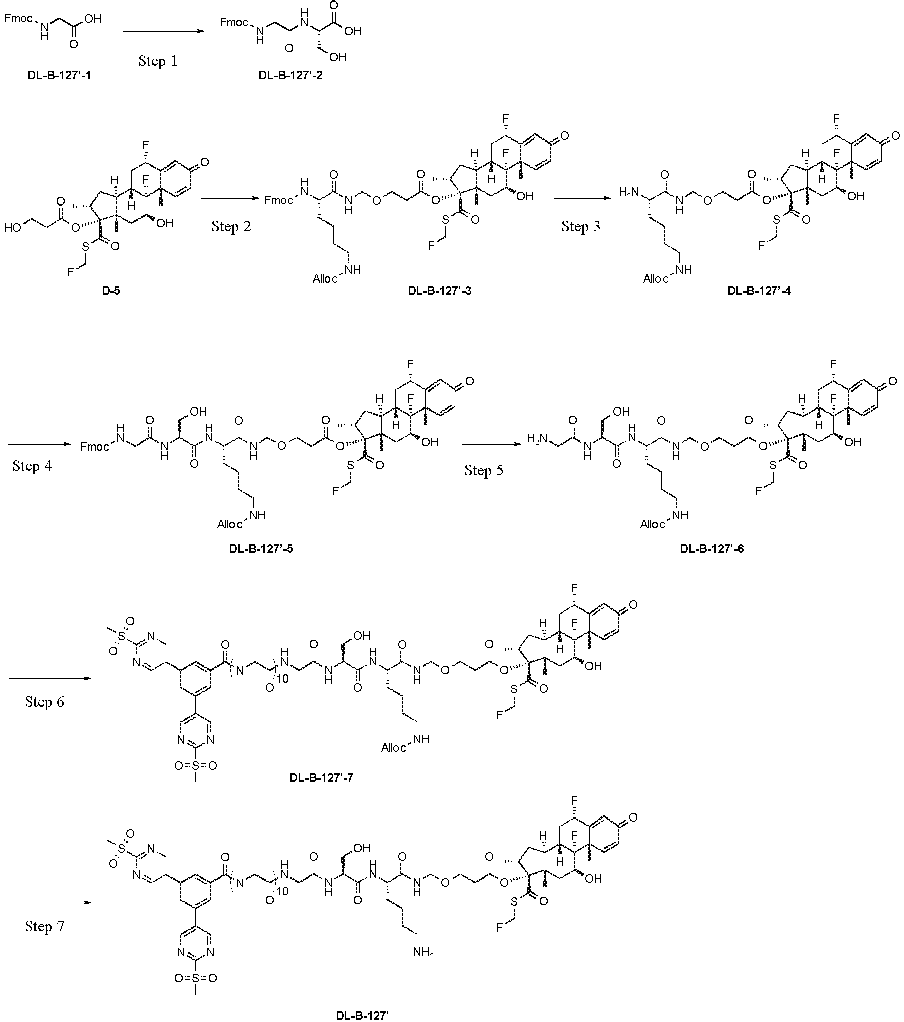

### Step 1: preparation of (((9H-fluoren-9-yl)methoxy)carbonyl)glycyl-L-serine (DL-B-127'-2)

(((9H-fluoren-9-yl)methoxy)carbonyl)glycine (3.00 g, 10.09 mmol), N-hydroxysuccinimide (1.39 g, 12.11 mmol) and DCC (2.50 g, 12.11 mmol) were added into DMF (30 mL), and the reaction solution was allowed to react for 2 h at 25°C. L-serine (1.06 g, 10.09 mmol) and DIPEA (1.30 g, 10.09 mmol) were added, and the reaction solution continued to react for 2 h at 25°C. The reaction solution was adjusted to pH 4-5 using formic acid, followed by filtration to remove insoluble solids. The filtrate was directly purified via C18 reverse phase column chromatography (ACN/H₂O = 10-60%) and then freeze-dried to obtain the title compound (2.10 g, 5.19 mmol).

The structure characterization data is as follows:
ESI-MS (m/z): 385.1 (M+H)⁺.

### Step 2: preparation of (6S,8S,9R,10S,11S,13 S, 14S, 17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (S)-8-(((9H-fluoren-9-yl)methoxy)carbonyl)amino)-7,14-dioxo-4,15-dioxa-6,13-diazaoctadec-17-enoate (DL-B-127'-3)

(6S,8S,9R,10S,11S,13S,14S,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 3-hydroxypropionate (100 mg, 193.58 µmol, FR) and methyl (S)-(2-(((9H-fluoren-9-yl)methoxy)carbonyl)amino)-6-((allyloxy)carbonyl)amino)hexanamido)acetate (152.0 mg, 290.38 µmol) were dissolved in dichloromethane (5 mL), pyridine p-toluenesulfonate (100.0 mg, 193.58 µmol) was added, and the reaction solution was heated to 40°C and allowed to react for 4 h. The reaction solution was then concentrated to obtain a crude product, which was purified via thin layer chromatography (methanol/dichloromethane = 1:20) to obtain the title compound (123.0 mg, 125.50 µmol).

The structure characterization data is as follows:
ESI-MS (m/z): 980.3 (M+H)⁺.

### Step 3: preparation of (6S,8S,9R,10S,11S,13S,14S,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthrene-17-amino-7,14-dioxo-4,15-dioxa-6,13-diazaoctadec-17-enoate (DL-B-127'-4)

(6S,8S,9R,10S,11S,13S,14S,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (S)-8-(((9H-fluoren-9-yl)methoxy)carbonyl)amino)-7,14-dioxo-4,15-dioxa-6,13-diazaoctadec-17-enoate (123.0 mg, 125.50 µmol, FR) was dissolved in DMF (2 mL), diethylamine (45.9 mg, 627.49 µmol) was added, and the reaction solution was allowed to react for 1 h at 25°C. The reaction solution was directly purified via high performance liquid chromatography to obtain the title compound (77.0 mg, 101.60 µmol).

The purification method is as follows:
Chromatographic column: SunFire Prep C18 OBD 19*150 mm*5 µm
Mobile phase A: acetonitrile; mobile phase B: water (0.05% ammonium bicarbonate)

| Time [min] | Mobile phase A [%] | Mobile phase B [%] | Flow rate [mL/min] |
|---|---|---|---|
| 0.00 | 30 | 70 | 32 |
| 4.00 | 30 | 70 | 32 |
| 24.00 | 90 | 10 | 32 |

The structure characterization data is as follows:
ESI-MS (m/z): 758.3 (M+H)⁺.

### Step 4: preparation of (6S,8S,9R,10S,11S,13S,14S,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (8S,11S)-11-(4-(((allyloxy)carbonylamino)butyl)-1-(9H-fluoren-9-yl)-8-hydroxymethyl-3,6,9,12-tetraoxo-2,15-dioxa-4,7,10,13-tetraazaoctadec-18-enoate (DL-B-127'-5)

(((9H-fluoren-9-yl)methoxy)carbonyl)glycyl-L-serine (43.8 mg, 114.01 µmol, FR) was dissolved in DMF (2 mL), HATU (72.2 mg, 190.01 µmol), DIPEA (24.6 mg, 190.01 µmol) and (6S,8S,9R,10S,11S,13S,14S,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthrene-17-amino-7,14-dioxo-4,15-dioxa-6,13-diazaoctadec-17-enoate (72.0 mg, 95.00 µmol, FR) were added, and the reaction solution was allowed to react for 1 h at 25°C. Then the reaction solution was directly purified via high performance liquid chromatography to obtain the title compound (60.0 mg, 50.70 µmol).

The purification method is as follows:
Chromatographic column: SunFire Prep C18 OBD 19*150 mm*5 µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 50 | 50 | 30 |
| 2.00 | 50 | 50 | 30 |
| 18.00 | 95 | 5 | 30 |

The structure characterization data is as follows:
ESI-MS (m/z): 1124.6 (M+H)⁺.

### Step 5: preparation of (6S,8S,9R,10S,11S,13S,14S,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (S)-8-((S)-2-(2-aminoacetamido)-3-hydroxypropanamido)-7,14-dioxo-4,15-dioxa-6,13-diazaoctadec-17-enoate (DL-B-127'-6)

(6S,8S,9R,10S,11S,13S,14S,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (8S,11S)-11-(4-(((allyloxy)carbonylamino)butyl)-1-(9H-fluoren-9-yl)-8-hydroxymethyl-3,6,9,12-tetraoxo-2,15-dioxa-4,7,10,13-tetraazaoctadec-18-enoate (60.0 mg, 53.37 µmol) was dissolved in DMF (1 mL), diethylamine (19.5 mg, 266.85 µmol) was added, and the reaction solution was allowed to react for 1 h at 25°C. Then the reaction solution was directly purified via high performance liquid chromatography to obtain the title compound (38 mg, 40.02 µmol).

The purification method is as follows:
Chromatographic column: SunFire Prep C18 OBD 19*150 mm*5 µm
Mobile phase A: acetonitrile; mobile phase B: water (0.05% ammonium bicarbonate)

| Time [min] | Mobile phase A [%] | Mobile phase B [%] | Flow rate [mL/min] |
|---|---|---|---|
| 0.00 | 30 | 70 | 30 |
| 3.00 | 30 | 70 | 30 |
| 24.00 | 80 | 20 | 30 |

The structure characterization data is as follows:
ESI-MS (m/z): 902.3 (M+H)⁺.

### Step 6: preparation of (6S,8S,9R,10S,11S,13S,14S,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (36S,39S)-39-(4-(((allyloxy)carbonyl)amino)butyl)-1-(3,5-bis(2-(methylsulfonyl)pyrimidin-5-yl)phenyl)-36-hydroxymethyl-2,8,11,14,20,23,26,29-decamethyl-1,4,7,10,13,16,19,22,25,28,31,34,37,40-tetradecaoxo-43-oxa-2,5,8,11,14,17,20,23,26,29,32,35,38,41-tetradecaazahexatetracontane-46-carboxylate (DL-B-127'-7)

(6S,8S,9R,10S,11S,13S,14S,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (S)-8-((S)-2-(2-aminoacetamido)-3-hydroxypropanamido)-7,14-dioxo-4,15-dioxa-6,13-diazaoctadec-17-enoate (15.0 mg, 16.63 µmol), 1-(3,5-bis(2-(methylsulfonyl)pyrimidin-5-yl)phenyl)-2,5,8,11,14,17,20,23,26,29-decamethyl-1,4,7,10,13,16,19,22,25,28-decaoxo-2,5,8,11,14,17,20,23,26,29-decaazahentriacontane-31-carboxylic acid (21.0 mg, 18.29 µmol) was dissolved in DMF (0.5 mL), HATU (12.6 mg, 33.26 µmol) and DIPEA (6.4 mg, 49.89 µmol) were added, and the reaction solution was allowed to react for 1 h at 25°C. Then the reaction solution was directly purified via high performance liquid chromatography to obtain the title compound (26.0 mg, 12.17 µmol).

The purification method is as follows:
Chromatographic column: SunFire Prep C18 OBD 19*150 mm*5 µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 30 | 70 | 32 |
| 4.00 | 30 | 70 | 32 |
| 24.00 | 90 | 10 | 32 |

The structure characterization data is as follows:
ESI-MS (m/z): 1015.0 (M/2+H)⁺.

### Step 7: preparation of (6S,8S,9R,10S,11S,13S,14S,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (36S,39S)-39-(4-aminobutyl)-1-(3,5-bis(2-(methylsulfonyl)pyrimidin-5-yl)phenyl)-36-hydroxymethyl-2,5,8,11,14,20,23,26,29-decamethyl-1,7,10,13,16,19,22,25,28,31,34,37,40-tetradecaoxo-43-oxa-2,5,8,11,14,17,20,23,26,29,32,35,38,41-tetradecaazahexatetracontane-46-carboxylate (DL-B-127')

Under the protection of nitrogen, compound (6S,8S,9R,10S,11S,13S,14S,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (36S,39S)-39-(4-(((allyloxy)carbonyl)amino)butyl)-1-(3,5-bis(2-(methylsulfonyl)pyrimidin-5-yl)phenyl)-36-hydroxymethyl-2,8,11,14,20,23,26,29-decamethyl-1,4,7,10,13,16,19,22,25,28,31,34,37,40-tetradecaoxo-43-oxa-2,5,8,11,14,17,20,23,26,29,32,35,38,41-tetradecaazahexatetracontane-46-carboxylate (26.0 mg, 12.81 µmol) was dissolved in DMF (1 mL), formic acid (9.2 mg, 192.19 µmol), N-methylmorpholine (13.0 mg, 128.13 µmol) and tetra(triphenylphosphine)palladium (7.4 mg, 6.41 µmol) were successively added, and the reaction solution was allowed to react for 2 h at 25°C. Then the reaction solution was directly purified via high performance liquid chromatography to obtain the title compound (6.6 mg, 3.31 µmol).

The purification method is as follows:
Chromatographic column: SunFire Prep C18 OBD 19*150 mm*5 µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 10 | 90 | 30 |
| 2.00 | 10 | 90 | 30 |
| 24.00 | 80 | 20 | 30 |

The structure characterization data is as follows:
ESI-MS (m/z): 1944.8 (M+H)⁺.

### Preparation example 68: Preparation of (6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (33S,36S,39S)-1-(3,5-bis(2-(methylsulfonyl)pyrimidin-5-yl)phenyl)-2,5,8,11,14,17,20,23,26,29,33,36,39-tridecamethyl-1,4,7,10,13,16,19,22,25,28,31,34,37,40-tetradecaoxo-43-oxa-2,5,8,11,14,17,20,23,26,29,32,35,38,41-tetradecaazahexatetracontane-46-carboxylate (DL-B-128')

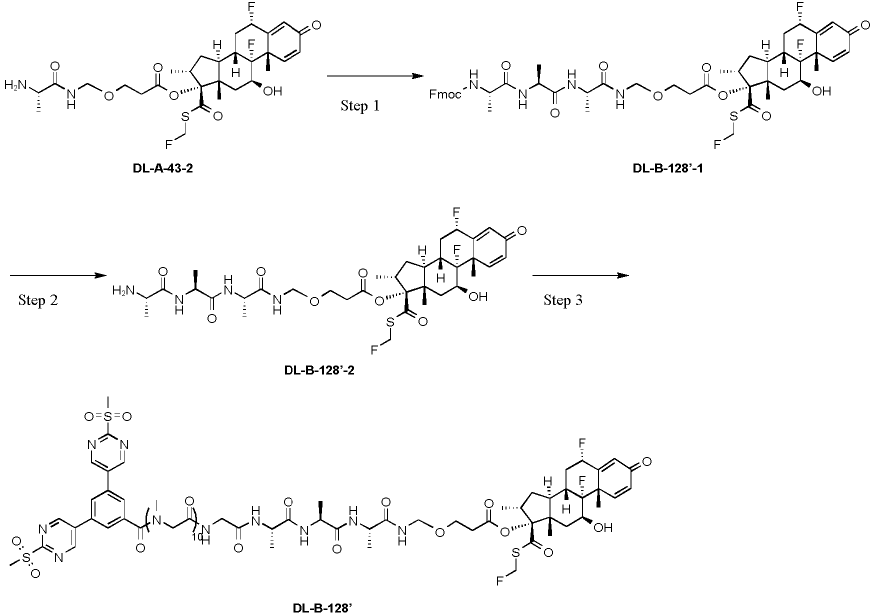

### Step 1: preparation of (6S,8S,9R,10S,118,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (5S,8S,11S)-1-(9H-fluoren-9-yl)-5,8,11-trimethyl-3,6,9,12-tetraoxo-2,15-dioxa-4,7,10,13-tetraazaoctadecane-18-carboxylate (DL-B-128'-1)

(6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 3-((S)-2-aminopropanamide)methoxy)propionate (69 mg, 111.89 µmol), (((9H-fluoren-9-yl)methoxy)carbonyl)-L-alanine-L-alanine (42.79 mg, 111.89 µmol), DIPEA (43.38 mg, 335.66 µmol) and Carter condenser (98.97 mg, 223.78 µmol) were added into DMF (2 mL), and then the reaction solution was allowed to react for 2 h at 22°C under stirring after the addition was finished. After the reaction was completed, the reaction solution was purified via high performance liquid chromatography and then freeze-dried to obtain the title compound (69 mg, 67.52 µmol).

The purification method is as follows:
Chromatographic column: SunFire Prep C18 OBD 19×150 mm×5 µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 45 | 55 | 30 |
| 2.00 | 45 | 55 | 30 |
| 24.00 | 90 | 10 | 30 |

The structure characterization data is as follows:
ESI-MS (m/z): 981.5(M+H)⁺.

### Step 2: preparation of (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (8S,11S,14S)-14-amino-8,11-dimethyl-7,10,13-trioxo-4-oxa-6,9,12-triazadecadecane carboxylate (DL-B-128'-2)

(6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (5S,8S,11S)-1-(9H-fluoren-9-yl)-5,8,11-trimethyl-3,6,9,12-tetraoxo-2,15-dioxa-4,7,10,13-tetraazaoctadecane-18-carboxylate (40mg, 40.77 µmol) and diethylamine (8.95mg, 122.31 µmol) were added into DMF (2 mL), and then the reaction solution was allowed to react for 2 h at room temperature under stirring after the addition was finished. After the reaction was completed, the reaction solution was spinned to dryness under reduced pressure to remove the residual diethylamine to obtain the title compound as a crude product (30 mg), which was directly used in the next step without purification.

The structure characterization data is as follows:
ESI-MS (m/z): 759.4(M+H)⁺.

### Step 3: preparation of (6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (33S,36S,39S)-1-(3,5-bis(2-(methylsulfonyl)pyrimidin-5-yl)phenyl)-2,5,8,11,14,17,20,23,26,29,33,36,39-tridecamethyl-1,4,7,10,13,16,19,22,25,28,31,34,37,40-tetradecaoxo-43-oxa-2,5,8,11,14,17,20,23,26,29,32,35,38,41-tetradecaazahexatetracontane-46-carboxylate (DL-B-128')

The (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (8S,11S,14S)-14-amino-8,11-dimethyl-7,10,13-trioxo-4-oxa-6,9,12-triazadecadecane carboxylate crude product (30 mg), 1-(3,5-bis(2-(methylsulfonyl)pyrimidin-5-yl)phenyl)-2,5,8,11,14,17,20,23,26,29-decamethyl-1,4,7,10,13,16,19,22,25,28-decaoxo-2,5,8,11,14,17,20,23,26,29-decaazahentriacontane-31-carboxylic acid (45.28 mg, 35.93 µmol), DIPEA (15.33 mg, 118.60 µmol) and HATU (18.04 mg, 47.44 µmol) were added into DMF (2 mL), and then the reaction solution was allowed to react for 2 h at 22°C under stirring. After the reaction was completed, the reaction solution was purified via high performance liquid chromatography and then freeze-dried to obtain the title compound (39.49 mg, 20.73 µmol).

The purification method is as follows:
Chromatographic column: SunFire Prep C18 OBD 19×150 mm×5 µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0 | 10 | 90 | 20 |
| 2 | 10 | 90 | 30 |
| 24 | 90 | 10 | 30 |

The structure characterization data is as follows:
ESI-MS (m/z):1854.5 (M+18)⁺.

### Preparation example 69: Preparation of (6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((33S,36S)-1-(3,5-bis(2-(methylsulfonyl)pyrimidin-5-yl)phenyl)-2,5,8,11,14,20,23,26,29,33,36,36-dodecamethyl-1,4,7,10,13,16,19,22,25,28,31,34,34-dodecaoxo-2,5,8,11,14,17,20,23,26,29,32,35-dodecaazaheptatriacontan-37-amino)-4-fluorobenzoate (DL-B-130')

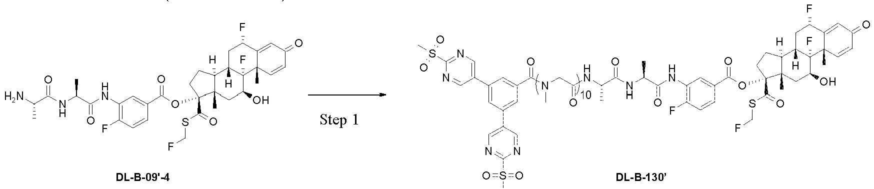

(6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-2-((S)-2-aminopropylamino)propylamino)-4-fluorobenzoate (10 mg, 14.09 µmol), DIPEA (9.10 mg, 70.45 µmol) and 1-(3,5-bis(2-(methylsulfonyl)pyrimidin-5-yl)phenyl)-2,5,8,11,14,17,23,26,29-decamethyl-1,4,7,10,13,16,19,22,25,28-decaoxo-2,5,8,11,14,17,20,23,26,29-decaazahentriacontane-31-carboxylic acid (24.20 mg, 21.13 µmol) and HATU (10.71 mg, 28.18 µmol) were added into DMF (3.00 mL), and then the reaction solution was allowed to react for 3 h at 25°C. The reaction solutuon was filtered to obtain a crude product, which was purified via high performance liquid chromatography and then freeze-dried to obtain the title compound (5 mg, 2.67µmol).

The purification method is as follows:
Chromatographic column: Phenomenex C18 250mm×50mm×10µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 30 | 70 | 30 |
| 2.00 | 30 | 70 | 30 |
| 18.00 | 90 | 10 | 30 |

The structure characterization data is as follows:
ESI-MS (m/z):1854.5 (M+18)⁺.

### Preparation example 70: preparation of (S)-1-(3,5-bis(2-(methylsulfonyl)pyrimidin-5-yl)phenyl)-36-(5-((((6S,8S,9R,10S,11S,13S,14S,17R)-6,9-difluoro-17-((fluoromethyl)thio)carbonyl)-11-hydroxy-10,13-dimethyl-3-oxo-6,7,8,9,10,12,13,14,15,16,17-dodecahydro-3H-cyclopenta[a]phenanthren-17-yl)oxy)carbonyl)-2-fluorophenyl)carbamoyl)-2,5,8,11,14,17,23,26,29-decamethyl-1,4,7,10,16,19,22,25,28,31,34-dodecaoxo-2,5,8,14,17,20,23,26,29,32,35-dodecaazanonatriacontane-39-carboxylic acid (DL-B-131')

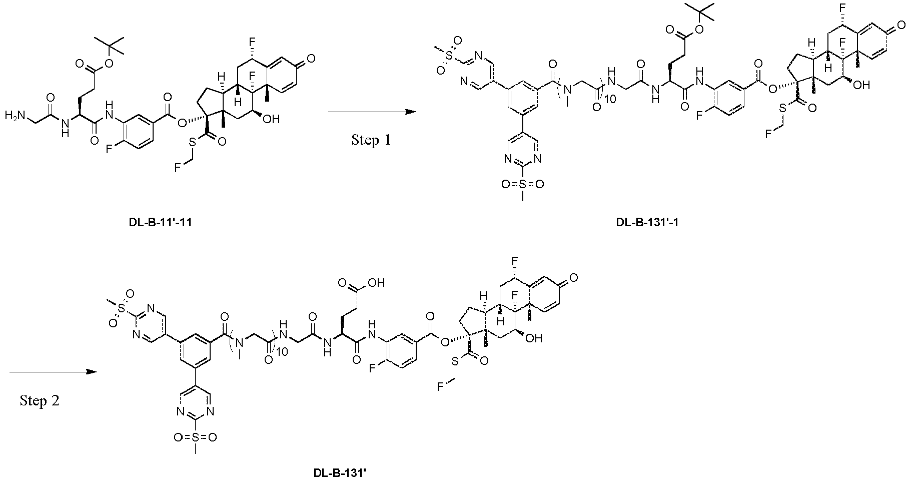

### Step 1: preparation of tert-butyl (S)-1-(3,5-bis(2-(methylsulfonyl)pyrimidin-5-yl)phenyl)-36-(5-((((6S,8S,9R,10S,11S,13S,14S,17R)-6,9-difluoro-17-((fluoromethyl)thio)carbonyl)-11-hydroxy-10,13-dimethyl-3-oxo-6,7,8,9,10,12,13,14,15,16,17-dodecahydro-3H-cyclopenta[a]phenanthren-17-yl)oxy)carbonyl)-2-fluorophenyl)carbamoyl)-2,5,8,11,14,17,23,26,29-decamethyl-1,4,7,10,16,19,22,25,28,31,34-dodecaoxo-2,5,8,14,17,20,23,26,29,32,35-dodecaazanonatriacontane-39-carboxylate (DL-B-131'-1)

1-(3,5-bis(2-(methylsulfonyl)pyrimidin-5-yl)phenyl)-2,5,8,11,14,17,23,26,29-decamethyl-1,4,7,10,13,16,19,22,25,28-decaoxo-2,5,8,11,14,17,20,23,26,29-decaazahentriacontane-31-carboxylic acid (21.21 mg, 18.52 µmol), DIPEA (7.18 mg, 55.57 µmol), HATU (8.45 mg, 22.23 µmol) and (6S,8S,9R,10S,118,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-2-(2-aminoacetamido)-5-(tert-butoxy)-5-oxopentamido)-4-fluorobenzoate (15 mg, 18.52 µmol) were added into DMF (3 mL), and then the reaction solution was allowed to react for 3 h at 23°C under stirring after the addition was finished. After the reaction was completed, a solvent was removed from the reaction solution under reduced pressure to obtain the title compound as a crude product (30 mg), which was directly used in the next step without purification.

The structure characterization data is as follows:
ESI-MS (m/z): 1954.3(M+H₂O)⁺.

### Step 2: preparation of (S)-1-(3,5-bis(2-(methylsulfonyl)pyrimidin-5-yl)phenyl)-36-(5-((((6S,8S,9R,10S,11S,13S,14S,17R)-6,9-difluoro-17-((fluoromethyl)thio)carbonyl)-11-hydroxy-10,13-dimethyl-3-oxo-6,7,8,9,10,12,13,14,15,16,17-dodecahydro-3H-cyclopenta[a]phenanthren-17-yl)oxy)carbonyl)-2-fluorophenyl)carbamoyl)-2,5,8,11,14,17,23,26,29-decamethyl-1,4,7,10,16,19,22,25,28,31,34-dodecaoxo-2,5,8,14,17,20,23,26,29,32,35-dodecaazanonatriacontane-39-carboxylic acid (DL-B-131')

Tert-butyl (S)-1-(3,5-bis(2-(methylsulfonyl)pyrimidin-5-yl)phenyl)-36-(5-((((6S,8S,9R,10S,11S,13S,14S,17R)-6,9-difluoro-17-((fluoromethyl)thio)carbonyl)-11-hydroxy-10,13-dimethyl-3-oxo-6,7,8,9,10,12,13,14,15,16,17-dodecahydro-3H-cyclopenta[a]phenanthren-17-yl)oxy)carbonyl)-2-fluorophenyl)carbamoyl)-2,5,8,11,14,17,23,26,29-decamethyl-1,4,7,10,16,19,22,25,28,31,34-dodecaoxo-2,5,8,14,17,20,23,26,29,32,35-dodecaazanonatriacontane-39-carboxylate (30 mg, crude) and trifluoroacetic acid (1 mL) were added into DCM (2 mL), and then the reaction solution was stirred for 2 h at 23°C under stirring after the addition was finished. The reaction solution was directly purified via high performance liquid chromatography and then freeze-dried to obtain the title compound (5.97 mg, 3.08 µmol).

The purification method is as follows:
Chromatographic column: SunFire Prep C18 OBD 19×150 mm×5 µm
Mobile phase A: acetonitrile; mobile B: water (0.05% TFA)

| Time [min] | Mobile phase A [%] | Mobile phase B [%] | Flow rate [mL/min] |
|---|---|---|---|
| 0.00 | 40 | 60 | 30 |
| 2.00 | 40 | 60 | 30 |
| 18.00 | 90 | 10 | 30 |

The structure characterization data is as follows:
ESI-MS (m/z): 1898.4(M+H₂O)⁺.

### Preparation example 71: Preparation of (6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((36S,39S)-39-(4-aminobutyl)-1-(3,5-bis(2-(methylsulfonyl)pyrimidin-5-yl)phenyl)-36-hydroxymethyl-2,5,8,11,14,17,20,23,26,29-decamethyl-1,4,7,10,13,16,19,22,25,28,31,34,37-tridecaoxo-2,5,8,11,14,17,20,23,26,29,32,35,38-tridecaazatetracontane-40-amino)-4-fluorobenzoate (DL-B-132')

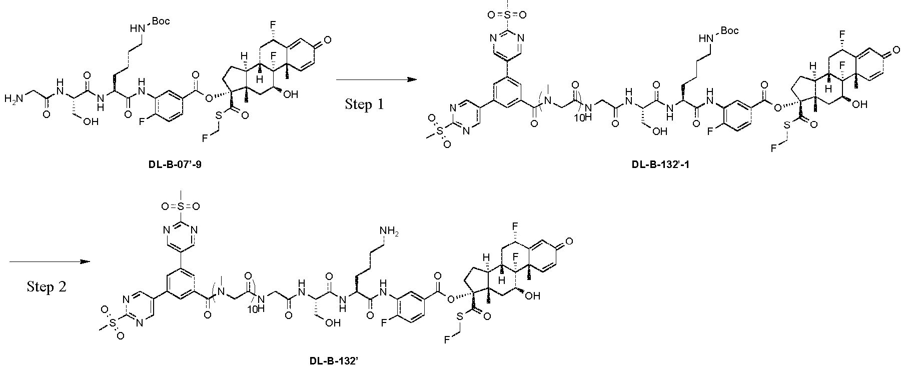

### Step 1: preparation of (6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]henanthren-17-yl 3-((36S,39S)-1-(3,5-bis(2-(methylsulfonyl)pyrimidin-5-yl)phenyl)-39-(4-((tert-butoxycarbonyl)amino)butyl)-36-hydroxymethyl-2,5,8,11,14,17,20,23,26,29-decamethyl-1,4,7,10,13,16,19,22,25,28,31,34,37-tridecaoxo-2,5,8,11,14,17,20,23,26,29,32,35,38-tridecaazatetracontane-40-amino)-4-fluorobenzoate (DL-B-132'-1)

1-(3,5-bis(2-(methylsulfonyl)pyrimidin-5-yl)phenyl)-2,5,8,11,14,17,20,23,26,29-decamethyl-1,4,7,10,13,16,19,22,25,28-decaoxo-2,5,8,11,14,17,20,23,26,29-decaazahentriacontane-31-carboxylic acid (24.4 mg, 21.30 µmol) was dissolved in DMF (1 mL), HATU (15.42 mg, 40.57 µmol), DIPEA (7.86 mg, 60.85 µmol) and compound DL-B-07'-9 (20.0 mg, 20.28 µmol) were successively added, and the reaction solution was allowed to react for 1 h at 25°C. Then the reaction solution was directly purified via high performance liquid chromatography and then freeze-dried to obtain the title compound (6.0 mg, 2.82 µmol).

The purification method is as follows:
Chromatographic column: SunFire Prep C18 OBD 19*150 mm*5 µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 30 | 70 | 30 |
| 2.00 | 30 | 70 | 30 |
| 18.00 | 90 | 10 | 30 |

The structure characterization data is as follows:
ESI-MS (m/z): 1034.1 (M/2+H)⁺.

### Step 2: preparation of (6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((36S,39S)-39-(4-aminobutyl)-1-(3,5-bis(2-(methylsulfonyl)pyrimidin-5-yl)phenyl)-36-hydroxymethyl-2,5,8,11,14,17,20,23,26,29-decamethyl-1,4,7,10,13,16,19,22,25,28,31,34,37-tridecaoxo-2,5,8,11,14,17,20,23,26,29,32,35,38-tridecaazatetracontane-40-amino)-4-fluorobenzoate (DL-B-132')

Compound (6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]henanthren-17-yl 3-((36S,39S)-1-(3,5-bis(2-(methylsulfonyl)pyrimidin-5-yl)phenyl)-39-(4-((tert-butoxycarbonyl)amino)butyl)-36-hydroxymethyl-2,5,8,11,14,17,20,23,26,29-decamethyl-1,4,7,10,13,16,19,22,25,28,31,34,37-tridecaoxo-2,5,8,11,14,17,20,23,26,29,32,35,38-tridecaazatetracontane-40-amino)-4-fluorobenzoate (6.0 mg, 2.90 µmol) was dissolved in DCM (0.5 mL), trifluoroacetic acid (16.55 mg, 145.12 µmol) was added, and the reaction solution was allowed to react for 1 h at 25°C. Then the reaction solution was directly purified via high performance liquid chromatography and then freeze-dried to obtain the title compound (3.44 mg, 1.60 µmol).

The purification method is as follows:
Chromatographic column: SunFire Prep C18 OBD 19*150 mm*5 µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 10 | 90 | 30 |
| 2.00 | 10 | 90 | 30 |
| 20.00 | 70 | 30 | 30 |

The structure characterization data is as follows:
ESI-MS (m/z): 1966.7 (M+H)⁺.

### Preparation example 72: Preparation of (6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((33S,36S,39S)-1-(3,5-bis(2-(methylsulfonyl)pyrimidin-5-yl)phenyl)-2,5,8,11,14,20,23,26,29,33,36,39-tridecamethyl-1,4,7,10,13,16,19,22,25,28,31,34,37-tridecaoxo-2,5,8,11,14,17,20,23,26,29,32,35,38-tridecaazatetracontane-40-amino)-4-fluorobenzoate (DL-B-133')

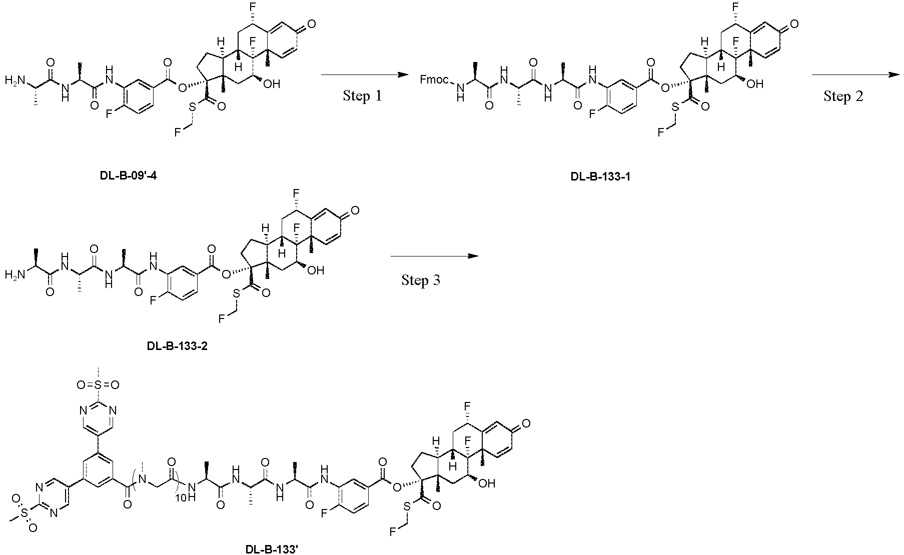

### Step 1: preparation of (6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((5S,8S,11S)-1-(9H-fluoren-9-yl)-5,8,11-trimethyl-3,6,9-trioxo-2-oxa-4,7,10-triazadodecan-12-amino)-4-fluorobenzoate (DL-B-133-1)

(6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-2-((S)-2-aminopropylamino)propylamino)-4-fluorobenzoate (30 mg, 36,42 µmol), (2S)-2-(9H-fluoren-9-ylmethoxycarbonylamino)propionic acid (17.01 mg, 54.63 µmol) and DIPEA (18.83 mg, 145.67 µmol) were successively added into DMF (2 mL), then DMTMM (16.10 mg, 54.63 mg) was added, and the reaction solution was allowed to react for 2 h at 25°C under stirring. The reaction solution was purified via reverse phase column chromatography (acetonitrile/0.5% formic acid aqueous solution = 20-25%) and then freeze-dried to obtain the title compound (15 mg, 14.95 µmol).

### Step 2: preparation of (6S,8S,9R,10S,11S,13 S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-2-((S)-2-aminopropylamino)propylamino)propylamino)-4-fluorobenzoate (DL-B-133-2)

(6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((5S,8S,11S)-1-(9H-fluoren-9-yl)-5,8,11-trimethyl-3,6,9-trioxo-2-oxa-4,7,10-triazadodecan-12-amino)-4-fluorobenzoate (15 mg, 14.95 µmol) was dissolved in N,N-dimethylformamide (2 mL), and diethylamine (1.09 mg, 14.95 µmol) was added, and then the reaction solution was allowed to react for 1 h at 25°C under stirring. The reaction solution was directly placed on a freeze-dryer to remove the solvent and residual diethylamine to obtain the title compound as a crude product (12 mg), which was directly used in the next step without purification.

### Step 3: preparation of (6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((33S,36S,39S)-1-(3,5-bis(2-(methylsulfonyl)pyrimidin-5-yl)phenyl)-2,5,8,11,14,20,23,26,29,33,36,39-tridecamethyl-1,4,7,10,13,16,19,22,25,28,31,34,37-tridecaoxo-2,5,8,11,14,17,20,23,26,29,32,35,38-tridecaazatetracontane-40-amino)-4-fluorobenzoate (DL-B-133)

(6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-2-((S)-2-aminopropylamino)propylamino)propylamino)-4-fluorobenzoate (12 mg, 15.37 µmol), 1-(3,5-bis(2-(methylsulfonyl)pyrimidin-5-yl)phenyl)-2,5,8,11,14,17,20,23,26,29-decamethyl-1,4,7,10,13,16,19,22,25,28-decaoxo-2,5,8,11,14,17,20,23,26,29-decaazahentriacontane-31-carboxylic acid (26.40 mg, 23.05 µmol), DIPEA (7.94 mg, 61.47 µmol) and DMTMM (6.79 mg, 23.05 µmol) were successively added into DMF (2 mL), and then the reaction solution was allowed to react for 1.0 h at 20°C under stirring after the addition was finished. The reaction solution was directly purified via high performance liquid chromatography and then freeze-dried to obtain the title compound (13.52 mg, 6.94 µmol).

The purification method is as follows:
Chromatographic column: SunFire Prep C18 OBD 19*150 mm*5 µm
Mobile phase A: acetonitrile; mobile phase B: water (0.05% FA)

| Time [min] | Mobile phase A [%] | Mobile phase B [%] | Flow rate [mL/min] |
|---|---|---|---|
| 0.00 | 40 | 60 | 30 |
| 2.00 | 40 | 60 | 30 |
| 18.00 | 95 | 5 | 30 |

The structure characterization data is as follows:
ESI-MS (m/z): 1908.08 (M+H)⁺.

### Preparation example 73: Preparation of (6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-6-amino-2-((S)-2-(2-(3,5-bis(2-(methylsulfonyl)pyrimidin-5-yl)benzamido)acetamido)-3-hydroxypropanamido)hexanamido)-4-fluorobenzoate (DL-B-01)

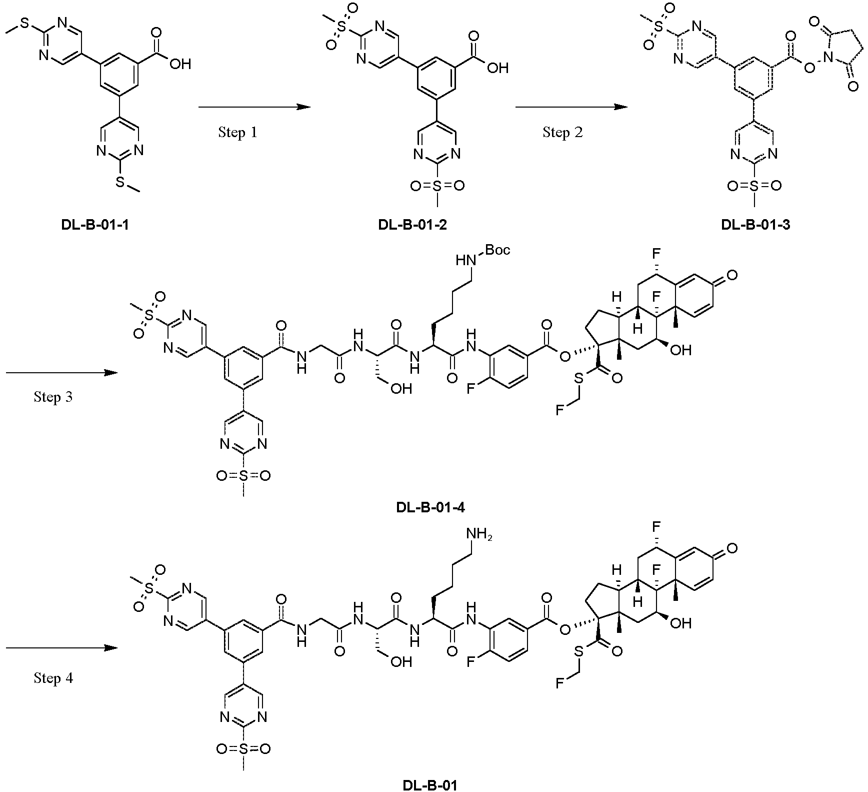

### Step 1: preparation of 3,5-bis(2-(methylsulfonyl)pyrimidin-5-yl)benzoic acid (DL-B-01-2)

3,5-bis(2-(methylthio)pyrimidin-5-yl)benzoic acid (200 mg, 539.89 µmol) was dissolved in a mixed solvent of water (5 mL) and acetonitrile (10 mL), ruthenium trichloride monohydrate (11.20 mg, 53.99 µmol) and sodium periodate (923.82 mg, 4.32 mmol) were successively added, and then the reaction solution was stirred for 2 h at 25°C. Water (30 ml) was added, and then the reaction solution was stirred for 0.5 h, filtered and washed with water (10 ml). The filter cake was collected and dried to obtain the title compound (161 mg, 352.06 µmol).

The structure characterization data is as follows:
ESI-MS (m/z): 435.0 (M+H)⁺.

### Step 2: preparation of 2,5-dioxopyrrolidin-1-yl 3,5-bis(2-(methylsulfonyl)pyrimidin-5-yl)benzoate (DL-B-01-3)

3,5-bis(2-(methylsulfonyl)pyrimidin-5-yl)benzoic acid (85 mg, 195.65 µmol), 1-hydroxypyrrolidine-2,5-dione (27.02 mg, 234.78 µmol) and EDCI (112.52 mg, 586.95 µmol) were successively added into DMF (5 mL), and the reaction solution was allowed to react for 2 h at 25°C under stirring. The reaction solution was quenched using water (5 mL), and extracted with ethyl acetate (15 mL × 3). The organic phase was concentrated to obtain the title compound as a crude product (90 mg), which was directly used in the next step without purification.

### Step 3: preparation of (6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-2-((S)-2-(2-(3,5-bis(2-(methylsulfonyl)pyrimidin-5-yl)benzamido)acetamido-3-hydroxypropanamido)-6-(tert-butoxycarbonyl)amino)hexanamido)-4-fluorobenzoate (DL-B-01-4)

(6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-2-((S)-2-(2-aminoacetamide)-3-hydroxypropionylamino)-6-(tert-butoxycarbonyl)amino)hexanylamino)-4-fluorobenzoate (25 mg, 26.60 µmol), 2,5-dioxopyrrolidin-1-yl 3,5-bis(2-(methylsulfonyl)pyrimidin-5-yl)benzoate (21.20 mg, 39.89 µmol) and DIPEA (6.87 mg, 53.19 µmol) were successively added into DMF (2 mL), and then the reaction solution was allowed to react for 1 h at 20°C under stirring after the addition was finished. The reaction solution was directly purified via high performance liquid chromatography and then freeze-dried to obtain the title compound (18.00 mg, 13.27 µmol).

The purification method is as follows:
Chromatographic column: Agilent Prep C18 OBD 19*150 mm*5 µm
Mobile phase A: acetonitrile; mobile phase B: water (0.05% FA)

| Time [min] | Mobile phase A [%] | Mobile phase B [%] | Flow rate [mL/min] |
|---|---|---|---|
| 0.00 | 40 | 60 | 30 |
| 2.00 | 40 | 60 | 30 |
| 18.00 | 90 | 10 | 30 |

The structure characterization data is as follows:
ESI-MS (m/z): 1256.4 (M -100+H)⁺.

### Step 4: preparation of (6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-6-amino-2-((S)-2-(2-(3,5-bis(2-(methylsulfonyl)pyrimidin-5-yl)benzamido)acetamido)-3-hydroxypropanamido)hexanamido)-4-fluorobenzoate (DL-B-01)

(6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-2-((S)-2-(2-(3,5-bis(2-(methylsulfonyl)pyrimidin-5-yl)benzamido)acetamido-3-hydroxypropanamido)-6-(tert-butoxycarbonyl)amino)hexanamido)-4-fluorobenzoate (30 mg, 22.12 µmol) and trifluoroacetic acid (75.65 mg, 663.50 µmol) were successively added into dichloromethane (2 mL), and then the reaction solution was allowed to react for 1 h at 20°C under stirring after the addition was finished. The reaction solution was directly purified via high performance liquid chromatography and then freeze-dried to obtain the title compound (17.40 mg, 12.96 µmol).

The purification method is as follows:
Chromatographic column: Agilent Prep C18 OBD 19*150 mm*5 µm
Mobile phase A: acetonitrile; mobile phase B: water (0.05% FA)

| Time [min] | Mobile phase A [%] | Mobile phase B [%] | Flow rate [mL/min] |
|---|---|---|---|
| 0.00 | 20 | 80 | 32 |
| 4.00 | 20 | 80 | 32 |
| 24.00 | 90 | 10 | 32 |

The structure characterization data is as follows:
ESI-MS (m/z): 1256.4 (M+H)⁺.

### Preparation example 74: Preparation of (S)-4-(2-(3,5-bis(2-(methylsulfonyl)pyrimidin-5-yl)benzamido)acetamido)-5-(5-(((6S,8S,9R,10S,11S,13S,14S,17R)-6,9-difluoro-17-((fluoromethyl)thio)carbonyl)-11-hydroxy-10,13-dimethyl-3-oxo-6,7,8,10,11,12,13,14,15,16,17-dodecahydro-3H-cyclopenta[a]phenanthren-17-yl)oxy)carbonyl)-2-fluorophenyl)amino)-5-oxovaleric acid (DL-B-05)

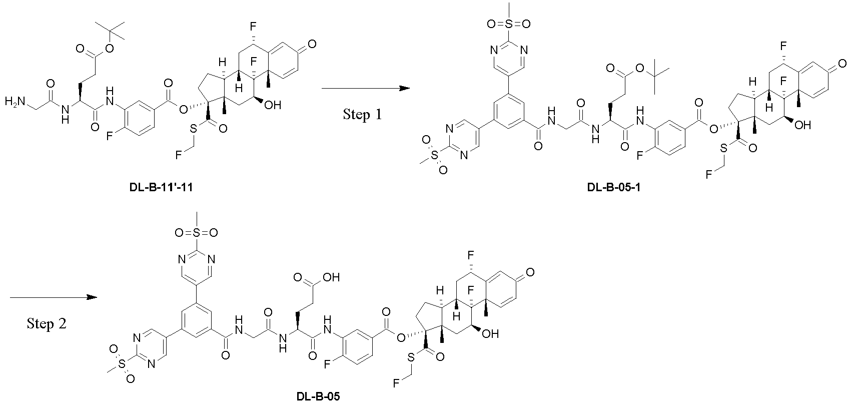

### Step 1: preparation of (6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-2-(2-(3,5-bis(2-(methylsulfonyl)pyrimidin-5-yl)benzamido)acetamido)-5-(tert-butoxy)-5-oxopentanamido)-4-fluorobenzoate (DL-B-05-1)

(6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-2-(2-aminoacetamide)-5-(tert-butoxy)-5-oxopentanamide)-4-fluorobenzoate (30 mg, 38.72 µmol) was dissolved in DMF (2 mL), 3,5-bis(2-(methylsulfonyl)pyrimidin-5-yl)benzoic acid (20.02 mg, 46.44 µmol), DIPEA (24.26 mg, 193.56 µmol) and HATU (30.07 mg, 77.27 µmol) were added, and then the reaction solution was allowed to react for 4 h at 25°C under stirring after the addition was finished. The reaction solution was directly purified via high performance liquid chromatography and then freeze-dried to obtain the title compound (35.00 mg,19.42 µmol).

The purification method is as follows:
Chromatographic column: SunFire Prep C18 OBD 19*150 mm*5 µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 10 | 90 | 30 |
| 2.00 | 10 | 90 | 30 |
| 18.00 | 90 | 10 | 30 |

The structure characterization data is as follows:
ESI-MS (m/z): 1227.29 (M+H)⁺.

### Step 2: preparation of (S)-4-(2-(3,5-bis(2-(methylsulfonyl)pyrimidin-5-yl)benzamido)acetamido)-5-(5-(((6S,8S,9R,10S,11S,13S,14S,17R)-6,9-difluoro-17-((fluoromethyl)thio)carbonyl)-11-hydroxy-10,13-dimethyl-3-oxo-6,7,8,10,11,12,13,14,15,16,17-dodecahydro-3H-cyclopenta[a]phenanthren-17-yl)oxy)carbonyl)-2-fluorophenyl)amino)-5-oxovaleric acid (DL-B-05)

(6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-2-(2-(3,5-bis(2-(methylsulfonyl)pyrimidin-5-yl)benzamido)acetamido)-5-(tert-butoxy)-5-oxopentanamido)-4-fluorobenzoate (35.00 mg, 19.42 µmol) was dissolved in dichloromethane (2 mL), trifluoroacetic acid (2 mL) was added, and then the reaction solution was allowed to react for 6 h at 25°C under stirring after the addition was finished. The reaction solution was directly purified via high performance liquid chromatography and then freeze-dried to obtain the title compound (17.20 mg,19.42 µmol). The purification method is as follows:
Chromatographic column: SunFire Prep C18 OBD 19*150 mm*5 µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 10 | 90 | 30 |
| 2.00 | 10 | 90 | 30 |
| 18.00 | 90 | 10 | 30 |

The structure characterization data is as follows:
ESI-MS (m/z): 1171.19 (M+H)⁺.

### Preparation example 75: Preparation of (S)-1-(3,5-bis(2-(methylsulfonyl)pyrimidin-5-yl)phenyl)-34-((2-(((6S,8S,9R,10S,11S,13S,14S,16R,17R)-6,9-difluoro-17-(furan-2-carbonyl)oxy)-11-hydroxy-10,13,16-trimethyl-3-oxo-6,7,8,9,10,11,12,13,14,15,16,17-dodecahydro-3H-cyclopenta[a]phenanthrene-17-carbonyl)thio)ethoxy)methyl)carbamoyl)-1,29,32-trioxo-5,8,11,14,17,20,23,26-octaoxa-2,30,33-triazaheptatriacontane-37-carboxylic acid (DL-B-91')

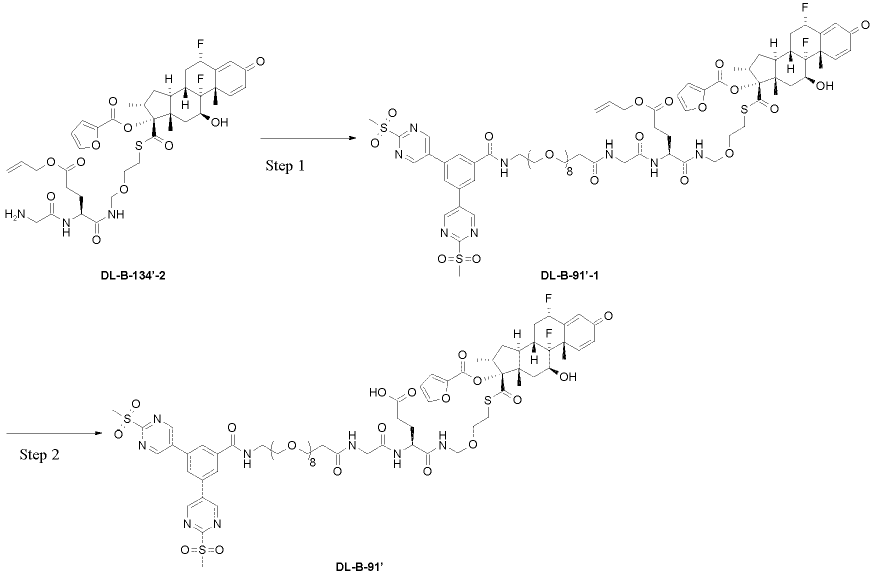

### Step 1: preparation of allyl (S)-1-(3,5-bis(2-(methylsulfonyl)pyrimidin-5-yl)phenyl)-34-((2-(((6S,8S,9R,10S,11S,13S,14S,16R,17R)-6,9-difluoro-17-(furan-2-carbonyl)oxy)-11-hydroxy-10,13,16-trimethyl-3-oxo-6,7,8,9,10,11,12,13,14,15,16,17-dodecahydro-3H-cyclopenta[a]phenanthrene-17-carbonyl)thio)ethoxy)methyl)carbamoyl)-1,29,32-trioxo-5,8,11,14,17,20,23,26-octaoxa-2,30,33-triazaheptatriacontane-37-carboxylate (DL-B-91'-1)

(6S,8S,9R,10S,11S,13S,14S,16R,17R)-17-((S)-9-(2-aminoacetamido)-8,12-dioxo-5,13-dioxa-2-thio-7-azahexadec-15-enoyl)-6,9-difluoro-11-hydroxy-10,13,16-trimethyl-3-oxo-6,7,8,9,10,11,12,13,14,15,16,17-dodecahydro-3H-cyclopenta[a]phenanthren-17-yl furan-2-carboxylate (39 mg, 48.39 µmol), 1-(3,5-bis(2-(methylsulfonyl)pyrimidin-5-yl)phenyl)-1-oxo-5,8,11,14,17,20,23,26-octaoxa-2-azanonacosane-29-carboxylic acid (41.52 mg, 48.39 µmol), DIPEA (18.76 mg, 145.18 µmol) and HATU (22.07 mg, 58.07 µmol) were added into DMF (2 mL), and then the reaction solution was allowed to react for 3 h at 23°C under stirring after the addition was finished. The reaction solution was purified via high performance liquid chromatography and then freeze-dried to obtain the title compound (54 mg, 32.48 µmol).

The purification method is as follows:
Chromatographic column: SunFire Prep C18 OBD 19*150 mm*5 µm
Mobile phase A: acetonitrile; mobile phase B: water (0.05% FA)

| Time [min] | Mobile phase A [%] | Mobile phase B [%] | Flow rate [mL/min] |
|---|---|---|---|
| 0.00 | 30 | 70 | 30 |
| 4.00 | 30 | 70 | 30 |
| 24.00 | 90 | 10 | 30 |

The structure characterization data is as follows:
ESI-MS (m/z): 1645.58 (M+H)⁺.

### Step 2: preparation of (S)-1-(3,5-bis(2-(methylsulfonyl)pyrimidin-5-yl)phenyl)-34-((2-(((6S,8S,9R,10S,11S,13S,14S,16R,17R)-6,9-difluoro-17-(furan-2-carbonyl)oxy)-11-hydroxy-10,13,16-trimethyl-3-oxo-6,7,8,9,10,11,12,13,14,15,16,17-dodecahydro-3H-cyclopenta[a]phenanthrene-17-carbonyl)thio)ethoxy)methyl)carbamoyl)-1,29,32-trioxo-5,8,11,14,17,20,23,26-octaoxa-2,30,33-triazaheptatriacontane-37-carboxylic acid(DL-B-91')

Allyl (S)-1-(3,5-bis(2-(methylsulfonyl)pyrimidin-5-yl)phenyl)-34-((2-(((6S,8S,9R,10S,11S,13S,14S,16R,17R)-6,9-difluoro-17-(furan-2-carbonyl)oxy)-11-hydroxy-10,13,16-trimethyl-3-oxo-6,7,8,9,10,11,12,13,14,15,16,17-dodecahydro-3H-cyclopenta[a]phenanthrene-17-carbonyl)thio)ethoxy)methyl)carbamoyl)-1,29,32-trioxo-5,8,11,14,17,20,23,26-octaoxa-2,30,33-triazaheptatriacontane-37-carboxylate (40.00 mg, 24.30 µmol) and tetra(triphenylphosphine)palladium (14.04 mg, 12.15 µmol) were added into DMF (1 mL), and the system was introduced with nitrogen for replacement. Then formic acid (20 µL) and N-methylmorpholine (40 µL) were added, and the reaction solution was allowed to react for 2 h at 22°C under stirring after the addition was finished. The reaction solution was purified via high performance liquid chromatography and then freeze-dried to obtain the title compound (30.81 mg, 19.00 µmol).

The purification method is as follows:
Chromatographic column: SunFire Prep C18 OBD 19*150 mm*5 µm
Mobile phase A: acetonitrile; mobile phase B: water (0.05% FA)

| Time [min] | Mobile phase A [%] | Mobile phase B [%] | Flow rate [mL/min] |
|---|---|---|---|
| 0.00 | 30 | 70 | 30 |
| 4.00 | 30 | 70 | 30 |
| 32.00 | 90 | 10 | 30 |

The structure characterization data is as follows:
ESI-MS (m/z): 1605.53 (M+H)⁺.

### Preparation example 76: Preparation of (S)-1-(3,5-bis(2-(methylsulfonyl)pyrimidin-5-yl)phenyl)-36-((2-(((6S,8S,9R,10S,11S,13S,14S,16R,17R)-6,9-difluoro-17-(furan-2-carbonyl)oxy)-11-hydroxy-10,13,16-trimethyl-3-oxo)-6,7,8,9,10,11,12,13,14,15,16,17-dodecahydro-3H-cyclopenta[a]phenanthrene-17-carbonyl)thio)ethoxy)methyl)carbamoyl)-2,5,8,11,14,17,20,26,29-decamethyl-1,4,7,10,13,16, 19,22,25,28,31,34-dodecaoxo-2,5,8, 11, 14, 14, 17,20,23,26,29,32,35-dodecaazanonatriacontane-39-carboxylic acid (DL-B-134')

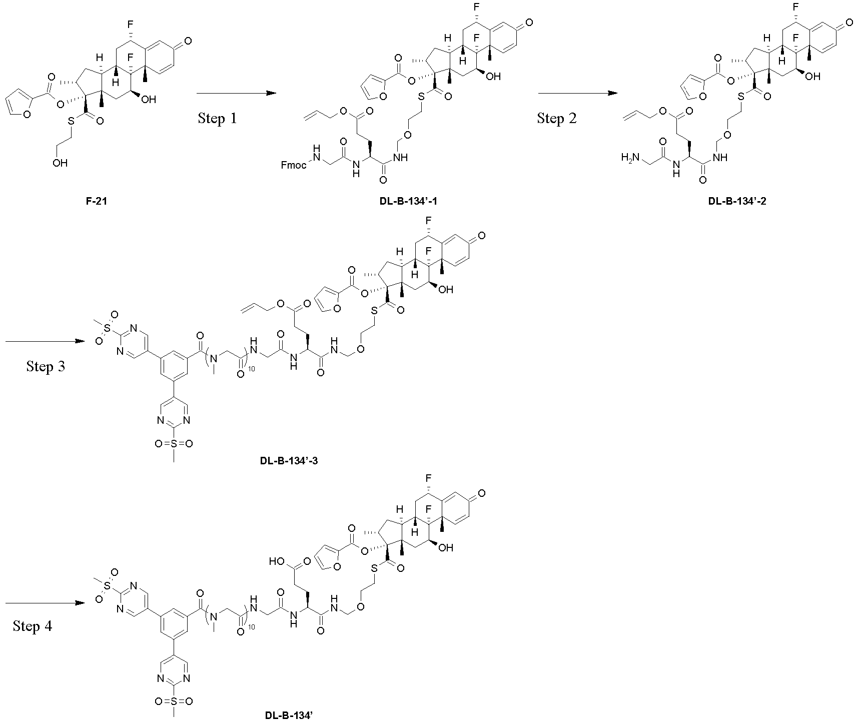

### Step 1: preparation of (6S,8S,9R,10S,11S,13S,14S,16R,17R)-17-((S)-8-(3-(allyloxy)-3-oxopropyl)-1-(9H-fluoren-9-yl)-3,6,9-trioxo-2,12-dioxa-15-thia-4,7,10-triazahexadecane-16-yl)-6,9-difluoro-11-hydroxy-10,13,16-trimethyl-3-oxo-6,7,8,9,10,11,12,13,14,15,16,17-dodecahydro-3H-cyclopenta[a]phenanthren-17-yl furan-2-carboxylate (DL-B-134'-1)

(6S,8S,9R,10S,11S,13S,14S,16R,17R)-6,9-difluoro-11-hydroxy-17-((2-hydroxyethyl)thio)carbonyl)-10,13,16-trimethyl-3-oxo-6,7,8,9,10,11,12,13,14,15,16,17-dodecahydro-3H-cyclopentane[a]phenanthren-17-yl furan-2-carboxylate (1.2 g, 2.18 mmol) and allyl (S)-4-(2-(((9H-fluoren-9-yl)methoxy)carbonyl)amino)acetamido-5-(acetoxymethyl)amino)-5-oxovalerate (1.32 g, 2.46 mmol) were dissolved in dichloromethane (30.0 mL), PPTS (547mg, 2.18 mmol) was added, and then the reaction solution was heated to 45°C and allowed to react for 12 h. Water (100 mL) was added into the reaction system, followed by extraction with dichloromethane for 3 times (30 mL x 3). The organic phases were combined, dried over anhydrous sodium sulfate, filtered and concentrated to obtain a crude product, which was purified via a silica gel column (dichloromethane/methanol = 100/1 to 10/1) and then concentrated again to obtain the title compound (610 mg, 577 µmol).

The structure characterization data is as follows:
ESI-MS (m/z): 1028.4 (M+H)⁺.

### Step 2: preparation of (6S,8S,9R,10S,11S,13S,14S,16R,17R)-17-((S)-9-(2-aminoacetamido)-8,12-dioxo-5,13-dioxa-2-thio-7-azahexadecan-15-enoyl)-6,9-difluoro-11-hydroxy-10,13,16-trimethyl-3-oxo-6,7,8,9,10,11,12,13,14,15,16,17-decahydro-3H-cyclopentane[a]phenanthren-17-yl furan-2-carboxylate (DL-B-134'-2)

(6S,8S,9R,10S,11S,13S,14S,16R,17R)-17-((S)-8-(3-(allyloxy)-3-oxopropyl)-1-(9H-fluoren-9-yl)-3,6,9-trioxo-2,12-dioxa-15-thia-4,7,10-triazahexadecane-16-yl)-6,9-difluoro-11-hydroxy-10,13,16-trimethyl-3-oxo-6,7,8,9,10,11,12,13,14,15,16,17-dodecahydro-3H-cyclopenta[a]phenanthren-17-yl furan-2-carboxylate (100 mg, 97.27 µmol) and diethylamine (21.34 mg, 291.80 µmol) were added into DMF (2 mL), and the reaction solution was allowed to react for 2 h at 25°C under stirring after the addition was finished. The reaction solution was directly placed on a freeze-dryer to remove the solvent and residual diethylamine to obtain the title compound as a crude product (78 mg), which was directly used in the next step without purification.

### Step 3: preparation of allyl (S)-1-(3,5-bis(2-(methylsulfonyl)pyrimidin-5-yl)phenyl)-36-((2-(((6S,8S,9R,10S,11S,13S,14S,16R,17R)-6,9-difluoro-17-(furan-2-carbonyl)oxy)-11-hydroxy-10,13,16-trimethyl-3-oxo-6,7,8,9,10,11,12,13,14,15,16,17-dodecahydro-3H-cyclopentane[a]phenanthren-17-carbonyl)thio)ethoxy)methyl)carbamoyl)-2,5,8,11,14,17,20,26,29-decamethyl-1,4,7,10,13,16,19,22,25,28,31,34-dodecaoxo-2,5,8,11,14,17,20,23,26,29,32,35-dodecaazanonatriacontane-39-carboxylate (DL-B-134'-3)

(6S,8S,9R,10S,11S,13S,14S,16R,17R)-17-((S)-9-(2-aminoacetamido)-8,12-dioxo-5,13-dioxa-2-thio-7-azahexadecan-15-enoyl)-6,9-difluoro-11-hydroxy-10,13,16-trimethyl-3-oxo-6,7,8,9,10,11,12,13,14,15,16,17-decahydro-3H-cyclopentane[a]phenanthren-17-yl furan-2-carboxylate (39 mg, 48.39 µmol) was dissolved in DMF (2 mL), 1-(3,5-bis(2-(methylsulfonyl)pyrimidin-5-yl)phenyl)-2,5,8,11,14,17,23,26,29-decamethyl-1,4,7,10,13,16,19,22,25,28-decaoxo-2,5,8,11,14,17,20,23,26,29-decaazahentriacontane-31-carboxylic acid (55.42 mg, 48.39 µmol), DIPEA (18.76 mg, 145.18 µmol) and HATU (22.07mg, 58.07 µmol) were added, and then the reaction solution was allowed to react for 4 h at 25°C under stirring after the addition was finished. The reaction solution was directly purified via high performance liquid chromatography and then freeze-dried to obtain the title compound (30.00 mg,15.52 µmol).

The purification method is as follows:
Chromatographic column: SunFire Prep C18 OBD 19*150 mm*5 µm
Mobile phase A: acetonitrile; mobile phase B: water (0.05% FA)

| Time [min] | Mobile phase A [%] | Mobile phase B [%] | Flow rate [mL/min] |
|---|---|---|---|
| 0.00 | 10 | 90 | 30 |
| 2.00 | 10 | 90 | 30 |
| 18.00 | 70 | 30 | 30 |

The structure characterization data is as follows:
ESI-MS (m/z): 1934.08 (M+H)⁺.

### Step 4: preparation of (S)-1-(3,5-bis(2-(methylsulfonyl)pyrimidin-5-yl)phenyl)-36-((2-(((6S,8S,9R,10S,11S,13S,14S,16R,17R)-6,9-difluoro-17-(furan-2-carbonyl)oxy)-11-hydroxy-10,13,16-trimethyl-3-oxo)-6,7,8,9,10,11,12,13,14,15,16,17-dodecahydro-3H-cyclopenta[a]phenanthrene-17-carbonyl)thio)ethoxy)methyl)carbamoyl)-2,5,8,11,14,17,20,26,29-decamethyl-1,4,7,10,13,16,19,22,25,28,31,34-dodecaoxo-2,5,8,11,14,14,17,20,23,26,29,32,35-dodecaazanonatriacontane-39-carboxylic acid (DL-B-134')

Allyl (S)-1-(3,5-bis(2-(methylsulfonyl)pyrimidin-5-yl)phenyl)-36-((2-(((6S,8S,9R,10S,11S,13S,14S,16R,17R)-6,9-difluoro-17-(furan-2-carbonyl)oxy)-11-hydroxy-10,13,16-trimethyl-3-oxo-6,7,8,9,10,11,12,13,14,15,16,17-dodecahydro-3H-cyclopentane[a]phenanthren-17-carbonyl)thio)ethoxy)methyl)carbamoyl)-2,5,8,11,14,17,20,26,29-decamethyl-1,4,7,10,13,16,19,22,25,28,3 1,34-dodecaoxo-2,5,8,11,14,17,20,23,26,29,32,35-dodecaazanonatriacontane-39-carboxylate (30.00 mg, 15.52 µmol), formic acid (30 µL, 10.35 µmol), N-methylmorpholine (50 µL, 10.35 µmol) and tetra(triphenylphosphine)palladium (5.98 mg, 5.17 µmol) were successively added into DMF (2 mL), and then the reaction solution was allowed to react for 2.0 h at 20°C under stirring after the addition was finished. The reaction solution was directly purified via high performance liquid chromatography and then freeze-dried to obtain the title compound (4.52 mg,2.27 µmol).

The purification method is as follows:
Chromatographic column: SunFire Prep C18 OBD 19*150 mm*5 µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 10 | 90 | 30 |
| 2.00 | 10 | 90 | 30 |
| 18.00 | 90 | 10 | 30 |

The structure characterization data is as follows:
ESI-MS (m/z): 1894.02 (M+H)⁺.

### Preparation example 77: Preparation of (6S,8S,9R,108S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (3S,6S,9S)-1-(3,5-bis(2-(methylsulfonyl)pyrimidin-5-yl)phenyl)-3,6,9-trimethyl-1,4,7,10-tetraoxo-13-oxo-2,5,8,11-tetraazahexadecane-16-carboxylate (DL-B-135)

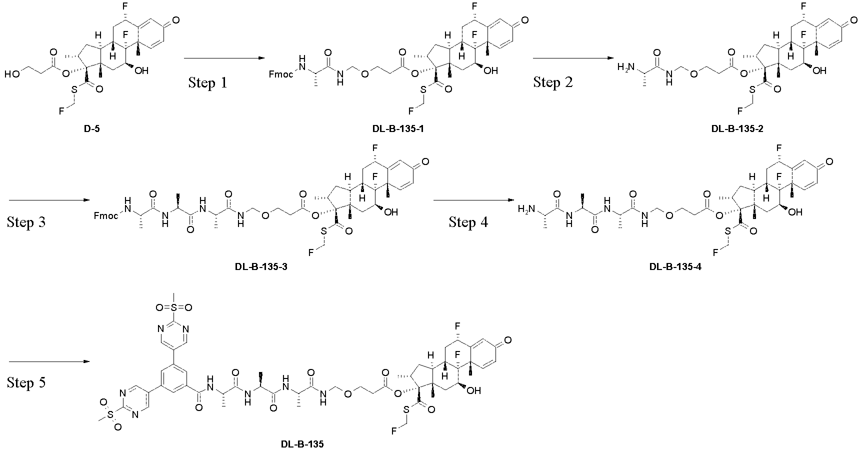

### Step 1: preparation of (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (S)-1-(9H-fluoren-9-yl)-5-methyl-3,6-dioxo-2,9-dioxa-4,7-diazadodecane-12-carboxylate (DL-B-135-1)

(6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 3-hydroxypropionate (173 mg, 334.9 µmol), (S)-(2-((((9H-fluoren-9-yl)methoxy)carbonyl)amino)propionamido)methyl acetate (200 mg, 523 µmol) and pyridinium p-toluenesulfonate (84.16 mg, 334.90 µmol) were successively added to DCM (10 mL). The reaction solution was allowed to react for 4 h at 25°C under stirring. The reaction solution was purified via silica gel column chromatography (dichloromethane/methanol = 100/1 to 10/1), and then spinned to dryness to remove the solvent to obtain the title compound (50 mg, 53.64 µmol).

The structure characterization data is as follows:
ESI-MS (m/z): 839.3 (M+H)⁺.

### Step 2: preparation of (6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 3-(((S)-2-aminopropanamido)methoxy)propionate (DL-B-135-2)

(6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (S)-1-(9H-fluoren-9-yl)-5-methyl-3,6-dioxo-2,9-dioxa-4,7-diazadodecane-12-carboxylate (50 mg, 59.60 µmol) was dissolved in N,N-dimethylformamide (2 mL), diethylamine (8.72 mg, 119.20 µmol) was added, and then the reaction solution was allowed to react for 1 h at 25°C under stirring. The reaction solution was directly purified via high performance liquid chromatography and then freeze-dried to obtain the title compound (35 mg, 53.92 µmol).

The purification method is as follows:
Chromatographic column: SunFire Prep C18 OBD 19*150 mm*5 µm
Mobile phase A: acetonitrile; mobile phase B: water (0.05% FA)

| Time [min] | Mobile phase A [%] | Mobile phase B [%] | Flow rate [mL/min] |
|---|---|---|---|
| 0.00 | 10 | 90 | 30 |
| 2.00 | 10 | 90 | 30 |
| 18.00 | 70 | 30 | 30 |

The structure characterization data is as follows:
ESI-MS (m/z): 617.3 (M+H)⁺.

### Step 3: preparation of (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (5S,8S,11S)-1-(9H-fluoren-9-yl)-5,8,11-trimethyl-3,6,9,12-tetraoxo-2,15-dioxa-4,7,10,13-tetraazaoctadecane-18-carboxylate (DL-B-135-3)

(6S,8S,9R,10S,11S,13S,14S,16R,17R)-6,9-difluoro-17-(((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl 3-(((S)-2-aminopropanamido)methoxy)propionate (35 mg, 56.75 µmol), ((9H-fluoren-9-yl)methoxy)carbonyl)-L-alanine-L-alanine (21.70 mg, 56.75 µmol), DIPEA (22.01 mg, 170.26 µmol) and Carter condenser (50.20 mg, 113.51 µmol) were successively added into DMF (2 mL), and then the reaction solution was allowed to react for 1.0 h at 20°C under stirring after the addition was finished. The reaction solution was directly purified via high performance liquid chromatography and then freeze-dried to obtain the title compound (22 mg, 21.30 µmol).

The purification method is as follows:
Chromatographic column: SunFire Prep C18 OBD 19*150 mm*5 µm
Mobile phase A: acetonitrile; mobile phase B: water (0.05% FA)

| Time [min] | Mobile phase A [%] | Mobile phase B [%] | Flow rate [mL/min] |
|---|---|---|---|
| 0.00 | 50 | 50 | 30 |
| 2.00 | 50 | 50 | 30 |
| 18.00 | 95 | 5 | 30 |

The structure characterization data is as follows:
ESI-MS (m/z): 981.4 (M+H)⁺.

### Step 4: preparation of (6S,8S,9R,108,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (8S,11S,14S)-14-amino-8,11-dimethyl-7,10,13-trioxo-4-oxa-6,9,12-triazapentadecane carboxylate (DL-B-135-4)

(6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (5S,8S,11S)-1-(9H-fluoren-9-yl)-5,8,11-trimethyl-3,6,9,12-tetraoxo-2,15-dioxa-4,7,10,13-tetraazaoctadecane-18-carboxylate (22 mg, 21.30 µmol) was dissolved in N,N-dimethylformamide (2 mL), diethylamine (3.28 mg, 44.85 µmol) was added, and then the reaction solution was allowed to react for 1 h at 25°C under stirring after the addition was finished. The reaction solution was directly purified via high performance liquid chromatography and then freeze-dried to obtain the title compound (10 mg, 11.86 µmol).

The purification method is as follows:
Chromatographic column: SunFire Prep C18 OBD 19*150 mm*5 µm
Mobile phase A: acetonitrile; mobile phase B: water (0.05% FA)

| Time [min] | Mobile phase A [%] | Mobile phase B [%] | Flow rate [mL/min] |
|---|---|---|---|
| 0.00 | 10 | 90 | 30 |
| 2.00 | 10 | 90 | 30 |
| 18.00 | 70 | 30 | 30 |

The structure characterization data is as follows:
ESI-MS (m/z): 759.4 (M+H)⁺.

### Step 5: preparation of (6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (3S,6S,9S)-1-(3,5-bis(2-(methylsulfonyl)pyrimidin-5-yl)phenyl)-3,6,9-trimethyl-1,4,7,10-tetraoxo-13-oxo-2,5,8,11-tetraazahexadecane-16-carboxylate (DL-B-135)

(6S,8S,9R,10S,11S,13S,14S,16R,17R)-6,9-difluoro-17-((fluoromethyl)thio)carbonyl)-11-hydroxy-10,13,16-trimethyl-3-oxo-6,7,8,9,10,11,12,13,14,15,16,17-dodecahydro-3H-cyclopenta[a]phenanthren-17-yl (8S,11S,14S)-14-amino-8,11-dimethyl-7,10,13-trioxo-4-oxa-6,9,12-triazapentadecane carboxylate (18 mg, 22.36 µmol), 2,5-dioxopyrrolidin-1-yl 3,5-bis(2-(methylsulfonyl)pyrimidin-5-yl)benzoate (17.83 mg, 33.55 µmol) and DIPEA (5.78 mg, 44.73 µmol) were successively added into DMF (2 mL), and then the reaction solution was allowed to react for 1.0 h at 20°C under stirring after the addition was finished. The reaction solution was directly purified via high performance liquid chromatography and then freeze-dried to obtain the title compound (6.85 mg, 5.71 µmol).

The purification method is as follows:
Chromatographic column: SunFire Prep C18 OBD 19*150 mm*5 µm
Mobile phase A: acetonitrile; mobile phase B: water (0.05% FA)

| Time [min] | Mobile phase A [%] | Mobile phase B [%] | Flow rate [mL/min] |
|---|---|---|---|
| 0.00 | 10 | 90 | 30 |
| 2.00 | 10 | 90 | 30 |
| 18.00 | 70 | 30 | 30 |

The structure characterization data is as follows:
ESI-MS (m/z): 1175.3 (M+H)⁺.

### Preparation example 78: Preparation of (6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-2-((S)-2-((S)-2-(3,5-bis(2-(methylsulfonyl)pyrimidin-5-yl)benzamido)propionamido)propionamido)propionamido)-4-fluorobenzoate (DL-B-136)

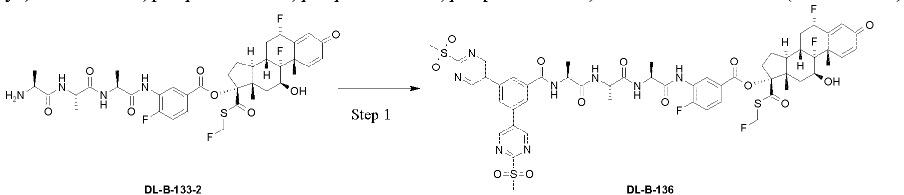

### Step 1 : (6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-2-((S)-2-((S)-2-(3,5-bis(2-(methylsulfonyl)pyrimidin-5-yl)benzamido)propionamido)propionamido)propionamido)-4-fluorobenzoate (DL-B-136)

(6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-2-((S)-2-((S)-2-aminopropanamido)propanamido)propanamido)-4-fluorobenzoate (30 mg, 36.28 µmol), 2,5-dioxopyrrolidin-1-yl 3,5-bis(2-(methylsulfonyl)pyrimidin-5-yl)benzoate (28.93 mg, 54.42 µmol) and DIPEA (46.89 mg, 362.82 µmol) were successively added into DMF (5 mL), and then the reaction solution was allowed to react for 1 h at 20°C under stirring after the addition was finished. The reaction solution was directly purified via high performance liquid chromatography and then freeze-dried to obtain the title compound (10.42 mg, 8.44 µmol).

The purification method is as follows:
Chromatographic column: Agilent Prep C18 OBD 19*150 mm*5 µm
Mobile phase A: acetonitrile; mobile phase B: water (0.05% NH₄HCO₃)

| Time [min] | Mobile phase A [%] | Mobile phase B [%] | Flow rate [mL/min] |
|---|---|---|---|
| 0.00 | 35 | 65 | 30 |
| 2.00 | 35 | 65 | 30 |
| 18.00 | 90 | 10 | 30 |

The structure characterization data is as follows:
ESI-MS (m/z): 1198.7 (M+H)⁺.

### Preparation example 79: Preparation of (6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-6-amino-2-((S)-2-(3,5-bis(2-(methylsulfonyl)pyrimidin-5-yl)benzamido)-3-methylbutanamido)hexanamido)-4-fluorobenzoate (DL-B-137)

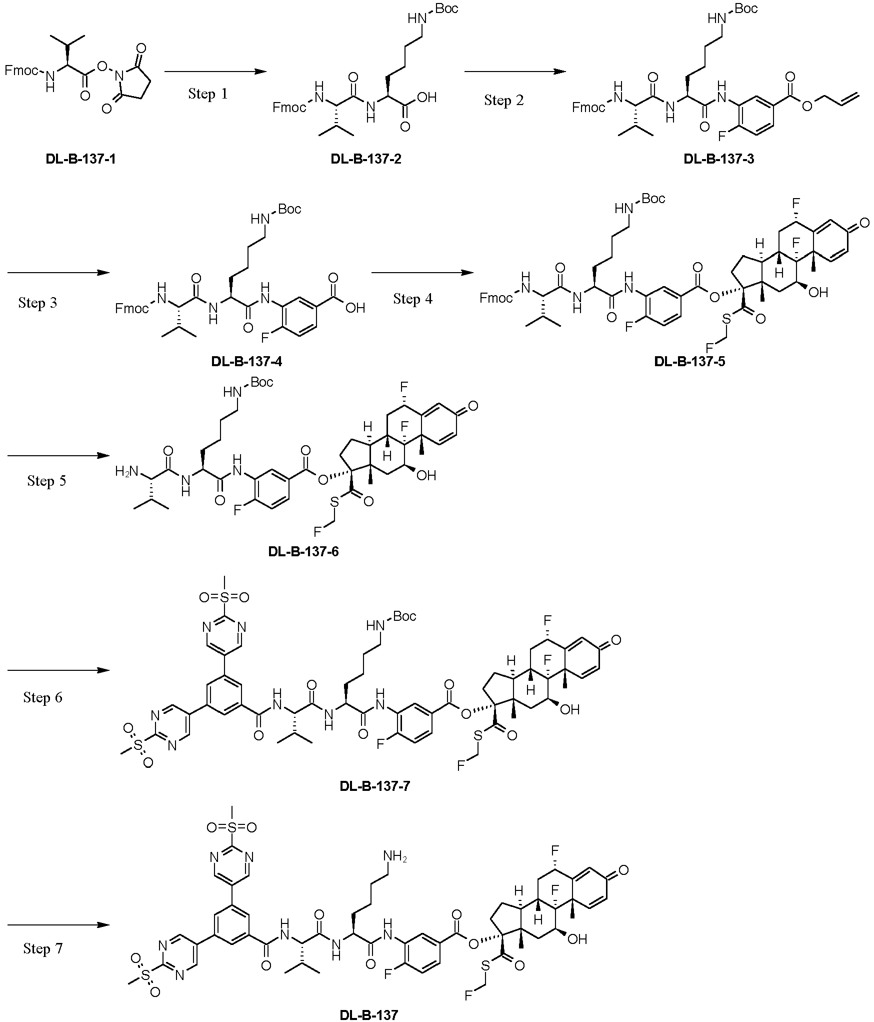

### Step 1: preparation of N²-(((9H-fluoren-9-yl)methoxy)carbonyl)-L-valyl)-N⁶-(tert-butoxycarbonyl)-L-lysine (DL-B-137-2)

2,5-dioxopyrrolidin-1-yl (((9H-fluoren-9-yl)methoxy)carbonyl)-L-valinate (1.05 g, 2.29 mmol) and N⁶-(tert-butoxycarbonyl)-L-lysine (564.32 g, 2.29 mmol) were dissolved in acetone (10 mL), then a sodium bicarbonate (192.48 mg, 2.29 mmol) aqueous solution was added, and then the reaction solution was allowed to react for 6 h at 25°C under stirring after the addition was finished. After the reaction was completed, the reaction solution was adjusted to acidic pH, directly purified via high performance liquid chromatography and then freeze-dried to obtain the title compound (1.1 g, 1.94 mmol). The purification method is as follows:
Chromatographic column: SunFire Prep C18 OBD 19*150 mm*5 µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 10 | 90 | 28 |
| 2.00 | 10 | 90 | 28 |
| 18.00 | 90 | 10 | 28 |

The structure characterization data is as follows:
ESI-MS (m/z): 568.67 (M+H)⁺.

### Step 2: preparation of allyl 3-((S)-2-((S)-2((((9H-fluoren-9-yl)methoxy)carbonyl)amino)-3-methylbutanamido)-6-((tert-butoxycarbonyl)amino)hexanamido)-4-fluorobenzoate (DL-B-137-3)

N²-(((9H-fluoren-9-yl)methoxy)carbonyl)-L-valyl)-N⁶-(tert-butoxycarbonyl)-L-lysine (1.1 g, 1.94 mmol) and allyl 3-amino-4-fluorobenzoate (433.24 mg, 2.22 mmol) were dissolved in DCM (2 mL), pyridine (438.92 mg, 5.55 mmol) and T3P (6.51g, 10.63 mmol) were added, and then the reaction solution was allowed to react for 6 h at 25°C under stirring after the addition was finished. The reaction solution was directly purified via high performance liquid chromatography and then freeze-dried to obtain the title compound (0.73 g,1.23 mmol). The purification method is as follows:
Chromatographic column: SunFire Prep C18 OBD 19*150 mm*5 µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 10 | 90 | 30 |
| 2.00 | 10 | 90 | 30 |
| 18.00 | 90 | 10 | 30 |

The structure characterization data is as follows:
ESI-MS (m/z): 745.35 (M+H)⁺.

### Step 3: preparation of 3-((S)-2-((S)-2((((9H-fluoren-9-yl)methoxy)carbonyl)amino)-3-methylbutanamido)-6-((tert-butoxycarbonyl)amino)hexanamido)-4-fluorobenzoic acid (DL-B-137-4)

Allyl 3-((S)-2-((S)-2((((9H-fluoren-9-yl)methoxy)carbonyl)amino)-3-methylbutanamido)-6-((tert-butoxycarbonyl)amino)hexanamido)-4-fluorobenzoate (0.73 g, 1.23 mmol), formic acid (150 µL, 1.21 mmol), N-methylmorpholine (300 µL, 1.21 mmol) and tetra(triphenylphosphine)palladium (418.88 mg, 362.49 µmol) were successively added into DMF (2 mL), and then the reaction solution was allowed to react for 2.0 h at 20°C under stirring after the addition was finished. The reaction solution was directly purified via high performance liquid chromatography and then freeze-dried to obtain the title compound (700.0 mg,993.21 µmol). The purification method is as follows:
Chromatographic column: SunFire Prep C18 OBD 19*150 mm*5 µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 10 | 90 | 30 |
| 2.00 | 10 | 90 | 30 |
| 18.00 | 90 | 10 | 30 |

The structure characterization data is as follows:
ESI-MS (m/z): 705.78 (M+H)⁺.

### Step 4: preparation of (6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-2-(((9H-fluoren-9-yl)methoxy)carbonyl)amino)-3-methylbutanamido)-6-((tert-butoxycarbonyl)amino)hexanamido)-4-fluorobenzoate (DL-B-137-5)

3-((S)-2-((S)-2((((9H-fluoren-9-yl)methoxy)carbonyl)amino)-3-methylbutanamido)-6-((tert-butoxycarbonyl)amino)hexanamido)-4-fluorobenzoic acid (150 mg, 212.83 µmol), (6S, 8S, 9R, 10S, 11S, 13S, 14S, 17R)-6,9-difluoro-11,17-dihydroxy-10,13-dimethyl-3-oxo-6,7,8,10,12,13,14,15,16,17-dodecahydro-3H-cyclopenta[a]phenanthrene-17-thiocarboxylic acid (121.15 mg, 212.85 mg), DIPEA (82.52 mg, 638.49 µmol) and HATU (80.92 mg, 212.83 µmol) were successively added into DMF (2 mL), and then the reaction solution was allowed to react for 2.0 h at 20°C under stirring after the addition was finished. Fluoroiodomethane (101.86 mg, 638.49 µmol) was added into the reaction solution, and then the reaction solution was allowed to react for 8.0 h at 50°C under stirring after the addition was finished. The reaction solution was directly purified via high performance liquid chromatography and then freeze-dried to obtain the title compound (20.0 mg, 22.35 µmol). The purification method is as follows:
Chromatographic column: SunFire Prep C18 OBD 19*150 mm*5 µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 10 | 90 | 30 |
| 2.00 | 10 | 90 | 30 |
| 18.00 | 90 | 10 | 30 |

The structure characterization data is as follows:
ESI-MS (m/z): 1118.25 (M+H)⁺.

### Step 5: preparation of (6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-2-((S)-2-amino-3-methylbutanamido)-6-(tert-butoxycarbonyl)amino)hexanamido)-4-fluorobenzoate (DL-B-137-6)

(6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-2-(((9H-fluoren-9-yl)methoxy)carbonyl)amino)-3-methylbutanamido)-6-((tert-butoxycarbonyl)amino)hexanamido)-4-fluorobenzoate (30.0 mg, 26.85 µmol) and diethylamine (5.89 mg, 80.56 µmol) were successively added into DMF (2 mL), and then the reaction solution was allowed to react for 3.0 h at 25°C under stirring after the addition was finished. The reaction solution was directly used in the next step without purification.

### Step 6: preparation of (6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-2-((S)-2-(3,5-bis(2-(methylsulfonyl)pyrimidin-5-yl)benzamido)-3-methylbutanamido)-6-(tert-butoxycarbonyl)amino)hexanamido)-4-fluorobenzoate (DL-B-137-7)

(6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-2-((S)-2-amino-3-methylbutanamido)-6-(tert-butoxycarbonyl)amino)hexanamido)-4-fluorobenzoate (20.0 mg, 22.35 µmol), 2,5-dioxopyrrolidin-1-yl 3,5-bis(2-(methylsulfonyl)pyrimidin-5-yl)benzoate (17.82 mg, 33.52 µmol) and DIPEA (5.78 mg, 44.69 µmol) were successively added into DMF (2 mL), and then the reaction solution was allowed to react for 3.0 h at 25°C under stirring after the addition was finished. The reaction solution was directly purified via high performance liquid chromatography and then freeze-dried to obtain the title compound (18.0 mg,13.73 µmol). The purification method is as follows:
Chromatographic column: SunFire Prep C18 OBD 19*150 mm*5 µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 10 | 90 | 30 |
| 2.00 | 10 | 90 | 30 |
| 18.00 | 90 | 10 | 30 |

The structure characterization data is as follows:
ESI-MS (m/z): 1312.44 (M+H)⁺.

### Step 7: preparation of (6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-6-amino-2-((S)-2-(3,5-bis(2-(methylsulfonyl)pyrimidin-5-yl)benzamido)-3-methylbutanamido)hexanamido)-4-fluorobenzoate (DL-B-137)

(6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-2-((S)-2-(3,5-bis(2-(methylsulfonyl)pyrimidin-5-yl)benzamido)-3-methylbutanamido)-6-(tert-butoxycarbonyl)amino)hexanamido)-4-fluorobenzoate (20.0 mg, 22.35 µmol) and trifluoroacetic acid (3 mL) were successively added into DCM (2 mL), and then the reaction solution was allowed to react for 5.0 h at 25°C under stirring after the addition was finished. The reaction solution was directly purified via high performance liquid chromatography and then freeze-dried to obtain the title compound (6.96 mg,5.67 µmol). The purification method is as follows:
Chromatographic column: SunFire Prep C18 OBD 19*150 mm*5 µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 10 | 90 | 30 |
| 2.00 | 10 | 90 | 30 |
| 18.00 | 90 | 10 | 30 |

The structure characterization data is as follows:
ESI-MS (m/z): 1212.33 (M+H)⁺.

### Preparation example 80: Preparation of (6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-(2-(S)-6-amino)-2-(S)-2-(3,5-bis(2-(methylsulfonyl)pyrimidin-5-yl)benzamido)-3-methylbutanamido)hexanamido)acetamido)-4-fluorobenzoate(DL-B-138)

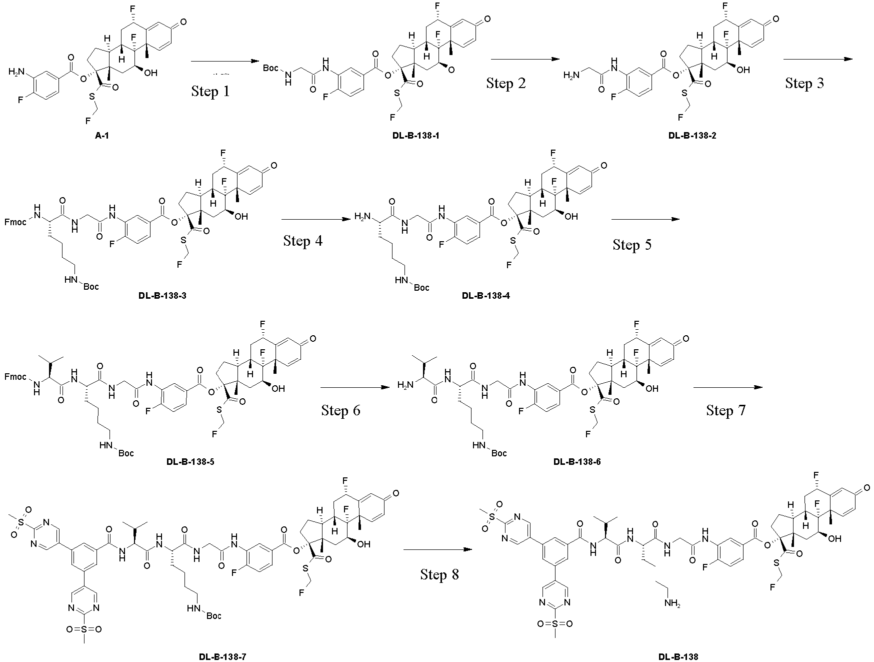

### Step 1: preparation of (6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-(2-(tert-butoxycarbonyl)amino)acetamido)-4-fluorobenzoate (DL-B-138-1)

(6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-amino-4-fluorobenzoate trifluoroacetate (1.50 g, 2.20 mmol) and (tert-butoxycarbonyl)glycine (385 mg, 2.20 mmol) were dissolved in dichloromethane (20.0 mL), pyridine (522 mg, 6.60 mmol, 532 µL) and T3P (10.6 g, 16.7 mmol, 9.98 mL, 50% purity) were added, and then the reaction solution was allowed to react for 1 h at 25°C. The reaction solution was added into water (30.0 mL), followed by extraction with ethyl acetate for 3 times (20.0 mL x 3). The organic phases were combined, dried over anhydrous sodium sulfate, filtered and concentrated to obtain a crude product, which was purified via a silica gel column (petroleum ether/ethyl acetate = 100/1 to 2/1) and then concentrated again to obtain the title compound (650 mg, 869.95 µmol).

The structure characterization data is as follows:
ESI-MS (m/z): 725.3 (M+H)⁺.

### Step 2: preparation of (6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-(2-aminoacetamido)-4-fluorobenzoate (DL-B-138-2)

(6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-(2-(tert-butoxycarbonyl)amino)acetamido)-4-fluorobenzoate (650 mg, 897 µmol) was dissolved in dichloromethane (2.00 mL), trifluoroacetic acid (6140 mg, 5.38 mmol, 0.4 mL) was added, and then the reaction solution was stirred for 1 h at 25°C. The reaction solution was directly concentrated to obtain a trifluoroacetate of the title compound as a crude product (730 mg, crude), which was directly used in the next step without purification.

The structure characterization data is as follows:
ESI-MS (m/z): 625.2 (M+H)⁺.

### Step 3: preparation of (6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-(2-((S)-2-(((9H-fluoren-9-yl)methoxy)carbonyl)amino)-6-((tert-butoxycarbonyl)amino)hexanamido)acetamido-4-fluorobenzoate (DL-B-138-3)

(6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-(2-aminoacetamido)-4-fluorobenzoate (730 mg, 988 µmol) and N²-(((9H-fluoren-9-yl)methoxy)carbonyl)-N⁶-(tert-butoxycarbonyl)-L-lysine (463 mg, 988 µmol) were dissolved in DMF (7.00 mL), HATU (563 mg, 1.48 mmol) and DIPEA (383 mg, 2.96 mmol, 516 µL) were added, and then the reaction solution was stirred for 1 h at 25°C. Water was added into the reaction solution until a large amount of white solids were precipitated out, and the reaction solution was extracted with ethyl acetate for 3 times. The organic phases were combined, dried over anhydrous sodium sulfate, filtered and concentrated to obtain the title compound as a crude product (750 mg, 660.59 µmol), which was directly used in the next step without purification.

The structure characterization data is as follows:
ESI-MS (m/z): 1075.6 (M+H)⁺.

### Step 4: preparation of (6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-(2-((S)-2-amino)-6-(tert-butoxycarbonyl)amino)hexanamido)acetamido)-4-fluorobenzoate (DL-B-138-4)

(6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-(2-((S)-2-(((9H-fluoren-9-yl)methoxy)carbonyl)amino)-6-((tert-butoxycarbonyl)amino)hexanamido)acetamido-4-fluorobenzoate (700 mg, 651 µmol) was dissolved in DMF (7.00 mL), DBU (79.2 mg, 520 µmol, 78.5 µL) was added, and then the reaction solution was stirred for half an hour at 25°C. The reaction solution was directly used in the next step without post-treatment.

The structure characterization data is as follows:
ESI-MS (m/z): 853.4 (M+H)⁺.

### Step 5: preparation of (6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((5S,8S)-8-(4-(tert-butoxycarbonyl)amino)butyl)-1-(9H-fluoren-9-yl)-5-isopropyl-3,6,9-trioxo-2-oxa-4,7,10-triazadodecan-12-amino)-4-fluorobenzoate (DL-B-138-5)

EDCI (187 mg, 976 µmol) and HOBt (131 mg, 976 µmol) were added into the reaction solution obtained in the previous step, and then the reaction solution was stirred for 1 h at 25°C. Water was added into the reaction solution until a large amount of solids were precipitated out, followed by filtration. The filter cake was dried to obtain the title compound as a crude product (380 mg, 288 µmol), which was directly used in the next step without purification.

The structure characterization data is as follows:
ESI-MS (m/z): 1274.5 (M+H)⁺.

### Step 6: preparation of (6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-(2-(S)-2-((S)-2-amino-3-methylbutanamido)-6-(tert-butoxycarbonyl)amino)hexanamido)acetamido)-4-fluorobenzoate (DL-B-138-6)

(6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((5S,8S)-8-(4-(tert-butoxycarbonyl)amino)butyl)-1-(9H-fluoren-9-yl)-5-isopropyl-3,6,9-trioxo-2-oxa-4,7,10-triazadodecan-12-amino)-4-fluorobenzoate (330 mg, 281 µmol) was dissolved in acetonitrile (2.00 mL), DEA(105 mg, 281 µmol, 0.40 mL) was added, and then the reaction solution was stirred for half an hour at 25°C. The reaction solution was directly concentrated to obtain the title compound as a crude product (380 mg, crude), which was directly used in the next step without purification.

### Step 7: preparation of (6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-(2-(S)-2-((S)-2-(3,5-bis(2-(methylsulfonyl)pyrimidin-5-yl)benzamido)-3-methylbutanamido-6-(tert-butoxycarbonyl)amino)hexanamido)acetamido)-4-fluorobenzoate (DL-B-138-7)

(6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-(2-(S)-2-((S)-2-amino-3-methylbutanamido)-6-(tert-butoxycarbonyl)amino)hexanamido)acetamido)-4-fluorobenzoate (237 mg, 248 µmol) and 3,5-bis(2-(methylsulfonyl)pyrimidin-5-yl)benzoic acid (108 mg, 248 µmol) were dissolved in DMF (5.00 mL). DIPEA (96.5 mg, 746 µmol, 130 µL) and T3P (1.28 g, 2.01 mmol, 1.20 mL, 50% purity) were added, and then the reaction solution was stirred for half an hour at 25°C. Water was added into the reaction solution until a large amount of solids were precipitated out, and the precipitated solids were filtered and dried to obtain a crude product, which was purified via high performance liquid chromatography and then freeze-dried to obtain the title compound (120 mg, 78.9 µmol).

The purification method is as follows:
Chromatographic column: CD02-Waters Xbidge BEH C1825*150 mm*10 µm
Mobile phase A: acetonitrile; mobile phase B: water (0.05% ammonium bicarbonate)

| Time [min] | Mobile phase A [%] | Mobile phase B [%] | Flow rate [mL/min] |
|---|---|---|---|
| 0.00 | 40 | 60 | 25 |
| 13.00 | 70 | 30 | 25 |

The structure characterization data is as follows:
ESI-MS (m/z): 1268.6 (M+H)⁺.

### Step 8: preparation of (6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-(2-(S)-6-amino)-2-(S)-2-(3,5-bis(2-(methylsulfonyl)pyrimidin-5-yl)benzamido)-3-methylbutanamido)hexanamido)acetamido)-4-fluorobenzoate (DL-B-138)

(6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-(2-(S)-2-((S)-2-(3,5-bis(2-(methylsulfonyl)pyrimidin-5-yl)benzamido)-3-methylbutanamido-6-(tert-butoxycarbonyl)amino)hexanamido)acetamido)-4-fluorobenzoate (60.0 mg, 43.8 µmol) was dissolved in dichloromethane (2.00 mL), trifluoroacetic acid (614 mg, 5.38 µmol, 0.40 mL) was added, and then the reaction solution was stirred for half an hour at 25°C. The reaction solution was directly concentrated to obtain a trifluoroacetate of the title compound (45.9 mg, 34.4 µmol).

The structure characterization data is as follows:
ESI-MS (m/z): 1268.4 (M+H)⁺.

### Preparation example 81: Preparation of (6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((9S,12S)-1-(3,5-bis(2-(methylsulfonyl)pyrimidin-5-yl)phenyl)-9-isopropyl-12-methyl-1,4,7,10-tetraoxo-2,5,8,11-tetraazatridecan-13-amido)-4-fluorobenzoate (DL-B-139)

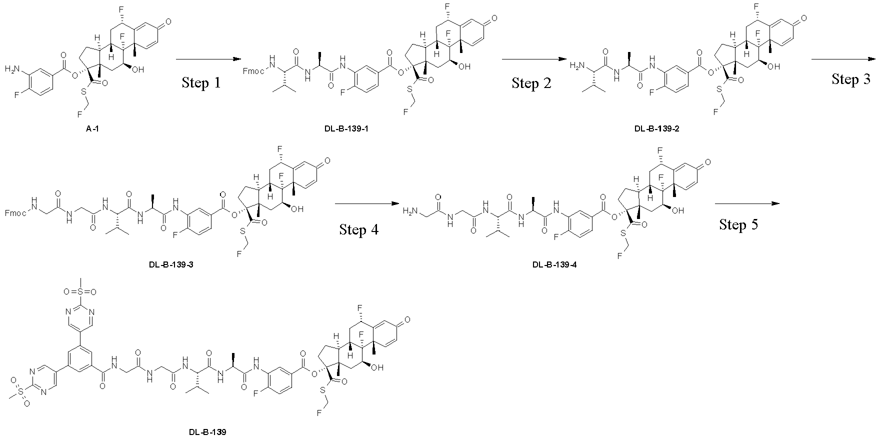

### Step 1: preparation of (6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-2-(((9H-fluoren-9-yl)methoxy)carbonyl)amino)-3-methylbutanamido)propanamido)-4-fluorobenzoate (DL-B-139-1)

(6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-amino-4-fluorobenzoate trifluoroacetate (900 mg, 1.32 mmol) and (((9H-fluoren-9-yl)methoxy)carbonyl)-L-valyl-L-alanine (650 mg, 1.58 mmol) were dissolved in dichloromethane (10 mL), pyridine (522 mg, 6.60 mmol, 532 µL) and T3P (4.41 g, 6.93 mmol, 4.13 mL, 50% purity) were added, and then the reaction solution was stirred for 1 h at 25°C. Water (300 mL) was added into the reaction solution, followed by extraction with dichloromethane for 3 times (200 mL x 3). The organic phases were combined, dried over anhydrous sodium sulfate, filtered and concentrated to obtain a crude product, which was purified via a silica gel column (petroleum ether/ethyl acetate=100/1 to 1/1) and then concentrated again to obtain the title compound (400 mg, 416 µmol).

The structure characterization data is as follows:
ESI-MS (m/z): 960.4 (M+H)⁺.

### Step 2: preparation of (6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-2-((S)-2-amino-3-methylbutanamido)propanamido)-4-fluorobenzoate (DL-B-139-2)

(6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-2-(((9H-fluoren-9-yl)methoxy)carbonyl)amino)-3-methylbutanamido)propanamido)-4-fluorobenzoate (350 mg, 364 µmol) was dissolved in DMF (5.00 mL), DBU (49.9 mg, 328 µmol, 49.4 µL) was added, and then the reaction solution was stirred for half an hour at 25°C. The reaction solution was directly used in the next step without treatment.

The structure characterization data is as follows:
ESI-MS (m/z): 738.3 (M+H)⁺.

### Step 3: preparation of (6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((11S,14S)-1-(9H-fluoren-9-yl)-11-isopropyl-14-methyl-3,6,9,12-tetraoxo-2-oxa-4,7,10,13-tetraazapentadecan-15-amido)-4-fluorobenzoate (DL-B-139-3)

(((9H-fluoren-9-yl)methoxy)carbonyl)glycylglycine (141 mg, 399 µmol), EDCI (90.5 mg, 472 µmol) and HOBt (63.8 mg, 472 µmol) were successively added into the reaction solution obtained in the previous step, and then the reaction solution was stirred for 1 h at 25°C. Water (100 mL) was added into the reaction solution, and the reaction solution was filtered and dried to obtain the title compound as a crude product (390 mg, crude), which was directly used in the next step without purification.

The structure characterization data is as follows:
ESI-MS (m/z): 1074.5 (M+H)⁺.

### Step 4: preparation of (6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-2-(2-(2-aminoacetamido)acetamido)-3-methylbutanamido)propionamido)-4-fluorobenzoate (DL-B-139-4)

(6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((11S,14S)-1-(9H-fluoren-9-yl)-11-isopropyl-14-methyl-3,6,9,12-tetraoxo-2-oxa-4,7,10,13-tetraazapentadecan-15-amido)-4-fluorobenzoate (350 mg, 325 µg) was dissolved in acetonitrile (10.0 mL), DEA (497 mg, 6.80 mmol, 0.70 mL) was added, and then the reaction solution was stirred for 1 h at 25°C. The reaction solution was directly concentrated to obtain the title compound as a crude product (350 mg, crude), which was directly used in the next step without purification.

The structure characterization data is as follows:
ESI-MS (m/z): 852.4 (M+H)⁺.

### Step 5: preparation of (6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((9S,12S)-1-(3,5-bis(2-(methylsulfonyl)pyrimidin-5-yl)phenyl)-9-isopropyl-12-methyl-1,4,7,10-tetraoxo-2,5,8,11-tetraazatridecan-13-amido)-4-fluorobenzoate (DL-B-139)

(6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-2-(2-(2-aminoacetamido)acetamido)-3-methylbutanamido)propionamido)-4-fluorobenzoate (270 mg, 316 µmol) and 3,5-bis(2-(methylsulfonyl)pyrimidin-5-yl)benzoic acid (137 mg, 316 µmol) were dissolved in DMF (10.0 mL), DIPEA (163 mg, 1.27 mmol, 220 µL) and T3P (1.69 g, 2.66 mmol, 1.58 mL, 50% purity) were added, and then the reaction solution was stirred for 2 h at 25°C. Water (100 mL) was added into the reaction solution, and the reaction solution was filtered and dried to obtain a crude product, which was purified via high performance liquid chromatography and then freeze-dried to obtain the title compound (22.0 mg, 17.2 µmol).

The purification method is as follows:
Chromatographic column: CD02-Waters Xbidge BEH C1825*150 mm*10 µm
Mobile phase A: acetonitrile; mobile phase B: water (0.05% ammonium bicarbonate)

| Time [min] | Mobile phase A [%] | Mobile phase B [%] | Flow rate [mL/min] |
|---|---|---|---|
| 0.00 | 32 | 68 | 25 |
| 13.00 | 62 | 38 | 25 |

The structure characterization data is as follows:
ESI-MS (m/z): 1268.6 (M+H)⁺.

### Preparation example 82: Preparation of (S)-4-(S)-2-(R)-2-(3,5-bis(2-(methylsulfonyl)pyrimidin-5-yl)benzamido)-4-methylpentanamido)propanamido)-5-(5-(((6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl)oxy)carbonyl)-2-fluorophenyl)amino)-5-oxovaleric acid (DL-B-140)

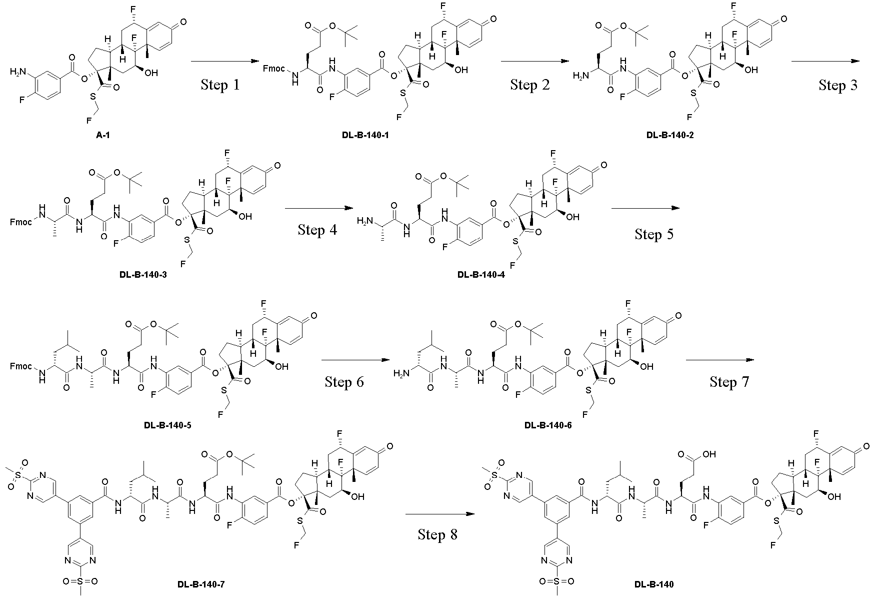

### Step 1: preparation of (6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-2-(((9H-fluoren-9-yl)methoxy)carbonyl)amino)-5-tert-butoxy-5-oxopentanamido)-4-fluorobenzoate (DL-B-140-1)

(6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-amino-4-fluorobenzoate trifluoroacetate (1.50 g, 2.20 mmol) and (S)-2-((((9H-fluoren-9-yl)methoxy)carbonyl)amino)-5-tert-butoxy-5-oxovaleric acid (936 mg, 2.20 mmol) were dissolved in dichloromethane (20.0 mL), pyridine (522 mg, 6.60 mmol, 533 µL) and T3P (10.8 g, 17.0 mmol, 10.1 mL, 50% purity) were added, and then the reaction solution was stirred for 1 h at 25°C. Water (200 mL) was added into the reaction solution, followed by extraction with dichloromethane for 3 times (100 mL x 3). The organic phases were combined, dried over anhydrous sodium sulfate, filtered and concentrated to obtain a crude product, which was purified via a silica gel column (petroleum ether/ethyl acetate = 20/1 to 1/1) and then concentrated again to obtain the title compound (610 mg, 604 µmol).

The structure characterization data is as follows:
ESI-MS (m/z): 975.4 (M+H)⁺.

### Step 2: preparation of (6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-2-amino-5-(tert-butoxy)-5-oxopentanamido)-4-fluorobenzoate (DL-B-140-2)

(6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-2-(((9H-fluoren-9-yl)methoxy)carbonyl)amino)-5-tert-butoxy-5-oxopentanamido)-4-fluorobenzoat (560 mg, 574 µmol) was dissolved in DMF (5.60 mL), DBU (69.9 mg, 459 µmol, 69.3 µL) was added, and the reaction solution was stirred for half an hour at 25°C and then directly used in the next step without treatment.

### Step 3: preparation of (6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-2-((S)-2-(((9H-fluoren-9-yl)methoxy)carbonyl)amino)propanamido)-5-tert-butoxy-5-oxopentanamido)-4-fluorobenzoate (DL-B-140-3)

((9H-fluoren-9-yl)methoxy)carbonyl)-L-alanine (178 mg, 572.6 µmol), EDCI (165 mg, 859 µmol) and HOBt (116 mg, 859 µmol) were successively added into the reaction solution obtained in the previous step, and then the reaction solution was stirred for 1 h at 25°C. The reaction solution was poured into water (56.0 mL), and stirred for additional 30 min. The reaction solution was filtered, and the filter cake was washed with water for 3 times (10.0 mL x 3), and then dried to obtain the title compound as a crude product (666 mg, crude), which was directly used in the next step without purification.

The structure characterization data is as follows:
ESI-MS (m/z): 1046.5 (M+H)⁺.

### Step 4: preparation of (6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-2-((S)-2-aminopropanamido)-5-tert-butoxy-5-oxopentanamido)-4-fluorobenzoate (DL-B-140-4)

(6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-2-((S)-2-(((9H-fluoren-9-yl)methoxy)carbonyl)amino)propanamido)-5-tert-butoxy-5-oxopentanamido)-4-fluorobenzoate (540 mg, 516 µmol) was dissolved in DMF (5.00 µmol), DBU (62.9 mg, 413 µmol, 62.2 µL) was added, and then the reaction solution was stirred for half an hour at 25°C. The reaction solution was directly used in the next step without treatment.

### Step 5: preparation of tert-butyl (5R,8S,11S)-11-((5-((((6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl)oxy)carbonyl)-2-fluorophenyl)carbamoyl)-1-(9H-fluoren-9-yl)-5-isobutyl-8-methyl-3,6,9-trioxo-4,7,10-triazatetradecane-14-carboxylate (DL-B-140-5)

(((9H-fluoren-9-yl)methoxy)carbonyl)- D-leucine (182 mg, 516 µmol), EDCI (148 mg, 774 µmol) and HOBt (105 mg, 777 µmol) were successively added into the reaction solution obtained in the previous step, and then the reaction solution was stirred for 2 h at 25°C. The reaction solution was poured into water (50.0 mL), and then was stirred for additional half an hour. The reaction solution was filtered, and the filter cake was washed with water for 3 times (10.0 mL x 3) and then dried to obtain the title compound as a crude product, which was purified by slurrying (petroleum ether/ethyl acetate = 1/1, 8.00 mL, 2 h) and then filtered and dried again to obtain the title compound (450 mg, 334 µmol).

The structure characterization data is as follows:
ESI-MS (m/z): 1159.3 (M+H)⁺.

### Step 6: preparation of (6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-2-((S)-2-((R)-2-amino-4-methylpentanamido)propanamido)-5-tert-butoxy-5-oxopentanamido)-4-fluorobenzoate (DL-B-140-6)

Tert-butyl (5R,8S,11S)-11-((5-((((6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl)oxy)carbonyl)-2-fluorophenyl)carbamoyl)-1-(9H-fluoren-9-yl)-5-isobutyl-8-methyl-3,6,9-trioxo-4,7,10-triazatetradecane-14-carboxylate (400 mg, 345 µmol) was dissolved in acetonitrile (2.00 mL), DEA (130 mg, 345 µmol, 0.4 mL) was added, and then the reaction solution was stirred for half an hour at 25°C. The reaction solution was directly concentrated to obtain the title compound as a crude product (323 mg, crude), which was directly used in the next step without purification.

The structure characterization data is as follows;
ESI-MS (m/z): 937.6 (M+H)⁺.

### Step 7: preparation of (6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-2-((S)-2-((R)-2-(3,5-bis(2-(methylsulfonyl)pyrimidin-5-yl)benzamido)-4-methylpentanamido)-5-tert-butoxy-5-oxopentanamido)-4-fluorobenzoate (DL-B-140-7)

(6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-2-((S)-2-((R)-2-amino-4-methylpentanamido)propanamido)-5-tert-butoxy-5-oxopentanamido)-4-fluorobenzoate (293 mg, 313 µmol) and 3,5-bis(2-(methylsulfonyl)pyrimidin-5-yl)benzoic acid (136 mg, 313 µmol) were dissolved in DMF (5.00 mL), T3P (1.47 g, 2.31 mmol, 1.38 mL, 50% purity) and DIPEA (121 mg, 938 µmol, 163 µL) were added, and then the reaction solution was stirred for 1 h at 25°C. The reaction solution was poured into water (25.0 mL), and then stirred for additional half an hour. The reaction solution was filtered, and the filter cake was washed with water for 3 times (10.0 mL x 3) and then dried to obtain a crude product, which was purified via high performance liquid chromatography and then freeze-dried to obtain the title compound (140 mg, 100.33 µmol).

The purification method is as follows:
chromatographic column: CD04-Welch Utimate C1825*150 mm*7 µm
Mobile phase A: acetonitrile; mobile B: water (0.05% TFA)

| Time [min] | Mobile phase A [%] | Mobile phase B [%] | Flow rate [mL/min] |
|---|---|---|---|
| 0.00 | 48 | 52 | 25 |
| 13.00 | 78 | 22 | 25 |

The structure characterization data is as follows:
ESI-MS (m/z): 1354.6 (M+H)⁺.

### Step 8: preparation of (S)-4-(S)-2-(R)-2-(3,5-bis(2-(methylsulfonyl)pyrimidin-5-yl)benzamido)-4-methylpentanamido)propanamido)-5-(5-(((6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl)oxy)carbonyl)-2-fluorophenyl)amino)-5-oxovaleric acid (DL-B-140)

(6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-2-((S)-2-((R)-2-(3,5-bis(2-(methylsulfonyl)pyrimidin-5-yl)benzamido)-4-methylpentanamido)-5-tert-butoxy-5-oxopentanamido)-4-fluorobenzoate (135 mg, 99.7 µmol) was dissolved in dichloromethane (1.5 mL), trifluoroacetic acid (461 mg, 4.04 mmol, 0.3 mL) was added, and then the reaction solution was stirred for half an hour at 25°C. The reaction solution was directly concentrated to obtain a crude product, which was purified via preparative SFC supercritical fluid chromatography and then freeze-dried to obtain the title compound (77.0 mg, 59.3 µmol).

The purification method is as follows:
Chromatographic column: DAICEL CHIRALPAK IK30*250 mm*10 µm
Mobile phase A: acetonitrile; mobile B: CO₂-MeOH, (B%: 70%), (Rt = 2.194)

The structure characterization data is as follows:
ESI-MS (m/z): 1297.4 (M+H)⁺.

### Preparation example 83: Preparation of (S)-4-(3,5-bis(2-(methylsulfonyl)pyrimidin-5-yl)benzamido)-5-((S)-1-((S)-1-(5-(((6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl)oxy)carbonyl)-2-fluorophenyl)amino)-1-oxopropan-2-yl)amino)-3-methyl-1-oxobutan-2-yl)amino)-5-oxovaleric acid (DL-B-141)

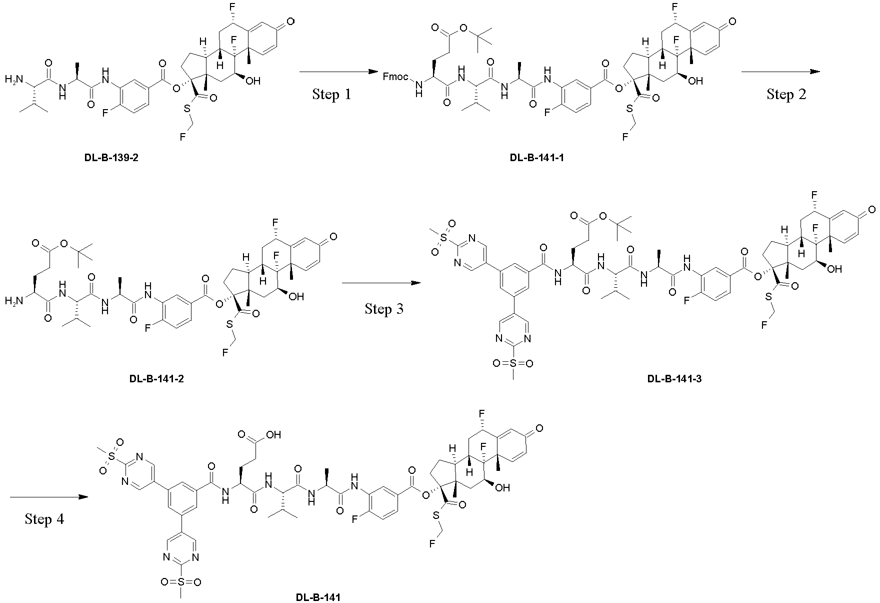

### Step 1: preparation of (6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((5S,8S,11S)-5-(3-(tert-butoxy)-3-oxopropyl)-1-(9H-fluoren-9-yl)-8-isopropyl-11-methyl-3,6,9-trioxo-2-oxa-4,7,10-triazadodecan-12-amido)-4-fluorobenzoate (DL-B-141-1)

(6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-2-((S)-2-amino-3-methylbutanamido)propanamido)-4-fluorobenzoate (468 mg, 634 µmol) and (S)-2-((((9H-fluoren-9-yl)methoxy)carbonyl)amino)-5-tert-butyloxy-5-oxovaleric acid (269 mg, 634 µmol) were dissolved in DMF (7.00 mL), HOBt (128 mg, 952 µmol) and EDCI (182 mg, 951 µmol) were added, and then the reaction solution was stirred for 1 h at 25°C. The reaction solution was poured into water (40.0 mL) and then filtered. The filter cake was dried to obtain a crude product, which was purified via a silica gel column (dichloromethane/methanol=100/1 to 10/1) and then concentrated again to obtain the title compound (380 mg, 298 µmol).

The structure characterization data is as follows:
ESI-MS (m/z): 1145.5 (M+H)⁺.

### Step 2: preparation of (6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-2-((S)-2-amino-5-(tert-butoxy)-5-oxopentanamido)-3-methylbutamido)propionamido)-4-fluorobenzoate (DL-B-141-2)

(6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((5S,8S,11S)-5-(3-(tert-butoxy)-3-oxopropyl)-1-(9H-fluoren-9-yl)-8-isopropyl-11-methyl-3,6,9-trioxo-2-oxa-4,7,10-triazadodecan-12-amido)-4-fluorobenzoate (370 mg, 323 µmol) was dissolved in acetonitrile (3.00 mL), DEA (426 mg, 5.82 mmol, 0.60 mL) was added, and then the reaction solution was stirred for 2 h at 25°C. The reaction solution was directly concentrated to obtain the title compound as a crude product (420 mg, 209 µmol), which was directly used in the next step without purification.

The structure characterization data is as follows:
ESI-MS (m/z): 923.5 (M+H)⁺.

### Step 3: preparation of (6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-2-((S)-2-((S)-2-(3,5-bis(2-(methylsulfonyl)pyrimidin-5-yl)benzamido)-5-(tert-butoxy)-5-oxopentanamido)-3-methylbutanamido)propionamido)-4-fluorobenzoate (DL-B-141-3)

(6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-2-((S)-2-amino-5-(tert-butoxy)-5-oxopentanamido)-3-methylbutamido)propionamido)-4-fluorobenzoate (290 mg, 141 µmol) and 3,5-bis(2-(methylsulfonyl)pyrimidin-5-yl)benzoic acid (61.4 mg, 141 µmol) were dissolved in DMF (5.00 mL), DIPEA (73.1 mg, 565 µmol, 98.5 µL) and HATU (161 mg, 424 µmol) were added, and then the reaction solution was stirred for 1 h at 25°C. After being filtered via silica gel, the reaction solution was added with water (100 mL), and then extracted with an ethyl acetate/methanol mixed solution for 3 times (10/1, 70.0 mL x 3). The organic phases were combined, dried over anhydrous sodium sulfate, filtered and concentrated to obtain a crude product, which was slurried using methanol/methyl tert-butyl ether (1/1, 10 mL) and then dried to obtain the title compound (200 mg, 138 µmol).

The structure characterization data is as follows:
ESI-MS (m/z):1339.4 (M+H)⁺

### Step 4: preparation of (S)-4-(3,5-bis(2-(methylsulfonyl)pyrimidin-5-yl)benzamido)-5-((S)-1-((S)-1-(5-(((6S,8S,9R,10S,11S,13S,14S,17R)-6,9-difluoro-17-((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl)oxy)carbonyl)-2-fluorophenyl)amino)-1-oxopropan-2-yl)amino)-3-methyl-1-oxobutan-2-yl)amino)-5-oxovaleric acid (DL-B-141)

(6S,8S,9R,10S,11S,13S,14S,17R)-6,9-difluoro-17-(((fluoromethyl)thio)carbonyl)-11-hydroxy-10,13-dimethyl-3-oxo-6,7,8,9,10,11,12,13,14,15,16,17-dodecahydro-3H-cyclopenta[a]phenanthren-17-yl 3-((S)-2-((S)-2-((S)-2-(3,5-bis(2-(methylsulfonyl)pyrimidin-5-yl)benzamido)-5-(tert-butoxy)-5-oxopentanamido)-3-methylbutanamido)propionamido)-4-fluorobenzoate (200 mg, 149 µmol) was dissolved in dichloromethane (3.00 mL), trifluoroacetic acid (1.54 g, 13.5 mmol, 1.00 mL) was added, and then the reaction solution was stirred for 1 h at 25°C. The reaction solution was directly concentrated to obtain a crude product, which was purified via high performance liquid chromatography and then freeze-dried to obtain the title compound (72.3 mg, 54.9 µmol).

The purification method is as follows:
chromatographic column: CD04-Welch Utimate C1825*150 mm*7 µm
Mobile phase A: acetonitrile; mobile B: water (0.05% TFA)

| Time [min] | Mobile phase A [%] | Mobile phase B [%] | Flow rate [mL/min] |
|---|---|---|---|
| 0.00 | 40 | 60 | 25 |
| 10.00 | 60 | 40 | 25 |

The structure characterization data is as follows:
ESI-MS (m/z): 1284.6 (M+H)⁺.

### Preparation examples of the antibody-drug conjugate

### Example A. Preparation of Adalimumab-G2

G2 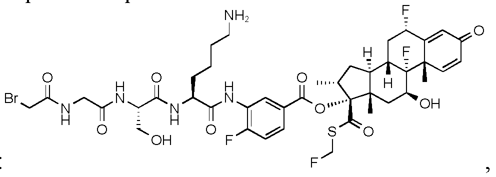 which was prepared by referring to WO2021161263A1.

### Adalimumab-G2

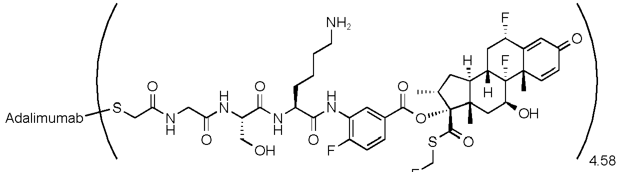

0.81 ml of Adalimumab (12.353 mg/mL) was taken, diluted with 40.5 µL of 20 mM PB+1 M EDTA (pH 7.60), and then adjusted to pH 8.0 using a 1 M Na₂HPO₄ solution. A 10 mM solution of TCEP (tris(2-carboxyethyl)phosphine, 17.19 µL, pH 7.60) was added, and the reaction mixture was homogeneously mixed and then subjected to standing for 1.5 h at room temperature. A solution of G2 (54 µL, 10 mM) that was 5.5 times in molar amount in dimethyl sulfoxide was added, and then the reaction mixture was homogeneously mixed and subjected to standing for 18 h at room temperature. After that, a NAP-5 gel column (Cytova) was used to replace the buffer solution with a 20 mM histidine buffer solution having a pH of 6.0 to obtain an antibody-drug conjugate (i.e., Adalimumab-G2). By mass spectrometry, the DAR value was measured as 4.58.

### Example 1. Preparation of Adalimumab-A-01

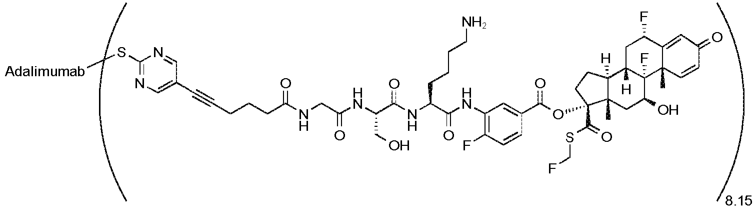

0.14 ml of Adalimumab (14.3 mg/mL) was taken, diluted with 7 µL of 20 mM PB+1 M EDTA(pH 7.60), and then adjusted to pH 7.62 using a 1 M Na₂HPO₄ solution. A 10 mM solution of TCEP (tris(2-carboxyethyl)phosphine, 7.57 µL, pH 7.60) was added, and the reaction mixture was homogeneously mixed and then subjected to standing for 1.5 h at room temperature. A solution of DL-A-01 (14.2 µL, 10 mM) that was 10 times in molar amount in dimethyl sulfoxide was added, and then the reaction mixture was homogeneously mixed and subjected to standing for 2 h at room temperature. After that, a NAP-5 gel column (Cytova) was used to replace the buffer solution with a 20 mM histidine buffer solution having a pH of 6.0 to obtain an antibody-drug conjugate (i.e., Adalimumab -A-01). By mass spectrometry, the DAR value was measured as 8.15.

### Example 2. Preparation of Tocilizumab-m-A-01

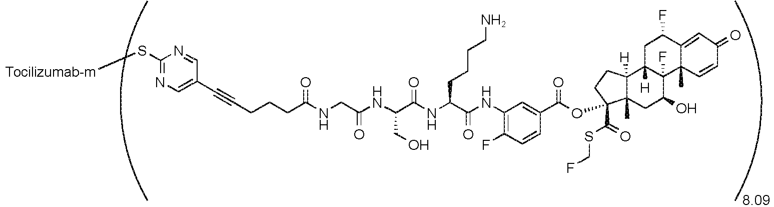

0.339 ml of Tocilizumab-m (8.85 mg/mL) was taken, diluted with 17 µL of 20 mM PB+1 M EDTA (pH 7.60), and then adjusted to pH 7.60 using a 1 M Na₂HPO₄ solution. A 10 mM solution of TCEP (tris(2-carboxyethyl)phosphine, 11.38 µL, pH 7.60) was added, and the reaction mixture was homogeneously mixed and then subjected to standing for 1.5 h at room temperature. A solution of DL-A-01 (21.33 µL,10 mM) that was 10 times in molar amount in dimethyl sulfoxide was added, and then the reaction mixture was homogeneously mixed and subjected to standing for 2 h at room temperature. After that, a NAP-5 gel column (Cytova) was used to replace the buffer solution with a 20 mM histidine buffer solution having a pH of 6.0 to obtain an antibody-drug conjugate (i.e., Tocilizumab-m-A-01). By mass spectrometry, the DAR value was measured as 8.09.

### Example 3. Preparation of Adalimumab-A-03

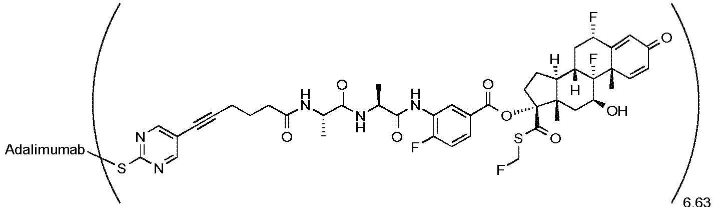

0.21 ml of Adalimumab (14.3 mg/mL) was taken, diluted with 20.5 µL of 20 mM PB+1 M EDTA(pH 7.60), and then adjusted to pH 7.60 using a 1 M Na₂HPO₄ solution. A 10 mM solution of TCEP (tris(2-carboxyethyl)phosphine, 11.38 µL, pH 7.60) was added, and the reaction mixture was homogeneously mixed and then subjected to standing for 1.5 h at room temperature. A solution of DL-A-03 (20.9 µL, 10 mM) that was 10 times in molar amount in dimethyl sulfoxide was added, and then the reaction mixture was homogeneously mixed and subjected to standing for 2 h at room temperature. After that, a NAP-5 gel column (Cytova) was used to replace the buffer solution with a 20 mM histidine buffer solution having a pH of 6.0 to obtain an antibody-drug conjugate (i.e., Adalimumab-A-03). By mass spectrometry, the DAR value was measured as 6.63.

### Example 4. Preparation of Adalimumab-A-05

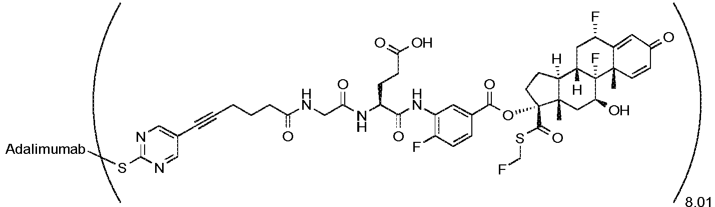

0.35 ml of Adalimumab (14.3 mg/mL) was taken, diluted with 17.5 µL of 20 mM PB+1 M EDTA (pH 7.60), and then adjusted to pH 7.67 using a 1 M Na₂HPO₄ solution. A 10 mM solution of TCEP (tris(2-carboxyethyl)phosphine, 18.91 µL, pH 7.60) was added, and the reaction mixture was homogeneously mixed and then subjected to standing for 1.5 h at room temperature. A solution of DL-A-05 (34.73 µL, 10 mM) that was 10 times in molar amount in dimethyl sulfoxide was added, and then the reaction mixture was homogeneously mixed and subjected to standing for 2 h at room temperature. After that, a NAP-5 gel column (Cytova) was used to replace the buffer solution with a 20 mM histidine buffer solution having a pH of 6.0 to obtain an antibody-drug conjugate (i.e., Adalimumab-A-05). By mass spectrometry, the DAR value was measured as 8.01.

### Example 5. Preparation of Tocilizumab-m-A-05

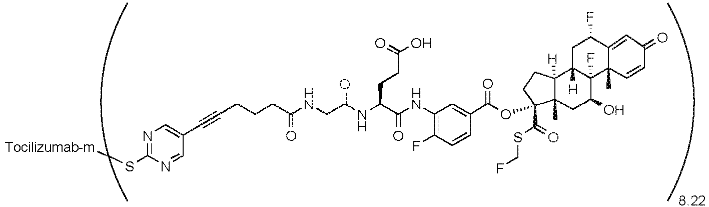

0.565 ml of Tocilizumab-m (8.85 mg/mL) was taken, diluted with 28.2 µL of 20 mM PB+1 M EDTA (pH 7.60), and then adjusted to pH 7.60 using a 1 M Na₂HPO₄ solution. A 10 mM solution of TCEP (tris(2-carboxyethyl)phosphine, 18.96 µL, pH 7.60) was added, and the reaction mixture was homogeneously mixed and then subjected to standing for 1.5 h at room temperature. A solution of DL-A-05 (34.83 µL, 10 mM) that was 10 times in molar amount in dimethyl sulfoxide was added, and then the reaction mixture was homogeneously mixed and subjected to standing for 2 h at room temperature. After that, a NAP-5 gel column (Cytova) was used to replace the buffer solution with a 20 mM histidine buffer solution having a pH of 6.0 to obtain an antibody-drug conjugate (i.e., Tocilizumab-m-A-05). By mass spectrometry, the DAR value was measured as 8.22.

### Example 6. Preparation of Adalimumab-A-47

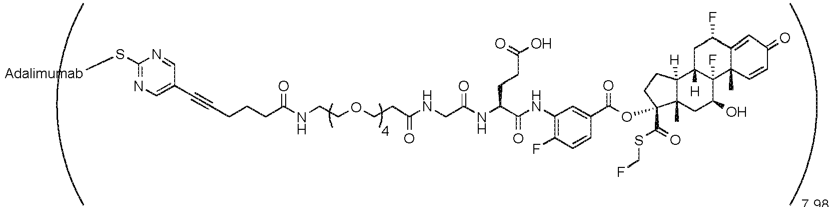

0.21 ml of Adalimumab (14.3 mg/mL) was taken, diluted with 20.5 µL of 20 mM PB+1 M EDTA(pH 7.60), and then adjusted to pH 7.60 using a 1 M Na₂HPO₄ solution. A 10 mM solution of TCEP (tris(2-carboxyethyl)phosphine, 11.38 µL, pH 7.60) was added, and the reaction mixture was homogeneously mixed and then subjected to standing for 1.5 h at room temperature. A solution of DL-A-47 (20.9 µL, 10 mM) that was 10 times in molar amount in dimethyl sulfoxide was added, and then the reaction mixture was homogeneously mixed and subjected to standing for 18 h at room temperature. After that, a NAP-5 gel column (Cytova) was used to replace the buffer solution with a 20 mM histidine buffer solution having a pH of 6.0 to obtain an antibody-drug conjugate (i.e., Adalimumab-A-47). By mass spectrometry, the DAR value was measured as 7.98.

### Example 7 Preparation of Tocilizumab-m-A-47

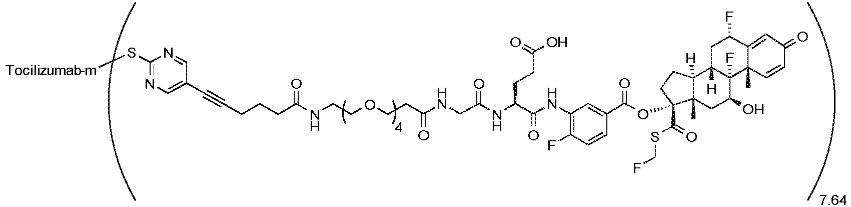

0.565 ml of Tocilizumab-m (8.85 mg/mL) was taken, diluted with 28.2 µL of 20 mM PB+1 M EDTA (pH 7.60), and then adjusted to pH 7.60 using a 1 M Na₂HPO₄ solution. A 10 mM solution of TCEP (tris(2-carboxyethyl)phosphine, 18.91 µL, pH 7.60) was added, and the reaction mixture was homogeneously mixed and then subjected to standing for 1.5 h at room temperature. A solution of DL-A-47 (34.74 µL, 10 mM) that was 10 times in molar amount in dimethyl sulfoxide was added, and then the reaction mixture was homogeneously mixed and subjected to standing for 2 h at room temperature. After that, a NAP-5 gel column (Cytova) was used to replace the buffer solution with a 20 mM histidine buffer solution having a pH of 6.0 to obtain an antibody-drug conjugate (i.e., Tocilizumab-m-A-47). By mass spectrometry, the DAR value was measured as 7.64.

### Example 8. Preparation of Adalimumab-A-48

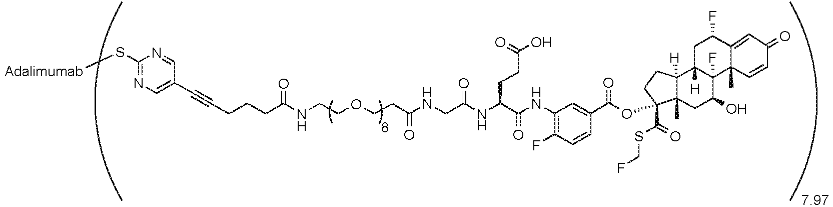

0.25ml of Adalimumab (12.1 mg/mL) was taken, diluted with 22.4 µL of 20 mM PB+1 M EDTA(pH 7.60), and then adjusted to pH 7.60 using a 1 M Na₂HPO₄ solution. A 10 mM solution of TCEP (tris(2-carboxyethyl)phosphine, 11.38 µL, pH 7.60) was added, and the reaction mixture was homogeneously mixed and then subjected to standing for 1.5 h at room temperature. A solution of DL-A-48 (21.78 µL, 10 mM) that was 10 times in molar amount in dimethyl sulfoxide was added, and then the reaction mixture was homogeneously mixed and subjected to standing for 2 h at room temperature. After that, a NAP-5 gel column (Cytova) was used to replace the buffer solution with a 20 mM histidine buffer solution having a pH of 6.0 to obtain an antibody-drug conjugate (i.e., Adalimumab-A-48). By mass spectrometry, the DAR value was measured as 7.97.

### Example 9. Preparation of Tocilizumab-m-A-48

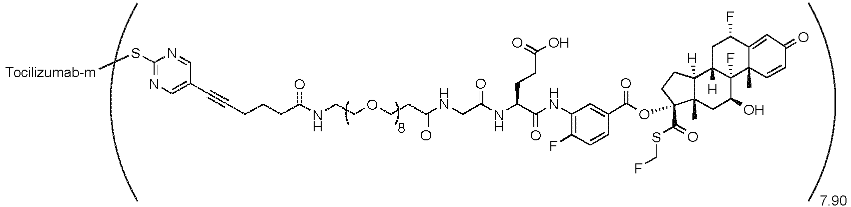

0.226 ml of Tocilizumab-m (8.85 mg/mL) was taken, diluted with 21.3 µL of 20 mM PB+1 M EDTA (pH 7.60), and then adjusted to pH 7.60 using a 1 M Na₂HPO₄ solution. A 10 mM solution of TCEP (tris(2-carboxyethyl)phosphine, 7.56 µL, pH 7.60) was added, and the reaction mixture was homogeneously mixed and then subjected to standing for 1.5 h at room temperature. A solution of DL-A-48 (14.47 µL, 10 mM) that was 10 times in molar amount in dimethyl sulfoxide was added, and then the reaction mixture was homogeneously mixed and subjected to standing for 2 h at room temperature. After that, a NAP-5 gel column (Cytova) was used to replace the buffer solution with a 20 mM histidine buffer solution having a pH of 6.0 to obtain an antibody-drug conjugate (i.e., Tocilizumab-m-A-48). By mass spectrometry, the DAR value was measured as 7.90.

### Example 10. Preparation of Adalimumab-A-49

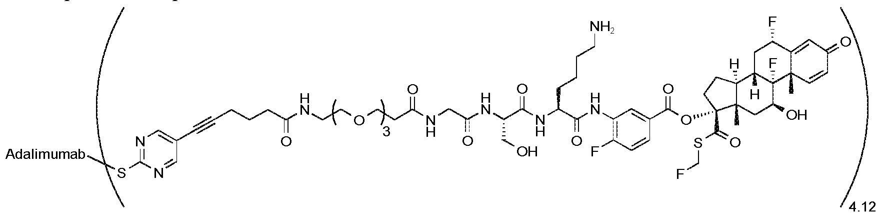

0.21ml of Adalimumab (14.3 mg/mL) was taken, diluted with 20.5 µL of 20 mM PB+1 M EDTA(pH 7.60), and then adjusted to pH 7.60 using a 1 M Na₂HPO₄ solution. A 10 mM solution of TCEP (tris(2-carboxyethyl)phosphine, 11.38 µL, pH 7.60) was added, and the reaction mixture was homogeneously mixed and then subjected to standing for 1.5 h at room temperature. A solution of DL-A-49 (21.33 µL,10 mM) that was 10 times in molar amount in dimethyl sulfoxide was added, and then the reaction mixture was homogeneously mixed and subjected to standing for 2 h at room temperature. After that, a NAP-5 gel column (Cytova) was used to replace the buffer solution with a 20 mM histidine buffer solution having a pH of 6.0 to obtain an antibody-drug conjugate (i.e., Adalimumab-A-49). By mass spectrometry, the DAR value was measured as 4.12.

### Example 11. Preparation of Adalimumab-A-50

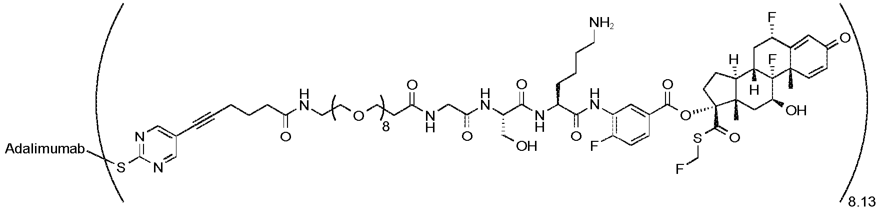

0.21ml of Adalimumab (14.3 mg/mL) was taken, diluted with 20.5 µL of 20 mM PB+1 M EDTA(pH 7.60), and then adjusted to pH 7.60 using a 1 M Na₂HPO₄ solution. A 10 mM solution of TCEP (tris(2-carboxyethyl)phosphine, 11.38 µL, pH 7.60) was added, and the reaction mixture was homogeneously mixed and then subjected to standing for 1.5 h at room temperature. A solution of DL-A-50 (21.3 µL, 10 mM) that was 10 times in molar amount in dimethyl sulfoxide was added, and then the reaction mixture was homogeneously mixed and subjected to standing for 2 h at room temperature. After that, a NAP-5 gel column (Cytova) was used to replace the buffer solution with a 20 mM histidine buffer solution having a pH of 6.0 to obtain an antibody-drug conjugate (i.e., Adalimumab-A-50). By mass spectrometry, the DAR value was measured as 8.13.

### Example 12. Preparation of Tocilizumab-m-A-50

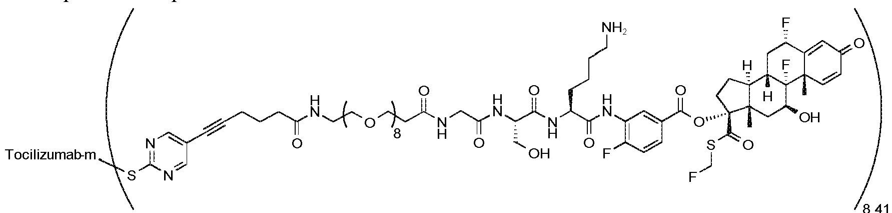

0.565 ml of Tocilizumab-m (8.85 mg/mL) was taken, diluted with 28.2 µL of 20 mM PB+1 M EDTA (pH 7.60), and then adjusted to pH 7.60 using a 1 M Na₂HPO₄ solution. A 10 mM solution of TCEP (tris(2-carboxyethyl)phosphine, 18.91 µL, pH 7.60) was added, and the reaction mixture was homogeneously mixed and then subjected to standing for 1.5 h at room temperature. A solution of DL-A-50 (35.45 µL, 10 mM) that was 10 times in molar amount in dimethyl sulfoxide was added, and then the reaction mixture was homogeneously mixed and subjected to standing for 2 h at room temperature. After that, a NAP-5 gel column (Cytova) was used to replace the buffer solution with a 20 mM histidine buffer solution having a pH of 6.0 to obtain an antibody-drug conjugate (i.e., Tocilizumab-m-A-50). By mass spectrometry, the DAR value was measured as 8.41.

### Example 13. Preparation of Adalimumab-A-139

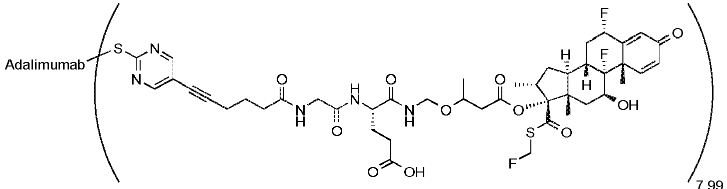

0.25ml of Adalimumab (12.1 mg/mL) was taken, diluted with 22.4 µL of 20 mM PB+1 M EDTA(pH 7.60), and then adjusted to pH 7.60 using a 1 M Na₂HPO₄ solution. A 10 mM solution of TCEP (tris(2-carboxyethyl)phosphine, 11.38 µL, pH 7.60) was added, and the reaction mixture was homogeneously mixed and then subjected to standing for 1.5 h at room temperature. A solution of DL-A-139 (21.33 µL,10 mM) that was 10 times in molar amount in dimethyl sulfoxide was added, and then the reaction mixture was homogeneously mixed and subjected to standing for 2 h at room temperature. After that, a NAP-5 gel column (Cytova) was used to replace the buffer solution with a 20 mM histidine buffer solution having a pH of 6.0 to obtain an antibody-drug conjugate (i.e., Adalimumab-A-139). By mass spectrometry, the DAR value was measured as 7.99.

### Example 14. Preparation of Tocilizumab-wt-A-139

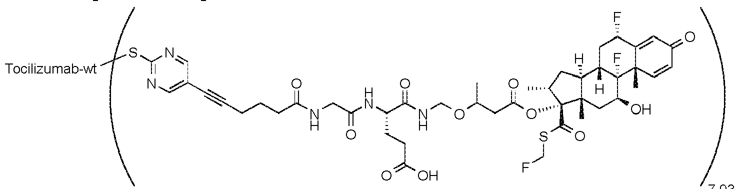

0.2 ml of Tocilizumab (15 mg/mL) was taken, diluted with 20 µL of 20 mM PB+1 M EDTA (pH 7.60), and then adjusted to pH 7.60 using a 1 M Na₂HPO₄ solution. A 10 mM solution of TCEP (tris(2-carboxyethyl)phosphine, 11.38 µL, pH 7.60) was added, and the reaction mixture was homogeneously mixed and then subjected to standing for 1.5 h at room temperature. A solution of DL-A-139 (21.33 µL, 10 mM) that was 10 times in molar amount in dimethyl sulfoxide was added, and then the reaction mixture was homogeneously mixed and subjected to standing for 2 h at room temperature. After that, a NAP-5 gel column (Cytova) was used to replace the buffer solution with a 20 mM histidine buffer solution having a pH of 6.0 to obtain an antibody-drug conjugate (i.e., Tocilizumab-wt-A-139). By mass spectrometry, the DAR value was measured as 7.93.

### Example 15. Preparation of Adalimumab-B-07

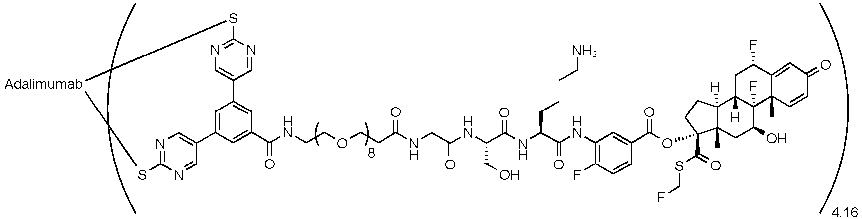

0.14 ml of Adalimumab (14.3 mg/mL) was taken, diluted with 7 µL of 20 mM PB+1 M EDTA(pH 7.60), and then adjusted to pH 7.62 using a 1 M Na₂HPO₄ solution. A 10 mM solution of TCEP (tris(2-carboxyethyl)phosphine, 7.57 µL, pH 7.60) was added, and the reaction mixture was homogeneously mixed and then subjected to standing for 1.5 h at room temperature. A solution of DL-B-07' (8.51 µL, 10 mM) that was 6 times in molar amount in dimethyl sulfoxide was slowly added, and then the reaction mixture was homogeneously mixed and subjected to standing for 18 h at room temperature. After that, a NAP-5 gel column (Cytova) was used to replace the buffer solution with a 20 mM histidine buffer solution having a pH of 6.0 to obtain an antibody-drug conjugate (i.e., Adalimumab-B-07). By mass spectrometry, the DAR value was measured as 4.16.

**Table 1. Measured molecular weight and DAR calculation of Adalimumab-B-07**

| Load | MW Exp. | Maximum signal intensity | Percentage | DAR |
|---|---|---|---|---|
| Unconjugated antibody | 145185.99 | 0.00 | 0.00% | 4.16 |
| DAR1 | 146718.63 | 0.00 | 0.00% | |
| DAR2 | 148236.63 | 0.00 | 0.00% | |
| DAR3 | 149754.63 | 10.14 | 10.75% | |
| DAR4 | 151272.63 | 60.95 | 64.59% | |
| DAR5 | 152795.92 | 21.08 | 22.33% | |
| DAR6 | 154325.07 | 2.20 | 2.33% | |
| DAR7 | 155845.07 | 0.00 | 0.00% | |
| DAR8 | 157365.07 | 0.00 | 0.00% | |

| | | | | |
|---|---|---|---|---|
| LC-MS molecular weight analysis was performed on the conjugated ADC samples. | | | | |

Chromatographic measurement conditions:
Liquid chromatography column: ACQUITY UPLC MAbPac BEH SEC;
Mobile phase A: 20 mM NH4Ac;
Flow rate: 0.1 ml/min;
Temperature of sample chamber: 8°C;
Column temperature: 60°C;
Injection volume: 2 µl;

| | | | | | | |
|---|---|---|---|---|---|---|
| Time (min) | 2 | 20 | 22 | 25 | 26 | 30 |
| Mobile phase A (vol.%) | 75 | 20 | 5 | 5 | 75 | 75 |
| Mobile phase B (vol.%) | 25 | 80 | 95 | 95 | 25 | 25 |

### Conditions for mass spectrum measurement:

Mass spectromter model: AB Sciex Triple TOF 5600+;
GS155; GS255; CUR 30; TEM 450; ISVF5500; DP75; CE5; Accumulation time 0.5s; m/z900-7000; Time bins to sum 40.

### Example 16. Preparation of Tocilizumab-m-B-07

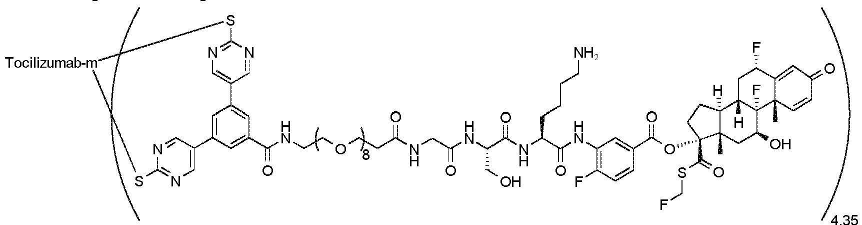

0.226 ml of Tocilizumab-m (8.85 mg/mL) was taken, diluted with 21 µL of 20 mM PB+1 M EDTA (pH 7.60), and then adjusted to pH 7.60 using a 1 M Na₂HPO₄ solution. A 10 mM solution of TCEP (tris(2-carboxyethyl)phosphine, 7.59 µL, pH 7.60) was added, and the reaction mixture was homogeneously mixed and then subjected to standing for 1.5 h at room temperature. A solution of DL-B-07' (6.12 µL, 10 mM) that was 6 times in molar amount in dimethyl sulfoxide was added, and then the reaction mixture was homogeneously mixed and subjected to standing for 18 h at room temperature. After that, a NAP-5 gel column (Cytova) was used to replace the buffer solution with a 20 mM histidine buffer solution having a pH of 6.0 to obtain an antibody-drug conjugate (i.e., Tocilizumab-m-B-07). By mass spectrometry, the DAR value was measured as 4.35.

**Table 2. Measured molecular weight and DAR calculation of Tocilizumab-m-B-07**

| Load | MW_{Exp.} | Maximum signal intensity | Percentage | DAR |
|---|---|---|---|---|
| Unconjugated antibody | 144830.93 | 0.00 | 0.00% | 4.35 |
| DAR1 | 146348.93 | 0.00 | 0.00% | |
| DAR2 | 147866.93 | 0.00 | 0.00% | |
| DAR3 | 149392.54 | 1.22 | 0.79% | |
| DAR4 | 150912.11 | 99.93 | 65.16% | |
| DAR5 | 152428.32 | 50.05 | 32.63% | |
| DAR6 | 153968.79 | 2.17 | 1.42% | |
| DAR7 | 155464.32 | 0.00 | 0.00% | |
| DAR8 | 156982.32 | 0.00 | 0.00% | |

### Example 17. Preparation of Adalimumab-B-09

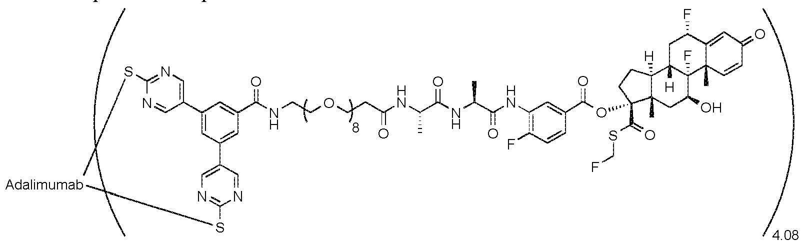

0.21 ml of Adalimumab (14.3 mg/mL) was taken, diluted with 20.5 µL of 20 mM PB+1 M EDTA(pH 7.60), and then adjusted to pH 7.60 using a 1 M Na₂HPO₄ solution. A 10 mM solution of TCEP (tris(2-carboxyethyl)phosphine, 11.38 µL, pH 7.60) was added, and the reaction mixture was homogeneously mixed and then subjected to standing for 1.5 h at room temperature. A solution of DL-B-09' (16.72 µL, 10 mM) that was 8 times in molar amount in dimethyl sulfoxide was added, and then the reaction mixture was homogeneously mixed and subjected to standing for 18 h at room temperature. After that, a NAP-5 gel column (Cytova) was used to replace the buffer solution with a 20 mM histidine buffer solution having a pH of 6.0 to obtain an antibody-drug conjugate (i.e., Adalimumab-B-09). By mass spectrometry, the DAR value was measured as 4.08.

**Table 3. Measured molecular weight and DAR calculation of Adalimumab-B-09**

| Load | MW_{EXp}. | Maximum signal intensity | Percentage | DAR |
|---|---|---|---|---|
| Unconjugated antibody | 145185.99 | 0.00 | 0.00% | 4.08 |
| DAR1 | 146574.99 | 0.00 | 0.00% | |
| DAR2 | 147974.82 | 1.44 | 0.99% | |
| DAR3 | 149373.38 | 6.82 | 4.69% | |
| DAR4 | 150761.07 | 116.35 | 80.00% | |
| DAR5 | 152141.39 | 20.84 | 14.33% | |
| DAR6 | 153541.07 | 0.00 | 0.00% | |
| DAR7 | 154939.07 | 0.00 | 0.00% | |
| DAR8 | 156337.07 | 0.00 | 0.00% | |

### Example 18: Preparation of Adalimumab-B-11

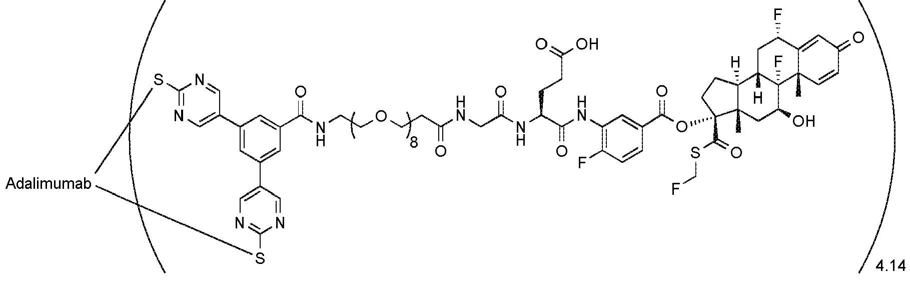

0.70 ml of Adalimumab (14.3 mg/mL) was taken, diluted with 35 µL of 20 mM PB+1 M EDTA (pH 7.60), and then adjusted to pH 7.6 using a 1 M Na₂HPO₄ solution. A 10 mM solution of TCEP (tris(2-carboxyethyl)phosphine, 37.83 µL, pH 7.60) was added, and the reaction mixture was homogeneously mixed and then subjected to standing for 1.5 h at room temperature. A solution of DL-B-11' (50.68 µL, 10 mM) that was 6 times in molar amount in dimethyl sulfoxide was slowly added, and then the reaction mixture was homogeneously mixed and subjected to standing for 18 h at room temperature. After that, a NAP-5 gel column (Cytova) was used to replace the buffer solution with a 20 mM histidine buffer solution having a pH of 6.0 to obtain an antibody-drug conjugate (i.e., Adalimumab-B-11). By mass spectrometry, the DAR value was measured as 4.14.

**Table 4. Measured molecular weight and DAR calculation of Adalimumab-B-11**

| Load | MW_{Exp}. | Maximum signal intensity | Percentage | DAR |
|---|---|---|---|---|
| Unconjugated antibody | 145204.10 | 3.10 | 0.70% | 4.14 |
| DAR1 | 146641.16 | 0.00 | 0.00% | |
| DAR2 | 148061.16 | 0.00 | 0.00% | |
| DAR3 | 149471.49 | 2.68 | 0.60% | |
| DAR4 | 150888.77 | 350.93 | 79.06% | |
| DAR5 | 152301.65 | 73.45 | 17.08% | |
| DAR6 | 153721.65 | 0.00 | 0.00% | |
| DAR7 | 155141.65 | 0.00 | 0.00% | |
| DAR8 | 156561.65 | 0.00 | 0.00% | |

| | | | | |
|---|---|---|---|---|
| LC-MS molecular weight analysis was performed on conjugated ADC samples. | | | | |

Chromatographic measurement conditions:
Liquid chromatographic column: ACQUITY UPLC MAbPac BEH SEC;
Mobile phase A: 20 mM NH₄Ac;
Flow rate: 0.1 ml/min; emperature of sample chamber: 8°C; column temperature: 60 °C; injection volume: 2 µl

| | | | | | | |
|---|---|---|---|---|---|---|
| Time (min) | 2 | 20 | 22 | 25 | 26 | 30 |
| Mobile phase A (vol.%) | 75 | 20 | 5 | 5 | 75 | 75 |
| Mobile phase B (vol.%) | 25 | 80 | 95 | 95 | 25 | 25 |

Conditions for mass spectrum measurement:
Mass spectrum model: AB Sciex Triple TOF 5600+;
GS155; GS255; CUR30; TEM450; ISVF5500; DP75; CE5; Accumulation time 0.5 s; m/z 900-7000; Time bins to sum 40.

### Example 19. Preparation of Tocilizumab-wt-B-11

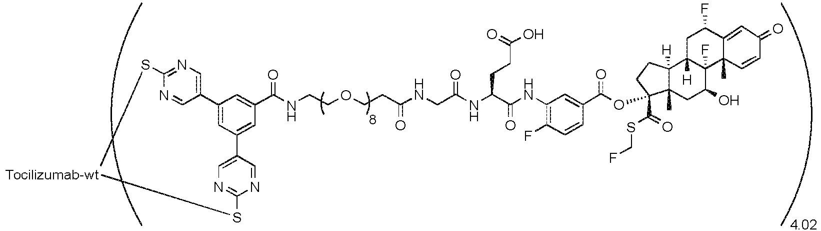

0.2 ml of Tocilizumab-wt (8.85 mg/mL) was taken, diluted with 10 µL of 20 mM PB+1 M EDTA (pH 7.60), and then adjusted to pH 7.60 using a 1 M Na₂HPO₄ solution. A 10 mM solution of TCEP (tris(2-carboxyethyl)phosphine, 11.38 µL, pH 7.60) was added, and the reaction mixture was homogeneously mixed and then subjected to standing for 1.5 h at room temperature. A solution of DL-B-11' (9.5 µL, 10 mM) that was 4.5 times in molar amount in dimethyl sulfoxide was added, and then the reaction mixture was homogeneously mixed and subjected to standing for 18 h at room temperature. After that, a NAP-5 gel column (Cytova) was used to replace the buffer solution with a 20 mM histidine buffer solution having a pH of 6.0 to obtain an antibody-drug conjugate (i.e., Tocilizumab-wt-B-11) By mass spectrometry, the DAR value was measured as 4.02.

**Table 5. Measured molecular weight and DAR calculation of Tocilizumab-wt-B-11**

| Load | MW_{EXp}. | Maximum signal intensity | Percentage | DAR |
|---|---|---|---|---|
| Unconjugated antibody | 147932.00 | 0.00 | 0.00% | 4.02 |
| DAR1 | 149365.00 | 0.00 | 0.00% | |
| DAR2 | 150818.64 | 0.99 | 5.49% | |
| DAR3 | 152240.82 | 0.80 | 4.46% | |
| DAR4 | 153683.85 | 13.06 | 72.36% | |
| DAR5 | 155107.55 | 3.19 | 17.70% | |
| DAR6 | 156549.85 | 0.00 | 0.00% | |
| DAR7 | 157982.85 | 0.00 | 0.00% | |
| DAR8 | 159415.85 | 0.00 | 0.00% | |

### Example 20. Preparation of Adalimumab-B-17

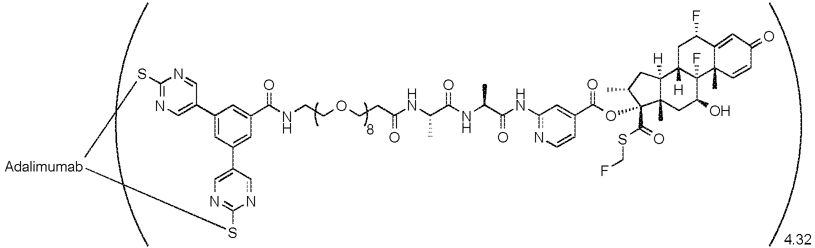

0.35 ml of Adalimumab (14.3 mg/mL) was taken, diluted with 17.5 µL of 20 mM PB+1 M EDTA (pH 7.60), and then adjusted to pH 7.6 using a 1 M Na₂HPO₄ solution. A 10 mM solution of TCEP (tris(2-carboxyethyl)phosphine, 18.92 µL, pH 7.60) was added, and the reaction mixture was homogeneously mixed and then subjected to standing for 1.5 h at room temperature. A solution of DL-B-17' (21.05 µL, 10 mM) that was 6 times in molar amount in dimethyl sulfoxide was added, and then the reaction mixture was homogeneously mixed and subjected to standing for 18 h at room temperature. After that, a NAP-5 gel column (Cytova) was used to replace the buffer solution with a 20 mM histidine buffer solution having a pH of 6.0 to obtain an antibody-drug conjugate (i.e., Adalimumab-B-17). By mass spectrometry, the DAR value was measured as 4.32.

**Table 6. Measured molecular weight and DAR calculation of Adalimumab-B-17**

| Load | MW_{Exp}. | Maximum signal intensity | Percentage | DAR |
|---|---|---|---|---|
| Unconjugated antibody | 145204.31 | 0.00 | 0.00% | 4.32 |
| DAR1 | 146590.31 | 0.00 | 0.00% | |
| DAR2 | 147976.31 | 0.00 | 0.00% | |
| DAR3 | 149362.31 | 0.00 | 0.00% | |
| DAR4 | 150748.13 | 941.00 | 75.65% | |
| DAR5 | 152137.46 | 274.90 | 22.10% | |
| DAR6 | 153523.46 | 3.42 | 0.28% | |
| DAR7 | 154909.46 | 0.00 | 0.00% | |
| DAR8 | 156132.65 | 24.50 | 1.97% | |

### Example 21. Preparation of Adalimumab-B-65

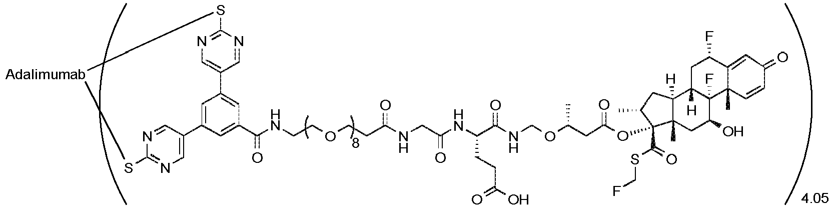

0.248 ml of Adalimumab (12.1 mg/mL) was taken, diluted with 12.4 µL of 20 mM PB+1 M EDTA (pH 7.60), and then adjusted to pH 7.60 using a 1 M Na₂HPO₄ solution. A 10 mM solution of TCEP (tris(2-carboxyethyl)phosphine, 11.38 µL, pH 7.60) was added, and the reaction mixture was homogeneously mixed and then subjected to standing for 1.5 h at room temperature. A solution of DL-B-65' (13.58 µL, 10 mM) that was 6.5 times in molar amount in dimethyl sulfoxide was added, and then the reaction mixture was homogeneously mixed and subjected to standing for 18 h at room temperature. After that, a NAP-5 gel column (Cytova) was used to replace the buffer solution with a 20 mM histidine buffer solution having a pH of 6.0 to obtain an antibody-drug conjugate (i.e., Adalimumab-B-65). By mass spectrometry, the DAR value was measured as 4.05.

**Table 7. Measured molecular weight and DAR calculation of Adalimumab-B-65**

| Load | MW_{Exp}. | Maximum signal intensity | Percentage | DAR |
|---|---|---|---|---|
| Unconjugated antibody | 148040.83 | 0.00 | 0.00% | 4.05 |
| DAR1 | 149465.83 | 0.00 | 0.00% | |
| DAR2 | 150890.83 | 0.70 | 1.89% | |
| DAR3 | 152353.75 | 0.47 | 1.28% | |
| DAR4 | 153781.47 | 31.94 | 86.64% | |
| DAR5 | 155208.98 | 3.76 | 10.19% | |
| DAR6 | 156633.98 | 0.00 | 0.00% | |
| DAR7 | 158058.98 | 0.00 | 0.00% | |
| DAR8 | 159483.98 | 0.00 | 0.00% | |

### Example 22. Preparation of Tocilizumab-wt-B-65

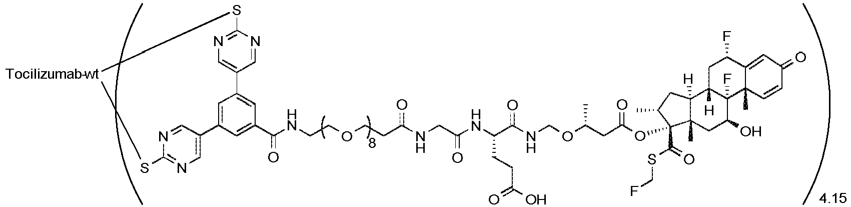

0.2 ml of Tocilizumab-wt (15 mg/mL) was taken, diluted with 10 µL of 20 mM PB+1 M EDTA(pH 7.60), and then adjusted to pH 7.60 using a 1 M Na₂HPO₄ solution. A 10 mM solution of TCEP (tris(2-carboxyethyl)phosphine, 11.38 µL, pH 7.60) was added, and the reaction mixture was homogeneously mixed and then subjected to standing for 1.5 h at room temperature. A solution of DL-B-65' (13.58 µL, 10 mM) that was 6.5 times in molar amount in dimethyl sulfoxide was added, and then the reaction mixture was homogeneously mixed and subjected to standing for 18 h at room temperature. After that, a NAP-5 gel column (Cytova) was used to replace the buffer solution with a 20 mM histidine buffer solution having a pH of 6.0 to obtain an antibody-drug conjugate (i.e., Adalimumab-wt-B-65). By mass spectrometry, the DAR value was measured as 4.15.

**Table 8. Measured molecular weight and DAR calculation of Tocilizumab-wt-B-65**

| Load | MW_{Exp}. | Maximum signal intensity | Rate | DAR |
|---|---|---|---|---|
| Unconjugated antibody | 147932.00 | 0.00 | 0.00% | 4.15 |
| DAR1 | 149357.00 | 0.00 | 0.00% | |
| DAR2 | 150782.00 | 0.00 | 0.00% | |
| DAR3 | 152209.91 | 0.32 | 0.96% | |
| DAR4 | 153641.06 | 27.95 | 83.25% | |
| DAR5 | 155062.09 | 5.30 | 15.80% | |
| DAR6 | 156487.09 | 0.00 | 0.00% | |
| DAR7 | 157912.09 | 0.00 | 0.00% | |
| DAR8 | 159337.09 | 0.00 | 0.00% | |

### Example 23. Preparation of Adalimumab-B-126

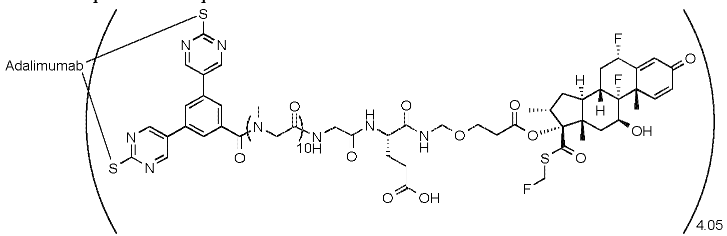

2.752 ml of Adalimumab (10.9 mg/mL) was taken, diluted with 137 µL of 20 mM PB+1 M EDTA (pH 7.60), and then adjusted to pH 7.60 using a 1 M Na₂HPO₄ solution. A 10 mM solution of TCEP (tris(2-carboxyethyl)phosphine, 113.8 µL, pH 7.60) was added, and the reaction mixture was homogeneously mixed and then subjected to standing for 1.5 h at room temperature. A solution of DL-B-126' (125.4 µL, 10 mM) that was 6 times in molar amount in dimethyl sulfoxide was slowly added, and then the reaction mixture was homogeneously mixed and subjected to standing for 18 h at room temperature. After that, a NAP-5 gel column (Cytova) was used to replace the buffer solution with a 20 mM histidine buffer solution having a pH of 6.0 to obtain an antibody-drug conjugate (i.e., Adalimumab-B-126). By mass spectrometry, the DAR value was measured as 4.05.

**Table 9. Measured molecular weight and DAR calculation of Adalimumab-B-126**

| Load | MW_{Exp}. | Maximum signal intensity | Percentage | DAR |
|---|---|---|---|---|
| Unconjugated antibody | 148,123.69 | 0.00 | 0.00% | 4.05 |
| DAR1 | 149,821.69 | 0.00 | 0.00% | |
| DAR2 | 151,519.69 | 0.00 | 0.00% | |
| DAR3 | 153,217.69 | 0.00 | 0.00% | |
| DAR4 | 154,898.19 | 23.21 | 95.14% | |
| DAR5 | 156,596.19 | 1.18 | 4.86% | |
| DAR6 | 158,294.19 | 0.00 | 0.00% | |
| DAR7 | 159,992.19 | 0.00 | 0.00% | |
| DAR8 | 161,690.19 | 0.00 | 0.00% | |

### Example 24. Preparation of Tocilizumab-wt-B-126

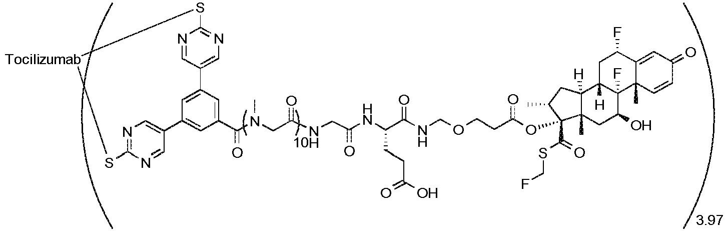

0.2 ml of Tocilizumab-wt (12.353 mg/mL) was taken, diluted with 20 µL of 20 mM PB+1 M EDTA (pH 7.60), and then adjusted to pH 7.60 using a 1 M Na₂HPO₄ solution. A 10 mM solution of TCEP (tris(2-carboxyethyl)phosphine, 11.35 µL, pH 7.60) was added, and the reaction mixture was homogeneously mixed and then subjected to standing for 1.5 h at room temperature. A solution of DL-B-126' (12.54 µL, 10 mM) that was 6 times in molar amount in dimethyl sulfoxide was slowly added, and then the reaction mixture was homogeneously mixed and subjected to standing for 18 h at room temperature. After that, a NAP-5 gel column (Cytova) was used to replace the buffer solution with a 20 mM histidine buffer solution having a pH of 6.0 to obtain an antibody-drug conjugate (i.e., Adalimumab-wt-B-126). By mass spectrometry, the DAR value was measured as 3.97.

**Table 10. Measured molecular weight and DAR calculation of Tocilizumab-wt-B-126**

| Load | MW_{Exp}. | Maximum signal intensity | Percentage | DAR |
|---|---|---|---|---|
| Unconjugated antibody | 147941.30 | 0.00 | 0.00% | 3.97 |
| DAR1 | 149640.30 | 0.00 | 0.00% | |
| DAR2 | 151339.30 | 0.00 | 0.00% | |
| DAR3 | 153038.30 | 7.33 | 4.69% | |
| DAR4 | 154740.66 | 146.33 | 93.58% | |
| DAR5 | 156439.66 | 2.70 | 1.73% | |
| DAR6 | 158138.66 | 0.00 | 0.00% | |
| DAR7 | 159837.66 | 0.00 | 0.00% | |
| DAR8 | 161536.66 | 0.00 | 0.00% | |

### Example 25. Preparation of Adalimumab-B-132

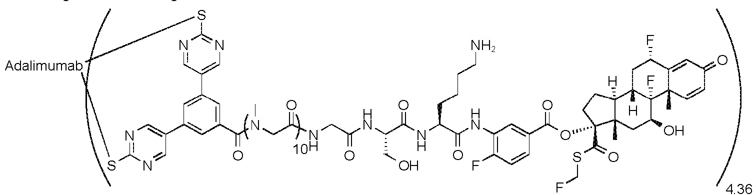

2.752 ml of Adalimumab (10.9 mg/mL) was taken, diluted with 137 µL of 20 mM PB+1 M EDTA (pH 7.60), and then adjusted to pH 7.6 using a 1 M Na₂HPO₄ solution. A 10 mM solution of TCEP (tris(2-carboxyethyl)phosphine, 113.8 µL, pH 7.60) was added, and the reaction mixture was homogeneously mixed and then subjected to standing for 1.5 h at room temperature. A solution of DL-B-132' (128.0 µL, 10 mM) that was 6 times in molar amount in dimethyl sulfoxide was slowly added, and then the reaction mixture was homogeneously mixed and subjected to standing for 18 h at room temperature. After that, a NAP-5 gel column (Cytova) was used to replace the buffer solution with a 20 mM histidine buffer solution having a pH of 6.0 to obtain an antibody-drug conjugate (i.e., Adalimumab-B-132). By mass spectrometry, the DAR value was measured as 4.36.

**Table 11. Measured molecular weight and DAR calculation of Adalimumab-B-132**

| Load | MW_{Exp}. | Maximum signal intensity | Percentage | DAR |
|---|---|---|---|---|
| Unconjugated antibody | 148,123.69 | 0.00 | 0.00% | 4.36 |
| DAR1 | 149,928.69 | 0.00 | 0.00% | |
| DAR2 | 151,733.69 | 0.00 | 0.00% | |
| DAR3 | 153,538.69 | 0.00 | 0.00% | |
| DAR4 | 155,343.69 | 14.53 | 63.61% | |
| DAR5 | 157,148.69 | 8.31 | 36.39% | |
| DAR6 | 158,953.69 | 0.00 | 0.00% | |
| DAR7 | 160,758.69 | 0.00 | 0.00% | |
| DAR8 | 162,563.69 | 0.00 | 0.00% | |

### Example 26. Preparation of Tocilizumab-wt-B-132

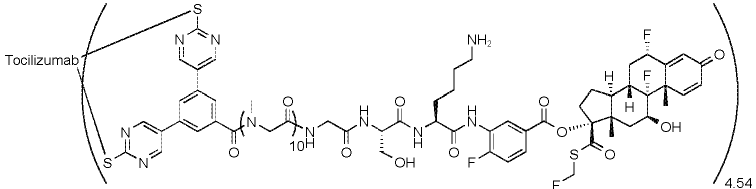

0.2 ml of Tocilizumab-wt (15 mg/mL) was taken, diluted with 20 µL of 20 mM PB+1 M EDTA(pH 7.60), and then adjusted to pH 7.60 using a 1 M Na₂HPO₄ solution. A 10 mM solution of TCEP (tris(2-carboxyethyl)phosphine, 11.35 µL, pH 7.60) was added, and the reaction mixture was homogeneously mixed and then subjected to standing for 1.5 h at room temperature. A solution of DL-B-132' (12.8 µL, 10 mM) that was 6 times in molar amount in dimethyl sulfoxide was slowly added, and then the reaction mixture was homogeneously mixed and subjected to standing for 18 h at room temperature. After that, a NAP-5 gel column (Cytova) was used to replace the buffer solution with a 20 mM histidine buffer solution having a pH of 6.0 to obtain an antibody-drug conjugate (i.e., Tocilizumab-wt-B-132). By mass spectrometry, the DAR value was measured as 4.54.

**Table 12. Measured molecular weight and DAR calculation of Tocilizumab-wt-B-132**

| Load | MW_{Exp}. | Maximum signal intensity | Percentage | DAR |
|---|---|---|---|---|
| Unconjugated antibody | 147966.62 | 0.00 | 0.00% | 4.54 |
| DAR1 | 149773.62 | 0.00 | 0.00% | |
| DAR2 | 151580.62 | 0.00 | 0.00% | |
| DAR3 | 153387.62 | 0.00 | 0.00% | |
| DAR4 | 155194.62 | 4.34 | 54.37% | |
| DAR5 | 157001.62 | 2.97 | 37.17% | |
| DAR6 | 158808.62 | 0.68 | 8.46% | |
| DAR7 | 160615.62 | 0.00 | 0.00% | |
| DAR8 | 162422.62 | 0.00 | 0.00% | |

### Example 27. Preparation of mIL6R mAb-B-126

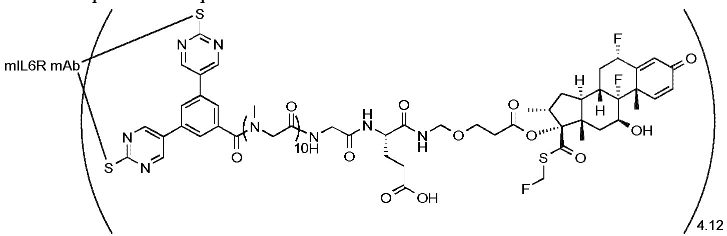

1.917 ml of mIL6R mAb antibody (11.8 mg/mL) was taken, diluted with 96 µL of 20 mM PB+1 M EDTA (pH 7.60), and then adjusted to pH 8.0 using a 1 M Na₂HPO₄ solution. A 10 mM solution of TCEP (tris(2-carboxyethyl)phosphine, 85.8 µL, pH 7.60) was added, and the reaction mixture was homogeneously mixed and then subjected to standing for 1.5 h at room temperature. A solution of DL-B-126' (126.16 µL, 10 mM) that was 8 times in molar amount in dimethyl sulfoxide was slowly added, and then the reaction mixture was homogeneously mixed and subjected to standing for 18 h at room temperature. After that, a NAP-5 gel column (Cytova) was used to replace the buffer solution with a 20 mM histidine buffer solution having a pH of 6.0 to obtain an antibody-drug conjugate (i.e., mIL6R mAb-B-126). By mass spectrometry, the DAR value was measured as 4.12.

### Example 28. Preparation of mIL6R mAb-B-132

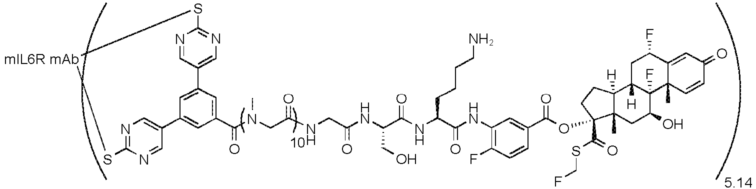

1.917 ml of mIL6R mAb antibody (11.8 mg/mL) was taken, diluted with 96 µL of 20 mM PB+1 M EDTA (pH 7.60), and then adjusted to pH 8.0 using a 1 M Na₂HPO₄ solution. A 10 mM solution of TCEP (tris(2-carboxyethyl)phosphine, 85.8 µL, pH 7.60) was added, and the reaction mixture was homogeneously mixed and then subjected to standing for 1.5 h at room temperature. A solution of DL-B-132' (128.8 µL, 10 mM) that was 8 times in molar amount in dimethyl sulfoxide was slowly added, and then the reaction mixture was homogeneously mixed and subjected to standing for 18 h at room temperature. After that, a NAP-5 gel column (Cytova) was used to replace the buffer solution with a 20 mM histidine buffer solution having a pH of 6.0 to obtain an antibody-drug conjugate (i.e., mIL6R mAb-B-132). By mass spectrometry, the DAR value was measured as 5.14.

### Example 29. Preparation of Adalimumab-B-0 1

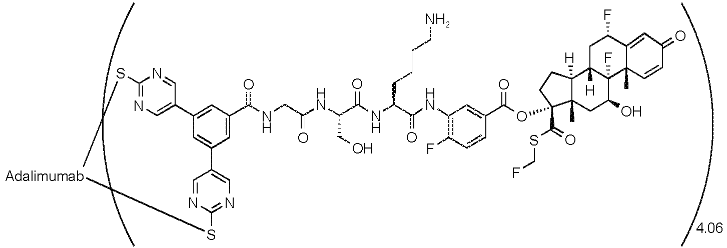

4.587 mL of Adalimumab (10.9 mg/mL) was taken, diluted with 229 µL of 20 mM PB+1 M EDTA (pH 7.60), and then adjusted to pH 7.60 using a 1 M Na₂HPO₄ solution. A 10 mM solution of TCEP (tris(2-carboxyethyl)phosphine, 189.7 µL, pH 7.60) was added, and the reaction mixture was homogeneously mixed and then subjected to standing for 1.5 h at room temperature. A solution of DL-B-01 (236 µL, 10 mM) that was 6.5 times in molar amount in dimethyl sulfoxide was added, and then the reaction mixture was homogeneously mixed and subjected to standing for 18 h at room temperature. After that, a NAP-5 gel column (Cytova) was used to replace the buffer solution with a 20 mM histidine buffer solution having a pH of 6.0 to obtain an antibody-drug conjugate (i.e., Adalimumab-B-01). By mass spectrometry, the DAR value was measured as 4.06.

**Table 13. Measured molecular weight and DAR calculation of Adalimumab-B-01**

| Load | MW_{Exp}. | Maximum signal intensity | Ratio | DAR |
|---|---|---|---|---|
| Unconjugated antibody | 148093.44 | 0.00 | 0.00% | 4.06 |
| DAR1 | 149188.36 | 0.00 | 0.00% | |
| DAR2 | 150283.27 | 0.00 | 0.00% | |
| DAR3 | 151378.19 | 4.96 | 3.49% | |
| DAR4 | 152472.59 | 124.13 | 87.38% | |
| DAR5 | 153655.02 | 12.96 | 9.12% | |
| DAR6 | 154662.59 | 0.00 | 0.00% | |
| DAR7 | 155757.59 | 0.00 | 0.00% | |
| DAR8 | 156852.59 | 0.00 | 0.00% | |

### Example 30. Preparation of Tocilizumab-wt-B-01

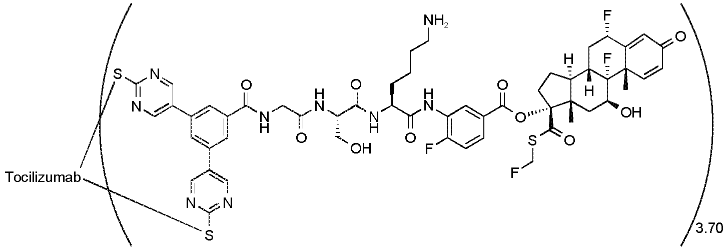

0.338 mL of Tocilizumab-wt (14.79 mg/mL) was taken, diluted with 26.9 µL of 20 mM PB+1 M EDTA (pH 7.60), and then adjusted to pH 7.60 using a 1 M Na₂HPO₄ solution. A 10 mM solution of TCEP (tris(2-carboxyethyl)phosphine, 18.91 µL, pH 7.60) was added, and the reaction mixture was homogeneously mixed and then subjected to standing for 1.5 h at room temperature. A solution of DL-B-01 (21.72 µL, 10 mM) that was 6 times in molar amount in dimethyl sulfoxide was slowly added, and then the reaction mixture was homogeneously mixed and subjected to standing for 18 h at room temperature. After that, a NAP-5 gel column (Cytova) was used to replace the buffer solution with a 20 mM histidine buffer solution having a pH of 6.0 to obtain an antibody-drug conjugate (i.e., Tocilizumab-wt-B-01). By mass spectrometry, the DAR value was measured as 3.70.

**Table 14. Measured molecular weight and DAR calculation of Tocilizumab-wt-B-01**

| Load | MW_{Exp}. | Maximum signal intensity | Percentage | DAR |
|---|---|---|---|---|
| Unconjugated antibody | 147967.79 | 18.27 | 10.52% | 3.70 |
| DAR1 | 149062.79 | 0.00 | 0.00% | |
| DAR2 | 150157.79 | 0.00 | 0.00% | |
| DAR3 | 151252.79 | 0.00 | 0.00% | |
| DAR4 | 152342.89 | 133.97 | 77.08% | |
| DAR5 | 153564.69 | 21.55 | 12.40% | |
| DAR6 | 154532.89 | 0.00 | 0.00% | |
| DAR7 | 155627.89 | 0.00 | 0.00% | |
| DAR8 | 156722.89 | 0.00 | 0.00% | |

### Example 31. Preparation of Tocilizumab-wt-B-05

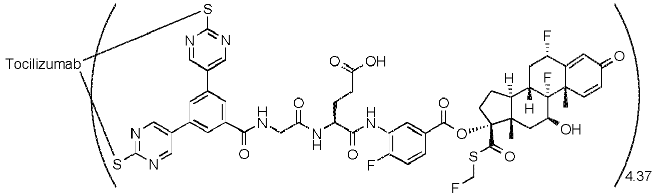

0.338 Ml of Tocilizumab-wt (14.79 mg/mL) was taken, diluted with 16.9 µL of 20 mM PB+1 M EDTA (pH 7.60), and then adjusted to pH 7.60 using a 1 M Na₂HPO₄ solution. A 10 mM solution of TCEP (tris(2-carboxyethyl)phosphine, 18.91 µL, pH 7.60) was added, and the reaction mixture was homogeneously mixed and then subjected to standing for 1.5 h at room temperature. A solution of DL-B-05 (21.05 µL, 10 mM) that was 6 times in molar amount in dimethyl sulfoxide was slowly added, and then the reaction mixture was homogeneously mixed and subjected to standing for 18 h at room temperature. After that, a NAP-5 gel column (Cytova) was used to replace the buffer solution with a 20 mM histidine buffer solution having a pH of 6.0 to obtain an antibody-drug conjugate (i.e., Tocilizumab-wt-B-05). By mass spectrometry, the DAR value was measured as 4.37.

**Table 15. Measured molecular weight and DAR calculation of Tocilizumab-wt-B-05**

| Load | MW_{Exp}. | Maximum signal intensity | Percentage | DAR |
|---|---|---|---|---|
| Unconjugated antibody | 147947.00 | 0.00 | 0.00% | 4.37 |
| DAR1 | 148957.00 | 0.00 | 0.00% | |
| DAR2 | 149967.00 | 0.00 | 0.00% | |
| DAR3 | 150977.00 | 0.00 | 0.00% | |
| DAR4 | 151987.56 | 91.30 | 68.41% | |
| DAR5 | 153163.55 | 34.52 | 25.86% | |
| DAR6 | 154245.36 | 7.64 | 5.73% | |
| DAR7 | 155017.56 | 0.00 | 0.00% | |
| DAR8 | 156027.56 | 0.00 | 0.00% | |

### Example 32 Preparation of Tocilizumab-wt-B-138

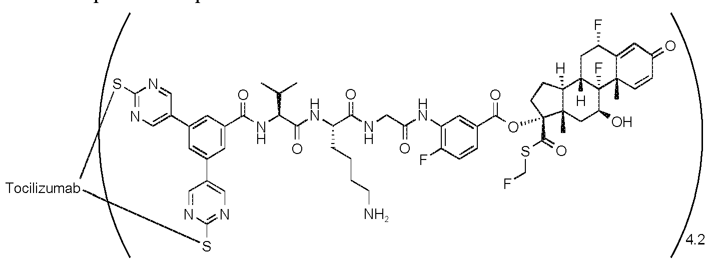

0.27 mL of Tocilizumab-wt (14.79 mg/mL) was taken, diluted with 13.5 µL of 20 mM PB+1 M EDTA (pH 7.60), and then adjusted to pH 7.60 using a 1 M Na₂HPO₄ solution. A 10 mM solution of TCEP (tris(2-carboxyethyl)phosphine, 15.17 µL, pH 7.60) was added, and the reaction mixture was homogeneously mixed and then subjected to standing for 1.5 h at room temperature. A solution of DL-B-138 (17.2 µL, 10 mM) that was 6 times in molar amount in dimethyl sulfoxide was slowly added, and then the reaction mixture was homogeneously mixed and subjected to standing for 18 h at room temperature. After that, a NAP-5 gel column (Cytova) was used to replace the buffer solution with a 20 mM histidine buffer solution having a pH of 6.0 to obtain an antibody-drug conjugate (i.e., Tocilizumab-wt-B-138). By mass spectrometry, the DAR value was measured as 4.2.

**Table 16. Measured molecular weight and DAR calculation of Tocilizumab-wt-B-138**

| Load | MW_{Exp}. | Maximum signal intensity | Percentage | DAR |
|---|---|---|---|---|
| Unconjugated antibody | 147953.08 | 0.00 | 0.00% | 4.2 |
| DAR1 | 149057.62 | 0.00 | 0.00% | |
| DAR2 | 150170.18 | 0.44 | 0.31% | |
| DAR3 | 151279.54 | 0.00 | 0.00% | |
| DAR4 | 152388.37 | 113.86 | 79.54% | |
| DAR5 | 153654.63 | 28.85 | 20.15% | |
| DAR6 | 154604.37 | 0.00 | 0.00% | |
| DAR7 | 155712.37 | 0.00 | 0.00% | |
| DAR8 | 156820.37 | 0.00 | 0.00% | |

### Example 33. Preparation of Tocilizumab-wt-B-135

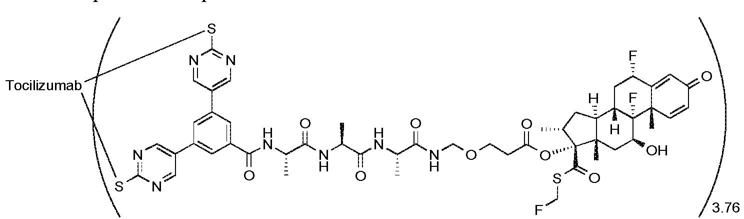

0.203 mL of Tocilizumab-wt (14.79 mg/mL) was taken, diluted with 10.1 µL of 20 mM PB+1 M EDTA (pH 7.60), and then adjusted to pH 7.60 using a 1 M Na₂HPO₄ solution. A 10 mM solution of TCEP (tris(2-carboxyethyl)phosphine, 11.38 µL, pH 7.60) was added, and the reaction mixture was homogeneously mixed and then subjected to standing for 1.5 h at room temperature. A solution of DL-B-135 (12.7 µL, 10 mM) that was 6 times in molar amount in dimethyl sulfoxide was slowly added, and then the reaction mixture was homogeneously mixed and subjected to standing for 18 h at room temperature. After that, a NAP-5 gel column (Cytova) was used to replace the buffer solution with a 20 mM histidine buffer solution having a pH of 6.0 to obtain an antibody-drug conjugate (i.e., Tocilizumab-wt-B-135). By mass spectrometry, the DAR value was measured as 3.76.

**Table 17. Measured molecular weight and DAR calculation of Tocilizumab-wt-B-135**

| Load | MW_{Exp}. | Maximum signal intensity | Rate | DAR |
|---|---|---|---|---|
| Unconjugated antibody | 147952.38 | 13.20 | 7.03% | 3.76 |
| DAR1 | 148967.65 | 0.00 | 0.00% | |
| DAR2 | 149982.92 | 0.00 | 0.00% | |
| DAR3 | 150998.19 | 0.00 | 0.00% | |
| DAR4 | 152016.35 | 167.32 | 89.10% | |
| DAR5 | 153194.66 | 7.27 | 3.87% | |
| DAR6 | 154046.35 | 0.00 | 0.00% | |
| DAR7 | 155061.35 | 0.00 | 0.00% | |
| DAR8 | 156076.35 | 0.00 | 0.00% | |

### Biological data

### Experiment example 1: GRE reporter gene test

### 1. Test method

### (1) Formulation of the compound

The compound of the present invention to be tested was formulated into a 10 mM solution with DMSO, and then subjected to 4-fold dilution at 10 concentration points using DMSO to obtain a 1000 x mother liquor, with a final test concentration of 0.000038-10µM. Dexamethasone was formulated into a 2 mM solution with DMSO, and then subjected to 3-fold dilution at 10 concentration points using DMSO to obtain a 1000 x mother liquor, with a final test concentration of 0.0001-2 µM.

### (2) Cell transfection

HEK293T cells (ATCC) were plated in a 100 mm culture dish at a density of 6*10⁶ cells/culture dish, and cultured for 16 h under the conditions of 37°C and 5% CO₂. Plasmids (pGL4.35 [luc2P/9XGAL4 UAS/Hygro], p-Bind GR Vector, Promega) and Lipofectamine^{®} 3000 (Invitrogen) were mixed, added into the culture dish, and then incubated for 5-6 h under the conditions of 37°C and 5% CO₂.

### (3) Sampling

In a 384-well plate (PerkinElmer), 25 nL of the compound mother liquor was added, and then HEK293T cells transfected with plasmids were added at a density of 18000 cells/well. The cells were incubated for 18-20 h under the conditions of 37°C and 5% CO₂.

### (4) Detection

After the incubation was ended, 25 µL of britelite plus luciferase assay reagent (PerkinElmer) was added, and then reading was performed using an Envision 2105 multiplate reader (PerkinElmer).

### (5) Data analysis

The activity was calculated using the following formula, % activity = 100 * (signal of compound well - average signal of negative control well) / (average signal of positive control well - average signal of negative control well). In the positive control well is 2 µM dexamethasone, and in the negative control well is 0.1% DMSO. Fitting calculation of EC₅₀ was performed on % activity and the logarithmic concentration of the compound using Graphpad 8.0, Y=Bottom + (Top-Bottom)/(1+10^((LogEC₅₀-X)*HillSlope)), wherein Y is % activity, and X is the logarithmic concentration of the compound.

### 2. Test results

EC₅₀ and Eₘₐₓ were obtained according to the fitting results. Details are seen in Table 18.

**Table 18. Test results of GRE reporter genes**

| Compound | EC₅₀(µM) | Eₘₐₓ(%)# |
|---|---|---|
| Dexamethasone (system positive) | 0.0035 | 100.26 |
| Preparation example 5/O-1 | 0.0002 | 87.34 |
| Preparation example 2/P-1 | 0.0014 | 91.15 |
| Preparation example 1/A-1 | 0.0002 | 102.50 |
| Preparation example 4/C-1 | 0.0009 | 102.40 |
| Preparation example 6/O-3 | 0.0004 | 87.72 |
| Preparation example 3/F-2 | 0.0012 | 124.60 |
| Preparation example 7/E-3 | 0.0012 | 97.25 |
| Preparation example 15B-1 | 0.00019 | 105.30 |
| Preparation example 16/B-13 | 0.00027 | 100.10 |
| Preparation example 17/B-25 | 0.00013 | 85.57 |
| Preparation example 18/H-1 | 0.00018 | 109.70 |
| Preparation example 19/H-5 | 0.00016 | 106.80 |

| | | |
|---|---|---|
| #: Relative to the positive control well (2 µM dexamethasone) | | |

The results show that under the test system disclosed in this application, the compounds disclosed in table 9 of this application are all superior to dexamethasone in terms of the agonistic activity (EC₅₀) on the glucocorticoid receptor. For the maximum agonistic effect (Eₘₐₓ), compounds A-1, C-1, F-2, B-1, H-1 and H-5 are superior to dexamethasone.

### Experiment example 2: GRE reporter gene test

### 1. Test method

### (1) Formulation of the compound

The compound of the present invention to be tested was formulated into a 1 mM solution with DMSO, and then subjected to 4-fold dilution at 10 concentration points using DMSO to obtain a 1000 x mother liquor, with a final test concentration of 0.0000038-1 µM. Dexamethasone was formulated into a 2 mM solution with DMSO and then subjected to 3-fold dilution at 10 concentration points using DMSO to obtain a 1000 x mother liquor, with a final test concentration of 0.0001-2 µM.

### (2) Cell transfection

HEK293T cells (ATCC) were plated in a 100 mm culture dish at a density of 6*10⁶ cells/culture dish, and cultured for 16 h under the conditions of 37°C and 5% CO₂. Plasmids (pGL4.35 [luc2P/9XGAL4 UAS/Hygro], p-Bind GR Vector, Promega) and Lipofectamine^{®} 3000 (Invitrogen) were mixed, added into the culture dish, and then incubated for 5-6 h under the conditions of 37°C and 5% CO₂.

### (3) Sampling

In a 384-well plate (PerkinElmer), 25 nL of the compound mother liquor was added, and then HEK293T cells transfected with plasmids were added at a density of 18000 cells/well (25 µL). The cells were incubated for 18-20 h under the conditions of 37°C and 5% CO₂.

### (4) Detection

After the incubation was ended, 25 µL of britelite plus luciferase assay reagent (PerkinElmer) was added, and then reading was performed using an Envision 2105 multimode reader (PerkinElmer).

### (5) Data analysis

The activity was calculated using the following formula, % activity = 100 * (signal of compound well - average signal of negative control well) / (average signal of positive control well - average signal of negative control well). In the positive control well is 2 µM dexamethasone, and in the negative control well is 0.1% DMSO. Fitting calculation of EC₅₀ was performed on % activity and the logarithmic concentration of the compound using Graphpad 8.0, Y=Bottom + (Top-Bottom)/(1+10^((LogEC₅₀-X)*HillSlope)), wherein Y is % activity, and X is the logarithmic concentration of the compound.

### 2. Test results

EC₅₀ and Eₘₐₓ were obtained according to the fitting results. Details are seen in Table 19.

**Table 19. Test results of GRE reporter genes**

| Compound | EC₅₀(µM) | Eₘₐₓ(%)# |
|---|---|---|
| Dexamethasone (system positive) | 0.0064 | 98.35 |
| Preparation example 21/D-9 | 0.0036 | 145.90 |
| Preparation example 22/D-5 | 0.0013 | 132.00 |
| Preparation example 23/D-11 | 0.0008 | 114.80 |
| Preparation example 24/D-15 | 0.0014 | 102.50 |
| Preparation example 25/D-19 | 0.0012 | 119.60 |
| Preparation example 30/A-2 | 0.0017 | 93.68 |
| Preparation example 31/F-9 | 0.0004 | 133.70 |
| Preparation example 32/F-10 | 0.0006 | 150.30 |
| Preparation example 33/F-13 | 0.0013 | 103.70 |
| Preparation example 34/F-21 | 0.0013 | 139.00 |
| Preparation example 35/E-4 | 0.0010 | 84.76 |
| Preparation example 36/B-31 | 0.00032 | 92.49 |
| Preparation example 37/B-35 | 0.0024 | 66.17 |

| | | |
|---|---|---|
| #: Relative to the positive control well (2 µM dexamethasone) | | |

The results show that under the test system disclosed in this application, the compounds disclosed in table 10 of this application are all superior to dexamethasone in terms of the agonistic activity (EC₅₀). For the maximum agonistic activity (Eₘₐₓ), compounds D-5, D-9, D-11, D-19, F-9, F-10 and F-21 are superior to dexamethasone.

### Experiment example 3: Cell activity detection of the antibody conjugate

Hela cells (Nanjing Kebai Biotechnology Co., Ltd) were cultured using 1640+10%FBS (treated by activated carbon). The Hela cells were transfected with PGL4.36 [luc2P-MMTV Hygro] (Promega) plasmids, and screened with hygromycin to sort monoclonal antibodies so as to obtain stable cells Hela-MMTV-luc2P. Human TNFα (Uniprot: P01375), mouse TNFα (Uniprot: P06804), human IL6R (Uniprot: P08887), and mouse IL6R (Uniprot: P22272) genes were entrusted to Nanjing Kingsray Biotechnology Co., Ltd. for optimized synthesis and constructed into a lentiviral vector. After being packaged in viruses, Hela-MMTV-luc2P cells were infected and subjected to double pressure screening to obtain stable cells Hela-MMTV-luc2P-hTNFα, Hela-MMTV-luc2P-mTNFα, Hela-MMTV-luc2P-hIL6R and Hela-MMTV-luc2P-mIL6R.The cells were treated by trypsinization, and added into a 96-well blank plate in a density of 1.5×10⁴/100 µL/well after counting. The antibody conjugate was subjected to gradient dilution with a culture medium and formulated into a 2-fold mother liquor, which was respectively added into cells in a density of 100µL/well. The cells were cultured for additional 48 h under the conditions of 37°C and 5% CO₂. After a luciferase substrate was added, reading was performed using a microplate reader, and the glucocorticoid receptor activation activity EC₅₀ of each antibody conjugate was calculated using four-parameter fitting.

**Table 20. Test results of cell activity detection**

| ADC (Examples) | EC₅₀ (nM) |
|---|---|
| Adalimumab-G2 (Example A, control group) | 0.1256 |
| Adalimumab-A-01 (Example 1) | 0.01528 |
| Adalimumab-A-02 (Example 4) | 0.08159 |
| Adalimumab-B-07 (Example 15) | 0.02250 |

The results show that under the test system disclosed in the present application, the antibody-drug conjugates Adalimumab-A-01, Adalimumab-A-05 and Adalimumab-B-07 have significant glucocorticoid receptor activation functions in a Hela-MMTV-luc2P-mTNFα cell level, and Adalimumab-A-01, Adalimumab-A-05 and Adalimumab-B-07 antibody-drug conjugates exhibit superior glucocorticoid receptor activation activity to the control group.

**Table 21. Test results for cell activity detection**

| ADC (Examples) | EC₅₀ (nM) |
|---|---|
| Tocilizumab-m-A-01 (Example 2) | 0.02907 |
| Tocilizumab-m-A-05 (Example 5) | 0.08288 |
| Tocilizumab-m-B-07 (Example 16) | 0.05805 |

The results show that under the test system disclosed in the present application, the antibody-drug conjugates Tocilizumab-m-A-01, Tocilizumab-m-A-05 and Tocilizumab-m-B-07 have significant glucocorticoid receptor activation functions in a Hela-MMTV-luc2P-hIL6R cell level.

### Experiment example 4: In vitro inhibition of the proliferation of mouse B cells by the glucocorticoid

According to instructions of the EasySEP mouse panB cell sorting kit (Stemcell/1984/1x100e9 cells), B cells were isolated from mouse spleen cells. After obtaining the B cells, they were resuspended in complete medium (1640+10% FBS+50uM β-mercaptoethanol), and added to a 96-well cell culture plate at 1E5 cells/100 µL per well. 4 x LPS (20 µ g/ml) was prepared, and add to the plated cells at 50 µL per well, with a final concentration of 5 µ g/ml. The glucocorticoid was diluted with complete medium in a 5-fold gradient, starting at a final concentration of 20 nM, and added to the plated cells at 50 µL per well. The cells were incubated in a 37 °C cell culture incubator for 4 days. Then PI was added to the cells for dead cell staining, and the number of live cells was detected by flow cytometry. The cell viability was calculated using the number of live cells in the untreated wells as the denominator. The the cell viability values was imported into the GraphPad Prism 8 software to calculate IC₅₀. The structure of the comparative compound 1 is as follows:

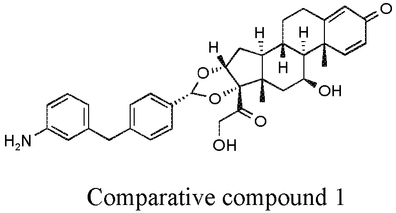

**Table 22. Inhibition of the proliferation of mouse B cells by the glucocorticoid**

| Examples | IC₅₀(nM) |
|---|---|
| A-1 | 0.032 |
| D-5 | 0.312 |
| Comparative example 1 | 0.825 |

The results show that for the in-vitro inhibitory activity on the proliferation of mouse B cells, compound A-1 of this invention has the most strongest inhibitory activity, followed by D-5, and comparative compound 1 has relatively the weakest inhibitory activity.

### Experiment example 5: In vitro activation activity of the glucocorticoid on the glucocorticoid receptor in cells

The activation activity of the glucocorticoid on glucocorticoid receptor was tested in the constructed Hela-MMTV luciferase reporter system cells. Adherent Hela-MMTV cells were digested using a Trypsin-EDTA (0.25%) solution, counted, and the cells in a required number were resuspended in complete medium (1640 + 10% FBS). The cells were then plated in a 96-well cell culture plate at 10,000 cells/100 µl. The glucocorticoid was diluted in complete medium, starting at a final concentration of 75 nM, followed by 5-fold gradient dilution, and was added to the plated cells at 100 µl per well. The cells were incubated in a cell culture incubator at 37°C for 48 hours. Then Bio-Lite luciferase assay reagent (Vazyme DD1201-03) was added to the cells at 20 µl per well, followed by homogeneously shaking for 10 minutes, and luminescence readings were obtained using a microplate reader. The data were imported into GraphPad Prism 8 software to calculate the EC₅₀ value.

**Table 23. Activation activity of the glucocorticoid on the glucocorticoid receptor in cells**

| Examples | EC₅₀(nM) |
|---|---|
| A-1 | 0.0097 |
| D-5 | 0.154 |
| D-15 | 0.074 |
| Comparative compound 1 | 0.956 |

The results show that compounds A-1, D-5 and D-15 of this invention are superior to comparative compound 1 in terms of in vitro activation activity on the glucocorticoid receptor in the cells.

### Experimental example 6: in-vivo efficacy study of the ADC molecule on the mouse chronic arthritis model

### 1. Test method

(1) Preparation of moldeling reagent: Bovine type II collagen (Chordrex, 2 mg/ml) and complete Freund's adjuvant (Chordrex, CFA, 4 mg/ml) were prepared into an emulsifier using an emulsifier in a volume ratio of 1:1, which was placed on ice for later use.
(2) Animal modeling: The test was performed using DBA/1J mice. D0 was a sensitization stage, and the modeling animals were injected with 100 µL of the emulsifier (containing bovine type II collagen+CFA) intradermally at the base of tail on day 0. D21 was a stimulation stage, and the modeling animals were injected with 100 µL of the emulsifier (containing bovine type II collagen+CFA) intradermally at the base of tail. The normal control group was injected with PBS.
(3) Grouping of animals and administration: On D29, the modeling animals were grouped according to the scores. The scoring standard is as shown in Table 24 below. The doses of model group (n=7) and mIL6R mAb group were 10 mg/kg (n=6), and the dose of mIL6R mAb-B-132 group was 10 mg/kg (n=6). Administration was performed on each group on D29 and D43, twice in total. After administration, continuous observation was conducted for body weight measurement and arthritis scoring

**Table 24. Scoring standard for arthritis**

| | |
|---|---|
| 0 score | No redness and swelling |
| 1 score | Redness and swelling of the little toe joint |
| 2 scores | Swelling of toe joints and toes |
| 3 scores | Swelling of the feet below the ankle joint |
| 4 scores | Swelling of all paws within the ankle joint |
| Counting | All 4 feet of each animal were scored, and the highest arthritis index score for each mouse was 16 scores |

### 2. Test results

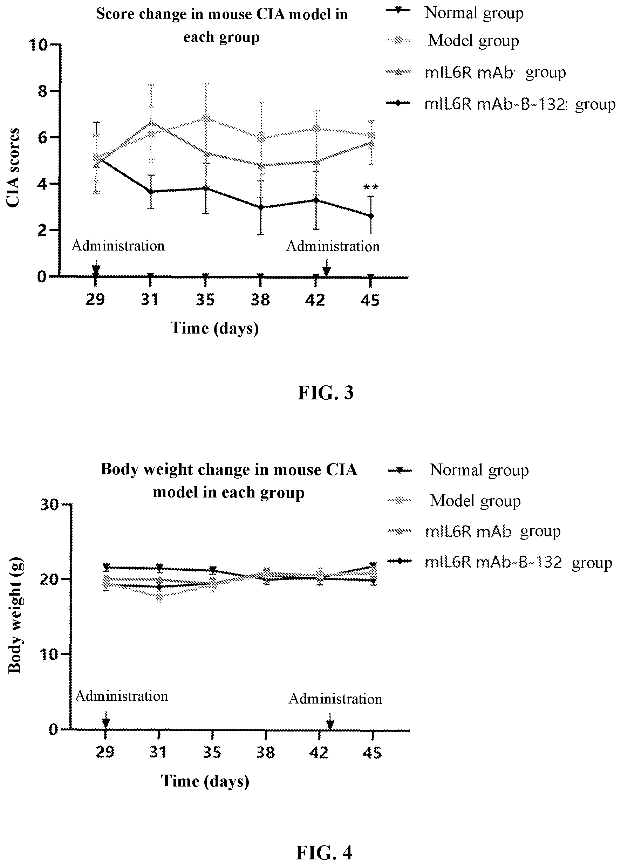

After administration, as compared with the model group, the mIL6R mAb unconjugated antibody group has a reduced arthritis score to a certain extent; and the mIL6R mAb-B-132 ADC group has a gradually reduced arthritis score. There is a statistical difference (** represents p<0.01) compared with model group on D45 (FIG. 3). The drug of the present invention has no obvious influence on the body weight of the animal (FIG. 4).

Although the specific embodiments of the present invention have been described in detail, those skilled in the art will understand that various modifications and replacements can be made to these details according to all the teachings that have been disclosed. All of these variations are included within the scope of protection of the present invention. All the scope of the present invention is given by the appended claims and any equivalents.

## Claims

1. A compound of formula I, a pharmaceutically acceptable salt, a stereisomer, a tautomer, a polymorph, a solvate, an N-oxide, an isotope labeled compound, a metabolite, or a prodrug thereof: 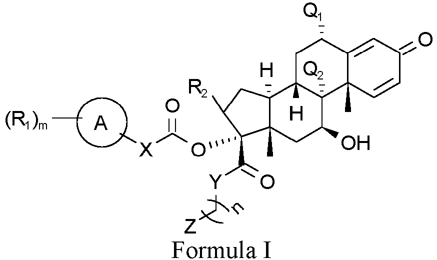 wherein,
R₁ is each independently selected from a group consisting of hydrogen, halogen, - NR^{a}R^{b}, hydroxyl, cyano, C₂₋₆ alkenyl, C₂₋₆ alkynyl, C₁₋₆ alkyl, C₁₋₆ alkyl substituted with one or more hydroxyls, C₁₋₆ alkoxy and C₁₋₆ haloalkyl; preferably, R₁ is each independently selected from a group consisting of hydrogen, halogen, -NR^{a}R^{b}, hydroxyl, C₁₋₆ alkyl, C₁₋₆ alkyl-OH and C₁₋₆ alkoxy; preferably, R₁ is each independently selected from a group consisting of hydrogen, fluorine, chlorine, amino, hydroxyl, methyl, ethyl and hydroxymethyl; preferably, R₁ is each independently selected from a group consisting of hydrogen, fluorine, amino, hydroxyl, methyl, ethyl and hydroxymethyl;
R₂ is selected from a group consisting of hydrogen, C₁₋₆ alkyl, hydroxyl and C₁₋₆ alkoxy; preferably, R₂ is selected from a group consisting of hydrogen, methyl, hydroxyl and methyoxy; preferably, R₂ is selected from a group consisting of hydrogen and methyl;
ring A is selected from a group consisting of a single bond, a C₆₋₁₀ aromatic ring and a 5-6 membered heteroaromatic ring; preferably, ring A is selected from a group consisting of a single bond, a benzene ring, and a 5-6 membered heteroaromatic ring; preferably, ring A is selected from a group consisting of a single bond, a benzene ring, a pyridine ring, a furan ring and a thiophene ring;
X is selected from a group consisting of a single bond, C₁₋₆ alkylene, C₃₋₆ cycloalkylene, C₂₋₆ alkenylidene, C₂₋₆ alkynylidene and -NR^{a}-; preferably, X is selected from a group consisting of a single bond, methylene, ethylene, propylene, butylene, cyclopropylene, cyclohexylene, ethenylidene and imino; preferably, X is selected from a group consisting of a single bond, methylene, ethylene, propylene, butylene, 1,1-cyclopropylene, 1,2-cyclopropylene, 1,4-cyclohexylene and ethenylidene;
Y is selected from a group consisting of -O- and -S-;
Z is selected from a group consisting of hydroxyl, halogen and cyano; preferably, Z is selected from a group consisting of hydroxyl, fluorine, chlorine and cyano;
Q₁ and Q₂ are each independently selected from a group consisting of hydrogen and halogen; preferably, Q₁ and Q₂ are each independently selected from a group consisting of hydrogen, fluorine and chlorine; preferably, Q₁ is selected from a group consisting of hydrogen and fluorine, and Q₂ is fluorine;
m is selected from a group consisting of 1, 2, 3, 4 and 5; preferably, m is selected from a group consisting of 1 and 2;
n is selected from a group consisting of 1 and 2; preferably, n is 1;
R^{a} and R^{b} are each independently selected from a group consisting of hydrogen and C₁₋₆ alkyl;
provided that: when R₁ is each independently selected from a group consisting of halogen, amino and methyl, m is 2, R₂ is methyl, X is a single bond, Y is -S-, Z is fluorine, n is 1, Q₁ is hydrogen or fluorine, and Q₂ is fluorine, ring A is not phenyl;
when ring A is a single bond, R₁ is not hydrogen or C₁₋₆ alkyl; and
when ring A is a 5-6 membered heteroaromatic ring, R₁ is hydrogen and R₂ is C₁₋₆ alkyl, Q₁ is not halogen.

2. The compound, pharmaceutically acceptable salt, stereisomer, tautomer, polymorph, solvate, N-oxide, isotope labeled compound, metabolite or prodrug thereof according to claim 1, wherein the compound is a compound of formula II:

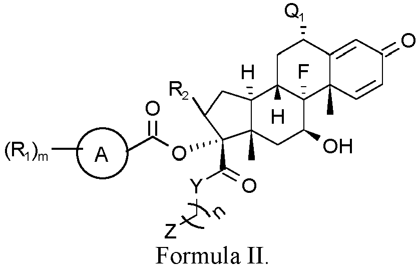

3. The compound, pharmaceutically acceptable salt, stereisomer, tautomer, polymorph, solvate, N-oxide, isotope labeled compound, metabolite or prodrug thereof according to claim 2, wherein the compound is a compound of formula III:

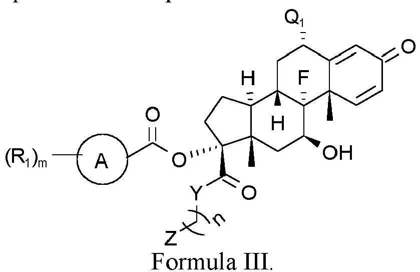

4. The compound, pharmaceutically acceptable salt, stereisomer, tautomer, polymorph, solvate, N-oxide, isotope labeled compound, metabolite or prodrug thereof according to claim 2, wherein the compound is a compound of formula IV:

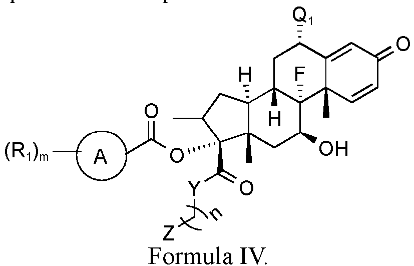

5. The compound, pharmaceutically acceptable salt, stereisomer, tautomer, polymorph, solvate, N-oxide, isotope labeled compound, metabolite or prodrug thereof according to claim 1, wherein the compound is a compound of formula V:

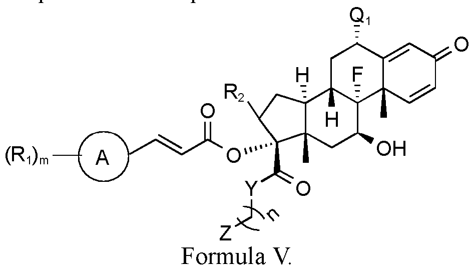

6. The compound, pharmaceutically acceptable salt, stereisomer, tautomer, polymorph, solvate, N-oxide, isotope labeled compound, metabolite or prodrug thereof according to claim 1, wherein the compound is a compound of formula VI: 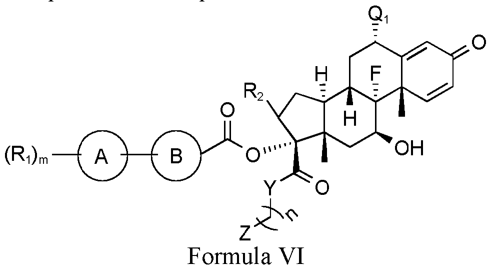 wherein,
ring B is C₃₋₆ cycloalkane, preferably cyclopropane.

7. The compound, pharmaceutically acceptable salt, stereisomer, tautomer, polymorph, solvate, N-oxide, isotope labeled compound, metabolite or prodrug thereof according to any one of claims 1-6, wherein the compound is selected from a group consisting of: 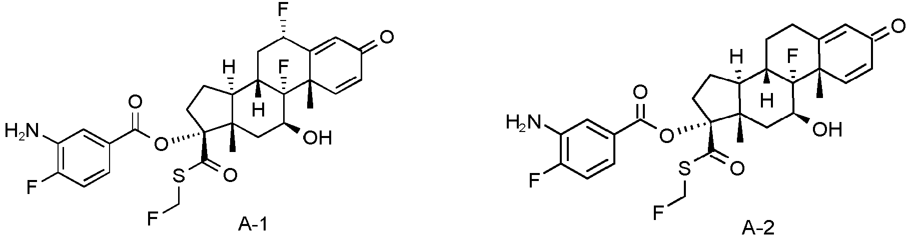 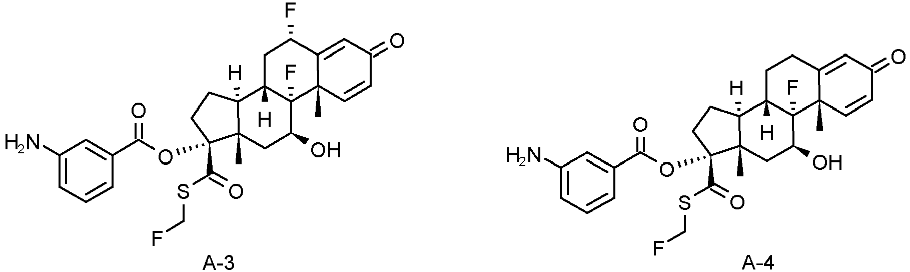 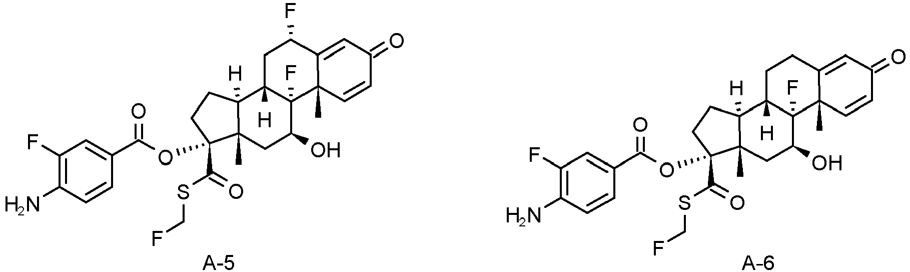 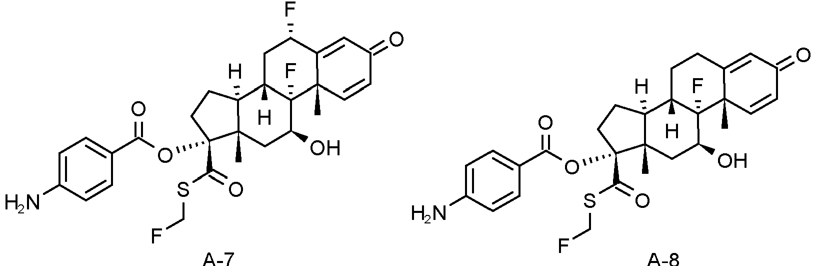 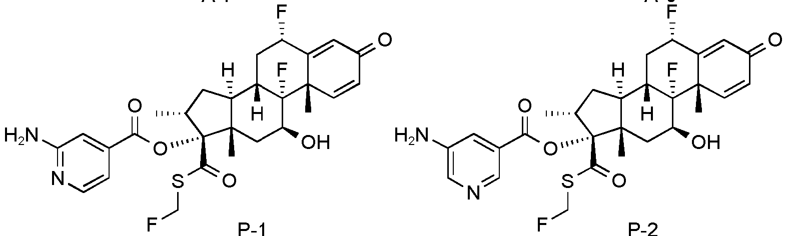 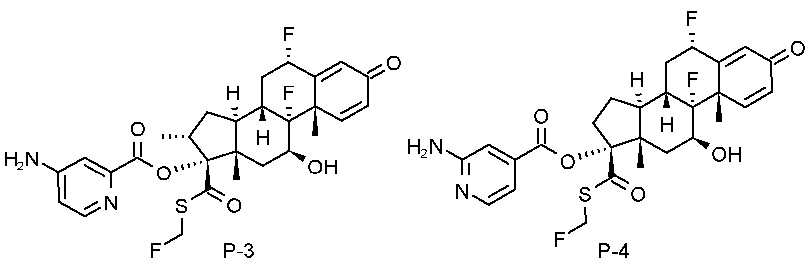 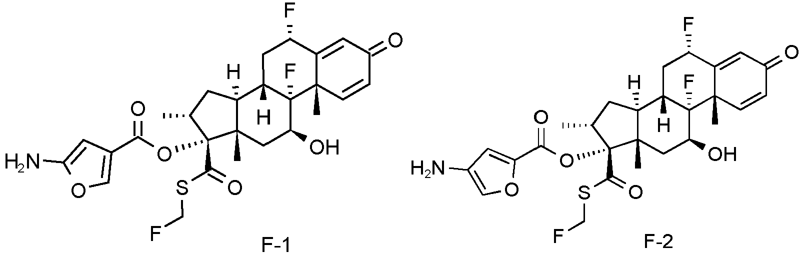 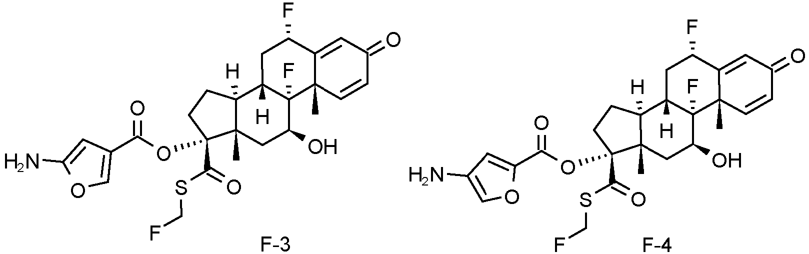 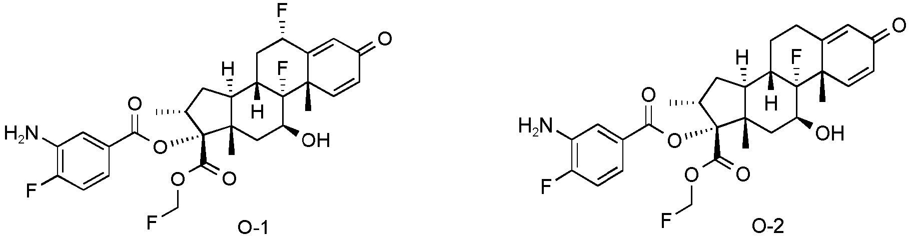 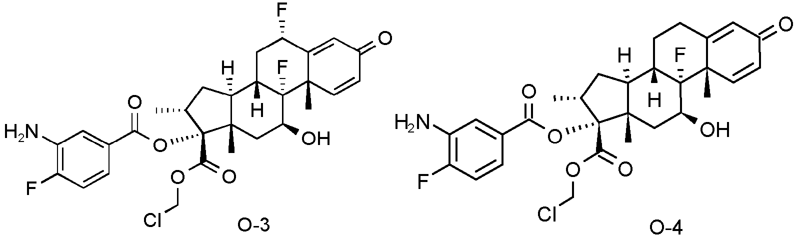 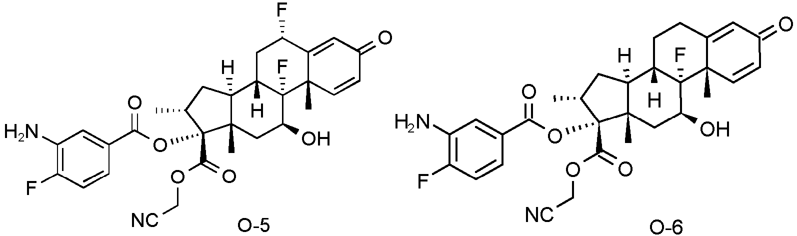 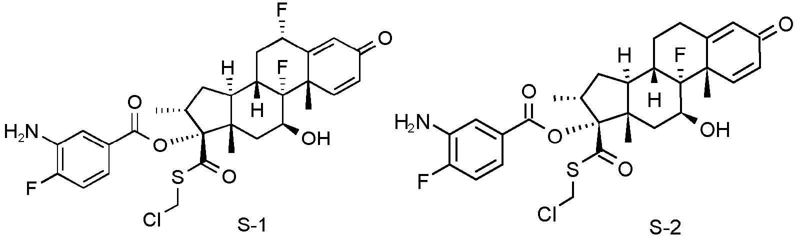 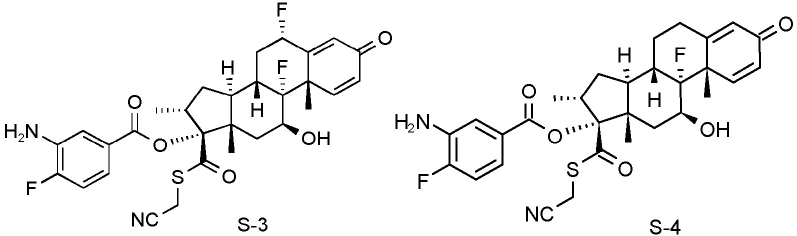 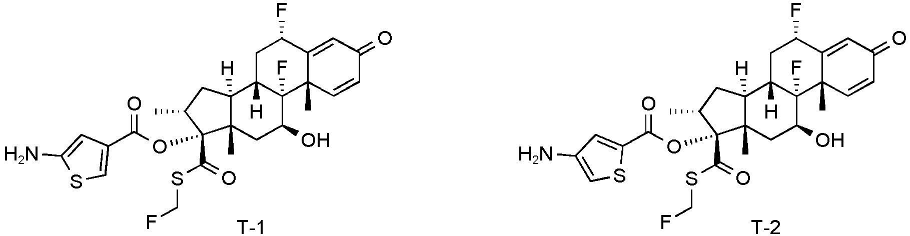 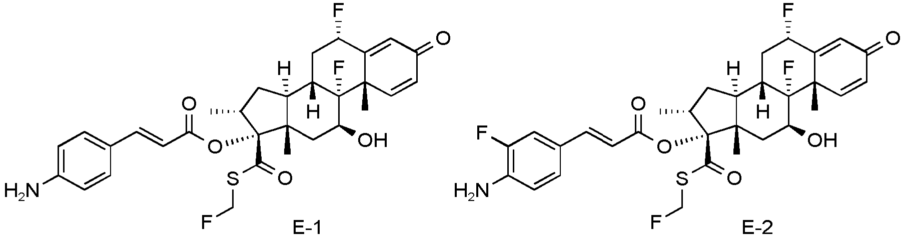 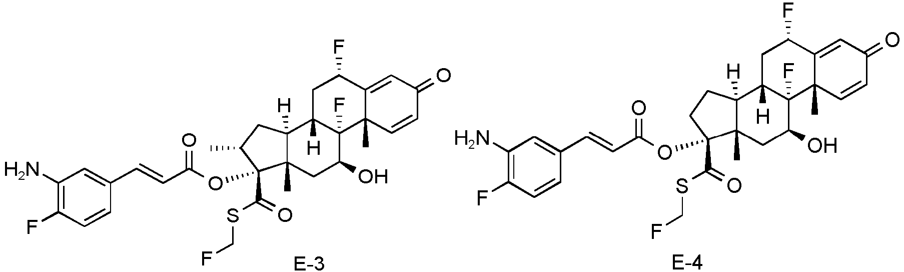 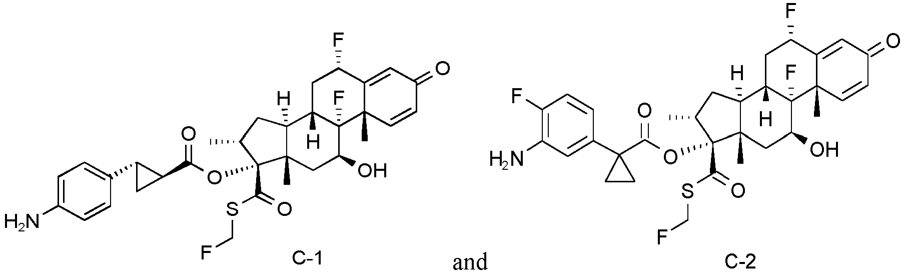 preferably selected from a group consisting of: 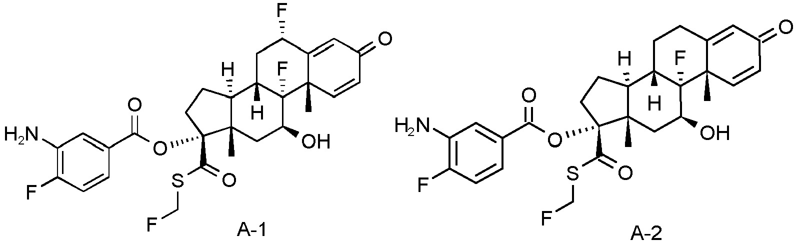 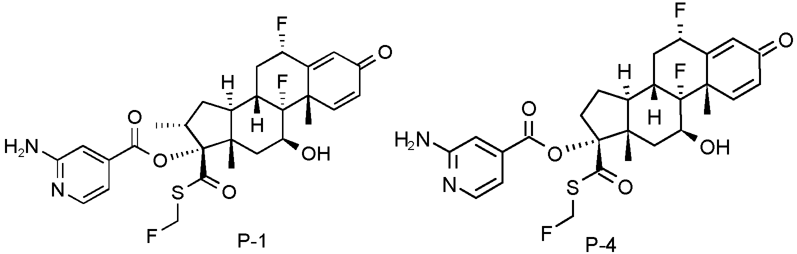 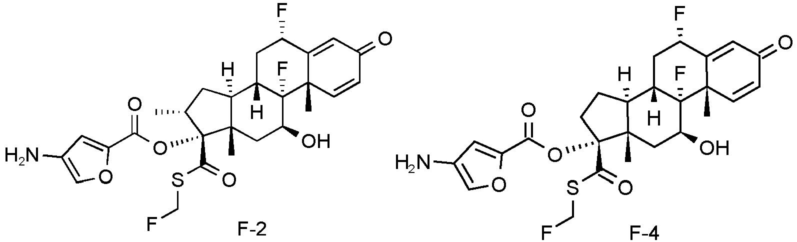 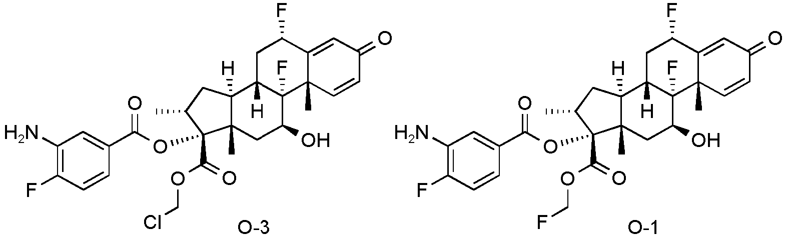 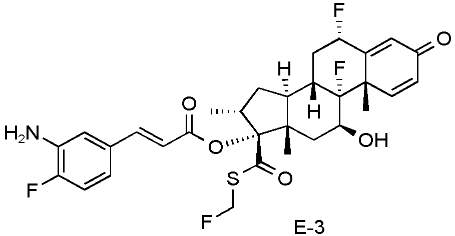 and

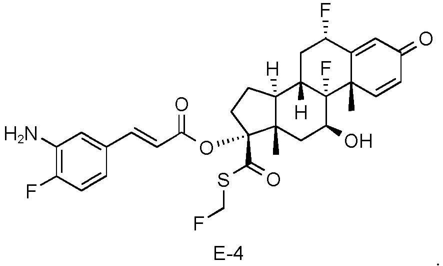

8. A compound, a pharmaceutically acceptable salt, a stereisomer, a tautomer, a polymorph, a solvate, an N-oxide, an isotope labeled compound, a metabolite, or a prodrug thereof, wherein the compound is selected from a group consisting of: 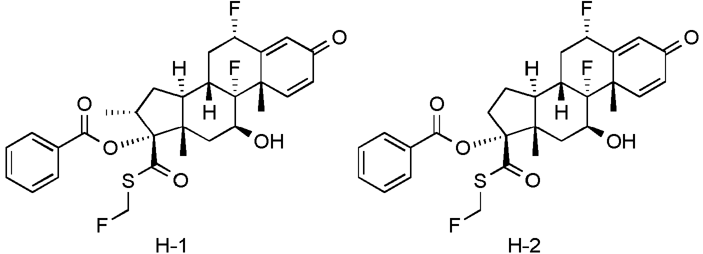 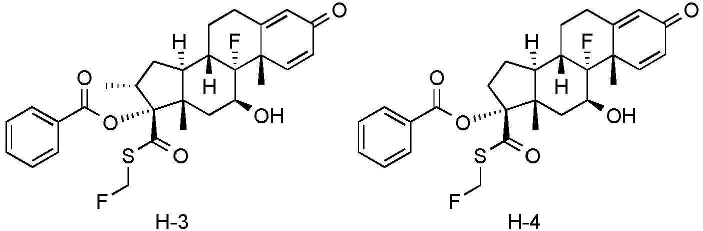 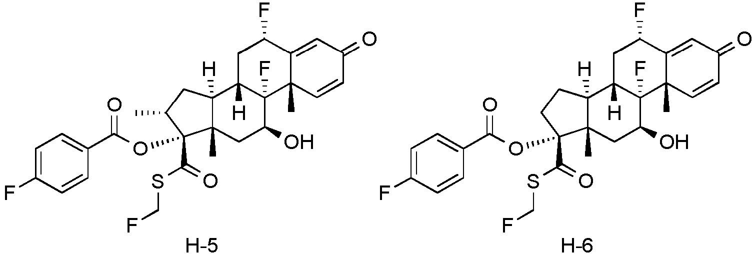 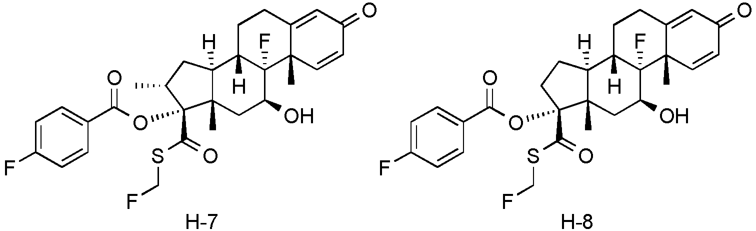 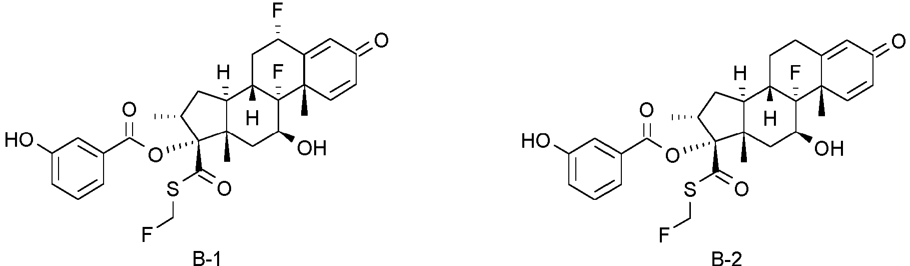 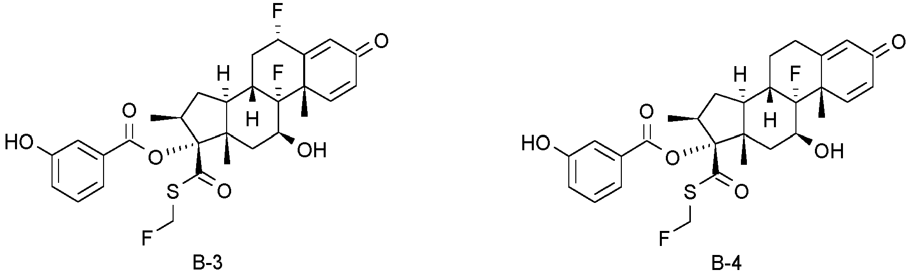 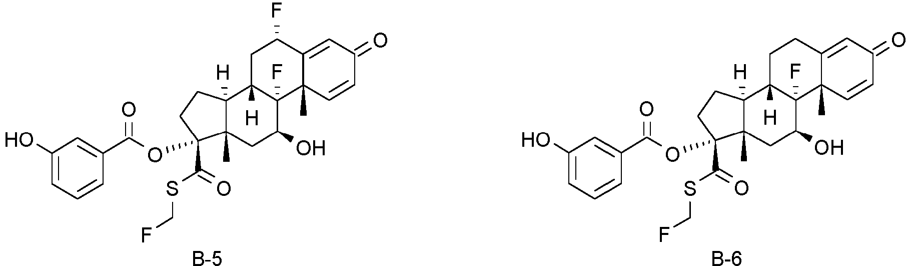 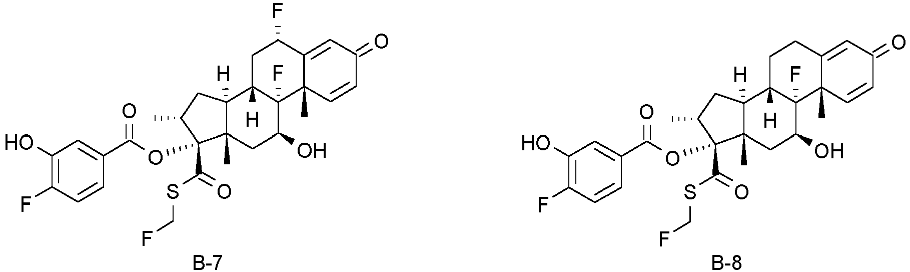 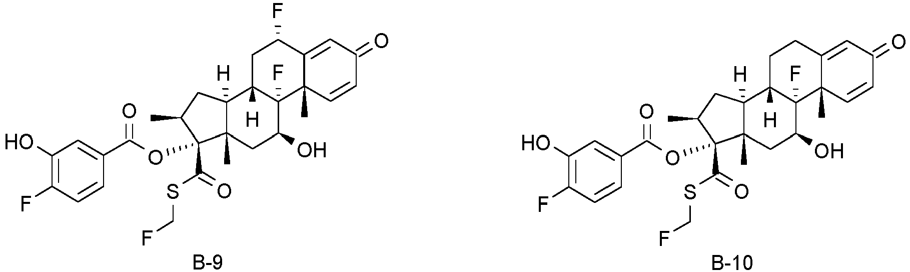 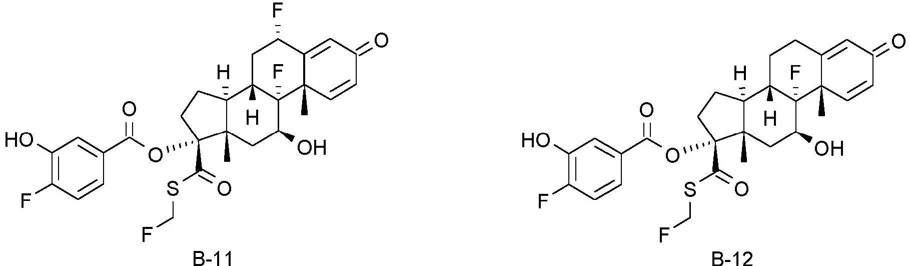 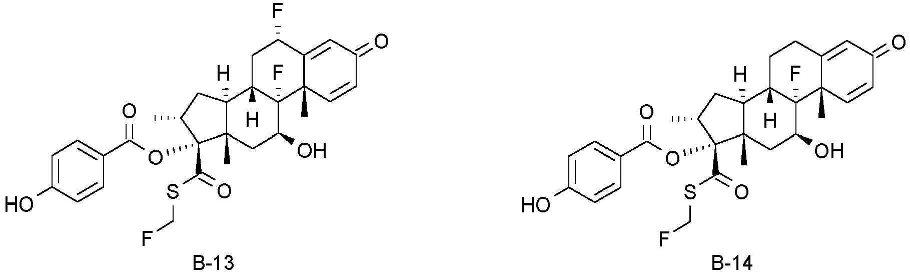 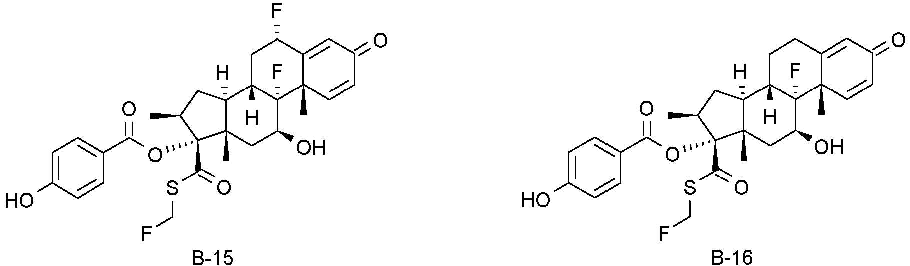 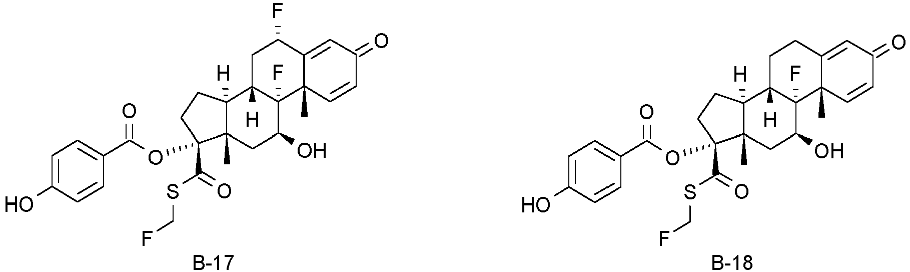 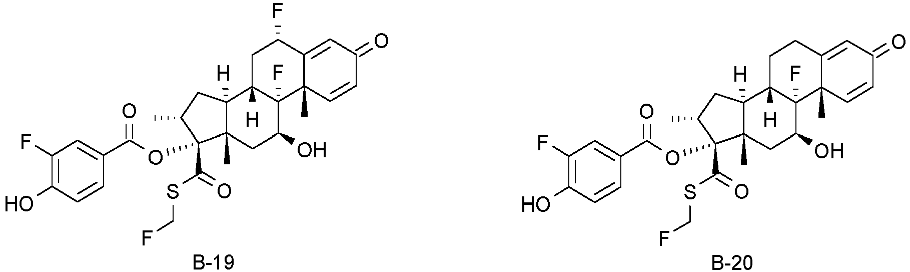 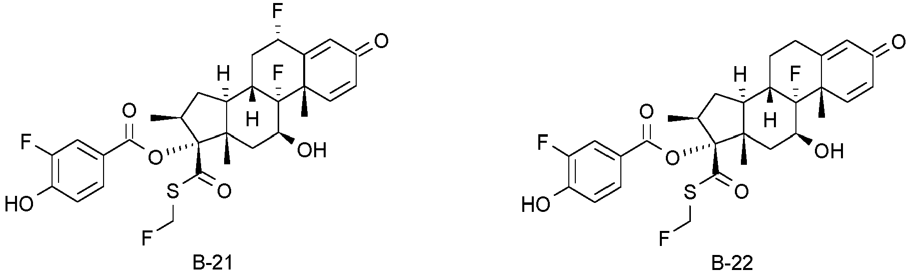 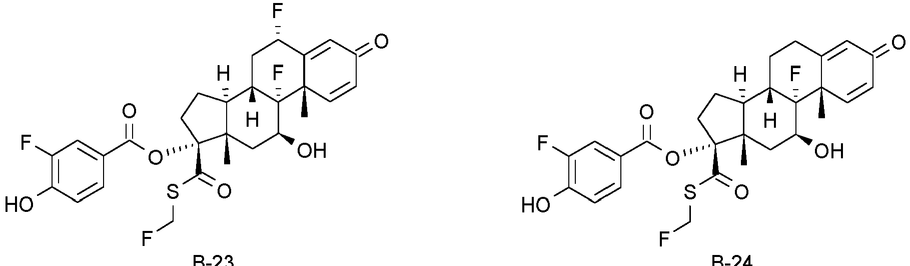 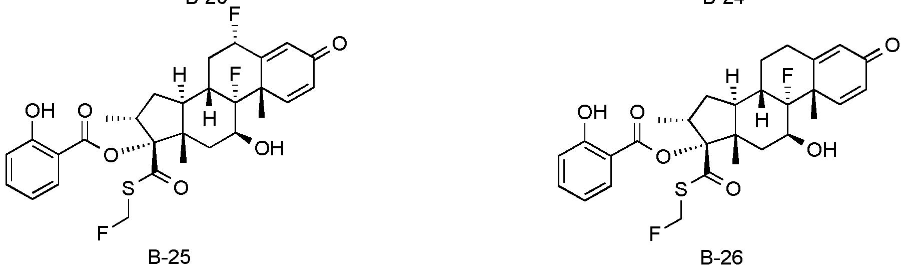 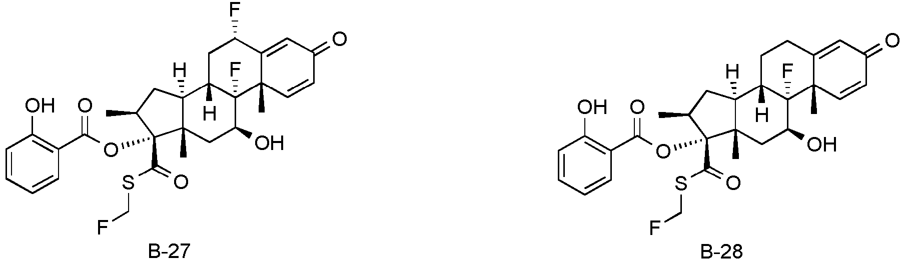 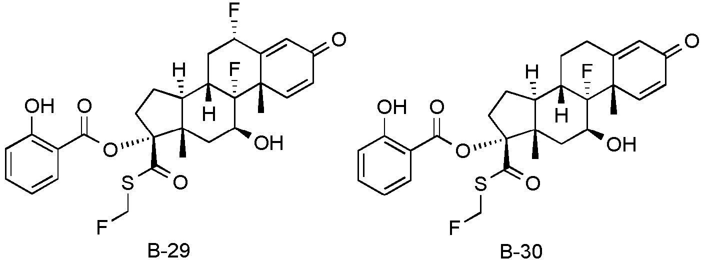 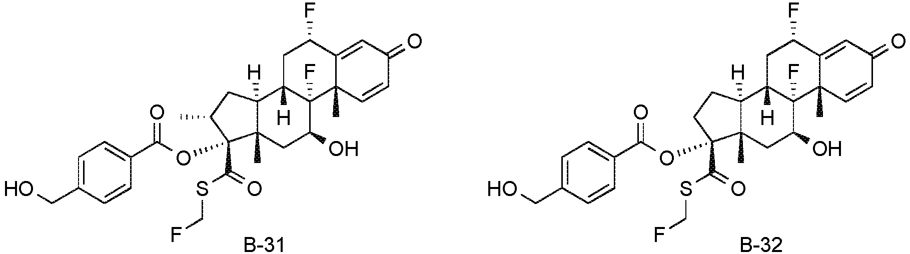 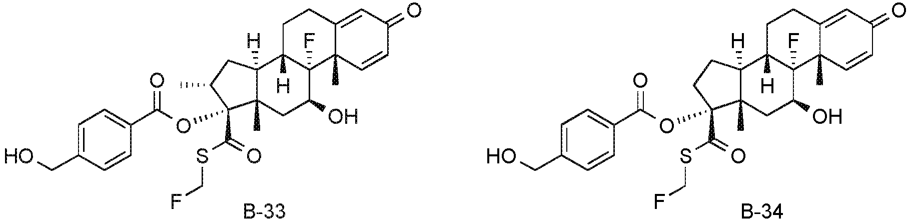 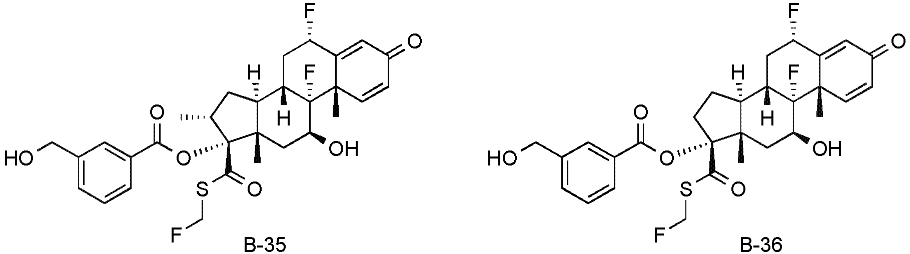 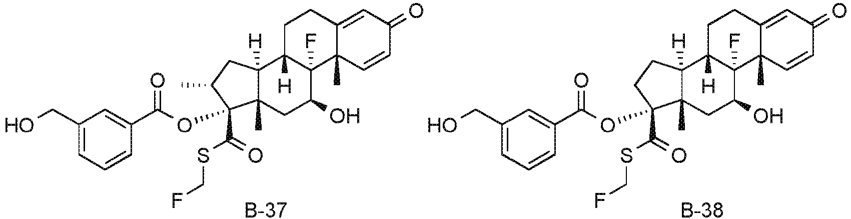 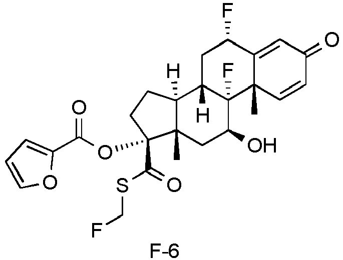 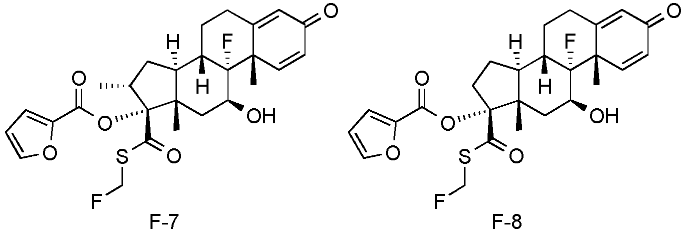 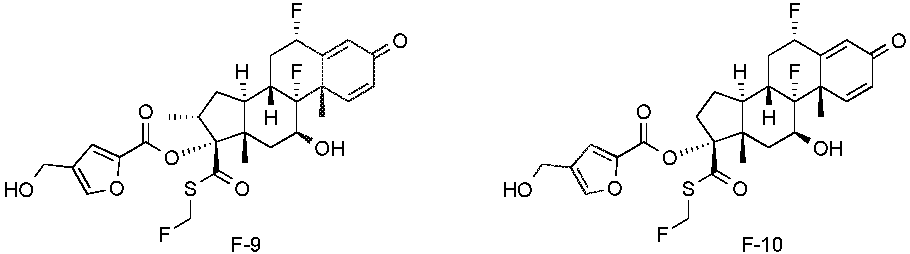 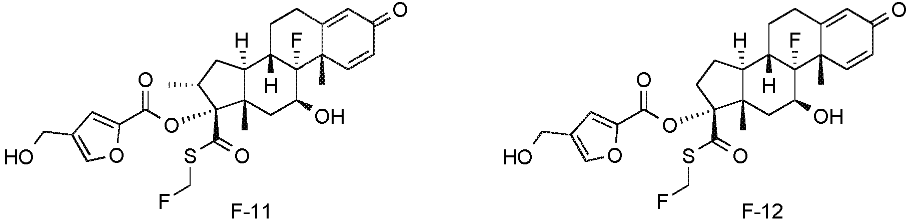 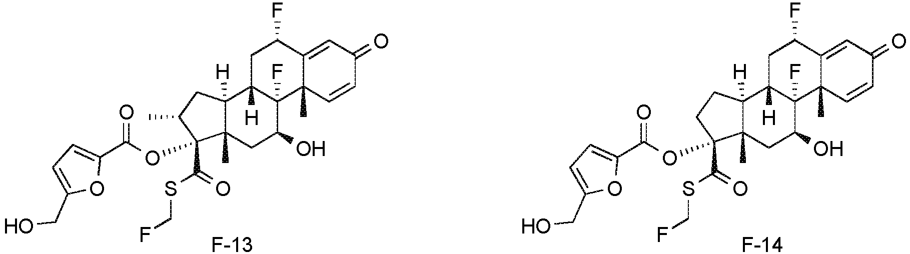 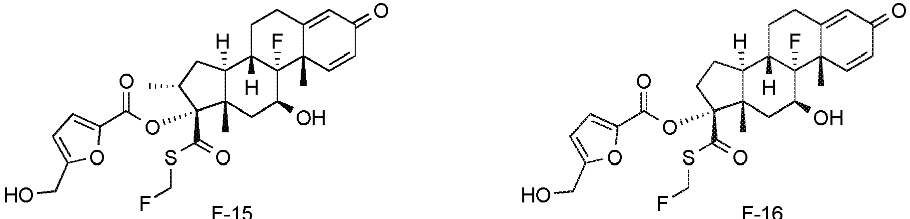 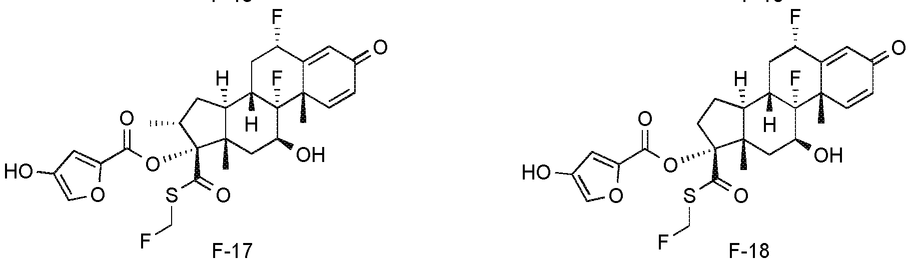 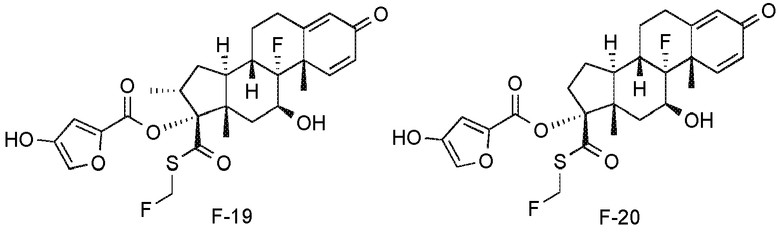 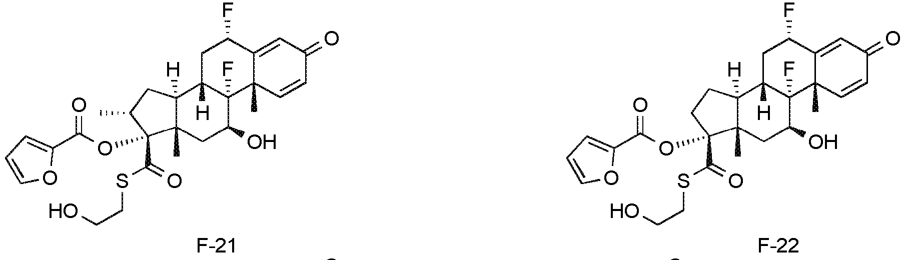 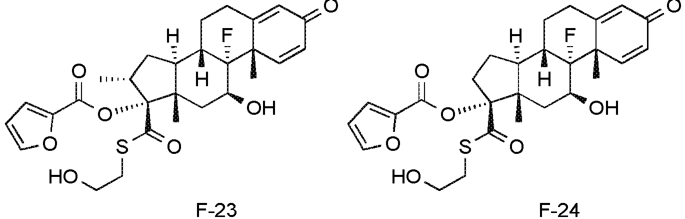 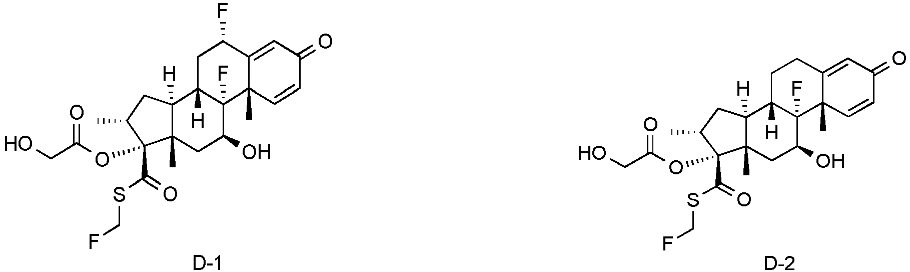 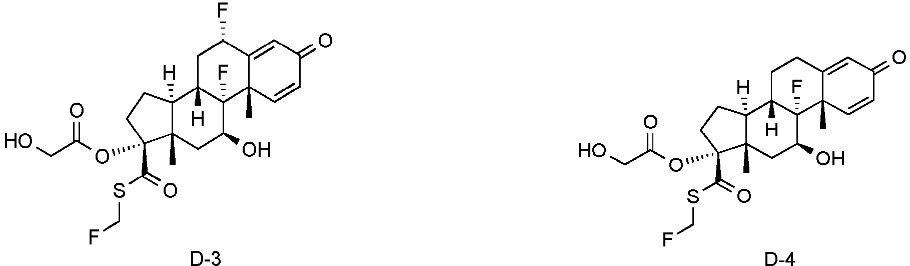 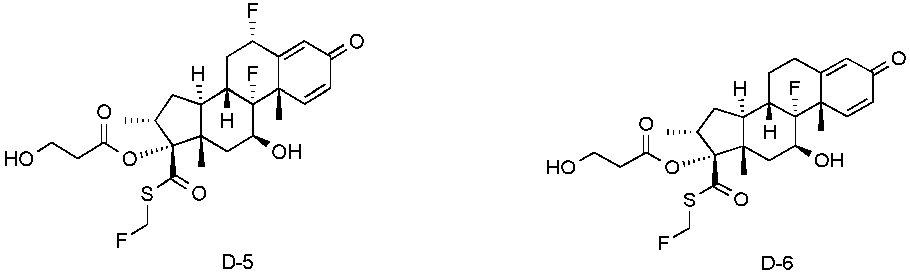 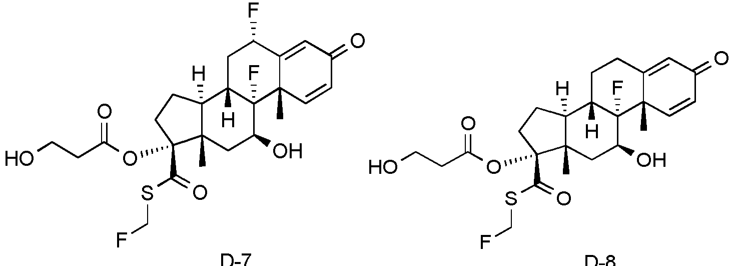 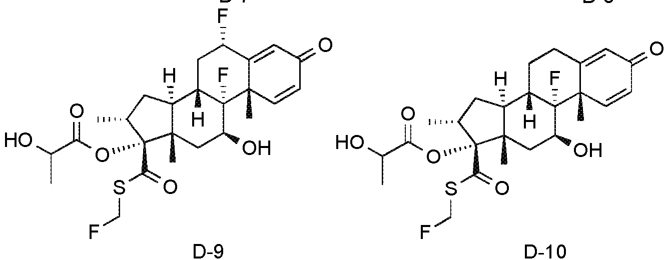 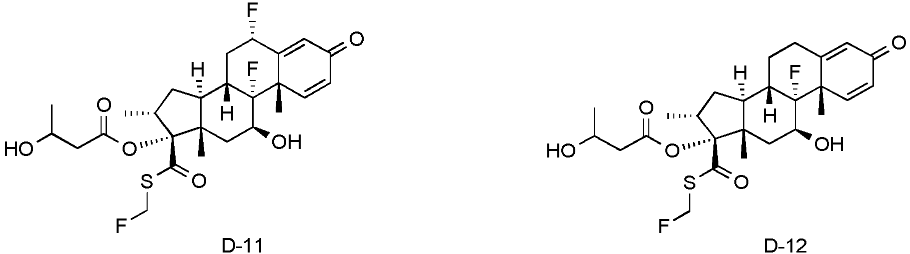 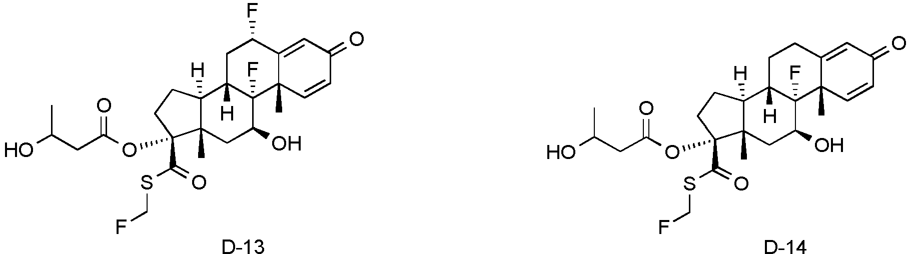 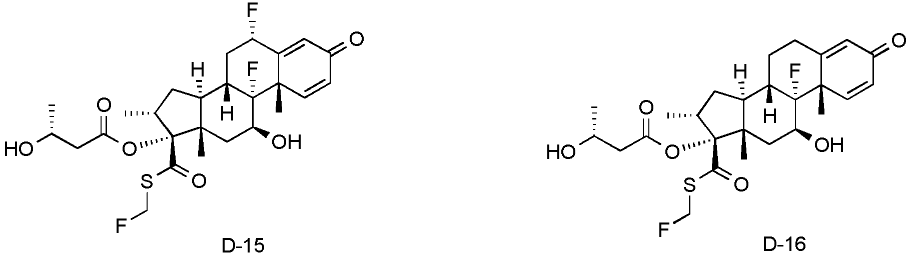 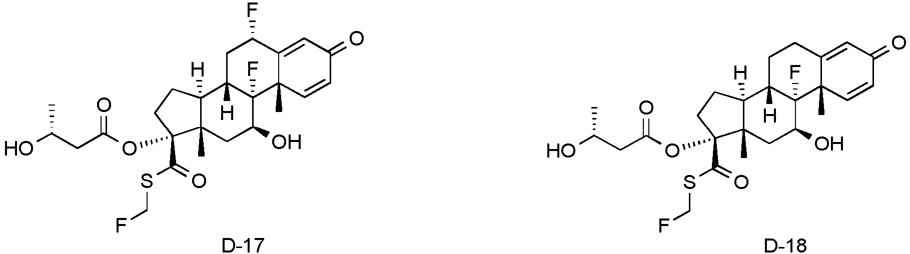 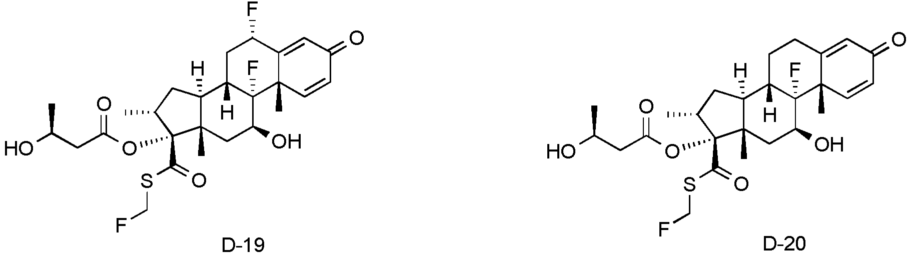 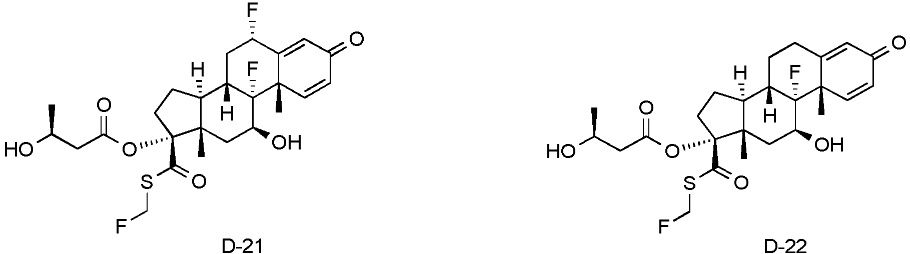 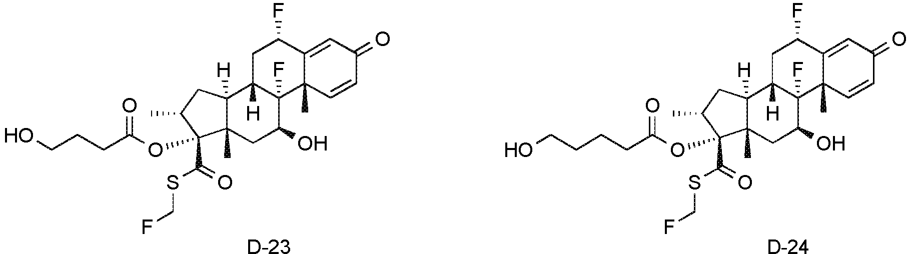 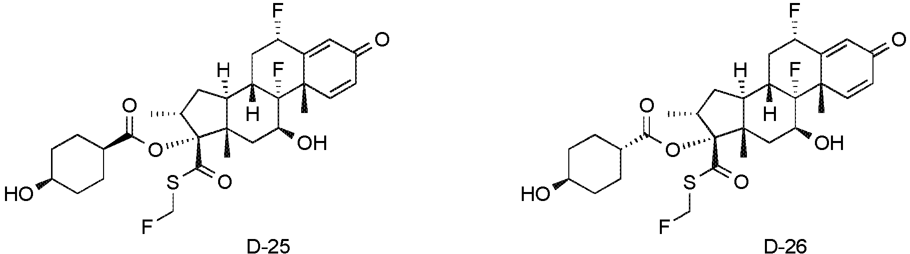 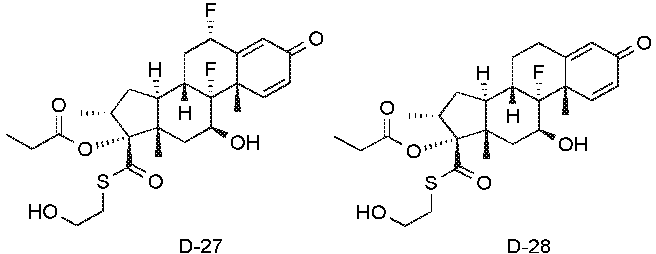 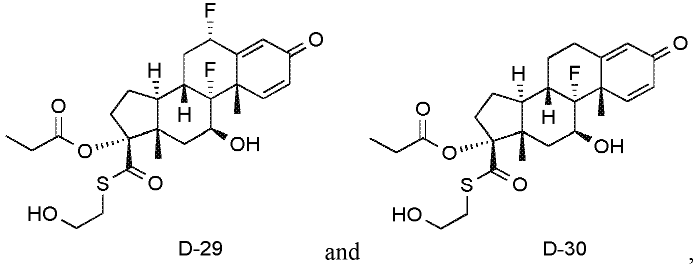 preferably selected from a group consisting of: 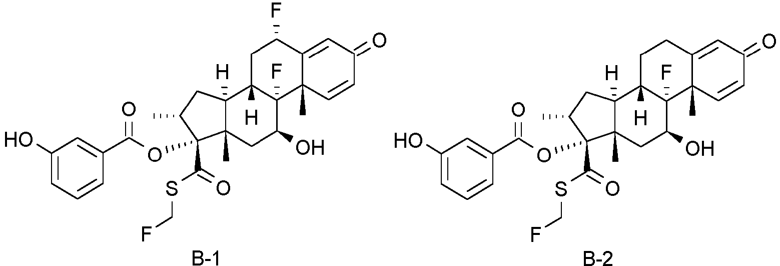 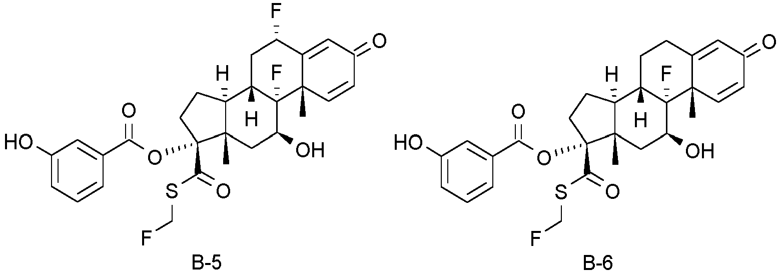 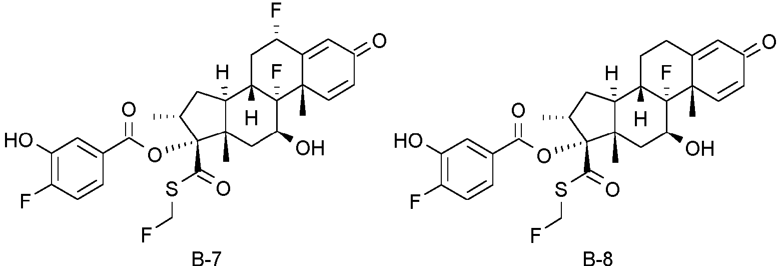 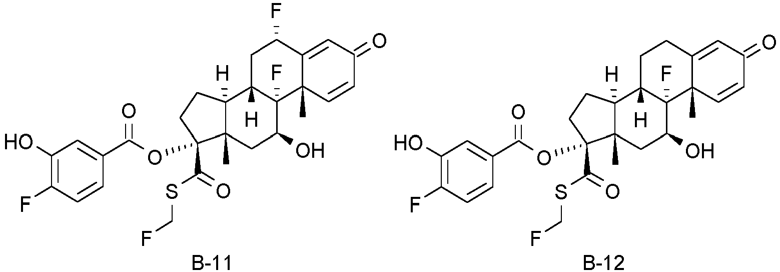 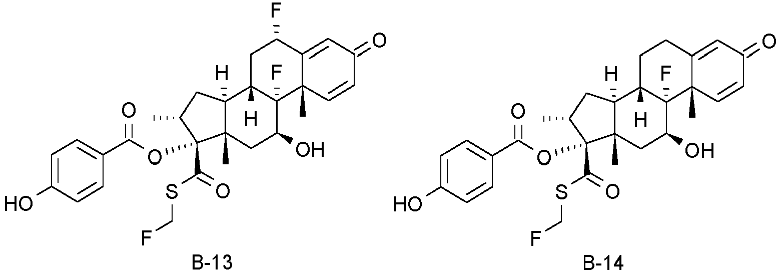 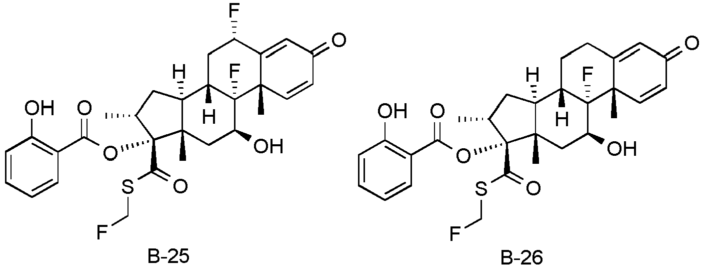 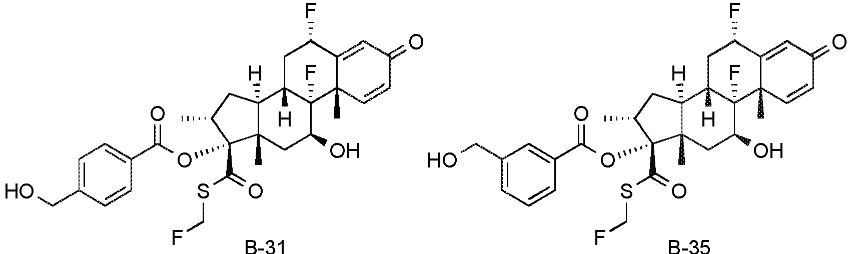 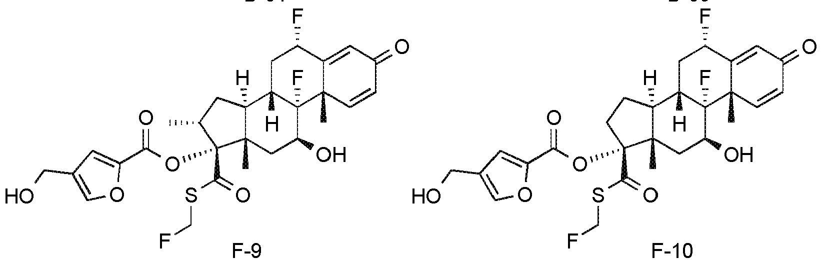 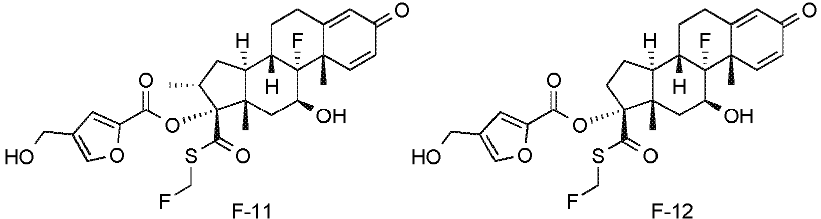 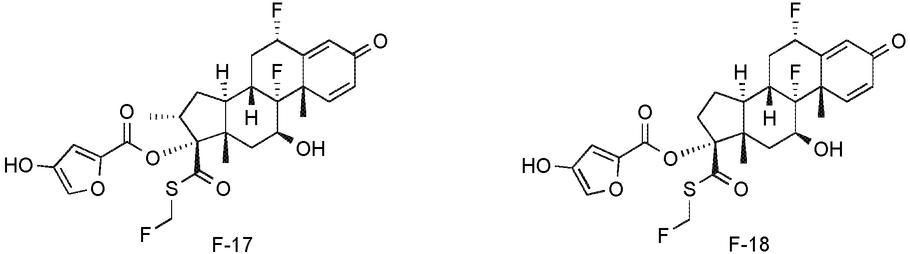 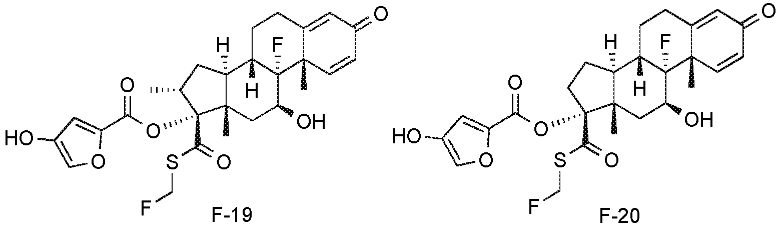 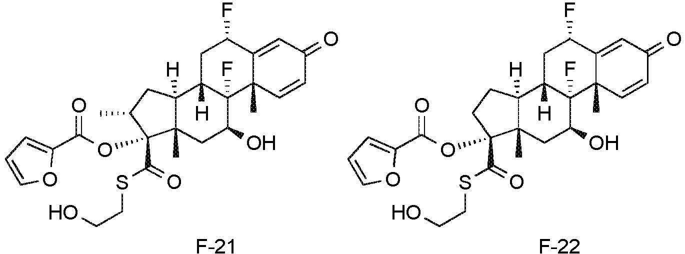 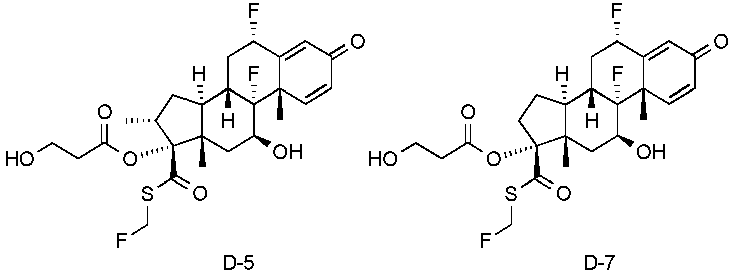 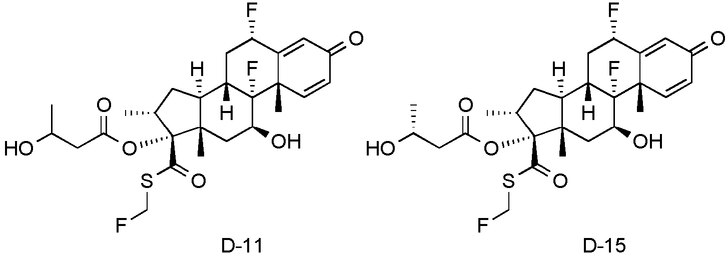 and

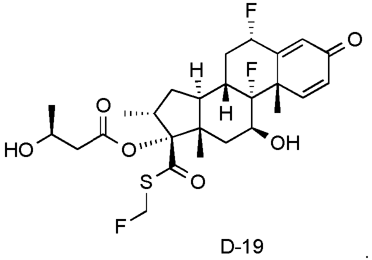

9. An antibody-drug conjugate having a structure represented by formula Ab-[M-L-E-D]ₓ, wherein:
Ab is an antibody specifically binding to an antigen or an antigen binding fragment thereof;
M is a linkage site to the antibody or the antigen binding fragment thereof;
L is a linker between the linkage site M and E;
E is a structural fragment linking L with D;
D is a glucocorticoid drug fragment which is a monovalent structure obtained by losing one H from -OH, -NH₂ or secondary amino on the compound, pharmaceutically acceptable salt, stereisomer, tautomer, polymorph, solvate, N-oxide, isotope labeled compound, metabolite or prodrug thereof according to any one of claims 1-8;
x is an integer selected from 1 to 10, preferably an integer selected from 3 to 8;
preferably, D is selected from the following structures: 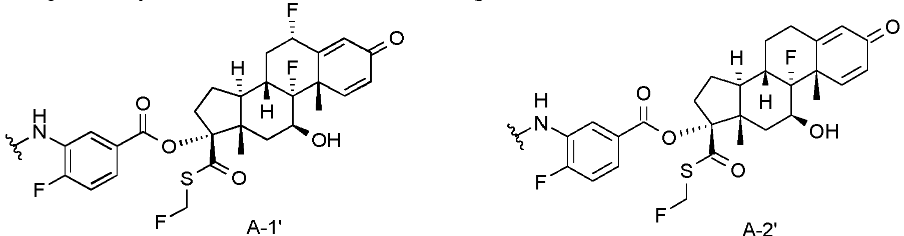 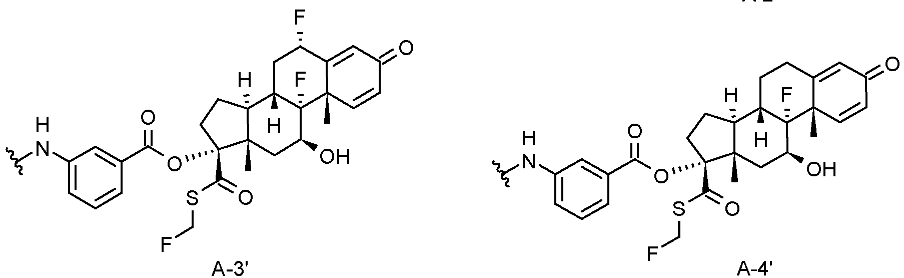 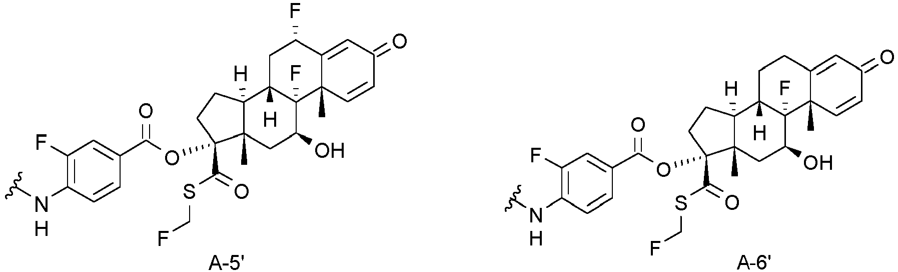 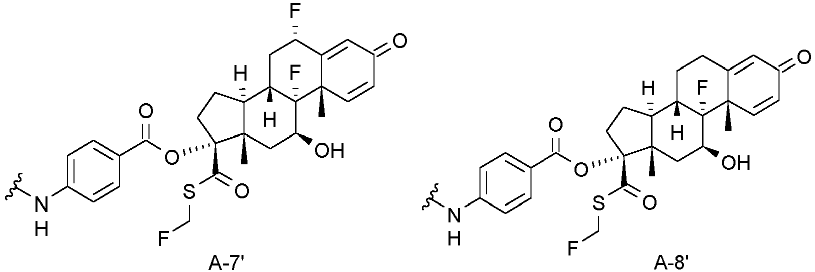 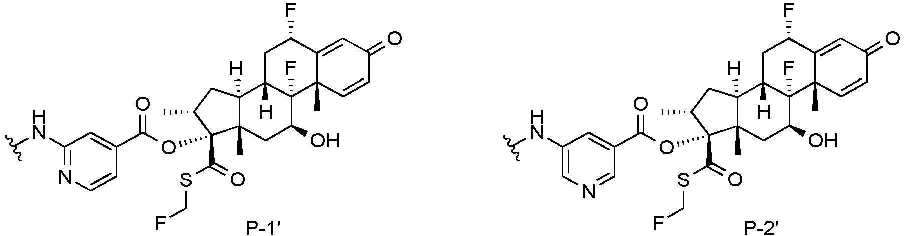 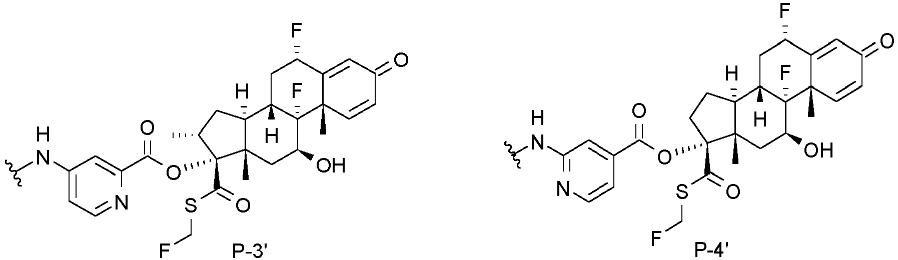 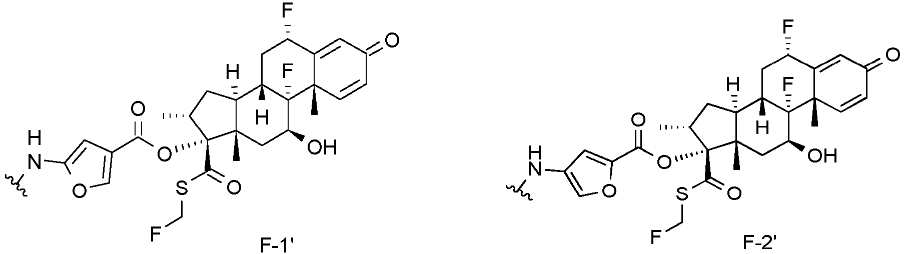 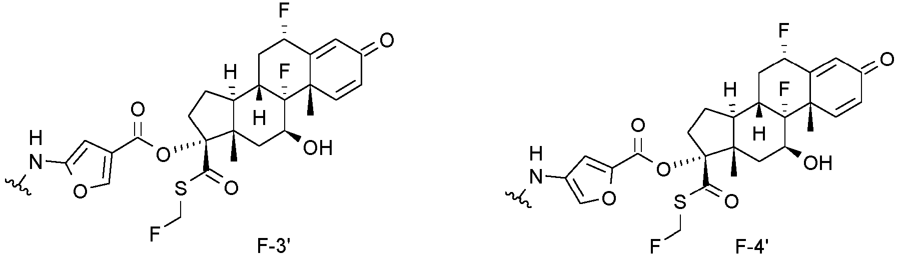 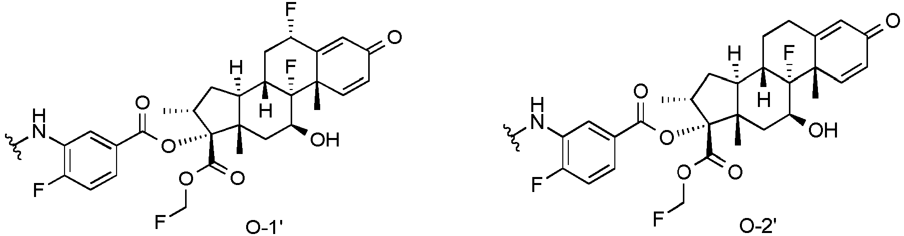 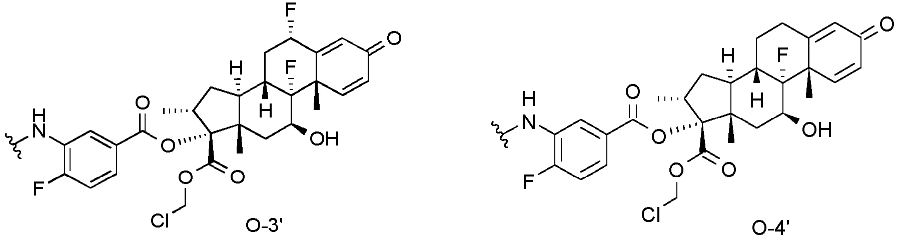 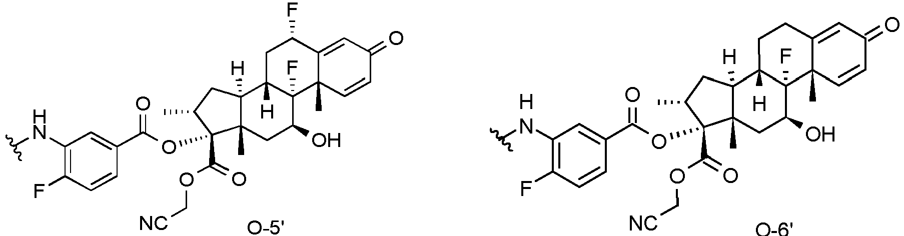 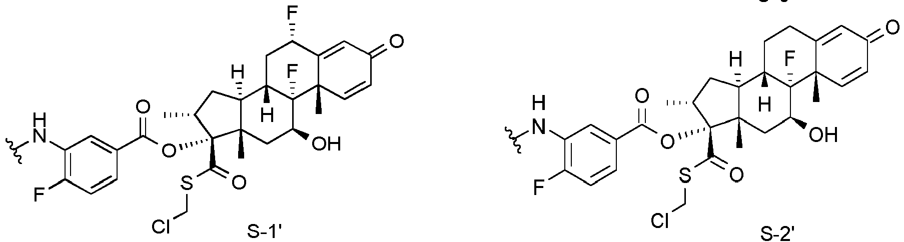 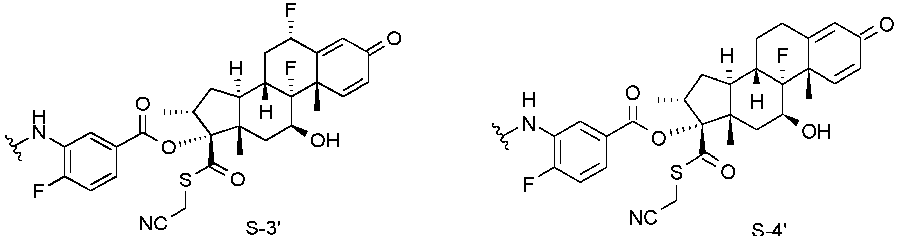 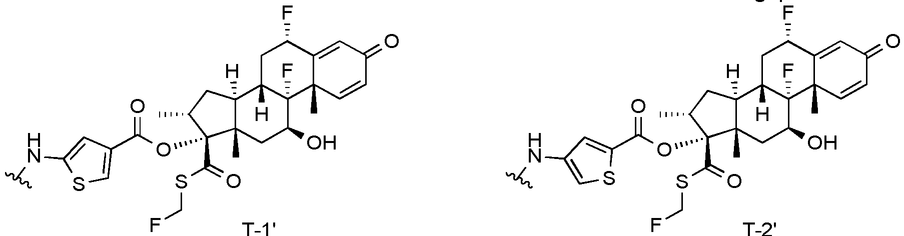 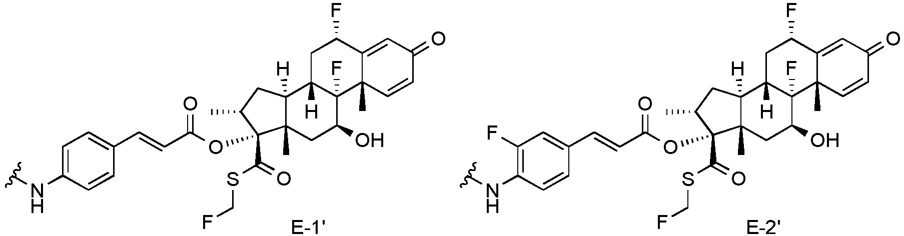 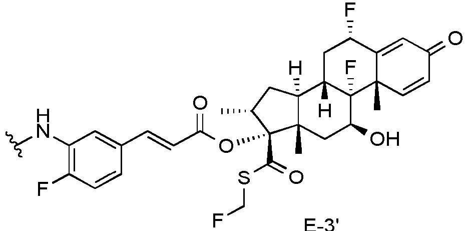 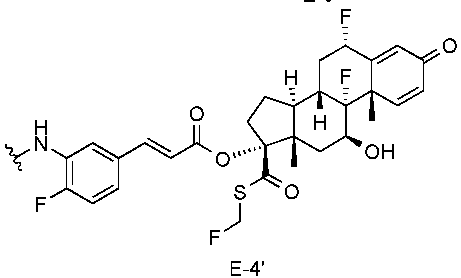 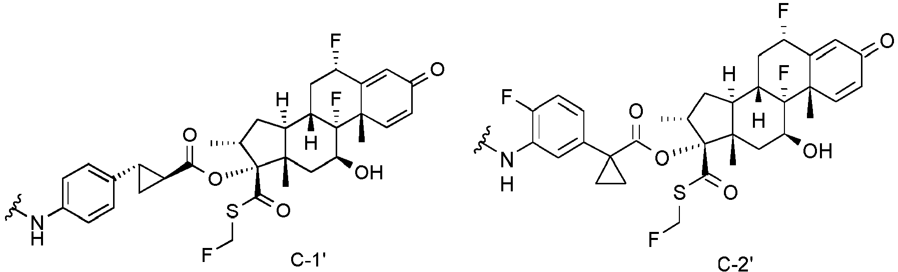 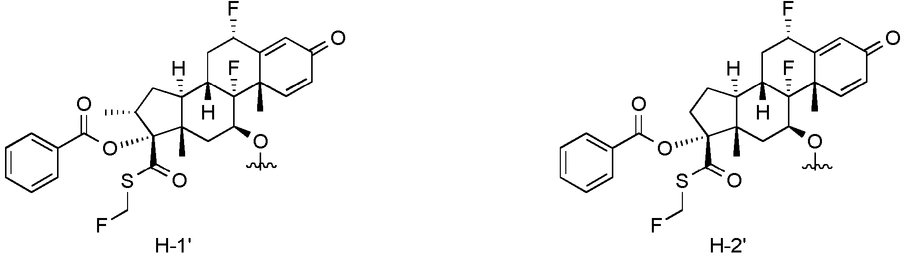 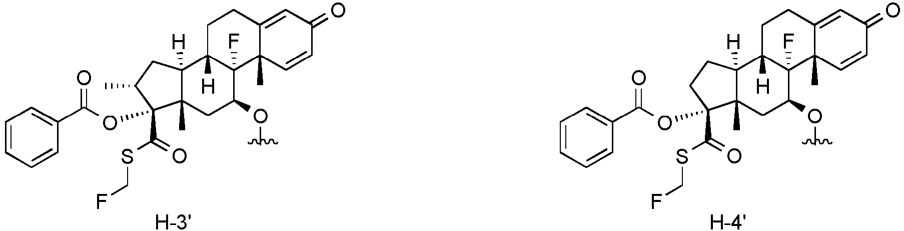 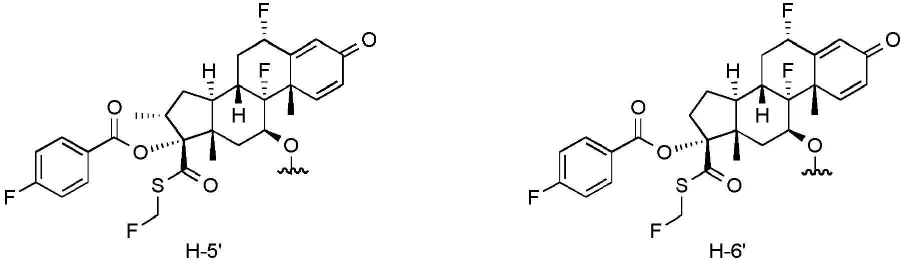 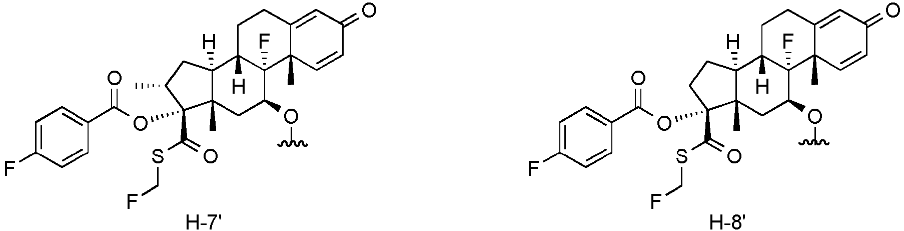 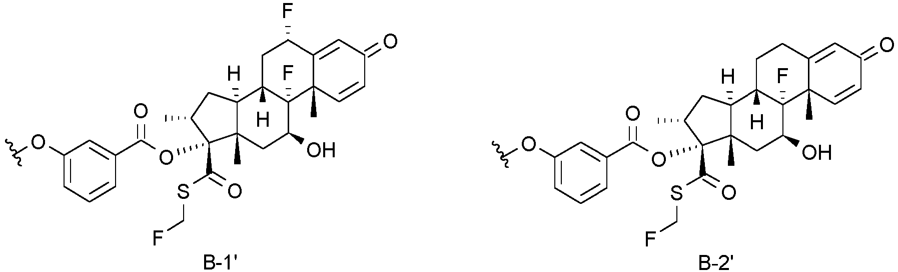 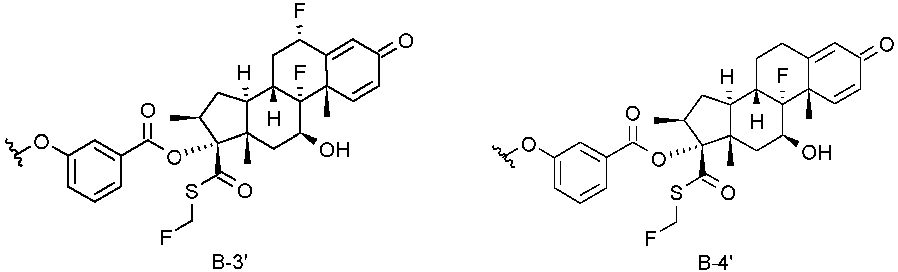 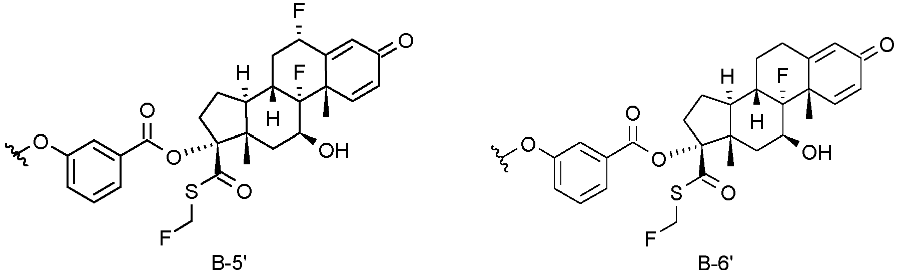 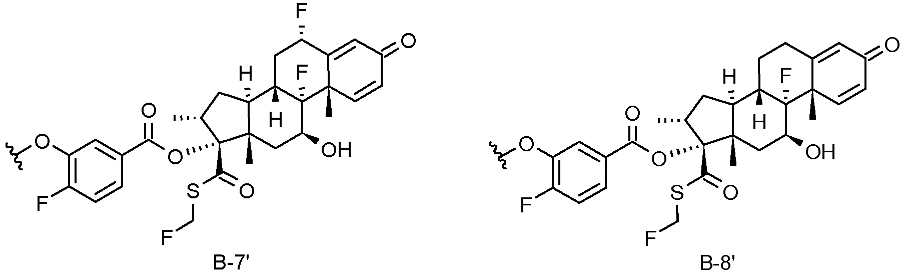 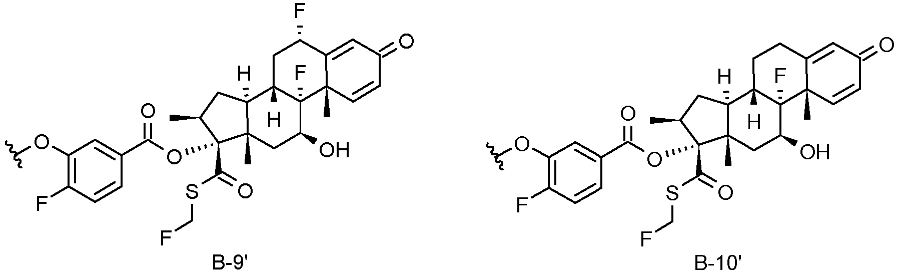 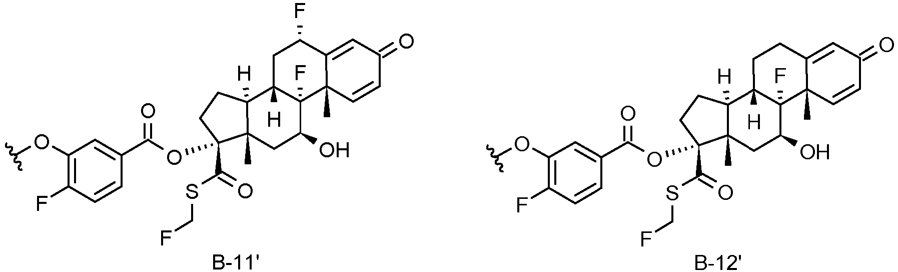 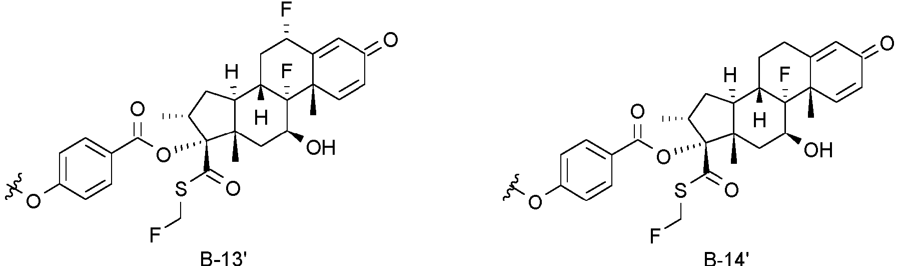 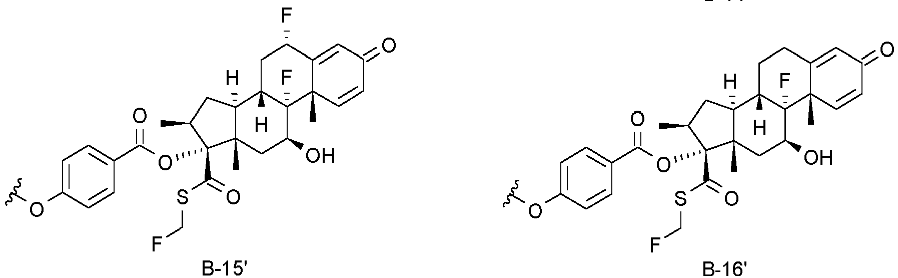 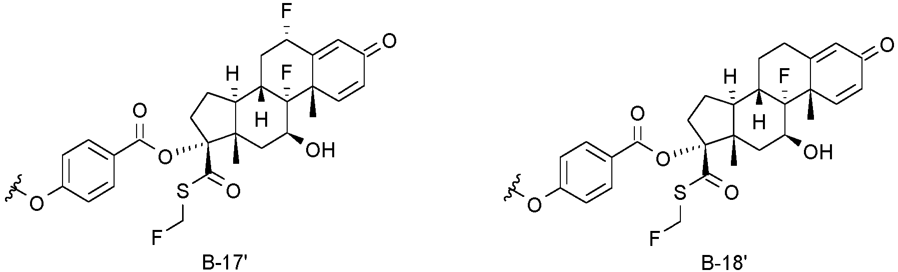 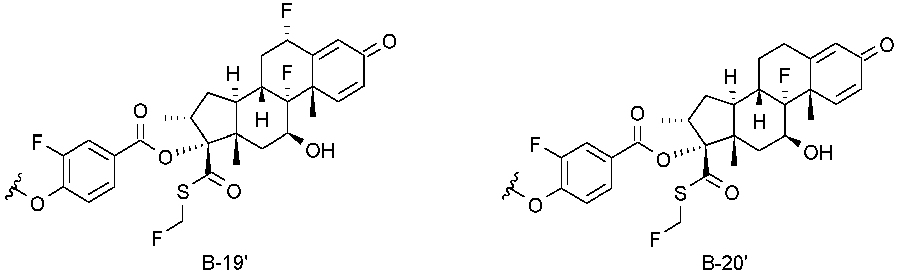 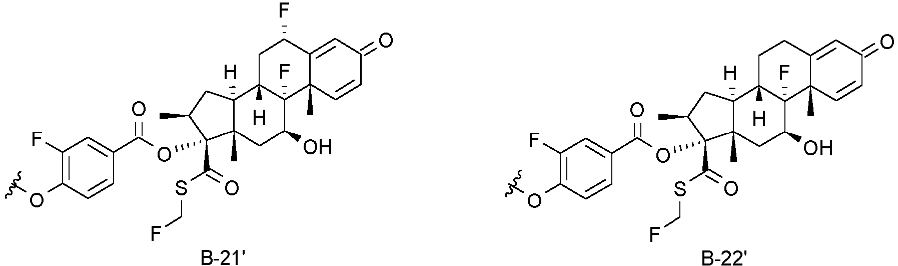 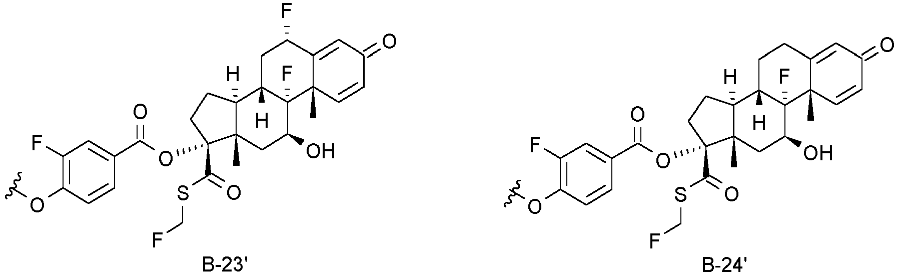 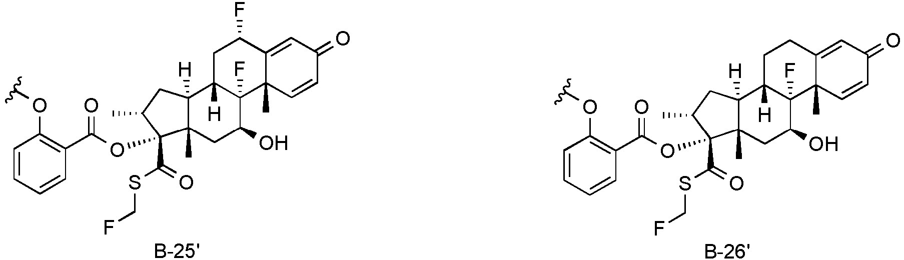 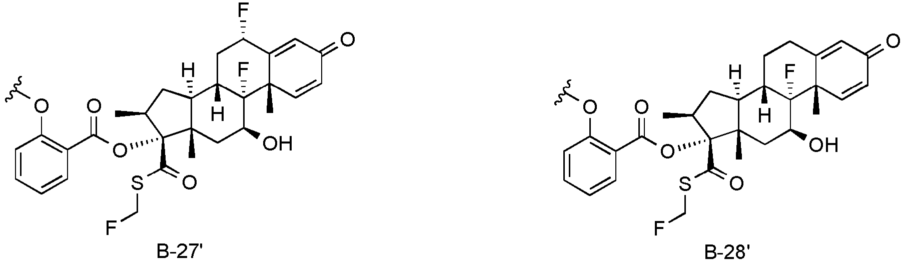 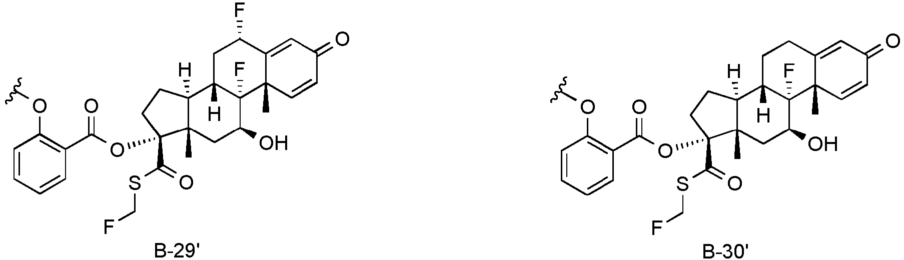 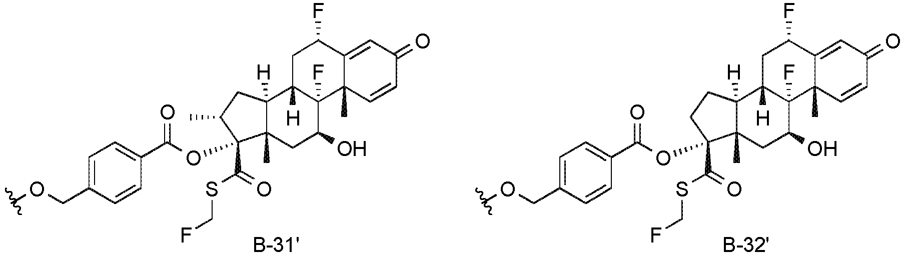 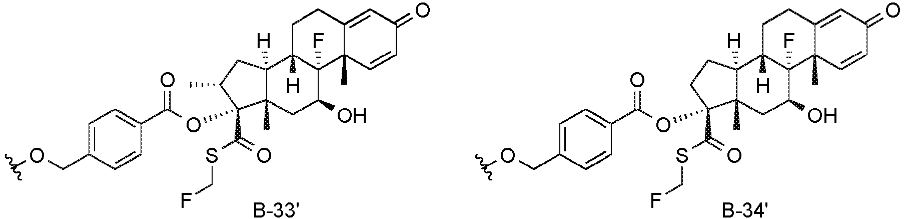 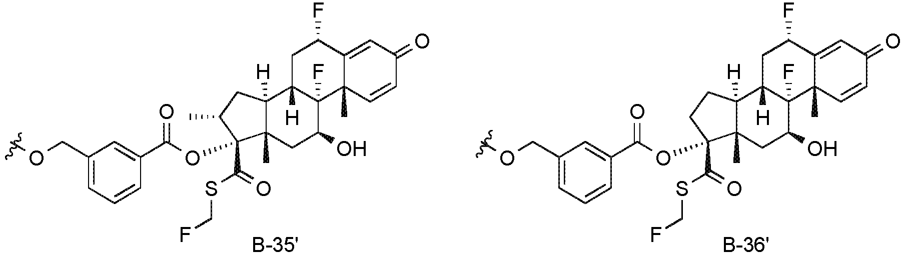 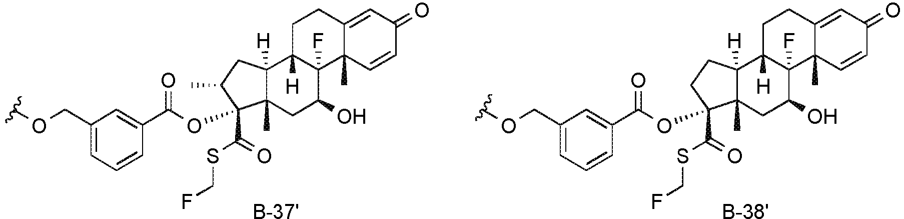 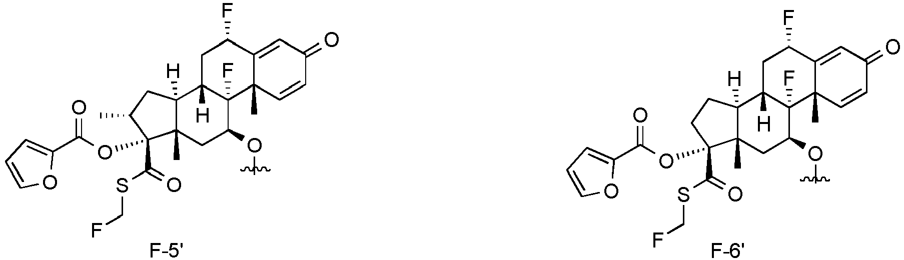 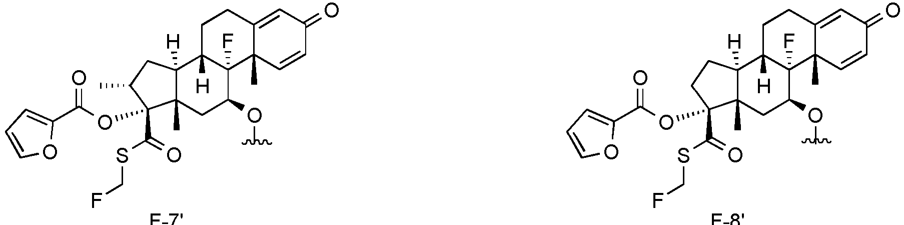 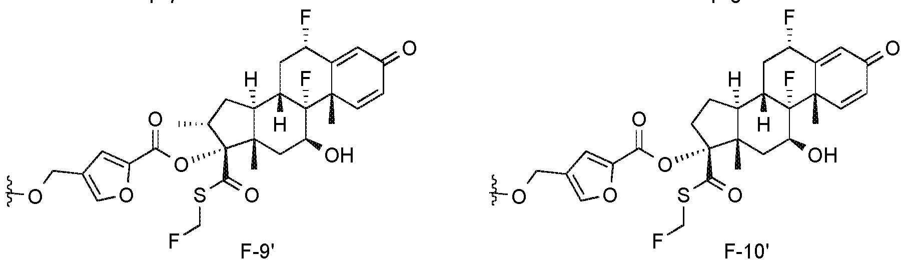 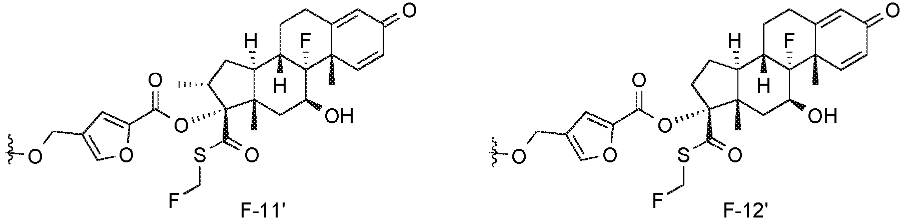 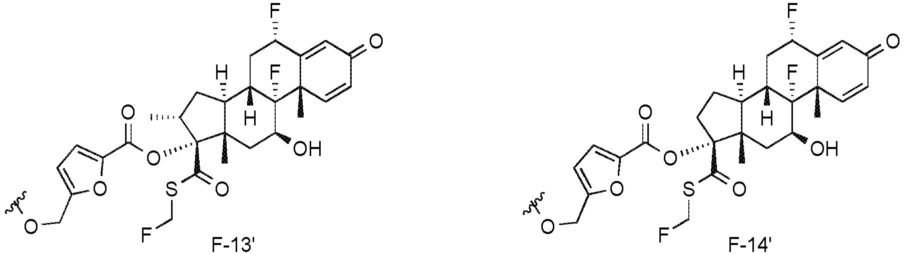 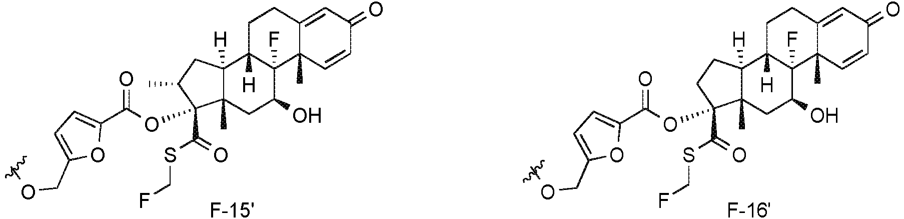 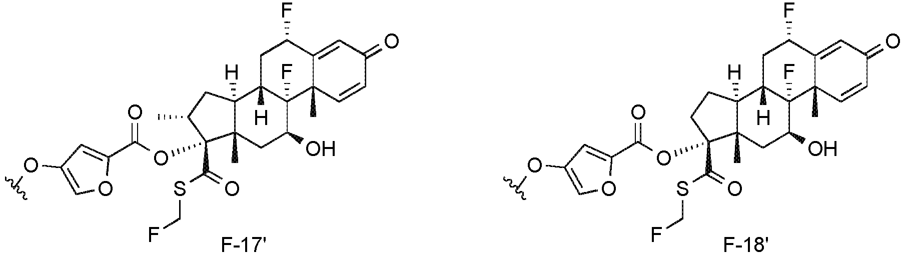 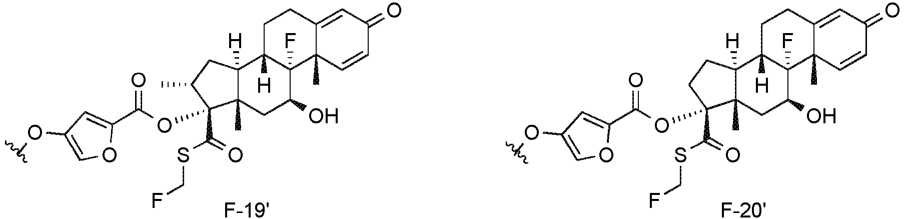 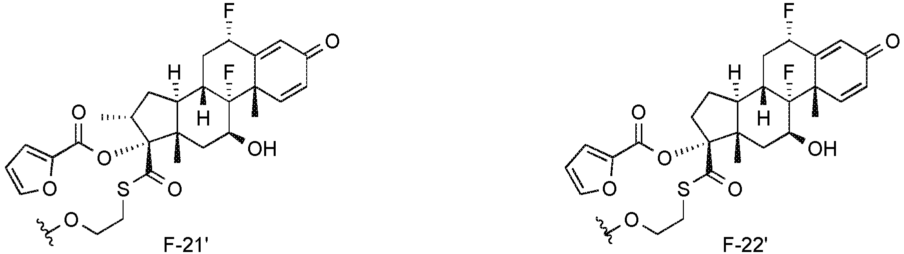 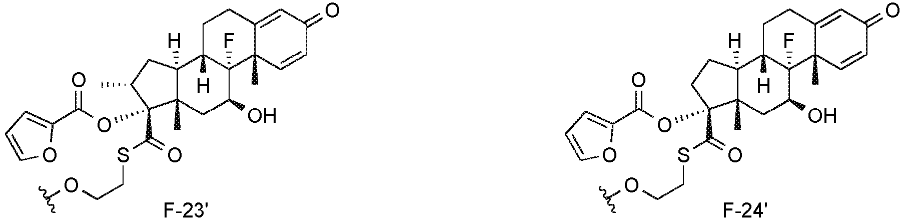 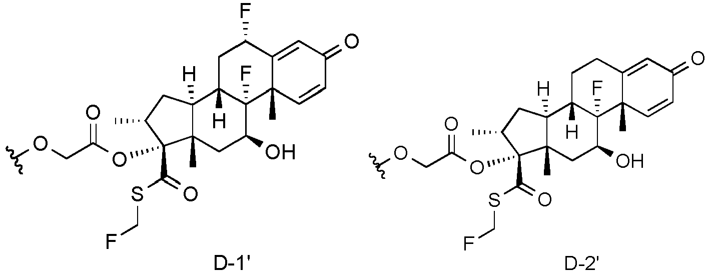 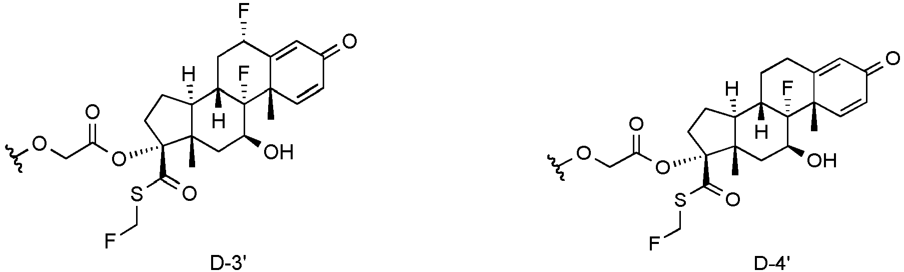 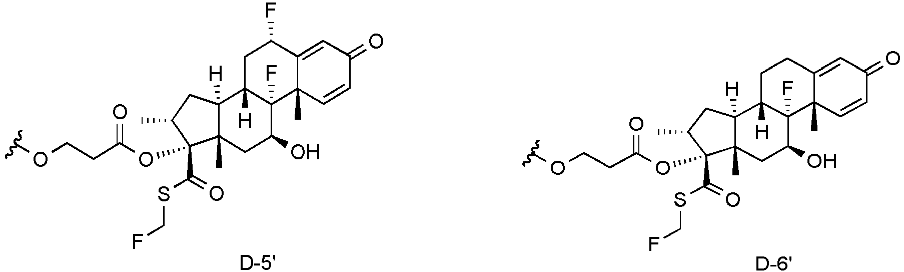 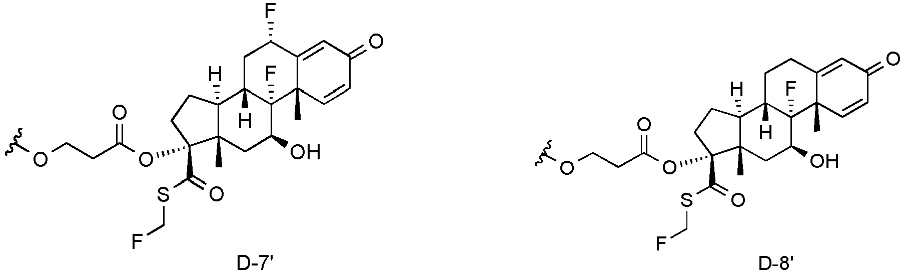 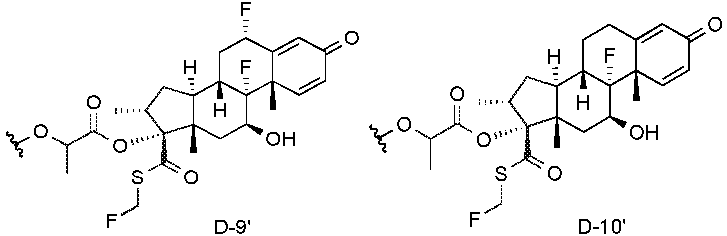 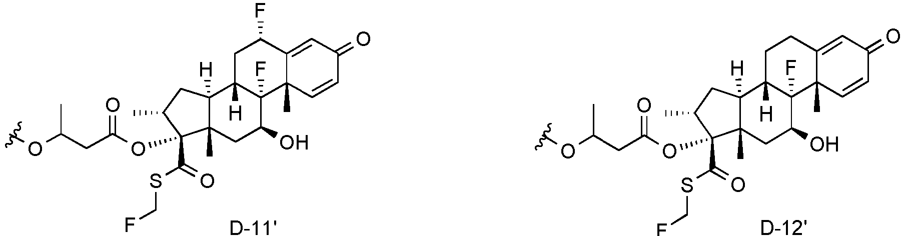 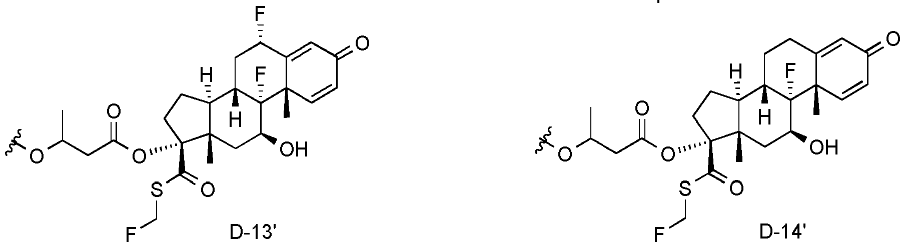 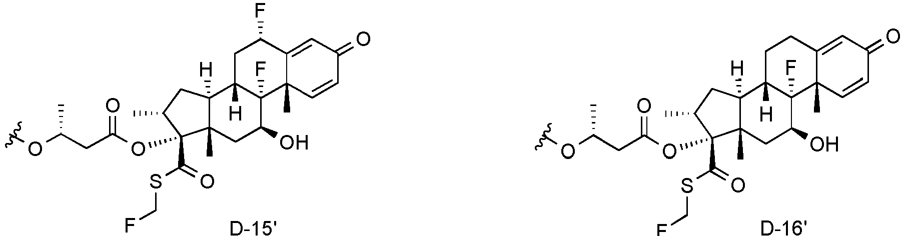 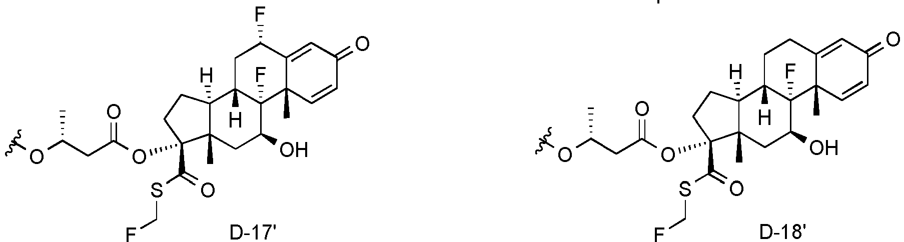 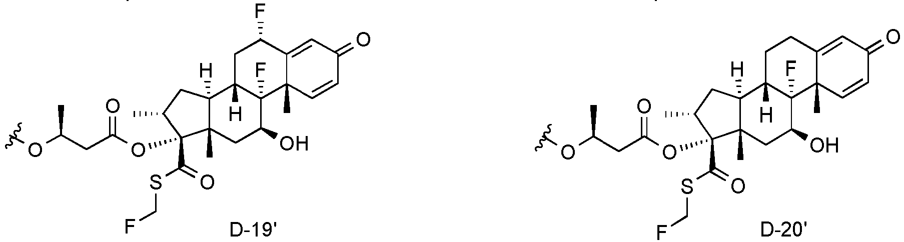 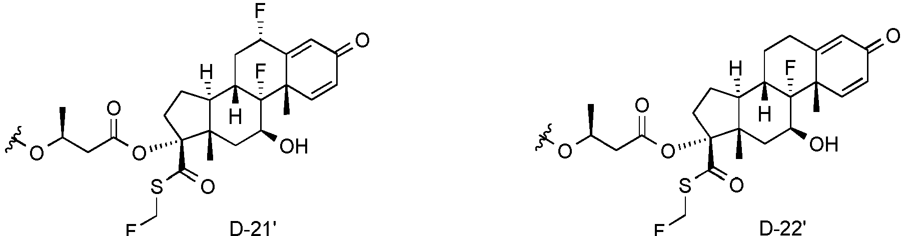 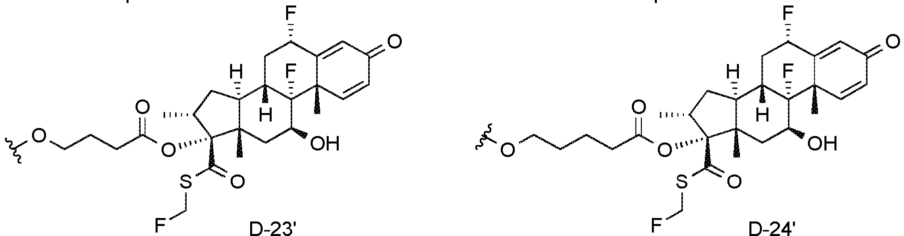 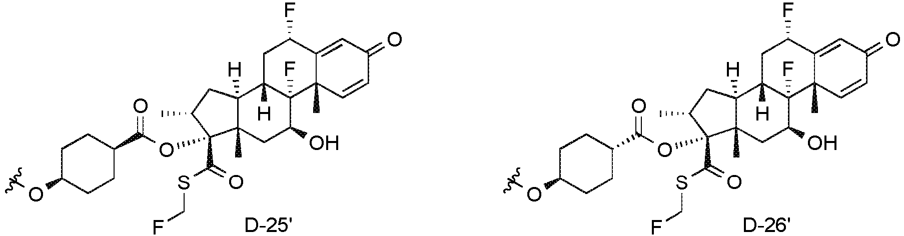 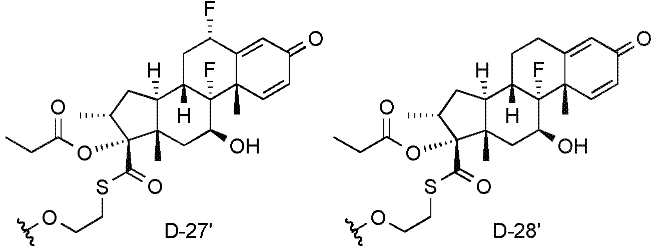 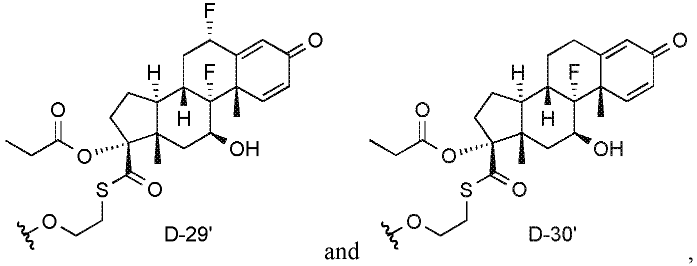
preferably, D is selected from the following structures: 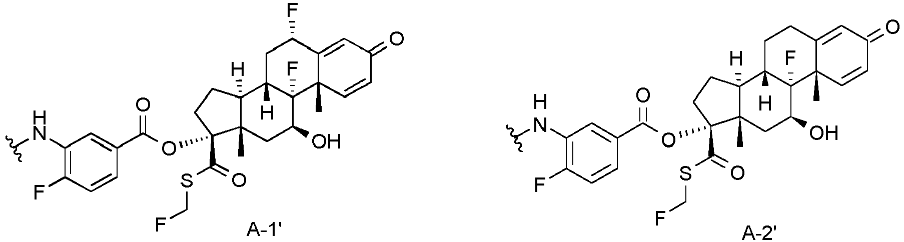 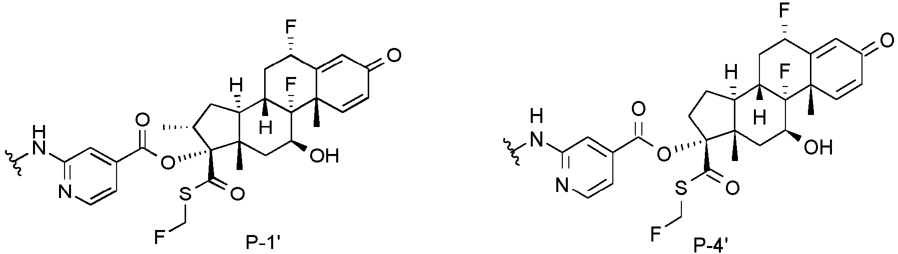 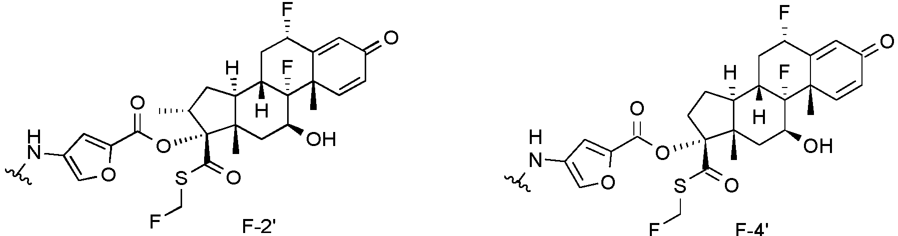 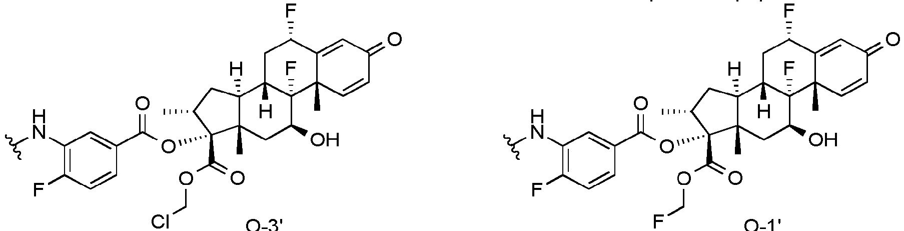 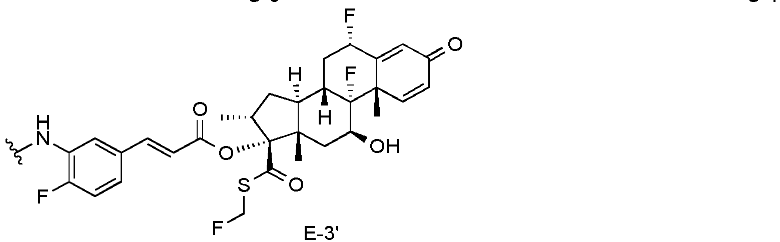 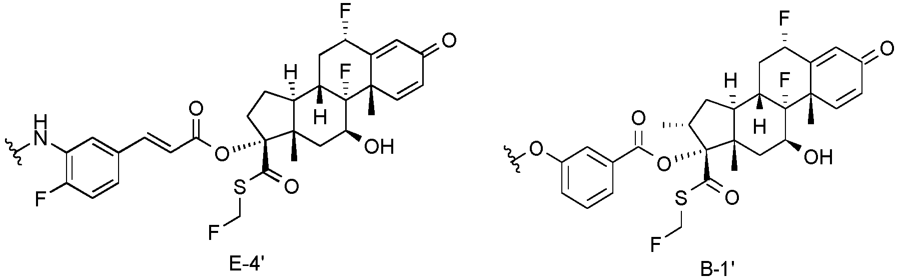 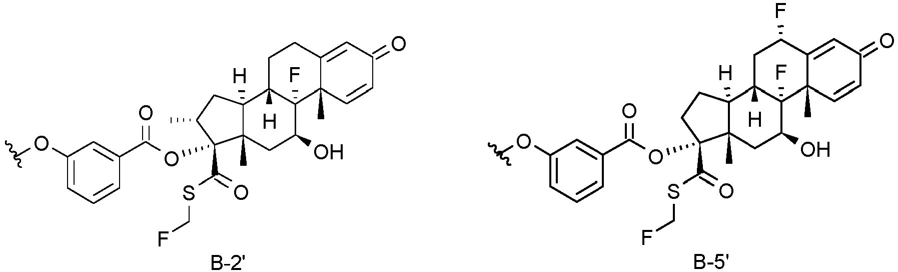 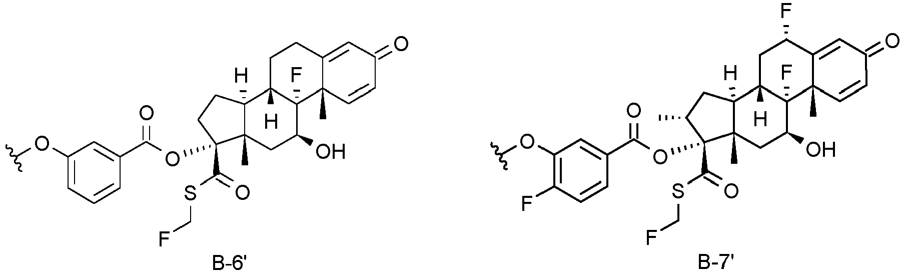 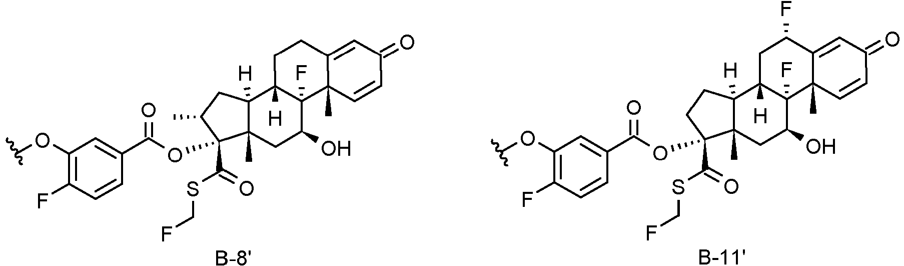 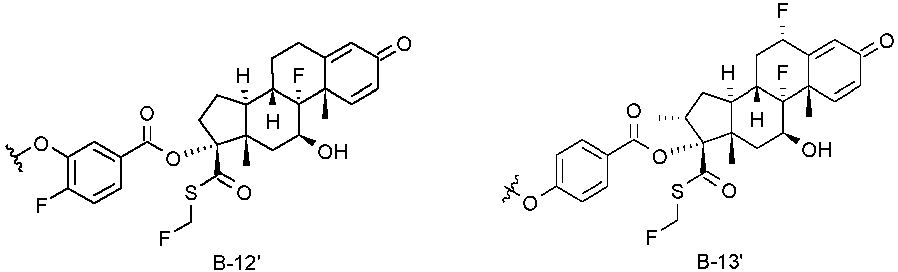 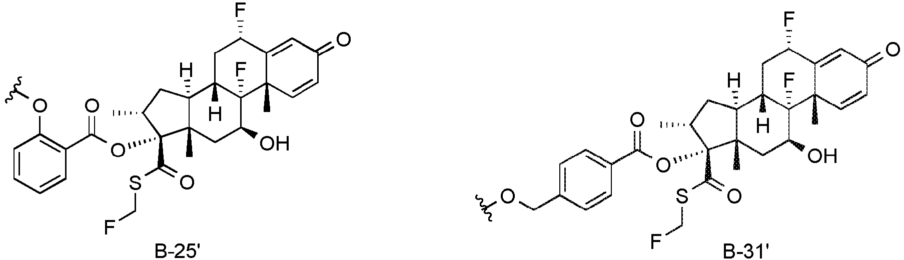 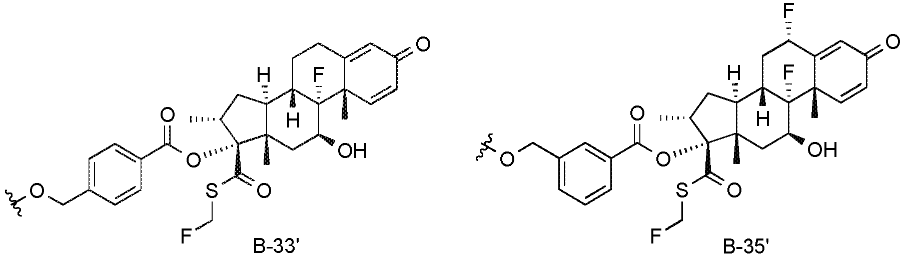 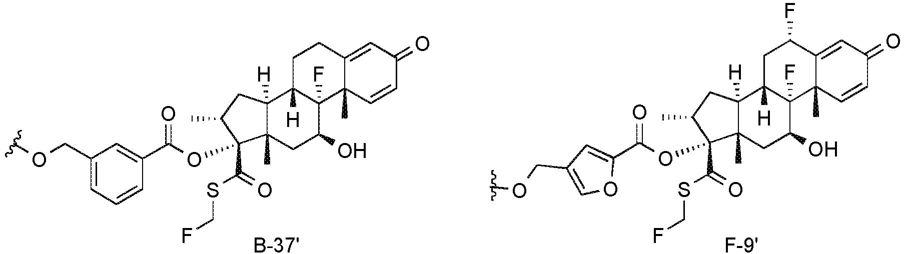 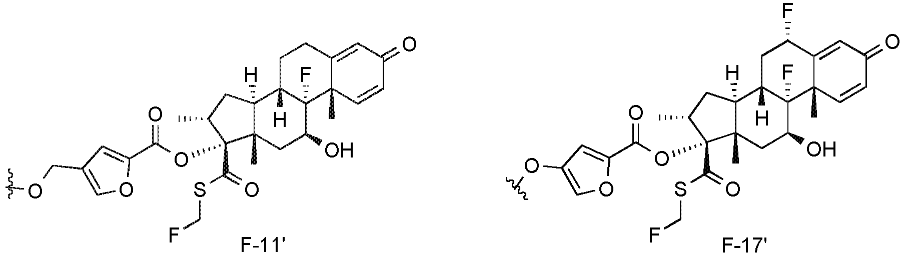 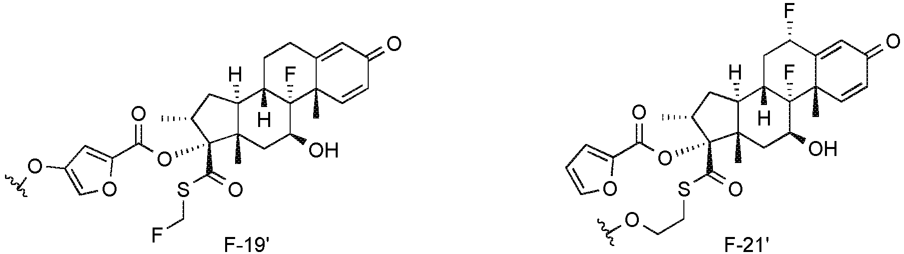 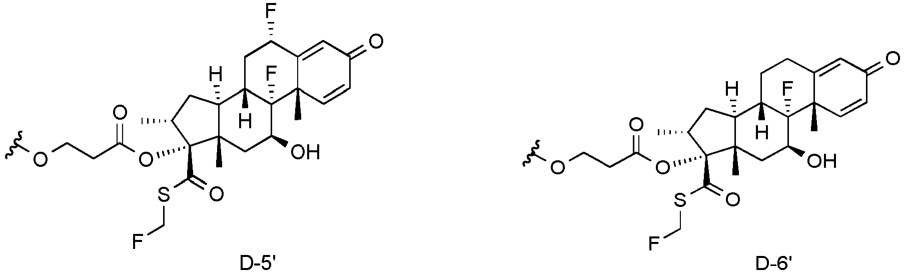 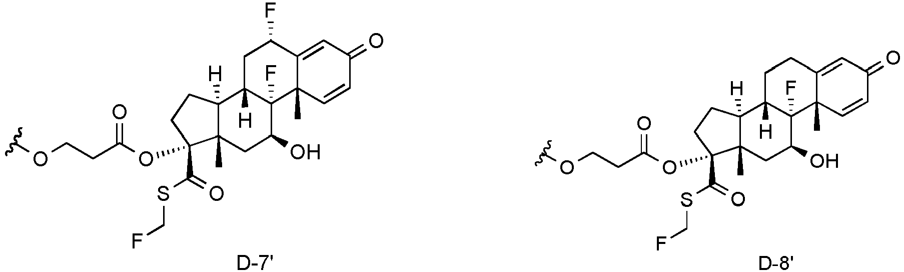 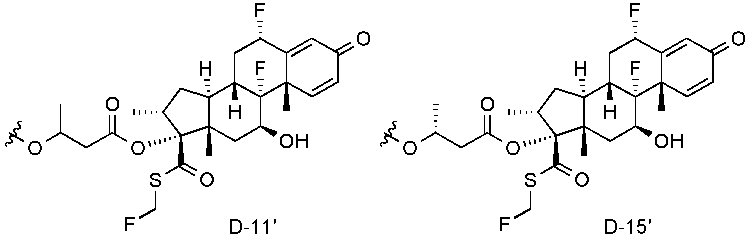 and 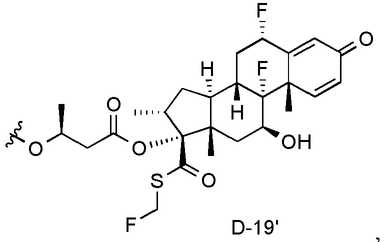
more preferably, D is selected from the following structures: 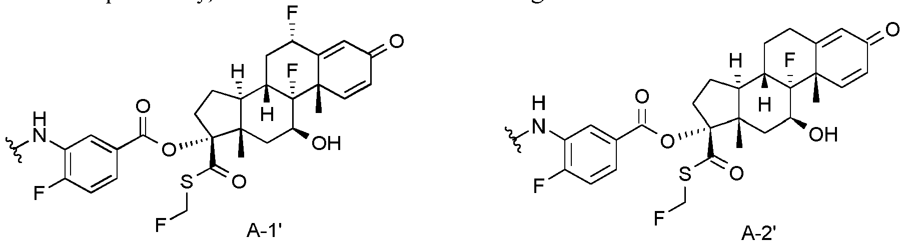 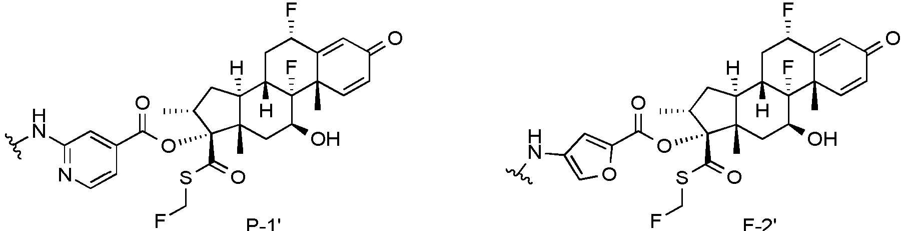 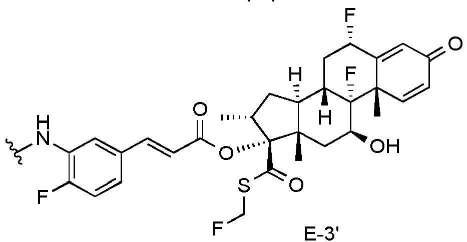 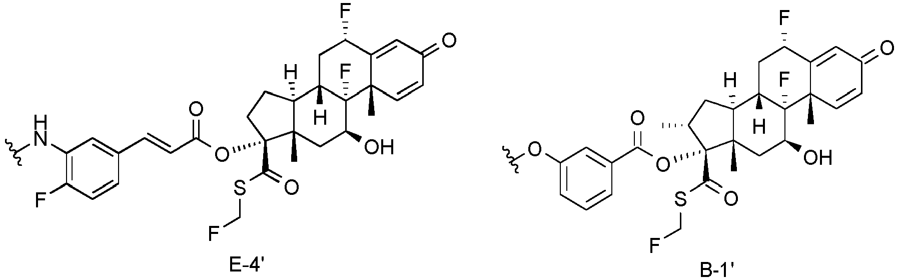 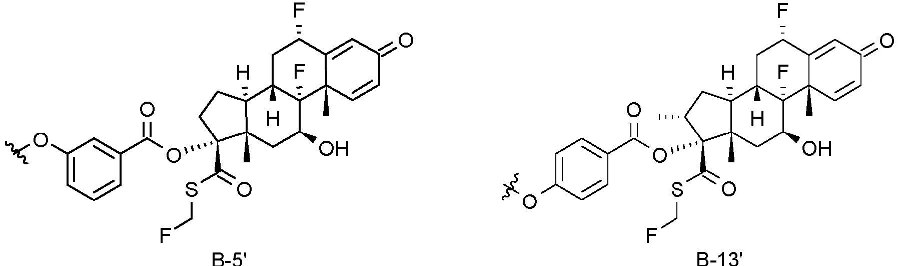 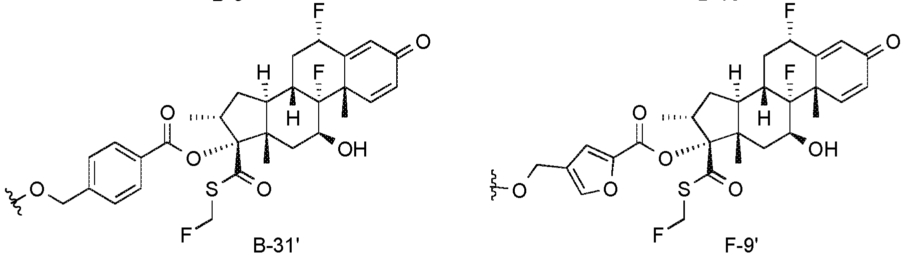 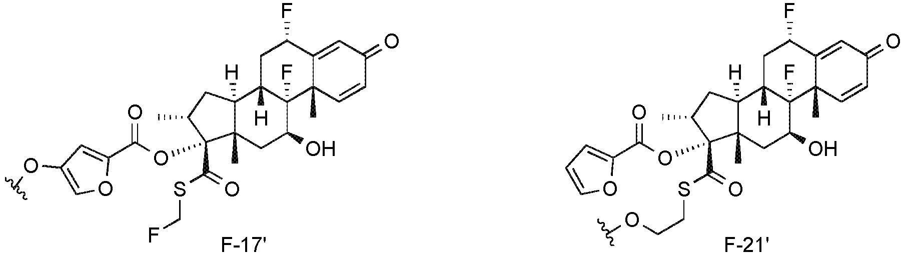 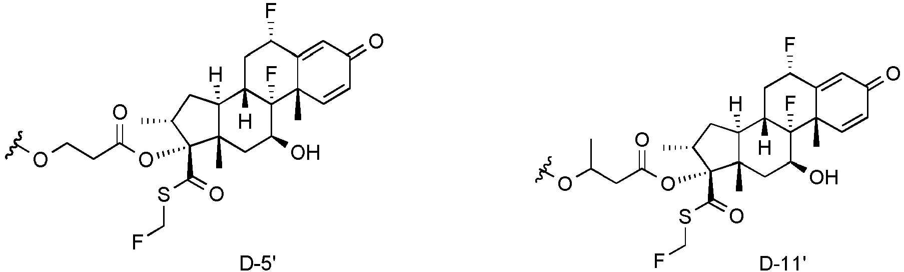 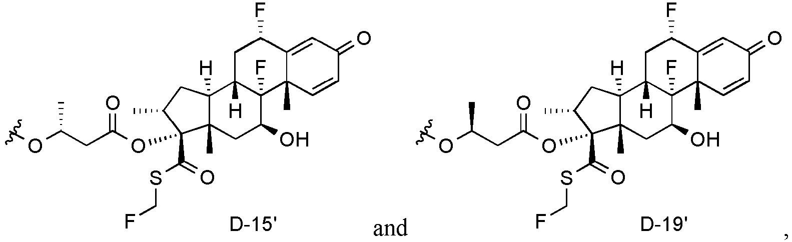
wherein, the wavy line 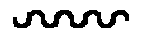 indicates the point of attachment between the moiety and the remainder of the molecule.

10. The antibody-drug conjugate according to claim 9, wherein the antigen is selected from a group consisting of TNFα, IL6R, BDCA2, NR3C1, MSR1, PRLR, CD25, CD40, CD70, CD74 and CD163.

11. The antibody-drug conjugate according to claim 9 or 10, wherein the antibody is selected from a group consisting of Adalimumab and Tocilizumab.

12. The antibody-drug conjugate according to any one of claims 9-11, wherein
M is 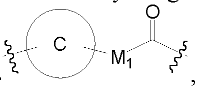 wherein ring C is a single bond, halogen, a 5-6 membered aliphatic heterocyclic ring or a 5-20 membered aromatic ring system, the aliphatic heterocyclic ring or the aromatic ring system being optionally substituted with one or more groups each independently selected from a group consisting of oxo, halogen, cyano, amino, carboxyl, mercapto and C₁₋₆ alkyl; and M₁ is selected from a group consisting of a single bond or a fragment consisting of one or more groups selected from -O-, -NH-, -C(=O)-, - S(=O)₂-, -C=N-O-, -NH-S(=O)₂-NH-, phenylene, 5-10 membered heteroarylidene, C₁₋₂₀ alkylene, C₂₋₂₀ alkenylidene and C₂₋₂₀ alkynylidene;
preferably, M is 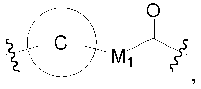 wherein ring C is a single bond, halogen, a 5 membered aliphatic heterocyclic ring, a 6 membered heteroaromatic ring, or a polycyclic ring formed by 2-5 (preferably 3) units selected from a 6 membered heteroaromatic ring and a benzene ring linked through a single bond, the aliphatic heterocyclic ring, heteroaromatic ring or polycyclic ring being optionally substituted with one or more groups selected from a group consisting of oxo, halogen and C₁₋₄ alkyl; and M₁ is selected from a group consisting of a single bond or a fragment consisting of one or more groups selected from -NH-, -C(=O)-, -NH-S(=O)₂-NH-, C₁₋₁₀ alkylene, C₂₋₁₀ alkenylidene and C₂₋₁₀ alkynylidene;
preferably, M is 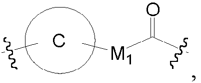 wherein ring C is selected from a group consisting of 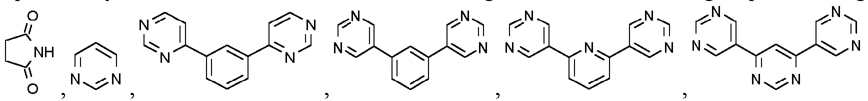 and 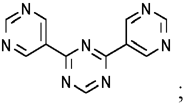 and M₁ is selected from a group consisting of a single bond, -NH-, - NH-S(=O)₂-NH-, C₁₋₆ alkylene, C₂₋₆ alkenylidene and C₂₋₆ alkynylidene;
preferably, M is selected from a group consisting of 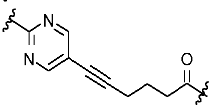 and 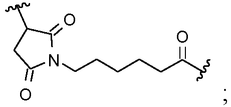
preferably, M is selected from 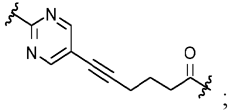
preferably, M is selected from a group consisting of 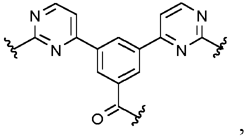 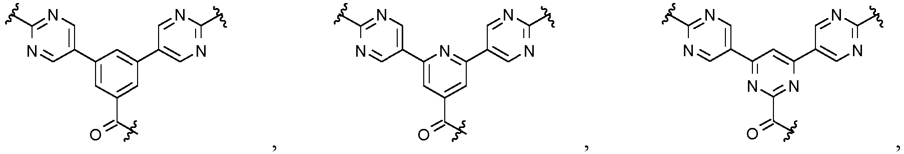 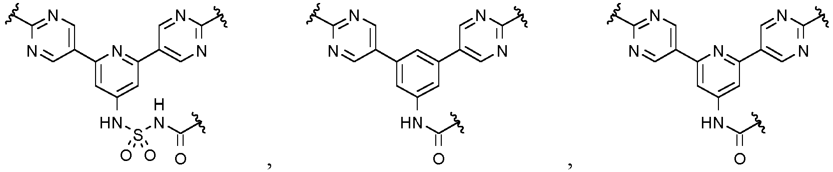 and 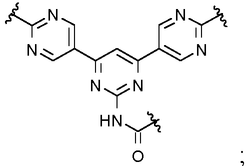
preferably, M is selected from a group consisting of 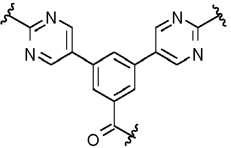 and 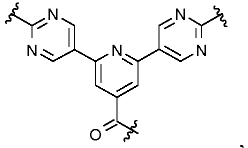
wherein, the wavy line 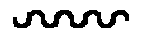 indicates the point of attachment between the moiety and the remainder of the molecule.

13. The antibody-drug conjugate according to any one of claims 9-12, wherein L is a structure consisting of one or more moieties selected from a group consisting of C₁₋₆ alkylene, -N(R')-, carbonyl, -O-, a natural amino acid or an unnatural amino acid and an analogue or a derivative thereof (such as Ala, Arg, Asn, Asp, Cit, Cys, Gln, Glu, Gly, His, Ile, Leu, Lys, Met, Phe, Pro, Ser, Thr, Trp, Tyr, Val, Lys(COCH₂CH₂(OCH₂CH₂)ᵣOCH₃)), and a short chain polypeptide consisting of amino acids (such as Ala-Ala, Ala-Lys, Ala-Lys(Ac), Ala-Pro, Gly-Glu, Gly-Gly, Phe-Lys, Phe-Lys(Ac), Val-Ala, Val-Lys, Val-Lys(Ac), Val-Cit, Ala-Ala-Ala, Ala-Ala-Asn, Leu-Ala-Glu, Gly-Gly-Arg, Gly-Glu-Gly, Gly-Gly-Gly, Gly-Ser-Lys, Glu-Val-Ala, Glu-Val-Cit, Ser-Ala-Pro, Val-Leu-Lys, Val-Lys-Ala, Val-Lys-Gly, Gly-Gly-Phe-Gly, Gly-Gly-Val-Ala, Gly-Phe-Leu-Gly, Glu-Ala-Ala-Ala, Gly-Gly-Gly-Gly-Gly), 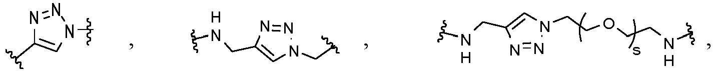 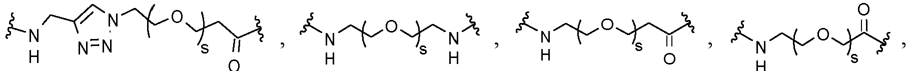 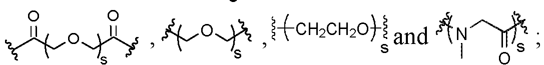
wherein R' represents hydrogen, C₁₋₆ alkyl or C₄₋₃₀ alkyl containing -(CH₂CH₂O)ᵣ-; r is an integer selected from 1 to 10, preferably an integer selected from 1 to 6; s is an integer selected from 1 to 20, preferably an integer selected from 1 to 10;
preferably, L is a structure consisting of one or more moieties selected from a group consisting of C₁₋₆ alkylene, carbonyl, -NH-, Ala-Ala, Ala-Lys, Ala-Pro, Gly-Glu, Gly-Gly, Phe-Lys, Val-Ala, Val-Lys, Val-Cit, Ala-Ala-Ala, Ala-Ala-Asn, Leu-Ala-Glu, Gly-Gly-Arg, Gly-Glu-Gly, Gly-Gly-Gly, Gly-Ser-Lys, Glu-Val-Ala, Glu-Val-Cit, Ser-Ala-Pro, Val-Leu-Lys,Val-Lys-Ala, Val-Lys-Gly, Gly-Gly-Phe-Gly, Gly-Gly-Val-Ala, Gly-Phe-Leu-Gly, Glu-Ala-Ala-Ala, Gly-Gly-Gly-Gly-Gly, 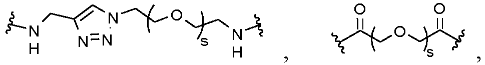 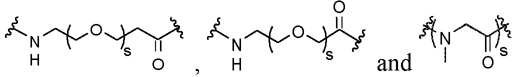 wherein s is an integer selected from 1 to 20, preferably an integer selected from 1 to 10;
preferably, L is a structure consisting of one or more moieties selected from a group consisting of: 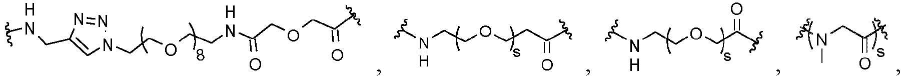 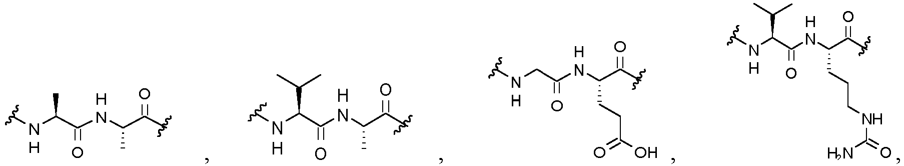 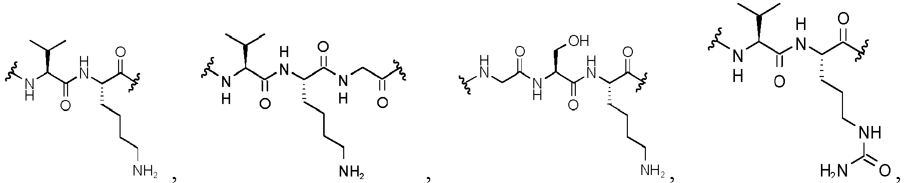 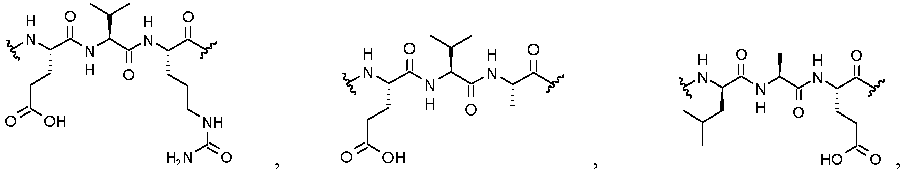 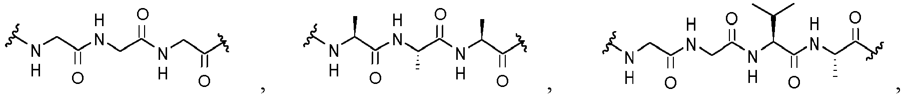 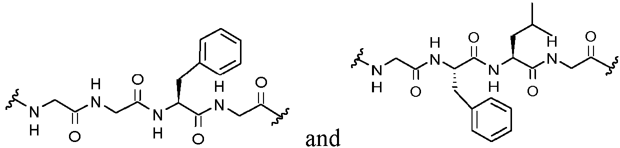 wherein s is an integer selected from 1 to 20, preferably an integer selected from 1 to 10;
preferably, L is selected from the following structures: 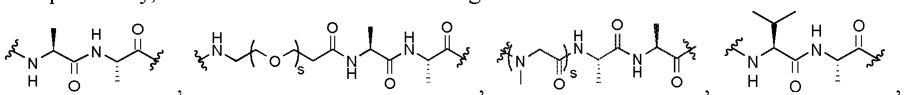 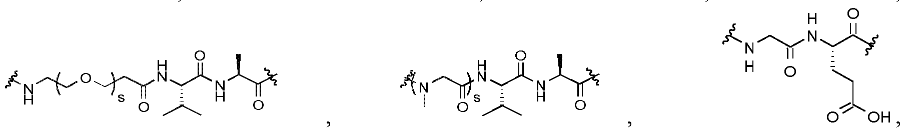 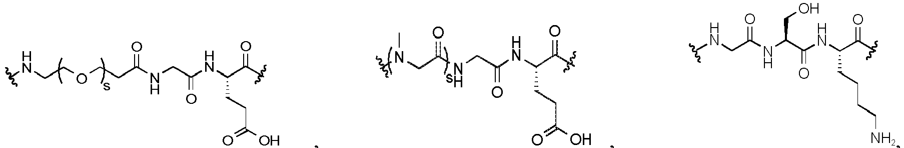 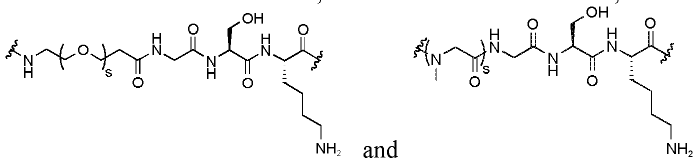 wherein s is an integer selected from 1 to 20, preferably an integer selected from 1 to 10;
preferably, L is selected from the following structures: 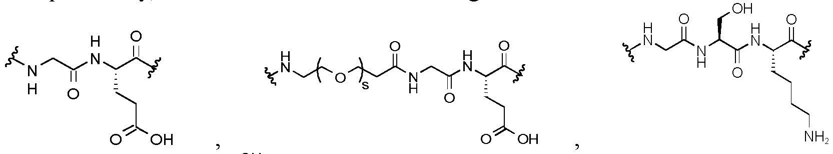 and 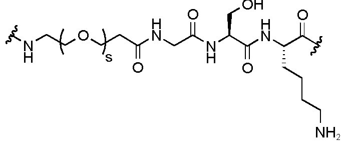 wherein s is an integer selected from 1 to 20, preferably an integer selected from 1 to 10;
wherein, the wavy line 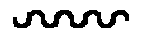 indicates the point of attachment between the moiety and the remainder of the molecule.

14. The antibody-drug conjugate according to any one of claims 9-13, wherein E is a single bond -NHCH₂-, or a structure selected from a group consisting of: 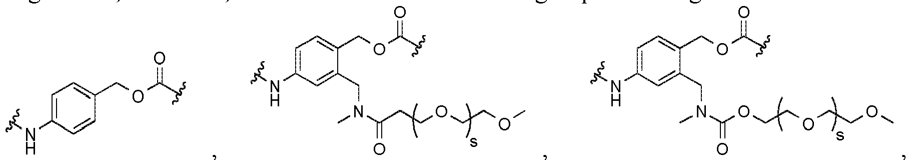 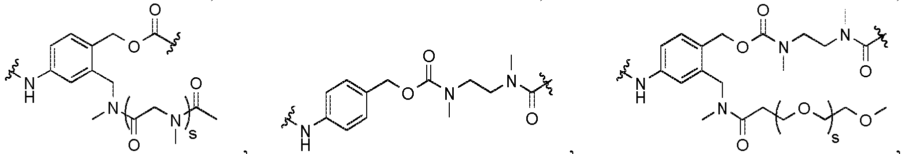 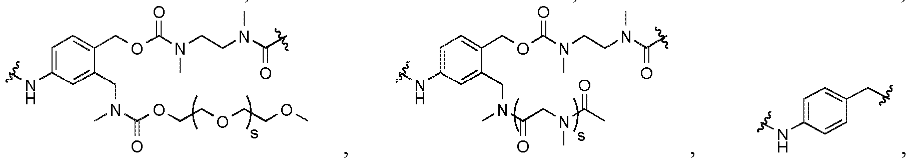 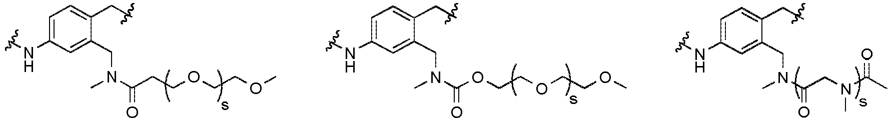 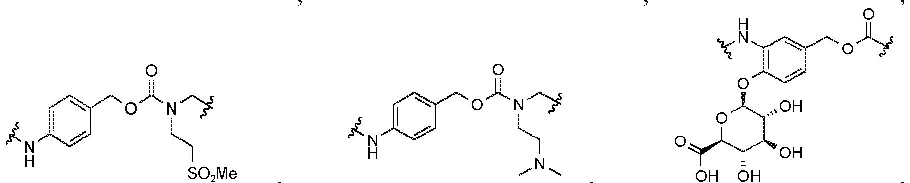 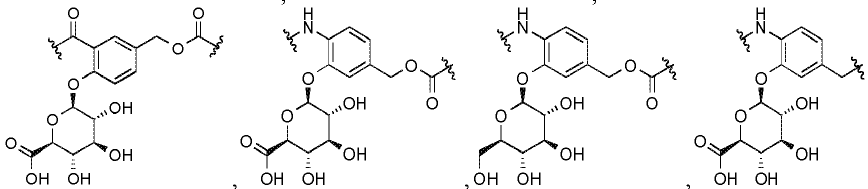 and 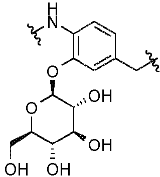 wherein s is an integer selected from 1 to 20, preferably an integer selected from 1 to 10;
preferably, E is a single bond or -NHCH₂-;
preferably, E is a single bond;
wherein, the wavy line 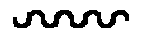 indicates the point of attachment between the moiety and the remainder of the molecule.

15. The antibody-drug conjugate according to any one of claims 9-14, wherein 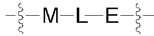 is selected from the following structures: 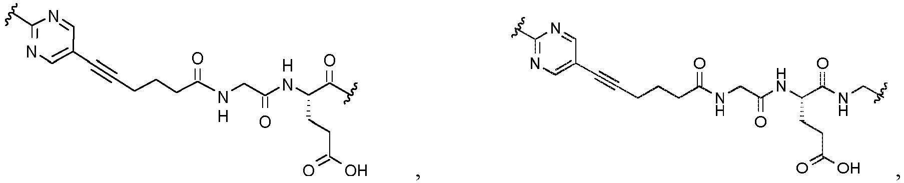 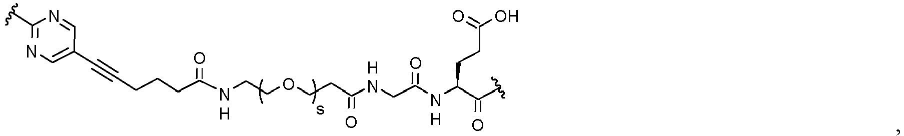 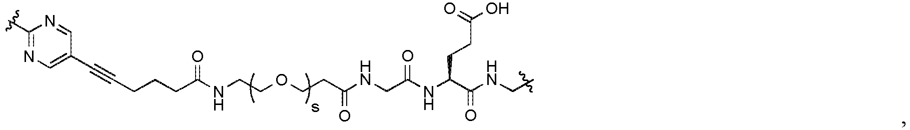 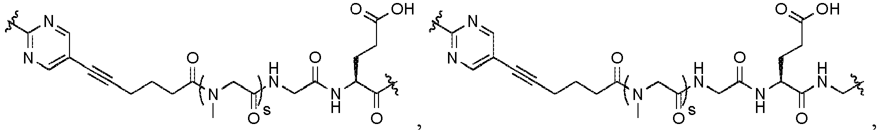 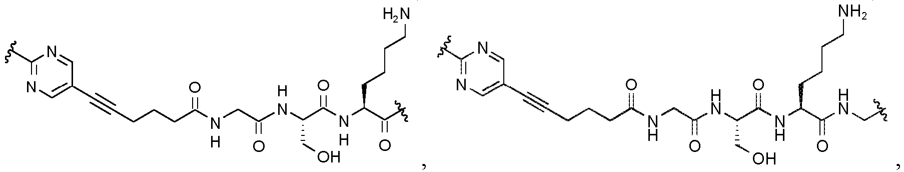 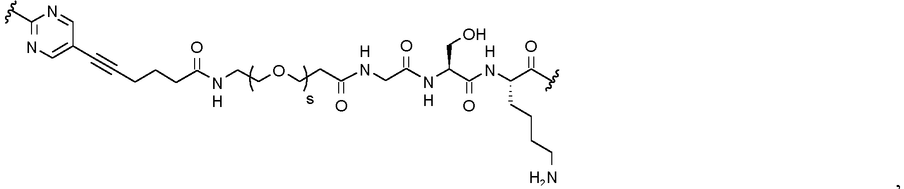 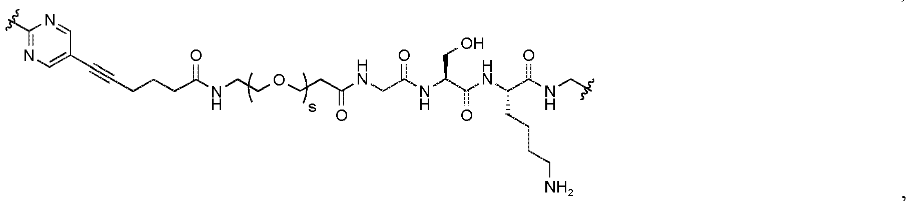 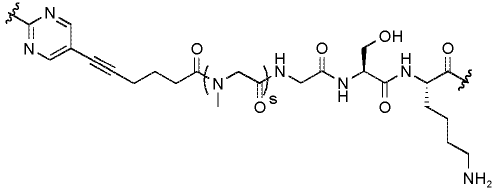 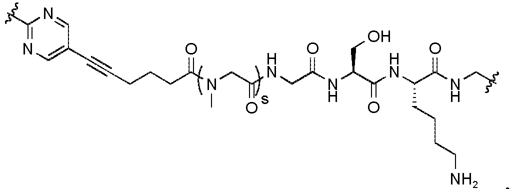 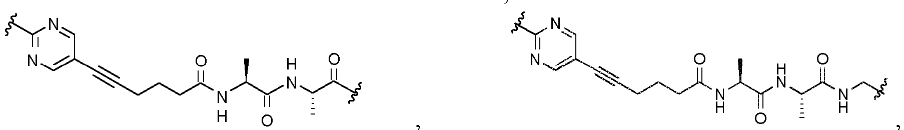 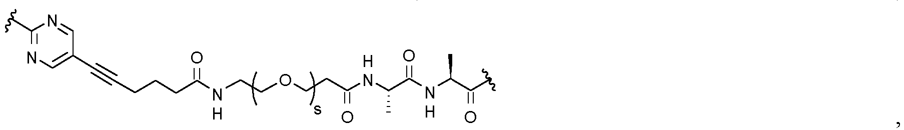 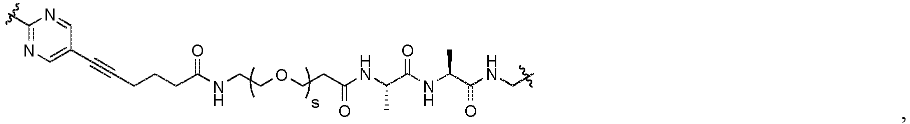 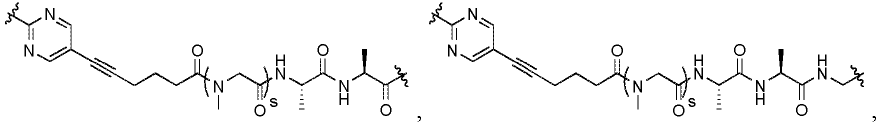 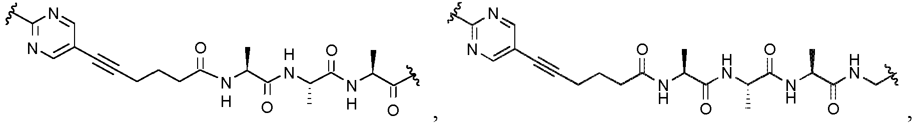 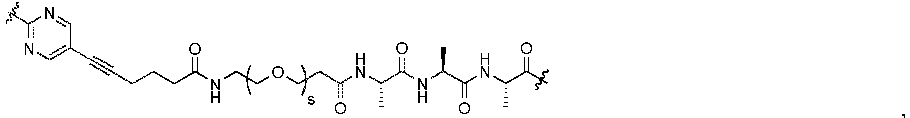 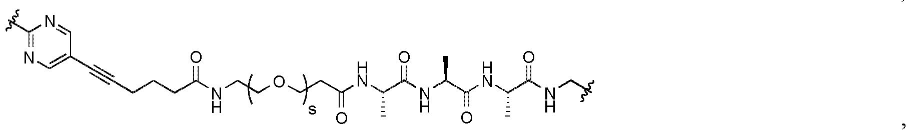 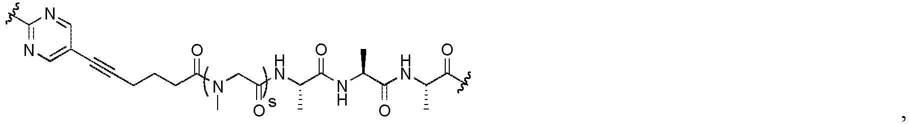 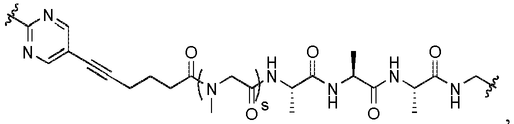 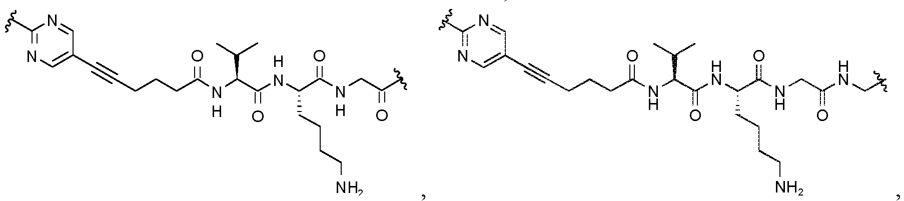 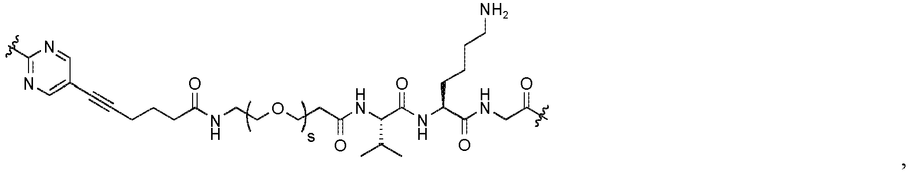 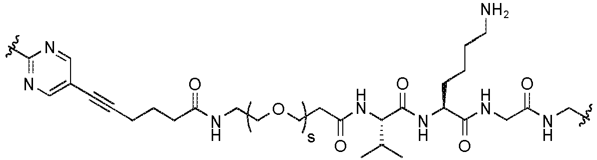 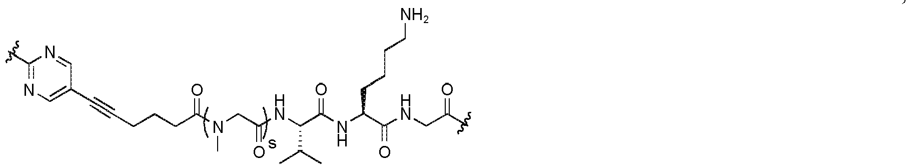 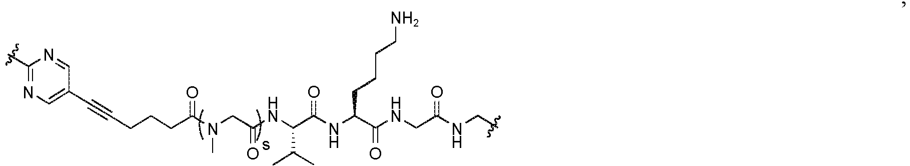 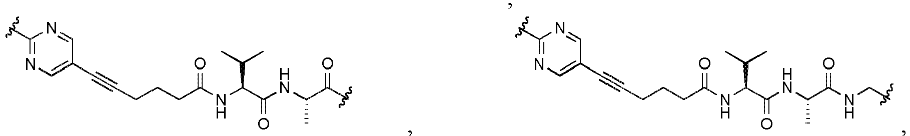 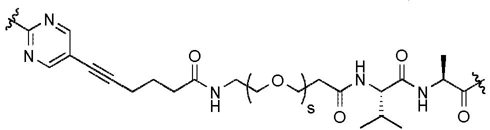 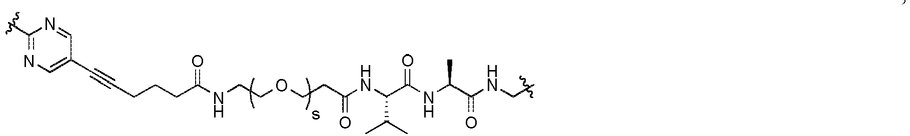 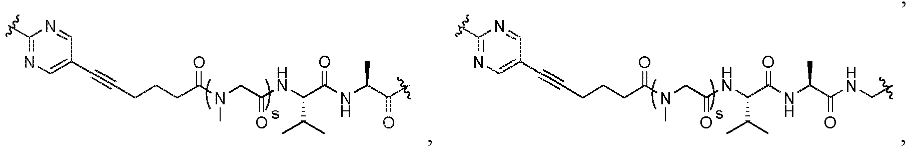 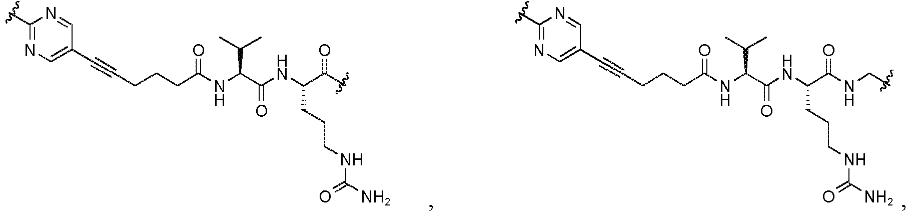 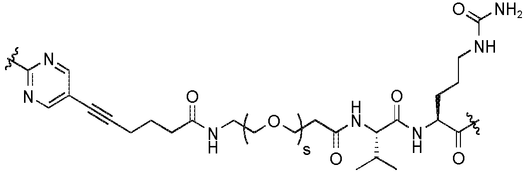 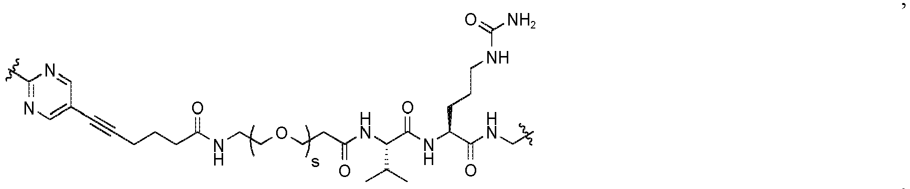 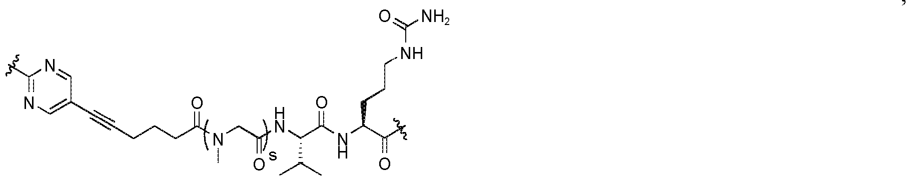 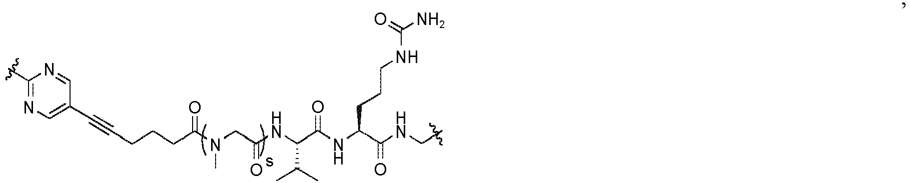 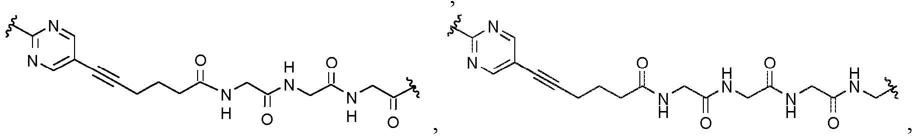 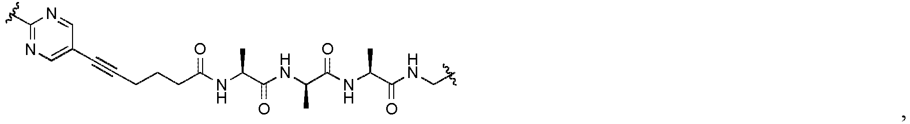 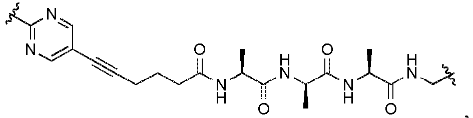 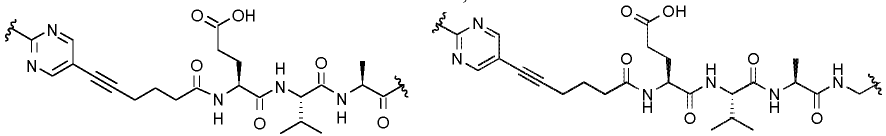 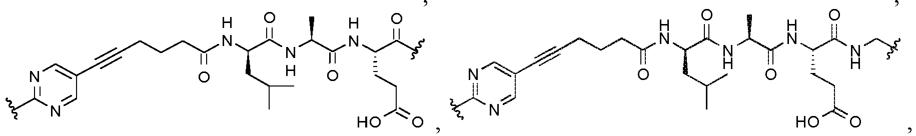 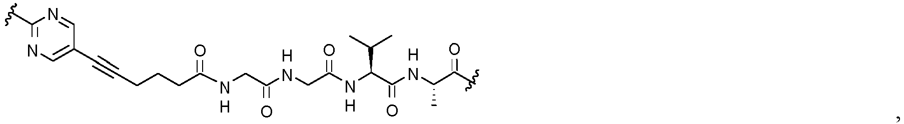 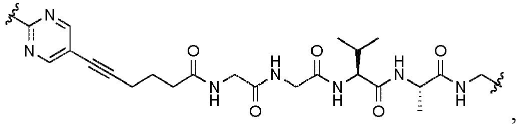 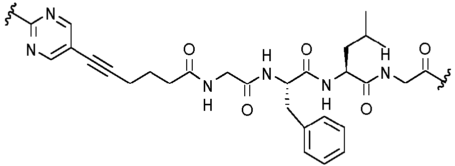 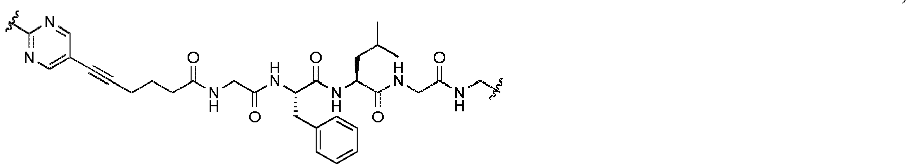 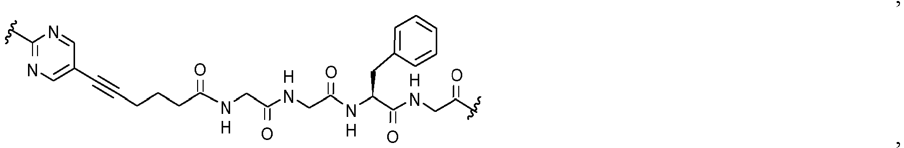 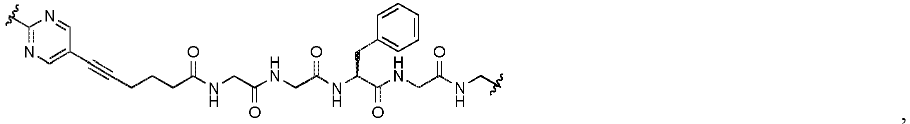 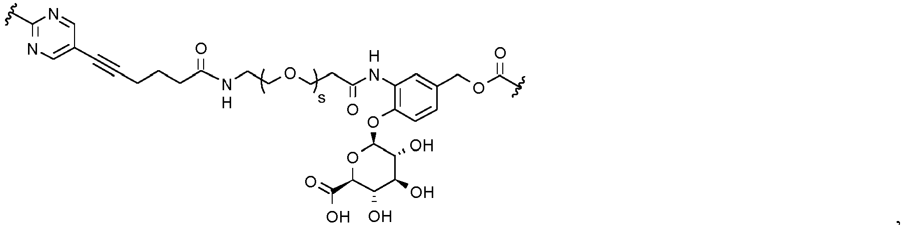 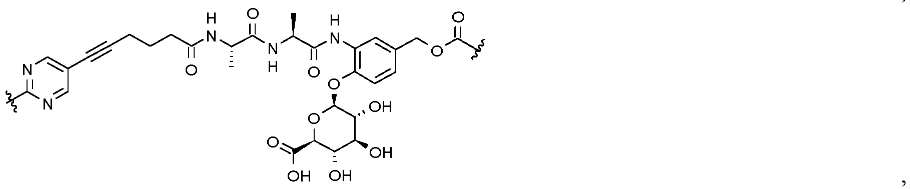 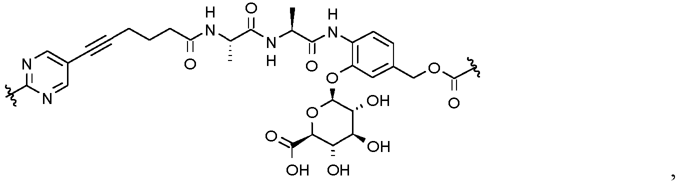 and 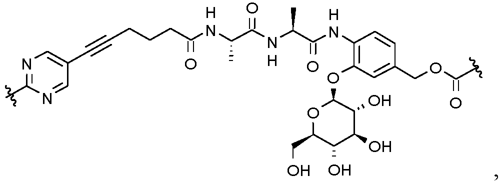
wherein, s is an integer selected from 1 to 20, preferably an integer selected from 1 to 10;
preferably, 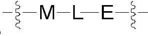
is selected from the following structures: 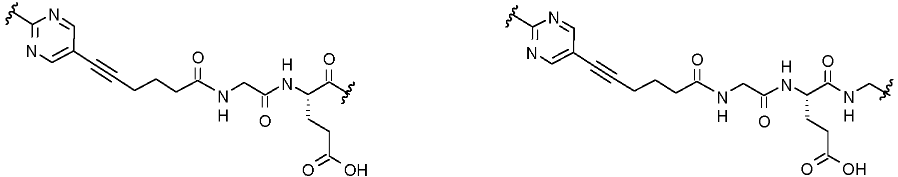 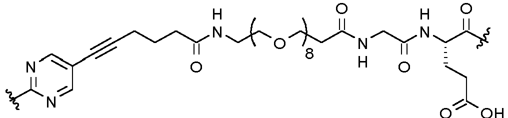 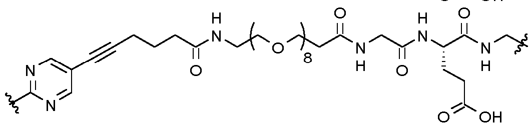 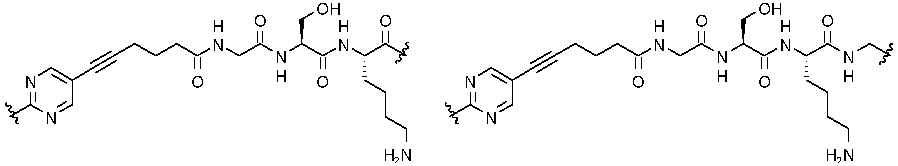 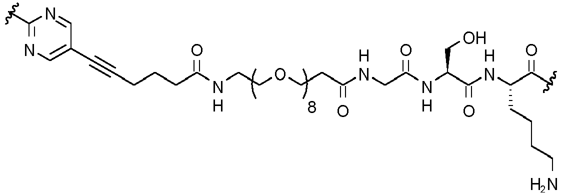 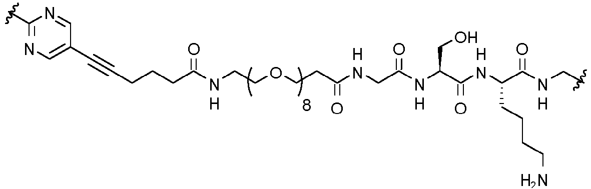 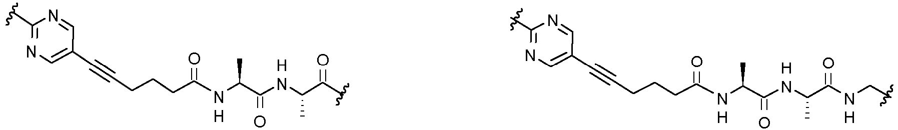 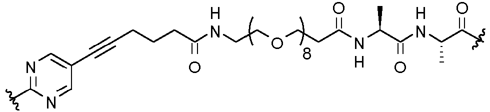 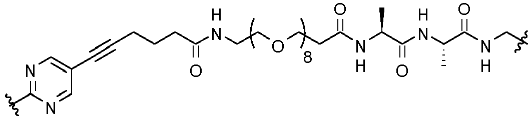 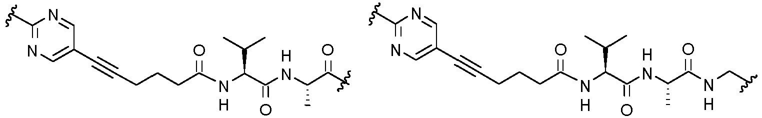 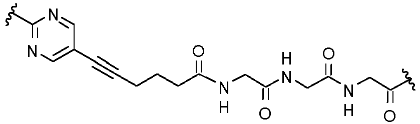 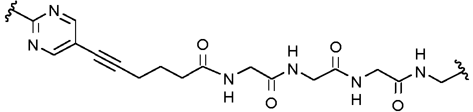 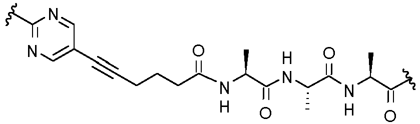 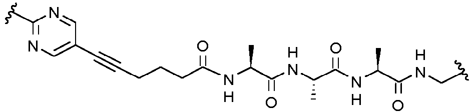 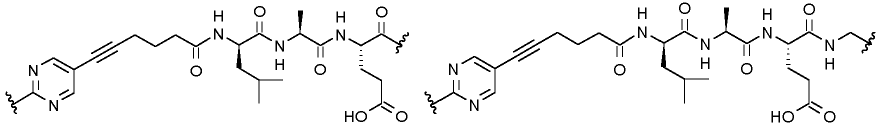 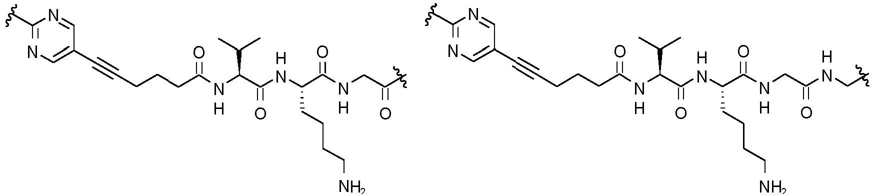 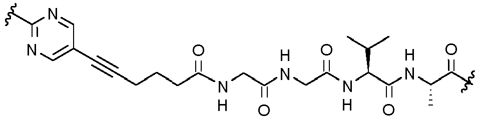 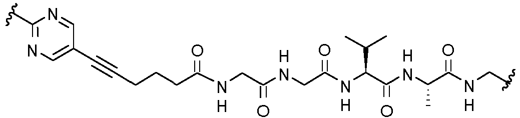 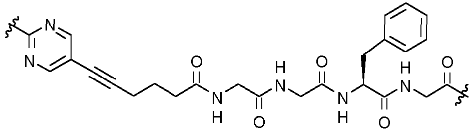 and

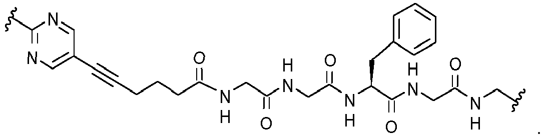

16. The antibody-drug conjugate according to any one of claims 9-14, wherein 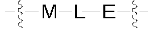 is selected from the following structures: 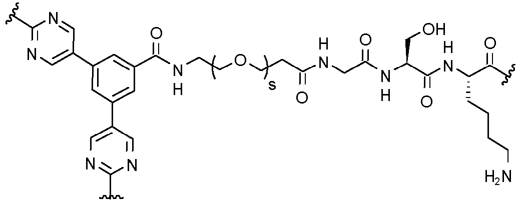 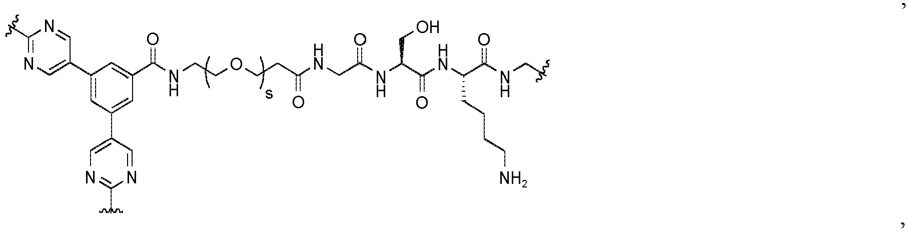 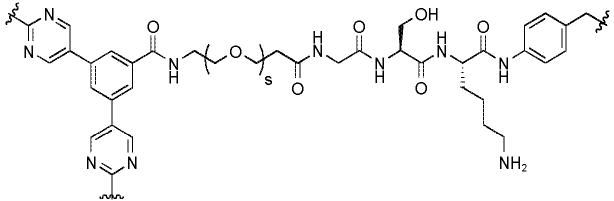 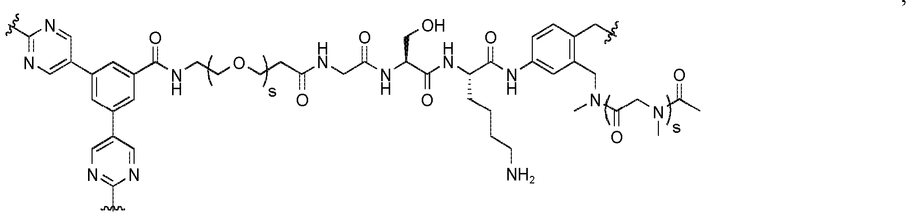 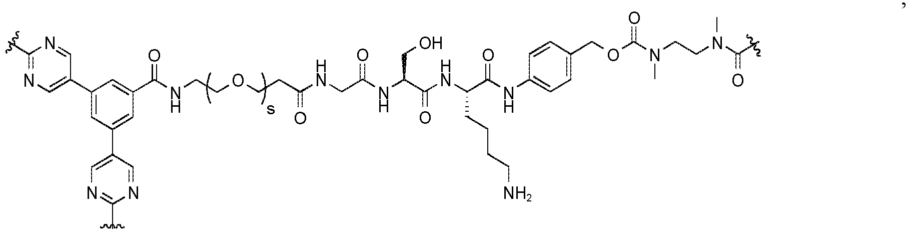 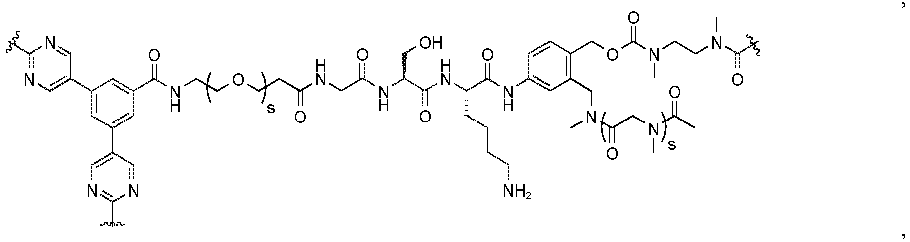 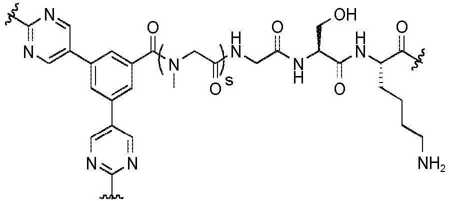 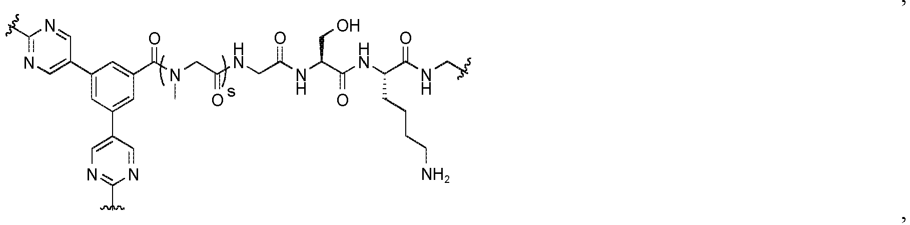 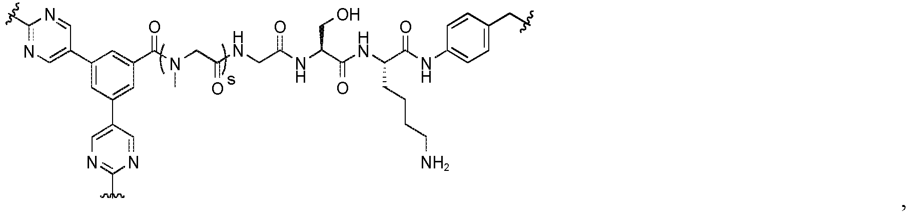 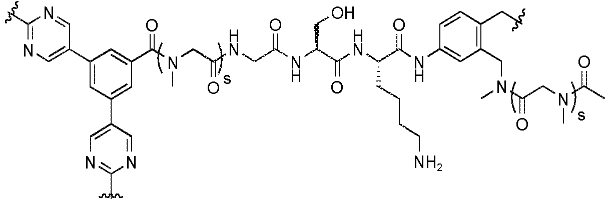 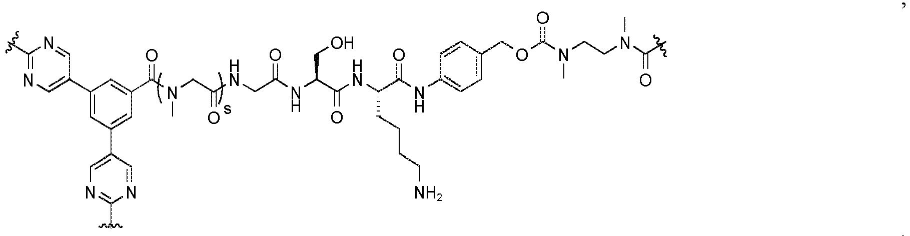 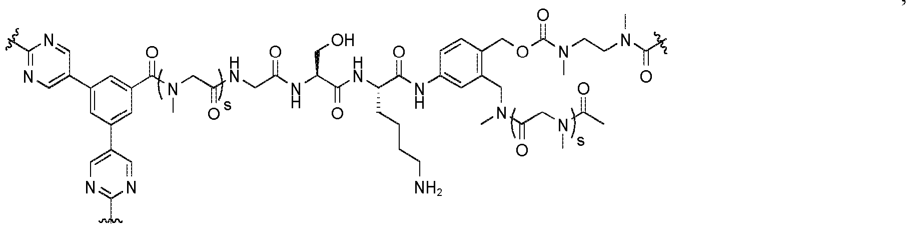 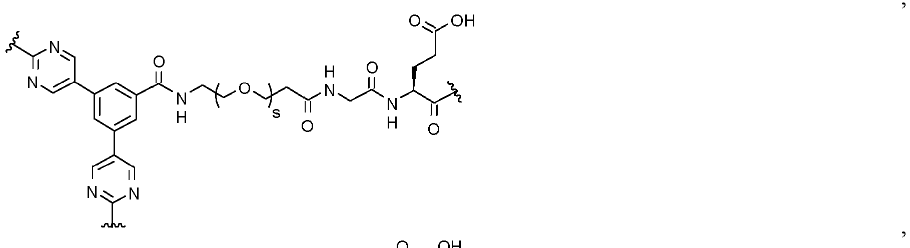 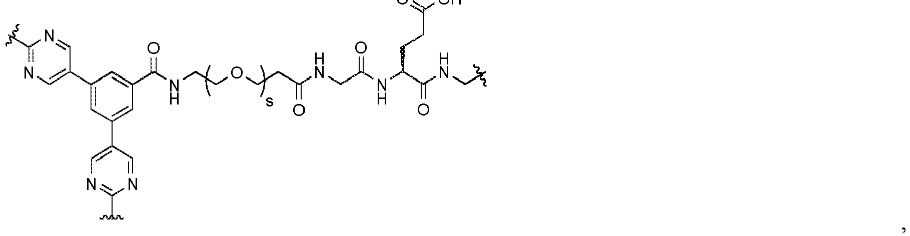 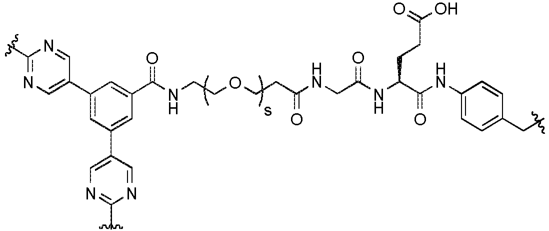 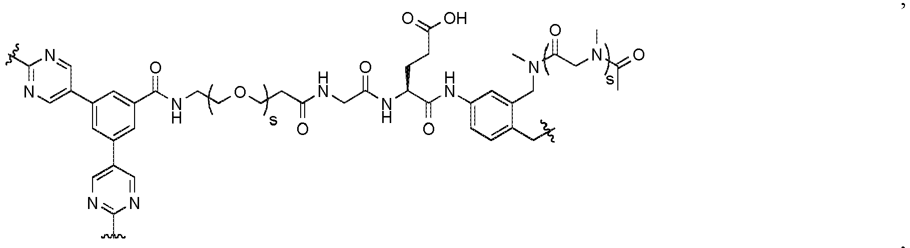 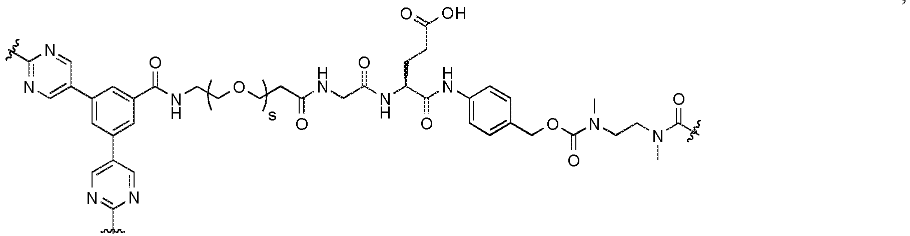 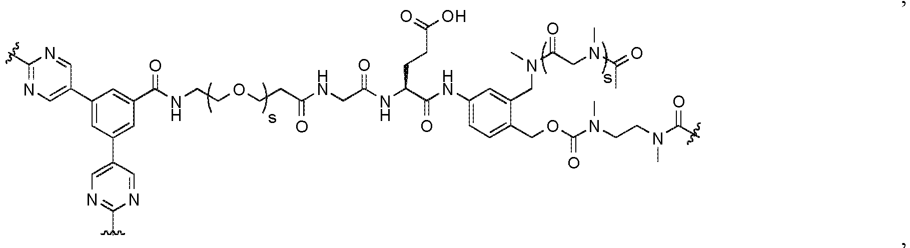 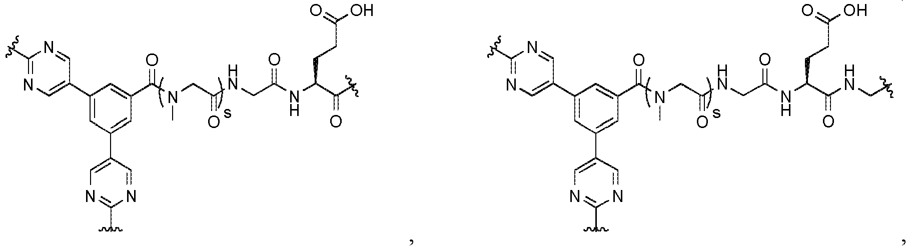 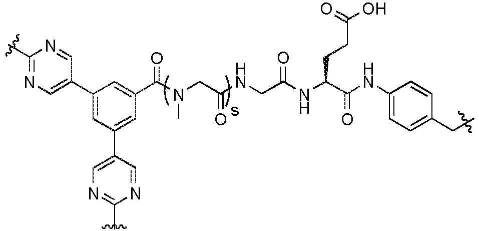 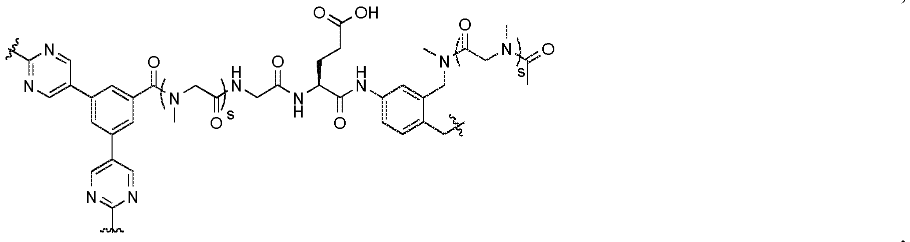 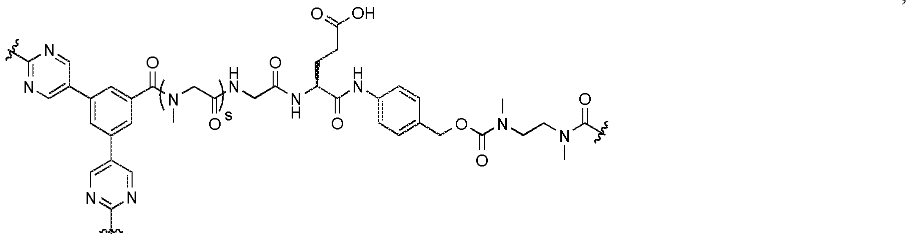 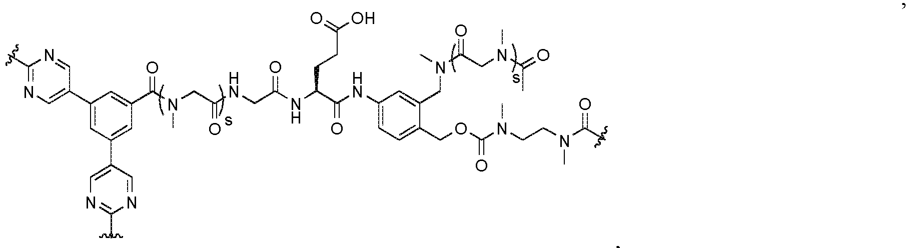 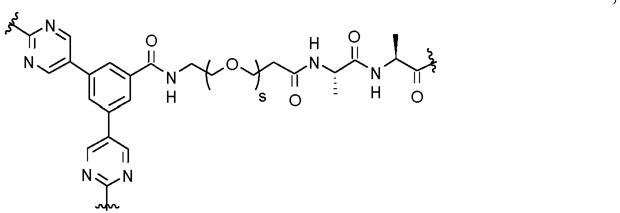 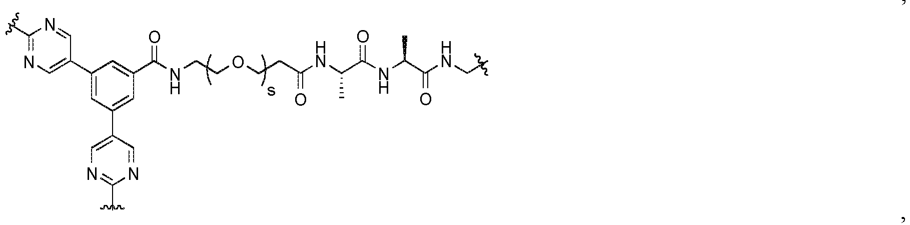 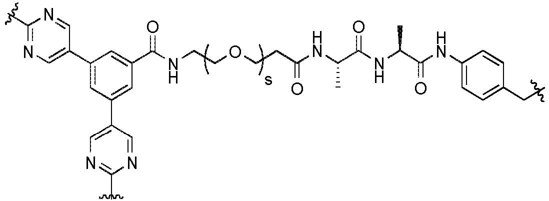 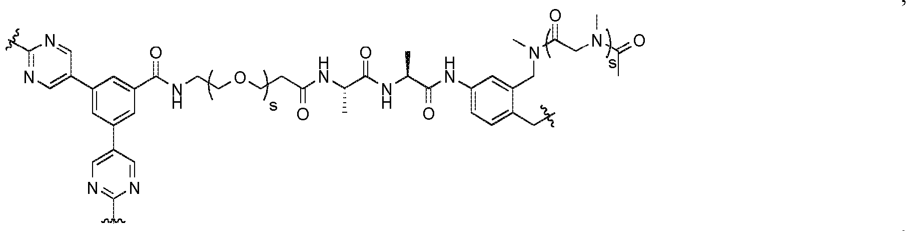 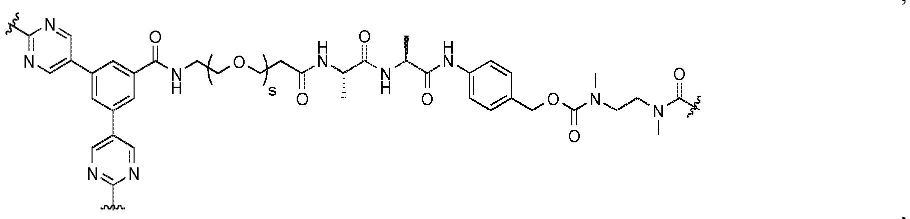 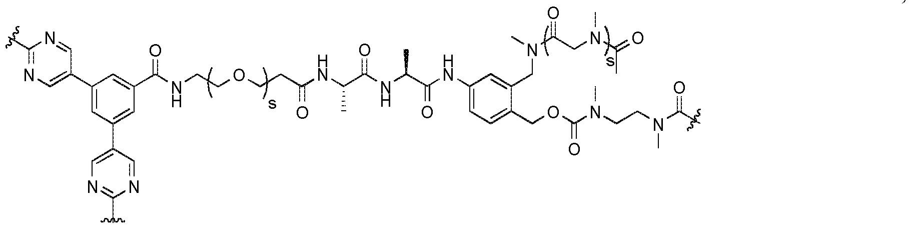 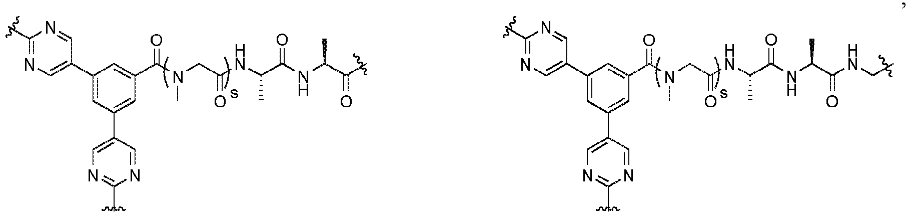 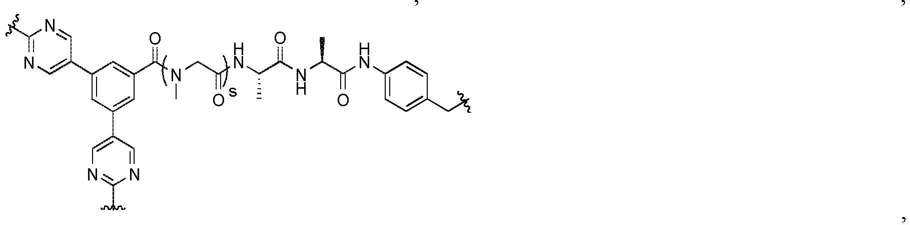 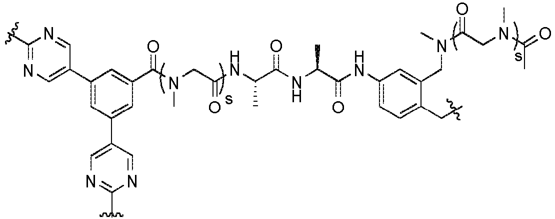 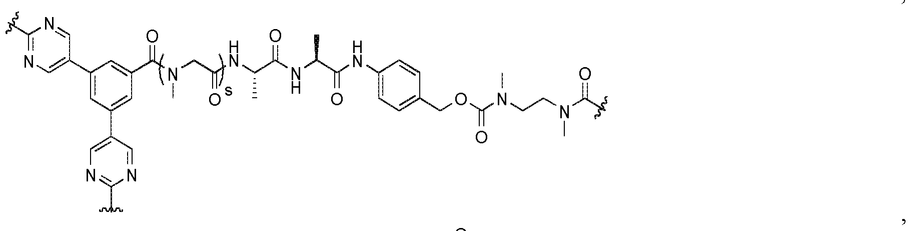 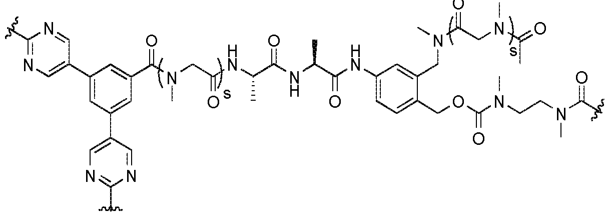 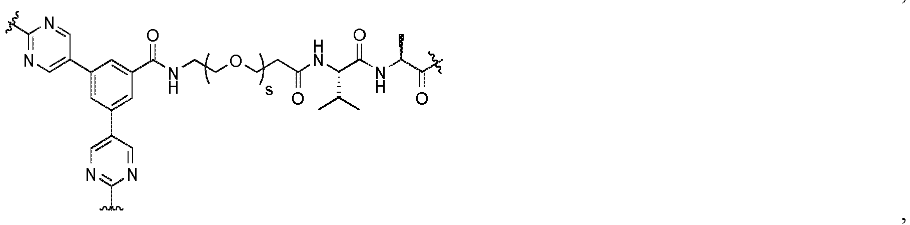 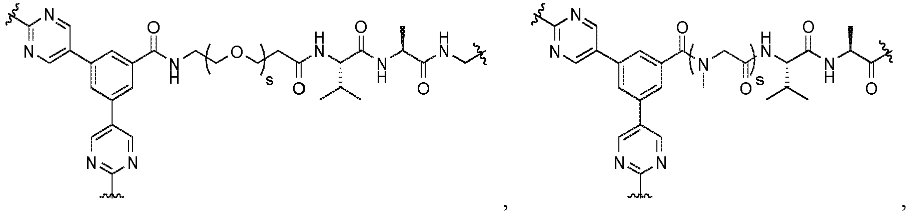 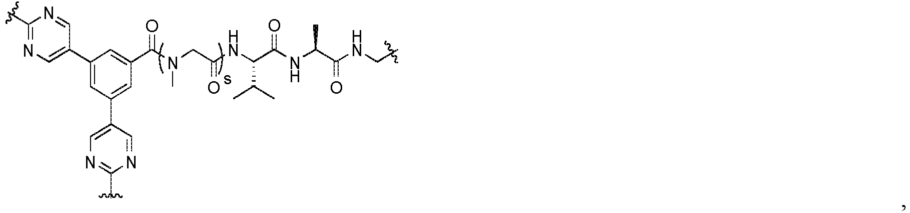 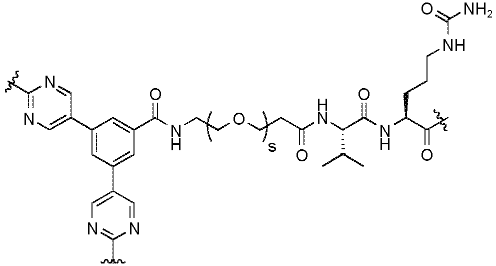 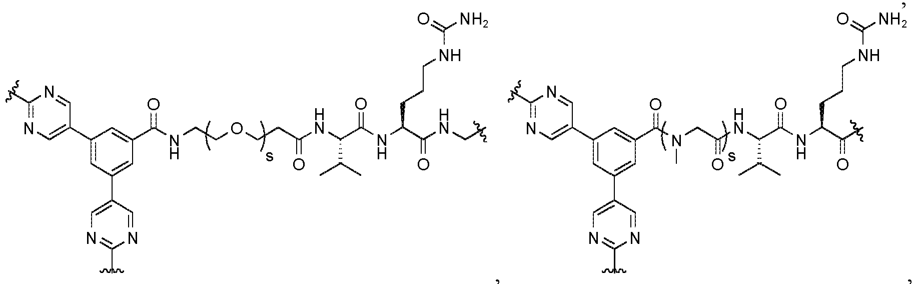 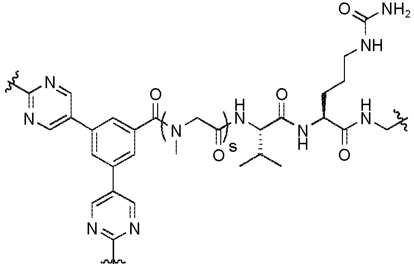 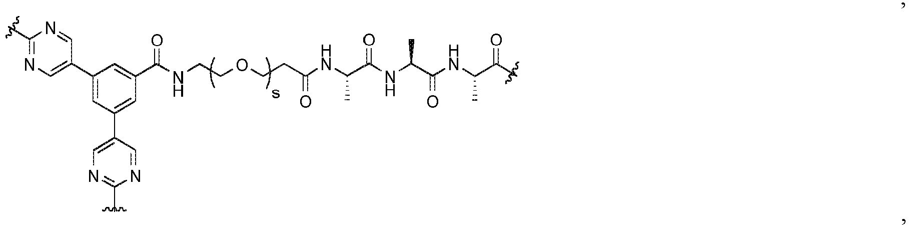 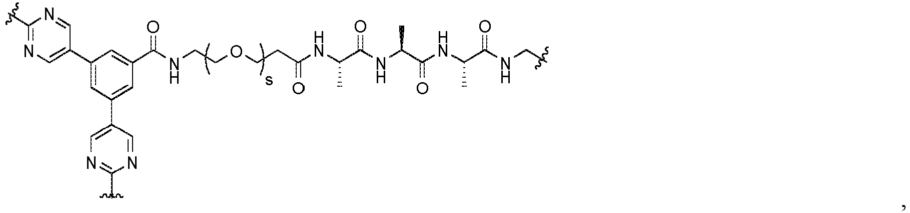 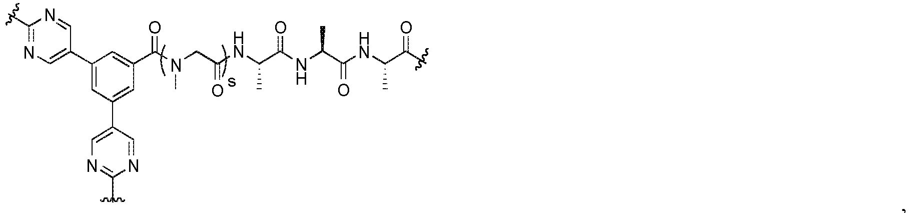 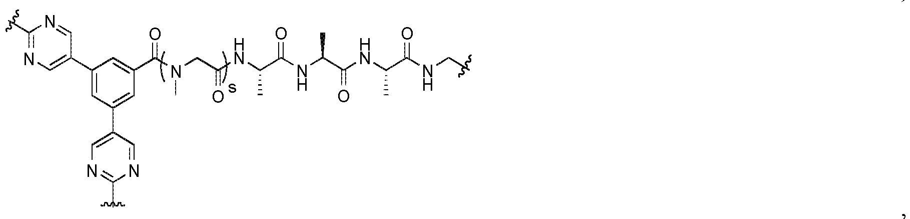 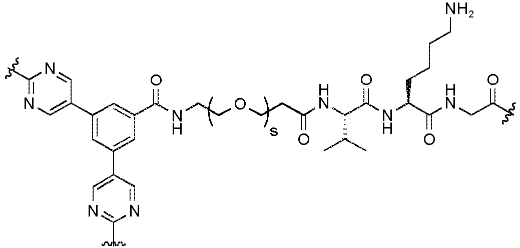 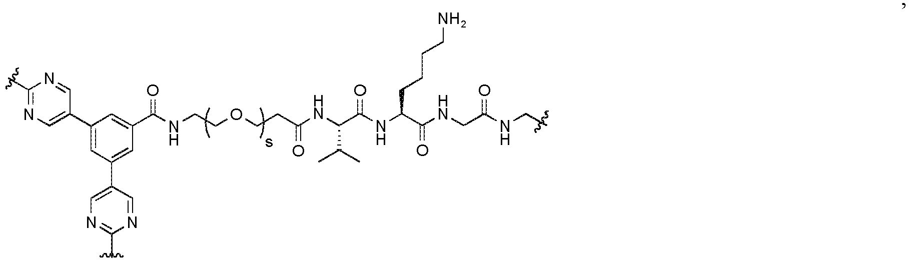 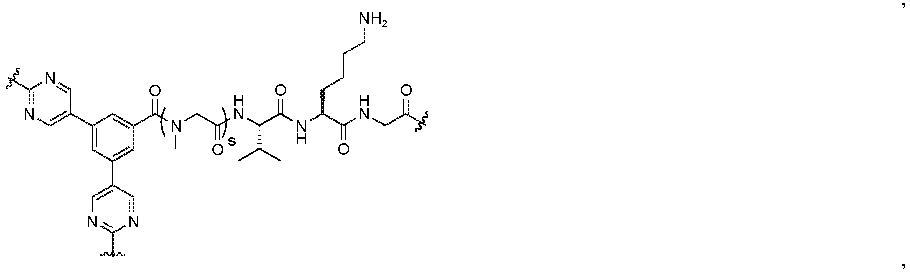 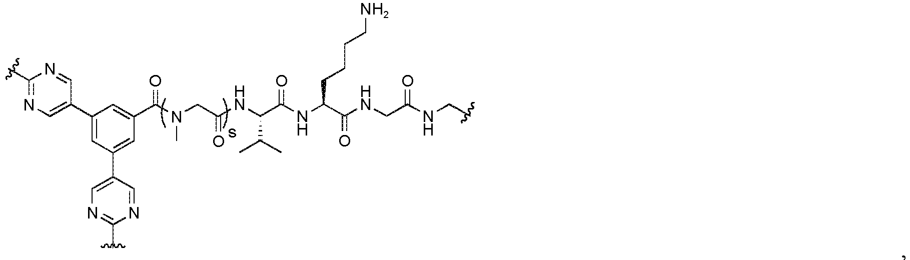 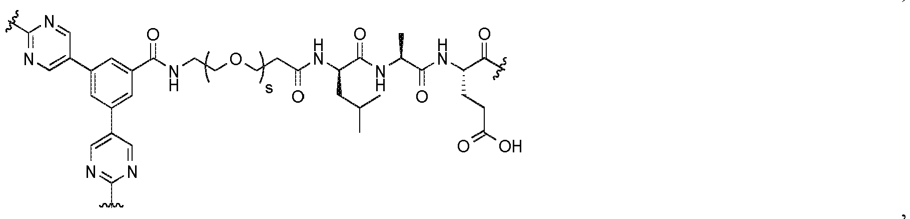 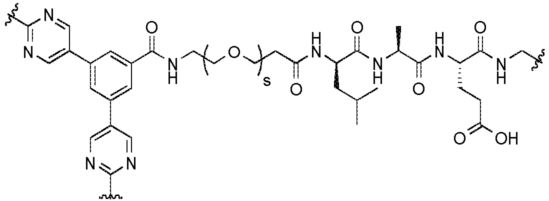 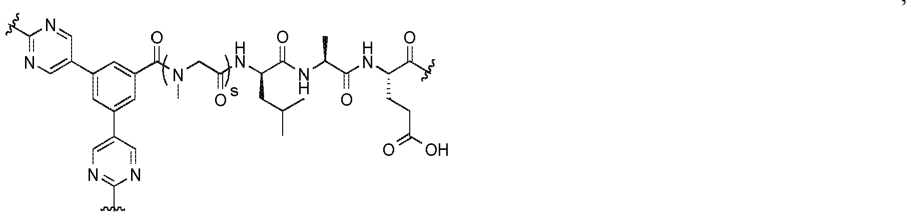 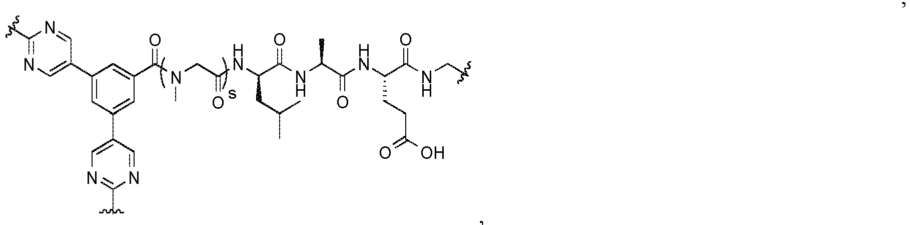 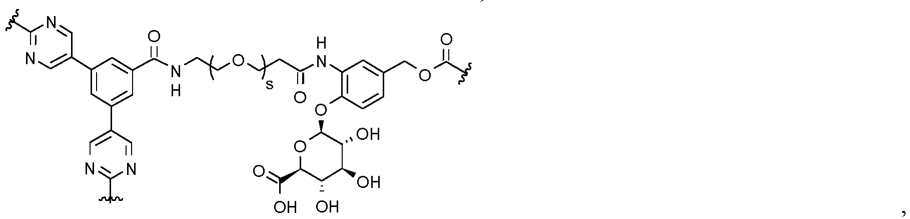 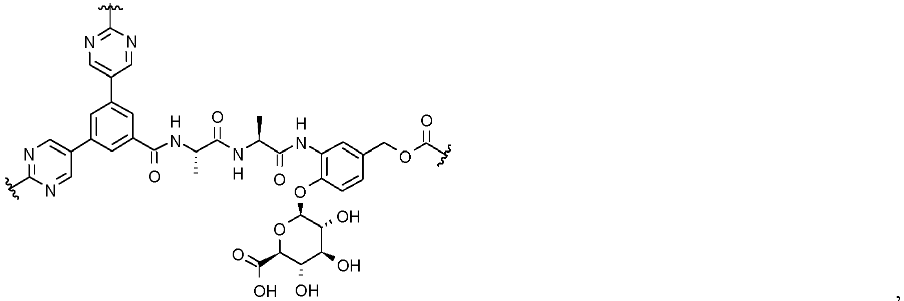 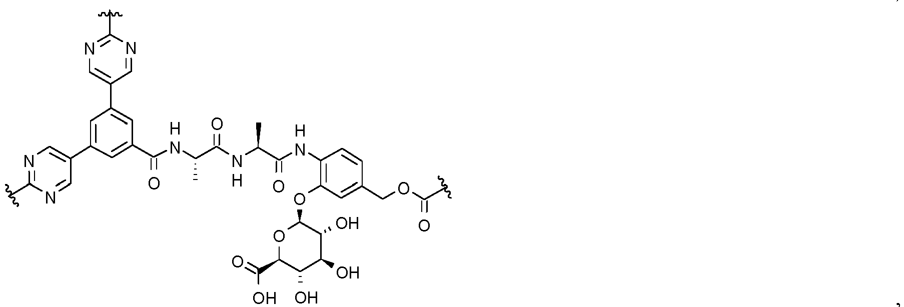 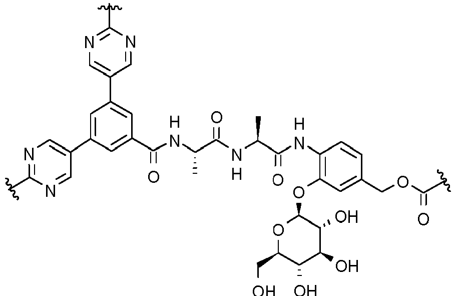
wherein, s is an integer selected from 1 to 20, preferably an integer selected from 1 to 10;
preferably, 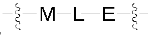
is selected from the following structures: 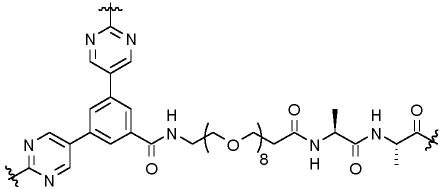 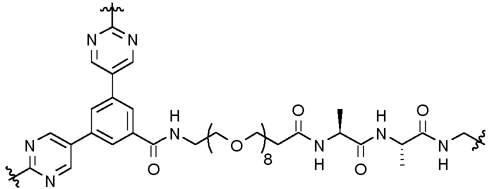 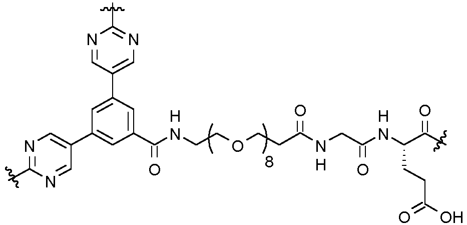 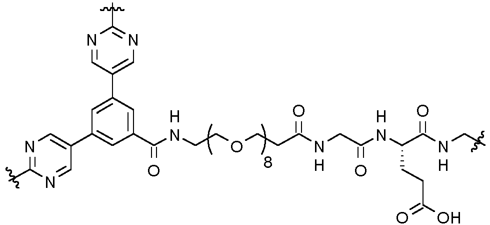 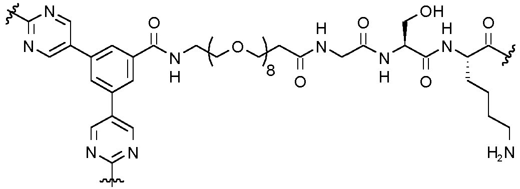 and

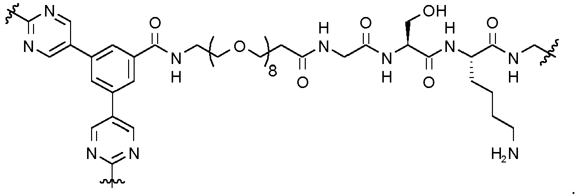

17. The antibody-drug conjugate according to any one of claims 9-16, which is selected from a group consisting of: 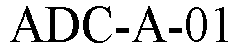 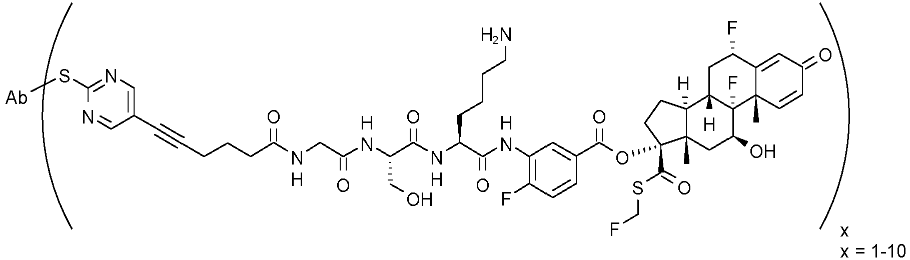 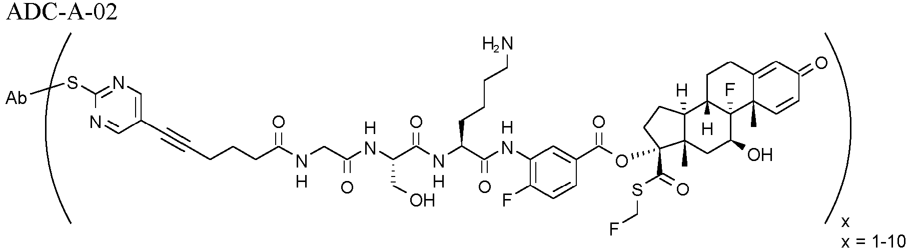 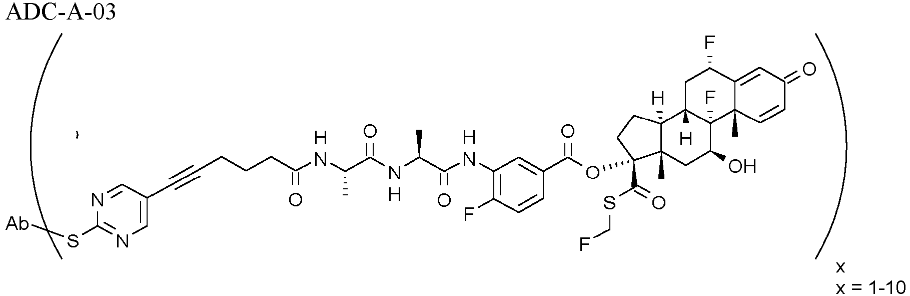 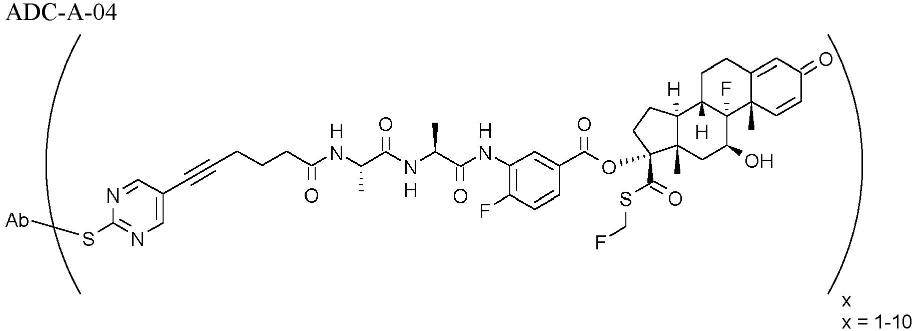 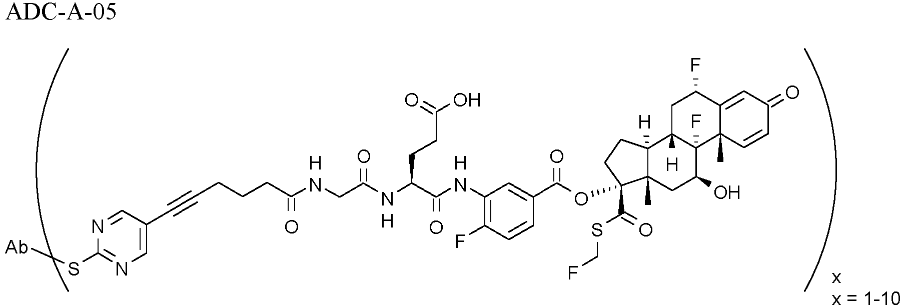 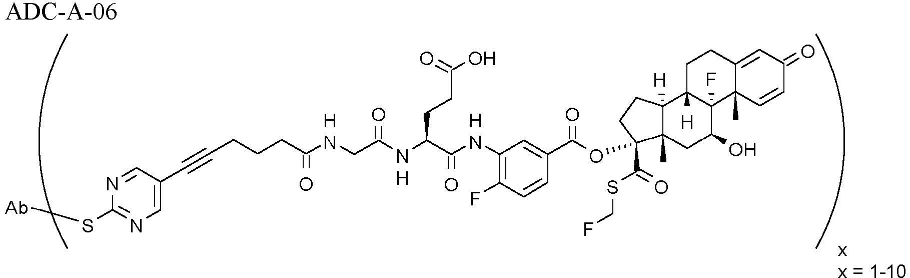 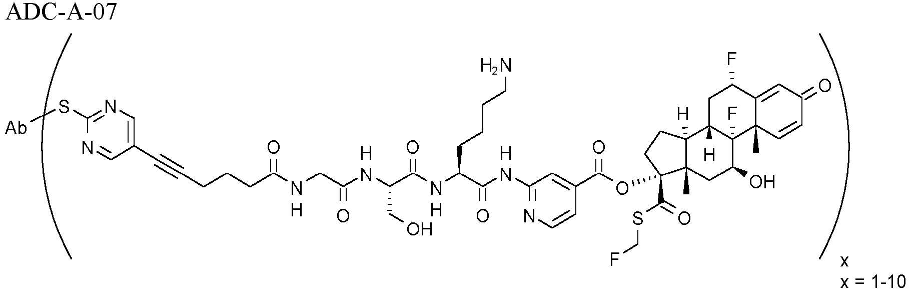 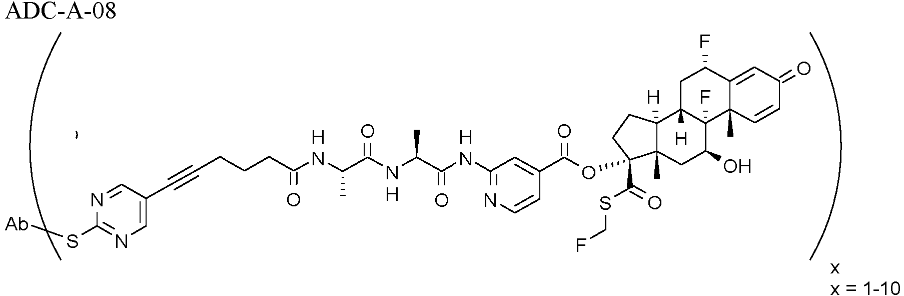 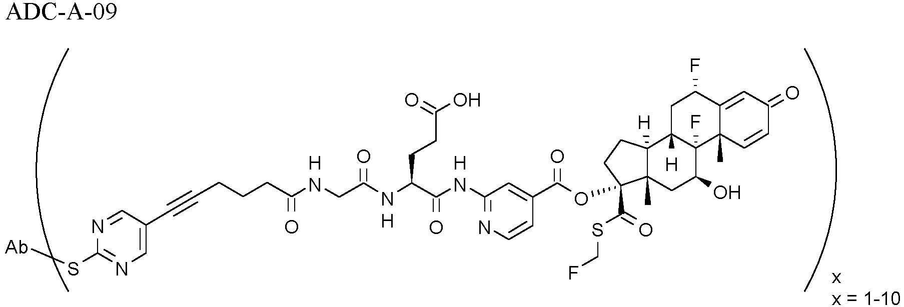 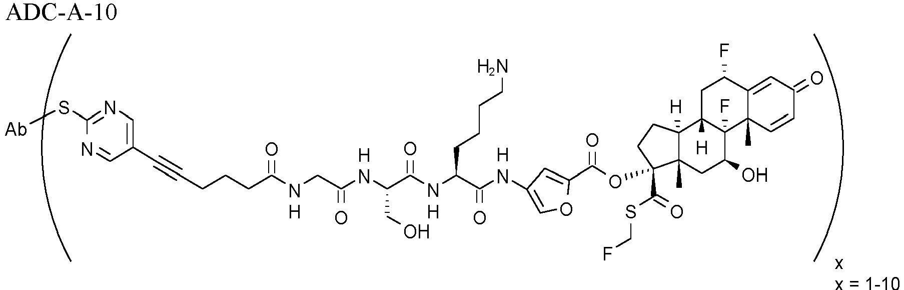 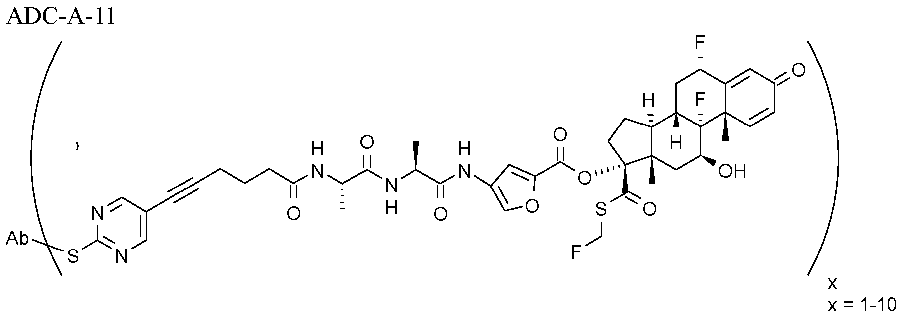 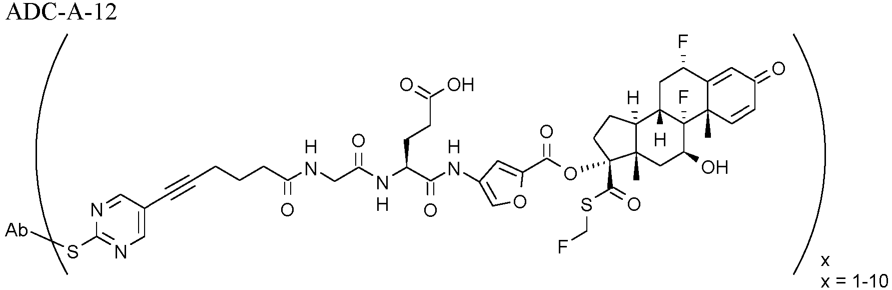 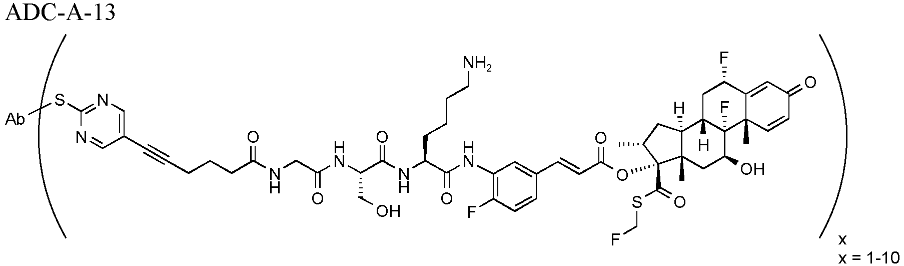 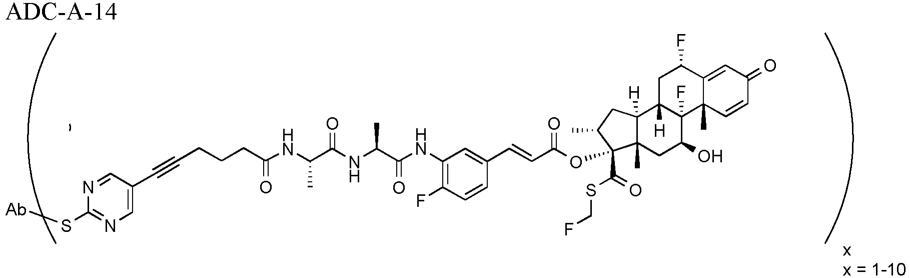 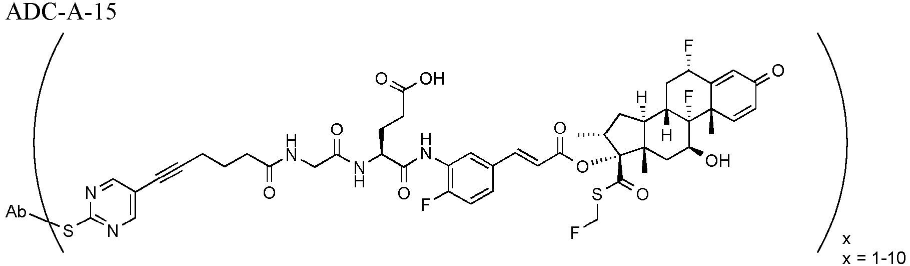 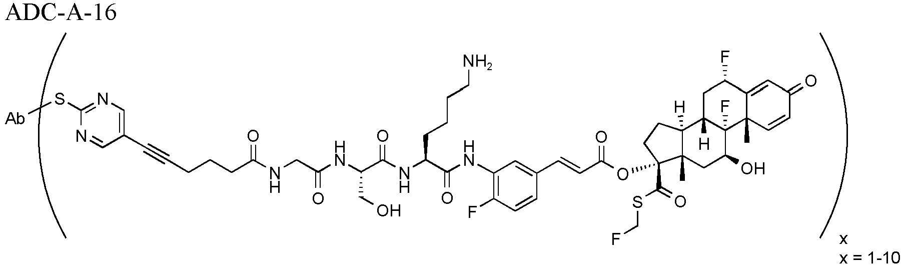 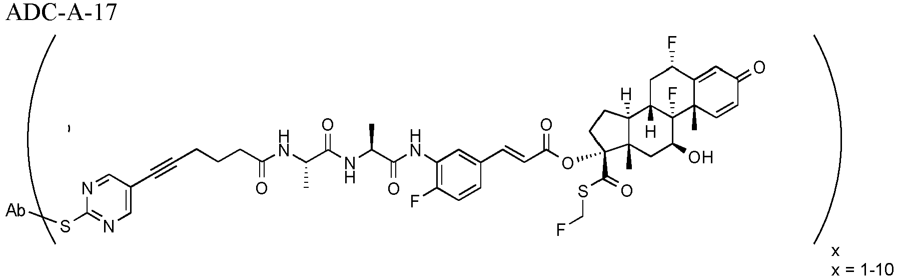 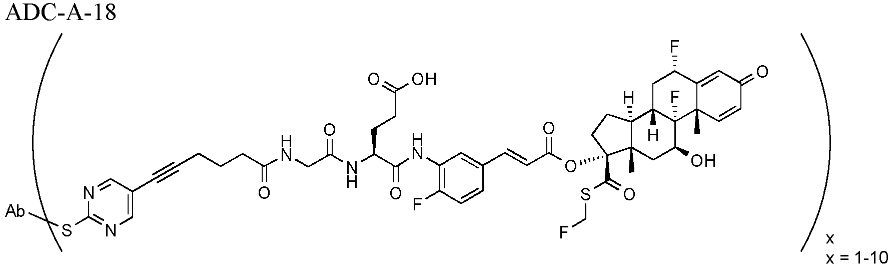 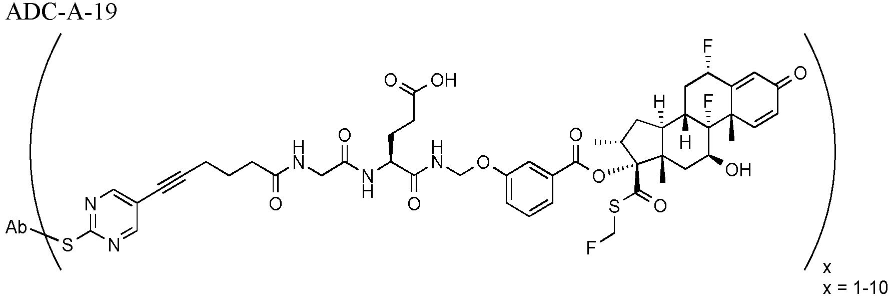 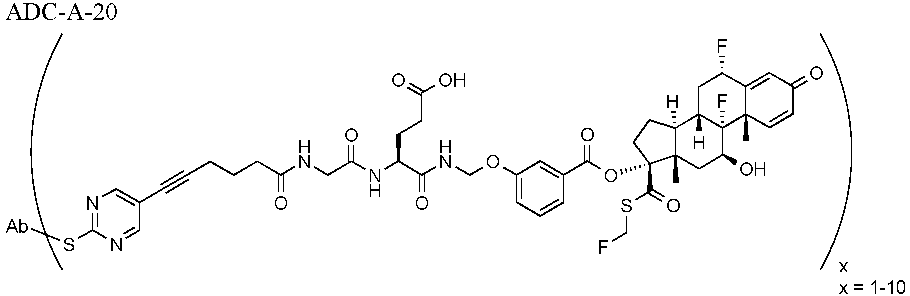 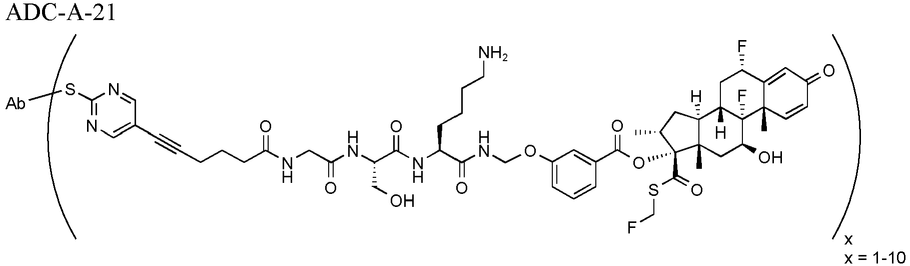 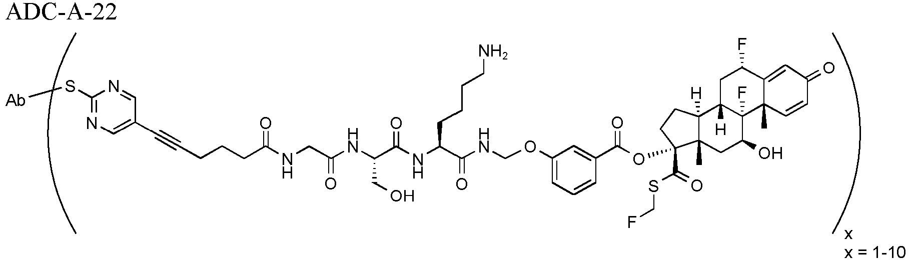 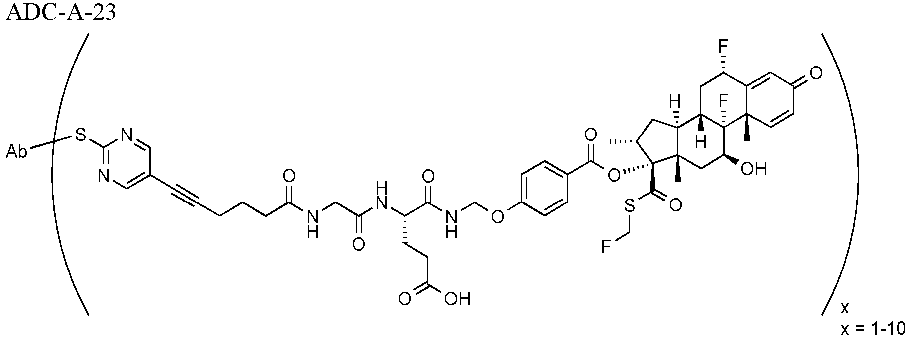 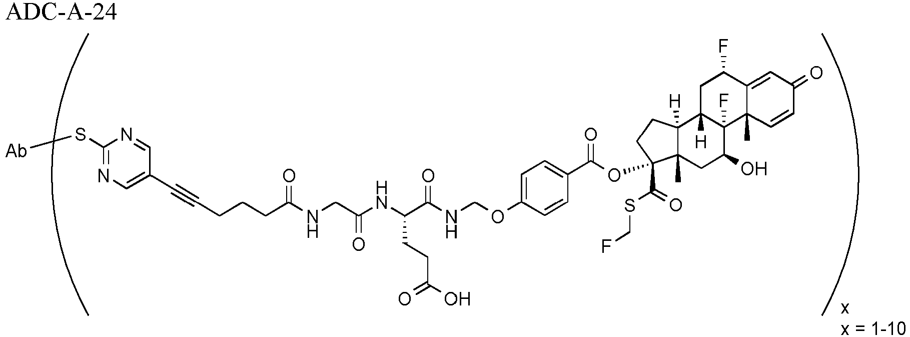 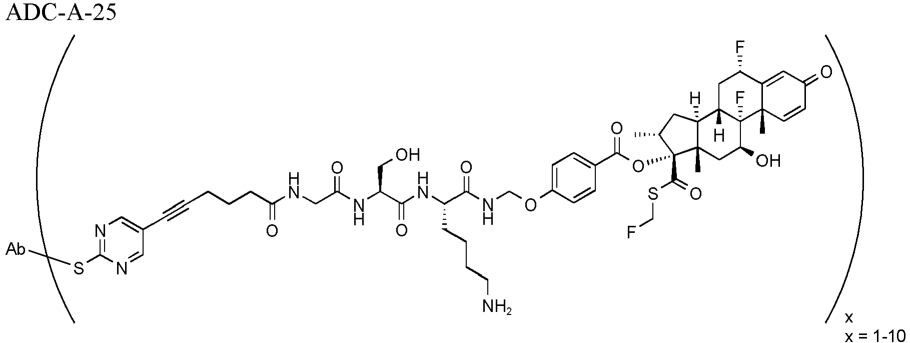 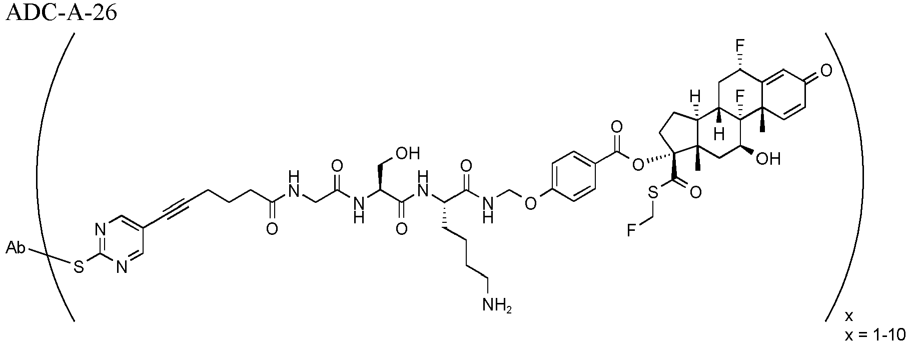 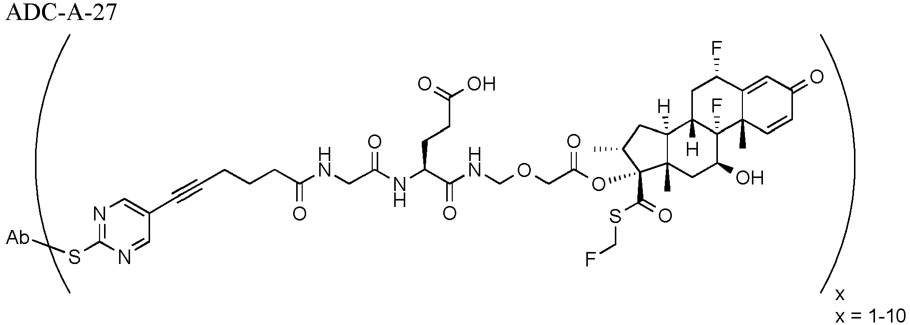 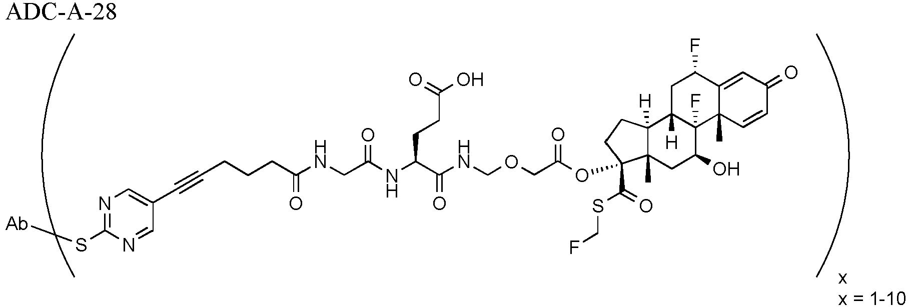 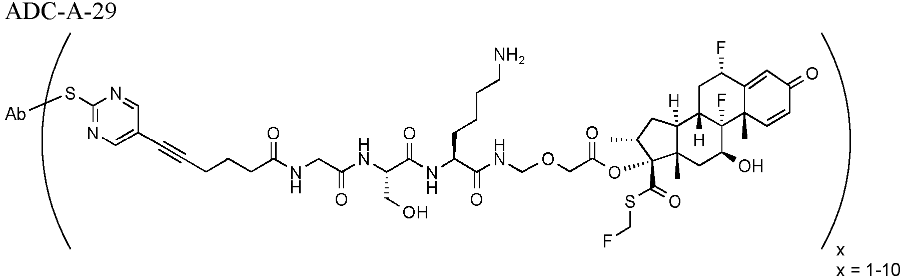 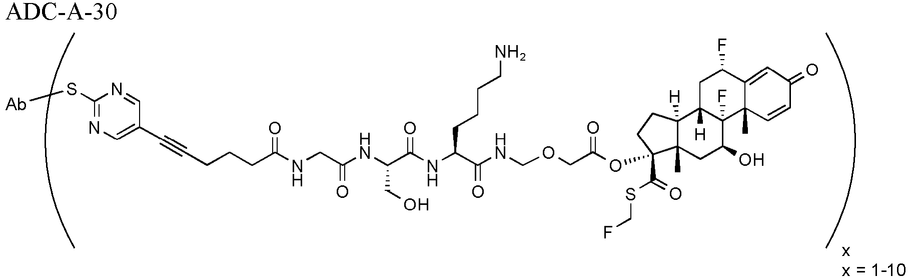 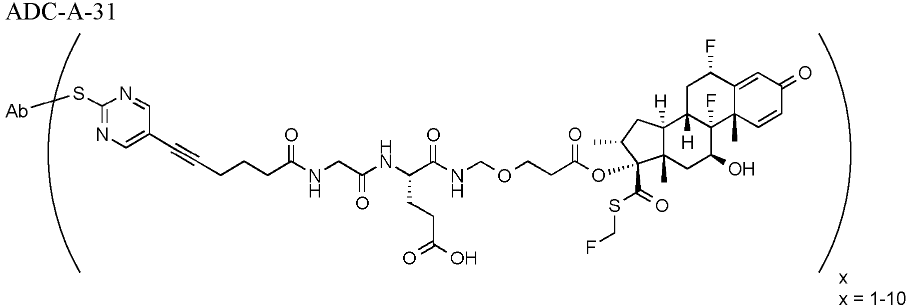 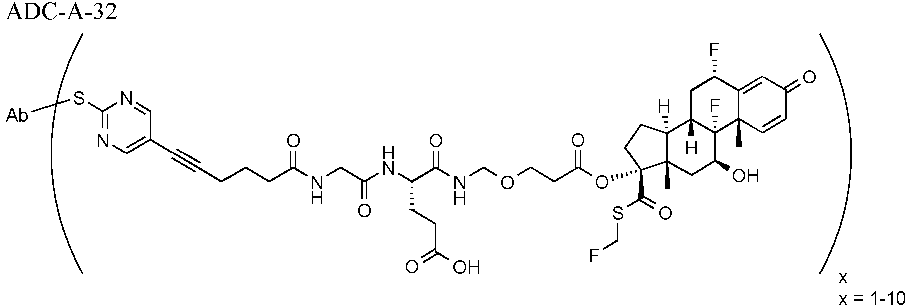 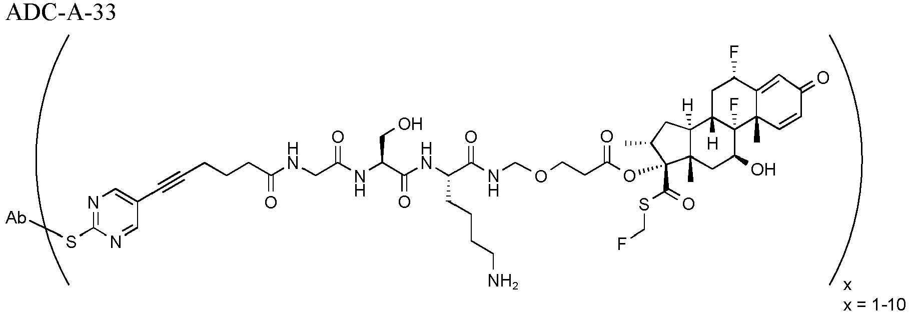 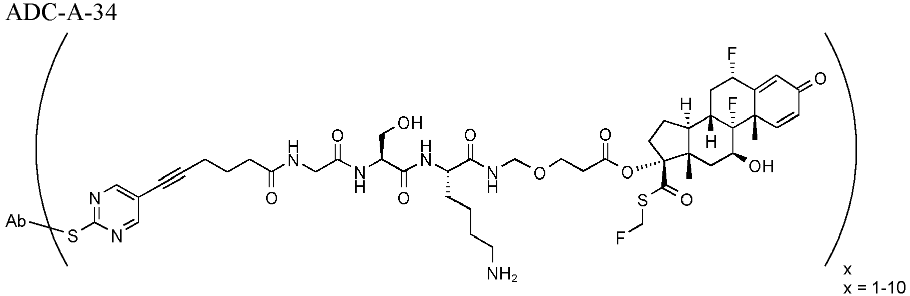 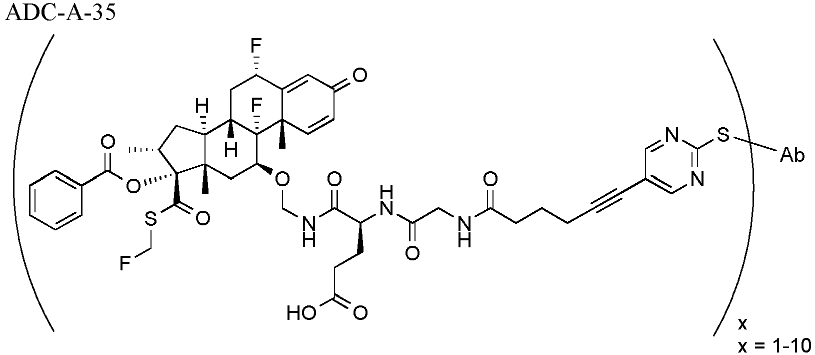 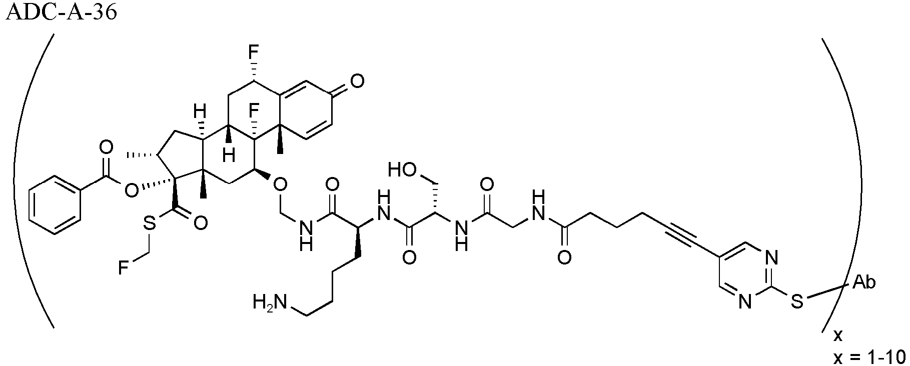 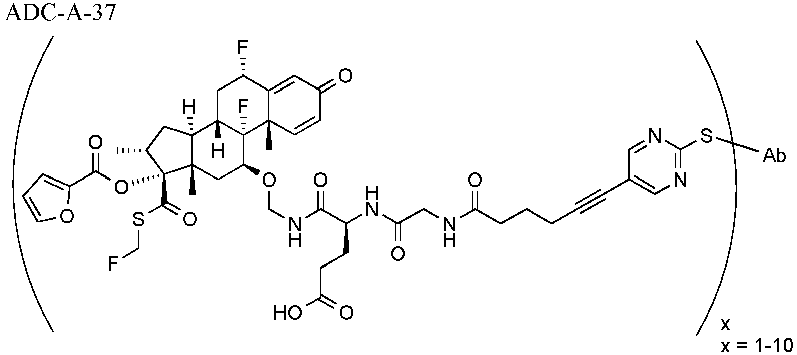 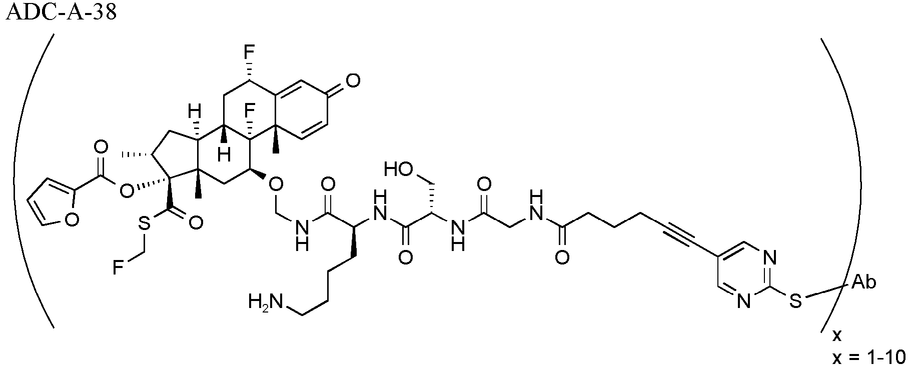 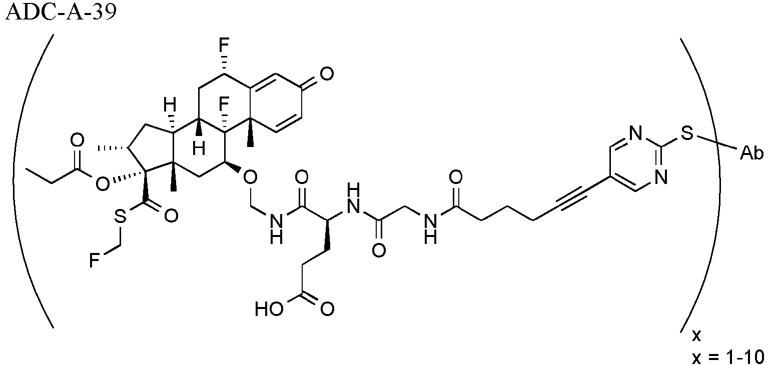 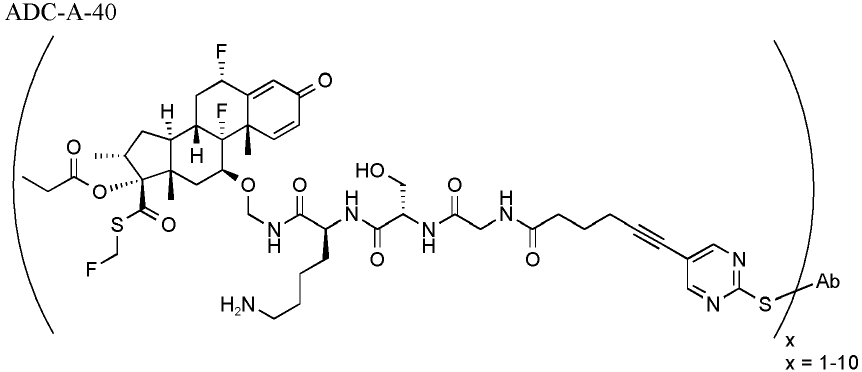 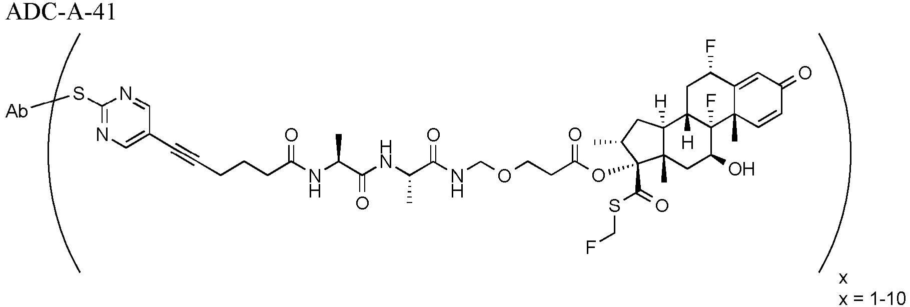 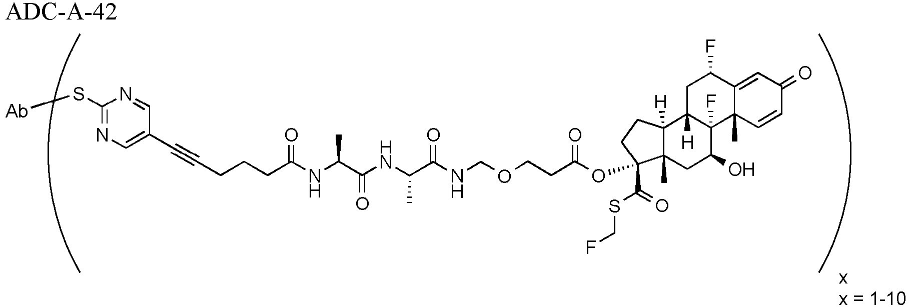 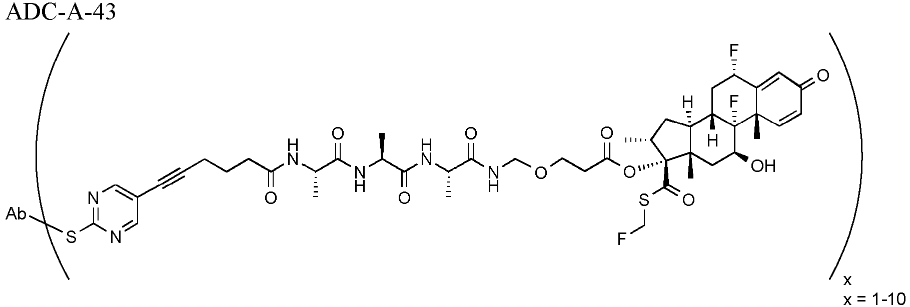 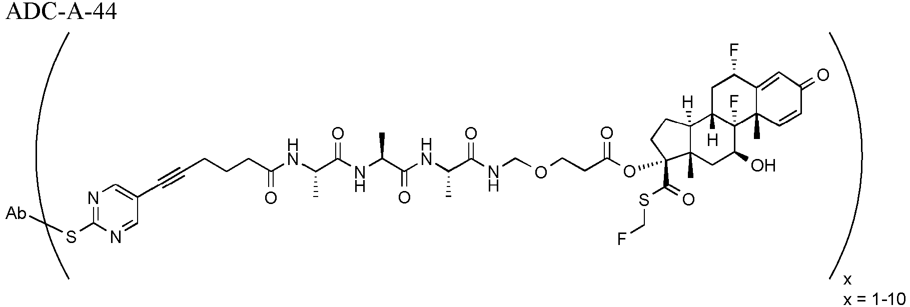 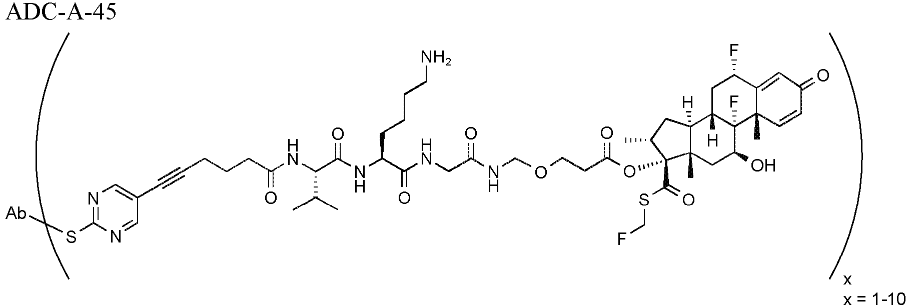 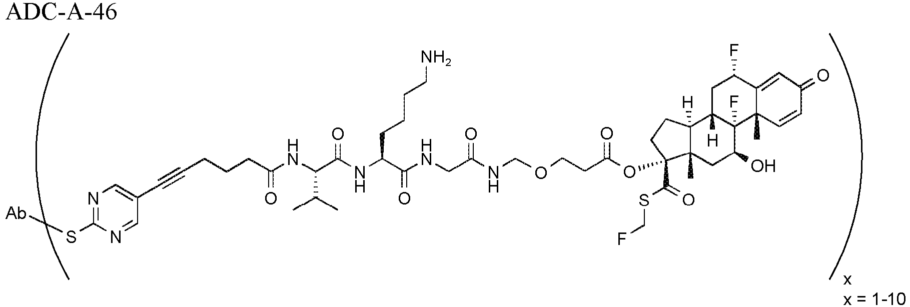 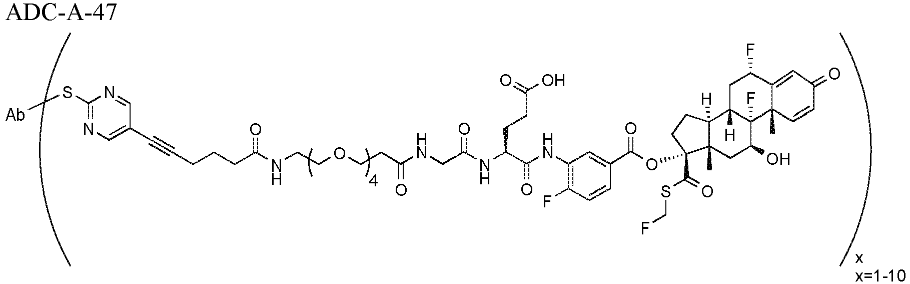 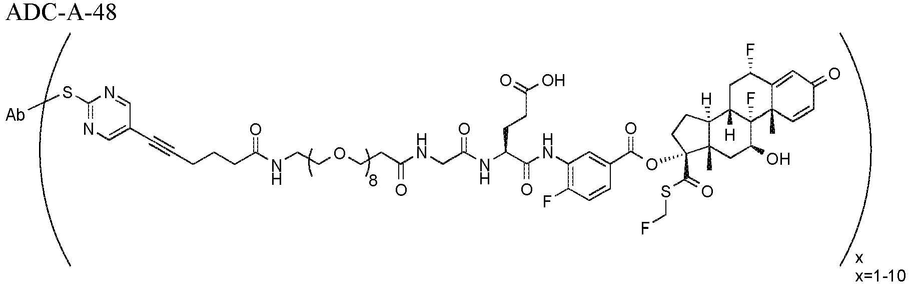 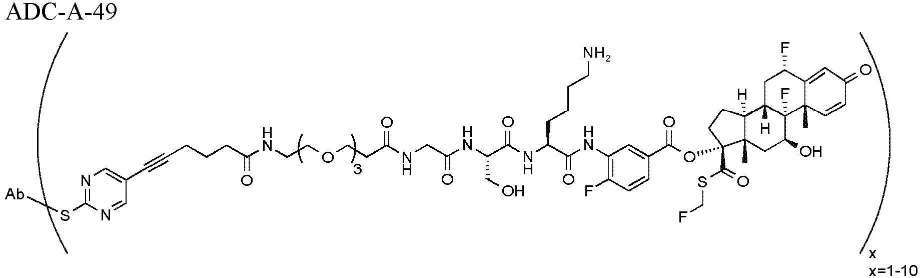 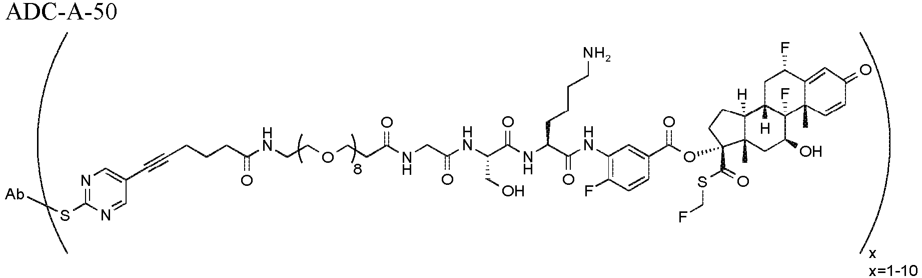 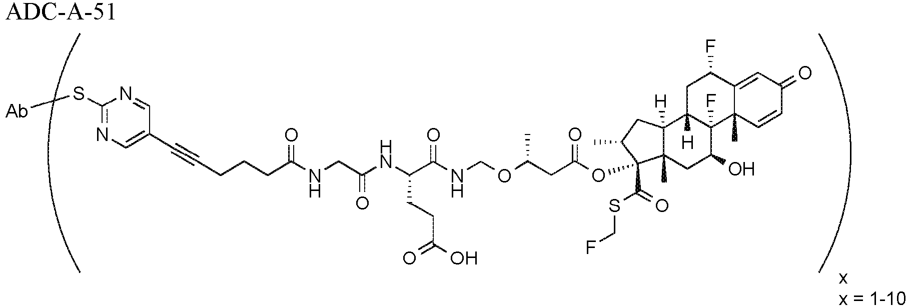 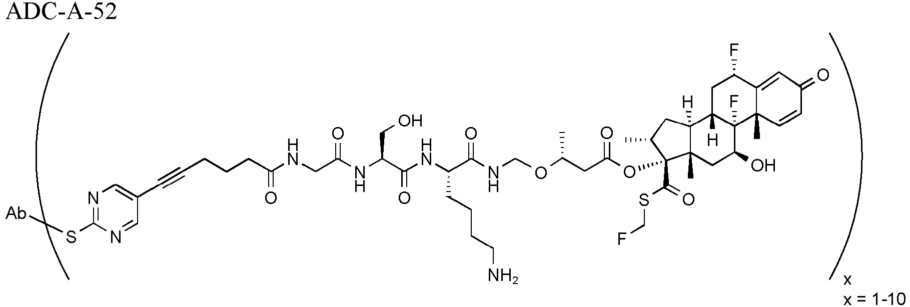 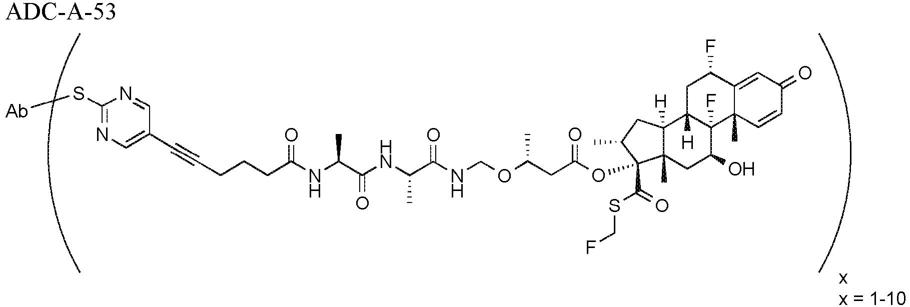 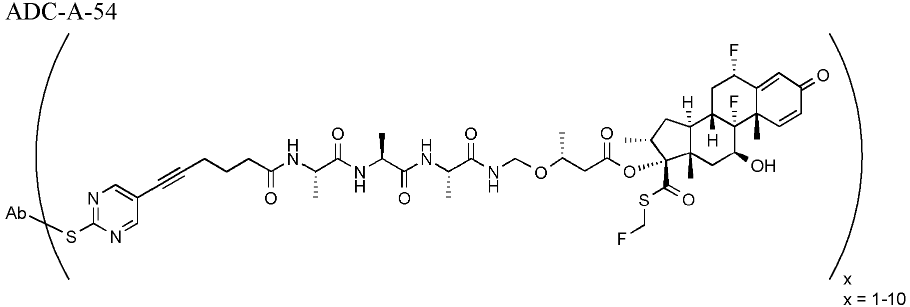 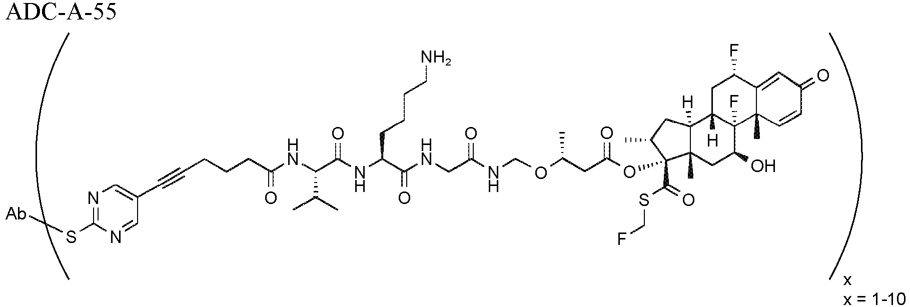 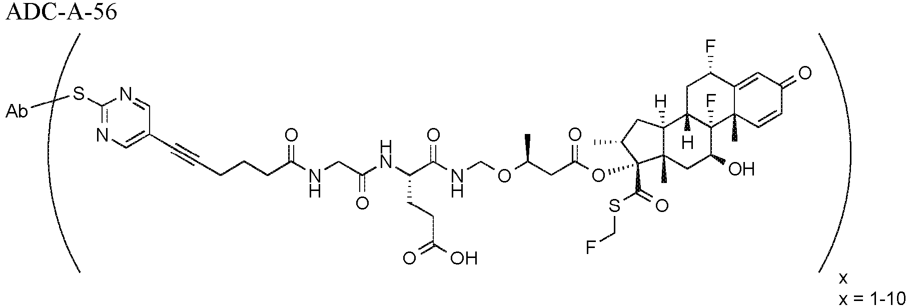 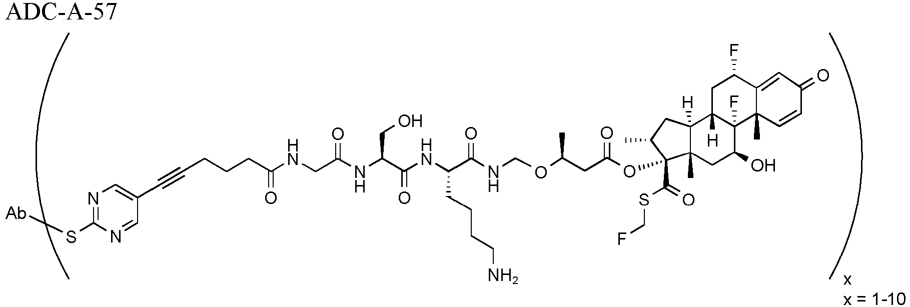 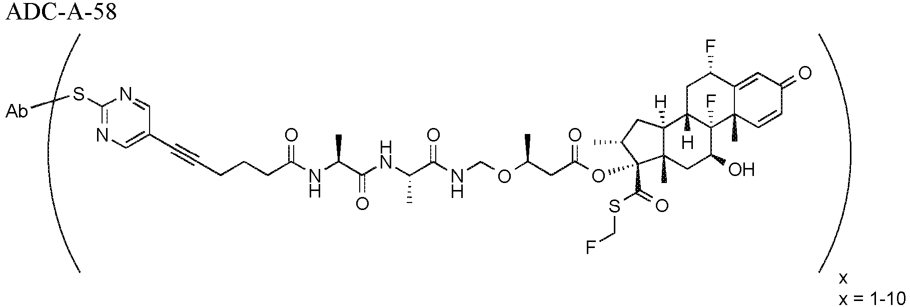 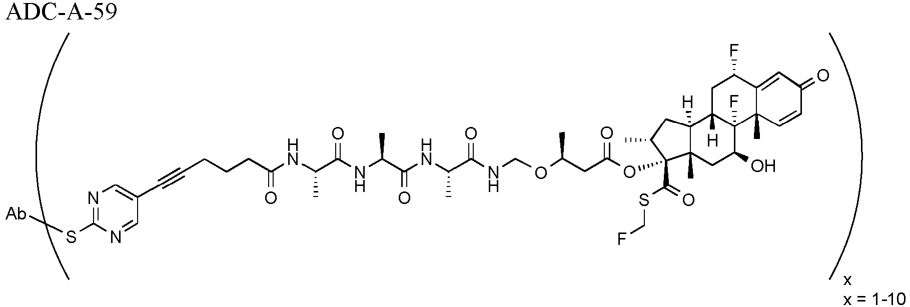 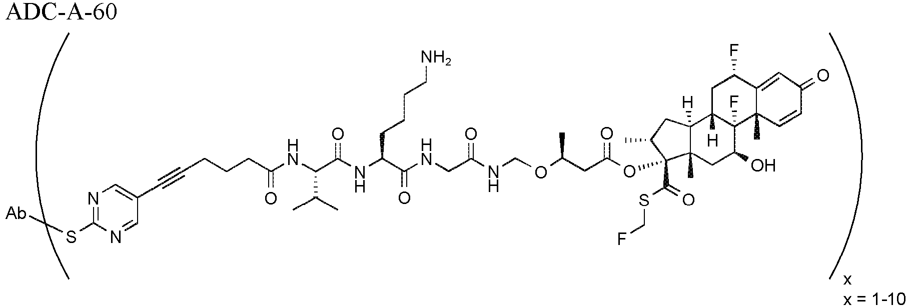 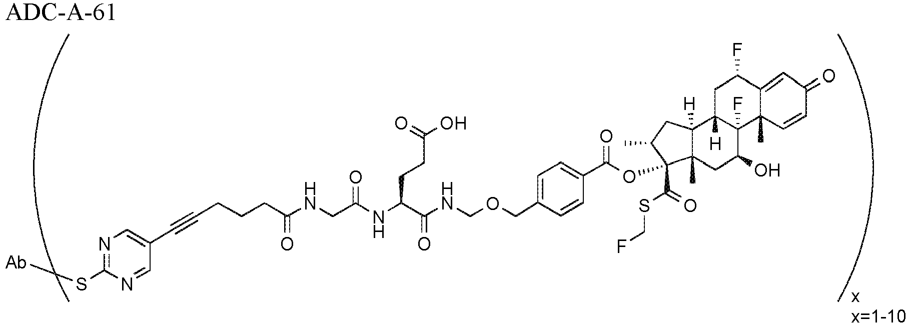 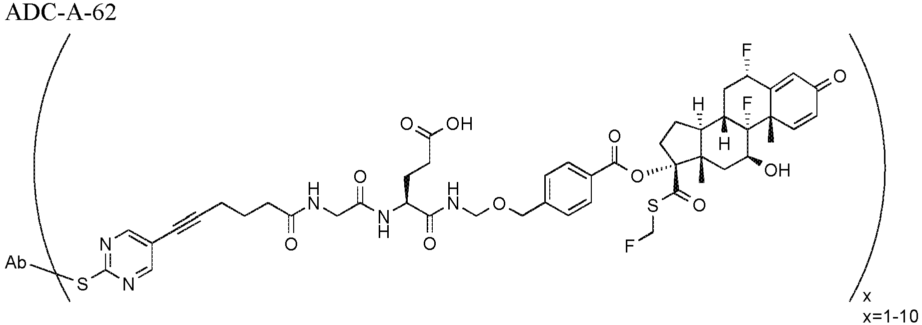 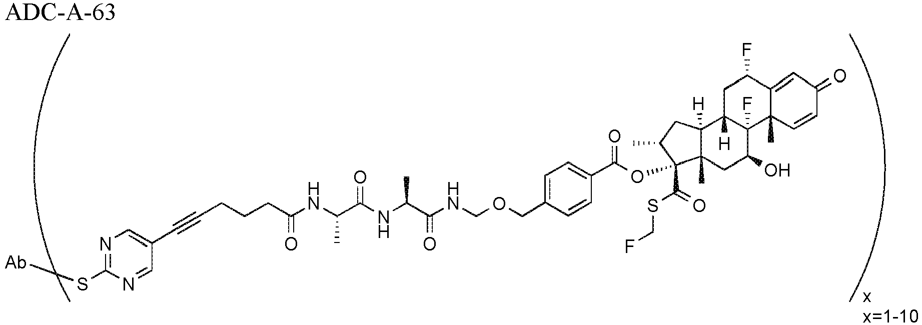 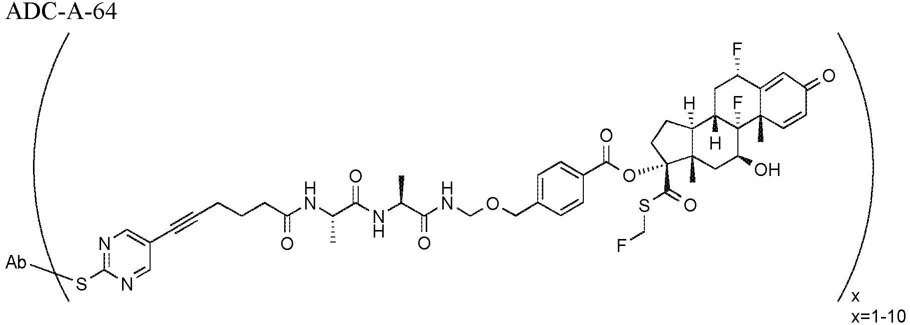 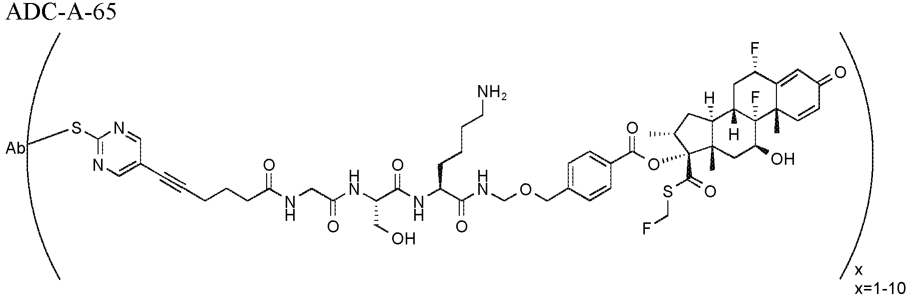 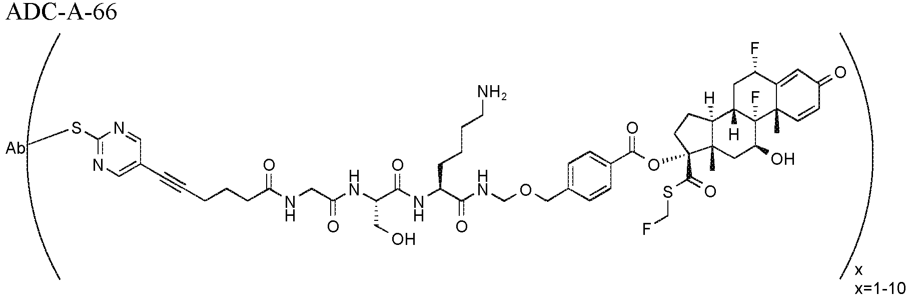 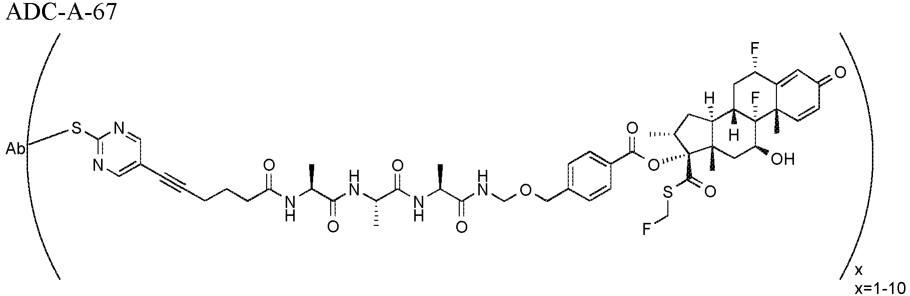 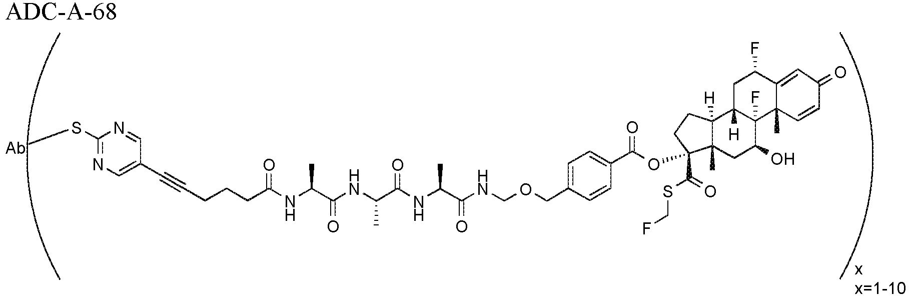 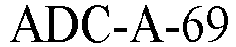 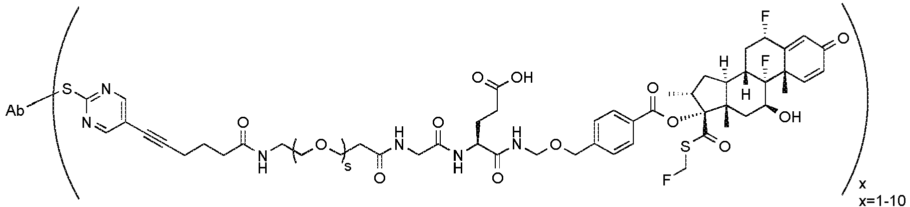 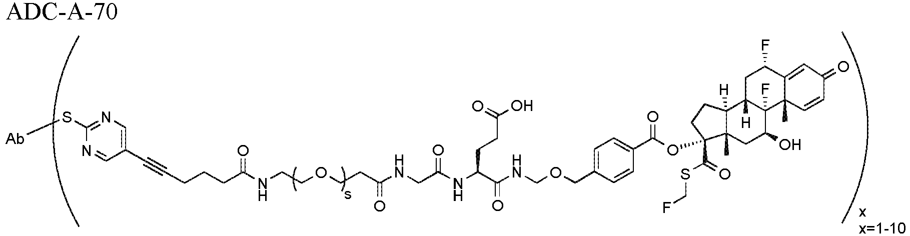 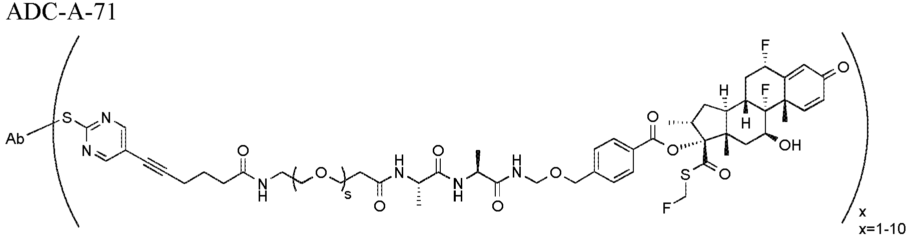 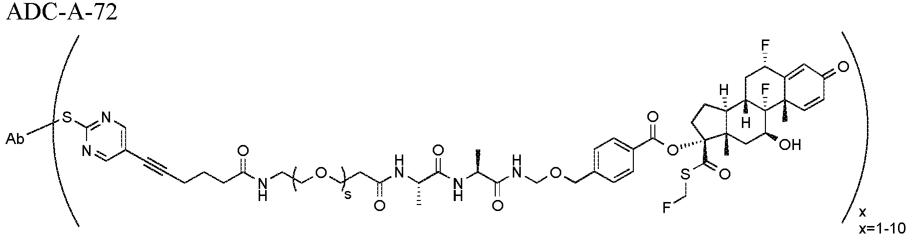 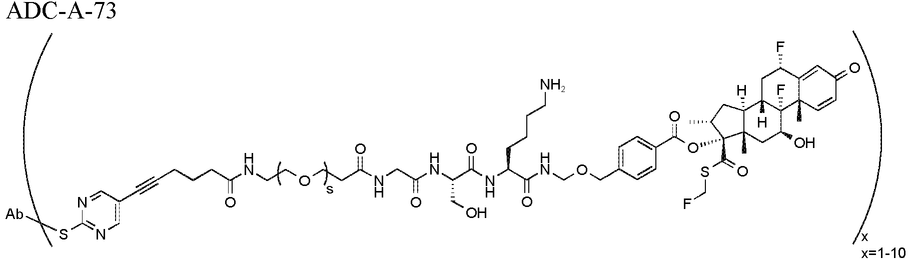 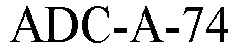 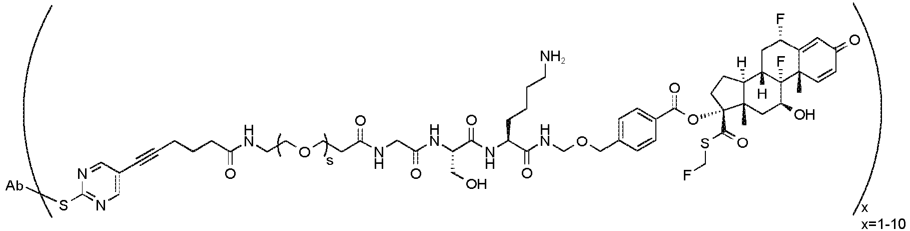 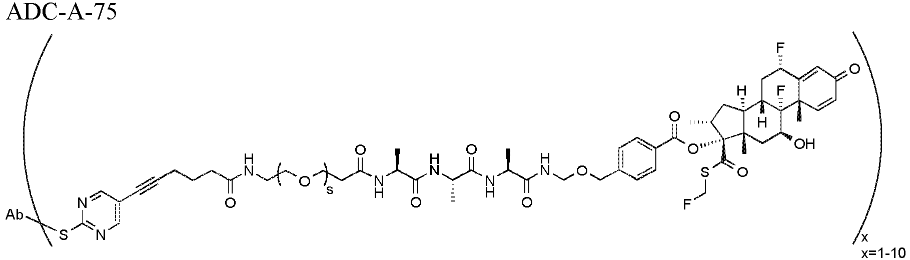 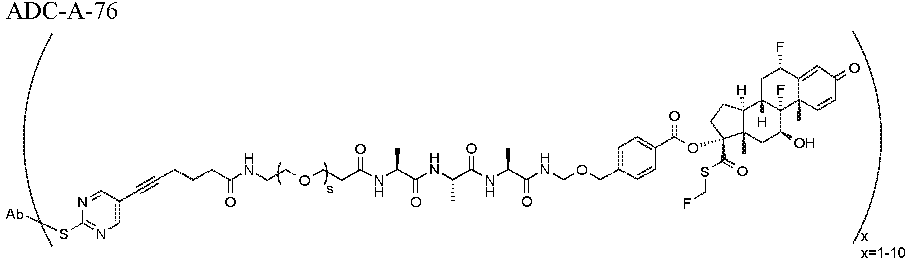 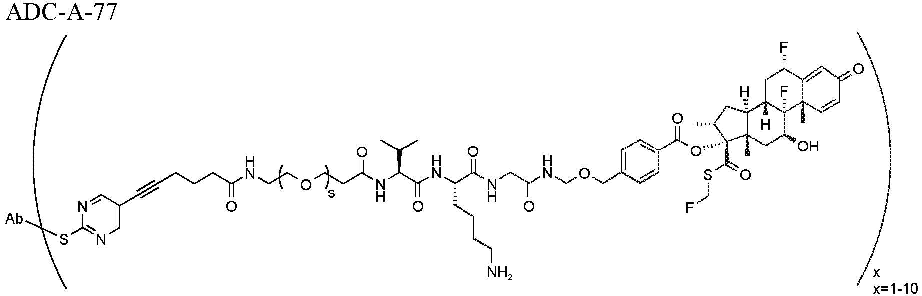 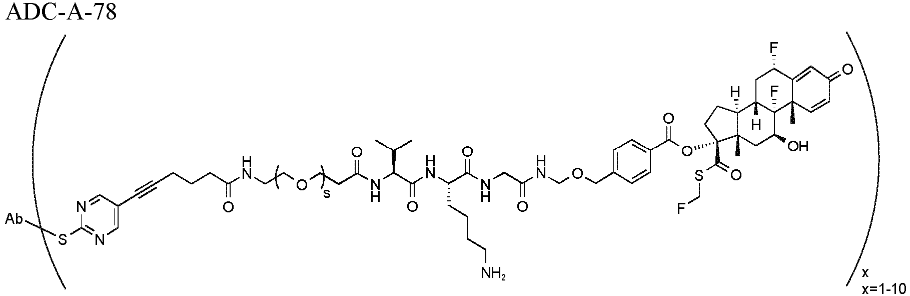 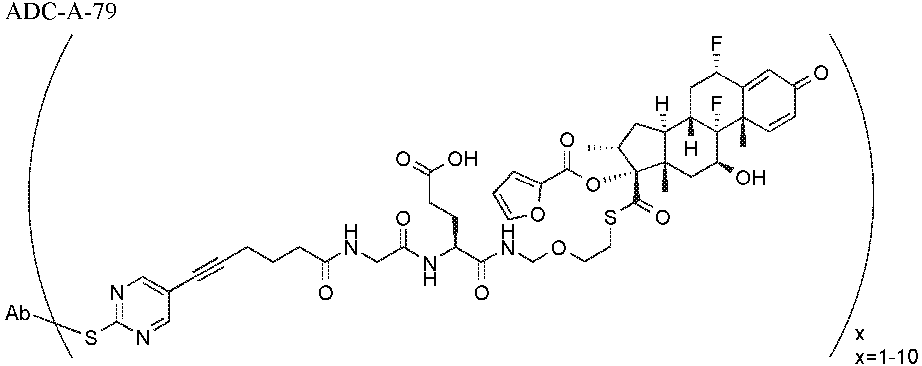 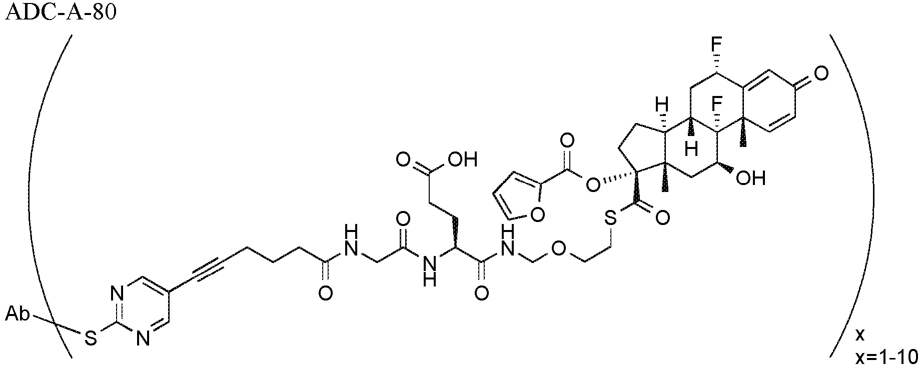 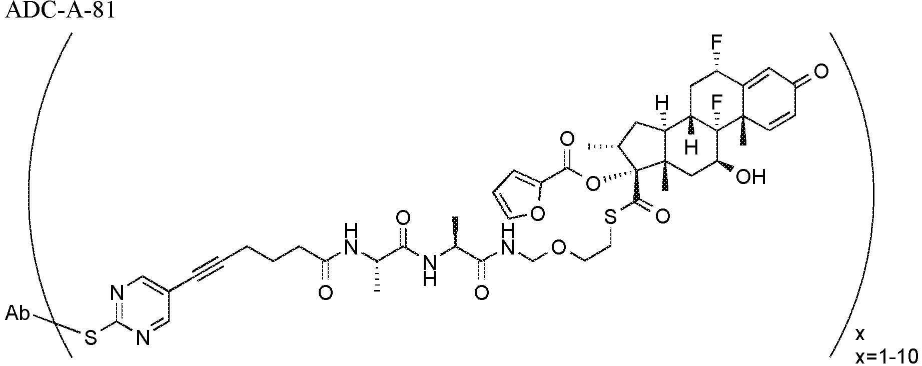 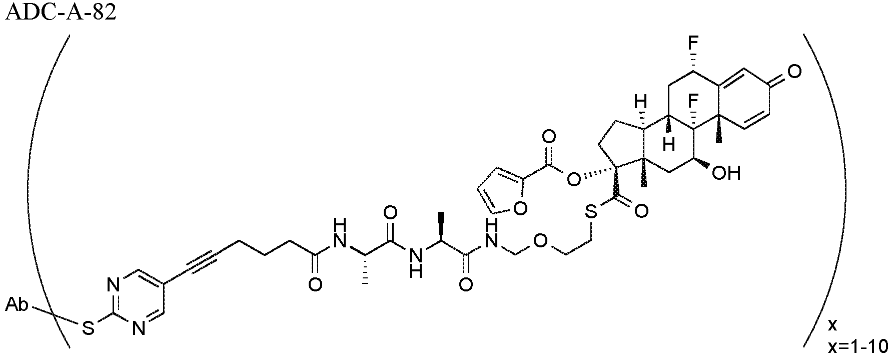 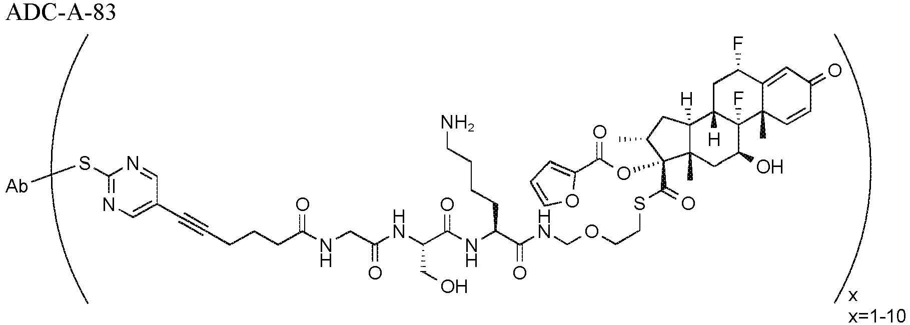 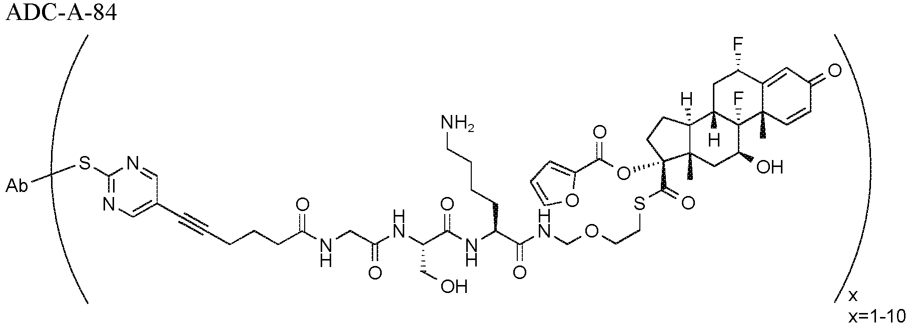 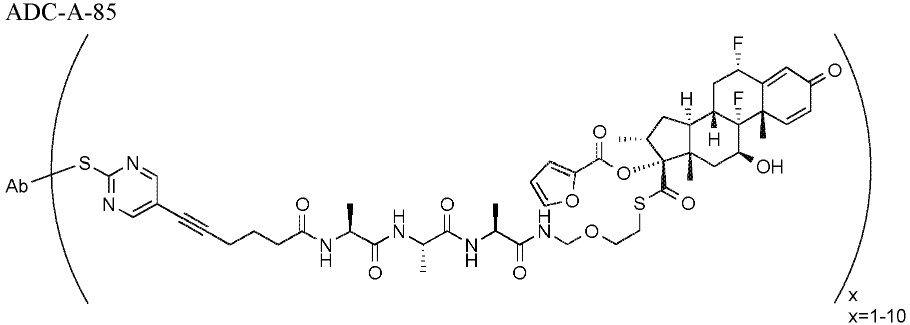 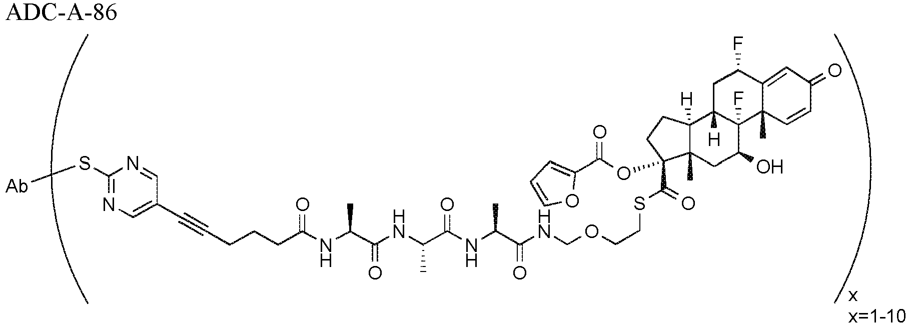 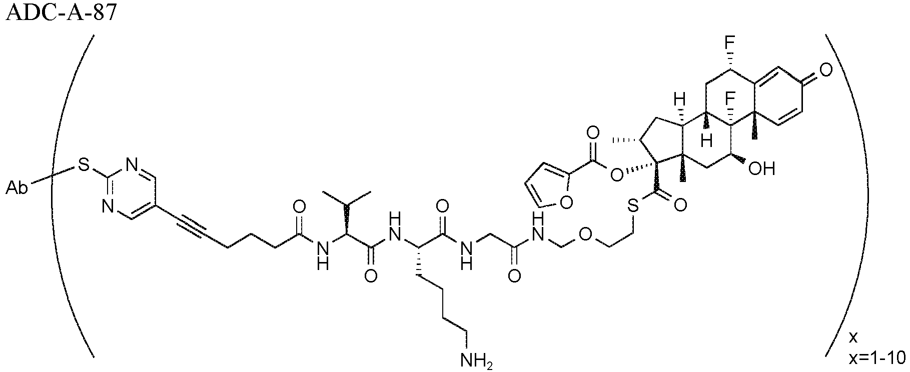 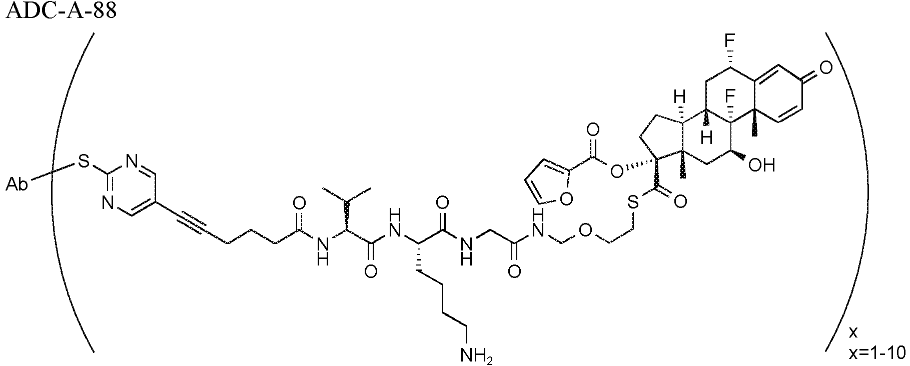 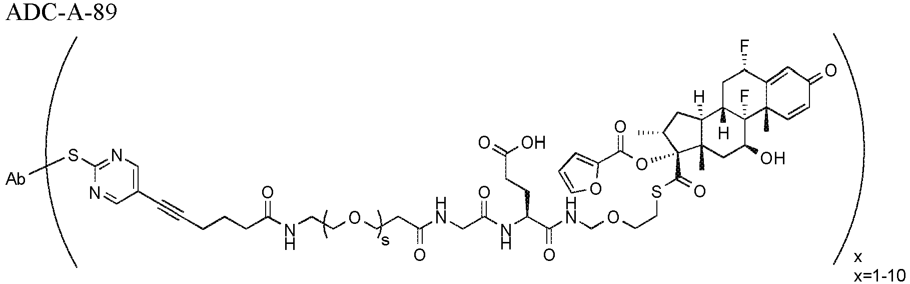 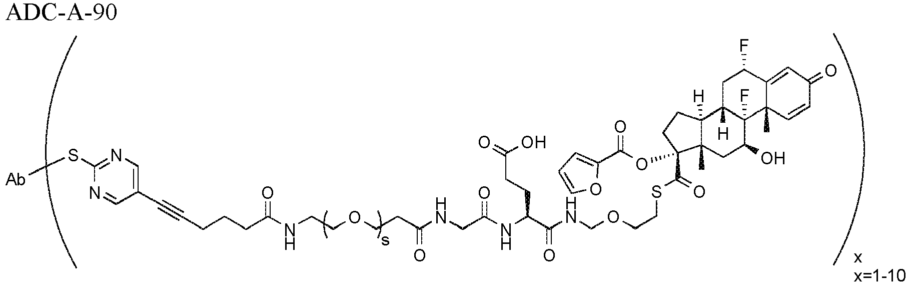 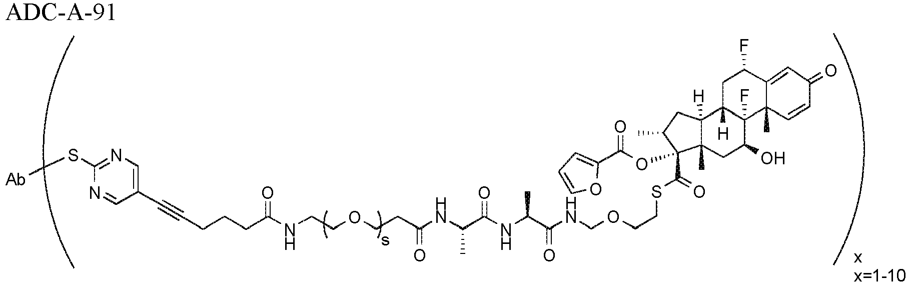 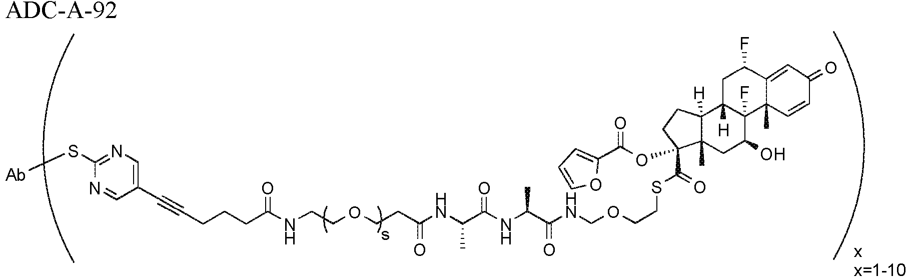 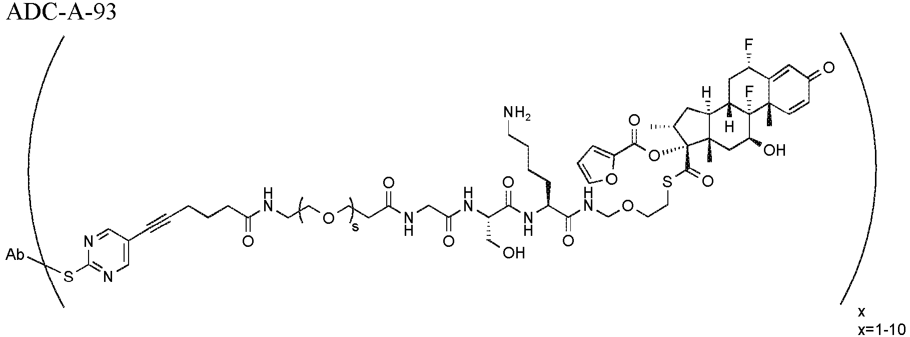 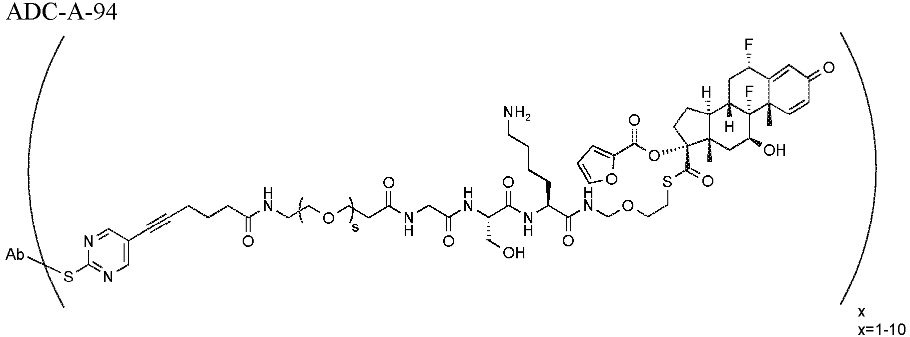 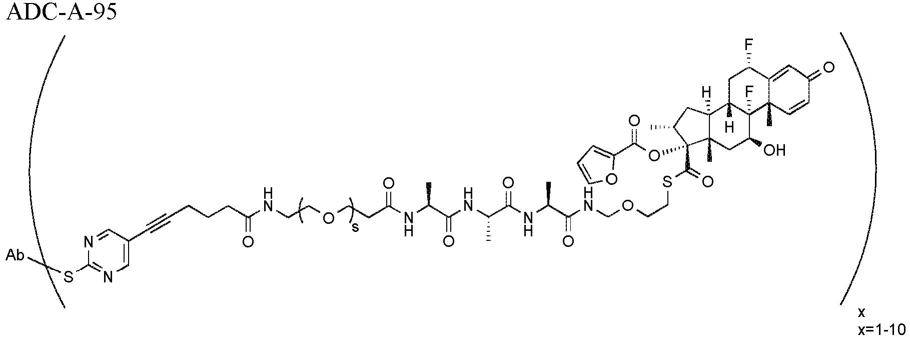 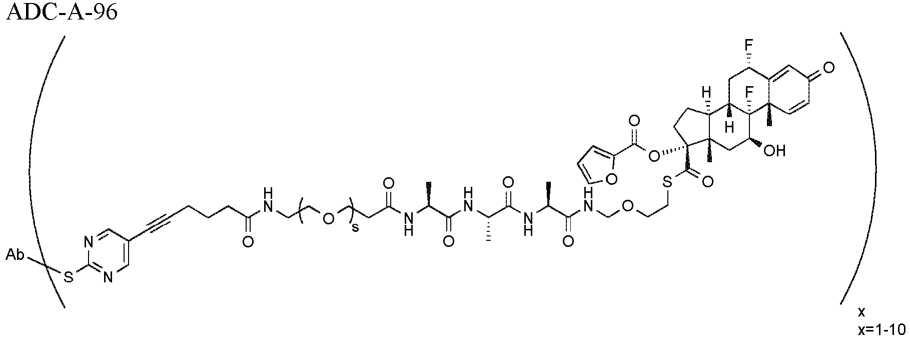 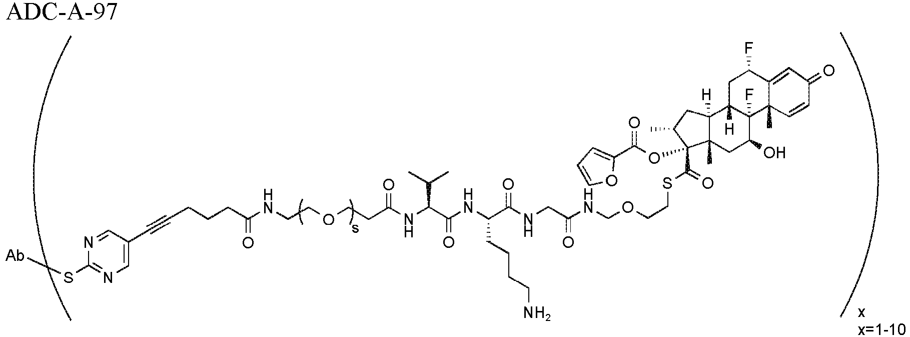 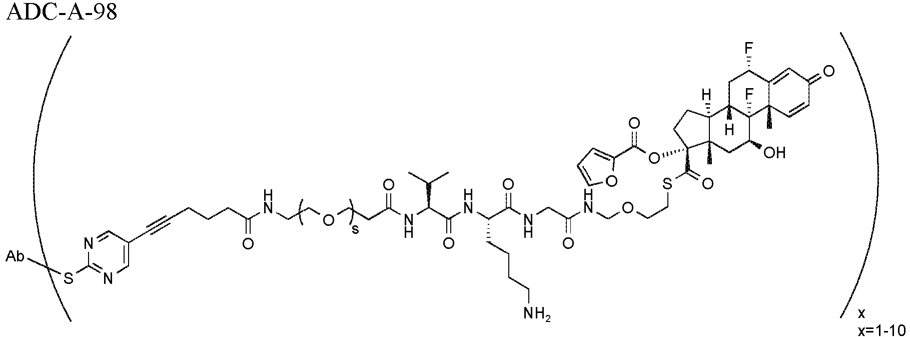 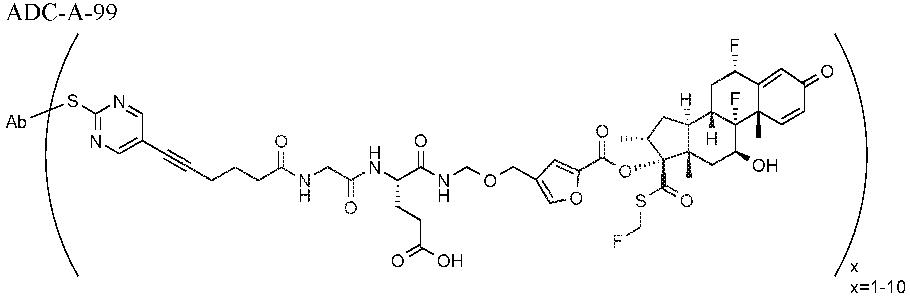 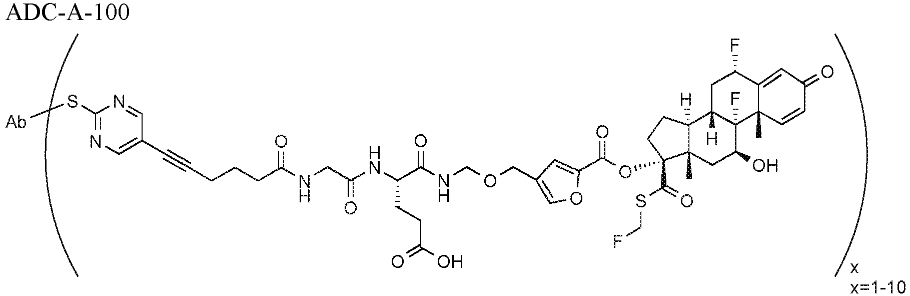 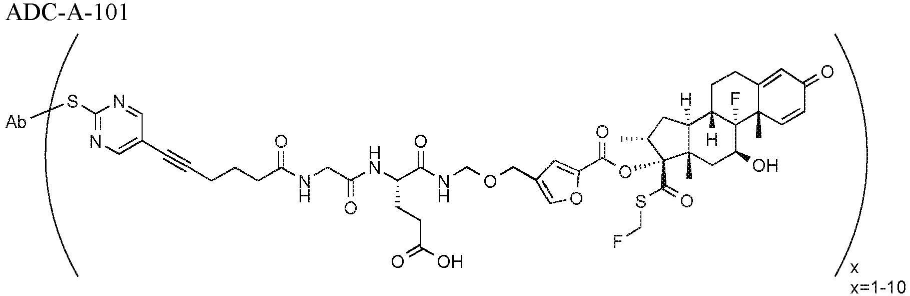 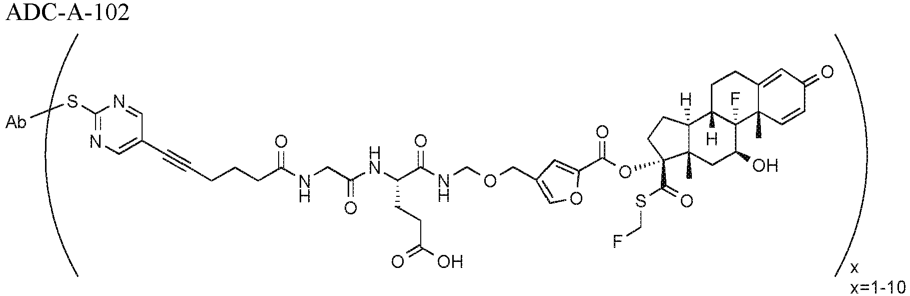 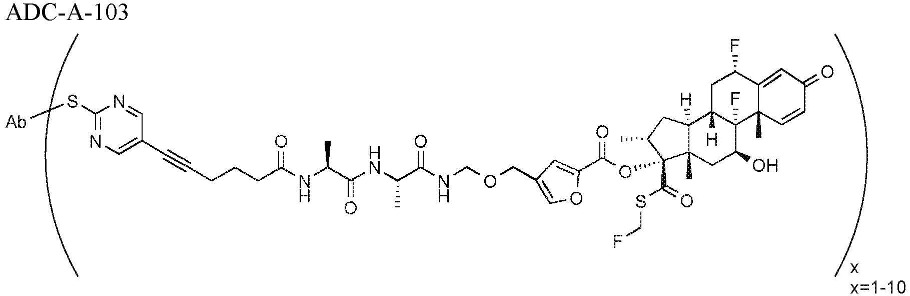 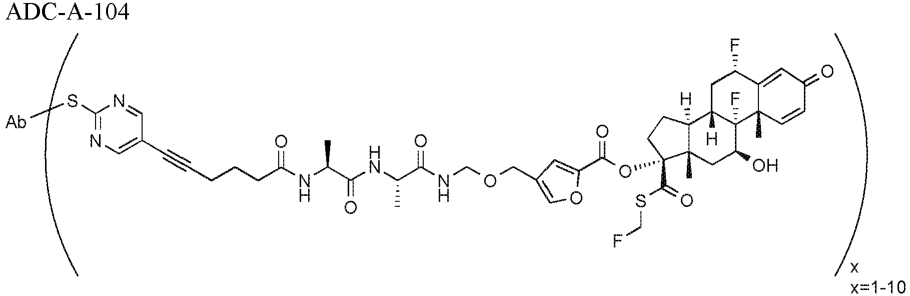 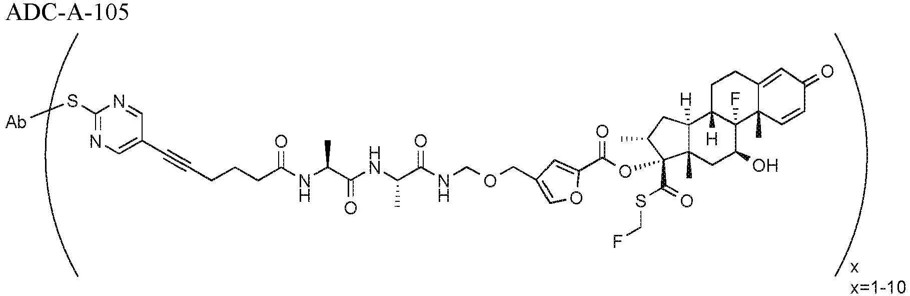 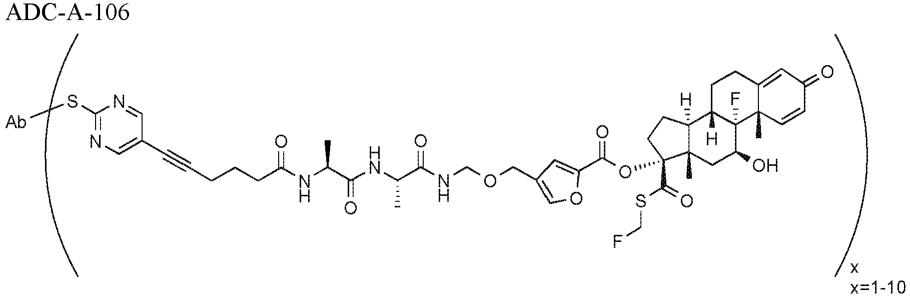 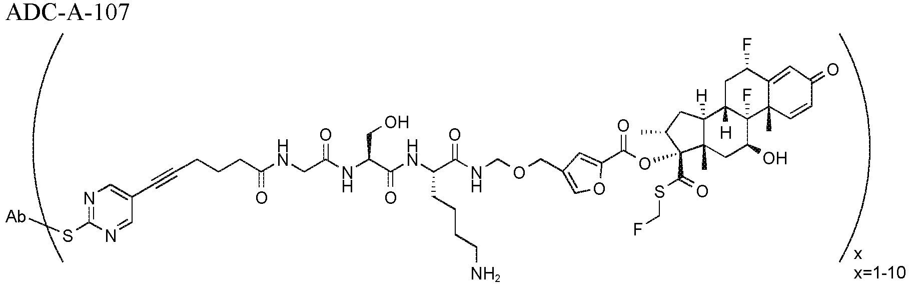 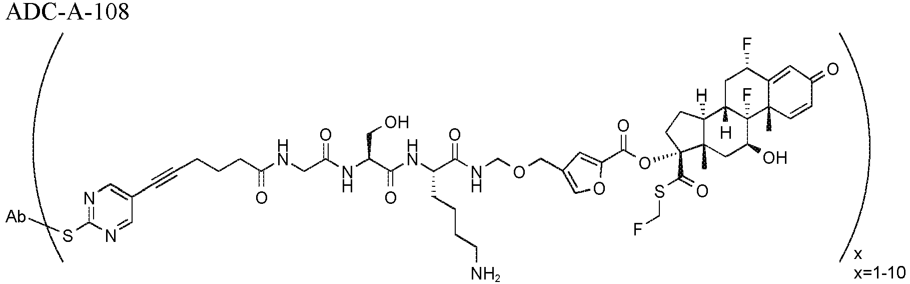 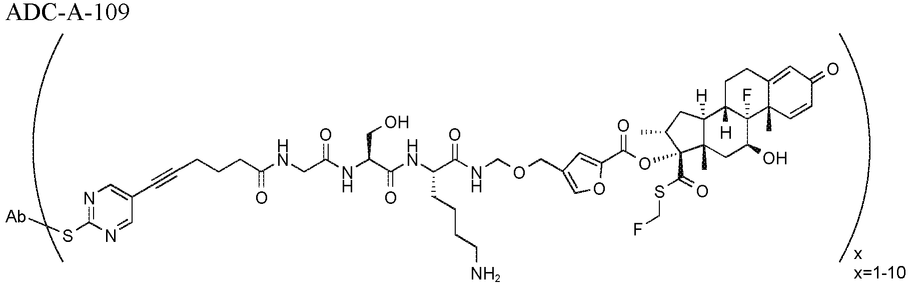 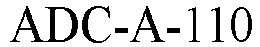 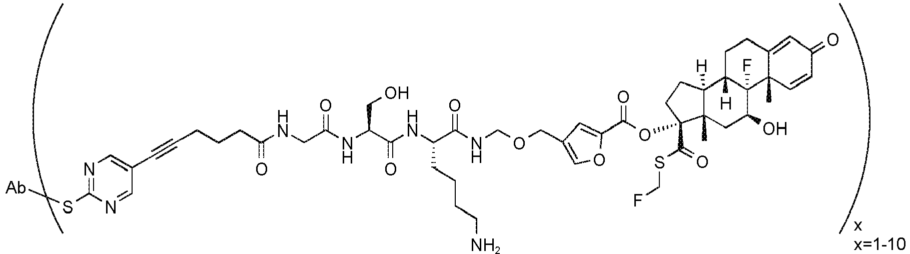 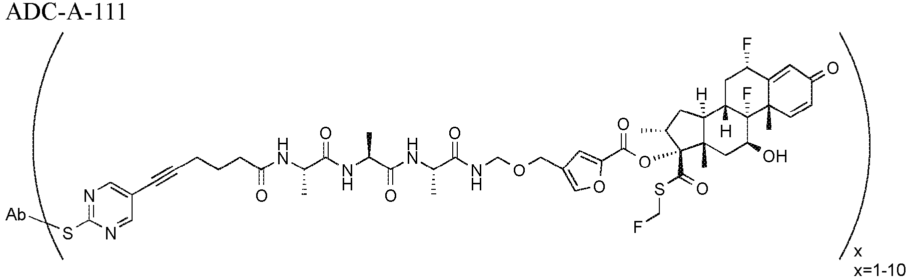 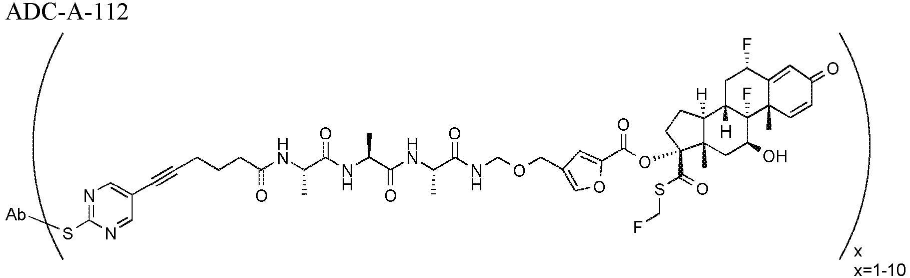 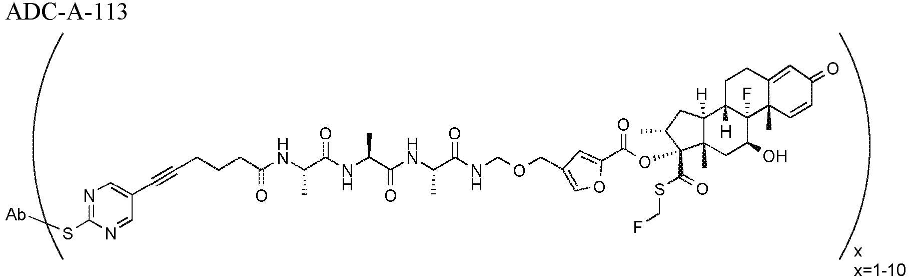 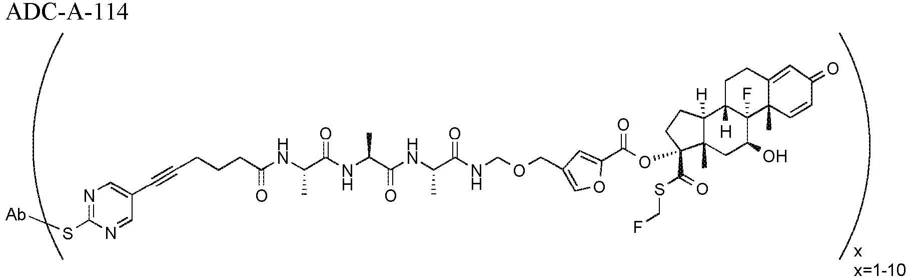 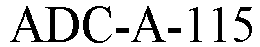 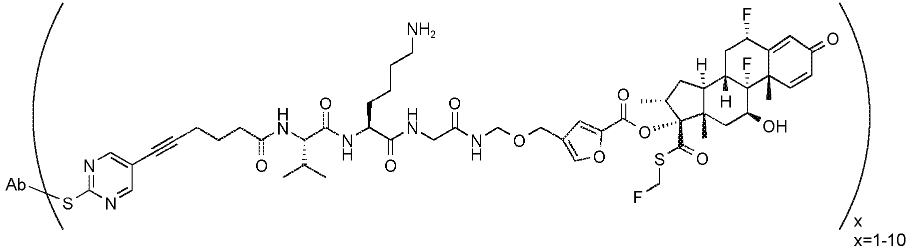 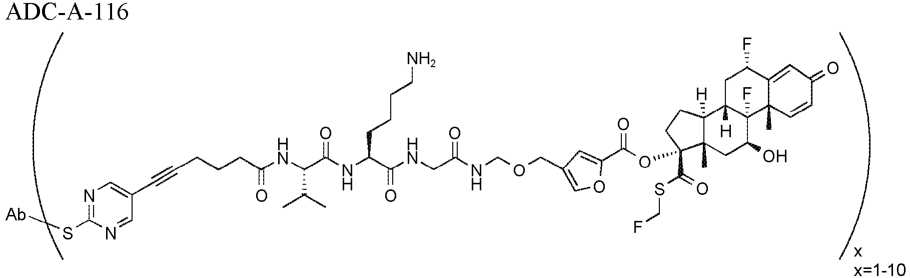 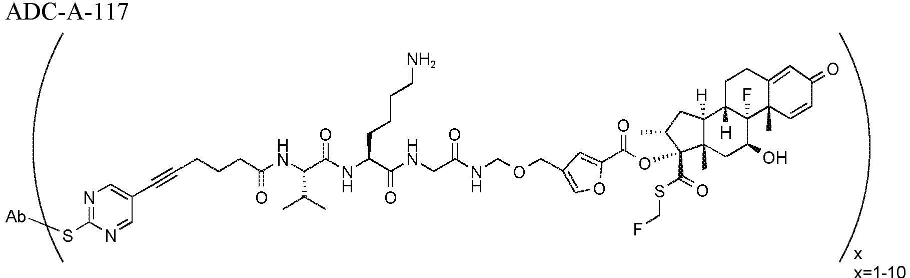 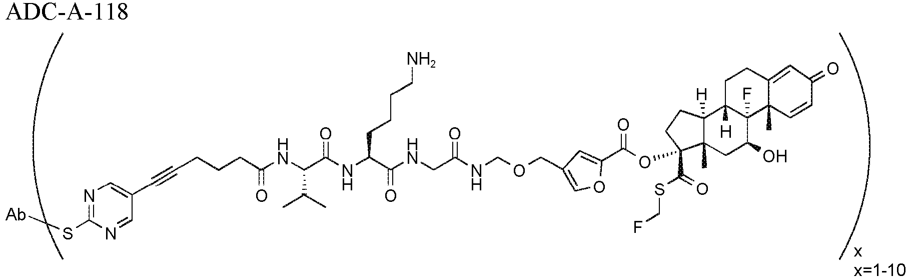 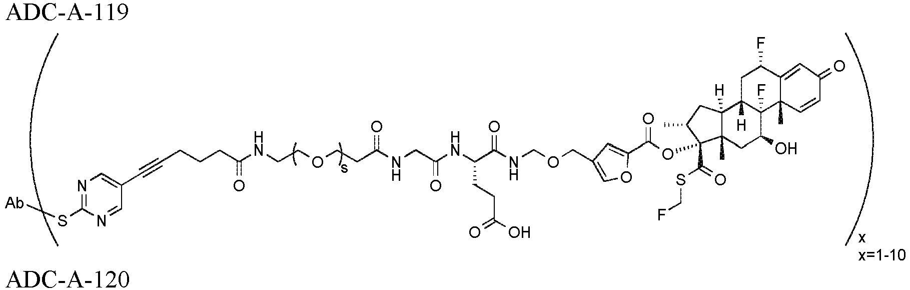 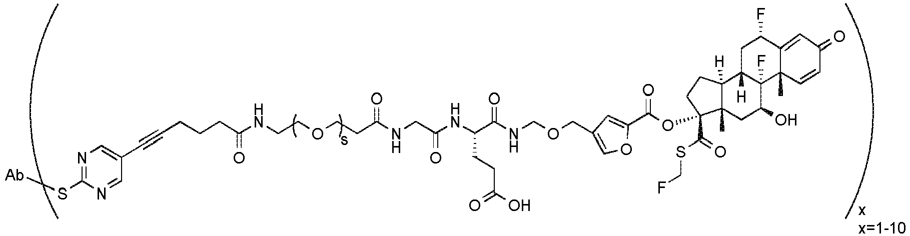 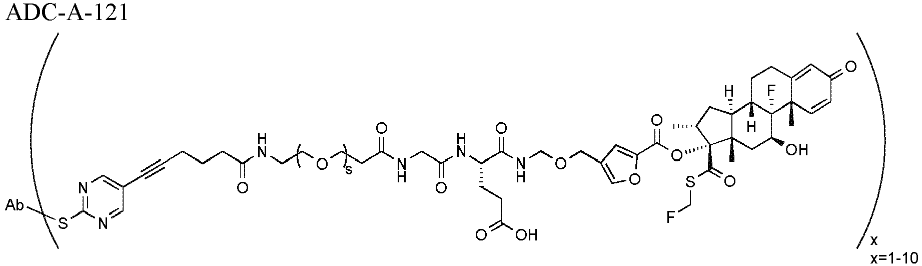 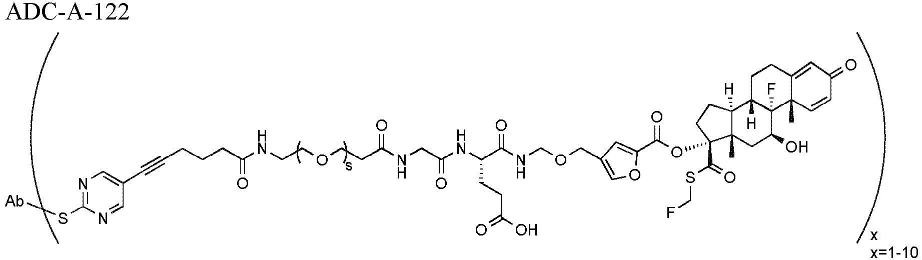 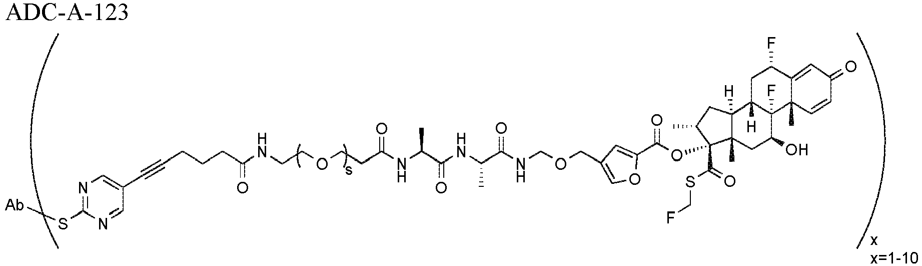 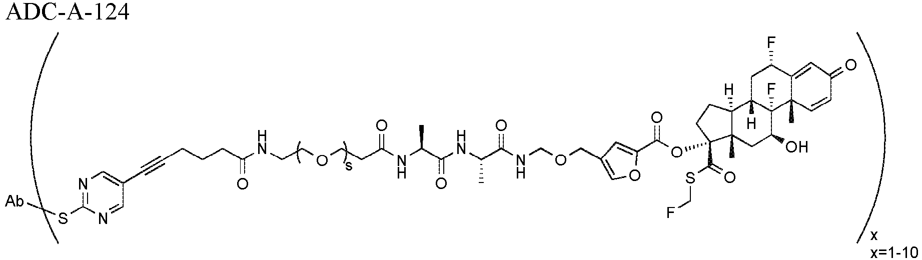 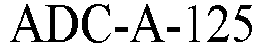 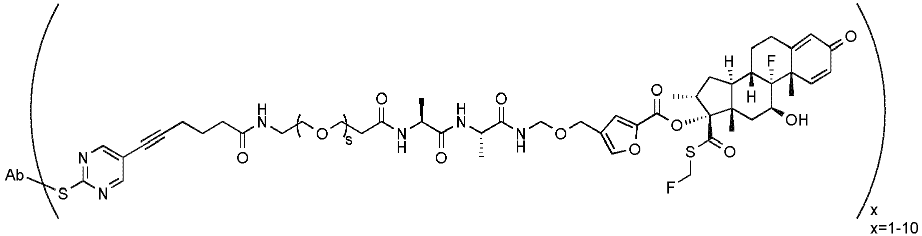 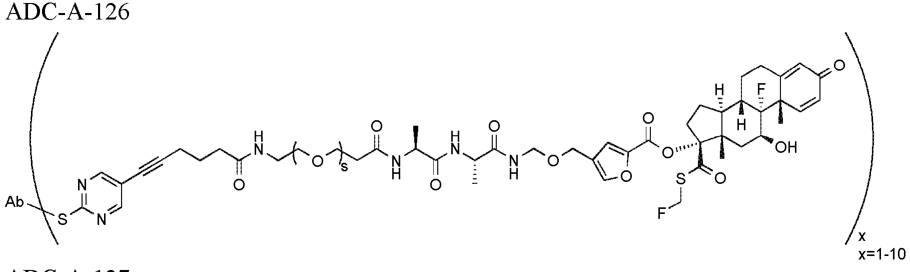 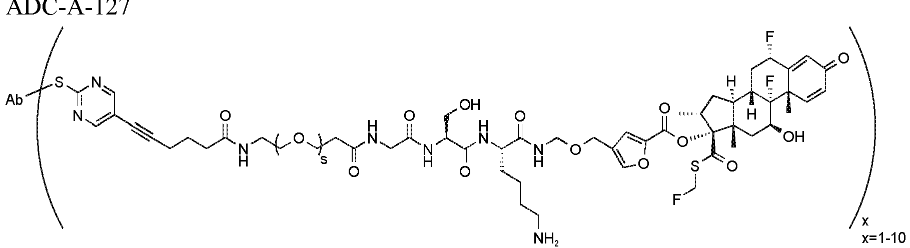 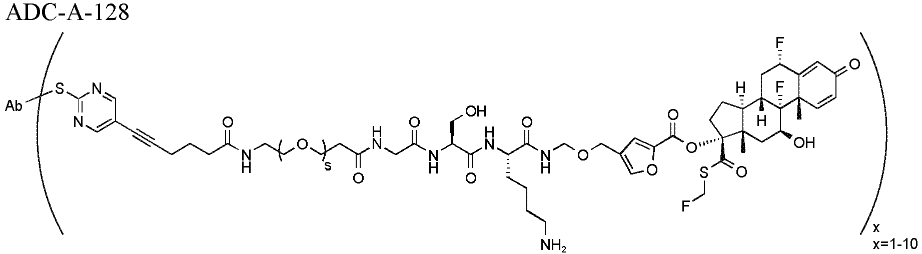 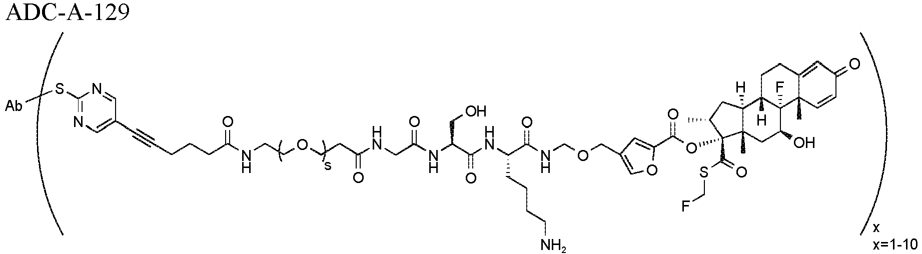 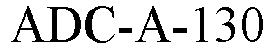 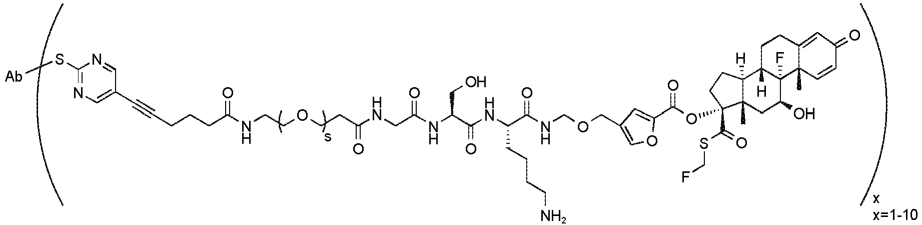 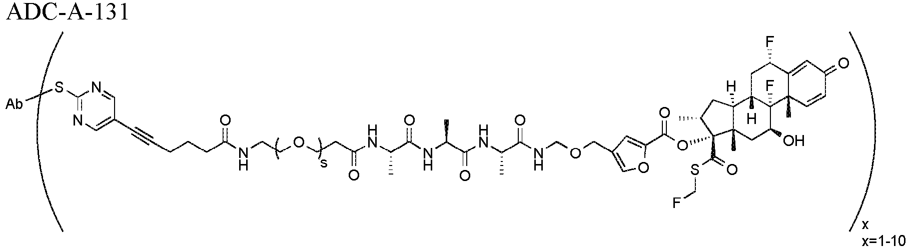 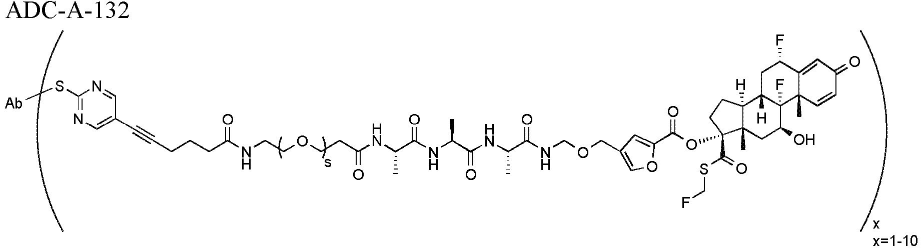 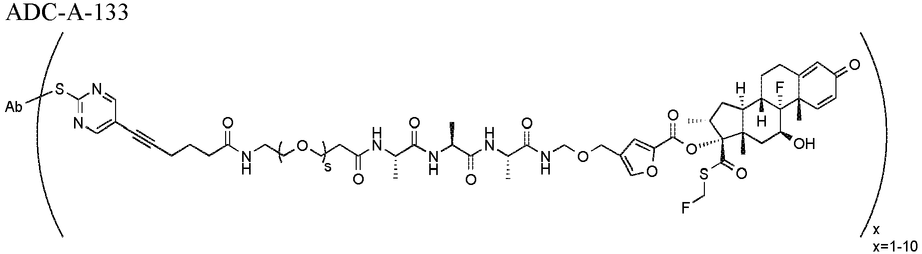 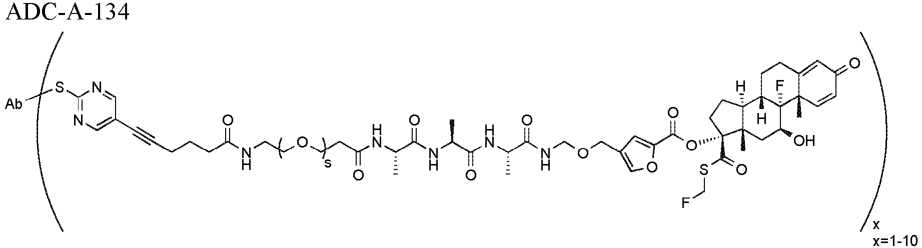 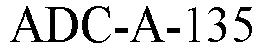 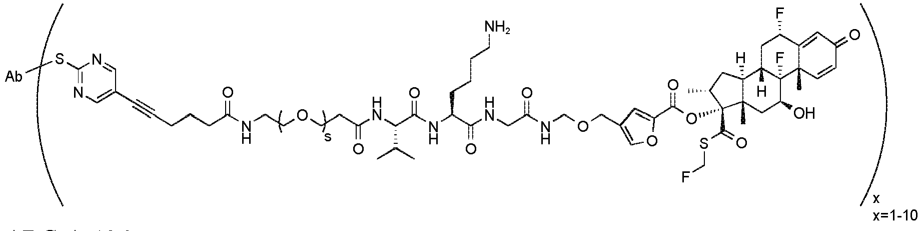 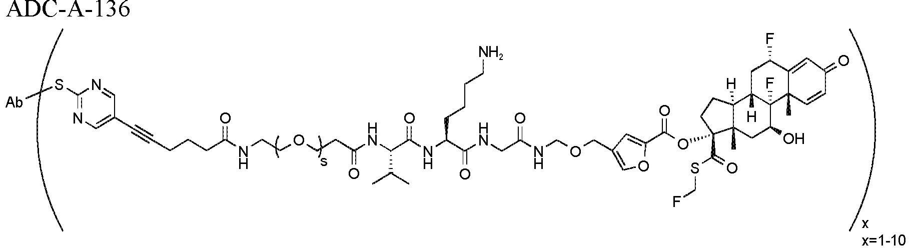 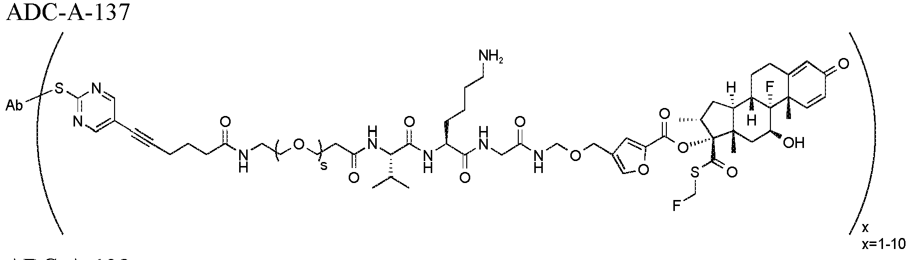 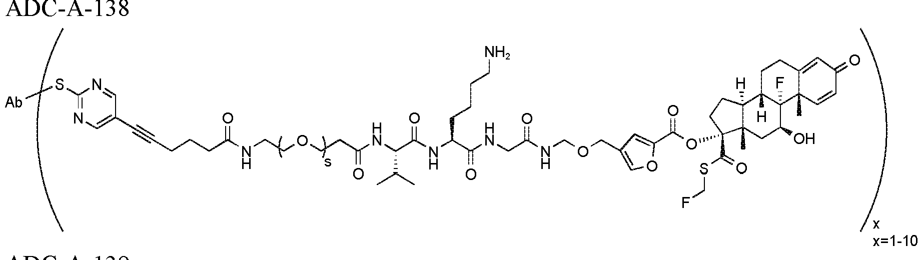 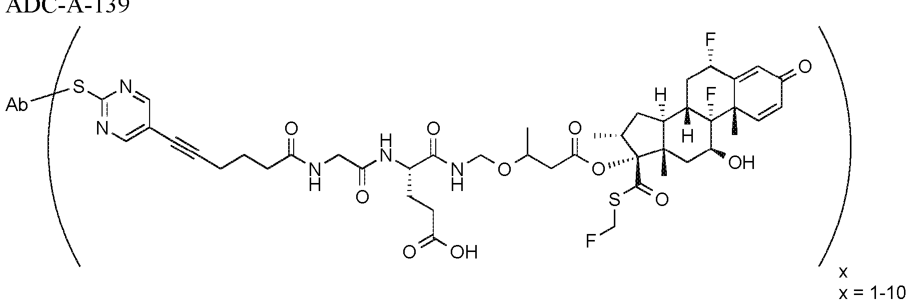 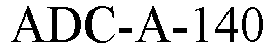 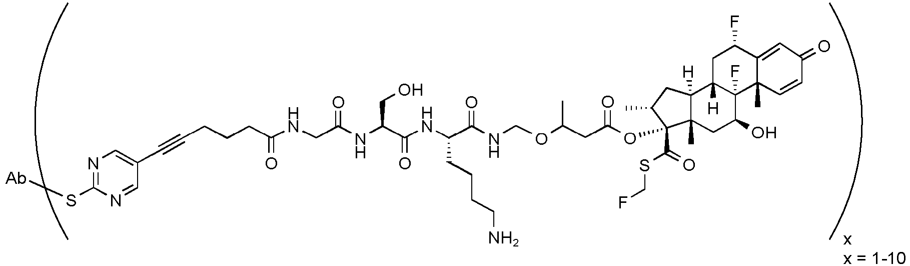 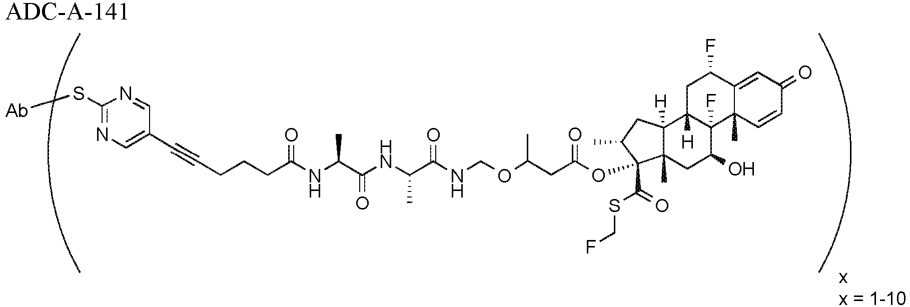 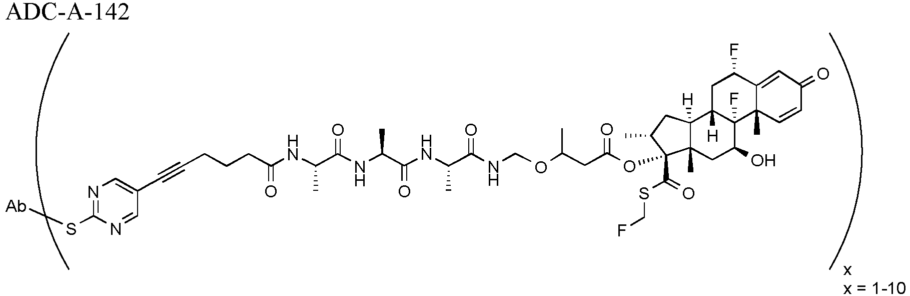 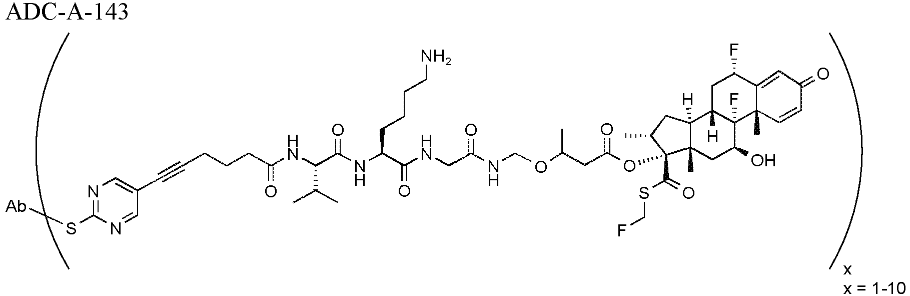 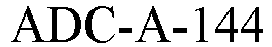 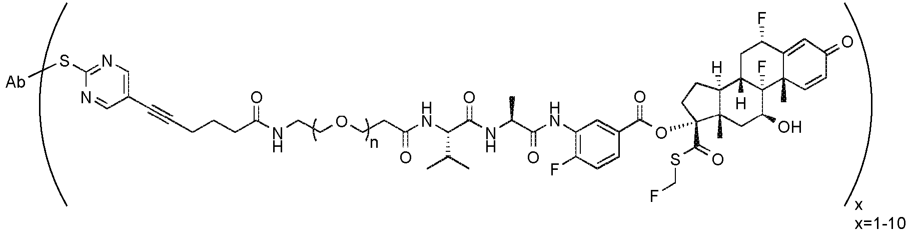 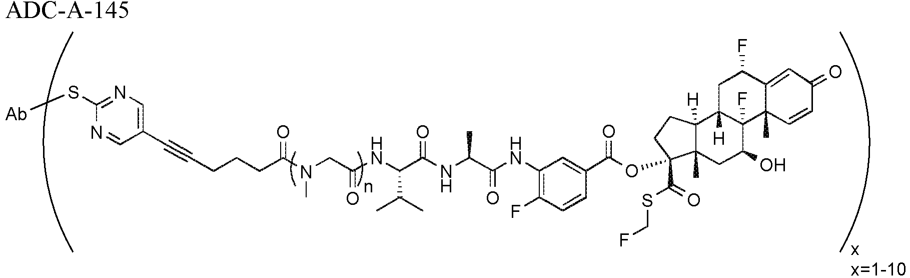 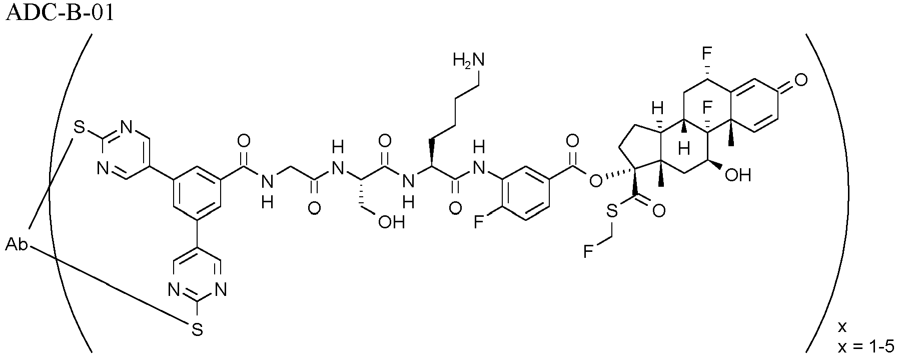 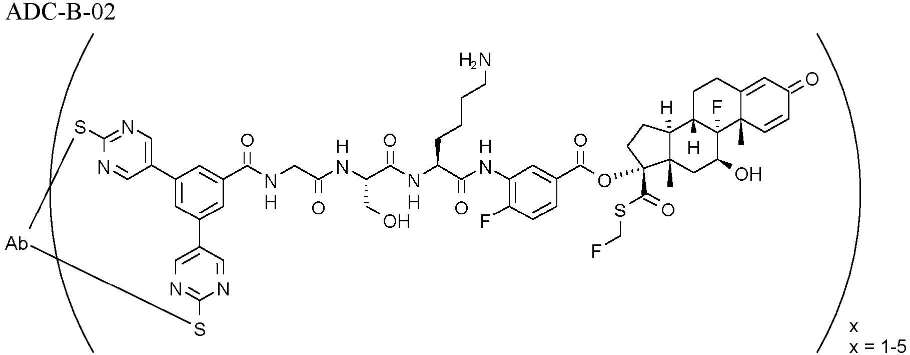 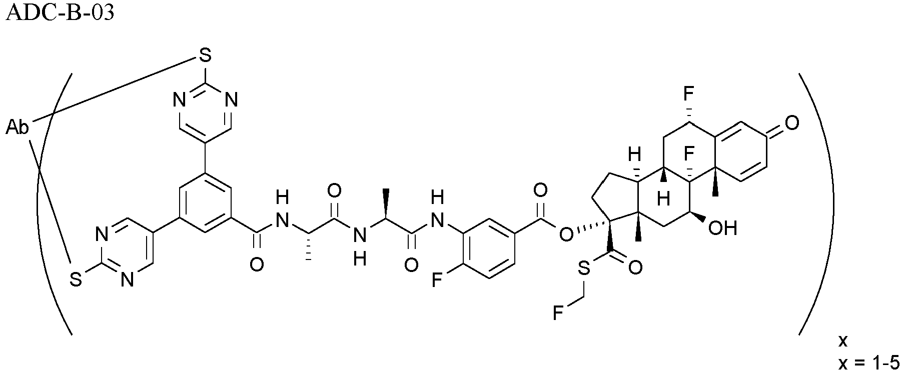 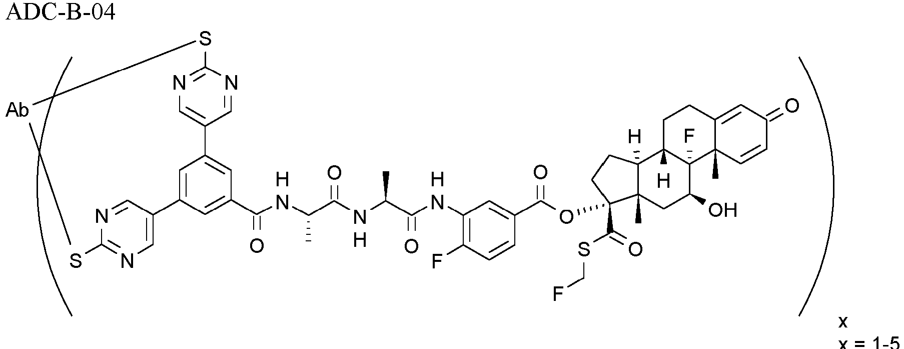 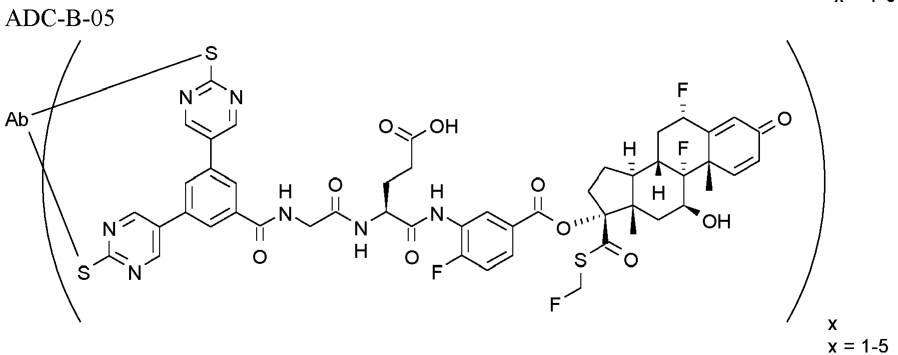 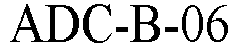 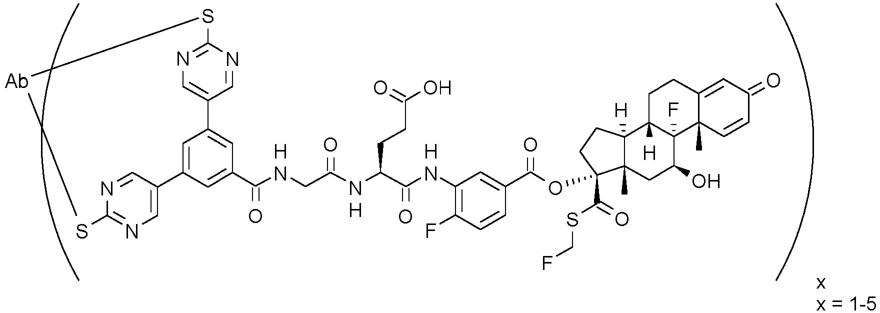 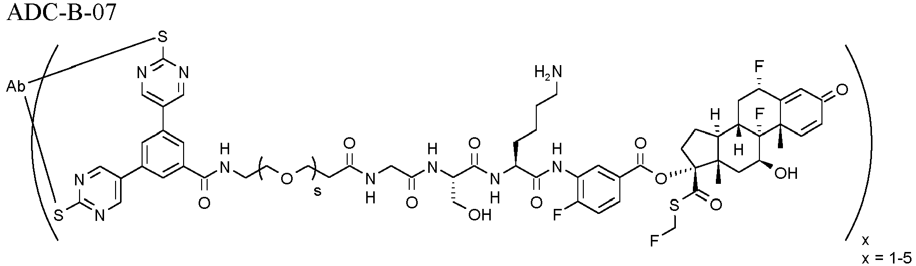 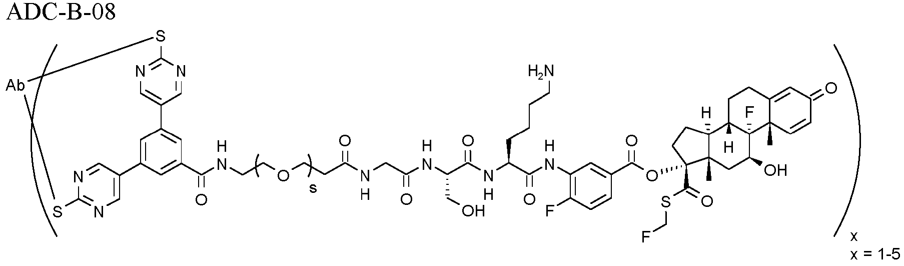 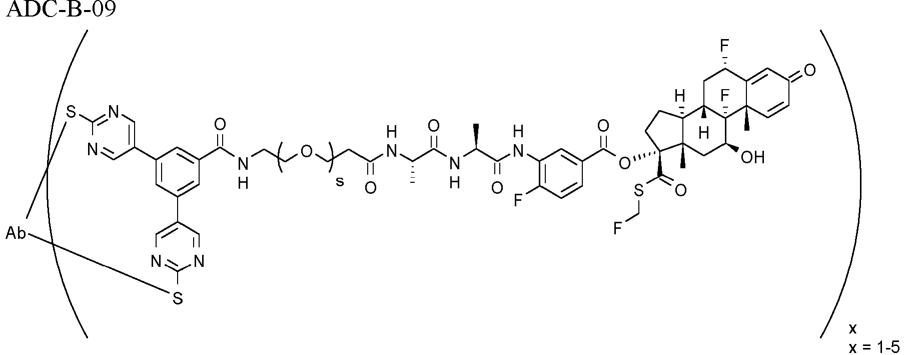 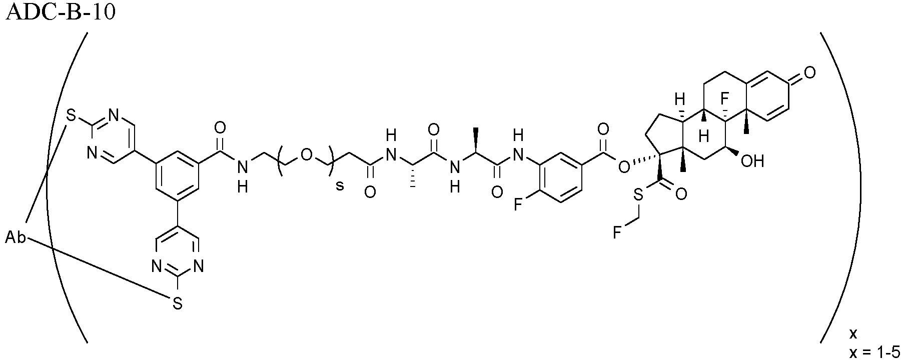 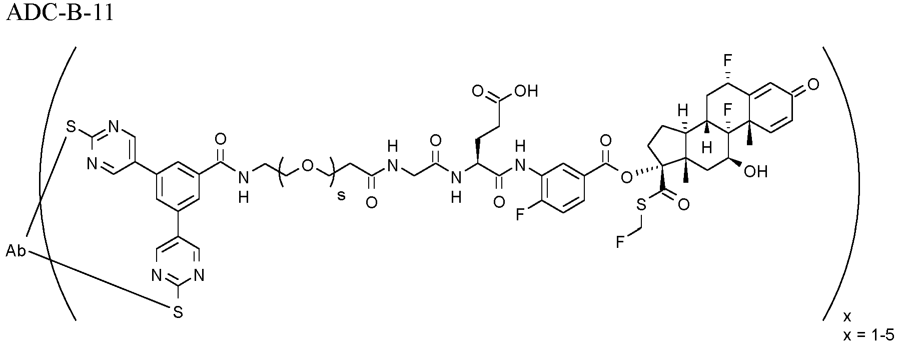 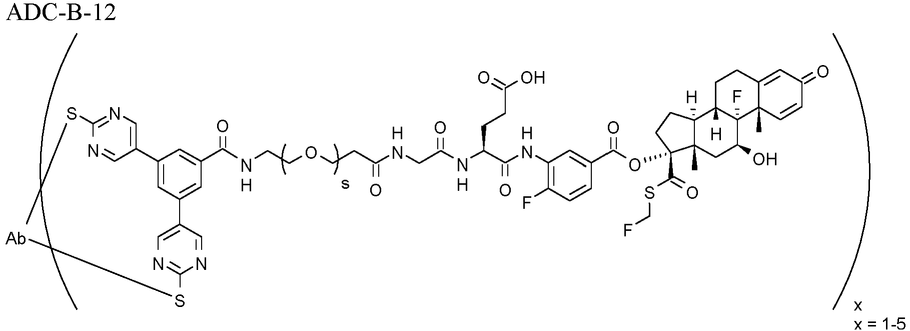 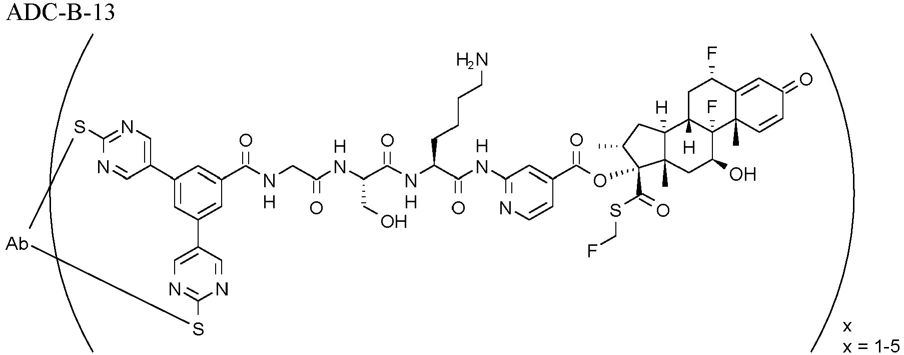 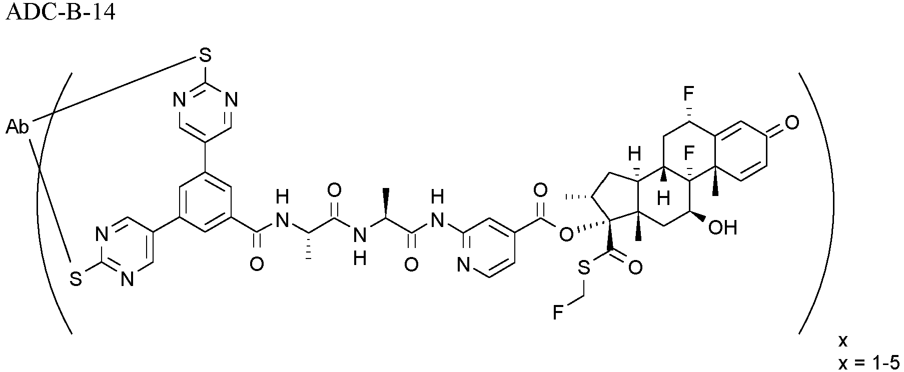 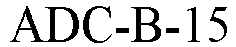 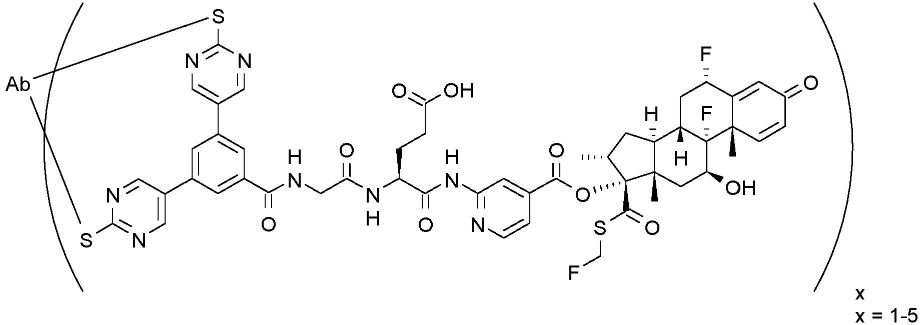 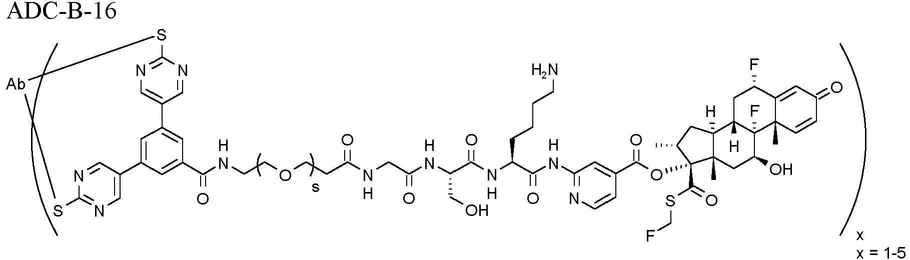 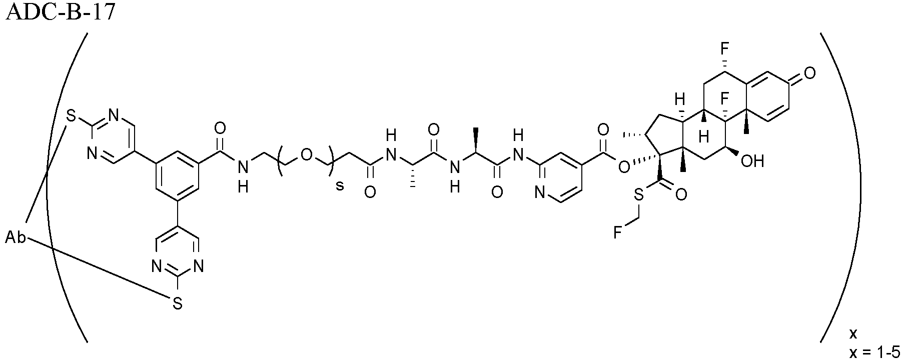 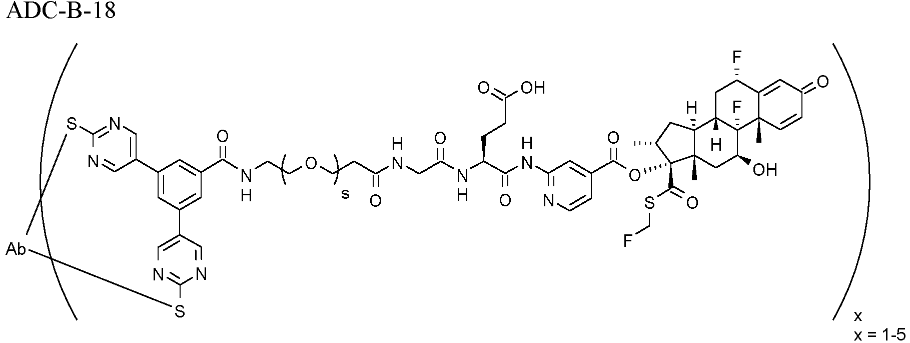 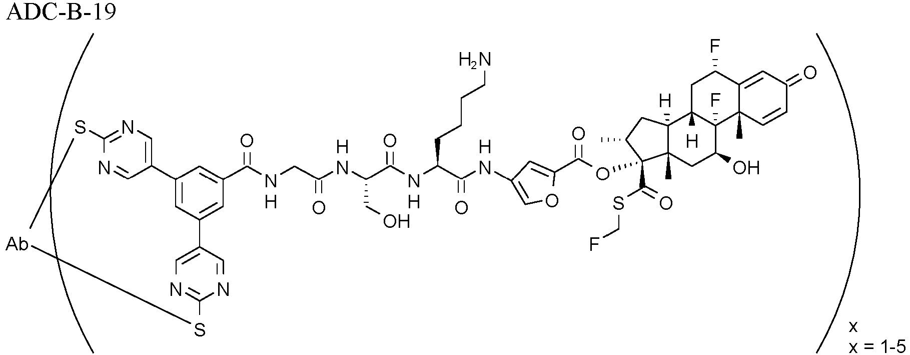 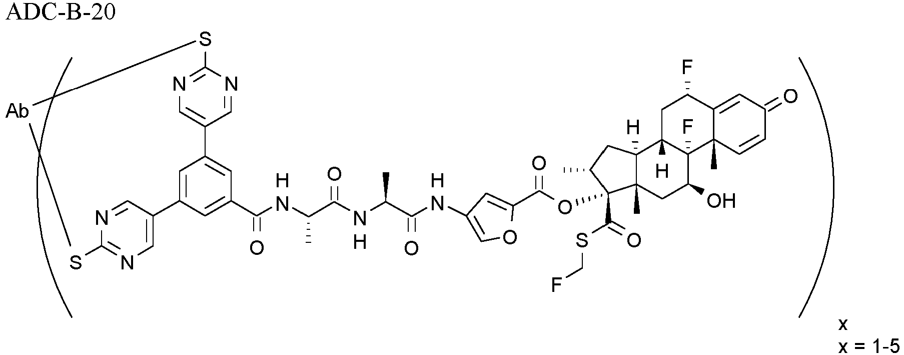 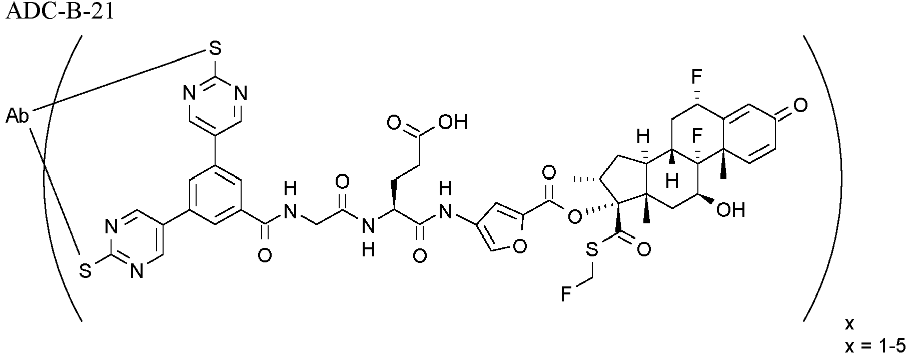 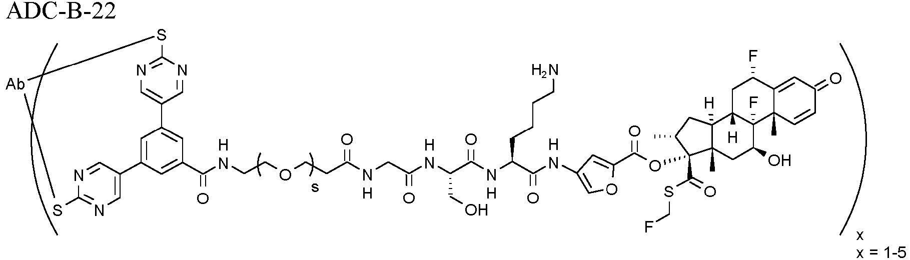 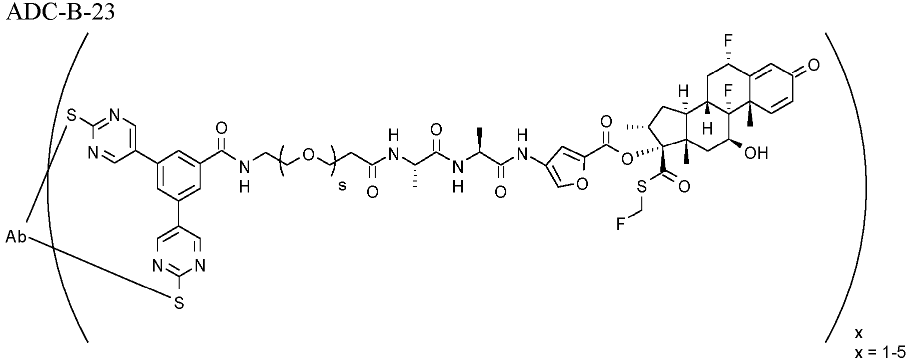 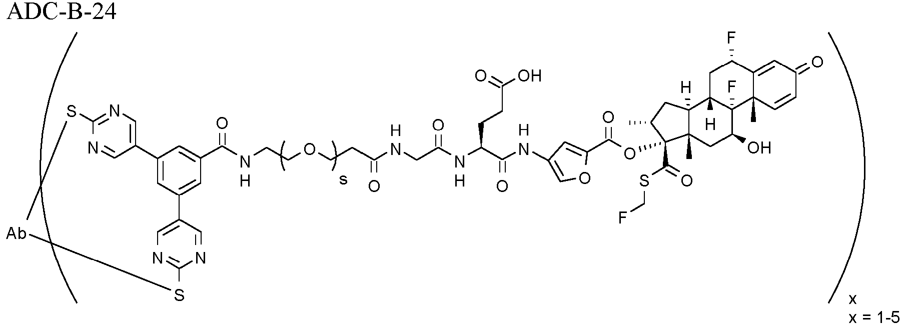 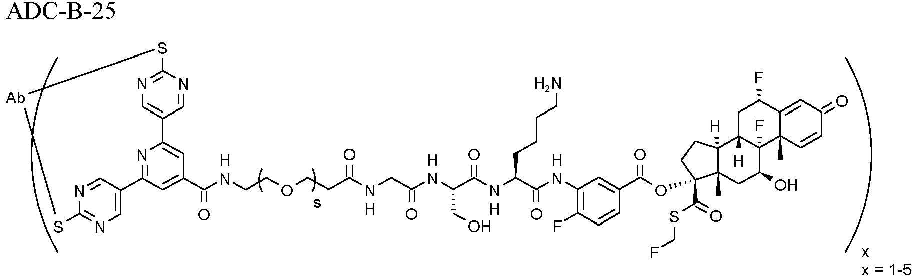 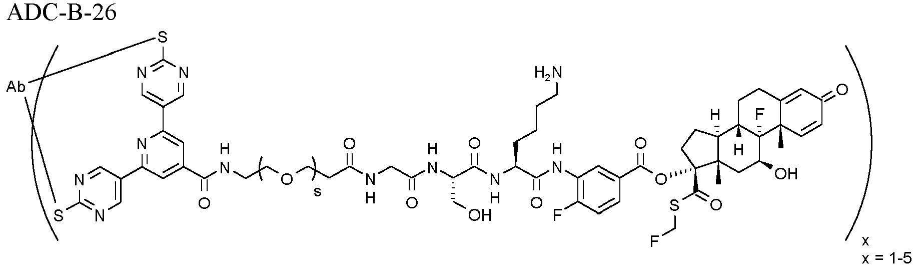 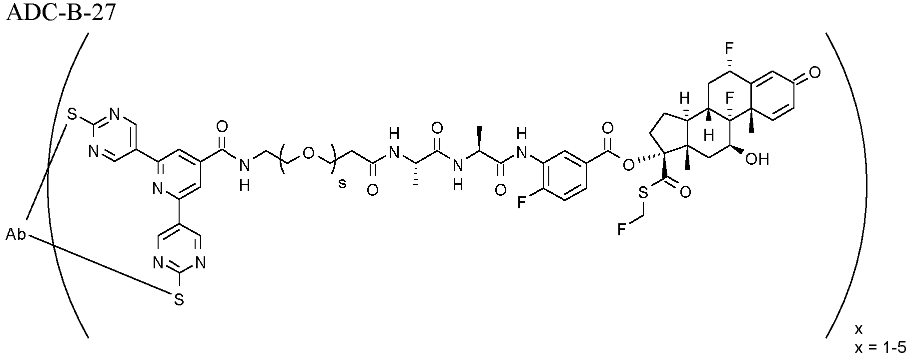 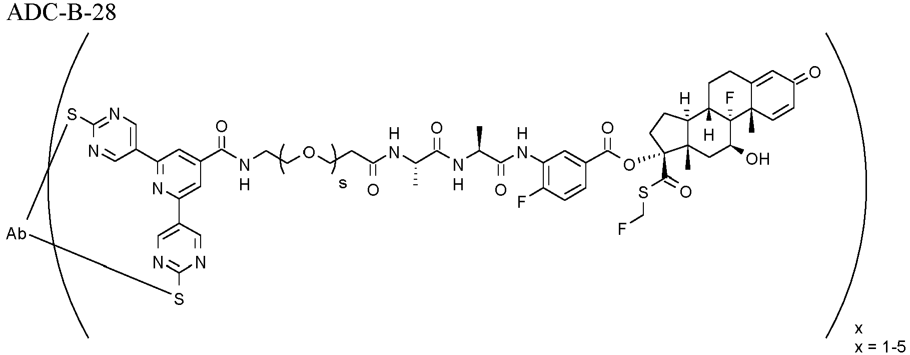 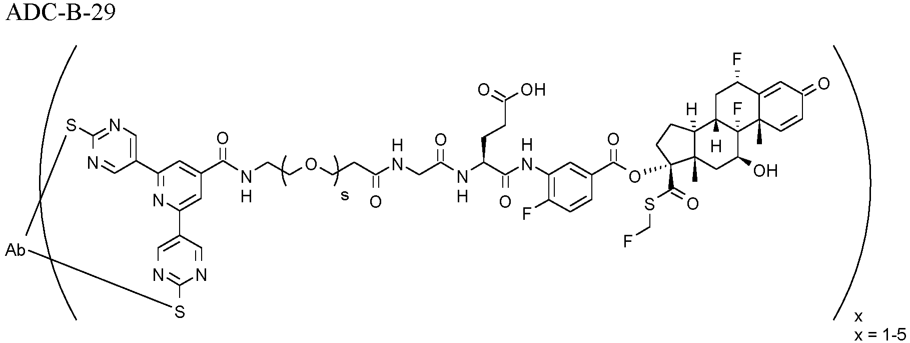 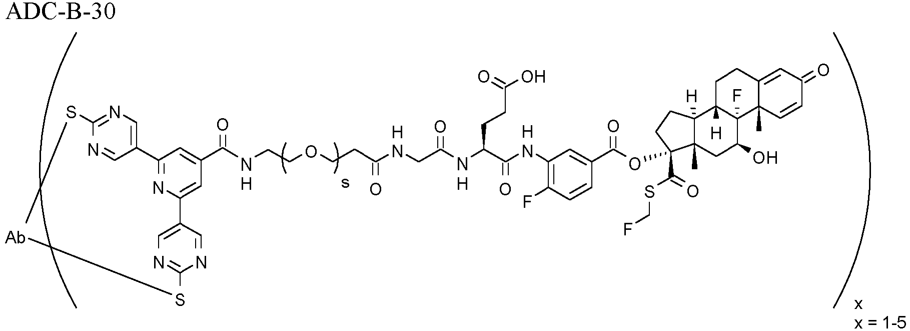 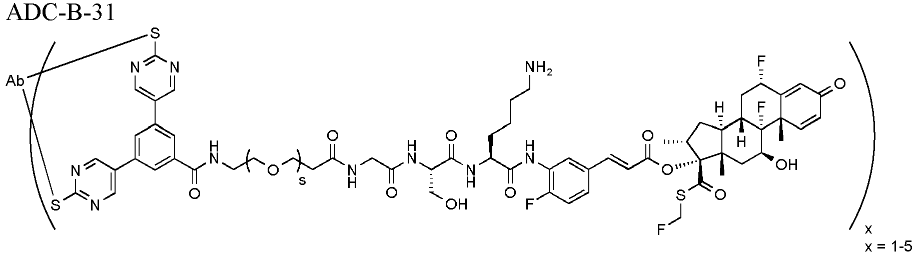 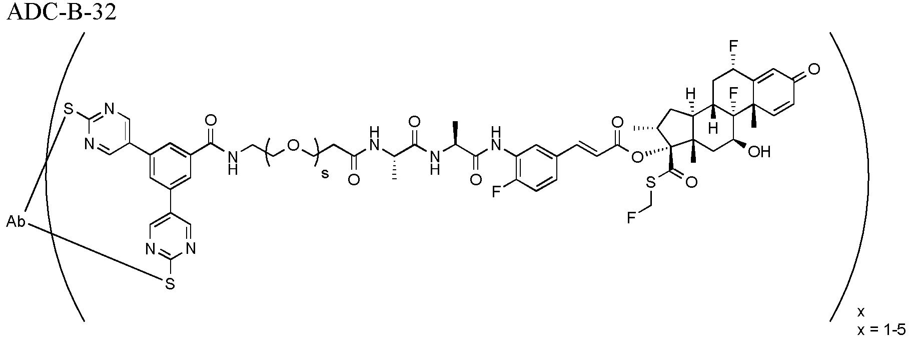 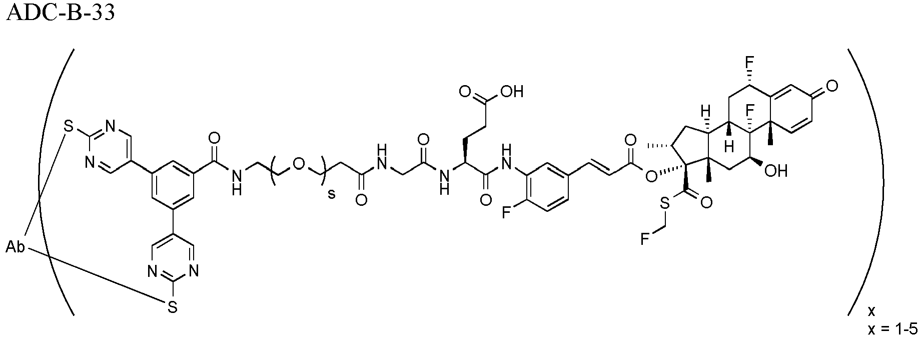 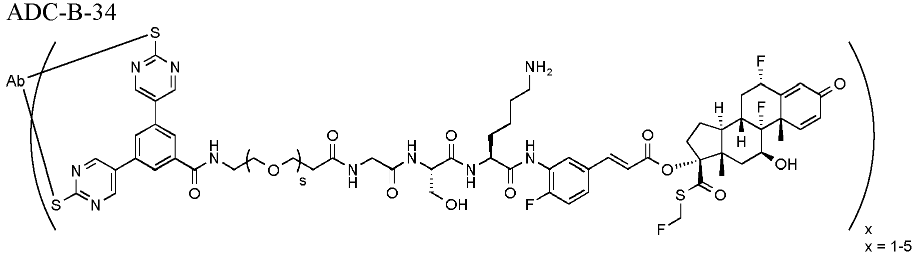 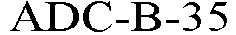 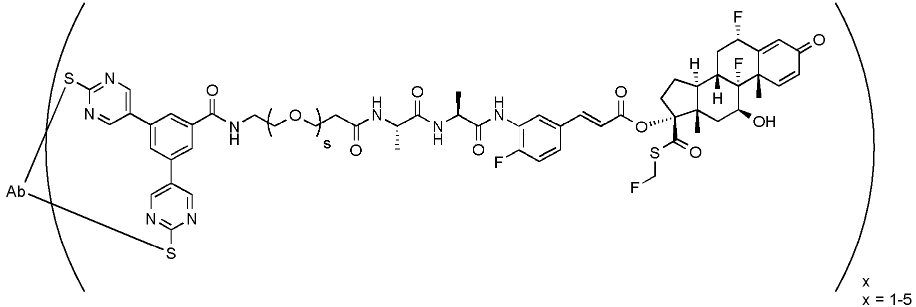 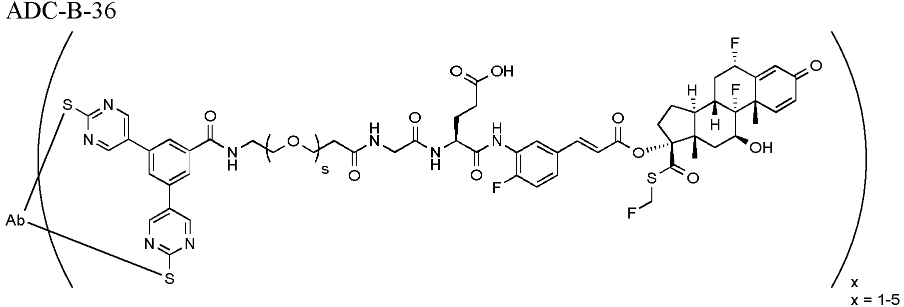 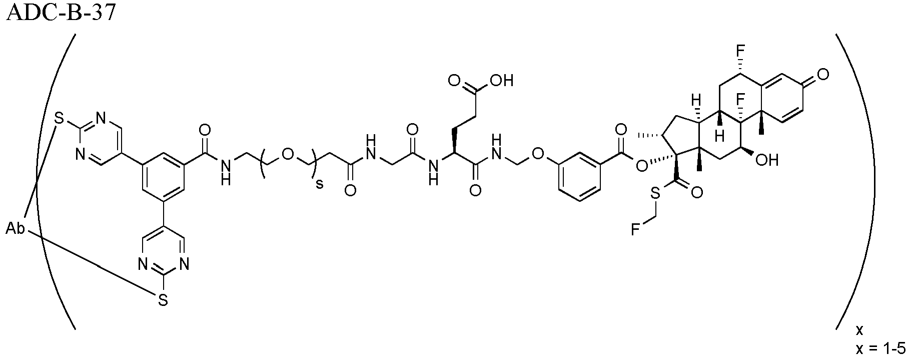 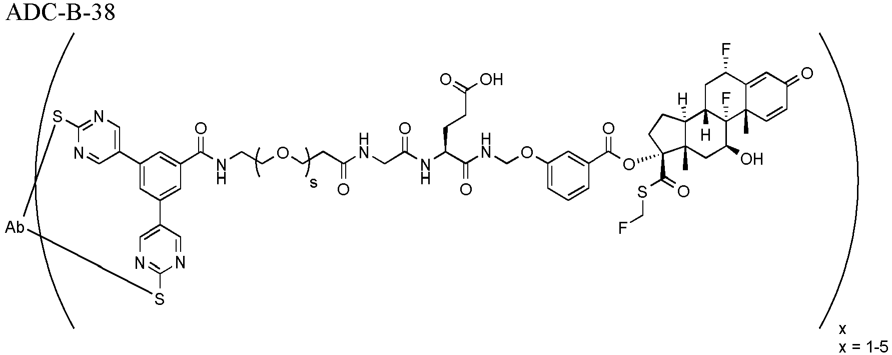 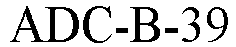 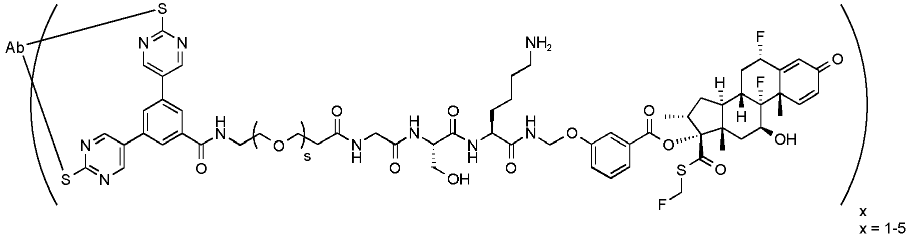 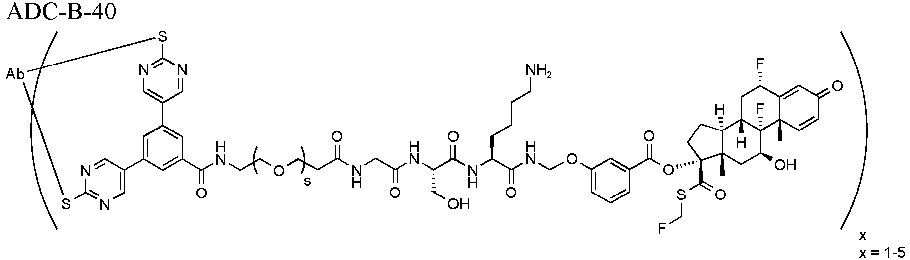 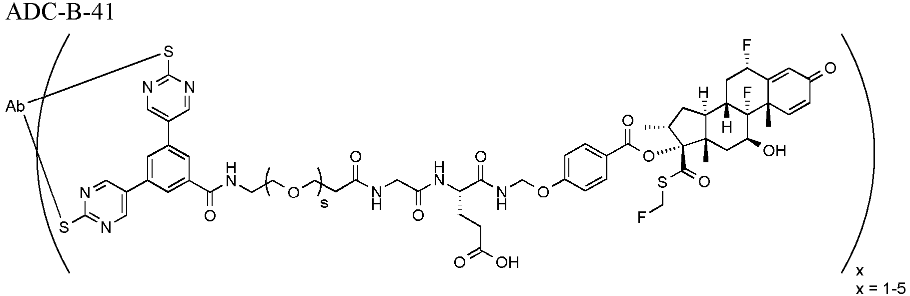 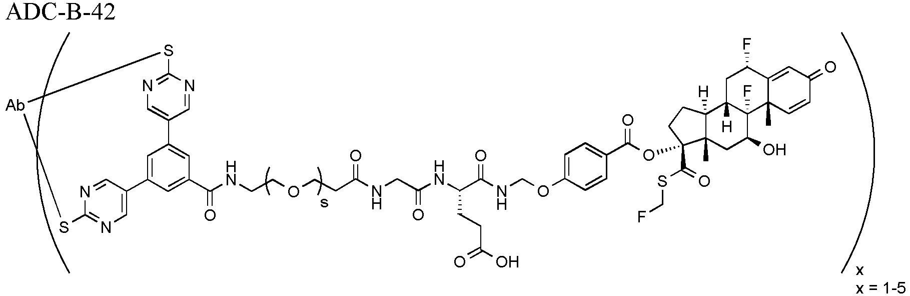 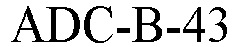 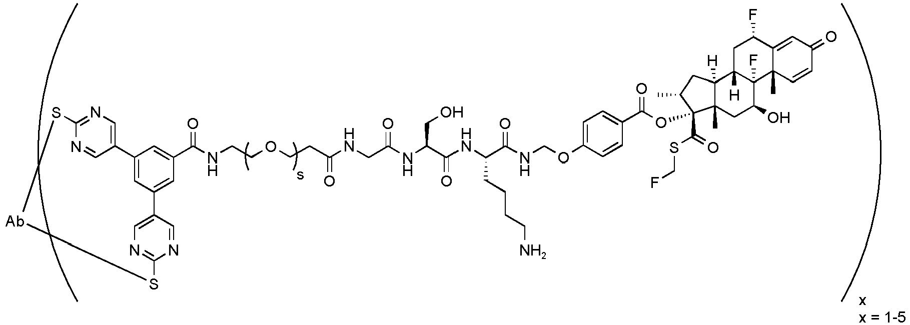 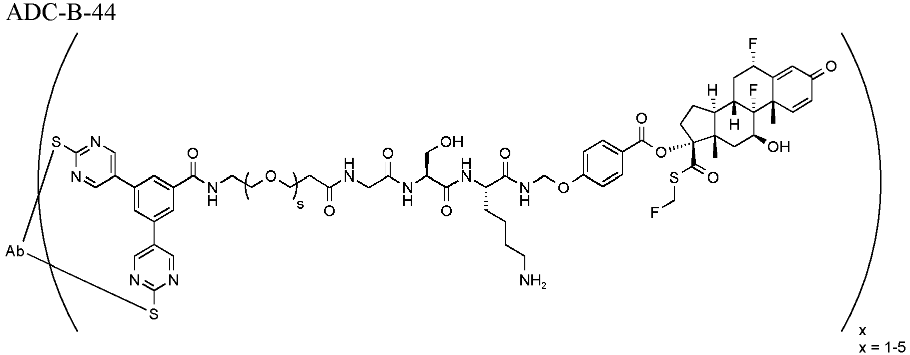 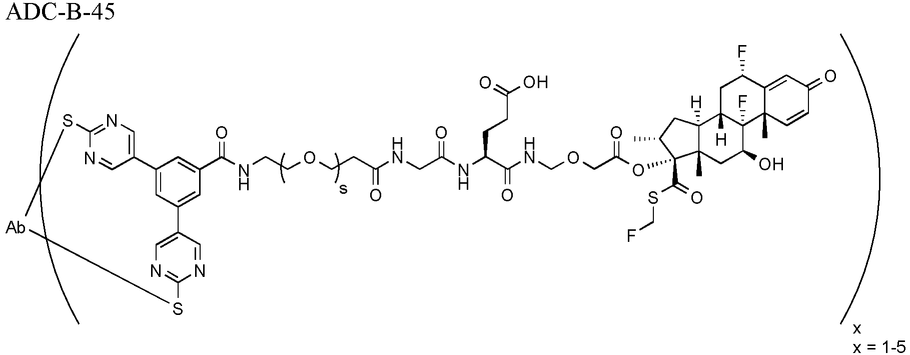 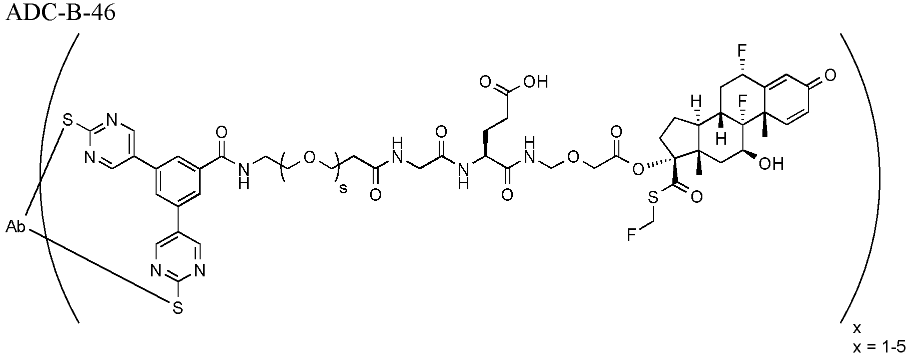 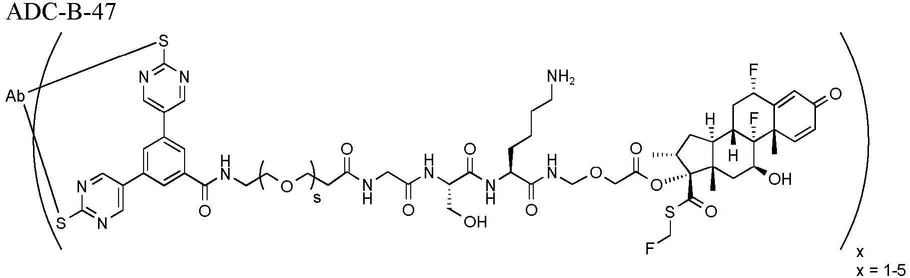 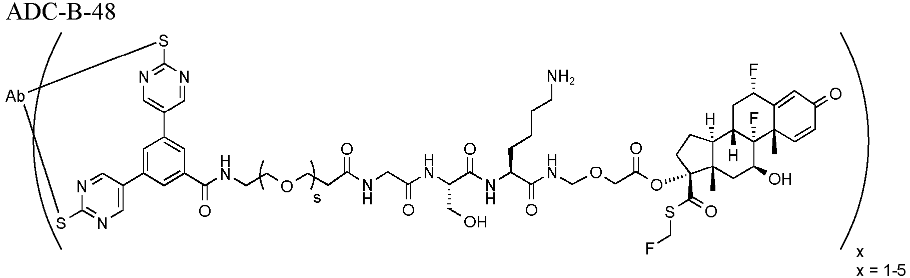 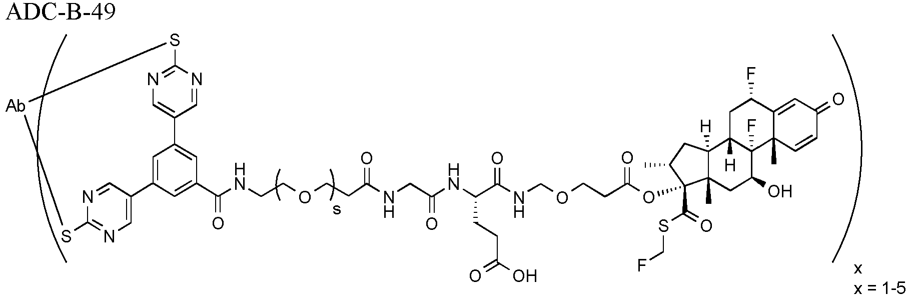 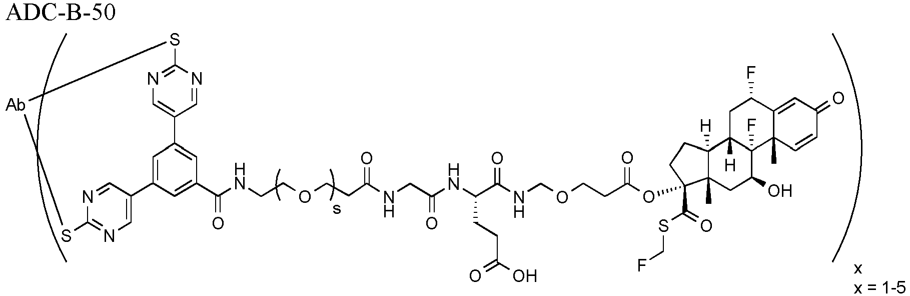 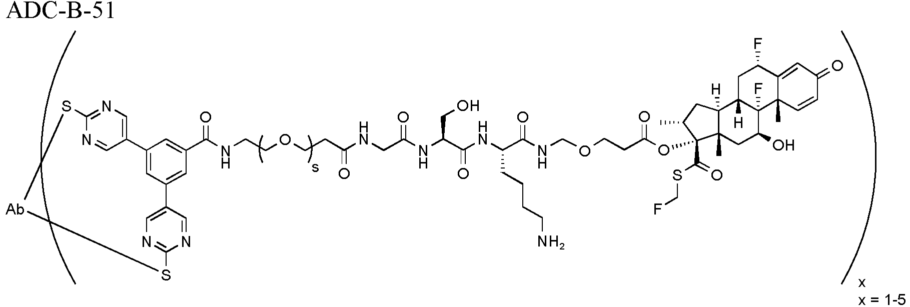 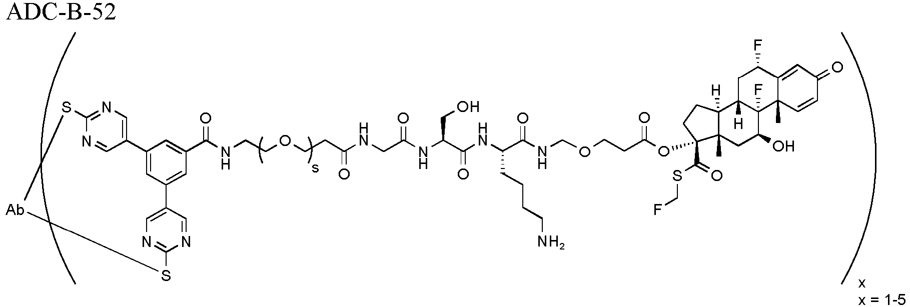 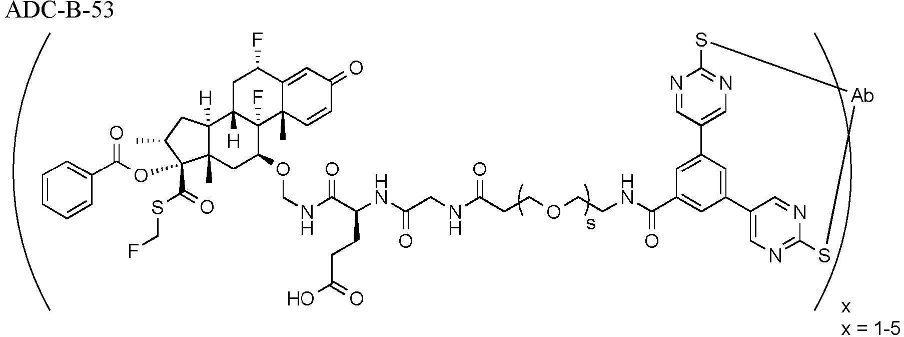 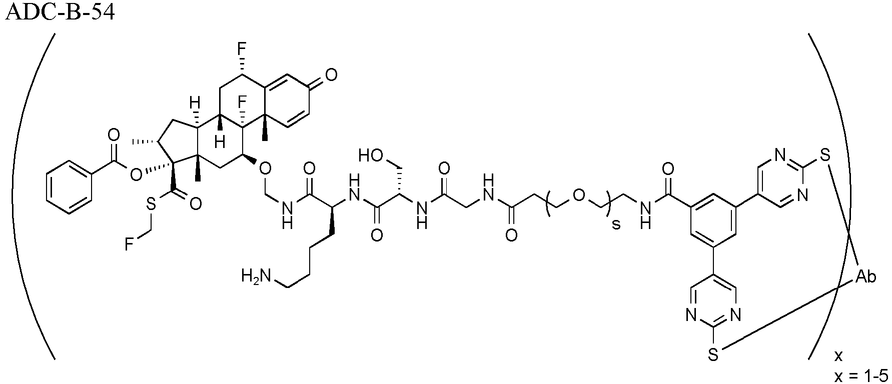 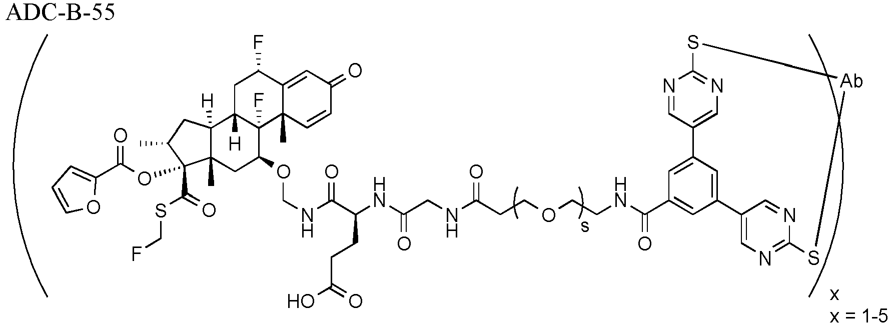 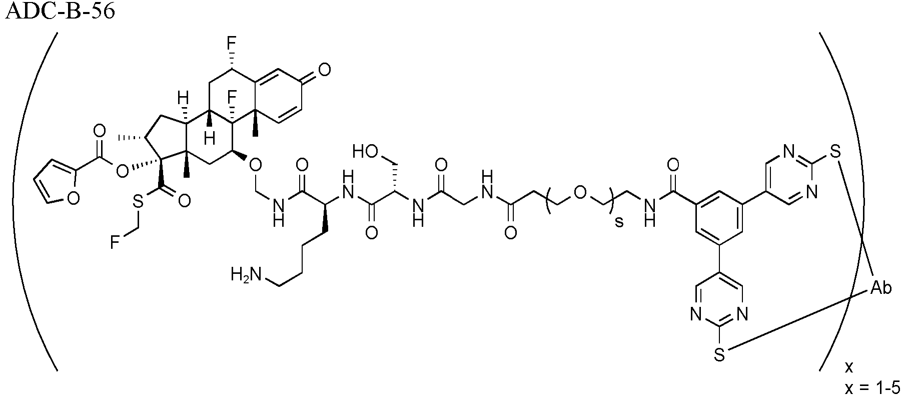 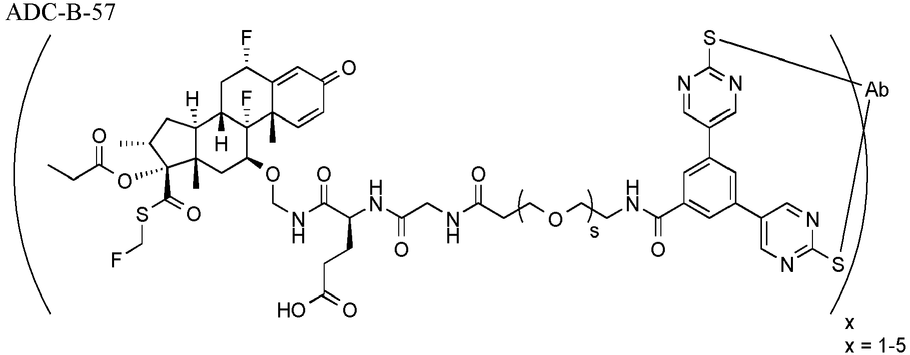 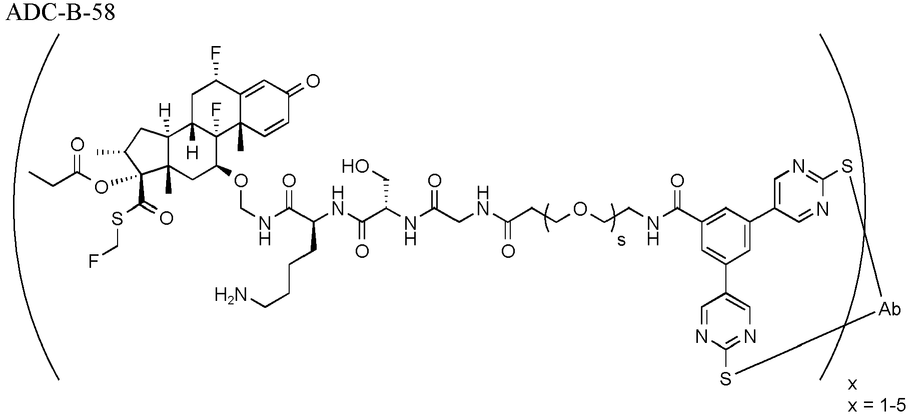 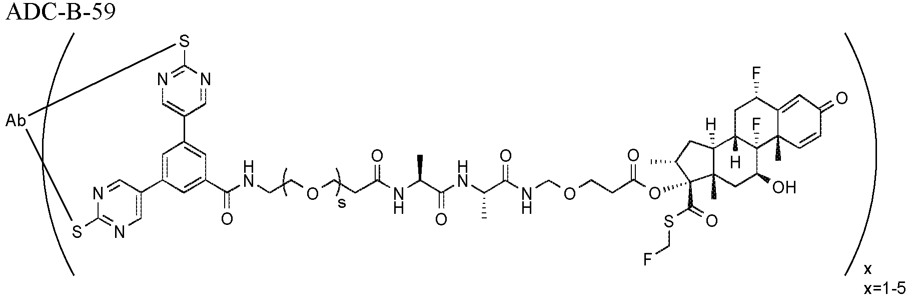 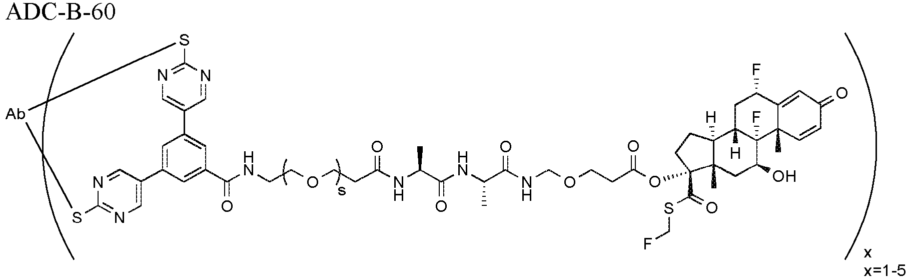 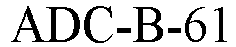 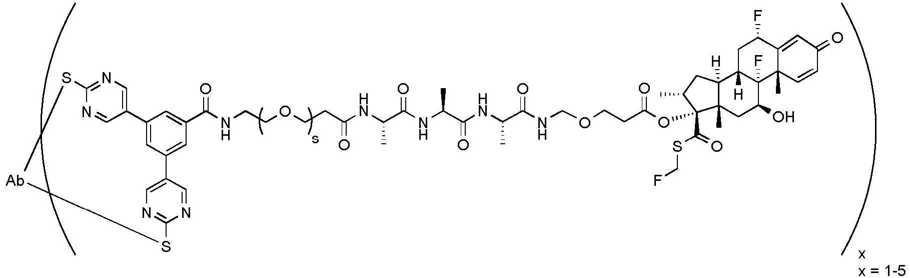 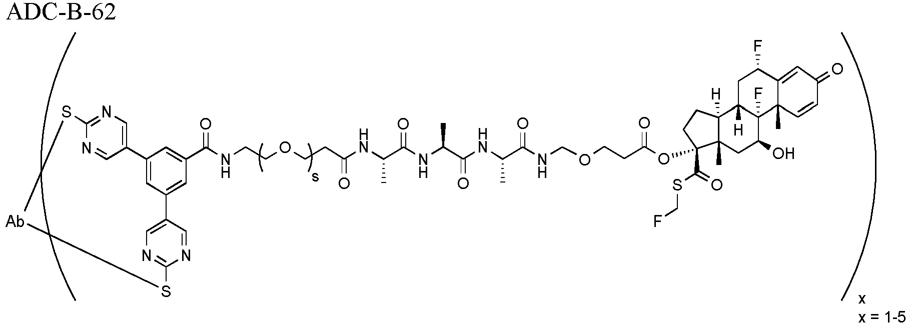 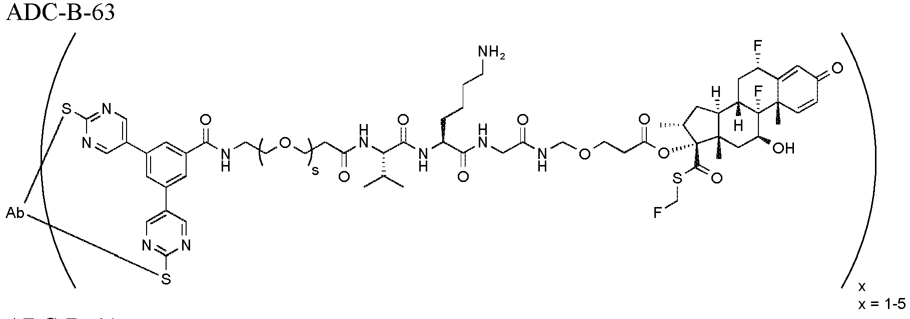 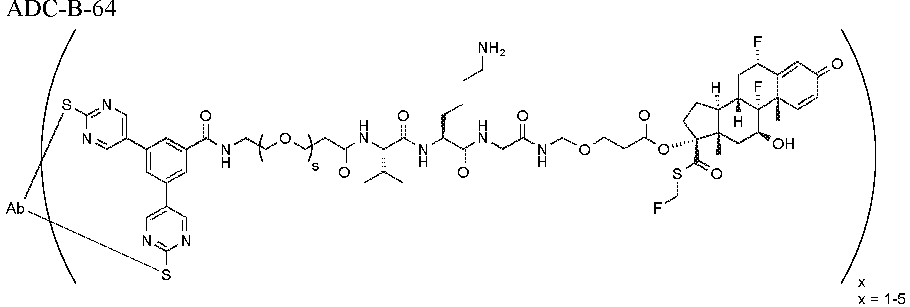 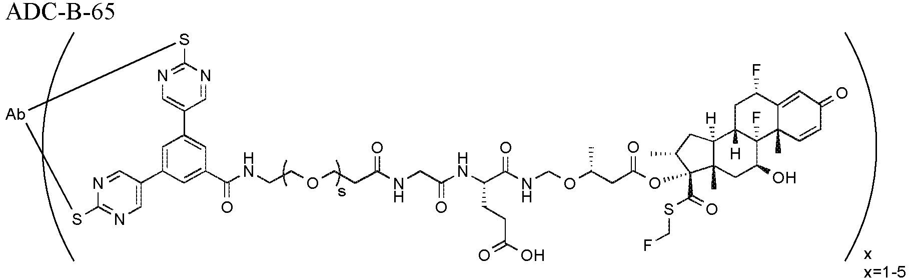 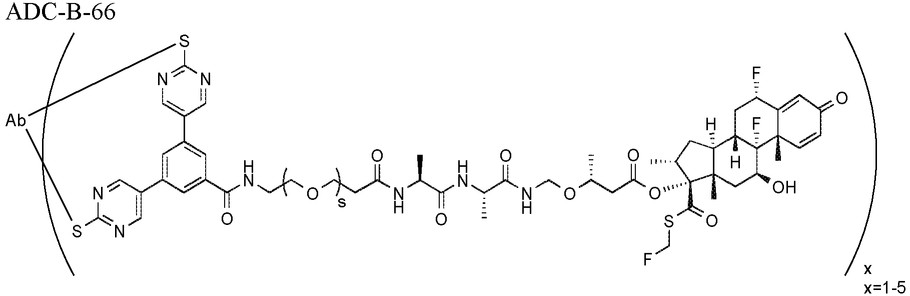 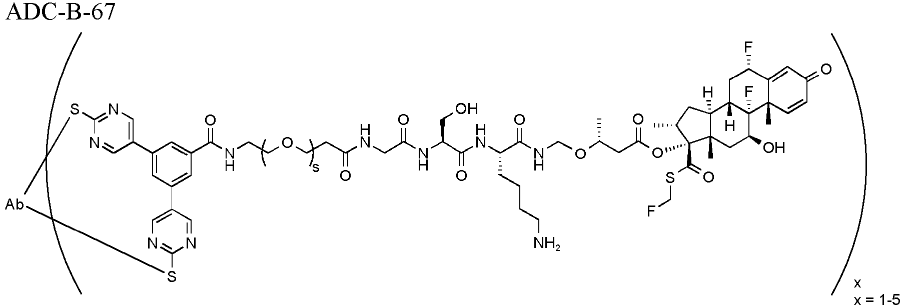 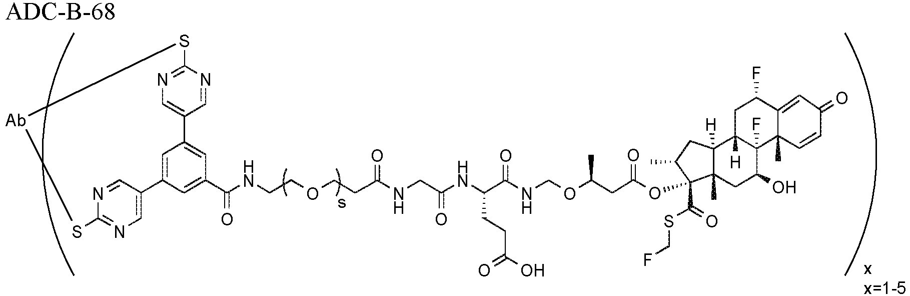 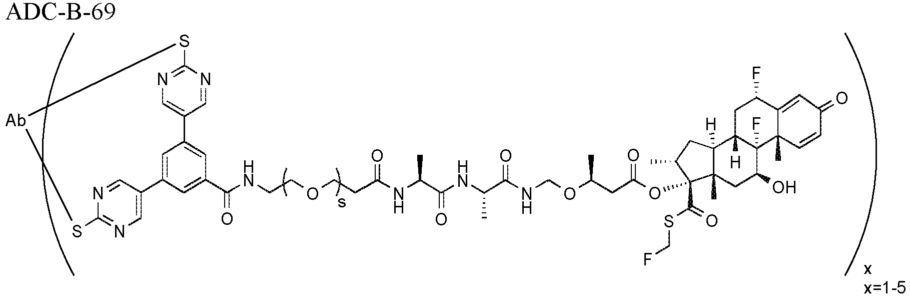 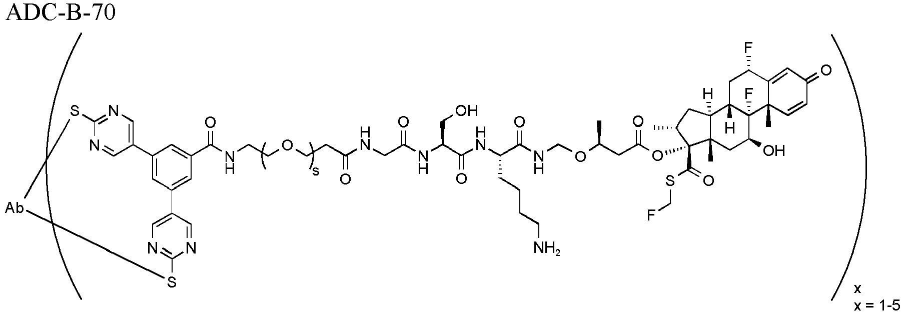 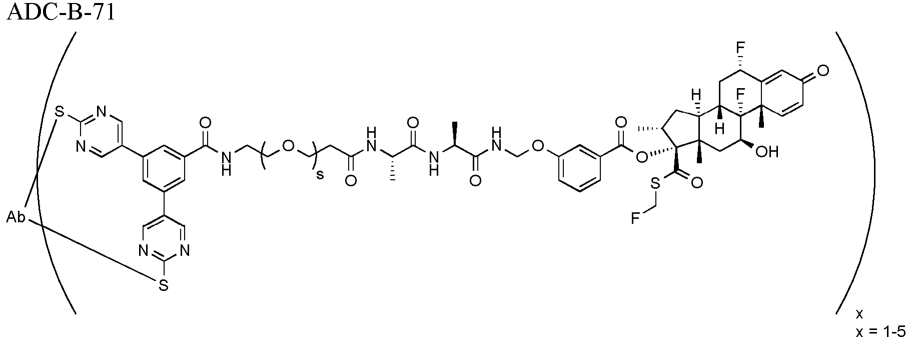 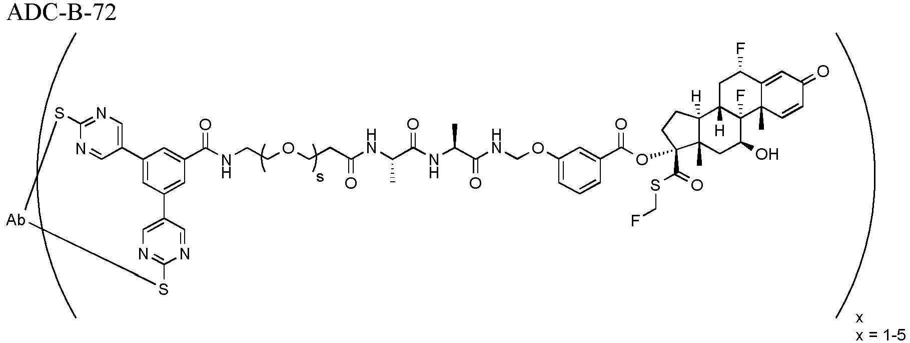 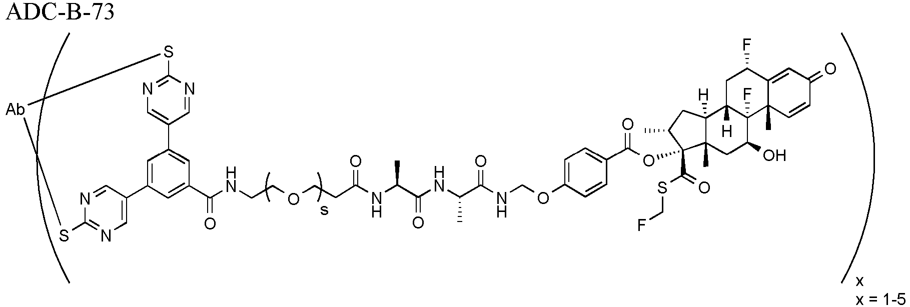 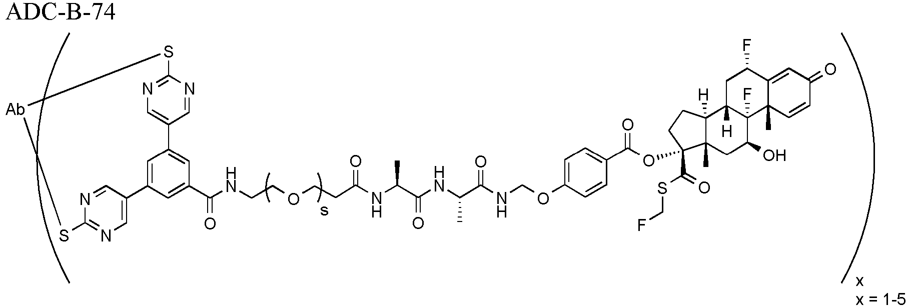 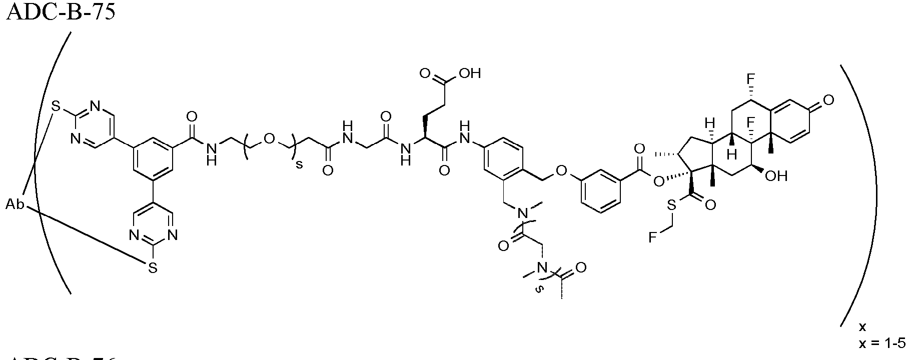 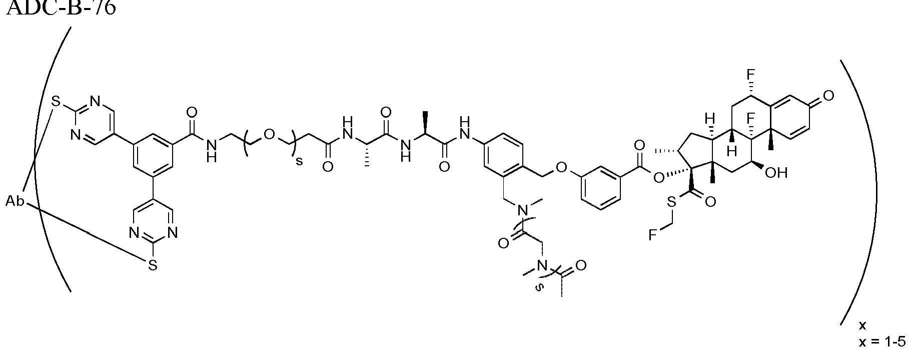 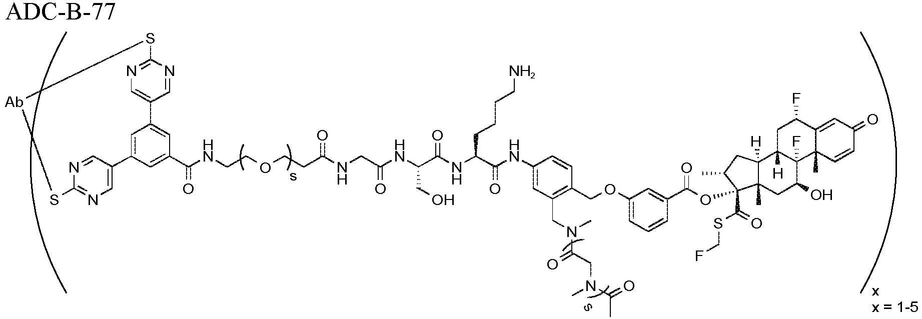 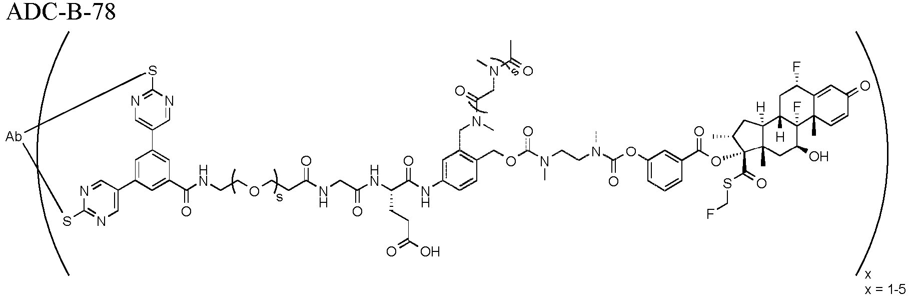 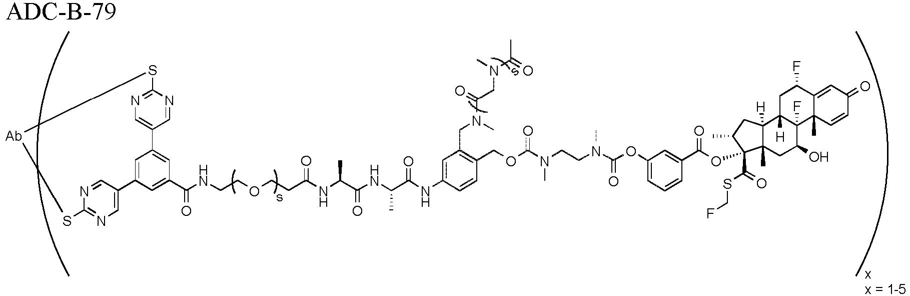 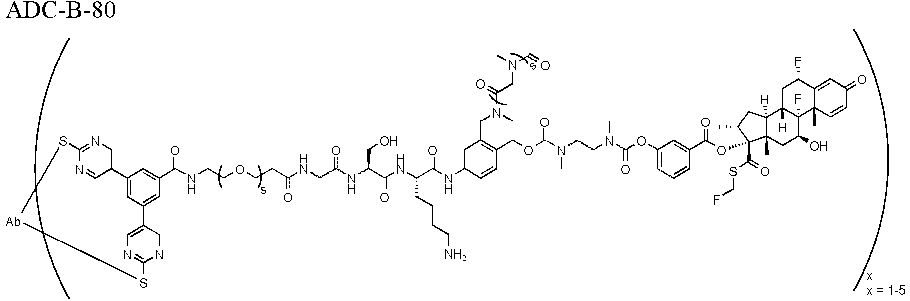 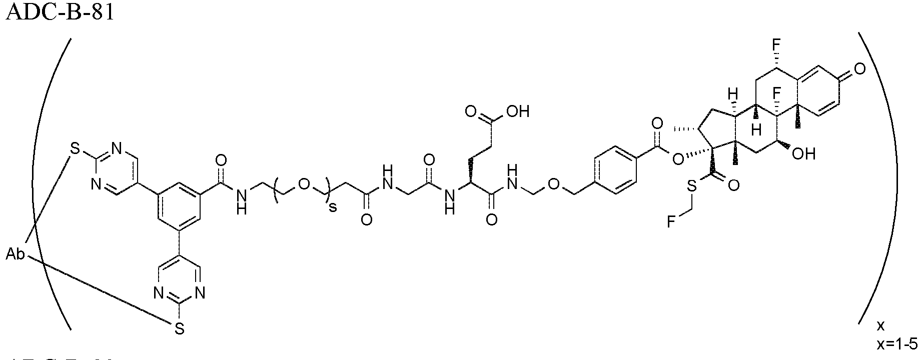 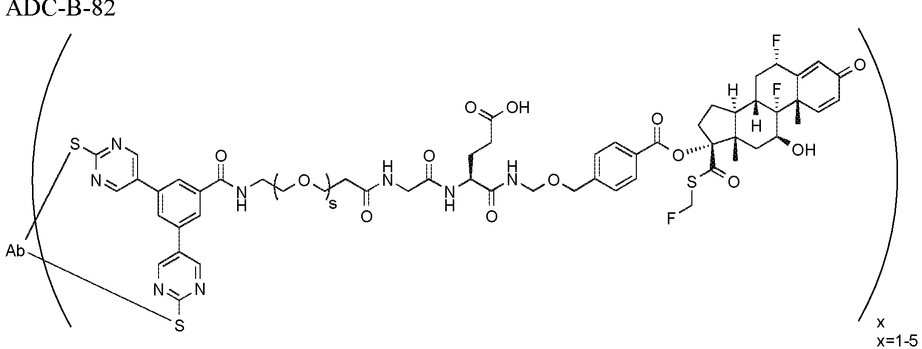 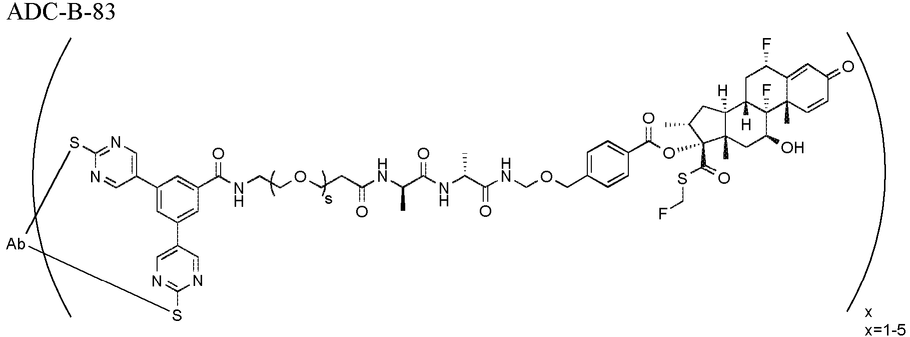 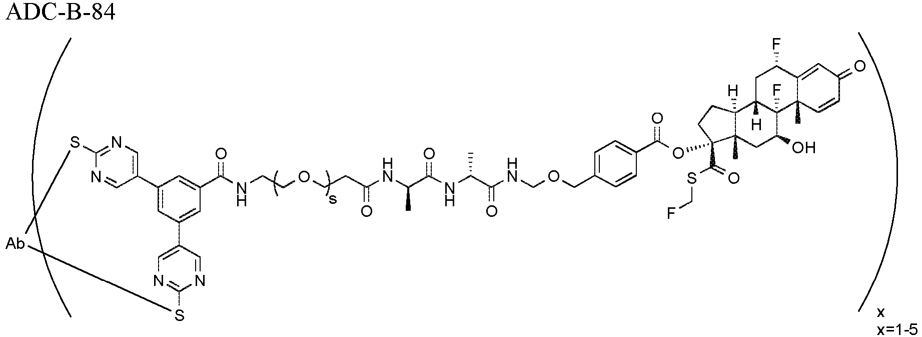 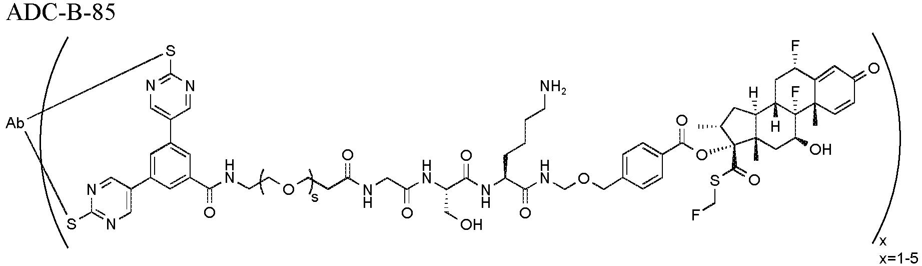 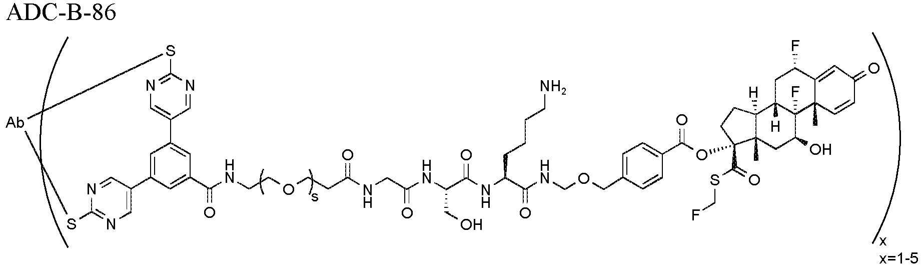 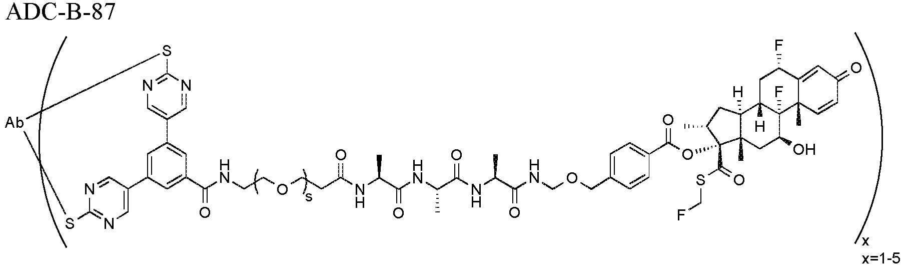 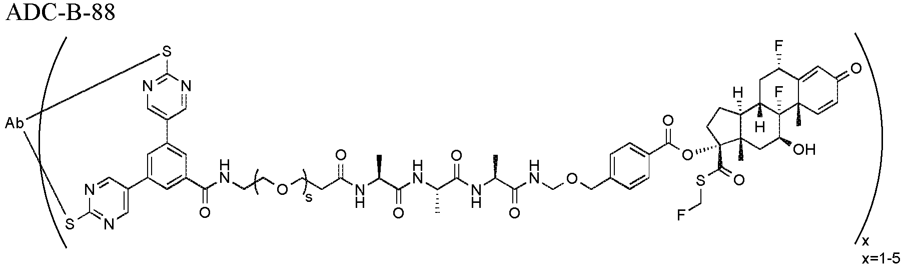 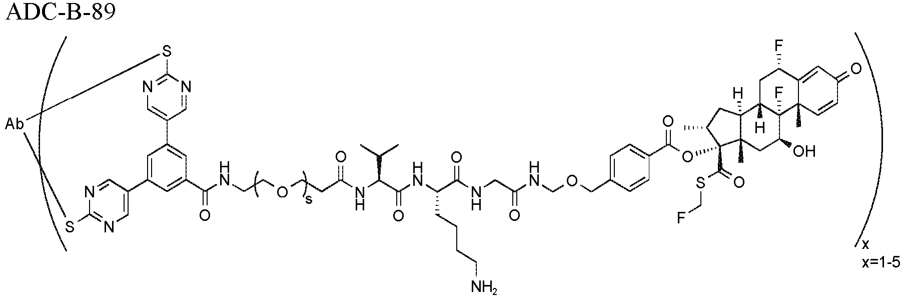 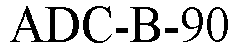 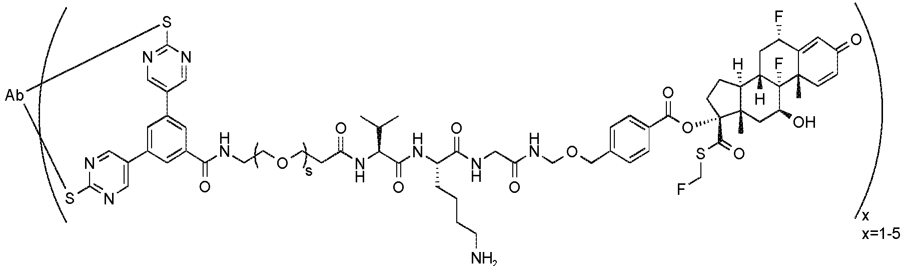 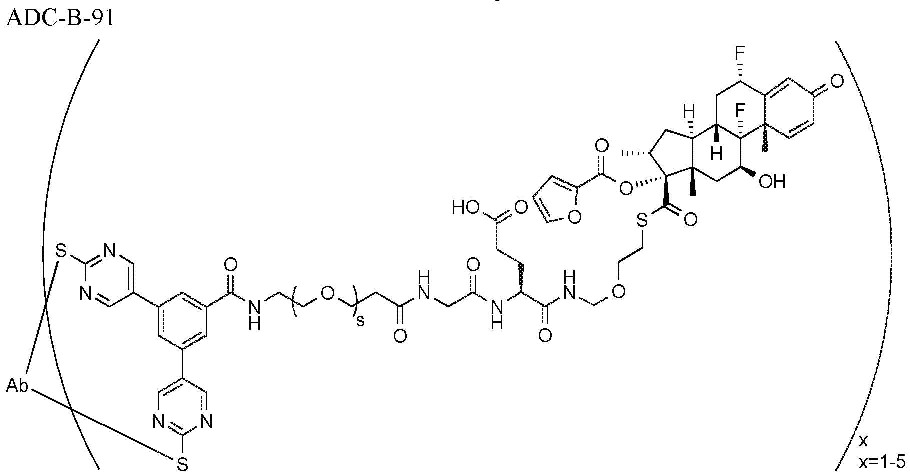 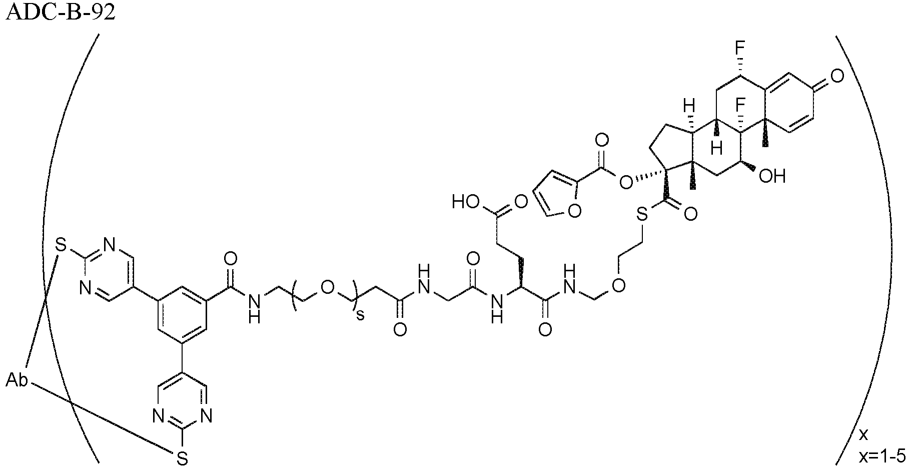 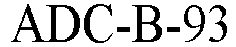 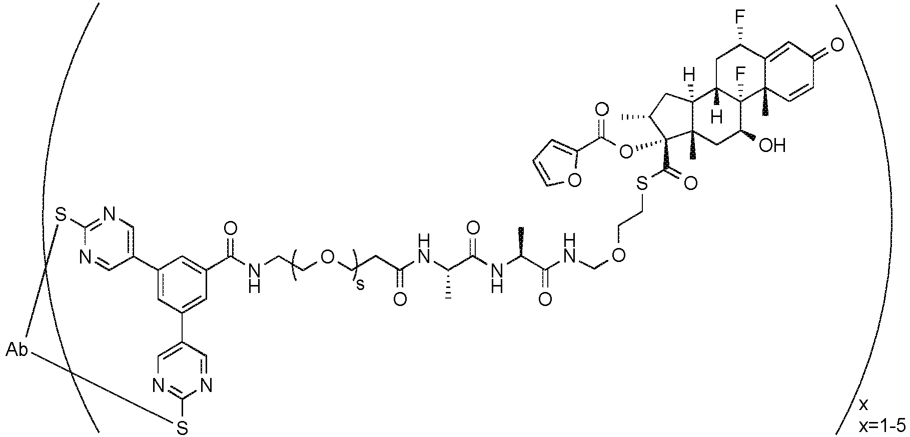 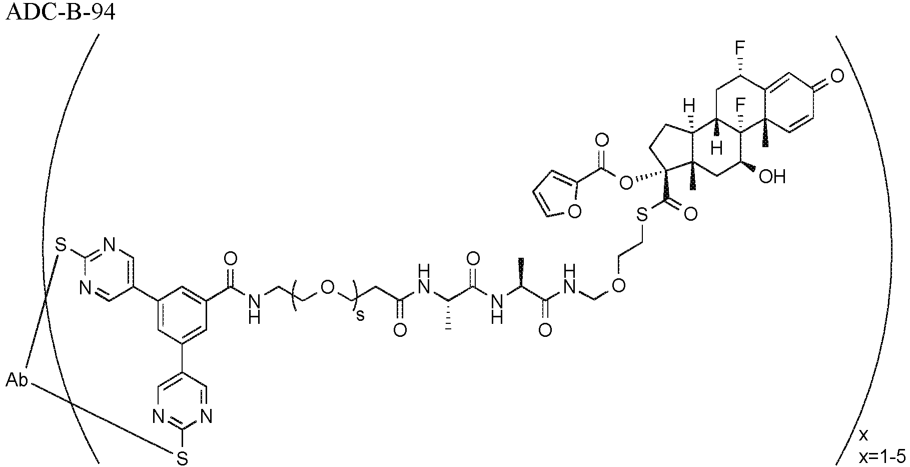 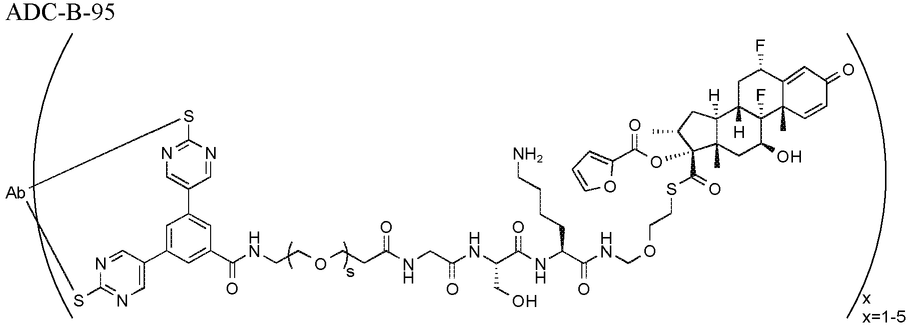 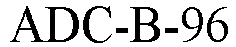 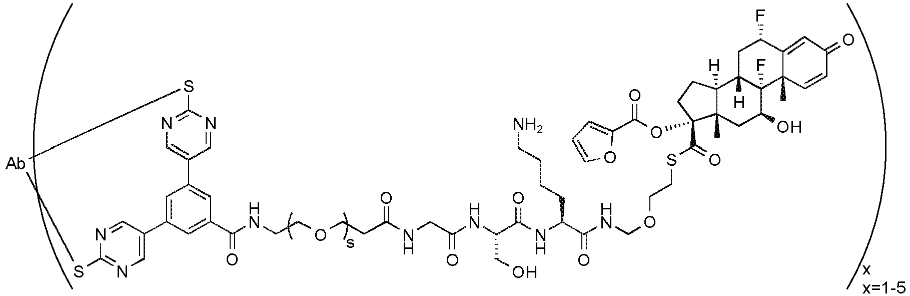 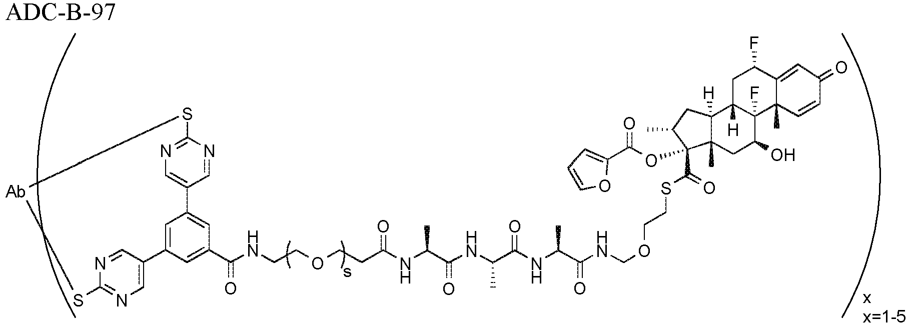 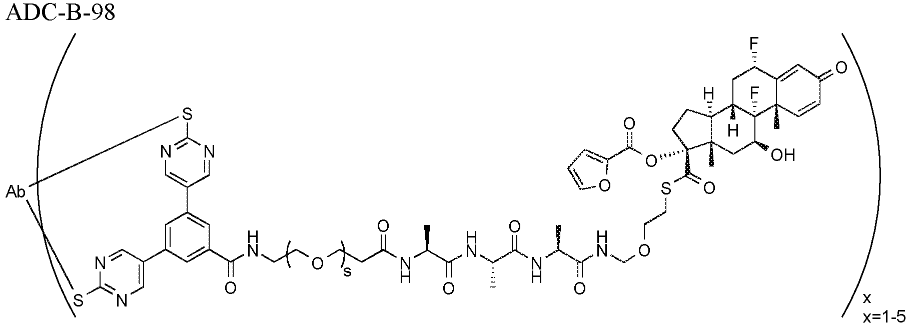 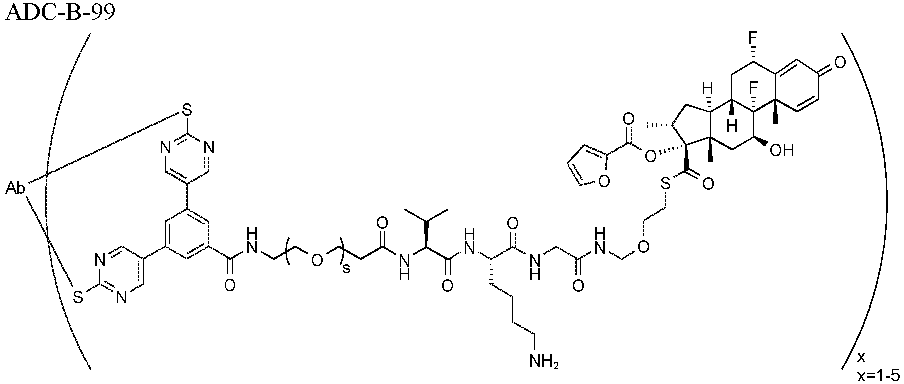 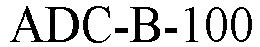 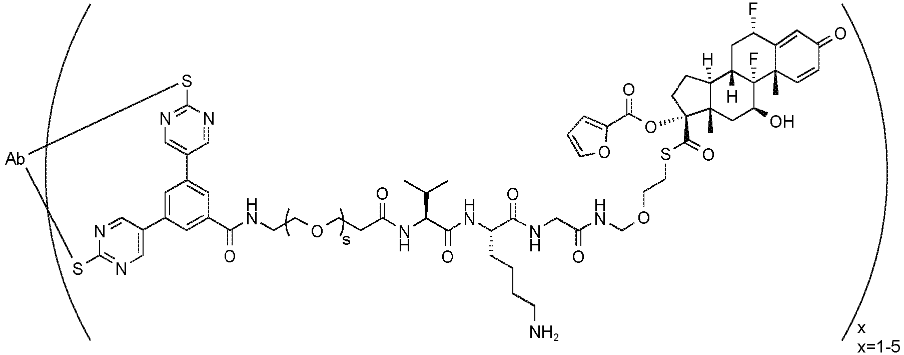 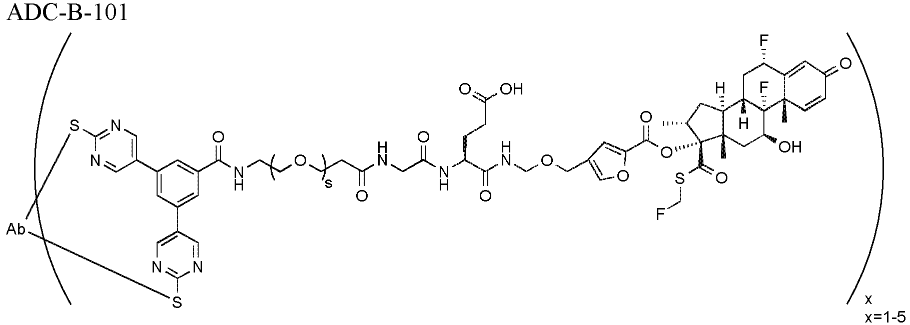 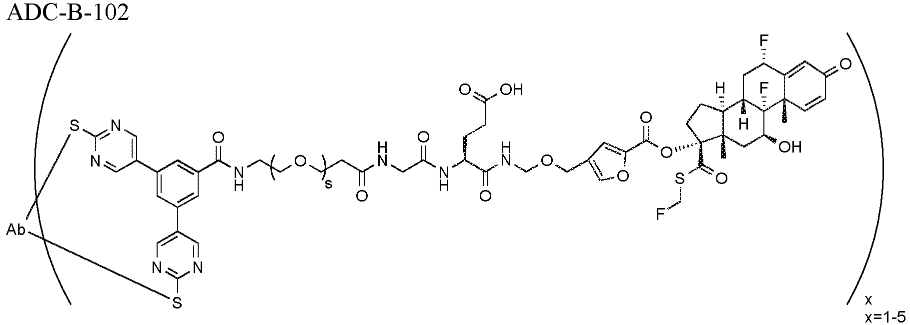 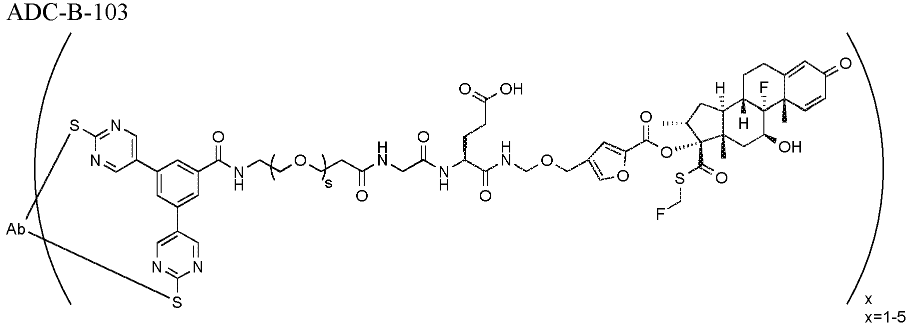 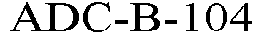 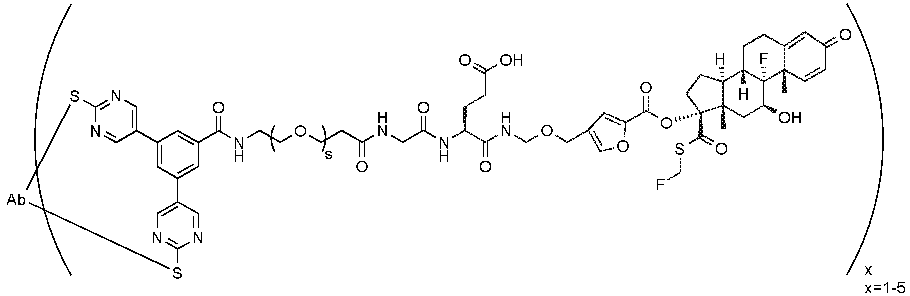 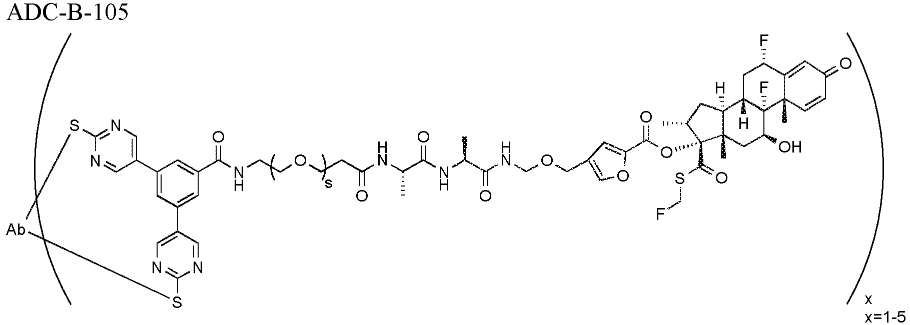 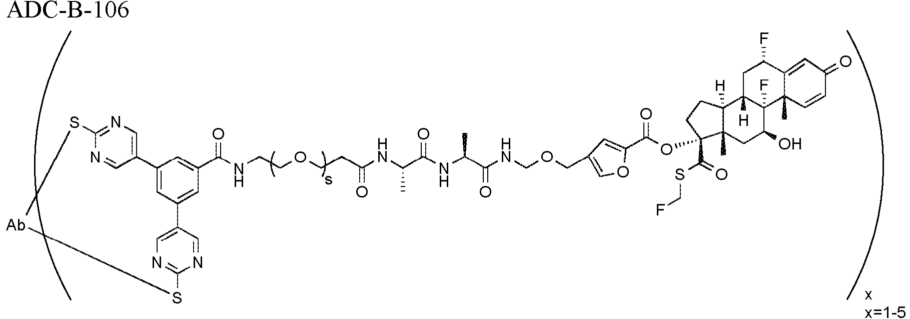 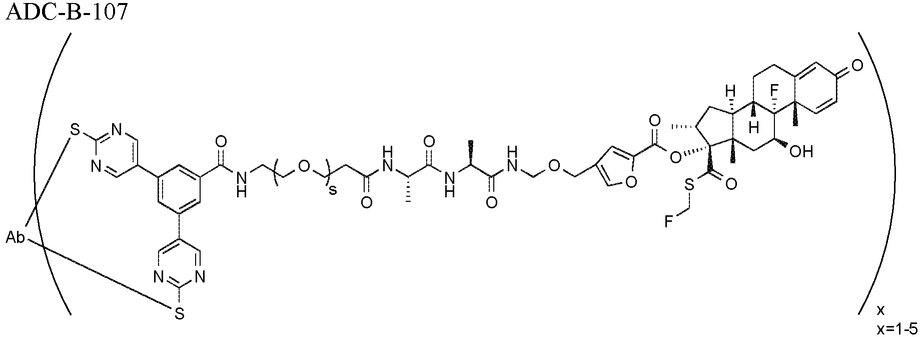 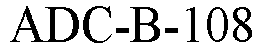 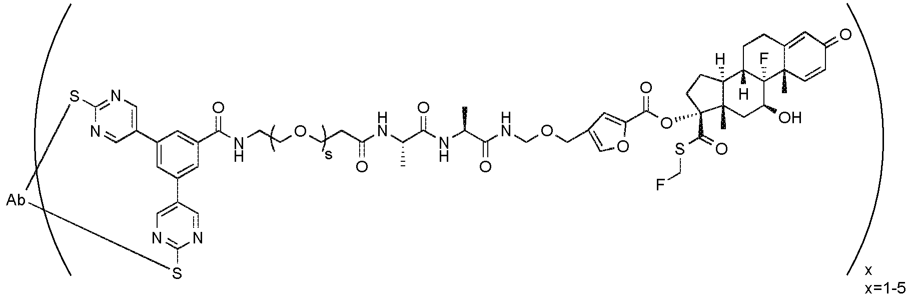 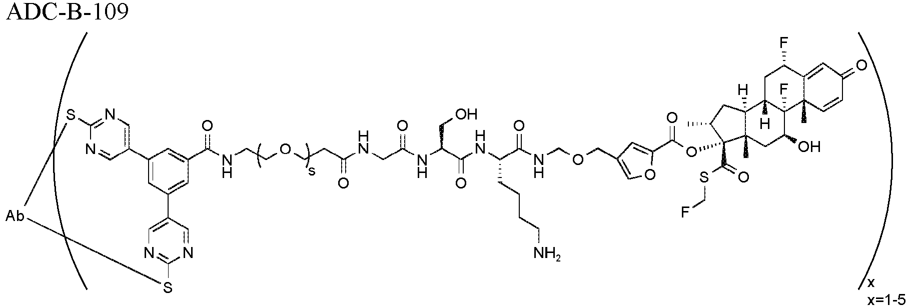 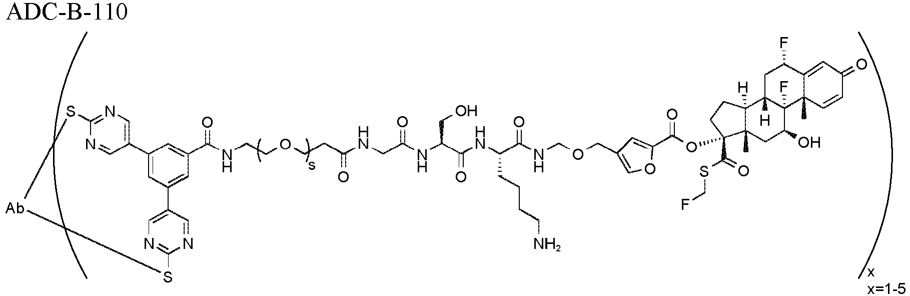 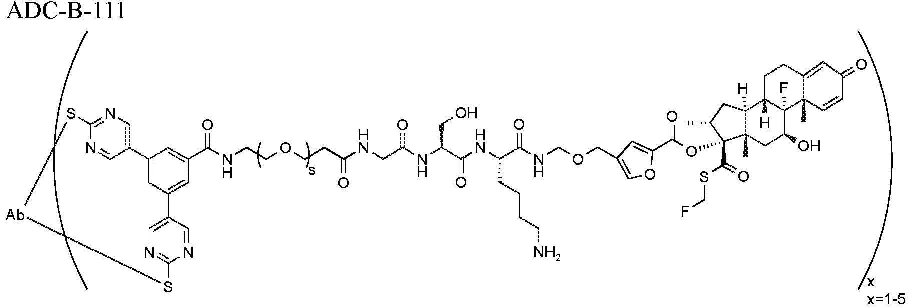 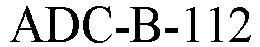 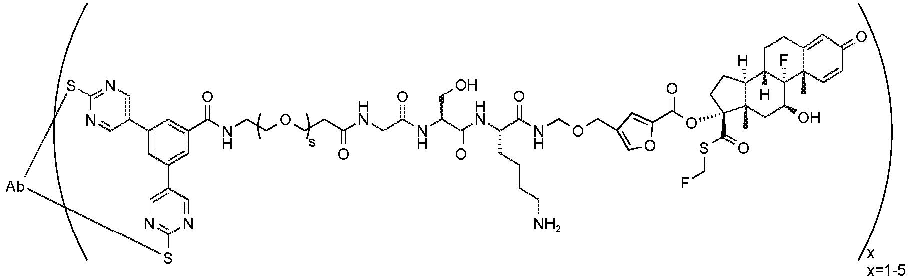 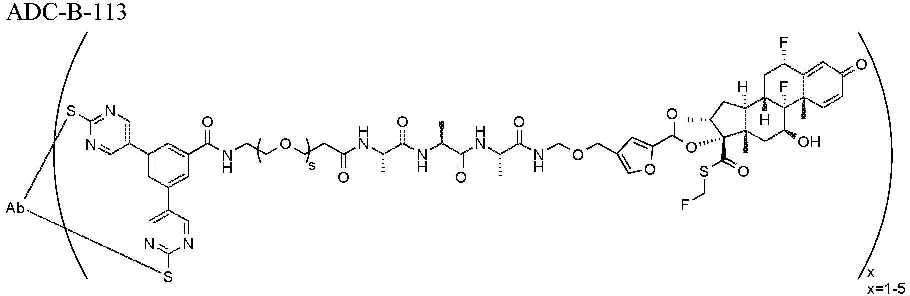 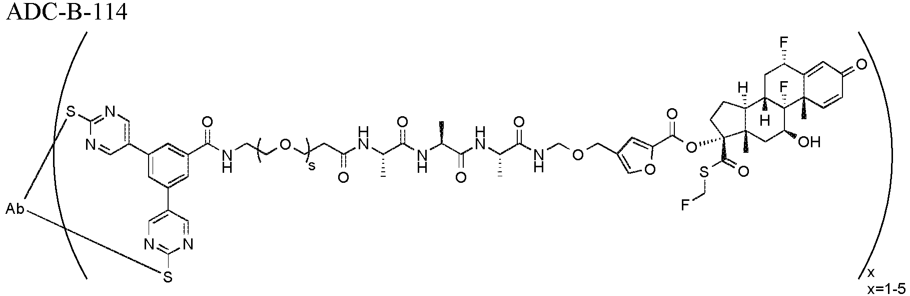 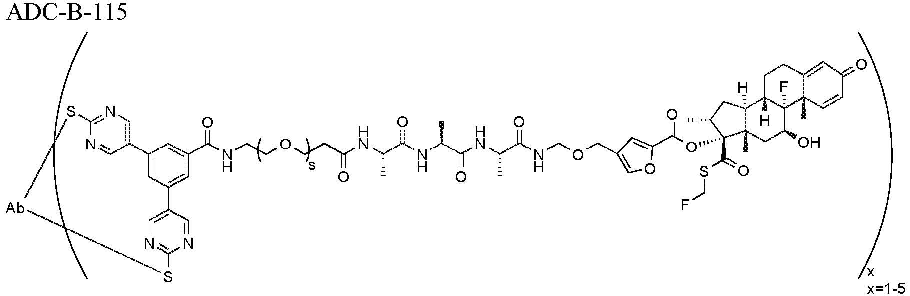 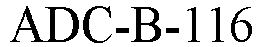 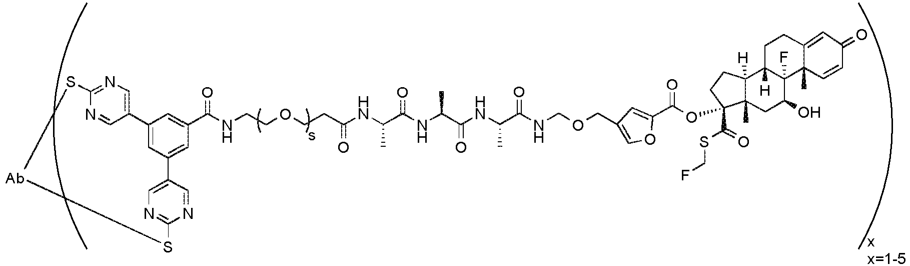 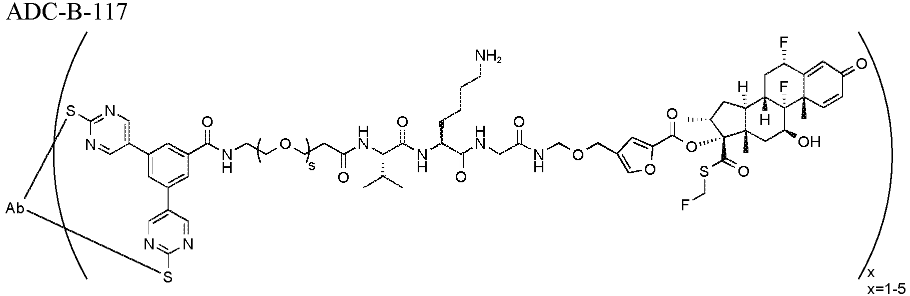 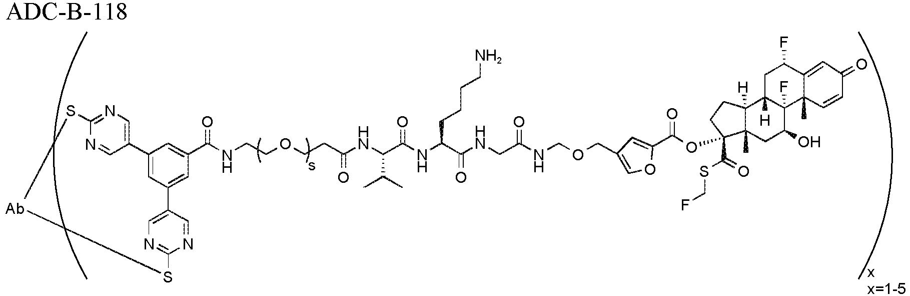 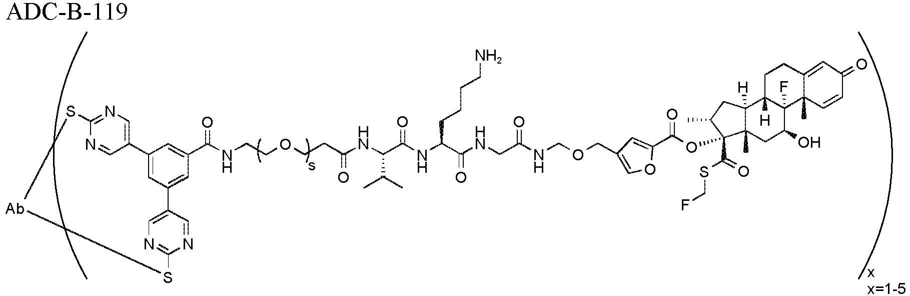 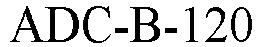 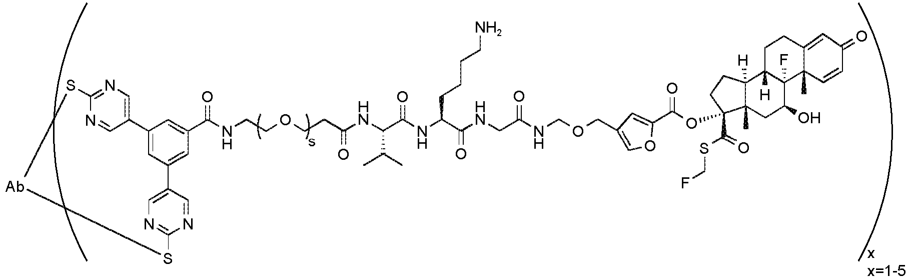 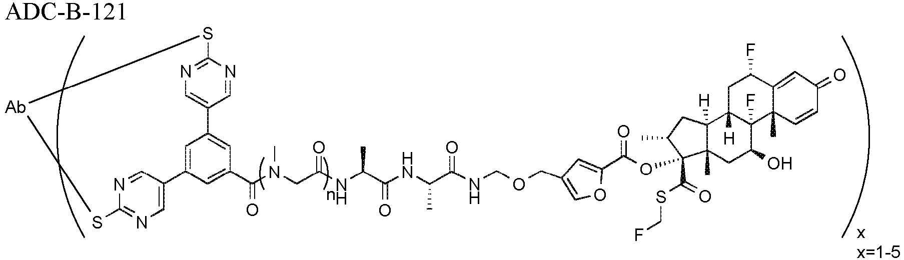 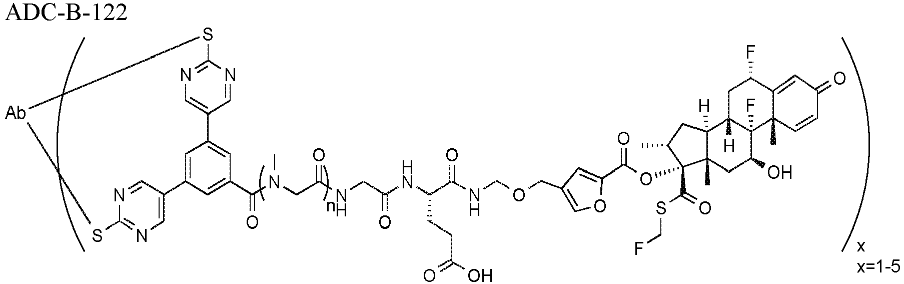 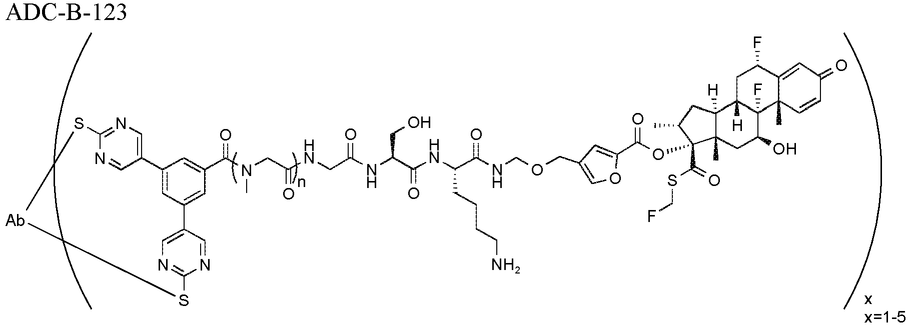 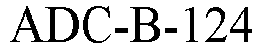 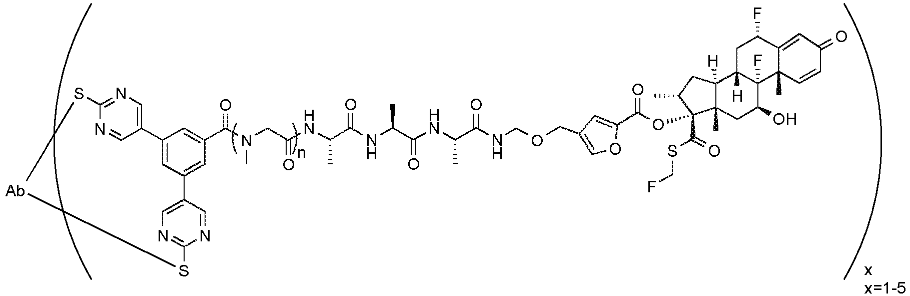 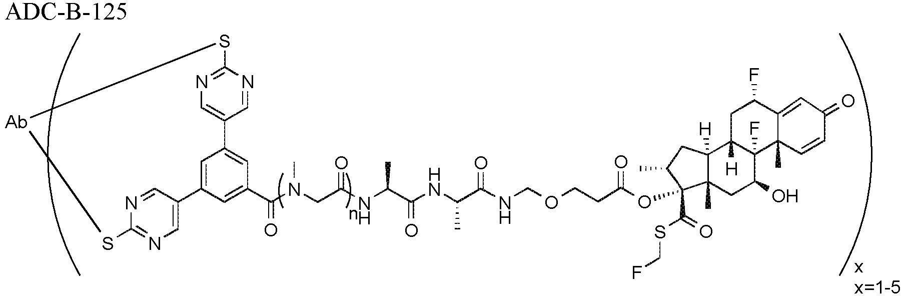 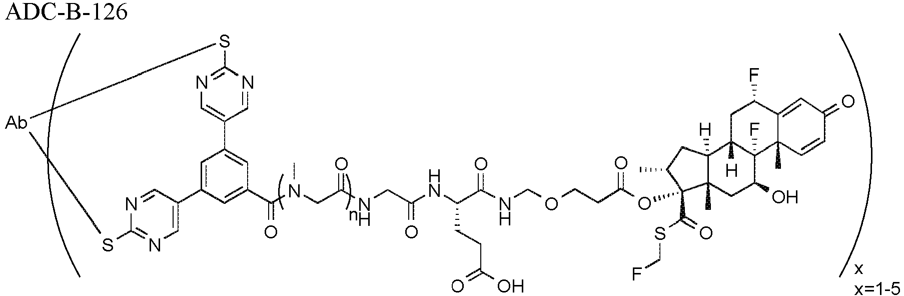 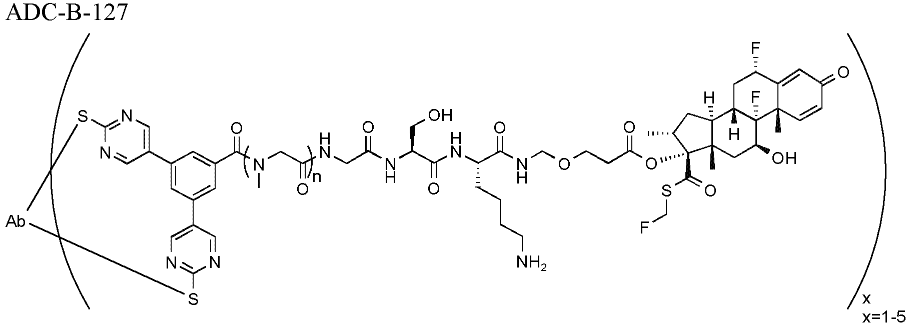 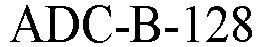 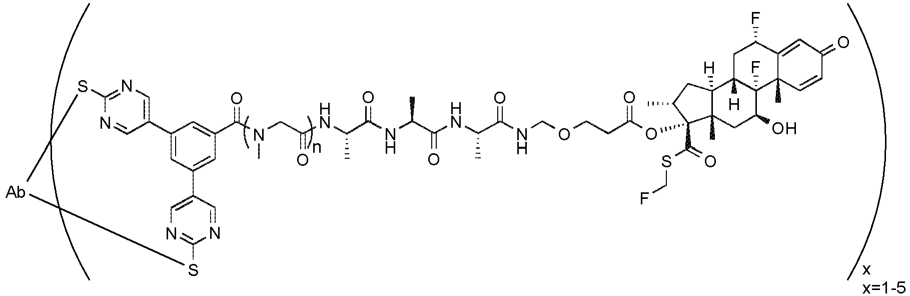 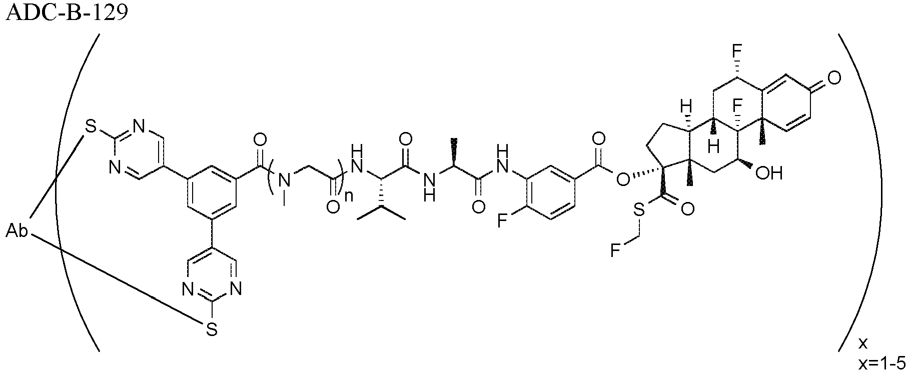 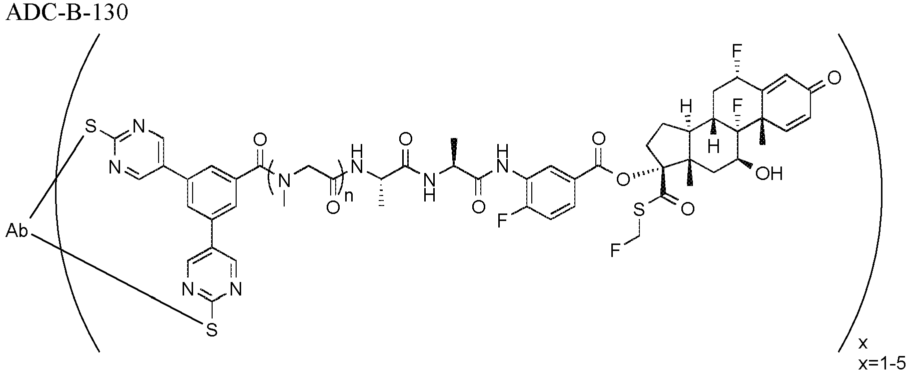 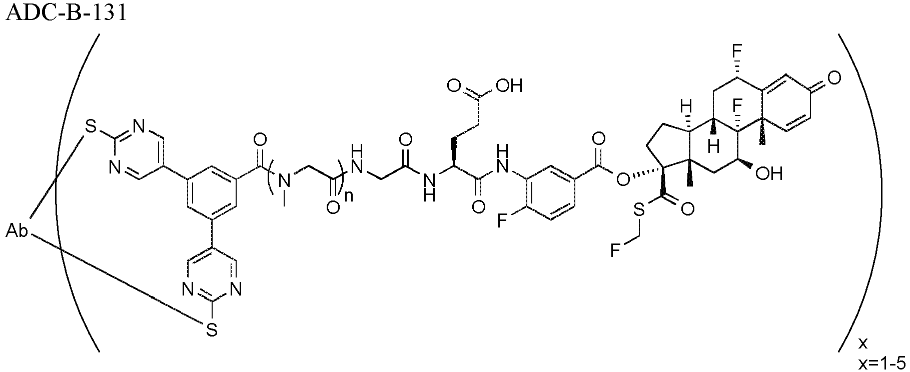 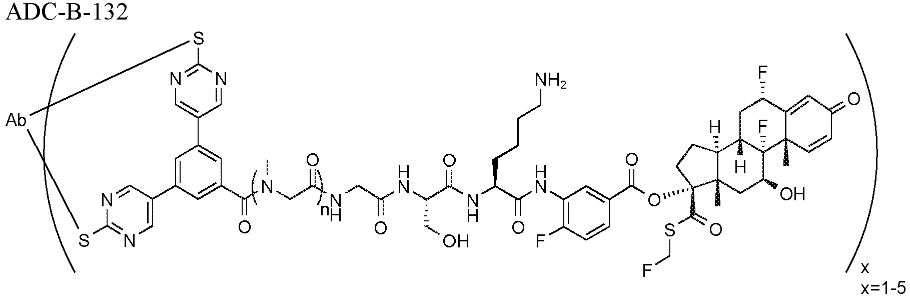 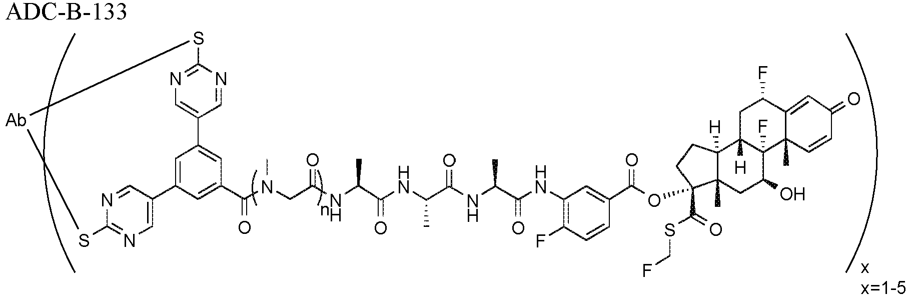 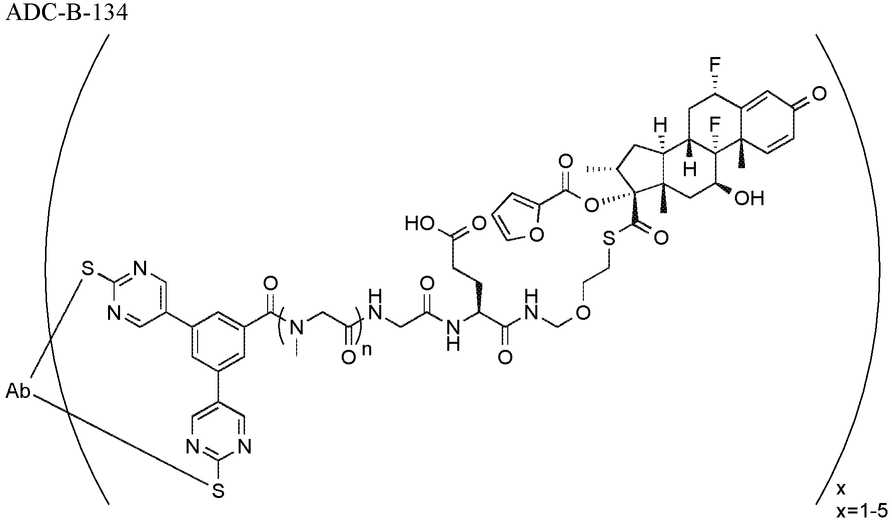 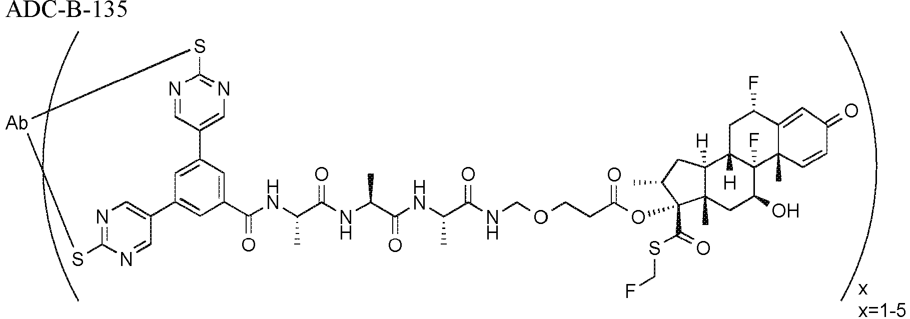 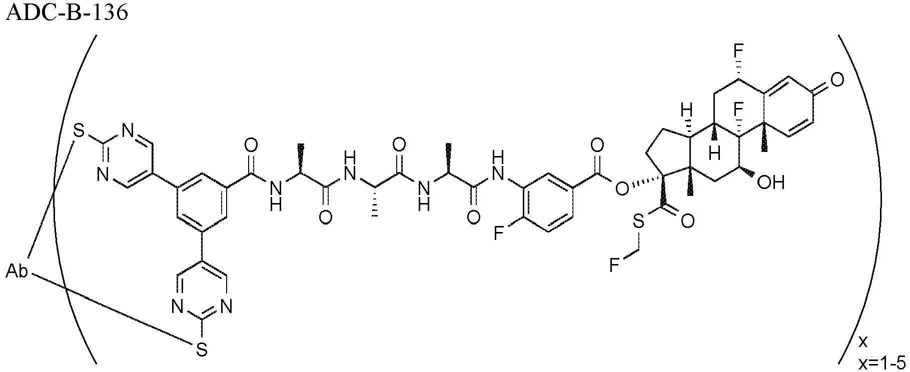 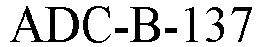 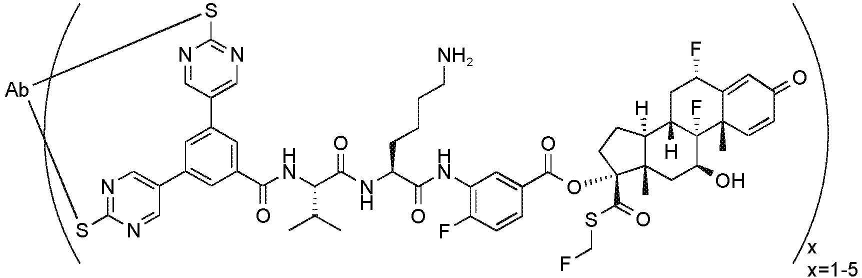 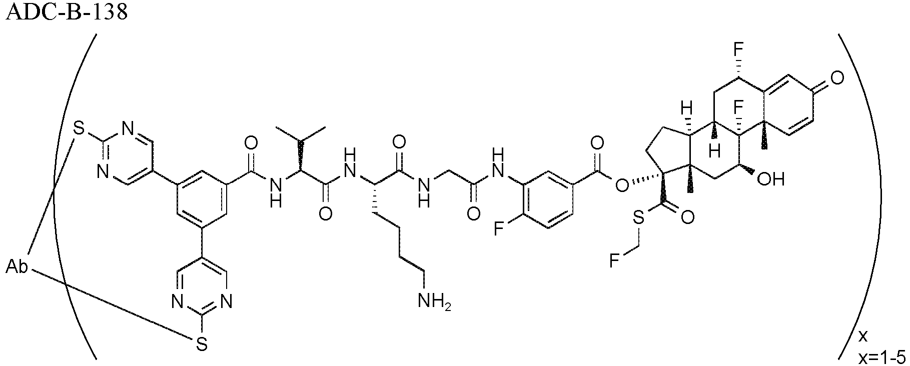 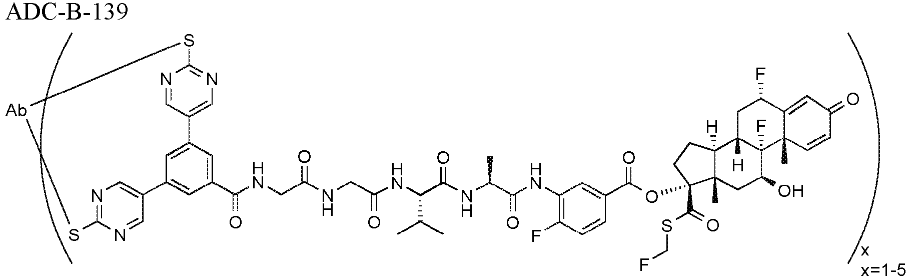 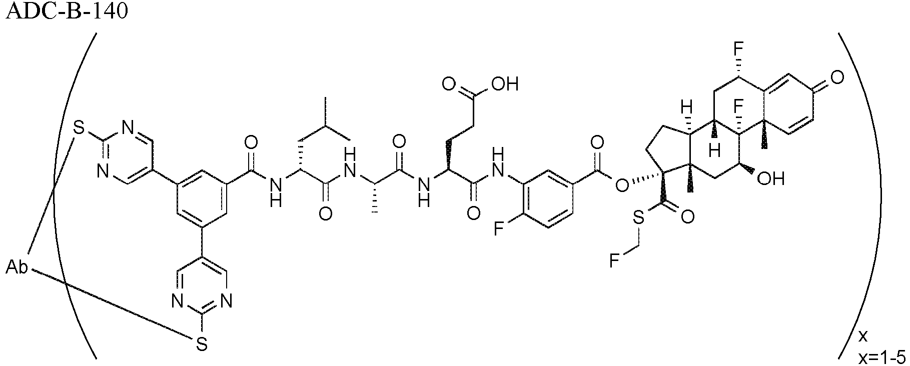 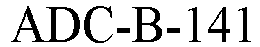 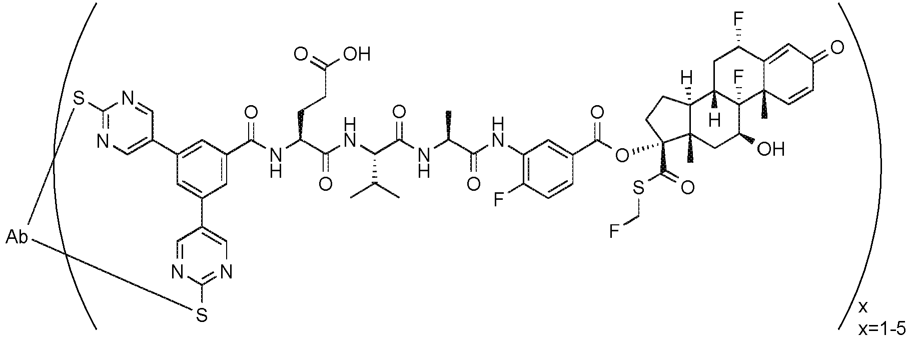 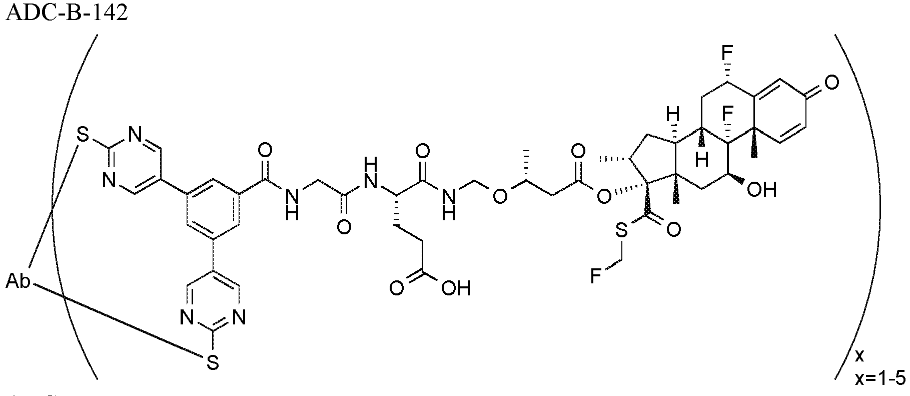 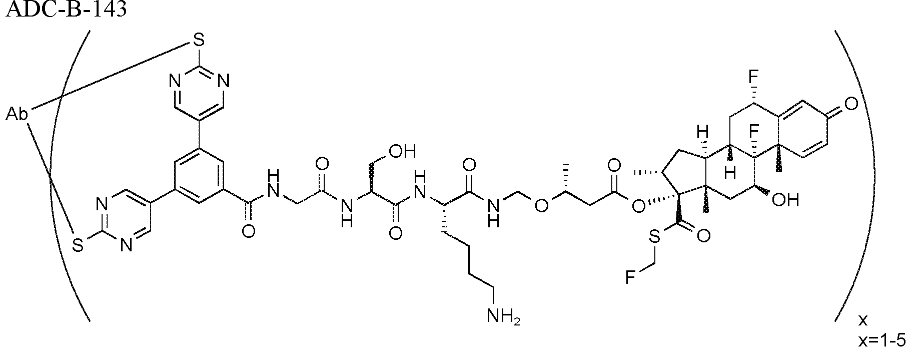 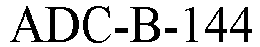 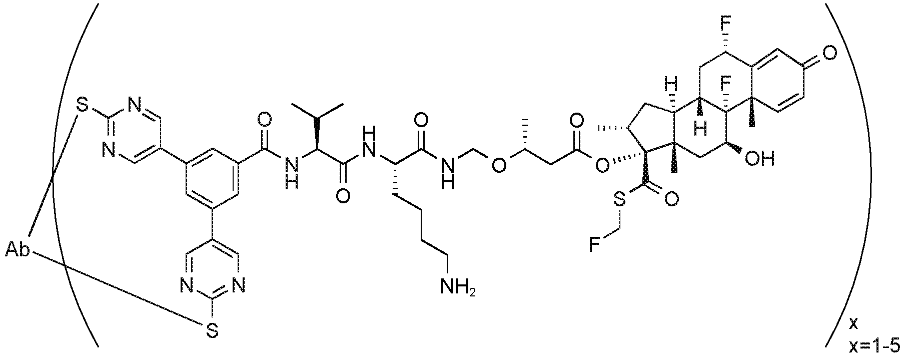 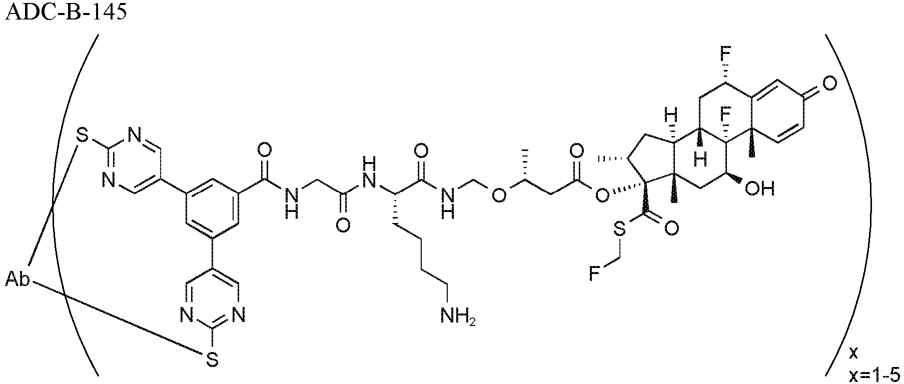 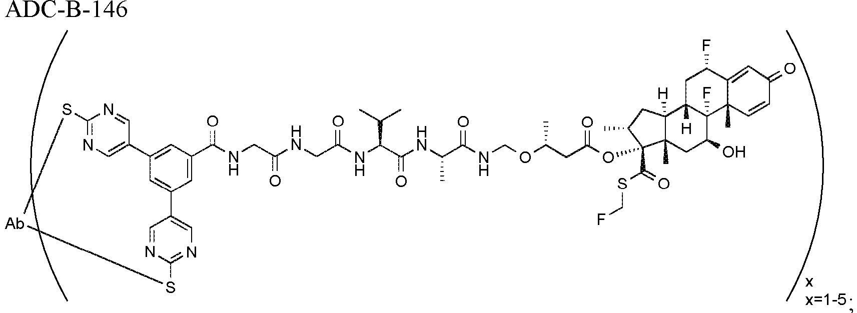 wherein,
s is an integer selected from 1 to 20; and
n is an integer selected from 0 to 20.

18. The antibody-drug conjugate according to any one of claims 9-17, having a drug-to-antibody conjugating ratio of 1-10, for example 1-2, 1-3, 1-4, 1-5, 1-6, 1-7, 1-8, 1-9, 1-10, 2-3, 2-4, 2-5, 2-6, 2-7, 2-8, 2-9, 2-10, 3-4, 3-5, 3-6, 3-7, 3-8, 3-9, 3-10, 4-5, 4-6, 4-7, 4-8, 4-9, 4-10, 5-6, 5-7, 5-8, 5-9, 5-10, 6-7, 6-8, 6-9, 6-10, 7-8, 7-9, 7-10, 8-9, 8-10, or 9-10, preferably 3-9, for example 3.0-3.5, 3.0-4.0, 3.0-4.5, 3.0-5.0, 3.0-5.5, 3.0-6.0, 3.5-4.0, 3.5-4.5, 3.5-5.0, 3.5-5.5, 3.5-6.0, 3.5-6.5, 3.5-7.0, 3.5-7.5, 3.5-8.0, 4.0-4.5, 4.0-5.0, 4.0-5.5, 4.0-6.0, 4.0-6.5, 4.0-7.0, 4.0-7.5, 4.0-8.0, 4.5-5.0, 4.5-5.5, 4.5-6.0, 4.5-6.5, 4.5-7.0, 4.5-7.5, 4.5-8.0, 5.0-5.5, 5.0-6.0, 5.0-6.5, 5.0-7.0, 5.0-7.5, 5.0-8.0, 5.5-6.0, 5.5-6.5, 5.5-7.0, 5.5-7.5, 5.5-8.0, 6.0-6.5, 6.0-7.0, 6.0-7.5, 6.0-8.5, 6.5-7.0, 6.5-7.5, 6.5-8.5, 7.0-7.5, 7.0-9.0, or 7.5-9.0.

19. A drug-linker compound having a structure represented by formula G-M-[L-E-D]ₓ, wherein:
G is a functional group or a leaving group which reacts with a specific amino acid or glycosyl and a derivative thereof in an antibody or an antigen binding fragment; preferably, G is selected from a group consisting of halogen, halophenoxy, C₁₋₆ haloalkyl, sulfonate, C₁₋₆ alkylsulfonyl, C₁₋₆ haloalkylsulfonyl, halosulfonyl, C₁₋₆ alkyl sulfonate, C₁₋₆ haloalkyl sulfonate, C₁₋₆ alkyl sulfite, halosulfonate, C₁₋₆ alkyl sulfoxide, methylsulfonyl methacryloyl, dimethylsulfonyl methacryloyl, haloformyl, haloacetyl, formyl, acetyl, nitro, azido, cyano, cyanovinyl, N-methyl-vinylsulfonamido, tetrazinyl, methyl tetrazinyl, trans-cyclooctenyl carbonate, C₂₋₆ alkenyl, C₂₋₆ alkynyl, benzazacyclooctynyl, and (1R,8S,9S)-bicyclo[6.1.0]non-4-yn-9-ylmethoxy, preferably, G is selected from a group consistine of halogen, methylsulfonyl, haloacetyl, fluorophenoxy, 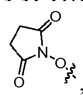 methylsulfonyl methacryloyl, cyanovinyl, N-methyl-vinylsulfonamido, azido, tetrazinyl, methyltetrazinyl, trans-cyclooctenyl carbonate, C₂₋₆ alkynyl, benzazacyclooctynyl, and (1R,8S,9S)-bicyclo[6.1.0]non-4-yn-9-ylmethoxy; and
M, L, E, D and x are as defined in any one of claims 9-18.

20. The drug-linker compound according to claim 19, which is selected from a group consisting of: 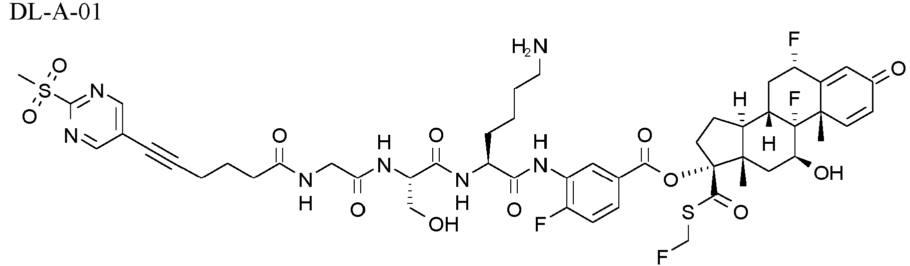 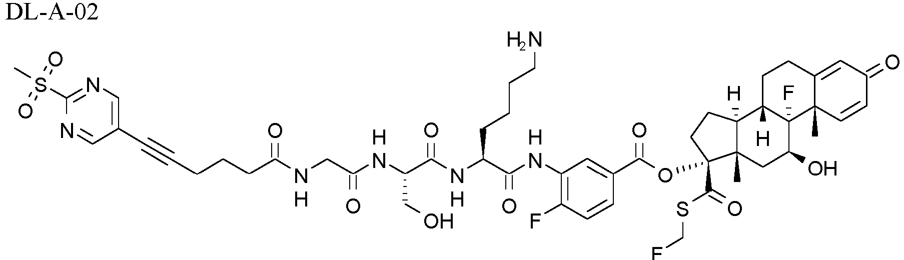 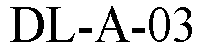 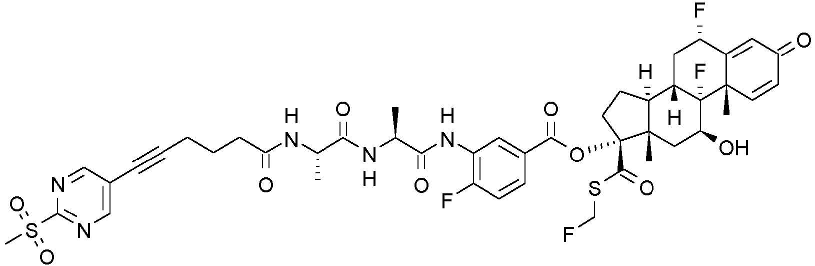 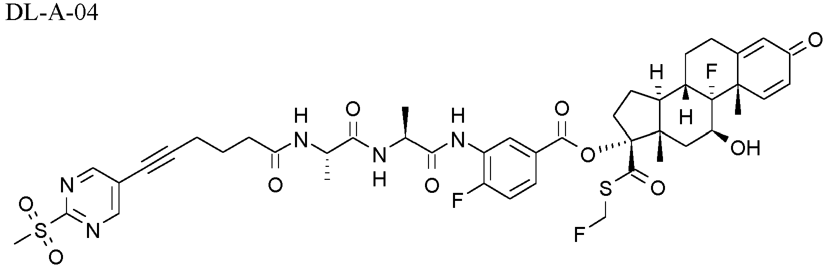 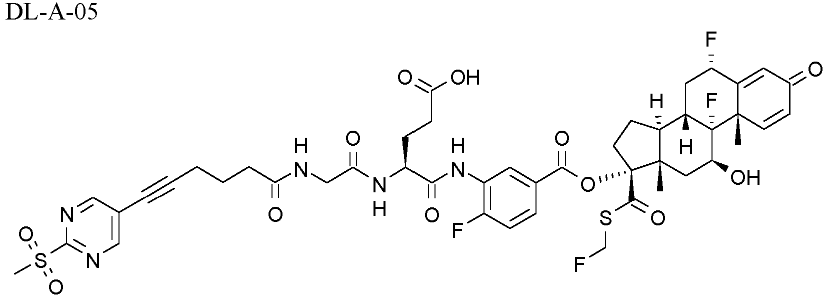 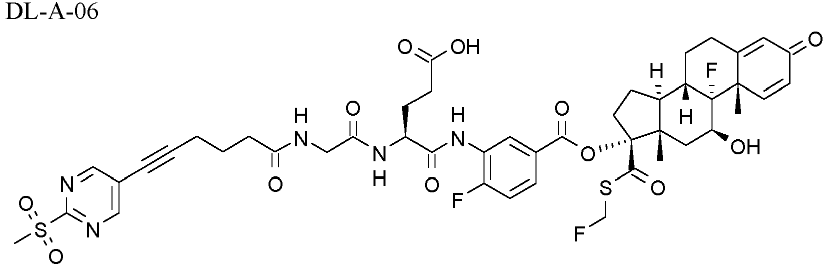 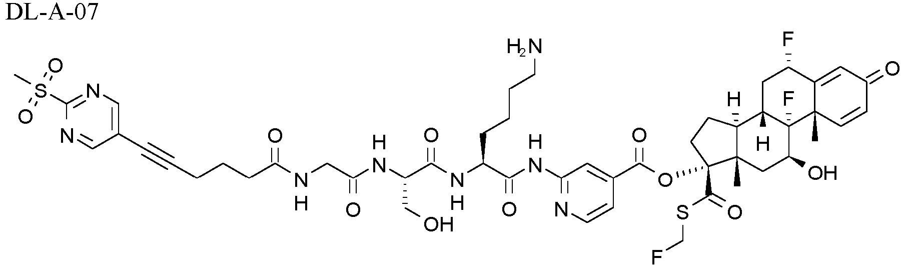 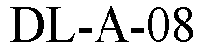 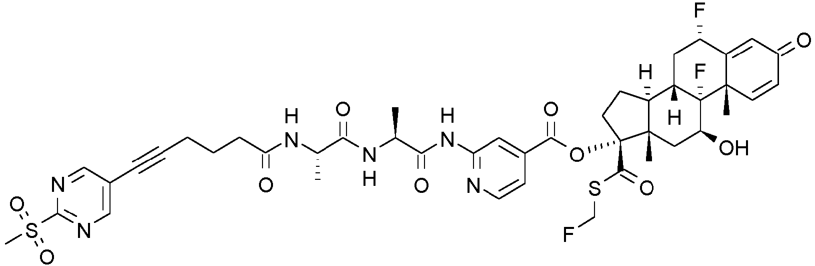 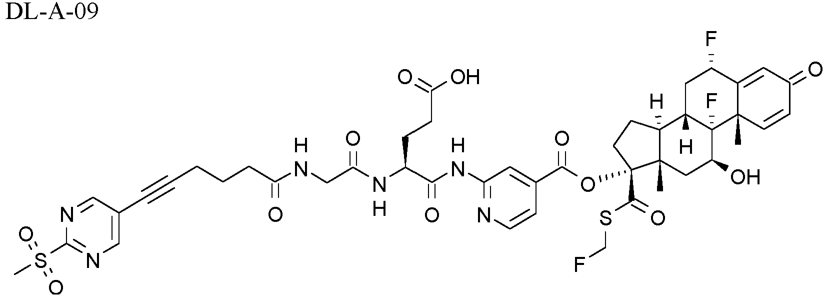 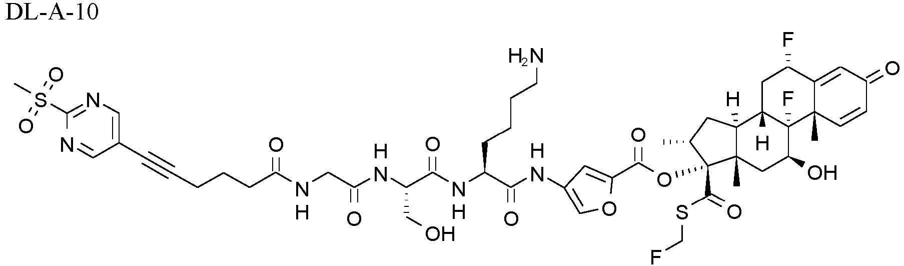 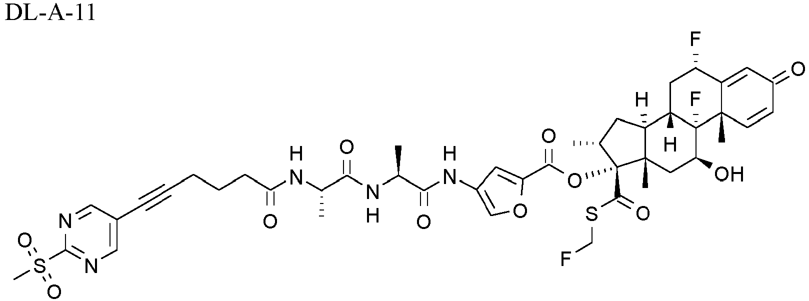 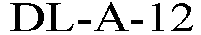 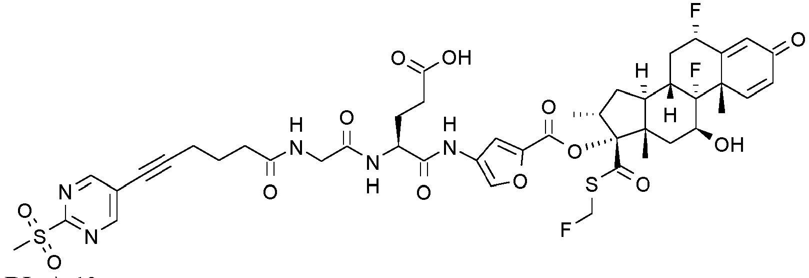 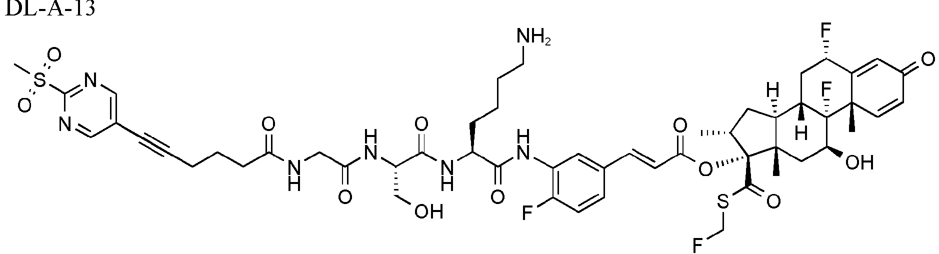 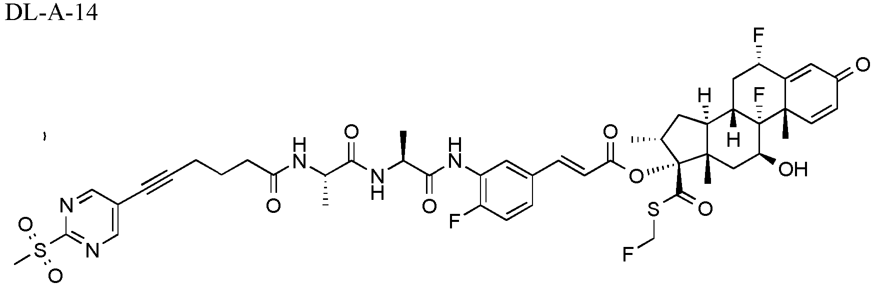 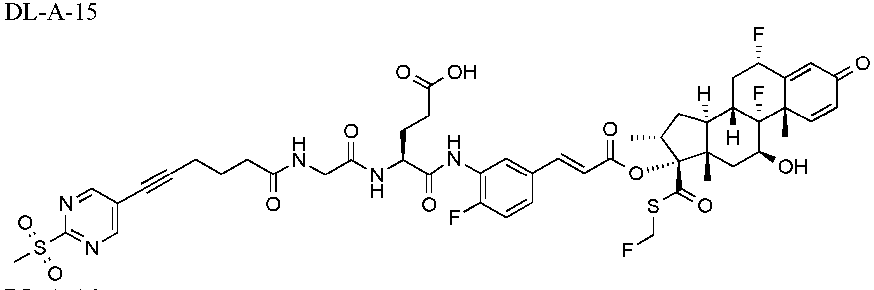 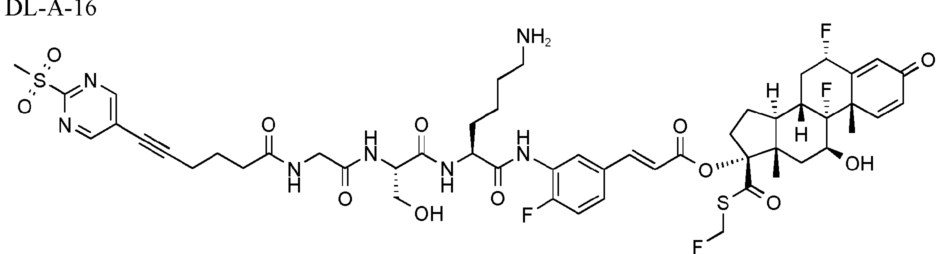 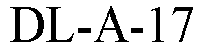 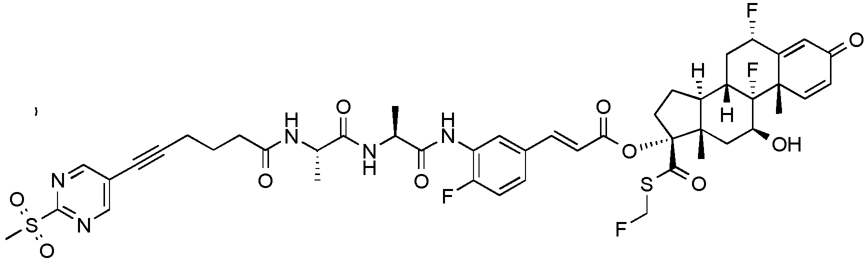 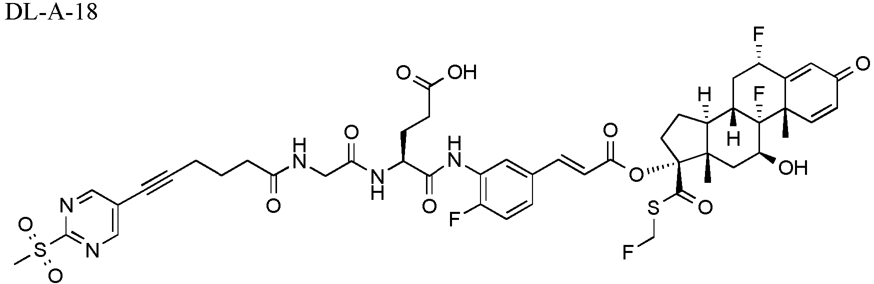 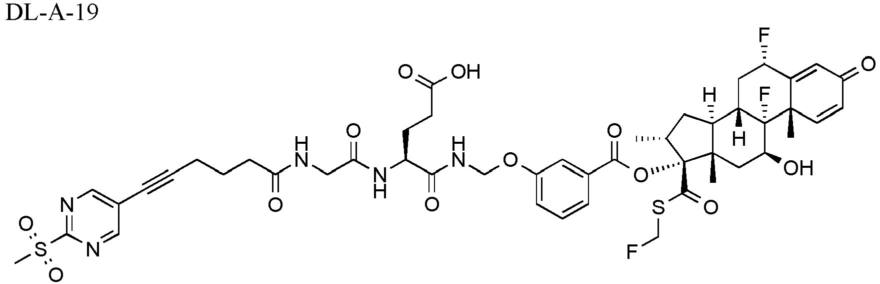 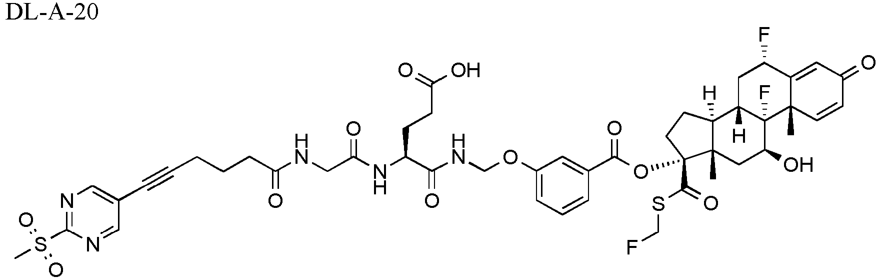 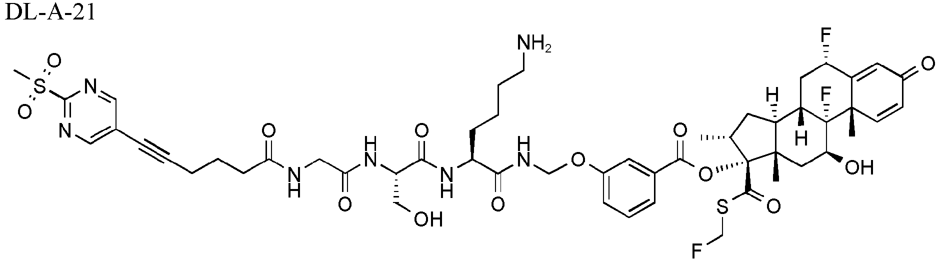 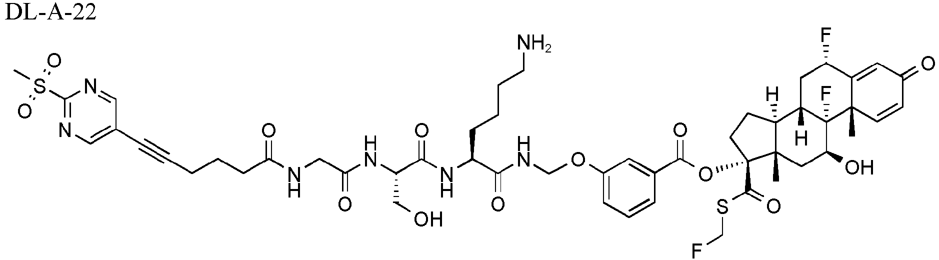 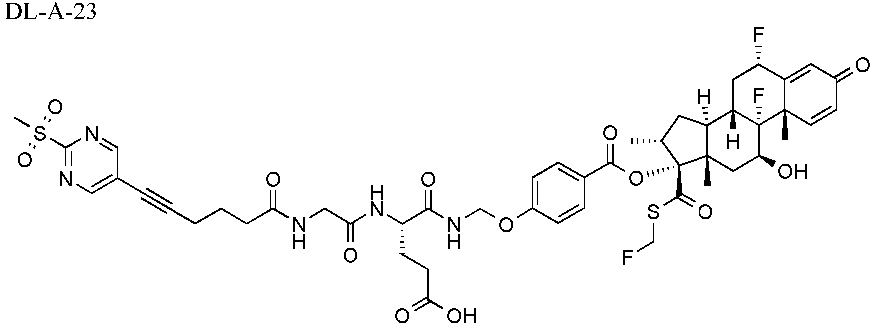 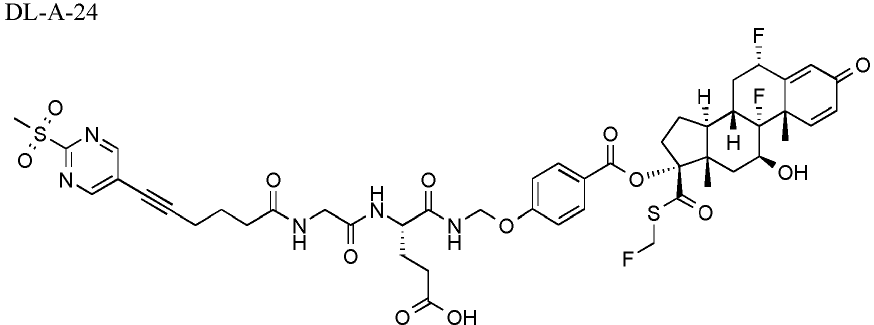 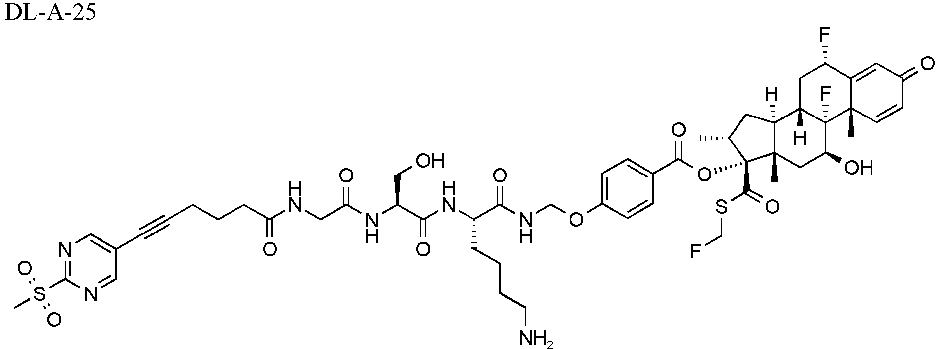 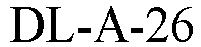 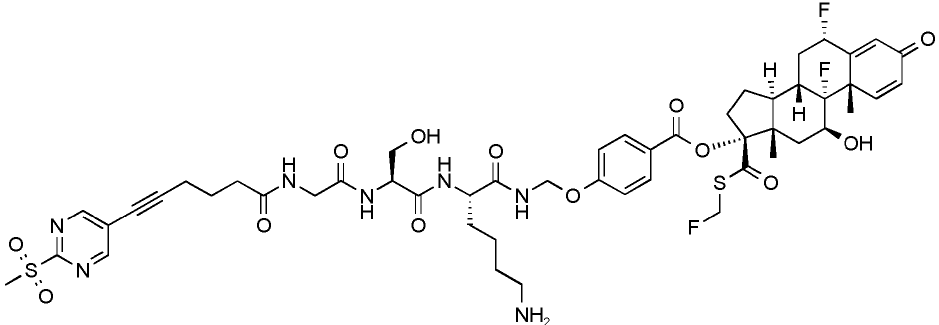 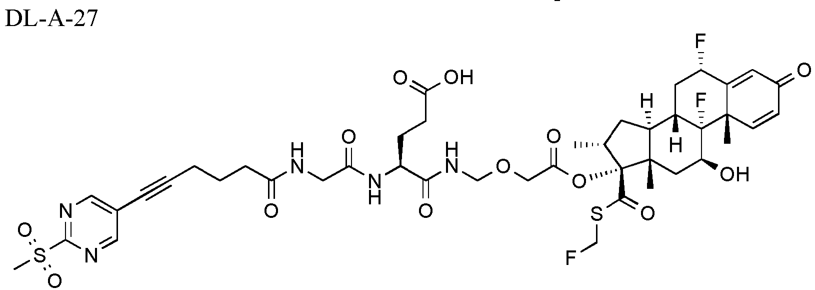 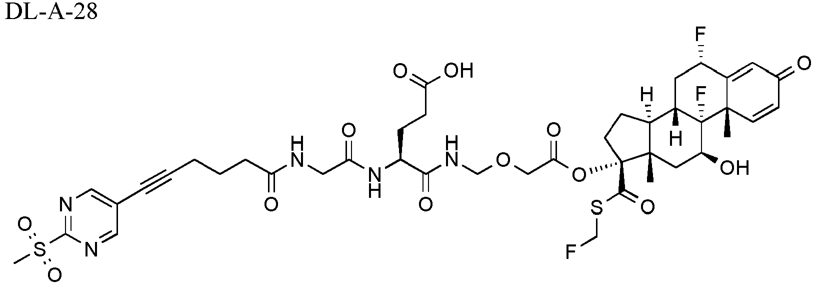 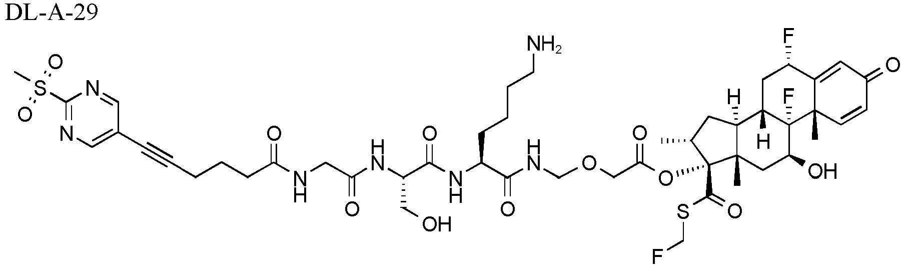 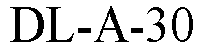 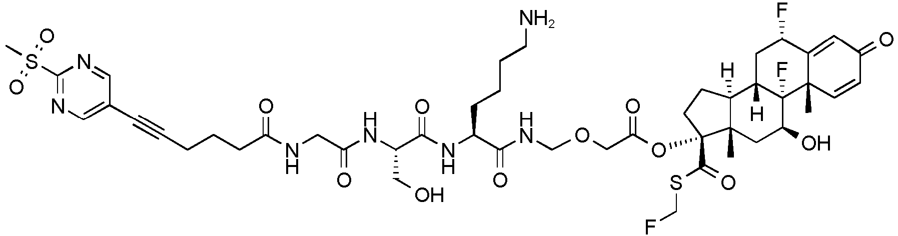 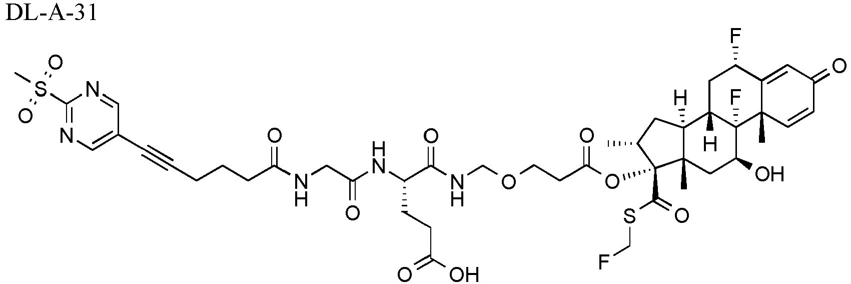 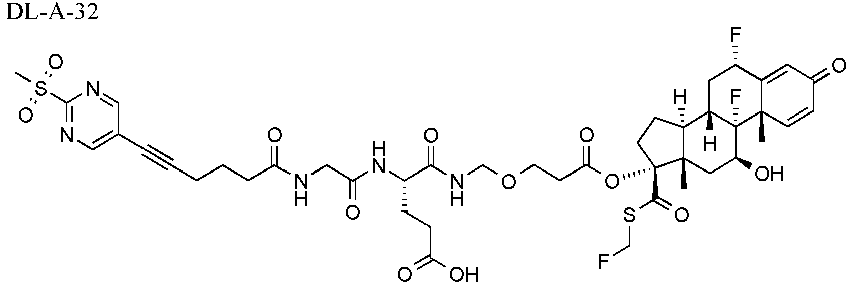 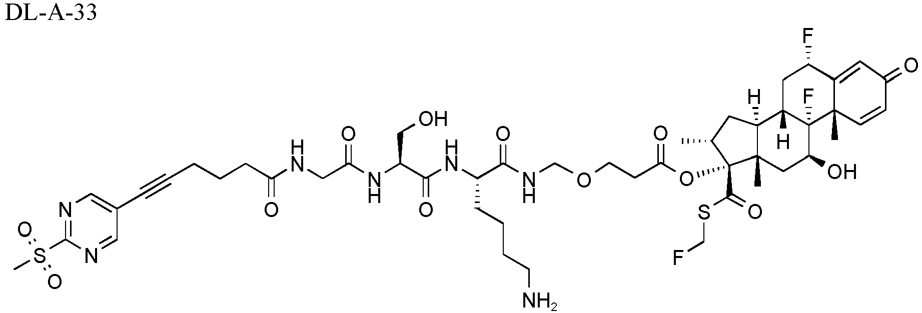 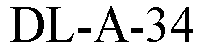 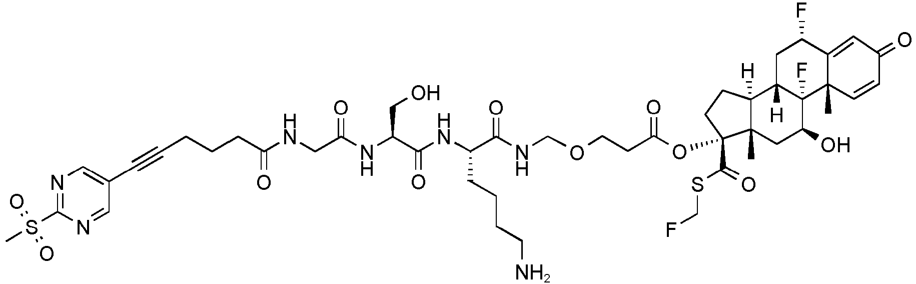 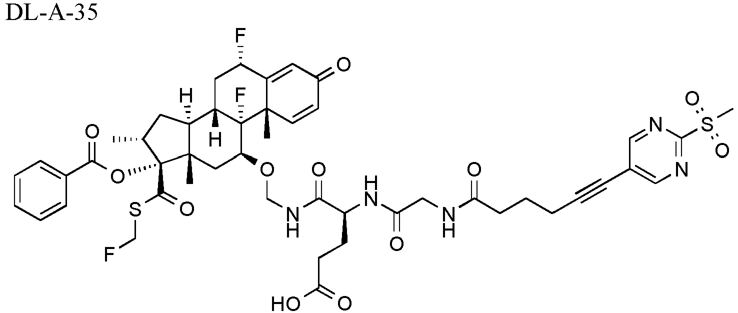 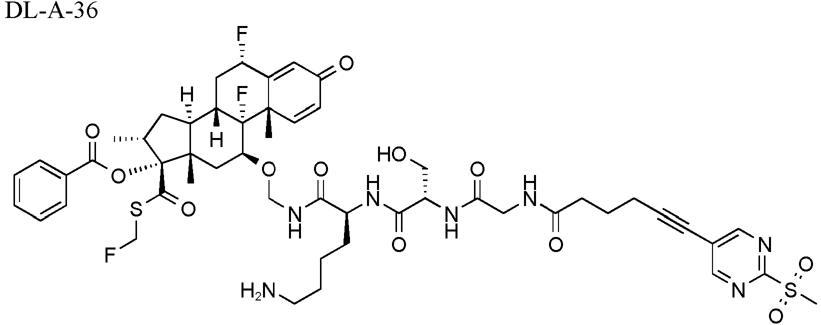 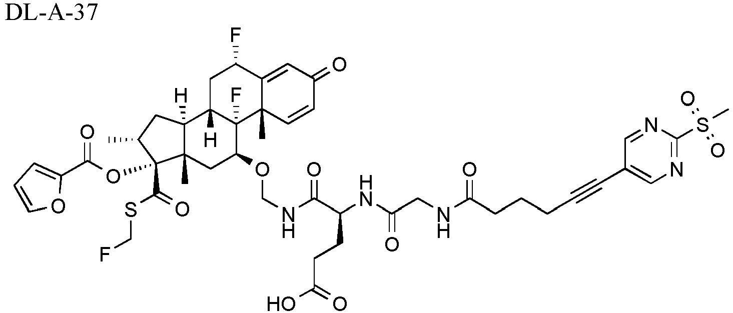 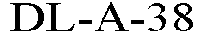 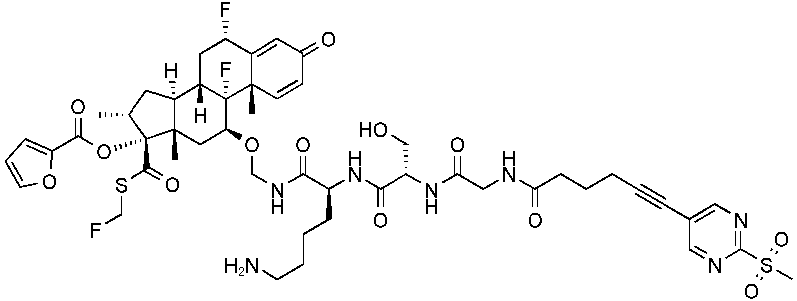 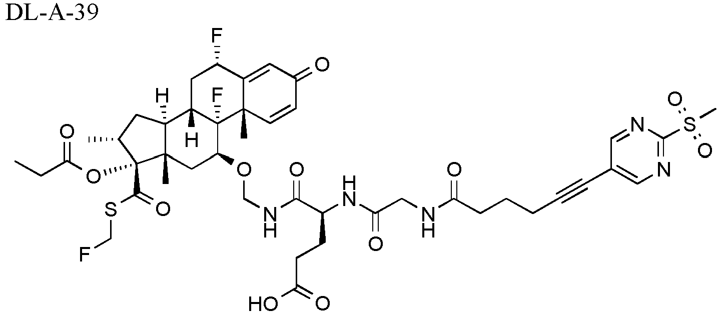 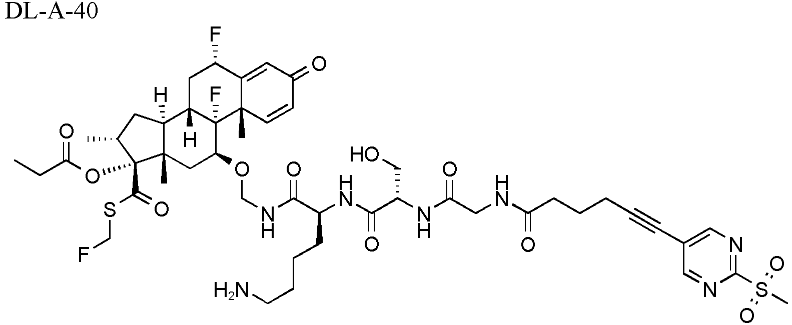 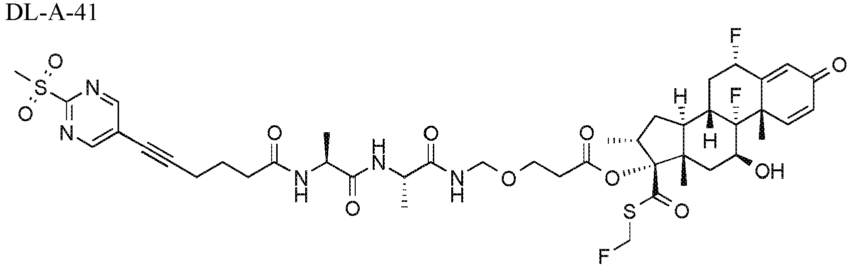 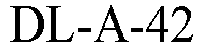 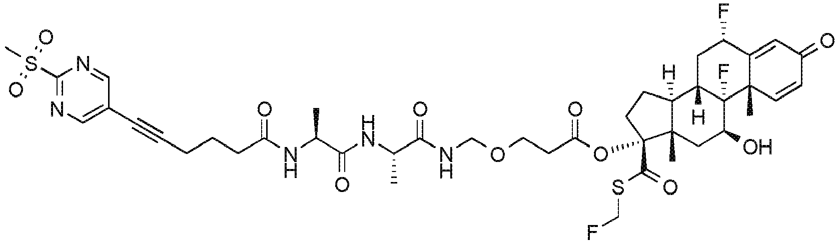 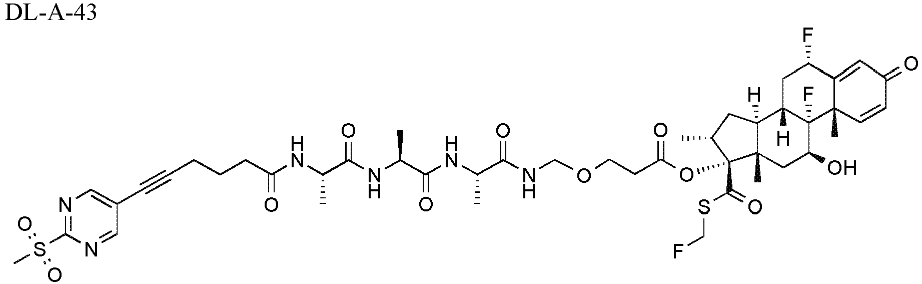 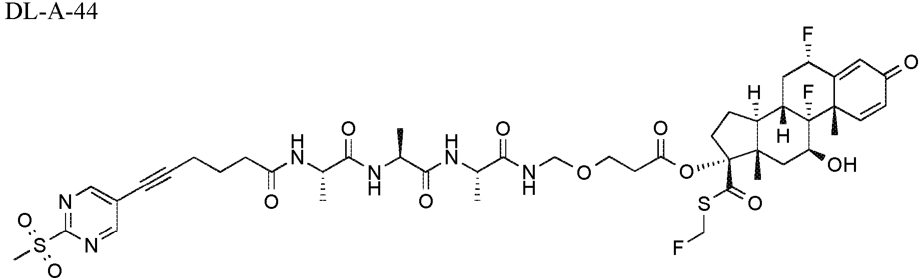 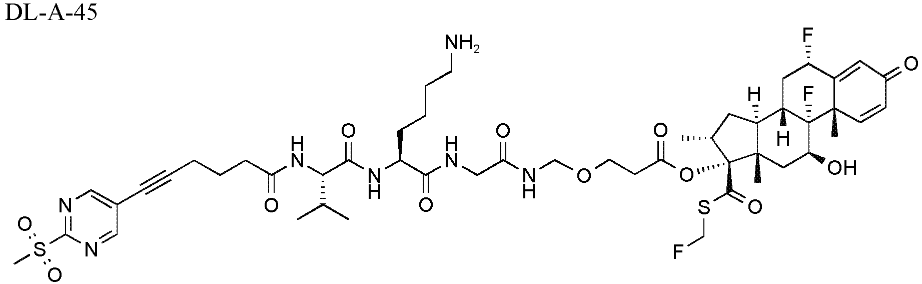 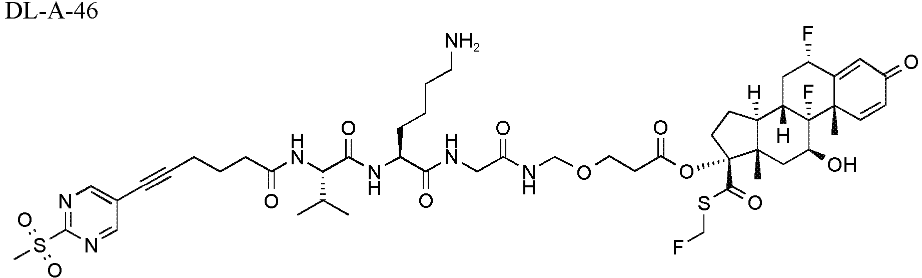 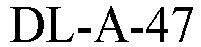 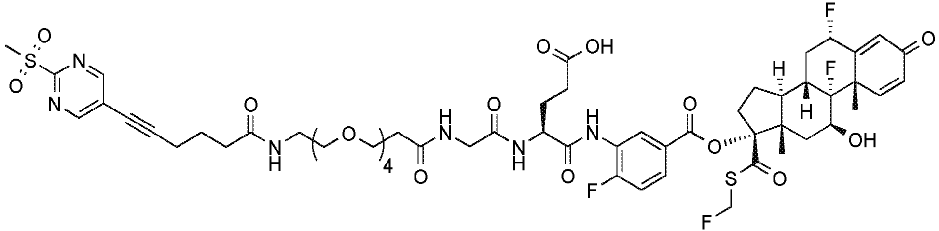 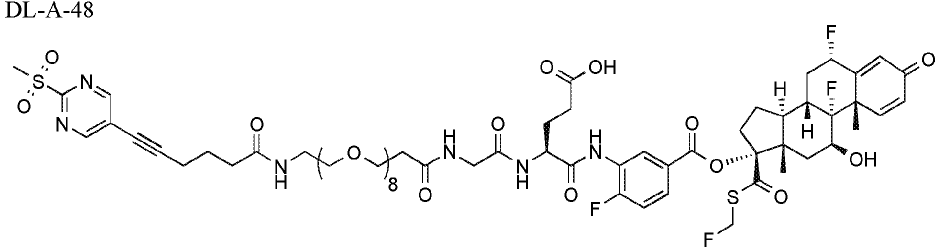 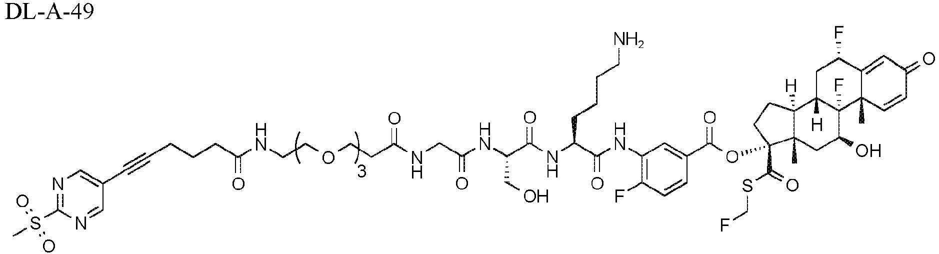 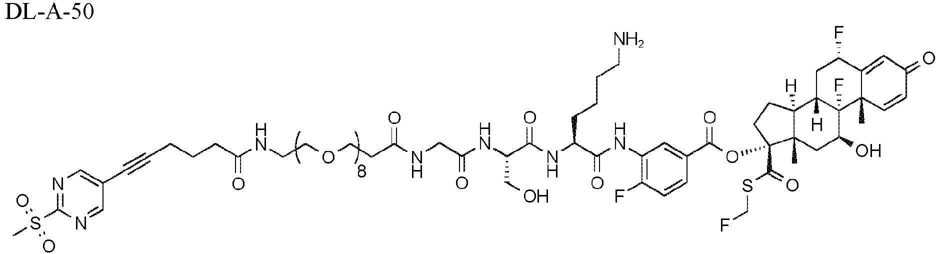 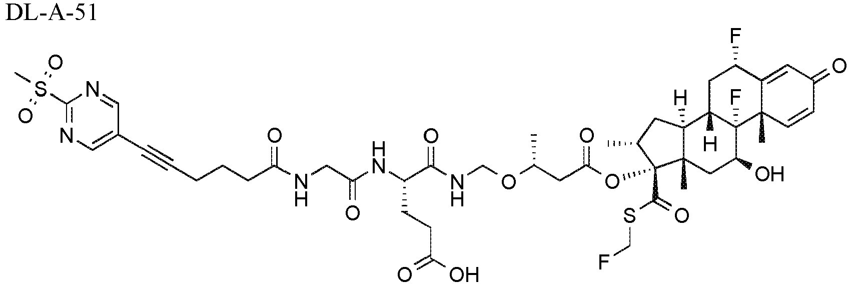 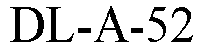 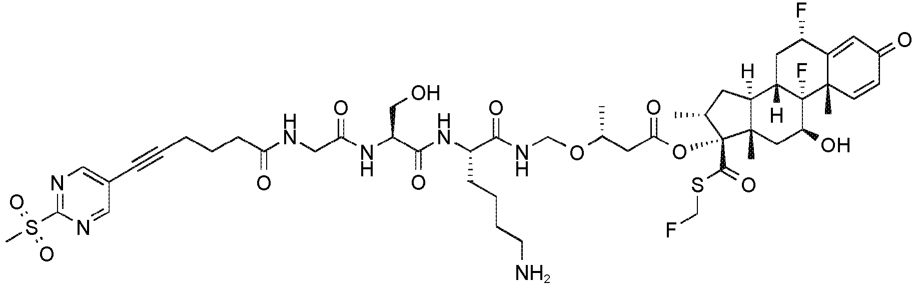 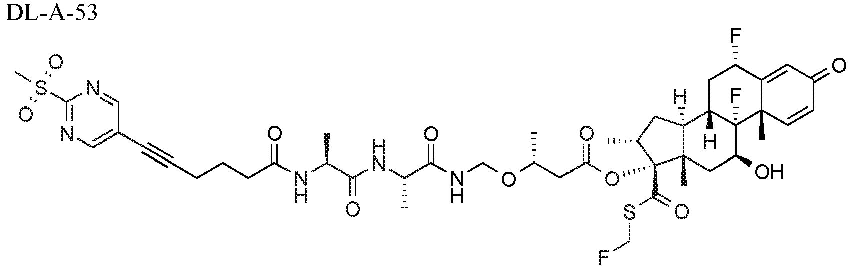 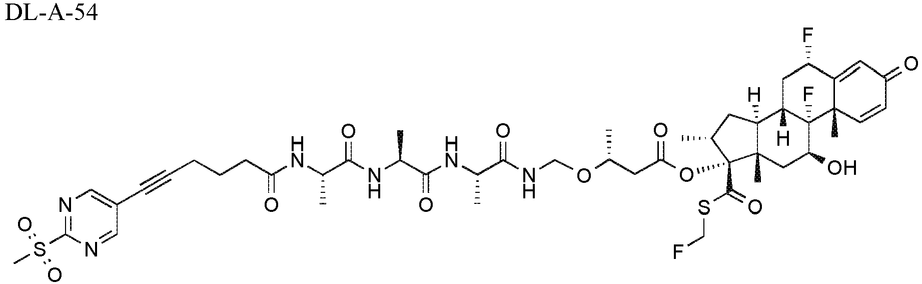 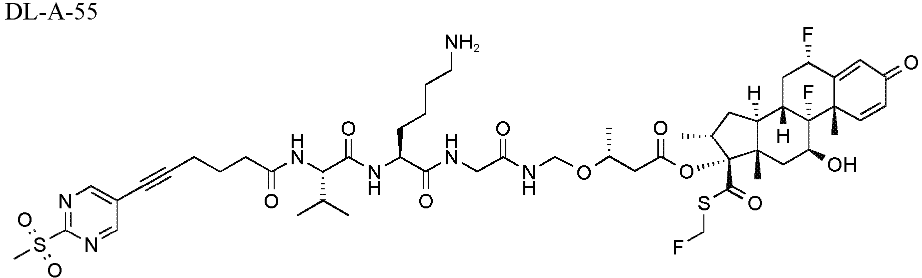 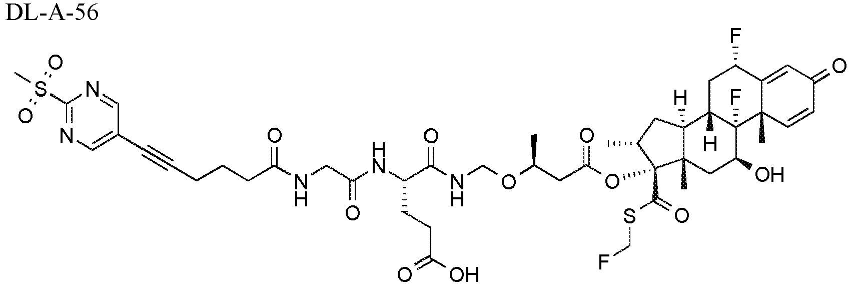 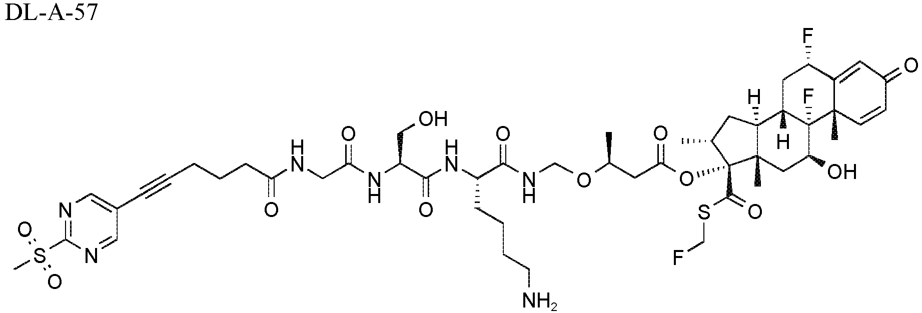 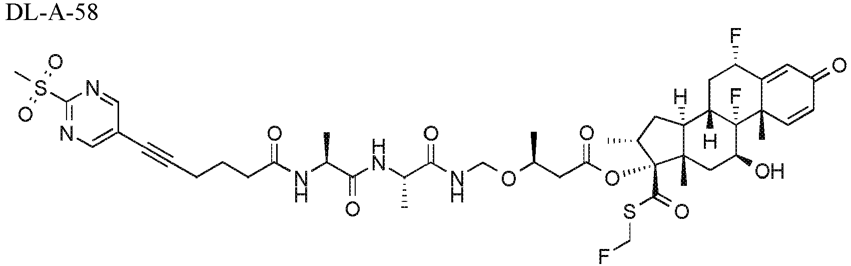 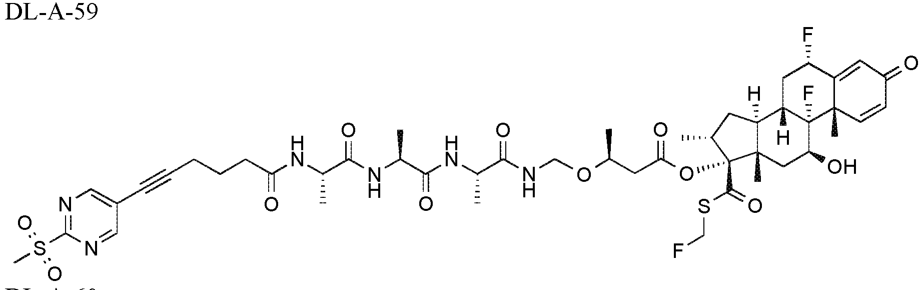 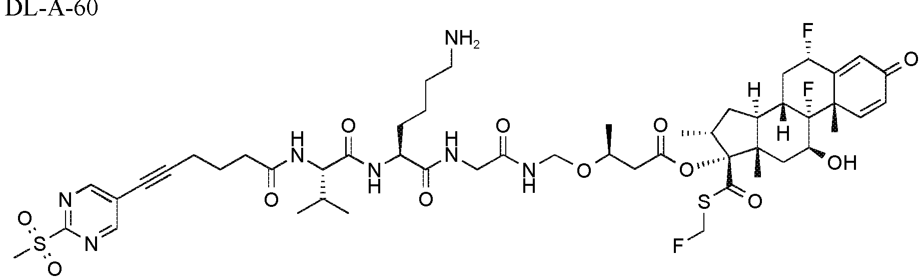 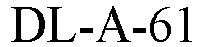 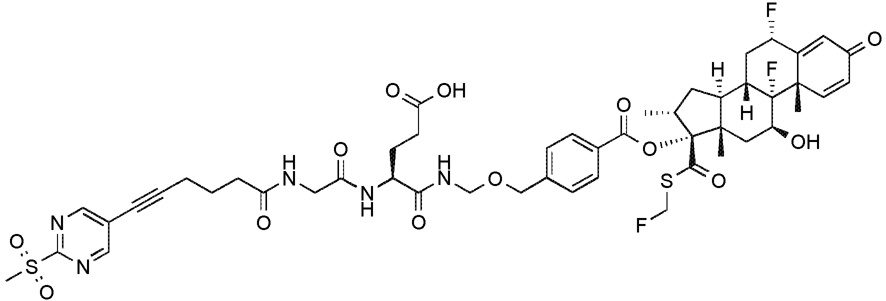 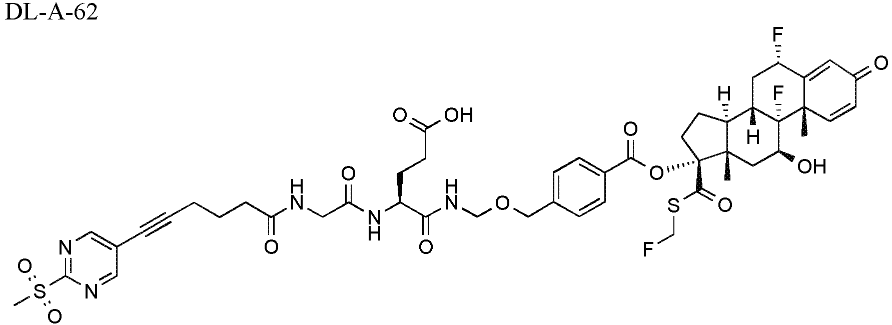 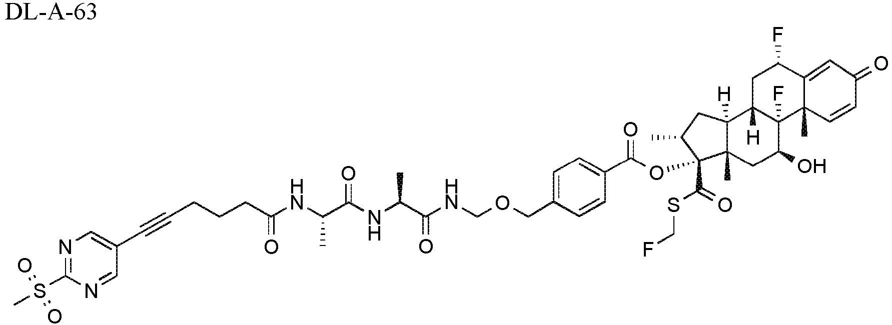 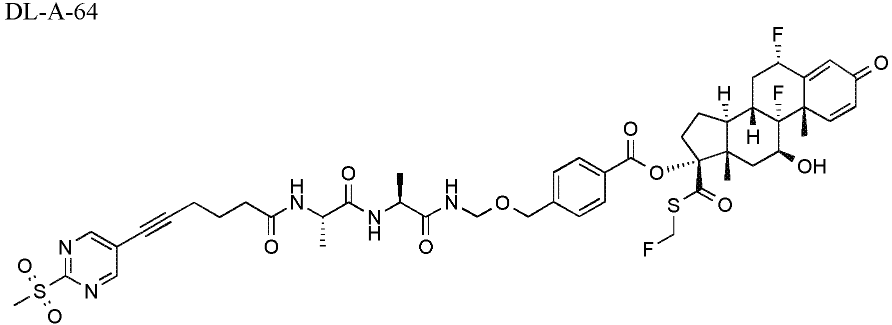 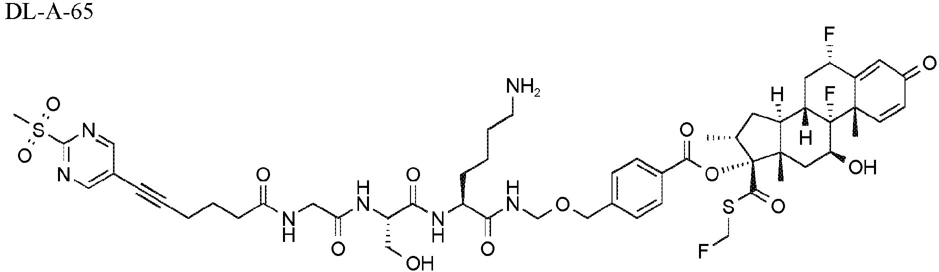 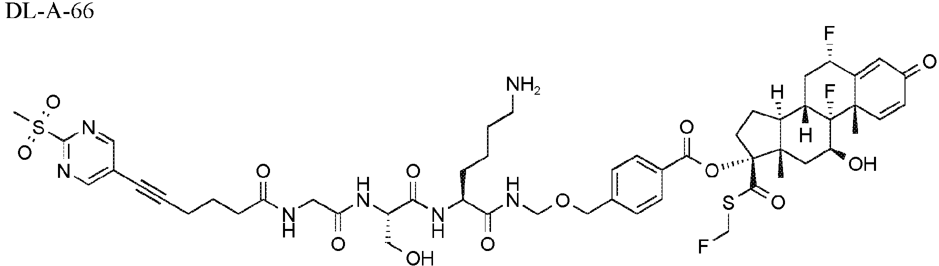 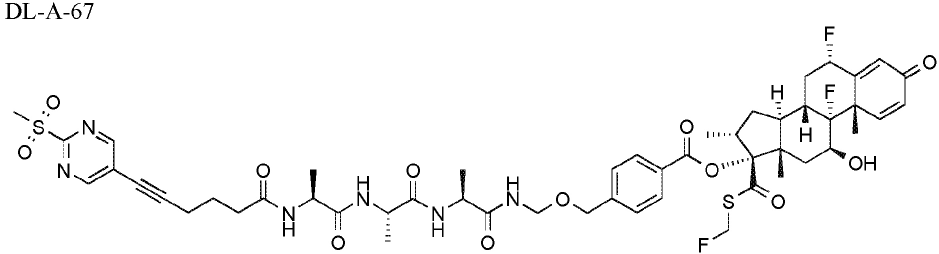 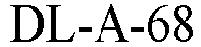 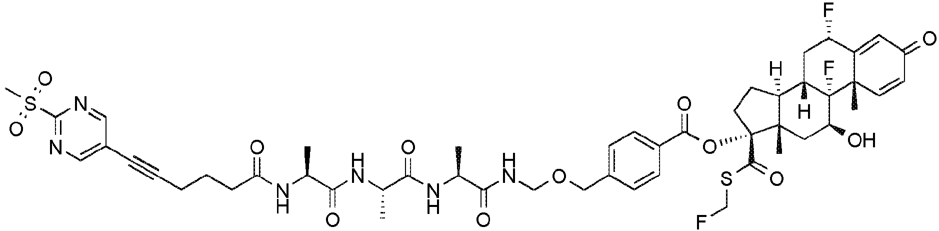 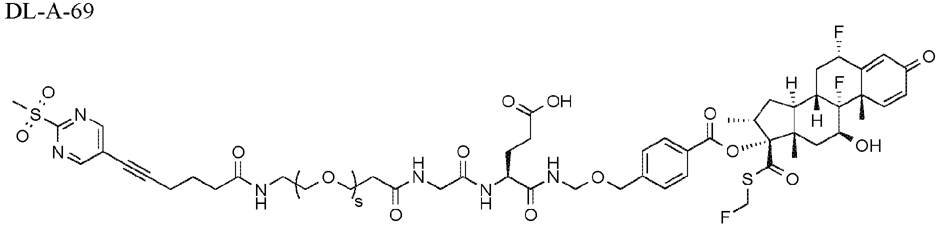 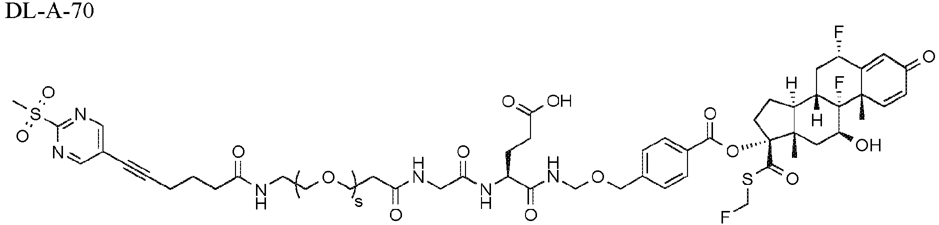 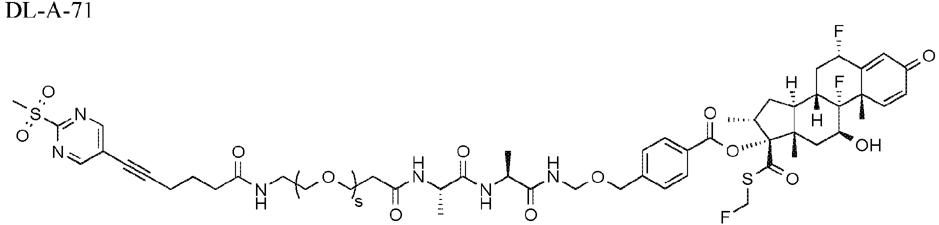 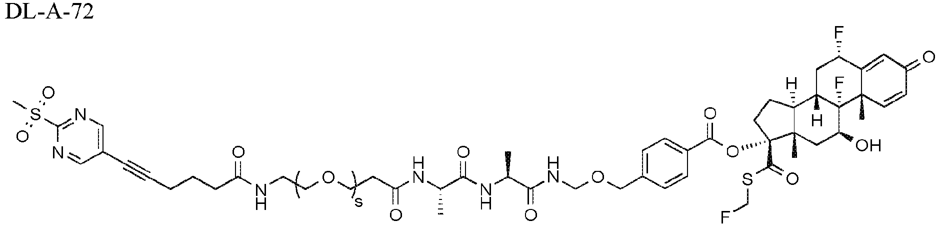 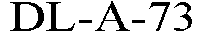 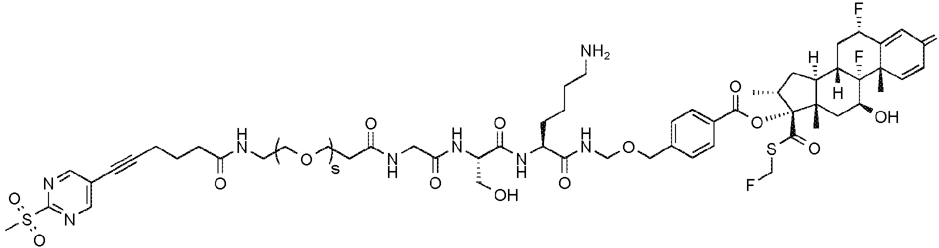 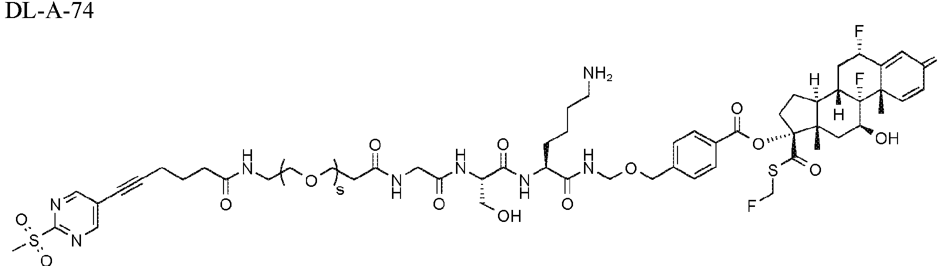 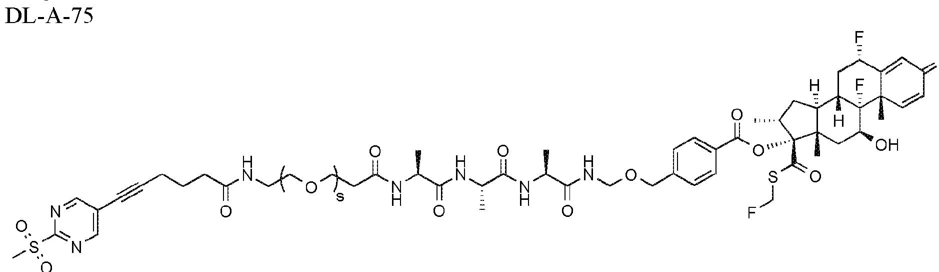 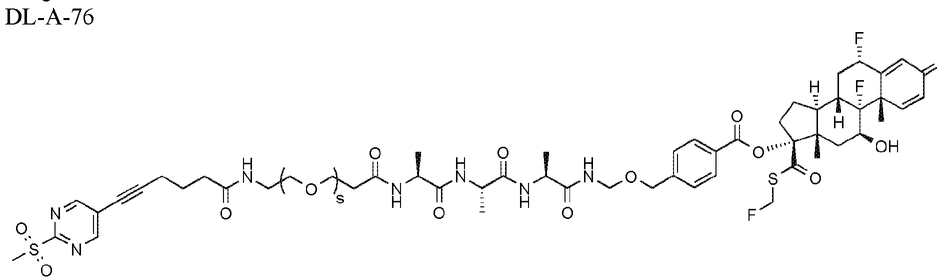 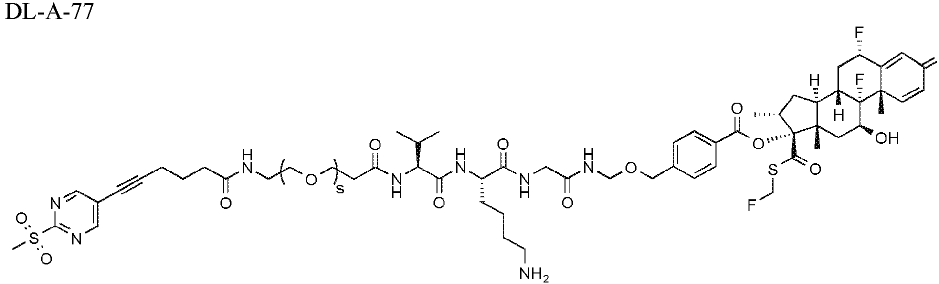 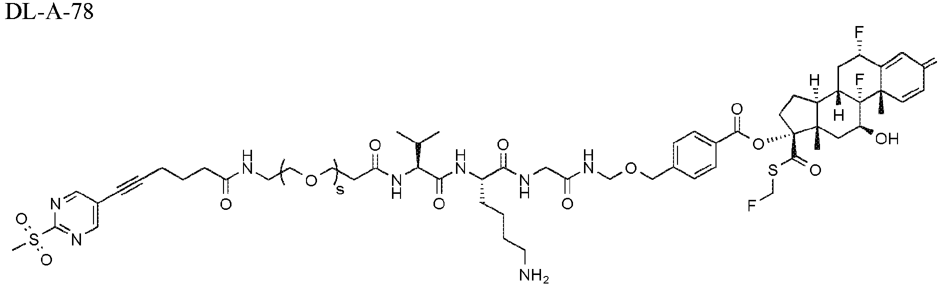 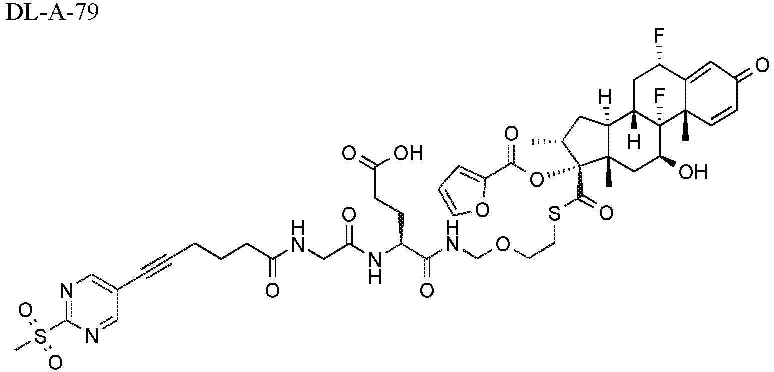 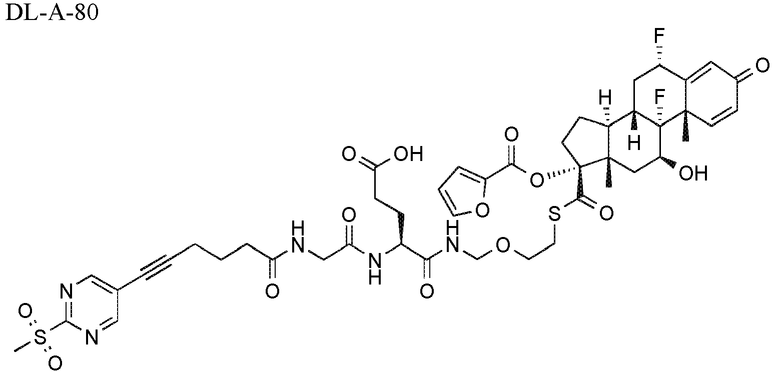 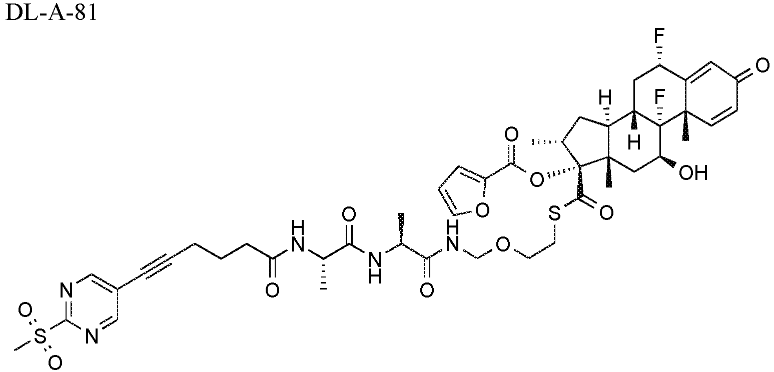 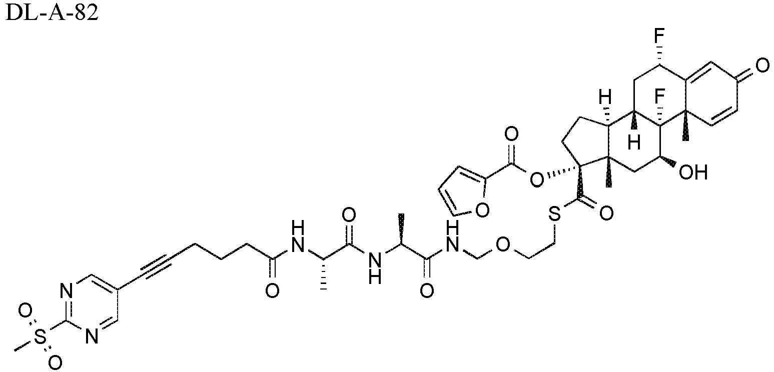 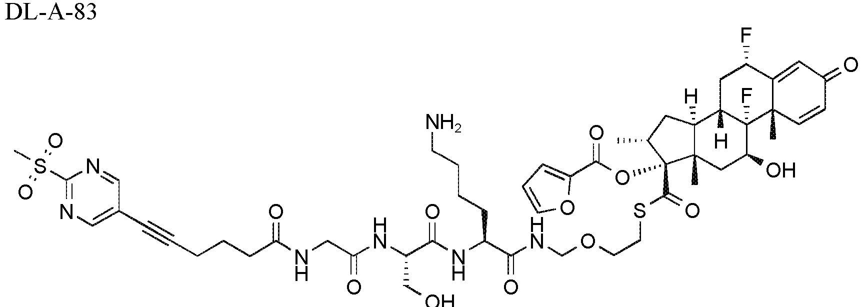 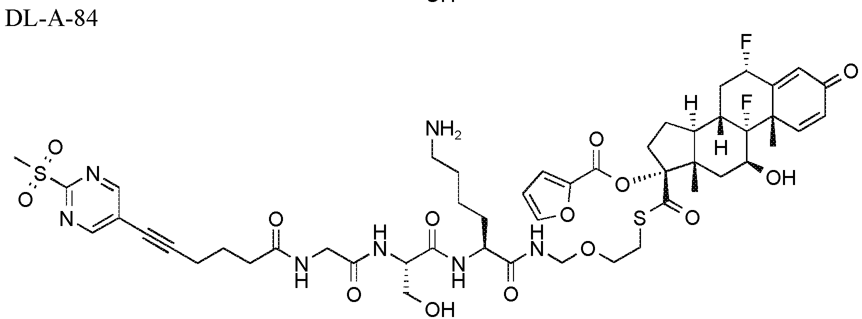 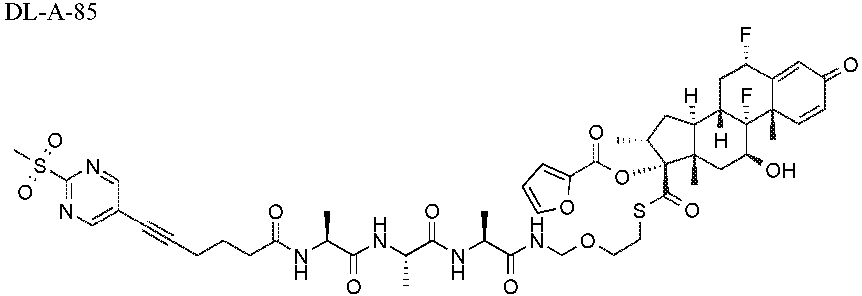 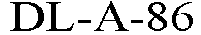 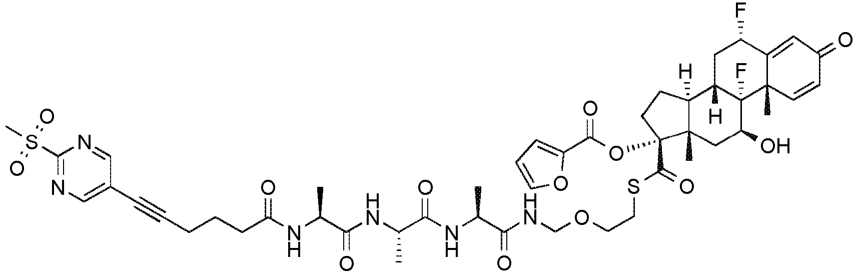 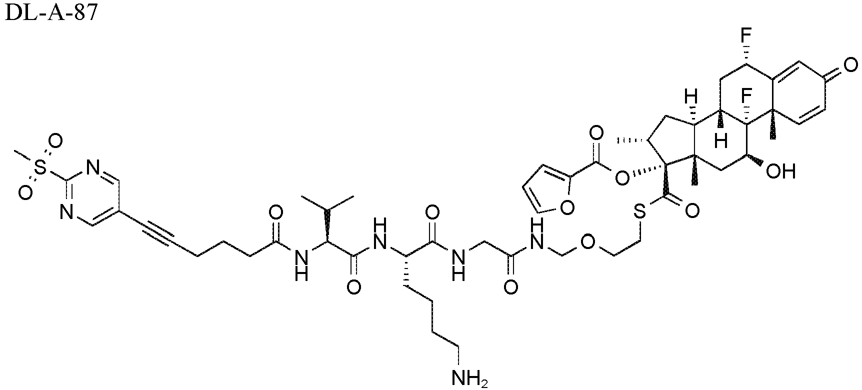 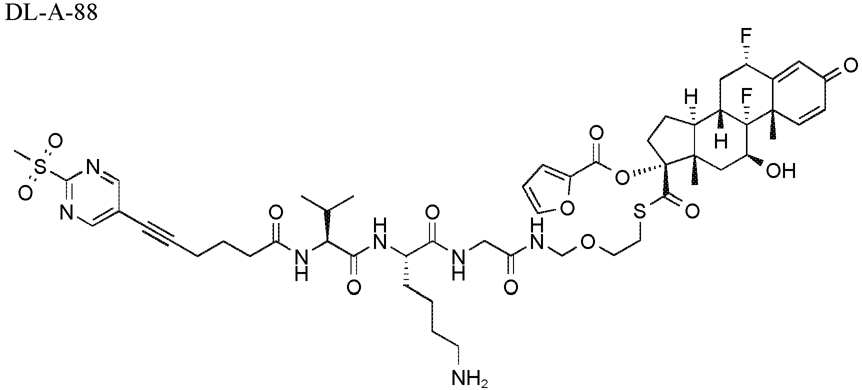 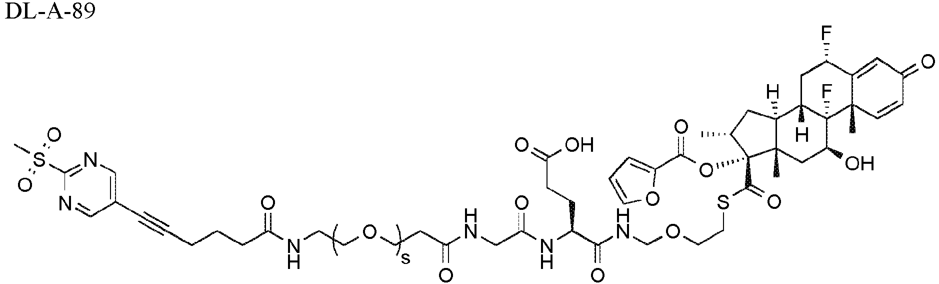 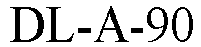 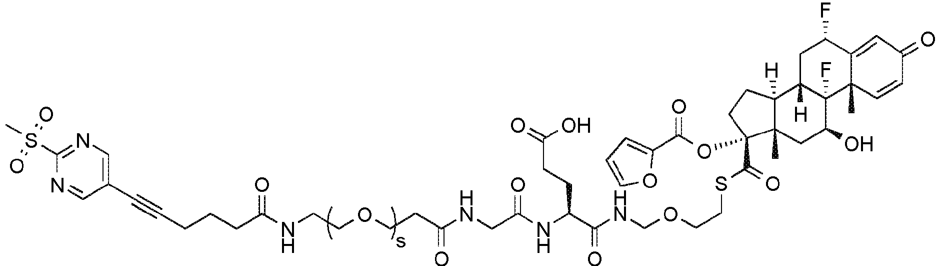 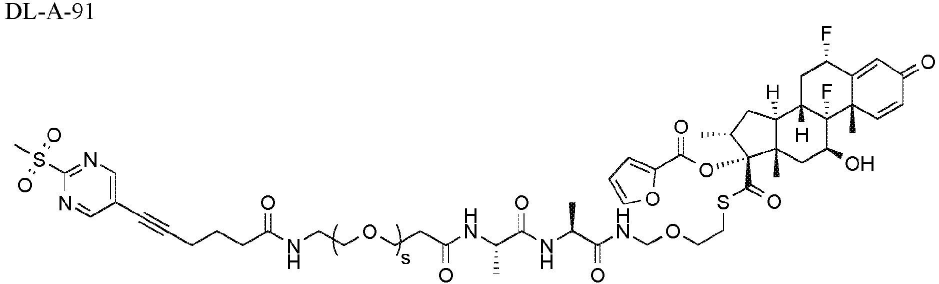 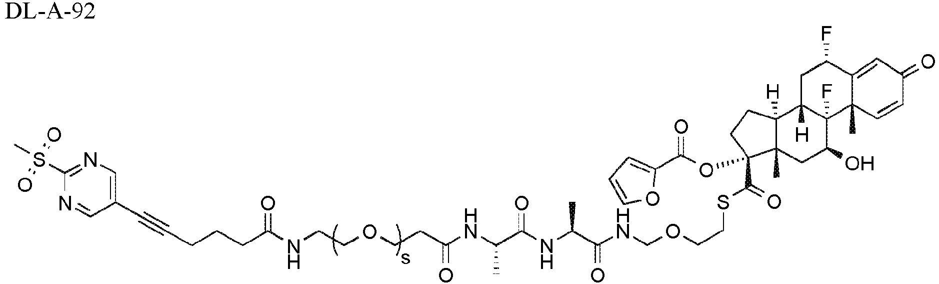 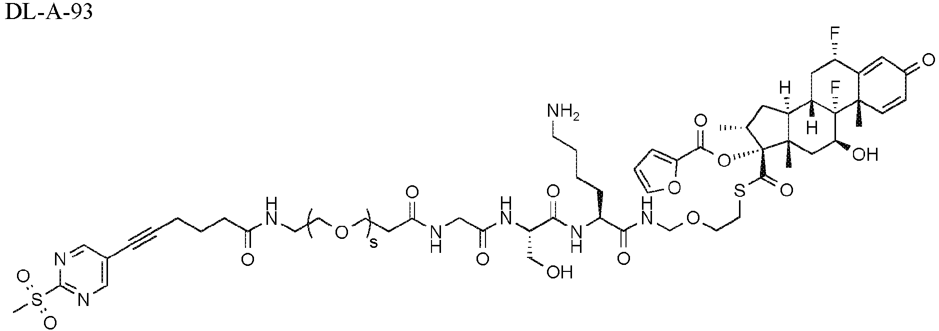 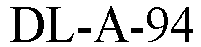 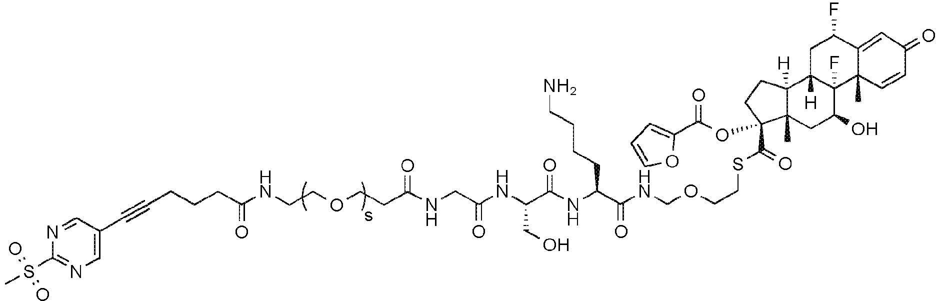 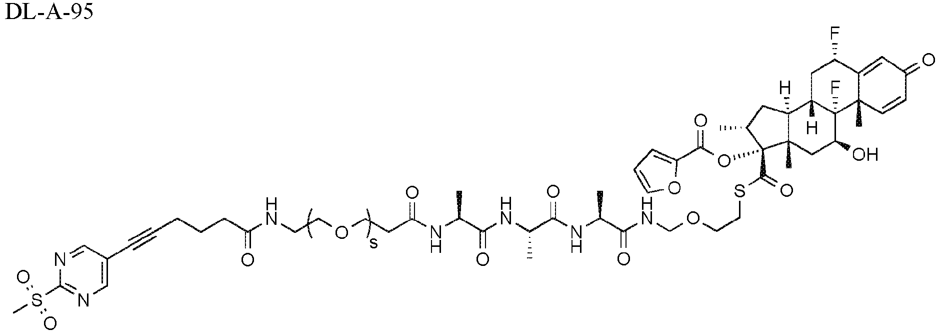 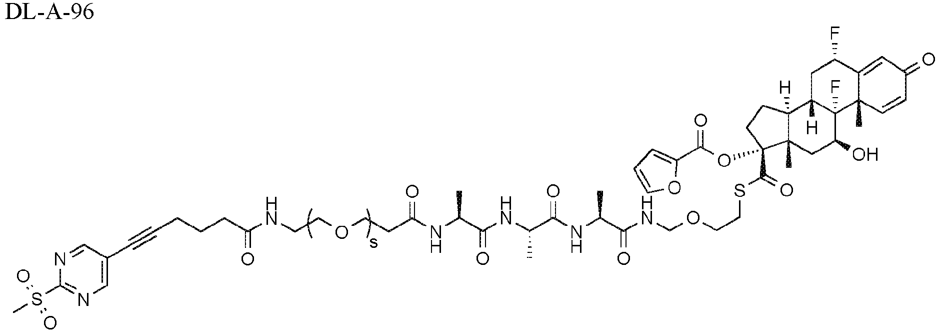 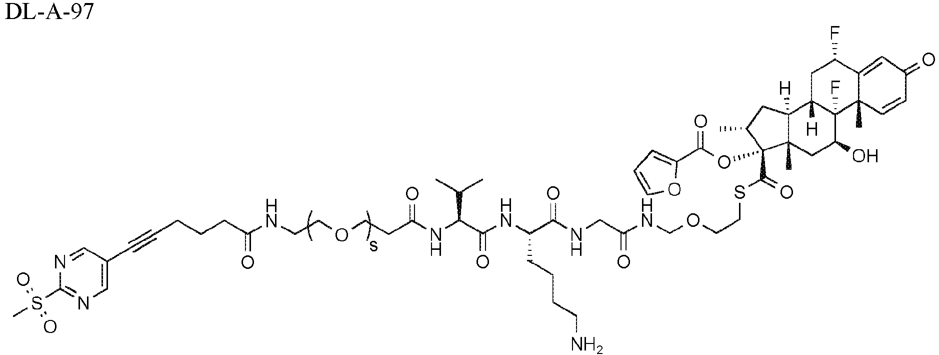 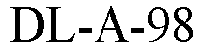 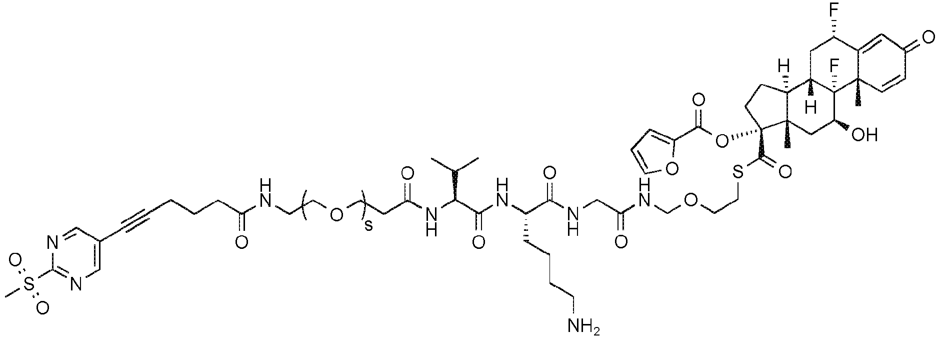 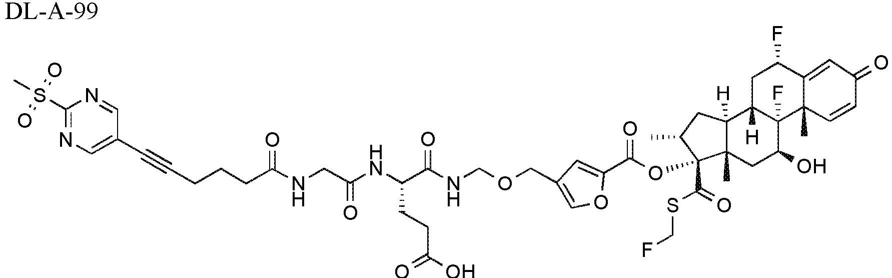 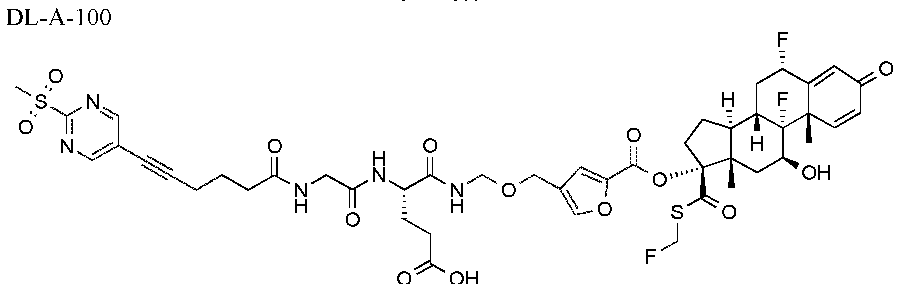 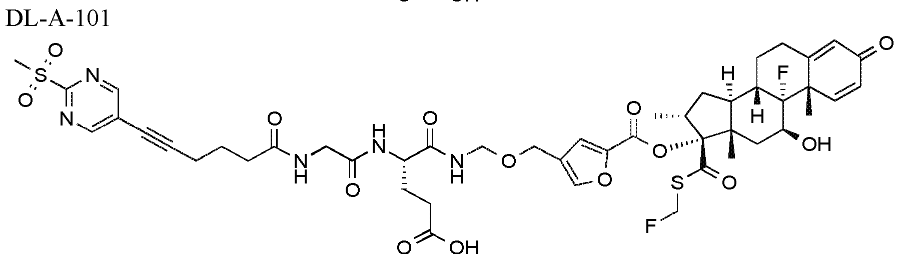 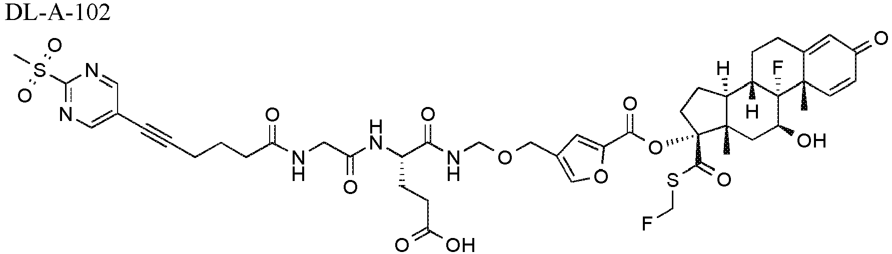 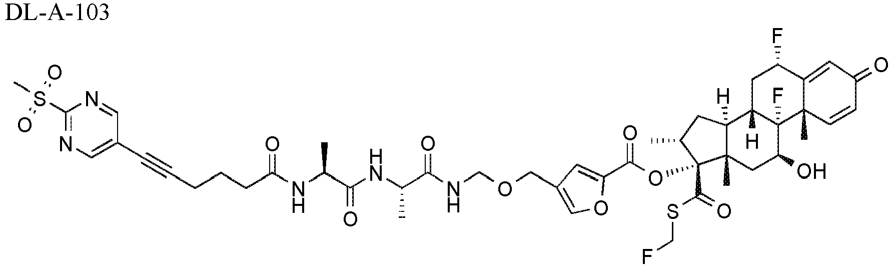 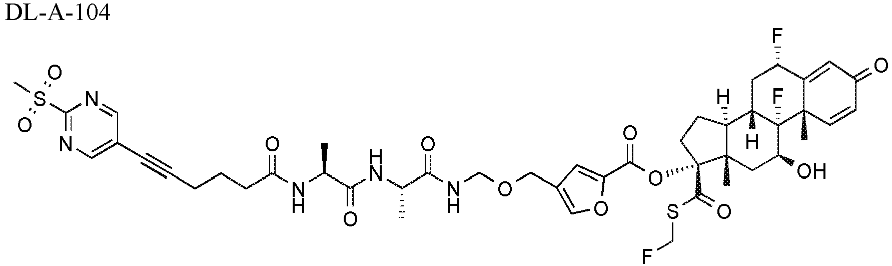 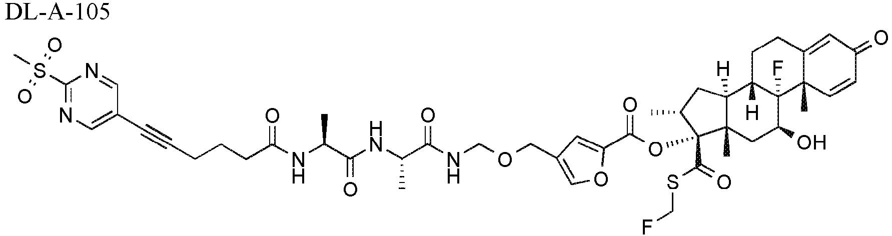 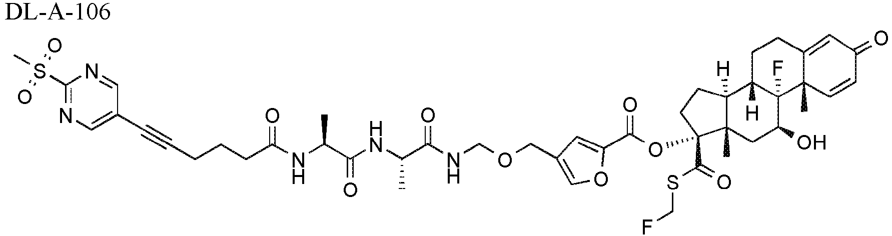 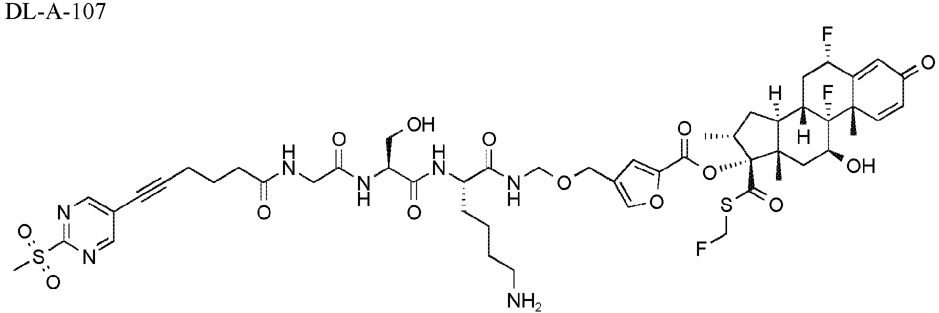 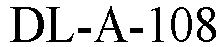 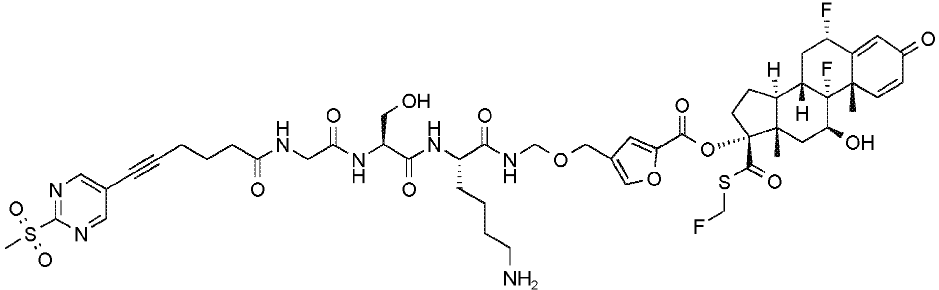 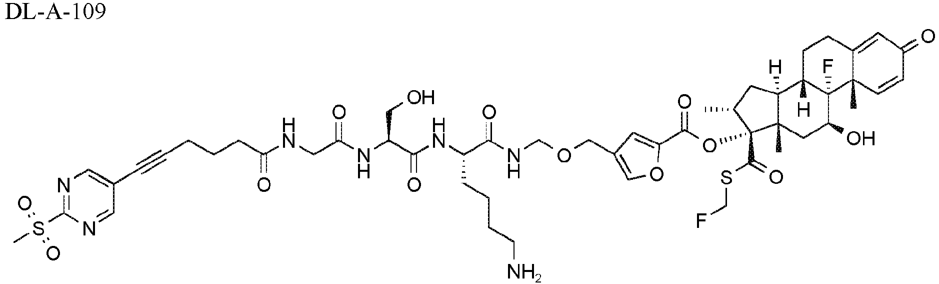 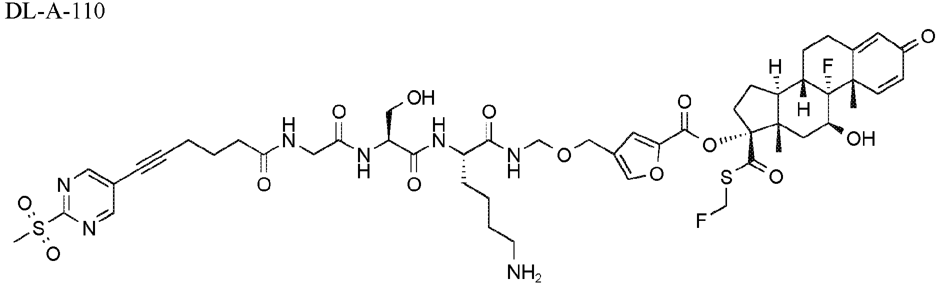 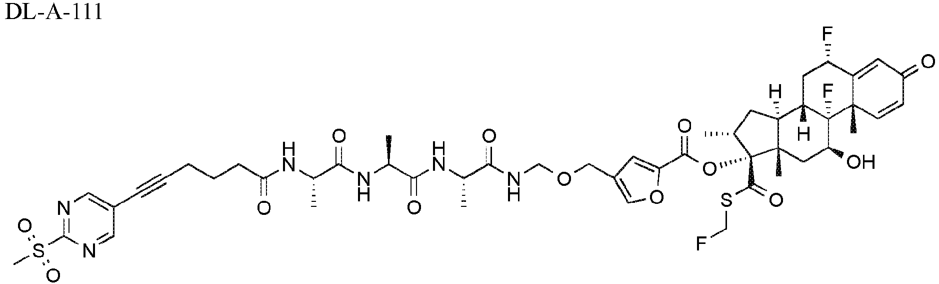 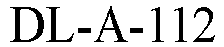 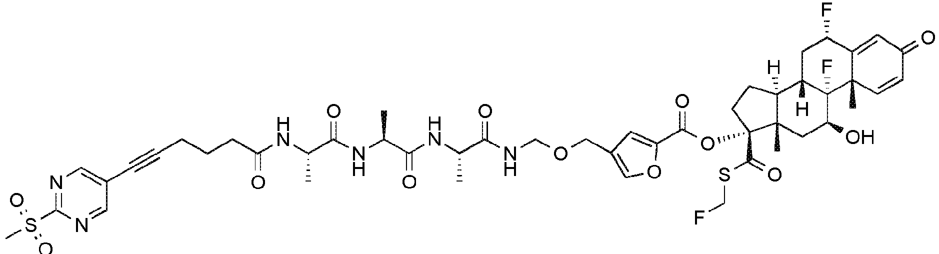 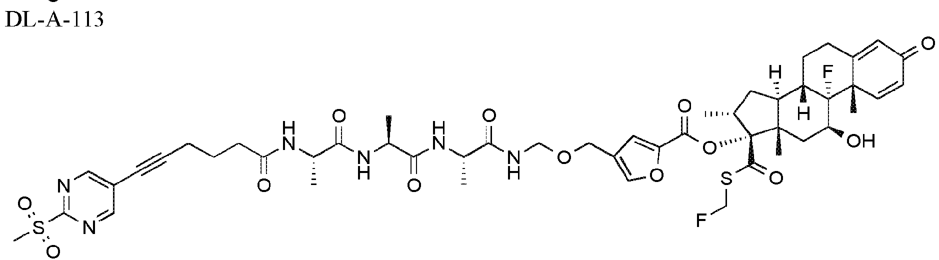 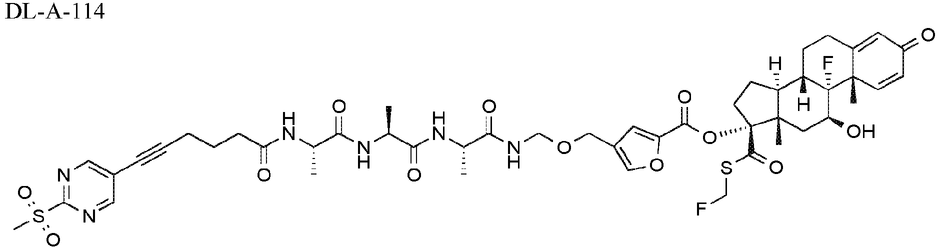 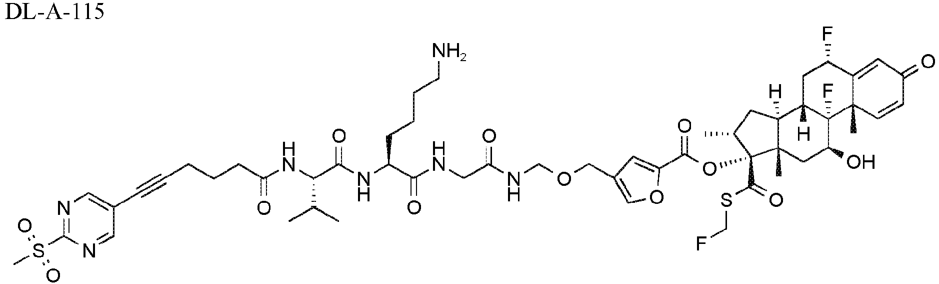 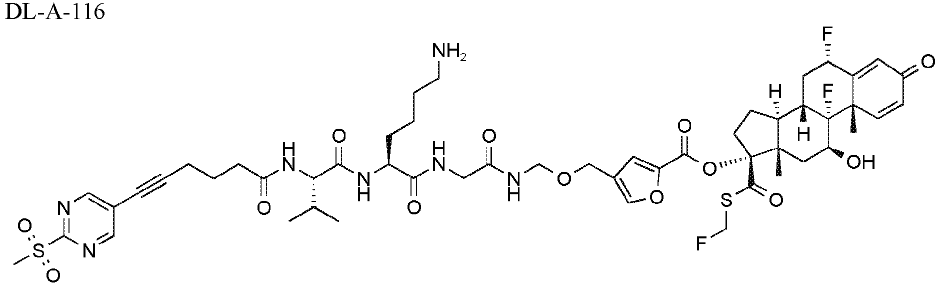 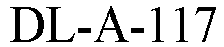 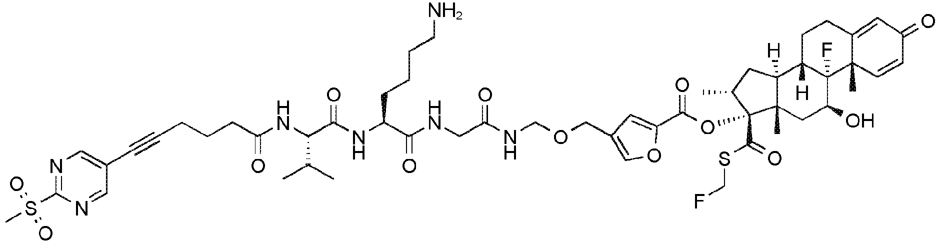 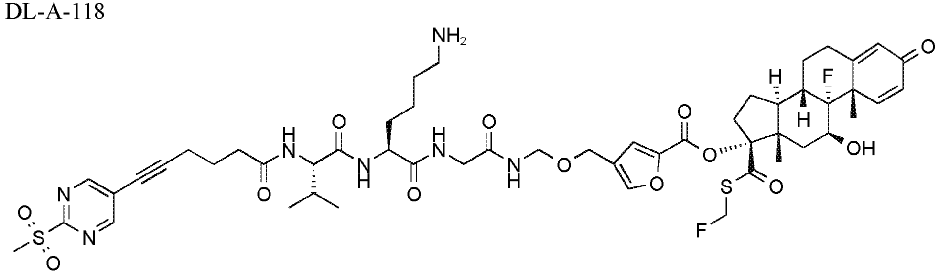 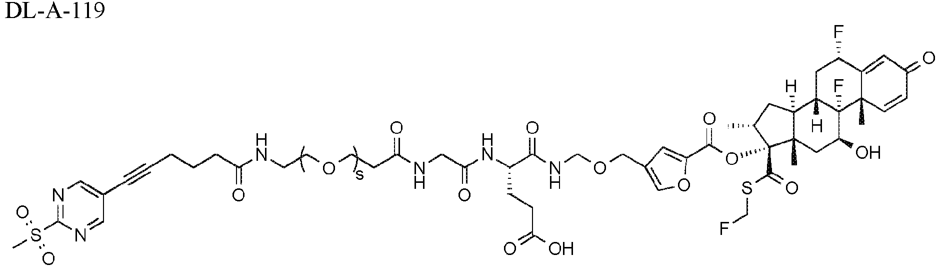 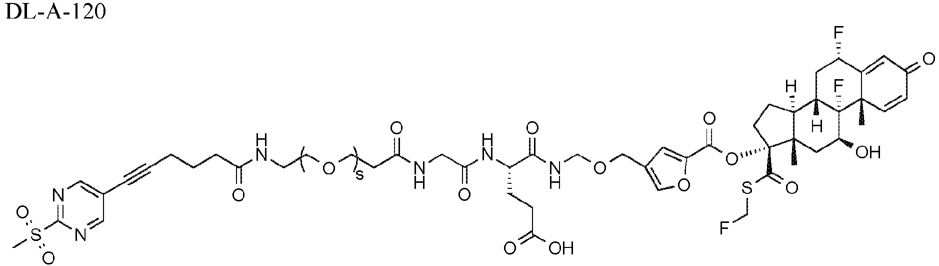 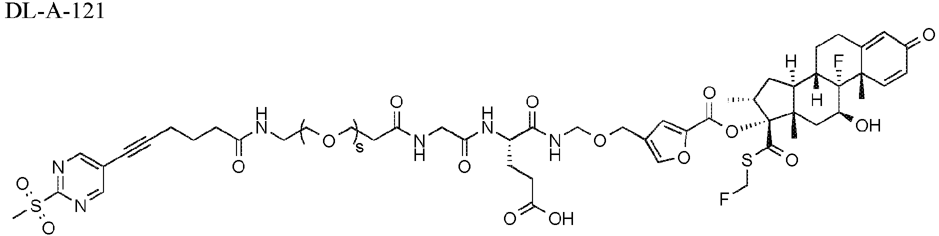 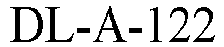 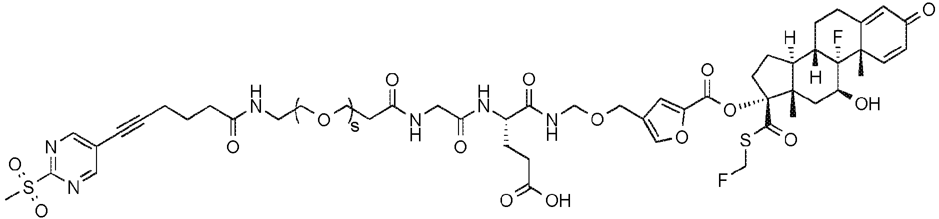 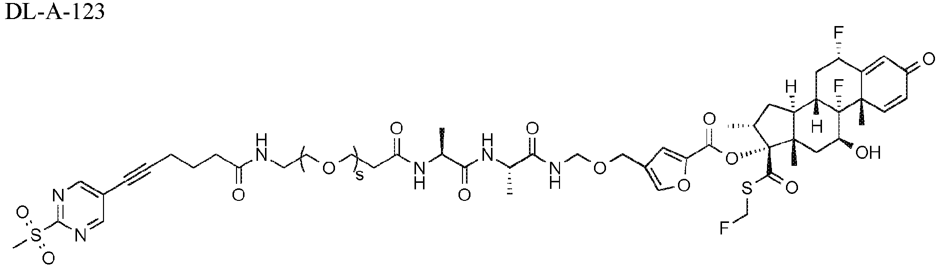 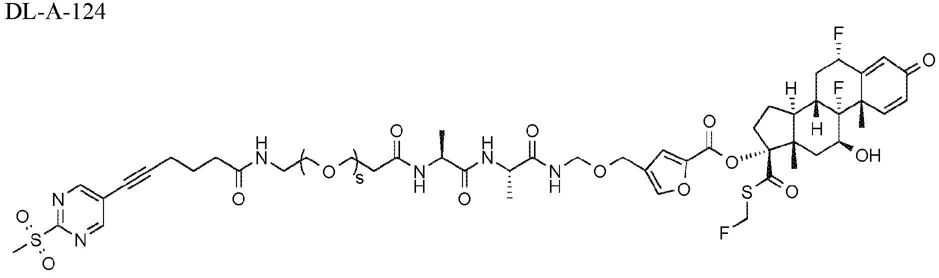 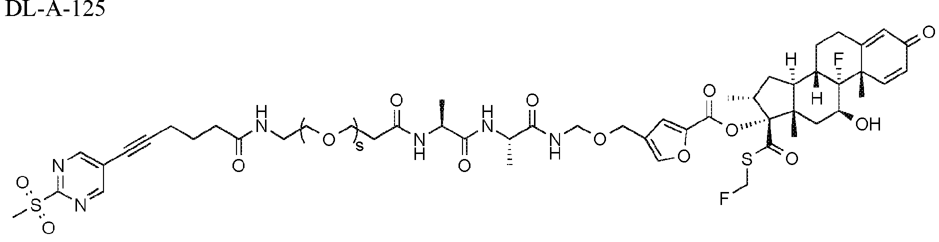 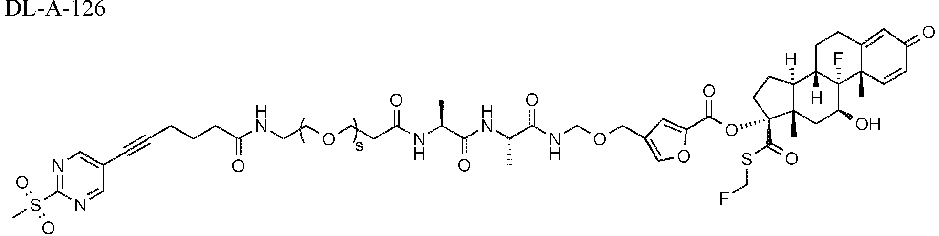 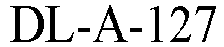 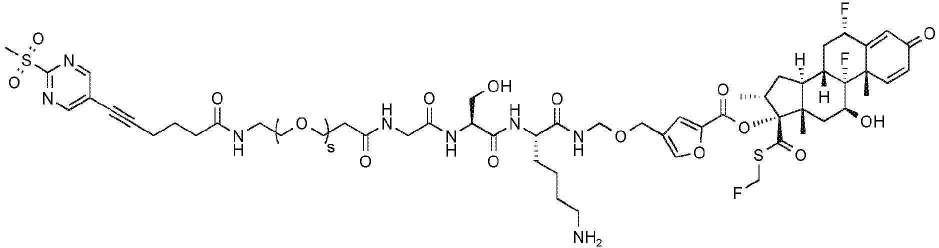 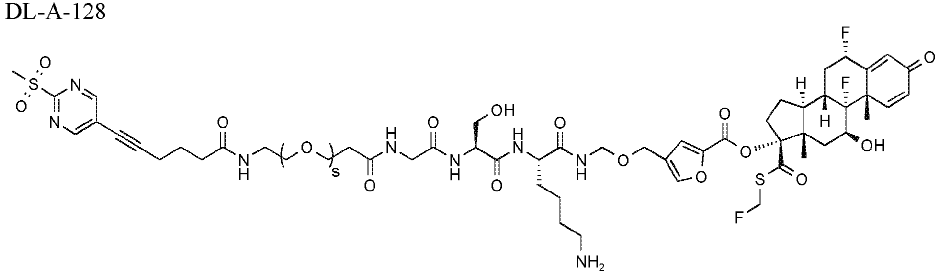 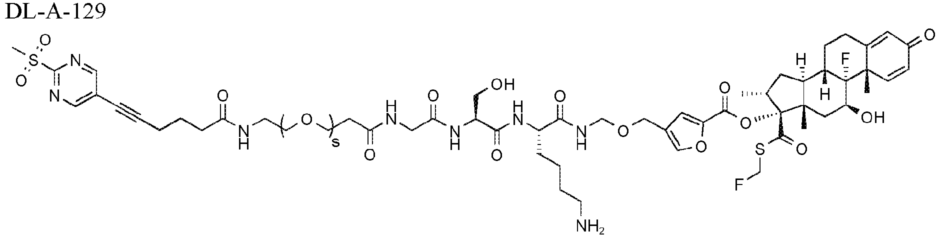 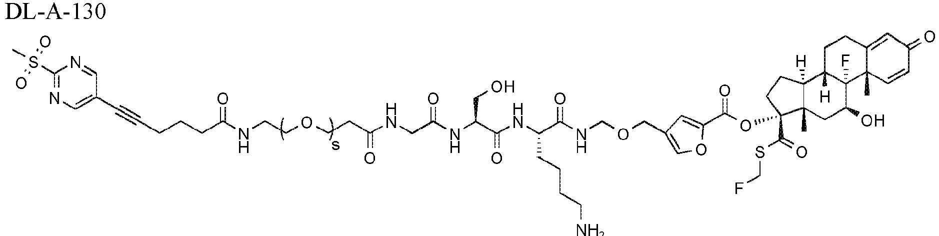 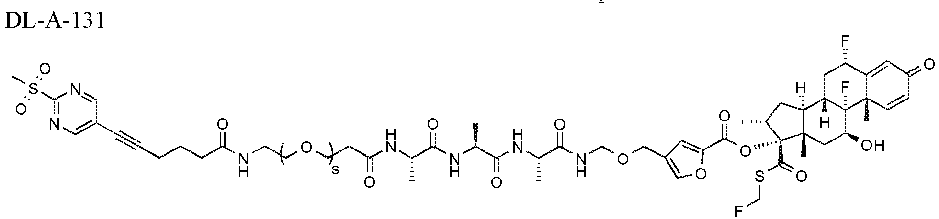 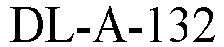 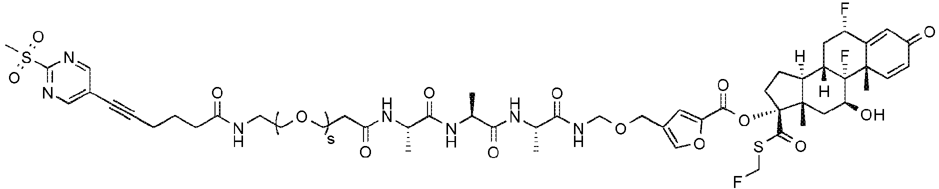 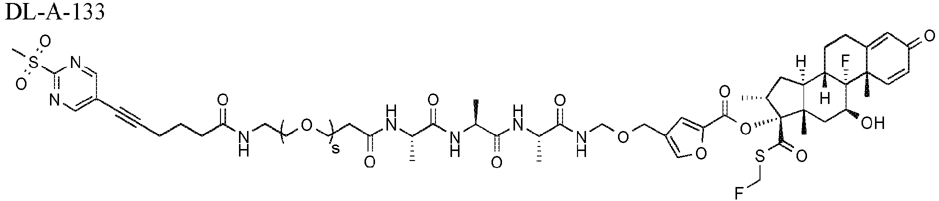 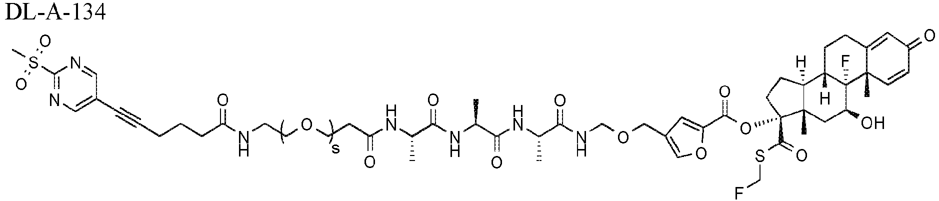 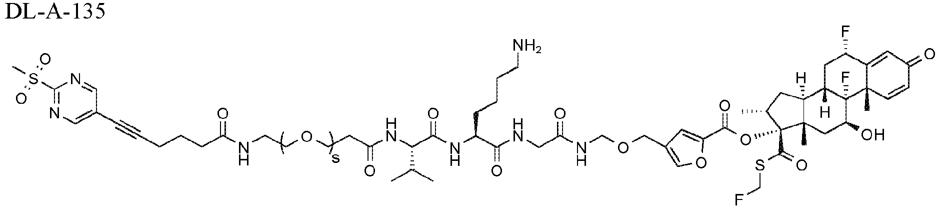 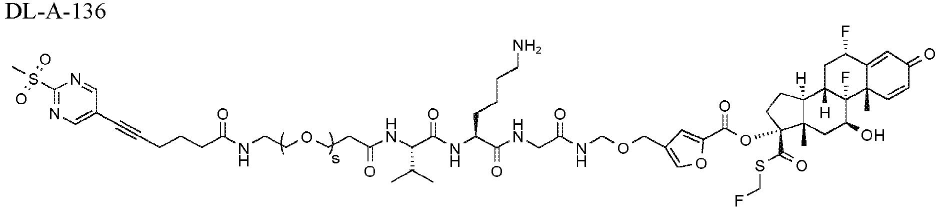 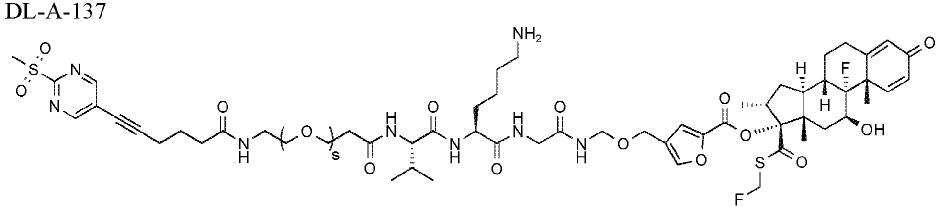 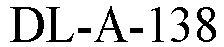 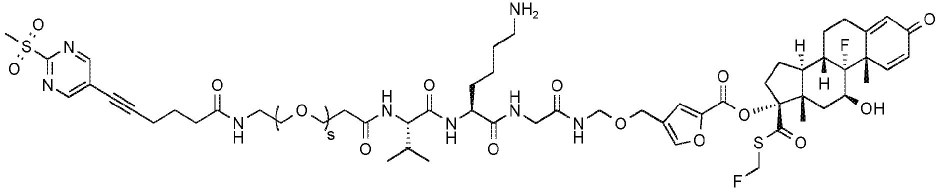 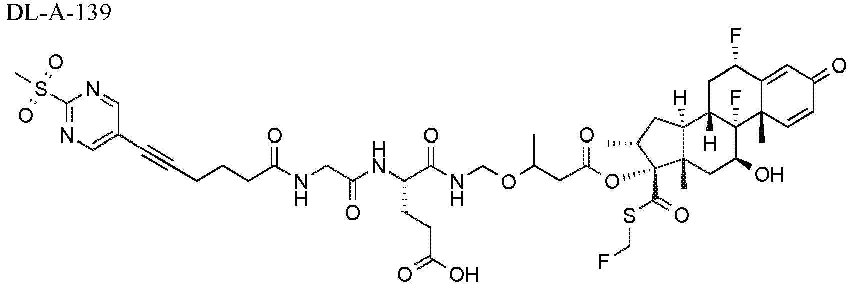 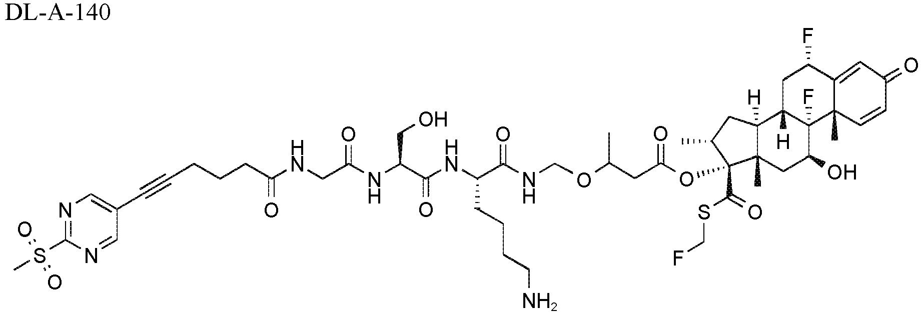 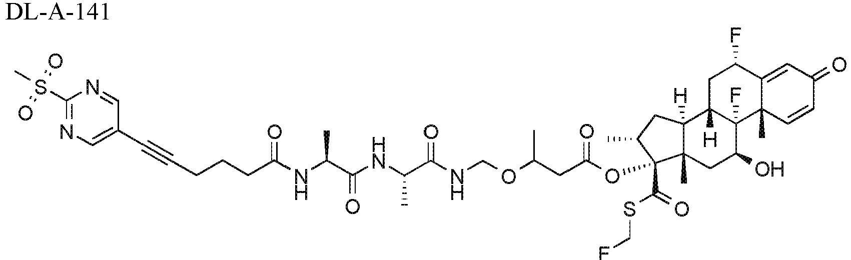 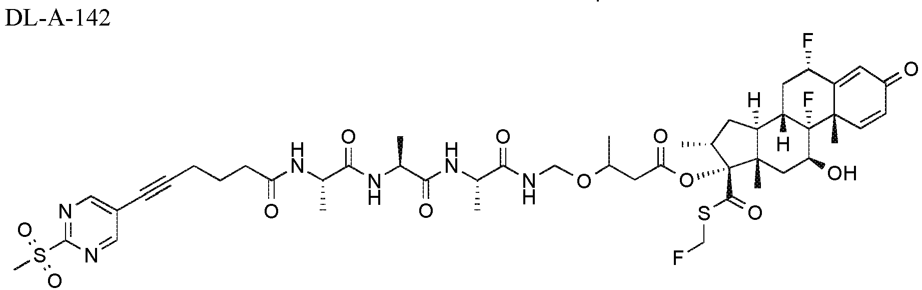 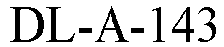 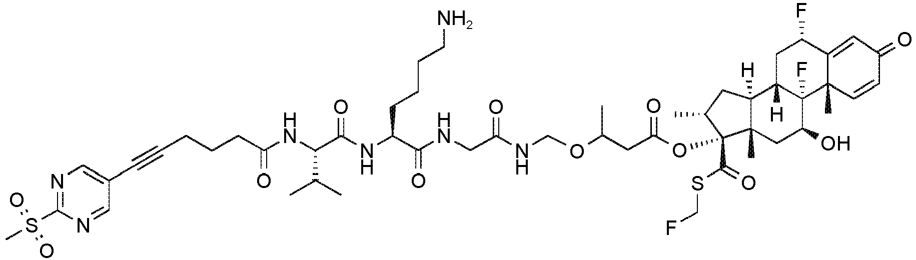 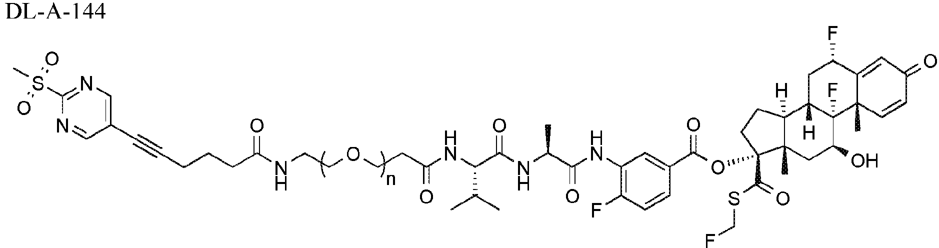 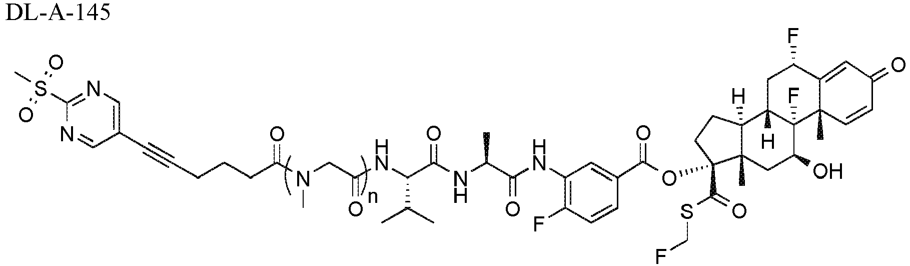 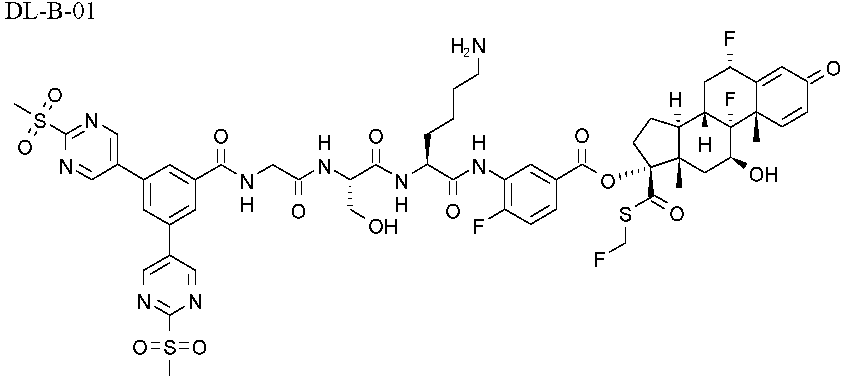 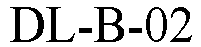 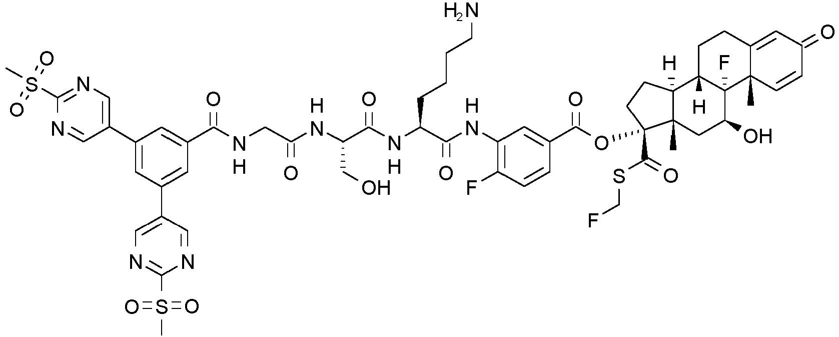 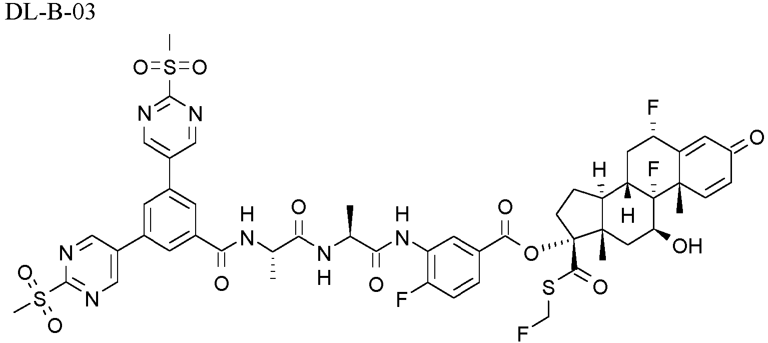 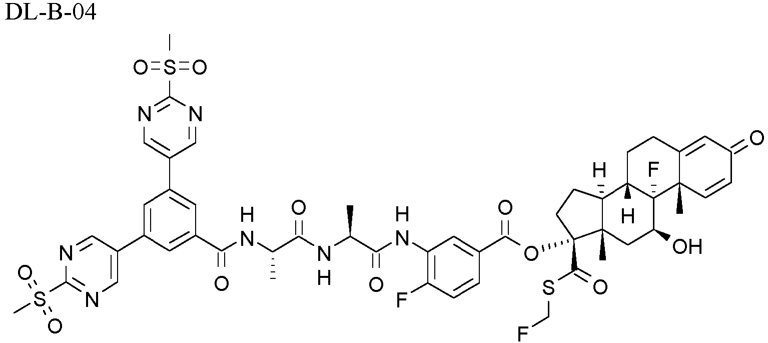 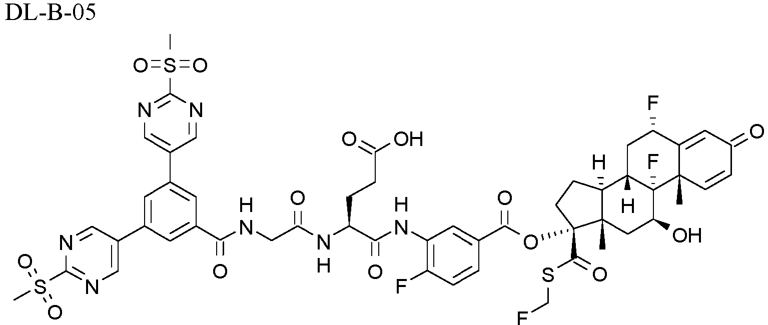 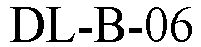 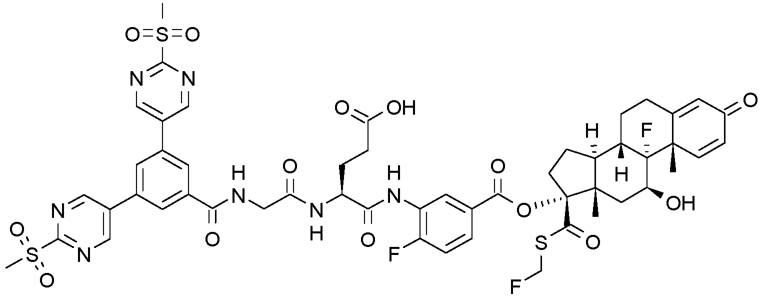 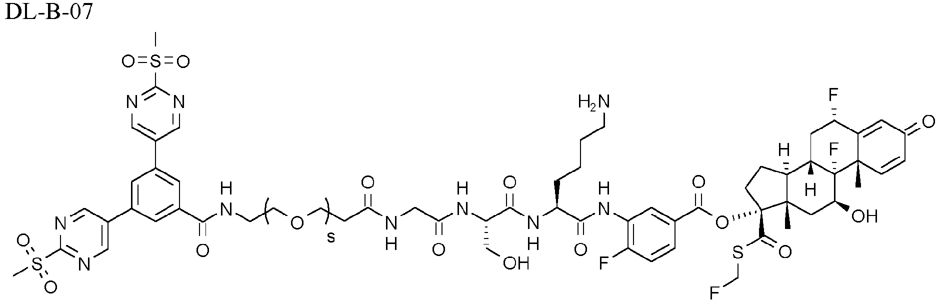 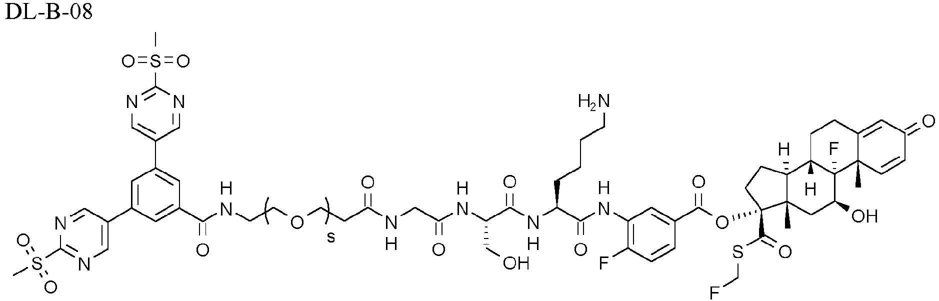 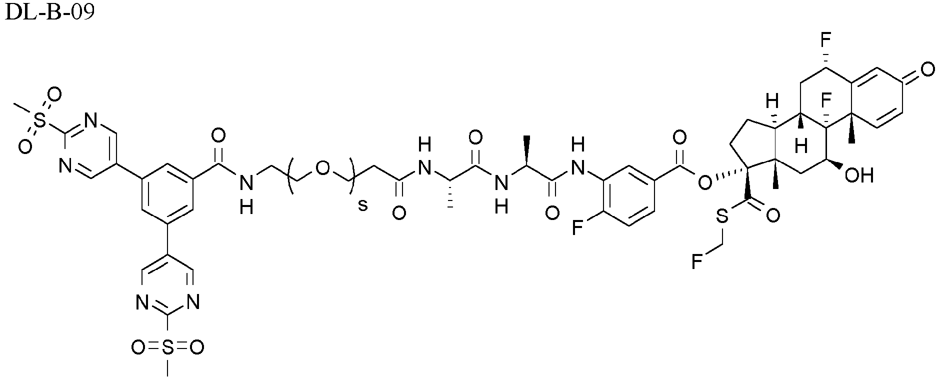 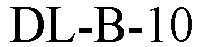 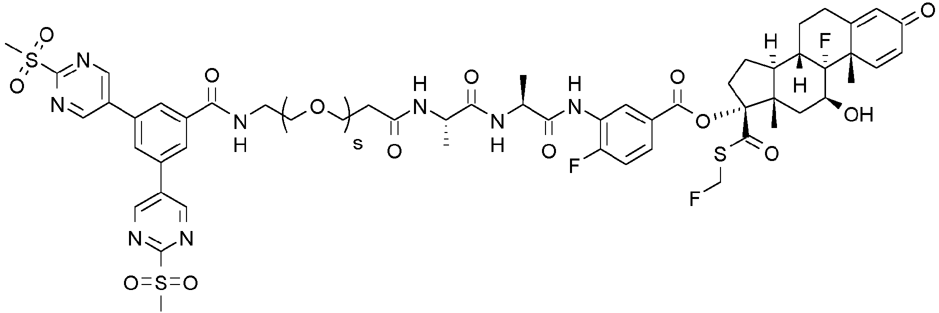 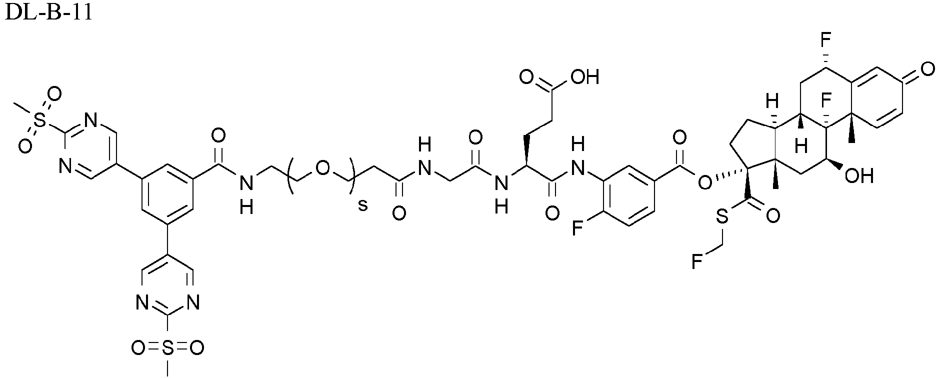 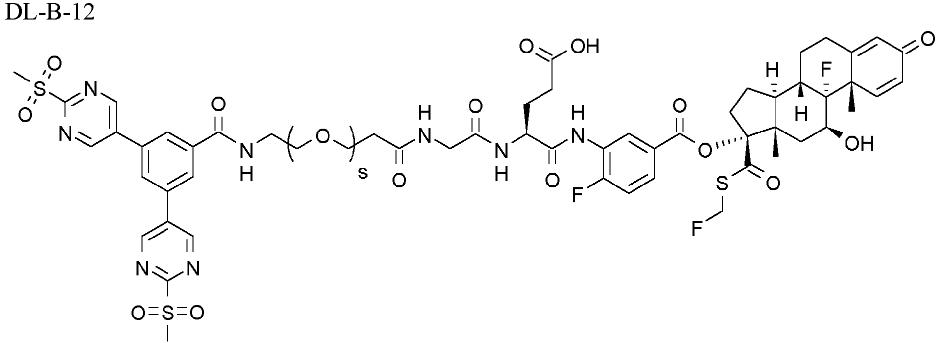 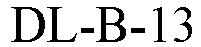 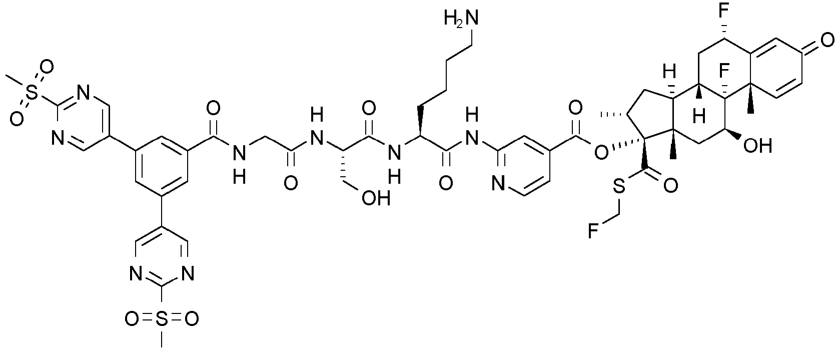 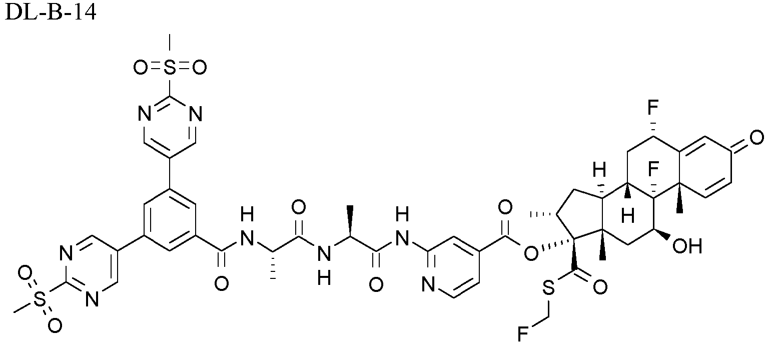 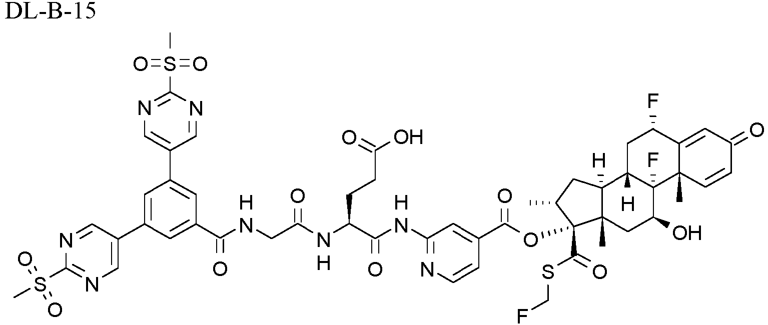 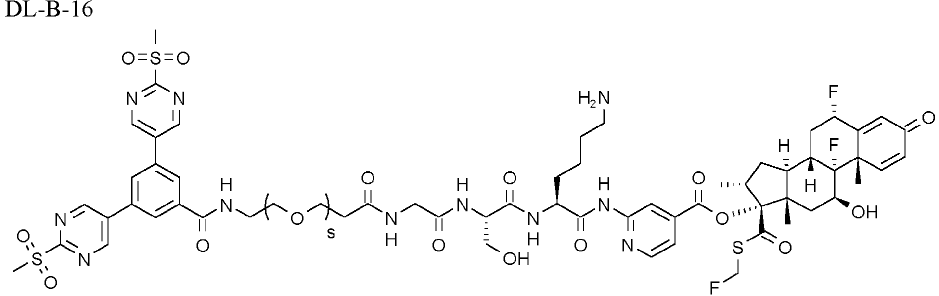 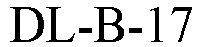 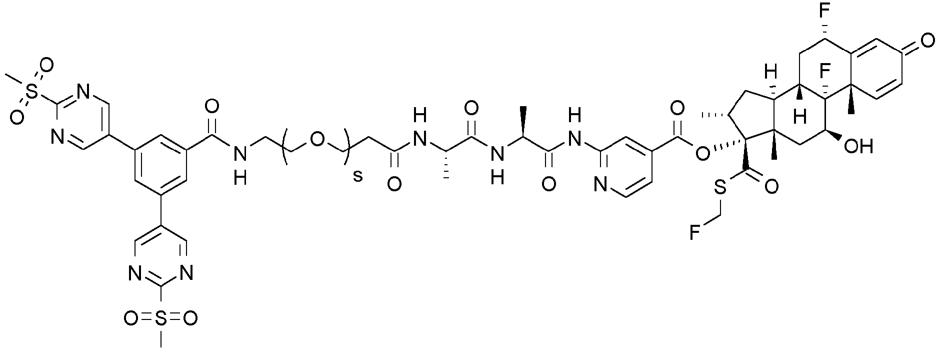 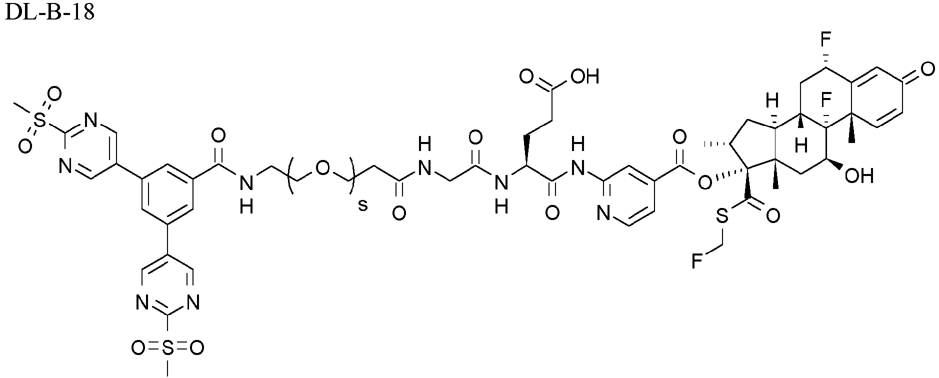 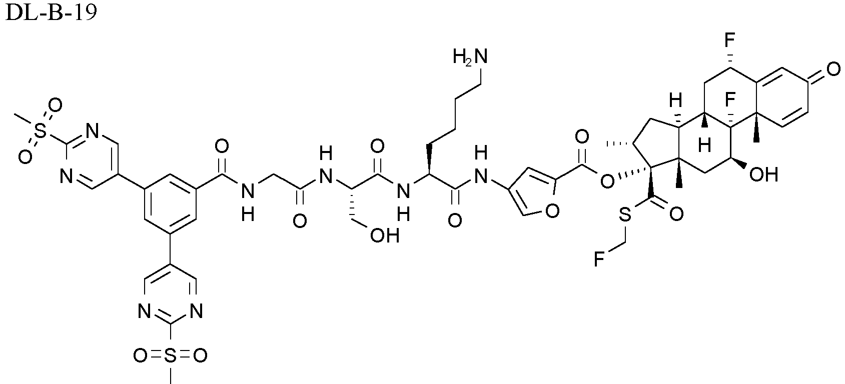 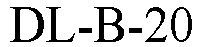 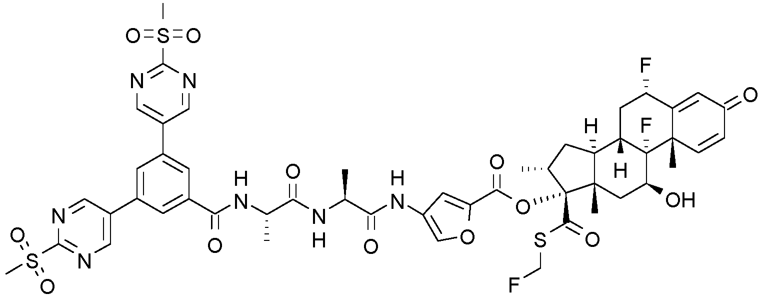 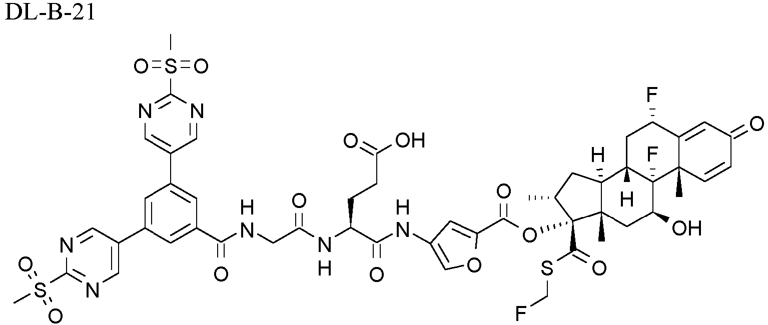 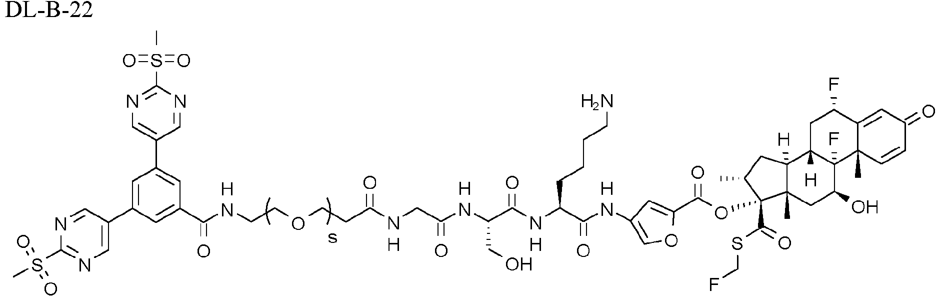 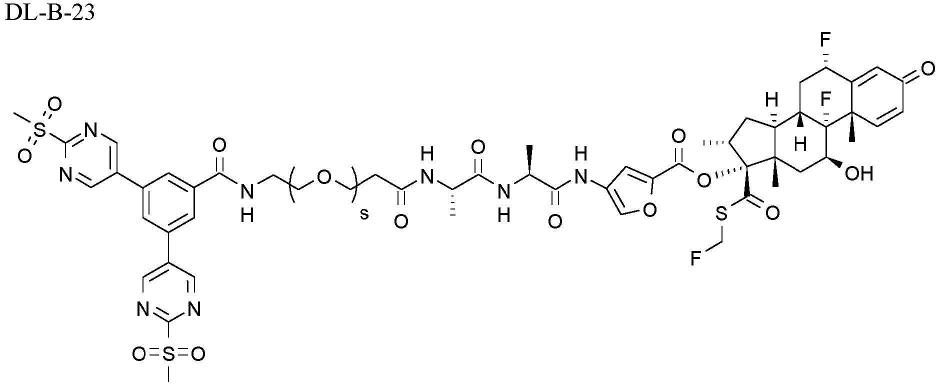 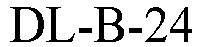 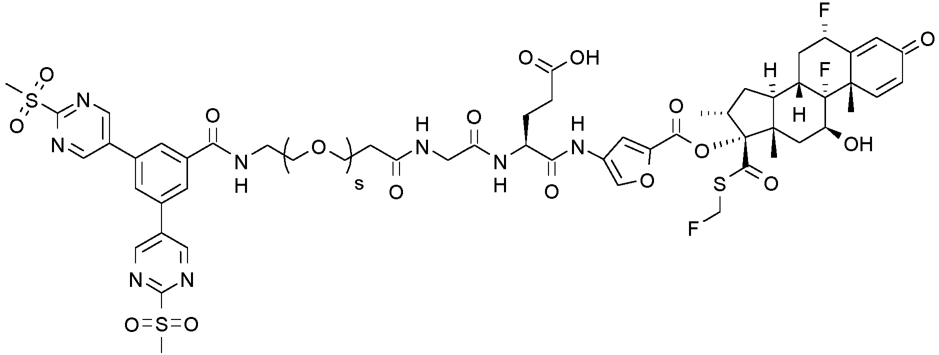 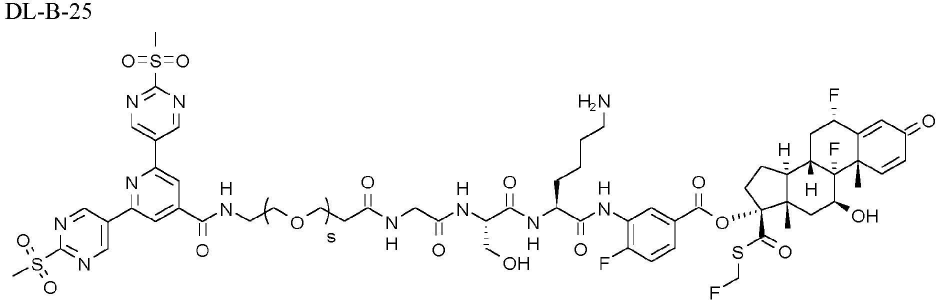 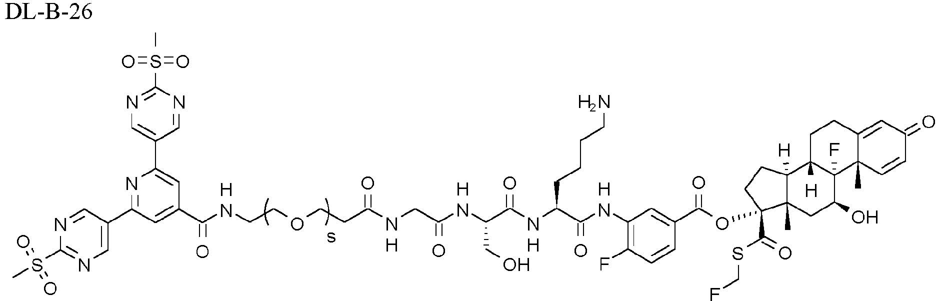 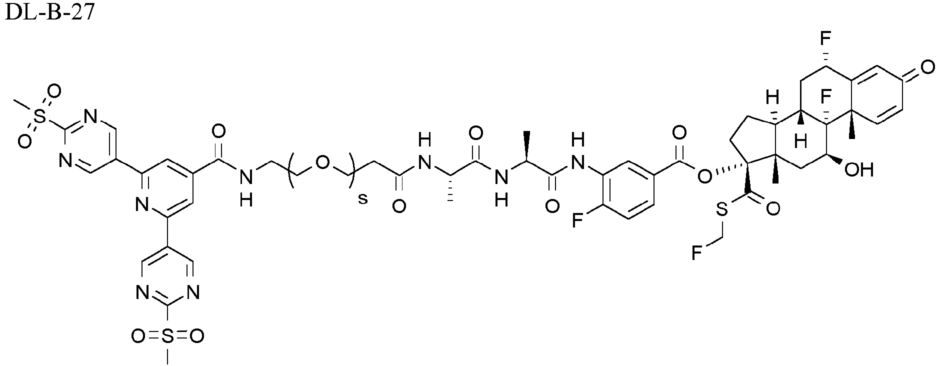 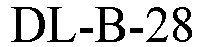 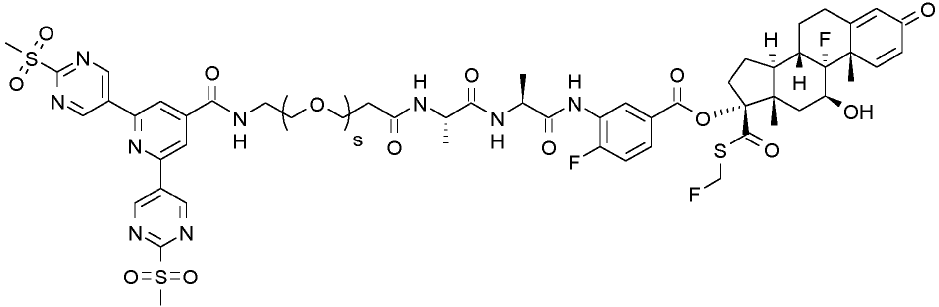 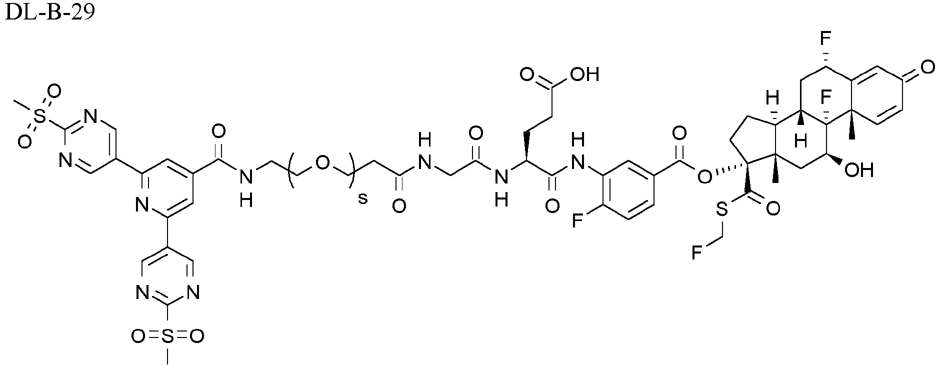 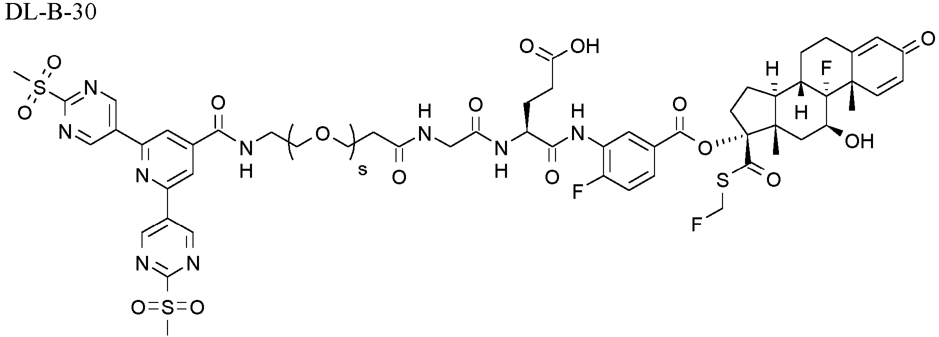 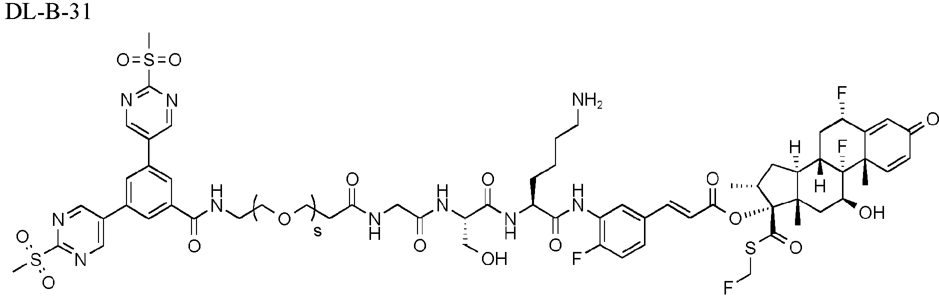 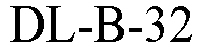 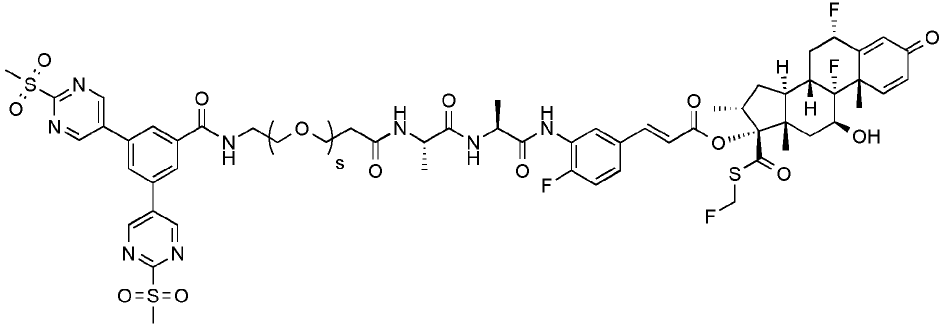 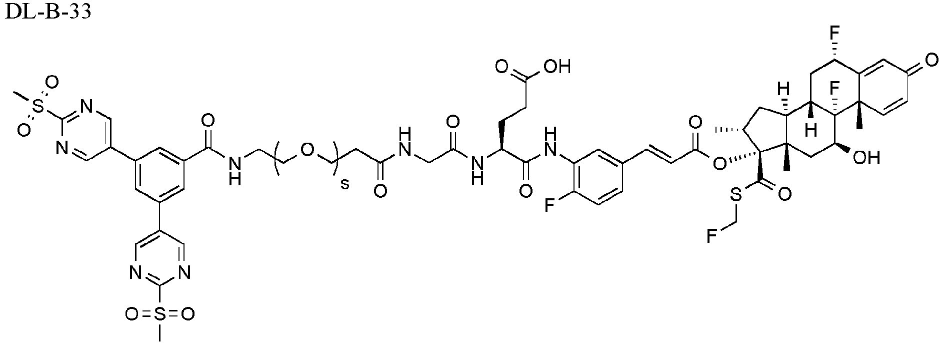 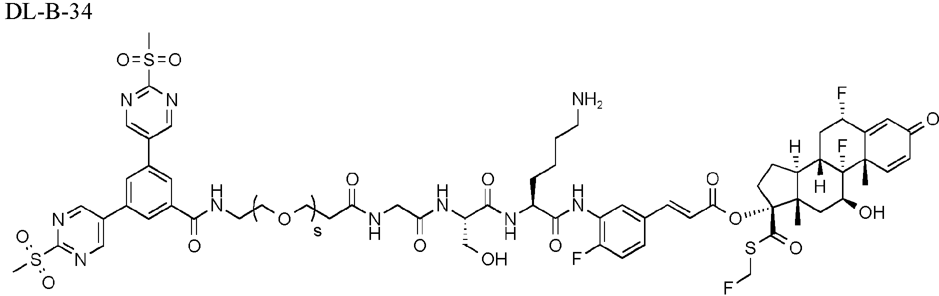 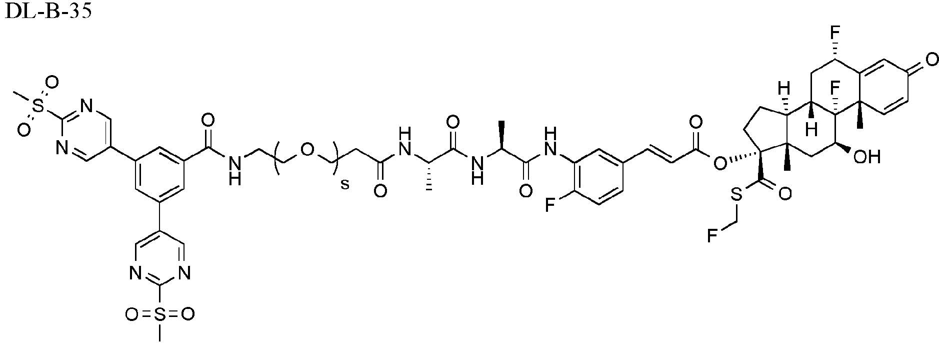 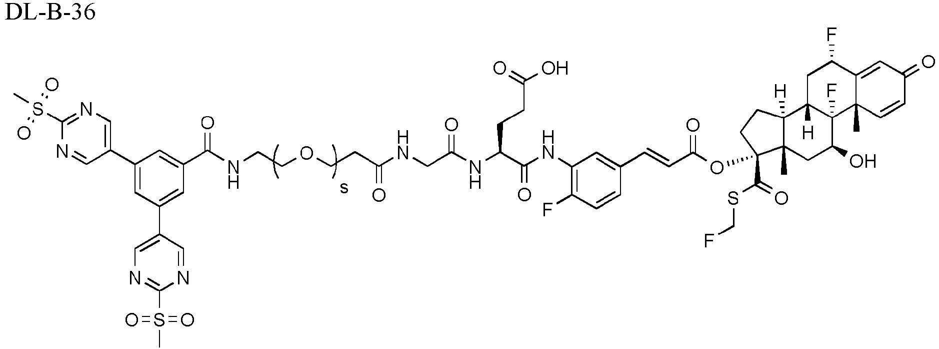 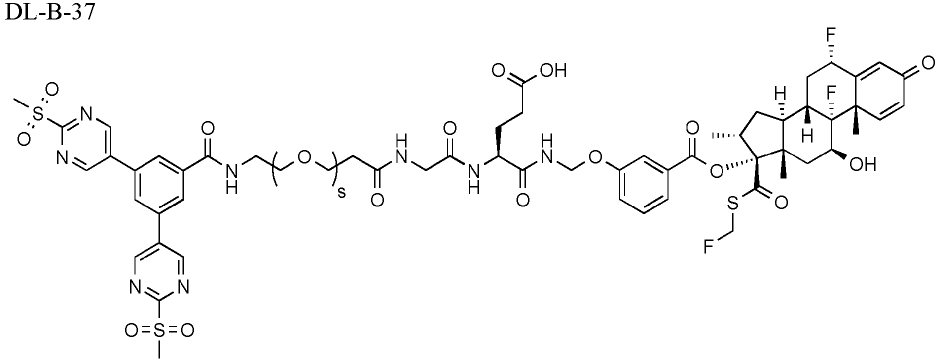 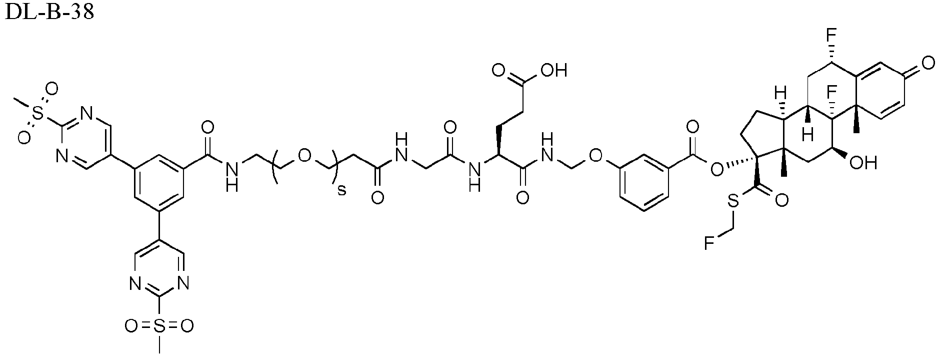 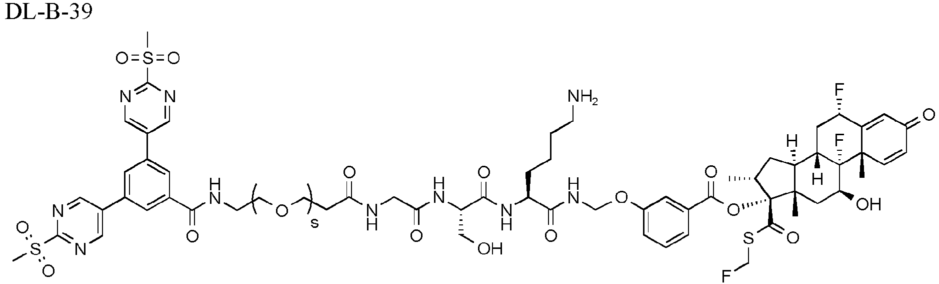 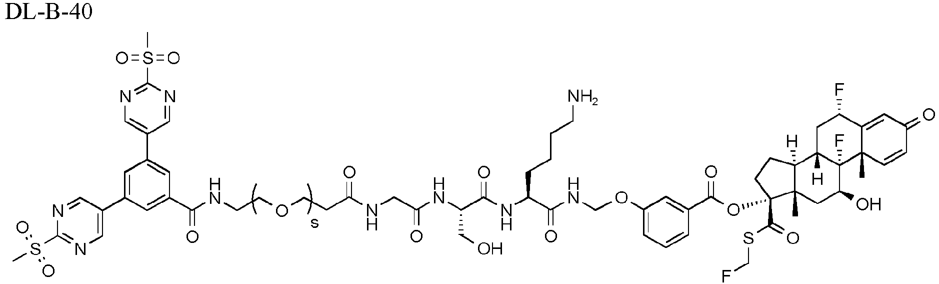 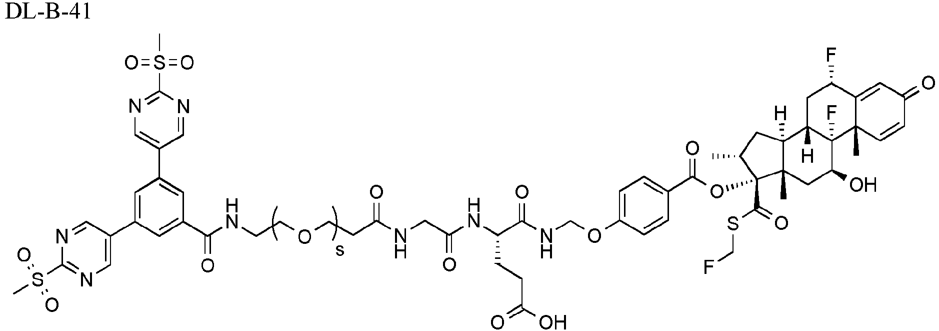 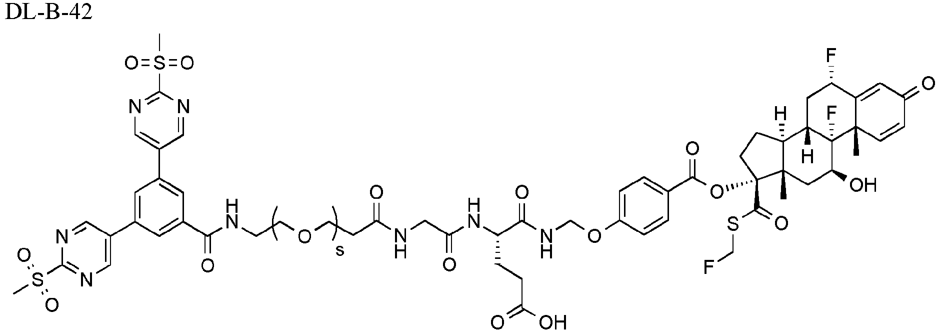 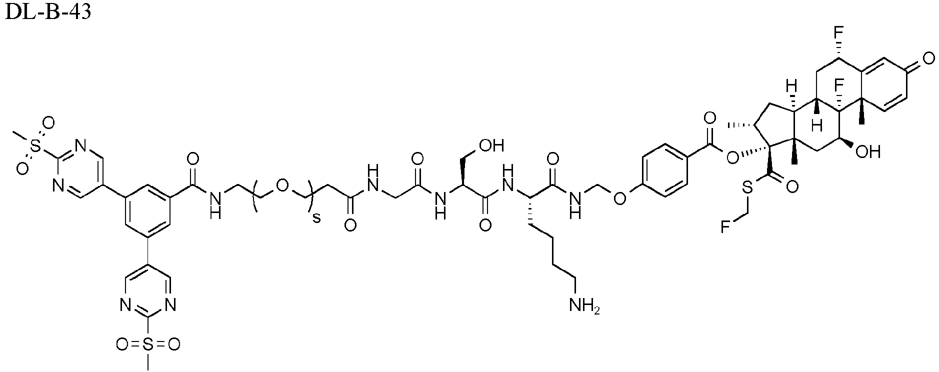 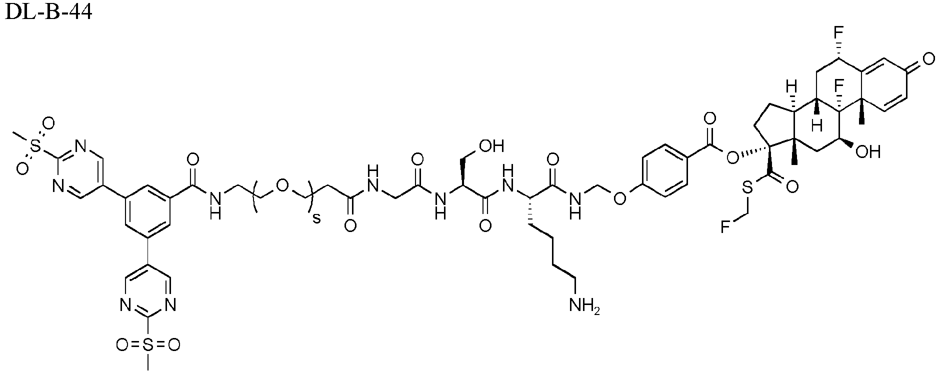 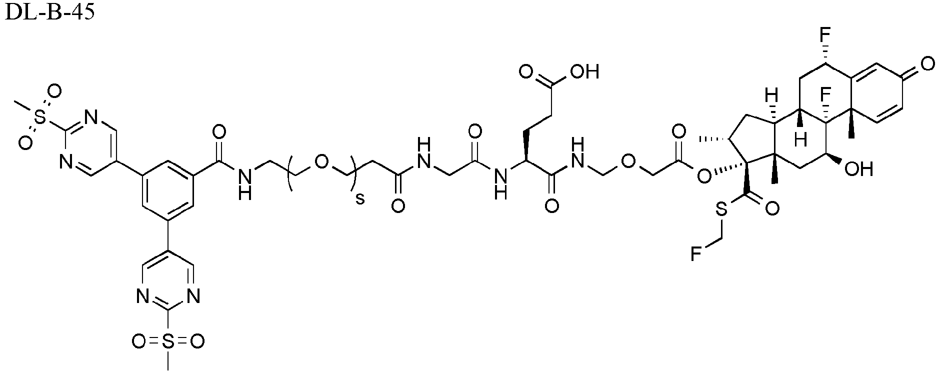 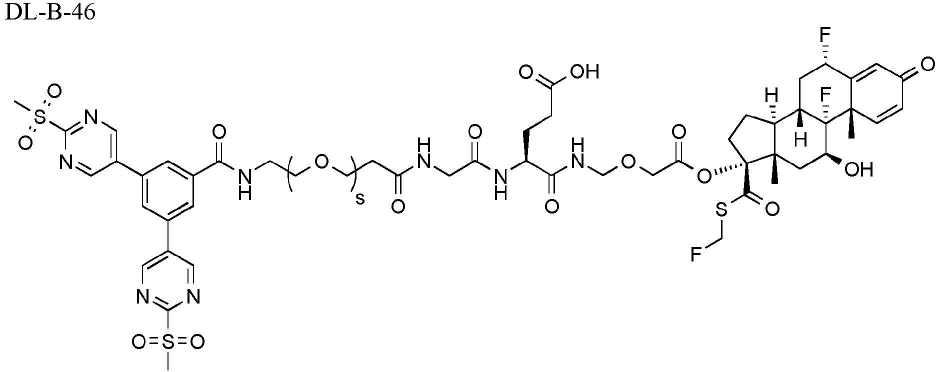 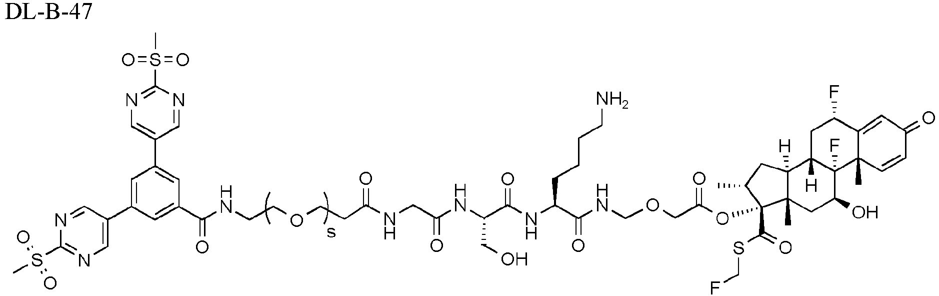 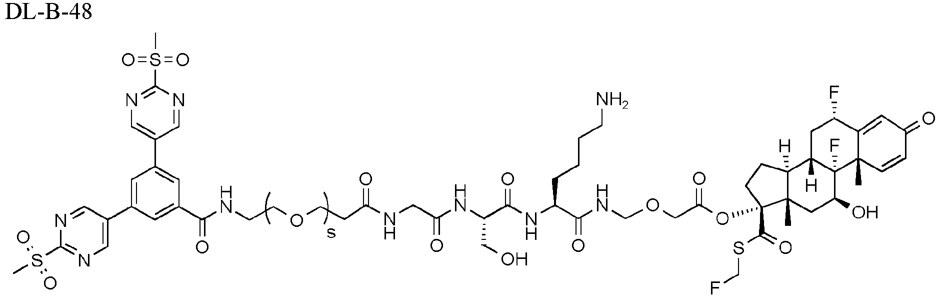 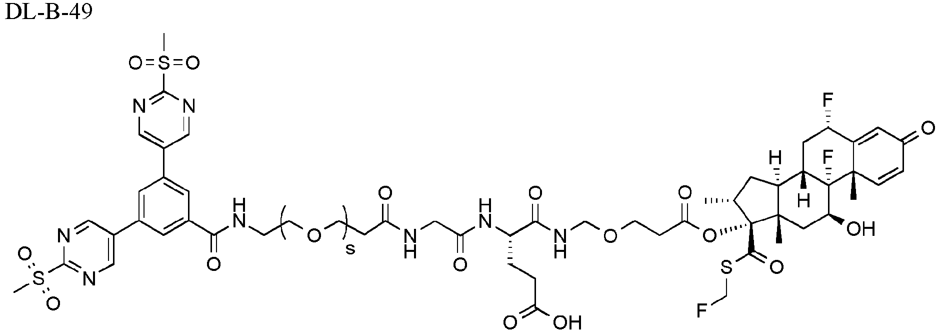 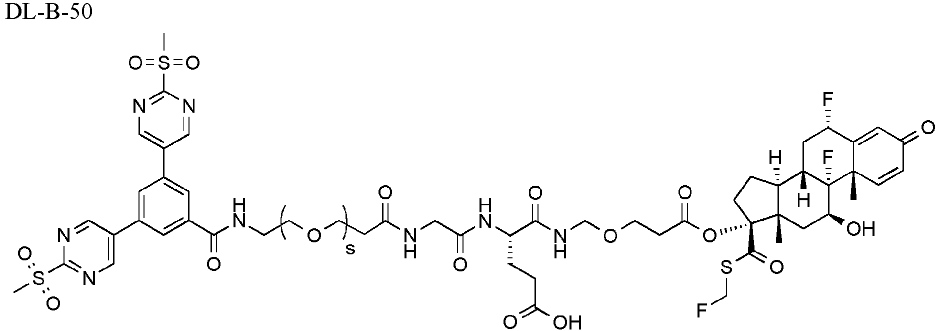 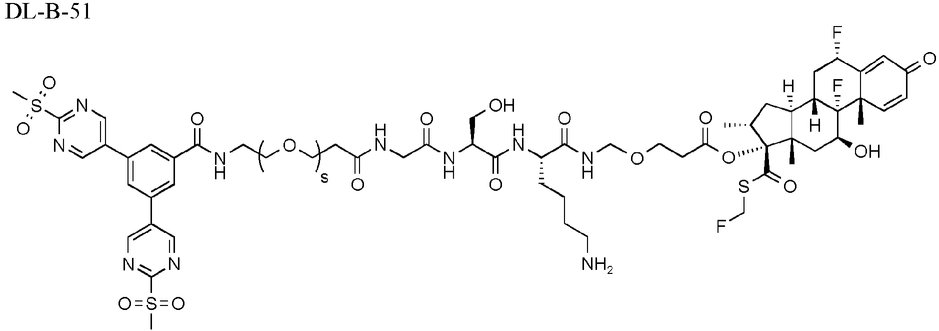 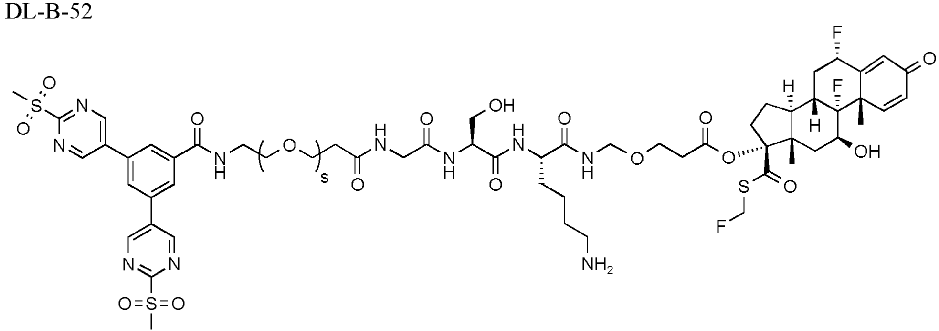 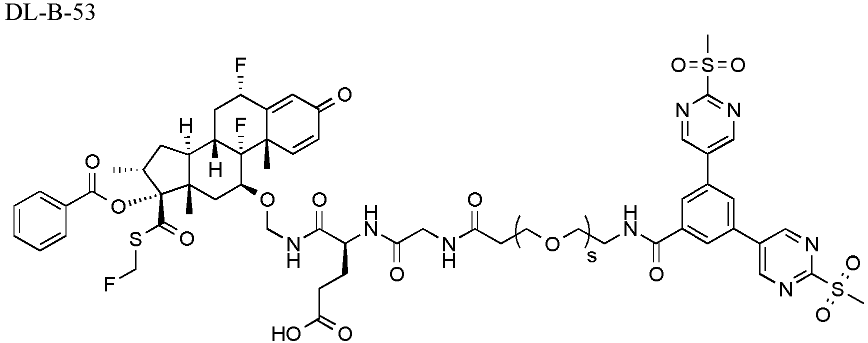 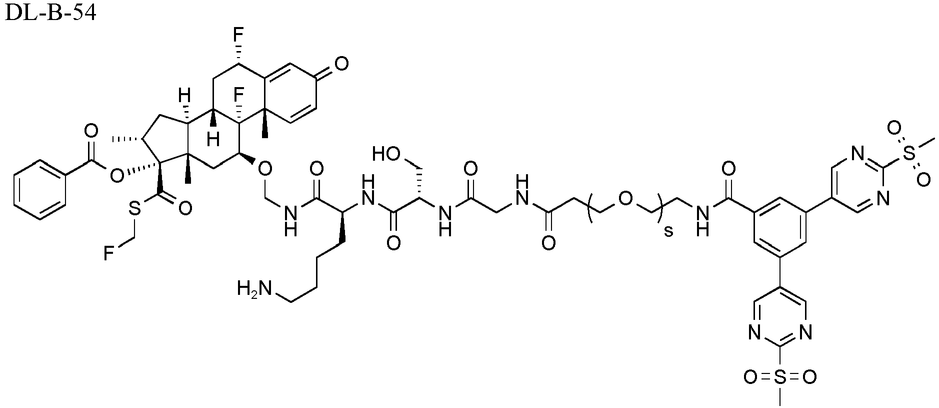 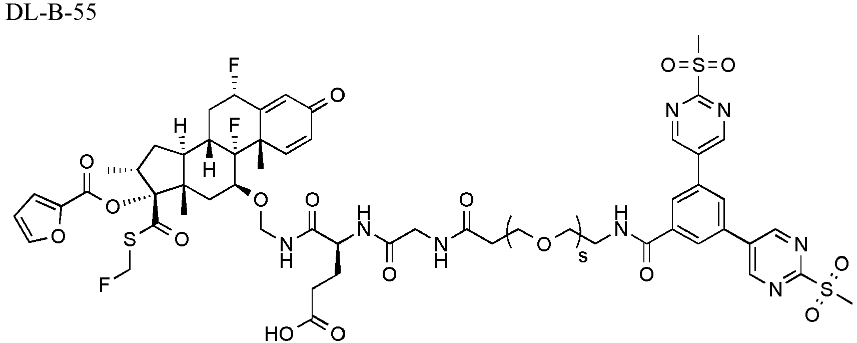 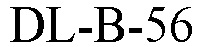 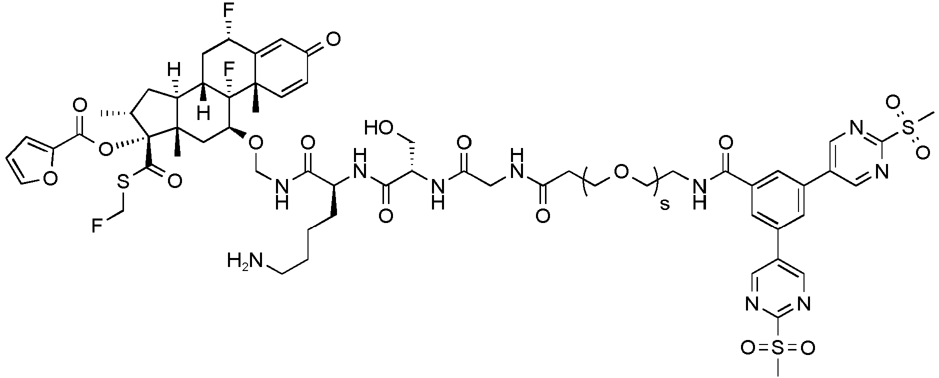 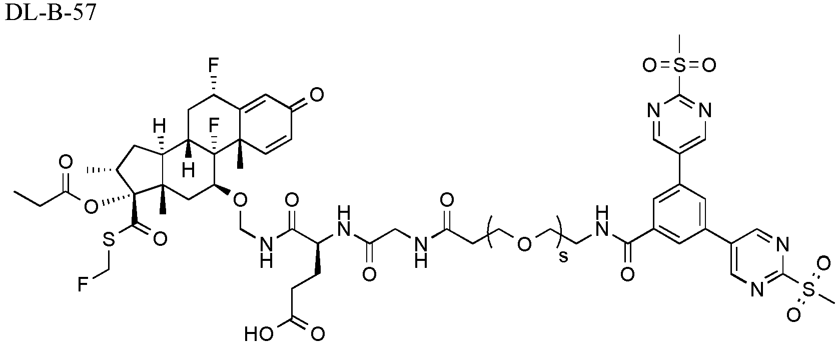 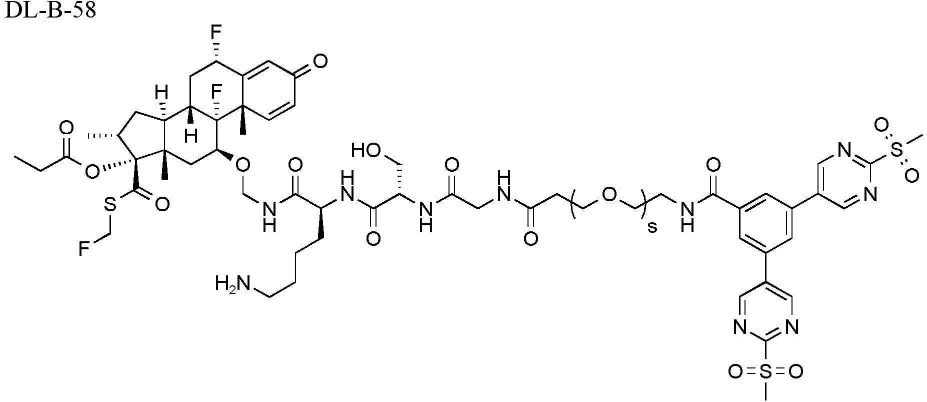 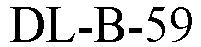 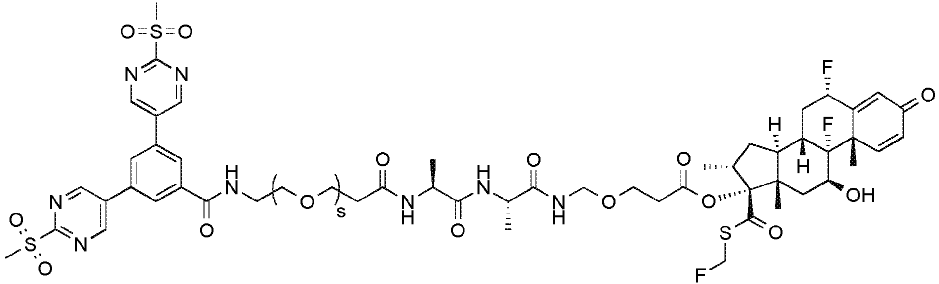 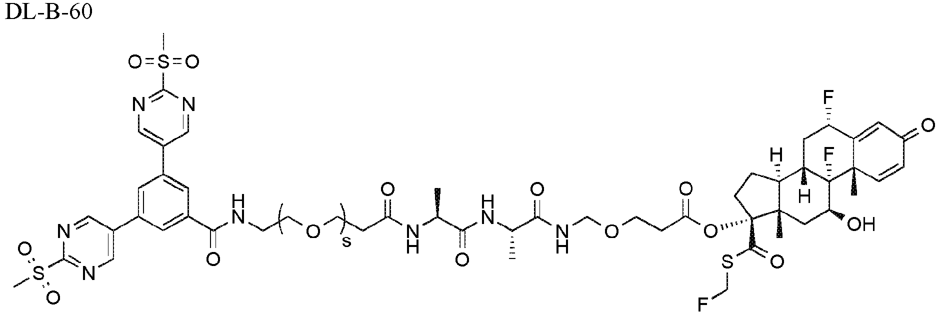 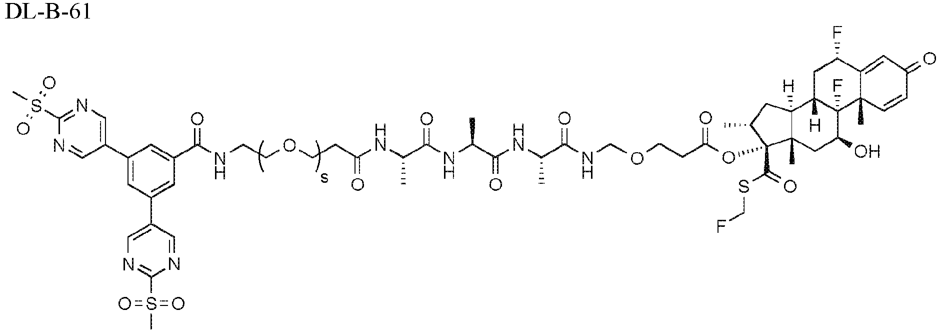 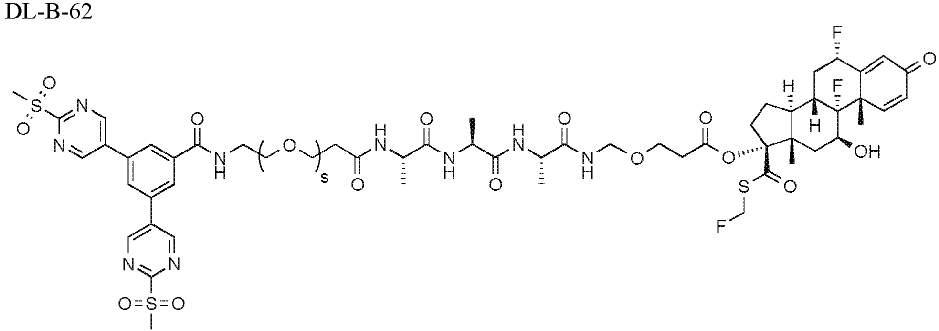 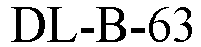 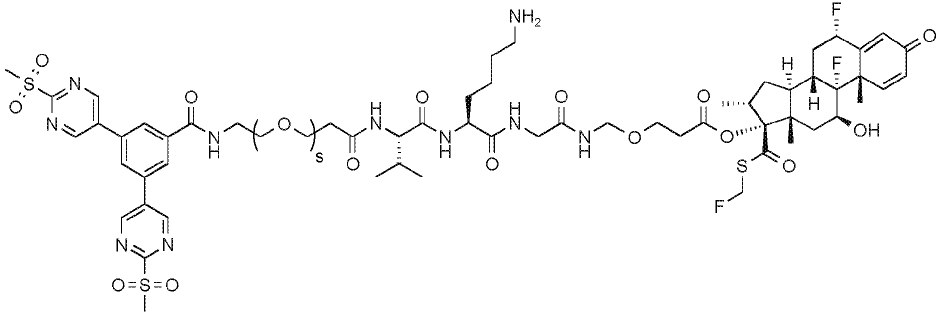 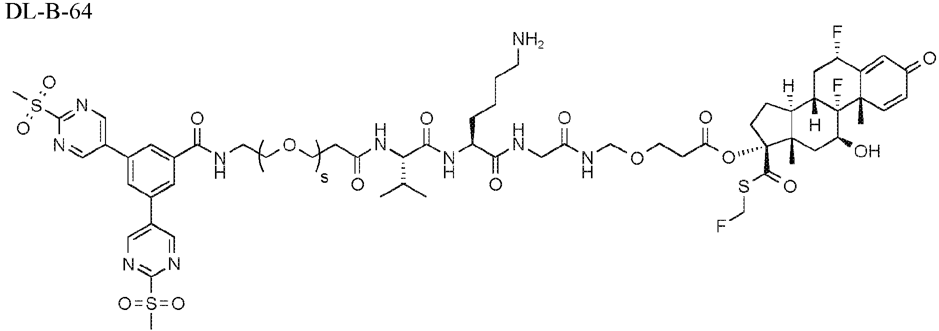 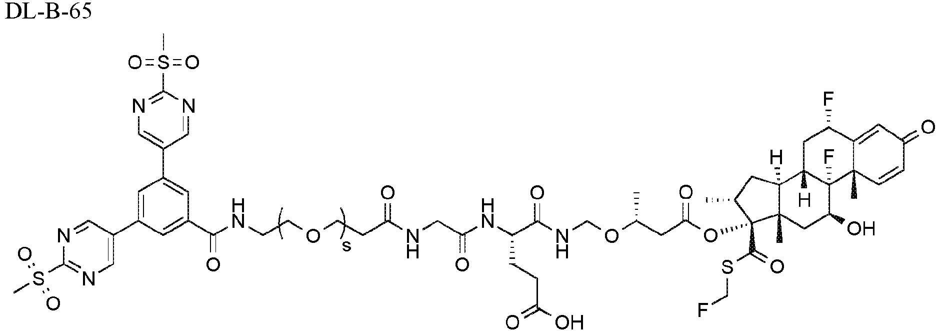 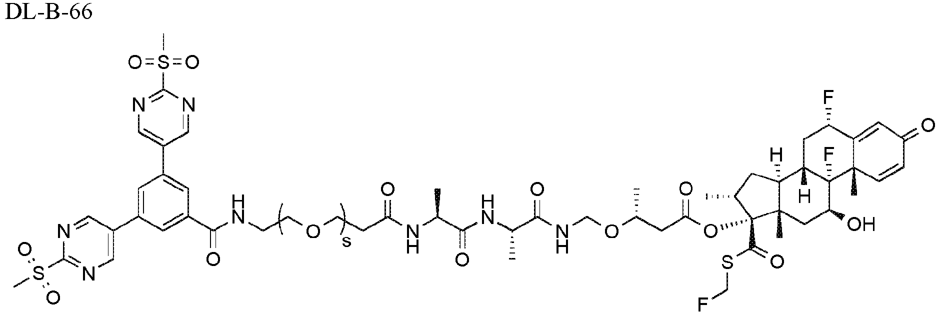 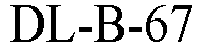 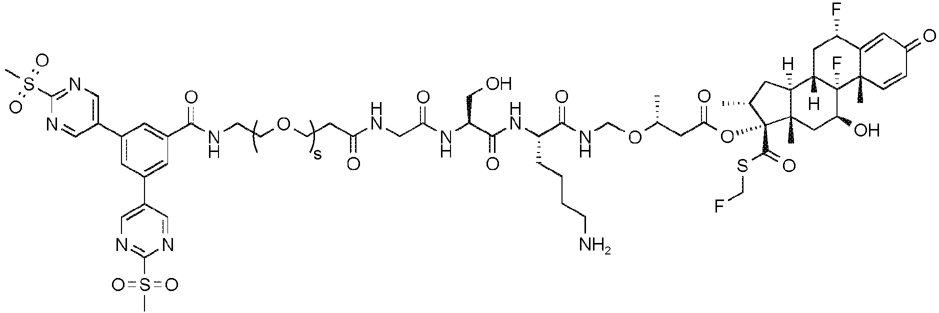 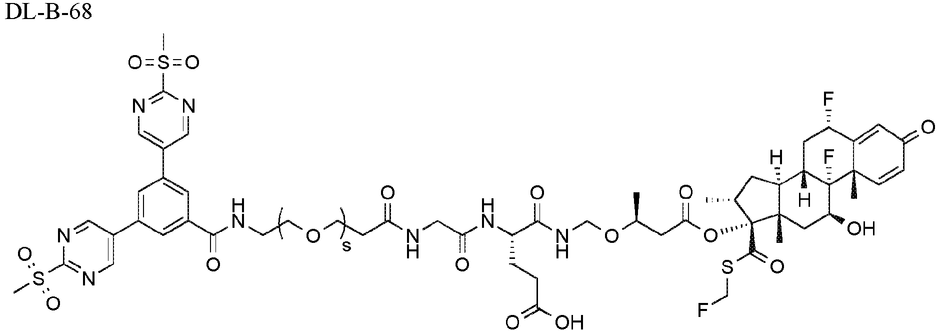 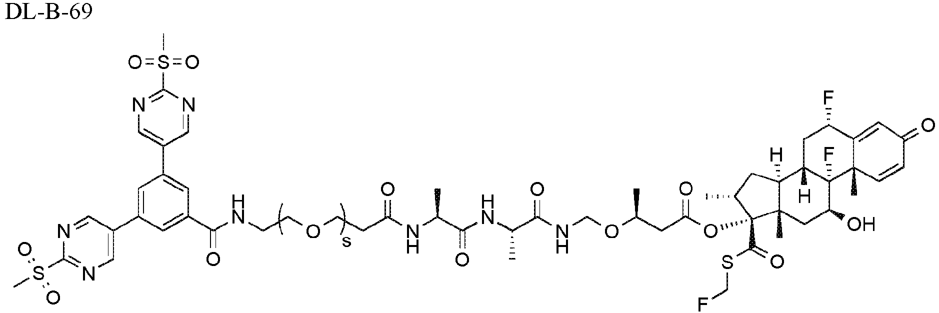 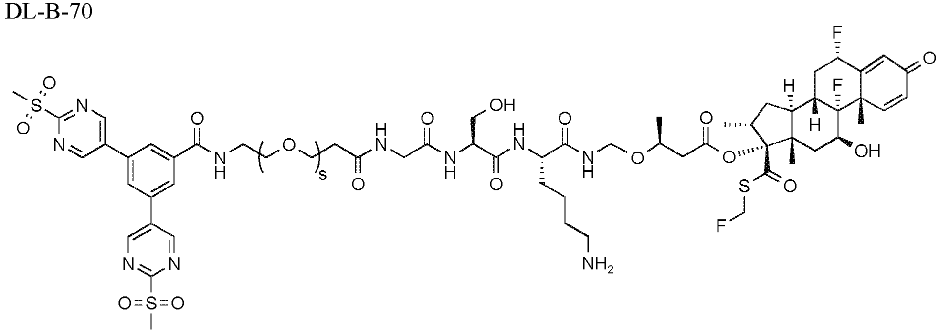 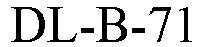 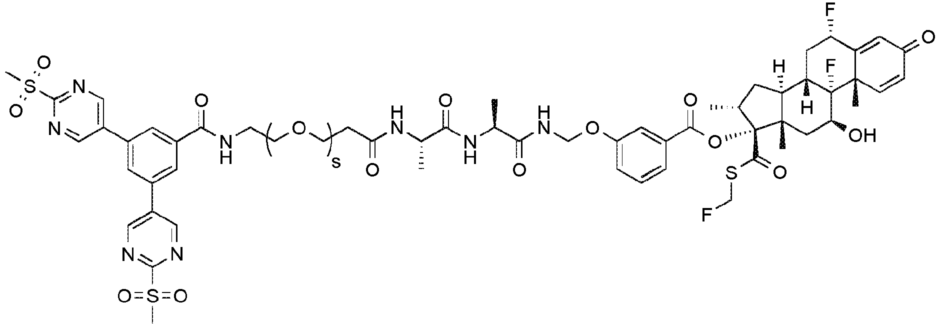 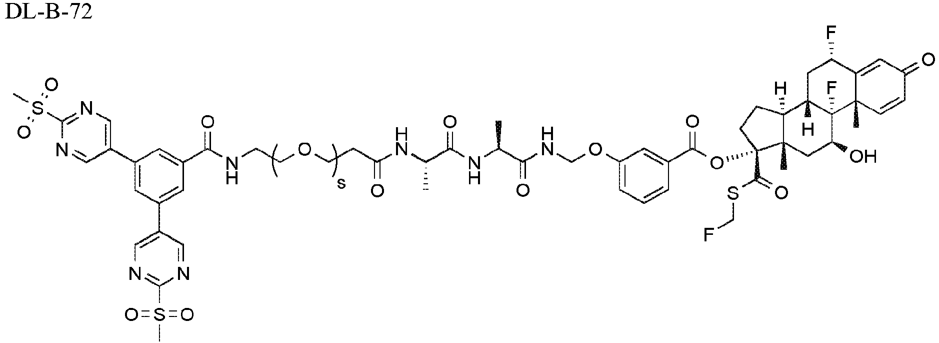 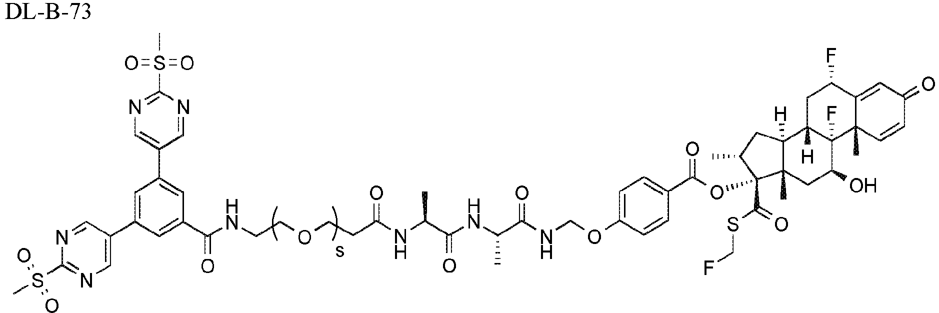 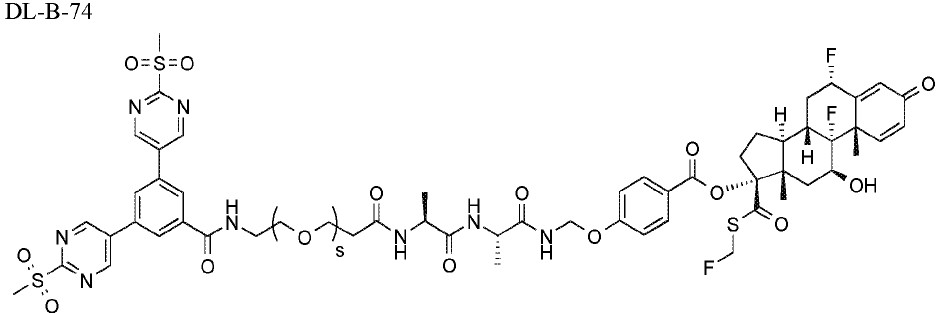 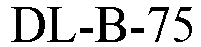 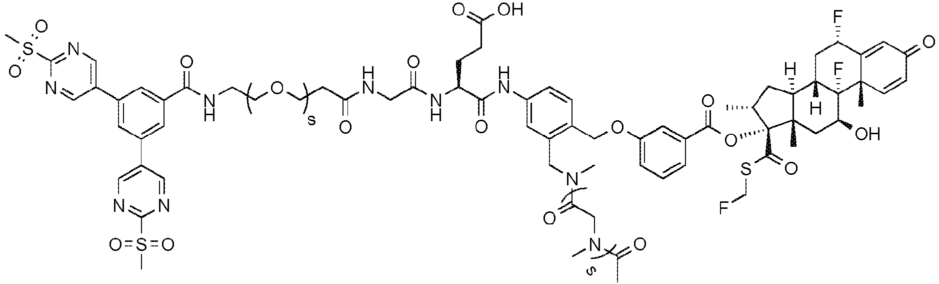 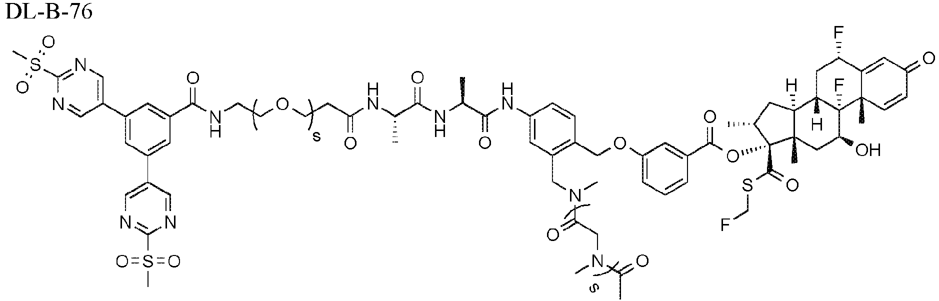 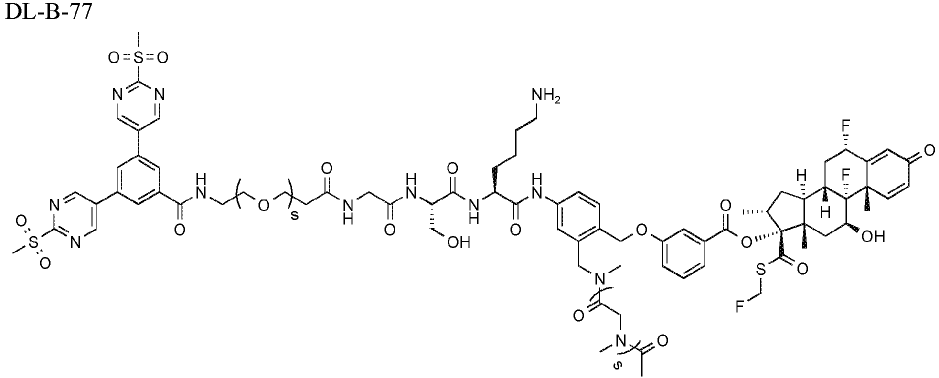 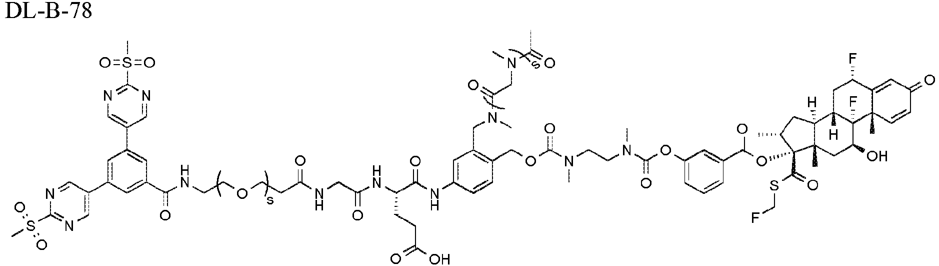 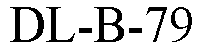 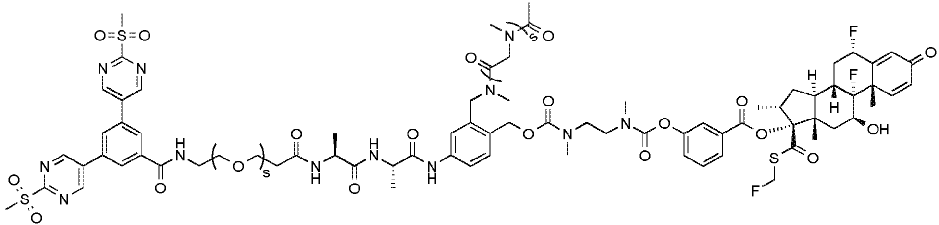 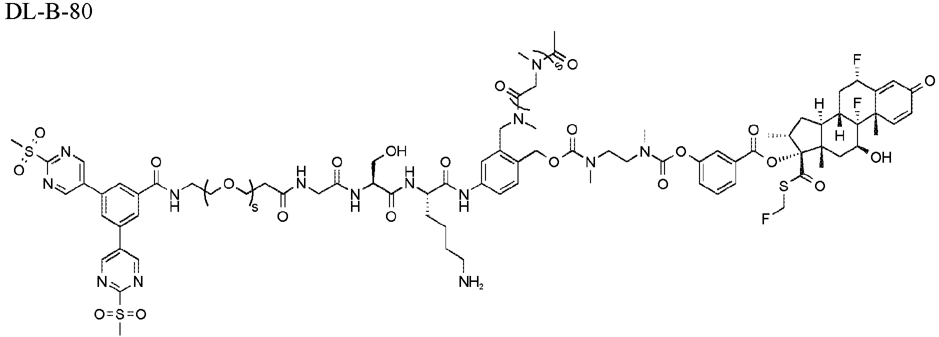 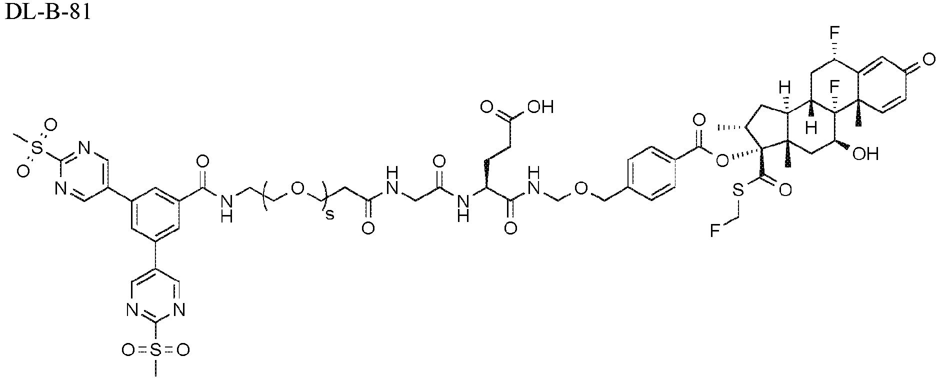 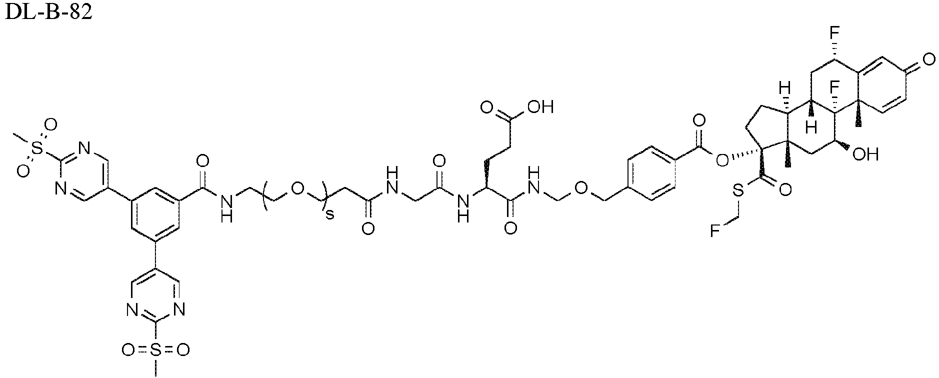 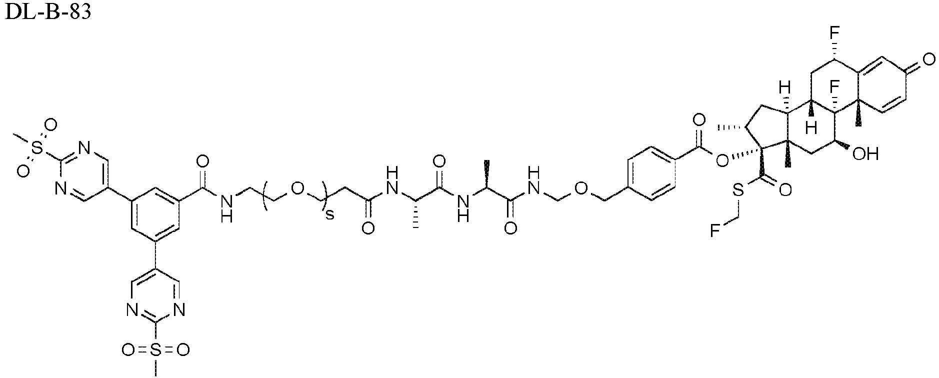 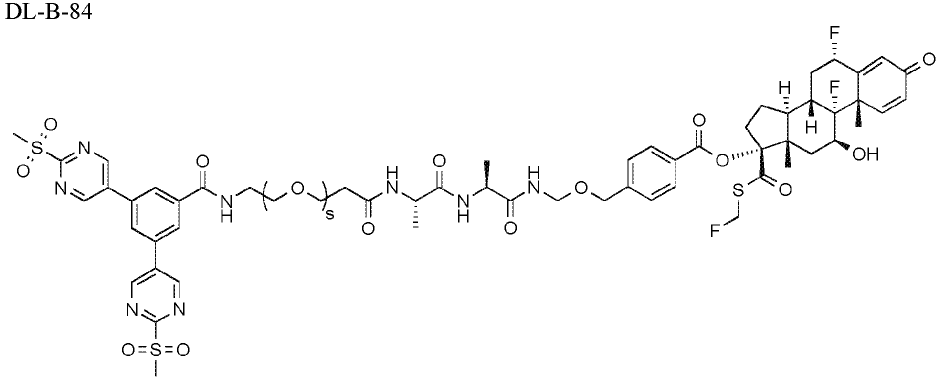 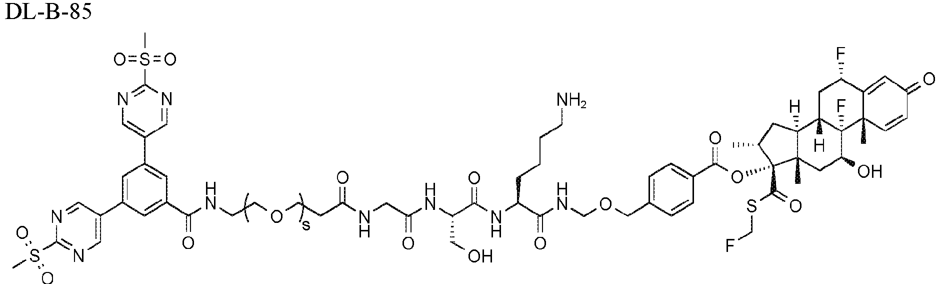 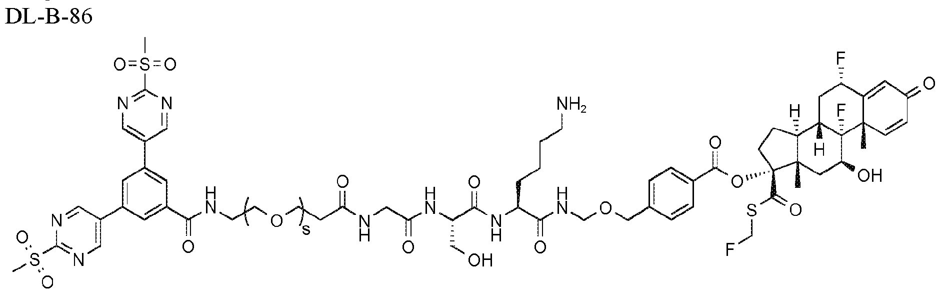 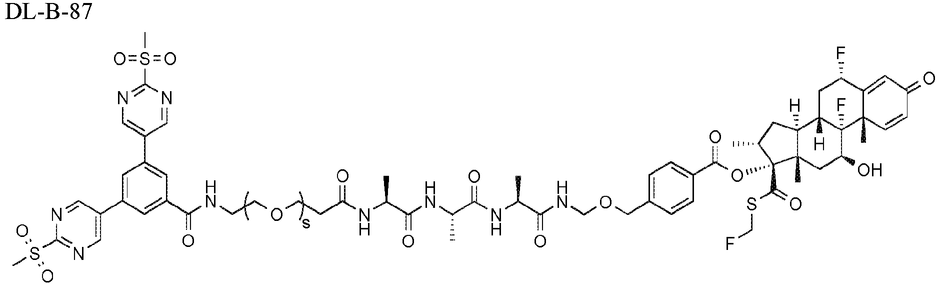 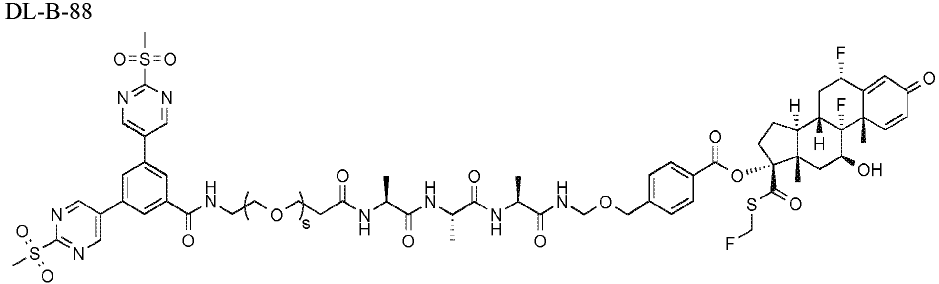 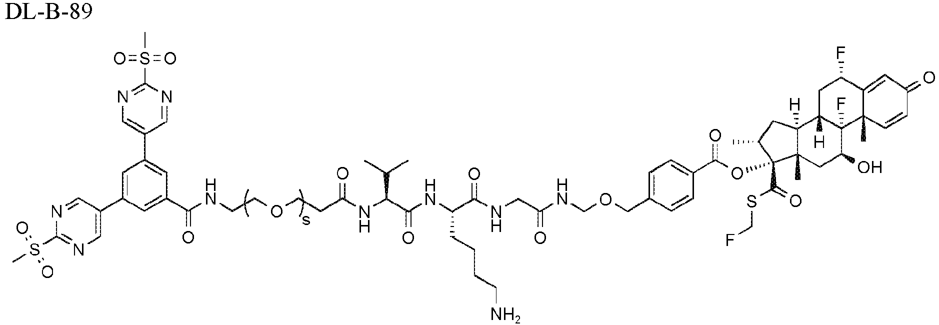 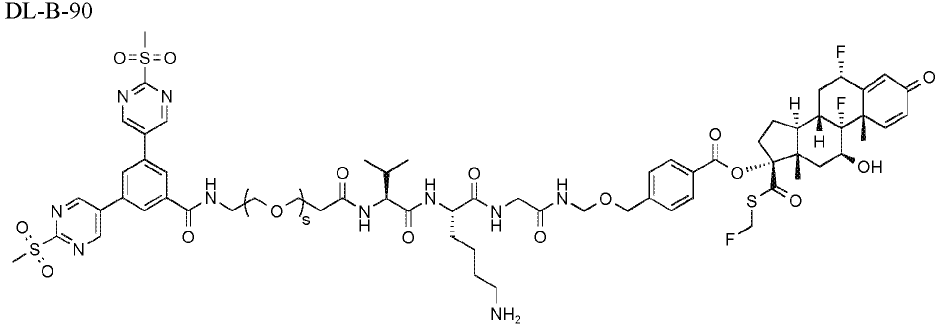 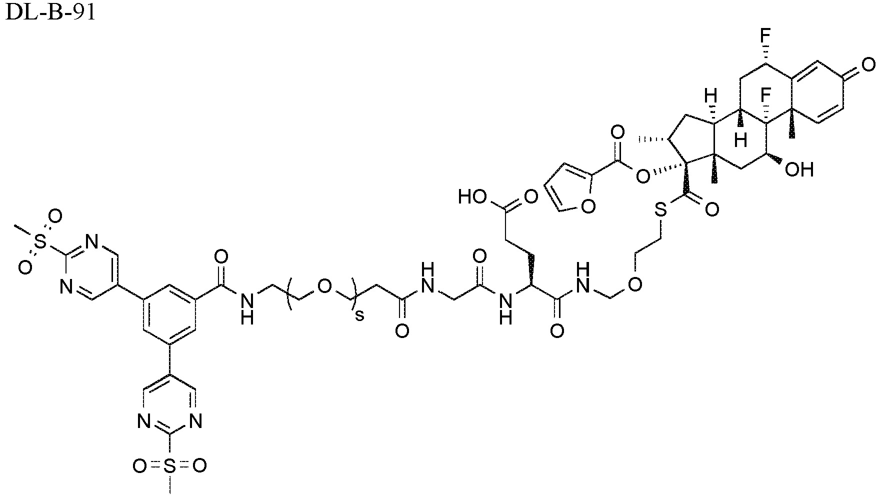 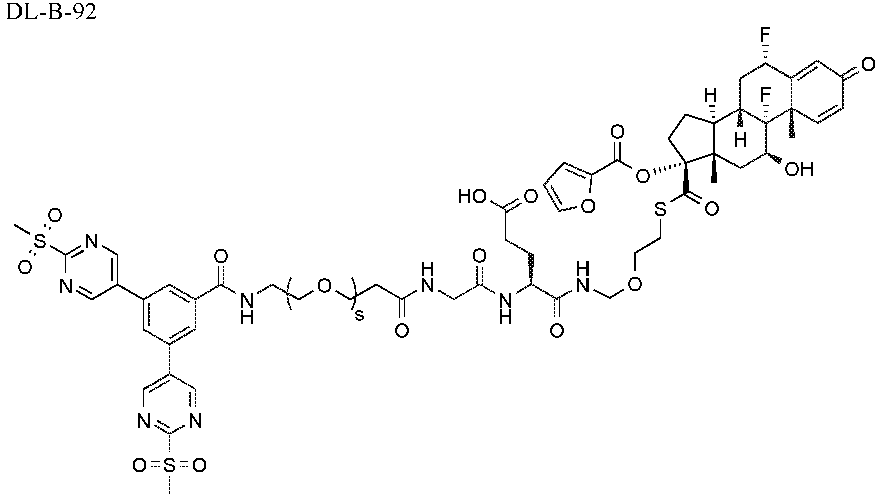 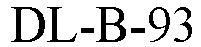 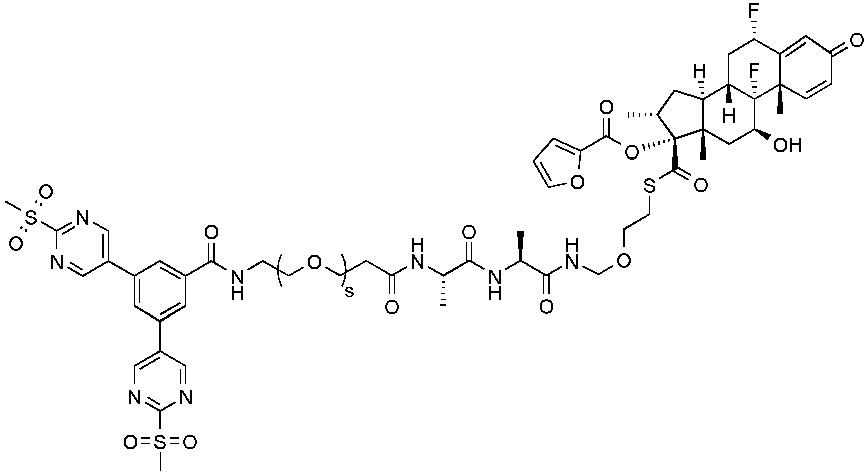 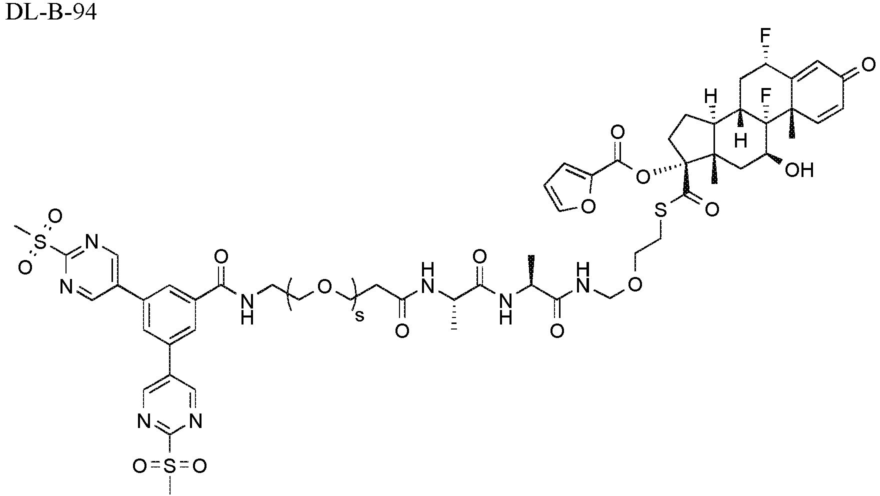 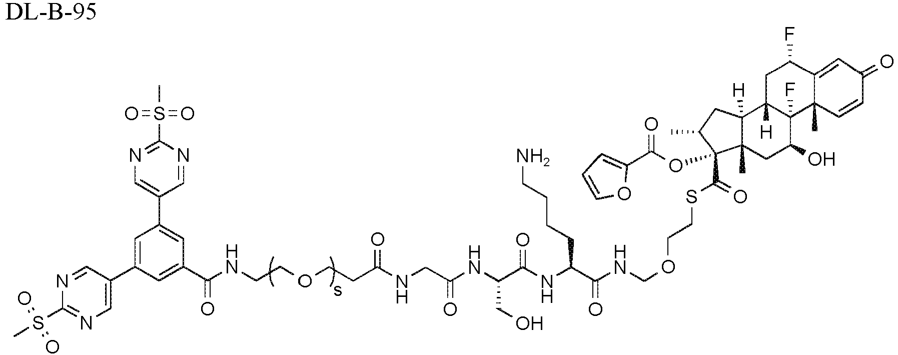 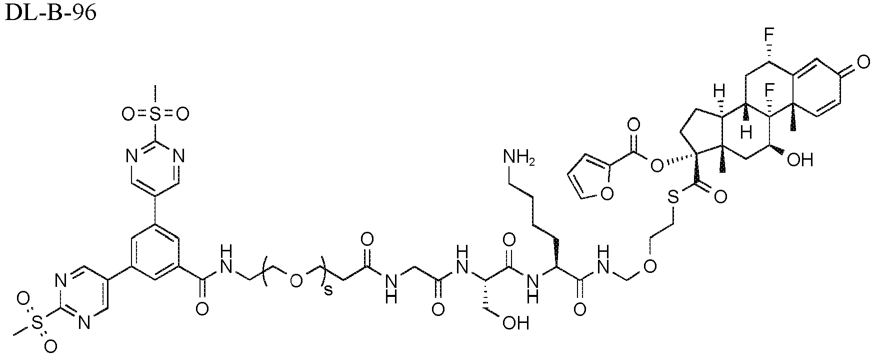 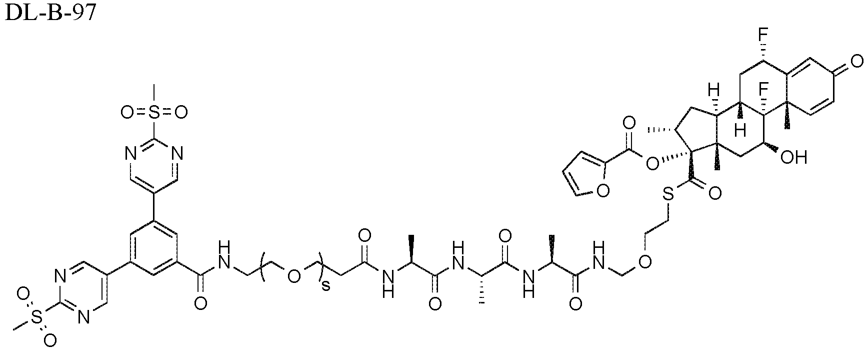 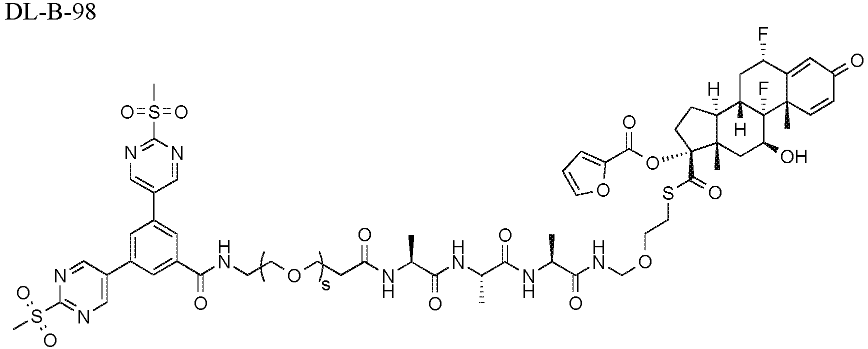 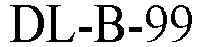 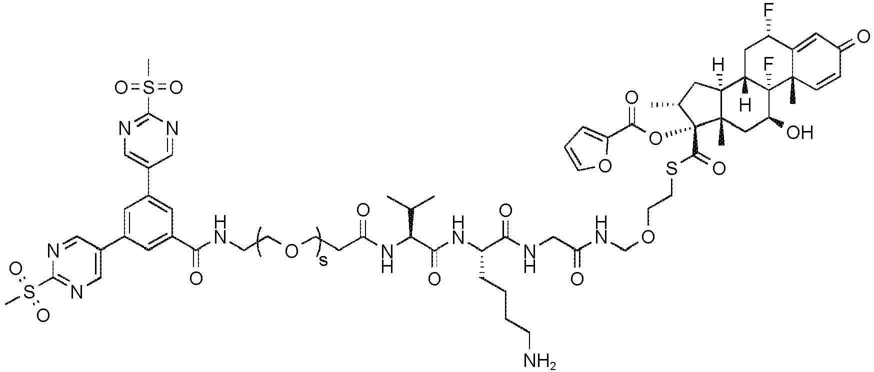 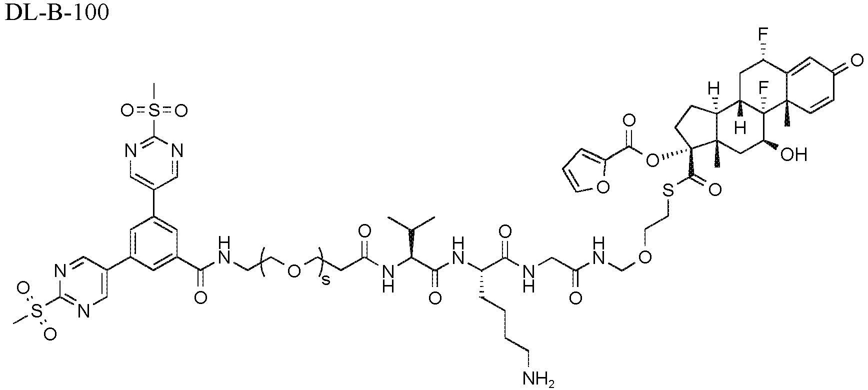 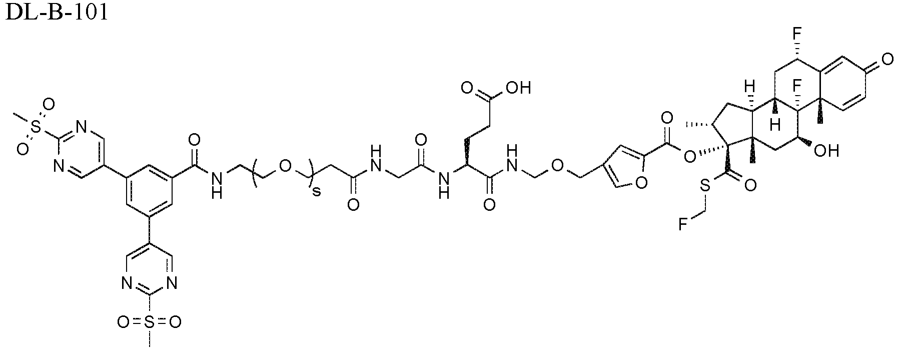 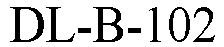 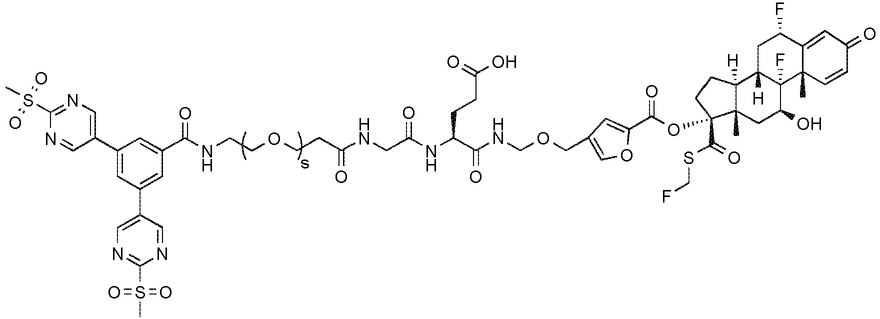 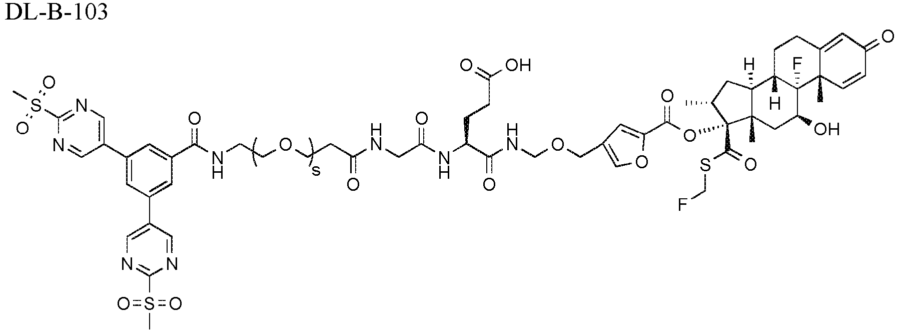 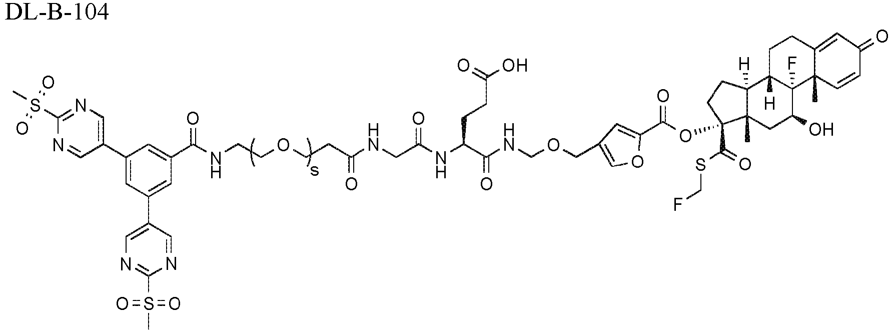 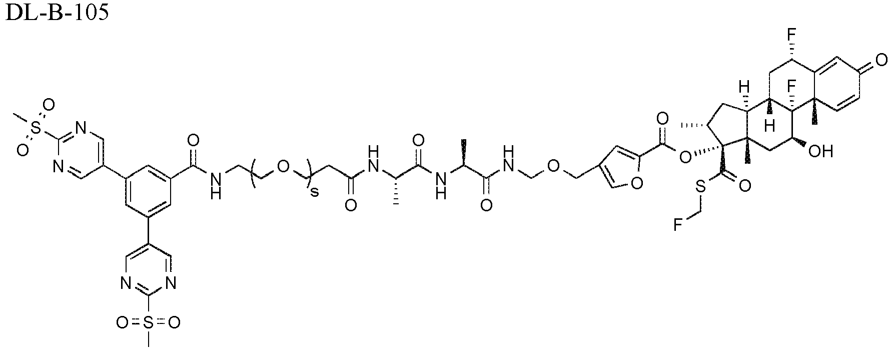 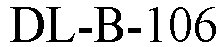 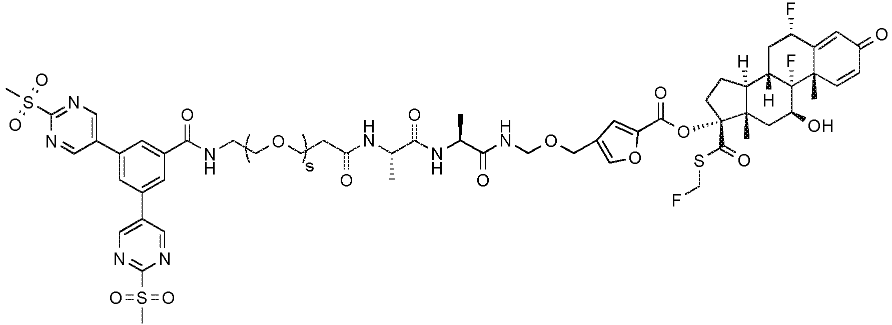 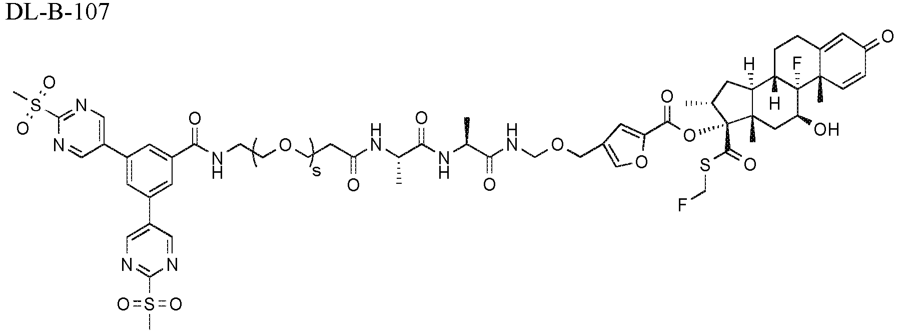 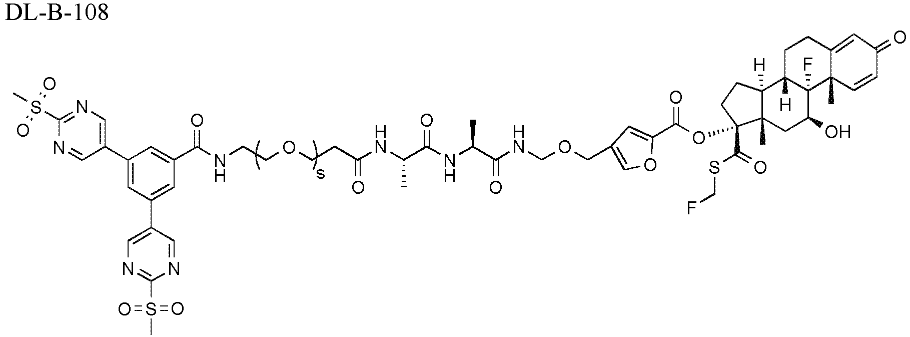 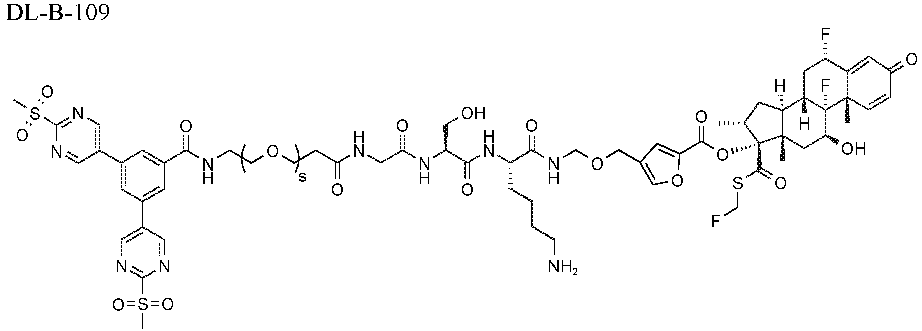 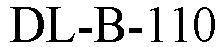 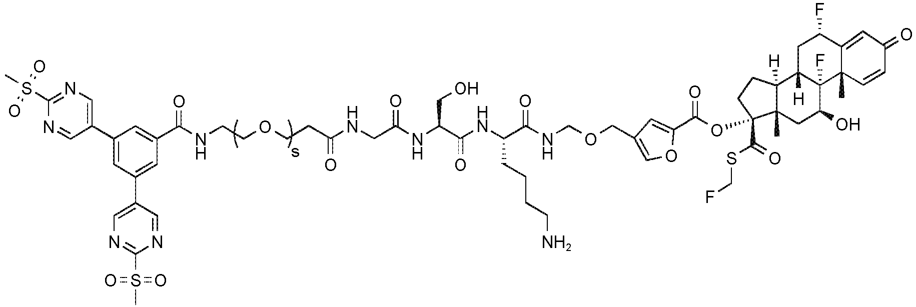 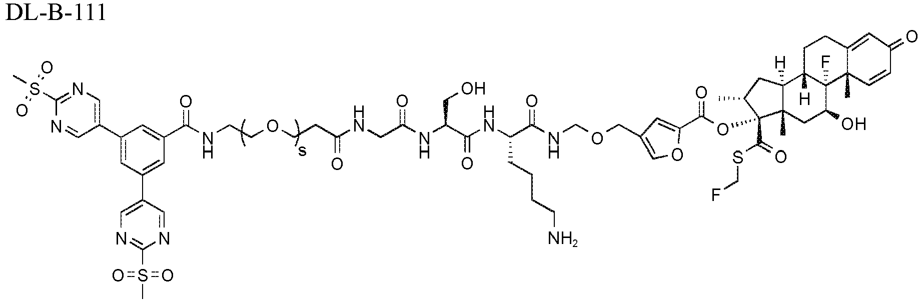 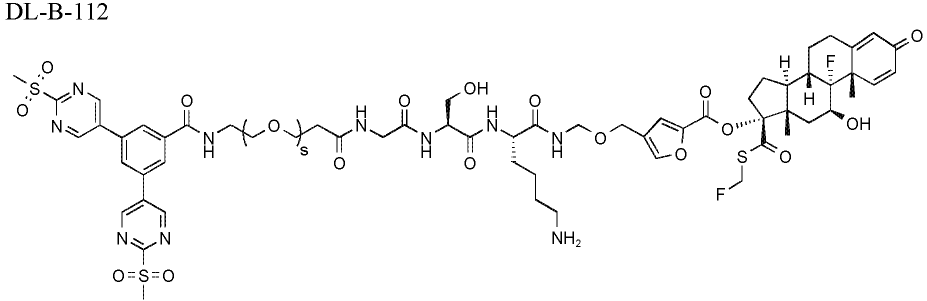 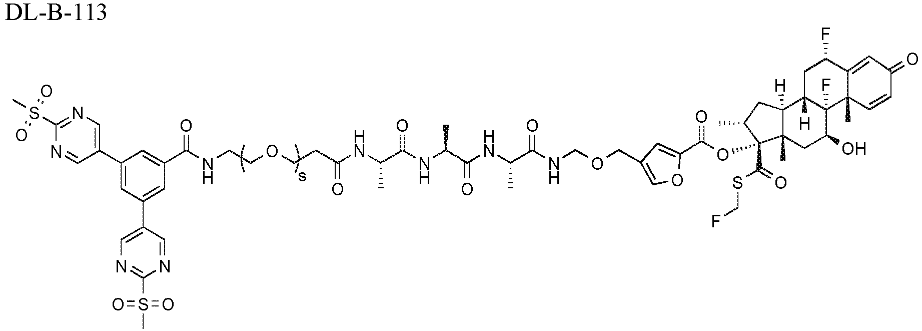 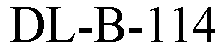 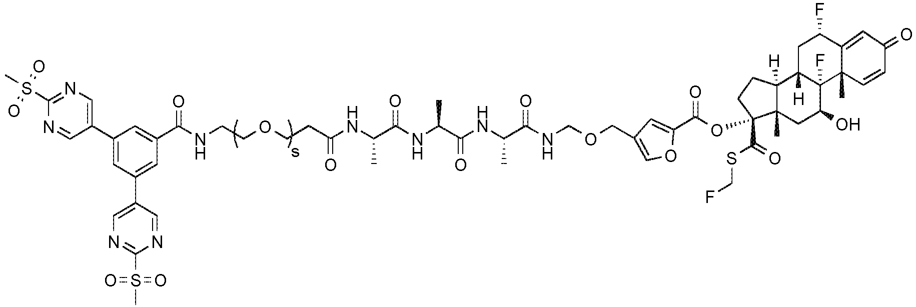 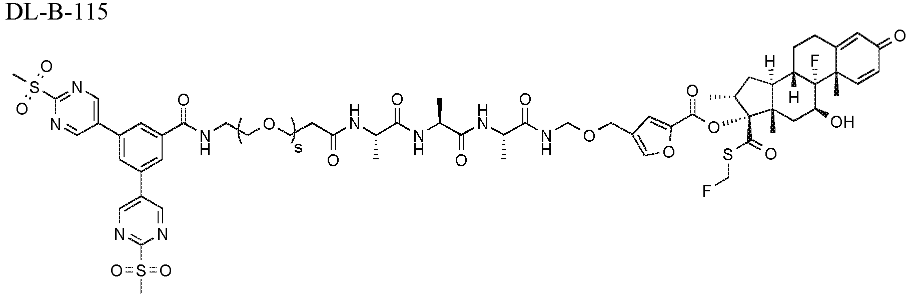 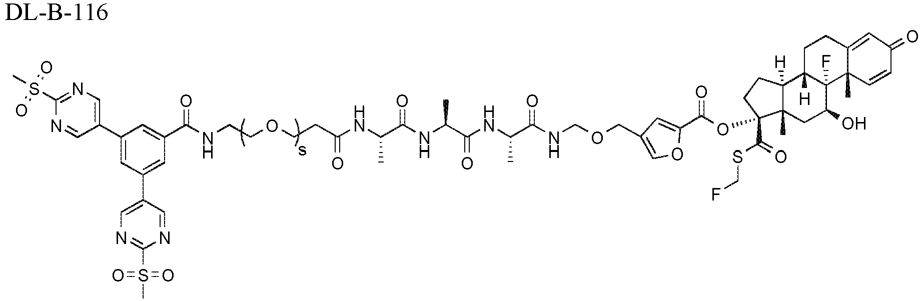 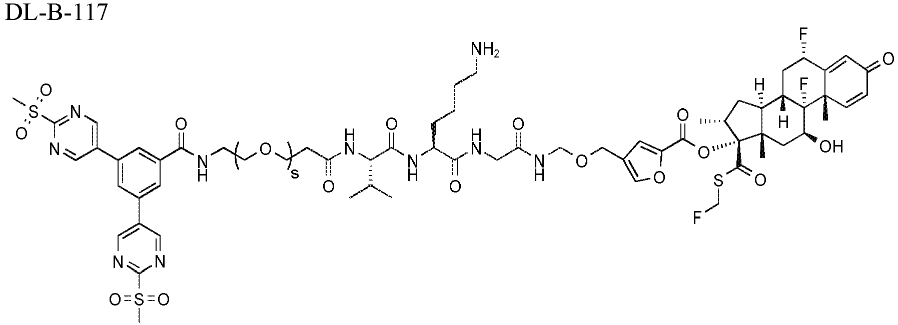 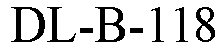 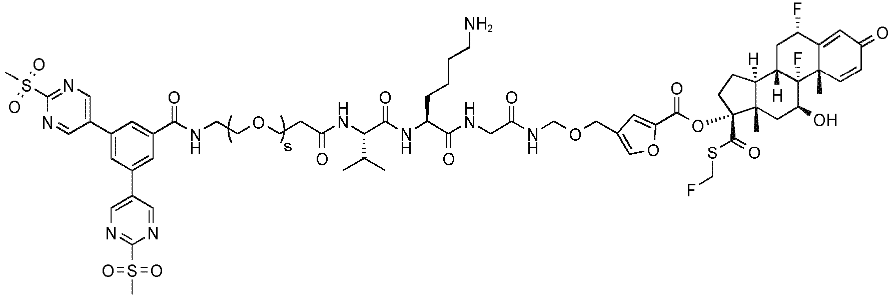 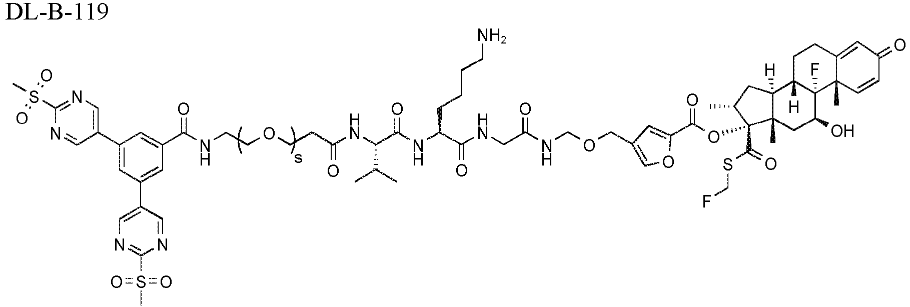 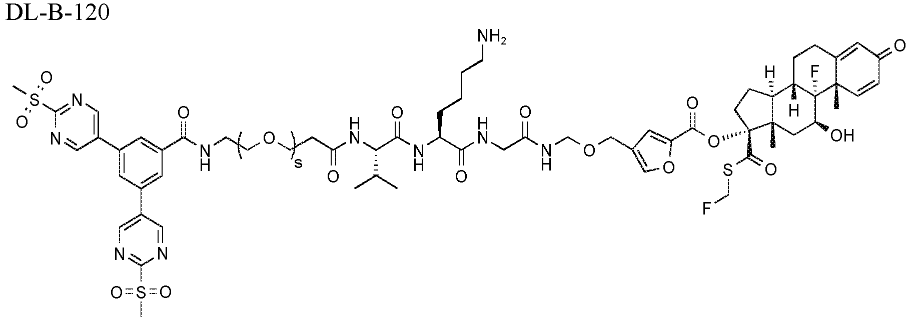 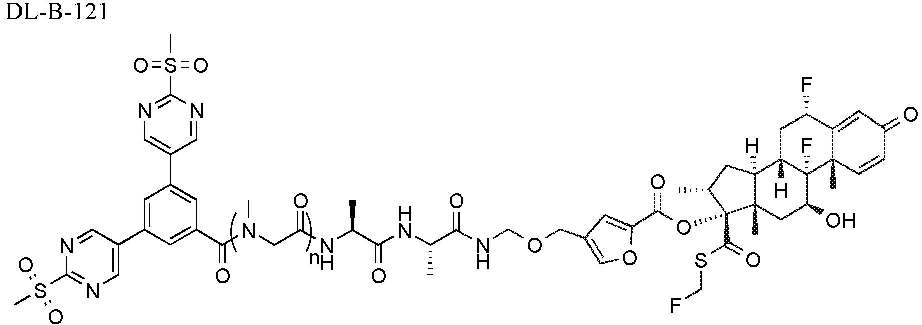 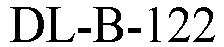 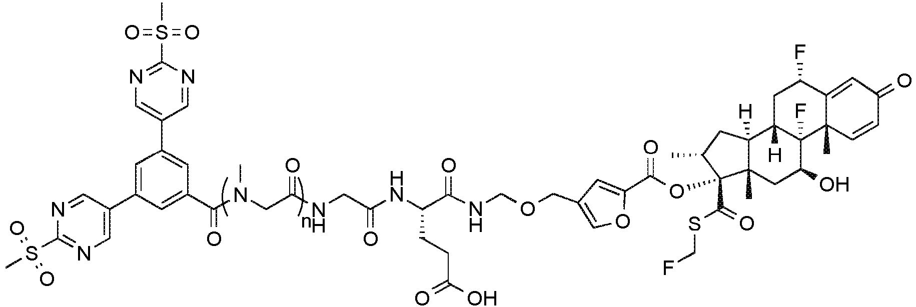 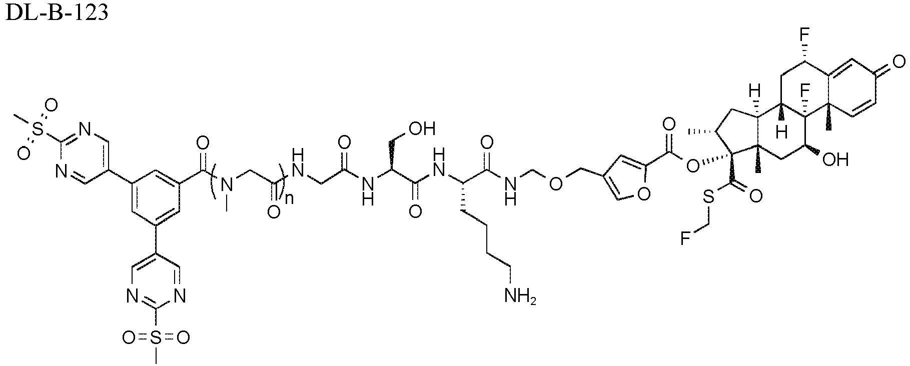 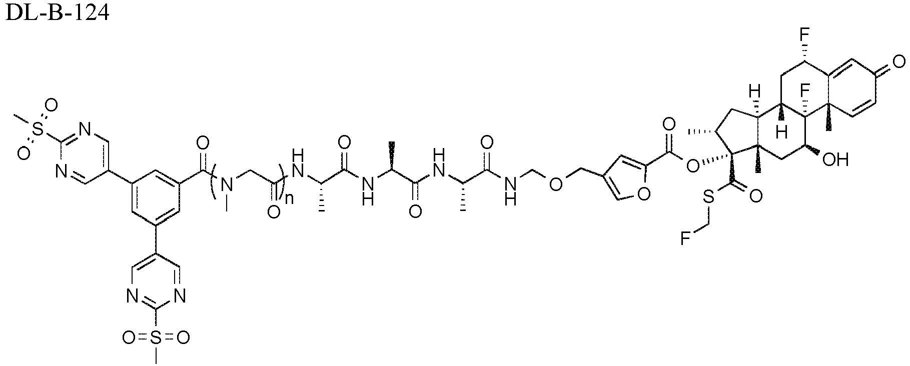 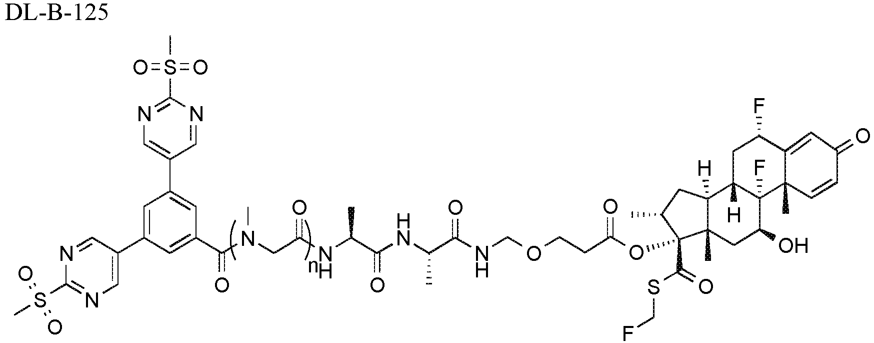 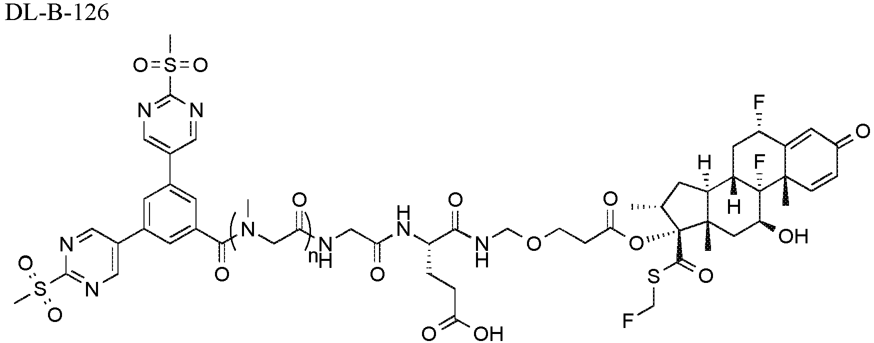 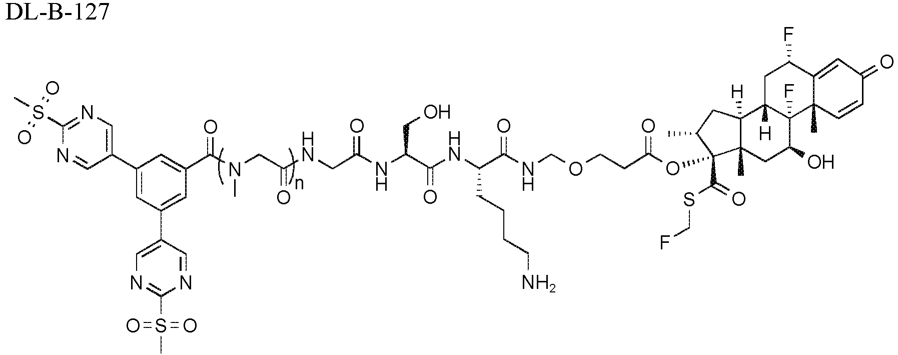 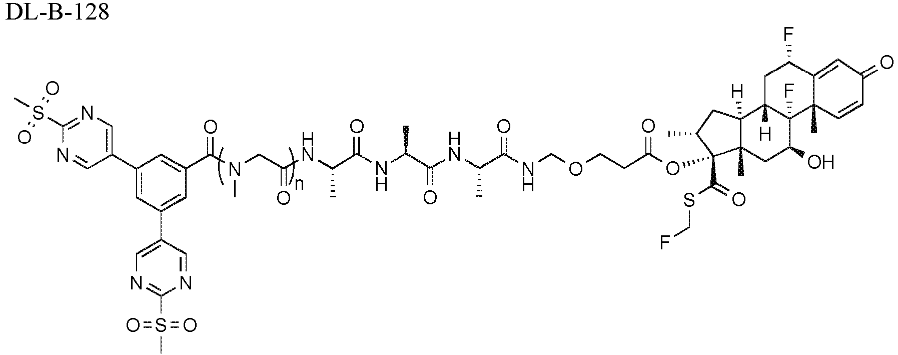 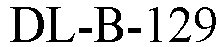 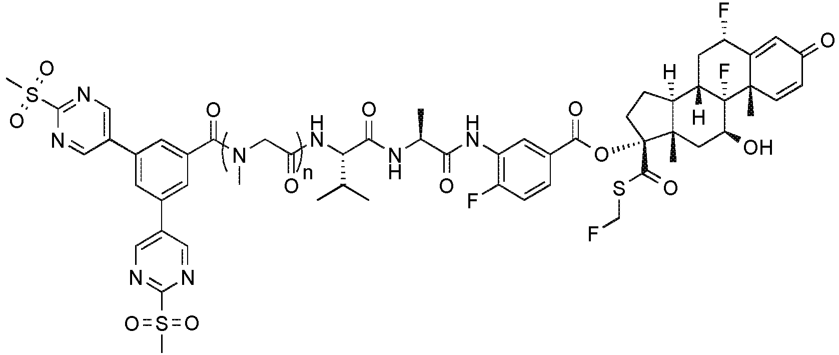 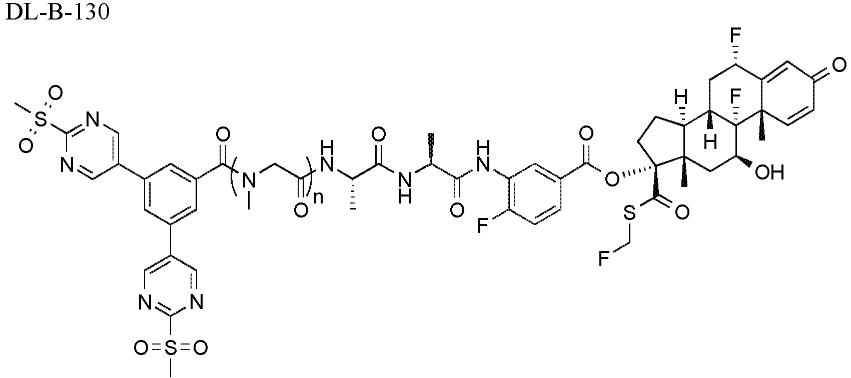 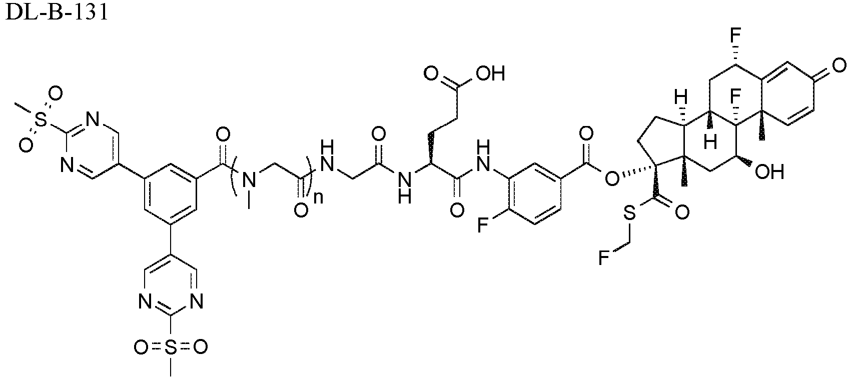 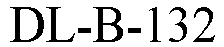 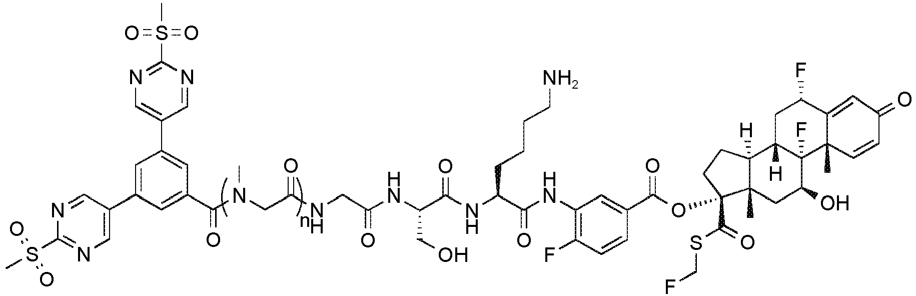 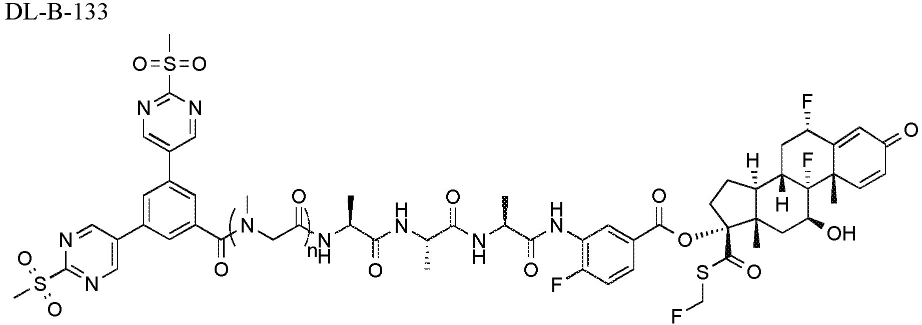 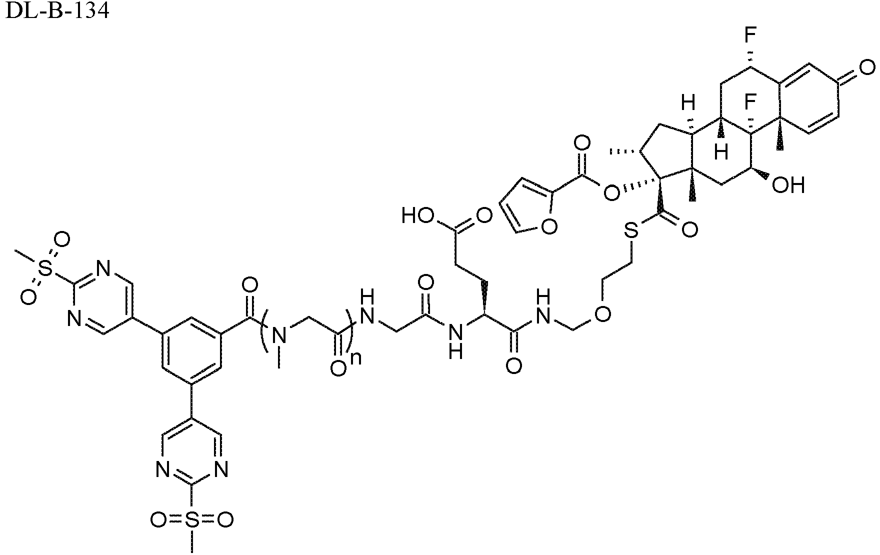 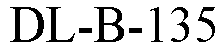 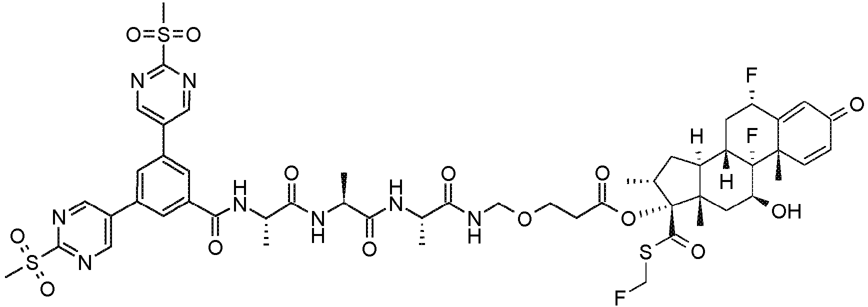 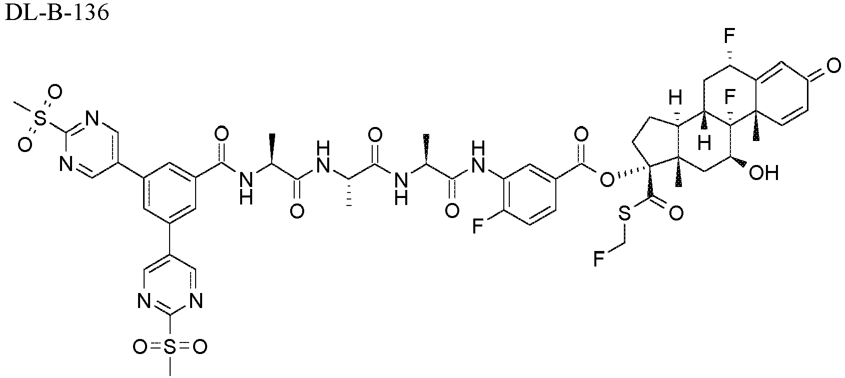 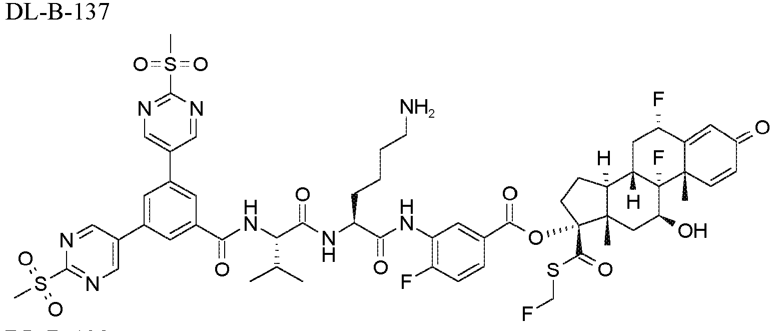 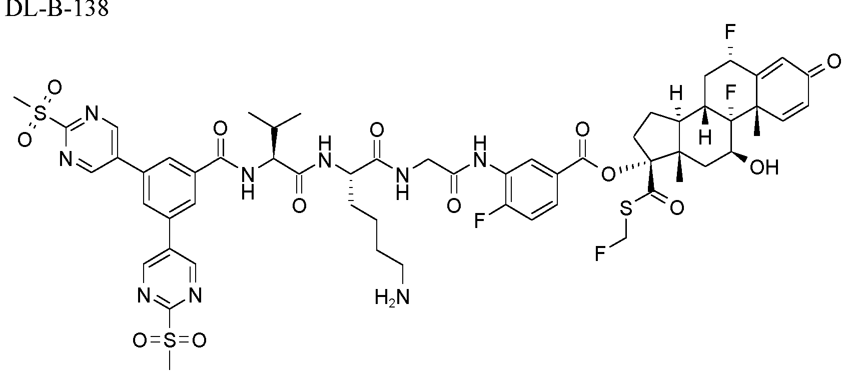 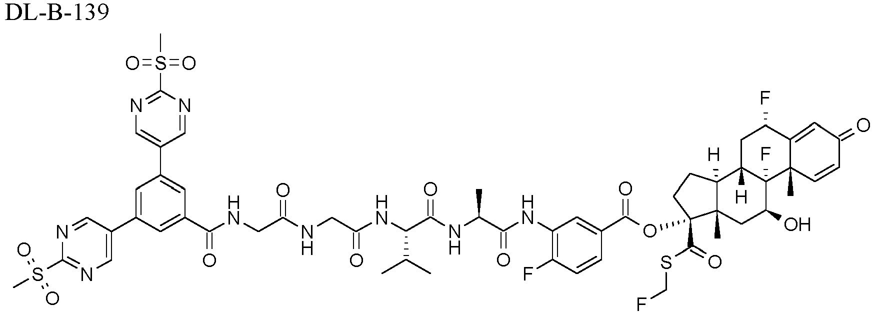 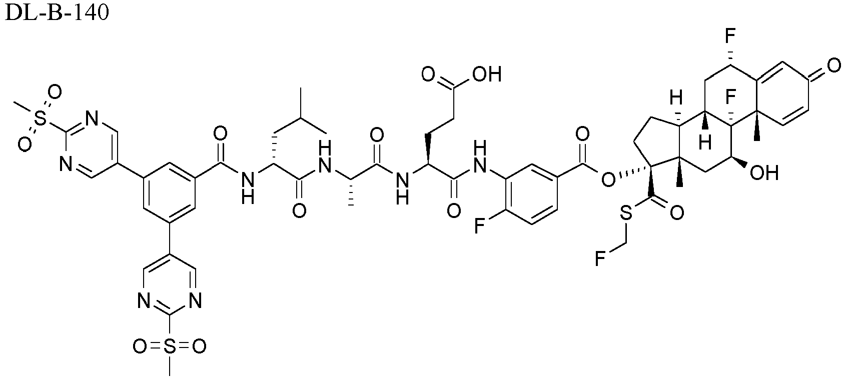 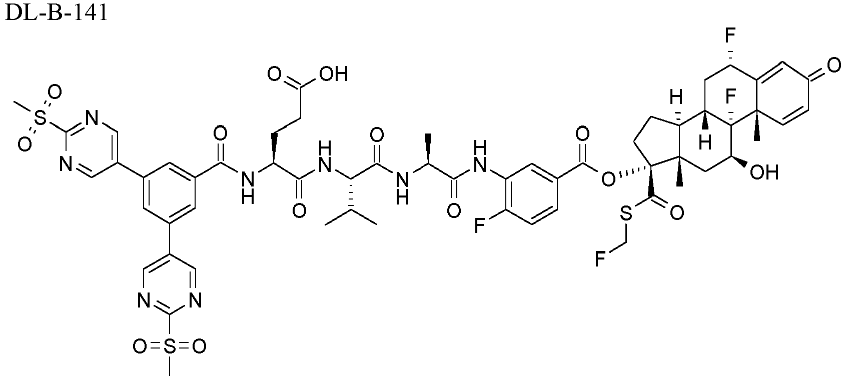 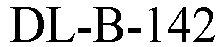 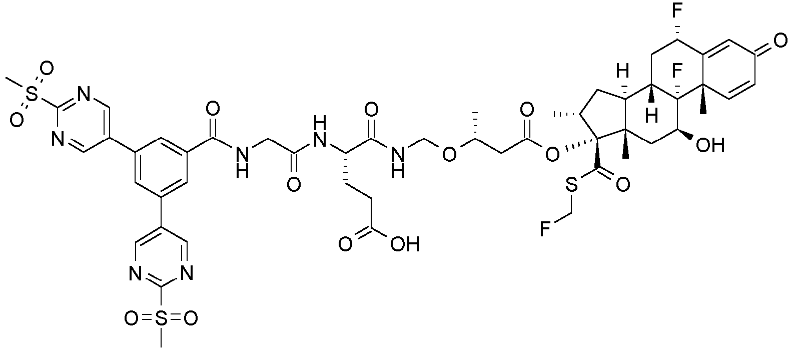 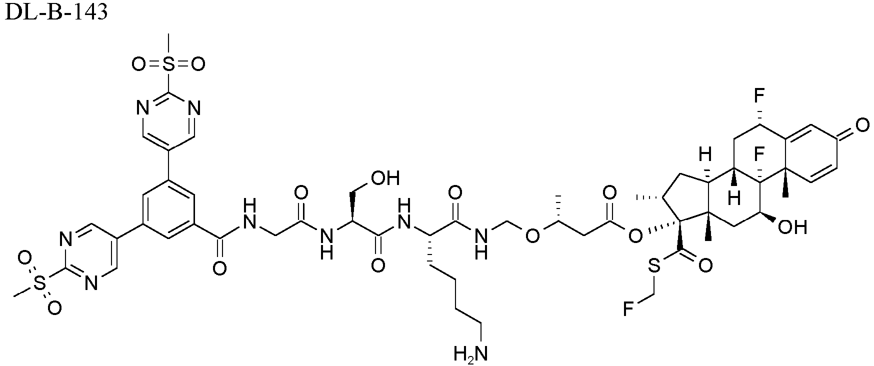 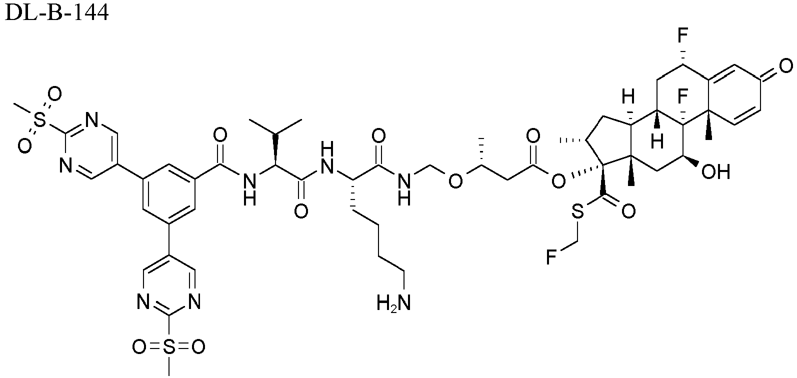 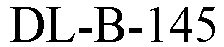 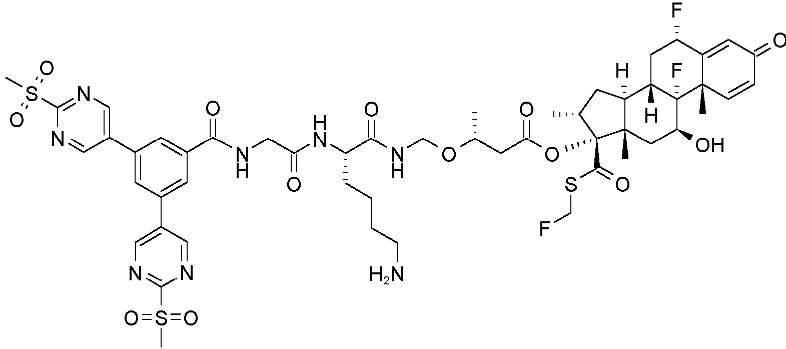 and 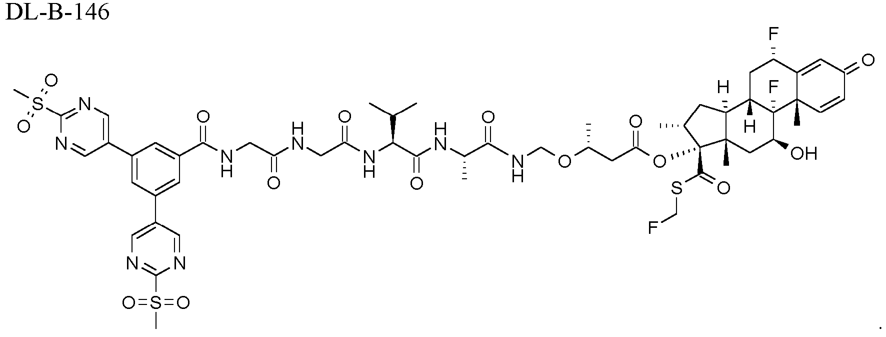 wherein,
s is an integer selected from 1 to 20; and
n is an integer selected from 0 to 20.

21. A pharmaceutical composition, comprising the compound, pharmaceutically acceptable salt, stereisomer, tautomer, polymorph, solvate, N-oxide, isotope labeled compound, metabolite or prodrug thereof according to any one of claims 1-8, the antibody-drug conjugate according to any one of claims 9-18 or the drug-linker compound according to claim 19 or 20, and one or more pharmaceutically acceptable carriers.

22. Use of the compound, pharmaceutically acceptable salt, stereisomer, tautomer, polymorph, solvate, N-oxide, isotope labeled compound, metabolite or prodrug thereof according to any one of claims 1-8, the antibody-drug conjugate according to any one of claims 9-18, the drug-linker compound according to claim 19 or 20 or the pharmaceutical composition according to claim 21 in the manufacture of a medicament for treating inflammatory or immune diseases;
preferably, the inflammatory or immune diseases are selected from a group consisting of rheumatoid arthritis, idiopathic arthritis, asthma, ulcerative colitis, optic neuromyelitis and autoimmune liver diseases.

23. A method for preparing the compound according to any one of claims 1-8, comprising the following steps: 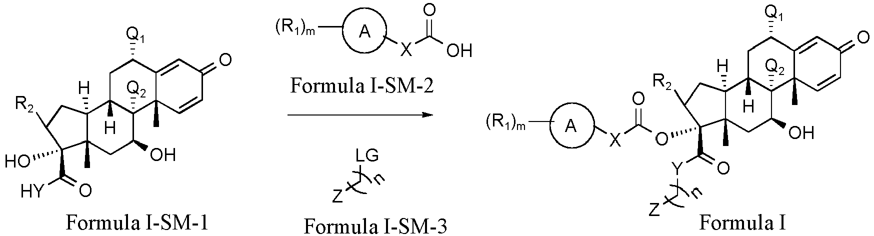
wherein, ring A, R₁, R₂, X, Y, Z, Q₁, Q₂, m and n are as defined in any one of claims 1-8; and
LG is a leaving group, such as halogen, methylsulfonyloxy or trifluoromethylsulfonyloxy, preferably iodine.

24. A method for preparing the drug-linker compound according to any one of claims 19-20, comprising the following steps: 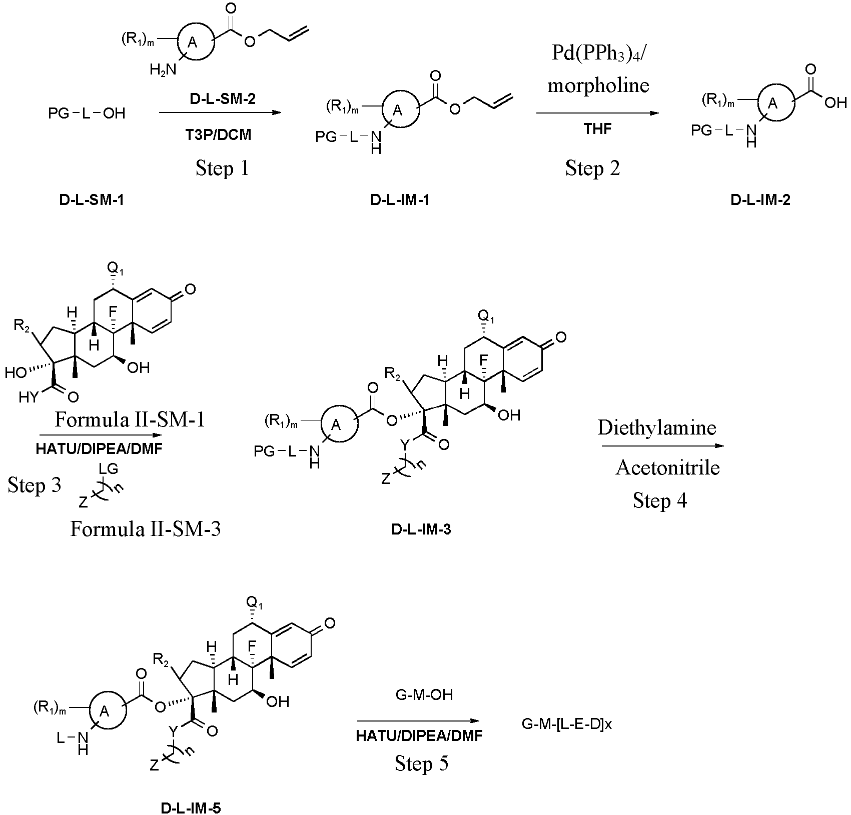 wherein,
ring A, R₁, R₂, X, Y, Z, Q₁, G, M, L, D, m and n are as defined in any one of claims 1-20;
x=1;
E is a single bond;
LG is a leaving group, such as halogen, methylsulfonyloxy or trifluoromethylsulfonyloxy, preferably iodine; and
PG is an amino protecting group, such as 9-fluorenylmethoxycarbonyl, tert-butoxycarbonyl, p-methoxytriphenylmethyl and allyloxycarbonyl, preferably 9-fluorenylmethoxycarbonyl.

25. A method for preparing the antibody-drug conjugate according to any one of claims 9-18, which is selected from a group consisting of:
conjugation method A:
conjugating the drug-linker compound according to any one of claims 19-20 with an antibody in a molar ratio of (8-10):1 to obtain the antibody-drug conjugate;
conjugation method B:
conjugating the drug-linker compound according to any one of claims 19-20 with an antibody in a molar ratio of (4-6):1 to obtain the antibody-drug conjugate;
conjugation method C:
conjugating the drug-linker compound according to any one of claims 19-20 with an antibody in a molar ratio of (4.0-4.5):1 to obtain the antibody-drug conjugate.
